# EUROPÄISCHE PATENTSCHRIFT

(11) **EP 3 455 224 B1**
(45) Veröffentlichungstag und Bekanntmachung des Hinweises auf die Patenterteilung: **27.03.2024**
(21) Anmeldenummer: 17723053.9
(22) Anmeldetag: 08.05.2017
(51) Int. Cl.: C07D 471/04, C07D 519/00, A61K 31/437, A61K 31/444, A61K 31/4375, A61K 31/4709, A61K 31/506, A61K 31/497, A61P 11/00, A61P 9/10, A61K 45/06

(54) **SUBSTITUIERTE 5,6,7,8-TETRAHYDRO[1,2,4]TRIAZOLO[4,3-A]PYRIDIN-3(2H)-ONE UND 2,5,6,7-TETRAHYDRO-3H-PYRROLO[2,1-C][1,2,4]TRIAZOL-3-ONE UND IHRE VERWENDUNG**
SUBSTITUTED 5,6,7,8-TETRAHYDRO[1,2,4]TRIAZOLO[4,3-A]PYRIDINE-3(2H)-ONES AND 2,5,6,7-TETRAHYDRO-3H-PYRROLO[2,1-C][1,2,4]TRIAZOL-3-ONES, AND USE THEREOF
5,6,7,8-TÉTRAHYDRO[1,2,4]TRIAZOLO[4,3- A]PYRIDIN-3(2H)-ONES ET 2,5,6,7-TÉTRAHYDRO-3H-PYRROLO[2,1 -C][1,2,4]TRIAZOL-3-ONES SUBSTITUÉES ET LEUR UTILISATION

(30) Priorität: 09.05.2016 WO PCT/EP2016/168809
(43) Veröffentlichungstag der Anmeldung: 20.03.2019
(73) Patentinhaber: Bayer Aktiengesellschaft, 51373 Leverkusen (DE); Bayer Pharma Aktiengesellschaft, 13353 Berlin (DE)
(72) Erfinder: BIBER, Nicole, 42115 Wuppertal (DE); BROCKSCHNIEDER, Damian, 42781 Haan (DE); GERICKE, Kersten Matthias, 42115 Wuppertal (DE); KÖLLING, Florian, 42115 Wuppertal (DE); LUSTIG, Klemens, 42113 Wuppertal (DE); MEDING, Jörg, 42115 Wuppertal (DE); MEIER, Heinrich, 42115 Wuppertal (DE); NEUBAUER, Thomas, 42111 Wuppertal (DE); SCHÄFER, Martina, 13465 Berlin (DE); TIMMERMANN, Andreas, 40237 Düsseldorf (DE); ZUBOV, Dmitry, 42857 Remscheid (DE); TERJUNG, Carsten, 44799 Bochum (DE); LINDNER, Niels, 42115 Wuppertal (DE); BADOCK, Volker, 13086 Berlin (DE); MOOSMAYER, Dieter, 10115 Berlin (DE); MIYATAKE ONDOZABAL, Hideki, 10115 Berlin (DE); MOORE, Stephen, Sheffield S118RZ (GB); SCHULZ, Alexander, 30179 Hannover (DE)
(74) Vertreter: BIP Patents
(86) Internationale Anmeldenummer: PCT/EP2017/060900
(87) Internationale Veröffentlichungsnummer: WO 2017/194459

(56) Entgegenhaltungen:
- WO-A1-97/07116
- WO-A1-2008/144748
- WO-A2-2006/052962

## Beschreibung

Die vorliegende Anmeldung betrifft neue substituierte 5,6,7,8-Tetrahydro[1,2,4]triazolo[4,3-a]pyridin-3(2H)-one und 2,5,6,7-tetrahydro-3H-pyrrolo[2,1-c][1,2,4]triazol-3-one, Verfahren zu ihrer Herstellung, diese zur Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von inflammatorischen Lungen-Erkrankungen.

Das Enzym Prolyl-Endopeptidase (PREP, Prolyl-Oligopetidase, PE, POP) ist eine Serinprotease, welche Peptide bis zu einer Länge von 30 Aminosäuren hinter der Aminosäure Prolin spaltet. [Moriyama et al., J. Biochem. 1988, 104:112-117]. PREP wird in allen Organen und Geweben konstitutiv, unter anderem von Leukozyten und Epithelzellen exprimiert und sezerniert. Die Freisetzung wird bei Kontakt mit reizenden, entzündlichen Stoffen erhöht. [Szul et al., Am. J. Respir. Cell Mol. Biol. 2016, 54:359-369] Während PREP im zentralen Nervensystem am Abbau von peptidischen Neurotransmittern beteiligt ist, ist PREP in der gesamten Peripherie, so auch in der Lunge, ein Enzym, das in degenerativen bzw. entzündlichen Prozessen Abbauprodukte des Kollagens u.a. spaltet. Aufgrund des hohen Gehaltes von Prolin und Glyzin in der Aminosäuresequenz des Kollagens, werden dort Tripeptide mit der Sequenz Prolin-Glyzin-Prolin (PGP) durch den Abbau der Kollagenfragmente gebildet [Weathington et al., Nat. Med. 2006, 12:317-323].ln Sputen von Patienten mit chronischen, inflammatorischen Lungenerkrankungen, wie der Chronisch Obstruktiven Lungenerkrankung (Chronic Obstructive Pulmonary Disease, COPD) oder der Zystischen Fibrose (Mukoviszidose, CF) wurden signifikant erhöhte PGP-Konzentrationen beschrieben [O'Reilly et al., Respir. Res. 2009, 10:38 doi:10.1186/1465-9921-10-38; Gaggar et al., J. Immunol. 2008, 180:5662-5669]. PGP, das von PREP produziert wird, ist ein Chemokin für neutrophile Granulozyten (kurz: Neutrophile), d. h. PGP führt zu einer Einwanderung von Neutrophilen in Gewebe mit erhöhten PGP-Konzentrationen. Es gibt Hinweise, dass der Mechanismus dieses Anlockens von Neutrophilen auf einer direkten Stimulierung der Neutrophilen über PGP-sensitive Rezeptoren auf der neutrophilen Zellmembran (z. B. CXCR1, CXCR2) beruht oder indirekt durch die Freisetzung von weiteren Chemokinen, wie beispielsweise Interleukinen (z. B. Interleukin-8, CXCL8) durch andere Zelltypen (z. B. Makrophagen, Epithelzellen) bewirkt wird. Die chemokine Wirkung von PGP auf Neutrophile wurde mehrfach in vitro und in vivo demonstriert. [Weathington et al., Nat. Med. 2006, 12:317-323; De Kruijf et al., Eur. J. Pharmacol. 2010, 643:29-33; Overbeek et al., Eur. J. Pharmacol. 2011, 668:428-434; Braber et al., Eur. J. Pharmacol. 2011, 668:443-449] Im Tierversuch konnte die Wirkung von PGP durch die Gabe eines komplementären Tripeptides (Threonin-Arginin-Threonin, RTR) sowie durch die Applikation eines CXCR2-Rezeptor-Blockers aufgehoben werden. Es wurde gezeigt, dass eine wiederholte Applikation von PGP in die Lunge von Mäusen ein Lungenemphysem auslösen kann. Zudem wird durch Zigarettenrauch die PGP-Konzentration in den Lungen von Mäusen, die Zigarettenrauch ausgesetzt werden, erhöht. Die gleichzeitige Gabe von PGP-neutralisierendem RTR kann die oben genannten, Zigarettenrauch-induzierten Effekte aufheben [Braber et al., Eur J. Pharmacol. 2011, 668:443-449; Braber et al., Am. J. Physiol. Lung Cell Mol. Pysiol. 2011, 300:L255-L265].

Die Chronisch Obstruktive Lungenerkrankung (Chronic Obstructive Pulmonary Disease, COPD) ist eine Erkrankung der Lunge, die mit chronischer Bronchitis, Atemnot, Husten und Auswurf sowie mit dem Untergang von Lungengewebe (Emphysem) einhergeht. Die Lungenfunktion ist aufgrund der obstruktiven Veränderung der Bronchien und durch den Verlust von funktionalem Lungengewebe mit fortschreitender Erkrankung zunehmend eingeschränkt. Eine Überblähung der Lunge ist aufgrund der behinderten Ausatmung häufig.

Die häufigste Ursache für die Entstehung einer COPD ist eine chronische Inhalation von Zigarettenrauch. Darüber hinaus entwickeln weltweit, mit regionalen Unterschieden 10 bis 40 % der Patienten eine COPD, die nicht auf Zigarettenrauch, sondern vermutlich auf die Exposition mit Umweltgiften, wie beispielsweise Rauch von Kohle- bzw. Holzfeuer oder Dieselabgasen zurückzuführen sind [Salvi, Clin. Chest Med. 2014, 35:17-27].

Derzeit kann die COPD nur symptomatisch behandelt werden. In erster Linie erhalten COPD-Patienten bronchienerweiternde Medikamente, die das Atmen erleichtern. Der Einsatz entzündungshemmender Medikamente ist bislang limitiert.

Neben der beschriebenen chronischen Symptomatik leiden COPD-Patienten häufig unter akut einsetzenden, zeitlich begrenzten Verschlechterungen des ohnehin beeinträchtigten Gesundheitszustandes, die eine zusätzliche Behandlung erforderlich macht [Ewig, Klinikarzt 2013, 42:182-187]. Die Therapie dieser, als akute Exazerbationen bezeichneten Krankheitsschübe beschränkt sich bislang auf die Gabe von Sauerstoff und systemisch verabreichten Kortikosteroiden.

In einer klinischen COPD-Studie wurde gezeigt, dass das antiinflammatorisch wirksame Roflumilast (PDE4-Inhibitor) zu einer Verringerung der Konzentration von PGP in Sputum und Serum der behandelten COPD-Patienten führt [Wells et al., Am. J. Respir. Crit. Care Med. 2015, 192:934-942].

Erhöhte, proinflammatorische PGP-Konzentrationen wurden darüber hinaus unter anderem in der Schocklunge (Akutes, respiratorisches Syndrom, ARDS) und der Hornhautverletzung des Auges beschrieben. [Hahn et al., Sei. Adv. 2015, 1: e1500175; Pfister et al., Invest. Ophthalmol. Vis. Sci. 1998, 39:1744-1750] Auch hier wird ein proentzündlicher Einfluss von PGP formuliert, der zum einen zu erhöhter Permeabilität von Gefäßen, zum anderen, wie bereits oben beschreiben, zu einer vermehrten Rekrutierung von Neutrophilen (Neutrophilie) und damit zu einer gesteigerten Entzündung führt. Da die Bildung von PGP bei entzündlichen Prozessen unmittelbar mit der Zerstörung von Geweben verbunden ist, und PGP wiederum die Entzündung fördert, ist eine Beteiligung von PGP an sich selbsterhaltenden, chronischentzündlichen Prozessen wahrscheinlich. Hier ist insbesondere die COPD bzw. die akut exazerbierende COPD (AE-COPD) zu nennen, der eine chronische Inflammation zugrunde liegt [Russell et al., Curr Opin Pilm Med. 2016, 22:91-99; Anzueto, Eur. Respir. Rev. 2010, 19:113-118]. Aber auch andere, chronisch-entzündliche Erkrankungen und Wundheilungsstörungen der Lunge und anderer Gewebe und Organe, wie z. B. der Haut, des Auges, der Blutgefäße, von Bindegeweben, des Skelettes oder der Muskulatur könnten von einer Reduzierung der PGP-Konzentrationen durch die PREP-Inhibierung profitieren.

Potentielle Anwendungsgebiete für PREP-Inhibitoren sind akute und chronische, pathologische Prozesse bei denen PREP bzw. Substrate und Produkte von PREP beteiligt sind. Da diese anti-entzündliche Wirkung der PREP-Inhibition vermutlich nicht direkt in die Funktion des Immunsystems eingreift, und daher möglicherweise keine immunsupressive Wirkung zu erwarten ist, könnte ein Vorteil der PREP-Inhibiton darin bestehen, dass diesbezüglich weniger Nebenwirkungen bei behandelten Patienten auftreten könnten als mit klassischen immunmodulierenden Wirkprinzipien. Da PGP über das konstitutiv exprimierte, d. h. ständig vorhandene Enzym PREP aus Kollagenfragmenten gebildet wird, und die PGP-Produktion daher nicht vom Immunsystem kontrolliert bzw. reguliert wird, ist zu erwarten, dass die antiinflammatorische Wirkung auch in Patienten wirkt, bei denen etwa aufgrund von Resistenzen, die Wirksamkeit gegen Kortikosteroide verringert ist. Kortikosteroidresistenzen sind insbesondere bei COPD-Patienten in stabilen Phasen außerhalb von akuten Exazerbationen beschrieben. Des Weiteren ist eine additive bzw. synergistische Wirksamkeit von der Kombination mit Kortikosteroiden auch bei vollständig vorhandener, sowie bei eingeschränkter oder stark verminderter Wirkung von Kortikosteroiden zu erwarten. Ebenso ist eine Kombination mit sämtlichen anderen inflammatorischen Wirkmechanismen möglich.

Da PGP, dessen Bildung durch PREP-Inhibition verhindert wird, vermutlich in sämtlichen Erkrankungen mit inflammatorischen Komponenten und Beteiligung des Kollagens bzw. dessen Fragmente eine treibende Rolle beim Entzündungsgeschehen hat, kann eine PREP-Inhibition positive Einflüsse auf viele Erkrankungen wie Autoimmunerkrankungen, chronisch-entzündliche Erkrankungen wie rheumatoide Erkrankungen, infektiösen Geschehen, degenerativen Prozessen (Haut, Organe, Knochen, Muskulatur) haben.

Die Aufgabe der vorliegenden Erfindung war die Identifizierung und Bereitstellung neuer, niedermolekularer Verbindungen, die als potente Inhibitoren des Enzyms Prolylendopeptidase (PREP, Prolyloligopeptidase, PE, POP) wirken, und bei akuten oder chronischen, pathologischen bzw. entzündlich-degenerativen Prozessen über die Reduktion der PREPabhängigen PGP-Produktion vor allem die Rekrutierung von neutrophilen Granulozyten in Organe, insbesondere der Lunge reduzieren.

In der Anmeldung WO2006052962A2 werden bicyclische Triazole zur Bekämpfung von über Integrin-Inhibition verlaufende Krankheiten beschrieben. 1,2-Dihydro-3H-indazol-3-one als NOX-Inhibitoren zur Bekämpfung von Krankheiten wie COPD, Alzheimer, inflammatorischen oder fibrotischen Erkrankungen sind aus WO2011062864A2 bekannt. WO97007116A1 beschreibt die Verwendung von Inhibitoren der Prolyl Endopeptidase zur Bekämpfung von neutrophilen Entzündungen, WO2008144748A1 Prolin-Derivate als Inhibitoren der Prolyl Endopeptidase zur Behandlung von Erkrankungen des ZNS.

Die Erfindung wird durch die Ansprüche definiert. Jeglicher Gegenstand der Beschreibung, der über den Umfang der Ansprüche hinausgeht, ist als Hintergrundwissen offenbart und nicht Teil der Erfindung.

Gegenstand der vorliegenden Erfindung sind Verbindungen der allgemeinen Formel (I) 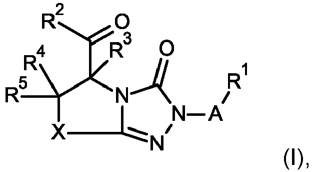 in welcher
- A: für (C₁-C₄)-Alkylen oder CD₂ steht,
wobei (C₁-C₄)-Alkylen mit Hydroxy, (C₁-C₄)-Alkoxy sowie bis zu fünfmal mit Fluor substituiert sein kann, oder für eine Gruppe der Formel 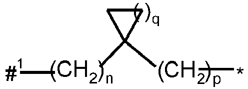 steht, worin
n für 0 oder 1 steht,
p für 0 oder 1 steht,
q für 1 oder 2 steht,
wobei
#¹ die Verknüpfung zum Stickstoffatom des 5-Oxo-4,5-dihydro-1H-1,2,4-triazol-1-yl-Ringes markiert,
* die Verknüpfung zu R¹ markiert,
- X: für -CR⁶R⁷-, #²-CR⁶R⁷-CR⁸R⁹-**, #²-CR⁶=CR⁸-** oder #²-CR⁶R⁷-CR⁸R⁹-CR¹⁰R¹¹-
- **: steht,
wobei #² die Verknüpfung zum Kohlenstoffatom der CR⁴R⁵-Gruppe markiert,
wobei ** die Verknüpfung zum Kohlenstoffatom des 5-Oxo-4,5-dihydro-1H-1,2,4-triazol-1-yl-Ringes markiert,
   wobei

R⁶ für Wasserstoff, Fluor, (C₁-C₄)-Alkyl, (C₁-C₄)-Alkoxy, Trifluormethoxy, Hydroxy, Mono-(C₁-C₄)-alkylamino oder Di-(C₁-C₄)-alkylamino steht,
worin (C₁-C₄)-Alkyl mit (C₁-C₄)-Alkoxy, Hydroxy, Mono-(C₁-C₄)-alkylamino oder Di-(C₁-C₄)-alkylamino sowie bis zu fünfmal mit Fluor substituiert sein kann,
R⁷ für Wasserstoff, Fluor oder (C₁-C₄)-Alkyl steht,
worin (C₁-C₄)-Alkyl bis zu fünfmal mit Fluor substituiert sein kann,
oder
R⁶ und R⁷ zusammen mit dem Kohlenstoffatom, an das sie gebunden sind eine Carbonylgruppe bilden,
oder
R⁶ und R⁷ zusammen mit dem Kohlenstoffatom, an das sie gebunden sind, einen Cyclopropyl-, Cyclobutyl-Ring oder Cyclopentyl-Ring bilden,
oder
R⁶ und R⁴ zusammen mit dem Kohlenstoffatom, an die sie gebunden sind, einen Cyclopropyl-, Cyclobutyl-Ring oder Cyclopentyl-Ring bilden,
R⁸ für Wasserstoff, (C₁-C₄)-Alkyl, (C₁-C₄)-Alkoxy, Fluor, Hydroxy, Mono-(C₁-C₄)-alkylamino oder Di-(C₁-C₄)-alkylamino steht,
worin (C₁-C₄)-Alkyl mit (C₁-C₄)-Alkoxy, Hydroxy, Mono-(C₁-C₄)-alkylamino oder Di-(C₁-C₄)-alkylamino sowie bis zu fünfmal mit Fluor substituiert sein kann,
R⁹ für Wasserstoff, Fluor oder (C₁-C₄)-Alkyl steht,
worin (C₁-C₄)-Alkyl bis zu fünfmal mit Fluor substituiert sein kann,
oder
R⁸ und R⁹ zusammen mit dem Kohlenstoffatom, an das sie gebunden sind, einen Cyclopropyl-, Cyclobutyl-Ring oder Cyclopentyl-Ring bilden,
oder
R⁷ und R⁹ zusammen mit den Kohlenstoffatomen, an die sie gebunden sind, einen Cyclopropyl-, Cyclobutyl-Ring oder Cyclopentyl-Ring bilden,
R¹⁰ für Wasserstoff, (C₁-C₄)-Alkyl, (C₁-C₄)-Alkoxy, Fluor, Hydroxy, Mono-(C₁-C₄)-alkylamino oder Di-(C₁-C₄)-alkylamino steht,
worin (C₁-C₄)-Alkyl mit (C₁-C₄)-Alkoxy, Hydroxy, Mono-(C₁-C₄)-alkylamino oder Di-(C₁-C₄)-alkylamino sowie bis zu fünfmal mit Fluor substituiert sein kann,
R¹¹ für Wasserstoff, Fluor oder (C₁-C₄)-Alkyl steht,
worin (C₁-C₄)-Alkyl bis zu fünfmal mit Fluor substituiert sein kann,
oder
R¹⁰ und R¹¹ zusammen mit dem Kohlenstoffatom, an das sie gebunden sind, einen Cyclopropyl-, Cyclobutyl-Ring oder Cyclopentyl-Ring bilden,
R¹ für Phenyl oder 5- bis 6-gliedriges Heteroaryl steht,
wobei Phenyl mit 1 bis 3 Substituenten unabhängig voneinander ausgewählt aus der Gruppe Fluor, Chlor, Cyano, Methyl, Ethyl, Trifluormethyl, Methoxy, Difluormethoxy, Trifluormethoxy, Hydroxycarbonyl, Methoxycarbonyl, Ethoxycarbonyl, tert-Butoxycarbonyl, Methylaminocarbonyl, Dimethylaminocarbonyl, Methylsulfanyl, Methylsulfonyl, Methylsulfonimidoyl, Aminosulfonyl oder Methylsulfinyl substituiert ist,
   oder
wobei Phenyl mit Cyclopentyl, Cyclohexyl, 5- bis 6-gliedriges Heterocyclyl oder 5- bis 6-gliedriges Heteroaryl anneliert sein kann,
   worin (Cyclopentyl, Cyclohexyl, (C₅-C₆)-Heterocycyl und 5- bis 6-gliedriges Heteroaryl mit 1 oder 2 Substituenten Methyl oder Ethyl substituiert sein können,
   oder
wobei 5- bis 6-gliedriges Heteroaryl mit 1 bis 3 Substituenten unabhängig voneinander ausgewählt aus der Gruppe Fluor, Chlor, Cyano, Methyl, Ethyl, Trifluormethyl, Methoxy, Ethoxy, Difluormethoxy, Trifluormethoxy, 2,2,2-Trifluorethoxy, Methylaminocarbonyl, tert-Butylaminocarbonyl, Dimethylaminocarbonyl, substituiert sein kann,
wobei 5- bis 6-gliedriges Heteroaryl mit Cyclopentyl, Cyclohexyl, 5- bis 6-gliedriges Heterocyclyl, Phenyl oder 5- bis 6-gliedriges Heteroaryl anneliert sein kann,
   worin 5- bis 6-gliedriges Heteroaryl mit Methyl, Ethyl, Chlor, Fluor oder Methoxy substituiert sein kann,
   worin Cyclopentyl, Cyclohexyl 5- bis 6-gliedriges Heterocyclyl, Phenyl und 5- bis 6-gliedriges Heteroaryl mit 1 oder 2 Substituenten (C₁-C₄)-Alkyl substituiert sein können,
- R²: für eine Gruppe der Formel 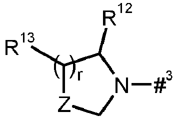 steht, wobei
#³ die Verknüpfung zum Carbonyl-Kohlenstoffatom markiert,
r für 0 oder 1 steht,
Z für O, NR¹⁸, S, SO, SO₂ oder CR^{14A}R^{14B} steht,
worin
R^{14A} für Wasserstoff, Halogen, Cyano, (C₁-C₄)-Alkyl, (C₃-C₆)-Cycloalkyl, Trifluormethyl, Difluormethyl, Fluormethyl, Hydroxy, (C₁-C₄)-Alkoxy, (C₃-
C₅)-Cycloalkoxy, Difluormethoxy, Trifluormethoxy oder 2,2,2-Trifluorethoxy steht,
worin (C₁-C₄)-Alkyl mit Hydroxy, Amino, Mono-(C₁-C₄)-alkylamino oder Di-(C₁-C₄)-alkylamino substituiert sein kann,
R^{14B} für Wasserstoff, Fluor oder (C₁-C₄)-Alkyl, steht,
worin (C₁-C₄)-Alkyl bis zu fünfmal mit Fluor substituiert sein kann,
oder
R^{14A} und R^{14B} zusammen mit dem Kohlenstoffatom, an das sie gebunden sind eine Carbonylgruppe bilden,
R¹⁸ für Wasserstoff oder Methyl steht,
R¹² für Wasserstoff, Cyano, (C₁-C₄)-Alkyl, Acetyl oder Formyl steht,
worin (C₁-C₄)-Alkyl mit Hydroxy oder bis zu fünfmal mit Fluor substituiert sein kann,
worin Acetyl mit Hydroxy oder bis zu dreimal mit Fluor substituiert sein kann,

R¹³ für Wasserstoff, Fluor oder (C₁-C₄)-Alkyl steht,
oder
R¹² und R¹³ Cyclopropyl- zusammen mit den Kohlenstoffatomen, an die sie gebunden sind, einen oder Cyclobutyl-Ring bilden,
worin der Cyclopropyl- oder Cyclobutyl-Ring bis zu zweimal mit Fluor substituiert sein kann,
oder
R¹³ und R^{14A} zusammen mit den Kohlenstoffatomen, an die sie gebunden sind, einen Cyclopropyl- oder Cyclobutyl-Ring bilden,
worin der Cyclopropyl- oder Cyclobutyl -Ring bis zu zweimal mit Fluor substituiert sein kann,
oder
R^{14A} und R^{14B} zusammen mit dem Kohlenstoffatom, an die sie gebunden sind, einen Cyclopropyl- oder Cyclobutyl-Ring bilden,
worin der Cyclopropyl- oder Cyclobutyl-Ring bis zu zweimal mit Fluor substituiert sein kann,
wobei R¹³, R^{14A} und R^{14B} für Wasserstoff stehen, wenn R¹² nicht für Wasserstoff steht,
wobei R¹² für Wasserstoff steht, wenn einer der Substituenten R¹³, R^{14A} oder R^{14B} nicht für Wasserstoff steht,
oder
für eine Gruppe der Formel 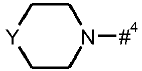 steht, wobei
#⁴ die Verknüpfung zum Carbonyl-Kohlenstoffatom markiert,
Y für NR¹⁵, CR^{16A}R^{16B}, Sauerstoff oder Schwefel steht,
worin
R¹⁵ für Wasserstoff oder Methyl steht,
R^{16A} für Wasserstoff oder Methyl steht,
R^{16B} für Wasserstoff oder Methyl steht,
- R³: für Wasserstoff oder (C₁-C₄)-Alkyl steht,
- R⁴: für Wasserstoff, (C₁-C₄)-Alkyl, (C₁-C₄)-Alkoxy, Fluor, Hydroxy, Mono-(C₁-C₄)-alkylamino oder Di-(C₁-C₄)-alkylamino steht,
worin (C₁-C₄)-Alkyl mit (C₁-C₄)-Alkoxy, Hydroxy, Mono-(C₁-C₄)-alkylamino oder Di-(C₁-C₄)-alkylamino sowie bis zu fünfmal mit Fluor substituiert sein kann,
- R⁵: für Wasserstoff oder (C₁-C₄)-Alkyl steht,
worin (C₁-C₄)-Alkyl bis zu fünfmal mit Fluor substituiert sein kann,
oder
- R⁴ und R⁵: zusammen mit dem Kohlenstoffatom, an das sie gebunden sind eine Carbonylgruppe bilden,
oder
- R⁴ und R⁵: zusammen mit dem Kohlenstoffatom, an das sie gebunden sind, einen Cyclopropyl-, Cyclobutyl-Ring oder Cyclopentyl-Ring bilden,
sowie die Salze, Solvate und Solvate der Salze der Verbindungen der Formel (I).

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.lm Falle von Carbonsäuren als Zwischen- oder Endprodukten kann alternativ auch eine Trennung über diastereomere Salze mit Hilfe chiraler Amin-Basen erfolgen.

Sofern die erfindungsgemäßen Verbindungen in tautomeren Formen vorkommen können, umfasst die vorliegende Erfindung sämtliche tautomere Formen.

Die Verbindungen der allgemeinen Formel (I) können als isotopische Varianten vorliegen. Die Erfindung umfasst daher eine oder mehrere isotopische Varianten der Verbindungen der allgemeinen Formel (I), insbesondere deuteriumhaltige Verbindungen der allgemeinen Formel (I).

Der Begriff "isotopische Variante" einer Verbindung oder eines Reagenzes ist definiert als eine Verbindung mit einem unnatürlichen Anteil eines oder mehrerer der Isotope, aus denen eine derartige Verbindung aufgebaut ist.

Der Begriff "isotopische Variante der Verbindung der allgemeinen Formel (I)" ist definiert als eine Verbindung der allgemeinen Formel (I) mit einem unnatürlichen Anteil eines oder mehrerer der Isotope, aus denen eine derartige Verbindung aufgebaut ist.

Unter dem Ausdruck "unnatürlicher Anteil" ist ein Anteil eines derartigen Isotops zu verstehen, der höher als dessen natürliche Häufigkeit ist. Die in diesem Zusammenhang anzuwendenden natürlichen Häufigkeiten von Isotopen finden sich in "Isotopic Compositions of the Elements 1997", Pure Appl. Chem., 70(1), 217-235, 1998.

Beispiele für derartige Isotope sind stabile und radioaktive Isotope von Wasserstoff, Kohlenstoff, Stickstoff, Sauerstoff, Phosphor, Schwefel, Fluor, Chlor, Brom und lod, wie ²H (Deuterium), ³H (Tritium), ¹¹C, ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁵I, ¹²⁹I bzw. ¹³¹I.

Im Hinblick auf die Behandlung und/oder Prophylaxe der hier angegebenen Störungen enthalten die isotopischen Variante(n) der Verbindungen der allgemeinen Formel (I) vorzugsweise Deuterium ("deuteriumhaltige Verbindungen der allgemeinen Formel (I)"). isotopische Varianten der Verbindungen der allgemeinen Formel (I), in die ein oder mehrere radioaktive Isotope, wie ³H oder ¹⁴C, eingebaut sind, sind beispielsweise bei Arzneistoff- und/oder Substratsgewebeverteilungsstudien von Nutzen. Diese Isotope sind wegen ihrer leichten Einbaubarkeit und Nachweisbarkeit besonders bevorzugt. In eine Verbindung der allgemeinen Formel (I) können Positronen emittierende Isotope wie ¹⁸F oder ¹¹C eingebaut werden. Diese isotopischen Varianten der Verbindungen der allgemeinen Formel (I) eignen sich zur Verwendung bei in-vivo-Bildgebungsanwendungen. Deuteriumhaltige und ¹³C-haltige Verbindungen der allgemeinen Formel (I) können im Rahmen präklinischer oder klinischer Studien bei Massenspektrometrie-Analysen verwendet werden (H. J. Leis et al., Curr. Org. Chem., 1998, 2, 131).

Isotopische Varianten der Verbindungen der allgemeinen Formel (I) können im Allgemeinen nach dem Fachmann bekannten Verfahren wie den in den hier beschriebenen Schemata und/oder Beispielen beschrieben hergestellt werden, indem man ein Reagenz durch eine isotopische Variante des Reagenzes, vorzugsweise ein deuteriumhaltiges Reagenz, ersetzt. Je nach den gewünschten Deuterierungsstellen kann in einigen Fällen Deuterium aus D₂O entweder direkt in die Verbindungen oder in Reagenzien, die für die Synthese derartiger Verbindungen verwendet werden können, eingebaut werden (Esaki et al., Tetrahedron, 2006, 62, 10954; Esaki et al., Chem. Eur. J., 2007, 13, 4052). Ein nützliches Reagenz zum Einbau von Deuterium in Moleküle ist auch Deuteriumgas. Eine schnelle Route zum Einbau von Deuterium ist die katalytische Deuterierung von olefinischen Bindungen (H. J. Leis et al., Curr. Org. Chem., 1998, 2, 131; J. R. Morandi et al., J. Org. Chem., 1969, 34 (6), 1889) und acetylenischen Bindungen (N. H. Khan, J. Am. Chem. Soc., 1952, 74 (12), 3018; S. Chandrasekhar et al., Tetrahedron Letters, 2011, 52, 3865). Zum direkten Austausch von Wasserstoff gegen Deuterium in funktionelle Gruppen enthaltenden Kohlenwasserstoffen können auch Metallkatalysatoren (d.h. Pd, Pt und Rh) in Gegenwart von Deuteriumgas verwendet werden (J. G. Atkinson et al., US-Patent 3966781). Verschiedene deuterierte Reagenzien und Synthesebausteine sind im Handel von Firmen wie beispielsweise C/D/N Isotopes, Quebec, Kanada; Cambridge Isotope Laboratories Inc., Andover, MA, USA; und CombiPhos Catalysts, Inc., Princeton, NJ, USA, erhältlich. Weitere Informationen bezüglich des Standes der Technik im Hinblick auf Deuterium-Wasserstoff-Austausch finden sich beispielsweise in Hanzlik et al., J. Org. Chem. 55, 3992-3997, 1990; R. P. Hanzlik et al., Biochem. Biophys. Res. Commun. 160, 844, 1989; P. J. Reider et al., J. Org. Chem. 52, 3326-3334, 1987; M. Jarman et al., Carcinogenesis 16(4), 683-688, 1995; J. Atzrodt et al., Angew. Chem., Int. Ed. 2007, 46, 7744; K. Matoishi et al., Chem. Commun. 2000, 1519-1520; K. Kassahun et al., WO2012/112363.

Der Begriff "deuteriumhaltige Verbindung der allgemeinen Formel (I)" ist definiert als eine Verbindung der allgemeinen Formel (I), in der ein oder mehrere Wasserstoffatome durch ein oder mehrere Deuteriumatome ersetzt sind und in der die Häufigkeit von Deuterium in jeder deuterierten Position der Verbindung der allgemeinen Formel (I) höher ist als die natürliche Häufigkeit von Deuterium, die etwa 0,015% beträgt. Insbesondere ist in einer deuteriumhaltigen Verbindung der allgemeinen Formel (I) die Häufigkeit von Deuterium in jeder deuterierten Position der Verbindung der allgemeinen Formel (I) höher als 10%, 20%, 30%, 40%, 50%, 60%, 70% oder 80%, vorzugsweise höher als 90%, 95%, 96% oder 97%, noch weiter bevorzugt höher als 98% oder 99% in dieser Position bzw. diesen Positionen. Es versteht sich, dass die Häufigkeit von Deuterium in jeder deuterierten Position von der Häufigkeit von Deuterium in anderen deuterierten Positionen unabhängig ist.

Durch den selektiven Einbau eines oder mehrerer Deuteriumatome in eine Verbindung der allgemeinen Formel (I) können die physikalisch-chemischen Eigenschaften (wie beispielsweise Azidität [C. L. Perrin, et al., J. Am. Chem. Soc., 2007, 129, 4490; A. Streitwieser et al., J. Am. Chem. Soc., 1963, 85, 2759;], Basizität [C. L. Perrin et al., J. Am. Chem. Soc., 2005, 127, 9641; C. L. Perrin, et al., J. Am. Chem. Soc., 2003, 125, 15008; C. L. Perrin in Advances in Physical Organic Chemistry, 44, 144], Lipophilie [B. Testa et al., Int. J. Pharm., 1984, 19(3), 271]) und/oder das Stoffwechselprofil des Moleküls verändert und Veränderungen des Verhältnisses von Stammverbindung zu Metaboliten oder der gebildeten Mengen von Metaboliten verursacht werden. Derartige Veränderungen können zu bestimmten therapeutischen Vorteilen führen und daher unter bestimmten Umständen bevorzugt sein. Es ist über verringerte Stoffwechselraten und Stoffwechselumschaltung, bei der sich das Verhältnis von Metaboliten ändert, berichtet worden (A. E. Mutlib et al., Toxicol. Appl. Pharmacol., 2000, 169, 102; D. J. Kushner et al., Can. J. Physiol. Pharmacol., 1999, 77, 79). Diese Veränderungen der Exposition gegenüber Stammverbindung und Metaboliten können wichtige Konsequenzen hinsichtlich Pharmakodynamik, Verträglichkeit und Wirksamkeit einer deuteriumhaltigen Verbindung der allgemeinen Formel (I) haben. In einigen Fällen wird durch den Deuteriumersatz die Bildung eines unerwünschten oder toxischen Metaboliten verringert oder eliminiert und die Bildung eines gewünschten Metaboliten verstärkt (z.B. Nevirapin: A. M. Sharma et al., Chem. Res. Toxicol., 2013, 26, 410; Efavirenz: A. E. Mutlib et al., Toxicol. Appl. Pharmacol., 2000, 169, 102). In anderen Fällen besteht der Haupteffekt der Deuterierung darin, die Geschwindigkeit der systemischen Clearance zu verringern. Dadurch wird die biologische Halbwertszeit der Verbindung erhöht. Zu den potentiellen klinischen Vorteilen gehören die Fähigkeit zur Aufrechterhaltung einer ähnlichen systemischen Exposition mit verringerten Spitzenspiegeln und erhöhten Talspiegeln. Dies könnte je nach der Pharmakokinetik/Pharmakodynamik-Beziehung der jeweiligen Verbindung zu geringeren Nebenwirkungen und erhöhter Wirksamkeit führen. Beispiele für diesen Deuterium-Effekt sind ML-337 (C. J. Wenthur et al., J. Med. Chem., 2013, 56, 5208) und Odanacatib (K. Kassahun et al., WO2012/112363). Es ist auch noch über weitere Fälle berichtet worden, bei denen verringerte Verstoffwechslungsraten zu einer Erhöhung der Arzneistoffexposition ohne Änderung der Rate der systemischen Clearance führen (z.B. Rofecoxib: F. Schneider et al., Arzneim. Forsch. / Drug. Res., 2006, 56, 295; Telaprevir: F. Maltais et al., J. Med. Chem., 2009, 52, 7993). Deuterierte Arzneistoffe, die diesen Effekt zeigen, können verringerte Dosierungsanforderungen haben (z.B. geringere Zahl von Dosen oder niedrigere Dosierung zur Erzielung der gewünschten Wirkung) und/oder zu geringeren Metabolitenbelastungen führen.

Eine Verbindung der allgemeinen Formel (I) kann mehrere potenzielle Angriffsstellen für die Verstoffwechslung aufweisen. Zur Optimierung der oben beschriebenen Effekte auf die physikalisch-chemischen Eigenschaften und das Stoffwechselprofil können deuteriumhaltige Verbindungen der allgemeinen Formel (I) mit einem bestimmten Muster eines oder mehrerer Deuterium-Wasserstoff-Austausche gewählt werden. Insbesondere ist/sind das Deuteriumatom/die Deuteriumatome deuteriumhaltiger Verbindung(en) der allgemeinen Formel (I) an ein Kohlenstoffatom gebunden und/oder steht/stehen in den Positionen der Verbindung der allgemeinen Formel (I), bei denen es sich um Angriffsstellen für Verstoffwechslungsenzyme wie z.B. Cytochrom P₄₅₀ handelt.

Der Begriff "Prodrugs" bezeichnet Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit der jeweils angegebenen Anzahl an Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl, 2-Ethylbutyl, 1,4-Dimethylpentyl, 4,4-Dimethylpentyl und 1,4,4-Trimethylpentyl.
Alkylcarbonyl stehen im Rahmen der Erfindung für einen linearen oder verzweigten Alkylrest mit der jeweils angegebenen Anzahl an Kohlenstoffatomen und einer am Kohlenstoffatom angebundenen Carbonylgruppe. Beispielhaft und vorzugsweise seien genannt: Methylcarbonyl, Ethylcarbonyl, n-Propylcarbonyl, Isopropylcarbonyl und tert.-Butylcarbonyl.
Alkoxy steht im Rahmen der Erfindung für einen linearen oder verzweigten Alkoxyrest mit der jeweils angegebenen Anzahl an Kohlenstoffatomen. Beispielhaft und vorzugsweise seien genannt: Methoxy, Ethoxy, n-Propoxy, Isopropoxy, n-Butoxy und tert.-Butoxy.
Cycloalkoxy steht im Rahmen der Erfindung für einen einen monocyclischen, gesättigten Alkylrest mit der jeweils angegebenen Anzahl an Ring-Kohlenstoffatomen, der über ein Sauerstoffatom verknüpft ist. Beispielhaft und vorzugsweise seien genannt: Cyclopropyloxy, Cyclobutyloxy, Cyclopentyloxy und Cyclohexyloxy.
Cycloalkyl bzw. Carbocyclus steht in Rahmen der Erfindung für einen monocyclischen, gesättigten Alkylrest mit der jeweils angegebenen Anzahl an Ring-Kohlenstoffatomen. Beispielhaft und vorzugsweise seien genannt: Cyclopropyl, Cyclobutyl, Cyclopentyl, Cyclohexyl und Cycloheptyl.
Cycloalkylcarbonyl stehen im Rahmen der Erfindung für einen einen monocyclischen, gesättigten Alkylrest mit der jeweils angegebenen Anzahl an Ring-Kohlenstoffatomen und einer am Kohlenstoffatom angebundenen Carbonylgruppe. Beispielhaft und vorzugsweise seien genannt: Cyclopropylcarbonyl, Cyclobutylcarbonyl, Cyclopentylcarbonyl, Cyclohexylcarbonyl und Cycloheptylcarbonyl.
Alkoxycarbonyl stehen im Rahmen der Erfindung für einen linearen oder verzweigten Alkoxyrest mit der jeweils angegebenen Anzahl an Kohlenstoffatomen und einer am Sauerstoffatom angebundenen Carbonylgruppe. Beispielhaft und vorzugsweise seien genannt: Methoxycarbonyl, Ethoxycarbonyl, n-Propoxycarbonyl, Isopropoxycarbonyl und tert.-Butoxycarbonyl.
Heterocyclus bzw Heterocyclyl steht im Rahmen der Erfindung für einen monocyclischen oder bicyclischen, gesättigten Heterocyclus mit der jeweils angegebenen Anzahl an Ringatomen, der ein oder zwei Ring-Heteroatome aus der Reihe N, O, S, SO und/oder SO₂ enthält und über ein Ring-Kohlenstoffatom oder gegebenenfalls ein Ring-Stickstoffatom verknüpft ist. Beispielhaft seien genannt: Azetidinyl, Oxetanyl, Pyrrolidinyl, Pyrazolidinyl, Tetrahydrofuranyl, Thiolanyl, Piperidinyl, Piperazinyl, Tetrahydropyranyl, Tetrahydrothiopyranyl, Morpholinyl, Thiomorpholinyl, Hexahydroazepinyl und Hexahydro-1,4-diazepinyl. Bevorzugt sind Azetidinyl, Oxetanyl, Pyrrolidinyl, Tetrahydrofuranyl, Piperidinyl, Piperazinyl, Tetrahydropyranyl und Morpholinyl.
Aza-Heterocyclyl steht im Rahmen der Erfindung für einen monocyclischen oder bicyclischen, gesättigten oder teilweise ungesättigten Heterocyclus mit mit der jeweils angegebenen Anzahl an Ringatomen, der ein Stickstoffatom enthält und darüberhinaus ein oder zwei weitere Ring-Heteroatome aus der Reihe N, O, S, SO und/oder SO₂ enthalten kann und über ein Ring-Stickstoffatom verknüpft ist. Beispielhaft seien genannt: Pyrrolidinyl, Pyrazolidinyl, Piperidinyl, Piperazinyl, Morpholinyl, Thiomorpholinyl, 1,1-Dioxothiomorpholinyl, Hexahydroazepinyl, Hexahydro-1,4-diazepinyl, 1,2,3,4-Tetrahydroisochinolinyl, 1,2,3,4-Tetrahydrochinolinyl, Indolinyl, 8-Azabicyclo[3.2.1]octanyl, 9-Azabicyclo[3.3.1]nonanyl, 3-Azabicyclo[4.1.0]heptanyl und Quinuclidinyl.
Heteroaryl steht im Rahmen der Erfindung für einen monocyclischen oder bicyclischen aromatischen Heterocyclus (Heteroaromaten) mit der jeweils angegebenen Anzahl an Ringatomen, der bis zu vier gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft und vorzugsweise seien genannt: Furyl, Pyrrolyl, Thienyl, Pyrazolyl, Imidazolyl, Chinolinyl, Thiazolyl, Oxazolyl, Isoxazolyl, Isothiazolyl, Triazolyl, Oxadiazolyl, Thiadiazolyl, Tetrazolyl, Pyridyl, Pyrimidinyl, Pyridazinyl, Pyrazinyl und Triazinyl.
Mono-alkylaminocarbonyl steht im Rahmen der Erfindung für eine Amino-Gruppe, die über eine Carbonylgruppe verknüpft ist und die einen linearen oder verzweigten Alkylsubstituenten mit der jeweils angegebenen Anzahl an Kohlenstoffatomen aufweist. Beispielhaft und vorzugsweise seien genannt: Methylaminocarbonyl, Ethylaminocarbonyl, *n-*Propylaminocarbonyl, Isopropylaminocarbonyl, *n*-Butylaminocarbonyl, *tert*.-Butylaminocarbonyl, *n*-Pentylaminocarbonyl und *n*-Hexylaminocarbonyl.
Di-alkylaminocarbonyl steht im Rahmen der Erfindung für eine Amino-Gruppe, die über eine Carbonylgruppe verknüpft ist und die zwei gleiche oder verschiedene lineare oder verzweigte
Alkylsubstituenten mit der jeweils angegebenen Anzahl an Kohlenstoffatomen aufweist. Beispielhaft und vorzugsweise seien genannt: *N,N*-Dimethylaminocarbonyl, *N,N*-Diethylaminocarbonyl, *N*-Ethyl-*N*-methylaminocarbonyl, *N-*Methyl-*N*-*n*-propylaminocarbonyl, *N-n-*Butyl-*N-*methylaminocarbonyl,*N*-tert.-Butyl-*N*-methylaminocarbonyl,*N*-*n*-Pentyl-*N*-methylaminocarbonyl und *N*-*n*-Hexyl-*N*-methylaminocarbonyl.
Mono-alkylaminosulfonyl steht im Rahmen der Erfindung für eine Amino-Gruppe, die über eine Sulfonylgruppe verknüpft ist und die einen linearen oder verzweigten Alkylsubstituenten mit der jeweils angegebenen Anzahl an Kohlenstoffatomen aufweist. Beispielhaft und vorzugsweise seien genannt: Methylaminosulfonyl, Ethylaminosulfonnyl, *n-*Propylaminosulfonyl, Isopropylaminosulfonyl, *n*-Butylaminosulfonyl, *tert*.-Butylaminosulfonyl, *n-*Pentylaminosulfonyl und *n*-Hexylaminosulfonyl.
Di-alkylaminosulfonyl steht im Rahmen der Erfindung für eine Amino-Gruppe, die über eine Sulfonylgruppe verknüpft ist und die zwei gleiche oder verschiedene lineare oder verzweigte Alkylsubstituenten mit der jeweils angegebenen Anzahl an Kohlenstoffatomen aufweist. Beispielhaft und vorzugsweise seien genannt: *N,N*-Dimethylaminosulfonyl, *N*,*N*-Diethylamino-sulfonyl, *N*-Ethyl-*N*-methylaminosulfonyl, N-Methyl-*N-n*-propylaminosulfonyl, *N-n-*Butyl*-N-*methylaminosulfonyl, *N-*tert.-Butyl-*N*-methylaminosulfonyl, *N-n*-Pentyl-*N*-methylaminosulfonyl und *N*-n-Hexyl*-N*-methylaminosulfonyl.
Mono-alkylamino steht im Rahmen der Erfindung für eine Amino-Gruppe, die einen linearen oder verzweigten Alkylsubstituenten mit der jeweils angegebenen Anzahl an Kohlenstoffatomen aufweist. Beispielhaft und vorzugsweise seien genannt: Methylamino, Ethylamino, *n*-Propylamino, Isopropylamino, *n*-Butylamino, *tert*.-Butylamino, *n*-Pentylamino und *n*-Hexylamino.
Di-alkylamino steht im Rahmen der Erfindung für eine Amino-Gruppe, die zwei gleiche oder verschiedene lineare oder verzweigte Alkylsubstituenten mit der jeweils angegebenen Anzahl an Kohlenstoffatomen aufweist. Beispielhaft und vorzugsweise seien genannt: *N,N-*Dimethylamino, *N,N-*Diethylamino, *N*-Ethyl-*N*-methylamino, *N*-Methyl-*N-n*-propylamino, *N-n-*Butyl-*N*-methylamino, *N*-*tert*.-Butyl-*N*-methylamino, *N-n*-Pentyl-*N*-methylaminol und *N-n*-Hexyl-*N*-methylamino.
Halogen schließt im Rahmen der Erfindung Fluor, Chlor, Brom und lod ein. Bevorzugt sind Chlor oder Fluor.
Eine Oxo-Gruppe steht im Rahmen der Erfindung für ein Sauerstoffatom, das über eine Doppelbindung an ein Kohlenstoffatom gebunden ist.

In den Formeln der Gruppe, für die A, X und R² stehen können, steht der Endpunkt der Linie, an dem ein Zeichen #¹ bzw. #² bzw. #³ bzw. #⁴ bzw. #⁵ bzw. * bzw. ** bzw. *** steht, nicht für ein Kohlenstoffatom beziehungsweise eine CH₂-Gruppe, sondern ist Bestandteil der Bindung zu dem jeweils bezeichneten Atom, an das A bzw. X bzw. R² gebunden sind.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit einem oder zwei gleichen oder verschiedenen Substituenten ist bevorzugt. Ganz besonders bevorzugt ist die Substitution mit einem Substituenten.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Offenbart, aber nicht Teil der vorliegenden Erfindung sind Verbindungen der allgemeinen Formel (I), in welcher
- A: für (C₁-C₄)-Alkylen steht, wobei (C₁-C₄)-Alkylen mit Hydroxy, Methoxy sowie bis zu dreimal mit Fluor substituiert sein kann,
oder
für eine Gruppe der Formel 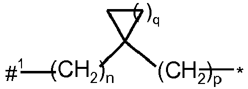 steht, worin
n für 1 steht,
p für 0 steht,
q für 1 steht,
wobei
#¹ die Verknüpfung zum Stickstoffatom des 5-Oxo-4,5-dihydro-1H-1,2,4-triazol-1-yl-Ringes markiert,
* die Verknüpfung zu R¹ markiert,
- X: für -CR⁶R⁷- oder #²-CR⁶R⁷-CR⁸R⁹-** steht,
wobei #² die Verknüpfung zum Kohlenstoffatom der CR⁴R⁵-Gruppe markiert,
wobei ** die Verknüpfung zum Kohlenstoffatom des 5-Oxo-4,5-dihydro-1H-1,2,4-triazol-1-yl-Ringes markiert,
wobei
   R⁶ für Wasserstoff, Fluor, Methyl, Ethyl, Methoxy, Ethoxy, Trifluormethoxy, Hydroxy, Mono-(C₁-C₄)-alkylamino oder Di-(C₁-C₄)-alkylamino steht,
   worin Methyl und Ethyl bis zu dreimal mit Fluor substituiert sein können,
   R⁷ für Wasserstoff, Fluor oder Methyl steht,
   worin Methyl bis zu dreimal mit Fluor substituiert sein kann,
   oder
   R⁶ und R⁷ zusammen mit dem Kohlenstoffatom, an das sie gebunden sind eine Carbonylgruppe bilden,
   oder
   R⁶ und R⁷ zusammen mit dem Kohlenstoffatom, an das sie gebunden sind, einen Cyclopropyl- oder Cyclobutyl-Ring bilden,
   oder
   R⁶ und R⁴ zusammen mit den Kohlenstoffatomen, an die sie gebunden sind, einen Cyclopropyl- oder Cyclobutyl-Ring bilden,
   R⁸ für Wasserstoff, Fluor, Methyl, Ethyl, Methoxy, Ethoxy, Trifluormethoxy, Hydroxy, Mono-(C₁-C₄)-alkylamino oder Di-(C₁-C₄)-alkylamino steht,
   worin Methyl und Ethyl bis zu dreimal mit Fluor substituiert sein können,
   R⁹ für Wasserstoff, Fluor oder Methyl steht,
   worin Methyl bis zu dreimal mit Fluor substituiert sein kann,
   oder
   R⁸ und R⁹ zusammen mit dem Kohlenstoffatom, an das sie gebunden sind, einen Cyclopropyl- oder Cyclobutyl-Ring bilden,
- R¹: für (C₅-C₆)-Cycloalkyl, Phenyl oder 5- bis 10-gliedriges Heteroaryl steht,
wobei (C₅-C₆)-Cycloalkyl mit 1 bis 3 Substituenten unabhängig voneinander ausgewählt aus der Gruppe Fluor, Cyano, Trifluormethyl, Methyl, Ethyl, Methoxy und Ethoxy substituiert sein kann,
   worin Methyl und Ethyl bis zu dreimal mit Fluor substituiert sein können,
wobei Phenyl mit 1 bis 4 Substituenten unabhängig voneinander ausgewählt aus der Gruppe Halogen, Cyano, (C₁-C₄)-Alkyl, (C₃-C₆)-Cycloalkyl, Trifluormethyl, (C₁-C₄)-Alkoxy, (C₃-C₅)-Cycloalkoxy, Monofluormethoxy, Difluormethoxy, Trifluormethoxy, Hydroxycarbonyl, (C₁-C₄)-Alkoxycarbonyl, Aminocarbonyl, Mono-(C₁-C₄)-alkylaminocarbonyl, Di-(C₁-C₄)-alkylaminocarbonyl, (C₁-C₄)-Alkylsulfanyl, (C₁-C₄)-Alkylsulfonyl, (C₁-C₄)-Alkylsulfonimidoyl, Aminosulfonyl, Mono-(C₁-C₄)-alkylaminosulfonyl, Di-(C₁-C₄)-alkylaminosulfonyl, (C₁-C₄)-Alkylsulfinyl, Amino, Mono-(C₁-C₄)-alkylamino oder Di-(C₁-C₄)-alkylamino substituiert ist,
   oder
wobei Phenyl mit Cyclopentyl, Cyclohexyl, 5- bis 6-gliedriges Heterocyclyl oder 5- bis 6-gliedriges Heteroaryl anneliert sein kann,
   worin Phenyl mit Methyl, Ethyl, Chlor, Fluor oder Methoxy substituiert sein kann, worin Cyclopentyl, Cyclohexyl, 5- bis 6-gliedriges Heterocyclyl und 5- bis 6-gliedriges Heteroaryl mit 1 oder 2 Substituenten Methyl oder Ethyl substituiert sein können,
   worin Methyl und Ethyl mit Methoxy, Hydroxy, Mono-Methylamino oder Di-Ethylamino sowie bis zu dreimal mit Fluor substituiert sein können,
   oder
wobei 5- bis 10-gliedriges Heteroaryl mit 1 bis 4 Substituenten unabhängig voneinander ausgewählt aus der Gruppe Halogen, Cyano, (C₁-C₄)-Alkyl, (C₃-C₆)-Cycloalkyl, Trifluormethyl, (C₁-C₄)-Alkoxy, (C₃-C₅)-Cycloalkoxy, Difluormethoxy, Trifluormethoxy, 2,2,2-Trifluorethoxy, Hydroxycarbonyl, (C₁-C₄)-Alkoxycarbonyl, Aminocarbonyl, Mono-(C₁-C₄)-alkylaminocarbonyl, Di-(C₁-C₄)-alkylaminocarbonyl, (C₁-C₄)-Alkylsulfanyl, (C₁-C₄)-Alkylsulfonyl, (C₁-C₄)-Alkylsulfonimidoyl, Aminosulfonyl, Mono-(C₁-C₄)-alkylaminosulfonyl, Di-(C₁-C₄)-alkylaminosulfonyl, (C₁-C₄)-Alkylsulfinyl, Amino, Mono-(C₁-C₄)-alkylamino oder Di-(C₁-C₄)-alkylamino substituiert sein kann,
   oder
wobei 5- bis 10-gliedriges Heteroaryl mit Cyclopentyl, Cyclohexyl oder 5- bis 6-gliedriges Heterocyclyl anneliert sein kann,
   worin 5- bis 10-gliedriges Heteroaryl mit Methyl, Ethyl, Chlor, Fluor oder Methoxy substituiert sein kann,
   worin Cyclopentyl, Cyclohexyl und 5- bis 6-gliedriges Heterocyclyl mit 1 oder 2 Substituenten (C₁-C₄)-Alkyl substituiert sein können,
      worin (C₁-C₄)-Alkyl mit (C₁-C₄)-Alkoxy, Hydroxy, Mono-(C₁-C₄)-alkylamino oder Di-(C₁-C₄)-alkylamino sowie bis zu fünfmal mit Fluor substituiert sein kann,
- R²: für eine Gruppe der Formel 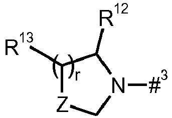 steht, wobei
- #³: die Verknüpfung zum Carbonyl-Kohlenstoffatom markiert,
r für 0 oder 1 steht,
Z für S, SO, SO₂ oder CR^{14A}R^{14B} steht,
worin
R^{14A} für Wasserstoff, Halogen, Cyano, (C₁-C₄)-Alkyl, Cyclopropyl, Trifluormethyl, Difluormethyl, Fluormethyl, Hydroxy, Methoxy, Ethoxy, Cyclopropoxy, Difluormethoxy, Trifluormethoxy, 2,2,2-Trifluorethoxy, Hydroxycarbonyl, Aminocarbonyl oder Amino steht,
worin (C₁-C₄)-Alkyl mit Hydroxy, Amino, Mono-(C₁-C₄)-alkylamino oder Di-(C₁-C₄)-alkylamino substituiert sein kann,
R^{14B} für Wasserstoff, Fluor, Methyl oder Trifluormethyl steht, oder
R^{14A} und R^{14B} zusammen mit dem Kohlenstoffatom, an das sie gebunden sind eine Carbonylgruppe bilden,
R¹² für Wasserstoff, Cyano, Methyl, Ethyl, Acetyl oder Formyl steht,
worin Methyl mit Hydroxy oder bis zu fünfmal mit Fluor substituiert sein kann,
worin Acetyl mit Hydroxy oder bis zu dreimal mit Fluor substituiert ist,

R¹³ für Wasserstoff, Fluor oder Methyl steht,
oder
R¹² und R¹³ zusammen mit den Kohlenstoffatomen, an die sie gebunden sind, einen Cyclopropyl-Ring bilden,
worin der Cyclopropyl-Ring bis zu zweimal mit Fluor substituiert sein kann,
oder
R¹³ und R^{14A} zusammen mit den Kohlenstoffatomen, an die sie gebunden sind, einen Cyclopropyl- oder Cyclobutyl-Ring bilden,
worin der Cyclopropyl- oder Cyclobutyl-Ring bis zu zweimal mit Fluor substituiert sein kann,
oder
R^{14A} und R^{14B} zusammen mit dem Kohlenstoffatom, an das sie gebunden sind, einen Cyclopropyl- oder Cyclobutyl-Ring bilden,
worin der Cyclopropyl- oder Cyclobutyl-Ring bis zu zweimal mit Fluor substituiert sein kann,
wobei R¹³, R^{14A} und R^{14B} für Wasserstoff stehen, wenn R¹² nicht für Wasserstoff steht,
wobei R¹² für Wasserstoff steht, wenn einer der Substituenten R¹³, R^{14A} oder R^{14B} nicht für Wasserstoff steht,
oder
für eine Gruppe der Formel 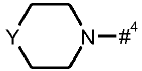 steht, wobei
#⁴ die Verknüpfung zum Carbonyl-Kohlenstoffatom markiert,
Y für NR¹⁵, CR^{16A}R^{16B}, Sauerstoff oder Schwefel steht,
worin
R¹⁵ für Wasserstoff oder Methyl steht,
R^{16A} für Wasserstoff oder Methyl steht,
R^{16B} für Wasserstoff oder Methyl steht,
- R³: für Wasserstoff oder Methyl steht,
- R⁴: für Wasserstoff, Fluor, Methyl, Ethyl, Methoxy, Ethoxy, Trifluormethoxy, Hydroxy, Mono-(C₁-C₄)-alkylamino oder Di-(C₁-C₄)-alkylamino steht, worin Methyl und Ethyl bis zu dreimal mit Fluor substituiert sein können,
- R⁵: für Wasserstoff, Fluor oder Methyl steht, worin Methyl bis zu dreimal mit Fluor substituiert sein kann,
oder
- R⁴ und R⁵: zusammen mit dem Kohlenstoffatom, an das sie gebunden sind, einen Cyclopropyl-Ring bilden,
sowie die Salze, Solvate und Solvate der Salze der Verbindungen der Formel (I).
Besonders bevorzugt im Rahmen der vorliegenden Erfindung sind Verbindungen der allgemeinen Formel (I), in welcher
- A: für -CH₂-,-CH(CH₃)-, -CH₂CH₂-, #⁵-CH₂CH(CH₃)-***, #⁵-CH₂C(CH₃)₂-***, #⁵-CH₂CHF-*** oder #⁵-CH₂CF₂-*** steht,
wobei #⁵ die Verknüpfung zum Stickstoffatom des 5-Oxo-4,5-dihydro-1H-1,2,4-triazol-1-yl-Ringes markiert,
wobei *** die Verknüpfung zu der R¹-Gruppe markiert,
- X: für - #²-CR⁶R⁷-CR⁸R⁹-** steht,
wobei #² die Verknüpfung zum Kohlenstoffatom der CR⁴R⁵-Gruppe markiert,
wobei ** die Verknüpfung zum Kohlenstoffatom des 5-Oxo-4,5-dihydro-1H-1,2,4-triazol-1-yl-Ringes markiert,
wobei
   R⁶ für Wasserstoff, Fluor, Methyl, Trifluormethyl oder Hydroxy steht,
   R⁷ für Wasserstoff, Fluor oder Methyl steht,
   oder
   R⁶ und R⁷ zusammen mit dem Kohlenstoffatom, an das sie gebunden sind, einen Cyclopropyl-Ring bilden,
   R⁸ für Wasserstoff, Fluor, Methyl oder Trifluormethyl steht,
   R⁹ für Wasserstoff, Fluor oder Methyl steht,
- R¹: für Phenyl oder 5- bis 6-gliedriges Heteroaryl steht, wobei Phenyl mit 1 bis 3 Substituenten wie in Anspruch 2 definiert substituiert ist,
oder
wobei Phenyl mit Cyclopentyl, Cyclohexyl, 5- bis 6-gliedriges Heterocyclyl oder 5- bis 6-gliedriges Heteroaryl anneliert sein kann,
   worin (Cyclopentyl, Cyclohexyl, (C₅-C₆)-Heterocycyl und 5- bis 6-gliedriges Heteroaryl mit 1 oder 2 Substituenten Methyl oder Ethyl substituiert sein können,
   oder
wobei 5- bis 6-gliedriges Heteroaryl mit 1 bis 3 Substituenten unabhängig voneinander ausgewählt aus der Gruppe Fluor, Chlor, Cyano, Methyl, Ethyl, Trifluormethyl, Methoxy, Ethoxy, Difluormethoxy, Trifluormethoxy, 2,2,2-Trifluorethoxy, Methylaminocarbonyl, tert-Butylaminocarbonyl, Dimethylaminocarbonyl, substituiert sein kann,
wobei 5- bis 6-gliedriges Heteroaryl mit Cyclopentyl, Cyclohexyl, 5- bis 6-gliedriges Heterocyclyl, Phenyl oder 5- bis 6-gliedriges Heteroaryl anneliert sein kann,
   worin 5- bis 6-gliedriges Heteroaryl mit Methyl, Ethyl, Chlor, Fluor oder Methoxy substituiert sein kann,
   worin Cyclopentyl, Cyclohexyl, 5- bis 6-gliedriges Heterocyclyl, Phenyl und 5-bis 6-gliedriges Heteroaryl mit 1 oder 2 Substituenten (C₁-C₄)-Alkyl substituiert sein können,
- R²: für eine Gruppe der Formel 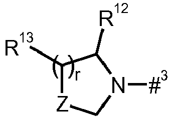 steht, wobei
#³ die Verknüpfung zum Carbonyl-Kohlenstoffatom markiert,
r für 0 oder 1 steht,
Z für S oder CR^{14A}R^{14B} steht, wenn r für 0 steht,
Z für S, SO, SO₂ oder CR^{14A}R^{14B} steht, wenn r für 1 steht,
worin jeweils
R^{14A} für Wasserstoff, Fluor, Methyl, Trifluormethyl, Difluormethyl, Fluormethyl, Hydroxy, Methoxy, Difluormethoxy oder Trifluormethoxy steht,
R^{14B} für Wasserstoff oder Fluor steht,
oder
R^{14A} und R^{14B} zusammen mit dem Kohlenstoffatom, an das sie gebunden sind eine Carbonylgruppe bilden,
R¹² für Wasserstoff, Cyano, Methyl, Acetyl oder Formyl steht,
worin Acetyl mit Hydroxy oder bis zu dreimal mit Fluor substituiert ist,
R¹³ für Wasserstoff, Fluor oder Methyl steht,
oder
R¹² und R¹³ zusammen mit den Kohlenstoffatomen, an die sie gebunden sind, einen Cyclopropyl-Ring bilden,
oder
R¹³ und R^{14A} zusammen mit dem Kohlenstoffatom, an die sie gebunden sind, einen Cyclopropyl -Ring bilden,
worin der Cyclopropyl-Ring bis zu zweimal mit Fluor substituiert sein kann,
oder
R^{14A} und R^{14B} zusammen mit dem Kohlenstoffatom, an das sie gebunden sind, einen Cyclopropyl-Ring bilden,
worin der Cyclopropyl-Ring bis zu zweimal mit Fluor substituiert sein kann,
wobei R¹³, R^{14A} und R^{14B} für Wasserstoff stehen, wenn R¹² nicht für Wasserstoff steht,
wobei R¹² für Wasserstoff steht, wenn einer der Substituenten R¹³, R^{14A} oder R^{14B} nicht für Wasserstoff steht,
- R³: für Wasserstoff steht,
- R⁴: für Wasserstoff, Fluor oder Methyl steht,
- R⁵: für Wasserstoff steht,
sowie die Salze, Solvate und Solvate der Salze der Verbindungen der Formel (I).

Besonders bevorzugt im Rahmen der vorliegenden Erfindung sind Verbindungen der allgemeinen Formel (I), in welcher
- A: für -CH₂-,-CH(CH₃)-, -CH₂CH₂-, #⁵-CH₂CH(CH₃)-***, #⁵-CH₂C(CH₃)₂-***, #⁵-CH₂CHF-*** oder #⁵-CH₂CF₂-*** steht,
wobei #⁵ die Verknüpfung zum Stickstoffatom des 5-Oxo-4,5-dihydro-1H-1,2,4-triazol-1-yl-Ringes markiert,
wobei *** die Verknüpfung zu der R¹-Gruppe markiert,
- X: für - #²-CR⁶R⁷-CR⁸R⁹-** steht,
wobei #² die Verknüpfung zum Kohlenstoffatom der CR⁴R⁵-Gruppe markiert,
wobei ** die Verknüpfung zum Kohlenstoffatom des 5-Oxo-4,5-dihydro-1H-1,2,4-triazol-1-yl-Ringes markiert,
wobei
   R⁶ für Wasserstoff, Fluor, Methyl, Trifluormethyl oder Hydroxy steht,
   R⁷ für Wasserstoff, Fluor oder Methyl steht,
   oder
   R⁶ und R⁷ zusammen mit dem Kohlenstoffatom, an das sie gebunden sind, einen Cyclopropyl-Ring bilden,
   R⁸ für Wasserstoff, Fluor, Methyl oder Trifluormethyl steht,
   R⁹ für Wasserstoff, Fluor oder Methyl steht,
- R¹: für Phenyl oder 5- bis 6-gliedriges Heteroaryl steht,
wobei Phenyl mit 1 bis 3 Substituenten unabhängig voneinander ausgewählt aus der Gruppe Fluor, Chlor, Cyano, Methyl, Ethyl, Trifluormethyl, Methoxy, Difluormethoxy, Trifluormethoxy, Hydroxycarbonyl, Methoxycarbonyl, Ethoxycarbonyl, tert-Butoxycarbonyl, Methylaminocarbonyl, Dimethylaminocarbonyl, Methylsulfanyl, Methylsulfonyl, Methylsulfonimidoyl, Aminosulfonyl oder Methylsulfinyl substituiert ist,
   oder
wobei Phenyl mit Cyclopentyl, Cyclohexyl, 5- bis 6-gliedriges Heterocyclyl oder 5- bis 6-gliedriges Heteroaryl anneliert sein kann,
   worin (Cyclopentyl, Cyclohexyl, 5- bis 6-gliedriges Heterocyclyl und 5- bis 6-gliedriges Heteroaryl mit 1 oder 2 Substituenten Methyl oder Ethyl substituiert sein können,
   oder
wobei 5- bis 6-gliedriges Heteroaryl mit 1 bis 3 Substituenten unabhängig voneinander ausgewählt aus der Gruppe Fluor, Chlor, Cyano, Methyl, Ethyl, Trifluormethyl, Methoxy, Ethoxy, Difluormethoxy, Trifluormethoxy, 2,2,2-Trifluorethoxy, Methylaminocarbonyl, tert-Butylaminocarbonyl, Dimethylaminocarbonyl, substituiert sein kann,
wobei 5- bis 6-gliedriges Heteroaryl mit Cyclopentyl, Cyclohexyl, 5- bis 6-gliedriges Heterocyclyl, Phenyl oder 5- bis 6-gliedriges Heteroaryl anneliert sein kann,
worin 5- bis 6-gliedriges Heteroaryl mit Methyl, Ethyl, Chlor, Fluor oder Methoxy substituiert sein kann,
worin Cyclopentyl, Cyclohexyl 5- bis 6-gliedriges Heterocyclyl, Phenyl und 5- bis 6-gliedriges Heteroaryl mit 1 oder 2 Substituenten (C₁-C₄)-Alkyl substituiert sein können,
- R²: für eine Gruppe der Formel 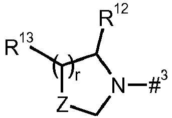 steht, wobei
#³ die Verknüpfung zum Carbonyl-Kohlenstoffatom markiert,
r für 0 oder 1 steht,
Z für S oder CR^{14A}R^{14B} steht, wenn r für 0 steht,
Z für S, SO, SO₂ oder CR^{14A}R^{14B} steht, wenn r für 1 steht,
worin jeweils
R^{14A} für Wasserstoff, Fluor, Methyl, Trifluormethyl, Difluormethyl, Fluormethyl, Hydroxy, Methoxy, Difluormethoxy oder Trifluormethoxy steht,
R^{14B} für Wasserstoff oder Fluor steht,
oder
R^{14A} und R^{14B} zusammen mit dem Kohlenstoffatom, an das sie gebunden sind eine Carbonylgruppe bilden,
R¹² für Wasserstoff, Cyano, Methyl, Acetyl oder Formyl steht, worin Acetyl mit Hydroxy oder bis zu dreimal mit Fluor substituiert ist,
R¹³ für Wasserstoff, Fluor oder Methyl steht,
oder
R¹² und R¹³ zusammen mit deen Kohlenstoffatomen, an die sie gebunden sind, einen Cyclopropyl-Ring bilden,
oder
R¹³ und R^{14A} zusammen mit dem Kohlenstoffatom, an die sie gebunden sind, einen Cyclopropyl -Ring bilden,
worin der Cyclopropyl-Ring bis zu zweimal mit Fluor substituiert sein kann, oder
R^{14A} und R^{14B} zusammen mit dem Kohlenstoffatom, an das sie gebunden sind, einen Cyclopropyl-Ring bilden,
worin der Cyclopropyl-Ring bis zu zweimal mit Fluor substituiert sein kann, wobei R¹³, R^{14A} und R^{14B} für Wasserstoff stehen, wenn R¹² nicht für Wasserstoff steht, wobei R¹² für Wasserstoff steht, wenn einer der Substituenten R¹³, R^{14A} oder R^{14B} nicht für Wasserstoff steht,
- R³: für Wasserstoff steht,
- R⁴: für Wasserstoff, Fluor oder Methyl steht,
- R⁵: für Wasserstoff steht,
sowie die Salze, Solvate und Solvate der Salze der Verbindungen der Formel (I).

Besonders bevorzugt im Rahmen der vorliegenden Erfindung sind Verbindungen der allgemeinen Formel (I), in welcher
- A: für -CH₂-,-CH(CH₃)-, -CH₂CH₂-, #⁵-CH₂CH(CH₃)-***, #⁵-CH₂C(CH₃)₂-***, #⁵-CH₂CHF-*** oder #⁵-CH₂CF₂-*** steht,
wobei #⁵ die Verknüpfung zum Stickstoffatom des 5-Oxo-4,5-dihydro-1H-1,2,4-triazol-1-yl-Ringes markiert,
wobei *** die Verknüpfung zu der R¹-Gruppe markiert,
- X: für - #²-CR⁶R⁷-CR⁸R⁹-** steht,
wobei #² die Verknüpfung zum Kohlenstoffatom der CR⁴R⁵-Gruppe markiert,
wobei ** die Verknüpfung zum Kohlenstoffatom des 5-Oxo-4,5-dihydro-1H-1,2,4-triazol-1-yl-Ringes markiert,
wobei
   R⁶ für Wasserstoff, Fluor, Methyl, Trifluormethyl oder Hydroxy steht,
   R⁷ für Wasserstoff, Fluor oder Methyl steht,
   oder
   R⁶ und R⁷ zusammen mit dem Kohlenstoffatom, an das sie gebunden sind, einen Cyclopropyl-Ring bilden,
   R⁸ für Wasserstoff, Fluor, Methyl oder Trifluormethyl steht,
   R⁹ für Wasserstoff, Fluor oder Methyl steht,
- R¹: für Phenyl, Pyrazolyl, Imidazolyl, Thiazolyl, Thiophenyl, Oxazolyl, Oxadiazolyl oder Pyridyl steht,
wobei Phenyl mit 1 bis 3 Substituenten unabhängig voneinander ausgewählt aus der Gruppe Fluor, Chlor, Cyano, Methyl, Ethyl, Trifluormethyl, Methoxy, Difluormethoxy, Trifluormethoxy, Hydroxycarbonyl, Methoxycarbonyl, Ethoxycarbonyl, tert-Butoxycarbonyl, Methylaminocarbonyl, Dimethylaminocarbonyl, Methylsulfanyl, Methylsulfonyl, Methylsulfonimidoyl, Aminosulfonyl oder Methylsulfinyl substituiert ist, oder
wobei Phenyl mit Cyclopentyl, Cyclohexyl, Pyrazolyl oder Pyridyl anneliert sein kann, worin (Cyclopentyl, Cyclohexyl, Pyrazolyl oder Pyridyl mit 1 oder 2 Substituenten Methyl oder Ethyl substituiert sein können,
   oder
wobei Pyrazolyl, Imidazolyl, Thiazolyl, Thiophenyl, Oxazolyl, Oxadiazolyl oder Pyridyl mit 1 bis 3 Substituenten unabhängig voneinander ausgewählt aus der Gruppe Fluor, Chlor, Cyano, Methyl, Ethyl, Trifluormethyl, Methoxy, Ethoxy, Difluormethoxy, Trifluormethoxy, 2,2,2-Trifluorethoxy, Methylaminocarbonyl, tert-Butylaminocarbonyl, Dimethylaminocarbonyl, substituiert sein können,
wobei Pyrazolyl, Imidazolyl, Thiazolyl, Thiophenyl, Oxazolyl, Oxadiazolyl oder Pyridyl mit Cyclopentyl, Cyclohexyl, Phenyl oder Pyrdiyl anneliert sein können,
   worin Pyridyl mit Methyl, Ethyl, Chlor, Fluor oder Methoxy substituiert sein kann,
   worin Cyclopentyl, Cyclohexyl, Phenyl und Pyridyl mit 1 oder 2 Substituenten (C₁-C₄)-Alkyl substituiert sein können,
- R²: für eine Gruppe der Formel 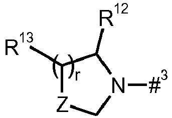 steht, wobei
#³ die Verknüpfung zum Carbonyl-Kohlenstoffatom markiert,
r für 0 oder 1 steht,
Z für S oder CR^{14A}R^{14B} steht, wenn r für 0 steht,
Z für S, SO, SO₂ oder CR^{14A}R^{14B} steht, wenn r für 1 steht,
worin jeweils
R^{14A} für Wasserstoff, Fluor, Methyl, Trifluormethyl, Difluormethyl, Fluormethyl, Hydroxy, Methoxy, Difluormethoxy oder Trifluormethoxy steht,
R^{14B} für Wasserstoff oder Fluor steht,
oder
R^{14A} und R^{14B} zusammen mit dem Kohlenstoffatom, an das sie gebunden sind eine Carbonylgruppe bilden,
R¹² für Wasserstoff, Cyano, Methyl, Acetyl oder Formyl steht,
worin Acetyl mit Hydroxy oder bis zu dreimal mit Fluor substituiert ist,
R¹³ für Wasserstoff, Fluor oder Methyl steht,
oder
R¹² und R¹³ zusammen mit deen Kohlenstoffatomen, an die sie gebunden sind, einen Cyclopropyl-Ring bilden,
oder
R¹³ und R^{14A} zusammen mit dem Kohlenstoffatom, an die sie gebunden sind, einen Cyclopropyl -Ring bilden,
worin der Cyclopropyl-Ring bis zu zweimal mit Fluor substituiert sein kann, oder
R^{14A} und R^{14B} zusammen mit dem Kohlenstoffatom, an das sie gebunden sind, einen Cyclopropyl-Ring bilden,
worin der Cyclopropyl-Ring bis zu zweimal mit Fluor substituiert sein kann, wobei R¹³, R^{14A} und R^{14B} für Wasserstoff stehen, wenn R¹² nicht für Wasserstoff steht, wobei R¹² für Wasserstoff steht, wenn einer der Substituenten R¹³, R^{14A} oder R^{14B} nicht für Wasserstoff steht,
- R³: für Wasserstoff steht,
- R⁴: für Wasserstoff, Fluor oder Methyl steht,
- R⁵: für Wasserstoff steht,
sowie die Salze, Solvate und Solvate der Salze der Verbindungen der Formel (I).

Bevorzugt sind im Rahmen der vorliegenden Erfindung auch Verbindungen der Formel (I), in welcher
- A: für -CH₂-,-CH(CH₃)-, -CH₂CH₂-, #⁵-CH₂CH(CH₃)-***, #⁵-CH₂C(CH₃)₂-***, #⁵-CH₂CHF-*** oder #⁵-CH₂CF₂-*** steht,
wobei #⁵ die Verknüpfung zum Stickstoffatom des 5-Oxo-4,5-dihydro-1H-1,2,4-triazol-1-yl-Ringes markiert,
wobei *** die Verknüpfung zu der R¹-Gruppe markiert,
sowie ihre Solvate, Salze und Solvate Salze.

Besonders bevorzugt sind im Rahmen der vorliegenden Erfindung auch Verbindungen der Formel (I), in welcher
- A: für -CH₂-,-CH(CH₃)- oder -CH₂CH₂-, steht,
sowie ihre Solvate, Salze und Solvate Salze.

Besonders bevorzugt sind im Rahmen der vorliegenden Erfindung auch Verbindungen der Formel (I), in welcher
- A: für -CH₂- steht,
sowie ihre Solvate, Salze und Solvate Salze.

Bevorzugt sind im Rahmen der vorliegenden Erfindung auch Verbindungen der Formel (I), in welcher
- X für -: #²-CR⁶R⁷-CR⁸R⁹-** steht,
wobei #² die Verknüpfung zum Kohlenstoffatom der CR⁴R⁵-Gruppe markiert,
wobei ** die Verknüpfung zum Kohlenstoffatom des 5-Oxo-4,5-dihydro-1H-1,2,4-triazol-1-yl-Ringes markiert,
wobei
   R⁶ für Wasserstoff, Fluor, Methyl, Trifluormethyl oder Hydroxy steht,
   R⁷ für Wasserstoff, Fluor oder Methyl steht,
   oder
   R⁶ und R⁷ zusammen mit dem Kohlenstoffatom, an das sie gebunden sind, einen Cyclopropyl-Ring bilden,
sowie ihre Solvate, Salze und Solvate Salze.

Bevorzugt sind im Rahmen der vorliegenden Erfindung auch Verbindungen der Formel (I), in welcher
- X: für - #²-CR⁶R⁷-CR⁸R⁹-** steht,
wobei #² die Verknüpfung zum Kohlenstoffatom der CR⁴R⁵-Gruppe markiert,
wobei ** die Verknüpfung zum Kohlenstoffatom des 5-Oxo-4,5-dihydro-1H-1,2,4-triazol-1-yl-Ringes markiert,
wobei
   R⁶ für Wasserstoff, Fluor, Methyl, Trifluormethyl oder Hydroxy steht,
   R⁷ für Wasserstoff, Fluor oder Methyl steht,
   oder
   R⁶ und R⁷ zusammen mit dem Kohlenstoffatom, an das sie gebunden sind, einen Cyclopropyl-Ring bilden,
   R⁸ für Wasserstoff, Fluor, Methyl oder Trifluormethyl steht,
   R⁹ für Wasserstoff, Fluor oder Methyl steht,
sowie ihre Solvate, Salze und Solvate Salze.

Besonders bevorzugt sind im Rahmen der vorliegenden Erfindung auch Verbindungen der Formel (I), in welcher
- X: für - #²-CR⁶R⁷-CR⁸R⁹-** steht,
wobei #² die Verknüpfung zum Kohlenstoffatom der CR⁴R⁵-Gruppe markiert,
wobei ** die Verknüpfung zum Kohlenstoffatom des 5-Oxo-4,5-dihydro-1H-1,2,4-triazol-1-yl-Ringes markiert,
wobei
   R⁶ für Wasserstoff, Fluor, Methyl oder Trifluormethyl steht,
   R⁷ für Wasserstoff steht,
sowie ihre Solvate, Salze und Solvate Salze.

Besonders bevorzugt sind im Rahmen der vorliegenden Erfindung auch Verbindungen der Formel (I), in welcher
- X: für - #²-CR⁶R⁷-CR⁸R⁹-** steht,
wobei #² die Verknüpfung zum Kohlenstoffatom der CR⁴R⁵-Gruppe markiert,
wobei ** die Verknüpfung zum Kohlenstoffatom des 5-Oxo-4,5-dihydro-1H-1,2,4-triazol-1-yl-Ringes markiert,
wobei
   R⁶ für Trifluormethyl steht,
   R⁷ für Wasserstoff steht,
   R⁸ für Wasserstoff steht,
   R⁹ für Wasserstoff steht,
sowie ihre Solvate, Salze und Solvate Salze.

Bevorzugt sind im Rahmen der vorliegenden Erfindung auch Verbindungen der Formel (I), in welcher
- X: für - #²-CR⁶R⁷-CR⁸R⁹-** steht,
wobei #² die Verknüpfung zum Kohlenstoffatom der CR⁴R⁵-Gruppe markiert,
wobei ** die Verknüpfung zum Kohlenstoffatom des 5-Oxo-4,5-dihydro-1H-1,2,4-triazol-1-yl-Ringes markiert,
wobei
   R⁶ für Wasserstoff steht,
   R⁷ für Wasserstoff steht,
   R⁸ für Wasserstoff, Fluor, Methyl oder Trifluormethyl steht,
   R⁹ für Wasserstoff, Fluor oder Methyl steht,
sowie ihre Solvate, Salze und Solvate Salze.

Besonders bevorzugt sind im Rahmen der vorliegenden Erfindung auch Verbindungen der Formel (I), in welcher
- X: für - #²-CR⁶R⁷-CR⁸R⁹-** steht,
wobei #² die Verknüpfung zum Kohlenstoffatom der CR⁴R⁵-Gruppe markiert,
wobei ** die Verknüpfung zum Kohlenstoffatom des 5-Oxo-4,5-dihydro-1H-1,2,4-triazol-1-yl-Ringes markiert,
wobei
   R⁶ für Wasserstoff steht,
   R⁷ für Wasserstoff steht,
   R⁸ für Fluor steht,
   R⁹ für Fluor steht,
sowie ihre Solvate, Salze und Solvate Salze.

Besonders bevorzugt sind im Rahmen der vorliegenden Erfindung auch Verbindungen der Formel (I), in welcher
- X: für - #²-CR⁶R⁷-CR⁸R⁹-** steht,
wobei #² die Verknüpfung zum Kohlenstoffatom der CR⁴R⁵-Gruppe markiert,
wobei ** die Verknüpfung zum Kohlenstoffatom des 5-Oxo-4,5-dihydro-1H-1,2,4-triazol-1-yl-Ringes markiert,
wobei
   R⁶ für Wasserstoff steht,
   R⁷ für Wasserstoff steht,
   R⁸ für Wasserstoff steht,
   R⁹ für Wasserstoff steht,
sowie ihre Solvate, Salze und Solvate Salze.

Die vorliegende Erfindung betrifft Verbindungen der Formel (I), in welcher
- R¹: für Phenyl oder 5- bis 6-gliedriges Heteroaryl steht,
wobei Phenyl mit 1 bis 3 Substituenten unabhängig voneinander ausgewählt aus der Gruppe Fluor, Chlor, Cyano, Methyl, Ethyl, Trifluormethyl, Methoxy, Difluormethoxy, Trifluormethoxy, Hydroxycarbonyl, Methoxycarbonyl, Ethoxycarbonyl, tert-Butoxycarbonyl, Methylaminocarbonyl, Dimethylaminocarbonyl, Methylsulfanyl, Methylsulfonyl, Methylsulfonimidoyl, Aminosulfonyl oder Methylsulfinyl substituiert ist,
oder
wobei Phenyl mit Cyclopentyl, Cyclohexyl, 5- bis 6-gliedriges Heterocyclyl oder 5- bis 6-gliedriges Heteroaryl anneliert sein kann,
   worin (Cyclopentyl, Cyclohexyl, (C₅-C₆)-Heterocycyl und 5- bis 6-gliedriges Heteroaryl mit 1 oder 2 Substituenten Methyl oder Ethyl substituiert sein können,
   oder
wobei 5- bis 6-gliedriges Heteroaryl mit 1 bis 3 Substituenten unabhängig voneinander ausgewählt aus der Gruppe Fluor, Chlor, Cyano, Methyl, Ethyl, Trifluormethyl, Methoxy, Ethoxy, Difluormethoxy, Trifluormethoxy, 2,2,2-Trifluorethoxy, Methylaminocarbonyl, tert-Butylaminocarbonyl, Dimethylaminocarbonyl, substituiert sein kann, wobei 5- bis 6-gliedriges Heteroaryl mit Cyclopentyl, Cyclohexyl, 5- bis 6-gliedriges Heterocyclyl, Phenyl oder 5- bis 6-gliedriges Heteroaryl anneliert sein kann,
   worin 5- bis 6-gliedriges Heteroaryl mit Methyl, Ethyl, Chlor, Fluor oder Methoxy substituiert sein kann,
   worin Cyclopentyl, Cyclohexyl 5- bis 6-gliedriges Heterocyclyl, Phenyl und 5- bis 6-gliedriges Heteroaryl mit 1 oder 2 Substituenten (C₁-C₄)-Alkyl substituiert sein können,
sowie ihre Solvate, Salze und Solvate Salze.

Bevorzugt sind im Rahmen der vorliegenden Erfindung auch Verbindungen der Formel (I), in welcher
- R¹: für 5- bis 6-gliedriges Heteroaryl steht,
wobei 5- bis 6-gliedriges Heteroaryl mit 1 bis 3 Substituenten unabhängig voneinander ausgewählt aus der Gruppe Fluor, Chlor, Cyano, Methyl, Ethyl, Trifluormethyl, Methoxy, Ethoxy, Difluormethoxy, Trifluormethoxy, 2,2,2-Trifluorethoxy, Methylaminocarbonyl, tert-Butylaminocarbonyl, Dimethylaminocarbonyl, substituiert sein kann,
wobei 5- bis 6-gliedriges Heteroaryl mit Cyclopentyl, Cyclohexyl, 5- bis 6-gliedriges Heterocyclyl, Phenyl oder 5- bis 6-gliedriges Heteroaryl anneliert sein kann,
   worin 5- bis 6-gliedriges Heteroaryl mit Methyl, Ethyl, Chlor, Fluor oder Methoxy substituiert sein kann,
   worin Cyclopentyl, Cyclohexyl 5- bis 6-gliedriges Heterocyclyl, Phenyl und 5- bis 6-gliedriges Heteroaryl mit 1 oder 2 Substituenten (C₁-C₄)-Alkyl substituiert sein können,
sowie ihre Solvate, Salze und Solvate Salze.

Bevorzugt sind im Rahmen der vorliegenden Erfindung auch Verbindungen der Formel (I), in welcher
- R¹: für Phenyl, Pyrazolyl, Imidazolyl, Thiazolyl, Thiophenyl, Oxazolyl, Oxadiazolyl oder Pyridyl steht,
wobei Phenyl mit 1 bis 3 Substituenten unabhängig voneinander ausgewählt aus der Gruppe Fluor, Chlor, Cyano, Methyl, Ethyl, Trifluormethyl, Methoxy, Difluormethoxy, Trifluormethoxy, Hydroxycarbonyl, Methoxycarbonyl, Ethoxycarbonyl, tert-Butoxycarbonyl, Methylaminocarbonyl, Dimethylaminocarbonyl, Methylsulfanyl, Methylsulfonyl, Methylsulfonimidoyl, Aminosulfonyl oder Methylsulfinyl substituiert ist,
   oder
wobei Phenyl mit Cyclopentyl, Cyclohexyl, Pyrazolyl oder Pyridyl anneliert sein kann,
   worin (Cyclopentyl, Cyclohexyl, Pyrazolyl oder Pyridyl mit 1 oder 2 Substituenten Methyl oder Ethyl substituiert sein können,
   oder
wobei Pyrazolyl, Imidazolyl, Thiazolyl, Thiophenyl, Oxazolyl, Oxadiazolyl oder Pyridyl mit 1 bis 3 Substituenten unabhängig voneinander ausgewählt aus der Gruppe Fluor, Chlor, Cyano, Methyl, Ethyl, Trifluormethyl, Methoxy, Ethoxy, Difluormethoxy, Trifluormethoxy, 2,2,2-Trifluorethoxy, Methylaminocarbonyl, tert-Butylaminocarbonyl, Dimethylaminocarbonyl, substituiert sein können,
wobei Pyrazolyl, Imidazolyl, Thiazolyl, Thiophenyl, Oxazolyl, Oxadiazolyl oder Pyridyl mit Cyclopentyl, Cyclohexyl, Phenyl oder Pyrdiyl anneliert sein können,
   worin Pyridyl mit Methyl, Ethyl, Chlor, Fluor oder Methoxy substituiert sein kann,
   worin Cyclopentyl, Cyclohexyl, Phenyl und Pyridyl mit 1 oder 2 Substituenten (C₁-C₄)-Alkyl substituiert sein können,
sowie ihre Solvate, Salze und Solvate Salze.

Bevorzugt sind im Rahmen der vorliegenden Erfindung auch Verbindungen der Formel (I), in welcher
- R¹: für Phenyl, steht,
wobei Phenyl mit 1 bis 3 Substituenten unabhängig voneinander ausgewählt aus der Gruppe Fluor, Chlor, Cyano, Methyl, Ethyl, Trifluormethyl, Methoxy, Difluormethoxy, Trifluormethoxy, Hydroxycarbonyl, Methoxycarbonyl, Ethoxycarbonyl, tert-Butoxycarbonyl, Methylaminocarbonyl, Dimethylaminocarbonyl, Methylsulfanyl, Methylsulfonyl, Methylsulfonimidoyl, Aminosulfonyl oder Methylsulfinyl substituiert ist,
   oder
wobei Phenyl mit Cyclopentyl, Cyclohexyl, Pyrazolyl oder Pyridyl anneliert sein kann,
   worin (Cyclopentyl, Cyclohexyl, Pyrazolyl oder Pyridyl mit 1 oder 2 Substituenten Methyl oder Ethyl substituiert sein können,
sowie ihre Solvate, Salze und Solvate Salze.

Besonders bevorzugt sind im Rahmen der vorliegenden Erfindung auch Verbindungen der Formel (I), in welcher
- R¹: für Pyrazolyl, Imidazolyl, Thiazolyl, Thiophenyl, Oxazolyl oder Oxadiazolyl steht,
wobei Phenyl mit Cyclopentyl, Cyclohexyl, Pyrazolyl oder Pyridyl anneliert sein kann,
   worin (Cyclopentyl, Cyclohexyl, Pyrazolyl oder Pyridyl mit 1 oder 2 Substituenten Methyl oder Ethyl substituiert sein können,
wobei Pyrazolyl, Imidazolyl, Thiazolyl, Thiophenyl, Oxazolyl, Oxadiazolyl oder mit 1 bis 3 Substituenten unabhängig voneinander ausgewählt aus der Gruppe Fluor, Chlor, Cyano, Methyl, Ethyl, Trifluormethyl, Methoxy, Ethoxy, Difluormethoxy, Trifluormethoxy, 2,2,2-Trifluorethoxy, Methylaminocarbonyl, tert-Butylaminocarbonyl, Dimethylaminocarbonyl, substituiert sein können,
wobei Pyrazolyl, Imidazolyl, Thiazolyl, Thiophenyl, Oxazolyl, Oxadiazolyl oder Pyridyl mit Cyclopentyl, Cyclohexyl, Phenyl oder Pyrdiyl anneliert sein können,
   worin Pyridyl mit Methyl, Ethyl, Chlor, Fluor oder Methoxy substituiert sein kann,
   worin Cyclopentyl, Cyclohexyl, Phenyl und Pyridyl mit 1 oder 2 Substituenten (C₁-C₄)-Alkyl substituiert sein können,
sowie ihre Solvate, Salze und Solvate Salze.

Besonders bevorzugt sind im Rahmen der vorliegenden Erfindung auch Verbindungen der Formel (I), in welcher
- R¹: für Pyridyl steht,
wobei Pyridyl mit 1 oder 2 Substituenten unabhängig voneinander ausgewählt aus der Gruppe Fluor, Chlor, Trifluormethyl und Methoxy substituiert sein kann,
sowie ihre Solvate, Salze und Solvate Salze.

Bevorzugt sind im Rahmen der vorliegenden Erfindung auch Verbindungen der Formel (I), in welcher
- R²: für eine Gruppe der Formel 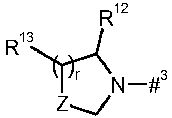 steht, wobei
#³ die Verknüpfung zum Carbonyl-Kohlenstoffatom markiert,
r für 0 oder 1 steht,
Z für S oder CR^{14A}R^{14B} steht, wenn r für 0 steht,
Z für S, SO, SO₂ oder CR^{14A}R^{14B} steht, wenn r für 1 steht,
worin jeweils
R^{14A} für Wasserstoff, Fluor, Methyl, Trifluormethyl, Difluormethyl, Fluormethyl, Hydroxy, Methoxy, Difluormethoxy oder Trifluormethoxy steht,
R^{14B} für Wasserstoff oder Fluor steht,
oder
R^{14A} und R^{14B} zusammen mit dem Kohlenstoffatom, an das sie gebunden sind eine Carbonylgruppe bilden,
R¹² für Wasserstoff, Cyano, Methyl, Acetyl oder Formyl steht,
worin Acetyl mit Hydroxy oder bis zu dreimal mit Fluor substituiert ist,
R¹³ für Wasserstoff, Fluor oder Methyl steht,
oder
R¹² und R¹³ zusammen mit deen Kohlenstoffatomen, an die sie gebunden sind, einen Cyclopropyl-Ring bilden,
oder
R¹³ und R^{14A} zusammen mit den Kohlenstoffatomen, an die sie gebunden sind, einen Cyclopropyl -Ring bilden,
worin der Cyclopropyl-Ring bis zu zweimal mit Fluor substituiert sein kann, R¹² und R¹³
oder
R^{14A} und R^{14B} zusammen mit dem Kohlenstoffatom, an das sie gebunden sind, einen Cyclopropyl-Ring bilden,
worin der Cyclopropyl-Ring bis zu zweimal mit Fluor substituiert sein kann, wobei R¹³, R^{14A} und R^{14B} für Wasserstoff stehen, wenn R¹² nicht für Wasserstoff steht, wobei R¹² für Wasserstoff steht, wenn einer der Substituenten R¹³, R^{14A} oder R^{14B} nicht für Wasserstoff steht,
sowie ihre Solvate, Salze und Solvate Salze.

Bevorzugt sind im Rahmen der vorliegenden Erfindung auch Verbindungen der Formel (I), in welcher
- R³: für Wasserstoff steht,
sowie ihre Solvate, Salze und Solvate Salze.

Bevorzugt sind im Rahmen der vorliegenden Erfindung auch Verbindungen der Formel (I), in welcher
- R⁴: für Wasserstoff, Fluor oder Methyl steht,
sowie ihre Solvate, Salze und Solvate Salze.

Bevorzugt sind im Rahmen der vorliegenden Erfindung auch Verbindungen der Formel (I), in welcher
- R⁵: für Wasserstoff steht,
sowie ihre Solvate, Salze und Solvate Salze.

Weiterer Gegenstand der Erfindung ist ein Verfahren zur Herstellung der erfindungsgemäßen Verbindungen der Formel (I) dadurch gekennzeichnet, dass man
[A] eine Verbindung der Formel (II) 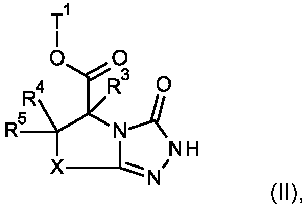 in welcher R³, R⁴, R⁵ und X jeweils die in Anspruch 1 genannten Bedeutungen haben,
   und
   - T¹: für (C₁-C₄)-Alkyl oder Benzyl steht,
   in einem inerten Lösungsmittel in Gegenwart einer geeigneten Base mit einer Verbindung der Formel (III) 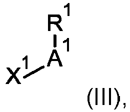 in welcher
   - A': für -CH₂-,-CH(CH₃)-, -CH₂CH₂-, #⁵-CH₂CH(CH₃)-***, #⁵-CH₂C(CH₃)₂-***, #⁵-CH₂CHF-*** oder #⁵-CH₂CF₂-*** steht,
   wobei #⁵ die Verknüpfung zu X¹ markiert,
   wobei *** die Verknüpfung zu der R¹-Gruppe markiert,
   - R¹: die in Anspruch 1 genannten Bedeutungen hat,
   und
   - X¹: für eine geeignete Abgangsgruppe, insbesondere Chlor, Brom, lod, Mesylat {(methylsulfonyl)oxy}, Triflat {[(trifluormethyl)sulfonyl]oxy}, Nonaflat {[(nonafluor-butyl)sulfonyl]oxy}, Nosylat {[(4-nitro-phenyl)sulfonyl]oxy} oder Tosylat {[(4-methyl-phenyl)sulfonyl]oxy} steht,
   zu einer Verbindung der Formel (IV) 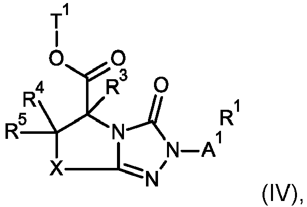 in welcher A¹, R¹, R³, R⁴, R⁵, X jeweils die oben genannten Bedeutungen haben,und
   - T¹: für (C₁-C₄)-Alkyl oder Benzyl steht,
   umsetzt,
   diese dann durch Entfernen der Gruppe "T¹" in einem inerten Lösungsmittel in Gegenwart einer geeigneten Base oder Säure in eine Verbindung der Formel (V) 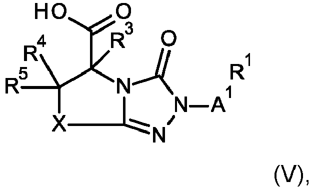 überführt,
   in welcher A¹, R¹, R³, R⁴, R⁵ und X jeweils die in Anspruch 1 genannten Bedeutungen haben,
   und diese dann in einem inerten Lösungsmittel unter Amidkupplungsbedingungen mit einem Amin der Formel (VI-A) bzw. (VI-B), 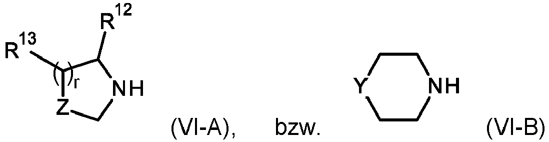
   in welcher r, Y, Z, R¹² und R¹³ jeweils die Anspruch 1 genannten Bedeutungen haben,
   umsetzt und anschließend gegebenfalls vorhandene Schutzgruppen nach dem Fachmann bekannten Methoden abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt,
   oder
[B] eine Verbindung der Formel (II) 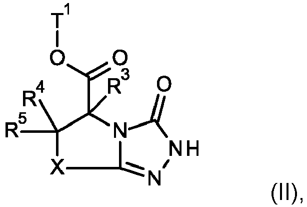 in welcher R³, R⁴, R⁵ und X jeweils die in Anspruch 1 genannten Bedeutungen haben,
   und
   - T¹: für (C₁-C₄)-Alkyl oder Benzyl steht,
   in einem inerten Lösungsmittel in Gegenwart einer geeigneten Base mit einem einem Amin der Formel (VI-A) bzw. (VI-B), 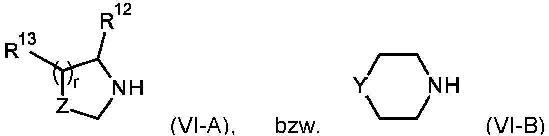 in welcher r, Y, Z, R¹² und R¹³ jeweils die oben genannten Bedeutungen haben, zu einer Verbindung der Formel (XXIII-A) bzw. (XXIII-B), 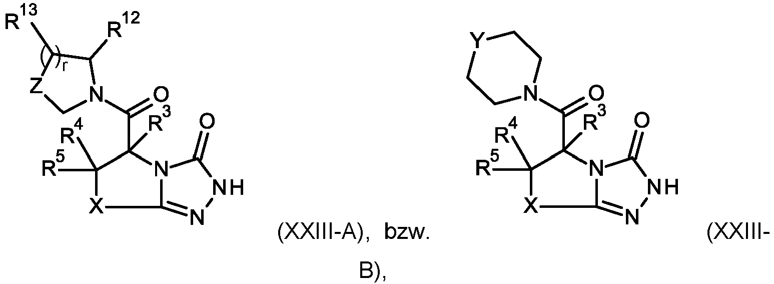
   in welcher r, R³, R⁴, R⁵, R¹², R¹³, X, Y und Z jeweils die in Anspruch 1 genannten Bedeutungen haben,
   umsetzt,
   diese in einem inerten Lösungsmittel in Gegenwart einer geeigneten Base mit einer Verbindung der Formel (III) 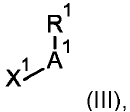 in welcher
      - A': für -CH₂-,-CH(CH₃)-, -CH₂CH₂-, #⁵-CH₂CH(CH₃)-***, #⁵-CH₂C(CH₃)₂-***, #⁵-CH₂CHF-*** oder #⁵-CH₂CF₂-*** steht, wobei #⁵ die Verknüpfung zu X¹ markiert, wobei *** die Verknüpfung zu der R¹-Gruppe markiert,
      - R¹: die oben genannten Bedeutungen hat,
      und
      - X¹: für eine geeignete Abgangsgruppe, insbesondere Chlor, Brom, lod, Mesylat {(methylsulfonyl)oxy}, Triflat {[(trifluormethyl)sulfonyl]oxy}, Nonaflat {[(nonafluor-butyl)sulfonyl]oxy}, Nosylat {[(4-nitro-phenyl)sulfonyl]oxy} oder Tosylat {[(4-methyl-phenyl)sulfonyl]oxy} steht,
   umsetzt und anschließend gegebenfalls vorhandene Schutzgruppen nach dem Fachmann bekannten Methoden abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt,
   oder
[C] eine Verbindung der Formel (VII) 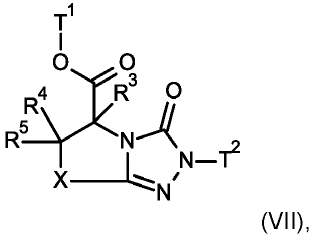 in welcher R³, R⁴, R⁵ und X jeweils die In Anspruch 1 genannten Bedeutungen haben und
   - T¹: für (C₁-C₄)-Alkyl oder Benzyl steht,
   - T²: für 4-Methoxy-Benzyl, Benzyl, Allyl, β-(Trimethylsilyl)ethoxymethyl (SEM), Methoxymethyl (MOM) oder Benzyloxymethyl steht,
   durch Hydrolyse der Estergruppe in einem inerten Lösungsmittel in Gegenwart einer geeigneten Base oder Säure in eine Verbindung der Formel (VIII) 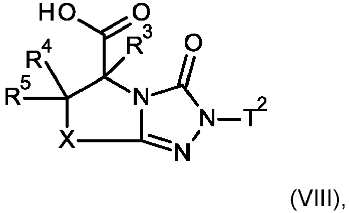 überführt,
   in welcher R³, R⁴, R⁵ und X jeweils die in Anspruch 1 sowie die oben genannten Bedeutungen haben,
   und
   - T²: für 4-Methoxy-Benzyl, Benzyl, Allyl, β-(Trimethylsilyl)ethoxymethyl (SEM), Methoxymethyl (MOM) oder Benzyloxymethyl steht,
   diese dann in einem inerten Lösungsmittel unter Amidkupplungsbedingungen mit einem Amin der Formel (VI-A) 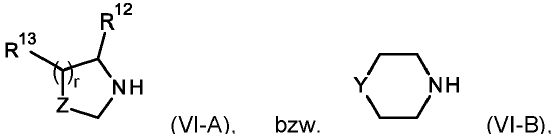 in welcher r, Y, Z, R¹² und R¹³ jeweils die in Anspruch 1 genannten Bedeutungen haben,
   zu einer Verbindung der Formel (IX-A) bzw. (IX-B), 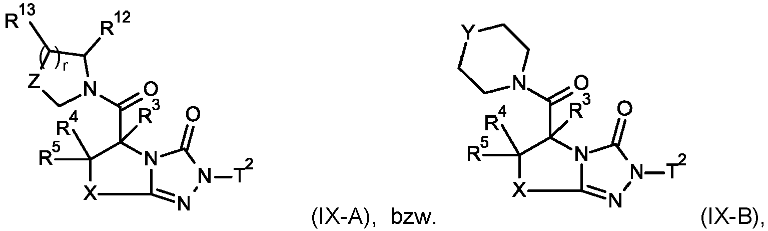
   in welcher r, R³, R⁴, R⁵, R¹², R¹³, T², X, Y und Z jeweils die oben genannten Bedeutungen haben,
      umsetzt,
   die Schutzgruppe "T²" in einem inerten Lösungsmittel in Gegenwart einer geeigneten Base oder Säure oder gegebenenfalls in Anwesenheit eines geeigneten Palladium-Katalysators entfernt und die resultierende Verbindung der Formel (X-A) bzw. (X-B), 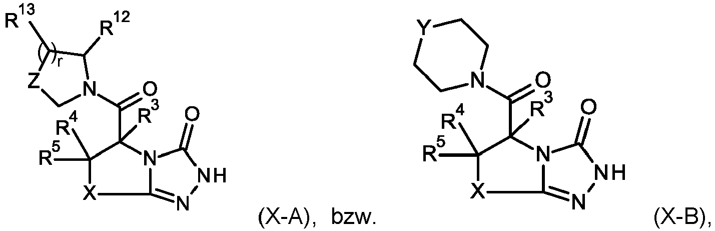
   in welcher r, R³, R⁴, R⁵, R¹², R¹³, X, Y und Z jeweils die in Anspruch 1 genannten Bedeutungen haben,
   in einem inerten Lösungsmittel in Gegenwart einer geeigneten Base mit einer Verbindung der Formel (III) 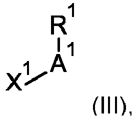 in welcher
      - A': für -CH₂-,-CH(CH₃)-, -CH₂CH₂-, #⁵-CH₂CH(CH₃)-***, #⁵-CH₂C(CH₃)₂-***, #⁵-CH₂CHF-*** oder #⁵-CH₂CF₂-*** steht, wobei #⁵ die Verknüpfung zu X¹ markiert, wobei *** die Verknüpfung zu der R¹-Gruppe markiert,
      - R¹: die oben genannten Bedeutungen hat,
      und
      - X¹: für eine geeignete Abgangsgruppe, insbesondere Chlor, Brom, lod, Mesylat {(methylsulfonyl)oxy}, Triflat {[(trifluormethyl)sulfonyl]oxy}, Nonaflat {[(nonafluor-butyl)sulfonyl]oxy}, Nosylat {[(4-nitro-phenyl)sulfonyl]oxy} oder Tosylat {[(4-methyl-phenyl)sulfonyl]oxy} steht,
   umsetzt und anschließend gegebenfalls vorhandene Schutzgruppen nach dem Fachmann bekannten Methoden abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

Die Verbindungen der Formeln (III), (VI-A) und (VI-B) sind kommerziell erhältlich, literaturbekannt oder können in Analogie zu literaturbekannten Verfahren hergestellt werden.

Inerte Lösungsmittel für den Verfahrensschritt (II) + (III) -> (IV) bzw. (X) + (III) → (I) sind beispielsweise Halogenkohlenwasserstoffe wie Dichlormethan, Tetrachlormethan, Trichlorethylen oder Chlorbenzol, Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Alkohole wie Methanol, Ethanol, n-Propanol, Isopropanol, n-Butanol, 2,2,2-Trifluorethanol oder andere Lösungsmittel wie Aceton, Methylethylketon, Essigsäureethylester, Acetonitril, *N,N*-Dimethylformamid, *N,N-*Dimethylacetamid, Dimethylsulfoxid, *N*,*N*'-Dimethylpropylenharnstoff(DMPU), *N*-Methylpyrrolidon (NMP) oder Pyridin. Ebenso ist es möglich, Gemische der genannten Lösungsmittel einzusetzen. Bevorzugt wird Dimethylformamid oder Acetonitril verwendet.

Als Basen für den Verfahrensschritt (II) + (III) -> (IV) bzw. (X) + (III) → (I) eignen sich die üblichen anorganischen oder organischen Basen. Hierzu gehören bevorzugt Lithium, Natrium, Kalium, Alkalihydroxide wie beispielsweise Lithium-, Natrium- oder Kaliumhydroxid, Alkoholate wie Kalium-tert-butylat, Methanolat, Ethanolat, Alkali- oder carbonate wie Lithium-, Natrium-, Kalium-, Calcium- oder Cäsiumcarbonat gegebenenfalls unter Zusatz eines
Alkaliiodids wie beispielsweise Natriumiodid oder Kaliumiodid oder organische Amine wie Triethylamin, *N*-Methylmorpholin, *N*-Methylpiperidin, *N*,*N*-Diisopropylethylamin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Cäsiumcarbonat verwendet.

Die Reaktion erfolgt im Allgemeinen in einem Temperaturbereich von 0°C bis +120°C, bevorzugt bei +20°C bis +80°C, ganz besonders bevorzugt bei Raumtemperatur, gegebenenfalls in einer Mikrowelle. Die Umsetzung kann bei normalem, erhöhtem oder bei erniedrigtem Druck durchgeführt werden (z.B. von 0.5 bis 5 bar).

Die Hydrolyse der Ester der Verbindungen (IV) zu Verbindungen der Formel (V) bzw. der Verbindungen (VII) zu Verbindungen der Formel (VIII) erfolgt nach üblichen Methoden, indem man die Ester in inerten Lösungsmitteln mit Säuren oder Basen behandelt, wobei bei letzterem die zunächst entstehenden Salze durch Behandeln mit Säure in die freien Carbonsäuren überführt werden. Im Falle der tert.-Butylester erfolgt die Esterspaltung bevorzugt mit Säuren.

Als inerte Lösungsmittel eignen sich für diese Reaktionen Wasser oder die für eine Esterspaltung üblichen organischen Lösungsmittel. Hierzu gehören bevorzugt Alkohole wie Methanol, Ethanol, n-Propanol, Isopropanol, n-Butanol oder tert.-Butanol, oder Ether wie Diethylether, Tetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit Dioxan, Tetrahydrofuran, Methanol und/oder Ethanol und/oder n-Propanol eingesetzt. Im Falle der Umsetzung mit Trifluoressigsäure wird bevorzugt Dichlormethan und im Falle der Umsetzung mit Chlorwasserstoff bevorzugt Tetrahydrofuran, Diethylether, Dioxan oder Wasser verwendet.

Als Basen sind die üblichen anorganischen Basen geeignet. Hierzu gehören bevorzugt Alkali- oder Erdalkalihydroxide wie beispielsweise Natrium-, Lithium-, Kalium- oder Bariumhydroxid, oder Alkali- oder Erdalkalicarbonate wie Natrium-, Kalium- oder Calciumcarbonat. Besonders bevorzugt sind Natrium- oder Lithiumhydroxid.

Als Säuren eignen sich für die Esterspaltung im Allgemeinen Schwefelsäure, Chlorwasserstoff/Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der tert.-Butylester und Salzsäure im Falle der Methylester.

Die Esterspaltung erfolgt im Allgemeinen in einem Temperaturbereich von 0°C bis +100°C, bevorzugt bei +0°C bis +50°C, besonders bevorzugt bei Raumtemperatur.

Die genannten Umsetzungen können bei normalem, erhöhtem oder bei erniedrigtem Druck durchgeführt werden (z.B. von 0.5 bis 5 bar). Im Allgemeinen arbeitet man jeweils bei Normaldruck.

Inerte Lösungsmittel für die Amidkupplung (V) + (VI-A) → (I) bzw. (V) + (VI-B) → (I) bzw. (VIII) + (VI-A) → (IX-A) bzw. (VIII) + (VI-B) → (IX-B) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Essigsäureethylester, Acetonitril, Pyridin, Dimethylsulfoxid, N,N-Dimethylformamid, N,N-Dimethylacetamid, *N,N'-*Dimethylpropylenharnstoff (DMPU) oder N-Methylpyrrolidon (NMP). Ebenso ist es möglich, Gemische der genannten Lösungsmittel zu verwenden. Bevorzugt sind Dichlormethan, Tetrahydrofuran, Dimethylformamid oder Gemische dieser Lösungsmittel.

Als Kondensationsmittel für die Amidbildung v bzw. (VIII) + (VI-A) -> (IX-A) bzw. (VIII) + (VI-B) -> (IX-B) eignen sich beispielsweise Carbodiimide wie N,N'-Diethyl-, N,N'-Dipropyl-, N,N'-Diisopropyl-, N,N'-Dicyclohexylcarbodiimid (DCC) oder N-(3-Dimethylaminopropyl)-N'-ethylcarbo-diimid-Hydrochlorid (EDC), Phosgen-Derivate wie N,N'-Carbonyldiimidazol (CDI), 1,2-Oxazoliumverbindungen wie 2-Ethyl-5-phenyl-1,2-oxazolium-3-sulfat oder 2-tert.-Butyl-5-methyl-isoxazolium-perchlorat, Acylaminoverbindungen wie 2-Ethoxy-1-ethoxycarbonyl-1,2-di-hydrochinolin, oder Isobutylchlorformiat, Propanphosphonsäureanhydrid (T3P), 1-Chlor-N,N,2-trimethylprop1-en-1-amin, Cyanophosphonsäurediethylester, Bis-(2-oxo-3-oxazolidinyl)-phos-phorylchlorid, Benzotriazol-1-yloxy-tris(dimethylamino)phosphonium-hexafluorphosphat, Benzotriazol-1-yloxy-tris(pyrrolidino)phosphonium-hexafluorphosphat (PyBOP), *O*-(Benzotriazol-1-yl)-*N*,*N*,*N*',*N*'-tetramethyluronium-tetrafluorborat (TBTU), *O*-(Benzotriazol-1-yl)-*N*,*N*,*N*',*N*'-tetramethyluronium-hexafluorphosphat (HBTU), 2-(2-Oxo-1-(2*H*)-pyridyl)-1,1,3,3-tetramethyluronium-tetrafluorborat (TPTU), *O*-(7-Azabenzotriazol-1-yl)-*N,N,N',N*-tetramethyl-uronium-hexafluorphosphat (HATU) oder *O*-(1*H*-6-Chlorbenzotriazol-1-yl)-1,1,3,3-tetramethyl-uronium-tetrafluorborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat oder -hydrogencarbonat, oder organische Basen wie Trialkylamine, z.B. Triethylamin, *N*-Methylmorpholin, *N*-Methylpiperidin oder *N,N-*Diisopropylethylamin. Bevorzugt wird HATU verwendet.

Die Kondensationen (V) + (VI-A) -> (I) bzw. (V) + (VI-B) -> (I) bzw. (VIII) + (VI-A) -> (IX-A) bzw. (VIII) + (VI-B) -> (IX-B) wird im Allgemeinen in einem Temperaturbereich von -20°C bis +100°C, bevorzugt bei 0°C bis +60°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Alternativ kann die Carbonsäure der Formel (V) bzw (VIII) auch zunächst in das entsprechende Carbonsäurechlorid überführt werden und dieses dann direkt oder in einer separaten Umsetzung mit einem Amin der Formel (VI) zu den erfindungsgemäßen Verbindungen umgesetzt werden. Die Bildung von Carbonsäurechloriden aus Carbonsäuren erfolgt nach den dem Fachmann bekannten Methoden, beispielsweise durch Behandlung mit Thionylchlorid, oder Oxalylchlorid in Gegenwart einer geeigneten Base, beispielsweise in Gegenwart von Pyridin, sowie optional unter Zusatz von Dimethylformamid, optional in einem geeigneten inerten Lösemittel.

Die Abspaltung der Schutzgruppe im Reaktionsschritt (IX) -> (X) erfolgt hierbei nach üblichen, aus der Schutzgruppenchemie bekannten Methoden, vorzugsweise durch Umsetzung mit Säure wie beispielsweise Trifluoressigsäure in Dichlormethan, durch Base wie beispielsweise Ammoniak in Methanol, Hydrogenolyse in Gegenwart eines Palladiumkatalysators, wie beispielsweise Palladium auf Aktivkohle, in einem inerten Lösungsmittel, wie beispielsweise Ethanol oder Essigsäureethylester, 2,3-Dichlor-5,6-dicyano-1,4-benzochinon (DDQ) oder Ammoniumcer(IV)-nitrat (CAN) [siehe auch z.B. T.W. Greene und P.G.M. Wuts, Protective Groups in Organic Synthesis, Wiley, New York, 1999].

Inerte Lösungsmittel für Umsetzung (II) + (VI-A) -> (XXIII-A) bzw. (II) + (VI-B) -> (XXIII-B) sind beispielsweise Pyrrolidin, Toluol, Acetonitril, Tetrahydrofuran oder Dichlormethan. Bevorzugt wird Pyrrolidin verwendet.

Umsetzung (II) + (VI-A) -> (XXIII-A) bzw. (II) + (VI-B) -> (XXIII-B) wird im Allgemeinen in einem Temperaturbereich von 20°C bis +200°C, bevorzugt bei 80°C bis +200°C durchgeführt. Die Umsetzung kann bei normalem oder erhöhtem Druck erfolgen (z.B. von 1 bis 5 bar). Im Allgemeinen arbeitet man bei Normaldruck

Die Verbindungen der Formel (II), in denen X für #²-CR⁶R⁷-CR⁸R⁹-** und R³, R⁷ und R⁹ für Wasserstoff stehen, können hergestellt werden, indem eine Verbindung der Formel (XI) 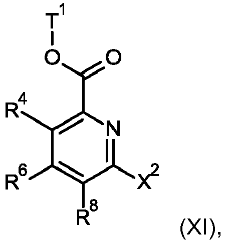 in welcher
T¹ für (C₁-C₄)-Alkyl oder Benzyl steht,
X² für Fluor Chlor, Brom oder lod steht,
und R⁴, R⁶ und R⁸ jeweils die oben genannten Bedeutungen haben,

in einem inerten Lösungsmittel in Gegenwart einer geeigneten Base und gegebenenfalls eines Palladiumkatalysators mit einer Verbindung der Formel XII, 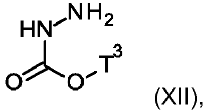 in welcher
T³ für Benzyl oder tert-Butyl steht,
zu einer Verbindung der Formel (XIII), 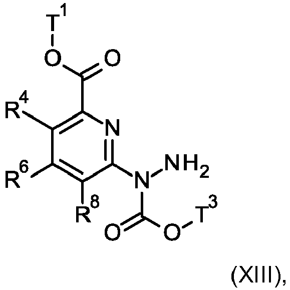 in welcher
R⁴, R⁶, R⁸,T¹ und T³ jeweils die oben genannten Bedeutungen haben,
umsetzt, die Schutzgruppe T³ in einem geeigneten inerten Lösungsmittel durch Hydrogenolyse in Gegenwart eines Palladiumkatalysators abgespalten wird und die daraus resultierende Verbindung der Formel (XIV) 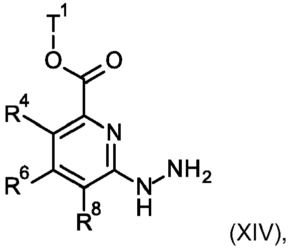 in welcher
R⁴, R⁶, R⁸ und T¹ jeweils die oben genannten Bedeutungen haben,
in einem geeigneten Lösungsmittel mit einem Phosgen-Derivat zu einer Verbindung der Formel (XV), 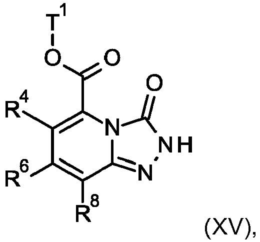
in welcher R⁴, R⁶, R⁸ und T¹ jeweils die oben genannten Bedeutungen haben,
cyclisiert werden und anschließend in einem geeigneten Lösungsmittel in Gegenwart eines Palladiumkatalysators in einer Wasserstoffatmosphäre hydriert wird,
und die resultierenden Verbindungen der Formel (II) gegebenenfalls mit den entsprechenden (i) Lösungsmitteln und/oder (ii) Säuren oder Basen in ihre Solvate, Salze und/oder Solvate der Salze überführt.

Die Verbindungen der Formel (II) bzw. (IV), in denen X für #²-CR⁶R⁷-CR⁸R⁹-** steht, können hergestellt werden, indem eine Verbindung der Formel (XVI), 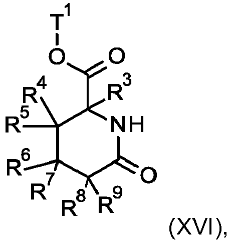
in welcher T¹ für (C₁-C₄)-Alkyl oder Benzyl steht,
und R³, R⁴, R⁵, R⁶, R⁷, R⁸ und R⁹ jeweils die oben genannten Bedeutungen haben,
in einem geeigneten Lösungsmittel in Anwesenheit von Trimethyloxonium-tetrafluoroborat mit einer Verbindung der Formel (XVII-A) bzw. (XVII-B), 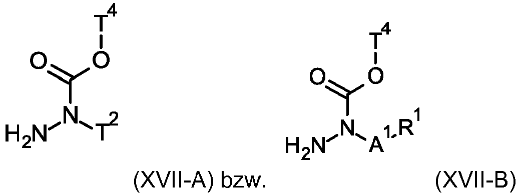 in welcher
   - T²: für 4-Methoxy-Benzyl steht,
   - T⁴: für Methyl oder Ethyl steht,
   und A¹ und R¹ jeweils die oben genannten Bedeutungen haben,
   zu einer Verbindung der Formel (XVIII-A) bzw. (XVIII-B) 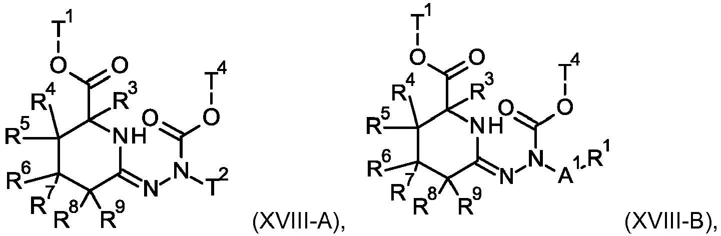 in welcher
A¹, R¹, R³, R⁴, R⁵, R⁶, R⁷, R⁸, R⁹ T¹, T² und T⁴ jeweils die oben genannten Bedeutungen haben,
umsetzt, diese in einem geeigneten Lösungsmittel zu einer Verbindung der Formel (XIX-A) bzw. (XIX-B), 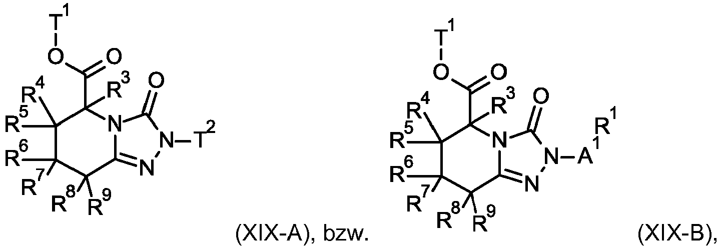
in welcher A¹, R¹,R³, R⁴, R⁵, R⁶, R⁷, R⁸, R⁹ T¹ und T² jeweils die oben genannten Bedeutungen haben,
cyclisiert, und für die Verbindungen der Formel (XIX-A) anschließend die T²-Schutzgruppe in einem inerten Lösungsmittel in Gegenwart einer geeigneten Säure in entfernt,
und die resultierenden Verbindungen der Formel (II) gegebenenfalls mit den entsprechenden (i) Lösungsmitteln und/oder (ii) Säuren oder Basen in ihre Solvate, Salze und/oder Solvate der Salze überführt.

Die Verbindungen der Formel (XIX-B) entsprechen der Verbindungen der Formel (IV), wenn in Formel (IV) X für #²-CR⁶R⁷-CR⁸R⁹-** steht.

Die Verbindungen der Formel (VII) entsprechen der Verbindungen der Formel (XIX), wenn in Formel (VII) X für #²-CR⁶R⁷-CR⁸R⁹-** steht.

Die Verbindungen der Formel (II), in denen X für -CR⁶R⁷- steht, können hergestellt werden, indem eine Verbindung der Formel (XX), 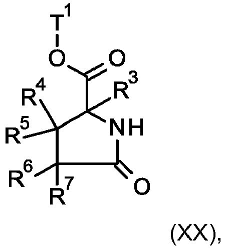
in welcher T¹ für (C₁-C₄)-Alkyl oder Benzyl steht,
und R³, R⁴, R⁵, R⁶ und R⁷ jeweils die oben genannten Bedeutungen haben,
in einem geeigneten Lösungsmittel zunächst in Anwesenheit von Trimethyloxonium-tetrafluoroborat umsetzt und anschließend mit einer Verbindung der Formel (XXI), 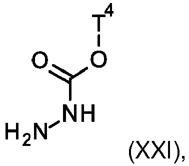 in welcher
   - T⁴: für Methyl, Ethyl, tert-Butyl oder Benzyl steht,
   zu einer Verbindung der Formel (XXII) 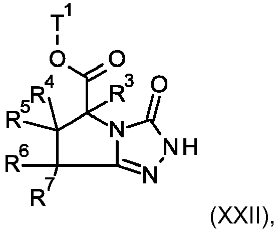
In welcher R³, R⁴, R⁵, R⁶, R⁷ und T¹ jeweils die oben genannten Bedeutungen haben,
umsetzt.

Die Verbindungen der Formeln (XI), (XII), (XVII-A), (XVII-B), (XX) und (XXI) sind kommerziell erhältlich, literaturbekannt oder können in Analogie zu literaturbekannten Verfahren hergestellt werden.

Die beschriebenen Verfahren werden durch die nachfolgenden Schemata (Schema 1-3) beispielhaft verdeutlicht: 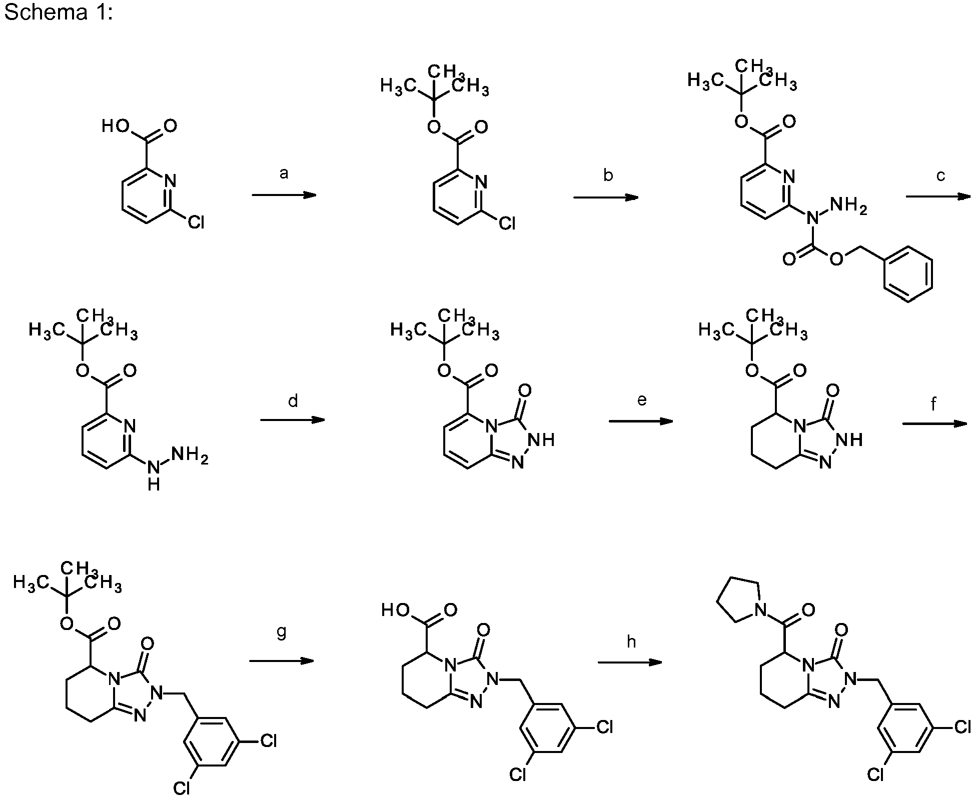 a) tert-Butanol, Pyridin, p-Toluolsulfonylchlorid, RT; b) Benzylcarbazat, Cäsiumcarbonat, 1,1'-Bis(diphenylphosphino)ferrocen, Bis(dibenzylidenaceton)palladium(0) in Toluol 80°C; c) Pd/C 5%, 1 bar H₂ in Toluol / Methanol, RT; d) Carbonyldiimidazol, THF, RT; e) Pd/C 5%, 34.5 bar H₂ in Toluol / Methanol, RT; f) Cäsiumcarbonat, 1-(Brommethyl)-3,5-dichlorbenzol in Acetonitril, RT; g) HCl in 1,4-Dioxan, RT; h) HATU, Triethylamin, Pyrrolidin in THF, RT. 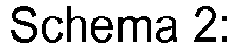 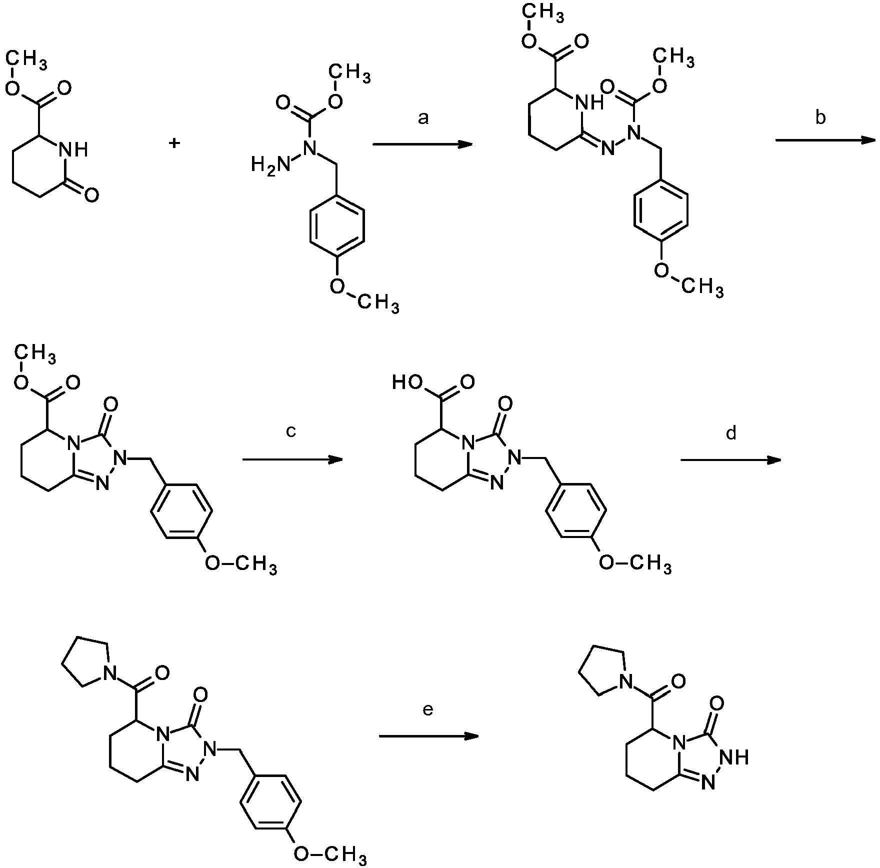 a)1.Methyl-(5RS)-6-oxopiperidin-2-carboxylat,Trimethyloxoniumtetrafluoroborat, Dichlormethan, RT; 2. Methyl-1-(4-methoxybenzyl)hydrazincarboxylat, Dichlormethan, RT; b) DMF, 150 °C; c) ) Lithiumhydroxid, THF / Wasser, RT; d) HATU, Triethylamin, Pyrrolidin in DMF, RT; e) Trifluoressigsäure, 150 °C. 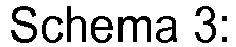 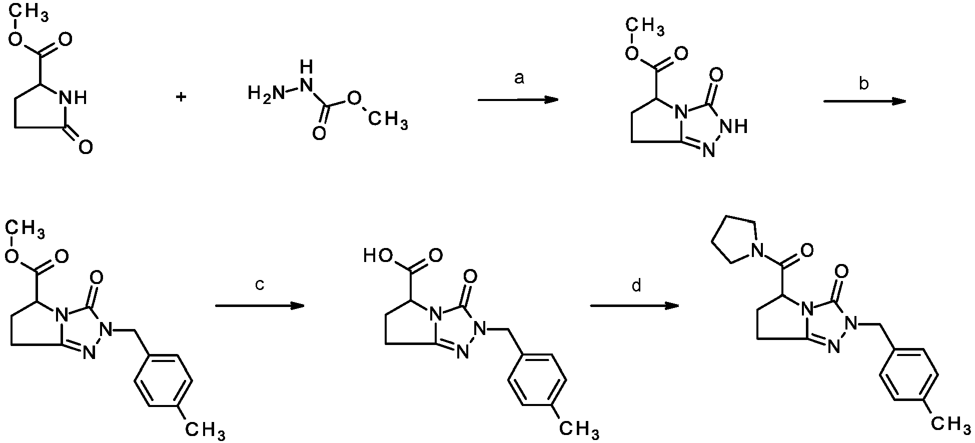 a) 1. Trimethyloxoniumtetrafluoroborat und Methyl-5-oxo-L-prolinat in Dichlormethan, RT; 2. Hydrazinoameisensaeuremethylester, RT 3. DMF, 170 °C; b) Cäsiumcarbonat, 1-(Brommethyl)-4-methylbenzol in Acetonitril, RT; c) Lithiumhydroxid in Wasser / THF, RT; d) HATU, Diisopropylethylamin, Pyrrolidin in DMF / Dichlormethan, RT.

Der Verfahrensschritt (XI) + (XII) → (XIII) erfolgt in einem unter den Reaktionsbedingungen inerten Lösungmittel. Geeignete Lösungsmittel sind beispielsweise Ether wie 1,4-Dioxan, Tetrahydrofuran, 2-Methyltetrahydrofuran, Diethylether, Di-*n*-butylether, Glykoldimethylether oder Diethylenglykoldimethylether, Alkohole wie tert.-Butanol oder Amylalkohole oder anderen Lösungsmitteln wie Dimethylformamid (DMF), Dimethylsulfoxid (DMSO), Dimethylacetamid (DMA), Toluol oder Acetonitril. Ebenso ist es möglich, Gemische der genannten Lösungsmittel einzusetzen. Bevorzugt sind *tert*.-Butanol, 1,4-Dioxan und Toluol.

Der Verfahrensschritt (XI) + (XII) → (XIII) erfolgt in Gegenwart eines geeigneten Palladiumkatalysators. Als Palladium-Katalysator ist beispielsweise Palladium auf Aktivkohle, Palladium(II)-acetat, Bis(dibenzylidenaceton)palladium(0), Tetrakis-(triphenylphosphin)-palladium(0), Bis-(triphenylphosphin)-palladium(II)-chlorid, Bis-(acetonitril)-palladium(II)-chlorid, [1,1'-Bis(diphenylphosphino)ferrocen]dichlorpalladium(II) und entsprechender Dichlormethan-Komplex, gegebenenfalls in Verbindung mit zusätzlichen Phosphanliganden wie beispielsweise 2,2'-Bis(diphenylphosphino)-1,1'-binaphthyl (BINAP), (2-Biphenyl)di-tert.-butylphosphin, Dicyclohexyl[2',4',6'-tris(1-methylethyl)biphenyl-2-yl]phosphan (XPhos), Bis(2-phenylphosphinophenyl)ether (DPEphos) or 4,5-Bis(diphenylphosphino)-9,9-dimethylxanthen (Xantphos) [vgl. z.B. Hassan J. et al., Chem. Rev. 2002, 102, 1359-1469], 2-(Dicyclohexylphosphin)-3,6-dimethoxy-2',4',6'-triisopropyl-1,1 '-biphenyl (BrettPhos), 2-Dicyclohexylphosphino-2',6'-dimethoxybiphenyl (SPhos), 2-Dicyclohexylphosphino-2',6'-diisopropoxybiphenyl (RuPhos), 2-(Di-t-butylphosphino)-3-methoxy-6-methyl-2',4',6'-tri-i-propyl-1,1'-biphenyl (RockPhos) und 2-Di-tert-butylphosphino-2',4',6'-triisopropylbiphenyl (*tert-*ButylXPhos) geeignet. Weiterhin ist es möglich entsprechende Präkatalysatoren wie Chlor-[2-(dicyclohexylphosphin)-3,6-dimethoxy-2',4',6'-triisopropyl-1,1'-biphenyl][2-(2-aminoethyl)-phenyl]palladium(II) (BrettPhos Präkatalysator) [vgl. z.B. S. L. Buchwald et al., Chem. Sci. 2013, 4, 916] gegebenenfalls in Verbindung mit zusätzlichen Phosphanliganden wie 2-(Dicyclohexylphosphin)-3,6-dimethoxy-2',4',6'-triisopropyl-1,1 '-biphenyl (BrettPhos) einzusetzen. Bevorzugt sind Bis(dibenzylidenaceton)palladium(0) in Kombination mit 4,5-Bis-(diphenylphosphino)-9,9-dimethylxanthen (Xantphos) sowie Chlor-[2-(dicyclohexylphosphin)-3,6-dimethoxy-2',4',6'-triisopropyl-1,1'-biphenyl][2-(2-aminoethyl)-phenyl]palladium(II) (BrettPhos Präkatalysator).

Die Umsetzung (XI) + (XII) → (XIII) erfolgt in Gegenwart einer geeigneten Base. Geeignete Basen für diese Umsetzung sind die üblichen anorganischen oder organischen Basen. Hierzu gehören bevorzugt Alkali- oder Erdalkalicarbonate wie Lithium-, Natrium-, Kalium-, Calcium- oder Cäsiumcarbonat, Alkali- oder Erdalkalihydroxide wie Natrium-, Kalium- oder Bariumhydroxid, Alkali- oder Erdalkaliphosphate wie Kaliumphosphat, Alkali-Alkoholate wie Natrium- oder Kalium-*tert*.-butylat und Natriummethanolat, Alkaliphenolate wie Natriumphenolat, Amide wie Natriumamid, Lithium-, Natrium- oder Kalium-bis(trimethylsilyl)-amid oder Lithiumdiisopropylamid oder organische Amine wie 1,5-Diazabicyclo[4.3.0]non-5-en (DBN), 1,8-Diazabicyclo[5.4.0]undec-7-en (DBU). Bevorzugt werden Cäsiumcarbonat, Kaliumcarbonat, Natrium- oder Kalium-tert.-butylat oder Lithiumbis(trimethylsilyl)amid verwendet.

Der Verfahrensschritt (XI) + (XII) → (XIII) wird im Allgemeinen in einem Temperaturbereich von 0°C bis +200°C, bevorzugt bei +10°C bis +150°C durchgeführt. Die Umsetzungen können auch in geschlossenen Gefäßen (Mikrowellenröhren) in der Mikrowelle durchgeführt werden. Die Umsetzung kann bei normalem, erhöhtem oder bei erniedrigtem Druck erfolgen (z.B. von 0.5 bis 5 bar). Im Allgemeinen arbeitet man bei Normaldruck oder in geschlossenen Gefäßen (Mikrowellenröhren) unter- oder oberhalb des Siedepunktes des verwendeten Lösungsmittels. Bevorzugt sind Reaktionen in geschlossenen Gefäßen (Mikrowellenröhrchen), bei Temperaturen oberhalb des Siedepunktes des Lösungsmittels und unter erhöhtem Druck mit oder ohne Verwendung einer Mikrowelle.

Die Abspaltung der Schutzgruppe im Reaktionsschritt (XIII) → (XIV) erfolgt hierbei nach üblichen, aus der Schutzgruppenchemie bekannten Methoden, vorzugsweise durch Umsetzung mit Säure wie beispielsweise Trifluoressigsäure, Hydrogenolyse in Gegenwart eines Palladiumkatalysators, wie beispielsweise Palladium auf Aktivkohle, in einem inerten Lösungsmittel, wie beispielsweise Ethanol oder Essigsäureethylester [siehe auch z.B. T.W. Greene und P.G.M. Wuts, Protective Groups in Organic Synthesis, Wiley, New York, 1999].

Inerte Lösungsmittel für den Verfahrensschritt (XIV) → (XV) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Essigsäureethylester, Acetonitril, Pyridin, Dimethylsulfoxid, *N*,*N*-Dimethylformamid, *N,N-*Dimethylacetamid, *N,N'*-Dimethylpropylenharnstoff (DMPU) oder *N*-Methylpyrrolidon (NMP). Ebenso ist es möglich, Gemische der genannten Lösungsmittel zu verwenden. Bevorzugt sind Dichlormethan, Tetrahydrofuran, Dimethylformamid oder Gemische dieser Lösungsmittel.

Als Phosgenderivat für den Verfahrensschritt (XIV) → (XV) eignen sich beispielsweise *N*,*N*'-Carbonyldiimidazol (CDI), Trichlormethylchlorkohlensäureester (Diphosgen), Bis(trichlormethyl)carbonat (Triphosgen) oder Chlorameisensäurearylester.Bevorzugt wird *N,N'*-Carbonyldiimidazol (CDI) verwendet.

Der Verfahrensschritt (XIV) → (XV) wird im Allgemeinen in einem Temperaturbereich von - 20°C bis +100°C, bevorzugt bei 0°C bis +60°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für den Verfahrensschritt (XV) → (II) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Aceton, Essigsäureethylester, Acetonitril, *N*,*N*-Dimethylformamid, *N*,*N*-Dimethylacetamid, *N,N'*-Dimethylpropylenharnstoff (DMPU) oder *N*-Methylpyrrolidon (NMP). Ebenso ist es möglich, Gemische der genannten Lösungsmittel zu verwenden. Bevorzugt sind Dichlormethan, Tetrahydrofuran, Dimethylformamid oder Gemische dieser Lösungsmittel.

Der Verfahrensschritt (XV) → (II) wird im Allgemeinen in einem Temperaturbereich von -20°C bis +100°C, bevorzugt bei 0°C bis +60°C durchgeführt. Die Umsetzung kann bei normalem oder erhöhtem Wasserstoff-Druck erfolgen (z.B. von 1.0 bis 100 bar). Im Allgemeinen arbeitet man bei erhöhtem Wasserstoff-Druck.

Inerte Lösungsmittel für den Verfahrensschritt (XVI) + (XVII-A) → (XVIII-A) bzw. (XVI) + (XVII-B) → (XVIII-B) bzw. (XXI + (XXI) → (XXII) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie *N*,*N*-Dimethylformamid, *N,N-*Dimethylacetamid, *N,N'*-Dimethyl-propylenharnstoff (DMPU) oder *N*-Methylpyrrolidon (NMP). Ebenso ist es möglich, Gemische der genannten Lösungsmittel zu verwenden. Bevorzugt ist Dichlormethan.

Der Verfahrensschritt (XVI) + (XVII-A) → (XVIII-A) bzw. (XVI) + (XVII-B) → (XVIII-B) bzw. (XXI + (XXI) -> (XXII) wird im Allgemeinen in einem Temperaturbereich von -20°C bis +100°C, bevorzugt bei 0°C bis +60°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für den Verfahrensschritt (XVI) + (XVII-A) → (XVIII-A) bzw. (XVI) + (XVII-B) → (XVIII-B) bzw. (XXI + (XXI) → (XXII) sind beispielsweise Ether wie Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Trichlormethan, Tetrachlormethan, 1,2-Dichlorethan, Trichlorethylen oder Chlorbenzol, oder andere Lösungsmittel wie Aceton, Essigsäureethylester, Acetonitril, Pyridin, Dimethylsulfoxid, *N*,*N*-Dimethylformamid, *N*,*N*-Dimethylacetamid, *N,N'-*Dimethylpropylenharnstoff (DMPU) oder *N*-Methylpyrrolidon (NMP). Ebenso ist es möglich, Gemische der genannten Lösungsmittel zu verwenden. Bevorzugt ist *N*,*N*-Dimethylformamid.

Der Verfahrensschritt (XVI) + (XVII-A) → (XVIII-A) bzw. (XVI) + (XVII-B) → (XVIII-B) wird im Allgemeinen in einem Temperaturbereich von 20°C bis +100°C, bevorzugt bei 0°C bis +60°C 20°C bis +250°C, bevorzugt bei 100°C bis +200°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für den Verfahrensschritt (XVIII-A) → (XIX-A) bzw. (XVIII-B) → (XIX-B) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Essigsäureethylester, Acetonitril, Pyridin, Dimethylsulfoxid, *N*,*N*-Dimethylformamid, *N,N*-Dimethylacetamid, *N*,*N*'-Dimethylpropylenharnstoff (DMPU) oder *N*-Methylpyrrolidon (NMP). Ebenso ist es möglich, Gemische der genannten Lösungsmittel zu verwenden. Bevorzugt sind Dichlormethan und Acetonitril.

Der Verfahrensschritt (XVIII-A) → (XIX-A) bzw. (XVIII-B) → (XIX-B) wird im Allgemeinen in einem Temperaturbereich von 20°C bis +250°C, bevorzugt bei 100°C bis +200°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

In den Ausgangsverbindungen gemäß Formel (II) und (VII) gegebenenfalls vorhandene Hydroxy-, Amino und/oder Amido-Gruppen können, falls zweckmäßig oder erforderlich, auch in temporärer geschützter Form eingesetzt und dann am Ende der jeweiligen Reaktionssequenz wieder freigesetzt werden [zur Eignung, Enführung und Entfernung solcher Schutzgruppen siehe z.B. T.W.Greene und P.G.M. Wuts, Protective Groups in Organic Synthesis, Wiley, New York, 1999).

Weitere erfindungsgemäße Verbindungen können gegebenenfalls auch hergestellt werden durch Umwandlungen von funktionellen Gruppen einzelner Substituenten, insbesondere den unter R³, R⁴, R⁵, R⁶, R⁷, R⁸, R⁹ aufgeführten, ausgehend von den nach obigen Verfahren erhaltenen Verbindungen der Formel (I). Diese Umwandlungen werden nach üblichen, dem Fachmann bekannten Methoden durchgeführt und umfassen beispielsweise Reaktionen wie nukleophile und elektrophile Substitutionen, Oxidationen, Reduktionen, Hydrierungen, Übergangsmetall-katalysierte Kupplungsreaktionen, Eliminierungen, Alkylierung, Aminierung, Veresterung, Esterspaltung, Veretherung, Etherspaltung, Bildung von Carbonamiden, Dehydratisierungen sowie Einführung und Entfernung temporärer Schutzgruppen.

Detaillierte Vorschriften befinden sich auch im Experimentellen Teil im Abschnitt zur Herstellung der Ausgangsverbindungen und Intermediate.

### Erklärungen zu den Abbildungen:

Abbildung 1: Beispiel 237 im Komplex mit pig PREP.
Abbildung 2: Beispiel 358 im Komplex mit pig PREP.
Abbildung 3: Beispiel 454 im Komplex mit pig PREP.
Abbildung 4: Beispiel 108 im Komplex mit pig PREP.
Abbildung 5: Beispiel 113 im Komplex mit pig PREP.
Abbildung 6: Beispiel 157 im Komplex mit pig PREP.
Abbildung 7: Beispiel 026 im Komplex mit pig PREP.

Die erfindungsgemäßen Verbindungen besitzen wertvolle pharmakologische Eigenschaften und können zur Vorbeugung und Behandlung von Erkrankungen bei Menschen und Tieren verwendet werden.

Die erfindungsgemäßen Verbindungen stellen potente, chemisch stabile Inhibitoren der humanen Prolyl-Endopeptidase (PREP, PE, Prolyl-Oligopeptidase, POP) dar und eignen sich daher zur Behandlung und/oder Prävention von Erkrankungen und pathologischen Prozessen, insbesondere solcher, bei denen im Zuge eines infektiösen oder nicht-infektiösen Entzündungsgeschehens und/oder eines Gewebe- oder Gefäßumbaus die PREP bzw. das PREP-Produkt PGP (Prolin-Glyzin-Prolin) involviert ist.

Dazu zählen im Sinne der vorliegenden Erfindung insbesondere Erkrankungen der Atemwege und der Lunge, wie die chronisch-obstruktive Lungenerkrankung (COPD), die zystische Fibrose (Mukoviszidose, CF), Asthma und die Gruppe der interstitiellen Lungenerkrankungen (ILD), sowie Erkrankungen des Herz-Kreislauf-Systems, wie die Arteriosklerose und Myokarditis.

Zu den Ausprägungen der COPD gehören insbesondere das z.B. durch Zigarettenrauch induzierte Lungenemphysem, die chronische Bronchitis (CB), die pulmonale Hypertonie in der COPD (PH-COPD), Bronchiektasie (BE) und Kombinationen hiervon, insbesondere in akut exazerbierenden Stadien der Erkrankung (AE-COPD).

Zu den Ausprägungen von Asthma gehören asthmatische Erkrankungen unterschiedlicher Schweregrade mit intermittierendem oder persistierendem Verlauf, wie refraktäres Asthma, bronchiales Asthma, allergisches Asthma, intrinsisches Asthma, extrinsisches Asthma und durch Medikamente oder Staub induziertes Asthma.

Zu der Gruppe der interstitiellen Lungenerkrankungen (ILD) gehören die idiopathische pulmonale Fibrose (IPF), die Lungensarkoidose und die akute interstitielle Pneumonie, nichtspezifische interstitielle Pneumonien, lymphoide interstitielle Pneumonien, respiratorische Bronchiolitis mit interstitieller Lungenerkrankung, kryptogene organisierende Pneumonien, desquamative interstitielle Pneumonien und nicht-klassifizierbare idiopathische interstitielle Pneumonien, ferner granulomatöse interstitielle Lungenerkrankungen, interstitielle Lungenerkrankungen bekannter Ursache und andere interstitielle Lungenerkrankungen unbekannter Ursache.

Die erfindungsgemäßen Verbindungen können auch zur Behandlung und/oder Prävention von weiteren Erkrankungen der Atemwege und der Lunge verwendet werden, wie z.B. der pulmonalen arteriellen Hypertonie (PAH) und anderer Formen der pulmonalen Hypertonie (PH), des Bronchiolitis obliterans-Syndroms (BOS), des akuten Atemwegssyndroms (ARDS), der akuten Lungenschädigung (ALI), der alpha-1-Antitrypsin-Defizienz (AATD) und der zystischen Fibrose (CF), von verschiedenen Formen der Bronchitis (chronische Bronchitis, infektiöse Bronchitis, eosinophile Bronchitis), von Bronchiektasie, Pneumonie, Farmerlunge und verwandten Krankheiten, infektiös und nicht-infektiös bedingten Husten- und Erkaltungskrankheiten (chronischer entzündlicher Husten, iatrogener Husten), Nasenschleimhautentzündungen (einschließlich medikamentöse Rhinitis, vasomotorische Rhinitis und Jahreszeit-abhängige, allergische Rhinitis, z.B. Heuschnupfen) und von Polypen.

Die erfindungsgemäßen Verbindungen können darüber hinaus zur Behandlung und/oder Prävention von kardiovaskulären Erkrankungen eingesetzt werden, wie beispielsweise Bluthochdruck (Hypertonie), Herzinsuffizienz, koronare Herzerkrankung, stabile und instabile Angina pectoris, renale Hypertonie, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden des Grades I-III, supraventrikuläre Tachyarrhythmie, Vorhofflimmern, Vorhofflattern, Kammerflimmern, Kammerflattern, ventrikuläre Tachyarrhythmie, Torsade de pointes-Tachykardie, Extrasystolen des Vorhofs und des Ventrikels, AV-junktionale Extrasystolen, Sick-Sinus-Syndrom, Synkopen, AV-Knoten-Reentry-Tachykardie, Wolff-Parkinson-White-Syndrom, akutes Koronarsyndrom (ACS), autoimmune Herzerkrankungen (Perikarditis, Endokarditis, Valvolitis, Aortitis, Kardiomyopathien), Boxerkardiomyopathie, Aneurysmen, Schock wie kardiogener Schock, septischer Schock und anaphylaktischer Schock, ferner zur Behandlung und/oder Prävention von thromboembolischen Erkrankungen und Ischämien, wie myokardiale Ischämie, Myokardinfarkt, Hirnschlag, Herzhypertrophie, transistorische und ischämische Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, periphere Durchblutungsstörungen, Reperfusionsschäden, arterielle und venöse Thrombosen, Mikroalbuminurie, Herzmuskelschwäche, endotheliale Dysfunktion, mikro- und makrovaskuläre Schädigungen (Vaskulitis), sowie zur Verhinderung von Restenosen beispielsweise nach Thrombolyse-Therapien, percutan-transluminalen Angioplastien (PTA), percutan-transluminalen Koronarangioplastien (PTCA), Herztransplantationen und Bypass-Operationen.

Im Sinne der vorliegenden Erfindung umfasst der Begriff Herzinsuffizienz sowohl akute als auch chronische Erscheinungsformen der Herzinsuffizienz wie auch spezifische oder verwandte Krankheitsformen hiervon, wie akute dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen sowie diastolische und systolische Herzinsuffizienz.

Die erfindungsgemäßen Verbindungen eignen sich außerdem zur Behandlung und/oder Prävention von Nierenerkrankungen, insbesondere von Niereninsuffizienz und Nierenversagen. Im Sinne der vorliegenden Erfindung umfassen die Begriffe Niereninsuffizienz und Nierenversagen sowohl akute als auch chronische Erscheinungsformen hiervon wie auch diesen zugrundeliegende oder verwandte Nierenerkrankungen, wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantat-Abstoßung und Immunkomplexinduzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prävention von Folgeerscheinungen einer Niereninsuffizienz, wie beispielsweise Hypertonie, Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Darüber hinaus sind die erfindungsgemäßen Verbindungen zur Behandlung und/oder Prävention von Erkrankungen des Urogenitalsystems geeignet, wie beispielsweise benignes Prostata-Syndrom (BPS), benigne Prostatahyperplasie (BPH), benigne Prostatavergrößerung (BPE), Blasenentleerungsstörungen (BOO), untere Harnwegssyndrome (LUTS), neurogene überaktive Blase (OAB), Inkontinenz wie beispielsweise Misch-, Drang-, Stress- oder Überlauf-Inkontinenz (MUI, UUI, SUI, OUI), Beckenschmerzen sowie erektile Dysfunktion und weibliche sexuelle Dysfunktion.

Die erfindungsgemäßen Verbindungen können auch zur Behandlung von Erkrankungen des weiblichen Reproduktionssystems, wie Uterusmyome, Endometriose, Dysmenorrhöe und vorzeitige Geburtswehen, verwendet werden. Weiterhin eignen sie sich zur Prophylaxe oder Behandlung von Hirsutismus und Hypertrichose.

Zudem besitzen die erfindungsgemäßen Verbindungen anti-inflammatorische Wirkung und können daher als entzündungshemmende Mittel zur Behandlung und/oder Prävention von Sepsis (SIRS), multiplem Organversagen (MODS, MOF), entzündlichen Erkrankungen der Niere, chronischen Darmentzündungen (IBD, Morbus Crohn, Colitis ulcerosa), Pankreatitis, Peritonitis, Cystitis, Urethritis, Prostatitis, Epidimytitis, Oophoritis, Salpingitis, Vulvovaginitis, rheumatoiden Erkrankungen, Arthrose, entzündlichen Erkrankungen des Zentralnervensystems, multipler Sklerose, entzündlichen Hauterkrankungen und entzündlichen Augenerkrankungen eingesetzt werden.

Die erfindungsgemäßen Verbindungen sind ferner zur Behandlung und/oder Prävention von fibrotischen Erkrankungen der inneren Organe, wie beispielsweise der Lunge, des Herzens, der Niere, des Knochenmarks und insbesondere der Leber, sowie von dermatologischen Fibrosen und fibrotischen Erkrankungen des Auges geeignet. Im Sinne der vorliegenden Erfindung umfasst der Begriff fibrotische Erkrankungen insbesondere solche Erkrankungen wie Leberfibrose, Leberzirrhose, Lungenfibrose, Endomyokardfibrose, Nephropathie, Glomerulonephritis, interstitielle Nierenfibrose, fibrotische Schäden in Folge von Diabetes, Knochenmarksfibrose, Peritonealfibrose und ähnliche fibrotische Erkrankungen, Sklerodermie, Morphaea, Keloide, hypertrophe Narbenbildung, Naevi, diabetische Retinopathie, proliferative Vitroretinopathie und Erkrankungen des Bindegewebes (z.B. Sarkoidose). Die erfindungsgemäßen Verbindungen können ebenso verwendet werden zur Förderung der Wundheilung, zur Bekämpfung postoperativer Narbenbildung, z.B. nach Glaukom-Operationen, und zu kosmetischen Zwecken bei alternder oder verhornender Haut.

Auch können die erfindungsgemäßen Verbindungen zur Behandlung und/oder Prävention von Anämien verwendet werden, wie hämolytischen Anämien, insbesondere Hämoglobinopathien wie Sichelzellanämie und Thalassämien, megaloblastären Anämien, Eisenmangel-Anämien, Anämien durch akuten Blutverlust, Verdrängungsanämien und aplastischen Anämien.

Die erfindungsgemäßen Verbindungen sind zudem zur Behandlung von Krebserkrankungen geeignet, wie beispielsweise von Hautkrebs, Hirntumoren, Brustkrebs, Knochenmarktumoren, Leukämien, Liposarcomen, Karzinomen des Magen-Darm-Traktes, der Leber, Bauchspeicheldrüse, Lunge, Niere, Harnleiter, Prostata und des Genitaltraktes sowie von bösartigen Tumoren des lymphoproliferativen Systems, wie z.B. Hodgkin's und Non-Hodgkin's Lymphom.

Darüber hinaus können die erfindungsgemäßen Verbindungen eingesetzt werden zur Behandlung und/oder Prävention von Arteriosklerose, Lipidstoffwechselstörungen und Dyslipidämien (Hypolipoproteinämie, Hypertriglyceridämie, Hyperlipidämie, kombinierte Hyperlipidämien, Hypercholesterolämie, Abetalipoproteinämie, Sitosterolämie), Xanthomatose, Tangier-Krankheit, Fettsucht (Adipositas), Fettleibigkeit (Obesitas), metabolischen Erkrankungen (Metabolisches Syndrom, Hyperglykämie, Insulin-abhängiger Diabetes, nicht-Insulin-abhängiger Diabetes, Gestationsdiabetes, Hyperinsulinämie, Insulinresistenz, Glukose-Intoleranz und diabetische Spätfolgen wie Retinopathie, Nephropathie und Neuropathie), von Erkrankungen des Gastrointestinaltrakts und des Abdomen (Glossitis, Gingivitis, Periodontitis, Oesophagitis, eosinophile Gastroenteritis, Mastocytose, Morbus Crohn, Colitis, Proctitis, Pruritis ani, Diarrhöe, Zöliakie, Hepatitis, Leberfibrose, Leberzirrhose, Pankreatitis und Cholecystitis), von Erkrankungen des Zentralen Nervensystems und von neurodegenerativen Störungen (Schlaganfall, Alzheimer'sche Krankheit, Parkinson'sche Krankheit, Demenz, Epilepsie, Depressionen, Multiple Sklerose), Immunerkrankungen, Schilddrüsenerkrankungen (Hyperthyreose), Hauterkrankungen (Psoriasis, Akne, Ekzeme, Neurodermitis, vielfältige Formen der Dermatitis wie z.B. Dermatitis abacribus, Dermatitis actinica, Dermatitis allergica, Dermatitis ammoniacalis, Dermatitis artefacta, Dermatitis autogenica, Dermatitis atrophicans, Dermatitis calorica, Dermatitis combustionis, Dermatitis congelationis, Dermatitis cosmetica, Dermatitis escharotica, Dermatitis exfoliativa, Dermatitis gangraenose, Dermatitis haemostatica, Dermatitis herpetiformis, Dermatitis lichenoides, Dermatitis linearis, Dermatitis maligna, Dermatitis medimencatosa, Dermatitis palmaris et plantaris, Dermatitis parasitaria, Dermatitis photoallergica, Dermatitis phototoxica, Dermatitis pustularis, Dermatitis seborrhoica, Dermatitis solaris, Dermatitis toxica, Dermatitis ulcerosa, Dermatitis veneata, infektiöse Dermatitis, pyogene Dermatitis und Rosazea-artige Dermatitis, sowie Keratitis, Bullosis, Vasculitis, Cellulitis, Panniculitis, Lupus erythematodes, Erythema, Lymphome, Hautkrebs, Sweet-Syndrom, Weber-Christian-Syndrom, Narbenbildung, Warzenbildung, Frostbeulen), von entzündlichen Augenerkrankungen (Saccoidosis, Blepharitis, Conjunctivitis, Iritis, Uveitis, Chorioiditis, Ophthalmitis), viralen Erkrankungen (durch Influenza-, Adeno- und Coronaviren, wie z.B. HPV, HCMV, HIV, SARS), von Erkrankungen des Skelettknochens und der Gelenke sowie der Skelettmuskel (vielfältige Formen der Arthritis wie z.B. Arthritis alcaptonurica, Arthritis ankylosans, Arthritis dysenterica, Arthritis exsudativa, Arthritis fungosa, Arthritis gonorrhoica, Arthritis mutilans, Arthritis psoriatica, Arthritis purulenta, Arthritis rheumatica, Arthritis serosa, Arthritis syphilitica, Arthritis tuberculosa, Arthritis urica, Arthritis villonodularis pigmentosa, atypische Arthritis, hämophile Arthritis, juvenile chronische Arthritis, rheumatoide Arthritis und metastatische Arthritis, des weiteren das Still-Syndrom, Felty-Syndrom, Sjörgen-Syndrom, Clutton-Syndrom, Poncet-Syndrom, Pott-Syndrom und Reiter-Syndrom, vielfältige Formen der Arthropathien wie z.B. Arthropathie deformans, Arthropathie neuropathica, Arthropathie ovaripriva, Arthropathie psoriatica und Arthropathie tabica, systemische Sklerosen, vielfältige Formen der entzündlichen Myopathien wie z.B. Myopathie epidemica, Myopathie fibrosa, Myopathie myoglobinurica, Myopathie ossificans, Myopathie ossificans neurotica, Myopathie ossificans progressiva multiplex, Myopathie purulenta, Myopathie rheumatica, Myopathie trichinosa, Myopathie tropica und Myopathie typhosa, sowie das Günther-Syndrom und das Münchmeyer-Syndrom), von entzündlichen Arterienveränderungen (vielfältige Formen der Arteritis wie z.B. Endarteritis, Mesarteritis, Periarteritis, Panarteritis, Arteritis rheumatica, Arteritis deformans, Arteritis temporalis, Arteritis cranialis, Arteritis gigantocellularis und Arteritis granulomatosa, sowie das Horton-Syndrom, Churg-Strauss-Syndrom und die Takayasu-Arteritis), des Muckle-Well-Syndroms, der Kikuchi-Krankheit, von Polychondritis, Sklerodermia sowie von weiteren Erkrankungen mit einer entzündlichen oder immunologischen Komponente, wie beispielsweise Katarakt, Kachexie, Osteoporose, Gicht, Inkontinenz, Lepra, Sezary-Syndrom und paraneoplastisches Syndrom, bei Abstossungsreaktionen nach Organtransplantationen und zur Wundheilung und Angiogenese insbesondere bei chronischen Wunden.

Aufgrund ihres biochemischen und pharmakologischen Eigenschaftsprofils eignen sich die erfindungsgemäßen Verbindungen insbesondere zur Behandlung und/oder Prävention von inflammatorischen Lungenerkrankungen, vor allem der chronisch-obstruktiven Lungenerkrankung (COPD), des Lungenemphysems, der chronischen Bronchitis, der Bronchiektasie, der pulmonalen Hypertonie in der COPD (PH-COPD), der akuten Exazerbation in der COPD, der zystischen Fibrose (Mukoviszidose, CF), des Asthmas, sowie der idiopathischen Lungenfibrose (IPF), des Bronchiolitis obliterans-Syndrom (BOS), der Arteriosklerose, der Myokarditis, sowie entzündlichen Haut- und Augenerkrankungen bzw. entzündlichen Erkrankungen der inneren Organe.

Aufgrund ihres biochemischen und pharmakologischen Eigenschaftsprofils eignen sich die erfindungsgemäßen Verbindungen ganz im Besonderen zur Behandlung und/oder Prävention von inflammatorischen Lungenerkrankungen, vor allem der chronisch-obstruktiven Lungenerkrankung (COPD), des Lungenemphysems, der chronischen Bronchitis, der Bronchiektasie, der pulmonalen Hypertonie in der COPD (PH-COPD), der akuten Exazerbation in der COPD, der zystischen Fibrose (Mukoviszidose, CF), des Asthmas, sowie der idiopathischen Lungenfibrose (IPF) und des Bronchiolitis obliterans-Syndroms (BOS).

Die zuvor genannten, gut charakterisierten Krankheiten des Menschen können mit vergleichbarer Ätiologie auch in anderen Säugetieren vorkommen und dort ebenfalls mit den Verbindungen der vorliegenden Erfindung behandelt werden.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Arzneimittel, enthaltend mindestens eine der erfindungsgemäßen Verbindungen, zur Behandlung und/oder Prävention von Erkrankungen, insbesondere der zuvor genannten Erkrankungen.

Die erfindungsgemäßen Verbindungen können allein oder bei Bedarf in Kombination mit einer oder mehreren anderen pharmakologisch wirksamen Substanzen eingesetzt werden, solange diese Kombination nicht zu unerwünschten und inakzeptablen Nebenwirkungen führt. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als hierfür geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil, Tadalafil, Udenafil, Dasantafil, Avanafil, Mirodenafil oder Lodenafil;
- NO- und Häm-unabhängige Aktivatoren der löslichen Guanylatcyclase (sGC), wie insbesondere die in WO 01/19355, WO 01/19776, WO 01/19778, WO 01/19780, WO 02/070462 und WO 02/070510 beschriebenen Verbindungen;
- NO-unabhängige, jedoch Häm-abhängige Stimulatoren der löslichen Guanylatcyclase (sGC), wie insbesondere Riociguat sowie die in WO 00/06568, WO 00/06569, WO 02/42301, WO 03/095451, WO 2011/147809, WO 2012/004258, WO 2012/028647 und WO 2012/059549 beschriebenen Verbindungen;
- Prostacyclin-Analoga und IP-Rezeptor-Agonisten, wie beispielhaft und vorzugsweise Iloprost, Beraprost, Treprostinil, Epoprostenol oder Selexipag;
- Endothelin-Rezeptor-Antagonisten, wie beispielhaft und vorzugsweise Bosentan, Darusentan, Ambrisentan oder Sitaxsentan;
- Verbindungen, die die humane neutrophile Elastase (HNE) inhibieren, wie beispielhaft und vorzugsweise Sivelestat oder DX-890 (Reltran);
- die Signaltransduktionskaskade inhibierende Verbindungen, beispielhaft und vorzugsweise aus der Gruppe der Kinase-Inhibitoren, insbesondere aus der Gruppe der Tyrosinkinase- und/oder Serin/Threoninkinase-Inhibitoren, wie beispielhaft und vorzugsweise Nintedanib, Dasatinib, Nilotinib, Bosutinib, Regorafenib, Sorafenib, Sunitinib, Cediranib, Axitinib, Telatinib, Imatinib, Brivanib, Pazopanib, Vatalanib, Gefitinib, Erlotinib, Lapatinib, Canertinib, Lestaurtinib, Pelitinib, Semaxanib oder Tandutinib;
- Verbindungen, die den Ab- und Umbau der Extrazellulärmatrix inhibieren, beispielhaft und vorzugsweise Inhibitoren der Matrix-Metalloproteasen (MMPs), insbesondere Inhibitoren von Stromelysin, Kollagenasen, Gelatinasen und Aggrecanasen (hierbei vor allem von MMP-1, MMP-3, MMP-8, MMP-9, MMP-10, MMP-11 und MMP-13) sowie der Metallo-Elastase (MMP-12);
- Verbindungen, die die Bindung von Serotonin an dessen Rezeptor blockieren, beispielhaft und vorzugsweise Antagonisten des 5-HT_{2B}-Rezeptors wie PRX-08066;
- Antagonisten von Wachstumsfaktoren, Zytokinen und Chemokinen, beispielhaft und vorzugsweise Antagonisten von TGF-β, CTGF, IL-1, IL-4, IL-5, IL-6, IL-8, IL-13 und Integrinen;
- die Rho-Kinase inhibierende Verbindungen, wie beispielhaft und vorzugsweise Fasudil, Y-27632, SLx-2119, BF-66851, BF-66852, BF-66853, KI-23095 oder BA-1049;
- Verbindungen, die die lösliche Epoxidhydrolase (sEH) inhibieren, wie beispielsweise N,N'-Dicyclohexylharnstoff, 12-(3-Adamantan-1-yl-ureido)-dodecansäure oder 1-Adamantan-1-yl-3-{5-[2-(2-ethoxyethoxy)ethoxy]pentyl}-harnstoff;
- den Energiestoffwechsel des Herzens beeinflussende Verbindungen, wie beispielhaft und vorzugsweise Etomoxir, Dichloracetat, Ranolazin oder Trimetazidin;
- anti-obstruktiv wirkende Mittel, wie sie z.B. zur Therapie der chronisch-obstruktiven Lungenerkrankung (COPD) oder eines Asthma bronchiale eingesetzt werden, beispielhaft und vorzugsweise aus der Gruppe der inhalativ oder systemisch angewendeten β-adrenergen Rezeptor-Agonisten (β-Mimetika) und der inhalativ angewendeten anti-muscarinergen Substanzen;
- entzündungshemmende, immunmodulierende, immunsuppressive und/oder zytotoxische Mittel, beispielhaft und vorzugsweise aus der Gruppe der systemisch oder inhalativ angewendeten Corticosteroide sowie Acetylcystein, Montelukast, Azathioprin, Cyclophosphamid, Hydroxycarbamid, Azithromycin, Pirfenidon oder Etanercept;
- antifibrotisch wirkende Mittel, wie beispielhaft und vorzugsweise Adenosin-A2b-Rezeptor-Antagonisten, Sphingosin-1-phosphat-Rezeptor 3 (S1P3)-Antagonisten, Autotaxin-Inhibitoren, Lysophosphatidsäure-Rezeptor 1 (LPA-1)- und Lysophosphatidsäure-Rezeptor 2 (LPA-2)-Antagonisten, Lysyloxidase (LOX)-Inhibitoren, Lysyloxidase-like-2-lnhibitoren, CTGF-Inhibitoren, IL-13-Antagonisten, αᵥβ₆-Integrin-Antagonisten, TGF-β-Antagonisten, Inhibitoren des Wnt-Signalwegs oder CCR2-Antagonisten;
- antithrombotisch wirkende Mittel, beispielhaft und vorzugsweise aus der Gruppe der Thrombozytenaggregationshemmer, der Antikoagulantien und der profibrinolytischen Substanzen;
- den Blutdruck senkende Wirkstoffe, beispielhaft und vorzugsweise aus der Gruppe der Calcium-Antagonisten, Angiotensin All-Antagonisten, ACE-Hemmer, Vasopeptidase-Inhibitoren, Endothelin-Antagonisten, Renin-Inhibitoren, α-Rezeptoren-Blocker, β-Rezeptoren-Blocker, Mineralocorticoid-Rezeptor-Antagonisten sowie der Diuretika;
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-α-, PPAR-γ- und/oder PPAR-δ-Agonisten, Cholesterin-Absorptionshemmer, Lipase-Inhibitoren, polymeren Gallensäureadsorber, Gallensäure-Reabsorptionshemmer und Lipoprotein(a)-Antagonisten; und/oder
- Chemotherapeutika, wie sie z.B. zur Therapie von Neubildungen (Neoplasien) der Lunge oder anderer Organe eingesetzt werden.

Bei einer bevorzugten Ausführungsform der Erfindung werden die erfindungsgemäßen Verbindungen in Kombination mit einem β-adrenergen Rezeptor-Agonisten, wie beispielhaft und vorzugsweise Albuterol, Isoproterenol, Metaproterenol, Terbutalin, Fenoterol, Formoterol, Reproterol, Salbutamol oder Salmeterol, verabreicht.

Bei einer bevorzugten Ausführungsform der Erfindung werden die erfindungsgemäßen Verbindungen in Kombination mit einer anti-muscarinergen Substanz, wie beispielhaft und vorzugsweise Ipratropiumbromid, Tiotropiumbromid oder Oxitropiumbromid, verabreicht.

Bei einer bevorzugten Ausführungsform der Erfindung werden die erfindungsgemäßen Verbindungen in Kombination mit einem Corticosteroid, wie beispielhaft und vorzugsweise Prednison, Prednisolon, Methylprednisolon, Triamcinolon, Dexamethason, Beclomethason, Betamethason, Flunisolid, Budesonid oder Fluticason, verabreicht.

Unter antithrombotisch wirkenden Mittel werden vorzugsweise Verbindungen aus der Gruppe der Thrombozytenaggregationshemmer, der Antikoagulantien und der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Dabigatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, Apixaban, Fidexaban, Razaxaban, Fondaparinux, Idraparinux, DU-176b, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin All-Antagonisten, ACE-Hemmer, Endothelin-Antagonisten, Renin-Inhibitoren, α-Rezeptoren-Blocker, β-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem α₁-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem β-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin All-Antagonisten, wie beispielhaft und vorzugsweise Losartan, Candesartan, Valsartan, Telmisartan, Embursartan, Irbesartan, Olmesartan, Eprosartan oder Azilsartan oder einem dualen Angiotensin All-Antagonisten/NEP-Inhibitor, wie beispielsweise und vorzugsweise LCZ696 (Valsartan/Sacubitril), verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton, Eplerenon oder Finerenon, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-α-, PPAR-γ- und/oder PPAR-δ-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-γ-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-δ-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Besonders bevorzugt sind Kombinationen der erfindungsgemäßen Verbindungen mit einem oder mehreren weiteren Wirkstoffen ausgewählt aus der Gruppe bestehend aus PDE 5-Inhibitoren, sGC-Aktivatoren, sGC-Stimulatoren, Prostacyclin-Analoga, IP-Rezeptor-Agonisten, Endothelin-Antagonisten, die Signaltransduktionskaskade inhibierenden Verbindungen und Pirfenidon.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat oder Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. inhalativ, intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer, Dosieraerosole), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale und die parenterale Applikation, insbesondere die orale, die intravenöse und die intrapulmonale (inhalative) Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0,001 bis 1 mg/kg, vorzugsweise etwa 0,01 bis 0,5 mg/kg Körpergewicht zur Erzielung wirksamer Ergebnisse zu verabreichen. Bei oraler Applikation beträgt die Dosierung etwa 0,01 bis 100 mg/kg, vorzugsweise etwa 0,01 bis 20 mg/kg und ganz besonders bevorzugt 0,1 bis 10 mg/kg Körpergewicht. Bei intrapulmonaler Applikation beträgt die Menge im Allgemeinen etwa 0,1 bis 50 mg je Inhalation.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Weiterer Gegenstand der vorliegenden Erfindung sind Arzneimittel pharmazeutische Zusammensetzungen, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, vaginal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan, intravitreal oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Augentropfen, Augensalben, Augenbäder, okulare Inserte, Ohrentropfen, -sprays, -pulver, -spülungen, - tampons, , Vaginalkapseln, wässrige Suspensionen (Lotionen, Schüttelmixturen), lipophile Suspensionen, Emulsionen, Mikroemulsionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit pharmazeutisch geeigneten Hilfsstoffen geschehen. Zu diesen Hilfsstoffen zählen u.a.
- Füll- und Trägerstoffe (beispielsweise Cellulose, mikrokristalline Cellulose wie z.B. Avicel^{®}, Laktose, Mannitol, Stärke, Calciumphosphate wie z.B. Di-Cafos^{®}),
- Salbengrundlagen (beispielsweise Vaselin, Paraffine, Triglyceride, Wachse, Wollwachs, Wollwachsalkohole, Lanolin, hydrophile Salbe, Polyethylenglycole),
- Suppositoriengrundlagen (zum Beispiel Polyethylenglycole, Kakaobutter, Hartfett),
- Lösungsmittel (z.B. Wasser, Ethanol, Isopropanol, Glycerol, Propylenglycol, mittelkettige Triglyceride fette Öle, flüssige Polyethylenglycole, Paraffine),
- Tenside, Emulgatoren, Dispergier- oder Netzmittel (beispielsweise Natriumdodecylsulfat, Lecithin, Phospholipide, Fettalkohole wie z.B. Lanette^{®}, Sorbitanfettsäureester wie z.B. Span^{®}, Polyoxy-ethylen-Sorbitanfettsäureester wie z.B. Tween^{®}, Polyoxyethylen-Fettsäureglyceride wie z.B. Cremophor^{®}, Polyoxethlyen-Fettsäureester, Polyoxethlyen-Fettalkoholether, Glycerolfettsäureester, Poloxamere wie z.B. Pluronic^{®}),
- Puffersubstanzen sowie Säuren und Basen (beispielsweise Phosphate, Carbonate, Citronensäure, Essigsäure, Salzsäure, Natronlauge, Ammoniumcarbonat, Trometamol, Triethanolamin),
- Isotonisierungsmittel (beispielsweise Glucose, Natriumchlorid),
- Adsorptionsmittel (beispielsweise hochdisperse Siliciumdioxide),
- Viskositätserhöhende Mittel, Gelbildner, Verdickungs- bzw. Bindemittel (beispielsweise Polyvinylpyrrolidon, Methylcellulose, Hydroxypropylmethylcellulose, Hydroxypropylcellulose, Carboxymethylcellulose-Natrium, Stärke, Carbomere, Polyacrylsäuren wie z.B. Carbopol^{®}, Alginate, Gelatine),
- Sprengmittel (beispielsweise modifizierte Stärke, Carboxymethylcellulose-Natrium, Natriumstärkeglycolat wie z.B. Explotab^{®}, quervernetztes Polyvinylpyrrolidon, Croscarmellose-Natrium wie z.B. AcDiSol^{®}),
- Fließregulier-, Schmier-, Gleit- und Formtrennmittel (beispielsweise Magnesiumstearat, Stearinsäure, Talkum, hochdisperse Siliciumdioxide wie z.B. Aerosil^{®}),
- Überzugsmittel (beispielsweise Zucker, Schellac) sowie Filmbildemittel für sich schnell oder modifiziert auflösende Filme bzw. Diffusionsmembranen (beispielsweise Polyvinylpyrrolidone wie z.B. Kollidon^{®}, Polyvinylalkohol, Hydroxypropylmethylcellulose, Hydroxypropylcellulose, Ethylcellulose, Hydroxypropylmethylcellulosephthalat, Celluloseacetat, Celluloseacetatphthtalat, Polyacrylate, Polymethacrylate wie z.B. Eudragit^{®}),
- Kapselmaterialien (z.B. Gelatine, Hydroxypropylmethylcellulose),
- Synthetische Polymere (beispielsweise Polylactide, Polyglycolide, Polyacrylate, Polymethacrylate wie z.B Eudragit^{®}, Polyvinylpyrrolidone wie z.B. Kollidon^{®}, Polyvinylalcohole, Polyvinylacetate, Polyethylenoxide, Polyethylenglycole und deren Copolymere und Blockcopolymere),
- Weichmacher (beispielsweise Polyethylenglycole, Propylenglykol, Glycerol, Triacetin, Triacetylcitrat, Dibutylphthalat),
- Penetrationsenhancer,
- Stabilisatoren (z.B. Antioxidantien wie beispielsweise Ascorbinsäure, Ascorbylpalmitat, Natriumascorbat, Butylhydroxyanisol, Butylhydroxytoluol, Propylgallat),
- Konservierungsmittel (beispielsweise Parabene, Sorbinsäure, Thiomersal, Benzalkoniumchlorid, Chlorhexidinacetat, Natriumbenzoat),
- Farbstoffe (z.B. anorganische Pigmente wie beispielsweise Eisenoxide, Titandioxid),
- Aromen, Süßungsmittel, Geschmacks- und / oder Geruchskorrigentien.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.1 bis 6 mg/kg zur Erzielung wirksamer Ergebnisse zu verabreichen. Bei oraler Applikation beträgt die Dosierung etwa 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

Nur Beispiele, die unter die Ansprüche fallen, sind Teil der Erfindung. Beispiele, die nicht unter die Ansprüche fallen, so wie beispielsweise Beispiele 40 und 41, sind nur als Referenz offenbart und nicht Teil der Erfindung.

### Abkürzungen und Akronyme:

| | |
|---|---|
| Ac | Acetyl |
| aq. | wässrig, wässrige Lösung |
| boc | tert.-Butoxycarbonyl |
| br.d | breites Dublett (NMR) |
| br.m | breites Multiplett (NMR) |
| br.s | breites Singulett (NMR) |
| br.t | breites Triplett (NMR) |
| c | Konzentration |
| cat. | katalytisch |
| d | Dublett (NMR) |
| de | Diastereomerenüberschuss |
| DC | Dünnschichtchromatographie |
| DCI | direkte chemische Ionisation (bei MS) |
| dd | Dublett von Dubletten (NMR) |
| ddd | Dublett von Dubletten von Dubletten (NMR) |
| dest. | destilliert |
| DIAD | Diisopropylzaodicarboxylat |
| DIEA | *N*,*N*-Diisopropylethylamin |
| DMAP | 4-*N,N*-Dimethylaminopyridin |
| DMF | Dimethylformamid |
| DMSO | Dimethylsulfoxid |
| DSC | Differential Scanning Thermography |
| dt | Dublett von Tripletten (NMR) |
| d. Th. | der Theorie (bei Ausbeute) |
| EDC | *N'*-(3-Dimethylaminopropyl)-*N*-ethylcarbodiimid-Hydrochlorid |
| ee | Enantiomerenüberschuss |
| ent | enantiomerenrein, Enantiomer |
| eq. | Äquivalent(e) |
| ESI | Elektrospray-Ionisation (bei MS) |
| Et | Ethyl |
| GC-MS | Gaschromatographie-gekoppelte Massenspektrometrie |
| h | Stunde(n) |
| HATU | *O*-(7-Azabenzotriazol-1-yl)-*N,N,N',N'*-tetramethyluronium-hexafluorophosphat |
| HBTU | *O*-(Benzotriazol-1-yl)-*N,N,N',N*'-tetramethyluronium-hexafluorphosphat |
| HOBt | 1 -Hydroxy-1*H*-benzotriazol-Hydrat |
| HPLC | Hochdruck-, Hochleistungsflüssigchromatographie |
| ID | Innendurchmesser |
| iPr | iso-Propyl |
| J | Kopplungskonstante (NMR) |
| konz. | Konzentriert |
| LC-MS | Flüssigchromatographie-gekoppelte Massenspektrometrie |
| m | Multiplett (NMR) |
| Me | Methyl |
| min | Minute(n) |
| MPLC | Mitteldruckflüssigchromatographie |
| MS | Massenspektrometrie |
| MTBE | Methyl-*tert*.-butylether |
| NMR | Kernresonanzspektrometrie |
| Pd/C | Palladium auf Aktivkohle |
| Ph | Phenyl |
| PyBOP | Benzotriazol-1-yloxy-tris(pyrrolidino)phosphonium-hexafluorophosphat |
| quant. | quantitativ (bei Ausbeute) |
| rac | racemisch, Racemat |
| RT | Raumtemperatur |
| Rₜ | Retentionszeit (bei HPLC) |
| Schmp. | Schmelzpunkt |
| SFC | Überkritische, superkritische Flüssigkeitschromatographie |
| t | Triplett (NMR) |
| tBu | *tert*.-Butyl |
| tert. | Tertiär |
| TFA | Trifluoressigsäure |
| TFAA | Trifluoressigsäureanhydrid |
| THF | Tetrahydrofuran |
| TPPO | Triphenylphosphinoxid |
| UV | Ultraviolett-Spektrometrie |
| vgl. | vergleiche |
| v/v | Volumen zu Volumen-Verhältnis (einer Lösung) |
| | |
| | |

### HPLC-, GC-MS und LC-MS-Methoden

### Methode 1:

Instrument: Agilent MS Quad 6150;HPLC: Agilent 1290; Säule: Waters Acquity UPLC HSS T3 1.8 µ 50 x 2.1 mm; Eluent A: 1 l Wasser + 0.25 ml 99%ige Ameisensäure , Eluent B: 1 l Acetonitril + 0.25 ml 99%ige Ameisensäure; Gradient: 0.0 min 90% A ----> 0.3 min 90% A ----> 1.7 min 5% A → 3.0 min 5% A Ofen: 50°C; Fluss: 1,20 ml/min; UV-Detektion: 205 - 305 nm.

### Methode 2:

Instrument: Thermo DFS, Trace GC Ultra; Säule: Restek RTX-35, 15 m x 200 µm x 0.33 µm; konstanter Fluss mit Helium: 1.20 ml/min; Ofen: 60°C; Inlet: 220°C; Gradient: 60°C, 30°C/min ----> 300°C (3.33 min halten).

### Methode 3:

Gerätetyp MS: Thermo Scientific FT-MS; Gerätetyp UHPLC+: Thermo Scientific UltiMate 3000; Säule: Waters, HSST3, 2.1 x 75 mm, C18 1.8 µm; Eluent A: 1 l Wasser + 0.01% Ameisensäure; Eluent B: 1 l Acetonitril + 0.01% Ameisensäure; Gradient: 0.0 min 10% B - 2.5 min 95% B → 3.5 min 95% B; Ofen: 50°C; Fluss: 0.90 ml/min; UV-Detektion: 210 nm/ Optimum Integration Path 210-300 nm

### Methode 4:

Instrument: Waters ACQUITY SQD UPLC System; Säule: Waters Acquity UPLC HSS T3 1.8 µ 50 x 1 mm; Eluent A: 1 l Wasser + 0.25 ml 99%ige Ameisensäure , Eluent B: 1 l Acetonitril + 0.25 ml 99%ige Ameisensäure; Gradient: 0.0 min 90% A ----> 1.2 min 5% A ----> 2.0 min 5% A Ofen: 50°C; Fluss: 0.40 ml/min; UV-Detektion: 208 - 400 nm.

### Methode 5:

Instrument: HP 1100 Series mit LC/MSD SL; Säule: CS MultoKrom 100-3 C18 60x4.6 mm; Eluent A: 1 l Wasser + 10 ml 99%ige Ameisensäure, Eluent B: 1 l + 10 ml 99%ige Ameisensäure; Gradient: 0.0 min 80% A -> 8.0 min 10% A -> 10.0 min 10% A; Fluss: 1.00 ml/min; DAD-Detektion: 120 - 800 nm.

### Methode 6:

Instrument MS: Waters (Micromass) Quattro Micro; Instrument Waters UPLC Acquity; Säule : Waters BEH C18 1.7 µ 50 x 2.1 mm; Eluent A: 1 l Wasser + 0.01 mol Ammoniumformiat, Eluent B: 1 l Acetonitril; Gradient: 0.0 min 95% A → 0.1 min 95% A → 2.0 min 15% A → 2.5 min 15% A→ 2.51 min 10% A ----> 3.0 min 10% A; Ofen: 40°C; Fluss: 0.5 ml/min; UV-Detektion: 210 nm

### Methode 7:

Instrument: Waters Acquity, Waters Acquity Autosampler; Säule XBridge BEH C18 2.5 µm 2.1 x 50 mm (UPLC LG 500 nm); Eluent A: 10 mM Ammoniumhydrogencarbonat pH 10, Eluent B: Acetonitril; Gradient: 2-98% B in 0.80 min, halten bei 98% B für 1.30 min.

### Methode 8:

Instrument: Waters ACQUITY SQD UPLC System; Säule: Waters Acquity UPLC HSS T3 1.8 µ 50 x 1 mm; Eluent A: 1 l Wasser + 0.25 ml 99%ige Ameisensäure , Eluent B: 1 l Acetonitril + 0.25 ml 99%ige Ameisensäure; Gradient: 0.0 min 95% A ----> 6.0 min 5% A ----> 7.5 min 5% A Ofen: 50°C; Fluss: 0.35 ml/min; UV-Detektion: 210 - 400 nm.

### Methode 9:

Gerätetyp MS: ThermoFisherScientific LTQ-Orbitrap-XL; Gerätetyp HPLC: Agilent 1200SL; Säule: Agilent, POROSHELL 120, 3 x 150 mm, SB - C18 2.7 µm; Eluent A: 1 l Wasser + 0.1% Trifluoressigsäure; Eluent B: 1 l Acetonitril + 0.1% Trifluoressigsäure; Gradient: 0.0 min 2% B → 0.3 min 2% B → 5.0 min 95% B → 10.0 min 95% B; Ofen: 40°C; Fluss: 0.75 ml/min; UV-Detektion: 210 nm.

### Methode 10:

Instrument: Waters Prep LC/MS System, Säule: XBridge C18 5µm 100x30 mm; Eluent A : Wasser, Eluent B : Acetonitril, Fluß: 65 ml/min plus 5ml 2%ige Ameisensäure in Wasser, Raumtemperatur, Wellenlänge 200-400 nm, At-Säule Injektion (Komplettinjektion); Gradientenprofil: 0 bis 2 min 10% Eluent B, 2 bis 2,2 min auf 20% Eluent B, 2,2 bis 7 min auf 60% Eluent B, 7 bis 7,5 min auf 92% Eluent B, 7,5 bis 9 min bei 92% B.

### Methode 11:

Instrument: Waters Prep LC/MS System, Säule: XBridge C18 5µm 100x30 mm; Eluent A : Wasser, Eluent B : Acetonitril, Fluß: 65 ml/min plus 5ml 2%ige Ameisensäure in Wasser, Raumtemperatur, Wellenlänge 200-400 nm, At-Säule Injektion (Komplettinjektion); Gradientenprofil: 0 bis 2 min 7,5% Eluent B, 2 bis 7 min auf 35% Eluent B, 7 bis 7,5 min auf 92% Eluent B, 7,5 bis 9 min bei 92% B.

### Methode 12:

Instrument: Waters Prep LC/MS System, Säule: XBridge C18 5µm 100x30 mm; Eluent A : Wasser, Eluent B : Acetonitril, Fluß: 65 ml/min plus 5ml 2%iger Ammoniak in Wasser, Raumtemperatur, Wellenlänge 200-400 nm, At-Säule Injektion (Komplettinjektion); Gradientenprofil: 0 bis 2 min 7,5% Eluent B, 2 bis 7 min auf 35% Eluent B, 7 bis 7,5 min auf 92% Eluent B, 7,5 bis 9 min bei 92% B.

### Methode 13:

Instrument MS: Waters SQD2; Instrument HPLC: Waters UPLC; Säule: Zorbax SB-Aq (Agilent), 50 mm x 2.1 mm, 1.8 µm; Eluent A: Wasser + 0.025% Ameisensäure, Eluent B: Acetonitril (ULC) + 0.025% Ameisensäure; Gradient: 0.0 min 98%A - 0.9 min 25%A - 1.0 min 5%A - 1.4 min 5%A - 1.41 min 98%A - 1.5 min 98%A; Ofen: 40°C; Fluss: 0.600 ml/min; UV-Detektion: DAD; 210 nm.

### Methode 14:

Instrument: Waters Prep LC/MS System, Säule: Phenomenex Kinetex C18 5µm 100x30 mmEluent A : Wasser, Eluent B : Acetonitril, Fluß: 65 ml/min plus 5ml 2%ige Ameisensäure in Wasser, Raumtemperatur, Wellenlänge 200-400 nm, At-Column Injektion (Komplettinjektion)Gradientenprofil: 0 bis 2 min 10% Eluent B, 2 bis 2,2 min auf 20% Eluent B, 2,2 bis 7 min auf 60% Eluent B, 7 bis 7,5 min auf 92% Eluent B, 7,5 bis 9 min bei 92% B.

### Methode 15:

Instrument: Waters Single Quad MS System; Instrument Waters UPLC Acquity; Säule : Waters BEH C18 1.7 µm 50 x 2.1 mm; Eluent A: 1 l Wasser + 1.0 mL (25%ig Ammoniak)/L, Eluent B: 1 l Acetonitril; Gradient: 0.0 min 92% A ----> 0.1 min 92% A ----> 1.8 min 5% A ----> 3.5 min 5% A; Ofen: 50°C; Fluss: 0.45 mL/min; UV-Detektion: 210 nm (208-400 nm).

### Weitere Angaben:

Bei Aufreinigungen von erfindungsgemäßen Verbindungen per Chromatographie, vor allem per Säulenchromatographie, werden vorgepackte Kieselgel-Kartuschen, wie z. B. Biotage SNAP Kartuschen, KP-Sil^{®} oder KP-NH^{®} in Kombination mit einem Biotage-System (SP4^{®} oder Isolera Four^{®}) verwendet. Als Laufmittel finden Gradienten aus Hexan/Ethylacetat oder Dichlormethan/Methanol Anwendung.

Bei Aufreinigungen von erfindungsgemäßen Verbindungen per präparativer HPLC nach den oben beschriebenen Methoden, in denen die Elutionsmittel Zusatzstoffe wie beispielsweise Trifluoressigsäure, Ameisensäure oder Ammoniak enthalten, können die erfindungsgemäßen Verbindungen in Salz-Form, beispielsweise als Trifluoracetat, Formiat oder Ammonium-Salz anfallen, sofern die erfindungsgemäßen Verbindungen ausreichend basische bzw. saure Funktionalitäten enthalten. Ein solches Salz kann durch verschiedene dem Fachmann bekannte Methoden in die entsprechende freie Base bzw. Säure überführt werden.

Des Weiteren können Amidine als freie Verbindungen oder anteilig (abhängig von der Präparation bei Beteiligung von Essigsäure) als Acetat-Salze oder Acetat-Solvate vorliegen.

Wenn bei den im Folgenden beschriebenen Synthese-Intermediaten und Ausführungsbeispielen der Erfindung eine Verbindung in der Form eines Salzes der korrespondierenden Base bzw. Säure aufgeführt ist, so ist die exakte stöchiometrische Zusammensetzung eines solchen Salzes, wie es nach dem jeweiligen Herstell- und/oder Reinigungsverfahren erhalten wurde, in der Regel nicht bekannt. Sofern nicht genauer spezifiziert, sind daher Namens- und Strukturformel-Zusätze wie beispielsweise "Hydrochlorid", "Trifluoracetat", "Natrium-Salz" bzw. "x Salzsäure", "x CF₃COOH", "x Na⁺" bei solchen Salzen nicht stöchiometrisch zu verstehen, sondern haben allein deskriptiven Charakter bezüglich der enthaltenen salzbildenden Komponenten.

Sinngemäß gleiches gilt für den Fall, dass Synthese-Intermediate oder Ausführungsbeispiele oder Salze hiervon nach den beschriebenen Herstell- und/oder Reinigungsverfahren in Form von Solvaten, wie beispielsweise Hydraten, erhalten wurden, deren stöchiometrische Zusammensetzung (sofern definierter Art) nicht bekannt ist.

Weiterhin können die erfindungsgemäßen sekundären Amide als Rotationsisomere/ Isomerengemische, insbesondere bei NMR-Untersuchungen, vorliegen. Reinheitsangaben beziehen sich in der Regel auf entsprechende Peak-Integrationen im LC/MS-Chromatogramm, können aber zusätzlich auch unter Zuhilfenahme des ¹H-NMR-Spektrums ermittelt worden sein. Wenn keine Reinheit angegeben ist, handelt es sich in der Regel um eine 100%-Reinheit laut automatischer Peak-Integration im LC/MS-Chromatogramm oder die Reinheit wurde nicht explizit ermittelt.

Angaben zu Ausbeuten in % d. Th. sind in der Regel reinheitskorrigiert, sofern eine Reinheit <100% angegeben ist. Bei lösungsmittelhaltigen oder verunreinigten Chargen kann die Ausbeute formal ">100%" betragen; in diesen Fällen ist die Ausbeute nicht lösungsmittel- bzw. reinheitskorrigiert.

Alle Angaben in ¹H-NMR-Spektren geben die Chemischen Verschiebungen δ[ppm] = in ppm an.

Die in den folgenden Paragraphen angegebenen Multiplizitäten von Protonensignalen in ¹H-NMR-Spektren geben die jeweils beobachtete Signalform wieder und berücksichtigen keine Signalphänomene höherer Ordnung. In der Regel bezieht sich die Angabe zur chemischen Verschiebung auf das Zentrum des betreffenden Signals. Bei breiten Multipletts erfolgt die Angabe eines Intervalls. Durch Lösungsmittel oder Wasser verdeckte Signale wurden entweder tentativ zugeordnet oder sind nicht aufgeführt. Stark verbreiterte Signale - z.B. verursacht durch schnelle Rotation von Molekülteilen oder aufgrund von austauschenden Protonen - wurden ebenfalls tentativ zugeordnet (oft als breites Multiplett oder breites Singulett bezeichnet) oder sind nicht aufgeführt.

Schmelzpunkte und Schmelzbereiche, soweit angegeben, sind nicht korrigiert.

Die ¹H-NMR-Daten ausgewählter Synthese-Intermediate und Ausführungsbeispiele werden in Form von ¹H-NMR-Peaklisten notiert. Zu jedem Signalpeak wird erst der δ[ppm] = -Wert in ppm und dann die Signalintensität in runden Klammem aufgeführt. Die δ[ppm] = -Wert-Signalintensitäts-Zahlenpaare von verschiedenen Signalpeaks werden durch Kommata voneinander getrennt aufgelistet. Die Peakliste eines Beispieles hat daher die Form: δ[ppm] = ₁ (Intensität₁), δ[ppm] = ₂ (Intensität₂), ... , δ[ppm] = i (Intensitätᵢ), ... , δ[ppm] = ₙ (Intensitätₙ).

Die Intensität scharfer Signale korreliert mit der Höhe der Signale in einem gedruckten Beispiel eines NMR-Spektrums in cm und zeigt im Vergleich mit anderen Signalen die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden. Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden. Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen. Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%). Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen. Ein Experte, der die Peaks der Zielverbindungen mit bekannten Verfahren (MestreC, ACD-Simulation, oder unter Verwendung von empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen ¹H-NMR-Interpretation. Eine detaillierte Beschreibung der Darstellung von NMR-Daten in Form von Peaklisten kann der Publikation "Citation of NMR Peaklist Data within Patent Applications" entnommen werden (vgl. Research Disclosure Database Number 605005, 2014, 1. August 2014 oder http://www.researchdisclosure.com/searching-disclosures). In der Peak Picking Routine, die in der Research Disclosure Database Number 605005 beschrieben ist, kann der Parameter "MinimumHeight" zwischen 1% und 4% eingestellt werden. Abhängig von der Art der chemischen Struktur und/oder abhängig von der Konzentration der zu vermessenden Verbindung kann es sinnvoll sein, den Parameter "MinimumHeight" auf Werte <1% einzustellen.

Für alle Reaktanden oder Reagenzien, deren Herstellung im Folgenden nicht explizit beschrieben ist, gilt, dass sie von allgemein zugänglichen Quellen kommerziell bezogen wurden. Für alle übrigen Reaktanden oder Reagenzien, deren Herstellung im Folgenden ebenfalls nicht beschrieben ist und die nicht kommerziell erhältlich waren oder von Quellen bezogen wurden, die nicht allgemein zugänglich sind, ist ein Verweis auf die veröffentlichte Literatur angegeben, in der ihre Herstellung beschrieben ist.

Bei den im Folgenden beschriebenen Intermediaten und Ausführungsbeispielen bedeutet eine im IUPAC-Namen des betreffenden Beispiels aufgeführte Bezeichnung "5RS" in Verbindung mit der Angabe "Racemat", dass es sich hierbei um ein racemisches Gemisch des 5*R*-Enantiomeren (→1. Buchstabe nach der Positionsziffer in "5RS") mit dem entsprechenden 5S-Enantiomeren (→2. Buchstabe nach der Positionsziffer) in handelt. Die Bezeichnung "5RS" in Verbindung mit den Angaben "Enantiomer 1" und "Enantiomer 2" bedeutet, dass es sich hierbei um die beiden Enantiomere in separierter, isolierter Form handelt, wobei eine Zuordnung der Absolutkonfiguration (5*R* oder 5*S*) zu diesen Enantiomeren nicht vorgenommen wurde. Ähnliche Bezeichnungen wie "5*SR* ", die sich aus der veränderten Priorität und/oder Reihenfolge von Namensbestandteilen aufgrund der IUPAC-Nomenklatur-Regeln ergeben, sind nach dieser Anleitung auf analoge Weise zu interpretieren

Bei den im Folgenden beschriebenen Intermediaten und Ausführungsbeispielen bedeutet eine im IUPAC-Namen des betreffenden Beispiels aufgeführte Bezeichnung "5*RS*,7*RS*" in Verbindung mit der Angabe "Racemat", dass es sich hierbei um ein racemisches Gemisch des 5*R*,7*R*-Enantiomeren (→ jeweils 1. Buchstabe nach der Positionsziffer in "5*RS*,7*RS* ") mit dem entsprechenden 5S,7S -Enantiomeren (→ jeweils 2. Buchstabe nach der Positionsziffer) handelt. Die Bezeichnung "5*RS*,7*RS*" in Verbindung mit den Angaben "Enantiomer 1" und "Enantiomer 2" bedeutet, dass es sich hierbei um die beiden Enantiomere in separierter, isolierter Form handelt, wobei eine Zuordnung der Absolutkonfiguration (5*R*,7*R* oder 5*S*,7*S*) zu diesen Enantiomeren nicht vorgenommen wurde. Ähnliche Bezeichnungen wie "5*SR,*7*SR* ", die sich aus der veränderten Priorität und/oder Reihenfolge von Namensbestandteilen aufgrund der IUPAC-Nomenklatur-Regeln ergeben, sind nach dieser Anleitung auf analoge Weise zu interpretieren.

Die (5S)-Konfiguration wurde aufgrund von Kristallstrukturaufklärung für Beispiel 26, 108, 113, 157, 237, 358 und 454 zugeordnet. In Analogie dazu wurden für die Beispiele 183-188, 190-219, 275-279, 342-402, 404-415, 418-563 die (5S)-Konfiguration zugeordnet.

### Ausgangsverbindungen und Intermediate:

### Intermediat 1

### Methyl-3-fluor-2-(trifluormethyl)isonicotinat

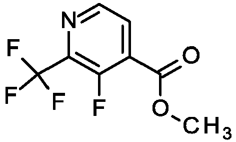

3-Fluor-2-(trifluormethyl)isonicotinsäure (1.00 g, 4.78 mmol) wurde in Methanol (10 ml) gelöst und mit Schwefelsäure (310 µl, 5.7 mmol) versetzt. Das Reaktionsgemisch wurde für 30 Minuten auf 60 °C erhitzt und anschließend über Nacht bei Raumtemperatur gerührt. Das Reaktionsgemisch wurde im Vakuum eingeengt, der Rückstand in Ethylacetat gelöst und mit gesättigter Natriumhydrogencarbonat-Lösung gewaschen. Die organische Phase wurde über Natriumsulfat getrocknet, filtriert und das Filtrat eingeengt. Es wurden 890 mg (70 % Reinheit, 58 % d. Th.) der Titelverbindung erhalten.
LC-MS (Methode 1): Rₜ = 1.15 min; MS (ESIpos): m/z = 224 [M+H]⁺

### Intermediat 2

### [3-Fluor-2-(trifluormethyl)pyridin-4-yl]methanol

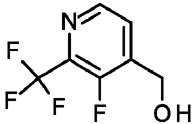

Methyl-3-fluor-2-(trifluormethyl)isonicotinat (890 mg, Reinheit 70%, 3.99 mmol) wurde in Methanol (5.0 ml) gelöst und bei 0°C portionsweise mit Natriumborhydrid (166 mg, 4.39 mmol) versetzt. Das Reaktionsgemisch wurde nach Rühren über Nacht bei Raumtemperatur mit gesättigter wässriger Ammoniumchlorid-Lösung versetzt und das Methanol im Vakuum entfernt. Der Rückstand wurde mit Ethylacetat und Wasser versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 420 mg (54 % d. Th.) der Titelverbindung erhalten. Die Verbindung wurde direkt weiter umgestzt.

### Intermediat 3

### 4-(Chlormethyl)-3-fluor-2-(trifluormethyl)pyridin Hydrochlorid

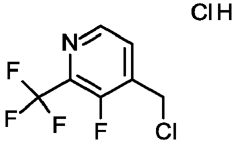

[3-Fluor-2-(trifluormethyl)pyridin-4-yl]methanol (420 mg, 2.15 mmol) wurde in Dichlormethan (10 ml) gelöst und bei Raumtemperatur mit Thionyldichlorid (310 µl, 4.3 mmol) versetzt. Nachdem das Reaktionsgemisch über Nacht bei Raumtemperatur gerührt wurde, wurde erneut Thionyldichlorid (310 µl, 4.3 mmol) und ein Tropfen Dimethylformamid zugegeben. Das Reaktionsgemisch wurde für eine Stunde bei Raumtemperatur gerührt und anschließend erneut mit Thionyldichlorid (620 µl, 8.6 mmol) und einem Tropfen Dimethylformamid versetzt. Nach erneutem Rühren für 2 Stunden bei Raumtemperatur wurde das Lösungsmittel unter Vakuum entfernt. Es wurden 379 mg (70 % d. Th.) der Titelverbindung erhalten.
GC-MS (Methode 2): Rₜ = 2.74 min; MS (ESIpos): m/z = 213 [M+H]⁺

### Intermediat 4

### 5-Chlor-4-(trifluormethyl)pyridin-2-yl]methanol

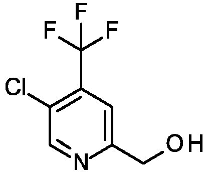

Methyl-5-chlor-4-(trifluormethyl)pyridin-2-carboxylat (412 mg, 1.72 mmol) wurde in Methanol (20 ml) gelöst und bei 0°C portionsweise mit Natriumborhydrid (78.1 mg, 2.06 mmol) versetzt. Das Reaktionsgemisch wurde nach Rühren über Nacht bei Raumtemperatur erneut mit Natriumborhydrid (35 mg, 0.9 mmol) versetzt. Nach 2 Tagen Rühren bei Raumtemperatur wurde das Reaktionsgemsich mit gesättigter wässriger Ammoniumchlorid-Lösung versetzt und das Methanol im Vakuum entfernt. Der Rückstand wurde mit Ethylacetat und Wasser versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 295 mg (77 % d. Th.) der Titelverbindung erhalten.
LC-MS (Methode 3): Rₜ = 1.32 min; MS (ESIpos): m/z = 212 [M+H]⁺

### Intermediat 5

### 5-Chlor-2-(chlormethyl)-4-(trifluormethyl)pyridin Hydrochlorid

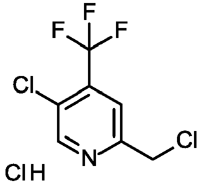

[5-Chlor-4-(trifluormethyl)pyridin-2-yl]methanol (295 mg, 1.39 mmol) wurde in Dichlormethan (20 ml) gelöst und bei Raumtemperatur mit Thionyldichlorid (200 µl, 2.8 mmol) versetzt. Nachdem das Reaktionsgemisch für 72 Stunden bei Raumtemperatur gerührt wurde, wurde das Lösungsmittel unter Vakuum entfernt. Es wurden 274 mg (85 % Reinheit, 63 % d. Th.) der Titelverbindung erhalten
¹H-NMR (400 MHz, DMSO-d6) δ [ppm]: 0.008 (0.42), 3.937 (2.06), 4.644 (1.11), 4.903 (16.00), 7.839 (0.47), 8.078 (5.73), 8.296 (0.43), 8.857 (0.51), 8.957 (5.04), 9.102 (0.45).

### Intermediat 6

### 4-(Chlormethyl)-2-(trifluormethyl)chinolin Hydrochlorid

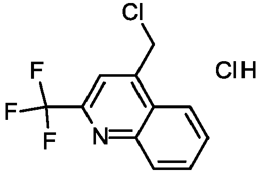

[2-(Trifluormethyl)chinolin-4-yl]methanol (350 mg, 1.54 mmol) wurde in Dichlormethan (10 ml) gelöst und bei 0°C mit Thionyldichlorid (220 µl, 3.1 mmol) versetzt. Nachdem das Reaktionsgemisch über Nacht bei Raumtemperatur gerührt wurde, wurde das Lösungsmittel unter Vakuum entfernt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 336 mg (74 % Reinheit, 57 % d. Th.) der Titelverbindung erhalten.
LC-MS (Methode 4): Rₜ = 1.06 min; MS (ESIpos): m/z = 246 [M+H]⁺

### Intermediat 7

### 4-(Chlormethyl)-2-(trifluormethyl)pyrimidin Hydrochlorid

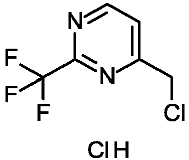

[2-(Trifluormethyl)pyrimidin-4-yl]methanol (313 mg, 1.76 mmol) wurde in Dichlormethan (10 ml) gelöst und bei Raumtemperatur mit Thionyldichlorid (260 µl, 3.5 mmol) versetzt. Nachdem das Reaktionsgemisch für 4 Stunden bei Raumtemperatur gerührt wurde, wurde das Lösungsmittel unter Vakuum entfernt. Es wurden 134 mg (61 % Reinheit, 20 % d. Th.) der Titelverbindung erhalten.

¹H-NMR (400 MHz, DMSO-d6) δ [ppm]: 3.678 (0.60), 4.946 (16.00), 5.366 (0.57), 7.993 (2.80), 8.006 (2.88), 9.129 (3.11), 9.142 (3.06).

### Intermediat 8

### Methyl-6-[1-(tert-butoxycarbonyl)hydrazino]pyridin-2-carboxylat

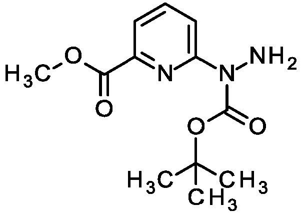

Methyl-6-chlorpyridin-2-carboxylat (32.5 g, 189 mmol), tert-Butyl-hydrazincarboxylat (25.0 g, 189 mmol), und Cäsiumcarbonat (61.6 g, 189 mmol) wurden in Toluol (325 ml) mit Argon entgast, bevor [1,1'-Bis(diphenylphosphino)ferrocen]dichloropalladium(II) (6.9 g, 10 mmol) zugegeben wurde. Die Reaktionsmischung wurde über Nacht bei 100°C gerührt. Das Reaktionsgemisch wurde mit Dichlormethan verdünnt und über Kieselgur filtriert. Das Filtrat wurde im Vakuum eingeengt und der Rückstand wurde über Säulenchromatographie aufgereinigt (Kieselgel; Laufmittel:, Methanol/Dichlormethan 4/96). Die produkthaltigen Fraktionen wurden im Vakuum eingeengt und mit Methyl-tert-butylether verrührt. Es wurden 28.8 g (57% d. Th.) der Titelverbindung erhalten.
LC-MS (Methode 5): Rₜ = 3.45 min; MS (ESIpos): m/z = 268 [M+H]⁺

### Intermediat 9

### 2-(Diazan-2-ium-1-yl)-6-(methoxycarbonyl)pyridiniumdichlorid

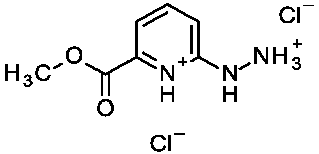

Methyl-6-{1-[(benzyloxy)carbonyl]hydrazino}pyridin-2-carboxylat (29.8 g, 112 mmol) wurde in Dioxan (150 ml) gelöst und mit Salzsäure (112 ml, 446 mmol, 4 M in Dioxan) versetzt. Das Reaktionsgemisch wurde für 4h bei Raumtemperatur gerührt und mit Methyl-tert-butylether (500 ml) verdünnt. Nach 1 h Rühren, wurde der Feststoff abfiltriert, mit Methyl-tert-butylether gewaschen und getrocknet. Es wurden 28.8 g (> 100 %) der Titelverbindung erhalten.
LC-MS (Methode 5): Rₜ = 0.88 min; MS (ESIpos): m/z = 168 [M+H]⁺

### Intermediat 10

### Methyl-3-oxo-2,3-dihydro[1,2,4]triazolo[4,3-a]pyridin-5-carboxylat

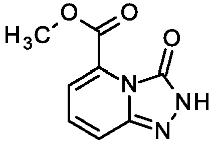

2-(Diazan-2-ium-1-yl)-6-(methoxycarbonyl)pyridiniumdichlorid (28.8 g, 112 mmol) wurde in THF (500 ml) gelöst und mit Triethylamin (46 ml, 335 mmol) versetzt. Das Reaktionsgemisch wurde für 15 min bei Raumtemperatur gerührt. Anschließend wurde 1,1-Carbonyldiimidazol (19.9 g, 123 mmol) zugegeben. Das Reaktionsgemisch wurde für 2 h bei 70°C gerührt. Das Reaktionsgemisch wurde filtriert und das Filtrat wurde im Vakuum eingeengt. Der Rückstand wurde in verdünnter wässrige Salzsäure aufgenommen und mit Dichlormethan/Methanol 9/1 extrahiert. Die vereinigten organischen Phasen wurden über Natriumsulfat getrocknet, filtriert und das Filtrat eingeengt. Der Rückstand wurde in Ethylacetat suspendiert und über Nacht gerührt. Nach Filtration und Trocknung wurden 11.4 g (53% d. Th.) der Titelverbindung erhalten.
LC-MS (Methode 5): Rₜ = 1.40 min; MS (ESIpos): m/z = 194 [M+H]⁺

### Intermediat 11

### Methy-(5RS)--3-oxo-2,3,5,6,7,8-hexahydro[1,2,4]triazolo[4,3-a]pyridin-5-carboxylat (Racemat)

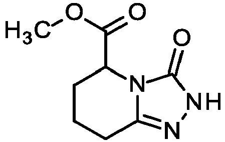

Methyl-3-oxo-2,3-dihydro[1,2,4]triazolo[4,3-a]pyridin-5-carboxylat (17.6 g, 91.1 mmol) und Palladium auf Kohle (1.0 g, 10%ig) wurden in Methanol (500 ml) suspendiert und über Nacht bei Raumtemperatur in einer Wasserstoffatmosphäre (10 bar) gerührt. Das Reaktionsgemisch wurde über Kieselgur filtriert. Das Filtrat wurde eingeengt und es wurden 17.8 g (99 % d. Th.) der Titelverbindung erhalten.
LC-MS (Methode 5): Rₜ = 1.35 min; MS (ESIpos): m/z = 198 [M+H]⁺
¹H-NMR (500 MHz, MeOD) δ [ppm]= 1.64 - 1.74 (br, 1H), 1.89 - 1.97 (br, 1H), 2.15 - 2.29 (br, 2H), 2.60 - 2.69 (m, 1H), 2.77 (dt, 1H), 3.79 (s, 3H), 4.63 (dd, 1H).

### Intermediat 12

### Methyl-(5RS)- 3-oxo-2,3,5,6,7,8-hexahydro[1,2,4]triazolo[4,3-a]pyridin-5-carboxylat (Enantiomer 1)

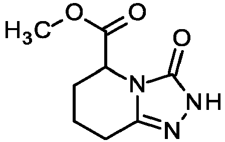

Methyl-(5RS)-3-oxo-2,3,5,6,7,8-hexahydro[1,2,4]triazolo[4,3-a]pyridin-5-carboxylat (Racemat) wurde durch chirale präparative SFC getrennt [Probenvorbereitung: 33.20g gelöst in 200 ml Methanol; Injektionsvolumen: 4.0 ml; Säule: Daicel Chiralpak^{®} IC 20 µm, 360 x 50 mm; Laufmittel: CO₂/i-Propanol 80/20; Fluß: 400 g/min; Temperature 35°C; UV Detektion: 210 nm]. Nach der Trennung, wurden 15.7 g von Enantiomer 1, welches zuerst eluierte, und 15.8 g von Enantiomer 2, welches später eluierte, isoliert.

### Enantiomer 1

Analytische chirale SFC: Rₜ = 2.76 min, d.e. = 100% [Säule: Daicel Chiralpak^{®} IC 50 x 4.6 mm; Laufmittel: CO₂/i-Propanol 80:20; Fluß: 3 ml/min; UV Detektion: 210 nm].
LC-MS (Methode 3): Rₜ = 0.54 min; MS (ESIpos): m/z = 198 [M+H]⁺
¹H-NMR (400 MHz, DMSO-d6) δ [ppm]= 1.45 - 1.60 (m, 1H), 1.75 - 1.84 (m, 1H), 1.98 - 2.17 (m, 2H), 2.48 - 2.58 (m, 1H), 2.59 - 2.68 (m, 1H), 3.69 (s, 3H), 4.51 (dd, 1H), 11.42 (s, 1H).

### Intermediat 13

### Methyl-(5RS)-3-oxo-2,3,5,6,7,8-hexahydro[1,2,4]triazolo[4,3-a]pyridin-5-carboxylat (Enantiomer 2)

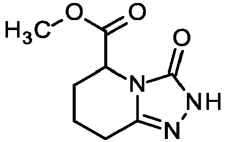

Methyl-(5RS)-3-oxo-2,3,5,6,7,8-hexahydro[1,2,4]triazolo[4,3-a]pyridin-5-carboxylat (Racemat) wurde durch chirale präparative SFC getrennt [Probenvorbereitung: 33.20 g gelöst in 200 ml Methanol; Injektionsvolumen: 4.0 ml; Säule: Daicel Chiralpak^{®} IC 20 µm, 360 x 50 mm; Laufmittel: CO₂/i-Propanol 80/20; Fluß: 400 g/min; Temperature 35°C; UV Detektion: 210 nm]. Nach der Trennung, wurden 15.7 g von Enantiomer 1, welches zuerst eluierte, und 15.8 g von Enantiomer 2, welches später eluierte, isoliert.

### Enantiomer 2

Analytische chirale SFC: Rₜ = 3.90 min, d.e. = 100% [Säule: Daicel Chiralpak^{®} IC 50 x 4.6 mm; Laufmittel: CO₂/i-Propanol 80:20; Fluß: 3 ml/min; UV Detektion: 210 nm].
LC-MS (Methode 3): Rₜ = 0.54 min; MS (ESIpos): m/z = 198 [M+H]⁺
¹H-NMR (400 MHz, DMSO-d6) δ [ppm]= 1.42 - 1.59 (m, 1H), 1.72 - 1.86 (m, 1H), 1.96 - 2.17 (m, 2H), 2.44 - 2.59 (m, 1H), 2.59 - 2.69 (m, 1H), 3.69 (s, 3H), 4.51 (dd, 1H), 11.42 (s, 1H).

Die (5S)-Konfiguration wurde aufgrund der Kristallstrukturaufklärung für Beispiel 26, 108, 113, 157, 237, 358 und 454 zugeordnet.
Methyl-(5S)-3-oxo-2,3,5,6,7,8-hexahydro[1,2,4]triazolo[4,3-a]pyridin-5-carboxylat

### Intermediat 14

### Methyl-1 -(4-methoxybenzyl)hydrazincarboxylat

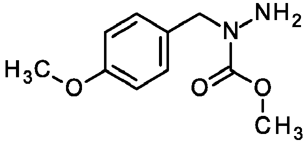

(4-Methoxybenzyl)hydrazindihydrochlorid (1.50 g, 80% Reinheit, 5.33 mmol) wurde in Dichlormethan (50 ml) gelöst, Triethylamin (2.6 ml, 19 mmol) zugegeben und das Gemisch 30 Minuten bei Raumtemperatur gerührt. Dieses wurde anschließend auf 0°C abgekühlt und mit Methylcarbonochloridat (450 µl, 5.9 mmol), gelöst in Dichlormethan (10 ml), versetzt. Das Reaktionsgemisch wurde nach Rühren über Nacht bei Raumtemperatur mit Wasser versetzt. Die organische Phase wurde abgetrennt, mit gesättigter wässriger Natriumchlorid-Lösung gewaschen, über Natriumsulfat getrocknet, filtriert und das Filtrat eingeengt. Der Rückstand wurde über Säulenchromatographie aufgereinigt (Kieselgel, Laufmittel: Cyclohexan/Ethylacetat-Gradient). Die produkthaltigen Fraktionen wurden im Vakuum eingeengt und es wurden 1.04 g (92 % Reinheit, 85 % d. Th.) der Titelverbindung erhalten.
LC-MS (Methode 3): Rₜ = 1.08 min; MS (ESIpos): m/z = 211 [M+H]⁺
¹H-NMR (400 MHz, DMSO-d6) δ [ppm]: 1.988 (0.46), 3.314 (7.32), 3.541 (0.79), 3.622 (16.00), 4.397 (7.56), 4.553 (4.94), 6.861 (0.47), 6.868 (3.43), 6.873 (1.30), 6.885 (1.38), 6.890 (3.96), 6.897 (0.47), 7.161 (3.22), 7.182 (2.79).

### Intermediat 15

### Methyl-(2S)-6-oxopiperidin-2-carboxylat (Enantiomer)

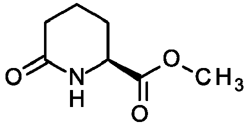

Methanol (15 ml) wurde vorgelegt und bei 0°C Thionyldichlorid (560 µl, 7.7 mmol) und anschließend (2S)-6-Oxopiperidin-2-carbonsäure (1.00 g, 6.99 mmol) portionsweise zugegeben. Nach Rühren über Nacht bei Raumtemperatur wurde das Lösungsmittel im Vakuum entfernt und der Rückstand mit Toluol und Triethylamin (1.9 ml, 14 mmol) versetzt. Das Gemisch wurde für 30 Minuten bei Raumtemperatur gerührt, anschließend filtrirt und das Filtrat eingeengt. Es wurden 805 mg (70 % d. Th.) der Titelverbindung erhalten.
LC-MS (Methode 4): Rₜ = 0.26 min; MS (ESIpos): m/z = 158 [M+H]⁺
¹H-NMR (400 MHz, DMSO-d6) δ [ppm]: -0.008 (1.70), 0.008 (1.69), 1.541 (0.78), 1.550 (1.05), 1.560 (1.24), 1.566 (1.90), 1.575 (2.78), 1.584 (3.48), 1.591 (3.19), 1.594 (3.57), 1.600 (4.36), 1.610 (3.31), 1.619 (4.16), 1.628 (3.71), 1.636 (3.43), 1.644 (5.02), 1.652 (4.23), 1.660 (4.33), 1.668 (4.60), 1.676 (2.96), 1.684 (2.78), 1.693 (1.99), 1.702 (1.75), 1.708 (1.01), 1.717 (0.85), 1.745 (1.91), 1.754 (2.09), 1.759 (2.49), 1.768 (2.39), 1.778 (3.46), 1.787 (3.35), 1.793 (3.60), 1.802 (3.22), 1.810 (2.52), 1.819 (1.83), 1.901 (2.92), 1.910 (3.42), 1.916 (3.20), 1.925 (4.72), 1.935 (4.50), 1.941 (3.46), 1.949 (4.49), 1.959 (3.35), 1.968 (1.78), 1.974 (1.91), 1.983 (1.62), 2.097 (0.90), 2.113 (0.85), 2.128 (11.31), 2.141 (16.00), 2.148 (10.19), 2.156 (7.22), 2.164 (7.72), 2.188 (0.61), 2.192 (0.69), 2.208 (0.70), 2.289 (0.72), 2.306 (1.34), 2.324 (0.85), 2.523 (0.72), 3.168 (4.31), 3.311 (4.19), 3.409 (0.45), 3.480 (0.73), 3.581 (8.47), 3.635 (7.91), 3.849 (0.72), 4.049 (4.37), 4.055 (4.73), 4.063 (8.78), 4.069 (8.78), 4.077 (4.66), 4.083 (4.33), 7.542 (6.15).

### Intermediat 16

### Methyl-(5S)-2-(4-methoxybenzyl)-3-oxo-2,3,5,6,7,8-hexahydro[1,2,4]triazolo[4,3-a]pyridin-5-carboxylat (Enantiomer)

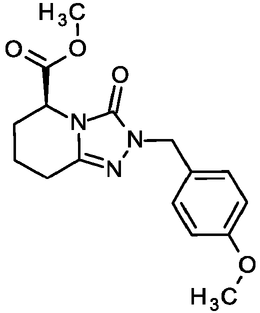

Methyl-(2S)-6-oxopiperidin-2-carboxylat (300 mg, 1.91 mmol) wurde in Dichlormethan (6.0 ml, 93 mmol) (Enantiomer) bei Raumtemperatur und unter Argon vorgelegt. Anschließend wurde Trimethyloxoniumtetrafluoroborat (311 mg, 2.10 mmol) zugegeben und das Reaktionsgemisch für 2 Tage bei Raumtemperatur gerührt. Das Lösungsmittel wurde im Vakuum entfernt und der Rückstand mit DMF (5 ml) versetzt. Anschließend wurde Methyl-1-(4-methoxybenzyl)-hydrazincarboxylat (401 mg, 1.91 mmol) zugegeben und das Reaktionsgemisch 4 Stunden bei 170 °C gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 156 mg (26 % d. Th.) der Titelverbindung erhalten.
LC-MS (Methode 3): Rₜ = 1.20 min; MS (ESIpos): m/z = 318 [M+H]⁺
¹H-NMR (400 MHz, DMSO-d6) δ [ppm]: -0.008 (1.02), 1.784 (0.43), 1.795 (0.46), 2.045 (0.41), 2.055 (0.68), 2.068 (0.64), 2.076 (0.62), 2.084 (0.47), 2.092 (0.40), 2.100 (0.48), 2.112 (0.44), 2.120 (0.47), 2.128 (0.45), 2.564 (0.76), 2.578 (0.61), 2.603 (0.56), 2.615 (1.06), 2.627 (0.59), 2.657 (0.47), 2.669 (0.42), 2.829 (0.43), 3.700 (14.01), 3.730 (16.00), 3.743 (0.70), 4.581 (0.93), 4.591 (1.04), 4.597 (1.20), 4.607 (0.91), 4.749 (6.71), 6.882 (3.25), 6.903 (3.86), 7.161 (3.37), 7.183 (2.94).

### Intermediat 17

### Methyl-(5S)-3-oxo-2,3,5,6,7,8-hexahydro[1,2,4]triazolo[4,3-a]pyridin-5-carboxylat (Enantiomer)

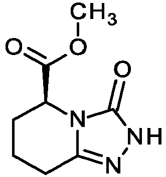

Methyl-(5S)-2-(4-methoxybenzyl)-3-oxo-2,3,5,6,7,8-hexahydro[1,2,4]triazolo[4,3-a]pyridin-5-carboxylat (155 mg, 488 µmol) (Enantiomer) wurde in Trifluoressigsäure (4.5 ml) gelöst und es wurde eine Stunde bei 150°C Mikrowellenapparatur gerührt. Zu dem Reaktionsgemisch wurde gesättigte wässrige Natriumchlorid-Lösung gegeben und die Lösung mit gesättigter wässriger Natriumhydrogencarbonat-Lösung auf pH 7 eingestellt. Es wurde Dichlormethan zugegeben und die wässrige Phase dreimal mit Dichlormethan extrahiert. Die vereinigten organischen Phasen wurden über Natriumsulfat getrocknet, filtriert und das Filtrat eingeengt. Der Rückstand wurde über Säulenchromatographie aufgereinigt (Kieselgel, Laufmittel: Dichlormethan/Methanol-Gradient). Die produkthaltigen Fraktionen wurden im Vakuum eingeengt mit Ethylacetat versetzt und filtriert. Das Filtrat wurde bis auf einen Milliliter eingengt und das Produkt mit 10 ml Petrolether auskristallisiert. Der Festoff wurde abfiltriert und im Vakuum getrocknet. Es wurden 53.1 mg (53 % d. Th.) der Titelverbindung mit einem ee von 88% erhalten.

### Die Bestimmung des Enantiomerenüberschusses erfolgt über analytische chirale HPLC:

### Vergleichswerte Racemat:

Analytische chirale HPLC: Enantiomer 1 Rₜ = 3.40, Enantiomer 2 Rₜ = 4.09 min, Verhältnis 1:1 [Säule: Daicel Chiraltek^{®} AY-3 3µm 50 x 4.6 mm; Laufmittel: i-Hexan/Ethanol 1:1, Fluß: 1 ml/min; UV Detektion: 220 nm]

### Enantiomer:

Analytische chirale HPLC: Enantiomer 1 Rₜ = 3.35, Enantiomer 2 Rₜ = 4.01 min, Verhältnis 6:94 [Säule: Daicel Chiraltek^{®} AY-3 3µm 50 x 4.6 mm; Laufmittel: i-Hexan/Ethanol 1:1, Fluß: 1 ml/min; UV Detektion: 220 nm]

¹H-NMR (400 MHz, DMSO-d6) δ [ppm]: 1.506 (0.41), 1.540 (0.44), 1.774 (0.50), 1.787 (0.56), 1.800 (0.48), 1.808 (0.40), 2.026 (0.49), 2.037 (0.75), 2.050 (0.70), 2.061 (0.71), 2.069 (0.52), 2.077 (0.48), 2.085 (0.55), 2.096 (0.54), 2.105 (0.55), 2.113 (0.53), 2.560 (0.95), 2.574 (0.73), 2.601 (0.65), 2.613 (1.22), 2.626 (0.67), 2.655 (0.55), 2.669 (0.46), 3.586 (0.54), 3.687 (16.00), 4.496 (1.07), 4.506 (1.20), 4.511 (1.37), 4.521 (1.06), 11.424 (1.40).

Die (5S)-Konfiguration wurde aufgrund der Kristallstrukturaufklärung für Beispiel 26, 108, 113, 157, 237, 358 und 454 zugeordnet.

### Intermediat 18

### Methyl-(5RS)-2-(4-methoxybenzyl)-5-methyl-3-oxo-2,3,5,6,7,8-hexahydro[1,2,4]triazolo[4,3-a]pyridin-5-carboxylat (Racemat)

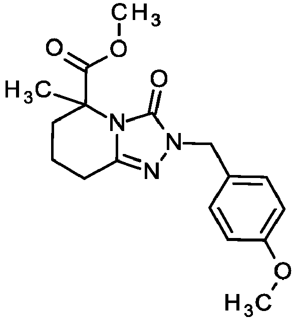

Methyl-(5RS)-2-methyl-6-oxopiperidin-2-carboxylat (Racemat) (500 mg, 93 % Reinheit, 2.72 mmol, CAS 89115-90-2) wurde in Dichlormethan (10 ml) bei Raumtemperatur und unter Argon vorgelegt. Anschließend wurde Trimethyloxoniumtetrafluoroborat (442 mg, 2.99 mmol) zugegeben und das Reaktionsgemisch für 2 Tage bei Raumtemperatur gerührt. Das Lösungsmittel wurde im Vakuum entfernt und der Rückstand mit DMF (5 ml) versetzt. Anschließend wurde Methyl-1-(4-methoxybenzyl)hydrazincarboxylat (571 mg, 2.72 mmol, 92 % Reinheit) zugegeben und das Reaktionsgemisch 8 Stunden bei 170 °C gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 186 mg (91 % Reinheit, 19 % d. Th.) der Titelverbindung erhalten.
LC-MS (Methode 3): Rₜ = 1.42 min; MS (ESIpos): m/z = 332 [M+H]⁺
¹H-NMR (400 MHz, DMSO-d6) δ [ppm]: 1.589 (0.43), 1.597 (0.42), 1.606 (0.51), 1.623 (0.42), 1.697 (11.51), 1.769 (0.47), 1.782 (0.56), 1.802 (0.43), 1.907 (0.40), 1.935 (0.74), 1.942 (0.63), 1.960 (0.63), 2.023 (0.59), 2.030 (0.65), 2.042 (0.59), 2.050 (0.53), 2.058 (0.40), 2.570 (2.79), 2.586 (1.50), 2.829 (3.14), 3.531 (0.70), 3.645 (0.69), 3.665 (13.44), 3.730 (16.00), 3.742 (4.13), 4.677 (0.71), 4.716 (2.98), 4.736 (2.97), 4.774 (0.71), 5.753 (5.67), 6.885 (3.42), 6.906 (4.16), 6.922 (0.90), 7.148 (3.49), 7.169 (3.14), 7.232 (0.92), 7.253 (0.84), 7.632 (0.93).

### Intermediat 19

### Methyl-(5RS)-methyl-3-oxo-2,3,5,6,7,8-hexahydro[1,2,4]triazolo[4,3-a]pyridin-5-carboxylat (Racemat)

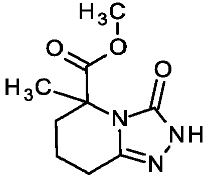

Methyl-2-(4-methoxybenzyl)-5-methyl-3-oxo-2,3,5,6,7,8-hexahydro[1,2,4]triazolo[4,3-a]pyridin-5-carboxylat (Racemat) (186 mg, 560 µmol) wurde in Trifluoressigsäure (5.0 ml) gelöst und es wurde eine Stunde bei 150°C (Mikrowellenapparatur) gerührt. Zu dem Reaktionsgemisch wurde gesättigte wässrige Natriumchlorid-Lösung gegeben und die Lösung mit gesättigter wässriger Natriumhydrogencarbonat-Lösung auf pH 7 eingestellt. Es wurde Dichlormethan zugegeben und die wässrige Phase dreimal mit Dichlormethan extrahiert. Die vereinigten organischen Phasen wurden über Natriumsulfat getrocknet, filtriert und das Filtrat eingeengt. Der Rückstand wurde über Säulenchromatographie aufgereinigt (Kieselgel, Laufmittel: Dichlormethan/Methanol-Gradient). Die produkthaltigen Fraktionen wurden im Vakuum eingeengt und es wurden 40.5 mg (34 % d. Th.) der Titelverbindung erhalten.
LC-MS (Methode 3): Rₜ = 0.70 min; MS (ESIpos): m/z = 212 [M+H]⁺
¹H-NMR (400 MHz, DMSO-d6) δ [ppm]: -0.008 (0.64), 0.008 (0.61), 1.622 (0.54), 1.631 (0.51), 1.640 (0.78), 1.654 (15.63), 1.757 (0.51), 1.771 (0.63), 1.777 (0.57), 1.785 (0.46), 1.792 (0.52), 1.880 (0.46), 1.887 (0.52), 1.903 (0.40), 1.915 (0.92), 1.922 (0.83), 1.938 (0.81), 1.946 (0.65), 1.990 (0.78), 1.998 (0.84), 2.010 (0.73), 2.018 (0.68), 2.025 (0.49), 2.033 (0.43), 2.045 (0.43), 2.568 (3.91), 2.584 (1.91), 3.309 (16.00), 11.369 (1.45).

### Intermediat 20

### tert-Butyl-6-chlorpyridin-2-carboxylat

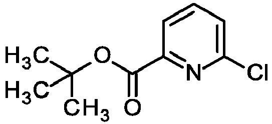

6-Chlorpicolinsäure (80.0 g, 0.51 mol) wurde in Pyridin (300 ml) und tert-Butanol (1.60 I) gelöst, dann wurde p-Toluolsulfonsäurechlorid (194 g, 1.02 mol) portionsweise zugegeben. Die Reaktionsmischung wurde über Nacht bei Raumtermperatur gerührt. Nach Zugabe von gesättigter wässriger Natriumhydrogencarbonat-Lösung wurde 1 h bei Raumtemperatur gerührt. Die Reaktionsmischung wurde auf ein Volumen von 1.50 l eingeengt und mit Heptan (500 ml), Ethylacetat (500 ml) und Wasser (500 ml) verdünnt. Die organische Phase wurde abgetrennt, mit gesättigter wässriger Natriumchlorid-Lösung gewaschen, über Magnesiumsulfat getrocknet, filtriert und das Filtrat eingeengt. Es wurden 104 g (96 % d. Th.) der Titelverbindung erhalten.

¹H-NMR (400 MHz, CDCl₃) δ [ppm]= 1.61 (s, 9H), 7.45 - 7.48 (dd, 1H), 7.74 - 7.77 (t, 1H), 7.93 - 7.95 (dd, 1H).

### Intermediat 21

### tert-Butyl-6-{1-[(benzyloxy)carbonyl]hydrazino}pyridin-2-carboxylat

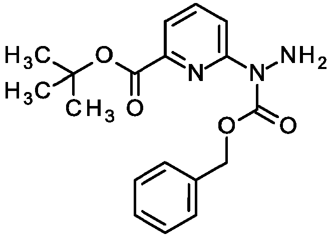

tert-Butyl-6-chlorpyridin-2-carboxylat (91.1 g, 0.43 mol), Benzyl-hydrazincarboxylat (70.9 g, 0.43 mol), Cäsiumcarbonat (174 g, 0.53 mol) und 1,1'-Bis(diphenylphosphino)ferrocen (17.1 g, 32.0 mmol) wurden unter Argon in Toluol (1.00 l) suspendiert. Bis(dibenzylidenaceton)palladium(0) (9.76 g, 10.7 mmol) wurde zugegeben und die Reaktionsmischung wurde 3 h bei 80°C gerührt. Das Reaktionsgemisch wurde mit Wasser und Ethylacetat versetzt, über Kieselgur filtriert und mit Ethylacetat nachgewaschen. Die organische Phase wurde abgetrennt, mit Wasser und gesättigter wässriger Natriumchlorid-Lösung gewaschen, über Magnesiumsulfat getrocknet und filtriert. Das Filtrat wurde mit Kieselgel versetzt, für 10 min gerührt, anschließend filtriert und mit Ethylacetat nachgewaschen. Das Filtrat wurde eingeengt und der Rückstand wurde über Säulenchromatographie aufgereinigt (Kieselgel; Laufmittel:, Heptan/Ethylacetat 4/1, 2/1, 1/1). Die produkthaltigen Fraktionen wurden im Vakuum eingeengt und es wurden 112 g (77 % d. Th.) der Titelverbindung erhalten.
LC-MS (Methode 7): Rₜ = 0.81 min; MS (ESIpos): m/z = 344 [M+H]⁺
¹H-NMR (400 MHz, CDCl₃) δ [ppm]= 1.59 (s, 9H), 5.30 (s, 2H), 7.31 - 7.39 (m, 3H), 7.42 - 7.45 (m, 2H), 7.76 - 7.78 (m, 2H), 7.98 - 8.12 (dd, 1H).

### Intermediat 22

### tert-Butyl-6-hydrazinopyridin-2-carboxylat

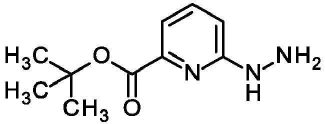

tert-Butyl-6-{1-[(benzyloxy)carbonyl]hydrazino}pyridin-2-carboxylat (112 g, 0.33 mol) und Palladium auf Kohle (11.2 g, 5%ig) wurden in Toluol (100 ml) und Methanol (1.12 I) suspendiert und über Nacht bei Raumtemperatur in einer Wasserstoffatmosphäre (1 bar) gerührt. Das Reaktionsgemisch wurde über Kieselgur filtriert und mit Methanol gewaschen. Das Lösungsmittel wurde unter Vakuum entfernt und es wurden 79.9 g (>100%) der Titelverbindung erhalten.

¹H-NMR (400 MHz, CDCl₃) δ [ppm]= 1.61 (s, 9H), 6.92 - 6.95 (d, 1H), 7.38 - 7.41 (d, 1H), 7.53 - 7.58 (dd, 1H).

### Intermediat 23

### tert-Butyl-3-oxo-2,3-dihydro[1,2,4]triazolo[4,3-a]pyridin-5-carboxylat

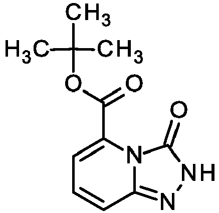

tert-Butyl-6-hydrazinopyridin-2-carboxylat (79.9 g, 0.33 mol) wurde in THF (1.40 l) gelöst und unter Rühren bei Raumtemperartur wurde 1,1-Carbonyldiimidazol (74.3 g, 0.46 mol) zugegeben. Das Reaktionsgemisch wurde über Nacht bei Raumtemperatur gerührt. Nach Zugabe von Wasser (1.0 l) wurde für 15 min gerührt und flüchtige Lösungsmittel wurden im Vakuum entfernt. Der wässrige Rückstand wurde mit Natriumchlorid gesättigt und mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Die organische Phase wurde abgetrennt, über Magnesiumsulfat und Kieselgel getrocknet und filtriert. Das Filtrat wurde eingeengt und der Rückstand wurde über Säulenchromatographie aufgereinigt (SiO₂; Laufmittel: Heptan/Ethylacetat 4/1, 2/1, 1/1, 1/2). Die produkthaltigen Fraktionen wurden im Vakuum eingeengt und es wurden 62.4 g (80 % d. Th. über zwei Stufen) der Titelverbindung erhalten.
LC-MS (Methode 7): Rₜ = 0.50 min; MS (ESIpos): m/z = 180 [M+H]⁺
¹H-NMR (400 MHz, CDCl₃) δ [ppm]= 1.62 (s, 9H), 6.62 - 6.64 (dd, 1H), 7.00 - 7.06 (dd, 1H), 7.15 - 7.19 (dd, 1H), 10.86 (br s, 1H).

### Intermediat 24

### tert-Butyl-(5RS)-3-oxo-2,3,5,6,7,8-hexahydro[1,2,4]triazolo[4,3-a]pyridin-5-carboxylat (Racemat)

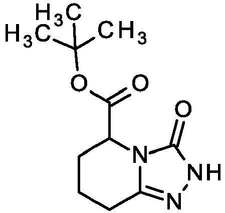

tert-Butyl-3-oxo-2,3-dihydro[1,2,4]triazolo[4,3-a]pyridin-5-carboxylat (34.5 g, 0.15 mol) und Palladium auf Kohle (6.90 g, 5%ig) wurden in Toluol (60 ml) und Methanol (500 ml) suspendiert und für 24 h bei Raumtemperatur in einer Wasserstoffatmosphäre (34.5 bar) gerührt. Das Reaktionsgemisch wurde über Kieselgur filtriert und mit Dichlormethan/Methanol 9/1 gewaschen. Das Filtrat wurde eingeengt und der Rückstand wurde mit Ethylacetat für 30 min gerührt, dann filtriert und getrocknet. Es wurden 36.8 g (89 % d. Th.) der Titelverbindung erhalten.
LC-MS (Methode 7): Rₜ = 1.28 min; MS (ESIpos): m/z = 240 [M+H]⁺
¹H-NMR (400 MHz, CDCl₃) δ [ppm]= 0.53 (s, 9H), 0.74 - 0.91 (m, 1H), 1.01 - 1.13 (m, 1H), 1.21 - 1.32 (m, 1H), 1.47 - 1.56 (m, 1H), 1.88 - 2.00 (m, 1H), 2.17 - 2.26 (dt, 1H), 4.29 - 4.34 (dd, 1H), 11.9 (br s, 1H).

### Intermediat 25

### tert-Butyl-(5RS)-3-oxo-2,3,5,6,7,8-hexahydro[1,2,4]triazolo[4,3-a]pyridin-5-carboxylat (Enantiomer 2)

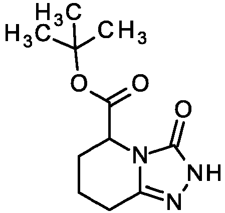

### tert-Butyl-(5RS)-3-oxo-2,3,5,6,7,8-hexahydro[1,2,4]triazolo[4,3-a]pyridin-5-carboxylat

(Racemat) wurde durch chirale präparative SFC getrennt [Probenvorbereitung: 6.00 g gelöst in 88 ml Methanol; Injektionsvolumen: 4.5 ml; Säule: Daicel Chiralpak^{®} IC 20 µm, 370 x 50 mm; Laufmittel: CO₂/i-Propanol: 0.0 min 35% i-Propanol, Fluß: 200 g/min; 14.0 min 35% isoPropanol, Fluß: 200 g/min;15.0 min 60% i-Propanol, Fluß: 115 g/min; 37.0 min 60% i-Propanol, Fluß: 115 g/min; 38.0 min, 35% i-Propanol, Fluß: 200 g/min; 42.0 min, 35% i-Propanol, Fluß: 200 g/min; Temperature 38°C; UV Detektion: 210 nm]. Nach der Trennung, wurden 2.78 g von Enantiomer 1, welches zuerst eluierte, und 2.79 g von Enantiomer 2, welches später eluierte, isoliert.

### Enantiomer 2

Analytische chirale SFC: Rₜ = 4.20 min, d.e. = 100% [Säule: Daicel Chiralpak^{®} IC 50 x 4.6 mm; Laufmittel: CO₂/i-Propanol 70:30; Fluß: 3 ml/min; UV Detektion: 210 nm].
LC-MS (Methode 4): Rₜ = 0.60 min; MS (ESIpos): m/z = 239 [M+H]⁺
¹H-NMR (400 MHz, DMSO-d6) δ [ppm]= 1.41 (s, 9H), 1.49 (dq, 1H), 1.72 - 1.84 (m, 1H), 1.95 - 2.10 (m, 2H), 2.46 - 2.56 (m, 1H), 2.59 - 2.68 (m, 1H), 4.35 (t, 1H), 11.36 (s, 1H).

Die (5S)-Konfiguration wurde aufgrund der Kristallstrukturaufklärung für Beispiel 26, 108, 113, 157, 237, 358 und 454 zugeordnet.

### tert-Butyl-(5S)-3-oxo-2,3,5,6,7,8-hexahydro[1,2,4]triazolo[4,3-a]pyridin-5-carboxylat

### Intermediat 26

### tert-Butyl-(5RS)3-oxo-2,3,5,6,7,8-hexahydro[1,2,4]triazolo[4,3-a]pyridin-5-carboxylat (Enantiomer 1)

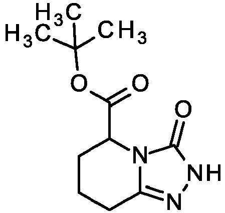

### tert-Butyl-(5RS)-3-oxo-2,3,5,6,7,8-hexahydro[1,2,4]triazolo[4,3-a]pyridin-5-carboxylat

(Racemat) wurde durch chirale präparative SFC getrennt [Probenvorbereitung: 6.00 g gelöst in 88 ml Methanol; Injektionsvolumen: 4.5 ml; Säule: Daicel Chiralpak^{®} IC 20 µm, 370 x 50 mm; Laufmittel: CO₂/i-Propanol: 0.0 min 35% i-Propanol, Fluß: 200 g/min; 14.0 min 35% isoPropanol, Fluß: 200 g/min;15.0 min 60% i-Propanol, Fluß: 115 g/min; 37.0 min 60% i-Propanol, Fluß: 115 g/min; 38.0 min, 35% i-Propanol, Fluß: 200 g/min; 42.0 min, 35% i-Propanol, Fluß: 200 g/min; Temperature 38°C; UV Detektion: 210 nm]. Nach der Trennung, wurden 2.78 g von Enantiomer 1, welches zuerst eluierte, und 2.79 g von Enantiomer 2, welches später eluierte, isoliert.

### Enantiomer 1

Analytische chirale SFC: Rₜ = 1.67 min, d.e. = 100% [Säule: Daicel Chiralpak^{®} IC 50 x 4.6 mm; Laufmittel: CO₂/i-Propanol 70:30; Fluß: 3 ml/min; UV Detektion: 210 nm].
LC-MS (Methode 4): Rₜ = 0.60 min; MS (ESIpos): m/z = 239 [M+H]⁺
¹H-NMR (400 MHz, DMSO-d6) δ [ppm]= 1.41 (s, 9H), 1.49 (dq, 1H), 1.81 (dt, 1H), 2.00 - 2.10 (m, 2H), 2.45 - 2.56 (m, 1H), 2.58 - 2.67 (m, 1H), 4.35 (t, 1H), 11.36 (s, 1H).

### Intermediat 27

### tert-Butyl-6-chlor-4-methylpyridin-2-carboxylat

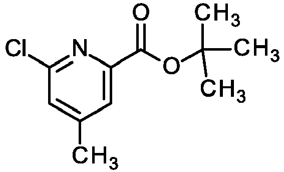

6-Chlor-4-methylpyridin-2-carbonsäure (2.50 g, 14.6 mmol) wurde in Pyridin (10 ml) und tert-Butanol (50 ml, 520 mmol) gelöst und mit 4-Methylbenzolsulfonylchlorid (5.56 g, 29.1 mmol) versetzt. Das Reaktionsgemisch wurde über Nacht bei Raumtemperatur gerührt und mit gesättigter wässriger Natriumhydrogencarbonat-Lösung und Cyclohexan/Ethylacetat 90/10 verdünnt. Die organische Phase wurde abgetrennt und die wässrige Phase mit Cyclohexan/Ethylacetat 90/10 extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 2.89 g (87 % Reinheit, 75 % d. Th.) der Titelverbindung erhalten.

¹H-NMR (400 MHz, DMSO-d6) δ [ppm]: 1.325 (0.86), 1.340 (0.86), 1.553 (16.00), 7.614 (0.68), 7.830 (0.80).

### Intermediat 28

### tert-Butyl-6-{1-[(benzyloxy)carbonyl]hydrazino}-4-methylpyridin-2-carboxylat

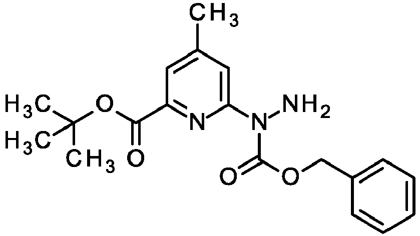

Unter Argon wurde tert-Butyl-6-chlor-4-methylpyridin-2-carboxylat (2.89 g, 12.7 mmol) und Benzyl-hydrazincarboxylat (2.32 g, 14.0 mmol) in Toluol (32 ml) gelöst und 1,1'-Bis(diphenylphosphino)ferrocen (657 mg, 635 µmol), Tris(dibenzylidenaceton)dipalladium-Chloroform-Komplex (704 mg, 1.27 mmol) und Cäsiumcarbonat (4.96 g, 15.2 mmol) zugegeben. Das Reaktionsgemisch wurde 4 h bei 80°C gerührt und dann mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Magnesiumsulfat getrocknet, filtriert und das Filtrat eingeengt. Der Rückstand wurde über Säulenchromatographie aufgereinigt (Kieselgel; Laufmittel: isokratisch, Methanol/Dichlormethan 4/96). Die produkthaltigen Fraktionen wurden im Vakuum eingeengt und es wurden 4.25 g (86 % Reinheit, 81 % d. Th.) der Titelverbindung erhalten.
LC-MS (Methode 3): Rₜ = 1.96 min; MS (ESlpos): m/z = 358 [M+H]⁺
¹H-NMR (400 MHz, DMSO-d6) δ [ppm]: 1.317 (0.87), 1.329 (0.87), 1.549 (16.00), 2.387 (5.39), 5.192 (4.02), 5.277 (3.65), 7.314 (0.79), 7.331 (1.05), 7.344 (1.30), 7.360 (1.75), 7.380 (0.82), 7.421 (2.12), 7.440 (1.40), 7.618 (1.55), 7.649 (1.81).

### Intermediat 29

### tert-Butyl-7-methyl-3-oxo-2,3-dihydro[1,2,4]triazolo[4,3-a]pyridin-5-carboxylat

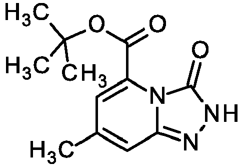

Unter Argon wurde Palladium auf Kohle (299 mg, 10%) vorgelegt und tert-Butyl-6-{1-[(benzyloxy)carbonyl]hydrazino}-4-methylpyridin-2-carboxylat (1.80 g, 5.04 mmol) in Methanol (20 ml) zugegeben. Das Reaktionsgemisch wurde über Nacht bei Raumtemperatur in einer Wasserstoffatmosphäre (1 bar) gerührt. Die Suspension wurde mit Methanol verdünnt, über Kieselgur filtriert und mit Methanol nachgewaschen. Das Reaktionsgemisch wurde eingeengt und direkt weiter umgesetzt.

Der Rückstand wurde in THF (40 ml) gelöst und mit Di-1H-imidazol-1-ylmethanon (980 mg, 6.04 mmol) versetzt. Das Reaktionsgemisch wurde für 30 min bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 1.05 g (90 % Reinheit, 75 % d. Th.) der Titelverbindung erhalten.
LC-MS (Methode 1): Rₜ = 0.98 min; MS (ESlneg): m/z = 248 [M-H]⁻
¹H-NMR (400 MHz, DMSO-d6) δ [ppm]: 1.536 (16.00), 1.553 (0.97), 2.218 (3.39), 3.318 (0.65), 6.597 (1.20), 7.051 (0.96), 12.345 (0.65).

### Intermediat 30

### tert-Butyl-(5RS,7RS)-7-methyl-3-oxo-2,3,5,6,7,8-hexahydro[1,2,4]triazolo[4,3-a]pyridin-5-carboxylat (Diastereomerengemisch; 4 Isomere)

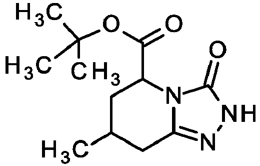

Unter Argon wurde Palladium auf Kohle (740 mg, 10%) vorgelegt und tert-Butyl-7-methyl-3-oxo-2,3-dihydro[1,2,4]triazolo[4,3-a]pyridin-5-carboxylat (3.1 g, 12.44 mmol) in Methanol (124 ml) zugegeben. Das Reaktionsgemisch wurde über Nacht bei Raumtemperatur in einer Wasserstoffatmosphäre (1 bar) gerührt. Die Suspension wurde mit Methanol verdünnt und über Kieselgur filtriert und mit Methanol nachgewaschen. Das Lösungsmittel wurde unter Vakuum entfernt. Der Rückstand wurde in Methanol (124 ml) aufgenommen und mit Palladium auf Kohle (740 mg, 10%) versetzt. Das Reaktionsgemisch wurde für 48 h bei Raumtemperatur in einer Wasserstoffatmosphäre (2 bar) gerührt. Die Suspension wurde mit Methanol verdünnt und über Kieselgur filtriert und mit Methanol nachgewaschen. Das Lösungsmittel wurde unter Vakuum entfernt und es wurden 2.71 g (86 % d. Th.) der Titelverbindung erhalten.
LC-MS (Methode 3): Rₜ = 1.24 min; MS (ESIpos): m/z = 507 [2M+H]⁺
¹H-NMR (400 MHz, DMSO-d6) δ [ppm]: 1.015 (2.13), 1.032 (2.20), 1.400 (0.73), 1.405 (1.16), 1.416 (16.00), 1.432 (0.48), 2.181 (0.46), 2.209 (0.56), 4.184 (0.45), 4.195 (0.43), 11.342 (0.73).

### Intermediat 31

### Ethyl-6-{1-[(benzyloxy)carbonyl]hydrazino}-3-methylpyridin-2-carboxylat

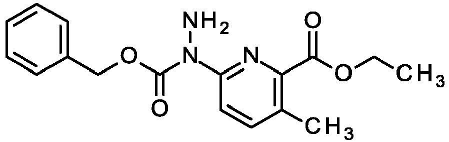

Unter Argon wurden Ethyl-6-chlor-3-methylpyridin-2-carboxylat (970 mg, 4.86 mmol) und Benzyl-hydrazincarboxylat (969 mg, 5.83 mmol) in Toluol (9.7 ml) gelöst, und Tris(dibenzylidenaceton)dipalladium-Chloroform-Komplex (251 mg, 243 µmol), 1,1'-Bis(diphenylphosphino)ferrocen (269 mg, 486 µmol) und Cäsiumcarbonat (1.90 g, 5.83 mmol) zugegeben. Das Reaktionsgemisch wurde 4 h bei 80°C gerührt und dann mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Magnesiumsulfat getrocknet, filtriert und das Filtrat eingeengt. Der Rückstand wurde über Säulenchromatographie aufgereinigt (SiO₂; Laufmittel: isokratisch, Methanol/Dichlormethan 8/92). Die produkthaltigen Fraktionen wurden im Vakuum eingeengt und es wurden 1.89 g (47 % Reinheit, 55 % d. Th.) der Titelverbindung erhalten.
LC-MS (Methode 4): Rt = 0.89 min; MS (ESlpos): m/z = 330 [M+H]⁺

### Intermediat 32

### Ethyl-6-hydrazino-3-methylpyridin-2-carboxylatdihydrochlorid

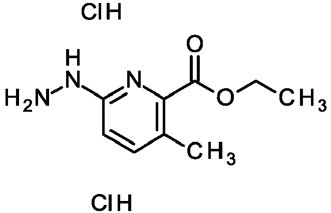

Unter Argon wurde Palladium auf Kohle (232 mg, 10%) vorgelegt und Ethyl-6-{1-[(benzyloxy)carbonyl]hydrazino}-3-methylpyridin-2-carboxylat (1.89 g, 5.74 mmol) in Methanol (60 ml) zugegeben. Das Reaktionsgemisch wurde über Nacht bei Raumtemperatur in einer Wasserstoffatmosphäre (1 bar) gerührt. Die Suspension wurde mit Methanol verdünnt und über Kieselgur filtriert und mit Methanol nachgewaschen. Das Lösungsmittel wurde unter Vakuum entfernt und der Rückstand wurde in Dioxan aufgenommen und mit Dioxan/Salzsäure (4M) versetzt. Der ausgefallene Feststoff wurde filtriert und mit Methyl-tert-butylether gewaschen und direkt weiter umgesetzt.

### Intermediat 33

### Ethyl-6-methyl-3-oxo-2,3-dihydro[1,2,4]triazolo[4,3-a]pyridin-5-carboxylat

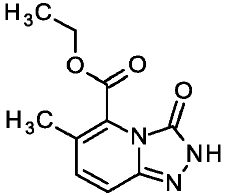

Ethyl-6-hydrazino-3-methylpyridin-2-carboxylatdihydrochlorid (419 mg, 1.56 mmol) wurde in THF (12 ml) aufgenommen und mit Di-1H-imidazol-1-ylmethanon (261 mg, 1.61 mmol) versetzt. Das Reaktionsgemisch wurde für 2 h bei Raumtemperatur gerührt. Das Reaktionsgemisch wurde mit Wasser und Dichlormethan versetzt und mit gesättigter wässriger Natriumhydrogencarbonat-Lösung basisch gestellt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Magnesiumsulfat getrocknet, filtriert und das Filtrat eingeengt. Es wurden 347 mg (89 % Reinheit, 104 % d. Th.) der Titelverbindung erhalten.
LC-MS (Methode 1): Rₜ = 0.77 min; MS (ESlneg): m/z = 220 [M-H]⁻
¹H-NMR (400 MHz, DMSO-d6) δ [ppm]: 1.282 (5.00), 1.299 (10.60), 1.317 (5.79), 1.335 (0.84), 1.356 (2.69), 2.078 (16.00), 2.118 (0.45), 2.280 (0.45), 2.395 (0.71), 2.428 (0.75), 4.331 (1.67), 4.349 (4.92), 4.367 (4.80), 4.385 (1.58), 7.060 (2.57), 7.084 (3.35), 7.239 (3.24), 7.263 (2.51), 7.632 (0.45), 12.533 (1.43).

### Intermediat 34

### Ethyl-(5RS,6RS)-6-methyl-3-oxo-2,3,5,6,7,8-hexahydro[1,2,4]triazolo[4,3-a]pyridin-5-carboxylat (Diastereomerengemisch; 4 Isomere)

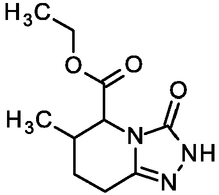

Unter Argon wurde Palladium auf Kohle (63 mg, 10%) vorgelegt und Ethyl-6-methyl-3-oxo-2,3-dihydro[1,2,4]triazolo[4,3-a]pyridin-5-carboxylat (297 mg, 1.34 mmol) in Methanol (10 ml) zugegeben, das Reaktionsgemisch wurde über Nacht bei Raumtemperatur in einer Wasserstoffatmosphäre (1 bar) gerührt. Die Suspension wurde mit Methanol verdünnt und über Kieselgur filtriert und mit Methanol nachgewaschen. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt.

Der Rückstand wurde in Methanol (5.0 ml) gelöst und nach Zugabe von Palladium auf Kohle (25 mg, 10%) wurde das Reaktionsgemisch über Nacht bei Raumtemperatur in einer Wasserstoffatmosphäre (1 bar) gerührt. Die Suspension wurde mit Methanol verdünnt und über Kieselgur filtriert und mit Methanol nachgewaschen. Das Lösungsmittel wurde unter Vakuum entfernt und es wurden 117 mg (39 % d. Th.) der Titelverbindung erhalten.
LC-MS (Methode 3): Rₜ = 0.93 min; MS (ESIpos): m/z = 226 [M+H]⁺
¹H-NMR (400 MHz, DMSO-d6) δ [ppm]: 1.015 (11.82), 1.033 (12.24), 1.175 (7.82), 1.193 (16.00), 1.211 (8.16), 1.236 (0.40), 1.428 (0.40), 1.441 (0.50), 1.459 (1.16), 1.473 (1.29), 1.492 (1.47), 1.505 (1.43), 1.522 (0.71), 1.536 (0.62), 1.691 (1.35), 1.698 (1.45), 1.706 (1.50), 1.717 (0.89), 1.724 (1.18), 1.732 (1.11), 1.739 (1.07), 2.246 (0.67), 2.252 (0.78), 2.263 (1.08), 2.269 (1.18), 2.278 (1.20), 2.284 (1.18), 2.292 (1.12), 2.299 (1.03), 2.309 (0.72), 2.316 (0.63), 2.557 (1.30), 2.569 (1.98), 2.584 (1.68), 2.600 (1.66), 2.615 (1.39), 2.656 (1.71), 2.661 (2.10), 2.669 (2.15), 2.675 (1.92), 2.698 (0.96), 2.703 (1.08), 2.711 (1.03), 2.717 (0.80), 4.098 (0.91), 4.107 (1.22), 4.116 (1.20), 4.125 (4.30), 4.134 (1.05), 4.142 (6.35), 4.151 (1.07), 4.160 (4.32), 4.169 (1.26), 4.178 (1.27), 4.187 (0.95), 4.419 (4.25), 4.435 (4.22), 11.412 (1.99).

### Intermediat 35

### Ethyl-6-hydrazino-4-(trifluormethyl)pyridin-2-carboxylat

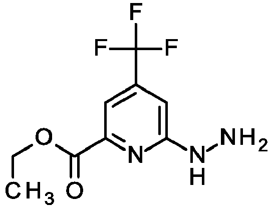

Unter Argon wurde Ethyl-6-chlor-4-(trifluormethyl)pyridin-2-carboxylat (1000 mg, 3.94 mmol) und Benzyl-hydrazincarboxylat (721 mg, 4.34 mmol) in Toluol (10 ml) gelöst, und Tris(dibenzylidenaceton)dipalladium-Chloroform-Komplex (204 mg, 197 µmol), 1,1'-Bis(diphenylphosphino)ferrocen (219 mg, 394 µmol) und Cäsiumcarbonat (1.54 g, 4.73 mmol) zugegeben. Das Reaktionsgemisch wurde 4 h bei 80°C gerührt und mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Magnesiumsulfat getrocknet, filtriert und das Filtrat eingeengt. Der Rückstand wurde über Säulenchromatographie aufgereinigt (SiO₂; Laufmittel: isokratisch, Methanol/Dichlormethan 8/92). Die produkthaltigen Fraktionen wurden im Vakuum eingeengt und der erhaltene Rückstand wurde direkt weiter umgesetzt. Der Rückstand wurde unter Argon in Methanol (50 ml) gelöst und Palladium auf Kohle (223 mg, 10%) wurde zugegeben. Das Reaktionsgemisch wurde über Nacht bei Raumtemperatur in einer Wasserstoffatmosphäre (1 bar) gerührt. Es wurde weiteres Palladium auf Kohle (223 mg, 10%) zugegeben und über Nacht bei Raumtemperatur in einer Wasserstoffatmosphäre (1 bar) gerührt. Die Suspension wurde mit Methanol verdünnt, über Kieselgur filtriert und mit Methanol nachgewaschen. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 385 mg (93 % Reinheit, 38 % d. Th.) der Titelverbindung erhalten.
LC-MS (Methode 1): Rₜ = 0.91 min; MS (ESIpos): m/z = 250 [M+H]⁺

### Intermediat 36

### Ethyl-3-oxo-7-(trifluormethyl)-2,3-dihydro[1,2,4]triazolo[4,3-a]pyridin-5-carboxylat

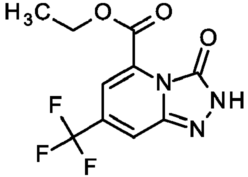

Ethyl-6-hydrazino-4-(trifluormethyl)pyridin-2-carboxylat (384 mg, 1.54 mmol) wurde in THF (19 ml) vorgelegt, mit Di-1H-imidazol-1-ylmethanon (300 mg, 1.85 mmol) versetzt und über Nacht bei Raumtemperatur gerührt. Das Reaktionsgemisch wurde mit Wasser und Dichlormethan versetzt. Die organische Phase wurde abgetrennt und die wässrige Phase dreimal mit Dichlormethan extrahiert. Die vereinigten organischen Phasen wurden mit Wasser, gesättigter wässriger Natriumchlorid-Lösung gewaschen, über Magnesiumsulfat getrocknet, filtriert und das Filtrat eingeengt. Es wurden 391 mg (94 % Reinheit, 87 % d. Th.) der Titelverbindung erhalten.
LC-MS (Methode 4): Rₜ = 0.71 min; MS (ESIpos): m/z = 276 [M+H]⁺
¹H-NMR (400 MHz, DMSO-d6) δ [ppm]: 1.299 (7.46), 1.316 (16.00), 1.334 (7.58), 1.345 (0.42), 1.357 (1.20), 1.363 (0.66), 2.525 (0.47), 4.336 (2.37), 4.354 (7.32), 4.371 (7.27), 4.389 (2.27), 7.044 (4.79), 7.048 (4.79), 7.954 (2.71), 7.957 (3.98), 7.960 (2.90).

### Intermediat 37

### Ethyl-(5RS,7RS)-3-oxo-7-(trifluormethyl)-2,3,5,6,7,8-hexahydro[1,2,4]triazolo[4,3-a]pyridin-5-carboxylat (Diastereomerengemisch; 4 Isomere)

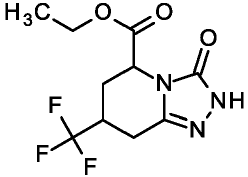

Unter Argon wurde Palladium auf Kohle (30 mg, 10%) vorgelegt und Ethyl-3-oxo-7-(trifluormethyl)-2,3-dihydro[1,2,4]triazolo[4,3-a]pyridin-5-carboxylat (100 mg, 363 µmol) in Methanol (5.0 ml) zugegeben. Das Reaktionsgemisch wurde 40 h bei Raumtemperatur in einer Wasserstoffatmosphäre (1 bar) gerührt. Die Suspension wurde mit Methanol verdünnt und über Kieselgur filtriert und mit Methanol nachgewaschen. Das Lösungsmittel wurde unter Vakuum entfernt und es wurden 97.8 mg (87 % Reinheit, 84 % d. Th.) der Titelverbindung erhalten.
LC-MS (Methode 4): Rₜ = 0.64 min; MS (ESIpos): m/z = 280 [M+H]⁺
¹H-NMR (500 MHz, DMSO-d6) δ [ppm]: 1.042 (1.06), 1.056 (2.04), 1.070 (1.10), 1.199 (8.42), 1.214 (16.00), 1.228 (8.00), 1.305 (0.45), 1.356 (1.36), 1.738 (1.02), 1.763 (2.47), 1.787 (2.58), 1.812 (1.20), 2.696 (1.56), 2.721 (2.08), 2.728 (2.47), 2.752 (2.57), 2.869 (2.23), 2.896 (1.50), 2.901 (1.51), 3.057 (1.19), 3.064 (1.13), 3.072 (1.13), 3.165 (1.54), 3.174 (1.56), 3.429 (0.48), 3.439 (0.55), 3.452 (0.48), 4.141 (0.78), 4.149 (1.47), 4.156 (2.22), 4.163 (4.00), 4.170 (4.33), 4.177 (4.39), 4.184 (3.95), 4.191 (2.27), 4.198 (1.47), 4.205 (0.83), 4.319 (0.42), 4.331 (0.60), 4.432 (2.24), 4.444 (2.52), 4.455 (2.47), 4.466 (2.20), 7.628 (0.41), 11.601 (3.48).

### Intermediat 38

### Methyl-(2RS)-6-[(4-methoxybenzyl)(methoxycarbonyl)hydrazono]piperidin-2-carboxylat (Racemat)

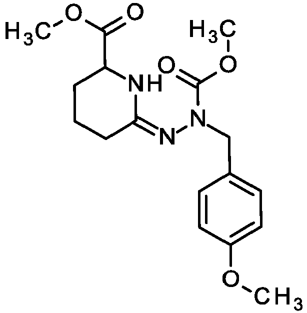

Zu Methyl-(2RS)-6-oxopiperidin-2-carboxylat (Racemat) (685 mg, 4.36 mmol) wurde in Dichlormethan (15 ml) unter Argon Trimethyloxoniumtetrafluoroborat (678 mg, 95 % Reinheit, 4.36 mmol) gegeben und das Reaktionsgemisch über Nacht bei Raumtemperatur gerührt. Sodann wurde Methyl-1-(4-methoxybenzyl)hydrazincarboxylat (1.29 g, 71 % Reinheit, 4.36 mmol) zugegeben und der Ansatz mehrere Tage unter gelegentlicher Umsatzkontrolle bei Raumtemperatur gerührt. Zur Aufarbeitung wurden die flüchtigen Anteile im Vakuum entfernt. Das verbleibende Rohprodukt (1.52 g, 100% d. Th.) wurde ohne zusätzliche Reinigung weiter umgesetzt.
LC-MS (Methode 4): Rₜ = 0.51 min; MS (ESIpos): m/z = 350 [M+H]⁺

### Intermediat 39

### Methyl-(5RS)-2-(4-methoxybenzyl)-3-oxo-2,3,5,6,7,8-hexahydro[1,2,4]triazolo[4,3-a]pyridin-5-carboxylat (Racemat)

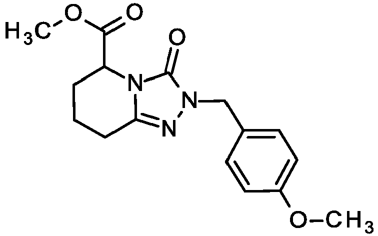

### Methyl-(2RS)-6-[(4-methoxybenzyl)(methoxycarbonyl)hydrazono]piperidin-2-carboxylat

(Racemat) (1.52 g, 4.35 mmol) in DMF (5 ml) wurden bei 150°C über Nacht gerührt. Zur Aufarbeitung wurde der abgekühlte Ansatz auf etwa 50 ml Wasser gegeben, die Mischung mit wässriger Natronlauge auf etwa pH 9 alkalisch gestellt und dreimal mit 50 ml Dichlormethan extrahiert. Die vereinigten organischen Phasen wurden mit gesättigter wässriger Natriumchlorid-Lösung gewaschen, über Magnesiumsulfat getrocknet, filtriert und im Vakuum eingeengt. Das so erhaltene Rohprodukt wurde durch Chromatographie an Kieselgel gereinigt (PuriFlash 50g Kieselgelkartusche, Dichlormethan 100% => Dichlormethan: MeOH 80:1). So wurden zwei Fraktionen der Titelverbindung erhalten: 327 mg in 60% Reinheit (14 % d. Th.) und 858 mg in 72% Reinheit (45% d. Th.).

### Analytik-Ergebnisse der zweiten Fraktion:

LC-MS (Methode 6): Rₜ = 1.33 min; MS (ESIpos): m/z = 318 [M+H]⁺
¹H-NMR (400 MHz, DMSO-d6) δ [ppm]: 2.615 (0.90), 2.731 (9.35), 2.890 (12.58), 3.700 (13.65), 3.730 (16.00), 3.736 (6.83), 3.742 (2.33), 4.492 (1.54), 4.582 (0.85), 4.591 (0.94), 4.597 (1.08), 4.607 (0.82), 4.750 (5.88), 6.882 (3.29), 6.887 (2.11), 6.899 (1.66), 6.904 (4.11), 6.908 (1.73), 7.162 (3.09), 7.167 (1.04), 7.178 (1.35), 7.183 (3.40), 7.203 (1.01), 7.923 (0.79), 7.952 (1.25).

### Intermediat 40

### (5RS)-2-(4-Methoxybenzyl)-3-oxo-2,3,5,6,7,8-hexahydro[1,2,4]triazolo[4,3-a]pyridin-5-carbonsäure (Racemat)

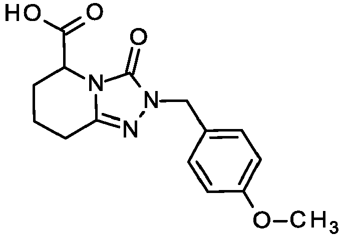

Methyl-(5RS)-2-(4-methoxybenzyl)-3-oxo-2,3,5,6,7,8-hexahydro[1,2,4]triazolo[4,3-a]pyridin-5-carboxylat (Racemat) (320 mg, 60 % Reinheit, 605 µmol) wurden in Wasser/THF (5 ml /5 ml) gelöst, mit Lithiumhydroxid (72.4 mg, 3.03 mmol) versetzt und die Reaktionsmischung über ein Wochenende bei Raumtemperatur gerührt. Zur Aufarbeitung wurde mit Wasser versetzt, mit 1N wässriger Salzsäure auf pH 3 gestellt, mit Natriumchlorid gesättigt und dreimal mit je 30 ml Ethylacetat extrahiert. Die vereinigten organischen Phasen wurden mit Magnesiumsulfat getrocknet, filtriert und im Vakuum eingeengt und getrocknet. Es wurden 250mg (71% Reinheit, 97 % d. Th.) der Titelverbindung erhalten. Das so erhaltene Produkt wurde ohne zusätzliche Reinigung weiter verwendet.

In einem zweiten, analog durchgeführten Ansatz wurden aus 850 mg Methyl-(5RS)-2-(4-methoxybenzyl)-3-oxo-2,3,5,6,7,8-hexahydro[1,2,4]triazolo[4,3-a]pyridin-5-carboxylat (72 % Reinheit, 1.93 mmol) 625 mg der Titelverbindung erhalten (89 % Reinheit, 95 % d. Th.).
LC-MS (Methode 3): Rₜ = 1.00 min; MS (ESIpos): m/z = 304 [M+H]⁺

### Intermediat 41

### (5RS)-5-(Pyrrolidin-1-ylcarbonyl)-5,6,7,8-tetrahydro[1,2,4]triazolo[4,3-a]pyridin-3(2H)-on (Racemat)

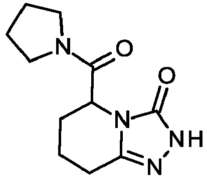

### Variante a)

(5RS)-2-(4-Methoxybenzyl)-5-(pyrrolidin-1-ylcarbonyl)-5,6,7,8-tetrahydro[1,2,4]triazolo[4,3-a]pyridin-3(2H)-on (Racemat) (670 mg, 1.88 mmol) wurde in Trifluoressigsäure (15 ml ) gelöst und in einer Mikrowellenapparatur bei 150°C für 1h gerührt. Zur Aufarbeitung wurde der abgekühlte Ansatz eingeengt und der Rückstand in 2 Injektionen über präparative HPLC gereinigt (Säule: Chromatorex C18 , 250x40mm 10µm. Flow: 50ml/min. Gradient ( A= Wasser + 0,1% Ameisensäure , B=Acetonitril): 0min 10% B, 6min 10% B, 27min 95% B, 38min 95 % B , 40min 10% B. Laufzeit pro Trennung 40min. Detektion: 210 nm). Produktfraktionen wurden eingeengt und lyophilisiert. So wurden 0.36 g (80 % d.Th.) der Titelverbindung erhalten.

### Variante b)

Methyl-(5RS)-3-oxo-2,3,5,6,7,8-hexahydro[1,2,4]triazolo[4,3-a]pyridin-5-carboxylat (Racemat) (500 mg, 2.54 mmol) wurde im Gemisch mit Pyrrolidin (5.0 ml, 60 mmol) zunächst für 2h, dann weitere 18h bei 50 °C gerührt, anschließend weitere etwa 24h bei 60°C (Umsatzkontrollen mit HPLC / LC/MS). Zur Aufarbeitung wurde der Ansatz mit Wasser versetzt, mit Ethylacetat verdünnt und extrahiert. Die wässrige Phase wurde noch zweimal mit Ethylacetat extrahiert. Die organischen Phasen wurden nach LC/MS-Kontrolle verworfen. Die produkthaltige wässrige Phase wurde eingeengt und im Vakuum getrocknet. Durch Verreiben des Rückstands mit Ethylacetat/Methanol, Einengen der Mutterlauge und erneutes Verreiben mit dem Lösungsmittelgemisch wurden zwei Fraktionen der Titelverbindung erhalten: 45.7 mg (8%) und 112 mg (19%).

### Analytikdaten der zweiten Fraktion:

LC-MS (Methode 6): Rₜ = 0.80 min; MS (ESIpos): m/z = 237 [M+H]⁺
¹H-NMR (500 MHz, DMSO-d6) δ [ppm]: 1.147 (0.62), 1.662 (1.69), 1.673 (2.39), 1.682 (4.36), 1.690 (6.79), 1.700 (8.57), 1.710 (6.71), 1.720 (3.20), 1.757 (2.60), 1.771 (9.56), 1.784 (16.00), 1.798 (11.83), 1.812 (3.41), 1.894 (3.39), 1.907 (11.19), 1.921 (15.40), 1.935 (10.59), 1.948 (4.95), 1.956 (5.06), 1.965 (4.50), 1.972 (2.37), 1.978 (2.52), 1.990 (3.30), 1.999 (3.57), 2.007 (3.10), 2.019 (2.87), 2.036 (1.28), 2.049 (0.83), 2.472 (2.42), 2.518 (5.78), 2.524 (4.83), 2.571 (3.49), 2.580 (6.69), 2.590 (3.51), 2.604 (1.94), 2.613 (3.22), 2.623 (1.57), 2.636 (0.62), 3.013 (0.68), 3.027 (1.51), 3.041 (0.64), 3.225 (2.19), 3.238 (4.42), 3.248 (4.27), 3.262 (7.12), 3.276 (3.96), 3.288 (5.24), 3.324 (12.41), 3.333 (4.62), 3.338 (5.10), 3.347 (4.83), 3.362 (2.48), 3.375 (0.68), 3.437 (2.33), 3.451 (4.93), 3.457 (3.84), 3.465 (3.28), 3.471 (6.13), 3.485 (2.81), 3.579 (2.85), 3.593 (5.88), 3.599 (3.34), 3.606 (3.61), 3.612 (4.77), 3.626 (2.17), 4.637 (5.45), 4.644 (6.46), 4.650 (7.06), 4.657 (5.49), 11.270 (6.83).

### Intermediat 42

### Ethyl-2-isopropyliden-1-(4-methylbenzyl)hydrazincarboxylat

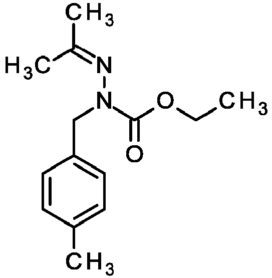

Zu Ethyl-2-isopropylidenhydrazincarboxylat (CAS 6637-60-1; 3.23 g, 22.4 mmol) in 55ml Toluol wurden unter Argon Kaliumhydroxidpulver (4.11 g, 29.7 mmol) und Tetra-n-butylammoniumhydrogensulfat (734 mg, 2.16 mmol) zugegeben. Zur besseren Rührbarkeit wurden 10 ml Toluol ergänzt. Nach Erwärmen auf 50 °C wurde langsam eine Lösung aus 1-(Brommethyl)-4-methylbenzol (5.00 g, 27.0 mmol) in 15 ml Toluol zugetropft, das Gemisch auf 80°C erhitzt und 2.5h nachgerührt. Zur Aufarbeitung wurde der Ansatz mit Wasser versetzt und die organische Phase nach Extraktion abgetrennt. Die organische Phase wurde noch zweimal mit Wasser extrahiert, die vereinigten wässrigen Phasen sodann einmal mit Toluol reextrahiert. Die organischen Phasen wurde mit gesättigter wässriger Natriumchloridlösung gewaschen, über Natriumsulfat getrocknet, eingeengt und im Vakuum getrocknet. Das erhaltene Rohprodukt wurde über Kieselgel chromatographisch getrennt (Instrument: Isolera Säule: 340g SNAP Laufmittel: Cyclohexan und EtOAc Gradient: 0min Cyclohexan, 10min Cyclohexan, 35min 30% EtOAc, 40min 30% EtOAc). Produkthaltige Fraktionen wurden vereinigt, eingeengt und getrocknet. So wurden 3.76 g (56 % d. Th.) der Titelverbindung erhalten.
LC-MS (Methode 4): Rₜ = 0.96 min; MS (ESIpos): m/z = 249 [M+H]⁺

### Intermediat 43

### Ethyl-1-(4-methylbenzyl)hydrazincarboxylat

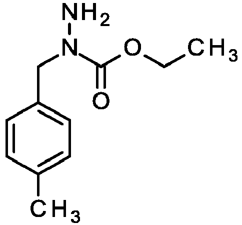

Ethyl-2-isopropyliden-1-(4-methylbenzyl)hydrazincarboxylat (3.77 g, 15.2 mmol, laut LC/MS bereits teilhydrolysiert zur Titelverbindung) wurde in Ethanol/Wasser(100 ml/66 ml) gelöst, zum Rückfluss erhitzt und 3h gerührt. Zur Aufarbeitung wurde anschließend weitestgehend eingeengt, der Rückstand in Dichlormethan/Wasser aufgenommen und extrahiert. Nach Trennung der Phasen wurde die wässrige Phase noch zweimal mit Dichlormethan extrahiert, die vereinigten organischen Phasen mit gesättigter Natriumchloridlösung gewaschen, über Natriumsulfat getrocknet, eingeengt und im Vakuum getrocknet. So wurden 3.25 g (87 % Reinheit, 89 % d. Th.) der Titelverbindung erhalten, die ohne weitere Reinigung weiter umgesetzt wurden.
LC-MS (Methode 6): Rₜ = 1.53 min; MS (ESIpos): m/z = 209 [M+H]⁺
¹H-NMR (400 MHz, DMSO-d6) δ [ppm]: 1.159 (0.80), 1.168 (1.91), 1.185 (3.86), 1.203 (1.95), 1.667 (1.33), 1.927 (1.25), 2.267 (1.24), 2.275 (9.41), 4.035 (1.18), 4.053 (3.64), 4.071 (3.59), 4.088 (1.13), 4.425 (5.48), 4.548 (3.26), 7.126 (16.00).

### Intermediat 44

### Methyl-(2RS)-6-[(ethoxycarbonyl)(4-methylbenzyl)hydrazono]piperidin-2-carboxylat (Racemat)

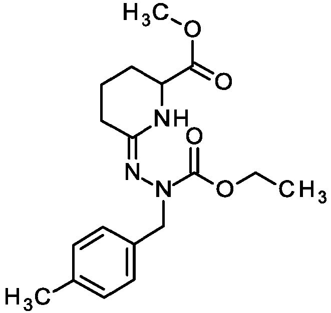

Zu Methyl-(2RS)-6-oxopiperidin-2-carboxylat (Racemat) (1.58 g, 10.1 mmol) wurde in 20 ml Dichlormethan unter Argon Trimethyloxoniumtetrafluoroborat (1.57 g, 95 % Reinheit, 10.1 mmol) gegeben und das Reaktionsgemisch 18h bei Raumtemperatur gerührt. Sodann wurde Ethyl-1-(4-methylbenzyl)hydrazincarboxylat (2.41 g, 87 % Reinheit, 10.1 mmol), gelöst in 10 ml Dichlormethan, zugetropft und 26h bei Raumtemperatur gerührt. Zur Aufarbeitung wurden die flüchtigen Anteile im Vakuum entfernt und der Rückstand über Kieselgel getrennt. (Instrument: Isolera; Säule: 100g SNAP-Katusche Laufmittel: Cyclohexan und EtOAc // EtOAc und MeOH Gradient: 0min 30% EtOAc, 3min 30% EtOAc, 13min 100% EtOAc, 18min 100% EtOAc, 18,01min 10% MeOH, 33min 10% MeOH). Produkthaltige Fraktionen wurden vereinigt, eingeengt und im Vakuum getrocknet. Man erhielt 1.46 g (88 % Reinheit, 37 % d. Th.) der Titelverbindung, die so weiter umgesetzt wurden.
LC-MS (Methode 3): Rₜ = 0.98 min; MS (ESIpos): m/z = 348 [M+H]⁺
¹H-NMR (400 MHz, DMSO-d6) δ [ppm]: 1.055 (0.58), 1.149 (1.00), 1.157 (1.01), 1.167 (1.78), 1.175 (1.73), 1.184 (0.92), 1.192 (0.87), 1.644 (0.91), 1.908 (0.76), 1.988 (2.45), 2.128 (2.05), 2.141 (2.19), 2.144 (2.23), 2.148 (1.42), 2.156 (0.95), 2.164 (0.99), 2.274 (3.39), 2.279 (4.35), 3.162 (0.90), 3.175 (0.90), 3.538 (3.77), 3.578 (0.99), 3.585 (1.56), 3.638 (3.98), 3.667 (16.00), 3.675 (1.67), 3.684 (2.39), 3.701 (3.26), 4.021 (0.67), 4.038 (0.81), 4.049 (1.01), 4.056 (1.07), 4.063 (1.50), 4.069 (1.44), 4.775 (1.21), 7.127 (1.68), 7.134 (2.17), 7.174 (1.17).

### Intermediat 45

### Methyl-(5RS)-2-(4-methylbenzyl)-3-oxo-2,3,5,6,7,8-hexahydro[1,2,4]triazolo[4,3-a]pyridin-5-carboxylat (Racemat)

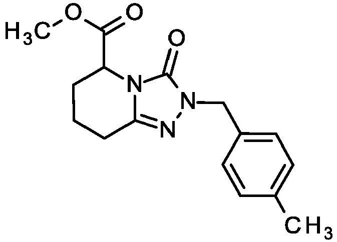

Methyl-(2RS)-6-[(ethoxycarbonyl)(4-methylbenzyl)hydrazono]piperidin-2-carboxylat (Racemat) (730 mg, 88 % Reinheit, 1.85 mmol) in 15 ml DMF wurde bei 150°C über Nacht gerührt. Zur Aufarbeitung wurde der Ansatz auf Wasser gegeben, die Mischung mit 3N Natronlauge alkalisch gestellt und mit Dichlormethan extrahiert. Die wässrige Phase wurde mit Natriumchlorid gesättigt und noch zweimal mit Dichlormethan extrahiert. Die vereinigten organischen Phasen wurden mit gesättigter wässriger Natriumchloridlösung gewaschen, über Natriumsulfat getrocknet, filtriert und im Vakuum eingeengt. Man erhielt so 288 mg (52 % d. Th.) der Titelverbindung.
LC-MS (Methode 3): Rₜ = 1.42 min; MS (ESIpos): m/z = 302 [M+H]⁺
¹H-NMR (400 MHz, DMSO-d6) δ [ppm]: 2.274 (12.04), 2.580 (0.80), 2.616 (1.19), 2.731 (10.86), 2.890 (12.92), 3.285 (1.12), 3.700 (16.00), 4.588 (1.05), 4.598 (1.19), 4.604 (1.34), 4.614 (1.04), 4.775 (6.63), 7.105 (0.94), 7.119 (1.09), 7.127 (7.35), 7.134 (6.83), 7.155 (1.02), 7.952 (1.56).

### Alternative Synthese:

Methyl-(5RS)-3-oxo-2,3,5,6,7,8-hexahydro[1,2,4]triazolo[4,3-a]pyridin-5-carboxylat (500 mg, 2.54 mmol) wurde in Acetonitril (10 ml) vorgelegt. Cäsiumcarbonat (909 mg, 2.79 mmol) und 1-(Brommethyl)-4-methylbenzol (493 mg, 2.66 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 560 mg (73 % d. Th.) der Titelverbindung erhalten.
LC-MS (Methode 1): Rₜ = 1.05 min; MS (ESIpos): m/z = 302 [M+H]⁺
¹H-NMR (400 MHz, DMSO-d6) δ [ppm]: -0.008 (0.61), 0.008 (0.56), 1.787 (0.45), 1.798 (0.49), 1.812 (0.42), 2.050 (0.42), 2.060 (0.71), 2.072 (0.83), 2.080 (0.64), 2.088 (0.52), 2.096 (0.44), 2.104 (0.52), 2.108 (0.45), 2.116 (0.53), 2.124 (0.51), 2.132 (0.48), 2.274 (11.08), 2.523 (0.63), 2.566 (0.81), 2.580 (0.64), 2.605 (0.58), 2.616 (1.09), 2.629 (0.60), 2.659 (0.49), 3.701 (16.00), 4.588 (0.99), 4.598 (1.12), 4.604 (1.28), 4.614 (0.97), 4.775 (6.33), 7.106 (0.87), 7.127 (6.84), 7.134 (6.17), 7.154 (0.82).

### Intermediat 46

### Methyl-(5RS)-2-(4-methylbenzyl)-3-oxo-2,3,5,6,7,8-hexahydro[1,2,4]triazolo[4,3-a]pyridin-5-carboxylat (Enantiomer 1)

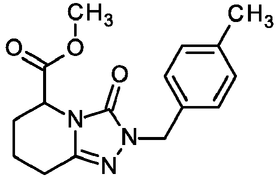

### Methyl-(5RS)-3-oxo-2,3,5,6,7,8-hexahydro[1,2,4]triazolo[4,3-a]pyridin-5-carboxylat

(Enantiomer 2) (700 mg, 3.55 mmol) wurde in Acetonitril (31 ml) vorgelegt. Cäsiumcarbonat (1.27 g, 3.90 mmol) und 1-(Brommethyl)-4-methylbenzol (690 mg, 3.73 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase zweimal mit Ethylacetat extrahiert. Die vereinigten organischen Phasen wurden mit gesättigter wässriger Natriumchlorid-Lösung gewaschen, über Magnesiumsulfat getrocknet, filtriert und das Filtrat eingeengt. Es wurden 1.05 g (91 % Reinheit, 89 % d. Th.) der Titelverbindung erhalten.
LC-MS (Methode 3): Rₜ = 1.46 min; MS (ESIpos): m/z = 302 [M+H]⁺
¹H-NMR (400 MHz, DMSO-d6) δ [ppm]: 1.497 (0.40), 1.519 (0.42), 1.524 (0.43), 1.532 (0.43), 1.788 (0.50), 1.797 (0.54), 1.811 (0.47), 1.821 (0.43), 1.988 (0.48), 2.049 (0.50), 2.060 (0.81), 2.073 (0.80), 2.080 (0.72), 2.087 (0.59), 2.095 (0.51), 2.104 (0.58), 2.115 (0.59), 2.124 (0.56), 2.131 (0.52), 2.273 (12.47), 2.292 (0.91), 2.523 (0.62), 2.566 (0.84), 2.580 (0.68), 2.604 (0.64), 2.616 (1.20), 2.628 (0.66), 2.658 (0.55), 3.310 (16.00), 3.683 (0.71), 4.588 (1.13), 4.598 (1.28), 4.604 (1.44), 4.614 (1.10), 4.674 (0.43), 4.775 (6.90), 7.106 (1.01), 7.127 (8.08), 7.133 (7.29), 7.154 (1.06).

Die (5S)-Konfiguration wurde aufgrund der Kristallstrukturaufklärung für Beispiel 26, 108, 113, 157, 237, 358 und 454 zugeordnet.

### Methyl-(5S)-2-(4-methylbenzyl)-3-oxo-2,3,5,6,7,8-hexahydro[1,2,4]triazolo[4,3-a]pyridin-5-carboxylat

### Intermediat 47

### Methyl-(5S)-3-oxo-2,5,6,7-tetrahydro-3H-pyrrolo[2,1-c][1,2,4]triazol-5-carboxylat (Enantiomer)

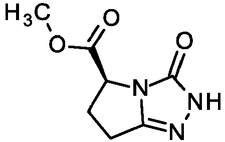

Methyl-5-oxo-L-prolinat (24.0 g, 168 mmol, CAS 4931-66-2) wurde in Dichlormethan (210 ml) bei Raumtemperatur vorgelegt. Anschließend wurde Trimethyloxoniumtetrafluoroborat (24.8 g, 168 mmol) zugegeben und das Reaktionsgemisch über Nacht bei Raumtemperatur gerührt. Das Lösungsmittel wurde im Vakuum entfernt und der Rückstand mit DMF (240 ml) versetzt. Anschließend wurde Methyl-hydrazincarboxylat (15.1 g, 168 mmol) zugegeben und das Reaktionsgemisch über Nacht bei 170 °C gerührt. Das Reaktionsgemisch wurde im Vakuum aufkonzentriert und der Rückstand wurde über Säulenchromatographie (Kieselgel, Laufmittel: Dichlormethan/Methanol-Gradient) gereinigt. Die produkthaltigen Fraktionen wurden im Vakuum eingeengt und es wurden 7.57 g (25 % d. Th.) der Titelverbindung erhalten. Diese wurde direkt weiter umgesetzt.
GC-MS (WUP-GC/MS): Rₜ = 6.48 min; MS (ESIpos): m/z = 183 [M+H]⁺

### Intermediat 48

### Methyl-(5S)-2-(4-methylbenzyl)-3-oxo-2,5,6,7-tetrahydro-3H-pyrrolo[2,1-c][1,2,4]triazol-5-carboxylat (Enantiomer)

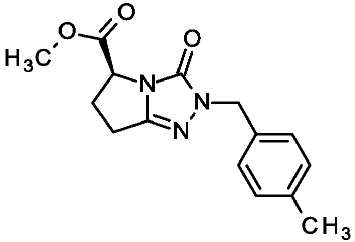

Methyl-(5S)-3-oxo-2,5,6,7-tetrahydro-3H-pyrrolo[2,1-c][1,2,4]triazol-5-carboxylat (Enantiomer) (250 mg, 1.36 mmol) wurde in Acetonitril (3.0 ml) vorgelegt. Cäsiumcarbonat (489 mg, 1.50 mmol) und 1-(Brommethyl)-4-methylbenzol (265 mg, 1.43 mmol, CAS 104-81-4) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Dichlormethan extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 412 mg (91 % Reinheit, 96 % d. Th.) der Titelverbindung erhalten.
LC-MS (Methode 3): Rₜ = 1.32 min; MS (ESIpos): m/z = 288 [M+H]⁺
¹H-NMR (400 MHz, DMSO-d6) δ [ppm]: 2.072 (1.11), 2.275 (9.70), 2.303 (0.99), 2.458 (0.51), 2.465 (0.54), 2.720 (1.21), 2.743 (1.93), 2.758 (1.66), 2.837 (0.42), 2.857 (0.41), 2.869 (0.51), 2.892 (0.49), 3.708 (1.25), 3.722 (12.20), 4.758 (2.95), 4.763 (3.01), 4.776 (1.05), 4.791 (1.07), 4.799 (0.95), 4.937 (0.40), 5.753 (0.46), 7.141 (16.00).

### Intermediat 49

### Methyl-(5S)-2-[(6-chlorpyridin-3-yl)methyl]-3-oxo-2,5,6,7-tetrahydro-3H-pyrrolo[2,1-c][1,2,4]triazol-5-carboxylat (Enantiomer)

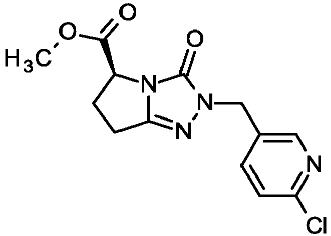

Methyl-(5S)-3-oxo-2,5,6,7-tetrahydro-3H-pyrrolo[2,1-c][1,2,4]triazol-5-carboxylat (Enantiomer) (300 mg, 1.64 mmol) wurde in Acetonitril (10 ml) vorgelegt. Nach 5 min Rühren wurden Cäsiumcarbonat (587 mg, 1.80 mmol) und 5-(Brommethyl)-2-chlorpyridin (355 mg, 1.72 mmol, CAS 182924-36-3) zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 430 mg (85 % d. Th.) der Titelverbindung erhalten.
LC-MS (Methode 1): Rₜ = 0.80 min; MS (ESIpos): m/z = 309 [M+H]⁺

### Intermediat 50

### Methyl-(5S)-3-oxo-2-{[6-(trifluormethyl)pyridin-3-yl]methyl}-2,5,6,7-tetrahydro-3H-pyrrolo[2,1-c][1,2,4]triazol-5-carboxylat (Enantiomer)

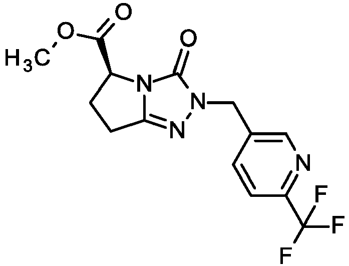

Methyl-(5S)-3-oxo-2,5,6,7-tetrahydro-3H-pyrrolo[2,1-c][1,2,4]triazol-5-carboxylat (Enantiomer) (300 mg, 1.64 mmol) wurde in Acetonitril (10 ml) vorgelegt. Cäsiumcarbonat (587 mg, 1.80 mmol) und 5-(Brommethyl)-2-(trifluormethyl)pyridin (413 mg, 1.72 mmol, CAS 108274-33-5) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 441 mg (79 % d. Th.) der Titelverbindung erhalten.
LC-MS (Methode 1): Rₜ = 0.92 min; MS (ESIpos): m/z = 343 [M+H]⁺

### Intermediat 51

### Methyl-(5S)-2-(3-chlor-4-fluorbenzyl)-3-oxo-2,5,6,7-tetrahydro-3H-pyrrolo[2,1-c][1,2,4]triazol-5-carboxylat (Enantiomer)

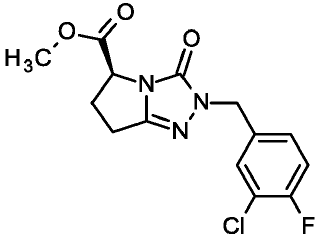

Methyl-(5S)-3-oxo-2,5,6,7-tetrahydro-3H-pyrrolo[2,1-c][1,2,4]triazol-5-carboxylat (Enantiomer) (300 mg, 1.64 mmol) wurde in Acetonitril (530 µl) vorgelegt. Nach 5 min Rühren wurden Cäsiumcarbonat (587 mg, 1.80 mmol) und 4-(Brommethyl)-2-chlor-1-fluorbenzol (384 mg, 1.72 mmol, CAS 192702-01-5) zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 460 mg (86 % d. Th.) der Titelverbindung erhalten.
LC-MS (Methode 3): Rₜ = 1.42 min; MS (ESIpos): m/z = 326 [M+H]⁺

### Intermediat 52

### Methyl-(5S)-3-oxo-2-[3-(trifluormethyl)benzyl]-2,5,6,7-tetrahydro-3H-pyrrolo[2,1-c][1,2,4]triazol-5-carboxylat (Enantiomer)

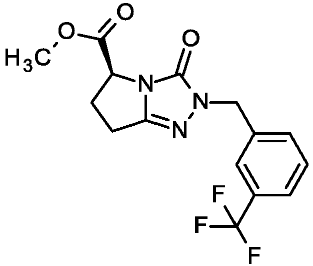

Methyl-(5S)-3-oxo-2,5,6,7-tetrahydro-3H-pyrrolo[2,1-c][1,2,4]triazol-5-carboxylat (Enantiomer) (150 mg, 819 µmol) wurde in Acetonitril (8.0 ml) vorgelegt. Nach 5 min Rühren wurden Cäsiumcarbonat (294 mg, 901 µmol) und 1-(Brommethyl)-3-(trifluormethyl)benzol (206 mg, 860 µmol, CAS 402-23-3) zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 150 mg (53 % d. Th.) der Titelverbindung erhalten.
LC-MS (Methode 1): Rₜ = 1.09 min; MS (ESIpos): m/z = 342 [M+H]⁺

### Intermediat 53

### Methyl-(5S)-2-[(2-chlorpyridin-4-yl)methyl]-3-oxo-2,5,6,7-tetrahydro-3H-pyrrolo[2,1-c][1,2,4]triazol-5-carboxylat (Enantiomer)

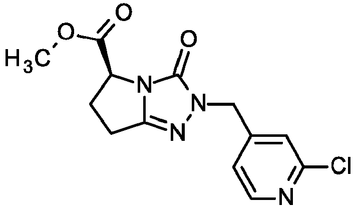

Methyl-(5S)-3-oxo-2,5,6,7-tetrahydro-3H-pyrrolo[2,1-c][1,2,4]triazol-5-carboxylat (Enantiomer) (150 mg, 819 µmol) wurde in Acetonitril (7.0 ml) vorgelegt. Nach 5 min Rühren wurden Cäsiumcarbonat (294 mg, 901 µmol) und 4-(Brommethyl)-2-chlorpyridin (178 mg, 860 µmol, CAS 83004-15-3) zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 250 mg (99 % d. Th.) der Titelverbindung erhalten.
LC-MS (Methode 1): Rₜ = 0.78 min; MS (ESIpos): m/z = 309 [M+H]⁺

### Intermediat 54

### Methyl-(5RS)-2-(4-chlorbenzyl)-3-oxo-2,3,5,6,7,8-hexahydro[1,2,4]triazolo[4,3-a]pyridin-5-carboxylat (Racemat)

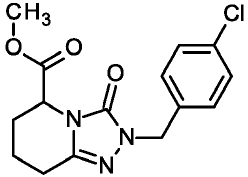

Methyl-(2RS)-6-[(tert-butoxycarbonyl)hydrazono]piperidin-2-carboxylat (Racemat) (2.20 g, 7.21 mmol) in 15 ml DMF wurde bei 150°C über Nacht gerührt. Das Reaktionsgemisch (theoretische Ausbeute: 1.42 g) wurde in Aliquots geteilt und direkt weiter verwendet.Zur Lösung eines Aliquots des Rohprodukts Methyl-(5RS)-3-oxo-2,3,5,6,7,8-hexahydro[1,2,4]triazolo[4,3-a]pyridin-5-carboxylat (Racemat) (berechnet als 473 mg, 2.40 mmol) in 5 ml DMF wurden Cäsiumcarbonat (1.17 g, 3.60 mmol) und 1-(Brommethyl)-4-chlorbenzol (518 mg, 2.52 mmol) gegeben. Das Reaktionsgemisch wurde über Nacht bei Raumtemperatur gerührt. Sodann wurden 1-(Brommethyl)-4-chlorbenzol nachgegeben (493 mg, 2.40 mmol) und über Nacht bei 50°C gerührt. Zur Aufarbeitung wurde mit Wasser versetzt, zweimal mit je 15 ml Ethylacetat extrahiert, die vereinigten organischen Phasen einmal mit gesättigter wässriger Natriumchloridlösung gewaschen, mit Magnesiumsulfat getrocknet, filtriert und im Vakuum eingeengt. Der Rückstand wurde in 2 Portionen über präparativeHPLC getrennt (Säule: Chromatorex, 125x40mm 10µm. Flow: 50ml/min. Gradient ( A= Wasser+ 0,1% Ameisensäure , B= Acetonitril ): 0min 10% B, 5min 10%B, 27min 98% B, 35min 98 % B, 35,01min 10%B , 38min 10%B. Laufzeit pro Trennung 38min. Detektion: 210 nm). Einengen der Produktfraktionen ergab 80 mg (96 % Reinheit, 9.6 % d. Th.) der Titelverbindung.
LC-MS (Methode 3): Rₜ = 1.78 min; MS (ESIpos): m/z = 388 [M+H]⁺

### Intermediat 55

### tert-Butyl-(5RS)-2-(4-methylbenzyl)-3-oxo-2,3,5,6,7,8-hexahydro[1,2,4]triazolo[4,3-a]pyridin-5-carboxylat (Enantiomer 1)

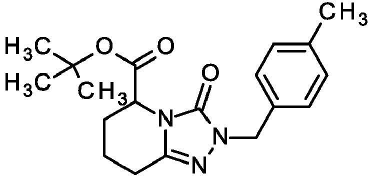

### tert-Butyl-(5RS)-3-oxo-2,3,5,6,7,8-hexahydro[1,2,4]triazolo[4,3-a]pyridin-5-carboxylat

(Enantiomer 2) (1.77 g, 7.38 mmol) wurde in Acetonitril (71 ml) vorgelegt. Cäsiumcarbonat (2.40 g, 7.38 mmol) und 1-(Brommethyl)-4-methylbenzol (1.37 g, 7.38 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase mit Ethylacetat extrahiert. Die vereinigten organischen Phasen wurden mit gesättigter wässriger Natriumchlorid-Lösung gewaschen, über Magnesiumsulfat getrocknet, filtriert und das Filtrat eingeengt. Es wurden 2.65 g (>100 % d. Th.) der Titelverbindung erhalten.
LC-MS (Methode 4): Rₜ = 0.97 min; MS (ESIpos): m/z = 343 [M+H]⁺

### Intermediat 56

### tert-Butyl-(5RS)-2-[(5-chlorpyridin-2-yl)methyl]-3-oxo-2,3,5,6,7,8-hexahydro[1,2,4]triazolo[4,3-a]pyridin-5-carboxylat (Racemat)

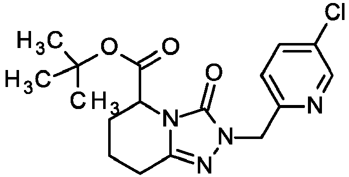

### tert-Butyl-(5RS)-3-oxo-2,3,5,6,7,8-hexahydro[1,2,4]triazolo[4,3-a]pyridin-5-carboxylat

(Racemat) (300 mg, 1.25 mmol) wurde in Acetonitril (6.0 ml) vorgelegt. Cäsiumcarbonat (613 mg, 1.88 mmol) und 2-(Brommethyl)-5-chlorpyridin (272 mg, 1.32 mmol, CAS 605681-01-4) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 306 mg (65 % d. Th.) der Titelverbindung erhalten.
LC-MS (Methode 3): Rₜ = 1.61 min; MS (ESIpos): m/z = 365 [M+H]⁺
¹H-NMR (600 MHz, DMSO-d6) δ [ppm]: 1.410 (16.00), 2.078 (0.62), 2.085 (0.56), 2.637 (0.42), 4.453 (0.46), 4.459 (0.48), 4.463 (0.58), 4.469 (0.43), 4.932 (1.44), 4.947 (1.43), 7.232 (0.89), 7.246 (0.91), 7.928 (0.56), 7.932 (0.56), 7.942 (0.53), 7.946 (0.52), 8.578 (0.90), 8.582 (0.88).

### Intermediat 57

### tert-Butyl-(5RS)-3-oxo-2-{[6-(trifluormethyl)pyridin-3-yl]methyl}-2,3,5,6,7,8-hexahydro-[1,2,4]triazolo[4,3-a]pyridin-5-carboxylat (Racemat)

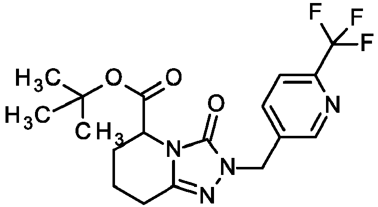

### tert-Butyl-(5RS)-3-oxo-2,3,5,6,7,8-hexahydro[1,2,4]triazolo[4,3-a]pyridin-5-carboxylat

(Racemat) (300 mg, 1.25 mmol) wurde in Acetonitril (6.0 ml) vorgelegt. Cäsiumcarbonat (613 mg, 1.88 mmol) und 5-(Brommethyl)-2-(trifluormethyl)pyridin (316 mg, 1.32 mmol, CAS 108274-33-5) wurden anschließend zugegeben. Das Reaktionsgemisch wurde nach 48 h Rühren bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde in Diethylether suspendiert und der Feststoff abfilrtiert und im Vakuum getrocknet .Es wurden 345 mg (66 % d. Th.) der Titelverbindung erhalten.
LC-MS (Methode 4): Rₜ = 0.92 min; MS (ESIpos): m/z = 399 [M+H]⁺
¹H-NMR (400 MHz, DMSO-d6) δ [ppm]: 1.383 (16.00), 1.404 (0.51), 2.069 (0.60), 2.081 (0.48), 4.457 (0.62), 5.036 (2.03), 7.923 (1.20), 7.933 (0.73), 7.937 (0.70), 8.665 (0.72).

### Intermediat 58

### tert-Butyl-(5RS)-2-(3,5-dichlorbenzyl)-3-oxo-2,3,5,6,7,8-hexahydro[1,2,4]triazolo[4,3-a]pyridin-5-carboxylat (Racemat)

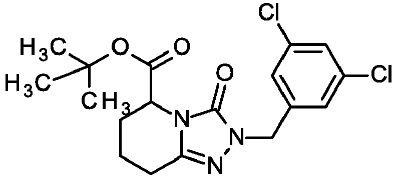

### tert-Butyl-(5RS)-3-oxo-2,3,5,6,7,8-hexahydro[1,2,4]triazolo[4,3-a]pyridin-5-carboxylat

(Racemat) (300 mg, 1.25 mmol) wurde in Acetonitril (6.0 ml) vorgelegt. Cäsiumcarbonat (613 mg, 1.88 mmol) und 1-(Brommethyl)-3,5-dichlorbenzol (316 mg, 1.32 mmol, CAS 7778-01-0) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 445 mg (82 % Reinheit, 89 % d. Th.) der Titelverbindung erhalten.
LC-MS (Methode 3): Rₜ = 2.12 min; MS (ESIpos): m/z = 342 [M+H]⁺

### Intermediat 59

### tert-Butyl-(5RS)-3-oxo-2-(pyridin-3-ylmethyl)-2,3,5,6,7,8-hexahydro[1,2,4]triazolo[4,3-a]pyridin-5-carboxylat (Racemat)

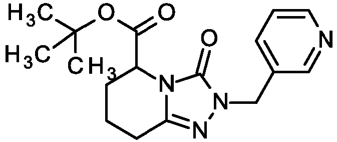

### tert-Butyl-(5RS)-3-oxo-2,3,5,6,7,8-hexahydro[1,2,4]triazolo[4,3-a]pyridin-5-carboxylat

(Racemat) (300 mg, 1.25 mmol) wurde in Acetonitril (6.0 ml) vorgelegt. Cäsiumcarbonat (1.02 g, 3.13 mmol) und 3-(Brommethyl)pyridinhydrobromid (333 mg, 1.32 mmol, CAS 4916-55-6) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 366 mg (88 % d. Th.) der Titelverbindung erhalten.
LC-MS (Methode 4): Rₜ = 0.58 min; MS (ESIpos): m/z = 331 [M+H]⁺
¹H-NMR (400 MHz, DMSO-d6) δ [ppm]: 1.390 (16.00), 1.405 (12.47), 2.037 (0.58), 2.050 (0.73), 2.060 (0.77), 2.070 (0.65), 2.523 (0.58), 2.617 (0.41), 2.627 (0.42), 4.348 (0.50), 4.444 (0.59), 4.526 (0.81), 4.896 (1.69), 7.359 (0.43), 7.371 (0.43), 7.378 (0.45), 7.391 (0.45), 7.642 (0.49), 7.661 (0.42), 8.483 (0.82), 8.500 (0.55), 8.504 (0.55), 8.513 (0.52).

### Intermediat 60

### Methyl-(5RS)-2-[(5-methyl-1,2-oxazol-3-yl)methyl]-3-oxo-2,3,5,6,7,8-hexahydro[1,2,4]triazolo[4,3-a]pyridin-5-carboxylat (Racemat)

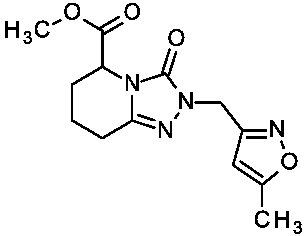

Methyl-(5RS)-3-oxo-2,3,5,6,7,8-hexahydro[1,2,4]triazolo[4,3-a]pyridin-5-carboxylat (Racemat) (50.0 mg, 254 µmol) wurde in Acetonitril (3.6 ml) vorgelegt. Cäsiumcarbonat (124 mg, 380 µmol) und 3-(Brommethyl)-5-methyl-1,2-oxazol (46.9 mg, 266 µmol, CAS 130628-75-0) wurden anschließend zugegeben. Das Reaktionsgemisch wurde nach 24 h Rühren bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 58.0 mg (93 % Reinheit, 73 % d. Th.) der Titelverbindung erhalten.
LC-MS (Methode 1): Rₜ = 0.78 min; MS (ESIpos): m/z = 293 [M+H]⁺
¹H-NMR (400 MHz, DMSO-d6) δ [ppm]: -0.008 (0.58), 0.008 (0.51), 1.527 (0.40), 1.532 (0.40), 1.540 (0.42), 1.798 (0.45), 1.806 (0.50), 1.820 (0.43), 2.055 (0.46), 2.072 (3.66), 2.084 (0.69), 2.092 (0.58), 2.100 (0.46), 2.108 (0.53), 2.113 (0.51), 2.120 (0.49), 2.129 (0.51), 2.136 (0.47), 2.206 (1.02), 2.376 (10.50), 2.518 (0.88), 2.560 (0.90), 2.575 (0.76), 2.587 (0.75), 2.601 (0.66), 2.624 (0.60), 2.636 (1.04), 2.648 (0.59), 2.678 (0.48), 3.687 (1.59), 3.703 (16.00), 4.591 (0.92), 4.600 (1.03), 4.607 (1.24), 4.616 (0.92), 4.854 (7.94), 4.981 (0.43), 6.065 (2.10).

### Intermediat 61

### tert-Butyl-(5RS)-2-(3,4-difluorbenzyl)-3-oxo-2,3,5,6,7,8-hexahydro[1,2,4]triazolo[4,3-a]pyridin-5-carboxylat (Racemat)

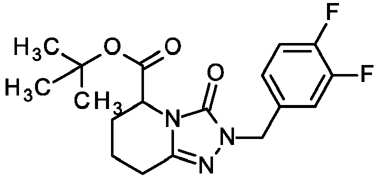

### tert-Butyl-(5RS)-3-oxo-2,3,5,6,7,8-hexahydro[1,2,4]triazolo[4,3-a]pyridin-5-carboxylat

(Racemat) (300 mg, 1.25 mmol) wurde in Acetonitril (10 ml) vorgelegt. Cäsiumcarbonat (429 mg, 1.32 mmol) und 4-(Brommethyl)-1,2-difluorbenzol (273 mg, 1.32 mmol, CAS 85118-01-0) wurden anschließend zugegeben. Das Reaktionsgemisch wurde über Nacht bei Raumtemperatur und eine Stunde bei 90 °C gerührt. Anschließend wurde das Reaktionsgemisch mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 455 mg (99 % d. Th.) der Titelverbindung erhalten.

¹H-NMR (400 MHz, DMSO-d6) δ [ppm]: -0.008 (0.57), 0.008 (0.51), 1.393 (16.00), 1.404 (3.82), 1.908 (1.06), 1.988 (0.61), 2.064 (0.60), 2.074 (0.56), 2.523 (0.73), 4.433 (0.44), 4.443 (0.41), 4.448 (0.56), 4.699 (0.90), 4.847 (1.99), 7.400 (0.49), 7.428 (0.45).

### Intermediat 62

### tert-Butyl-(5RS)-2-(3-chlor-4-fluorbenzyl)-3-oxo-2,3,5,6,7,8-hexahydro[1,2,4]triazolo[4,3-a]pyridin-5-carboxylat (Racemat)

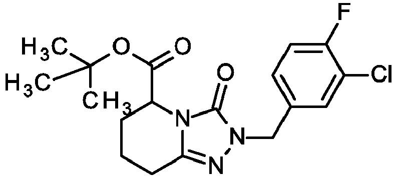

### tert-Butyl-(5RS)-3-oxo-2,3,5,6,7,8-hexahydro[1,2,4]triazolo[4,3-a]pyridin-5-carboxylat

(Racemat) (311 mg, 1.30 mmol) wurde in Acetonitril (10 ml) vorgelegt. Cäsiumcarbonat (445 mg, 1.36 mmol) und 4-(Brommethyl)-2-chlor-1-fluorbenzol (305 mg, 1.36 mmol, CAS 192702-01-5) wurden anschließend zugegeben. Das Reaktionsgemisch wurde über Nacht bei Raumtemperatur und eine Stunde bei 90 °C gerührt. Anschließend wurde das Reaktionsgemisch mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 491 mg (90 % Reinheit, 89 % d. Th.) der Titelverbindung erhalten.
LC-MS (Methode 1): Rₜ = 1.32 min; MS (ESIpos): m/z = 326 [M-tBu+H]⁺

### Intermediat 63

### Methyl-(5RS)-2-(3-chlorbenzyl)-3-oxo-2,3,5,6,7,8-hexahydro[1,2,4]triazolo[4,3-a]pyridin-5-carboxylat (Racemat)

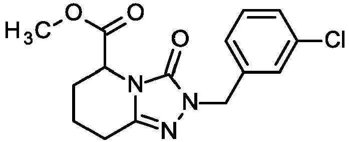

Methyl-(5RS)-3-oxo-2,3,5,6,7,8-hexahydro[1,2,4]triazolo[4,3-a]pyridin-5-carboxylat (Racemat) (50.0 mg, 254 µmol) wurde in Acetonitril (3.6 ml) vorgelegt. Cäsiumcarbonat (124 mg, 380 µmol) und 1-(Brommethyl)-3-chlorbenzol (54.7 mg, 266 µmol, CAS 766-80-3) wurden anschließend zugegeben. Das Reaktionsgemisch wurde nach 24 h Rühren bei Raumtemperatur mit Wasser, 1N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 66.0 mg (81 % d. Th.) der Titelverbindung erhalten.
LC-MS (Methode 4): Rₜ = 0.82 min; MS (ESIpos): m/z = 322 [M+H]⁺
¹H-NMR (400 MHz, DMSO-d6) δ [ppm]: -0.008 (1.42), 0.008 (1.25), 1.526 (0.41), 1.532 (0.41), 1.802 (0.49), 1.811 (0.51), 1.825 (0.45), 1.836 (0.42), 2.065 (0.46), 2.075 (0.77), 2.088 (0.74), 2.095 (0.70), 2.102 (0.56), 2.110 (0.46), 2.118 (0.54), 2.130 (0.51), 2.139 (0.53), 2.146 (0.50), 2.523 (1.49), 2.564 (1.03), 2.579 (0.83), 2.591 (0.83), 2.605 (0.68), 2.631 (0.63), 2.642 (1.16), 2.654 (0.67), 2.673 (0.53), 2.684 (0.51), 3.708 (16.00), 4.615 (1.04), 4.625 (1.16), 4.631 (1.33), 4.640 (1.01), 4.863 (5.05), 7.190 (1.12), 7.208 (1.43), 7.282 (1.86), 7.343 (0.46), 7.359 (1.33), 7.364 (2.10), 7.369 (2.85), 7.387 (1.85), 7.407 (0.62).

### Intermediat 64

### tert-Butyl-(5RS)-2-[(2-methoxypyridin-4-yl)methyl]-3-oxo-2,3,5,6,7,8-hexahydro[1,2,4]triazolo[4,3-a]pyridin-5-carboxylat (Racemat)

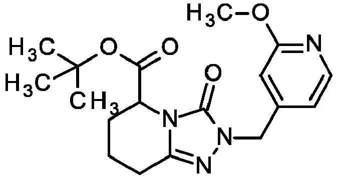

### tert-Butyl-(5RS)-3-oxo-2,3,5,6,7,8-hexahydro[1,2,4]triazolo[4,3-a]pyridin-5-carboxylat

(Racemat) (357 mg, 1.49 mmol) wurde in Acetonitril (10 ml) vorgelegt. Cäsiumcarbonat (510 mg, 1.57 mmol) und 4-(Brommethyl)-2-methoxypyridin (381 mg, 83 % Reinheit, 1.57 mmol, CAS 120277-15-8) wurden anschließend zugegeben. Das Reaktionsgemisch wurde nach Rühren über Nacht bei Raumtemperatur mit Wasser, 1N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 144 mg (27 % d. Th.) der Titelverbindung erhalten.
LC-MS (Methode 3): Rₜ = 1.52 min; MS (ESIpos): m/z = 361 [M+H]⁺

### Intermediat 65

### tert-Butyl-(5RS)-2-(3-fluorbenzyl)-3-oxo-2,3,5,6,7,8-hexahydro[1,2,4]triazolo[4,3-a]pyridin-5-carboxylat (Racemat)

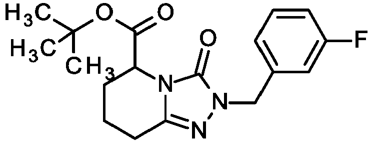

### tert-Butyl-(5RS)-3-oxo-2,3,5,6,7,8-hexahydro[1,2,4]triazolo[4,3-a]pyridin-5-carboxylat

(Racemat) (300 mg, 1.25 mmol) wurde in Acetonitril (10 ml) vorgelegt. Cäsiumcarbonat (429 mg, 1.32 mmol) und 1-(Brommethyl)-3-fluorbenzol (249 mg, 1.32 mmol, CAS 456-41-7) wurden anschließend zugegeben. Das Reaktionsgemisch wurde nach Rühren über Nacht bei Raumtemperatur mit Wasser, 1N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 297 mg (68 % d. Th.) der Titelverbindung erhalten.
LC-MS (Methode 1): Rₜ = 1.23 min; MS (ESIpos): m/z = 291 [M+H]⁺
¹H-NMR (400 MHz, DMSO-d6) δ [ppm]: -0.008 (1.11), 0.008 (0.97), 1.397 (16.00), 2.067 (0.49), 2.077 (0.52), 2.518 (1.08), 2.523 (0.83), 4.451 (0.57), 4.865 (1.75), 4.872 (1.37), 7.078 (0.48), 7.097 (0.65), 7.380 (0.40), 7.396 (0.42).

### Intermediat 66

### tert-Butyl-(5RS)-2-(3-chlor-4-methoxybenzyl)-3-oxo-2,3,5,6,7,8-hexahydro[1,2,4]triazolo[4,3-a]pyridin-5-carboxylat (Racemat)

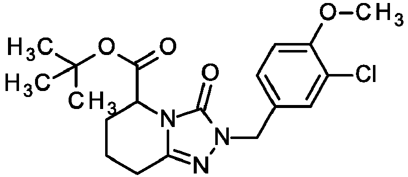

### tert-Butyl-(5RS)-3-oxo-2,3,5,6,7,8-hexahydro[1,2,4]triazolo[4,3-a]pyridin-5-carboxylat

(Racemat) (300 mg, 1.25 mmol) wurde in Acetonitril (6.0 ml) vorgelegt. Cäsiumcarbonat (613 mg, 1.88 mmol) und 4-(Brommethyl)-2-chlor-1-methoxybenzol (314 mg, 99 % Reinheit, 1.32 mmol, CAS 320407-92-9) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 365 mg (74 % d. Th.) der Titelverbindung erhalten.
LC-MS (Methode 4): Rₜ = 0.96 min; MS (ESIpos): m/z = 393 [M]⁺

### Intermediat 67

### tert-Butyl-(5RS)-3-oxo-2-[3-(trifluormethyl)benzyl]-2,3,5,6,7,8-hexahydro[1,2,4]triazolo[4,3-a]pyridin-5-carboxylat (Racemat)

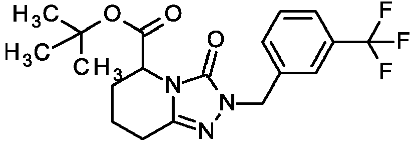

### tert-Butyl-(5RS)-3-oxo-2,3,5,6,7,8-hexahydro[1,2,4]triazolo[4,3-a]pyridin-5-carboxylat

(Racemat) (300 mg, 1.25 mmol) wurde in Acetonitril (6.0 ml) vorgelegt. Cäsiumcarbonat (613 mg, 1.88 mmol) und 1-(Brommethyl)-3-(trifluormethyl)benzol (315 mg, 1.32 mmol, CAS 402-23-3) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde in Diethylether suspendiert, der Feststoff abfiltriert und im Vakuum getrocknet. Es wurden 388 mg (75 % d. Th.) der Titelverbindung erhalten.
LC-MS (Methode 1): Rₜ = 1.35 min; MS (ESIpos): m/z = 342 [M+H]⁺

### Intermediat 68

### tert-Butyl-(5RS)-2-(3-methoxybenzyl)-3-oxo-2,3,5,6,7,8-hexahydro[1,2,4]triazolo[4,3-a]pyridin-5-carboxylat (Racemat)

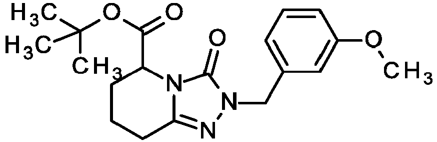

### tert-Butyl-(5RS)-3-oxo-2,3,5,6,7,8-hexahydro[1,2,4]triazolo[4,3-a]pyridin-5-carboxylat

(Racemat) (300 mg, 1.25 mmol) wurde in Acetonitril (6.0 ml) vorgelegt. Cäsiumcarbonat (613 mg, 1.88 mmol) und 1-(Brommethyl)-3-methoxybenzol (265 mg, 1.32 mmol, CAS 874-98-6) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 340 mg (94 % Reinheit, 71 % d. Th.) der Titelverbindung erhalten.
LC-MS (Methode 4): Rₜ = 0.91 min; MS (ESIpos): m/z = 360 [M+H]⁺

### Intermediat 69

### tert-Butyl-(5RS)-2-[(1-methyl-1H-pyrazol-3-yl)methyl]-3-oxo-2,3,5,6,7,8-hexahydro[1,2,4]triazolo[4,3-a]pyridin-5-carboxylat (Racemat)

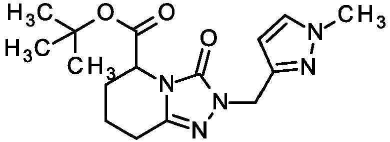

### tert-Butyl-(5RS)-3-oxo-2,3,5,6,7,8-hexahydro[1,2,4]triazolo[4,3-a]pyridin-5-carboxylat

(Racemat) (145 mg, 606 µmol) wurde in Acetonitril (6.0 ml) vorgelegt. Cäsiumcarbonat (207 mg, 636 µmol) und 3-(Brommethyl)-1-methyl-1H-pyrazol (106 mg, 606 µmol, CAS 102846-13-9) wurden anschließend zugegeben. Das Reaktionsgemisch wurde über Nacht bei Raumtemperatur und anschließen für 3 h bei 70 °C gerührt. Das Reaktionsgemisch wurde auf Raumtemperatur abgekühlt und mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 150 mg (74 % d. Th.) der Titelverbindung erhalten.
LC-MS (Methode 1): Rₜ = 0.97 min; MS (ESIpos): m/z = 278 [M-tBu+H]⁺

### Intermediat 70

### tert-Butyl-(5RS)-3-oxo-2-(5,6,7,8-tetrahydronaphthalin-2-ylmethyl)-2,3,5,6,7,8-hexahydro[1,2,4]triazolo[4,3-a]pyridin-5-carboxylat (Racemat)

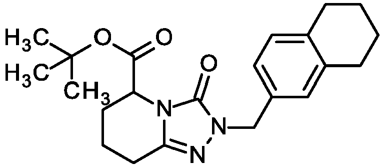

### tert-Butyl-(5RS)-3-oxo-2,3,5,6,7,8-hexahydro[1,2,4]triazolo[4,3-a]pyridin-5-carboxylat

(Racemat) (400 mg, 1.67 mmol) wurde in Acetonitril (10 ml) vorgelegt. Cäsiumcarbonat (572 mg, 1.76 mmol) und 6-(Chlormethyl)-1,2,3,4-tetrahydronaphthalin (317 mg, 1.76 mmol, CAS 17450-63-4) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 280 mg (43 % d. Th.) der Titelverbindung erhalten.
LC-MS (Methode 1): Rₜ = 1.44 min; MS (ESIpos): m/z = 328 [M-tBu+H]⁺

### Intermediat 71

### tert-Butyl-(5RS)-3-oxo-2-(2,4,5-trifluorbenzyl)-2,3,5,6,7,8-hexahydro[1,2,4]triazolo[4,3-a]pyridin-5-carboxylat (Racemat)

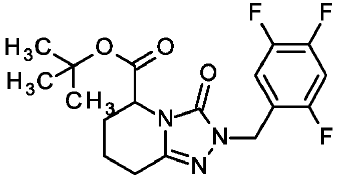

### tert-Butyl-(5RS)-3-oxo-2,3,5,6,7,8-hexahydro[1,2,4]triazolo[4,3-a]pyridin-5-carboxylat

(Racemat) (400 mg, 1.67 mmol) wurde in Acetonitril (10 ml) vorgelegt. Cäsiumcarbonat (572 mg, 1.76 mmol) und 1-(Chlormethyl)-2,4,5-trifluorbenzol (317 mg, 1.76 mmol, CAS 243139-71-1) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 390 mg (61 % d. Th.) der Titelverbindung erhalten.
LC-MS (Methode 1): Rₜ = 1.29 min; MS (ESIpos): m/z = 328 [M-tBu+H]⁺

### Intermediat 72

### tert-Butyl-(5RS)-2-[(2-chlorpyridin-4-yl)methyl]-3-oxo-2,3,5,6,7,8-hexahydro[1,2,4]triazolo[4,3-a]pyridin-5-carboxylat (Racemat)

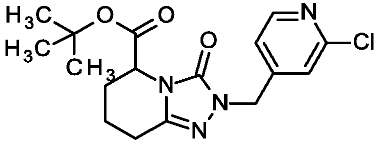

### tert-Butyl-(5RS)-3-oxo-2,3,5,6,7,8-hexahydro[1,2,4]triazolo[4,3-a]pyridin-5-carboxylat

(Racemat) (370 mg, 1.54 mmol) wurde in Acetonitril (10 ml) vorgelegt. Cäsiumcarbonat (528 mg, 1.62 mmol) und 4-(Brommethyl)-2-chlorpyridin (360 mg, 93 % Reinheit, 1.62 mmol, CAS 83004-15-3) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 490 mg (83 % d. Th.) der Titelverbindung erhalten.
LC-MS (Methode 3): Rₜ = 1.54 min; MS (ESIpos): m/z = 365 [M+H]⁺
¹H-NMR (400 MHz, DMSO-d6) δ [ppm]: -0.008 (1.57), 0.008 (1.54), 1.405 (16.00), 2.084 (0.54), 2.094 (0.44), 2.523 (0.99), 2.568 (0.41), 2.669 (0.51), 4.476 (0.54), 4.944 (1.40), 4.949 (1.35), 7.240 (0.51), 7.250 (0.53), 7.328 (0.82), 8.377 (0.75), 8.389 (0.75).

### Intermediat 73

### tert-Butyl-(5RS)-2-[(5-chlor-2-thienyl)methyl]-3-oxo-2,3,5,6,7,8-hexahydro[1,2,4]triazolo[4,3-a]pyridin-5-carboxylat (Racemat)

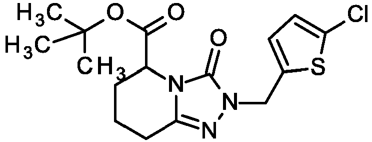

### tert-Butyl-(5RS)-3-oxo-2,3,5,6,7,8-hexahydro[1,2,4]triazolo[4,3-a]pyridin-5-carboxylat

(Racemat) (300 mg, 1.25 mmol) wurde in Acetonitril (10 ml) vorgelegt. Cäsiumcarbonat (429 mg, 1.32 mmol) und 2-Chlor-5-(chlormethyl)thiophen (220 mg, 1.32 mmol, CAS 23784-96-5) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 414 mg (83 % Reinheit, 74 % d. Th.) der Titelverbindung erhalten.
LC-MS (Methode 3): Rₜ = 1.88 min; MS (ESIpos): m/z = 313 [M+H]⁺
¹H-NMR (400 MHz, DMSO-d6) δ [ppm]: 1.175 (0.45), 1.335 (0.71), 1.384 (16.00), 1.397 (0.99), 1.988 (0.79), 2.045 (0.50), 2.057 (0.51), 4.420 (0.56), 4.950 (2.59), 4.986 (2.46), 6.922 (0.61), 6.931 (0.84), 6.976 (1.11), 6.986 (0.79), 7.011 (0.48), 7.020 (0.65), 7.079 (0.51).

### Intermediat 74

### Methyl-(5RS)-2-[(4-methyl-1,2,5-oxadiazol-3-yl)methyl]-3-oxo-2,3,5,6,7,8-hexahydro[1,2,4]triazolo[4,3-a]pyridin-5-carboxylat (Racemat)

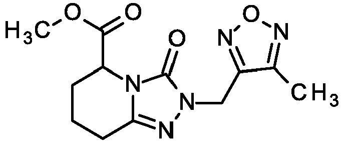

Methyl-(5RS)-3-oxo-2,3,5,6,7,8-hexahydro[1,2,4]triazolo[4,3-a]pyridin-5-carboxylat (Racemat) (300 mg, 1.52 mmol) wurde in Acetonitril (10 ml) vorgelegt. Cäsiumcarbonat (545 mg, 1.67 mmol) und 3-(Brommethyl)-4-methyl-1,2,5-oxadiazol (283 mg, 1.60 mmol, CAS 90507-32-7) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 281 mg (63 % d. Th.) der Titelverbindung erhalten.
LC-MS (Methode 3): Rₜ = 1.06 min; MS (ESIpos): m/z = 294 [M+H]⁺

### Intermediat 75

### Methyl-(5RS)-2-[(6-methylpyridin-3-yl)methyl]-3-oxo-2,3,5,6,7,8-hexahydro[1,2,4]triazolo[4,3-a]pyridin-5-carboxylat (Racemat)

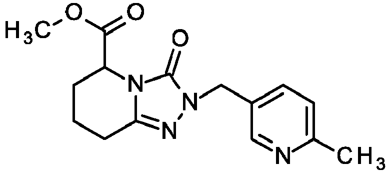

Methyl-(5RS)-3-oxo-2,3,5,6,7,8-hexahydro[1,2,4]triazolo[4,3-a]pyridin-5-carboxylat (Racemat) (300 mg, 1.52 mmol) wurde in Acetonitril (10 ml) vorgelegt. Cäsiumcarbonat (1.24 g, 3.80 mmol) und 5-(Chlormethyl)-2-methylpyridinhydrochlorid (298 mg, 1.67 mmol, CAS 106651-81-4) wurden anschließend zugegeben. Das Reaktionsgemisch wurde nach Rühren über Nacht bei Raumtemperatur mit Wasser, 1N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 236 mg (51 % d. Th.) der Titelverbindung erhalten.
LC-MS (Methode 4): Rₜ = 0.26 min; MS (ESIpos): m/z = 303 [M+H]⁺
¹H-NMR (400 MHz, DMSO-d6) δ [ppm]: -0.008 (0.57), 0.008 (0.57), 1.511 (0.41), 1.517 (0.41), 1.525 (0.42), 1.787 (0.49), 1.798 (0.53), 1.812 (0.45), 1.822 (0.42), 2.051 (0.47), 2.061 (0.80), 2.073 (3.59), 2.086 (0.68), 2.095 (0.50), 2.103 (0.56), 2.107 (0.49), 2.115 (0.52), 2.123 (0.54), 2.131 (0.51), 2.439 (12.07), 2.523 (0.56), 2.557 (0.88), 2.569 (0.86), 2.583 (0.68), 2.609 (0.63), 2.621 (1.16), 2.633 (0.65), 2.663 (0.57), 3.700 (16.00), 4.594 (1.02), 4.603 (1.12), 4.610 (1.31), 4.619 (1.00), 4.835 (5.24), 7.216 (1.67), 7.236 (1.95), 7.506 (1.19), 7.512 (1.20), 7.526 (1.07), 7.532 (1.07), 8.337 (1.47), 8.342 (1.45).

### Intermediat 76

### Methyl-(5RS)-2-[(6-methoxypyridin-3-yl)methyl]-3-oxo-2,3,5,6,7,8-hexahydro[1,2,4]triazolo[4,3-a]pyridin-5-carboxylat (Racemat)

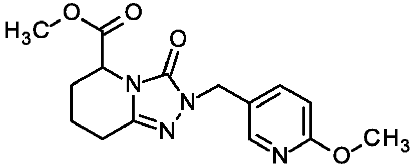

Methyl-(5RS)-3-oxo-2,3,5,6,7,8-hexahydro[1,2,4]triazolo[4,3-a]pyridin-5-carboxylat (Racemat) (300 mg, 1.52 mmol) wurde in Acetonitril (10 ml) vorgelegt. Cäsiumcarbonat (1.24 g, 3.80 mmol) und 5-(Chlormethyl)-2-methoxypyridinhydrochlorid (325 mg, 1.67 mmol, CAS 120276-36-0) wurden anschließend zugegeben. Das Reaktionsgemisch wurde nach Rühren über Nacht bei Raumtemperatur mit Wasser, 1N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 206 mg (42 % d. Th.) der Titelverbindung erhalten.
LC-MS (Methode 4): Rₜ = 0.61 min; MS (ESIpos): m/z = 319 [M+H]⁺
¹H-NMR (400 MHz, DMSO-d6) δ [ppm]: -0.008 (1.39), 0.008 (1.28), 1.795 (0.43), 2.073 (5.67), 2.091 (0.40), 2.099 (0.45), 2.111 (0.42), 2.120 (0.44), 2.127 (0.41), 2.523 (0.87), 2.568 (0.68), 2.582 (0.55), 2.608 (0.50), 2.620 (0.92), 2.631 (0.52), 2.662 (0.50), 3.698 (13.67), 3.828 (16.00), 3.851 (0.46), 4.585 (0.90), 4.594 (0.95), 4.600 (1.13), 4.610 (0.82), 4.794 (5.64), 6.793 (1.69), 6.815 (1.80), 7.560 (1.13), 7.566 (1.15), 7.582 (1.09), 7.588 (1.11), 8.066 (1.53), 8.071 (1.49).

### Intermediat 77

### Methyl-(5RS)-2-[2,5-bis(trifluormethyl)benzyl]-3-oxo-2,3,5,6,7,8-hexahydro[1,2,4]triazolo[4,3-a]pyridin-5-carboxylat (Racemat)

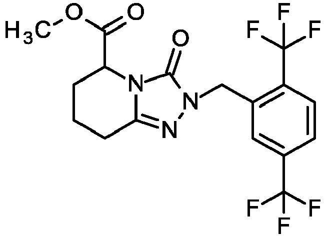

Methyl-(5RS)-3-oxo-2,3,5,6,7,8-hexahydro[1,2,4]triazolo[4,3-a]pyridin-5-carboxylat (Racemat) (300 mg, 1.52 mmol) wurde in Acetonitril (10 ml) vorgelegt. Cäsiumcarbonat (1.24 g, 3.80 mmol) und 2-(Brommethyl)-1,4-bis(trifluormethyl)benzol (514 mg, 1.67 mmol, CAS 302911-98-4) wurden anschließend zugegeben. Das Reaktionsgemisch wurde nach Rühren über Nacht bei Raumtemperatur mit Wasser, 1N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 649 mg (95 % d. Th.) der Titelverbindung erhalten.
LC-MS (Methode 4): Rₜ = 1.00 min; MS (ESIpos): m/z = 424 [M+H]⁺
¹H-NMR (400 MHz, DMSO-d6) δ [ppm]: -0.008 (0.90), 0.008 (0.79), 1.157 (0.56), 1.175 (1.14), 1.193 (0.59), 1.539 (0.40), 1.815 (0.51), 1.825 (0.49), 1.839 (0.42), 1.850 (0.43), 1.988 (2.13), 2.073 (0.47), 2.091 (0.47), 2.103 (0.89), 2.111 (1.07), 2.134 (0.56), 2.147 (0.48), 2.155 (0.51), 2.163 (0.47), 2.576 (0.89), 2.591 (0.71), 2.603 (0.74), 2.617 (0.61), 2.649 (0.57), 2.660 (1.10), 2.671 (0.76), 2.702 (0.47), 3.702 (16.00), 4.021 (0.51), 4.038 (0.50), 4.660 (1.05), 4.669 (1.11), 4.675 (1.37), 4.684 (1.00), 5.056 (0.60), 5.097 (1.96), 5.130 (2.04), 5.171 (0.61), 7.632 (2.12), 7.934 (0.89), 7.953 (1.38), 8.025 (1.90), 8.045 (1.28).

### Intermediat 78

### Methyl-(5RS)-3-oxo-2-({5-[3-(trifluormethyl)phenyl]-1,2,4-oxadiazol-3-yl}methyl)-2,3,5,6,7,8-hexahydro[1,2,4]triazolo[4,3-a]pyridin-5-carboxylat (Racemat)

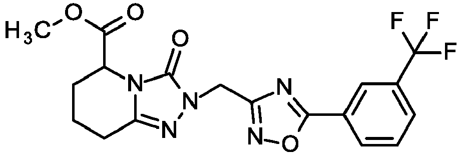

Methyl-(5RS)-3-oxo-2,3,5,6,7,8-hexahydro[1,2,4]triazolo[4,3-a]pyridin-5-carboxylat (Racemat) (300 mg, 1.52 mmol) wurde in Acetonitril (10 ml) vorgelegt. Cäsiumcarbonat (1.24 g, 3.80 mmol) und 3-(Chlormethyl)-5-[3-(trifluormethyl)phenyl]-1,2,4-oxadiazol (439 mg, 1.67 mmol) wurden anschließend zugegeben. Das Reaktionsgemisch wurde nach Rühren über Nacht bei Raumtemperatur mit Wasser, 1N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 642 mg (90 % Reinheit, 90 % d. Th.) der Titelverbindung erhalten.
LC-MS (Methode 3): Rₜ = 1.68 min; MS (ESIpos): m/z = 424 [M+H]⁺
¹H-NMR (400 MHz, DMSO-d6) δ [ppm]: -0.008 (0.48), 0.008 (0.43), 1.158 (0.79), 1.175 (1.64), 1.193 (0.83), 1.530 (0.40), 1.563 (0.43), 1.802 (0.50), 1.814 (0.55), 1.828 (0.47), 1.989 (3.08), 2.067 (0.47), 2.077 (0.73), 2.090 (0.67), 2.105 (0.47), 2.113 (0.50), 2.121 (0.46), 2.129 (0.54), 2.141 (0.53), 2.149 (0.56), 2.157 (0.53), 2.577 (0.87), 2.592 (0.78), 2.603 (0.82), 2.617 (0.63), 2.640 (0.62), 2.652 (1.20), 2.665 (0.72), 2.695 (0.51), 3.311 (16.00), 3.701 (0.48), 4.021 (0.74), 4.039 (0.71), 4.632 (1.16), 4.641 (1.31), 4.647 (1.53), 4.657 (1.11), 5.001 (2.12), 5.074 (0.77), 5.115 (4.74), 5.128 (4.74), 5.168 (0.79), 7.878 (0.89), 7.897 (2.02), 7.917 (1.26), 8.101 (1.45), 8.120 (1.38), 8.334 (2.40), 8.391 (1.51), 8.410 (1.42).

### Intermediat 79

### Methyl-(5RS)-2-(2-methylbenzyl)-3-oxo-2,3,5,6,7,8-hexahydro[1,2,4]triazolo[4,3-a]pyridin-5-carboxylat (Racemat)

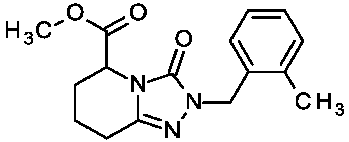

Methyl-(5RS)-3-oxo-2,3,5,6,7,8-hexahydro[1,2,4]triazolo[4,3-a]pyridin-5-carboxylat (Racemat) (200 mg, 1.01 mmol) wurde in Acetonitril (10 ml) vorgelegt. Cäsiumcarbonat (364 mg, 1.12 mmol) und 1-(Brommethyl)-2-methylbenzol (197 mg, 1.06 mmol, CAS 89-92-9) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 196 mg (93 % Reinheit, 60 % d. Th.) der Titelverbindung erhalten.
LC-MS (Methode 1): Rₜ = 1.04 min; MS (ESIpos): m/z = 302 [M+H]⁺

### Intermediat 80

### Methyl-(5RS)-2-[4-fluor-3-(trifluormethyl)benzyl]-3-oxo-2,3,5,6,7,8-hexahydro[1,2,4]triazolo[4,3-a]pyridin-5-carboxylat (Racemat)

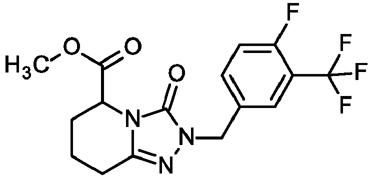

Methyl-(5RS)-3-oxo-2,3,5,6,7,8-hexahydro[1,2,4]triazolo[4,3-a]pyridin-5-carboxylat (Racemat) (300 mg, 1.52 mmol) wurde in Acetonitril (10 ml) vorgelegt. Cäsiumcarbonat (1.24 g, 3.80 mmol) und 4-(Brommethyl)-1-fluor-2-(trifluormethyl)benzol (430 mg, 1.67 mmol, CAS 184970-26-1) wurden anschließend zugegeben. Das Reaktionsgemisch wurde nach Rühren über Nacht bei Raumtemperatur mit Wasser, 1N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 569 mg (90 % Reinheit, 90 % d. Th.) der Titelverbindung erhalten.
LC-MS (Methode 4): Rₜ = 0.90 min; MS (ESIpos): m/z = 374 [M+H]⁺

### Intermediat 81

### Methyl-(5RS)-2-[4-methoxy-3-(trifluormethyl)benzyl]-3-oxo-2,3,5,6,7,8-hexahydro[1,2,4]triazolo[4,3-a]pyridin-5-carboxylat (Racemat)

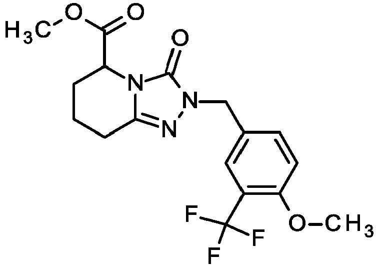

Methyl-(5RS)-3-oxo-2,3,5,6,7,8-hexahydro[1,2,4]triazolo[4,3-a]pyridin-5-carboxylat (Racemat) (200 mg, 1.01 mmol) wurde in Acetonitril (10 ml) vorgelegt. Cäsiumcarbonat (347 mg, 1.06 mmol) und 4-(Brommethyl)-1-methoxy-2-(trifluormethyl)benzol (300 mg, 1.11 mmol, CAS 261951-89-7) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 316 mg (80 % d. Th.) der Titelverbindung erhalten.
LC-MS (Methode 3): Rₜ = 1.60 min; MS (ESIpos): m/z = 386 [M+H]⁺
¹H-NMR (400 MHz, DMSO-d6) δ [ppm]: -0.008 (0.97), 0.008 (0.99), 1.492 (0.42), 1.513 (0.43), 1.519 (0.45), 1.526 (0.45), 1.789 (0.53), 1.798 (0.55), 1.813 (0.47), 1.823 (0.45), 2.052 (0.49), 2.062 (0.82), 2.073 (0.90), 2.096 (0.50), 2.104 (0.57), 2.116 (0.52), 2.125 (0.55), 2.132 (0.51), 2.560 (0.84), 2.572 (0.84), 2.587 (0.67), 2.613 (0.64), 2.625 (1.19), 2.637 (0.68), 2.668 (0.66), 3.696 (15.68), 3.873 (16.00), 4.600 (1.06), 4.609 (1.20), 4.615 (1.38), 4.625 (1.04), 4.844 (4.92), 7.239 (1.72), 7.260 (2.01), 7.489 (1.35), 7.510 (1.41), 7.520 (2.68).

### Intermediat 82

### Methyl-(5RS)-2-[(1-methyl-1H-indazol-5-yl)methyl]-3-oxo-2,3,5,6,7,8-hexahydro[1,2,4]triazolo[4,3-a]pyridin-5-carboxylat (Racemat)

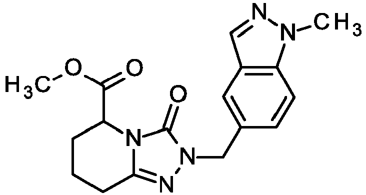

Methyl-(5RS)-3-oxo-2,3,5,6,7,8-hexahydro[1,2,4]triazolo[4,3-a]pyridin-5-carboxylat (Racemat) (200 mg, 1.01 mmol) wurde in Acetonitril (10 ml) vorgelegt. Cäsiumcarbonat (347 mg, 1.06 mmol) und 5-(Brommethyl)-1-methyl-1H-indazol (251 mg, 1.11 mmol, CAS 1092961-01-7) wurden anschließend zugegeben. Das Reaktionsgemisch wurde nach 72 h Rühren bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 87.0 mg (25 % d. Th.) der Titelverbindung erhalten.
LC-MS (Methode 3): Rₜ = 1.10 min; MS (ESIpos): m/z = 342 [M+H]⁺
¹H-NMR (400 MHz, DMSO-d6) δ [ppm]: -0.008 (1.77), 0.008 (1.59), 1.447 (0.66), 1.456 (0.44), 1.783 (0.44), 1.794 (0.48), 1.808 (0.41), 2.049 (0.42), 2.060 (0.69), 2.073 (0.71), 2.080 (0.63), 2.087 (0.51), 2.095 (0.41), 2.103 (0.49), 2.115 (0.45), 2.123 (0.48), 2.131 (0.44), 2.563 (0.85), 2.578 (0.63), 2.602 (0.59), 2.613 (1.08), 2.626 (0.61), 2.656 (0.49), 2.669 (0.50), 3.706 (14.99), 4.024 (16.00), 4.597 (0.97), 4.607 (1.09), 4.613 (1.27), 4.623 (0.94), 4.922 (6.84), 7.285 (1.33), 7.307 (1.37), 7.310 (1.55), 7.593 (1.89), 7.614 (3.97), 8.019 (3.08).

### Intermediat 83

### Methyl-(5RS)-2-[(1-ethyl-1H-imidazol-2-yl)methyl]-3-oxo-2,3,5,6,7,8-hexahydro[1,2,4]triazolo[4,3-a]pyridin-5-carboxylat (Racemat)

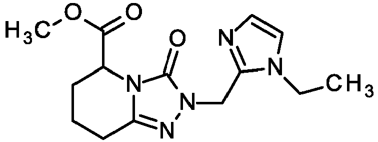

Methyl-(5RS)-3-oxo-2,3,5,6,7,8-hexahydro[1,2,4]triazolo[4,3-a]pyridin-5-carboxylat (Racemat) (200 mg, 1.01 mmol) wurde in Acetonitril (6.0 ml) vorgelegt. Cäsiumcarbonat (364 mg, 1.12 mmol) und 2-(Chlormethyl)-1-ethyl-1H-imidazol (154 mg, 1.06 mmol, CAS 780722-30-7) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 54.0 mg (17 % d. Th.) der Titelverbindung erhalten.
LC-MS (Methode 1): Rₜ = 0.29 min; MS (ESIpos): m/z = 306 [M+H]⁺

### Intermediat 84

### Methyl-(5RS)-2-[(1-methyl-1H-benzimidazol-2-yl)methyl]-3-oxo-2,3,5,6,7,8-hexahydro[1,2,4]triazolo[4,3-a]pyridin-5-carboxylat (Racemat)

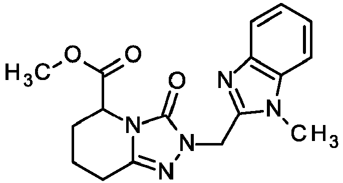

Methyl-(5RS)-3-oxo-2,3,5,6,7,8-hexahydro[1,2,4]triazolo[4,3-a]pyridin-5-carboxylat (Racemat) (200 mg, 1.01 mmol) wurde in Acetonitril (6.0 ml) vorgelegt. Cäsiumcarbonat (364 mg, 1.12 mmol) und 2-(Brommethyl)-1-methyl-1H-benzimidazol (240 mg, 1.06 mmol, CAS 136099-52-0) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 137 mg (40 % d. Th.) der Titelverbindung erhalten.
LC-MS (Methode 1): Rₜ = 0.73 min; MS (ESIpos): m/z = 342 [M+H]⁺

### Intermediat 85

### Methyl-(5RS)-2-[3-chlor-4-(trifluormethoxy)benzyl]-3-oxo-2,3,5,6,7,8-hexahydro[1,2,4]triazolo[4,3-a]pyridin-5-carboxylat (Racemat)

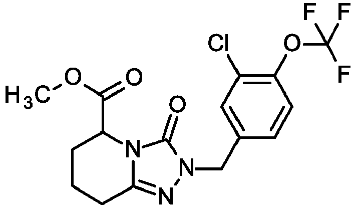

Methyl-(5RS)-3-oxo-2,3,5,6,7,8-hexahydro[1,2,4]triazolo[4,3-a]pyridin-5-carboxylat (Racemat) (200 mg, 1.01 mmol) wurde in Acetonitril (10 ml) vorgelegt. Cäsiumcarbonat (347 mg, 1.06 mmol) und 4-(Brommethyl)-2-chlorphenyl-trifluormethylether (323 mg, 1.11 mmol, CAS 261763-18-2) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 302 mg (73 % d. Th.) der Titelverbindung erhalten.
LC-MS (Methode 3): Rₜ = 1.81 min; MS (ESIpos): m/z = 406 [M+H]⁺
¹H-NMR (400 MHz, DMSO-d6) δ [ppm]: -0.008 (0.94), 0.008 (0.87), 1.805 (0.43), 1.815 (0.45), 2.070 (0.41), 2.079 (0.69), 2.094 (0.77), 2.104 (0.63), 2.112 (0.43), 2.120 (0.49), 2.124 (0.42), 2.132 (0.44), 2.140 (0.47), 2.148 (0.44), 2.524 (0.61), 2.569 (0.83), 2.583 (0.70), 2.595 (0.73), 2.609 (0.58), 2.636 (0.54), 2.647 (1.01), 2.660 (0.61), 2.690 (0.45), 3.708 (16.00), 4.619 (0.97), 4.629 (1.06), 4.635 (1.29), 4.644 (0.95), 4.911 (5.85), 7.317 (1.07), 7.322 (1.12), 7.338 (1.27), 7.344 (1.31), 7.533 (2.04), 7.539 (1.93), 7.564 (1.23), 7.567 (1.21), 7.585 (1.04), 7.588 (1.02).

### Intermediat 86

### Methyl-(5RS)-2-{[3-chlor-5-(trifluormethyl)pyridin-2-yl]methyl}-3-oxo-2,3,5,6,7,8-hexahydro[1,2,4]triazolo[4,3-a]pyridin-5-carboxylat (Racemat)

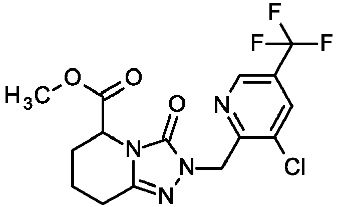

Methyl-(5RS)-3-oxo-2,3,5,6,7,8-hexahydro[1,2,4]triazolo[4,3-a]pyridin-5-carboxylat (Racemat) (200 mg, 1.01 mmol) wurde in Acetonitril (5.0 ml,) vorgelegt. Cäsiumcarbonat (364 mg, 1.12 mmol) und 3-Chlor-2-(chlormethyl)-5-(trifluormethyl)pyridin (245 mg, 1.06 mmol, CAS 175277-52-8) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 293 mg (74 % d. Th.) der Titelverbindung erhalten.
LC-MS (Methode 1): Rₜ = 1.08 min; MS (ESIpos): m/z = 391 [M+H]⁺

### Intermediat 87

### Methyl-(5RS)-2-[3-fluor-4-(trifluormethoxy)benzyl]-3-oxo-2,3,5,6,7,8-hexahydro[1,2,4]triazolo[4,3-a]pyridin-5-carboxylat (Racemat)

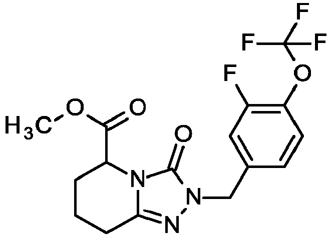

Methyl-(5RS)-3-oxo-2,3,5,6,7,8-hexahydro[1,2,4]triazolo[4,3-a]pyridin-5-carboxylat (Racemat) (200 mg, 1.01 mmol) wurde in Acetonitril (6.0 ml) vorgelegt. Cäsiumcarbonat (364 mg, 1.12 mmol) und 4-(Brommethyl)-2-fluorphenyl-trifluormethylether (291 mg, 1.06 mmol, CAS 886499-04-3) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 263 mg (66 % d. Th.) der Titelverbindung erhalten.
LC-MS (Methode 1): Rₜ = 1.22 min; MS (ESIpos): m/z = 390 [M+H]⁺

### Intermediat 88

### Methyl-(5RS)-2-{[2-methyl-4-(trifluormethyl)-1,3-thiazol-5-yl]methyl}-3-oxo-2,3,5,6,7,8-hexahydro[1,2,4]triazolo[4,3-a]pyridin-5-carboxylat (Racemat)

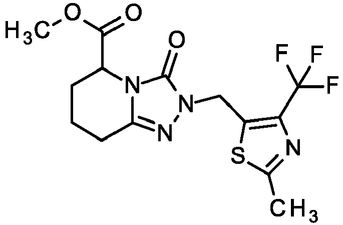

Methyl-(5RS)-3-oxo-2,3,5,6,7,8-hexahydro[1,2,4]triazolo[4,3-a]pyridin-5-carboxylat (Racemat) (200 mg, 1.01 mmol) wurde in Acetonitril (10 ml) vorgelegt. Cäsiumcarbonat (347 mg, 1.06 mmol) und 5-(Brommethyl)-2-methyl-4-(trifluormethyl)-1,3-thiazol (290 mg, 1.11 mmol, CAS 1000339-73-0) wurden anschließend zugegeben. Das Reaktionsgemisch wurde nach 72 h Rühren bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 384 mg (99 % d. Th.) der Titelverbindung erhalten.
LC-MS (Methode 3): Rₜ = 1.33 min; MS (ESIpos): m/z = 377 [M+H]⁺
¹H-NMR (400 MHz, DMSO-d6) δ [ppm]: -0.008 (1.01), 0.008 (1.10), 1.157 (1.51), 1.175 (3.06), 1.193 (1.55), 1.793 (0.43), 1.804 (0.48), 1.818 (0.41), 1.988 (5.82), 2.051 (0.42), 2.062 (0.77), 2.073 (0.86), 2.086 (0.67), 2.094 (0.45), 2.102 (0.51), 2.114 (0.46), 2.122 (0.50), 2.130 (0.46), 2.524 (0.72), 2.568 (0.84), 2.583 (0.72), 2.594 (0.74), 2.608 (0.61), 2.639 (1.12), 2.651 (15.16), 2.668 (1.55), 2.679 (0.51), 2.687 (0.68), 3.697 (16.00), 4.003 (0.46), 4.021 (1.39), 4.038 (1.38), 4.056 (0.46), 4.594 (1.02), 4.603 (1.12), 4.609 (1.32), 4.619 (0.98), 5.171 (4.60).

### Intermediat 89

### Methyl-(5RS)-2-[(3-methyl-1,2-oxazol-5-yl)methyl]-3-oxo-2,3,5,6,7,8-hexahydro[1,2,4]triazolo[4,3-a]pyridin-5-carboxylat (Racemat)

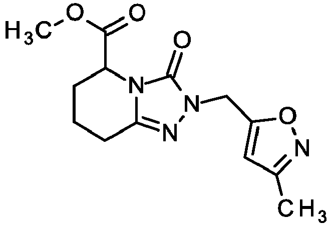

Methyl-(5RS)-3-oxo-2,3,5,6,7,8-hexahydro[1,2,4]triazolo[4,3-a]pyridin-5-carboxylat (Racemat) (200 mg, 1.01 mmol) wurde in Acetonitril (10 ml) vorgelegt. Cäsiumcarbonat (347 mg, 1.06 mmol) und 5-(Brommethyl)-3-methyl-1,2-oxazol (196 mg, 1.11 mmol, CAS 36958-61-9) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 259 mg (86 % Reinheit, 75 % d. Th.) der Titelverbindung erhalten.
LC-MS (Methode 3): Rₜ = 0.94 min; MS (ESIpos): m/z = 293 [M+H]⁺
¹H-NMR (400 MHz, DMSO-d6) δ [ppm]: -0.008 (0.44), 1.174 (0.64), 1.510 (0.40), 1.531 (0.43), 1.536 (0.43), 1.544 (0.42), 1.798 (0.49), 1.809 (0.54), 1.824 (0.47), 1.988 (1.18), 2.042 (0.41), 2.055 (0.52), 2.065 (0.75), 2.071 (0.80), 2.078 (0.73), 2.087 (0.86), 2.096 (0.58), 2.104 (0.53), 2.112 (0.61), 2.117 (0.52), 2.124 (0.64), 2.133 (0.58), 2.141 (0.55), 2.205 (15.12), 2.218 (2.90), 2.518 (0.41), 2.524 (0.52), 2.567 (0.86), 2.581 (0.78), 2.593 (0.80), 2.607 (0.67), 2.629 (0.63), 2.641 (1.15), 2.653 (0.66), 2.683 (0.47), 3.687 (1.47), 3.691 (0.75), 3.703 (16.00), 4.596 (1.03), 4.606 (1.14), 4.612 (1.28), 4.622 (0.98), 4.773 (1.74), 4.981 (6.40), 6.237 (3.04), 6.458 (0.50).

### Intermediat 90

### Methyl-(5RS)-2-[(1-methyl-1H-1,2,4-triazol-3-yl)methyl]-3-oxo-2,3,5,6,7,8-hexahydro[1,2,4]triazolo[4,3-a]pyridin-5-carboxylat (Racemat)

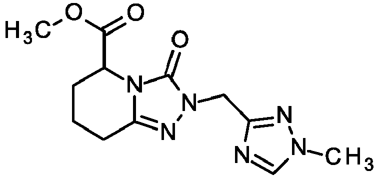

Methyl-(5RS)-3-oxo-2,3,5,6,7,8-hexahydro[1,2,4]triazolo[4,3-a]pyridin-5-carboxylat (Racemat) (200 mg, 1.01 mmol) wurde in Acetonitril (11 ml) vorgelegt. Cäsiumcarbonat (793 mg, 2.43 mmol) und 3-(Chlormethyl)-1-methyl-1H-1,2,4-triazolhydrochlorid (187 mg, 1.11 mmol, CAS 135206-76-7) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 178 mg (74 % Reinheit, 44 % d. Th.) der Titelverbindung erhalten.
LC-MS (Methode 3): Rₜ = 0.61 min; MS (ESIpos): m/z = 293 [M+H]⁺

### Intermediat 91

### Methyl-(5RS)-2-[2-fluor-3-(trifluormethyl)benzyl]-3-oxo-2,3,5,6,7,8-hexahydro[1,2,4]triazolo[4,3-a]pyridin-5-carboxylat (Racemat)

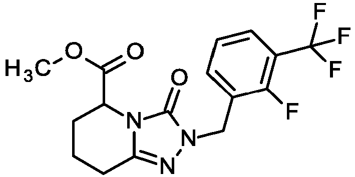

Methyl-(5RS)-3-oxo-2,3,5,6,7,8-hexahydro[1,2,4]triazolo[4,3-a]pyridin-5-carboxylat (Racemat) (200 mg, 1.01 mmol) wurde in Acetonitril (5.0 ml) vorgelegt. Cäsiumcarbonat (364 mg, 1.12 mmol) und 1-(Brommethyl)-2-fluor-3-(trifluormethyl)benzol (274 mg, 1.06 mmol, CAS 184970-25-0) wurden nach 5 min Rühren zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 184 mg (49 % d. Th.) der Titelverbindung erhalten.
LC-MS (Methode 1): Rₜ = 1.16 min; MS (ESIpos): m/z = 374 [M+H]⁺

### Intermediat 92

### Methyl-(5RS)-2-[3-fluor-5-(trifluormethyl)benzyl]-3-oxo-2,3,5,6,7,8-hexahydro[1,2,4]triazolo[4,3-a]pyridin-5-carboxylat (Racemat)

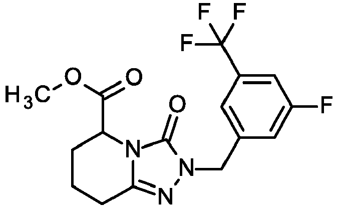

Methyl-(5RS)-3-oxo-2,3,5,6,7,8-hexahydro[1,2,4]triazolo[4,3-a]pyridin-5-carboxylat (Racemat) (200 mg, 1.01 mmol) wurde in Acetonitril (5.0 ml) vorgelegt. Cäsiumcarbonat (364 mg, 1.12 mmol) und 1-(Brommethyl)-3-fluor-5-(trifluormethyl)benzol (274 mg, 1.06 mmol, CAS 239087-09-3) wurden nach 5 min Rühren zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 154 mg (41 % d. Th.) der Titelverbindung erhalten.
LC-MS (Methode 1): Rₜ = 1.19 min; MS (ESIpos): m/z = 374 [M+H]⁺

### Intermediat 93

### Methyl-(5RS)-3-oxo-2-{[2-(trifluormethyl)pyridin-4-yl]methyl}-2,3,5,6,7,8-hexahydro[1,2,4]triazolo[4,3-a]pyridin-5-carboxylat (Enantiomer 1)

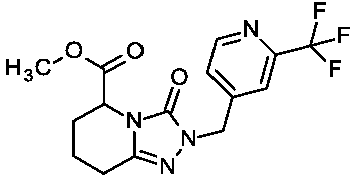

### Methyl-(5RS)-3-oxo-2,3,5,6,7,8-hexahydro[1,2,4]triazolo[4,3-a]pyridin-5-carboxylat

(Enantiomer 2) (200 mg, 1.01 mmol) wurde in Acetonitril (5.0 ml) vorgelegt. Cäsiumcarbonat (364 mg, 1.12 mmol) und 4-(Chlormethyl)-2-(trifluormethyl)pyridinhydrochlorid (247 mg, 1.06 mmol) wurden nach 5 min Rühren zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 279 mg (77 % d. Th.) der Titelverbindung erhalten.
LC-MS (Methode 4): Rₜ = 0.71 min; MS (ESIpos): m/z = 357 [M+H]⁺
Die (5S)-Konfiguration wurde aufgrund der Kristallstrukturaufklärung für Beispiel 26, 108, 113, 157, 237, 358 und 454 zugeordnet.

### Methyl-(5S)-3-oxo-2-{[2-(trifluormethyl)pyridin-4-yl]methyl}-2,3,5,6,7,8-hexahydro[1,2,4]triazolo[4,3-a]pyridin-5-carboxylat

### Intermediat 94

### Methyl-(5RS)-2-{[3-fluor-2-(trifluormethyl)pyridin-4-yl]methyl}-3-oxo-2,3,5,6,7,8-hexahydro[1,2,4]triazolo[4,3-a]pyridin-5-carboxylat (Enantiomer 1)

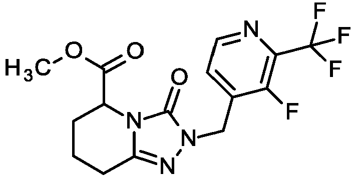

### Methyl-(5RS)-3-oxo-2,3,5,6,7,8-hexahydro[1,2,4]triazolo[4,3-a]pyridin-5-carboxylat

(Enantiomer 2) (200 mg, 1.01 mmol) wurde in Acetonitril (5.0 ml) vorgelegt. Cäsiumcarbonat (364 mg, 1.12 mmol) und 4-(Chlormethyl)-3-fluor-2-(trifluormethyl)pyridin Hydrochlorid (266 mg, 1.06 mmol) wurden anschließend zugegeben. Das Reaktionsgemisch wurde nach Rühren über Nacht bei Raumtemperatur erneut mit Cäsiumcarbonat (199 mg, 0.61 mmol) versetzt und 2 Stunden bei 60 °C gerührt. Anschließend wurde das Reaktionsgemisch auf Raumtemperatur abgekühlt und mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 181 mg (48 % d. Th.) der Titelverbindung erhalten.
LC-MS (Methode 3): Rₜ = 1.42 min; MS (ESIpos): m/z = 375 [M+H]⁺
Die (5S)-Konfiguration wurde aufgrund der Kristallstrukturaufklärung für Beispiel 26, 108, 113, 157, 237, 358 und 454 zugeordnet.
Methyl-(5S)-2-{[3-fluor-2-(trifluormethyl)pyridin-4-yl]methyl}-3-oxo-2,3,5,6,7,8-hexahydro[1,2,4]triazolo[4,3-a]pyridin-5-carboxylat

### Intermediat 95

### Methyl-(5RS)-2-{[5-chlor-4-(trifluormethyl)pyridin-2-yl]methyl}-3-oxo-2,3,5,6,7,8-hexahydro[1,2,4]triazolo[4,3-a]pyridin-5-carboxylat (Enantiomer 1)

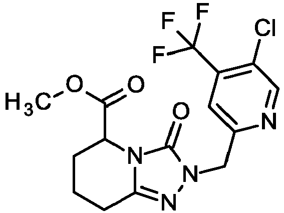

### Methyl-(5RS)-3-oxo-2,3,5,6,7,8-hexahydro[1,2,4]triazolo[4,3-a]pyridin-5-carboxylat

(Enantiomer 2) (164 mg, 829 µmol) wurde in Acetonitril (4.0 ml) vorgelegt. Cäsiumcarbonat (594 mg, 1.82 mmol) und 5-Chlor-2-(chlormethyl)-4-(trifluormethyl)pyridin Hydrochlorid (274 mg, 85 % Reinheit, 871 µmol) wurden anschließend zugegeben. Das Reaktionsgemisch wurde über Nacht bei Raumtemperatur, für 5 h bei 85 °C und anschließend über Nacht erneut bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 269 mg (93 % Reinheit, 77 % d. Th.) der Titelverbindung erhalten.
LC-MS (Methode 3): Rₜ = 1.55 min; MS (ESIpos): m/z = 391 [M+H]⁺
¹H-NMR (400 MHz, DMSO-d6) δ [ppm]: -0.008 (0.42), 0.008 (0.46), 1.157 (1.75), 1.175 (3.60), 1.193 (1.82), 1.352 (0.61), 1.534 (0.40), 1.540 (0.41), 1.807 (0.47), 1.816 (0.49), 1.830 (0.41), 1.989 (6.73), 2.074 (0.58), 2.086 (0.74), 2.098 (0.77), 2.119 (0.46), 2.126 (0.53), 2.131 (0.46), 2.139 (0.47), 2.148 (0.52), 2.155 (0.46), 2.520 (0.80), 2.563 (0.87), 2.578 (0.72), 2.590 (0.72), 2.604 (0.62), 2.630 (0.57), 2.642 (1.01), 2.654 (0.60), 2.671 (0.40), 2.684 (0.45), 3.688 (1.91), 3.704 (16.00), 3.936 (0.71), 4.003 (0.54), 4.021 (1.59), 4.039 (1.58), 4.057 (0.52), 4.628 (1.01), 4.637 (1.55), 4.643 (1.37), 4.652 (1.42), 5.074 (7.18), 7.727 (3.53), 7.832 (0.45), 8.858 (0.45), 8.910 (3.33).
Die (5S)-Konfiguration wurde aufgrund der Kristallstrukturaufklärung für Beispiel 26, 108, 113, 157, 237, 358 und 454 zugeordnet.

### Methyl-(5S)-2-{[5-chlor-4-(trifluormethyl)pyridin-2-yl]methyl}-3-oxo-2,3,5,6,7,8-hexahydro[1,2,4]triazolo[4,3-a]pyridin-5-carboxylat

### Intermediat 96

### Methyl-(5RS)-3-oxo-2-{[2-(trifluormethyl)chinolin-4-yl]methyl}-2,3,5,6,7,8-hexahydro[1,2,4]triazolo[4,3-a]pyridin-5-carboxylat (Enantiomer 1)

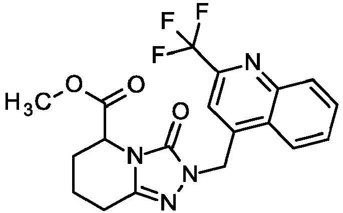

### Methyl-(5RS)-3-oxo-2,3,5,6,7,8-hexahydro[1,2,4]triazolo[4,3-a]pyridin-5-carboxylat

(Enantiomer 2) (166 mg, 839 µmol) wurde in Acetonitril (4.1 ml) vorgelegt. Cäsiumcarbonat (301 mg, 923 µmol) und 4-(Chlormethyl)-2-(trifluormethyl)chinolin Hydrochlorid (336 mg, 74 % Reinheit, 881 µmol) wurden nach 5 min Rühren zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 111 mg (33 % d. Th.) der Titelverbindung erhalten.
LC-MS (Methode 1): Rₜ = 1.19 min; MS (ESIpos): m/z = 407 [M+H]⁺
Die (5S)-Konfiguration wurde aufgrund der Kristallstrukturaufklärung für Beispiel 26, 108, 113, 157, 237, 358 und 454 zugeordnet.

### Methyl-(5S)-3-oxo-2-{[2-(trifluormethyl)chinolin-4-yl]methyl}-2,3,5,6,7,8-hexahydro[1,2,4]triazolo[4,3-a]pyridin-5-carboxylat

### Intermediat 97

### Methyl-(5RS)-2-{[3-chlor-5-(trifluormethyl)pyridin-2-yl]methyl}-3-oxo-2,3,5,6,7,8-hexahydro[1,2,4]triazolo[4,3-a]pyridin-5-carboxylat (Enantiomer 1)

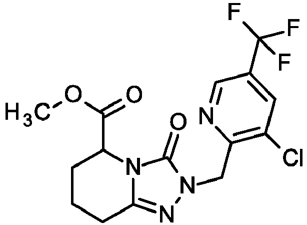

### Methyl-(5RS)-3-oxo-2,3,5,6,7,8-hexahydro[1,2,4]triazolo[4,3-a]pyridin-5-carboxylat

(Enantiomer 2) (200 mg, 1.01 mmol) wurde in Acetonitril (5.0 ml) vorgelegt. Cäsiumcarbonat (364 mg, 1.12 mmol) und 3-Chlor-2-(chlormethyl)-5-(trifluormethyl)pyridin (245 mg, 1.06 mmol, CAS 175277-52-8) wurden nach 5 Min Rühren zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 216 mg (55 % d. Th.) der Titelverbindung erhalten.
LC-MS (Methode 3): Rₜ = 1.49 min; MS (ESIpos): m/z = 391 [M+H]⁺
Die (5S)-Konfiguration wurde aufgrund der Kristallstrukturaufklärung für Beispiel 26, 108, 113, 157, 237, 358 und 454 zugeordnet.

### Methyl-(5S)-2-{[3-chlor-5-(trifluormethyl)pyridin-2-yl]methyl}-3-oxo-2,3,5,6,7,8-hexahydro[1,2,4]triazolo[4,3-a]pyridin-5-carboxylat

### Intermediat 98

### Methyl-(5RS)-3-oxo-2-[(3,5,6-trimethylpyrazin-2-yl)methyl]-2,3,5,6,7,8-hexahydro[1,2,4]triazolo[4,3-a]pyridin-5-carboxylat (Enantiomer 1)

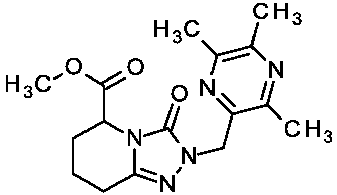

### Methyl-(5RS)-3-oxo-2,3,5,6,7,8-hexahydro[1,2,4]triazolo[4,3-a]pyridin-5-carboxylat

(Enantiomer 2) (250 mg, 1.27 mmol) wurde in Acetonitril (5.0 ml) vorgelegt. Cäsiumcarbonat (454 mg, 1.39 mmol) und 2-(Brommethyl)-3,5,6-trimethylpyrazin (286 mg, 1.33 mmol, CAS 79074-45-6) wurden nach 5 min Rühren zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 261 mg (62 % d. Th.) der Titelverbindung erhalten.
LC-MS (Methode 3): Rₜ = 1.02 min; MS (ESIpos): m/z = 332 [M+H]⁺

### Intermediat 99

### Methyl-(5RS)-3-oxo-2-{[4-(trifluormethyl)pyridin-2-yl]methyl}-2,3,5,6,7,8-hexahydro[1,2,4]triazolo[4,3-a]pyridin-5-carboxylat (Enantiomer 1)

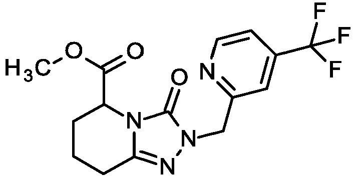

### Methyl-(5RS)-3-oxo-2,3,5,6,7,8-hexahydro[1,2,4]triazolo[4,3-a]pyridin-5-carboxylat

(Enantiomer 2) (300 mg, 1.52 mmol) wurde in Acetonitril (6.0 ml) vorgelegt. Cäsiumcarbonat (744 mg, 2.28 mmol) und 2-(Chlormethyl)-4-(trifluormethyl)pyridinhydrochlorid (371 mg, 1.60 mmol, CAS 215867-87-1) wurden anschließend zugegeben. Das Reaktionsgemisch wurde nach 4 Stunden Rühren bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 282 mg (52 % d. Th.) der Titelverbindung erhalten.
LC-MS (Methode 3): Rₜ = 1.35 min; MS (ESIpos): m/z = 357 [M+H]⁺
¹H-NMR (400 MHz, DMSO-d6) δ [ppm]: -0.008 (0.50), 1.811 (0.48), 1.821 (0.49), 1.835 (0.42), 2.073 (0.44), 2.079 (0.46), 2.090 (0.74), 2.103 (0.69), 2.110 (0.66), 2.118 (0.53), 2.126 (0.46), 2.133 (0.53), 2.138 (0.46), 2.146 (0.48), 2.154 (0.50), 2.162 (0.46), 2.519 (0.45), 2.570 (0.85), 2.585 (0.73), 2.597 (0.75), 2.611 (0.61), 2.636 (0.59), 2.647 (1.03), 2.660 (0.59), 2.690 (0.44), 3.706 (16.00), 4.636 (1.03), 4.646 (1.11), 4.652 (1.26), 4.661 (0.93), 5.075 (7.03), 7.551 (2.03), 7.713 (1.09), 7.724 (1.09), 8.822 (1.50), 8.834 (1.43).

Die (5S)-Konfiguration wurde aufgrund der Kristallstrukturaufklärung für Beispiel 26, 108, 113, 157, 237, 358 und 454 zugeordnet.

### Methyl-(5S)-3-oxo-2-{[4-(trifluormethyl)pyridin-2-yl]methyl}-2,3,5,6,7,8-hexahydro[1,2,4]triazolo[4,3-a]pyridin-5-carboxylat

### Intermediat 100

### Methyl-(5RS)-3-oxo-2-{[2-(trifluormethyl)pyrimidin-4-yl]methyl}-2,3,5,6,7,8-hexahydro[1,2,4]triazolo[4,3-a]pyridin-5-carboxylat (Enantiomer 1)

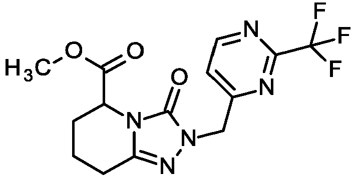

### Methyl-(5RS)-3-oxo-2,3,5,6,7,8-hexahydro[1,2,4]triazolo[4,3-a]pyridin-5-carboxylat

(Enantiomer 2) (108 mg, 548 µmol) wurde in Acetonitril (2.0 ml) vorgelegt. Cäsiumcarbonat (268 mg, 822 µmol) und 4-(Chlormethyl)-2-(trifluormethyl)pyrimidin Hydrochlorid (134 mg, 575 µmol) wurden anschließend zugegeben. Das Reaktionsgemisch wurde nach Rühren über Nacht bei Raumtemperatur und weiteren 2 Stunden unter Rückfluss mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 49.3 mg (25 % d. Th.) der Titelverbindung erhalten.
LC-MS (Methode 4): Rt = 0.69 min; MS (ESIpos): m/z = 358 [M+H]⁺
Die (5S)-Konfiguration wurde aufgrund der Kristallstrukturaufklärung für Beispiel 26, 108, 113, 157, 237, 358 und 454 zugeordnet.

### Methyl-(5S)-3-oxo-2-{[2-(trifluormethyl)pyrimidin-4-yl]methyl}-2,3,5,6,7,8-hexahydro[1,2,4]triazolo[4,3-a]pyridin-5-carboxylat

### Intermediat 101

### Methyl-(5RS)-3-oxo-2-(2,4,5-trimethylbenzyl)-2,3,5,6,7,8-hexahydro[1,2,4]triazolo[4,3-a]pyridin-5-carboxylat (Enantiomer 1)

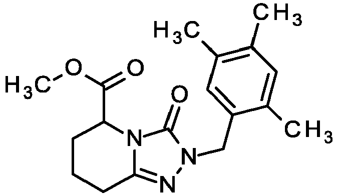

### Methyl-(5RS)-3-oxo-2,3,5,6,7,8-hexahydro[1,2,4]triazolo[4,3-a]pyridin-5-carboxylat

(Enantiomer 2) (200 mg, 1.01 mmol) wurde in Acetonitril (5.0 ml) vorgelegt. Cäsiumcarbonat (364 mg, 1.12 mmol) und 1-(Brommethyl)-2,4,5-trimethylbenzol (227 mg, 1.06 mmol, CAS 35509-98-9) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 198 mg (59 % d. Th.) der Titelverbindung erhalten.
LC-MS (Methode 4): Rₜ = 0.91 min; MS (ESIpos): m/z = 330 [M+H]⁺
Die (5S)-Konfiguration wurde aufgrund der Kristallstrukturaufklärung für Beispiel 26, 108, 113, 157, 237, 358 und 454 zugeordnet.

### Methyl-(5S)-3-oxo-2-(2,4,5-trimethylbenzyl)-2,3,5,6,7,8-hexahydro[1,2,4]triazolo[4,3-a]pyridin-5-carboxylat

### Intermediat 102

### Methyl-(5RS)-2-[4-(tert-butoxycarbonyl)benzyl]-3-oxo-2,3,5,6,7,8-hexahydro[1,2,4]triazolo[4,3-a]pyridin-5-carboxylat (Racemat)

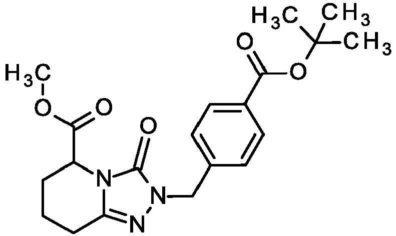

Zu Methyl-(5RS)-3-oxo-2,3,5,6,7,8-hexahydro[1,2,4]triazolo[4,3-a]pyridin-5-carboxylat (Racemat) (300 mg, 1.52 mmol) und Cäsiumcarbonat (744 mg, 2.28 mmol) in 15 ml Acetonitril wurde tert-Butyl-4-(brommethyl)benzoat (433 mg, 1.60 mmol) gegeben, dann das Reaktionsgemisch über Nacht bei Raumtemperatur gerührt. Zur Aufarbeitung wurde der vorhandene Niederschlag abfiltriert, das Filtrat mit Ethylacetat/Wasser versetzt und extrahiert. Die wässrige Phase wurde noch zweimal mit Ethylacetat extrahiert. Die vereinigten organischen Phasen wurden mit gesättigter Natriumchloridlösung gewaschen, mit Natriumsulfat getrocknet, filtriert und im Vakuum eingeengt und getrocknet. Es wurden 676 mg (88 % Reinheit, 101 % d. Th.) der Titelverbindung erhalten, die so weiter verwendet wurden.
LC-MS (Methode 3): Rₜ = 1.78 min; MS (ESIpos): m/z = 388 [M+H]⁺
¹H-NMR (400 MHz, DMSO-d6) δ [ppm]: -0.008 (1.08), 0.008 (1.04), 1.175 (0.71), 1.537 (16.00), 1.988 (1.29), 3.286 (1.03), 3.709 (5.39), 4.915 (1.64), 7.323 (0.99), 7.344 (1.09), 7.857 (1.29), 7.878 (1.18).

### Intermediat 103

### tert-Butyl-(5RS)-2-(4-methylbenzyl)-3-oxo-2,3,5,6,7,8-hexahydro[1,2,4]triazolo[4,3-a]pyridin-5-carboxylat (Racemat)

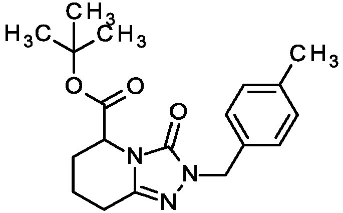

Zu tert-Butyl-(5RS)-3-oxo-2,3,5,6,7,8-hexahydro[1,2,4]triazolo[4,3-a]pyridin-5-carboxylat (Racemat) (5.00 g, 20.9 mmol) und Cäsiumcarbonat (10.2 g, 31.3 mmol) in 180 ml Acetonitril wurde 1-(Brommethyl)-4-methylbenzol (4.06 g, 21.9 mmol) zugegeben, dann das Reaktionsgemisch über Nacht bei Raumtemperatur gerührt. Zur Aufarbeitung wurde der vorhandene Niederschlag abfiltriert, das Filtrat am Rotationsverdampfer weitgehend eingeengt, der Rückstand mit Ethylacetat/Wasser aufgenommen und extrahiert. Dabei wurden zur besseren Phasentrennung gesättigte wässrige Natriumchloridlösung und etwas Methanol zugesetzt. Die wässrige Phase wurde noch zweimal mit Ethylacetat extrahiert. Die vereinigten organischen Phasen wurden mit gesättigter wässriger Natriumchloridlösung gewaschen, mit Natriumsulfat getrocknet, filtriert und im Vakuum eingeengt und getrocknet. Es wurden 7.14 g (94 % Reinheit, 94 % d. Th.) der Titelverbindung erhalten, die so weiter verwendet wurden.
LC-MS (Methode 6): Rₜ = 1.80 min; MS (ESIpos): m/z = 344 [M+H]⁺
¹H-NMR (400 MHz, DMSO-d6) δ [ppm]: 1.396 (16.00), 1.402 (2.28), 2.270 (4.79), 4.771 (2.95), 7.129 (7.75).

### Intermediat 104

### tert-Butyl-(5RS)-2-[(1RS)-1-(4-chlorphenyl)ethyl]-3-oxo-2,3,5,6,7,8-hexahydro[1,2,4]triazolo[4,3-a]pyridin-5-carboxylat (Diastereomerengemisch; 4 Isomere)

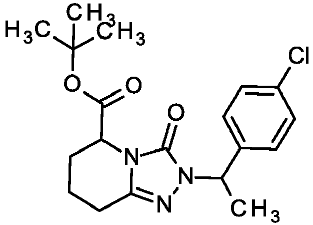

Zu tert-Butyl-(5RS)-3-oxo-2,3,5,6,7,8-hexahydro[1,2,4]triazolo[4,3-a]pyridin-5-carboxylat (Racemat) (500 mg, 2.09 mmol) und Cäsiumcarbonat (1.02 g, 3.13 mmol) in 30 ml Acetonitril wurde 1-[(1RS)-1-Bromethyl]-4-chlorbenzol (Racemat) (550 mg, 2.51 mmol) gegeben, dann das Reaktionsgemisch über Nacht bei Raumtemperatur gerührt. Zur Aufarbeitung wurde der vorhandene Niederschlag abfiltriert, das Filtrat am Rotationsverdampfer weitgehend eingeengt, der Rückstand mit Ethylacetat/Wasser aufgenommen und extrahiert. Dabei wurden zur besseren Phasentrennung gesättigte wässrige Natriumchloridlösung und etwas Methanol zugesetzt. Die wässrige Phase wurde noch zweimal mit Ethylacetat extrahiert. Die vereinigten organischen Phasen wurden mit gesättigter Natriumchloridlösung gewaschen, mit Natriumsulfat getrocknet, filtriert und im Vakuum eingeengt und getrocknet. Es wurden 755 mg (87 % Reinheit, 83 % d. Th.) der Titelverbindung als Diastereomerengemisch erhalten, die so weiter verwendet wurden.
LC-MS (Methode 8): Rₜ = 3.28 min; MS (ESIpos): m/z = 378 [M+H]⁺; Rₜ = 3.33 min; MS (ESIpos): m/z = 378 [M+H]⁺
¹H-NMR (400 MHz, DMSO-d6) δ [ppm]: -0.008 (1.98), 0.008 (1.51), 1.157 (0.78), 1.175 (1.54), 1.192 (0.85), 1.329 (15.89), 1.342 (1.34), 1.405 (16.00), 1.416 (1.42), 1.438 (1.47), 1.597 (2.79), 1.603 (2.55), 1.614 (2.71), 1.620 (2.46), 1.988 (2.92), 2.063 (1.04), 2.661 (0.78), 3.287 (1.01), 4.020 (0.72), 4.038 (0.70), 4.403 (0.81), 4.418 (0.94), 7.323 (1.58), 7.344 (3.21), 7.378 (2.24), 7.385 (2.41), 7.399 (1.19), 7.406 (1.69).

### Intermediat 105

### tert-Butyl-(5R, S)-3-oxo-2-(4-sulfamoylbenzyl)-2,3,5,6,7,8-hexahydro[1,2,4]triazolo[4,3-a]pyridin-5-carboxylat (Racemat)

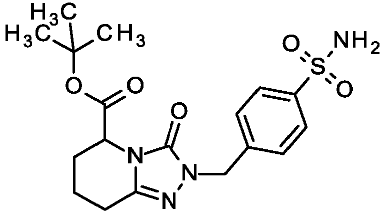

Zu tert-Butyl-(5RS)-3-oxo-2,3,5,6,7,8-hexahydro[1,2,4]triazolo[4,3-a]pyridin-5-carboxylat (Racemat) (500 mg, 2.09 mmol) in 10 ml Acetonitril wurden Cäsiumcarbonat (1.02 g, 3.13 mmol) und 4-(Brommethyl)benzolsulfonamid (627 mg, 2.51 mmol) zugegeben, dann das Reaktionsgemisch zweimal über Nacht unter Argon bei Raumtemperatur gerührt. Zur Aufarbeitung wurde der Ansatz mit Wasser versetzt und das Gemisch zweimal mit Dichlormethan extrahiert. Die vereinigten organischen Phasen wurden mit Magnesiumsulfat getrocknet, filtriert und im Vakuum eingeengt. Der verbliebene Rückstand wurde über präparative HPLC getrennt (Säule: RP, Chromatorex C18, 250x40mm 10µm. Flow: 50ml/min. Gradient (A= Wasser+ 0,1% Ameisensäure , B= Acetonitril): 0min 10% B, 5min 10%B, 27min 98% B, 35min 98 % B, 35,01min 10%B , 38min 10%B. Laufzeit pro Trennung 38min. Detektion: 210 nm) Es wurden 755 mg (87 % Reinheit, 83 % d. Th.). Durch Einengen der Produktfraktionen wurden 37 mg (4.3 % d. Th.) der Titelverbindung erhalten.
LC-MS (Methode 6): Rₜ = 1.35 min; MS (ESlneg): m/z = 407 [M-H]⁻
¹H-NMR (400 MHz, DMSO-d6) δ [ppm]: 1.404 (16.00), 2.072 (1.17), 4.451 (0.63), 4.920 (2.40), 7.331 (2.07), 7.401 (1.29), 7.422 (1.42), 7.772 (1.69), 7.793 (1.46).

### Intermediat 106

### tert-Butyl-(5RS)-2-[4-(methylsulfanyl)benzyl]-3-oxo-2,3,5,6,7,8-hexahydro[1,2,4]triazolo[4,3-a]pyridin-5-carboxylat (Racemat)

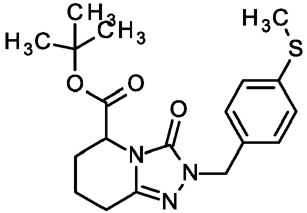

Zu tert-Butyl-(5RS)-3-oxo-2,3,5,6,7,8-hexahydro[1,2,4]triazolo[4,3-a]pyridin-5-carboxylat (Racemat) (500 mg, 2.09 mmol) und Cäsiumcarbonat (1.02 g, 3.13 mmol) in 18 ml Acetonitril wurden 1-(Brommethyl)-4-(methylsulfanyl)benzol (476 mg, 2.19 mmol) zugegeben, dann das Reaktionsgemisch 4h bei Raumtemperatur gerührt. Zur Aufarbeitung wurde der vorhandene Niederschlag abfiltriert, das Filtrat mit Ethylacetat/Wasser versetzt und extrahiert. Die wässrige Phase wurde noch zweimal mit Ethylacetat extrahiert. Die vereinigten organischen Phasen wurden mit gesättigter Natriumchloridlösung gewaschen, mit Natriumsulfat getrocknet, filtriert und im Vakuum eingeengt und getrocknet. Es wurden 776 mg (92 % Reinheit, 91 % d. Th.) der Titelverbindung erhalten, die so weiter verwendet wurden.
LC-MS (Methode 3): Rₜ = 1.88 min; MS (ESIpos): m/z = 376 [M+H]⁺
¹H-NMR (500 MHz, DMSO-d6) δ [ppm]: 1.395 (16.00), 1.403 (1.34), 1.988 (0.68), 2.452 (7.33), 4.783 (2.62), 7.181 (0.76), 7.198 (1.88), 7.221 (2.16), 7.238 (0.85).

### Intermediat 107

### tert-Butyl-(5RS)-2-[(6-chlorpyridin-3-yl)methyl]-3-oxo-2,3,5,6,7,8-hexahydro[1,2,4]triazolo[4,3-a]pyridin-5-carboxylat (Racemat)

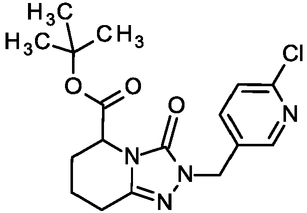

Zu tert-Butyl-(5RS)-3-oxo-2,3,5,6,7,8-hexahydro[1,2,4]triazolo[4,3-a]pyridin-5-carboxylat (Racemat) (1.00 g, 4.18 mmol) und Cäsiumcarbonat (2.04 g, 6.27 mmol) in 25 ml Acetonitril wurden 2-Chlor-5-(chlormethyl)pyridin (711 mg, 4.39 mmol) zugegeben, dann das Reaktionsgemisch über Nacht bei Raumtemperatur gerührt. Zur Aufarbeitung wurde der Ansatz auf Wasser gegeben und das Gemisch zweimal mit Ethylacetat extrahiert. Die vereinigten organischen Phasen wurden mit gesättigter Natriumchloridlösung gewaschen, mit Magnesiumsulfat getrocknet, filtriert und im Vakuum eingeengt und getrocknet. Es wurden 1.48 g (89 % Reinheit, 87 % d. Th.) der Titelverbindung erhalten, die so weiter verwendet wurden.
LC-MS (Methode 4): Rₜ = 0.84 min; MS (ESIpos): m/z = 365 [M+H]⁺
¹H-NMR (400 MHz, DMSO-d6) δ [ppm]: 1.369 (0.99), 1.387 (16.00), 1.405 (3.22), 1.988 (0.89), 2.060 (0.76), 2.496 (4.74), 2.501 (6.51), 2.505 (5.26), 4.443 (0.68), 4.910 (2.74), 7.510 (0.80), 7.530 (0.98), 8.313 (0.82), 8.319 (0.82).

### Intermediat 108

### tert-Butyl-(5RS)-2-[(6-cyanpyridin-3-yl)methyl]-3-oxo-2,3,5,6,7,8-hexahydro[1,2,4]triazolo[4,3-a]pyridin-5-carboxylat (Racemat)

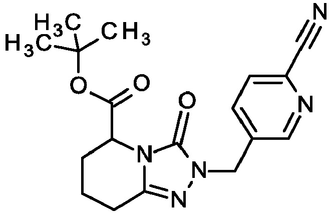

Zu tert-Butyl-(5RS)-3-oxo-2,3,5,6,7,8-hexahydro[1,2,4]triazolo[4,3-a]pyridin-5-carboxylat (Racemat) (200 mg, 836 µmοl) und Cäsiumcarbonat (409 mg, 1.25 mmol) in 8.0 ml Acetonitril wurden 5-(Brommethyl)pyridin-2-carbonitril (173 mg, 878 µmοl) zugegeben, dann das Reaktionsgemisch zunächst 2h, dann über Nacht bei Raumtemperatur gerührt. Sodann wurde noch einmal 5-(Brommethyl)pyridin-2-carbonitril (32.9 mg, 167 µmοl) zugegeben (Umsatzkontrolle durch HPLC). Zur Aufarbeitung nach weiteren 2h Rühren bei Raumtemperatur wurde der vorhandene Niederschlag abfiltriert, das Filtrat mit Ethylacetat/Wasser versetzt und extrahiert. Die wässrige Phase wurde noch zweimal mit Ethylacetat extrahiert. Die vereinigten organischen Phasen wurden mit gesättigter Natriumchloridlösung gewaschen, mit Natriumsulfat getrocknet, filtriert und im Vakuum eingeengt und getrocknet. Es wurden 303 mg (> 100 % d. Th.) der Titelverbindung erhalten.
LC-MS (Methode 4): Rₜ = 0.79 min; MS (ESIpos): m/z = 356 [M+H]⁺
¹H-NMR (400 MHz, DMSO-d6) δ [ppm]: -0.008 (1.79), 0.008 (1.80), 1.175 (0.81), 1.394 (16.00), 1.406 (1.87), 1.988 (1.44), 3.287 (1.33), 4.459 (0.60), 5.033 (2.16), 8.034 (0.73), 8.037 (0.79).

### Intermediat 109

### tert-Butyl-(5RS)-2-[4-chlor-3-(trifluormethyl)benzyl]-3-oxo-2,3,5,6,7,8-hexahydro[1,2,4]triazolo[4,3-a]pyridin-5-carboxylat (Racemat)

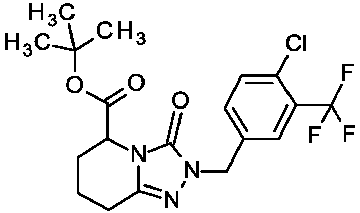

Zu tert-Butyl-(5RS)-3-oxo-2,3,5,6,7,8-hexahydro[1,2,4]triazolo[4,3-a]pyridin-5-carboxylat (Racemat) (200 mg, 836 µmοl) und Cäsiumcarbonat (409 mg, 1.25 mmol) in 8.0 ml Acetonitril wurden 4-(Brommethyl)-1-chlor-2-(trifluormethyl)benzol (274 mg, 1.00 mmol) zugegeben, dann das Reaktionsgemisch zunächst 4h, dann über Nacht bei Raumtemperatur gerührt (Umsatzkontrolle durch HPLC). Zur Aufarbeitung wurde der vorhandene Niederschlag abfiltriert, das Filtrat mit Ethylacetat/Wasser versetzt und extrahiert. Die wässrige Phase wurde noch zweimal mit Ethylacetat extrahiert. Die vereinigten organischen Phasen wurden mit gesättigter Natriumchloridlösung gewaschen, mit Natriumsulfat getrocknet, filtriert und im Vakuum eingeengt und getrocknet. Es wurden 323 mg (87 % d. Th.) der Titelverbindung erhalten, die so weiter verwendet wurden.
LC-MS (Methode 3): Rₜ = 2.12 min; MS (ESIpos): m/z = 376 [M+H]⁺
¹H-NMR (400 MHz, DMSO-d6) δ [ppm]: 1.377 (16.00), 4.946 (1.13), 4.962 (1.09).

### Intermediat 110

### tert-Butyl-(5RS)-2-(3-chlorbenzyl)-3-oxo-2,3,5,6,7,8-hexahydro[1,2,4]triazolo[4,3-a]pyridin-5-carboxylat (Racemat)

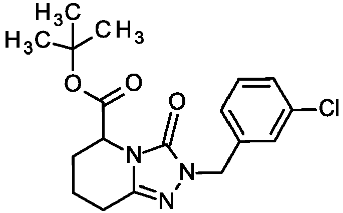

Zu tert-Butyl-(5RS)-3-oxo-2,3,5,6,7,8-hexahydro[1,2,4]triazolo[4,3-a]pyridin-5-carboxylat (Racemat) (200 mg, 836 µmοl) und Cäsiumcarbonat (409 mg, 1.25 mmol) in 8.0 ml Acetonitril wurden 1-(Brommethyl)-3-chlorbenzol (130 µl, 1.0 mmol) zugegeben, dann das Reaktionsgemisch zunächst 4h, dann über Nacht bei Raumtemperatur gerührt (Umsatzkontrolle durch HPLC). Zur Aufarbeitung wurde der vorhandene Niederschlag abfiltriert, das Filtrat mit Ethylacetat/Wasser versetzt und extrahiert. Die wässrige Phase wurde noch zweimal mit Ethylacetat extrahiert. Die vereinigten organischen Phasen wurden mit gesättigter Natriumchloridlösung gewaschen, mit Natriumsulfat getrocknet, filtriert und im Vakuum eingeengt und getrocknet. Es wurden 284 mg (84 % Reinheit, 78 % d. Th.) der Titelverbindung erhalten, die so weiter verwendet wurden.
LC-MS (Methode 3): Rₜ = 1.91 min; MS (ESIpos): m/z = 308 [M+H]⁺
¹H-NMR (400 MHz, DMSO-d6) δ [ppm]: 1.395 (16.00), 1.406 (0.97), 1.988 (0.71), 4.856 (1.40), 4.862 (1.37), 7.360 (1.24), 7.379 (0.68).

### Intermediat 111

### tert-Butyl-(5RS)-2-(3,4-dichlorbenzyl)-3-oxo-2,3,5,6,7,8-hexahydro[1,2,4]triazolo[4,3-a]pyridin-5-carboxylat (Racemat)

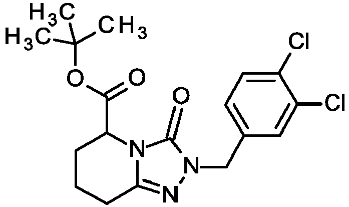

Zu tert-Butyl-(5RS)-3-oxo-2,3,5,6,7,8-hexahydro[1,2,4]triazolo[4,3-a]pyridin-5-carboxylat (Racemat) (200 mg, 836 µmοl) und Cäsiumcarbonat (409 mg, 1.25 mmol) in 8.0 ml Acetonitril wurden 4-(Brommethyl)-1,2-dichlorbenzol (241 mg, 1.00 mmol) zugegeben, dann das Reaktionsgemisch über ein Wochenende bei Raumtemperatur gerührt. Zur Aufarbeitung wurde der vorhandene Niederschlag abfiltriert, das Filtrat mit Ethylacetat/Wasser versetzt und extrahiert. Die wässrige Phase wurde noch zweimal mit Ethylacetat extrahiert. Die vereinigten organischen Phasen wurden mit gesättigter Natriumchloridlösung gewaschen, mit Natriumsulfat getrocknet, filtriert und im Vakuum eingeengt und getrocknet. Es wurden 330 mg (78 % Reinheit, 77 % d. Th.) der Titelverbindung erhalten, die so weiter verwendet wurden.
LC-MS (Methode 4): Rₜ = 1.08 min; MS (ESIpos): m/z = 398 [M+H]⁺
¹H-NMR (400 MHz, DMSO-d6) δ [ppm]: 1.392 (16.00), 4.867 (1.86), 7.503 (0.82), 7.508 (0.81), 7.610 (1.04), 7.631 (0.95).

### Intermediat 112

### Methyl -(5RS)-methyl-2-(4-methylbenzyl)-3-oxo-2,3,5,6,7,8-hexahydro[1,2,4]triazolo[4,3-a]pyridin-5-carboxylat (Racemat)

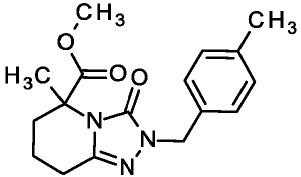

### Methyl-(5RS)-methyl-3-oxo-2,3,5,6,7,8-hexahydro[1,2,4]triazolo[4,3-a]pyridin-5-carboxylat

(Racemat) (40.0 mg, 189 µmοl) wurde in Acetonitril (2.0 ml) vorgelegt. Cäsiumcarbonat (67.9 mg, 208 µmοl) und 1-(Brommethyl)-4-methylbenzol (36.8 mg, 199 µmοl) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 32.0 mg (54 % d. Th.) der Titelverbindung erhalten.
LC-MS (Methode 3): Rₜ = 1.59 min; MS (ESIpos): m/z = 316 [M+H]⁺

### Intermediat 113

### tert-Butyl-(5RS,7RS)-7-methyl-3-oxo-2-{[6-(trifluormethyl)pyridin-3-yl]methyl}-2,3,5,6,7,8-hexahydro[1,2,4]triazolo[4,3-a]pyridin-5-carboxylat (Diastereomerengemisch; 4 Isomere)

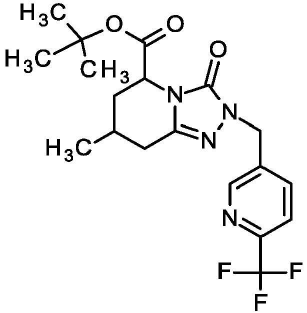

tert-Butyl-(5RS,7RS)-7-methyl-3-oxo-2,3,5,6,7,8-hexahydro[1,2,4]triazolo[4,3-a]pyridin-5-carboxylat (Diastereomerengemisch; 4 Isomere) (238 mg, 940 µmοl) wurde in Acetonitril (10 ml) vorgelegt. Cäsiumcarbonat (612 mg, 1.88 mmol) und 5-(Brommethyl)-2-(trifluormethyl)pyridin (237 mg, 987 µmοl) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Magnesiumsulfat getrocknet, filtriert und das Filtrat eingeengt. Es wurden 383 mg (92 % Reinheit, 91 % d. Th.) der Titelverbindung erhalten.
LC-MS (Methode 1): Rₜ = 1.30 min; MS (ESIpos): m/z = 357 [M-tBu+H]⁺
¹H-NMR (400 MHz, DMSO-d6) δ [ppm]: -0.008 (1.52), 0.008 (1.72), 1.012 (2.06), 1.028 (2.14), 1.371 (0.43), 1.406 (16.00), 2.217 (0.48), 2.230 (0.62), 2.258 (0.49), 4.295 (0.45), 4.305 (0.44), 4.835 (0.45), 5.019 (2.25), 7.922 (1.31), 7.936 (0.83), 8.668 (0.83).

### Intermediat 114

### Ethyl-(5RS,6RS)-6-methyl-2-(4-methylbenzyl)-3-oxo-2,3,5,6,7,8-hexahydro[1,2,4]triazolo[4,3-a]pyridin-5-carboxylat (Diastereomerengemisch; 4 Isomere)

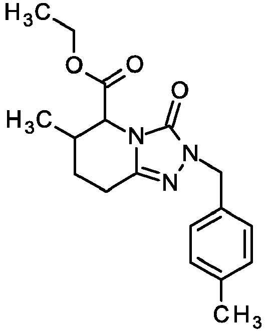

Ethyl-(5RS,6RS)-6-methyl-3-oxo-2,3,5,6,7,8-hexahydro[1,2,4]triazolo[4,3-a]pyridin-5-carboxylat (Diastereomerengemisch; 4 Isomere) (80.0 mg, 355 µmοl) wurde in Acetonitril (3.0 ml) vorgelegt. Cäsiumcarbonat (174 mg, 533 µmοl) und 1-(Brommethyl)-4-methylbenzol (69.0 mg, 373 µmοl) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Magnesiumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 108 mg (71 % Reinheit, 65 % d. Th.) der Titelverbindung erhalten.
LC-MS (Methode 4): Rₜ = 0.94 min; MS (ESIpos): m/z = 330 [M+H]⁺

### Intermediat 115

### Ethyl-(5RS,7RS)-2-(4-methylbenzyl)-3-oxo-7-(trifluormethyl)-2,3,5,6,7,8-hexahydro[1,2,4]triazolo[4,3-a]pyridin-5-carboxylat (Diastereomerengemisch; 4 Isomere)

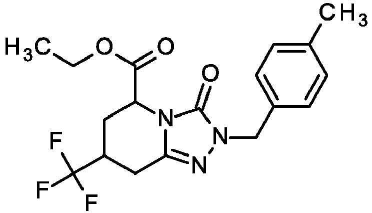

Ethyl-(5RS,7RS)-3-oxo-7-(trifluormethyl)-2,3,5,6,7,8-hexahydro[1,2,4]triazolo[4,3-a]pyridin-5-carboxylat (Diastereomerengemisch; 4 Isomere) (80.0 mg, 287 µmοl) wurde in Acetonitril (3.5 ml) vorgelegt. Cäsiumcarbonat (140 mg, 430 µmοl) und 1-(Brommethyl)-4-methylbenzol (55.7 mg, 301 µmοl) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Magnesiumsulfat getrocknet, filtriert und das Filtrat eingeengt. Es wurden 102 mg (81 % Reinheit, 75 % d. Th.) der Titelverbindung erhalten.
LC-MS (Methode 1): Rₜ = 1.33 min; MS (ESIpos): m/z = 384 [M+H]⁺

### Intermediat 116

### Methyl-(5RS)-2-[2-(4-methylphenyl)ethyl]-3-oxo-2,3,5,6,7,8-hexahydro[1,2,4]triazolo[4,3-a]pyridin-5-carboxylat (Racemat)

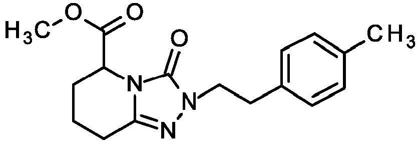

Methyl-(5RS)-3-oxo-2,3,5,6,7,8-hexahydro[1,2,4]triazolo[4,3-a]pyridin-5-carboxylat (Racemat) (180 mg, 913 µmοl) wurde in Acetonitril (8.0 ml) vorgelegt. Cäsiumcarbonat (312 mg, 959 µmοl) und 1-(2-Bromethyl)-4-methylbenzol (200 mg, 1.00 mmol, CAS 6529-51-7) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 88.0 mg (30 % d. Th.) der Titelverbindung erhalten.
LC-MS (Methode 4): Rₜ = 0.85 min; MS (ESIpos): m/z = 316 [M+H]⁺
¹H-NMR (500 MHz, DMSO-d6) δ [ppm]: 1.356 (0.66), 2.257 (8.94), 2.567 (0.54), 2.579 (0.46), 2.588 (0.48), 2.649 (0.66), 2.870 (0.95), 2.885 (1.81), 2.900 (1.02), 3.680 (12.74), 3.783 (0.71), 3.788 (0.75), 3.798 (1.46), 3.804 (1.47), 3.813 (0.68), 3.819 (0.66), 4.525 (0.70), 4.533 (0.78), 4.538 (0.88), 4.546 (0.69), 7.085 (16.00).

### Intermediat 117

### tert-Butyl-(5RS)-3-oxo-2-{[4-(trifluormethyl)cyclohexyl]methyl}-2,3,5,6,7,8-hexahydro[1,2,4]triazolo[4,3-a]pyridin-5-carboxylat (Diastereomerengemisch; 4 Isomere)

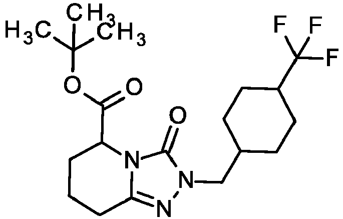

Zu tert-Butyl-(5RS)-3-oxo-2,3,5,6,7,8-hexahydro[1,2,4]triazolo[4,3-a]pyridin-5-carboxylat (Racemat) (250 mg, 1.04 mmol) und Cäsiumcarbonat (511 mg, 1.57 mmol) in 15 ml Acetonitril wurde 1-(Brommethyl)-4-(trifluormethyl)cyclohexan (Diastereomerengemisch; 2 Isomere) (307 mg, 1.25 mmol) zugegeben, dann das Reaktionsgemisch über Nacht, dann weitere 24h bei Raumtemperatur gerührt. Zur weiteren Umsetzung wurden noch zweimal 1-(Brommethyl)-4-(trifluormethyl)cyclohexan (je 128 mg, 522 µmοl) und Cäsiumcarbonat (je 272 mg, 836 µmοl) zugegeben und jeweils weitere 24h gerührt (Umsatzkontrolle durch HPLC). Zur Aufarbeitung wurde der Ansatz eingeengt, der Rückstand mit Ethylacetat/Wasser aufgenommen und extrahiert. Die wässrige Phase wurde noch zweimal mit Ethylacetat extrahiert. Die vereinigten organischen Phasen wurden mit gesättigter Natriumchloridlösung gewaschen, mit Natriumsulfat getrocknet, filtriert und im Vakuum eingeengt und getrocknet. Es wurden 466 mg (81 % Reinheit, 90 % d. Th.) der Titelverbindung erhalten, die so weiter verwendet wurden.
LC-MS (Methode 4): Rₜ = 1.08 min; MS (ESIpos): m/z = 404 [M+H]⁺
¹H-NMR (400 MHz, DMSO-d6) δ [ppm]: -0.008 (1.25), 1.395 (16.00), 1.406 (2.62), 1.420 (1.08), 1.499 (1.33), 1.508 (0.84), 1.522 (0.93), 1.546 (0.75), 1.595 (0.83), 1.606 (1.20), 1.625 (1.14), 1.643 (1.63), 3.287 (0.76), 3.563 (2.36), 3.581 (2.34).

### Intermediat 118

### tert-Butyl-(5RS)-2-[(4,4-difluorcyclohexyl)methyl]-3-oxo-2,3,5,6,7,8-hexahydro[1,2,4]triazolo[4,3-a]pyridin-5-carboxylat (Racemat)

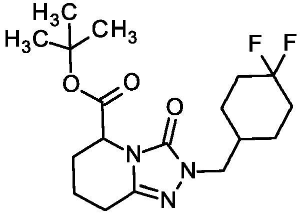

Zu tert-Butyl-(5RS)-3-oxo-2,3,5,6,7,8-hexahydro[1,2,4]triazolo[4,3-a]pyridin-5-carboxylat (Racemat) (67.4 mg, 282 µmοl) und Cäsiumcarbonat (138 mg, 422 µmοl) in 4.0 ml Acetonitril wurden 4-(Brommethyl)-1,1-difluorcyclohexan (72.0 mg, 338 µmοl) zugegeben, dann das Reaktionsgemisch über ein Wochenende bei Raumtemperatur gerührt. Zur Aufarbeitung wurde der vorhandene Niederschlag abfiltriert, das Filtrat eingeengt, der Rückstand mit Ethylacetat/Wasser aufgenommen und extrahiert. Die wässrige Phase wurde noch zweimal mit Ethylacetat extrahiert. Die vereinigten organischen Phasen wurden mit gesättigter Natriumchloridlösung gewaschen, mit Natriumsulfat getrocknet, filtriert und im Vakuum eingeengt und getrocknet. Der Rückstand wurde durch präparative HPLC getrennt (Säule: Kromasil C18, 125x30mm, 10µm, Eluent: Acetonitril (B) / Wasser + 0,1 % TFA (A), Gradient: 0min 90% A, 6min 90% A, 18min 5%A, 20min 5%A, 21min 90%A, Fluss: 75ml/min, Detektor: 210nm). Durch Vereinigen der produkthaltigen Fraktionen wurden nach Entfernen der Lösungsmittel im Vakuum 41.0 mg (39 % d. Th.) der Titelverbindung erhalten.
LC-MS (Methode 6): Rₜ = 1.76 min; MS (ESIpos): m/z = 372 [M+H]⁺
¹H-NMR (400 MHz, DMSO-d6) δ [ppm]: 1.396 (16.00), 2.072 (2.01), 3.528 (1.35), 3.545 (1.33), 4.399 (0.62).

### Intermediat 119

### (5S)-2-[(6-Chlorpyridin-3-yl)methyl]-3-oxo-2,5,6,7-tetrahydro-3H-pyrrolo[2,1-c][1,2,4]triazol-5-carbonsäure (Enantiomer)

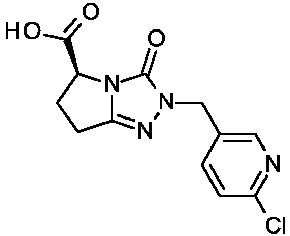

Methyl-(5S)-2-[(6-chlorpyridin-3-yl)methyl]-3-oxo-2,5,6,7-tetrahydro-3H-pyrrolo[2,1-c][1,2,4]triazol-5-carboxylat (Enantiomer) (430 mg, 1.39 mmol) wurde in THF (10 ml) vorgelegt und Lithiumhydroxid (167 mg, 6.96 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 185 mg (45 % d. Th.) der Titelverbindung erhalten.
LC-MS (Methode 3): Rₜ = 0.71 min; MS (ESIpos): m/z = 295 [M+H]⁺

### Intermediat 120

### (5S)-3-Oxo-2-{[6-(trifluormethyl)pyridin-3-yl]methyl}-2,5,6,7-tetrahydro-3H-pyrrolo[2,1-c][1,2,4]triazol-5-carbonsäure (Enantiomer)

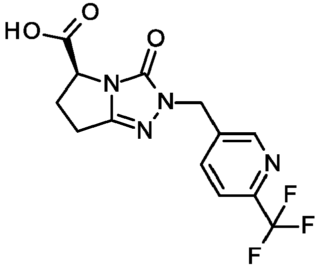

Methyl-(5S)-3-oxo-2-{[6-(trifluormethyl)pyridin-3-yl]methyl}-2,5,6,7-tetrahydro-3H-pyrrolo[2,1 - c][1,2,4]triazol-5-carboxylat (Enantiomer) (441 mg, 1.29 mmol) wurde in THF (10 ml) vorgelegt und Lithiumhydroxid (154 mg, 6.44 mmol) gelöst in Wasser zugegeben. Das Reaktionsgemisch wurde nach Rühren über Nacht bei Raumtemperatur mit Wasser, 1N wässriger Salzsäure und gesättigter wässriger Natriumchlorid-Lösung versetzt. Die organische Phase wurde abgetrennt und die wässrige Phase dreimal mit Ethylacetat extrahiert. Die vereinigten organischen Phasen wurden über Natriumsulfat getrocknet, filtriert und das Filtrat eingeengt. Es wurden 376 mg (89 % d. Th.) der Titelverbindung erhalten.
LC-MS (Methode 3): Rₜ = 0.85 min; MS (ESIpos): m/z = 329 [M+H]⁺

### Intermediat 121

### (5S)-2-(4-Methylbenzyl)-3-oxo-2,5,6,7-tetrahydro-3H-pyrrolo[2,1-c][1,2,4]triazol-5-carbonsäure (Enantiomer)

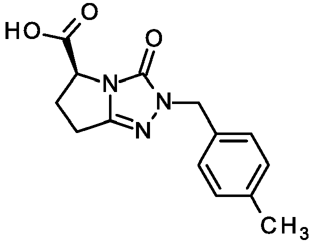

Methyl-(5S)-2-(4-methylbenzyl)-3-oxo-2,5,6,7-tetrahydro-3H-pyrrolo[2,1-c][1,2,4]triazol-5-carboxylat (Enantiomer) (280 mg, 975 µmοl) wurde in THF (4.0 ml) vorgelegt und Lithiumhydroxid (117 mg, 4.87 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 251 mg (94 % d. Th.) der Titelverbindung erhalten.
LC-MS (Methode 4): Rₜ = 0.59 min; MS (ESIpos): m/z = 274 [M+H]⁺
¹H-NMR (400 MHz, DMSO-d6) δ [ppm]: 0.008 (0.73), 1.157 (0.99), 1.174 (2.01), 1.192 (1.01), 1.988 (3.62), 2.274 (10.45), 2.423 (0.43), 2.430 (0.52), 2.439 (0.46), 2.446 (0.46), 2.455 (0.52), 2.463 (0.55), 2.708 (1.26), 2.720 (0.84), 2.730 (2.22), 2.745 (1.81), 2.822 (0.50), 2.841 (0.44), 2.853 (0.62), 2.876 (0.50), 4.020 (0.87), 4.038 (0.87), 4.603 (1.09), 4.610 (1.19), 4.625 (1.25), 4.633 (1.04), 4.708 (0.51), 4.747 (2.97), 4.761 (2.99), 4.800 (0.51), 7.140 (16.00), 13.346 (0.50).

### Intermediat 122

### (5S)-2-(3-Chlor-4-fluorbenzyl)-3-oxo-2,5,6,7-tetrahydro-3H-pyrrolo[2,1-c][1,2,4]triazol-5-carbonsäure (Enantiomer)

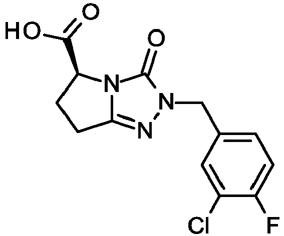

Methyl-(5S)-2-(3-chlor-4-fluorbenzyl)-3-oxo-2,5,6,7-tetrahydro-3H-pyrrolo[2,1-c][1,2,4]triazol-5-carboxylat (Enantiomer) (460 mg, 1.41 mmol) wurde in THF (10 ml) vorgelegt und Lithiumhydroxid (169 mg, 7.06 mmol) gelöst in Wasser zugegeben. Das Reaktionsgemisch wurde nach Rühren über Nacht bei Raumtemperatur mit Wasser, 1N wässriger Salzsäure und gesättigter wässriger Natriumchlorid-Lösung versetzt. Die organische Phase wurde abgetrennt und die wässrige Phase dreimal mit Ethylacetat extrahiert. Die vereinigten organischen Phasen wurden über Natriumsulfat getrocknet, filtriert und das Filtrat eingeengt. Es wurden 281 mg (64 % d. Th.) der Titelverbindung erhalten.
LC-MS (Methode 3): Rₜ = 1.12 min; MS (ESIpos): m/z = 312 [M+H]⁺

### Intermediat 123

### (5S)-2-[(2-Chlorpyridin-4-yl)methyl]-3-oxo-2,5,6,7-tetrahydro-3H-pyrrolo[2,1-c][1,2,4]triazol-5-carbonsäure (Enantiomer)

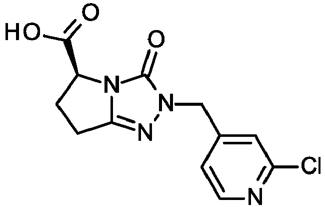

Methyl-(5S)-2-[(2-chlorpyridin-4-yl)methyl]-3-oxo-2,5,6,7-tetrahydro-3H-pyrrolo[2,1-c][1,2,4]triazol-5-carboxylat (Enantiomer) (250 mg, 810 µmοl) wurde in THF (7.5 ml) vorgelegt und Lithiumhydroxid (97.0 mg, 4.05 mmol) gelöst in Wasser zugegeben. Das Reaktionsgemisch wurde nach Rühren über Nacht bei Raumtemperatur mit Wasser, 1N wässriger Salzsäure und gesättigter wässriger Natriumchlorid-Lösung versetzt. Die organische Phase wurde abgetrennt und die wässrige Phase dreimal mit Ethylacetat extrahiert. Die vereinigten organischen Phasen wurden über Natriumsulfat getrocknet, filtriert und das Filtrat eingeengt. Es wurden 230 mg (96 % d. Th.) der Titelverbindung erhalten.
LC-MS (Methode 4): Rₜ = 0.43 min; MS (ESIpos): m/z = 295 [M+H]⁺
¹H-NMR (400 MHz, DMSO-d6) δ [ppm]: -0.149 (0.93), 0.145 (0.82), 2.371 (1.71), 2.715 (1.98), 2.758 (4.35), 2.781 (8.27), 2.795 (7.03), 2.838 (1.67), 2.861 (2.25), 2.891 (2.91), 2.915 (1.98), 4.532 (0.78), 4.662 (3.88), 4.670 (4.35), 4.685 (4.43), 4.692 (3.88), 4.887 (0.78), 4.931 (16.00), 7.253 (5.63), 7.266 (5.79), 7.340 (10.10), 8.377 (7.65), 8.389 (7.61), 11.255 (1.01).

### Intermediat 124

### (5S)-3-Oxo-2-[3-(trifluormethyl)benzyl]-2,5,6,7-tetrahydro-3H-pyrrolo[2,1-c][1,2,4]triazol-5-carbonsäure (Enantiomer)

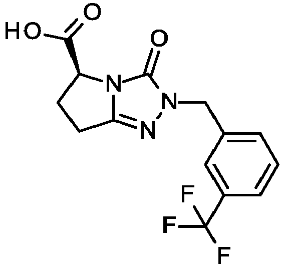

Methyl-(5S)-3-oxo-2-[3-(trifluormethyl)benzyl]-2,5,6,7-tetrahydro-3H-pyrrolo[2,1-c][1,2,4]triazol-5-carboxylat (Enantiomer) (150 mg, 440 µmοl) wurde in THF (4.5 ml) vorgelegt und Lithiumhydroxid (52.6 mg, 2.20 mmol) gelöst in Wasser zugegeben. Das Reaktionsgemisch wurde nach Rühren über Nacht bei Raumtemperatur mit Wasser, 1N wässriger Salzsäure und gesättigter wässriger Natriumchlorid-Lösung versetzt. Die organische Phase wurde abgetrennt und die wässrige Phase dreimal mit Ethylacetat extrahiert. Die vereinigten organischen Phasen wurden über Natriumsulfat getrocknet, filtriert und das Filtrat eingeengt. Es wurden 129 mg (90 % d. Th.) der Titelverbindung erhalten.
LC-MS (Methode 4): Rₜ = 0.70 min; MS (ESIpos): m/z = 328 [M+H]⁺

### Intermediat 125

### (5RS)-2-(4-Methylbenzyl)-3-oxo-2,3,5,6,7,8-hexahydro[1,2,4]triazolo[4,3-a]pyridin-5-carbonsäure (Racemat)

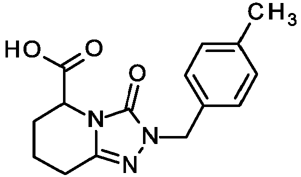

### Variante a)

Methyl-(5RS)-2-(4-methylbenzyl)-3-oxo-2,3,5,6,7,8-hexahydro[1,2,4]triazolo[4,3-a]pyridin-5-carboxylat (Racemat) (854 mg, 95 % Reinheit, 2.69 mmol) wurde in 20 ml THF/Wasser (3:1) vorgelegt, mit Lithiumhydroxid (322 mg, 13.5 mmol) versetzt und die Reaktionsmischung über Nacht bei Raumtemperatur gerührt. Zur Aufarbeitung wurde der Ansatz mit Wasser verdünnt, mit 1N Salzsäure sauer gestellt, mit Dichlormethan und wenig gesättigter Natriumchloridlösung versetzt und extrahiert. Die wässrige Phase wurde noch zweimal mit Dichlormethan extrahiert, die vereinigten organischen Phasen mit gesättigter Natriumchloridlösung gewaschen, über Natriumsulfat getrocknet, eingeengt und im Vakuum getrocknet. Man erhielt so 436 mg (56 % d. Th.) der Titelverbindung.
LC-MS (Methode 3): Rₜ = 1.15 min; MS (ESIpos): m/z = 288 [M+H]⁺
¹H-NMR (400 MHz, DMSO-d6) δ [ppm]: 2.075 (0.98), 2.084 (0.97), 2.092 (0.92), 2.272 (9.23), 4.458 (1.29), 4.770 (4.43), 7.128 (16.00).

### Variante b)

Zur Lösung von tert-Butyl-(5RS)-2-(4-methylbenzyl)-3-oxo-2,3,5,6,7,8-hexahydro[1,2,4]triazolo[4,3-a]pyridin-5-carboxylat (7.14 g, 20.8 mmol) in 100 ml Dichlormethan wurden 10 ml (130 mmol) Trifluoressigsäure gegeben und der Ansatz über Nacht bei Raumtemperatur gerührt. Nach Zugabe von weiteren 5ml (65 mmol) Trifluoressigsäure und einem weiteren Tag Rühren bei Raumtemperatur wurde der Ansatz unter Eisbadkühlung und kräftigem Rühren mit 14ml 3N NaOH versetzt, mit Dichlormethan/Wasser verdünnt und extrahiert. Die wässrige Phase wurde noch zweimal mit Dichlormethan extrahiert, die vereinigten organischen Phasen mit gesättigter Natriumchloridlösung gewaschen, über Natriumsulfat getrocknet, eingeengt und im Vakuum getrocknet. Das so erhaltene Produkt (6.63 g, > 100 % d. Th.) wurde ohne weitere Reinigung verwendet.
LC-MS (Methode 3): Rₜ = 1.18 min; MS (ESIpos): m/z = 288 [M+H]⁺
¹H-NMR (400 MHz, DMSO-d6) δ [ppm]: -0.008 (1.24), 0.008 (1.06), 1.397 (0.62), 1.534 (0.77), 2.272 (8.87), 4.459 (1.36), 4.770 (4.02), 7.128 (16.00).

### Intermediat 126

### (5RS)-2-[4-(tert-Butoxycarbonyl)benzyl]-3-oxo-2,3,5,6,7,8-hexahydro[1,2,4]triazolo[4,3-a]pyridin-5-carbonsäure (Racemat)

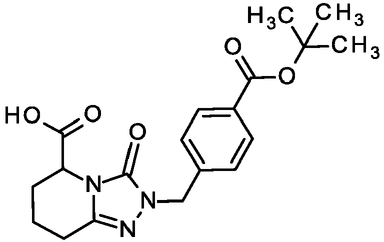

Methyl-(5RS)-2-[4-(tert-butoxycarbonyl)benzyl]-3-oxo-2,3,5,6,7,8-hexahydro[1,2,4]triazolo[4,3-a]pyridin-5-carboxylat (Racemat) (676 mg, 87 % Reinheit, 1.52 mmol) wurde in 15ml THF/Wasser vorgelegt, mit Lithiumhydroxid (182 mg, 7.59 mmol) versetzt und die Reaktionsmischung über Nacht bei Raumtemperatur gerührt. Zur Aufarbeitung wurde der Ansatz mit 1N Salzsäure auf pH6 gestellt, mit Dichlormethan versetzt und extrahiert. Die wässrige Phase wurde noch zweimal mit Dichlormethan extrahiert, die vereinigten organischen Phasen mit gesättigter Natriumchloridlösung gewaschen, über Natriumsulfat getrocknet, eingeengt und im Vakuum getrocknet. Man erhielt so eine Produktfraktion von 99 mg (71 % Reinheit, 12 % d. Th.).

Die ebenfalls produkthaltige wässrige Phase wurde mit 1N Salzsäure auf pH3 gebracht, mit Ethylacetat versetzt und insgesamt dreimal extrahiert. Die vereinigten Ethylacetat-Phasen wurden mit gesättigter Natriumchloridlösung gewaschen, über Natriumsulfat getrocknet, eingeengt und im Vakuum getrocknet. Man erhielt so 147 mg (26 % d. Th.) der Titelverbindung.
LC-MS (Methode 3): Rₜ = 1.53 min; MS (ESlneg): m/z = 372 [M-H]⁻
¹H-NMR (400 MHz, DMSO-d6) δ [ppm]: 1.536 (16.00), 4.911 (2.17), 7.328 (1.14), 7.349 (1.23), 7.846 (1.40), 7.867 (1.28).

### Intermediat 127

### (5RS)-2-[(1RS)-1-(4-Chlorphenyl)ethyl]-3-oxo-2,3,5,6,7,8-hexahydro[1,2,4]triazolo[4,3-a]pyridin-5-carbonsäure (Diastereomerengemisch; 4 Isomere)

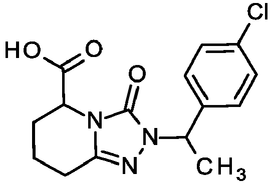

Zur Lösung von tert-Butyl-(5RS)-2-[(1RS)-1-(4-chlorphenyl)ethyl]-3-oxo-2,3,5,6,7,8-hexahydro[1,2,4]triazolo[4,3-a]pyridin-5-carboxylat (Diastereomerengemisch; 4 Isomere) (755 mg, 2.00 mmol) in 10 ml Dichlormethan wurden 1.2 ml (16 mmol) Trifluoressigsäure gegeben und der Ansatz über Nacht bei Raumtemperatur gerührt. Nach Zugabe von weiteren 600 µl (7.8 mmol) Trifluoressigsäure und weiteren 5h Rühren bei Raumtemperatur wurde der Ansatz unter Eisbadkühlung und kräftigem Rühren mit 3N NaOH auf pH 3 eingestellt, mit Dichlormethan/Wasser verdünnt und extrahiert. Die wässrige Phase wurde noch zweimal mit Dichlormethan extrahiert, die vereinigten organischen Phasen mit gesättigter Natriumchloridlösung gewaschen, über Natriumsulfat getrocknet, eingeengt und im Vakuum getrocknet. So wurden 392 mg (79 % Reinheit, 48 % d. Th.) der Titelverbindung als Diastereomerengemisch erhalten.
LC-MS (Methode 3): Rₜ = 1.33 min; MS (ESIpos): m/z = 322 [M+H]⁺; Rₜ = 1.36 min; MS (ESIpos): m/z = 322 [M+H]⁺
¹H-NMR (400 MHz, DMSO-d6) δ [ppm]: -0.149 (1.83), -0.008 (16.00), 0.008 (13.62), 0.146 (1.78), 1.110 (3.38), 1.288 (4.30), 1.304 (4.30), 1.329 (5.07), 1.406 (11.38), 1.595 (12.57), 1.607 (12.53), 1.613 (13.39), 1.625 (11.70), 2.076 (5.07), 2.580 (3.20), 2.664 (3.47), 2.710 (2.42), 3.286 (7.04), 4.426 (3.15), 4.450 (3.43), 5.326 (3.06), 5.344 (3.29), 7.309 (5.62), 7.319 (4.94), 7.325 (4.75), 7.331 (10.88), 7.340 (10.10), 7.346 (4.25), 7.353 (4.30), 7.355 (14.45), 7.375 (11.93), 7.379 (15.41), 7.385 (4.75), 7.396 (6.86), 7.401 (7.54), 7.484 (4.66).

### Intermediat 128

### (5RS)-3-Oxo-2-(4-sulfamoylbenzyl)-2,3,5,6,7,8-hexahydro[1,2,4]triazolo[4,3-a]pyridin-5-carbonsäure (Racemat)

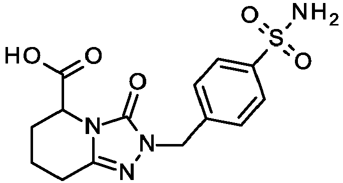

Zur Lösung von tert-Butyl-(5RS)-3-oxo-2-(4-sulfamoylbenzyl)-2,3,5,6,7,8-hexahydro[1,2,4]triazolo[4,3-a]pyridin-5-carboxylat (Racemat) (35.0 mg, 85.7 µmοl) in 5.0 ml Dichlormethan wurden 500 µl (6.5 mmol) Trifluoressigsäure gegeben und der Ansatz über ein Wochenende bei Raumtemperatur gerührt. Zur Aufarbeitung wurde der Ansatz im Vakuum eingeengt und getrocknet. So wurden 30 mg (99 % d. Th.) der Titelverbindung erhalten, die ohne zusätzliche Reinigung weiter verwendet wurden.
LC-MS (Methode 6): Rₜ = 0.66 min; MS (ESIpos): m/z = 353 [M+H]⁺

### Intermediat 129

### (5RS)-2-[4-(Methylsulfanyl)benzyl]-3-oxo-2,3,5,6,7,8-hexahydro[1,2,4]triazolo[4,3-a]pyridin-5-carbonsäure (Racemat)

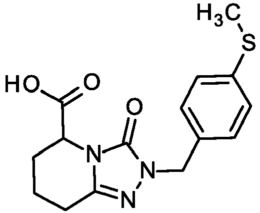

Zur Lösung von tert-Butyl-(5RS)-2-[4-(methylsulfanyl)benzyl]-3-oxo-2,3,5,6,7,8-hexahydro[1,2,4]triazolo[4,3-a]pyridin-5-carboxylat (Racemat) (160 mg, 89 % Reinheit, 379 µmοl) in 5.0 ml Dichlormethan wurden 500 µl (6.5 mmol) Trifluoressigsäure gegeben und der Ansatz über ein Wochenende bei Raumtemperatur gerührt. Zur weiteren Umsetzung wurden 100 µL (1.3 mmol) Trifluoressigsäure nachgegeben und etwa ein weierer Tag bei Raumtemperatur gerührt (Umsatzkontrolle durch HPLC). Zur Aufarbeitung wurde der Ansatz unter Eisbadkühlung und kräftigem Rühren mit 3N NaOH auf pH 3 eingestellt, mit Dichlormethan/Wasser verdünnt und extrahiert. Die wässrige Phase wurde noch zweimal mit Dichlormethan extrahiert, die vereinigten organischen Phasen mit gesättigter Natriumchloridlösung gewaschen, über Natriumsulfat getrocknet, eingeengt und im Vakuum getrocknet. Es wurden 137 mg (109 % d. Th.) der Titelverbindung erhalten, die so weiter umgesetzt wurden.
LC-MS (Methode 3): Rₜ = 1.20 min; MS (ESIpos): m/z = 320 [M+H]⁺
¹H-NMR (400 MHz, DMSO-d6) δ [ppm]: -0.008 (2.62), 0.008 (2.24), 1.396 (0.96), 2.452 (16.00), 4.461 (1.33), 4.782 (4.61), 5.754 (0.68), 7.174 (1.26), 7.196 (3.98), 7.215 (4.58), 7.221 (1.06), 7.237 (1.41).

### Intermediat 130

### (5RS)-2-[(6-Chlorpyridin-3-yl)methyl]-3-oxo-2,3,5,6,7,8-hexahydro[1,2,4]triazolo[4,3-a]pyridin-5-carbonsäure (Racemat)

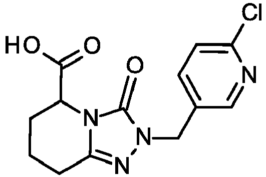

Zur Lösung von tert-Butyl-(5RS)-2-[(6-chlorpyridin-3-yl)methyl]-3-oxo-2,3,5,6,7,8-hexahydro[1,2,4]triazolo[4,3-a]pyridin-5-carboxylat (Racemat) (1.84 g, 5.04 mmol) in 25 ml Dichlormethan wurden 5.0 ml (65 mmol) Trifluoressigsäure gegeben und der Ansatz über Nacht bei Raumtemperatur gerührt. Zur Aufarbeitung wurde der Ansatz unter Eisbadkühlung und kräftigem Rühren mit 3N NaOH auf pH 3 eingestellt, mit Wasser verdünnt und zweimal mit Dichlormethan extrahiert. Die vereinigten organischen Phasen wurden mit gesättigter Natriumchloridlösung gewaschen, über Magnesiumsulfat getrocknet, eingeengt und im Vakuum getrocknet. Es wurden 1.59 g (92 % Reinheit, 94 % d. Th.)der Titelverbindung erhalten, die so weiter umgesetzt wurden.
LC-MS (Methode 3): Rₜ = 0.83 min; MS (ESIpos): m/z = 309 [M+H]⁺
¹H-NMR (400 MHz, DMSO-d6) δ [ppm]: -0.008 (1.94), 0.008 (1.83), 1.157 (3.09), 1.175 (6.27), 1.193 (3.15), 1.387 (3.56), 1.406 (0.95), 1.506 (0.97), 1.519 (1.00), 1.534 (0.85), 1.795 (0.84), 1.809 (1.23), 1.820 (1.25), 1.830 (1.04), 1.841 (1.01), 1.909 (1.25), 1.988 (11.31), 2.069 (1.57), 2.078 (2.11), 2.084 (2.94), 2.090 (3.28), 2.101 (3.07), 2.113 (1.63), 2.568 (1.70), 2.582 (1.28), 2.614 (1.39), 2.627 (2.10), 2.636 (1.49), 2.656 (0.78), 2.666 (1.09), 4.003 (1.24), 4.021 (3.03), 4.038 (3.02), 4.056 (1.29), 4.218 (0.77), 4.462 (2.60), 4.474 (4.68), 4.487 (2.49), 4.828 (1.17), 4.910 (16.00), 5.093 (0.74), 7.500 (4.57), 7.521 (5.54), 7.703 (3.17), 7.709 (3.32), 7.724 (2.78), 7.730 (2.79), 8.310 (4.06), 8.316 (3.98).

### Intermediat 131

### (5RS)-2-[(5-Chlorpyridin-2-yl)methyl]-3-oxo-2,3,5,6,7,8-hexahydro[1,2,4]triazolo[4,3-a]pyridin-5-carbonsäure (Racemat)

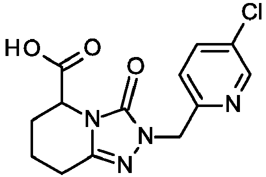

Zur Lösung von tert-Butyl-(5RS)-2-[(5-chlorpyridin-2-yl)methyl]-3-oxo-2,3,5,6,7,8-hexahydro[1,2,4]triazolo[4,3-a]pyridin-5-carboxylat (Racemat) (268 mg, 82 % Reinheit, 602 µmοl) in 3.0 ml Dichlormethan wurden 600 µl (7.8 mmol) Trifluoressigsäure gegeben und der Ansatz über Nacht bei Raumtemperatur gerührt. Zur Aufarbeitung wurde der Ansatz im Vakuum eingeengt und getrocknet. So wurden 220 mg (80 % Reinheit, 95 % d. Th.) der Titelverbindung erhalten, die ohne zusätzliche Reinigung weiter verwendet wurden.
LC-MS (Methode 3): Rₜ = 0.87 min; MS (ESIpos): m/z = 309 [M+H]⁺

### Intermediat 132

### (5RS)-2-[(6-Cyanpyridin-3-yl)methyl]-3-oxo-2,3,5,6,7,8-hexahydro[1,2,4]triazolo[4,3-a]pyridin-5-carbonsäure (Racemat)

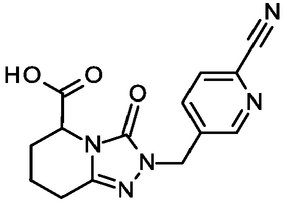

Zur Lösung von tert-Butyl-(5RS)-2-[(6-cyanpyridin-3-yl)methyl]-3-oxo-2,3,5,6,7,8-hexahydro[1,2,4]triazolo[4,3-a]pyridin-5-carboxylat (Racemat) (303 mg, 853 µmol)in 8.0 ml Dichlormethan wurden 1.0 ml (13 mmol) Trifluoressigsäure gegeben und der Ansatz über Nacht bei Raumtemperatur gerührt. Zur weiteren Umsetzung wurden noch je einmal 200 µL (2.6 mmol) und 100µl (1.3 mmol) Trifluoressigsäure nachgegeben und weitere 3.5 h bzw 2.5 h gerührt. (Umsatzkontrolle durch HPLC und LC/MS). Zur Aufarbeitung wurde der Ansatz mit Dichlormethan/Wasser verdünnt und extrahiert. Die wässrige Phase wurde noch zweimal mit Dichlormethan extrahiert, die vereinigten organischen Phasen mit gesättigter Natriumchloridlösung gewaschen, über Natriumsulfat getrocknet, eingeengt und im Vakuum getrocknet. Es wurden 191 mg (67 % Reinheit, 50 % d. Th.) der Titelverbindung erhalten, die so weiter umgesetzt wurden.
LC-MS (Methode 3): Rₜ = 0.72 min; MS (ESIpos): m/z = 300 [M+H]⁺
¹H-NMR (400 MHz, DMSO-d6) δ [ppm]: -0.149 (0.84), -0.008 (6.61), 0.008 (6.11), 0.146 (0.76), 1.394 (4.32), 1.400 (16.00), 2.094 (1.18), 2.366 (0.89), 2.665 (0.82), 2.710 (0.84), 4.489 (1.34), 4.985 (1.87), 5.034 (4.55), 5.754 (1.79), 7.851 (0.71), 7.857 (0.76), 7.871 (1.00), 7.876 (0.95), 7.978 (0.74), 8.000 (1.24), 8.023 (1.58), 8.043 (1.26), 8.633 (1.26), 8.638 (1.26).

### Intermediat 133

### (5RS)-2-[4-Chlor-3-(trifluormethyl)benzyl]-3-oxo-2,3,5,6,7,8-hexahydro[1,2,4]triazolo[4,3-a]pyridin-5-carbonsäure (Racemat)

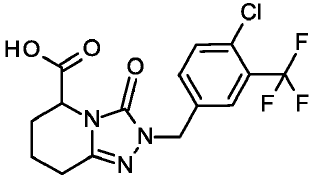

Zur Lösung von tert-Butyl-(5RS)-2-[4-chlor-3-(trifluormethyl)benzyl]-3-oxo-2,3,5,6,7,8-hexahydro[1,2,4]triazolo[4,3-a]pyridin-5-carboxylat (Racemat) (332 mg, 97 % Reinheit, 746 µmοl) in 7.0 ml Dichlormethan wurden 700 µl (9.1 mmol) Trifluoressigsäure gegeben und der Ansatz 4h bei Raumtemperatur gerührt. Zur weiteren Umsetzung wurden noch einmal 200 µL (2.6 mmol) Trifluoressigsäure nachgegeben und über Nacht weiter gerührt. Zur Aufarbeitung wurde der Ansatz mit Dichlormethan/Wasser verdünnt und extrahiert. Die wässrige Phase wurde noch zweimal mit Dichlormethan extrahiert, die vereinigten organischen Phasen mit gesättigter Natriumchloridlösung gewaschen, über Natriumsulfat getrocknet, eingeengt und im Vakuum getrocknet. Es wurden 273 mg (94 % d. Th.) der Titelverbindung erhalten, die so weiter verwendet wurden.
LC-MS (Methode 3): Rₜ = 1.50 min; MS (ESIpos): m/z = 376 [M+H]⁺
¹H-NMR (400 MHz, DMSO-d6) δ [ppm]: -0.149 (0.97), -0.008 (8.63), 0.008 (7.66), 0.146 (0.97), 1.034 (1.14), 1.050 (1.12), 1.377 (3.67), 1.506 (1.07), 1.519 (1.07), 1.534 (0.97), 1.800 (0.90), 1.814 (1.34), 1.824 (1.34), 1.835 (1.09), 1.846 (1.14), 2.076 (1.56), 2.084 (2.31), 2.098 (3.87), 2.108 (3.57), 2.120 (1.92), 2.366 (0.73), 2.519 (2.21), 2.561 (2.04), 2.574 (2.04), 2.588 (1.53), 2.620 (1.53), 2.633 (2.36), 2.643 (1.68), 2.662 (0.97), 2.675 (1.34), 2.685 (0.71), 2.710 (0.75), 3.168 (1.00), 3.508 (2.38), 4.477 (2.63), 4.488 (4.98), 4.501 (2.58), 4.956 (16.00), 7.523 (2.31), 7.529 (2.38), 7.544 (2.99), 7.549 (3.06), 7.706 (5.16), 7.727 (4.21), 7.748 (5.47), 7.754 (5.23), 13.264 (0.73).

### Intermediat 134

### (5RS)-2-(3-Chlorbenzyl)-3-oxo-2,3,5,6,7,8-hexahydro[1,2,4]triazolo[4,3-a]pyridin-5-carbonsäure (Racemat)

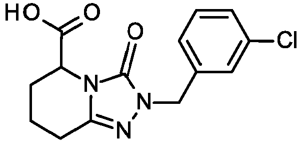

Zur Lösung von tert-Butyl-(5RS)-2-(3-chlorbenzyl)-3-oxo-2,3,5,6,7,8-hexahydro[1,2,4]triazolo[4,3-a]pyridin-5-carboxylat (Racemat) (284 mg, 84 % Reinheit, 656 µmοl) in 6.0 ml Dichlormethan wurden 600 µl (7.8 mmol) Trifluoressigsäure gegeben und der Ansatz 4h bei Raumtemperatur gerührt. Zur weiteren Umsetzung wurden noch einmal 200 µL (2.6 mmol) Trifluoressigsäure nachgegeben und über Nacht weiter gerührt. Zur Aufarbeitung wurde der Ansatz mit Dichlormethan/Wasser verdünnt und extrahiert. Die wässrige Phase wurde noch zweimal mit Dichlormethan extrahiert, die vereinigten organischen Phasen mit gesättigter Natriumchloridlösung gewaschen, über Natriumsulfat getrocknet, eingeengt und im Vakuum getrocknet. Es wurden 224 mg (83 % Reinheit, 92 % d. Th.)der Titelverbindung erhalten, die so weiter verwendet wurden.
LC-MS (Methode 3): Rₜ = 1.20 min; MS (ESIpos): m/z = 308 [M+H]⁺
¹H-NMR (400 MHz, DMSO-d6) δ [ppm]: -0.149 (1.79), -0.008 (16.00), 0.008 (14.92), 0.146 (1.88), 1.147 (1.13), 1.396 (0.56), 2.091 (0.71), 2.323 (0.56), 2.327 (0.89), 2.332 (0.66), 2.366 (1.46), 2.665 (0.80), 2.669 (1.04), 2.674 (0.94), 2.709 (1.55), 3.168 (1.55), 4.486 (0.89), 4.859 (3.48), 7.197 (0.56), 7.214 (0.71), 7.288 (0.99), 7.351 (0.85), 7.356 (2.02), 7.374 (0.89).

### Alternative Synthese:

Methyl-(5RS)-2-(3-chlorbenzyl)-3-oxo-2,3,5,6,7,8-hexahydro[1,2,4]triazolo[4,3-a]pyridin-5-carboxylat (Racemat) (63.0 mg, 196 µmοl) wurde in THF (2.5 ml) vorgelegt und Lithiumhydroxid (23.4 mg, 979 µmοl) gelöst in Wasser (2.5 ml) zugegeben. Das Reaktionsgemisch wurde für 65 h bei Raumtemperatur gerührt und anschließend mit Wasser und 1N wässriger Salzsäure versetzt. Die organische Phase wurde abgetrennt und die wässrige Phase mehrfach mit Ethylacetat extrahiert. Die vereinigten organischen Phasen wurden über Natriumsulfat getrocknet, filtriert und das Filtrat eingeengt. Es wurden 55.0 mg (91 % d. Th.) der Titelverbindung erhalten.
LC-MS (Methode 4): Rₜ = 0.68 min; MS (ESIpos): m/z = 308 [M+H]⁺

### Intermediat 135

### (5RS)-2-(3,4-Dichlorbenzyl)-3-oxo-2,3,5,6,7,8-hexahydro[1,2,4]triazolo[4,3-a]pyridin-5-carbonsäure (Racemat)

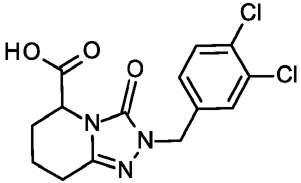

Zur Lösung von tert-Butyl-(5RS)-2-(3,4-dichlorbenzyl)-3-oxo-2,3,5,6,7,8-hexahydro[1,2,4]triazolo[4,3-a]pyridin-5-carboxylat (Racemat) (330 mg, 78 % Reinheit, 646 µmοl) in 8.0 ml Dichlormethan wurden 900 µl (12 mmol) Trifluoressigsäure gegeben und der Ansatz über Nacht bei Raumtemperatur gerührt. Zur weiteren Umsetzung wurden noch einmal 100 µL (1.3 mmol) Trifluoressigsäure nachgegeben und 5h weiter gerührt. Zur Aufarbeitung wurde der Ansatz mit Dichlormethan/Wasser verdünnt und extrahiert. Die wässrige Phase wurde noch zweimal mit Dichlormethan extrahiert, die vereinigten organischen Phasen mit gesättigter Natriumchloridlösung gewaschen, über Natriumsulfat getrocknet, eingeengt und im Vakuum getrocknet. Es wurden 282 mg (80 % Reinheit, >100 %) der Titelverbindung erhalten, die so weiter verwendet wurden.
LC-MS (Methode 3): Rₜ = 1.39 min; MS (ESIpos): m/z = 342 [M+H]⁺
¹H-NMR (400 MHz, DMSO-d6) δ [ppm]: -0.008 (0.99), 0.008 (0.93), 1.365 (0.65), 1.392 (6.32), 1.510 (0.93), 1.523 (0.96), 1.535 (1.07), 1.538 (0.85), 1.801 (0.81), 1.811 (1.13), 1.815 (1.18), 1.825 (1.21), 1.836 (1.01), 1.847 (1.01), 2.077 (1.57), 2.086 (2.08), 2.092 (2.91), 2.099 (3.24), 2.108 (3.04), 2.121 (1.63), 2.521 (1.06), 2.563 (1.64), 2.576 (1.70), 2.591 (1.29), 2.621 (1.34), 2.631 (1.97), 2.635 (2.09), 2.644 (1.52), 2.664 (0.79), 2.674 (1.03), 4.475 (2.28), 4.487 (4.46), 4.499 (2.26), 4.706 (0.69), 4.868 (16.00), 5.423 (1.28), 7.221 (2.81), 7.226 (2.94), 7.242 (3.18), 7.247 (3.31), 7.486 (4.76), 7.491 (4.55), 7.603 (7.05), 7.623 (6.45), 7.631 (0.66).

### Intermediat 136

### (5RS)-2-(3,5-Dichlorbenzyl)-3-oxo-2,3,5,6,7,8-hexahydro[1,2,4]triazolo[4,3-a]pyridin-5-carbonsäurehydrochlorid (Racemat)

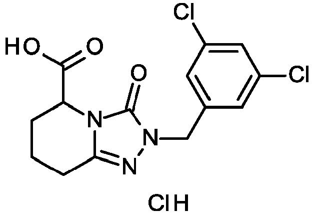

tert-Butyl-(5RS)-2-(3,5-dichlorbenzyl)-3-oxo-2,3,5,6,7,8-hexahydro[1,2,4]triazolo[4,3-a]pyridin-5-carboxylat(Racemat) (445 mg, 1.12 mmol) wurde in 1,4-Dioxan (2.0 ml) gelöst und Salzsäure gelöst in 1,4-Dioxan (2.8 ml, 4.0 M, 11 mmol) zugegeben. Das Reaktionsgemisch wurde für 2 h bei Raumtemperatur gerührt und anschließend erneut Salzsäure gelöst in 1,4-Dioxan (2.8 ml, 4.0 M, 11 mmol) zugegeben. Nach Rühren über Nacht bei Raumtemperatur wurde das Lösungsmittel im Vakuum entfernt und 493 mg (79 % Reinheit, 92 % d. Th.) der Titelverbindung erhalten.
LC-MS (Methode 4): Rₜ = 0.80 min; MS (ESIpos): m/z = 342 [M+H]⁺
¹H-NMR (400 MHz, DMSO-d6) δ [ppm]: -0.008 (0.80), 0.008 (0.72), 1.175 (0.65), 1.192 (0.78), 1.235 (2.38), 1.395 (4.05), 1.406 (0.88), 1.477 (0.72), 1.496 (1.12), 1.512 (1.51), 1.525 (1.58), 1.540 (1.34), 1.553 (0.93), 1.572 (0.46), 1.808 (1.37), 1.820 (1.91), 1.831 (1.83), 1.842 (1.54), 1.853 (1.50), 1.909 (0.89), 1.988 (0.76), 2.086 (2.68), 2.100 (4.49), 2.107 (4.86), 2.116 (4.43), 2.128 (2.41), 2.560 (3.08), 2.574 (2.23), 2.587 (2.34), 2.602 (1.96), 2.635 (1.94), 2.646 (3.14), 2.656 (2.27), 2.676 (1.29), 2.686 (1.41), 2.698 (0.88), 3.365 (2.96), 3.451 (0.75), 3.463 (0.96), 3.475 (1.00), 3.492 (0.94), 3.504 (0.77), 3.515 (0.42), 3.656 (0.43), 3.667 (0.74), 3.679 (0.85), 3.690 (0.41), 3.699 (0.83), 3.713 (0.61), 4.377 (0.62), 4.490 (3.41), 4.503 (9.30), 4.514 (3.22), 4.768 (0.61), 4.845 (0.78), 4.886 (13.41), 4.931 (0.74), 7.279 (15.72), 7.284 (16.00), 7.299 (0.60), 7.303 (0.53), 7.347 (2.08), 7.352 (2.18), 7.414 (0.50), 7.419 (0.52), 7.451 (1.08), 7.533 (3.64), 7.538 (6.48), 7.543 (3.88), 11.356 (0.56), 13.281 (0.55).

### Intermediat 137

### (5RS)-3-Oxo-2-{[6-(trifluormethyl)pyridin-3-yl]methyl}-2,3,5,6,7,8-hexahydro[1,2,4]triazolo[4,3-a]pyridin-5-carbonsäure -trifluoressigsäure (Racemat)

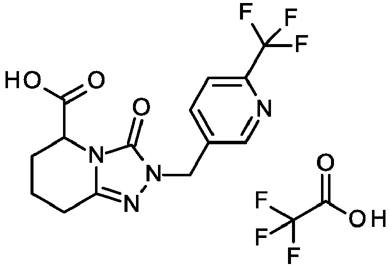

tert-Butyl-(5RS)-3-oxo-2-{[6-(trifluormethyl)pyridin-3-yl]methyl}-2,3,5,6,7,8-hexahydro[1,2,4]triazolo[4,3-a]pyridin-5-carboxylat (Racemat) (364 mg, 913 µmοl) wurde in Dichlormethan (10 ml) gelöst und bei Raumtemperatur mit Trifluoressigsäure (1.1 ml, 14 mmol) versetzt. Nachdem das Reaktionsgemisch über Nacht bei Raumtemperatur gerührt wurde, wurde das Lösungsmittel unter Vakuum entfernt. Es wurden 465 mg (> 100 %) der Titelverbindung erhalten.
LC-MS (Methode 4): Rₜ = 0.58 min; MS (ESIpos): m/z = 343 [M+H]⁺

### Intermediat 138

### (5RS)-2-(3,4-Difluorbenzyl)-3-oxo-2,3,5,6,7,8-hexahydro[1,2,4]triazolo[4,3-a]pyridin-5-carbonsäurehydrochlorid (Racemat)

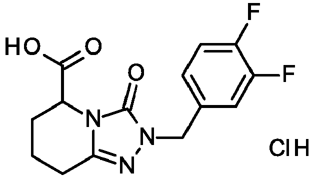

tert-Butyl-(5S, R)-2-(3,4-difluorbenzyl)-3-oxo-2,3,5,6,7,8-hexahydro[1,2,4]triazolo[4,3-a]pyridin-5-carboxylat (Racemat) (451 mg, 1.23 mmol) wurde in 1,4-Dioxan (10 ml, 120 mmol) gelöst und Salzsäure gelöst in 1,4-Dioxan (3.1 ml, 4.0 M, 12 mmol) zugegeben. Das Reaktionsgemisch wurde für 65 h bei Raumtemperatur und über Nacht bei 50 °C gerührt. Es wurde erneut Salzsäure gelöst in 1,4-Dioxan (3.1 ml, 4.0 M, 12 mmol) zugegeben und für 1 h bei Raumtemperatur gerührt. Das Lösungsmittel wurde im Vakuum entfernt und 420 mg (98 % d. Th.) der Titelverbindung erhalten.

¹H-NMR (400 MHz, DMSO-d6) δ [ppm]: -0.008 (3.02), 0.008 (2.73), 1.393 (1.69), 1.810 (0.44), 2.098 (1.23), 2.107 (1.13), 2.327 (0.53), 2.562 (0.67), 2.575 (0.60), 2.589 (0.48), 2.621 (0.48), 2.631 (0.77), 2.642 (0.55), 2.670 (0.77), 3.568 (16.00), 4.472 (0.85), 4.484 (1.55), 4.496 (0.75), 4.849 (4.79), 7.101 (0.55), 7.236 (0.48), 7.265 (0.57), 7.285 (0.50), 7.370 (0.55), 7.392 (1.04), 7.398 (0.62), 7.413 (0.59), 7.419 (1.09), 7.440 (0.52).

### Intermediat 139

### (5RS)-2-(3-Chlor-4-fluorbenzyl)-3-oxo-2,3,5,6,7,8-hexahydro[1,2,4]triazolo[4,3-a]pyridin-5-carbonsäurehydrochlorid (Racemat)

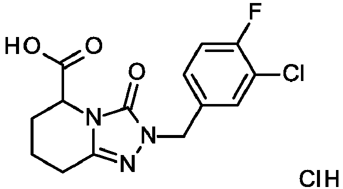

tert-Butyl-(5RS)-2-(3-chlor-4-fluorbenzyl)-3-oxo-2,3,5,6,7,8-hexahydro[1,2,4]triazolo[4,3-a]pyridin-5-carboxylat (Racemat) (391 mg, 1.02 mmol) wurde in 1,4-Dioxan (10 ml, 120 mmol) gelöst und Salzsäure gelöst in 1,4-Dioxan (2.6 ml, 4.0 M, 10 mmol) zugegeben. Das Reaktionsgemisch wurde für 65 h bei Raumtemperatur und 3 h bei 50 °C gerührt. Es wurde erneut Salzsäure gelöst in 1,4-Dioxan (1,3 ml, 4.0 M, 5 mmol) zugegeben und über Nacht bei 50 °C gerührt. Das Lösungsmittel wurde im Vakuum entfernt und 350 mg (94 % d. Th.) der Titelverbindung erhalten.

¹H-NMR (400 MHz, DMSO-d6) δ [ppm]: -0.008 (1.11), 0.008 (1.07), 1.391 (4.37), 1.476 (0.51), 1.492 (0.77), 1.510 (1.08), 1.523 (1.11), 1.538 (0.96), 1.551 (0.66), 1.799 (0.95), 1.812 (1.39), 1.822 (1.40), 1.833 (1.20), 1.844 (1.17), 1.909 (1.18), 2.090 (3.55), 2.097 (3.78), 2.106 (3.58), 2.118 (1.94), 2.519 (1.90), 2.561 (1.92), 2.574 (1.91), 2.589 (1.51), 2.620 (1.53), 2.633 (2.43), 2.643 (1.77), 2.662 (0.94), 2.672 (1.24), 2.685 (0.72), 3.390 (0.80), 3.484 (0.41), 3.492 (0.65), 3.502 (0.60), 3.593 (0.97), 3.671 (0.58), 3.681 (0.64), 3.700 (0.63), 3.709 (0.46), 3.733 (0.41), 3.831 (0.41), 3.840 (0.70), 3.860 (0.72), 4.020 (0.56), 4.189 (0.69), 4.210 (0.69), 4.323 (0.51), 4.329 (0.91), 4.472 (2.61), 4.484 (4.91), 4.496 (2.54), 4.767 (0.52), 4.852 (16.00), 6.348 (0.77), 7.239 (1.31), 7.245 (1.45), 7.251 (1.51), 7.256 (1.74), 7.260 (2.09), 7.266 (2.12), 7.272 (1.95), 7.278 (1.93), 7.368 (3.87), 7.377 (0.46), 7.391 (4.96), 7.413 (2.96), 7.431 (2.61), 7.436 (2.49), 7.448 (2.69), 7.454 (2.57), 8.133 (0.53).

### Intermediat 140

### (5RS)-2-[(2-Methoxypyridin-4-yl)methyl]-3-oxo-2,3,5,6,7,8-hexahydro[1,2,4]triazolo[4,3-a]pyridin-5-carbonsäurehydrochlorid (Racemat)

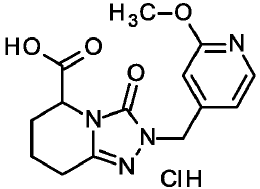

tert-Butyl-(5RS)-2-[(2-methoxypyridin-4-yl)methyl]-3-oxo-2,3,5,6,7,8-hexahydro[1,2,4]triazolo[4,3-a]pyridin-5-carboxylat (Racemat) (144 mg, 400 µmοl) wurde in 1,4-Dioxan (4.0 ml, 47 mmol) gelöst und Salzsäure gelöst in 1,4-Dioxan (1000 µl, 4.0 M, 4.0 mmol) zugegeben. Das Reaktionsgemisch wurde für 65 h bei Raumtemperatur und über Nacht bei 50 °C gerührt. Das Lösungsmittel wurde im Vakuum entfernt und 165 mg (> 100 %) der Titelverbindung erhalten.

1H-NMR (400 MHz, DMSO-d6): d [ppm] = 1.46 - 1.60 (m, 2H), 1.79 - 1.89 (m, 2H), 2.05 - 2.15 (m, 2H), 3.83 (s, 3H) 4.49 (d, 1H), 4.85 (d, 2H), 6.59 (s, 1H), 6.82 (d, 1H), 8.10 (d, 1H).

1.53 (s, 2H), 1.83 (s, 2H), 2.11 (br s, 5H), 2.63-2.71 (m, 5H), 3.83 (s, 3H), 4.49 (d, 2H), 4.68 (s, 1H), 4.66 - 4.70 (m, 1H), 4.85 (d, 2H), 6.04 (s, 1H), 6.59 (s, 1H), 6.82 (d, 1H), 7.32 (s, 1H), 8.10 (d, 1H).

### Intermediat 141

### (5RS)-2-(3-Chlor-4-methoxybenzyl)-3-oxo-2,3,5,6,7,8-hexahydro[1,2,4]triazolo[4,3-a]pyridin-5-carbonsäure -trifluoressigsäure (Racemat)

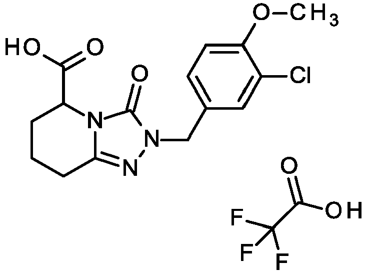

tert-Butyl-(5RS)-2-(3-chlor-4-methoxybenzyl)-3-oxo-2,3,5,6,7,8-hexahydro[1,2,4]triazolo[4,3-a]pyridin-5-carboxylat (Racemat) (365 mg, 925 µmοl) wurde in Dichlormethan (20 ml) gelöst und bei Raumtemperatur mit Trifluoressigsäure (710 µl, 9.3 mmol) versetzt. Nachdem das Reaktionsgemisch für 48h bei Raumtemperatur gerührt wurde, wurde das Lösungsmittel unter Vakuum entfernt. Es wurden 454 mg (> 100 %) der Titelverbindung erhalten.
LC-MS (Methode 4): Rₜ = 0.66 min; MS (ESIpos): m/z = 338 [M+H]⁺
¹H-NMR (400 MHz, DMSO-d6) δ [ppm]: -0.008 (0.90), 0.008 (0.79), 1.111 (1.03), 1.157 (0.90), 1.175 (1.82), 1.193 (0.92), 1.228 (0.41), 1.391 (1.94), 1.474 (0.43), 1.488 (0.67), 1.508 (0.88), 1.522 (0.94), 1.535 (2.44), 1.550 (0.56), 1.790 (0.81), 1.802 (1.21), 1.813 (1.21), 1.824 (1.06), 1.835 (1.01), 1.848 (0.68), 1.988 (3.28), 2.067 (1.72), 2.080 (3.10), 2.087 (3.19), 2.096 (2.97), 2.108 (1.57), 2.520 (1.36), 2.563 (1.66), 2.577 (1.26), 2.609 (1.33), 2.620 (2.11), 2.631 (1.48), 2.650 (0.75), 2.661 (1.03), 2.673 (0.66), 2.690 (0.53), 2.731 (2.17), 2.890 (2.79), 4.021 (0.77), 4.039 (0.76), 4.458 (2.30), 4.470 (4.46), 4.482 (2.24), 4.771 (16.00), 5.753 (1.45), 7.094 (4.56), 7.115 (6.97), 7.185 (3.46), 7.190 (3.61), 7.206 (2.20), 7.211 (2.39), 7.298 (5.41), 7.303 (4.82).

### Intermediat 142

### (5RS)-2-(3-Fluorbenzyl)-3-oxo-2,3,5,6,7,8-hexahydro[1,2,4]triazolo[4,3-a]pyridin-5-carbonsäurehydrochlorid (Racemat)

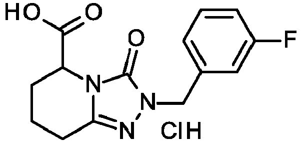

tert-Butyl-(5RS)-2-(3-fluorbenzyl)-3-oxo-2,3,5,6,7,8-hexahydro[1,2,4]triazolo[4,3-a]pyridin-5-carboxylat (Racemat) (150 mg, 432 µmοl) wurde in 1,4-Dioxan (4.3 ml, 51 mmol) gelöst und Salzsäure gelöst in 1,4-Dioxan (1.1 ml, 4.0 M, 4.3 mmol) zugegeben. Das Reaktionsgemisch wurde für 65 h bei Raumtemperatur gerührt. Es wurde erneut Salzsäure gelöst in 1,4-Dioxan (1.1 ml, 4.0 M, 4.3 mmol) zugegeben und über Nacht bei 50 °C gerührt. Das Lösungsmittel wurde im Vakuum entfernt und 165 mg (89 % Reinheit, > 100 %) der Titelverbindung erhalten.
LC-MS (Methode 4): Rₜ = 0.59 min; MS (ESIpos): m/z = 292 [M+H]⁺

### Intermediat 143

### (5RS)-2-(3-Methoxybenzyl)-3-oxo-2,3,5,6,7,8-hexahydro[1,2,4]triazolo[4,3-a]pyridin-5-carbonsäure -trifluoressigsäure (Racemat)

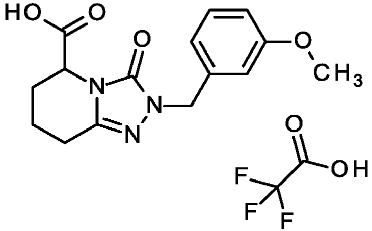

tert-Butyl-(5RS)-2-(3-methoxybenzyl)-3-oxo-2,3,5,6,7,8-hexahydro[1,2,4]triazolo[4,3-a]pyridin-5-carboxylat (Racemat) (340 mg, 947 µmοl) wurde in Dichlormethan (20 ml) gelöst und bei Raumtemperatur mit Trifluoressigsäure (730 µl, 9.5 mmol) versetzt. Nachdem das Reaktionsgemisch über Nacht bei Raumtemperatur gerührt wurde, wurde das Lösungsmittel unter Vakuum entfernt. Es wurden 435 mg (> 100 %) der Titelverbindung erhalten.
LC-MS (Methode 3): Rₜ = 1.04 min; MS (ESIpos): m/z = 304 [M+H]⁺
¹H-NMR (400 MHz, DMSO-d6) δ [ppm]: -0.008 (1.85), 0.008 (1.73), 1.111 (1.34), 1.399 (3.15), 1.482 (0.86), 1.515 (1.77), 1.534 (5.41), 1.544 (1.65), 1.557 (1.10), 1.576 (0.53), 1.797 (1.69), 1.810 (2.41), 1.820 (2.43), 1.831 (2.06), 1.843 (1.97), 1.855 (1.32), 1.988 (0.50), 2.047 (0.43), 2.075 (3.45), 2.088 (6.07), 2.095 (6.31), 2.105 (5.87), 2.117 (3.12), 2.328 (0.42), 2.524 (1.98), 2.569 (3.23), 2.583 (2.50), 2.613 (2.67), 2.626 (4.07), 2.636 (2.95), 2.655 (1.45), 2.666 (2.17), 2.678 (1.24), 3.696 (0.64), 3.705 (0.58), 3.750 (0.42), 3.765 (1.85), 4.466 (4.47), 4.477 (8.57), 4.490 (4.32), 4.759 (1.51), 4.799 (16.00), 4.805 (15.56), 4.845 (1.43), 5.398 (0.68), 5.753 (4.67), 6.789 (14.05), 6.809 (6.64), 6.824 (4.19), 6.830 (3.47), 6.845 (4.90), 6.851 (4.14), 7.220 (6.34), 7.240 (8.92), 7.259 (4.69).

### Intermediat 144

### (5RS)-3-Oxo-2-[3-(trifluormethyl)benzyl]-2,3,5,6,7,8-hexahydro[1,2,4]triazolo[4,3-a]pyridin-5-carbonsäure -trifluoressigsäure (Racemat)

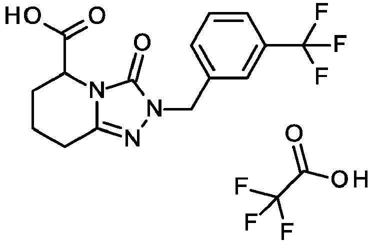

tert-Butyl-(5RS)-3-oxo-2-[3-(trifluormethyl)benzyl]-2,3,5,6,7,8-hexahydro[1,2,4]triazolo[4,3-a]pyridin-5-carboxylat (Racemat) (388 mg, 975 µmοl) wurde in Dichlormethan (20 ml) gelöst und bei Raumtemperatur mit Trifluoressigsäure (750 µl, 9.8 mmol) versetzt. Nachdem das Reaktionsgemisch über Nacht bei Raumtemperatur gerührt worden war, wurde erneut Trifluoressigsäure (750 µl, 9.8 mmol) zugegeben. Nach 2 h Rühren bei 40 °C wurde das Lösungsmittel unter Vakuum entfernt. Es wurden 534 mg (> 100 %) der Titelverbindung erhalten.
LC-MS (Methode 3): Rₜ = 1.34 min; MS (ESIpos): m/z = 342 [M+H]⁺
¹H-NMR (400 MHz, DMSO-d6) δ [ppm]: 1.110 (0.81), 1.385 (1.82), 1.535 (3.66), 1.815 (1.57), 2.100 (4.20), 2.328 (0.90), 2.562 (2.17), 2.575 (2.21), 2.590 (1.50), 2.634 (2.53), 2.674 (1.98), 4.482 (2.72), 4.493 (5.55), 4.506 (2.60), 4.958 (16.00), 7.525 (2.56), 7.544 (4.75), 7.569 (2.81), 7.588 (5.17), 7.615 (6.53), 7.649 (4.29), 7.668 (2.58).

### Intermediat 145

### (5RS)-2-[(1-Methyl-1H-pyrazol-3-yl)methyl]-3-oxo-2,3,5,6,7,8-hexahydro[1,2,4]triazolo[4,3-a]pyridin-5-carbonsäure -trifluoressigsäure (Racemat)

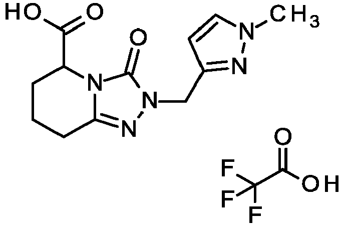

tert-Butyl-(5RS)-2-[(1-methyl-1H-pyrazol-3-yl)methyl]-3-oxo-2,3,5,6,7,8-hexahydro[1,2,4]triazolo[4,3-a]pyridin-5-carboxylat (Racemat) (150 mg, 450 µmοl) wurde in Dichlormethan (3.0 ml) gelöst und bei Raumtemperatur mit Trifluoressigsäure (350 µl, 4.5 mmol) versetzt. Das Reaktionsgemisch wurde über Nacht bei Raumtemperatur gerührt und anschließend erneut Trifluoressigsäure (350 µl, 4.5 mmol) zugegeben. Nachdem das Reaktionsgemisch über Nacht bei Raumtemperatur gerührt wurde, wurde das Lösungsmittel unter Vakuum entfernt. Es wurden 170 mg (97 % d. Th.) der Titelverbindung erhalten.
LC-MS (Methode 4): Rₜ = 0.36 min; MS (ESIpos): m/z = 278 [M+H]⁺

### Intermediat 146

### (5RS)-3-Oxo-2-(5,6,7,8-tetrahydronaphthalin-2-ylmethyl)-2,3,5,6,7,8-hexahydro[1,2,4]triazolo[4,3-a]pyridin-5-carbonsäure -trifluoressigsäure (Racemat)

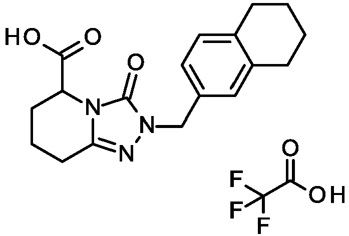

tert-Butyl-(5RS)-3-oxo-2-(5,6,7,8-tetrahydronaphthalin-2-ylmethyl)-2,3,5,6,7,8-hexahydro[1,2,4]triazolo[4,3-a]pyridin-5-carboxylat (Racemat) (280 mg, 730 µmοl) wurde in Dichlormethan (5.0 ml) gelöst und bei Raumtemperatur mit Trifluoressigsäure (2.0 ml, 26 mmol) versetzt. Nachdem das Reaktionsgemisch über Nacht bei Raumtemperatur gerührt wurde, wurde das Lösungsmittel unter Vakuum entfernt. Es wurden 320 mg (94 % d. Th.) der Titelverbindung erhalten.
LC-MS (Methode 4): Rₜ = 0.81 min; MS (ESIpos): m/z = 328 [M+H]⁺

### Intermediat 147

### (5RS)-2-[(5-Methyl-1,2-oxazol-3-yl)methyl]-3-oxo-2,3,5,6,7,8-hexahydro[1,2,4]triazolo[4,3-a]pyridin-5-carbonsäure (Racemat)

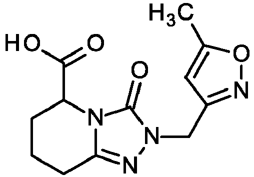

Methyl-(5RS)-2-[(5-methyl-1,2-oxazol-3-yl)methyl]-3-oxo-2,3,5,6,7,8-hexahydro[1,2,4]triazolo[4,3-a]pyridin-5-carboxylat (Racemat) (412 mg, 1.41 mmol) wurde in THF (14 ml) vorgelegt und Lithiumhydroxid (169 mg, 7.05 mmol) gelöst in Wasser (4.0 ml) zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 136 mg (83 % Reinheit, 29 % d. Th.) der Titelverbindung erhalten.
LC-MS (Methode 3): Rₜ = 0.67 min; MS (ESIpos): m/z = 279 [M+H]⁺
¹H-NMR (400 MHz, DMSO-d6) δ [ppm]: 1.157 (1.10), 1.174 (2.26), 1.181 (0.48), 1.192 (1.13), 1.356 (2.56), 1.405 (0.76), 1.499 (0.43), 1.513 (0.60), 1.526 (0.61), 1.541 (0.53), 1.797 (0.52), 1.809 (0.79), 1.820 (0.78), 1.831 (0.66), 1.842 (0.64), 1.908 (2.86), 1.988 (4.00), 2.083 (1.98), 2.089 (2.11), 2.099 (2.04), 2.111 (1.09), 2.201 (1.26), 2.369 (16.00), 2.561 (1.03), 2.573 (1.03), 2.587 (0.81), 2.617 (0.84), 2.629 (1.39), 2.639 (0.93), 2.659 (0.50), 2.670 (0.78), 4.020 (0.97), 4.038 (0.95), 4.426 (0.98), 4.441 (1.01), 4.450 (1.39), 4.462 (2.65), 4.474 (1.38), 4.807 (0.40), 4.847 (6.28), 4.852 (6.39), 4.892 (0.41), 6.069 (3.43), 6.180 (0.58).

### Intermediat 148

### (5RS)-3-Oxo-2-(pyridin-3-ylmethyl)-2,3,5,6,7,8-hexahydro[1,2,4]triazolo[4,3-a]pyridin-5-carbonsäure -trifluoressigsäure (Racemat)

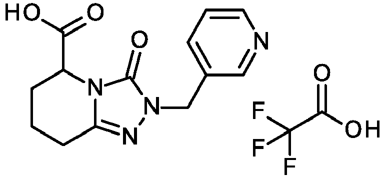

tert-Butyl-(5RS)-3-oxo-2-(pyridin-3-ylmethyl)-2,3,5,6,7,8-hexahydro[1,2,4]triazolo[4,3-a]pyridin-5-carboxylat (Racemat) (366 mg, 1.11 mmol) wurde in Dichlormethan (10 ml) gelöst und bei Raumtemperatur mit Trifluoressigsäure (1.7 ml, 22 mmol) versetzt. Nachdem das Reaktionsgemisch über Nacht bei Raumtemperatur gerührt wurde, wurde das Lösungsmittel unter Vakuum entfernt. Es wurden 442 mg (83 % Reinheit, 29 % d. Th.) der Titelverbindung erhalten.
LC-MS (Methode 9): Rₜ = 3.31 min; MS (ESIpos): m/z = 275 [M+H]⁺

### Intermediat 149

### (5RS)-3-Oxo-2-(2,4,5-trifluorbenzyl)-2,3,5,6,7,8-hexahydro[1,2,4]triazolo[4,3-a]pyridin-5-carbonsäure -trifluoressigsäure (Racemat)

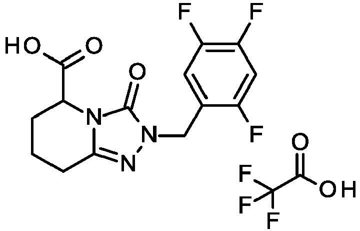

tert-Butyl-(5RS)-3-oxo-2-(2,4,5-trifluorbenzyl)-2,3,5,6,7,8-hexahydro[1,2,4]triazolo[4,3-a]pyridin-5-carboxylat (Racemat) (390 mg, 1.02 mmol) wurde in Dichlormethan (8 ml) gelöst und bei Raumtemperatur mit Trifluoressigsäure (3.3 ml, 43 mmol) versetzt. Nachdem das Reaktionsgemisch über Nacht bei Raumtemperatur gerührt wurde, wurde das Lösungsmittel unter Vakuum entfernt. Es wurden 445 mg (95 % d. Th.) der Titelverbindung erhalten.
LC-MS (Methode 3): Rₜ = 1.17 min; MS (ESIpos): m/z = 328 [M+H]⁺

### Intermediat 150

### (5RS)-2-[(2-Chlorpyridin-4-yl)methyl]-3-oxo-2,3,5,6,7,8-hexahydro[1,2,4]triazolo[4,3-a]pyridin-5-carbonsäure -trifluoressigsäure (Racemat)

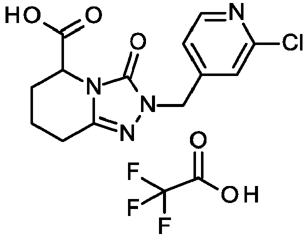

tert-Butyl-(5RS)-2-[(2-chlorpyridin-4-yl)methyl]-3-oxo-2,3,5,6,7,8-hexahydro[1,2,4]triazolo[4,3-a]pyridin-5-carboxylat (Racemat) (490 mg, 1.34 mmol) wurde in Dichlormethan (12 ml, 190 mmol) gelöst und bei Raumtemperatur mit Trifluoressigsäure (1.9 ml, 25 mmol) versetzt. Nachdem das Reaktionsgemisch über Nacht bei Raumtemperatur gerührt wurde, wurde das Lösungsmittel unter Vakuum entfernt. Es wurden 581 mg (94 % Reinheit, 96 % d. Th.) der Titelverbindung erhalten.
LC-MS (Methode 4): Rₜ = 0.50 min; MS (ESIpos): m/z = 309 [M+H]⁺

### Intermediat 151

### (5RS)-2-[(5-Chlor-2-thienyl)methyl]-3-oxo-2,3,5,6,7,8-hexahydro[1,2,4]triazolo[4,3-a]pyridin-5-carbonsäure -trifluoressigsäure (Racemat)

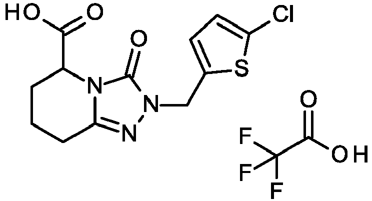

tert-Butyl-(5RS)-2-[(5-chlor-2-thienyl)methyl]-3-oxo-2,3,5,6,7,8-hexahydro[1,2,4]triazolo[4,3-a]pyridin-5-carboxylat (Racemat) (414 mg, 1.12 mmol) wurde in Dichlormethan (10 ml, 160 mmol) gelöst und bei Raumtemperatur mit Trifluoressigsäure (1.7 ml, 22 mmol) versetzt. Nachdem das Reaktionsgemisch für 5 Stunden bei Raumtemperatur gerührt wurde, wurde das Lösungsmittel unter Vakuum entfernt. Es wurden 516 mg (> 100 %) der Titelverbindung erhalten.
LC-MS (Methode 3): Rₜ = 1.16 min; MS (ESIpos): m/z = 314 [M+H]⁺
¹H-NMR (400 MHz, DMSO-d6) δ [ppm]: 1.110 (1.14), 1.384 (7.34), 1.535 (2.17), 1.568 (1.23), 1.802 (2.23), 1.814 (2.29), 1.824 (1.91), 2.058 (3.54), 2.071 (5.97), 2.081 (5.66), 2.328 (1.63), 2.366 (0.97), 2.565 (2.91), 2.578 (2.97), 2.592 (2.34), 2.623 (2.46), 2.635 (3.91), 2.646 (2.66), 2.675 (2.77), 2.710 (1.09), 4.438 (5.80), 4.451 (9.29), 4.463 (5.60), 4.555 (2.31), 4.906 (1.54), 4.945 (15.09), 4.951 (16.00), 4.986 (2.40), 5.560 (1.09), 6.916 (7.37), 6.926 (10.03), 6.973 (13.74), 6.983 (9.74), 7.020 (0.63), 7.079 (0.63).

### Intermediat 152

### (5RS)-2-[(4-Methyl-1,2,5-oxadiazol-3-yl)methyl]-3-oxo-2,3,5,6,7,8-hexahydro[1,2,4]triazolo[4,3-a]pyridin-5-carbonsäure (Racemat)

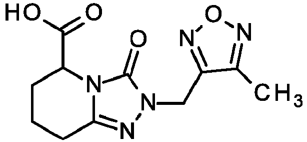

Methyl-(5RS)-2-[(4-methyl-1,2,5-oxadiazol-3-yl)methyl]-3-oxo-2,3,5,6,7,8-hexahydro[1,2,4]triazolo[4,3-a]pyridin-5-carboxylat (Racemat) (281 mg, 958 µmοl) wurde in THF (5.0 ml) vorgelegt und Lithiumhydroxid (115 mg, 4.79 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 91.0 mg (88 % Reinheit, 30 % d. Th.) der Titelverbindung erhalten.
LC-MS (Methode 1): Rₜ = 0.61 min; MS (ESIpos): m/z = 280 [M+H]⁺

### Intermediat 153

### (5RS)-2-(2-Methylbenzyl)-3-oxo-2,3,5,6,7,8-hexahydro[1,2,4]triazolo[4,3-a]pyridin-5-carbonsäure (Racemat)

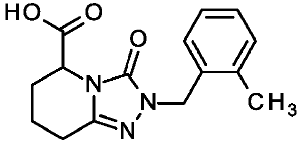

Methyl-(5RS)-2-(2-methylbenzyl)-3-oxo-2,3,5,6,7,8-hexahydro[1,2,4]triazolo[4,3-a]pyridin-5-carboxylat (Racemat) (196 mg, 650 µmοl) wurde in THF (5.0 ml) vorgelegt und Lithiumhydroxid (77.9 mg, 3.25 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 150 mg (80 % d. Th.) der Titelverbindung erhalten.
LC-MS (Methode 1): Rₜ = 0.88 min; MS (ESIpos): m/z = 288 [M+H]⁺

### Intermediat 154

### (5RS)-2-[4-Fluor-3-(trifluormethyl)benzyl]-3-oxo-2,3,5,6,7,8-hexahydro[1,2,4]triazolo[4,3-a]pyridin-5-carbonsäure (Racemat)

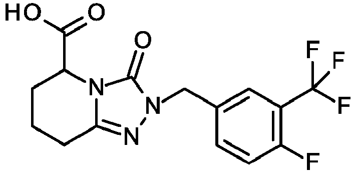

Methyl-(5RS)-2-[4-fluor-3-(trifluormethyl)benzyl]-3-oxo-2,3,5,6,7,8-hexahydro[1,2,4]triazolo[4,3-a]pyridin-5-carboxylat (Racemat) (569 mg, 1.52 mmol) wurde in THF (8.0 ml) vorgelegt und Lithiumhydroxid (183 mg, 7.62 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 251 mg (94 % Reinheit, 43 % d. Th.) der Titelverbindung erhalten.
LC-MS (Methode 4): Rₜ = 0.77 min; MS (ESIpos): m/z = 360 [M+H]⁺
¹H-NMR (400 MHz, DMSO-d6) δ [ppm]: -0.008 (2.89), 0.008 (2.76), 1.157 (2.61), 1.170 (1.52), 1.175 (5.50), 1.193 (2.73), 1.559 (1.10), 1.748 (1.40), 1.905 (1.45), 1.948 (1.17), 1.988 (10.96), 2.129 (1.67), 2.159 (1.21), 2.328 (0.45), 2.458 (0.96), 2.473 (1.31), 2.595 (2.13), 2.634 (1.08), 2.670 (0.46), 3.314 (0.58), 4.003 (0.74), 4.021 (2.44), 4.039 (2.45), 4.056 (0.80), 4.319 (2.46), 4.328 (2.44), 4.549 (0.88), 4.918 (16.00), 7.458 (2.32), 7.480 (3.39), 7.506 (3.05), 7.587 (1.95), 7.600 (2.29), 7.678 (3.14), 7.696 (3.13).

### Intermediat 155

### (5RS)-2-[2,5-Bis(trifluormethyl)benzyl]-3-oxo-2,3,5,6,7,8-hexahydro[1,2,4]triazolo[4,3-a]pyridin-5-carbonsäure (Racemat)

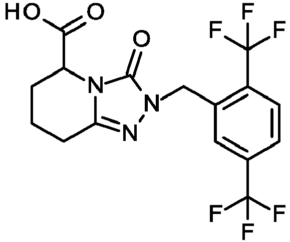

Methyl-(5RS)-2-[2,5-bis(trifluormethyl)benzyl]-3-oxo-2,3,5,6,7,8-hexahydro[1,2,4]triazolo[4,3-a]pyridin-5-carboxylat (Racemat) (634 mg, 1.50 mmol) wurde in THF (8.0 ml) vorgelegt und Lithiumhydroxid (179 mg, 7.48 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 242 mg (91 % Reinheit, 36 % d. Th.) der Titelverbindung erhalten.
LC-MS (Methode 4): Rₜ = 0.86 min; MS (ESIpos): m/z = 410 [M+H]⁺
¹H-NMR (400 MHz, DMSO-d6) δ [ppm]: -0.149 (0.54), -0.008 (4.55), 0.008 (3.70), 0.146 (0.50), 1.170 (2.38), 1.234 (0.54), 1.573 (1.56), 1.755 (1.93), 1.949 (1.55), 1.988 (2.59), 2.153 (2.28), 2.188 (1.65), 2.328 (1.13), 2.367 (0.71), 2.611 (2.68), 2.666 (1.51), 2.710 (0.66), 3.313 (2.26), 3.405 (1.49), 4.038 (0.40), 4.324 (2.78), 4.746 (1.49), 4.911 (2.40), 5.082 (16.00), 7.649 (8.11), 7.912 (3.70), 7.932 (6.12), 8.013 (7.38), 8.033 (4.99), 8.065 (1.22), 8.098 (0.71).

### Intermediat 156

### (5RS)-2-[(6-Methylpyridin-3-yl)methyl]-3-oxo-2,3,5,6,7,8-hexahydro[1,2,4]triazolo[4,3-a]pyridin-5-carbonsäure (Racemat)

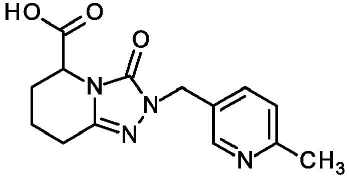

Methyl-(5RS)-2-[(6-methylpyridin-3-yl)methyl]-3-oxo-2,3,5,6,7,8-hexahydro[1,2,4]triazolo[4,3-a]pyridin-5-carboxylat (Racemat) (231 mg, 764 µmοl) wurde in THF (5.0 ml) vorgelegt und Lithiumhydroxid (91.5 mg, 3.82 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die wässrige Phase wurde eingeengt und mit Tetrahydrofuran/Ethanol (1/1) suspendiert. Der Feststoff wurde abgetrennt und das Filtrat zum Produkt eingeengt. Es wurden 262 mg (> 100 %) der Titelverbindung erhalten.
LC-MS (Methode 4): Rₜ = 0.19 min; MS (ESIpos): m/z = 288 [M]⁺
¹H-NMR (400 MHz, DMSO-d6) δ [ppm]: -0.149 (0.62), -0.008 (5.32), 0.008 (4.64), 0.146 (0.68), 1.038 (7.69), 1.055 (16.00), 1.072 (8.00), 1.356 (2.60), 1.743 (0.97), 1.760 (2.83), 1.776 (0.99), 2.090 (0.88), 2.328 (0.88), 2.366 (0.48), 2.561 (2.65), 2.620 (0.56), 2.670 (0.90), 2.710 (0.48), 3.413 (3.17), 3.431 (8.60), 3.448 (8.67), 3.466 (3.40), 3.585 (1.73), 3.601 (3.05), 3.618 (1.62), 4.465 (0.59), 4.475 (1.11), 4.936 (1.69), 8.492 (0.70).

### Intermediat 157

### (5RS)-2-[(6-Methoxypyridin-3-yl)methyl]-3-oxo-2,3,5,6,7,8-hexahydro[1,2,4]triazolo[4,3-a]pyridin-5-carbonsäure (Racemat)

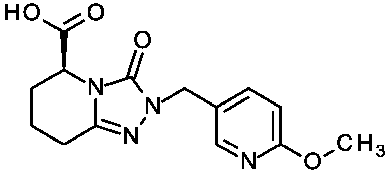

Methyl-(5RS)-2-[(6-methoxypyridin-3-yl)methyl]-3-oxo-2,3,5,6,7,8-hexahydro[1,2,4]triazolo[4,3-a]pyridin-5-carboxylat (Racemat) (202 mg, 636 µmοl) wurde in THF (5.0 ml) vorgelegt und Lithiumhydroxid (76.1 mg, 3.18 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 45 mg (23 % d. Th.) der Titelverbindung erhalten.
LC-MS (Methode 3): Rₜ = 0.78 min; MS (ESIpos): m/z = 305 [M]⁺

### Intermediat 158

### (5RS)-2-[4-Methoxy-3-(trifluormethyl)benzyl]-3-oxo-2,3,5,6,7,8-hexahydro[1,2,4]triazolo[4,3-a]pyridin-5-carbonsäure (Racemat)

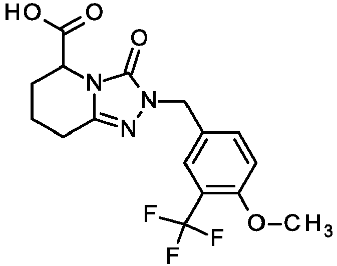

Methyl-(5RS)-2-[4-methoxy-3-(trifluormethyl)benzyl]-3-oxo-2,3,5,6,7,8-hexahydro[1,2,4]triazolo[4,3-a]pyridin-5-carboxylat (Racemat) (305 mg, 791 µmοl) wurde in THF (8.0 ml) vorgelegt und Lithiumhydroxid (94.8 mg, 3.96 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 151 mg (51 % d. Th.) der Titelverbindung erhalten.
LC-MS (Methode 3): Rₜ = 1.34 min; MS (ESIpos): m/z = 372 [M+H]⁺
¹H-NMR (400 MHz, DMSO-d6) δ [ppm]: -0.008 (1.34), 0.008 (1.63), 1.157 (2.20), 1.175 (4.44), 1.192 (2.26), 1.529 (0.44), 1.785 (0.53), 1.797 (0.50), 1.807 (0.43), 1.819 (0.43), 1.988 (8.02), 2.045 (0.43), 2.079 (1.23), 2.088 (1.19), 2.562 (0.66), 2.598 (0.58), 2.610 (0.95), 2.620 (0.64), 2.650 (0.43), 3.869 (16.00), 4.002 (0.65), 4.020 (1.93), 4.038 (1.92), 4.056 (0.63), 4.419 (0.71), 4.429 (0.98), 4.835 (7.06), 7.223 (1.56), 7.244 (1.81), 7.498 (1.32), 7.520 (4.16).

### Intermediat 159

### (5RS)-2-[(1-Methyl-1H-benzimidazol-2-yl)methyl]-3-oxo-2,3,5,6,7,8-hexahydro[1,2,4]triazolo[4,3-a]pyridin-5-carbonsäure (Racemat)

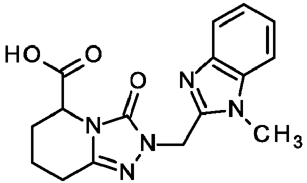

Methyl-(5RS)-2-[(1-methyl-1H-benzimidazol-2-yl)methyl]-3-oxo-2,3,5,6,7,8-hexahydro[1,2,4]triazolo[4,3-a]pyridin-5-carboxylat (Racemat) (137 mg, 401 µmοl) wurde in THF (3.0 ml) vorgelegt und Lithiumhydroxid (48.1 mg, 2.01 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 120 mg (91 % d. Th.) der Titelverbindung erhalten.
LC-MS (Methode 3): Rₜ = 0.60 min; MS (ESIpos): m/z = 328 [M+H]⁺

### Intermediat 160

### (5RS)-2-[(1-Ethyl-1H-imidazol-2-yl)methyl]-3-oxo-2,3,5,6,7,8-hexahydro[1,2,4]triazolo[4,3-a]pyridin-5-carbonsäure (Racemat)

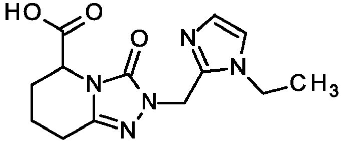

Methyl-(5RS)-2-[(1-ethyl-1H-imidazol-2-yl)methyl]-3-oxo-2,3,5,6,7,8-hexahydro[1,2,4]triazolo[4,3-a]pyridin-5-carboxylat (Racemat) (54.0 mg, 177 µmοl) wurde in THF (2.0 ml) vorgelegt und Lithiumhydroxid (21.2 mg, 884 µmοl) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 80.0 mg (60 % Reinheit, 93 % d. Th.) der Titelverbindung erhalten.
LC-MS (Methode 9): Rₜ = 3.44 min; MS (ESIpos): m/z = 292 [M+H]⁺

### Intermediat 161

### (5RS)-2-[(1-Methyl-1H-indazol-5-yl)methyl]-3-oxo-2,3,5,6,7,8-hexahydro[1,2,4]triazolo[4,3-a]pyridin-5-carbonsäure (Racemat)

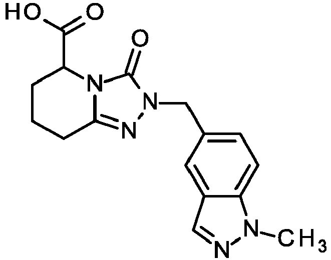

Methyl-(5RS)-2-[(1-methyl-1H-indazol-5-yl)methyl]-3-oxo-2,3,5,6,7,8-hexahydro[1,2,4]triazolo[4,3-a]pyridin-5-carboxylat (Racemat) (87.0 mg, 98 % Reinheit, 250 µmοl) wurde in THF (2.5 ml) vorgelegt und Lithiumhydroxid (29.9 mg, 1.25 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 67.0 mg (80 % d. Th.) der Titelverbindung erhalten.
LC-MS (Methode 3): Rₜ = 0.87 min; MS (ESIpos): m/z = 328 [M+H]⁺
¹H-NMR (400 MHz, DMSO-d6) δ [ppm]: -0.008 (0.92), 0.008 (0.91), 1.157 (1.12), 1.175 (2.28), 1.192 (1.13), 1.796 (0.45), 1.807 (0.50), 1.908 (0.74), 1.988 (4.27), 2.082 (1.22), 2.327 (0.41), 2.523 (2.15), 2.565 (0.61), 2.596 (0.54), 2.608 (0.89), 2.619 (0.60), 2.648 (0.42), 2.669 (0.50), 4.002 (0.41), 4.021 (16.00), 4.038 (1.11), 4.457 (0.87), 4.468 (1.72), 4.481 (0.89), 4.916 (3.28), 4.921 (3.27), 7.294 (1.26), 7.298 (1.26), 7.316 (1.45), 7.320 (1.49), 7.580 (1.84), 7.608 (2.57), 8.004 (3.39), 13.222 (0.63).

### Intermediat 162

### (5RS)-3-Oxo-2-({5-[3-(trifluormethyl)phenyl]-1,2,4-oxadiazol-3-yl}methyl)-2,3,5,6,7,8-hexahydro[1,2,4]triazolo[4,3-a]pyridin-5-carbonsäure (Racemat)

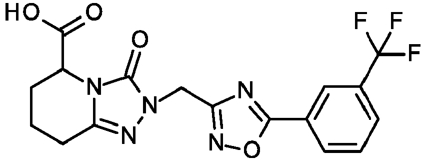

Methyl-(5RS)-3-oxo-2-({5-[3-(trifluormethyl)phenyl]-1,2,4-oxadiazol-3-yl}methyl)-2,3,5,6,7,8-hexahydro[1,2,4]triazolo[4,3-a]pyridin-5-carboxylat (Racemat) (640 mg, 1.51 mmol) wurde in THF (15 ml) vorgelegt und Lithiumhydroxid (181 mg, 7.56 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 588 mg (88 % Reinheit, 84 % d. Th.) der Titelverbindung erhalten.
LC-MS (Methode 3): Rₜ = 1.42 min; MS (ESIpos): m/z = 410 [M+H]⁺
¹H-NMR (400 MHz, DMSO-d6) δ [ppm]: -0.008 (1.41), 0.008 (1.36), 1.094 (1.28), 1.157 (4.33), 1.175 (8.82), 1.193 (4.46), 1.519 (0.93), 1.533 (1.05), 1.546 (1.17), 1.556 (1.03), 1.570 (0.87), 1.582 (0.66), 1.803 (0.93), 1.814 (1.46), 1.825 (1.48), 1.836 (1.29), 1.847 (1.23), 1.861 (0.85), 1.909 (2.18), 1.989 (16.00), 2.084 (2.29), 2.096 (4.23), 2.107 (4.15), 2.120 (2.23), 2.520 (2.12), 2.562 (2.37), 2.577 (1.87), 2.589 (1.95), 2.604 (1.50), 2.631 (1.58), 2.642 (2.66), 2.654 (1.73), 2.673 (1.07), 2.684 (1.24), 2.697 (0.66), 3.396 (0.92), 4.003 (1.25), 4.021 (3.77), 4.039 (3.73), 4.056 (1.23), 4.485 (2.76), 4.498 (5.49), 4.509 (2.71), 4.654 (0.81), 4.936 (1.26), 5.001 (1.44), 5.059 (3.19), 5.099 (10.70), 5.126 (10.72), 5.166 (3.27), 7.875 (2.15), 7.894 (4.91), 7.914 (3.01), 8.100 (3.54), 8.120 (3.11), 8.327 (5.27), 8.391 (3.58), 8.411 (3.46), 13.270 (1.29).

### Intermediat 163

### (5RS)-2-{[3-Chlor-5-(trifluormethyl)pyridin-2-yl]methyl}-3-oxo-2,3,5,6,7,8-hexahydro[1,2,4]triazolo[4,3-a]pyridin-5-carbonsäure (Racemat)

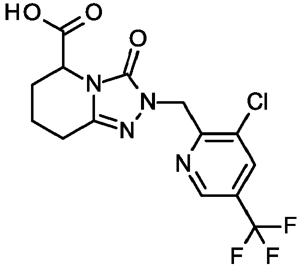

Methyl-(5RS)-2-{[3-chlor-5-(trifluormethyl)pyridin-2-yl]methyl}-3-oxo-2,3,5,6,7,8-hexahydro[1,2,4]triazolo[4,3-a]pyridin-5-carboxylat (Racemat) (293 mg, 750 µmοl) wurde in THF (6.0 ml) vorgelegt und Lithiumhydroxid (89.8 mg, 3.75 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 243 mg (86 % d. Th.) der Titelverbindung erhalten.
LC-MS (Methode 3): Rₜ = 1.16 min; MS (ESIpos): m/z = 377 [M+H]⁺
¹H-NMR (400 MHz, DMSO-d6) δ [ppm]: 1.175 (0.73), 1.988 (1.31), 2.079 (0.50), 2.091 (0.90), 2.101 (0.88), 2.107 (0.79), 2.558 (0.47), 2.610 (0.60), 2.621 (0.40), 4.466 (0.65), 4.479 (1.30), 4.491 (0.64), 5.151 (2.36), 5.155 (2.31), 5.753 (16.00), 8.487 (1.31), 8.491 (1.32), 8.903 (1.28), 8.905 (1.29).

### Intermediat 164

### (5RS)-2-[3-Fluor-4-(trifluormethoxy)benzyl]-3-oxo-2,3,5,6,7,8-hexahydro[1,2,4]triazolo[4,3-a]pyridin-5-carbonsäure (Racemat)

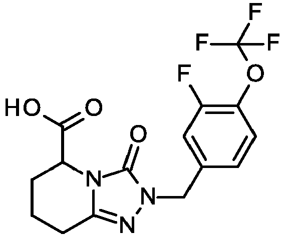

Methyl-(5RS)-2-[3-fluor-4-(trifluormethoxy)benzyl]-3-oxo-2,3,5,6,7,8-hexahydro[1,2,4]triazolo[4,3-a]pyridin-5-carboxylat (Racemat) (263 mg, 676 µmοl) wurde in THF (5.0 ml) vorgelegt und Lithiumhydroxid (80.9 mg, 3.38 mmol) gelöst in Wasser zugegeben. Das Reaktionsgemisch wurde nach Rühren für 72 Stunden bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 180 mg (71 % d. Th.) der Titelverbindung erhalten.
LC-MS (Methode 3): Rₜ = 1.43 min; MS (ESIpos): m/z = 376 [M+H]⁺

### Intermediat 165

### (5RS)-2-[3-Chlor-4-(trifluormethoxy)benzyl]-3-oxo-2,3,5,6,7,8-hexahydro[1,2,4]triazolo[4,3-a]pyridin-5-carbonsäure (Racemat)

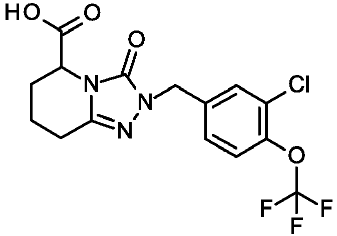

Methyl-(5RS)-2-[3-chlor-4-(trifluormethoxy)benzyl]-3-oxo-2,3,5,6,7,8-hexahydro[1,2,4]triazolo[4,3-a]pyridin-5-carboxylat (Racemat) (293 mg, 99 % Reinheit, 715 µmοl) wurde in THF (7.2 ml) vorgelegt und Lithiumhydroxid (85.6 mg, 3.57 mmol) gelöst in Wasser zugegeben. Das Reaktionsgemisch wurde nach 2 Stunden Rühren bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 85.0 mg (29 % d. Th.) der Titelverbindung erhalten.
LC-MS (Methode 3): Rₜ = 1.55 min; MS (ESIpos): m/z = 391 [M+H]⁺
¹H-NMR (400 MHz, DMSO-d6) δ [ppm]: -0.149 (0.78), -0.008 (7.90), 0.008 (6.70), 0.146 (0.88), 1.038 (0.50), 1.055 (1.00), 1.073 (0.50), 1.157 (1.52), 1.164 (0.90), 1.175 (3.06), 1.182 (1.88), 1.193 (1.64), 1.200 (0.90), 1.235 (0.48), 1.513 (0.96), 1.526 (0.98), 1.819 (1.28), 1.829 (1.24), 1.839 (1.04), 1.851 (1.04), 1.908 (12.72), 1.988 (5.68), 2.102 (3.40), 2.112 (3.14), 2.124 (1.72), 2.328 (0.90), 2.366 (0.68), 2.518 (3.92), 2.523 (3.12), 2.569 (1.76), 2.582 (1.78), 2.596 (1.36), 2.628 (1.36), 2.639 (2.16), 2.650 (1.68), 2.670 (1.68), 2.679 (1.30), 2.692 (0.64), 2.710 (0.66), 3.431 (3.30), 3.449 (3.50), 4.003 (0.46), 4.021 (1.34), 4.038 (1.36), 4.056 (0.50), 4.157 (0.42), 4.164 (0.42), 4.481 (2.44), 4.493 (4.72), 4.505 (2.42), 4.908 (16.00), 7.326 (3.02), 7.332 (2.84), 7.348 (3.62), 7.353 (3.38), 7.531 (5.82), 7.536 (5.94), 7.545 (3.42), 7.549 (3.28), 7.566 (2.76), 7.570 (2.58).

### Intermediat 166

### (5RS)-2-{[2-Methyl-4-(trifluormethyl)-1,3-thiazol-5-yl]methyl}-3-oxo-2,3,5,6,7,8-hexahydro[1,2,4]triazolo[4,3-a]pyridin-5-carbonsäure (Racemat)

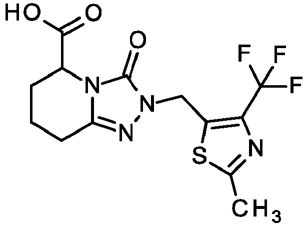

Methyl-(5RS)-2-{[2-methyl-4-(trifluormethyl)-1,3-thiazol-5-yl]methyl}-3-oxo-2,3,5,6,7,8-hexahydro[1,2,4]triazolo[4,3-a]pyridin-5-carboxylat (Racemat) (380 mg, 1.01 mmol) wurde in THF (10 ml) vorgelegt und Lithiumhydroxid (121 mg, 5.05 mmol) gelöst in Wasser zugegeben. Das Reaktionsgemisch wurde nach 3 Stunden Rühren bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 156 mg (41 % d. Th.) der Titelverbindung erhalten.
LC-MS (Methode 4): Rₜ = 0.61 min; MS (ESIpos): m/z = 363 [M+H]⁺
¹H-NMR (400 MHz, DMSO-d6) δ [ppm]: -0.008 (1.08), 0.008 (0.89), 1.157 (2.79), 1.175 (5.61), 1.193 (2.81), 1.356 (0.90), 1.797 (0.50), 1.810 (0.50), 1.820 (0.40), 1.832 (0.41), 1.908 (0.41), 1.988 (10.16), 2.050 (0.44), 2.064 (0.79), 2.075 (1.54), 2.086 (1.45), 2.097 (0.74), 2.343 (0.59), 2.519 (1.18), 2.563 (0.72), 2.576 (0.73), 2.590 (0.59), 2.627 (0.71), 2.643 (16.00), 2.670 (0.82), 2.679 (0.57), 4.003 (0.80), 4.021 (2.41), 4.038 (2.39), 4.056 (0.77), 4.431 (0.85), 4.441 (1.46), 4.455 (0.85), 5.162 (3.10).

### Intermediat 167

### (5RS)-2-[(3-Methyl-1,2-oxazol-5-yl)methyl]-3-oxo-2,3,5,6,7,8-hexahydro[1,2,4]triazolo[4,3-a]pyridin-5-carbonsäure (Racemat)

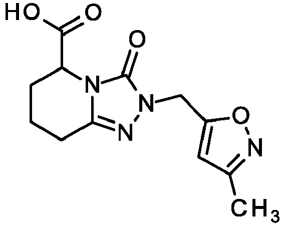

Methyl-(5RS)-2-[(3-methyl-1,2-oxazol-5-yl)methyl]-3-oxo-2,3,5,6,7,8-hexahydro[1,2,4]triazolo[4,3-a]pyridin-5-carboxylat (Racemat) (259 mg, 86 % Reinheit, 762 µmοl) wurde in THF (7.7 ml) vorgelegt und Lithiumhydroxid (91.2 mg, 3.81 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 101 mg (90 % Reinheit, 43 % d. Th.) der Titelverbindung erhalten.
LC-MS (Methode 3): Rₜ = 0.64 min; MS (ESIpos): m/z = 279 [M+H]⁺
¹H-NMR (400 MHz, DMSO-d6) δ [ppm]: -0.008 (1.37), 0.008 (1.35), 1.157 (0.64), 1.175 (1.32), 1.192 (0.66), 1.812 (0.53), 1.823 (0.54), 1.834 (0.46), 1.845 (0.44), 1.908 (0.48), 1.988 (2.37), 2.071 (0.84), 2.084 (1.48), 2.093 (1.42), 2.100 (1.31), 2.113 (0.67), 2.201 (16.00), 2.226 (0.83), 2.524 (0.85), 2.567 (0.76), 2.580 (0.76), 2.594 (0.61), 2.621 (0.61), 2.633 (0.96), 2.644 (0.67), 2.664 (0.43), 2.674 (0.56), 4.020 (0.57), 4.038 (0.55), 4.455 (1.00), 4.467 (1.96), 4.479 (1.00), 4.495 (0.61), 4.509 (0.60), 4.636 (0.43), 4.973 (4.04), 4.978 (4.07), 6.210 (0.49), 6.234 (3.48).

### Intermediat 168

### (5RS)-2-[2-Fluor-3-(trifluormethyl)benzyl]-3-oxo-2,3,5,6,7,8-hexahydro[1,2,4]triazolo[4,3-a]pyridin-5-carbonsäure (Racemat)

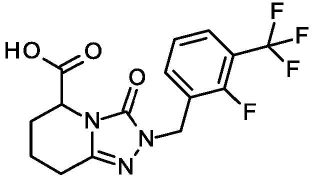

Methyl-(5RS)-2-[2-fluor-3-(trifluormethyl)benzyl]-3-oxo-2,3,5,6,7,8-hexahydro[1,2,4]triazolo[4,3-a]pyridin-5-carboxylat (Racemat) (184 mg, 493 µmοl) wurde in THF (3.0 ml) vorgelegt und Lithiumhydroxid (59.0 mg, 2.46 mmol) gelöst in Wasser zugegeben. Das Reaktionsgemisch wurde nach 72 Stunden Rühren bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 166 mg (94 % d. Th.) der Titelverbindung erhalten.
LC-MS (Methode 3): Rₜ = 1.35 min; MS (ESIpos): m/z = 360 [M+H]⁺

### Intermediat 169

### (5RS)-2-[3-Fluor-5-(trifluormethyl)benzyl]-3-oxo-2,3,5,6,7,8-hexahydro[1,2,4]triazolo[4,3-a]pyridin-5-carbonsäure (Racemat)

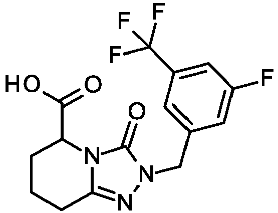

Methyl-(5RS)-2-[3-fluor-5-(trifluormethyl)benzyl]-3-oxo-2,3,5,6,7,8-hexahydro[1,2,4]triazolo[4,3-a]pyridin-5-carboxylat (Racemat) (154 mg, 413 µmοl) wurde in THF (3.0 ml) vorgelegt und Lithiumhydroxid (49.4 mg, 2.06 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 124 mg (84 % d. Th.) der Titelverbindung erhalten.
LC-MS (Methode 3): Rₜ = 1.39 min; MS (ESIpos): m/z = 360 [M+H]⁺

### Intermediat 170

### (5RS)-3-Oxo-2-[(3,5,6-trimethylpyrazin-2-yl)methyl]-2,3,5,6,7,8-hexahydro[1,2,4]triazolo[4,3-a]pyridin-5-carbonsäure (Racemat)

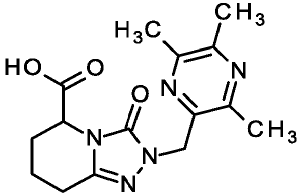

Methyl-(5RS)-3-oxo-2-[(3,5,6-trimethylpyrazin-2-yl)methyl]-2,3,5,6,7,8-hexahydro[1,2,4]triazolo[4,3-a]pyridin-5-carboxylat (Racemat) (179 mg, 540 µmοl) wurde in THF (5.0 ml) vorgelegt und Lithiumhydroxid (64.7 mg, 2.70 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 80.0 mg (47 % d. Th.) der Titelverbindung erhalten.
LC-MS (Methode 1): Rₜ = 0.65 min; MS (ESIpos): m/z = 318 [M+H]⁺

### Intermediat 171

### (5RS)-2-[(1-Methyl-1H-1,2,4-triazol-3-yl)methyl]-3-oxo-2,3,5,6,7,8-hexahydro[1,2,4]triazolo[4,3-a]pyridin-5-carbonsäure (Racemat)

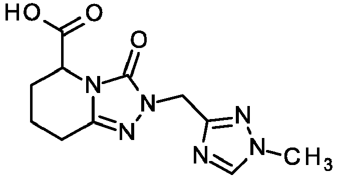

Methyl-(5RS)-2-[(1-methyl-1H-1,2,4-triazol-3-yl)methyl]-3-oxo-2,3,5,6,7,8-hexahydro[1,2,4]triazolo[4,3-a]pyridin-5-carboxylat (Racemat) (176 mg, 74 % Reinheit, 446 µmοl) wurde in THF (4.5 ml) vorgelegt und Lithiumhydroxid (53.4 mg, 2.23 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die wässrige Phase wurde eingeengt und so 268 mg (76 % Reinheit, >100 %) der Titelverbindung erhalten.
LC-MS (Methode 9): Rₜ = 3.37 min; MS (ESIpos): m/z = 279 [M+H]⁺
¹H-NMR (400 MHz, DMSO-d6) δ [ppm]: -0.149 (0.54), -0.008 (3.90), 0.146 (0.46), 1.592 (0.57), 1.705 (0.76), 1.857 (0.54), 2.119 (0.81), 2.166 (3.04), 2.329 (0.89), 2.368 (0.50), 2.452 (1.14), 2.670 (0.80), 2.712 (0.42), 3.041 (1.56), 3.407 (1.18), 3.815 (16.00), 3.839 (0.43), 3.851 (0.70), 3.924 (2.27), 4.152 (0.95), 4.672 (1.53), 4.688 (0.46), 4.710 (2.53), 4.758 (1.03), 4.815 (2.91), 4.854 (1.76), 8.345 (0.57), 8.360 (3.83), 8.675 (0.64), 11.177 (0.50).

### Intermediat 172

### (5RS)-3-Oxo-2-{[2-(trifluormethyl)pyridin-4-yl]methyl}-2,3,5,6,7,8-hexahydro[1,2,4]triazolo[4,3-a]pyridin-5-carbonsäure (Enantiomer 1)

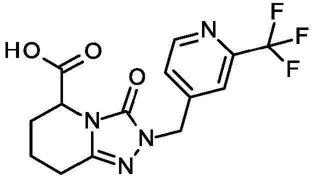

Methyl-(5RS)-3-oxo-2-{[2-(trifluormethyl)pyridin-4-yl]methyl}-2,3,5,6,7,8-hexahydro[1,2,4]triazolo[4,3-a]pyridin-5-carboxylat (Enantiomer 1) (279 mg, 783 µmοl) wurde in THF (5.0 ml) vorgelegt und Lithiumhydroxid (93.8 mg, 3.92 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 180 mg (67 % d. Th.) der Titelverbindung erhalten.
LC-MS (Methode 3): Rₜ = 0.95 min; MS (ESIpos): m/z = 343 [M+H]⁺

Die (5S)-Konfiguration wurde aufgrund der Kristallstrukturaufklärung für Beispiel 26, 108, 113, 157, 237, 358 und 454 zugeordnet.

### (5S)-3-Oxo-2-{[2-(trifluormethyl)pyridin-4-yl]methyl}-2,3,5,6,7,8-hexahydro[1,2,4]triazolo[4,3-a]pyridin-5-carbonsäure

### Intermediat 173

### (5RS)-2-{[3-Fluor-2-(trifluormethyl)pyridin-4-yl]methyl}-3-oxo-2,3,5,6,7,8-hexahydro[1,2,4]triazolo[4,3-a]pyridin-5-carbonsäure (Enantiomer 1)

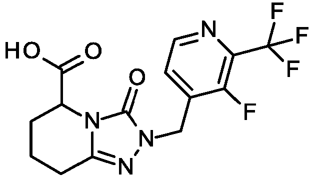

Methyl-(5RS)-2-{[3-fluor-2-(trifluormethyl)pyridin-4-yl]methyl}-3-oxo-2,3,5,6,7,8-hexahydro[1,2,4]triazolo[4,3-a]pyridin-5-carboxylat (Enantiomer 1) (181 mg, 484 µmοl) wurde in THF (3.0 ml) vorgelegt und Lithiumhydroxid (57.9 mg, 2.42 mmol) gelöst in Wasser zugegeben. Das Reaktionsgemisch wurde nach 72 Stunden Rühren bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 150 mg (86 % d. Th.) der Titelverbindung erhalten.
LC-MS (Methode 1): Rₜ = 0.89 min; MS (ESIpos): m/z = 361 [M+H]⁺

Die (5S)-Konfiguration wurde aufgrund der Kristallstrukturaufklärung für Beispiel 26, 108, 113, 157, 237, 358 und 454 zugeordnet.

### (5S)-2-{[3-Fluor-2-(trifluormethyl)pyridin-4-yl]methyl}-3-oxo-2,3,5,6,7,8-hexahydro[1,2,4]triazolo[4,3-a]pyridin-5-carbonsäure

### Intermediat 174

### (5RS)-3-Oxo-2-{[2-(trifluormethyl)chinolin-4-yl]methyl}-2,3,5,6,7,8-hexahydro[1,2,4]triazolo[4,3-a]pyridin-5-carbonsäure (Enantiomer 1)

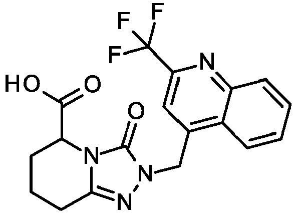

Methyl-(5RS)-3-oxo-2-{[2-(trifluormethyl)chinolin-4-yl]methyl}-2,3,5,6,7,8-hexahydro[1,2,4]triazolo[4,3-a]pyridin-5-carboxylat (Enantiomer 1) (111 mg, 273 µmοl) wurde in THF (3.0 ml) vorgelegt und Lithiumhydroxid (32.7 mg, 1.37 mmol) gelöst in Wasser zugegeben. Das Reaktionsgemisch wurde nach 72 Stunden Rühren bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 94.0 mg (88 % d. Th.) der Titelverbindung erhalten.
LC-MS (Methode 4): Rₜ = 0.76 min; MS (ESIpos): m/z = 393 [M+H]⁺

Die (5S)-Konfiguration wurde aufgrund der Kristallstrukturaufklärung für Beispiel 26, 108, 113, 157, 237, 358 und 454 zugeordnet.

### (5S)-3-Oxo-2-{[2-(trifluormethyl)chinolin-4-yl]methyl}-2,3,5,6,7,8-hexahydro[1,2,4]triazolo[4,3-a]pyridin-5-carbonsäure

### Intermediat 175

### (5RS)-2-{[5-Chlor-4-(trifluormethyl)pyridin-2-yl]methyl}-3-oxo-2,3,5,6,7,8-hexahydro[1,2,4]triazolo[4,3-a]pyridin-5-carbonsäure (Enantiomer 1)

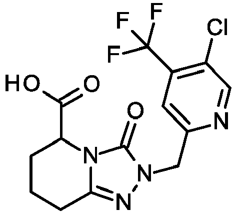

Methyl-(5RS)-2-{[5-chlor-4-(trifluormethyl)pyridin-2-yl]methyl}-3-oxo-2,3,5,6,7,8-hexahydro[1,2,4]triazolo[4,3-a]pyridin-5-carboxylat (Enantiomer 1) (269 mg, 688 µmοl) wurde in THF (3.0 ml) vorgelegt und Lithiumhydroxid (82.4 mg, 3.44 mmol) gelöst in Wasser zugegeben. Das Reaktionsgemisch wurde nach 72 Stunden Rühren bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 100 mg (39 % d. Th.) der Titelverbindung erhalten.
LC-MS (Methode 3): Rₜ = 1.29 min; MS (ESIpos): m/z = 377 [M+H]⁺

Die (5S)-Konfiguration wurde aufgrund der Kristallstrukturaufklärung für Beispiel 26, 108, 113, 157, 237, 358 und 454 zugeordnet.

### (5S)-2-{[5-Chlor-4-(trifluormethyl)pyridin-2-yl]methyl}-3-oxo-2,3,5,6,7,8-hexahydro[1,2,4]triazolo[4,3-a]pyridin-5-carbonsäure

### Intermediat 176

### (5RS)-3-Oxo-2-[(3,5,6-trimethylpyrazin-2-yl)methyl]-2,3,5,6,7,8-hexahydro[1,2,4]triazolo[4,3-a]pyridin-5-carbonsäure (Enantiomer 1)

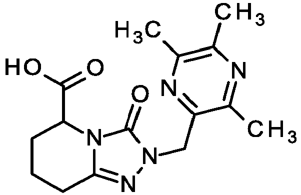

Methyl-(5RS)-3-oxo-2-[(3,5,6-trimethylpyrazin-2-yl)methyl]-2,3,5,6,7,8-hexahydro[1,2,4]triazolo[4,3-a]pyridin-5-carboxylat (Enantiomer 1) (261 mg, 788 µmοl) wurde in THF (5.0 ml) vorgelegt und Lithiumhydroxid (94.3 mg, 3.94 mmol) gelöst in Wasser zugegeben. Das Reaktionsgemisch wurde nach 72 Stunden Rühren bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 124 mg (50 % d. Th.) der Titelverbindung erhalten.
LC-MS (Methode 3): Rₜ = 0.77 min; MS (ESIpos): m/z = 318 [M+H]⁺

### Intermediat 177

### (5RS)-2-{[3-Chlor-5-(trifluormethyl)pyridin-2-yl]methyl}-3-oxo-2,3,5,6,7,8-hexahydro[1,2,4]triazolo[4,3-a]pyridin-5-carbonsäure (Enantiomer 1)

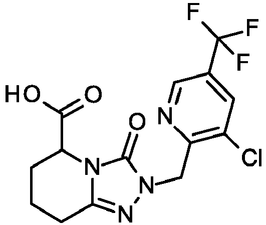

Methyl-(5RS)-2-{[3-chlor-5-(trifluormethyl)pyridin-2-yl]methyl}-3-oxo-2,3,5,6,7,8-hexahydro[1,2,4]triazolo[4,3-a]pyridin-5-carboxylat (Enantiomer 1) (216 mg, 553 µmοl) wurde in THF (4.0 ml) vorgelegt und Lithiumhydroxid (66.2 mg, 2.76 mmol) gelöst in Wasser zugegeben. Das Reaktionsgemisch wurde nach 72 Stunden Rühren bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 181 mg (87 % d. Th.) der Titelverbindung erhalten.
LC-MS (Methode 3): Rₜ = 1.21 min; MS (ESIpos): m/z = 377 [M+H]⁺

### Intermediat 178

### (5RS)-3-Oxo-2-{[4-(trifluormethyl)pyridin-2-yl]methyl}-2,3,5,6,7,8-hexahydro[1,2,4]triazolo[4,3-a]pyridin-5-carbonsäure (Enantiomer 1)

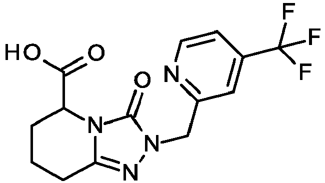

Methyl-(5RS)-3-oxo-2-{[4-(trifluormethyl)pyridin-2-yl]methyl}-2,3,5,6,7,8-hexahydro[1,2,4]triazolo[4,3-a]pyridin-5-carboxylat (Enantiomer 1) (275 mg, 770 µmοl) wurde in THF (10 ml) vorgelegt und Lithiumhydroxid (92.2 mg, 3.85 mmol) gelöst in Wasser zugegeben. Das Reaktionsgemisch wurde nach 5 Stunden Rühren bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 247 mg (94 % d. Th.) der Titelverbindung erhalten.
LC-MS (Methode 4): Rₜ = 0.59 min; MS (ESIpos): m/z = 343 [M+H]⁺

Die (5S)-Konfiguration wurde aufgrund der Kristallstrukturaufklärung für Beispiel 26, 108, 113, 157, 237, 358 und 454 zugeordnet.

### (5S)-3-Oxo-2-{[4-(trifluormethyl)pyridin-2-yl]methyl}-2,3,5,6,7,8-hexahydro[1,2,4]triazolo[4,3-a]pyridin-5-carbonsäure

### Intermediat 179

### (5RS)-3-Oxo-2-{[2-(trifluormethyl)pyrimidin-4-yl]methyl}-2,3,5,6,7,8-hexahydro[1,2,4]triazolo[4,3-a]pyridin-5-carbonsäure (Enantiomer 1)

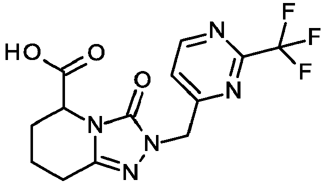

Methyl-(5RS)-3-oxo-2-{[2-(trifluormethyl)pyrimidin-4-yl]methyl}-2,3,5,6,7,8-hexahydro[1,2,4]triazolo[4,3-a]pyridin-5-carboxylat (Enantiomer 1) (49.5 mg, 139 µmοl) wurde in THF (2.0 ml) vorgelegt und Lithiumhydroxid (16.6 mg, 693 µmοl) gelöst in Wasser zugegeben. Das Reaktionsgemisch wurde nach 5 Stunden Rühren bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 45.0 mg (95 % d. Th.) der Titelverbindung erhalten.
LC-MS (Methode 4): Rₜ = 0.55 min; MS (ESIpos): m/z = 344 [M+H]⁺

Die (5S)-Konfiguration wurde aufgrund der Kristallstrukturaufklärung für Beispiel 26, 108, 113, 157, 237, 358 und 454 zugeordnet.

### (5S)-3-Oxo-2-{[2-(trifluormethyl)pyrimidin-4-yl]methyl}-2,3,5,6,7,8-hexahydro[1,2,4]triazolo[4,3-a]pyridin-5-carbonsäure

### Intermediat 180

### (5RS)-3-Oxo-2-(2,4,5-trimethylbenzyl)-2,3,5,6,7,8-hexahydro[1,2,4]triazolo[4,3-a]pyridin-5-carbonsäure (Enantiomer 1)

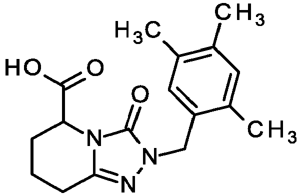

Methyl-(5RS)-3-oxo-2-(2,4,5-trimethylbenzyl)-2,3,5,6,7,8-hexahydro[1,2,4]triazolo[4,3-a]pyridin-5-carboxylat (Enantiomer 1) (198 mg, 601 µmοl) wurde in THF (2.0 ml) vorgelegt und Lithiumhydroxid (72.0 mg, 3.01 mmol) gelöst in Wasser zugegeben. Das Reaktionsgemisch wurde nach 72 Stunden Rühren bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 189 mg (99 % d. Th.) der Titelverbindung erhalten.
LC-MS (Methode 1): Rₜ = 1.05 min; MS (ESIpos): m/z = 316 [M+H]⁺

Die (5S)-Konfiguration wurde aufgrund der Kristallstrukturaufklärung für Beispiel 26, 108, 113, 157, 237, 358 und 454 zugeordnet.

### (5S)-3-Oxo-2-(2,4,5-trimethylbenzyl)-2,3,5,6,7,8-hexahydro[1,2,4]triazolo[4,3-a]pyridin-5-carbonsäure

### Intermediat 181

### (5RS)-2-(4-Methylbenzyl)-3-oxo-2,3,5,6,7,8-hexahydro[1,2,4]triazolo[4,3-a]pyridin-5-carbonsäure (Enantiomer 1)

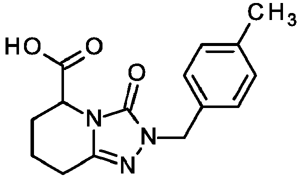

Methyl-(5RS)-2-(4-methylbenzyl)-3-oxo-2,3,5,6,7,8-hexahydro[1,2,4]triazolo[4,3-a]pyridin-5-carboxylat (Enantiomer 1) (1.05 g, 3.48 mmol) wurde in THF (29 ml) vorgelegt und Lithiumhydroxid (417 mg, 17.4 mmol) gelöst in Wasser zugegeben. Das Reaktionsgemisch wurde nach Rühren über Nacht bei Raumtemperatur mit Wasser, 1 N wässriger Salzsäure und gesättigter wässriger Natriumchlorid-Lösung versetzt. Die organische Phase wurde abgetrennt und die wässrige Phase dreimal mit Ethylacetat extrahiert. Die vereinigten organischen Phasen wurden über Natriumsulfat getrocknet, filtriert und das Filtrat eingeengt. Es wurden 733 mg (73 % d. Th.) der Titelverbindung erhalten.

### Alternative Synthese:

tert-Butyl-(5RS)-2-(4-methylbenzyl)-3-oxo-2,3,5,6,7,8-hexahydro[1,2,4]triazolo[4,3-a]pyridin-5-carboxylat (Enantiomer 1) (2.65 g, 7.72 mmol) wurde in Dichlormethan (45 ml) gelöst und bei Raumtemperatur mit Trifluoressigsäure (45 ml, 580 mmol) versetzt. Nachdem das Reaktionsgemisch über Nacht bei Raumtemperatur gerührt wurde, wurde das Lösungsmittel unter Vakuum entfernt. Der Rückstand wurde in Dichlormethan aufgenommen und mit Wasser versetzt. Die organische Phase wurde abgetrennt und die wässrige Phase zweimal mit Dichlormethan extrahiert. Die vereinigten organischen Phasen wurden über Magnesiumsulfat getrocknet, filtriert und das Filtrat eingeengt. Es wurden 2.16 g (97 % d. Th.) der Titelverbindung erhalten.
LC-MS (Methode 1): Rₜ = 0.91 min; MS (ESIpos): m/z = 288 [M+H]⁺
¹H-NMR (500 MHz, DMSO-d6) δ [ppm]: 1.356 (0.56), 2.065 (0.43), 2.072 (0.54), 2.076 (0.79), 2.082 (0.79), 2.089 (0.76), 2.100 (0.41), 2.271 (8.20), 2.526 (0.56), 2.612 (0.50), 3.321 (0.68), 4.449 (0.65), 4.458 (1.21), 4.468 (0.62), 4.770 (2.95), 7.128 (16.00).

Die (5S)-Konfiguration wurde aufgrund der Kristallstrukturaufklärung für Beispiel 26, 108, 113, 157, 237, 358 und 454 zugeordnet.

### (5S)-2-(4-Methylbenzyl)-3-oxo-2,3,5,6,7,8-hexahydro[1,2,4]triazolo[4,3-a]pyridin-5-carbonsäure

### Intermediat 182

### (5RS)-5-Methyl-2-(4-methylbenzyl)-3-oxo-2,3,5,6,7,8-hexahydro[1,2,4]triazolo[4,3-a]pyridin-5-carbonsäure (Racemat)

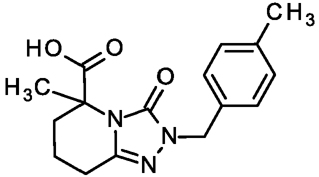

Methyl-(5RS)-5-methyl-2-(4-methylbenzyl)-3-oxo-2,3,5,6,7,8-hexahydro[1,2,4]triazolo[4,3-a]pyridin-5-carboxylat (Racemat) (32.0 mg, 101 µmοl) wurde in THF (1.0 ml) vorgelegt und Lithiumhydroxid (12.1 mg, 507 µmοl) gelöst in Wasser zugegeben. Das Reaktionsgemisch wurde nach 72 Stunden Rühren bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 30.0 mg (98 % d. Th.) der Titelverbindung erhalten.
LC-MS (Methode 1): Rₜ = 0.98 min; MS (ESIpos): m/z = 302 [M+H]⁺

### Intermediat 183

### (5RS,7RS)-7-Methyl-2-(4-methylbenzyl)-3-oxo-2,3,5,6,7,8-hexahydro[1,2,4]triazolo[4,3-a]pyridin-5-carbonsäure (Diastereomerengemisch; 4 Isomere)

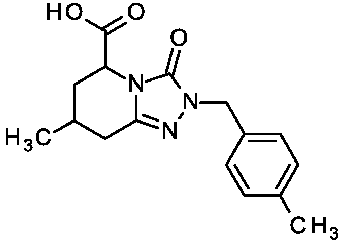

tert-Butyl-(5RS,7RS)-7-methyl-3-oxo-2,3,5,6,7,8-hexahydro[1,2,4]triazolo[4,3-a]pyridin-5-carboxylat (Diastereomerengemisch; 4 Isomere) (150 mg, 592 µmοl) wurde in Acetonitril (6.0 ml) vorgelegt. Cäsiumcarbonat (289 mg, 888 µmοl) und 1-(Brommethyl)-4-methylbenzol (115 mg, 622 µmοl) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Magnsiumsulfat getrocknet, filtriert und das Filtrat eingeengt. Der Rückstand wurde in Dichlormethan (5 ml) gelöst und bei Raumtemperatur mit Triflouressigsäure (2.5 mL) versetzt. Nachdem das Reaktionsgemisch über Nacht bei Raumtemperatur gerührt wurde, wurde das Lösungsmittel unter Vakuum entfernt. Der Rückstand wurde über präparative HPLC aufgereinigt (Methode 10). Die produkthaltigen Fraktionen wurden im Vakuum eingeengt und es wurden 120 mg (67 % d. Th.) der Titelverbindung erhalten.
LC-MS (Methode 4): Rₜ = 0.73 min; MS (ESIpos): m/z = 302 [M+H]⁺
¹H-NMR (400 MHz, DMSO-d6) δ [ppm]: -0.008 (0.53), 0.008 (0.47), 1.004 (3.87), 1.021 (4.00), 1.438 (0.57), 1.471 (0.61), 1.785 (0.92), 1.861 (0.40), 2.133 (0.60), 2.162 (0.50), 2.174 (0.69), 2.202 (0.57), 2.244 (0.44), 2.270 (10.91), 2.602 (0.51), 2.609 (0.51), 2.642 (0.45), 2.649 (0.43), 4.233 (0.46), 4.249 (0.53), 4.258 (0.53), 4.275 (0.45), 4.746 (5.02), 4.780 (0.47), 4.795 (0.45), 7.132 (16.00).

### Intermediat 184

### (5RS,7RS)-7-Methyl-3-oxo-2-{[6-(trifluormethyl)pyridin-3-yl]methyl}-2,3,5,6,7,8-hexahydro[1,2,4]triazolo[4,3-a]pyridin-5-carbonsäure (Diastereomerengemisch; 4 Isomere)

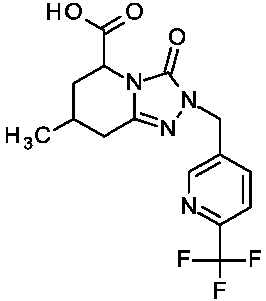

tert-Butyl-(5S,7S)-7-methyl-3-oxo-2-{[6-(trifluormethyl)pyridin-3-yl]methyl}-2,3,5,6,7,8-hexahydro[1,2,4]triazolo[4,3-a]pyridin-5-carboxylat (Diastereomerengemisch; 4 Isomere) (383 mg, 929 µmοl) wurde in Dichlormethan (5.0 ml) gelöst und bei Raumtemperatur mit Trifluoressigsäure (5.0 ml, 65 mmol) versetzt. Nachdem das Reaktionsgemisch für 2.5 h bei Raumtemperatur gerührt wurde, wurde das Lösungsmittel unter Vakuum entfernt. Der Rückstand wurde in Dichlormethan gelöst und mit Wasser gewaschen. Die wässrige Phase wurde mit Dichlormethan extrahiert, mit Natriumchlorid gesättigt und noch einmal mit Dichlormethan extrahiert. Die vereinigten organischen Phasen wurden über Magnesiumsulfat getrocknet, filtriert und das Filtrat eingeengt. Es wurden 325 mg (92 % d. Th.) der Titelverbindung erhalten.
LC-MS (Methode 3): Rₜ = 1.18 min; MS (ESIpos): m/z = 357 [M+H]⁺
¹H-NMR (400 MHz, DMSO-d6) δ [ppm]: -0.008 (4.92), 0.008 (2.52), 1.015 (15.45), 1.032 (15.37), 1.237 (0.63), 1.324 (0.45), 1.407 (3.53), 1.444 (1.27), 1.471 (2.46), 1.499 (1.90), 1.504 (2.44), 1.532 (1.40), 1.920 (1.12), 1.927 (1.22), 1.936 (1.30), 1.946 (1.16), 1.954 (1.10), 2.181 (2.72), 2.209 (2.30), 2.221 (3.18), 2.250 (3.93), 2.265 (1.63), 2.282 (1.40), 2.298 (1.26), 2.328 (0.46), 2.519 (2.23), 2.524 (2.06), 2.637 (2.03), 2.643 (2.06), 2.673 (1.93), 2.683 (1.64), 3.566 (1.59), 3.686 (0.42), 4.306 (2.98), 4.322 (3.48), 4.332 (3.20), 4.348 (2.66), 4.835 (1.62), 5.016 (16.00), 5.040 (0.46), 5.754 (1.60), 7.899 (2.66), 7.918 (9.08), 7.919 (8.98), 7.931 (5.18), 7.936 (4.87), 7.952 (1.45), 7.956 (1.45), 8.670 (5.04).

### Intermediat 185

### (5RS,6RS)-6-Methyl-2-(4-methylbenzyl)-3-oxo-2,3,5,6,7,8-hexahydro[1,2,4]triazolo[4,3-a]pyridin-5-carbonsäure (Diastereomerengemisch; 4 Isomere)

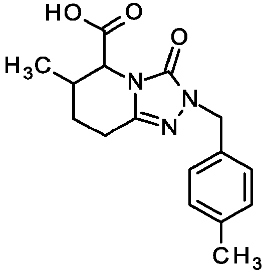

Ethyl-(5RS,6RS)-6-methyl-2-(4-methylbenzyl)-3-oxo-2,3,5,6,7,8-hexahydro[1,2,4]triazolo[4,3-a]pyridin-5-carboxylat (Diastereomerengemisch; 4 Isomere) (108 mg, 328 µmol) wurde in THF (1.5 ml) vorgelegt und Lithiumhydroxid (19.6 mg, 820 µmol) gelöst in Wasser (750 µl) zugegeben. Das Reaktionsgemisch wurde nach Rühren über Nacht bei Raumtemperatur mit gesättigter wässriger Ammoniumchlorid-Lösung und 1N wässriger Salzsäure versetzt. Die organische Phase wurde abgetrennt und die wässrige Phase dreimal mit Dichlormethan extrahiert. Die vereinigten organischen Phasen wurden über Magnesiumsulfat getrocknet, filtriert und das Filtrat eingeengt. Es wurden 95.3 mg (67 % Reinheit, 65 % d. Th.) der Titelverbindung erhalten.
LC-MS (Methode 8): Rₜ = 1.88 min; MS (ESIpos): m/z = 302 [M+H]⁺

### Intermediat 186

### (5RS,7RS)-2-(4-Methylbenzyl)-3-oxo-7-(trifluormethyl)-2,3,5,6,7,8-hexahydro[1,2,4]triazolo[4,3-a]pyridin-5-carbonsäure (Diastereomerengemisch; 4 Isomere)

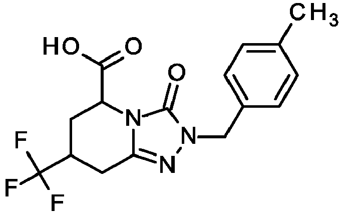

Ethyl-(5RS,7RS)-2-(4-methylbenzyl)-3-oxo-7-(trifluormethyl)-2,3,5,6,7,8-hexahydro[1,2,4]triazolo[4,3-a]pyridin-5-carboxylat (Diastereomerengemisch; 4 Isomere) (102 mg, 265 µmol) wurde in THF (1.0 ml) vorgelegt und Lithiumhydroxid (15.9 mg, 663 µmol) gelöst in Wasser (500 µl) zugegeben. Das Reaktionsgemisch wurde nach Rühren über Nacht bei Raumtemperatur mit gesättigter wässriger Ammoniumchlorid-Lösung und Dichlormethan/i-Propanol 1/5 versetzt. Die organische Phase wurde abgetrennt und die wässrige Phase zweimal mit Dichlormethan/i-Propanol 1/5 extrahiert. Die wässrige Phase wurde mit 1N wässriger Salzsäure versetzt und dreimal mit Dichlormethan/i-Propanol 1/5 extrahiert Die vereinigten organischen Phasen wurden über Magnesiumsulfat getrocknet, filtriert und das Filtrat eingeengt. Es wurden 98 mg (82 % Reinheit, 83 % d. Th der Titelverbindung erhalten.
LC-MS (Methode 1): Rₜ = 1.06 min; MS (ESIpos): m/z = 356 [M+H]⁺

### Intermediat 187

### (5RS)-2-[2-(4-Methylphenyl)ethyl]-3-oxo-2,3,5,6,7,8-hexahydro[1,2,4]triazolo[4,3-a]pyridin-5-carbonsäure (Racemat)

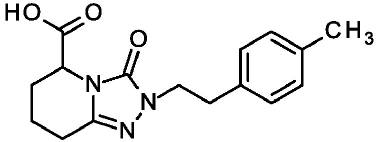

Methyl-(5RS)-2-[2-(4-methylphenyl)ethyl]-3-oxo-2,3,5,6,7,8-hexahydro[1,2,4]triazolo[4,3-a]pyridin-5-carboxylat (Racemat) (90.0 mg, 285 µmol) wurde in THF (3.0 ml) vorgelegt und Lithiumhydroxid (34.2 mg, 1.43 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 82.0 mg (95 % d. Th.) der Titelverbindung erhalten.
LC-MS (Methode 3): Rₜ = 1.27 min; MS (ESIpos): m/z = 302 [M+H]⁺
¹H-NMR (400 MHz, DMSO-d6) δ [ppm]: -0.150 (0.41), -0.008 (3.22), 0.008 (2.98), 0.146 (0.44), 1.908 (1.11), 1.988 (0.63), 2.071 (0.92), 2.256 (9.10), 2.327 (0.53), 2.523 (1.85), 2.580 (0.53), 2.643 (0.69), 2.674 (0.63), 2.862 (1.04), 2.881 (2.28), 2.900 (1.16), 3.765 (0.63), 3.783 (1.52), 3.803 (1.33), 3.823 (0.53), 4.389 (0.63), 4.400 (1.22), 4.413 (0.61), 7.084 (16.00), 13.178 (0.58).

### Intermediat 188

### (5RS)-3-Oxo-2-{[4-(trifluormethyl)cyclohexyl]methyl}-2,3,5,6,7,8-hexahydro[1,2,4]triazolo[4,3-a]pyridin-5-carbonsäure (Diastereomerengemisch; 4 Isomere)

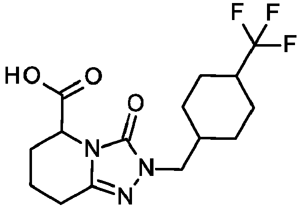

Zur Lösung von tert-Butyl-(5RS)-3-oxo-2-{[4-(trifluormethyl)cyclohexyl]methyl}-2,3,5,6,7,8-hexahydro[1,2,4]triazolo[4,3-a]pyridin-5-carboxylat (Diastereomerengemisch; 4 Isomere) (465 mg, 1.15 mmol) in 20 ml Dichlormethan wurden 2.0 ml (26 mmol) Trifluoressigsäure gegeben und der Ansatz über Nacht bei Raumtemperatur gerührt. Zur Aufarbeitung wurde unter Eisbadkühlung und kräftigem Rühren mit 3N Natronlauge auf pH 3 eingestellt, mit Dichlormethan/Wasser verdünnt und extrahiert. Die wässrige Phase wurde noch zweimal mit Dichlormethan extrahiert. Die vereinigten organischen Phasen wurden mit gesättigter Natriumchloridlösung gewaschen, über Natriumsulfat getrocknet, eingeengt und im Vakuum getrocknet. Es wurden 327 mg (82 % d. Th.) der Titelverbindung (als racemisches cis/trans-Gemisch) erhalten, die so weiter umgesetzt wurden.
LC-MS (Methode 4): Rₜ = 0.78 min; MS (ESIpos): m/z = 348 [M+H]⁺
¹H-NMR (400 MHz, DMSO-d6) δ [ppm]: -0.149 (1.44), -0.008 (12.24), 0.008 (10.19), 0.146 (1.37), 1.007 (1.88), 1.038 (2.38), 1.061 (1.44), 1.149 (2.53), 1.189 (2.71), 1.220 (2.78), 1.253 (1.77), 1.284 (0.94), 1.395 (6.68), 1.405 (3.32), 1.412 (2.20), 1.479 (7.12), 1.498 (9.86), 1.508 (5.85), 1.521 (7.22), 1.531 (5.74), 1.546 (5.89), 1.561 (4.66), 1.571 (3.61), 1.586 (4.88), 1.595 (5.96), 1.605 (8.52), 1.617 (7.01), 1.624 (7.77), 1.643 (10.76), 1.676 (4.01), 1.804 (3.29), 1.816 (3.72), 1.828 (4.80), 1.859 (3.65), 1.900 (1.95), 2.079 (7.58), 2.088 (6.79), 2.100 (3.68), 2.332 (2.09), 2.366 (1.91), 2.569 (2.93), 2.583 (2.20), 2.619 (2.31), 2.630 (4.01), 2.642 (2.53), 2.670 (2.74), 2.710 (1.48), 3.289 (7.19), 3.427 (2.74), 3.443 (2.85), 3.457 (3.97), 3.462 (4.19), 3.474 (4.19), 3.501 (1.12), 3.519 (0.98), 3.563 (16.00), 3.581 (15.67), 3.609 (3.14), 3.622 (3.61), 3.628 (3.40), 3.642 (3.11), 3.678 (0.61), 4.412 (2.13), 4.424 (5.53), 4.435 (5.16), 4.447 (1.95), 13.170 (3.58).

### Intermediat 189

### (5RS)-2-[(4,4-Difluorcyclohexyl)methyl]-3-oxo-2,3,5,6,7,8-hexahydro[1,2,4]triazolo[4,3-a]pyridin-5-carbonsäure (Racemat)

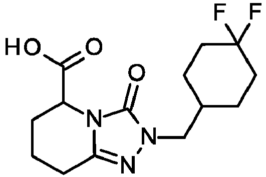

Zur Lösung von tert-Butyl-(5S)-2-[(4,4-difluorcyclohexyl)methyl]-3-oxo-2,3,5,6,7,8-hexahydro[1,2,4]triazolo[4,3-a]pyridin-5-carboxylat (Racemat) (41.2 mg, 111 µmol) in 2.0 ml Dichlormethan wurden 90 µl (1.2 mmol) Trifluoressigsäure gegeben und der Ansatz 5h bei Raumtemperatur gerührt. Anschließend wurden noch einmal 120µl (1.6 mmol) Trifluoressigsäure zugefügt und über Nacht weiter gerührt (Umsatzkontrolle durch HPLC). Zur Aufarbeitung wurde unter Eisbadkühlung und kräftigem Rühren mit 3N NaOH auf pH 3 eingestellt, mit Dichlormethan/Wasser verdünnt und extrahiert. Die wässrige Phase wurde noch zweimal mit Dichlormethan extrahiert. Die vereinigten organischen Phasen wurden mit gesättigter Natriumchloridlösung gewaschen, über Natriumsulfat getrocknet, eingeengt und im Vakuum getrocknet. Es wurden so 30.5 mg (87 % d. Th.) der Titelverbindung erhalten.
LC-MS (Methode 3): Rₜ = 1.08 min; MS (ESIpos): m/z = 316 [M+H]⁺
¹H-NMR (400 MHz, DMSO-d6) δ [ppm]: -0.149 (1.97), -0.008 (16.00), 0.008 (14.35), 0.146 (1.78), 1.227 (3.20), 1.396 (2.42), 1.515 (1.65), 1.694 (4.48), 1.806 (4.07), 1.975 (2.74), 2.085 (5.12), 2.327 (1.51), 2.366 (1.46), 2.558 (3.84), 2.571 (3.06), 2.634 (3.25), 2.675 (2.51), 2.709 (1.46), 3.286 (9.65), 3.523 (12.53), 3.541 (12.48), 4.417 (3.20), 4.428 (6.13), 4.441 (3.25), 5.754 (1.60), 13.179 (2.88).

### Intermediat 190

### [2-(Trifluormethyl)-1,8-naphthyridin-3-yl]methanol

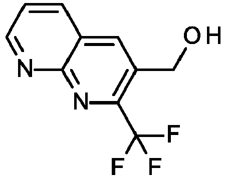

Ethyl-2-(trifluormethyl)-1,8-naphthyridin-3-carboxylat (1.00 g, 3.70 mmol) wurde in THF (25 ml) gelöst und es wurde bei -20°C Lithiumaluminiumhydrid (1.9 ml, 2.4 M, 4.4 mmol) zugegeben. Das Reaktionsgemisch wurde nach Rühren für 2 Stunden bei -20°C mit gesättigter wässriger Ammoniumchlorid-Lösung versetzt und das THF im Vakuum entfernt. Der Rückstand wurde mit Ethylacetat und Wasser versetzt. Die wässrige Phase wurde dreimal mit Ethylacetat extrahiert. Die vereinigten organischen Phasen wurden über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 230 mg (26 % d. Th.) der Titelverbindung erhalten.
LC-MS (Methode 3): Rₜ = 0.90 min; MS (ESIpos): m/z = 229 [M+H]⁺
¹H-NMR (400 MHz, DMSO-d6) δ [ppm]: -0.149 (0.86), -0.008 (7.77), 0.008 (6.74), 0.146 (0.86), 2.328 (1.09), 2.367 (0.80), 2.524 (3.56), 2.671 (1.18), 2.711 (0.95), 4.852 (0.43), 4.876 (15.05), 4.890 (15.34), 4.923 (0.52), 5.824 (7.51), 5.838 (15.34), 5.852 (7.14), 7.791 (8.06), 7.801 (8.32), 7.811 (8.49), 7.822 (8.66), 8.679 (8.49), 8.684 (8.66), 8.700 (8.63), 8.704 (8.17), 8.868 (16.00), 9.204 (7.51), 9.209 (7.68), 9.214 (7.80), 9.219 (7.23).

### Intermediat 191

### 3-(Chlormethyl)-2-(trifluormethyl)-1,8-naphthyridin

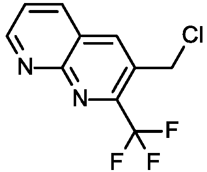

[2-(Trifluormethyl)-1,8-naphthyridin-3-yl]methanol (46.0 mg, 202 µmol) wurde in Dichlormethan (910 µl) gelöst und bei 0°C mit Thionyldichlorid (29 µl, 400 µmol) versetzt. Das Reaktionsgemisch wurde für 2 Stunden bei Raumtemperatur gerührt und anschließend das Lösungsmittel unter Vakuum entfernt. Es wurden 46.0 mg (93 % d. Th.) der Titelverbindung erhalten.

¹H-NMR (400 MHz, DMSO-d6) δ [ppm]: -0.008 (1.81), 0.008 (1.26), 2.330 (0.40), 2.521 (1.58), 2.526 (1.33), 2.672 (0.57), 2.712 (0.43), 5.126 (16.00), 5.177 (0.78), 5.756 (1.49), 7.026 (0.49), 7.041 (0.66), 7.059 (0.57), 7.853 (3.44), 7.863 (3.55), 7.873 (3.64), 7.883 (3.67), 8.038 (0.42), 8.676 (3.22), 8.681 (3.30), 8.697 (3.06), 8.701 (3.04), 8.995 (9.01), 9.278 (3.59), 9.283 (3.64), 9.288 (3.68), 9.293 (3.40).

### Intermediat 192

### 1-[1-(Brommethyl)cyclopropyl]-4-(trifluormethyl)benzol

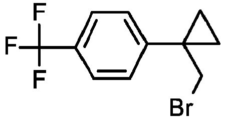

{1-[4-(Trifluormethyl)phenyl]cyclopropyl}methanol (350 mg, 1.62 mmol) wurde in Dichlormethan (10 ml) gelöst und N,N-Diisopropylethylamin (560 µl, 3.2 mmol) und Dibrom(triphenyl)phosphoran (854 mg, 96 % Reinheit, 1.94 mmol) wurden bei 0°C zugegeben. Das Reaktionsgemisch wurde über Nacht bei Raumtemperatur gerührt und erneut mit Dibrom(triphenyl)phosphoran (854 mg, 96 % Reinheit, 1.94 mmol) versetzt. Nach Rühren über Nacht bei Raumtemperatur wurde das Lösungsmittel im Vakuum entfernt und der Rückstand wurde über Säulenchromatographie aufgereinigt (Kieselgel, Dichlormethan/Ethylacetat). Die produkthaltigen Fraktionen wurden im Vakuum eingeengt und es wurden 110 mg (23 % d. Th.) der Titelverbindung erhalten.

¹H-NMR (400 MHz, DMSO-d6) δ [ppm]: 1.129 (1.56), 1.136 (1.65), 1.144 (4.59), 1.148 (5.92), 1.157 (3.27), 1.169 (1.19), 1.191 (1.17), 1.202 (3.09), 1.212 (5.74), 1.223 (1.45), 1.231 (1.56), 1.360 (1.16), 1.397 (5.40), 3.915 (16.00), 4.783 (0.41), 7.540 (4.22), 7.561 (5.81), 7.664 (5.75), 7.685 (4.22).

### Intermediat 193

### Methyl-1-(6-chlorpyridin-2-yl)cyclopropancarboxylat

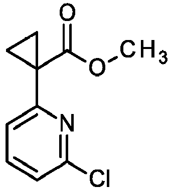

1-(6-Chlorpyridin-2-yl)cyclopropancarbonsäure (1.00 g, 5.06 mmol) wurde in Methanol (10 ml) gelöst und Schwefelsäure (54 µl, 1.0 mmol) wurde zugegeben. Das Reaktionsgemisch wurde über Nacht bei 60°C gerührt. Das Reaktionsgemisch wurde im Vakuum eingeengt, der Rückstand in Ethylacetat gelöst und mit gesättigter Natriumhydrogencarbonat-Lösung gewaschen. Die organische Phase wurde über Natriumsulfat getrocknet, filtriert und das Filtrat eingeengt. Es wurden 947 mg (87 % d. Th.) der Titelverbindung.
LC-MS (Methode 4): Rₜ = 0.83 min; MS (ESIpos): m/z = 212 [M+H]⁺
¹H-NMR (400 MHz, DMSO-d6) δ [ppm]: 1.394 (4.13), 1.405 (10.89), 1.414 (14.44), 1.422 (6.17), 1.461 (0.81), 1.484 (0.89), 1.521 (6.34), 1.530 (14.64), 1.539 (10.67), 1.550 (4.00), 2.732 (1.03), 2.892 (1.17), 3.314 (16.00), 3.798 (0.65), 7.384 (6.75), 7.404 (7.47), 7.558 (7.35), 7.577 (8.61), 7.803 (5.32), 7.823 (9.19), 7.842 (4.27).

### Intermediat 194

### [1-(6-Chlorpyridin-2-yl)cyclopropyl]methanol

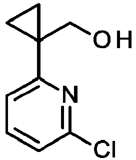

Methyl-1-(6-chlorpyridin-2-yl)cyclopropancarboxylat (947 mg, 4.47 mmol) wurde in THF (15 ml) gelöst und bei -78°C wurde Lithiumaluminiumhydrid (2.2 ml, 2.4 M, 5.4 mmol) zugegeben. Das Reaktionsgemisch wurde für 2 Stunden gerührt und anschließend mit gesättigter wässriger Ammoniumchlorid-Lösung versetzt. Das Lösungsmittel wurde im Vakuum entfernt und der Rückstand wurde mit Ethylacetat und Wasser versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 603 mg (92 % Reinheit, 68 % d. Th.) der Titelverbindung erhalten.
LC-MS (Methode 4): Rₜ = 0.68 min; MS (ESIpos): m/z = 184 [M+H]⁺
¹H-NMR (400 MHz, DMSO-d6) δ [ppm]: 0.918 (3.81), 0.928 (11.67), 0.933 (14.82), 0.980 (0.54), 1.021 (0.57), 1.058 (5.57), 1.066 (13.62), 1.072 (13.58), 1.082 (4.20), 1.358 (0.67), 1.663 (3.18), 3.718 (15.53), 3.732 (16.00), 4.787 (4.36), 4.800 (8.73), 4.814 (4.16), 7.229 (5.90), 7.248 (6.45), 7.495 (6.83), 7.515 (8.03), 7.695 (0.49), 7.725 (3.88), 7.744 (6.81), 7.764 (3.12), 7.853 (0.40), 9.297 (0.99).

### Intermediat 195

### 2-[1-(Brommethyl)cyclopropyl]-6-chlorpyridin

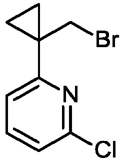

[1-(6-Chlorpyridin-2-yl)cyclopropyl]methanol (300 mg, 1.63 mmol) wurde in Dichlormethan (10 ml) gelöst und N,N-Diisopropylethylamin (570 µl, 3.3 mmol) und Dibrom(triphenyl)phosphoran (862 mg, 96 % Reinheit, 1.96 mmol) wurden bei 0°C zugegeben. Das Reaktionsgemisch wurde über Nacht bei Raumtemperatur gerührt und anschließend im Vakuum eingeengt. Der Rückstand wurde über Säulenchromatographie aufgereinigt (Kieselgel, Dichlormethan/Ethylacetat). Die produkthaltigen Fraktionen wurden im Vakuum eingeengt und es wurden 141 mg (34 % d. Th.) der Titelverbindung erhalten.
GC-MS (Methode 2): Rₜ = 5.32 min; MS (ESIpos): m/z = 213 [M+H]⁺

### Intermediat 196

### Ethyl-6-chlor-3-(trifluormethyl)pyridin-2-carboxylat

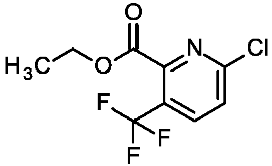

6-Chlor-3-(trifluormethyl)pyridin-2-carbonsäure (2.50 g, 11.1 mmol) wurde in Ethanol (29 ml) gelöst, Schwefelsäure (710 µl, 13 mmol) wurde zugegeben und das Reaktionsgemsich über Nacht unter Rückfluss gerührt. Das Reaktionsgemisch wurde im Vakuum eingeengt, der Rückstand in Ethylacetat gelöst und mit gesättigter Natriumhydrogencarbonat-Lösung gewaschen. Die organische Phase wurde über Natriumsulfat getrocknet, filtriert und das Filtrat eingeengt. Es wurden 2.24 g (88 % Reinheit, 70 % d. Th.) der Titelverbindung erhalten und direkt weiter umgesetzt.

### Intermediat 197

### 3-Azabicyclo[2.1.1]hexan-Trifluoressigsäure

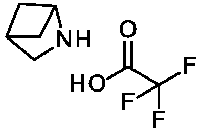

tert-Butyl-2-azabicyclo[2.1.1]hexan-2-carboxylat (70.0 mg, 382 µmol) wurde in Dichlormethan (10 ml) gelöst und bei Raumtemperatur mit Trifluoressigsäure (590 µl, 7.6 mmol) versetzt. Nachdem das Reaktionsgemisch über Nacht bei Raumtemperatur gerührt wurde, wurde das Lösungsmittel unter Vakuum entfernt. Es wurden der Titelverbindung erhalten. Die Verbindung wurde direkt weiter umgesetzt.

### Intermediat 198

### Ethyl-6-{1-[(benzyloxy)carbonyl]hydrazino}-3-(trifluormethyl)pyridin-2-carboxylat

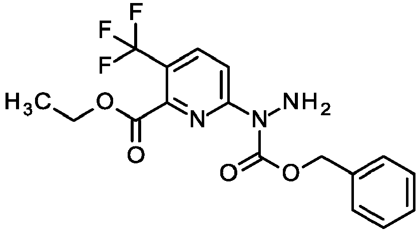

Unter Argon wurden Ethyl-6-chlor-3-(trifluormethyl)pyridin-2-carboxylat (2.24 g, 88 % Reinheit, 7.77 mmol) und Benzyl-hydrazincarboxylat (1.42 g, 8.55 mmol) in Toluol (20 ml) gelöst, und Tris(dibenzylidenaceton)dipalladium-Chloroform-Komplex (402 mg, 389 µmol), 1,1'-Bis(diphenylphosphino)ferrocen (441 mg, 777 µmol) und Cäsiumcarbonat (3.04 g, 9.33 mmol) zugegeben. Das Reaktionsgemisch wurde 3 h bei 80°C gerührt und dann mit Wasser und Ethylacetat versetzt. Die organische Phase wurde abgetrennt und die wässrige Phase mit Ethylacetat extrahiert. Die vereinigten organischen Phasen wurden über Natriumsulfat getrocknet, filtriert und das Filtrat eingeengt. Der Rückstand wurde über Säulenchromatographie aufgereinigt (Kieselgel; Laufmittel: isokratisch, Ethylacetat/Dichlormethan). Die produkthaltigen Fraktionen wurden im Vakuum eingeengt und es wurden 1.80 g (60 % d. Th.) der Titelverbindung erhalten.
LC-MS (Methode 4): Rₜ = 1.05 min; MS (ESIpos): m/z = 384 [M+H]⁺

### Intermediat 199

### Ethyl-6-hydrazino-3-(trifluormethyl)pyridin-2-carboxylat

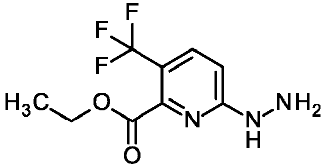

Ethyl-6-{1-[(benzyloxy)carbonyl]hydrazino}-3-(trifluormethyl)pyridin-2-carboxylat (1.80 g, 4.69 mmol) wurde in Ethanol (10 ml) gelöst und unter Verwendung einer Hydrierapparatur (H-Cube^{®}, Pd/C 10% Palladium, 1 Bar, 50°C, Fluss: 1ml/min) umgesetzt. Es wurden 1.03 g (88 % d. Th.) der Titelverbindung erhalten.
LC-MS (Methode 1): Rₜ = 0.94 min; MS (ESIpos): m/z = 250 [M+H]⁺

### Intermediat 200

### Ethyl-3-oxo-6-(trifluormethyl)-2,3-dihydro[1,2,4]triazolo[4,3-a]pyridin-5-carboxylat

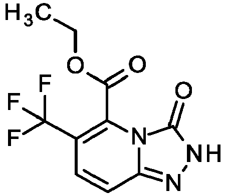

Ethyl-6-hydrazino-3-(trifluormethyl)pyridin-2-carboxylat (1.11 g, 4.45 mmol) wurde in THF (15 ml) aufgenommen und mit Di-1H-imidazol-1-ylmethanon (866 mg, 5.34 mmol) versetzt. Das Reaktionsgemisch wurde über Nacht bei Raumtemperatur gerührt. Das Reaktionsgemisch wurde mit Wasser und Dichlormethan versetzt und mit gesättigter wässriger Natriumchlorid-Lösung versetzt. Die organische Phase wurden mit gesättigter wässriger Natriumchlorid-Lösung gewaschen. Die wässrige Phase wurde mit Dichlormethan extrahiert. Die vereinigten organischen Phasen wurden über Natriumsulfat getrocknet, filtriert und das Filtrat eingeengt. Es wurden 1.22 g (100 % d. Th.) der Titelverbindung erhalten.
LC-MS (Methode 4): Rₜ = 0.74 min; MS (ESIpos): m/z = 276 [M+H]⁺

### Intermediat 201

### Ethyl-(5RS,6RS)-3-oxo-6-(trifluormethyl)-2,3,5,6,7,8-hexahydro[1,2,4]triazolo[4,3-a]pyridin-5-carboxylat (Diastereomerengemisch, 4 Isomere)

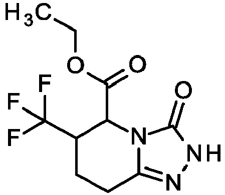

Ethyl-3-oxo-6-(trifluormethyl)-2,3-dihydro[1,2,4]triazolo[4,3-a]pyridin-5-carboxylat (1.22 g, 4.45 mmol) wurde in Ethanol (10 ml) gelöst und unter Verwendung einer Hydrierapparatur (H-Cube^{®}, Pd/C 10% Palladium, 1 bar, 80°C, Fluss: 1ml/min) umgesetzt. Es wurden 1.24 g (100 % d. Th.) der Titelverbindung erhalten.
LC-MS (Methode 1): Rₜ = 0.88 min; MS (ESIpos): m/z = 280 [M+H]⁺

### Intermediat 202

### Methyl-6-{1-[(benzyloxy)carbonyl]hydrazino}-5-(trifluormethyl)pyridin-2-carboxylat

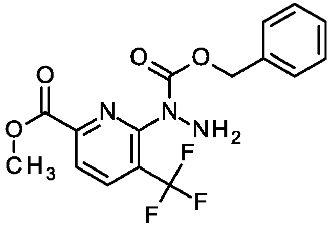

Methyl-6-chlor-5-(trifluormethyl)pyridin-2-carboxylat (3.00 g, 12.5 mmol), Benzyl-hydrazincarboxylat (2.29 g, 13.8 mmol), und Tris(dibenzylidenaceton)dipalladium (573 mg, 626 µmol) wurden unter Argon in Toluol (60 ml) suspendiert. 1,1'-Bis(diphenylphosphino)ferrocen (694 mg, 1.25 mmol) und Cäsiumcarbonat (4.90 g, 15.0 mmol) wurden zugegeben und die Reaktionsmischung wurde 3 h bei 80°C gerührt. Das Reaktionsgemisch wurde mit Wasser und Ethylacetat versetzt und die organische Phase wurde abgetrennt, mit Wasser und gesättigter wässriger Natriumchlorid-Lösung gewaschen, über Natriumsulfat getrocknet, filtriert und eingeengt. Der Rückstand wurde über Säulenchromatographie aufgereinigt (Kieselgel; Laufmittel: Cyclohexan/Ethylacetat 9/1, 0/1). Die produkthaltigen Fraktionen wurden im Vakuum eingeengt und es wurden 1.87 g (86 % Reinheit, 35 % d. Th.) der Titelverbindung erhalten.
LC-MS (Methode 1): Rₜ = 1.23 min; MS (ESIneg): m/z = 368 [M-H]⁻
¹H-NMR (500 MHz, DMSO-d6) δ [ppm]: -0.007 (1.19), 0.006 (0.87), 1.160 (2.79), 1.174 (5.66), 1.189 (2.80), 1.398 (1.98), 1.988 (10.25), 2.518 (0.42), 3.038 (0.55), 3.051 (0.56), 3.852 (16.00), 4.008 (0.77), 4.022 (2.29), 4.037 (2.24), 4.051 (0.73), 4.484 (1.47), 4.496 (1.51), 4.998 (0.87), 5.036 (1.06), 5.085 (0.93), 5.125 (7.85), 5.134 (1.18), 5.146 (1.34), 5.157 (0.63), 7.107 (0.52), 7.195 (0.84), 7.221 (0.64), 7.232 (0.48), 7.238 (0.48), 7.271 (0.56), 7.287 (0.79), 7.309 (5.08), 7.316 (2.18), 7.319 (2.37), 7.326 (1.95), 7.340 (1.80), 7.364 (2.08), 7.380 (3.95), 7.394 (6.93), 7.409 (1.97), 7.416 (1.42), 7.422 (1.02), 7.482 (1.90), 7.498 (2.13), 7.532 (0.42), 8.085 (2.06), 8.101 (1.89), 8.765 (2.66), 8.849 (0.45), 9.009 (0.47), 9.367 (2.77).

### Intermediat 203

### Methyl-6-hydrazino-5-(trifluormethyl)pyridin-2-carboxylat

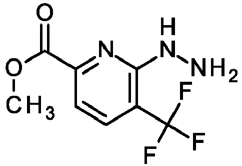

Methyl-6-{1-[(benzyloxy)carbonyl]hydrazino}-5-(trifluormethyl)pyridin-2-carboxylat (1.87 g, 86% Reinheit, 4.35 mmol) und Palladium auf Kohle (100 mg, 10% Palladium) wurden under Argon in Methanol (100 ml) suspendiert und über Nacht bei Raumtemperatur in einer Wasserstoffatmosphäre (1 bar) gerührt. Das Reaktionsgemisch wurde über Celite filtriert und mit Methanol nachgewaschen. Das Filtrat wurde im Vakuum eingeengt und es wurden 1.09 g (75 % Reinheit, 80 % d. Th.) der Titelverbindung erhalten.
LC-MS (Methode 3): Rₜ = 0.86 min; MS (ESIpos): m/z = 236 [M+H]⁺
¹H-NMR (400 MHz, DMSO-d6) δ [ppm]: 3.849 (0.45), 3.875 (16.00), 4.491 (0.97), 4.590 (0.43), 7.244 (0.61), 7.260 (0.47), 7.277 (0.47), 7.291 (1.32), 7.308 (3.01), 7.326 (2.14), 7.956 (2.10), 7.975 (2.01), 8.058 (1.29).

### Intermediat 204

### Methyl-3-oxo-8-(trifluormethyl)-2,3-dihydro[1,2,4]triazolo[4,3-a]pyridin-5-carboxylat

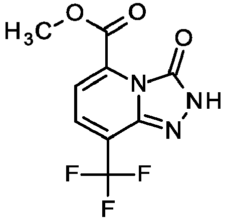

Methyl-6-hydrazino-5-(trifluormethyl)pyridin-2-carboxylat (1.09 g, 75 % Reinheit, 3.48 mmol) wurde in THF (30 ml) gelöst und unter Rühren bei Raumtemperatur wurde 1,1-Carbonyldiimidazol (1.01 g, 6.26 mmol) zugegeben. Das Reaktionsgemisch wurde über Nacht bei Raumtemperatur gerührt. Das Reaktionsgemisch wurde mit Wasser und Ethylacetat versetzt und die organische Phase wurde abgetrennt, mit Wasser und gesättigter wässriger Natriumchlorid-Lösung gewaschen, über Natriumsulfat getrocknet, filtriert und eingeengt. Es wurden 1.38 g (>100 % d. Th.) der Titelverbindung erhalten.
LC-MS (Methode 3): Rₜ = 1.07 min; MS (ESIpos): m/z = 262 [M+H]⁺

### Intermediat 205

### tert-Butyl-6-chlor-5-methylpyridin-2-carboxylat

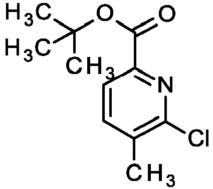

6-Chlor-5-methylpyridin-2-carbonsäure (2.10 g, 12.2 mmol) wurde in Pyridin (8.4 ml) und tert-Butanol (42 ml, 440 mmol) gelöst und mit 4-Methylbenzolsulfonylchlorid (4.67 g, 24.5 mmol) versetzt. Das Reaktionsgemisch wurde für 3h bei Raumtemperatur gerührt und mit gesättigter wässriger Natriumhydrogencarbonat-Lösung und Dichlormethan verdünnt. Die organische Phase wurde abgetrennt und die wässrige Phase mit Dichlormethan extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 2.50 g (>100%) der Titelverbindung erhalten.

¹H-NMR (400 MHz, DMSO-d6) δ [ppm]: 1.552 (16.00), 7.901 (0.52), 7.920 (1.04), 7.959 (0.77), 7.978 (0.43).

### Intermediat 206

### tert-Butyl-6-{1-[(benzyloxy)carbonyl]hydrazino}-5-methylpyridin-2-carboxylat

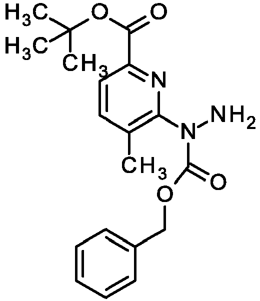

Unter Argon wurde tert-Butyl-6-chlor-5-methylpyridin-2-carboxylat (2.50 g, 11.0 mmol) und Benzyl-hydrazincarboxylat (3.28 g, 19.8 mmol) in Toluol (50 ml) gelöst, und Tris(dibenzylidenaceton)dipalladium-Chloroform-Komplex (568 mg, 549 µmol), Cäsiumcarbonat (4.29 g, 13.2 mmol) und 1,1'-Bis(diphenylphosphino)ferrocen (609 mg, 1.10 mmol) zugegeben. Das Reaktionsgemisch wurde 4 h bei 80°C gerührt und mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Magnesiumsulfat getrocknet, filtriert und das Filtrat eingeengt. Der Rückstand wurde über Säulenchromatographie aufgereinigt (SiO₂; Laufmittel: isokratisch, Ethylacetat/Cyclohexan 30/70). Die produkthaltigen Fraktionen wurden im Vakuum eingeengt und es wurden 1.81 g (46 % d. Th.) der Titelverbindung erhalten.
LC-MS (Methode 3): Rₜ = 1.91 min; MS (ESIpos): m/z = 358 [M+H]⁺
¹H-NMR (400 MHz, DMSO-d6) δ [ppm]: 1.514 (16.00), 2.157 (1.85), 5.112 (1.13), 7.292 (0.85), 7.310 (1.16), 7.377 (0.57), 7.396 (0.48), 7.408 (0.70), 7.446 (0.50), 8.196 (0.45), 9.097 (0.44).

### Intermediat 207

### tert-Butyl-6-hydrazino-5-methylpyridin-2-carboxylat

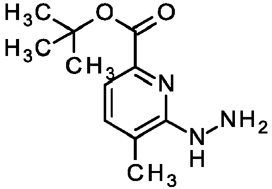

Unter Argon wurde Palladium auf Kohle (195 mg, 10% Palladium) vorgelegt und tert-Butyl-6-{1-[(benzyloxy)carbonyl]hydrazino}-5-methylpyridin-2-carboxylat (1.30 g, 3.64 mmol) in Methanol (60 ml) zugegeben. Das Reaktionsgemisch wurde für 5 h bei Raumtemperatur in einer Wasserstoffatmosphäre (1 bar) gerührt. Die Suspension wurde mit Methanol verdünnt und über Kieselgur filtriert und mit Methanol nachgewaschen. Das Lösungsmittel wurde unter Vakuum entfernt und es wurden 837 mg (99 % d. Th.) der Titelverbindung erhalten.
LC-MS (Methode 4): Rₜ = 0.50 min; MS (ESIpos): m/z = 224 [M+H]⁺
¹H-NMR (400 MHz, DMSO-d6) δ [ppm]: 1.527 (16.00), 1.548 (1.20), 2.080 (4.20), 4.258 (1.00), 7.174 (0.76), 7.193 (0.93), 7.343 (0.70), 7.361 (0.59), 7.420 (0.64).

### Intermediat 208

### tert-Butyl-8-methyl-3-oxo-2,3-dihydro[1,2,4]triazolo[4,3-a]pyridin-5-carboxylat

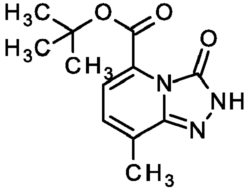

tert-Butyl-6-hydrazino-5-methylpyridin-2-carboxylat (1.18 g, 5.27 mmol) wurde in THF (100 ml) gelöst und unter Rühren bei Raumtemperatur wurde 1,1-Carbonyldiimidazol (1.03 g, 6.32 mmol) zugegeben. Das Reaktionsgemisch wurde für 1.5 h unter Rückfluss erwärmt. Das Reaktionsgemisch wurde mit Wasser und Ethylacetat versetzt und mit gesättigter wässriger Natriumhydrogencarbonat-Lösung basisch gestellt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Magnesiumsulfat getrocknet und filtriert., Das Filtrat wurde eingeengt und der Rückstand wurde über Säulenchromatographie aufgereinigt (SiO₂; Laufmittel: Cyclohexan/Ethylacetat 70/30 zu 50/50). Die produkthaltigen Fraktionen wurden im Vakuum eingeengt und es wurden 800 mg (61 % d. Th.) der Titelverbindung erhalten.
LC-MS (Methode 4): Rₜ = 0.71 min; MS (ESIneg): m/z = 248 [M-H]⁻
¹H-NMR (400 MHz, DMSO-d6) δ [ppm]: 1.529 (16.00), 2.245 (3.28), 6.683 (0.78), 6.700 (0.88), 6.940 (0.50), 6.943 (0.51), 6.957 (0.46), 6.960 (0.46), 12.505 (0.46).

### Intermediat 209

### tert-Butyl-(5RS,8RS)-8-methyl-3-oxo-2,3,5,6,7,8-hexahydro[1,2,4]triazolo[4,3-a]pyridin-5-carboxylat (Diastereomerengemisch; 4 Isomere)

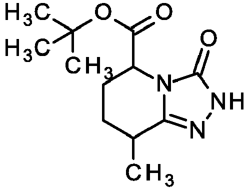

tert-Butyl-8-methyl-3-oxo-2,3-dihydro[1,2,4]triazolo[4,3-a]pyridin-5-carboxylat (200 mg, 802 µmol) und Palladium auf Kohle (40 mg, 10% Palladium) wurden in Methanol (12 ml) suspendiert und für 24 h bei Raumtemperatur in einer Wasserstoffatmosphäre (3 bar) gerührt. Das Reaktionsgemisch wurde über Kieselgur filtriert und mit Dichlormethan/Methanol 9/1 gewaschen. Das Filtrat wurde eingeengt und der Rückstand wurde mit Ethylacetat für 30 min gerührt, dann filtriert und getrocknet. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 150 mg (74 % d. Th.) der Titelverbindung als Gemisch von Diastereomeren erhalten.
LC-MS (Methode 4): Rₜ = 0.67 min; MS (ESIpos): m/z = 254 [M]⁺
¹H-NMR (400 MHz, DMSO-d6) δ [ppm]: 1.184 (2.53), 1.201 (2.50), 1.396 (16.00), 1.409 (0.40), 2.096 (0.48), 2.103 (0.44), 2.108 (0.52), 4.363 (0.46), 11.396 (0.58).

### Intermediat 210

### Methyl-(5S)-2-{[3-fluor-2-(trifluormethyl)pyridin-4-yl]methyl}-3-oxo-2,5,6,7-tetrahydro-3H-pyrrolo[2,1-c][1,2,4]triazol-5-carboxylat

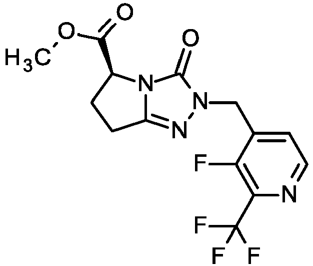

Methyl-(5S)-3-oxo-2,5,6,7-tetrahydro-3H-pyrrolo[2,1-c][1,2,4]triazol-5-carboxylat (150 mg, 819 µmol) wurde in Acetonitril (8.2 ml) vorgelegt. Cäsiumcarbonat (400 mg, 1.23 mmol) und 4-(Chlormethyl)-3-fluor-2-(trifluormethyl)pyridinhydrochlorid (231 mg, 860 µmol) wurden anschließend zugegeben. Zum Reaktionsgemisch wurde nach Rühren über Nacht bei Raumtemperatur Wasser und Ethylacetat zugegeben. Die organische Phase wurde abgetrennt, mit gesättigter wässriger Natriumchlorid-Lösung gewaschen, über Natriumsulfat getrocknet, filtriert und das Filtrat eingeengt. Es wurden 318 mg (75 % Reinheit, 81 % d. Th.) der Titelverbindung erhalten.
LC-MS (Methode 3): Rₜ = 1.39 min; MS (ESIpos): m/z = 361 [M+H]⁺

### Intermediat 211

### tert-Butyl-(5S)-2-(cyclopropylmethyl)-3-oxo-2,3,5,6,7,8-hexahydro[1,2,4]triazolo[4,3-a]pyridin-5-carboxylat

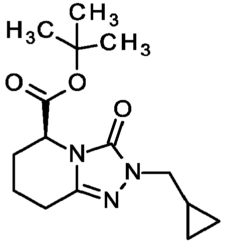

tert-Butyl-(5S)-3-oxo-2,3,5,6,7,8-hexahydro[1,2,4]triazolo[4,3-a]pyridin-5-carboxylat (150 mg, 627 µmol) wurde in Acetonitril (9.0 ml) vorgelegt. Cäsiumcarbonat (633 mg, 1.94 mmol) und (Brommethyl)cyclopropan (130 µl, 1.4 mmol) wurden anschließend zugegeben. Das Reaktionsgemisch wurde nach Rühren über das Wochenende bei Raumtemperatur im Vakuum eingeengt. Zum Rückstand wurden Wasser und Ethylacetat zugegeben. Die organische Phase wurde abgetrennt und die wässrige Phase wurde dreimal mit Ethylacetat extrahiert. Die vereinigten organischen Phasen wurden über Natriumsulfat getrocknet, filtriert und das Filtrat eingeengt. Es wurden 175 mg (95 % d. Th.) der Titelverbindung erhalten.
LC-MS (Methode 4): Rₜ = 0.81 min; MS (ESIpos): m/z = 294 [M+H]⁺
¹H-NMR (400 MHz, DMSO-d6) δ [ppm]: 0.261 (0.23), 0.273 (0.89), 0.286 (0.95), 0.298 (0.30), 0.323 (0.07), 0.427 (0.26), 0.437 (0.75), 0.441 (0.75), 0.457 (0.80), 0.461 (0.73), 0.472 (0.20), 0.527 (0.08), 0.545 (0.08), 1.055 (0.19), 1.072 (0.29), 1.091 (0.18), 1.241 (0.08), 1.402 (16.00), 1.416 (1.49), 1.491 (0.17), 1.526 (0.18), 1.558 (0.11), 1.807 (0.21), 1.830 (0.18), 1.841 (0.19), 1.988 (0.08), 2.048 (0.56), 2.060 (0.53), 2.070 (0.37), 2.082 (0.20), 2.089 (0.20), 2.097 (0.19), 2.500 (5.48), 2.565 (0.30), 2.578 (0.30), 2.592 (0.24), 2.628 (0.24), 2.639 (0.41), 2.651 (0.25), 2.670 (0.16), 2.681 (0.19), 3.485 (1.60), 3.502 (1.57), 4.379 (0.39), 4.389 (0.43), 4.394 (0.55), 4.404 (0.39).

### Intermediat 212

### Ethyl-(5RS,7RS)-2-[(6-chlorpyridin-3-yl)methyl]-3-oxo-7-(trifluormethyl)-2,3,5,6,7,8-hexahydro[1,2,4]triazolo[4,3-a]pyridin-5-carboxylat (Diastereomerengemisch; 4 Isomere)

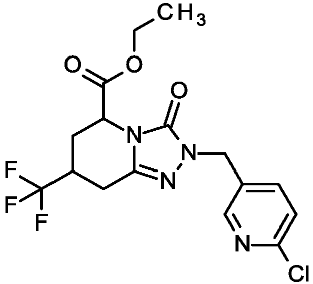

Ethyl-(5RS,7RS)-3-oxo-7-(trifluormethyl)-2,3,5,6,7,8-hexahydro[1,2,4]triazolo[4,3-a]pyridin-5-carboxylat (Diastereomerengemisch; 4 Isomere) (1.07 g, 3.83 mmol) wurde in Acetonitril (40 ml) vorgelegt. Cäsiumcarbonat (1.87 g, 5.75 mmol) und 2-Chlor-5-(chlormethyl)pyridin (652 mg, 4.02 mmol) wurden anschließend zugegeben. Das Reaktionsgemisch wurde nach Rühren über Nacht bei Raumtemperatur filtriert und Ethylacetat zugegeben. Das Filtrat wurde mit Wasser und gesättigter wässriger Natriumchlorid-Lösung gewaschen, über Natriumsulfat getrocknet, filtriert und das Filtrat eingeengt. Der Rückstand wurde über Säulenchromatographie (Kieselgel; Laufmittel:, Cyclohexan/Ethylacetat-Gradient 7/1, 0/1) aufgereinigt. Die produkthaltigen Fraktionen wurden im Vakuum eingeengt und es wurden 604 mg (89 % Reinheit, 34 % d. Th.) der Titelverbindung erhalten.
LC-MS (Methode 3): Rₜ = 1.67 min; MS (ESIpos): m/z = 405 [M+H]⁺
¹H-NMR (400 MHz, DMSO-d6) δ [ppm]: -0.008 (1.54), 0.008 (1.04), 1.164 (7.65), 1.182 (16.00), 1.194 (4.45), 1.199 (8.79), 1.212 (7.15), 1.229 (3.56), 1.305 (0.41), 1.398 (0.87), 1.797 (0.72), 1.828 (0.72), 1.988 (0.50), 2.256 (0.50), 2.275 (1.17), 2.291 (1.41), 2.304 (1.85), 2.318 (3.16), 2.346 (0.60), 2.523 (2.04), 2.678 (1.08), 2.708 (2.69), 2.743 (2.49), 2.760 (1.08), 2.768 (1.27), 2.778 (1.30), 2.808 (1.12), 2.881 (0.66), 2.887 (0.74), 2.921 (0.45), 2.927 (0.46), 2.952 (0.42), 2.978 (1.67), 3.008 (1.40), 3.059 (0.41), 4.114 (0.58), 4.132 (1.58), 4.141 (1.19), 4.150 (1.82), 4.159 (3.29), 4.168 (1.29), 4.177 (3.81), 4.191 (2.65), 4.196 (2.34), 4.208 (3.20), 4.215 (0.99), 4.226 (2.75), 4.235 (1.45), 4.243 (0.95), 4.252 (1.31), 4.270 (0.44), 4.509 (0.78), 4.524 (0.89), 4.537 (0.85), 4.551 (0.71), 4.816 (1.74), 4.822 (2.13), 4.829 (1.91), 4.836 (1.64), 4.910 (1.22), 4.922 (5.15), 4.950 (6.09), 4.959 (5.78), 4.999 (0.74), 5.754 (2.32), 7.521 (4.68), 7.541 (5.67), 7.713 (2.49), 7.719 (2.49), 7.734 (2.52), 7.740 (2.40), 7.751 (0.94), 7.758 (0.91), 8.315 (2.85), 8.321 (2.72), 8.333 (1.28), 8.339 (1.26).

### Intermediat 213

### Ethyl-(5RS,7RS)-3-oxo-7-(trifluormethyl)-2-{[6-(trifluormethyl)pyridin-3-yl]methyl}-2,3,5,6,7,8-hexahydro[1,2,4]triazolo[4,3-a]pyridin-5-carboxylat (Diastereomerengemisch; 4 Isomere)

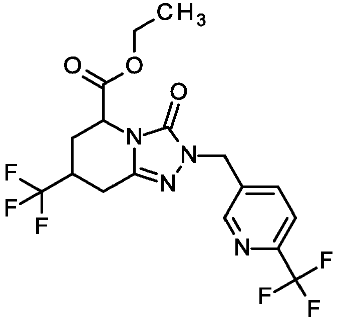

Ethyl-(5RS,7RS)-3-oxo-7-(trifluormethyl)-2,3,5,6,7,8-hexahydro[1,2,4]triazolo[4,3-a]pyridin-5-carboxylat (Diastereomerengemisch; 4 Isomere) (570 mg, 2.04 mmol) wurde in Acetonitril (20 ml) vorgelegt. Cäsiumcarbonat (998 mg, 3.06 mmol) und 5-(Chlormethyl)-2-(trifluormethyl)pyridin (419 mg, 2.14 mmol) wurden anschließend zugegeben. Zum Reaktionsgemisch wurde nach Rühren über Nacht bei Raumtemperatur Ethylacetat zugegeben. Die organische Phase wurde mit Wasser und gesättigter wässriger Natriumchlorid-Lösung gewaschen, über Natriumsulfat getrocknet, filtriert und das Filtrat eingeengt. Der Rückstand wurde über Säulenchromatographie (Kieselgel; Laufmittel:, Cyclohexan/Ethylacetat-Gradient 4/5, 1/16) aufgereinigt. Die produkthaltigen Fraktionen wurden im Vakuum eingeengt und es wurden 809 mg (86 % d. Th.) der Titelverbindung erhalten.
LC-MS (Methode 1): Rₜ = 1.25 min; MS (ESIpos): m/z = 439 [M+H]⁺
¹H-NMR (400 MHz, DMSO-d6) δ [ppm]: -0.008 (1.17), 0.008 (1.20), 1.159 (7.54), 1.177 (16.00), 1.192 (8.78), 1.194 (10.17), 1.210 (11.69), 1.228 (5.67), 1.398 (1.08), 1.776 (0.63), 1.806 (1.30), 1.838 (1.33), 1.867 (0.70), 1.908 (3.87), 1.988 (2.50), 2.282 (0.98), 2.298 (1.11), 2.311 (1.39), 2.325 (3.55), 2.366 (0.86), 2.670 (0.70), 2.687 (0.82), 2.717 (2.44), 2.746 (2.57), 2.777 (1.52), 2.787 (1.81), 2.817 (1.77), 2.890 (1.01), 2.896 (1.24), 2.930 (0.63), 2.936 (0.70), 2.986 (1.71), 3.017 (1.46), 4.021 (0.60), 4.038 (0.60), 4.117 (0.41), 4.135 (1.36), 4.144 (0.92), 4.152 (1.52), 4.161 (3.26), 4.176 (3.33), 4.181 (4.21), 4.194 (3.83), 4.199 (3.26), 4.211 (3.58), 4.217 (1.14), 4.229 (2.63), 4.238 (1.36), 4.247 (0.82), 4.256 (1.27), 4.274 (0.41), 4.521 (1.33), 4.536 (1.55), 4.549 (1.52), 4.563 (1.33), 4.831 (1.68), 4.838 (2.12), 4.853 (1.68), 4.931 (2.38), 5.036 (1.05), 5.048 (8.17), 5.077 (5.51), 5.087 (5.39), 5.127 (0.67), 7.913 (1.93), 7.933 (12.67), 7.950 (2.57), 7.954 (2.63), 7.974 (0.82), 8.661 (3.58), 8.679 (2.53).

### Intermediat 214

### Ethyl-(5RS,7RS)-2-{[3-fluor-2-(trifluormethyl)pyridin-4-yl]methyl}-3-oxo-7-(trifluormethyl)-2,3,5,6,7,8-hexahydro[1,2,4]triazolo[4,3-a]pyridin-5-carboxylat (Diastereomerengemisch; 4 Isomere)

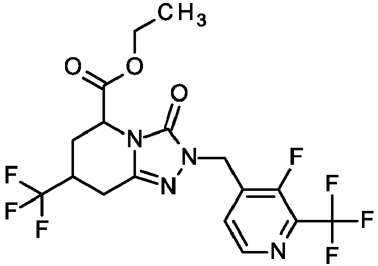

Ethyl-(5RS,7RS)-3-oxo-7-(trifluormethyl)-2,3,5,6,7,8-hexahydro[1,2,4]triazolo[4,3-a]pyridin-5-carboxylat (Diastereomerengemisch; 4 Isomere) (500 mg, 83 % Reinheit, 1.49 mmol) wurde in Acetonitril (15 ml) vorgelegt. Cäsiumcarbonat (1.21 g, 3.72 mmol) und 4-(Chlormethyl)-3-fluor-2-(trifluormethyl)pyridinhydrochlorid (420 mg, 1.56 mmol) wurden anschließend zugegeben. Zum Reaktionsgemisch wurde nach Rühren über Nacht bei Raumtemperatur Ethylacetat zugegeben. Die organische Phase wurde mit Wasser und gesättigter wässriger Natriumchlorid-Lösung gewaschen, über Natriumsulfat getrocknet, filtriert und das Filtrat eingeengt. Der Rückstand wurde über Säulenchromatographie (Kieselgel; Laufmittel:, Cyclohexan/Ethylacetat-Gradient 7/1, 1/16) aufgereinigt. Die produkthaltigen Fraktionen wurden im Vakuum eingeengt und es wurden 440 mg (65 % d. Th.) der Titelverbindung erhalten.
LC-MS (Methode 3): Rₜ = 1.92 min; MS (ESIpos): m/z = 457 [M+H]⁺
¹H-NMR (400 MHz, DMSO-d6) δ [ppm]: 0.008 (2.05), 1.157 (4.28), 1.170 (8.73), 1.175 (8.65), 1.187 (16.00), 1.192 (6.64), 1.205 (7.99), 1.210 (4.00), 1.228 (1.75), 1.398 (2.75), 1.819 (0.47), 1.850 (0.50), 1.988 (13.53), 2.253 (0.43), 2.269 (0.50), 2.288 (1.23), 2.303 (1.42), 2.317 (1.87), 2.332 (3.90), 2.365 (0.95), 2.523 (2.37), 2.670 (0.46), 2.696 (0.97), 2.709 (0.48), 2.726 (2.59), 2.762 (2.99), 2.798 (1.01), 2.828 (0.73), 2.908 (0.46), 2.999 (2.03), 3.028 (1.70), 4.003 (1.18), 4.021 (3.36), 4.038 (3.28), 4.056 (1.09), 4.123 (0.48), 4.140 (1.43), 4.149 (1.10), 4.158 (1.74), 4.167 (2.84), 4.175 (1.44), 4.185 (2.86), 4.192 (1.17), 4.199 (1.96), 4.216 (2.83), 4.226 (0.79), 4.234 (2.59), 4.243 (1.39), 4.252 (0.88), 4.261 (1.25), 4.532 (0.48), 4.547 (0.51), 4.560 (0.50), 4.574 (0.43), 4.848 (2.48), 4.857 (2.20), 5.098 (2.28), 5.134 (12.28), 7.594 (1.95), 7.607 (3.49), 7.620 (1.88), 7.638 (0.47), 7.650 (0.81), 7.663 (0.44), 8.581 (4.60), 8.593 (4.39).

### Intermediat 215

### tert-Butyl-(5S)-2-{[6-(difluormethyl)pyridin-3-yl]methyl}-3-oxo-2,3,5,6,7,8-hexahydro[1,2,4]triazolo[4,3-a]pyridin-5-carboxylat

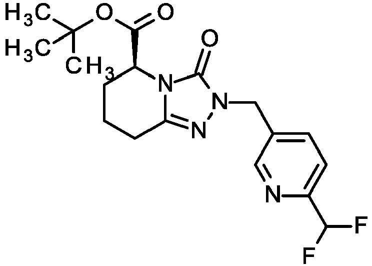

tert-Butyl-(5S)-3-oxo-2,3,5,6,7,8-hexahydro[1,2,4]triazolo[4,3-a]pyridin-5-carboxylat (239 mg, 999 µmol) wurde in Acetonitril (10 ml) vorgelegt. Anschließend wurden Cäsiumcarbonat (488 mg, 1.50 mmol) und 5-(Chlormethyl)-2-(difluormethyl)pyridin (186 mg, 1.05 mmol) zugegeben. Das Reaktionsgemisch wurde über Nacht bei Raumtemperatur gerührt. Es wurde erneut Cäsiumcarbonat (326 mg, 999 µmol) zugegeben und über Nacht bei Raumtemperatur gerührt. Das Reaktionsgemisch wurde mit Wasser und Ethylacetat versetzt und die organische Phase wurde abgetrennt. Die organische Phase wurde mit gesättigter wässriger Natriumchlorid-Lösung gewaschen, über Natriumsulfat getrocknet, filtriert und das Filtrat eingeengt. Es wurden 395 mg (83 % Reinheit, 86 % d. Th.) der Titelverbindung erhalten.
LC-MS (Methode 3): Rₜ = 1.58 min; MS (ESIpos): m/z = 381 [M+H]⁺

### Intermediat 216

### Ethyl-(5RS,7RS)-2-[(5-chlor-3-fluorpyridin-2-yl)methyl]-3-oxo-7-(trifluormethyl)-2,3,5,6,7,8-hexahydro[1,2,4]triazolo[4,3-a]pyridin-5-carboxylat (Diastereomerengemisch; 4 Isomere)

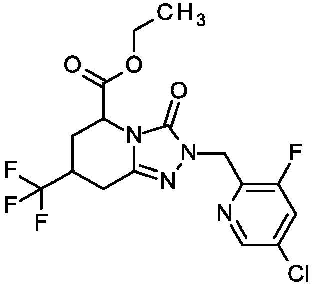

Ethyl-(5RS,7RS)-3-oxo-7-(trifluormethyl)-2,3,5,6,7,8-hexahydro[1,2,4]triazolo[4,3-a]pyridin-5-carboxylat (Diastereomerengemisch; 4 Isomere) (100 mg, 358 µmol) wurde in Acetonitril (4.0 ml) vorgelegt. Cäsiumcarbonat (175 mg, 537 µmol) und 5-Chlor-2-(chlormethyl)-3-fluorpyridin (67.7 mg, 376 µmol) wurden anschließend zugegeben. Zum Reaktionsgemisch wurde nach Rühren über Nacht bei Raumtemperatur Ethylacetat zugegeben. Die organische Phase wurde mit Wasser und gesättigter wässriger Natriumchlorid-Lösung gewaschen, über Natriumsulfat getrocknet, filtriert und das Filtrat eingeengt. Es wurden 135 mg (89 % d. Th.) der Titelverbindung erhalten.
LC-MS (Methode 4): Rₜ = 0.91 min; MS (ESIpos): m/z = 423 [M+H]⁺

### Intermediat 217

### Ethyl-(5RS,7RS)-2-[(5-chlorpyridin-3-yl)methyl]-3-oxo-7-(trifluormethyl)-2,3,5,6,7,8-hexahydro[1,2,4]triazolo[4,3-a]pyridin-5-carboxylat (Diastereomerengemisch; 4 Isomere)

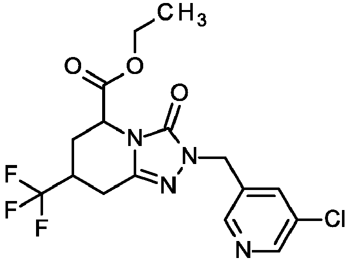

Ethyl-(5RS,7RS)-3-oxo-7-(trifluormethyl)-2,3,5,6,7,8-hexahydro[1,2,4]triazolo[4,3-a]pyridin-5-carboxylat (Diastereomerengemisch; 4 Isomere) (100 mg, 358 µmol) wurde in Acetonitril (4.0 ml) vorgelegt. Cäsiumcarbonat (175 mg, 537 µmol) und 3-Chlor-5-(chlormethyl)pyridin (60.9 mg, 376 µmol) wurden anschließend zugegeben. Zum Reaktionsgemisch wurde nach Rühren über Nacht bei Raumtemperatur Ethylacetat zugegeben. Die organische Phase wurde mit Wasser und gesättigter wässriger Natriumchlorid-Lösung gewaschen, über Natriumsulfat getrocknet, filtriert und das Filtrat eingeengt. Es wurden 128 mg (72 % Reinheit, 64 % d. Th.) der Titelverbindung erhalten.
LC-MS (Methode 4): Rₜ = 0.86 min; MS (ESIpos): m/z = 405 [M+H]⁺

### Intermediat 218

### tert-Butyl-(5S)-2-(4-brombenzyl)-3-oxo-2,3,5,6,7,8-hexahydro[1,2,4]triazolo[4,3-a]pyridin-5-carboxylat

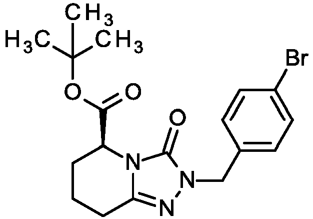

tert-Butyl-(5S)-3-oxo-2,3,5,6,7,8-hexahydro[1,2,4]triazolo[4,3-a]pyridin-5-carboxylat (150 mg, 627 µmol) wurde in Acetonitril (8.0 ml) vorgelegt. Cäsiumcarbonat (306 mg, 940 µmol) und 1-Brom-4-(chlormethyl)benzol (135 mg, 658 µmol) wurden anschließend zugegeben. Zum Reaktionsgemisch wurde nach Rühren über Nacht bei Raumtemperatur Ethylacetat zugegeben. Die organische Phase wurde mit Wasser und gesättigter wässriger Natriumchlorid-Lösung gewaschen, über Natriumsulfat getrocknet, filtriert und das Filtrat eingeengt. Es wurden 201 mg (74 % d. Th.) der Titelverbindung erhalten.
LC-MS (Methode 3): Rₜ = 1.94 min; MS (ESIpos): m/z = 408 [M+H]⁺

### Intermediat 219

### tert-Butyl-(5S)-2-(3-brombenzyl)-3-oxo-2,3,5,6,7,8-hexahydro[1,2,4]triazolo[4,3-a]pyridin-5-carboxylat

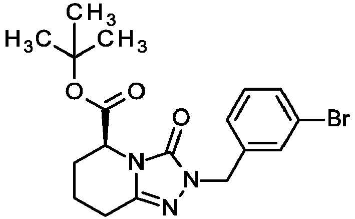

tert-Butyl-(5S)-3-oxo-2,3,5,6,7,8-hexahydro[1,2,4]triazolo[4,3-a]pyridin-5-carboxylat (150 mg, 627 µmol) wurde in Acetonitril (8.0 ml) vorgelegt. Cäsiumcarbonat (306 mg, 940 µmol) und 1-Brom-3-(chlormethyl)benzol (84 µl, 660 µmol) wurden anschließend zugegeben. Zum Reaktionsgemisch wurde nach Rühren über Nacht bei Raumtemperatur Ethylacetat zugegeben. Die organische Phase wurde mit Wasser und gesättigter wässriger Natriumchlorid-Lösung gewaschen, über Natriumsulfat getrocknet, filtriert und das Filtrat eingeengt. Es wurden 256 mg (94 % d. Th.) der Titelverbindung erhalten.
LC-MS (Methode 3): Rₜ = 1.94 min; MS (ESIpos): m/z = 408 [M+H]⁺

### Intermediat 220

### tert-Butyl-(5S)-2-(4-brom-2-fluorbenzyl)-3-oxo-2,3,5,6,7,8-hexahydro[1,2,4]triazolo[4,3-a]pyridin-5-carboxylat

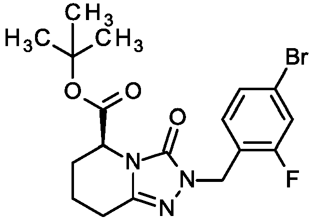

tert-Butyl-(5S)-3-oxo-2,3,5,6,7,8-hexahydro[1,2,4]triazolo[4,3-a]pyridin-5-carboxylat (150 mg, 627 µmol) wurde in Acetonitril (8.0 ml) vorgelegt. Cäsiumcarbonat (306 mg, 940 µmol) und 4-Brom-1-(chlormethyl)-2-fluorbenzol (147 mg, 658 µmol) wurden anschließend zugegeben. Das Reaktionsgemisch wurde über Nacht bei Raumtemperatur gerührt. Es wurden erneut 4-Brom-1-(chlormethyl)-2-fluorbenzol (30 mg, 132 µmol) und Cäsiumcarbonat (41 mg, 125 µmol) zugegeben und für weitere 6 Stunden gerührt. Zum Reaktionsgemisch wurde Ethylacetat zugegeben und die organische Phase wurde mit Wasser und gesättigter wässriger Natriumchlorid-Lösung gewaschen, über Natriumsulfat getrocknet, filtriert und das Filtrat eingeengt. Der Rückstand wurde über Säulenchromatographie (Kieselgel; Laufmittel:, Cyclohexan/Ethylacetat-Gradient 19/1, 0/1) aufgereinigt. Die produkthaltigen Fraktionen wurden im Vakuum eingeengt und es wurden 135 mg (51 % d. Th.) der Titelverbindung erhalten.
LC-MS (Methode 3): Rₜ = 2.01 min; MS (ESIpos): m/z = 426 [M+H]⁺
¹H-NMR (400 MHz, DMSO-d6) δ [ppm]: -0.174 (0.09), -0.033 (0.69), -0.017 (0.63), 0.121 (0.08), 1.132 (0.07), 1.150 (0.14), 1.167 (0.07), 1.208 (0.08), 1.368 (16.00), 1.445 (0.17), 1.480 (0.18), 1.773 (0.20), 1.795 (0.17), 1.807 (0.17), 1.963 (0.26), 2.029 (0.55), 2.037 (0.48), 2.063 (0.18), 2.071 (0.16), 2.303 (0.07), 2.578 (0.22), 2.589 (0.38), 2.601 (0.24), 2.620 (0.13), 2.631 (0.19), 2.644 (0.16), 3.995 (0.06), 4.013 (0.06), 4.391 (0.38), 4.406 (0.58), 4.416 (0.37), 4.785 (0.08), 4.826 (1.67), 4.867 (0.08), 7.187 (0.37), 7.208 (0.80), 7.228 (0.46), 7.380 (0.50), 7.384 (0.51), 7.401 (0.41), 7.405 (0.42), 7.522 (0.46), 7.527 (0.43), 7.546 (0.47), 7.550 (0.44).

### Intermediat 221

### tert-Butyl-(5S)-3-oxo-2-{(1RS)-1-[6-(trifluormethyl)pyridin-3-yl]ethyl}-2,3,5,6,7,8-hexahydro[1,2,4]triazolo[4,3-a]pyridin-5-carboxylat (Isomer 1)

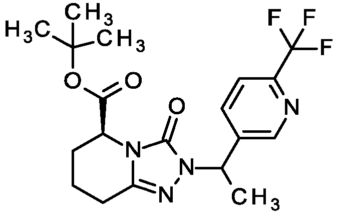

tert-Butyl-(5S)-3-oxo-2,3,5,6,7,8-hexahydro[1,2,4]triazolo[4,3-a]pyridin-5-carboxylat (201 mg, 840 µmol) wurde in Acetonitril (10 ml) vorgelegt. Cäasiumcarbonat (410 mg, 1.26 mmol) und 5-[(1RS)-1-Bromethyl]-2-(trifluormethyl)pyridin (320 mg, 70 % Reinheit, 882 µmol) wurden anschließend zugegeben. Zum Reaktionsgemisch wurde nach Rühren über Nacht bei Raumtemperatur Ethylacetat zugegeben. Die organische Phase wurde mit Wasser und gesättigter wässriger Natriumchlorid-Lösung gewaschen, über Natriumsulfat getrocknet, filtriert und das Filtrat eingeengt. Der Rückstand wurde über Säulenchromatographie (Kieselgel; Laufmittel:, Cyclohexan/Ethylacetat-Gradient 9/1, 0/1) aufgereinigt. Nach der Trennung, wurden 86.9 mg (24 % d. Th.) von Isomer 1, welches zuerst eluiert, und 97.7 mg (26 % d. Th.) von Isomer 2, welches später eluiert, isoliert.

### Isomer 1:

LC-MS (Methode 3): Rₜ = 1.84 min; MS (ESIpos): m/z = 413 [M+H]⁺
¹H-NMR (400 MHz, DMSO-d6) δ [ppm]: -0.149 (0.21), -0.024 (0.08), -0.008 (1.59), 0.008 (1.85), 0.146 (0.21), 1.301 (16.00), 1.382 (0.59), 1.398 (0.12), 1.405 (0.14), 1.457 (0.14), 1.467 (0.17), 1.479 (0.16), 1.489 (0.16), 1.501 (0.17), 1.514 (0.13), 1.527 (0.10), 1.686 (2.25), 1.703 (2.28), 1.820 (0.20), 1.831 (0.19), 1.842 (0.18), 1.852 (0.17), 1.864 (0.15), 1.988 (0.09), 2.057 (0.33), 2.067 (0.63), 2.080 (0.53), 2.099 (0.21), 2.107 (0.17), 2.322 (0.23), 2.327 (0.35), 2.332 (0.25), 2.366 (0.30), 2.522 (0.88), 2.557 (0.36), 2.562 (0.22), 2.572 (0.19), 2.587 (0.39), 2.602 (0.29), 2.614 (0.29), 2.627 (0.22), 2.665 (0.48), 2.669 (0.52), 2.674 (0.60), 2.687 (0.25), 2.710 (0.40), 2.718 (0.20), 2.731 (0.10), 4.432 (0.37), 4.446 (0.62), 4.456 (0.38), 5.520 (0.13), 5.536 (0.46), 5.554 (0.46), 5.572 (0.14), 7.906 (0.63), 7.927 (0.90), 8.015 (0.45), 8.019 (0.46), 8.040 (0.33), 8.699 (0.63), 8.703 (0.63).

### Intermediat 222

### tert-Butyl-(5S)-3-oxo-2-{(1RS)-1-[6-(trifluormethyl)pyridin-3-yl]ethyl}-2,3,5,6,7,8-hexahydro[1 ,2,4]triazolo[4,3-a]pyridin-5-carboxylat (Isomer 2)

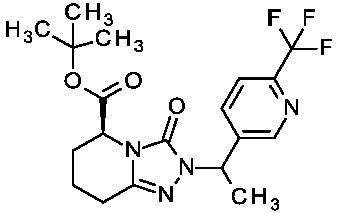

tert-Butyl-(5S)-3-oxo-2,3,5,6,7,8-hexahydro[1,2,4]triazolo[4,3-a]pyridin-5-carboxylat (201 mg, 840 µmol) wurde in Acetonitril (10 ml) vorgelegt. Cäasiumcarbonat (410 mg, 1.26 mmol) und 5-[(1RS)-1-Bromethyl]-2-(trifluormethyl)pyridin (320 mg, 70 % Reinheit, 882 µmol) wurden anschließend zugegeben. Zum Reaktionsgemisch wurde nach Rühren über Nacht bei Raumtemperatur Ethylacetat zugegeben. Die organische Phase wurde mit Wasser und gesättigter wässriger Natriumchlorid-Lösung gewaschen, über Natriumsulfat getrocknet, filtriert und das Filtrat eingeengt. Der Rückstand wurde über Säulenchromatographie (Kieselgel; Laufmittel:, Cyclohexan/Ethylacetat-Gradient 9/1, 0/1) aufgereinigt. Nach der Trennung, wurden 86.9 mg (24 % d. Th.) von Isomer 1, welches zuerst eluiert, und 97.7 mg (26 % d. Th.) von Isomer 2, welches später eluiert, isoliert.

### Isomer 2:

LC-MS (Methode 3): Rₜ = 1.86 min; MS (ESIpos): m/z = 413 [M+H]⁺
¹H-NMR (400 MHz, DMSO-d6) δ [ppm]: -0.014 (0.28), 1.152 (0.06), 1.169 (0.13), 1.187 (0.06), 1.240 (0.07), 1.372 (0.22), 1.401 (16.00), 1.480 (0.16), 1.503 (0.17), 1.515 (0.18), 1.529 (0.13), 1.557 (0.09), 1.680 (2.33), 1.698 (2.34), 1.809 (0.19), 1.820 (0.18), 1.831 (0.17), 1.842 (0.17), 1.983 (0.16), 2.049 (0.56), 2.061 (0.46), 2.089 (0.16), 2.322 (0.06), 2.518 (0.24), 2.560 (0.34), 2.575 (0.24), 2.587 (0.25), 2.602 (0.20), 2.659 (0.23), 2.670 (0.39), 2.682 (0.22), 2.712 (0.19), 2.724 (0.10), 3.093 (0.08), 4.399 (0.37), 4.414 (0.62), 4.424 (0.36), 4.635 (0.06), 4.650 (0.07), 5.511 (0.14), 5.529 (0.53), 5.547 (0.48), 5.564 (0.14), 7.881 (0.67), 7.901 (0.90), 8.011 (0.48), 8.016 (0.48), 8.037 (0.36), 8.723 (0.69).

### Intermediat 223

### tert-Butyl-(5S)-3-oxo-2-{[cisitrans-4-(trifluormethyl)cyclohexyl]methyl}-2,3,5,6,7,8-hexahydro[1,2,4]triazolo[4,3-a]pyridin-5-carboxylat (Diastereomerengemisch; 2 Isomere)

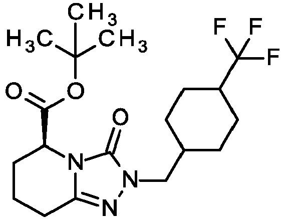

tert-Butyl-(5S)-3-oxo-2,3,5,6,7,8-hexahydro[1,2,4]triazolo[4,3-a]pyridin-5-carboxylat (450 mg, 1.88 mmol) wurde in Acetonitril (20 ml) vorgelegt. Cäsiumcarbonat (1.90 g, 5.83 mmol) und cis/trans-1-(Brommethyl)-4-(trifluormethyl)cyclohexan (660 µl, 4.1 mmol) wurden anschließend zugegeben und das Reaktionsgemisch wurde über Nacht bei Raumtemperatur gerührt. Es wurden erneut cis/trans-1-(Brommethyl)-4-(trifluormethyl)cyclohexan (660 µl, 4.1 mmol) und Cäsiumcarbonat (613 mg, 1.88 mmol) zugegeben und über das Wochenende gerührt. Das Reaktionsgemisch wurde filtriert und Ethylacetat zugegeben. Die organische Phase wurde mit Wasser und gesättigter wässriger Natriumchlorid-Lösung gewaschen, über Natriumsulfat getrocknet, filtriert und das Filtrat eingeengt. Der Rückstand wurde über Säulenchromatographie aufgereinigt (Kieselgel; Laufmittel: Dichlormethan/Methanol 99/1, 9/1). Es wurde ein weiteres Mal über Säulenchromatographie aufgereinigt (Kieselgel; Laufmittel: Cyclohexan/Ethylacetat 11/1, 1/2). Die produkthaltigen Fraktionen wurden im Vakuum eingeengt und es wurden 112 mg (14 % d. Th.) der Titelverbindung erhalten.
LC-MS (Methode 3): Rₜ = 2.08 min; MS (ESIpos): m/z = 404 [M+H]⁺

### Intermediat 224

### (5S)-5-{[(2S)-2-(Hydroxymethyl)pyrrolidin-1 -yl]carbonyl}-2-(4-methylbenzyl)-5,6,7,8-tetrahydro[1,2,4]triazolo[4,3-a]pyridin-3(2H)-on

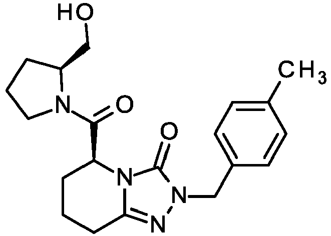

(5S)-2-(4-Methylbenzyl)-3-oxo-2,3,5,6,7,8-hexahydro[1,2,4]triazolo[4,3-a]pyridin-5-carbonsäure (100 mg, 348 µmol) wurde in Dichlormethan (2.0 ml) und DMF (4.0 ml) bei Raumtemperatur vorgelegt. Anschließend wurde 1-[Bis(dimethylamino)methylen]-1H-benzotriazol-1-ium-3-oxidhexafluorophosphat (172 mg, 452 µmol) und N,N-Diisopropylethylamin (85 µl, 490 µmol) zugegeben. Nach 15 min Rühren wurde (2S)-Pyrrolidin-2-ylmethanol (41 µl, 420 µmol) zugeben und das Reaktionsgemisch für 1.5 h bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 83.1 mg (64 % d. Th.) der Titelverbindung erhalten.
LC-MS (Methode 3): Rₜ = 0.93 min; MS (ESIpos): m/z = 371 [M+H]⁺
¹H-NMR (400 MHz, DMSO-d6) δ [ppm]: -0.149 (1.74), -0.008 (16.00), 0.008 (12.83), 0.146 (1.58), 2.271 (2.57), 2.327 (2.72), 2.366 (2.42), 2.523 (7.55), 2.669 (2.57), 2.709 (2.42), 4.741 (1.13), 5.753 (3.40), 7.122 (1.66).

### Intermediat 225

### 2-[(2S)-1-{[(5S)-2-(4-Methylbenzyl)-3-oxo-2,3,5,6,7,8-hexahydro[1,2,4]triazolo[4,3-a]pyridin-5-yl]carbonyl}pyrrolidin-2-yl]-2-oxoethyl-acetat

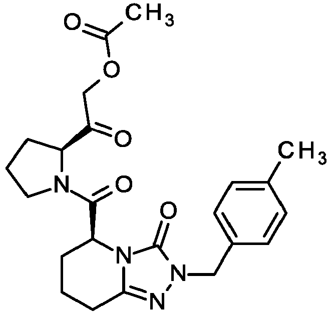

tert-Butyl-(2S)-2-(acetoxyacetyl)pyrrolidin-1-carboxylat (189 mg, 696 µmol) wurde in Dichlormethan (1 ml) gelöst und mit Trifluoressigsäure (1 ml) versetzt und 1 h bei Raumtemperatur nachgerührt, anschließend wurden die flüchtigen organischen Verbindungen im Vakuum entfernt. Das Rohprodukt von (2S)-2-(Acetoxyacetyl)pyrrolidin wurde in der nächsten Stufe direkt weiter umgesetzt.

(5S)-2-(4-Methylbenzyl)-3-oxo-2,3,5,6,7,8-hexahydro[1,2,4]triazolo[4,3-a]pyridin-5-carbonsäure (100 mg, 348 µmol) wurde in Dichlormethan (2 ml) gelöst und auf 0°C gekühlt. 1-Chlor-N,N,2-trimethylprop-1-en-1-amin (64 µl, 490 µmol) wurde zugegeben und bei 0°C 20 min nachgerührt. (2S)-2-(Acetoxyacetyl)pyrrolidin (Rohprodukt) wurde in Dichlormethan (1 ml) gelöst und bei 0°C langsam zum Reaktionsgemisch zugetropft, gefolgt von N,N-Diisopropylethylamin (180 µl, 1.0 mmol). Nach 5 min wurde das Lösungsmittel unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 112 mg (73 % d. Th.) der Titelverbindung erhalten.
LC-MS (Methode 1): Rₜ = 1.05 min; MS (ESIpos): m/z = 441 [M+H]⁺
¹H-NMR (400 MHz, DMSO-d6) δ [ppm]: -0.008 (2.75), 0.008 (1.89), 1.580 (0.46), 1.594 (0.46), 1.726 (0.58), 1.759 (0.48), 1.799 (0.53), 1.814 (0.75), 1.832 (0.76), 1.847 (0.76), 1.862 (0.49), 1.912 (0.68), 1.928 (1.30), 1.947 (1.68), 1.960 (1.27), 2.040 (1.43), 2.080 (16.00), 2.096 (4.78), 2.100 (1.50), 2.162 (0.56), 2.183 (0.63), 2.193 (0.66), 2.214 (0.65), 2.269 (11.51), 2.323 (0.52), 2.327 (0.68), 2.366 (0.59), 2.518 (2.35), 2.523 (2.26), 2.567 (1.05), 2.580 (0.71), 2.669 (0.65), 2.710 (0.55), 3.575 (0.79), 3.582 (0.56), 3.599 (0.99), 3.617 (0.43), 3.690 (0.46), 3.750 (0.45), 3.766 (0.94), 3.783 (0.53), 3.791 (0.71), 4.545 (1.05), 4.559 (1.17), 4.566 (1.15), 4.581 (1.01), 4.733 (4.26), 4.766 (0.86), 4.774 (0.45), 4.809 (4.21), 4.822 (5.23), 4.830 (1.60), 4.840 (0.86), 4.865 (1.73), 4.951 (1.12), 4.995 (0.62), 7.093 (0.92), 7.114 (7.45), 7.120 (6.87), 7.141 (0.89).

### Intermediat 226

### Methyl-3-oxo-8-(trifluormethyl)-2-{[6-(trifluormethyl)pyridin-3-yl]methyl}-2,3-dihydro[1,2,4]triazolo[4,3-a]pyridin-5-carboxylat

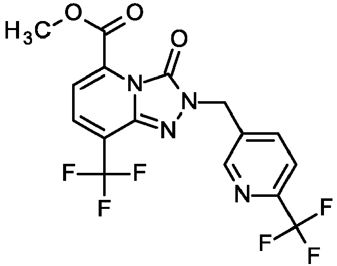

Methyl-3-oxo-8-(trifluormethyl)-2,3-dihydro[1,2,4]triazolo[4,3-a]pyridin-5-carboxylat (50.0 mg, 191 µmol) wurde in Acetonitril (2.0 ml) vorgelegt. Cäsiumcarbonat (93.6 mg, 287 µmol) und 5-(Chlormethyl)-2-(trifluormethyl)pyridin (39.3 mg, 201 µmol) wurden anschließend zugegeben. Zum Reaktionsgemisch wurde nach Rühren über Nacht bei Raumtemperatur Ethylacetat zugegeben. Die organische Phase wurde mit Wasser und gesättigter wässriger Natriumchlorid-Lösung gewaschen, über Natriumsulfat getrocknet, filtriert und das Filtrat eingeengt. Es wurden 86.9 mg (77 % Reinheit, 83 % d. Th.) der Titelverbindung erhalten.
LC-MS (Methode 3): Rₜ = 1.80 min; MS (ESIpos): m/z = 421 [M+H]⁺

### Intermediat 227

### Methyl-2-[(5-chlor-3-fluorpyridin-2-yl)methyl]-3-oxo-8-(trifluormethyl)-2,3-dihydro[1,2,4]triazolo[4,3-a]pyridin-5-carboxylat

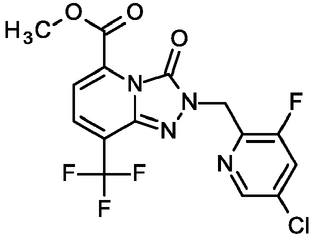

Methyl-3-oxo-8-(trifluormethyl)-2,3-dihydro[1,2,4]triazolo[4,3-a]pyridin-5-carboxylat (150 mg, 574 µmol) wurde in Acetonitril (5.0 ml) vorgelegt. Cäsiumcarbonat (281 mg, 862 µmol) und 5-Chlor-2-(chlormethyl)-3-fluorpyridin (109 mg, 603 µmol) wurden anschließend zugegeben. Zum Reaktionsgemisch wurde nach Rühren über Nacht bei Raumtemperatur Ethylacetat zugegeben. Die organische Phase wurde mit Wasser und gesättigter wässriger Natriumchlorid-Lösung gewaschen, über Natriumsulfat getrocknet, filtriert und das Filtrat eingeengt. Der Rückstand wurde über Säulenchromatographie (Kieselgel; Laufmittel:, Cyclohexan/Ethylacetat-Gradient 7/1, 0/1) aufgereinigt. Die produkthaltigen Fraktionen wurden im Vakuum eingeengt und es wurden 87.5 mg (38 % d. Th.) der Titelverbindung erhalten.
LC-MS (Methode 3): Rₜ = 1.77 min; MS (ESIpos): m/z = 405 [M+H]⁺
¹H-NMR (400 MHz, DMSO-d6) δ [ppm]: -0.150 (0.63), -0.008 (5.70), 0.008 (5.37), 0.146 (0.68), 1.157 (0.41), 1.175 (0.84), 1.398 (1.64), 1.988 (1.53), 2.327 (0.95), 2.670 (1.01), 3.847 (0.65), 3.901 (16.00), 5.334 (4.58), 5.338 (4.66), 6.984 (1.72), 7.001 (1.74), 7.780 (1.39), 7.798 (1.39), 8.146 (1.28), 8.151 (1.34), 8.170 (1.25), 8.175 (1.34), 8.481 (1.50), 8.484 (1.50).

### Intermediat 228

### tert-Butyl-(5RS,8RS)-8-methyl-2-(4-methylbenzyl)-3-oxo-2,3,5,6,7,8-hexahydro[1,2,4]triazolo[4,3-a]pyridin-5-carboxylat (Diastereomerengemisch; 4 Isomere)

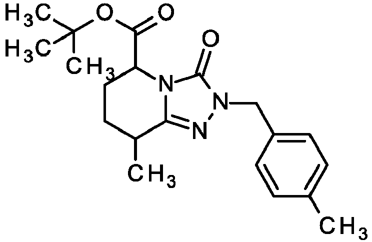

tert-Butyl-(5RS,8RS)-8-methyl-3-oxo-2,3,5,6,7,8-hexahydro[1,2,4]triazolo[4,3-a]pyridin-5-carboxylat (Diastereomerengemisch; 4 Isomere) (33.4 mg, 132 µmol) wurde in Acetonitril (5 ml) vorgelegt. Cäsiumcarbonat (51.6 mg, 158 µmol) und 1-(Brommethyl)-4-methylbenzol (25.6 mg, 138 µmol) wurden anschließend zugegeben. Das Reaktionsgemisch wurde über Nacht bei Raumtemperatur gerührt, anschließend wurde 1-(Brommethyl)-4-methylbenzol (5 mg, 2.6 µmol) zugegeben und für 2 h nachgerührt. Die Reaktionsmischung wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 39.5 mg (79 % d. Th.) der Titelverbindung als Gemisch von Diastereomeren erhalten.
LC-MS (Methode 3): Rₜ = 2.02 min; MS (ESIpos): m/z = 358 [M+H]⁺
¹H-NMR (400 MHz, DMSO-d6) δ [ppm]: -0.008 (1.06), 0.008 (0.92), 1.153 (0.40), 1.164 (2.69), 1.181 (2.84), 1.194 (0.43), 1.201 (0.63), 1.236 (0.88), 1.259 (0.60), 1.298 (0.42), 1.344 (0.64), 1.350 (0.72), 1.355 (1.41), 1.386 (16.00), 1.396 (3.32), 1.417 (0.68), 1.424 (0.49), 1.428 (0.55), 1.510 (0.69), 1.548 (0.87), 1.562 (0.66), 2.120 (0.63), 2.130 (0.54), 2.272 (5.05), 2.294 (0.49), 2.523 (0.91), 4.447 (0.54), 4.790 (1.10), 4.820 (1.08), 7.129 (6.94).

### Intermediat 229

### tert-Butyl-(5RS,8RS)-8-methyl-3-oxo-2-{[6-(trifluormethyl)pyridin-3-yl]methyl}-2,3,5,6,7,8-hexahydro[1,2,4]triazolo[4,3-a]pyridin-5-carboxylat (Diastereomerengemisch, 4 Isomere)

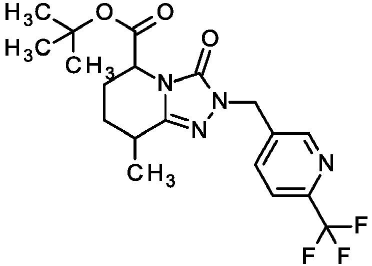

tert-Butyl-(5RS,8RS)-8-methyl-3-oxo-2,3,5,6,7,8-hexahydro[1,2,4]triazolo[4,3-a]pyridin-5-carboxylat (Diastereomerengemisch, 4 Isomere) (503 mg, 1.98 mmol) wurde in Acetonitril (20 ml) vorgelegt. Cäsiumcarbonat (970 mg, 2.98 mmol) und 5-(Brommethyl)-2-(trifluormethyl)pyridin (500 mg, 2.08 mmol) wurden anschließend zugegeben. Das Reaktionsgemisch wurde nach Rühren über Nacht bei Raumtemperatur und 1 h unter Rückfluss gerührt. Die Reaktionsmischung wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Magnseiumsulfat getrocknet, filtriert und das Filtrat eingeengt. Es wurden 854 mg (90 % Reinheit, 94 % d. Th.) der Titelverbindung als Gemisch von Diastereomeren erhalten
LC-MS (Methode 4): Rₜ = 1.00 min; MS (ESIpos): m/z = 413 [M+H]⁺
¹H-NMR (400 MHz, DMSO-d6) δ [ppm]: 1.157 (0.41), 1.177 (3.04), 1.194 (3.00), 1.367 (16.00), 1.548 (0.40), 1.988 (0.72), 2.135 (0.73), 2.145 (0.70), 2.154 (0.41), 4.466 (0.44), 4.475 (0.69), 4.487 (0.44), 5.067 (1.61), 5.074 (1.56), 7.926 (1.76), 8.659 (0.86).

### Intermediat 230

### tert-Butyl-(5S)-2-[(E)-2-(4-fluorphenyl)vinyl]-3-oxo-2,3,5,6,7,8-hexahydro[1,2,4]triazolo[4,3-a]pyridin-5-carboxylat (Enantiomer 1)

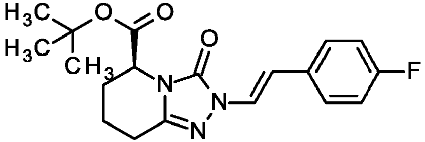

Molekularsieb (4A°, 20.0 mg) und [(E)-2-(4-Fluorphenyl)vinyl]borsäure (139 mg, 836 µmol) wurden in Dichlormethan (2.0 ml) bei Raumtemperatur vorgelegt. Anschließend wurden Triethylamin (120 µl, 840 µmol), Kupfer(II)acetat (85.4 mg, 460 µmol), tert-Butyl-(5S)-3-oxo-2,3,5,6,7,8-hexahydro[1,2,4]triazolo[4,3-a]pyridin-5-carboxylat (100 mg, 418 µmol) und 2,2,6,6-Tetramethylpiperidin-1-yloxyl (71.8 mg, 460 µmol) zugegeben. Das Reaktionsgemisch wurde über Nacht bei Raumtemperatur gerührt. Zum Reaktionsgemisch wurde 2 N Ammoniak in Methanol (50µl) zugegeben und anschließend eingeengt. Der Rückstand wurde über Säulenchromatographie (Kieselgel; Laufmittel:, Cyclohexan/Ethylacetat-Gradient 7/1, 0/1) aufgereinigt. Die produkthaltigen Fraktionen wurden im Vakuum eingeengt und es wurden 53.1 mg (35 % d. Th.) der Titelverbindung erhalten.
LC-MS (Methode 3): Rₜ = 2.10 min; MS (ESIpos): m/z = 360 [M+H]⁺
¹H-NMR (400 MHz, DMSO-d6) δ [ppm]: -0.008 (1.24), 0.008 (1.17), 1.014 (0.21), 1.098 (0.22), 1.423 (16.00), 1.864 (0.17), 1.872 (0.18), 2.086 (0.17), 2.097 (0.45), 2.107 (0.44), 2.120 (0.25), 2.134 (0.16), 2.141 (0.17), 2.323 (0.20), 2.327 (0.28), 2.332 (0.21), 2.366 (0.25), 2.523 (1.07), 2.636 (0.18), 2.665 (0.54), 2.669 (0.42), 2.674 (0.32), 2.679 (0.38), 2.691 (0.28), 2.710 (0.37), 2.738 (0.21), 2.750 (0.37), 2.762 (0.24), 2.792 (0.17), 4.502 (0.37), 4.511 (0.40), 4.517 (0.53), 4.526 (0.35), 6.753 (0.74), 6.789 (0.85), 7.121 (0.68), 7.143 (1.38), 7.165 (0.75), 7.393 (0.98), 7.429 (0.90), 7.543 (0.70), 7.557 (0.77), 7.565 (0.75), 7.574 (0.30), 7.579 (0.67).

### Intermediat 231

### Ethyl-(5RS,6RS)-2-{[3-fluor-2-(trifluormethyl)pyridin-4-yl]methyl}-3-oxo-6-(trifluormethyl)-2,3,5,6,7,8-hexahydro[1,2,4]triazolo[4,3-a]pyridin-5-carboxylat (Diastereomerengemisch, 4 Isomere)

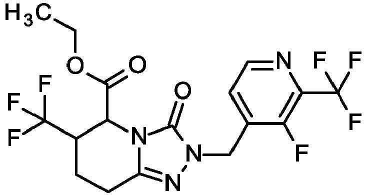

Ethyl-(5RS,6RS)-3-oxo-6-(trifluormethyl)-2,3,5,6,7,8-hexahydro[1,2,4]triazolo[4,3-a]pyridin-5-carboxylat (Diastereomerengemisch, 4 Isomere) (400 mg, 62 % Reinheit, 888 µmol) wurde in Acetonitril (6.2 ml) vorgelegt. Caesiumcarbonat (579 mg, 1.78 mmol) und 4-(Chlormethyl)-3-fluor-2-(trifluormethyl)pyridin (199 mg, 933 µmol) wurden anschließend zugegeben. Das Reaktionsgemisch wurde nach 48 Stunden Rühren bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 231 mg (73 % Reinheit, 42 % d. Th.) der Titelverbindung erhalten.
LC-MS (Methode 3): Rₜ = 1.82 min; MS (ESIpos): m/z = 457 [M+H]⁺ und Rₜ = 1.85 min; MS (ESIpos): m/z = 457 [M+H]⁺

### Intermediat 232

### Ethyl-(5RS,6RS)-3-oxo-6-(trifluormethyl)-2-{[6-(trifluormethyl)pyridin-3-yl]methyl}-2,3,5,6,7,8-hexahydro[1,2,4]triazolo[4,3-a]pyridin-5-carboxylat (Diastereomerengemisch, 4 Isomere)

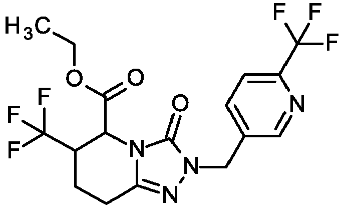

Ethyl-(5RS,6RS)-3-oxo-6-(trifluormethyl)-2,3,5,6,7,8-hexahydro[1,2,4]triazolo[4,3-a]pyridin-5-carboxylat (Diastereomerengemisch, 4 Isomere) (250 mg, 62 % Reinheit, 555 µmol) wurde in Acetonitril (3.9 ml) vorgelegt. Caesiumcarbonat (362 mg, 1.11 mmol) und 5-(Chlormethyl)-2-(trifluormethyl)pyridin (114 mg, 583 µmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 131 mg (54 % d. Th.) der Titelverbindung erhalten.
LC-MS (Methode 1): Rₜ = 1.23 min; MS (ESIpos): m/z = 439 [M+H]⁺ und Rₜ = 1.24 min; MS (ESIpos): m/z = 439 [M+H]⁺
¹H-NMR (400 MHz, DMSO-d6) δ [ppm]: -0.149 (1.37), -0.008 (13.25), 0.008 (11.69), 0.146 (1.37), 1.148 (3.00), 1.166 (6.37), 1.185 (9.50), 1.203 (16.00), 1.221 (7.63), 1.833 (0.50), 2.018 (2.69), 2.036 (2.06), 2.052 (0.94), 2.327 (2.19), 2.366 (2.31), 2.523 (6.81), 2.669 (2.50), 2.710 (3.06), 2.764 (1.19), 2.781 (1.88), 2.825 (0.94), 2.869 (0.63), 3.425 (1.00), 4.147 (1.44), 4.154 (1.56), 4.165 (1.62), 4.172 (1.62), 4.179 (2.50), 4.196 (7.44), 4.214 (7.37), 4.232 (2.31), 4.648 (4.00), 4.662 (3.87), 4.815 (1.50), 4.829 (1.62), 5.010 (0.63), 5.058 (9.50), 5.099 (0.69), 7.927 (14.25), 7.930 (11.12), 8.642 (1.69), 8.655 (3.81).

### Intermediat 233

### Ethyl-(5RS,6RS)-2-[(3,5-dichlorpyridin-2-yl)methyl]-3-oxo-6-(trifluormethyl)-2,3,5,6,7,8-hexahydro[1,2,4]triazolo[4,3-a]pyridin-5-carboxylat (Diastereomerengemisch, 4 Isomere)

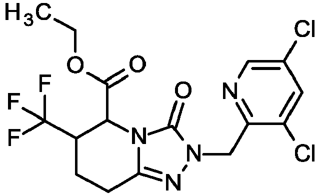

Ethyl-(5RS,6RS)-3-oxo-6-(trifluormethyl)-2,3,5,6,7,8-hexahydro[1,2,4]triazolo[4,3-a]pyridin-5-carboxylat (Diastereomerengemisch, 4 Isomere) (566 mg, 2.03 mmol) wurde in Acetonitril (15 ml) vorgelegt. Caesiumcarbonat (1.32 g, 4.05 mmol) und 3,5-Dichlor-2-(chlormethyl)pyridin (418 mg, 2.13 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 235 mg (86 % Reinheit, 23 % d. Th.) der Titelverbindung erhalten.
LC-MS (Methode 8): Rₜ = 2.82 min; MS (ESIpos): m/z = 439 [M+H]⁺ und Rₜ = 2.87 min; MS (ESIpos): m/z = 439 [M+H]⁺
¹H-NMR (400 MHz, DMSO-d6) δ [ppm]: -0.149 (1.83), -0.008 (16.00), 0.008 (12.50), 0.146 (1.68), 1.168 (2.67), 1.185 (5.56), 1.201 (8.91), 1.219 (15.85), 1.237 (7.62), 1.831 (0.46), 1.850 (0.46), 2.013 (2.74), 2.028 (2.51), 2.045 (1.22), 2.073 (1.22), 2.327 (1.98), 2.366 (2.44), 2.523 (5.94), 2.621 (0.84), 2.648 (1.07), 2.669 (3.28), 2.710 (2.44), 2.754 (1.98), 2.771 (1.07), 2.797 (0.99), 3.416 (1.07), 4.144 (1.30), 4.155 (1.30), 4.161 (1.37), 4.178 (2.74), 4.195 (7.62), 4.213 (7.47), 4.231 (2.36), 4.616 (4.11), 4.629 (4.04), 4.805 (1.52), 4.819 (1.45), 5.007 (1.22), 5.047 (7.70), 5.055 (6.78), 5.060 (8.15), 5.100 (1.22), 8.259 (3.28), 8.264 (4.11), 8.557 (4.27), 8.562 (4.11).

### Intermediat 234

### Ethyl-(5RS,6RS)-2-[(5-chlor-3-fluorpyridin-2-yl)methyl]-3-oxo-6-(trifluormethyl)-2,3,5,6,7,8-hexahydro[1,2,4]triazolo[4,3-a]pyridin-5-carboxylat (Diastereomerengemisch, 4 Isomere)

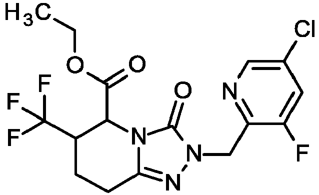

Ethyl-(5RS,6RS)-3-oxo-6-(trifluormethyl)-2,3,5,6,7,8-hexahydro[1,2,4]triazolo[4,3-a]pyridin-5-carboxylat (Diastereomerengemisch, 4 Isomere) (432 mg, 1.55 mmol) wurde in Acetonitril (10 ml) vorgelegt. Caesiumcarbonat (1.01 g, 3.09 mmol) und 5-Chlor-2-(chlormethyl)-3-fluorpyridin (292 mg, 1.62 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 235 mg (36 % d. Th.) der Titelverbindung erhalten.
LC-MS (Methode 4): Rₜ = 0.90 min; MS (ESIpos): m/z = 423 [M+H]⁺
¹H-NMR (400 MHz, DMSO-d6) δ [ppm]: -0.149 (1.95), -0.008 (16.00), 0.008 (15.09), 0.146 (1.90), 1.164 (2.12), 1.182 (4.63), 1.198 (4.02), 1.216 (5.88), 1.234 (2.85), 2.005 (0.91), 2.020 (0.82), 2.037 (0.48), 2.073 (1.08), 2.327 (0.95), 2.366 (1.08), 2.523 (3.11), 2.617 (0.43), 2.665 (1.08), 2.669 (1.17), 2.674 (1.08), 2.710 (1.21), 2.733 (0.56), 2.747 (0.82), 2.763 (0.61), 2.822 (0.43), 4.133 (0.48), 4.143 (1.08), 4.150 (1.17), 4.161 (1.17), 4.168 (1.08), 4.175 (1.04), 4.192 (2.81), 4.210 (2.72), 4.228 (0.86), 4.607 (1.43), 4.620 (1.43), 4.793 (1.08), 4.807 (1.08), 5.008 (2.68), 5.017 (2.64), 8.104 (1.30), 8.129 (1.38), 8.476 (1.86).

### Intermediat 235

### Ethyl-(5RS,6RS)-2-[(5-chlorpyridin-2-yl)methyl]-3-oxo-6-(trifluormethyl)-2,3,5,6,7,8-hexahydro[1,2,4]triazolo[4,3-a]pyridin-5-carboxylat (Diastereomerengemisch, 4 Isomere)

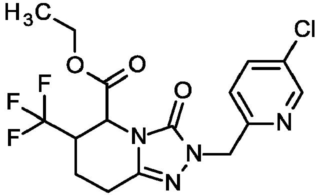

Ethyl-(5RS,6RS)-3-oxo-6-(trifluormethyl)-2,3,5,6,7,8-hexahydro[1,2,4]triazolo[4,3-a]pyridin-5-carboxylat (Diastereomerengemisch, 4 Isomere) (400 mg, 1.43 mmol) wurde in Acetonitril (7.1 ml) vorgelegt. Caesiumcarbonat (934 mg, 2.87 mmol) und 5-Chlor-2-(chlormethyl)pyridin (244 mg, 1.50 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 135 mg (23 % d. Th.) der Titelverbindung erhalten.
LC-MS (Methode 3): Rt = 1.65 min; MS (ESIpos): m/z = 405 [M+H]⁺ und Rₜ = 1.68 min; MS (ESIpos): m/z = 405 [M+H]⁺

### Intermediat 236

### tert-Butyl-(5RS,7RS)-2-{[3-fluor-2-(trifluormethyl)pyridin-4-yl]methyl}-7-methyl-3-oxo-2,3,5,6,7,8-hexahydro[1,2,4]triazolo[4,3-a]pyridin-5-carboxylat (Diastereomerengemisch, 4 Isomere)

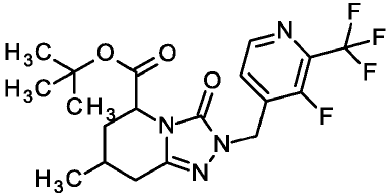

tert-Butyl-(5RS,7RS)-7-methyl-3-oxo-2,3,5,6,7,8-hexahydro[1,2,4]triazolo[4,3-a]pyridin-5-carboxylat (Diastereomerengemisch, 4 Isomere) (300 mg, 1.18 mmol) wurde in Acetonitril (6.0 ml) vorgelegt. Caesiumcarbonat (965 mg, 2.96 mmol) und 4-(Chlormethyl)-3-fluor-2-(trifluormethyl)pyridin (266 mg, 1.24 mmol) wurden anschließend zugegeben. Das Reaktionsgemisch wurde nach Rühren über Nacht bei 60°C mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 374 mg (73 % d. Th.) der Titelverbindung erhalten.
LC-MS (Methode 4): Rₜ = 1.02 min; MS (ESIpos): m/z = 431 [M+H]⁺
¹H-NMR (400 MHz, DMSO-d6) δ [ppm]: 1.018 (2.06), 1.035 (2.12), 1.354 (5.48), 1.393 (0.48), 1.410 (16.00), 1.911 (0.98), 2.228 (0.46), 2.241 (0.62), 2.269 (0.49), 2.650 (0.42), 4.303 (0.47), 4.313 (0.45), 5.067 (1.13), 5.079 (1.12), 5.133 (0.72), 7.619 (0.77), 7.632 (0.57), 8.569 (0.89), 8.581 (0.89).

### Intermediat 237

### tert-Butyl-(5RS,7RS)-7-methyl-3-oxo-2-{[6-(trifluormethyl)pyridin-3-yl]methyl}-2,3,5,6,7,8-hexahydro[1,2,4]triazolo[4,3-a]pyridin-5-carboxylat (Diastereomerengemisch, 4 Isomere)

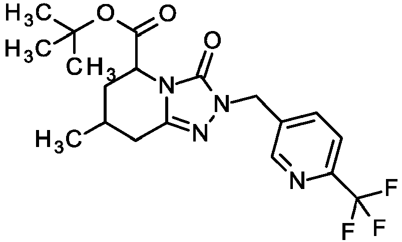

tert-Butyl-(5RS,7RS)-7-methyl-3-oxo-2,3,5,6,7,8-hexahydro[1,2,4]triazolo[4,3-a]pyridin-5-carboxylat (Diastereomerengemisch, 4 Isomere) (247 mg, 975 µmol) wurde in Acetonitril (5.0 ml) vorgelegt. Caesiumcarbonat (794 mg, 2.44 mmol) und 5-(Chlormethyl)-2-(trifluormethyl)pyridin (200 mg, 1.02 mmol) wurden anschließend zugegeben. Das Reaktionsgemisch wurde nach Rühren über Nacht bei 60°C mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 274 mg (68 % d. Th.) der Titelverbindung erhalten.
LC-MS (Methode 4): Rₜ = 0.98 min; MS (ESIpos): m/z = 413 [M+H]⁺
¹H-NMR (400 MHz, DMSO-d6) δ [ppm]: 1.011 (2.18), 1.028 (2.23), 1.337 (5.62), 1.385 (0.63), 1.406 (16.00), 1.905 (1.13), 2.217 (0.52), 2.230 (0.66), 2.258 (0.53), 2.641 (0.48), 4.279 (0.40), 4.295 (0.47), 4.305 (0.45), 5.019 (2.49), 5.077 (0.86), 7.921 (1.40), 7.931 (0.85), 7.936 (0.83), 8.667 (0.73).

### Intermediat 238

### tert-Butyl-(5RS,7RS)-2-(3-chlor-4-fluorbenzyl)-7-methyl-3-oxo-2,3,5,6,7,8-hexahydro[1,2,4]triazolo[4,3-a]pyridin-5-carboxylat (Diastereomerengemisch, 4 Isomere)

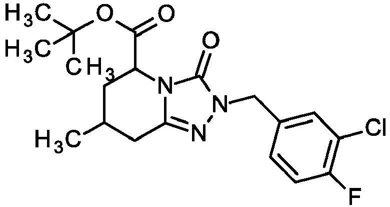

tert-Butyl-(5RS,7RS)-7-methyl-3-oxo-2,3,5,6,7,8-hexahydro[1,2,4]triazolo[4,3-a]pyridin-5-carboxylat (Diastereomerengemisch, 4 Isomere) (300 mg, 1.18 mmol) wurde in Acetonitril (6.0 ml) vorgelegt. Caesiumcarbonat (965 mg, 2.96 mmol) und 4-(Brommethyl)-2-chlor-1-fluorbenzol (278 mg, 1.24 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 327 mg (70 % d. Th.) der Titelverbindung erhalten.
LC-MS (Methode 3): Rₜ = 2.09 min; MS (ESIpos): m/z = 396 [M+H]⁺
¹H-NMR (400 MHz, DMSO-d6) δ [ppm]: -0.008 (0.46), 1.011 (2.15), 1.027 (2.14), 1.348 (0.46), 1.381 (0.71), 1.411 (16.00), 2.186 (0.41), 2.214 (0.51), 2.227 (0.60), 2.255 (0.51), 4.275 (0.41), 4.291 (0.48), 4.302 (0.45), 4.834 (1.64), 7.376 (0.59), 7.399 (0.79), 7.421 (0.44), 7.448 (0.45), 7.453 (0.42), 7.466 (0.45).

### Intermediat 239

### Ethyl-(5RS,7RS)-2-[(5-chlorpyridin-2-yl)methyl]-3-oxo-7-(trifluormethyl)-2,3,5,6,7,8-hexahydro[1,2,4]triazolo[4,3-a]pyridin-5-carboxylat (Diastereomerengemisch, 4 Isomere)

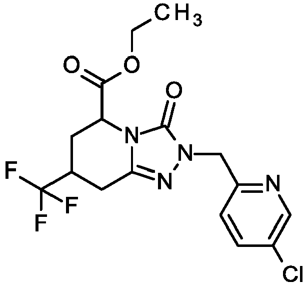

Ethyl-(5RS,7RS)-3-oxo-7-(trifluormethyl)-2,3,5,6,7,8-hexahydro[1,2,4]triazolo[4,3-a]pyridin-5-carboxylat (Diastereomerengemisch; 4 Isomere) (500 mg, 1.70 mmol) wurde in Acetonitril (20 ml) vorgelegt. Caesiumcarbonat (1.39 g, 4.25 mmol) und 5-Chlor-2-(chlormethyl)pyridinhydrochlorid (355 mg, 1.79 mmol) wurden anschließend zugegeben. Das Reaktionsgemisch wurde nach Rühren über Nacht zur Hälfte eingeengt und bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 248 mg (36 % d. Th.) der Titelverbindung erhalten.
LC-MS (Methode 4): Rₜ = 0.89 min; MS (ESIpos): m/z = 405 [M+H]⁺
¹H-NMR (400 MHz, DMSO-d6) δ [ppm]: -0.149 (0.41), 0.146 (0.41), 1.183 (4.99), 1.201 (15.06), 1.217 (16.00), 1.234 (6.56), 1.780 (0.75), 1.810 (1.63), 1.841 (1.78), 1.871 (0.84), 2.296 (0.77), 2.324 (2.75), 2.336 (2.13), 2.366 (0.42), 2.670 (0.49), 2.686 (0.67), 2.716 (1.72), 2.740 (1.79), 2.753 (1.97), 2.770 (1.78), 2.780 (2.11), 2.810 (2.19), 2.888 (1.32), 2.895 (1.52), 2.929 (0.83), 2.935 (0.84), 2.986 (1.41), 3.015 (1.12), 3.082 (0.72), 4.148 (0.92), 4.162 (2.00), 4.179 (5.57), 4.197 (5.43), 4.217 (2.69), 4.235 (1.82), 4.245 (0.93), 4.253 (0.62), 4.262 (0.85), 4.531 (1.67), 4.546 (1.90), 4.559 (1.81), 4.573 (1.61), 4.824 (1.22), 4.831 (1.64), 4.844 (1.28), 4.948 (11.83), 4.977 (9.93), 7.234 (2.72), 7.256 (2.91), 7.271 (3.45), 7.292 (3.70), 7.929 (3.74), 7.935 (3.92), 7.949 (3.59), 7.956 (3.73), 8.579 (4.25), 8.584 (4.25).

### Intermediat 240

### tert-Butyl-(5RS,8RS)-2-{[3-fluor-2-(trifluormethyl)pyridin-4-yl]methyl}-8-methyl-3-oxo-2,3,5,6,7,8-hexahydro[1,2,4]triazolo[4,3-a]pyridin-5-carboxylat (Diastereomerengemisch, 4 Isomere)

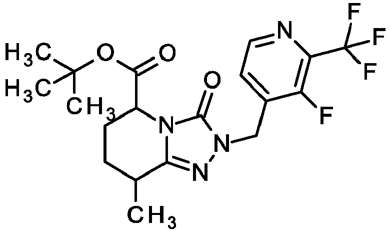

tert-Butyl-(5RS,8RS)-8-methyl-3-oxo-2,3,5,6,7,8-hexahydro[1,2,4]triazolo[4,3-a]pyridin-5-carboxylat (Diastereomerengemisch; 4 Isomere) (300 mg, 1.18 mmol) wurde in Acetonitril (10 ml) vorgelegt. Caesiumcarbonat (424 mg, 1.30 mmol) und 4-(Chlormethyl)-3-fluor-2-(trifluormethyl)pyridin (266 mg, 1.24 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 456 mg (89 % d. Th.) der Titelverbindung erhalten.
LC-MS (Methode 1): Rₜ = 2.09 min; MS (ESIpos): m/z = 429 [M-H]⁺

### Intermediat 241

### tert-Butyl-(5RS,8RS)-2-(3-chlor-4-fluorbenzyl)-8-methyl-3-oxo-2,3,5,6,7,8-hexahydro[1,2,4]triazolo[4,3-a]pyridin-5-carboxylat (Diastereomerengemisch, 4 Isomere)

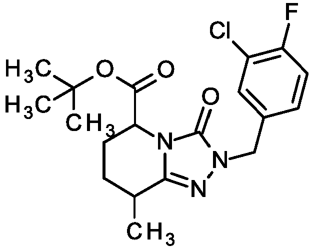

tert-Butyl-(5RS,8RS)-8-methyl-3-oxo-2,3,5,6,7,8-hexahydro[1,2,4]triazolo[4,3-a]pyridin-5-carboxylat (Diastereomerengemisch; 4 Isomere) (300 mg, 1.18 mmol) wurde in Acetonitril (10 ml) vorgelegt. Caesiumcarbonat (772 mg, 2.37 mmol) und 2-Chlor-4-(chlormethyl)-1-fluorbenzol (223 mg, 1.24 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 450 mg (87 % d. Th.) der Titelverbindung erhalten.
LC-MS (Methode 3): Rₜ = .08 min; MS (ESIpos): m/z = 340 [M-tBu+H]⁺

### Intermediat 242

### tert-Butyl-(5RS,8RS)-2-(2,4-difluorbenzyl)-8-methyl-3-oxo-2,3,5,6,7,8-hexahydro[1,2,4]triazolo[4,3-a]pyridin-5-carboxylat (Diastereomerengemisch, 4 Isomere)

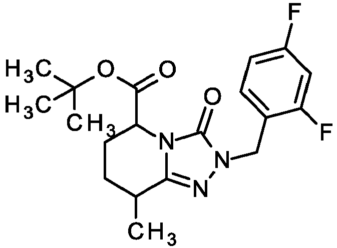

tert-Butyl-(5RS,8RS)-8-methyl-3-oxo-2,3,5,6,7,8-hexahydro[1,2,4]triazolo[4,3-a]pyridin-5-carboxylat (Diastereomerengemisch; 4 Isomere) (300 mg, 1.18 mmol) wurde in Acetonitril (10 ml) vorgelegt. Caesiumcarbonat (772 mg, 2.37 mmol) und 1-(Chlormethyl)-2,4-difluorbenzol (202 mg, 1.24 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 400 mg (84 % Reinheit, 75 % d. Th.) der Titelverbindung erhalten.
LC-MS (Methode 3): Rₜ = 1.44 min; MS (ESIpos): m/z = 380 [M+H]⁺

### Intermediat 243

### Methyl-(5S)-2-(3-chlor-4-fluorbenzyl)-3-oxo-2,3,5,6,7,8-hexahydro[1,2,4]triazolo[4,3-a]pyridin-5-carboxylat

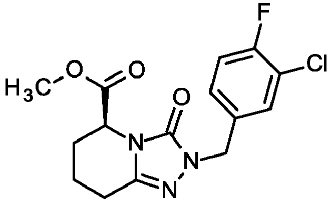

Methyl-(5S)-3-oxo-2,3,5,6,7,8-hexahydro[1,2,4]triazolo[4,3-a]pyridin-5-carboxylat (110 mg, 559 µmol) wurde in Acetonitril (2.0 ml, 38 mmol) vorgelegt. Caesiumcarbonat (273 mg, 838 µmol) und 2-Chlor-4-(chlormethyl)-1-fluorbenzol (105 mg, 587 µmol) wurden anschließend zugegeben. Das Reaktionsgemisch wurde nach Rühren über Nacht bei Rückfluss mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 58.5 mg (30 % d. Th.) der Titelverbindung erhalten.
LC-MS (Methode 1): Rₜ = 1.11 min; MS (ESIpos): m/z = 340 [M+H]⁺

### Intermediat 244

### Methyl-(5S)-3-oxo-2-[3-(trifluormethyl)benzyl]-2,3,5,6,7,8-hexahydro[1,2,4]triazolo[4,3-a]pyridin-5-carboxylat

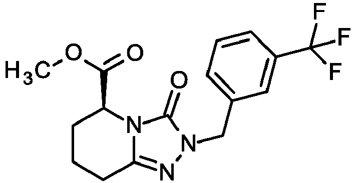

Methyl-(5S)-3-oxo-2,3,5,6,7,8-hexahydro[1,2,4]triazolo[4,3-a]pyridin-5-carboxylat (300 mg, 1.52 mmol) wurde in Acetonitril (6.0 ml) vorgelegt. Caesiumcarbonat (744 mg, 2.28 mmol) und 1-(Brommethyl)-3-(trifluormethyl)benzol (382 mg, 1.60 mmol) wurden anschließend zugegeben. Das Reaktionsgemisch wurde nach Rühren über Nacht unter Rückfluss mit Wasser, 1 N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 345 mg (64 % d. Th.) der Titelverbindung erhalten.
LC-MS (Methode 1): Rₜ = 1.15 min; MS (ESIpos): m/z = 356 [M+H]⁺
¹H-NMR (400 MHz, DMSO-d6) δ [ppm]: 1.196 (2.01), 1.212 (3.72), 1.226 (2.33), 1.502 (0.44), 1.530 (0.49), 1.802 (0.57), 1.826 (0.49), 1.835 (0.51), 2.078 (0.89), 2.093 (0.89), 2.120 (0.57), 2.131 (0.51), 2.139 (0.55), 2.147 (0.53), 2.328 (0.59), 2.562 (1.35), 2.577 (0.99), 2.589 (0.99), 2.603 (0.80), 2.629 (0.74), 2.641 (1.31), 2.653 (0.76), 2.671 (1.02), 2.711 (0.51), 2.891 (0.42), 3.702 (16.00), 4.622 (1.08), 4.631 (1.23), 4.637 (1.44), 4.647 (1.10), 4.960 (4.25), 4.965 (4.25), 7.101 (5.21), 7.519 (1.04), 7.538 (1.71), 7.584 (0.95), 7.603 (2.01), 7.615 (2.41), 7.658 (1.63), 7.676 (0.87).

### Intermediat 245

### Methyl-(5S)-3-oxo-2-{[5-(trifluormethyl)pyridin-2-yl]methyl}-2,3,5,6,7,8-hexahydro[1,2,4]triazolo[4,3-a]pyridin-5-carboxylat

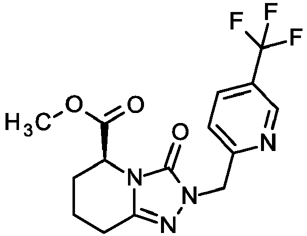

Methyl-(5S)-3-oxo-2,3,5,6,7,8-hexahydro[1,2,4]triazolo[4,3-a]pyridin-5-carboxylat (187 mg, 947 µmol) wurde in Acetonitril (4.0 ml) vorgelegt. Caesiumcarbonat (463 mg, 1.42 mmol) und 2-(Brommethyl)-5-(trifluormethyl)pyridin (250 mg, 1.04 mmol) wurden anschließend zugegeben. Das Reaktionsgemisch wurde nach Rühren über Nacht bei Raumtemperatur mit Wasser, 1 N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 185 mg (55 % d. Th.) der Titelverbindung erhalten.
LC-MS (Methode 3): Rₜ = 1.34 min; MS (ESIpos): m/z = 357 [M+H]⁺
¹H-NMR (400 MHz, DMSO-d6) δ [ppm]: 1.560 (0.40), 1.827 (0.50), 1.841 (0.44), 1.851 (0.42), 2.083 (0.45), 2.092 (0.76), 2.106 (0.76), 2.126 (0.45), 2.135 (0.53), 2.147 (0.47), 2.155 (0.52), 2.163 (0.48), 2.559 (0.44), 2.575 (0.90), 2.589 (0.77), 2.601 (0.78), 2.615 (0.64), 2.637 (0.60), 2.649 (1.13), 2.661 (0.72), 2.691 (0.47), 3.715 (16.00), 4.631 (1.05), 4.641 (1.16), 4.647 (1.36), 4.657 (1.01), 5.063 (5.88), 7.396 (1.64), 7.416 (1.71), 8.223 (1.06), 8.228 (1.09), 8.243 (1.02), 8.249 (1.04), 8.935 (1.74).

### Intermediat 246

### Methyl-(5S)-2-[(5-methyl-1H-pyrazol-3-yl)methyl]-3-oxo-2,3,5,6,7,8-hexahydro[1,2,4]triazolo[4,3-a]pyridin-5-carboxylat

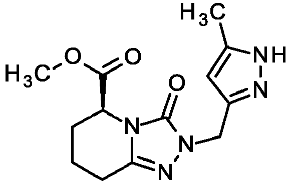

Methyl-(5S)-3-oxo-2,3,5,6,7,8-hexahydro[1,2,4]triazolo[4,3-a]pyridin-5-carboxylat (200 mg, 1.01 mmol) wurde in Acetonitril (5.0 ml) vorgelegt. Caesiumcarbonat (364 mg, 1.12 mmol) und 3-(Chlormethyl)-5-methyl-1H-pyrazolhydrochlorid (178 mg, 1.06 mmol) wurden anschließend zugegeben. Das Reaktionsgemisch wurde nach Rühren über Nacht bei Raumtemperatur mit Wasser, 1 N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 60.7 mg (21 % d. Th.) der Titelverbindung erhalten.
LC-MS (Methode 3): Rₜ = 0.85 min; MS (ESIpos): m/z = 292 [M+H]⁺

### Intermediat 247

### Methyl-(5S)-2-{[3-chlor-4-(trifluormethyl)pyridin-2-yl]methyl}-3-oxo-2,3,5,6,7,8-hexahydro[1,2,4]triazolo[4,3-a]pyridin-5-carboxylat

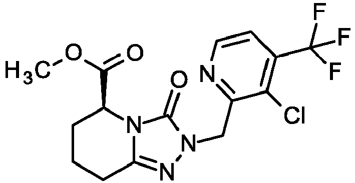

Methyl-(5S)-3-oxo-2,3,5,6,7,8-hexahydro[1,2,4]triazolo[4,3-a]pyridin-5-carboxylat (150 mg, 761 µmol) wurde in Acetonitril (5.0 ml) vorgelegt. Caesiumcarbonat (273 mg, 837 µmol) und 3-Chlor-2-(chlormethyl)-4-(trifluormethyl)pyridinhydrochlorid (532 mg, 40 % Reinheit, 799 µmol) wurden anschließend zugegeben. Das Reaktionsgemisch wurde nach Rühren über Nacht bei Raumtemperatur mit Wasser, 1 N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 137 mg (46 % d. Th.) der Titelverbindung erhalten.
LC-MS (Methode 1): Rₜ = 1.06 min; MS (ESIpos): m/z = 391 [M+H]⁺

### Intermediat 248

### Methyl-(5S)-2-[4-fluor-3-(trifluormethyl)benzyl]-3-oxo-2,3,5,6,7,8-hexahydro[1,2,4]triazolo[4,3-a]pyridin-5-carboxylat

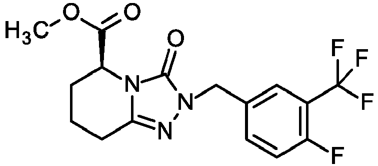

Methyl-(5S)-3-oxo-2,3,5,6,7,8-hexahydro[1,2,4]triazolo[4,3-a]pyridin-5-carboxylat (200 mg, 1.01 mmol) wurde in Acetonitril (5.0 ml) vorgelegt. Caesiumcarbonat (826 mg, 2.54 mmol) und 4-(Brommethyl)-1-fluor-2-(trifluormethyl)benzol (287 mg, 1.12 mmol) wurden anschließend zugegeben. Das Reaktionsgemisch wurde nach Rühren über Nacht bei Raumtemperatur mit Wasser, 1 N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 370 mg (98 % d. Th.) der Titelverbindung erhalten.
LC-MS (Methode 4): Rₜ = 0.88 min; MS (ESIpos): m/z = 374 [M+H]⁺

### Intermediat 249

### Methyl-(5S)-3-oxo-2-{[6-(trifluormethyl)pyridin-3-yl]methyl}-2,3,5,6,7,8-hexahydro[1,2,4]triazolo[4,3-a]pyridin-5-carboxylat

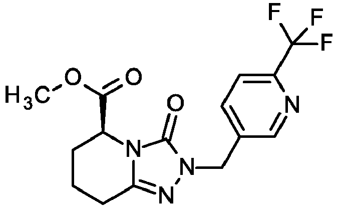

Methyl-(5S)-3-oxo-2,3,5,6,7,8-hexahydro[1,2,4]triazolo[4,3-a]pyridin-5-carboxylat (400 mg, 2.03 mmol) wurde in Acetonitril (10 ml) vorgelegt. Caesiumcarbonat (991 mg, 3.04 mmol) und 5-(Brommethyl)-2-(trifluormethyl)pyridin (511 mg, 2.13 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 622 mg (79 % d. Th.) der Titelverbindung erhalten.
LC-MS (Methode 4): Rₜ = 0.73 min; MS (ESIpos): m/z = 357 [M+H]⁺

### Intermediat 250

### Methyl-(5S)-2-{[6-fluor-2-(trifluormethyl)chinolin-4-yl]methyl}-3-oxo-2,3,5,6,7,8-hexahydro[1,2,4]triazolo[4,3-a]pyridin-5-carboxylat

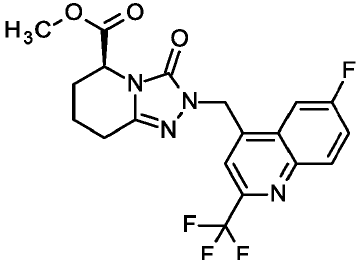

Methyl-(5S)-3-oxo-2,3,5,6,7,8-hexahydro[1,2,4]triazolo[4,3-a]pyridin-5-carboxylat (200 mg, 1.01 mmol) wurde in Acetonitril vorgelegt. Caesiumcarbonat (826 mg, 2.54 mmol) und 4-(Chlormethyl)-6-fluor-2-(trifluormethyl)chinolin (453 mg, 62 % Reinheit, 1.06 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 59.0 mg (14 % d. Th.) der Titelverbindung erhalten.
LC-MS (Methode 1): Rₜ = 1.24 min; MS (ESIpos): m/z = 425 [M+H]⁺

### Intermediat 251

### Methyl-(5S)-2-(6,7-dihydro-5H-cyclopenta[c]pyridin-3-ylmethyl)-3-oxo-2,3,5,6,7,8-hexahydro[1,2,4]triazolo[4,3-a]pyridin-5-carboxylat

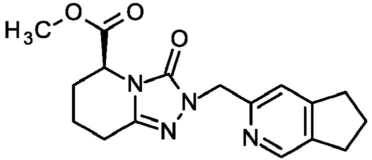

Methyl-(5S)-3-oxo-2,3,5,6,7,8-hexahydro[1,2,4]triazolo[4,3-a]pyridin-5-carboxylat (85.5 mg, 433 µmol) wurde in Acetonitril (10 ml) vorgelegt. Caesiumcarbonat (353 mg, 1.08 mmol) und 3-(Chlormethyl)-6,7-dihydro-5H-cyclopenta[b]pyridinhydrochlorid (115 mg, 563 µmol) wurden anschließend zugegeben. Das Reaktionsgemisch wurde, nach Rühren über Nacht bei Raumtemperatur und anschließend für 3 Stunden unter Rückfluss, abgekühlt und mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 106 mg (90 % Reinheit, 67 % d. Th.) der Titelverbindung erhalten.
LC-MS (Methode 3): Rₜ = 0.75 min; MS (ESIpos): m/z = 329 [M+H]⁺

### Intermediat 252

### Methyl-(5S)-3-oxo-2-{[5-(trifluormethyl)pyridin-3-yl]methyl}-2,3,5,6,7,8-hexahydro[1,2,4]triazolo[4,3-a]pyridin-5-carboxylat

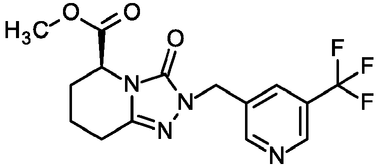

Methyl-(5S)-3-oxo-2,3,5,6,7,8-hexahydro[1,2,4]triazolo[4,3-a]pyridin-5-carboxylat (90.0 mg, 456 µmol) wurde in Acetonitril (10 ml) vorgelegt. Caesiumcarbonat (372 mg, 1.14 mmol) und 3-(Chlormethyl)-5-(trifluormethyl)pyridin (116 mg, 593 µmol) wurden anschließend zugegeben. Das Reaktionsgemisch wurde 3 Stunden unter Rückfluss, über Nacht bei Raumtemperatur und anschließend erneut für 4 Stunden unter Rückfluss gerührt. Das Reaktionsgemisch würde auf Raumtemperatur abgekühlt und mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 40.9 mg (25 % d. Th.) der Titelverbindung erhalten.
LC-MS (Methode 3): Rₜ = 1.29 min; MS (ESIpos): m/z = 357 [M+H]⁺

### Intermediat 253

### Methyl-(5S)-3-oxo-2-{[2-(trifluormethyl)-1,8-naphthyridin-3-yl]methyl}-2,3,5,6,7,8-hexahydro[1,2,4]triazolo[4,3-a]pyridin-5-carboxylat

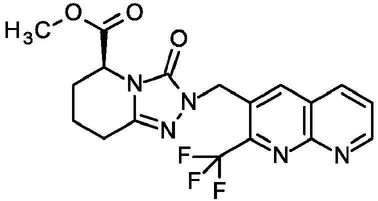

Methyl-(5S)-3-oxo-2,3,5,6,7,8-hexahydro[1,2,4]triazolo[4,3-a]pyridin-5-carboxylat (28.3 mg, 143 µmol) wurde in Acetonitril (3.0 ml) vorgelegt. Caesiumcarbonat (187 mg, 574 µmol) und 3-(Chlormethyl)-2-(trifluormethyl)-1,8-naphthyridin (46.0 mg, 187 µmol) wurden anschließend zugegeben. Das Reaktionsgemisch wurde nach 3 Stunden Rühren unter Rückfluss auf Raumtemperatur abgekühlt und mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 9.70 mg (17 % d. Th.) der Titelverbindung erhalten.
LC-MS (Methode 3): Rₜ = 1.23 min; MS (ESIpos): m/z = 408 [M+H]⁺
¹H-NMR (400 MHz, DMSO-d6) δ [ppm]: -0.008 (2.26), 0.008 (1.74), 1.572 (0.46), 1.832 (0.55), 1.843 (0.53), 1.857 (0.48), 1.867 (0.48), 2.111 (0.58), 2.135 (1.21), 2.144 (0.85), 2.152 (0.66), 2.164 (0.53), 2.172 (0.55), 2.179 (0.48), 2.327 (0.61), 2.366 (0.52), 2.523 (2.03), 2.561 (0.52), 2.589 (0.90), 2.604 (0.78), 2.616 (0.79), 2.630 (0.66), 2.656 (0.65), 2.667 (1.51), 2.679 (0.84), 2.709 (0.92), 3.732 (16.00), 4.672 (1.05), 4.682 (1.15), 4.688 (1.44), 4.697 (1.01), 5.239 (5.10), 7.818 (1.42), 7.828 (1.44), 7.838 (1.45), 7.849 (1.49), 8.434 (3.29), 8.583 (1.49), 8.588 (1.59), 8.604 (1.46), 8.609 (1.43), 9.248 (1.43), 9.253 (1.53), 9.258 (1.53), 9.263 (1.42).

### Intermediat 254

### tert-Butyl-(5S)-2-[(6-methoxypyridin-3-yl)methyl]-3-oxo-2,3,5,6,7,8-hexahydro[1,2,4]triazolo[4,3-a]pyridin-5-carboxylat

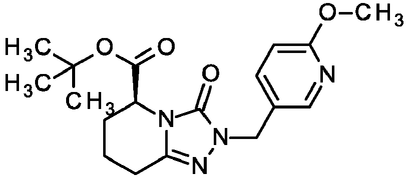

tert-Butyl-(5S)-3-oxo-2,3,5,6,7,8-hexahydro[1,2,4]triazolo[4,3-a]pyridin-5-carboxylat (1.33 g, 5.55 mmol) wurde in Acetonitril (30 ml) vorgelegt. Caesiumcarbonat (4.52 g, 13.9 mmol), 5-(Chlormethyl)-2-methoxypyridin (963 mg, 6.11 mmol) und Natriumiodid (5.00 mg, 0.03 mmol) wurden anschließend zugegeben. Das Reaktionsgemisch wurde nach Rühren für 4 Tage bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über Säulenchromatographie aufgereinigt (Kieselgel, Laufmittel: Cyclohexan/Ethylacetat-Gradient). Die produkthaltigen Fraktionen wurden im Vakuum eingeengt und es wurden 751 mg (37 % d. Th.) der Titelverbindung erhalten.
LC-MS (Methode 3): Rₜ = 1.56 min; MS (ESIpos): m/z = 361 [M+H]⁺
¹H-NMR (400 MHz, DMSO-d6) δ [ppm]: 1.385 (16.00), 2.048 (0.54), 2.058 (0.48), 2.525 (0.41), 3.825 (6.81), 4.424 (0.60), 4.791 (2.68), 6.785 (0.78), 6.807 (0.84), 7.574 (0.51), 7.580 (0.52), 7.595 (0.49), 7.602 (0.50), 8.074 (0.67), 8.080 (0.65).

### Intermediat 255

### tert-Butyl-(5S)-3-oxo-2-{[5-(trifluormethyl)pyrazin-2-yl]methyl}-2,3,5,6,7,8-hexahydro[1,2,4]triazolo[4,3-a]pyridin-5-carboxylat

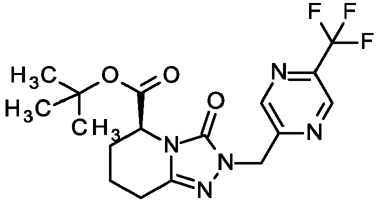

tert-Butyl-(5S)-3-oxo-2,3,5,6,7,8-hexahydro[1,2,4]triazolo[4,3-a]pyridin-5-carboxylat (300 mg, 1.25 mmol) wurde in Acetonitril (4.1 ml) vorgelegt. Caesiumcarbonat (1.02 g, 3.13 mmol) und 2-(Chlormethyl)-5-(trifluormethyl)pyrazin (259 mg, 1.32 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 111 mg (22 % d. Th.) der Titelverbindung erhalten.
LC-MS (Methode 1): Rₜ = 0.83 min; MS (ESlneg): m/z = 457 [M-tBu+H]⁺

### Intermediat 256

### tert-Butyl-(5S)-2-{[3-fluor-2-(trifluormethyl)pyridin-4-yl]methyl}-3-oxo-2,3,5,6,7,8-hexahydro[1,2,4]triazolo[4,3-a]pyridin-5-carboxylat

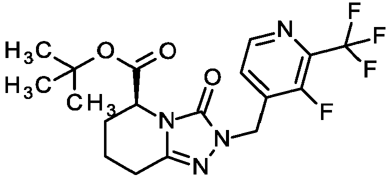

tert-Butyl-(5S)-3-oxo-2,3,5,6,7,8-hexahydro[1,2,4]triazolo[4,3-a]pyridin-5-carboxylat (970 mg, 4.05 mmol) wurde in Acetonitril (20 ml) vorgelegt. Caesiumcarbonat (3.30 g, 10.1 mmol) und 4-(Chlormethyl)-3-fluor-2-(trifluormethyl)pyridin (978 mg, 93 % Reinheit, 4.26 mmol) wurden anschließend zugegeben. Das Reaktionsgemisch wurde nach Rühren für 2 Stunden unter Rückfluss und Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 1.37 g (80 % d. Th.) der Titelverbindung erhalten.
LC-MS (Methode 3): Rₜ = 1.92 min; MS (ESIpos): m/z = 417 [M+H]⁺

### Intermediat 257

### tert-Butyl-(5S)-2-[(1RS)-1-(4-methylphenyl)ethyl]-3-oxo-2,3,5,6,7,8-hexahydro[1,2,4]triazolo[4,3-a]pyridin-5-carboxylat (Diastereomerengemisch, 2 Isomere)

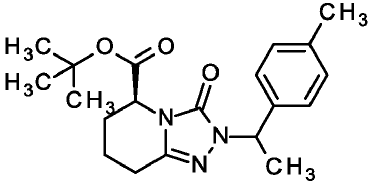

tert-Butyl-(5S)-3-oxo-2,3,5,6,7,8-hexahydro[1,2,4]triazolo[4,3-a]pyridin-5-carboxylat (200 mg, 836 µmol) wurde in Acetonitril (5.5 ml) vorgelegt. Caesiumcarbonat (681 mg, 2.09 mmol) und 1-[(1RS)-1-Chlorethyl]-4-methylbenzol (142 mg, 919 µmol) wurden anschließend zugegeben. Das Reaktionsgemisch wurde nach Rühren über Nacht bei Raumtemperatur mit Wasser, 1 N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 69.8 mg (23 % d. Th.) der Titelverbindung erhalten.
LC-MS (Methode 1): Rₜ = 1.35 min; MS (ESIpos): m/z = 302 [M+H]⁺
¹H-NMR (400 MHz, DMSO-d6) δ [ppm]: 1.322 (0.75), 1.341 (16.00), 1.587 (2.31), 1.605 (2.28), 2.048 (0.49), 2.059 (0.49), 2.266 (4.00), 2.518 (0.52), 4.407 (0.57), 5.271 (0.49), 5.288 (0.48), 7.111 (0.75), 7.131 (1.46), 7.173 (1.69), 7.193 (0.87).

### Intermediat 258

### tert-Butyl-(5S)-2-[2-(4-methylphenyl)ethyl]-3-oxo-2,3,5,6,7,8-hexahydro[1,2,4]triazolo[4,3-a]pyridin-5-carboxylat

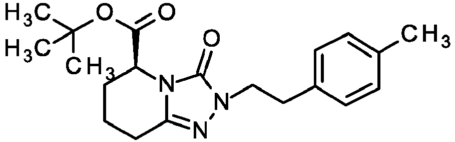

tert-Butyl-(5S)-3-oxo-2,3,5,6,7,8-hexahydro[1,2,4]triazolo[4,3-a]pyridin-5-carboxylat (250 mg, 1.04 mmol) wurde in Acetonitril (8.0 ml) vorgelegt. Caesiumcarbonat (851 mg, 2.61 mmol) und 1-(2-Bromethyl)-4-methylbenzol (180 µl, 1.1 mmol) wurden anschließend zugegeben. Das Reaktionsgemisch wurde nach Rühren über Nacht bei Raumtemperatur mit Wasser, 1 N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 238 mg (64 % d. Th.) der Titelverbindung erhalten.
LC-MS (Methode 1): Rₜ = 1.35 min; MS (ESIpos): m/z = 302 [M+H]⁺
¹H-NMR (400 MHz, DMSO-d6) δ [ppm]: 1.394 (16.00), 2.041 (0.53), 2.051 (0.48), 2.255 (4.71), 2.864 (0.59), 2.883 (1.12), 2.902 (0.64), 3.778 (0.43), 3.783 (0.44), 3.796 (0.81), 3.803 (0.81), 4.373 (0.59), 7.089 (6.23).

### Intermediat 259

### tert-Butyl-(5S)-2-{[3-chlor-4-(trifluormethyl)pyridin-2-yl]methyl}-3-oxo-2,3,5,6,7,8-hexahydro[1,2,4]triazolo[4,3-a]pyridin-5-carboxylat

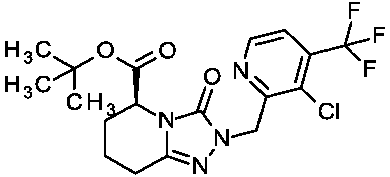

tert-Butyl-(5S)-3-oxo-2,3,5,6,7,8-hexahydro[1,2,4]triazolo[4,3-a]pyridin-5-carboxylat (991 mg, 4.14 mmol) wurde in Acetonitril (19 ml) vorgelegt. Caesiumcarbonat (1.48 g, 4.55 mmol) und 3-Chlor-2-(chlormethyl)-4-(trifluormethyl)pyridin (1.00 g, 4.35 mmol) wurden anschließend zugegeben. Das Reaktionsgemisch wurde nach Rühren über Nacht unter Rückfluss mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 794 mg (42 % d. Th.) der Titelverbindung erhalten.
LC-MS (Methode 1): Rₜ = 1.28 min; MS (ESIpos): m/z = 377 [M-tBu+H]⁺

### Intermediat 260

### tert-Butyl-(5S)-2-{[1-(4-methylphenyl)cyclopropyl]methyl}-3-oxo-2,3,5,6,7,8-hexahydro[1,2,4]triazolo[4,3-a]pyridin-5-carboxylat

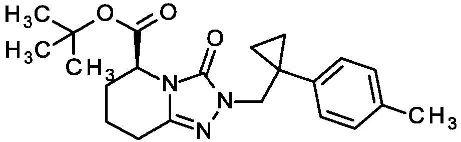

tert-Butyl-(5S)-3-oxo-2,3,5,6,7,8-hexahydro[1,2,4]triazolo[4,3-a]pyridin-5-carboxylat (120 mg, 500 µmol) wurde in Acetonitril (3.0 ml) vorgelegt. Caesiumcarbonat (408 mg, 1.25 mmol) und 1-[1-(Brommethyl)cyclopropyl]-4-methylbenzol (124 mg, 550 µmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 167 mg (90 % Reinheit, 78 % d. Th.) der Titelverbindung erhalten.
LC-MS (Methode 3): Rₜ = 2.13 min; MS (ESIpos): m/z = 384 [M+H]⁺
¹H-NMR (400 MHz, DMSO-d6) δ [ppm]: 0.752 (0.63), 0.772 (0.71), 0.977 (1.55), 1.157 (0.59), 1.175 (1.19), 1.192 (0.62), 1.344 (1.25), 1.378 (16.00), 1.405 (1.99), 1.417 (0.84), 1.988 (2.18), 2.016 (0.51), 2.027 (0.54), 2.037 (0.43), 2.232 (4.19), 2.249 (0.42), 2.519 (0.47), 3.755 (0.54), 3.792 (0.96), 3.866 (1.00), 3.902 (0.51), 4.020 (0.51), 4.038 (0.51), 4.322 (0.42), 4.333 (0.42), 4.337 (0.59), 4.347 (0.44), 7.025 (0.89), 7.045 (1.39), 7.119 (1.76), 7.139 (1.14).

### Intermediat 261

### tert-Butyl-(5S)-3-oxo-2-{[2-(trifluormethyl)-1,8-naphthyridin-3-yl]methyl}-2,3,5,6,7,8-hexahydro[1,2,4]triazolo[4,3-a]pyridin-5-carboxylat

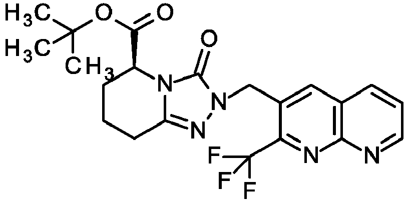

tert-Butyl-(5S)-3-oxo-2,3,5,6,7,8-hexahydro[1,2,4]triazolo[4,3-a]pyridin-5-carboxylat (208 mg, 868 µmol) wurde in Acetonitril (20 ml) vorgelegt. Caesiumcarbonat (1.13 g, 3.47 mmol) und 3-(Chlormethyl)-2-(trifluormethyl)-1,8-naphthyridin (278 mg, 1.13 mmol) wurden anschließend zugegeben. Das Reaktionsgemisch wurde nach Rühren für 48 Stunden bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 146 mg (38 % d. Th.) der Titelverbindung erhalten.
LC-MS (Methode 3): Rₜ = 1.65 min; MS (ESIpos): m/z = 450 [M+H]⁺
¹H-NMR (400 MHz, DMSO-d6) δ [ppm]: 1.421 (16.00), 2.113 (0.64), 2.125 (0.64), 2.665 (0.41), 4.520 (0.73), 5.235 (1.07), 7.820 (0.54), 7.830 (0.56), 7.840 (0.56), 7.851 (0.57), 8.449 (1.25), 8.564 (0.56), 8.569 (0.57), 8.585 (0.55), 8.589 (0.52), 9.245 (0.61), 9.250 (0.64), 9.256 (0.63), 9.261 (0.57).

### Intermediat 262

### tert-Butyl-(5S)-2-[(6-chlorpyridin-3-yl)methyl]-3-oxo-2,3,5,6,7,8-hexahydro[1,2,4]triazolo[4,3-a]pyridin-5-carboxylat

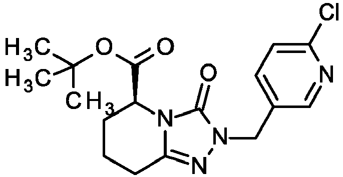

tert-Butyl-(5S)-3-oxo-2,3,5,6,7,8-hexahydro[1,2,4]triazolo[4,3-a]pyridin-5-carboxylat (552 mg, 2.31 mmol) wurde in Acetonitril (11 ml) vorgelegt. Cäsiumcarbonat (1.13 g, 3.46 mmol) und 5-(Brommethyl)-2-chlorpyridin (500 mg, 2.42 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 509 mg (93 % Reinheit, 56 % d. Th.) der Titelverbindung erhalten.
LC-MS (Methode 1): Rₜ = 1.13 min; MS (ESIpos): m/z = 365 [M+H]⁺

### Intermediat 263

### tert-Butyl-(5S)-3-oxo-2-{[6-(trifluormethyl)pyridin-3-yl]methyl}-2,3,5,6,7,8-hexahydro[1,2,4]triazolo[4,3-a]pyridin-5-carboxylat

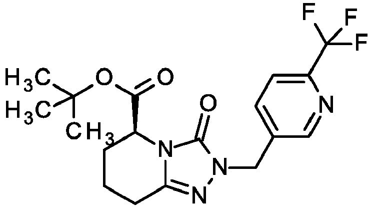

tert-Butyl-(5S)-3-oxo-2,3,5,6,7,8-hexahydro[1,2,4]triazolo[4,3-a]pyridin-5-carboxylat (1.00 g, 4.18 mmol) wurde in Acetonitril (21 ml) vorgelegt. Caesiumcarbonat (2.04 g, 6.27 mmol) und 5-(Chlormethyl)-2-(trifluormethyl)pyridin (858 mg, 4.39 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 1.71 g (100 % d. Th.) der Titelverbindung erhalten.
LC-MS (Methode 3): Rₜ = 1.73 min; MS (ESIpos): m/z = 399 [M+H]⁺

### Intermediat 264

### tert-Butyl-(5S)-2-{[5-chlor-6-(trifluormethyl)pyridin-3-yl]methyl}-3-oxo-2,3,5,6,7,8-hexahydro[1,2,4]triazolo[4,3-a]pyridin-5-carboxylat

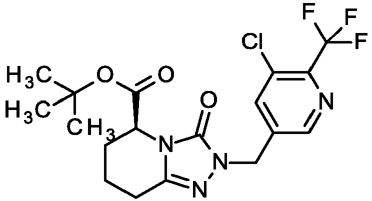

tert-Butyl-(5S)-3-oxo-2,3,5,6,7,8-hexahydro[1,2,4]triazolo[4,3-a]pyridin-5-carboxylat (163 mg, 682 µmol) wurde in Acetonitril (5.0 ml) vorgelegt. Caesiumcarbonat (555 mg, 1.70 mmol) und 3-Chlor-5-(chlormethyl)-2-(trifluormethyl)pyridin (549 mg, 30 % Reinheit, 716 µmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 156 mg (72 % Reinheit, 38 % d. Th.) der Titelverbindung erhalten.
LC-MS (Methode 1): Rₜ = 1.35 min; MS (ESIpos): m/z = 377 [M-tBu+H]⁺

### Intermediat 265

### tert-Butyl-(5S)-2-{2-[3-chlor-5-(trifluormethyl)pyridin-2-yl]ethyl}-3-oxo-2,3,5,6,7,8-hexahydro[1,2,4]triazolo[4,3-a]pyridin-5-carboxylat

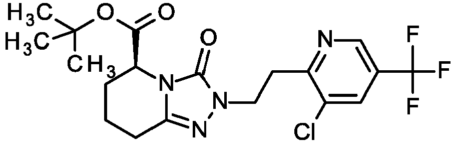

tert-Butyl-(5S)-3-oxo-2,3,5,6,7,8-hexahydro[1,2,4]triazolo[4,3-a]pyridin-5-carboxylat (95.0 mg, 397 µmol) wurde in Acetonitril (3.0 ml) vorgelegt. Caesiumcarbonat (323 mg, 992 µmol) und 3-Chlor-2-(2-chlorethyl)-5-(trifluormethyl)pyridin (113 mg, 94 % Reinheit, 437 µmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 74.5 mg (42 % d. Th.) der Titelverbindung erhalten.
LC-MS (Methode 1): Rₜ = 1.36 min; MS (ESIpos): m/z = 391 [M+H]⁺
¹H-NMR (400 MHz, DMSO-d6) δ [ppm]: -0.008 (2.40), 0.008 (1.82), 1.389 (16.00), 2.034 (0.55), 2.619 (0.43), 2.669 (0.51), 4.058 (0.75), 4.077 (1.16), 4.094 (0.66), 4.361 (0.59), 8.407 (0.85), 8.871 (0.84).

### Intermediat 266

### tert-Butyl-(5S)-3-oxo-2-[3-(trifluormethyl)benzyl]-2,3,5,6,7,8-hexahydro[1,2,4]triazolo[4,3-a]pyridin-5-carboxylat

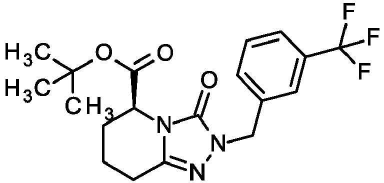

tert-Butyl-(5S)-3-oxo-2,3,5,6,7,8-hexahydro[1,2,4]triazolo[4,3-a]pyridin-5-carboxylat (400 mg, 1.67 mmol) wurde in Acetonitril (6.0 ml) vorgelegt. Caesiumcarbonat (817 mg, 2.51 mmol) und 1-(Brommethyl)-3-(trifluormethyl)benzol (420 mg, 1.76 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 581 mg (87 % d. Th.) der Titelverbindung erhalten.
LC-MS (Methode 3): Rₜ = 1.98 min; MS (ESIpos): m/z = 398 [M+H]⁺
¹H-NMR (400 MHz, DMSO-d6) δ [ppm]: -0.008 (0.69), 0.008 (0.57), 1.384 (16.00), 1.400 (0.78), 2.068 (0.55), 2.077 (0.49), 2.523 (0.41), 4.460 (0.57), 4.949 (1.19), 4.965 (1.15), 7.558 (0.62), 7.575 (0.40), 7.594 (0.64), 7.629 (0.79), 7.652 (0.50).

### Intermediat 267

### tert-Butyl-(5S)-3-oxo-2-(2,4,5-trifluorbenzyl)-2,3,5,6,7,8-hexahydro[1,2,4]triazolo[4,3-a]pyridin-5-carboxylat

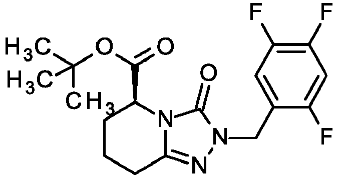

tert-Butyl-(5S)-3-oxo-2,3,5,6,7,8-hexahydro[1,2,4]triazolo[4,3-a]pyridin-5-carboxylat (400 mg, 1.67 mmol) wurde in Acetonitril (10 ml) vorgelegt. Caesiumcarbonat (572 mg, 1.76 mmol) und 1-(Chlormethyl)-2,4,5-trifluorbenzol (317 mg, 1.76 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 523 mg (80 % d. Th.) der Titelverbindung erhalten.
LC-MS (Methode 3): Rₜ = 1.87 min; MS (ESIpos): m/z = 328 [M+H]⁺
¹H-NMR (400 MHz, DMSO-d6) δ [ppm]: 1.175 (0.64), 1.394 (16.00), 1.988 (1.17), 2.060 (0.65), 2.069 (0.65), 2.072 (0.65), 2.627 (0.44), 4.428 (0.44), 4.442 (0.62), 4.864 (2.33).

### Intermediat 268

### tert-Butyl-(5S)-2-(3,4-difluorbenzyl)-3-oxo-2,3,5,6,7,8-hexahydro[1,2,4]triazolo[4,3-a]pyridin-5-carboxylat

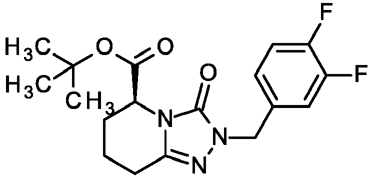

tert-Butyl-(5S)-3-oxo-2,3,5,6,7,8-hexahydro[1,2,4]triazolo[4,3-a]pyridin-5-carboxylat (500 mg, 2.09 mmol) wurde in Acetonitril (10 ml) vorgelegt. Caesiumcarbonat (1.70 g, 5.22 mmol) und 4-(Brommethyl)-1,2-difluorbenzol (454 mg, 2.19 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 900 mg (78 % Reinheit, 92 % d. Th.) der Titelverbindung erhalten.
LC-MS (Methode 1): Rₜ = 0.83 min; MS (ESIpos): m/z = 310 [M-tBu+H]⁺

### Intermediat 269

### tert-Butyl-(5S)-2-[3-chlor-4-(trifluormethyl)benzyl]-3-oxo-2,3,5,6,7,8-hexahydro[1,2,4]triazolo[4,3-a]pyridin-5-carboxylat

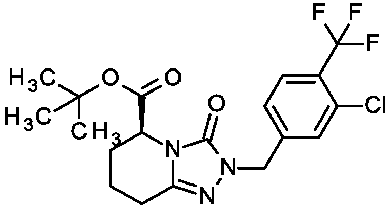

tert-Butyl-(5S)-3-oxo-2,3,5,6,7,8-hexahydro[1,2,4]triazolo[4,3-a]pyridin-5-carboxylat (400 mg, 1.67 mmol) wurde in Acetonitril (8.0 ml) vorgelegt. Caesiumcarbonat (1.36 g, 4.18 mmol) und 2-Chlor-4-(chlormethyl)-1-(trifluormethyl)benzol (402 mg, 1.76 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 190 mg (26 % d. Th.) der Titelverbindung erhalten.
LC-MS (Methode 3): Rₜ = 2.12 min; MS (ESIpos): m/z = 454 [M+Na]⁺
¹H-NMR (400 MHz, DMSO-d6) δ [ppm]: -0.008 (0.90), 0.008 (0.82), 1.390 (16.00), 2.075 (0.55), 4.466 (0.60), 4.970 (1.54), 7.404 (0.46), 7.425 (0.49), 7.584 (0.94), 7.844 (0.81), 7.865 (0.75).

### Intermediat 270

### tert-Butyl-(5S)-2-(3-chlor-4-fluorbenzyl)-3-oxo-2,3,5,6,7,8-hexahydro[1,2,4]triazolo[4,3-a]pyridin-5-carboxylat

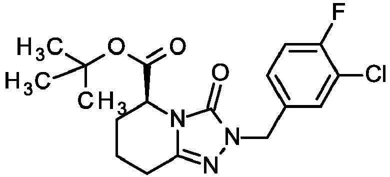

tert-Butyl-(5S)-3-oxo-2,3,5,6,7,8-hexahydro[1,2,4]triazolo[4,3-a]pyridin-5-carboxylat (500 mg, 2.09 mmol) wurde in Acetonitril (10 ml) vorgelegt. Caesiumcarbonat (1.70 g, 5.22 mmol) und 4-(Brommethyl)-2-chlor-1-fluorbenzol (490 mg, 2.19 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 600 mg (75 % d. Th.) der Titelverbindung erhalten.
LC-MS (Methode 4): Rₜ = 1.01 min; MS (ESIpos): m/z = 382 [M+H]⁺

### Intermediat 271

### tert-Butyl-(5S)-2-[3-fluor-4-(trifluormethyl)benzyl]-3-oxo-2,3,5,6,7,8-hexahydro[1,2,4]triazolo[4,3-a]pyridin-5-carboxylat

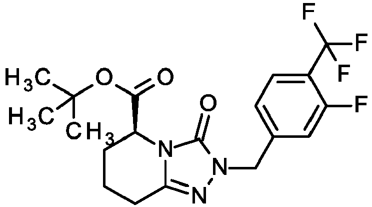

tert-Butyl-(5S)-3-oxo-2,3,5,6,7,8-hexahydro[1,2,4]triazolo[4,3-a]pyridin-5-carboxylat (500 mg, 2.09 mmol) wurde in Acetonitril (10 ml) vorgelegt. Caesiumcarbonat (1.70 g, 5.22 mmol) und 4-(Chlormethyl)-2-fluor-1-(trifluormethyl)benzol (466 mg, 2.19 mmol) wurden anschließend zugegeben. Das Reaktionsgemisch wurde nach Rühren für 72 Stunden bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 707 mg (91 % Reinheit, 74 % d. Th.) der Titelverbindung erhalten.
LC-MS (Methode 1): Rₜ = 0.83 min; MS (ESIpos): m/z = 360 [M-tBu+H]⁺

### Intermediat 272

### tert-Butyl-(5S)-2-{[3-fluor-2-(trifluormethyl)pyridin-4-yl]methyl}-3-oxo-2,3,5,6,7,8-hexahydro[1,2,4]triazolo[4,3-a]pyridin-5-carboxylat

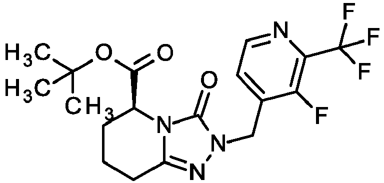

tert-Butyl-(5S)-3-oxo-2,3,5,6,7,8-hexahydro[1,2,4]triazolo[4,3-a]pyridin-5-carboxylat (371 mg, 1.55 mmol) wurde in Acetonitril (7.0 ml) vorgelegt. Caesiumcarbonat (1.11 g, 3.41 mmol) und 4-(Chlormethyl)-3-fluor-2-(trifluormethyl)pyridin (348 mg, 1.63 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 420 mg (65 % d. Th.) der Titelverbindung erhalten.
LC-MS (Methode 3): Rₜ = 1.83 min; MS (ESIpos): m/z = 417 [M+H]⁺
¹H-NMR (400 MHz, DMSO-d6) δ [ppm]: 1.228 (0.06), 1.359 (0.10), 1.388 (16.00), 1.471 (0.11), 1.484 (0.16), 1.497 (0.15), 1.506 (0.16), 1.518 (0.17), 1.531 (0.12), 1.545 (0.15), 1.815 (0.19), 1.825 (0.18), 1.837 (0.16), 1.849 (0.16), 2.068 (0.55), 2.081 (0.45), 2.106 (0.17), 2.114 (0.16), 2.322 (0.04), 2.361 (0.04), 2.568 (0.26), 2.581 (0.27), 2.595 (0.21), 2.630 (0.21), 2.641 (0.36), 2.653 (0.23), 2.672 (0.13), 2.683 (0.17), 2.695 (0.09), 4.447 (0.39), 4.461 (0.60), 4.471 (0.37), 4.942 (0.11), 5.083 (2.35), 7.596 (0.35), 7.609 (0.64), 7.622 (0.35), 8.566 (0.73), 8.578 (0.71).

### Intermediat 273

### tert-Butyl-(5S)-2-{[1-(6-chlorpyridin-2-yl)cyclopropyl]methyl}-3-oxo-2,3,5,6,7,8-hexahydro[1,2,4]triazolo[4,3-a]pyridin-5-carboxylat

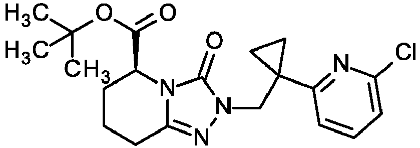

tert-Butyl-(5S)-3-oxo-2,3,5,6,7,8-hexahydro[1,2,4]triazolo[4,3-a]pyridin-5-carboxylat 129 mg, 541 µmol) wurde in Acetonitril (3.0 ml) vorgelegt. Caesiumcarbonat (441 mg, 1.35 mmol) und 2-[1-(Brommethyl)cyclopropyl]-6-chlorpyridin (140 mg, 568 µmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 224 mg (96 % d. Th.) der Titelverbindung erhalten.
LC-MS (Methode 3): Rₜ = 1.94 min; MS (ESIpos): m/z = 405 [M+H]⁺
¹H-NMR (400 MHz, DMSO-d6) δ [ppm]: 1.157 (2.03), 1.161 (1.97), 1.175 (3.58), 1.192 (1.29), 1.253 (0.99), 1.331 (0.71), 1.376 (16.00), 1.406 (3.62), 1.988 (4.27), 2.023 (0.56), 2.036 (0.59), 2.046 (0.41), 2.520 (0.43), 4.020 (0.99), 4.038 (1.01), 4.056 (0.41), 4.096 (2.12), 4.388 (0.40), 4.393 (0.56), 7.245 (0.76), 7.265 (0.83), 7.606 (0.68), 7.625 (0.99), 7.696 (0.72), 7.715 (1.13), 7.735 (0.49).

### Intermediat 274

### tert-Butyl-(5S)-2-{[1-(4-fluorphenyl)cyclopropyl]methyl}-3-oxo-2,3,5,6,7,8-hexahydro[1,2,4]triazolo[4,3-a]pyridin-5-carboxylat

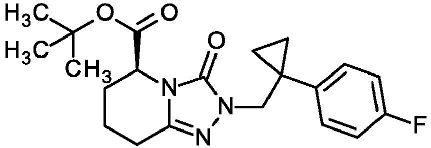

tert-Butyl-(5S)-3-oxo-2,3,5,6,7,8-hexahydro[1,2,4]triazolo[4,3-a]pyridin-5-carboxylat (344 mg, 1.44 mmol) wurde in Acetonitril (10 ml) vorgelegt. Caesiumcarbonat (1.17 g, 3.59 mmol) und 1-[1-(Brommethyl)cyclopropyl]-4-fluorbenzol (346 mg, 1.51 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 557 mg (100 % d. Th.) der Titelverbindung erhalten.
LC-MS (Methode 3): Rₜ = 2.04 min; MS (ESIpos): m/z = 388 [M+H]⁺

### Intermediat 275

### tert-Butyl-(5S)-2-{[1-(4-methoxyphenyl)cyclopropyl]methyl}-3-oxo-2,3,5,6,7,8-hexahydro[1,2,4]triazolo[4,3-a]pyridin-5-carboxylat

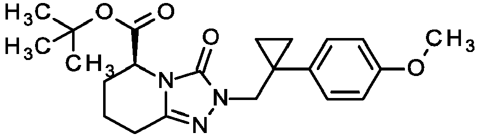

tert-Butyl-(5S)-3-oxo-2,3,5,6,7,8-hexahydro[1,2,4]triazolo[4,3-a]pyridin-5-carboxylat (172 mg, 717 µmol) wurde in Acetonitril (4.8 ml) vorgelegt. Caesiumcarbonat (584 mg, 1.79 mmol) und 1-[1-(Brommethyl)cyclopropyl]-4-methoxybenzol (190 mg, 789 µmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 222 mg (73 % d. Th.) der Titelverbindung erhalten.
LC-MS (Methode 3): Rₜ = 1.95 min; MS (ESIpos): m/z = 344 [M+H]⁺
¹H-NMR (400 MHz, DMSO-d6) δ [ppm]: 0.699 (0.14), 0.724 (0.72), 0.743 (0.79), 0.766 (0.18), 0.839 (0.08), 0.931 (0.17), 0.949 (1.72), 0.971 (0.15), 1.219 (0.07), 1.354 (1.10), 1.380 (16.00), 1.512 (0.20), 1.537 (0.16), 1.780 (0.22), 1.802 (0.20), 1.814 (0.20), 2.019 (0.54), 2.030 (0.56), 2.040 (0.39), 2.327 (0.07), 2.469 (0.14), 2.525 (0.43), 2.595 (0.25), 2.606 (0.42), 2.618 (0.25), 2.637 (0.15), 2.648 (0.21), 2.660 (0.13), 2.709 (0.06), 3.698 (6.61), 3.711 (0.51), 3.726 (0.56), 3.762 (1.00), 3.831 (1.00), 3.867 (0.53), 4.286 (0.06), 4.319 (0.40), 4.334 (0.59), 4.344 (0.39), 6.776 (1.42), 6.798 (1.61), 6.818 (0.10), 6.840 (0.10), 7.142 (1.66), 7.163 (1.47), 7.227 (0.10), 7.248 (0.09).

### Intermediat 276

### tert-Butyl-(5S)-2-{[1-(2,4-difluorphenyl)cyclopropyl]methyl}-3-oxo-2,3,5,6,7,8-hexahydro[1,2,4]triazolo[4,3-a]pyridin-5-carboxylat

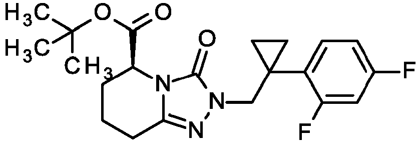

tert-Butyl-(5S)-3-oxo-2,3,5,6,7,8-hexahydro[1,2,4]triazolo[4,3-a]pyridin-5-carboxylat (313 mg, 1.31 mmol) wurde in Acetonitril (8.7 ml) vorgelegt. Caesiumcarbonat (1.06 g, 3.27 mmol) und 1-[1-(Brommethyl)cyclopropyl]-2,4-difluorbenzol (355 mg, 1.44 mmol) wurden anschließend zugegeben. Das Reaktionsgemisch wurde nach Rühren für 72 Stunden bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 256 mg (48 % d. Th.) der Titelverbindung erhalten.
LC-MS (Methode 1): Rₜ = 1.38 min; MS (ESIpos): m/z = 350 [M-tBu]⁺
¹H-NMR (400 MHz, DMSO-d6) δ [ppm]: 0.007 (0.24), 0.747 (0.38), 0.759 (1.23), 0.774 (0.45), 0.996 (0.45), 1.007 (1.20), 1.023 (0.37), 1.375 (16.00), 1.466 (0.16), 1.501 (0.17), 1.527 (0.11), 1.764 (0.19), 1.786 (0.17), 1.798 (0.16), 2.005 (0.56), 2.016 (0.45), 2.433 (0.12), 2.448 (0.15), 2.460 (0.14), 2.475 (0.36), 2.563 (0.22), 2.575 (0.38), 2.586 (0.22), 2.605 (0.13), 2.616 (0.19), 3.707 (0.32), 3.743 (1.13), 3.765 (1.16), 3.801 (0.32), 4.278 (0.39), 4.293 (0.62), 4.303 (0.37), 6.882 (0.18), 6.898 (0.37), 6.903 (0.38), 6.918 (0.20), 6.924 (0.21), 7.082 (0.23), 7.089 (0.24), 7.111 (0.34), 7.133 (0.26), 7.138 (0.45), 7.160 (0.46), 7.177 (0.46), 7.198 (0.21).

### Intermediat 277

### tert-Butyl-(5S)-3-oxo-2-({1-[4-(trifluormethyl)phenyl]cyclopropyl}methyl)-2,3,5,6,7,8-hexahydro[1,2,4]triazolo[4,3-a]pyridin-5-carboxylat

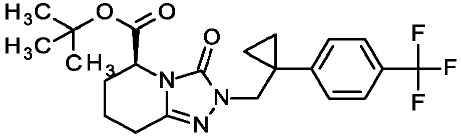

tert-Butyl-(5S)-3-oxo-2,3,5,6,7,8-hexahydro[1,2,4]triazolo[4,3-a]pyridin-5-carboxylat (80.4 mg, 336 µmol) wurde in Acetonitril (2.0 ml) vorgelegt. Caesiumcarbonat (274 mg, 840 µmol) und 1-[1-(Brommethyl)cyclopropyl]-4-(trifluormethyl)benzol (110 mg, 94 % Reinheit, 370 µmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 113 mg (74 % d. Th.) der Titelverbindung erhalten.
LC-MS (Methode 1): Rₜ = 1.47 min; MS (ESIpos): m/z = 382 [M-tBu]⁺

### Intermediat 278

### tert-Butyl-(5S)-2-(2,4-difluorbenzyl)-3-oxo-2,3,5,6,7,8-hexahydro[1,2,4]triazolo[4,3-a]pyridin-5-carboxylat

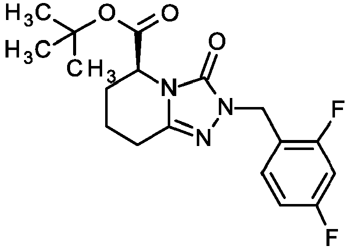

tert-Butyl-(5S)-3-oxo-2,3,5,6,7,8-hexahydro[1,2,4]triazolo[4,3-a]pyridin-5-carboxylat (300 mg, 1.25 mmol) wurde in Acetonitril (8.0 ml) vorgelegt. Caesiumcarbonat (1.02 g, 3.13 mmol) und 1-(Chlormethyl)-2,4-difluorbenzol (214 mg, 1.32 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 423 mg (92 % d. Th.) der Titelverbindung erhalten.
LC-MS (Methode 3): Rₜ = 1.81 min; MS (ESIpos): m/z = 366 [M+H]⁺

### Intermediat 279

### tert-Butyl-(5S)-2-(2-chlor-4-fluorbenzyl)-3-oxo-2,3,5,6,7,8-hexahydro[1,2,4]triazolo[4,3-a]pyridin-5-carboxylat

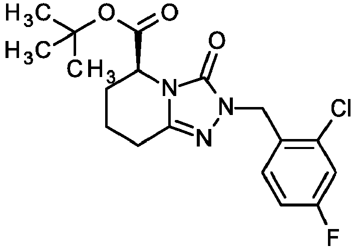

tert-Butyl-(5S)-3-oxo-2,3,5,6,7,8-hexahydro[1,2,4]triazolo[4,3-a]pyridin-5-carboxylat (250 mg, 1.04 mmol) wurde in Acetonitril (6.0 ml) vorgelegt. Caesiumcarbonat (851 mg, 2.61 mmol) und 1-(Brommethyl)-2-chlor-4-fluorbenzol (245 mg, 1.10 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 370 mg (88 % d. Th.) der Titelverbindung erhalten.
LC-MS (Methode 3): Rₜ = 0.83 min; MS (ESIpos): m/z = 326 [M-tBu+H]⁺

### Intermediat 280

### tert-Butyl-(5S)-2-[(5-chlor-6-methoxypyridin-3-yl)methyl]-3-oxo-2,3,5,6,7,8-hexahydro[1,2,4]triazolo[4,3-a]pyridin-5-carboxylat

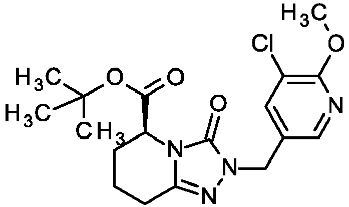

tert-Butyl-(5S)-3-oxo-2,3,5,6,7,8-hexahydro[1,2,4]triazolo[4,3-a]pyridin-5-carboxylat (664 mg, 2.78 mmol) wurde in Acetonitril (10 ml) vorgelegt. Caesiumcarbonat (1.36 g, 4.17 mmol) und 3-Chlor-5-(chlormethyl)-2-methoxypyridin (560 mg, 2.92 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 718 mg (90 % Reinheit, 59 % d. Th.) der Titelverbindung erhalten.
LC-MS (Methode 1): Rₜ = 1.25 min; MS (ESIpos): m/z = 395 [M+H]⁺

### Intermediat 281

### tert-Butyl-(5S)-2-(4-fluorbenzyl)-3-oxo-2,3,5,6,7,8-hexahydro[1,2,4]triazolo[4,3-a]pyridin-5-carboxylat

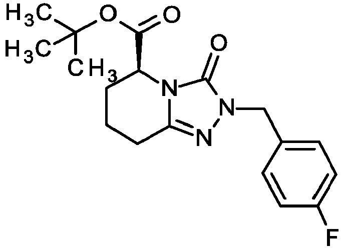

tert-Butyl-(5S)-3-oxo-2,3,5,6,7,8-hexahydro[1,2,4]triazolo[4,3-a]pyridin-5-carboxylat (250 mg, 1.04 mmol) wurde in Acetonitril (6.0 ml) vorgelegt. Caesiumcarbonat (851 mg, 2.61 mmol) und 1-(Brommethyl)-4-fluorbenzol (207 mg, 1.10 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 299 mg (82 % d. Th.) der Titelverbindung erhalten.
LC-MS (Methode 3): Rₜ = 1.75 min; MS (ESIpos): m/z = 348 [M+H]⁺
¹H-NMR (400 MHz, DMSO-d6) δ [ppm]: 1.334 (0.45), 1.392 (16.00), 2.057 (0.50), 2.067 (0.46), 2.073 (0.41), 4.435 (0.57), 4.823 (2.34), 7.143 (0.57), 7.165 (1.32), 7.188 (0.83), 7.276 (0.70), 7.290 (0.77), 7.298 (0.62), 7.312 (0.51).

### Intermediat 282

### tert-Butyl-(5S)-2-[(6-chlorpyridin-2-yl)methyl]-3-oxo-2,3,5,6,7,8-hexahydro[1,2,4]triazolo[4,3-a]pyridin-5-carboxylat

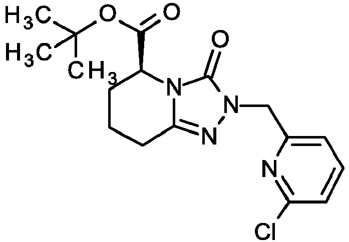

tert-Butyl-(5S)-3-oxo-2,3,5,6,7,8-hexahydro[1,2,4]triazolo[4,3-a]pyridin-5-carboxylat (266 mg, 1.11 mmol) wurde in Acetonitril (10 ml) vorgelegt. Caesiumcarbonat (904 mg, 2.77 mmol) und 2-Chlor-6-(chlormethyl)pyridin (189 mg, 1.17 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 372 mg (92 % d. Th.) der Titelverbindung erhalten.
LC-MS (Methode 3): Rₜ = 1.65 min; MS (ESIpos): m/z = 365 [M+H]⁺
¹H-NMR (400 MHz, DMSO-d6) δ [ppm]: -0.009 (0.09), 0.007 (0.09), 0.786 (0.01), 0.805 (0.03), 0.828 (0.02), 0.846 (0.04), 0.865 (0.02), 1.139 (0.04), 1.156 (0.65), 1.174 (1.28), 1.191 (0.66), 1.248 (0.07), 1.372 (0.24), 1.409 (16.00), 1.456 (0.05), 1.469 (0.08), 1.482 (0.12), 1.495 (0.17), 1.507 (0.16), 1.517 (0.17), 1.529 (0.18), 1.542 (0.13), 1.556 (0.10), 1.566 (0.12), 1.823 (0.21), 1.832 (0.19), 1.844 (0.18), 1.856 (0.17), 1.867 (0.13), 1.987 (2.34), 2.044 (0.07), 2.055 (0.08), 2.072 (0.92), 2.081 (0.55), 2.090 (0.52), 2.098 (0.37), 2.109 (0.21), 2.116 (0.19), 2.124 (0.17), 2.136 (0.06), 2.145 (0.05), 2.152 (0.05), 2.160 (0.04), 2.327 (0.02), 2.365 (0.02), 2.573 (0.27), 2.585 (0.28), 2.599 (0.22), 2.630 (0.22), 2.641 (0.38), 2.653 (0.24), 2.672 (0.13), 2.683 (0.18), 2.695 (0.10), 2.808 (0.01), 4.002 (0.19), 4.020 (0.56), 4.037 (0.55), 4.055 (0.18), 4.451 (0.39), 4.461 (0.41), 4.465 (0.58), 4.475 (0.38), 4.737 (0.02), 4.771 (0.08), 4.872 (0.06), 4.915 (2.29), 4.957 (0.06), 5.154 (0.03), 5.169 (0.03), 5.392 (0.02), 5.405 (0.02), 7.163 (0.72), 7.182 (0.77), 7.332 (0.02), 7.350 (0.02), 7.437 (0.62), 7.456 (0.69), 7.482 (0.03), 7.502 (0.03), 7.515 (0.01), 7.558 (0.01), 7.577 (0.01), 7.842 (0.58), 7.861 (1.02), 7.881 (0.51), 7.904 (0.03), 7.918 (0.02).

### Intermediat 283

### Methyl-(5S)-2-[2-(4-fluorphenyl)ethyl]-3-oxo-2,3,5,6,7,8-hexahydro[1,2,4]triazolo[4,3-a]pyridin-5-carboxylat

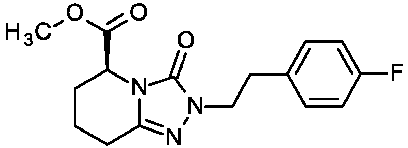

Methyl-(5S)-3-oxo-2,3,5,6,7,8-hexahydro[1,2,4]triazolo[4,3-a]pyridin-5-carboxylat (224 mg, 1.13 mmol) wurde in Acetonitril (8.0 ml) vorgelegt. Caesiumcarbonat (923 mg, 2.83 mmol) und 1-(2-Chlorethyl)-4-fluorbenzol (189 mg, 1.19 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 221 mg (61 % d. Th.) der Titelverbindung erhalten.
LC-MS (Methode 3): Rₜ = 1.43 min; MS (ESIpos): m/z = 320 [M+H]⁺
¹H-NMR (400 MHz, DMSO-d6) δ [ppm]: -0.149 (1.81), -0.008 (16.00), 0.008 (13.92), 0.146 (1.74), 1.157 (0.60), 1.175 (1.34), 1.193 (0.74), 1.988 (2.48), 2.073 (0.80), 2.328 (1.81), 2.366 (1.21), 2.523 (5.29), 2.602 (0.54), 2.670 (1.94), 2.710 (1.27), 2.910 (0.54), 2.929 (0.87), 2.947 (0.54), 3.677 (5.42), 3.687 (1.41), 3.808 (0.67), 3.826 (1.00), 3.844 (0.60), 4.021 (0.60), 4.038 (0.60), 4.533 (0.47), 7.067 (0.47), 7.090 (1.14), 7.112 (0.74), 7.208 (0.67), 7.222 (0.74), 7.243 (0.54).

### Intermediat 284

### tert-Butyl-(5S)-2-(4-methoxybenzyl)-3-oxo-2,3,5,6,7,8-hexahydro[1,2,4]triazolo[4,3-a]pyridin-5-carboxylat

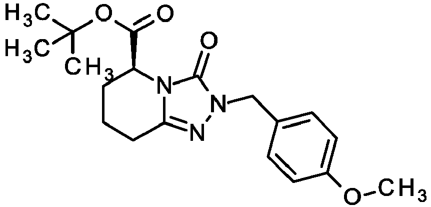

tert-Butyl-(5S)-3-oxo-2,3,5,6,7,8-hexahydro[1,2,4]triazolo[4,3-a]pyridin-5-carboxylat (100 mg, 418 µmol) wurde in Acetonitril (4.0 ml) vorgelegt. Caesiumcarbonat (272 mg, 836 µmol) und 1-(Brommethyl)-4-methoxybenzol (88.2 mg, 439 µmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 129 mg (90 % Reinheit, 77 % d. Th.) der Titelverbindung erhalten.
LC-MS (Methode 1): Rₜ = 1.21 min; MS (ESIpos): m/z = 304 [M-tBu+H]⁺

### Intermediat 285

### tert-Butyl-(5S)-2-[(5-chlor-3-fluorpyridin-2-yl)methyl]-3-oxo-2,3,5,6,7,8-hexahydro[1,2,4]triazolo[4,3-a]pyridin-5-carboxylat

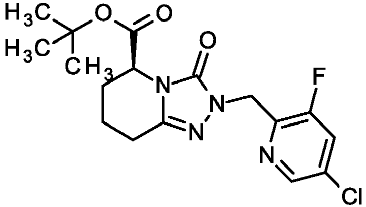

tert-Butyl-(5S)-3-oxo-2,3,5,6,7,8-hexahydro[1,2,4]triazolo[4,3-a]pyridin-5-carboxylat (300 mg, 1.25 mmol) wurde in Acetonitril (12 ml, 230 mmol) vorgelegt. Caesiumcarbonat (817 mg, 2.51 mmol) und 5-Chlor-2-(chlormethyl)-3-fluorpyridin (237 mg, 1.32 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 465 mg (95 % d. Th.) der Titelverbindung erhalten.
LC-MS (Methode 4): Rₜ = 0.88 min; MS (ESIpos): m/z = 383 [M+H]⁺

### Intermediat 286

### tert-Butyl-(5S)-2-[(5-fluorpyridin-2-yl)methyl]-3-oxo-2,3,5,6,7,8-hexahydro[1,2,4]triazolo[4,3-a]pyridin-5-carboxylat

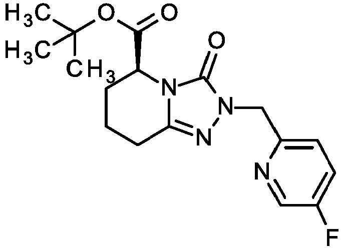

tert-Butyl-(5S)-3-oxo-2,3,5,6,7,8-hexahydro[1,2,4]triazolo[4,3-a]pyridin-5-carboxylat (275 mg, 1.15 mmol) wurde in Acetonitril (10 ml) vorgelegt. Caesiumcarbonat (936 mg, 2.87 mmol) und 2-(Chlormethyl)-5-fluorpyridinhydrochlorid (220 mg, 1.21 mmol) wurden anschließend zugegeben. Das Reaktionsgemisch wurde nach Rühren für 72 Stunden bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 345 mg (83 % d. Th.) der Titelverbindung erhalten.
LC-MS (Methode 3): Rₜ = 1.45 min; MS (ESIpos): m/z = 349 [M+H]⁺
¹H-NMR (400 MHz, DMSO-d6) δ [ppm]: 1.156 (0.20), 1.174 (0.36), 1.191 (0.20), 1.353 (0.26), 1.408 (16.00), 1.487 (0.22), 1.499 (0.22), 1.510 (0.24), 1.521 (0.25), 1.534 (0.19), 1.814 (0.28), 1.824 (0.28), 1.836 (0.24), 1.847 (0.24), 1.858 (0.18), 1.987 (0.66), 2.049 (0.18), 2.072 (0.93), 2.083 (0.69), 2.107 (0.24), 2.115 (0.20), 2.557 (0.30), 2.570 (0.30), 2.584 (0.24), 2.617 (0.29), 2.628 (0.49), 2.639 (0.29), 2.670 (0.26), 4.440 (0.45), 4.453 (0.67), 4.465 (0.42), 4.928 (1.68), 4.934 (1.63), 7.255 (0.43), 7.266 (0.44), 7.277 (0.49), 7.288 (0.46), 7.701 (0.26), 7.708 (0.25), 7.723 (0.49), 7.730 (0.47), 7.745 (0.24), 7.752 (0.24), 8.513 (0.78), 8.519 (0.73).

### Intermediat 287

### tert-Butyl-(5S)-2-[2-(4-fluorphenyl)ethyl]-3-oxo-2,3,5,6,7,8-hexahydro[1,2,4]triazolo[4,3-a]pyridin-5-carboxylat

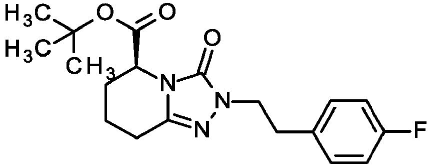

tert-Butyl-(5S)-3-oxo-2,3,5,6,7,8-hexahydro[1,2,4]triazolo[4,3-a]pyridin-5-carboxylat (250 mg, 1.04 mmol) wurde in Acetonitril (10 ml) vorgelegt. Caesiumcarbonat (851 mg, 2.61 mmol) und 1-(2-Bromethyl)-4-fluorbenzol (223 mg, 1.10 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 340 mg (83 % d. Th.) der Titelverbindung erhalten.
LC-MS (Methode 4): Rₜ = 0.98 min; MS (ESIpos): m/z = 362 [M+H]⁺
¹H-NMR (400 MHz, DMSO-d6) δ [ppm]: 1.364 (0.43), 1.391 (16.00), 1.406 (2.02), 1.414 (0.60), 2.039 (0.72), 2.049 (0.64), 2.638 (0.44), 2.909 (0.64), 2.927 (1.21), 2.946 (0.68), 3.807 (0.87), 3.825 (1.29), 3.843 (0.76), 4.352 (0.47), 4.366 (0.64), 4.376 (0.41), 7.062 (0.61), 7.084 (1.28), 7.106 (0.77), 7.219 (0.78), 7.233 (0.93), 7.240 (0.73), 7.254 (0.57).

### Intermediat 288

### tert-Butyl-(5S)-2-[(5-chlorpyridin-3-yl)methyl]-3-oxo-2,3,5,6,7,8-hexahydro[1,2,4]triazolo[4,3-a]pyridin-5-carboxylat

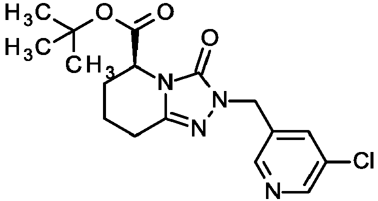

tert-Butyl-(5S)-3-oxo-2,3,5,6,7,8-hexahydro[1,2,4]triazolo[4,3-a]pyridin-5-carboxylat (300 mg, 1.25 mmol) wurde in Acetonitril (10 ml) vorgelegt. Caesiumcarbonat (1.02 g, 3.13 mmol) und 3-Chlor-5-(chlormethyl)pyridin (213 mg, 1.32 mmol) wurden anschließend zugegeben. Das Reaktionsgemisch wurde nach Rühren über Nacht bei Raumtemperatur erneut mit 3-Chlor-5-(chlormethyl)pyridin (213 mg, 1.32 mmol) versetzt und für 4 Stunden bei 60°C gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 450 mg (98 % d. Th.) der Titelverbindung erhalten.
LC-MS (Methode 4): Rₜ = 0.84 min; MS (ESIpos): m/z = 365 [M+H]⁺

### Intermediat 289

### tert-Butyl-(5S)-2-(4-chlor-3-fluorbenzyl)-3-oxo-2,3,5,6,7,8-hexahydro[1,2,4]triazolo[4,3-a]pyridin-5-carboxylat

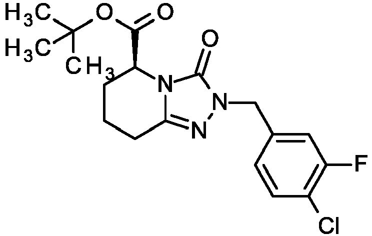

tert-Butyl-(5S)-3-oxo-2,3,5,6,7,8-hexahydro[1,2,4]triazolo[4,3-a]pyridin-5-carboxylat (300 mg, 1.25 mmol) wurde in Acetonitril (5.0 ml) vorgelegt. Caesiumcarbonat (449 mg, 1.38 mmol) und 4-(Brommethyl)-1-chlor-2-fluorbenzol (294 mg, 1.32 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 363 mg (76 % d. Th.) der Titelverbindung erhalten.
LC-MS (Methode 8): Rₜ = 3.17 min; MS (ESIpos): m/z = 382 [M+H]⁺

### Intermediat 290

### tert-Butyl-(5S)-2-[(5-methoxypyridin-2-yl)methyl]-3-oxo-2,3,5,6,7,8-hexahydro[1,2,4]triazolo[4,3-a]pyridin-5-carboxylat

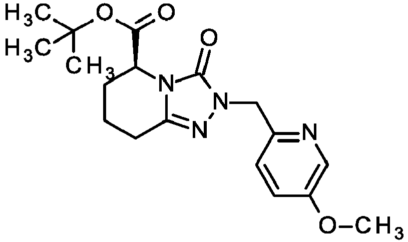

tert-Butyl-(5S)-3-oxo-2,3,5,6,7,8-hexahydro[1,2,4]triazolo[4,3-a]pyridin-5-carboxylat (200 mg, 836 µmol) wurde in Acetonitril (1.3 ml) vorgelegt. Caesiumcarbonat (545 mg, 1.67 mmol) und 2-(Chlormethyl)-5-methoxypyridin (138 mg, 878 µmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 196 mg (64 % d. Th.) der Titelverbindung erhalten.
LC-MS (Methode 3): Rₜ = 1.52 min; MS (ESIpos): m/z = 361 [M+H]⁺

### Intermediat 291

### tert-Butyl-(5S)-2-[(3,5-dichlorpyridin-2-yl)methyl]-3-oxo-2,3,5,6,7,8-hexahydro[1,2,4]triazolo[4,3-a]pyridin-5-carboxylat

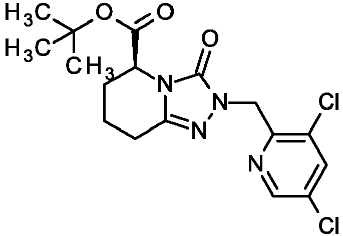

tert-Butyl-(5S)-3-oxo-2,3,5,6,7,8-hexahydro[1,2,4]triazolo[4,3-a]pyridin-5-carboxylat (300 mg, 1.25 mmol) wurde in Acetonitril (5.0 ml) vorgelegt. Caesiumcarbonat (449 mg, 1.38 mmol) und 3,5-Dichlor-2-(chlormethyl)pyridin (271 mg, 1.38 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 260 mg (52 % d. Th.) der Titelverbindung erhalten.
LC-MS (Methode 4): Rₜ = 0.93 min; MS (ESIpos): m/z = 399 [M+H]⁺

### Intermediat 292

### tert-Butyl-(5S)-2-[2-(4-methoxyphenyl)ethyl]-3-oxo-2,3,5,6,7,8-hexahydro[1,2,4]triazolo[4,3-a]pyridin-5-carboxylat

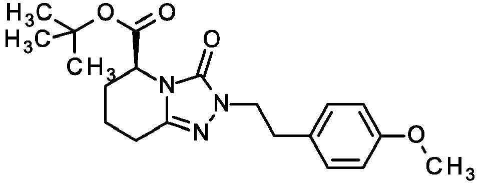

tert-Butyl-(5S)-3-oxo-2,3,5,6,7,8-hexahydro[1,2,4]triazolo[4,3-a]pyridin-5-carboxylat (250 mg, 1.04 mmol) wurde in Acetonitril (10 ml) vorgelegt. Caesiumcarbonat (851 mg, 2.61 mmol) und 1-(2-Bromethyl)-4-methoxybenzol (170 µl, 1.1 mmol) wurden anschließend zugegeben. Das Reaktionsgemisch wurde nach Rühren für 4 Stunden bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 369 mg (87 % d. Th.) der Titelverbindung erhalten.
LC-MS (Methode 3): Rₜ = 1.86 min; MS (ESIpos): m/z = 374 [M+H]⁺
¹H-NMR (400 MHz, DMSO-d6) δ [ppm]: 1.395 (16.00), 1.405 (1.18), 1.417 (0.64), 2.043 (0.56), 2.052 (0.51), 2.844 (0.59), 2.863 (1.09), 2.882 (0.63), 3.713 (6.85), 3.727 (0.83), 3.764 (0.44), 3.768 (0.48), 3.781 (0.80), 3.788 (0.83), 3.801 (0.41), 4.359 (0.41), 4.373 (0.62), 6.827 (1.37), 6.848 (1.63), 7.113 (1.43), 7.135 (1.23).

### Intermediat 293

### tert-Butyl-(5S)-3-oxo-2-{[3-(trifluormethyl)-1,2,4-oxadiazol-5-yl]methyl}-2,3,5,6,7,8-hexahydro[1,2,4]triazolo[4,3-a]pyridin-5-carboxylat

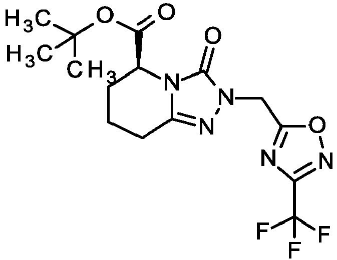

tert-Butyl-(5S)-3-oxo-2,3,5,6,7,8-hexahydro[1,2,4]triazolo[4,3-a]pyridin-5-carboxylat (300 mg, 1.25 mmol) wurde in Acetonitril (10 ml) gelöst und bei Raumtemperatur mit Caesiumcarbonat (1.02 g, 3.13 mmol) versetzt. Das Reaktionsgemisch wurde über Nacht bei Raumtemperatur und 1 Stunde bei 50°C gerührt. Es wurde erneut 5-(Chlormethyl)-3-(trifluormethyl)-1,2,4-oxadiazol (258 mg, 95 % Reinheit, 1.32 mmol) zugegeben und 5 Stunden bei 50°C und über Nacht bei Raumtemperatur gerührt. Es wurde erneut 5-(Chlormethyl)-3-(trifluormethyl)-1,2,4-oxadiazol (94 mg, 95 % Reinheit, 0.48 mmol) zugegben und über Nacht bei 50°C gerührt. Es wurde erneut 5-(Chlormethyl)-3-(trifluormethyl)-1,2,4-oxadiazol (94 mg, 95 % Reinheit, 0.48 mmol) zugegeben und 5 Stunden bei 50°C gerührt. Das Lösungsmittel wurde unter Vakuum entfernt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 322 mg (66 % d. Th.) der Titelverbindung erhalten.
LC-MS (Methode 3): Rₜ = 1.82 min; MS (ESIpos): m/z = 334 [M-tBu]⁺

### Intermediat 294

### tert-Butyl-(5S)-2-[(1-methyl-1H-pyrazolo[3,4-b]pyridin-3-yl)methyl]-3-oxo-2,3,5,6,7,8-hexahydro[1,2,4]triazolo[4,3-a]pyridin-5-carboxylat

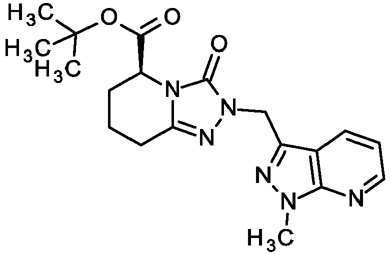

tert-Butyl-(5S)-3-oxo-2,3,5,6,7,8-hexahydro[1,2,4]triazolo[4,3-a]pyridin-5-carboxylat (199 mg, 833 µmol) wurde in Acetonitril (7.0 ml) vorgelegt. Caesiumcarbonat (815 mg, 2.50 mmol) und 3-(Chlormethyl)-1-methyl-1H-pyrazolo[3,4-b]pyridin (167 mg, 917 µmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 203 mg (88 % Reinheit, 56 % d. Th.) der Titelverbindung erhalten.
LC-MS (Methode 4): Rₜ = 0.78 min; MS (ESIpos): m/z = 385 [M+H]⁺
¹H-NMR (400 MHz, DMSO-d6) δ [ppm]: 0.008 (1.19), 1.175 (0.42), 1.372 (16.00), 1.406 (0.56), 1.988 (0.75), 2.033 (0.53), 2.041 (0.56), 2.524 (0.57), 2.579 (0.42), 4.007 (0.86), 4.028 (5.82), 4.433 (0.40), 4.443 (0.43), 4.448 (0.59), 5.124 (1.50), 5.142 (1.48), 7.178 (0.58), 7.189 (0.61), 7.198 (0.64), 7.209 (0.62), 8.193 (0.63), 8.197 (0.64), 8.213 (0.61), 8.217 (0.60), 8.546 (0.68), 8.550 (0.68), 8.557 (0.64), 8.561 (0.60).

### Intermediat 295

### tert-Butyl-(5S)-2-[(3,5-difluorpyridin-2-yl)methyl]-3-oxo-2,3,5,6,7,8-hexahydro[1,2,4]triazolo[4,3-a]pyridin-5-carboxylat

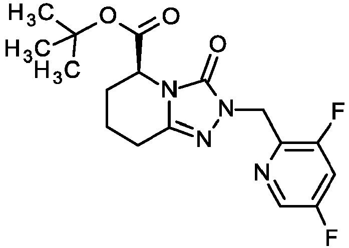

tert-Butyl-(5S)-3-oxo-2,3,5,6,7,8-hexahydro[1,2,4]triazolo[4,3-a]pyridin-5-carboxylat (258 mg, 1.08 mmol) wurde in Acetonitril (10 ml) vorgelegt. Caesiumcarbonat (879 mg, 2.70 mmol) und 2-(Chlormethyl)-3,5-difluorpyridin (218 mg, 89 % Reinheit, 1.19 mmol) wurden anschließend zugegeben. Das Reaktionsgemisch wurde für 4 Stunden bei Raumtemperatur, 8 Stunden bei 50°C und über Nacht bei Raumtemperatur gerührt und anschließend mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 90.1 mg (22 % d. Th.) der Titelverbindung erhalten.
LC-MS (Methode 1): Rₜ = 1.10 min; MS (ESIpos): m/z = 312 [M+H]⁺
¹H-NMR (400 MHz, DMSO-d6) δ [ppm]: 1.404 (16.00), 2.048 (0.56), 2.059 (0.56), 2.517 (0.51), 2.588 (0.42), 4.411 (0.42), 4.421 (0.44), 4.426 (0.58), 4.974 (0.83), 4.977 (0.82), 4.989 (0.82), 8.461 (0.78), 8.467 (0.74).

### Intermediat 296

### tert-Butyl-(5S)-2-[(3-chlorpyridin-2-yl)methyl]-3-oxo-2,3,5,6,7,8-hexahydro[1,2,4]triazolo[4,3-a]pyridin-5-carboxylat

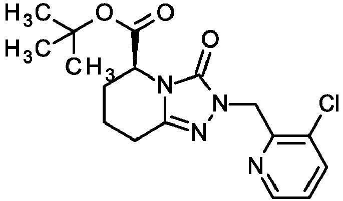

tert-Butyl-(5S)-3-oxo-2,3,5,6,7,8-hexahydro[1,2,4]triazolo[4,3-a]pyridin-5-carboxylat (352 mg, 1.47 mmol) wurde in Acetonitril (10 ml) vorgelegt. Caesiumcarbonat (1.20 g, 3.68 mmol) und 3-Chlor-2-(chlormethyl)pyridin (262 mg, 1.62 mmol) wurden anschließend zugegeben. Das Reaktionsgemisch wurde für 4 Stunden bei Raumtemperatur, 4 Stunden bei 50°C und über Nacht bei Raumtemperatur gerührt und anschließend mit Wasser und Ethylacetat versetzt.. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 269 mg (88 % Reinheit, 44 % d. Th.) der Titelverbindung erhalten.
LC-MS (Methode 1): Rₜ = 1.10 min; MS (ESIpos): m/z = 311 [M+H]⁺

### Intermediat 297

### tert-Butyl-(5S)-3-oxo-2-{[6-(trifluormethyl)pyridin-2-yl]methyl}-2,3,5,6,7,8-hexahydro[1,2,4]triazolo[4,3-a]pyridin-5-carboxylat

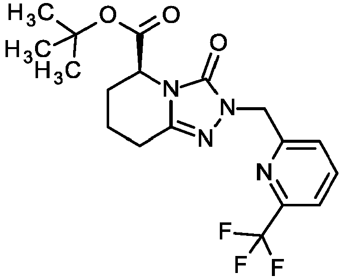

tert-Butyl-(5S)-3-oxo-2,3,5,6,7,8-hexahydro[1,2,4]triazolo[4,3-a]pyridin-5-carboxylat (176 mg, 737 µmol) wurde in Acetonitril (5.0 ml, 95 mmol) vorgelegt. Caesiumcarbonat (601 mg, 1.84 mmol) und 2-(Chlormethyl)-6-(trifluormethyl)pyridin (159 mg, 811 µmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 256 mg (88 % Reinheit, 76 % d. Th.) der Titelverbindung erhalten.
LC-MS (Methode 4): Rₜ = 0.94 min; MS (ESIpos): m/z = 399 [M+H]⁺

### Intermediat 298

### tert-Butyl-(5S)-3-oxo-2-{[5-(trifluormethyl)-1,3,4-oxadiazol-2-yl]methyl}-2,3,5,6,7,8-hexahydro[1,2,4]triazolo[4,3-a]pyridin-5-carboxylat

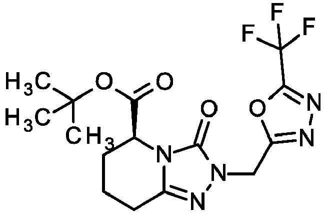

tert-Butyl-(5S)-3-oxo-2,3,5,6,7,8-hexahydro[1,2,4]triazolo[4,3-a]pyridin-5-carboxylat (300 mg, 1.25 mmol) wurde in Acetonitril (5.0 ml) vorgelegt. Caesiumcarbonat (817 mg, 2.51 mmol) und 2-(Chlormethyl)-5-(trifluormethyl)-1,3,4-oxadiazol (257 mg, 1.38 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 735 mg (66 % Reinheit, 99 % d. Th.) der Titelverbindung erhalten.
LC-MS (Methode 1): Rₜ = 1.18 min; MS (ESIpos): m/z = 334 [M-tBu+H]⁺

### Intermediat 299

### tert-Butyl-(5S)-2-{[1-ethyl-3-(trifluormethyl)-1H-pyrazol-5-yl]methyl}-3-oxo-2,3,5,6,7,8-hexahydro[1,2,4]triazolo[4,3-a]pyridin-5-carboxylat

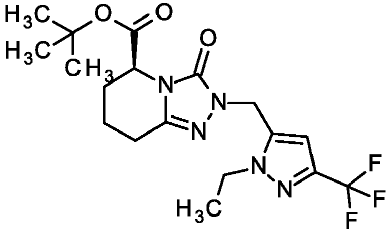

tert-Butyl-(5S)-3-oxo-2,3,5,6,7,8-hexahydro[1,2,4]triazolo[4,3-a]pyridin-5-carboxylat (174 mg, 725 µmol) wurde in Acetonitril (3.0 ml) vorgelegt. Caesiumcarbonat (473 mg, 1.45 mmol) und 5-(Chlormethyl)-1-ethyl-3-(trifluormethyl)-1H-pyrazol (170 mg, 798 µmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 283 mg (89 % Reinheit, 84 % d. Th.) der Titelverbindung erhalten.
LC-MS (Methode 1): Rₜ = 1.30 min; MS (ESIpos): m/z = 360 [M+H]⁺

### Intermediat 300

### tert-Butyl-(5S)-2-[(5-chlorpyridin-2-yl)methyl]-3-oxo-2,3,5,6,7,8-hexahydro[1,2,4]triazolo[4,3-a]pyridin-5-carboxylat

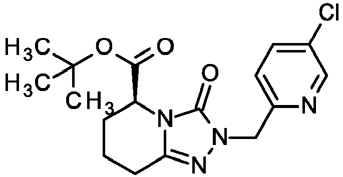

tert-Butyl-(5S)-3-oxo-2,3,5,6,7,8-hexahydro[1,2,4]triazolo[4,3-a]pyridin-5-carboxylat (300 mg, 1.25 mmol) wurde in Acetonitril (5.0 ml) vorgelegt. Caesiumcarbonat (817 mg, 2.51 mmol) und 5-Chlor-2-(chlormethyl)pyridin (223 mg, 1.38 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 450 mg (98 % d. Th.) der Titelverbindung erhalten.
LC-MS (Methode 3): Rₜ = 1.61 min; MS (ESIpos): m/z = 365 [M+H]⁺

### Intermediat 301

### tert-Butyl-(5S)-3-oxo-2-{[2-(trifluormethyl)-1,3-thiazol-4-yl]methyl}-2,3,5,6,7,8-hexahydro[1,2,4]triazolo[4,3-a]pyridin-5-carboxylat

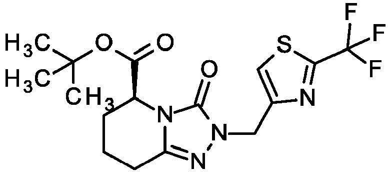

tert-Butyl-(5S)-3-oxo-2,3,5,6,7,8-hexahydro[1,2,4]triazolo[4,3-a]pyridin-5-carboxylat (300 mg, 1.25 mmol) wurde in Acetonitril (10 ml) vorgelegt. Caesiumcarbonat (817 mg, 2.51 mmol) und 4-(Chlormethyl)-2-(trifluormethyl)-1,3-thiazol (276 mg, 96 % Reinheit, 1.32 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 500 mg (98 % d. Th.) der Titelverbindung erhalten.

### Intermediat 302

### tert-Butyl-(5S)-2-{[1-methyl-5-(trifluormethyl)-1H-pyrazol-3-yl]methyl}-3-oxo-2,3,5,6,7,8-hexahydro[1,2,4]triazolo[4,3-a]pyridin-5-carboxylat

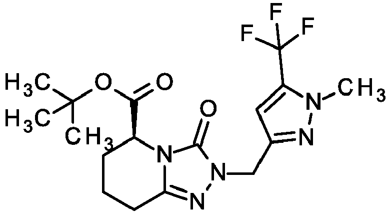

tert-Butyl-(5S)-3-oxo-2,3,5,6,7,8-hexahydro[1,2,4]triazolo[4,3-a]pyridin-5-carboxylat (234 mg, 978 µmol) wurde in Acetonitril (5.0 ml) vorgelegt. Caesiumcarbonat (638 mg, 1.96 mmol) und 3-(Chlormethyl)-1-methyl-5-(trifluormethyl)-1H-pyrazol (204 mg, 1.03 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 91.0 mg (23 % d. Th.) der Titelverbindung erhalten.
LC-MS (Methode 3): Rₜ = 1.75 min; MS (ESIpos): m/z = 402 [M+H]⁺

### Intermediat 303

### tert-Butyl-(5S)-2-{[1-benzyl-5-(trifluormethyl)-1H-pyrazol-3-yl]methyl}-3-oxo-2,3,5,6,7,8-hexahydro[1,2,4]triazolo[4,3-a]pyridin-5-carboxylat

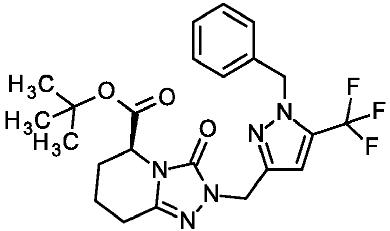

tert-Butyl-(5S)-3-oxo-2,3,5,6,7,8-hexahydro[1,2,4]triazolo[4,3-a]pyridin-5-carboxylat (548 mg, 2.29 mmol) wurde in Acetonitril (20 ml) vorgelegt. Caesiumcarbonat (1.49 g, 4.58 mmol) und 1-Benzyl-3-(chlormethyl)-5-(trifluormethyl)-1H-pyrazol (728 mg, 95 % Reinheit, 2.52 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 781 mg (76 % Reinheit, 54 % d. Th.) der Titelverbindung erhalten.
LC-MS (Methode 3): Rₜ = 2.12 min; MS (ESIpos): m/z = 478 [M+H]⁺

### Intermediat 304

### tert-Butyl-(5S)-2-[(5-brompyridin-3-yl)methyl]-3-oxo-2,3,5,6,7,8-hexahydro[1,2,4]triazolo[4,3-a]pyridin-5-carboxylat

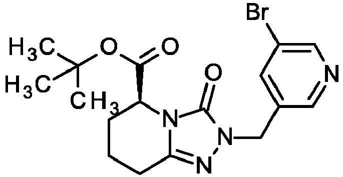

tert-Butyl-(5S)-3-oxo-2,3,5,6,7,8-hexahydro[1,2,4]triazolo[4,3-a]pyridin-5-carboxylat (396 mg, 1.66 mmol) wurde in Acetonitril (6.6 ml) vorgelegt. Caesiumcarbonat (1.08 g, 3.31 mmol) und 3-Brom-5-(chlormethyl)pyridin (376 mg, 1.82 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 146 mg (22 % d. Th.) der Titelverbindung erhalten.
LC-MS (Methode 3): Rₜ = 1.64 min; MS (ESIpos): m/z = 409 [M+H]⁺
¹H-NMR (400 MHz, DMSO-d6) δ [ppm]: 1.390 (16.00), 2.066 (0.53), 2.075 (0.53), 2.640 (0.40), 4.458 (0.61), 4.926 (1.27), 4.939 (1.25), 7.915 (0.87), 8.468 (0.83), 8.472 (0.90), 8.643 (0.71), 8.649 (0.75).

### Intermediat 305

### tert-Butyl-(5S)-2-[(5-brompyridin-2-yl)methyl]-3-oxo-2,3,5,6,7,8-hexahydro[1,2,4]triazolo[4,3-a]pyridin-5-carboxylat

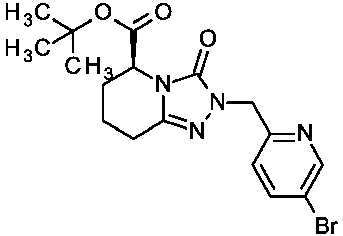

tert-Butyl-(5S)-3-oxo-2,3,5,6,7,8-hexahydro[1,2,4]triazolo[4,3-a]pyridin-5-carboxylat (309 mg, 1.29 mmol) wurde in Dichlormethan (7.3 ml) vorgelegt. Caesiumcarbonat (1.05 g, 3.23 mmol) und 5-Brom-2-(chlormethyl)pyridin (347 mg, 1.68 mmol) wurden anschließend zugegeben. Das Reaktionsgemisch wurde nach Rühren für 5 Stunden bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 580 mg (>100% d. Th.) der Titelverbindung erhalten.
LC-MS (Methode 3): Rₜ = 1.68 min; MS (ESIpos): m/z = 409 [M+H]⁺

### Intermediat 306

### tert-Butyl-(5S)-2-[2-(4-methylphenyl)-2-oxoethyl]-3-oxo-2,3,5,6,7,8-hexahydro[1,2,4]triazolo[4,3-a]pyridin-5-carboxylat

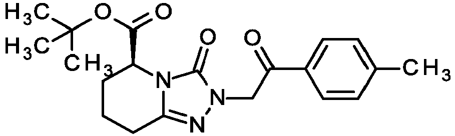

tert-Butyl-(5S)-3-oxo-2,3,5,6,7,8-hexahydro[1,2,4]triazolo[4,3-a]pyridin-5-carboxylat (645 mg, 2.70 mmol) wurde in Acetonitril (17 ml) vorgelegt. Caesiumcarbonat (2.20 g, 6.74 mmol) und 2-Chlor-1-(4-methylphenyl)ethanon (500 mg, 2.97 mmol) wurden anschließend zugegeben. Das Reaktionsgemisch wurde nach Rühren über Nacht bei Raumtemperatur mit Wasser, 1 N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 605 mg (60 % d. Th.) der Titelverbindung erhalten.
LC-MS (Methode 1): Rₜ = 1.23 min; MS (ESIpos): m/z = 316 [M+H]⁺

### Intermediat 307

### (5RS,6RS)-2-{[3-Fluor-2-(trifluormethyl)pyridin-4-yl]methyl}-3-oxo-6-(trifluormethyl)-2,3,5,6,7,8-hexahydro[1,2,4]triazolo[4,3-a]pyridin-5-carbonsäure (Diastereomerengemisch, 4 Isomere)

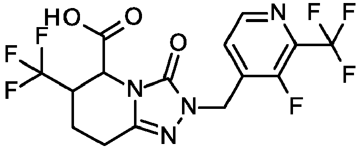

Ethyl-(5RS,6RS)-2-{[3-fluor-2-(trifluormethyl)pyridin-4-yl]methyl}-3-oxo-6-(trifluormethyl)-2,3,5,6,7,8-hexahydro[1,2,4]triazolo[4,3-a]pyridin-5-carboxylat (Diastereomerengemisch, 4 Isomere) (231 mg, 506 µmol) wurde in THF (5.0 ml) vorgelegt und Lithiumhydroxid (48.5 mg, 2.02 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 193 mg (89 % d. Th.) der Titelverbindung erhalten.
LC-MS (Methode 3): Rₜ = 1.38 min; MS (ESIpos): m/z = 429 [M+H]⁺

### Intermediat 308

### (5RS,6RS)-3-Oxo-6-(trifluormethyl)-2-{[6-(trifluormethyl)pyridin-3-yl]methyl}-2,3,5,6,7,8-hexahydro[1,2,4]triazolo[4,3-a]pyridin-5-carbonsäure (Diastereomerengemisch, 4 Isomere)

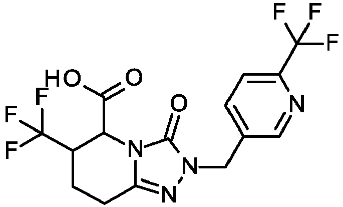

Ethyl-(5RS,6RS)-3-oxo-6-(trifluormethyl)-2-{[6-(trifluormethyl)pyridin-3-yl]methyl}-2,3,5,6,7,8-hexahydro[1,2,4]triazolo[4,3-a]pyridin-5-carboxylat (Diastereomerengemisch, 4 Isomere) (131 mg, 299 µmol) wurde in THF (2.0 ml) vorgelegt und Lithiumhydroxid (28.6 mg, 1.20 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 109 mg (89 % d. Th.) der Titelverbindung erhalten.
LC-MS (Methode 3): Rₜ = 1.35 min; MS (ESIpos): m/z = 411 [M+H]⁺
¹H-NMR (400 MHz, DMSO-d6) δ [ppm]: -0.149 (0.95), -0.008 (7.81), 0.008 (7.27), 0.146 (0.98), 1.157 (1.55), 1.169 (1.23), 1.175 (3.42), 1.181 (1.45), 1.192 (1.68), 1.236 (0.82), 1.356 (3.51), 1.908 (0.79), 1.946 (1.11), 1.959 (1.33), 1.973 (1.23), 1.988 (6.67), 2.061 (2.06), 2.075 (2.18), 2.097 (1.36), 2.112 (1.26), 2.183 (0.51), 2.328 (1.08), 2.366 (1.33), 2.523 (3.35), 2.600 (0.82), 2.615 (0.82), 2.642 (1.55), 2.669 (2.28), 2.710 (1.36), 2.747 (1.20), 2.761 (2.34), 2.775 (1.30), 2.791 (0.82), 2.804 (1.26), 2.820 (0.66), 3.428 (1.08), 3.439 (1.33), 3.452 (1.58), 3.463 (1.55), 4.002 (0.47), 4.020 (1.39), 4.038 (1.39), 4.564 (5.69), 4.573 (5.50), 5.015 (1.30), 5.056 (8.73), 5.069 (7.91), 5.109 (1.30), 7.900 (0.66), 7.920 (16.00), 8.646 (5.88).

### Intermediat 309

### (5RS,6RS)-2-[(3,5-Dichlorpyridin-2-yl)methyl]-3-oxo-6-(trifluormethyl)-2,3,5,6,7,8-hexahydro[1,2,4]triazolo[4,3-a]pyridin-5-carbonsäure (Diastereomerengemisch, 4 Isomere)

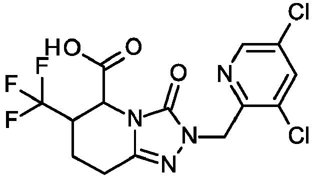

Ethyl-(5RS,6RS)-2-[(3,5-dichlorpyridin-2-yl)methyl]-3-oxo-6-(trifluormethyl)-2,3,5,6,7,8-hexahydro[1,2,4]triazolo[4,3-a]pyridin-5-carboxylat (Diastereomerengemisch, 4 Isomere) (235 mg, 535 µmol) wurde in THF (2.0 ml) vorgelegt und Lithiumhydroxid (51.3 mg, 2.14 mmol) gelöst in Wasser zugegegeben. Das Reaktionsgemisch wurde nach Rühren über Nacht bei Raumtemperatur im Vakuum eingeengt und anschließend mit Wasser, 1 N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 218 mg (99 % d. Th.) der Titelverbindung erhalten.
LC-MS (Methode 4): Rₜ = 0.69 min; MS (ESIpos): m/z = 411 [M+H]⁺
¹H-NMR (400 MHz, DMSO-d6) δ [ppm]: -0.149 (0.94), -0.008 (13.63), 0.008 (8.92), 0.146 (0.97), 1.157 (0.78), 1.169 (0.80), 1.175 (1.72), 1.181 (1.17), 1.192 (0.80), 1.356 (2.55), 1.908 (0.92), 1.950 (0.97), 1.965 (1.08), 1.974 (1.06), 1.988 (3.47), 2.031 (0.62), 2.044 (1.61), 2.058 (1.72), 2.080 (0.97), 2.094 (0.83), 2.327 (0.80), 2.366 (0.85), 2.523 (5.82), 2.574 (0.99), 2.603 (1.33), 2.619 (1.17), 2.643 (0.83), 2.670 (0.94), 2.710 (1.54), 2.728 (1.79), 2.742 (1.06), 2.758 (0.69), 2.773 (1.01), 3.434 (1.08), 3.445 (1.22), 3.456 (1.20), 4.020 (0.64), 4.038 (0.62), 4.529 (4.25), 4.538 (4.11), 5.056 (16.00), 8.253 (3.70), 8.258 (3.91), 8.553 (4.30), 8.558 (4.14), 13.919 (0.46).

### Intermediat 310

### (5RS,6RS)-2-[(5-Chlor-3-fluorpyridin-2-yl)methyl]-3-oxo-6-(trifluormethyl)-2,3,5,6,7,8-hexahydro[1,2,4]triazolo[4,3-a]pyridin-5-carboxylat (Diastereomerengemisch, 4 Isomere)

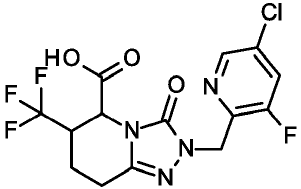

Ethyl-(5RS,6RS)-2-[(5-chlor-3-fluorpyridin-2-yl)methyl]-3-oxo-6-(trifluormethyl)-2,3,5,6,7,8-hexahydro[1,2,4]triazolo[4,3-a]pyridin-5-carboxylat (Diastereomerengemisch, 4 Isomere) (235 mg, 556 µmol) wurde in THF (3.0 ml) vorgelegt und Lithiumhydroxid (53.2 mg, 2.22 mmol) gelöst in Wasser zugegeben. Das Reaktionsgemisch wurde nach Rühren über Nacht bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 202 mg (92 % d. Th.) der Titelverbindung erhalten.
LC-MS (Methode 3): Rₜ = 1.17 min; MS (ESIpos): m/z = 395 [M+H]⁺

### Intermediat 311

### (5RS,6RS)-2-[(5-Chlorpyridin-2-yl)methyl]-3-oxo-6-(trifluormethyl)-2,3,5,6,7,8-hexahydro[1,2,4]triazolo[4,3-a]pyridin-5-carboxylat (Diastereomerengemisch, 4 Isomere)

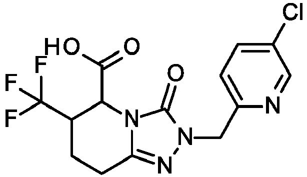

Ethyl-(5RS,6RS)-2-[(5-chlorpyridin-2-yl)methyl]-3-oxo-6-(trifluormethyl)-2,3,5,6,7,8-hexahydro[1,2,4]triazolo[4,3-a]pyridin-5-carboxylat (Diastereomerengemisch, 4 Isomere) (150 mg, 371 µmol) wurde in THF (3.0 ml) vorgelegt und Lithiumhydroxid (35.5 mg, 1.48 mmol) gelöst in Wasser zugegeben. Das Reaktionsgemisch wurde nach Rühren über Nacht bei Raumtemperatur im Vakuum eingeengt und anschließend mit Wasser, 1 N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 139 mg (100 % d. Th.) der Titelverbindung erhalten.
LC-MS (Methode 3): Rₜ = 1.13 min; MS (ESIpos): m/z = 377 [M+H]⁺

### Intermediat 312

### (5RS,7RS)-2-{[3-Fluor-2-(trifluormethyl)pyridin-4-yl]methyl}-7-methyl-3-oxo-2,3,5,6,7,8-hexahydro[1,2,4]triazolo[4,3-a]pyridin-5-carboxylat (Diastereomerengemisch, 4 Isomere)

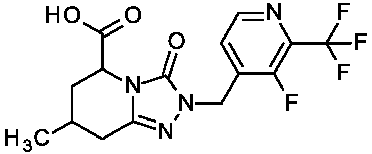

tert-Butyl-(5RS,7RS)-2-{[3-fluor-2-(trifluormethyl)pyridin-4-yl]methyl}-7-methyl-3-oxo-2,3,5,6,7,8-hexahydro[1,2,4]triazolo[4,3-a]pyridin-5-carboxylat (Diastereomerengemisch, 4 Isomere) (375 mg, 870 µmol) wurde in Dichlormethan (10 ml) gelöst und bei Raumtemperatur mit Trifluoressigsäure (1.3 ml, 17 mmol) versetzt. Nachdem das Reaktionsgemisch über Nacht bei Raumtemperatur gerührt worden war, wurde das Lösungsmittel unter Vakuum entfernt. Es wurden 422 mg (90 % Reinheit, >100% d. Th.) der Titelverbindung erhalten.
LC-MS (Methode 4): Rₜ = 0.71 min; MS (ESIpos): m/z = 375 [M+H]⁺
¹H-NMR (400 MHz, DMSO-d6) δ [ppm]: -0.149 (0.49), -0.008 (6.45), 0.008 (2.97), 0.146 (0.43), 1.022 (14.53), 1.039 (14.32), 1.111 (1.07), 1.354 (5.64), 1.385 (0.49), 1.410 (10.08), 1.456 (1.36), 1.484 (2.44), 1.511 (1.83), 1.517 (2.24), 1.535 (0.60), 1.544 (1.24), 1.912 (7.07), 1.932 (1.18), 1.939 (1.27), 1.949 (1.28), 1.966 (1.07), 2.195 (2.45), 2.223 (2.20), 2.235 (3.03), 2.263 (3.53), 2.276 (1.56), 2.294 (1.26), 2.309 (1.13), 2.329 (0.54), 2.367 (0.46), 2.524 (3.54), 2.606 (0.40), 2.650 (3.07), 2.671 (1.00), 2.690 (3.64), 2.696 (2.78), 2.711 (0.81), 2.966 (0.44), 2.987 (0.46), 4.316 (2.81), 4.332 (3.28), 4.342 (3.04), 4.358 (2.54), 4.782 (1.33), 4.801 (1.31), 5.070 (16.00), 5.092 (1.30), 5.134 (3.28), 5.153 (2.60), 5.195 (0.93), 5.535 (0.63), 5.754 (1.36), 6.149 (1.64), 7.567 (0.74), 7.580 (1.34), 7.599 (2.58), 7.612 (4.55), 7.625 (2.75), 8.548 (1.57), 8.564 (5.45), 8.576 (5.15).

### Intermediat 313

### (5RS,7RS)-7-Methyl-3-oxo-2-{[6-(trifluormethyl)pyridin-3-yl]methyl}-2,3,5,6,7,8-hexahydro[1,2,4]triazolo[4,3-a]pyridin-5-carboxylat (Diastereomerengemisch, 4 Isomere)

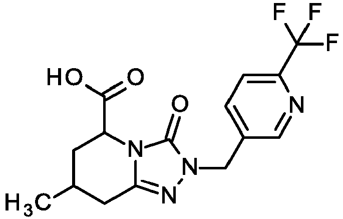

tert-Butyl-(5RS,7RS)-7-methyl-3-oxo-2-{[6-(trifluormethyl)pyridin-3-yl]methyl}-2,3,5,6,7,8-hexahydro[1,2,4]triazolo[4,3-a]pyridin-5-carboxylat (Diastereomerengemisch, 4 Isomere) (274 mg, 664 µmol) wurde in Dichlormethan (8.0 ml) gelöst und bei Raumtemperatur mit Trifluoressigsäure (1.0 ml, 13 mmol) versetzt. Nachdem das Reaktionsgemisch über Nacht bei Raumtemperatur gerührt worden war, wurde das Lösungsmittel unter Vakuum entfernt. Es wurden 438 mg (>100% d. Th.) der Titelverbindung erhalten.
LC-MS (Methode 4): Rₜ = 0.66 min; MS (ESIpos): m/z = 357 [M+H]⁺
¹H-NMR (400 MHz, DMSO-d6) δ [ppm]: -0.149 (0.46), 0.008 (3.92), 0.146 (0.46), 1.016 (14.93), 1.032 (15.11), 1.111 (1.31), 1.337 (0.57), 1.407 (2.26), 1.444 (1.19), 1.471 (2.41), 1.504 (2.44), 1.532 (1.43), 1.833 (0.54), 1.904 (7.33), 1.927 (1.28), 1.937 (1.34), 1.988 (0.61), 2.073 (0.93), 2.181 (2.59), 2.209 (2.18), 2.222 (3.05), 2.250 (3.80), 2.266 (1.58), 2.283 (1.37), 2.298 (1.24), 2.329 (0.52), 2.367 (0.47), 2.524 (3.38), 2.638 (2.93), 2.642 (2.91), 2.677 (2.32), 2.684 (2.91), 2.711 (0.61), 2.953 (0.46), 2.974 (0.47), 4.306 (2.84), 4.322 (3.29), 4.333 (3.06), 4.348 (2.60), 4.764 (1.36), 4.783 (1.34), 5.016 (16.00), 5.083 (5.43), 5.754 (1.00), 6.132 (2.18), 7.889 (1.01), 7.899 (2.82), 7.909 (3.67), 7.919 (10.17), 7.932 (5.19), 7.936 (5.01), 7.952 (1.48), 8.670 (4.96).

### Intermediat 314

### (5RS,7RS)-2-(3-Chlor-4-fluorbenzyl)-7-methyl-3-oxo-2,3,5,6,7,8-hexahydro[1,2,4]triazolo[4,3-a]pyridin-5-carboxylat (Diastereomerengemisch, 4 Isomere)

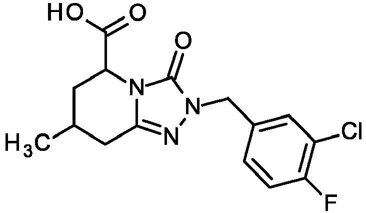

tert-Butyl-(5RS,7RS)-2-(3-chlor-4-fluorbenzyl)-7-methyl-3-oxo-2,3,5,6,7,8-hexahydro[1,2,4]triazolo[4,3-a]pyridin-5-carboxylat (Diastereomerengemisch, 4 Isomere) (327 mg, 827 µmol) wurde in Dichlormethan (10 ml) gelöst und bei Raumtemperatur mit Trifluoressigsäure (1.3 ml, 17 mmol) versetzt. Nachdem das Reaktionsgemisch über Nacht bei Raumtemperatur gerührt worden war, wurde das Lösungsmittel unter Vakuum entfernt. Es wurden 465 mg (>100 % d. Th.) der Titelverbindung erhalten.
LC-MS (Methode 1): Rₜ = 1.06 min; MS (ESIpos): m/z = 340 [M+H]⁺

### Intermediat 315

### (5RS,7RS)-2-[(5-Chlorpyridin-2-yl)methyl]-3-oxo-7-(trifluormethyl)-2,3,5,6,7,8-hexahydro[1,2,4]triazolo[4,3-a]pyridin-5-carbonsäure (Diastereomerengemisch, 4 Isomere)

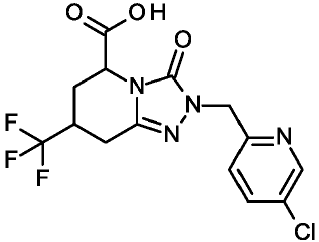

### Ethyl-(5RS,7RS)-2-[(5-chlorpyridin-2-yl)methyl]-3-oxo-7-(trifluormethyl)-2,3,5,6,7,8-hexahydro[1,2,4]triazolo[4,3-a]pyridin-5-carboxylat (Diastereomerengemisch, 4 Isomere)

(248 mg, 612 µmol) wurde in THF (8.0 ml) gelöst und Natriumethanolat (367 mg, 21 % Gew, 1.13 mmol) wurde bei 0°C zugegeben. Das Reaktionsgemisch wurde, nach Rühren für 20 Minuten bei 0°C und über Nacht bei Raumtemperatur, mit Wasser, 1N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 206 mg (89 % d. Th.) der Titelverbindung erhalten.
LC-MS (Methode 3): Rₜ = 1.15 min; MS (ESIpos): m/z = 377 [M+H]⁺
¹H-NMR (400 MHz, DMSO-d6) δ [ppm]: -0.149 (1.11), 0.146 (1.07), 1.157 (1.49), 1.169 (3.71), 1.174 (3.55), 1.192 (1.45), 1.235 (0.59), 1.270 (0.59), 1.356 (1.21), 1.371 (0.50), 1.413 (0.52), 1.801 (0.42), 1.908 (2.00), 1.988 (5.04), 2.244 (0.83), 2.279 (2.20), 2.295 (2.52), 2.308 (3.01), 2.327 (7.89), 2.366 (2.38), 2.675 (2.62), 2.705 (6.74), 2.730 (6.62), 2.879 (0.40), 2.954 (0.93), 2.984 (4.62), 3.012 (3.79), 3.039 (0.65), 4.002 (0.44), 4.020 (1.25), 4.038 (1.19), 4.441 (0.42), 4.701 (5.49), 4.711 (4.94), 4.924 (2.18), 4.946 (2.82), 4.965 (16.00), 4.976 (15.98), 5.016 (2.18), 6.578 (0.50), 7.240 (9.06), 7.261 (9.87), 7.277 (0.95), 7.298 (0.83), 7.911 (5.79), 7.917 (6.17), 7.932 (5.93), 7.938 (6.01), 8.579 (7.32), 8.585 (7.57), 13.685 (0.99).

### Intermediat 316

### (5RS,8RS)-2-{[3-Fluor-2-(trifluormethyl)pyridin-4-yl]methyl}-8-methyl-3-oxo-2,3,5,6,7,8-hexahydro[1,2,4]triazolo[4,3-a]pyridin-5-carboxylat (Diastereomerengemisch, 4 Isomere)

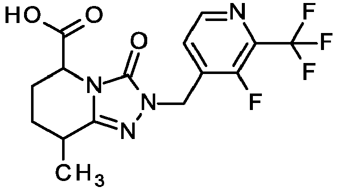

tert-Butyl-(5RS,8RS)-2-{[3-fluor-2-(trifluormethyl)pyridin-4-yl]methyl}-8-methyl-3-oxo-2,3,5,6,7,8-hexahydro[1,2,4]triazolo[4,3-a]pyridin-5-carboxylat (Diastereomerengemisch, 4 Isomere) (546 mg, 1.27 mmol) wurde in Dichlormethan (10 ml) gelöst und bei Raumtemperatur mit Trifluoressigsäure (2.0 ml, 25 mmol) versetzt. Nachdem das Reaktionsgemisch über Nacht bei Raumtemperatur gerührt worden war, wurde das Lösungsmittel unter Vakuum entfernt. Es wurden 474 mg (94 % d. Th.) der Titelverbindung erhalten.
LC-MS (Methode 3): Rₜ = 1.29 min; MS (ESIpos): m/z = 375 [M+H]⁺

### Intermediat 317

### (5RS,8RS)-2-(3-Chlor-4-fluorbenzyl)-8-methyl-3-oxo-2,3,5,6,7,8-hexahydro[1,2,4]triazolo[4,3-a]pyridin-5-carboxylat (Diastereomerengemisch, 4 Isomere)

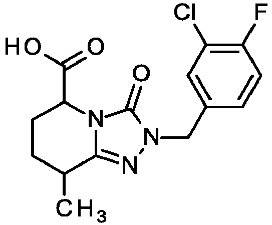

tert-Butyl-(5RS,8RS)-2-(3-chlor-4-fluorbenzyl)-8-methyl-3-oxo-2,3,5,6,7,8-hexahydro[1,2,4]triazolo[4,3-a]pyridin-5-carboxylat (Diastereomerengemisch, 4 Isomere) (450 mg, 1.14 mmol) wurde in Dichlormethan (10 ml) gelöst und bei Raumtemperatur mit Trifluoressigsäure (1.8 ml, 23 mmol) versetzt. Nachdem das Reaktionsgemisch über Nacht bei Raumtemperatur gerührt worden war, wurde das Lösungsmittel unter Vakuum entfernt. Es wurden 470 mg (79 % Reinheit, 96 % d. Th.) der Titelverbindung erhalten.
LC-MS (Methode 3): Rₜ = 1.41 min; MS (ESIpos): m/z = 340 [M+H]⁺

### Intermediat 318

### (5RS,8RS)-2-(2,4-Difluorbenzyl)-8-methyl-3-oxo-2,3,5,6,7,8-hexahydro[1,2,4]triazolo[4,3-a]pyridin-5-carboxylat (Diastereomerengemisch, 4 Isomere)

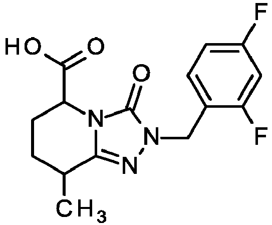

tert-Butyl-(5RS,8RS)-2-(2,4-difluorbenzyl)-8-methyl-3-oxo-2,3,5,6,7,8-hexahydro[1,2,4]triazolo[4,3-a]pyridin-5-carboxylat (Diastereomerengemisch, 4 Isomere) (400 mg, 1.05 mmol) wurde in Dichlormethan (10 ml) gelöst und bei Raumtemperatur mit Trifluoressigsäure (1.6 ml, 21 mmol) versetzt. Nachdem das Reaktionsgemisch über Nacht bei Raumtemperatur gerührt worden war, wurde das Lösungsmittel unter Vakuum entfernt. Es wurden 578 mg (87 % Reinheit, 148 % d. Th.) der Titelverbindung erhalten.
LC-MS (Methode 3): Rₜ = 1.26 min; MS (ESIpos): m/z = 324 [M+H]⁺

### Intermediat 319

### (5RS,8RS)-8-Methyl-3-oxo-2-{[6-(trifluormethyl)pyridin-3-yl]methyl}-2,3,5,6,7,8-hexahydro[1,2,4]triazolo[4,3-a]pyridin-5-carbonsäure (Diastereomerengemisch, 4 Isomere)

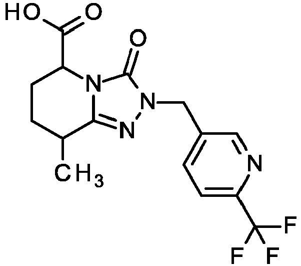

tert-Butyl-(5RS,8RS)-8-methyl-3-oxo-2-{[6-(trifluormethyl)pyridin-3-yl]methyl}-2,3,5,6,7,8-hexahydro[1,2,4]triazolo[4,3-a]pyridin-5-carboxylat (Diastereomerengemisch, 4 Isomere) (854 mg, 2.07 mmol) wurde in Dichlormethan (10 ml) gelöst und bei Raumtemperatur mit Trifluoressigsäure (10 ml, 130 mmol) versetzt. Nachdem das Reaktionsgemisch für 2 h bei Raumtemperatur gerührt wurde, wurde das Lösungsmittel unter Vakuum entfernt. Der Rückstand wurde mit Wasser und Dichlormethan versetzt. Die organische Phase wurde abgetrennt und die wässrige Phase dreimal mit Dichlormethan extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 727 mg (99 % d. Th.) der Titelverbindung erhalten.
LC-MS (Methode 3): Rₜ = 1.20 min; MS (ESIpos): m/z = 357 [M+H]⁺
¹H-NMR (400 MHz, DMSO-d6) δ [ppm]: -0.008 (2.12), 0.008 (1.76), 1.142 (0.42), 1.175 (15.73), 1.192 (16.00), 1.206 (1.64), 1.228 (2.06), 1.239 (1.49), 1.259 (0.64), 1.270 (0.63), 1.320 (0.68), 1.886 (1.20), 1.898 (1.32), 1.908 (1.05), 1.920 (1.15), 2.106 (0.43), 2.119 (1.06), 2.126 (1.10), 2.135 (1.16), 2.142 (1.21), 2.151 (2.06), 2.162 (3.13), 2.168 (3.21), 2.197 (0.61), 2.328 (0.44), 2.524 (1.33), 2.670 (0.41), 2.717 (1.01), 2.731 (1.49), 2.747 (1.88), 2.763 (1.41), 2.778 (0.89), 3.568 (3.56), 4.501 (2.48), 4.507 (3.45), 4.517 (2.38), 4.522 (2.56), 5.023 (1.32), 5.064 (6.79), 5.081 (6.73), 5.122 (1.30), 7.892 (0.60), 7.910 (13.13), 7.912 (13.01), 7.936 (0.51), 8.645 (4.58).

### Intermediat 320

### (5S)-2-(3-Chlor-4-fluorbenzyl)-3-oxo-2,3,5,6,7,8-hexahydro[1,2,4]triazolo[4,3-a]pyridin-5-carbonsäure

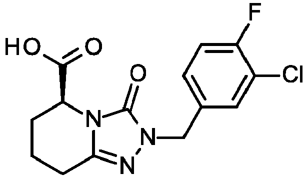

Methyl-(5S)-2-(3-chlor-4-fluorbenzyl)-3-oxo-2,3,5,6,7,8-hexahydro[1,2,4]triazolo[4,3-a]pyridin-5-carboxylat (58.5 mg, 172 µmol) wurde in THF (570 µl) vorgelegt und Lithiumhydroxid (20.6 mg, 861 µmol) gelöst in Wasser zugegeben. Das Reaktionsgemisch wurde nach Rühren über Nacht bei Raumtemperatur im Vakuum eingeengt und anschließend mit Wasser, 1 N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 55.0 mg (98 % d. Th.) der Titelverbindung erhalten.
LC-MS (Methode 4): Rₜ = 0.70 min; MS (ESIpos): m/z = 326 [M+H]⁺

### Intermediat 321

### (5S)-3-Oxo-2-[3-(trifluormethyl)benzyl]-2,3,5,6,7,8-hexahydro[1,2,4]triazolo[4,3-a]pyridin-5-carbonsäure

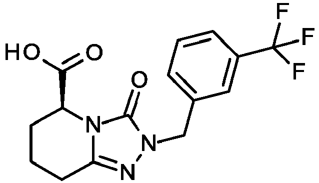

Methyl-(5S)-3-oxo-2-[3-(trifluormethyl)benzyl]-2,3,5,6,7,8-hexahydro[1,2,4]triazolo[4,3-a]pyridin-5-carboxylat (336 mg, 947 µmol) wurde in THF (10 ml) gelöst und bei Raumtemperatur mit Trifluoressigsäure (113 mg, 4.73 mmol) versetzt. Nachdem das Reaktionsgemisch für 5 Stunden bei Raumtemperatur gerührt worden war, wurde das Lösungsmittel unter Vakuum entfernt. Es wurden 218 mg (67 % d. Th.) der Titelverbindung erhalten.
LC-MS (Methode 1): Rₜ = 1.01 min; MS (ESIpos): m/z = 342 [M+H]⁺

### Intermediat 322

### (5S)-3-Oxo-2-{[4-(trifluormethyl)pyridin-2-yl]methyl}-2,3,5,6,7,8-hexahydro[1,2,4]triazolo[4,3-a]pyridin-5-carbonsäure

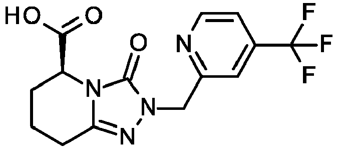

Methyl-(5S)-3-oxo-2-{[4-(trifluormethyl)pyridin-2-yl]methyl}-2,3,5,6,7,8-hexahydro[1,2,4] triazolo[4,3-a]pyridin-5-carboxylat (275 mg, 770 µmol) wurde in THF (10 ml) vorgelegt und Lithiumhydroxid (92.2 mg, 3.85 mmol) gelöst in Wasser zugegeben. Das Reaktionsgemisch wurde nach Rühren für 5 Stunden bei Raumtemperatur im Vakuum eingeengt und anschließend mit Wasser, 1N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 247 mg (94 % d. Th.) der Titelverbindung erhalten.
LC-MS (Methode 4): Rₜ = 0.59 min; MS (ESIpos): m/z = 343 [M+H]⁺

### Intermediat 323

### (5S)-3-Oxo-2-{[2-(trifluormethyl)pyrimidin-4-yl]methyl}-2,3,5,6,7,8-hexahydro[1,2,4]triazolo[4,3-a]pyridin-5-carbonsäure

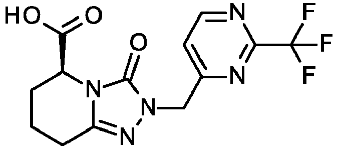

Methyl-(5S)-3-oxo-2-{[2-(trifluormethyl)pyrimidin-4-yl]methyl}-2,3,5,6,7,8-hexahydro[1,2,4]triazolo[4,3-a]pyridin-5-carboxylat (49.5 mg, 139 µmol) wurde in THF (2.0 ml) vorgelegt und Lithiumhydroxid (16.6 mg, 693 µmol) gelöst in Wasser zugegeben. Das Reaktionsgemisch wurde nach Rühren für 5 Stunden bei Raumtemperatur im Vakuum eingeengt und anschließend mit Wasser, 1 N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 45.0 mg (95 % d. Th.) der Titelverbindung erhalten.
LC-MS (Methode 4): Rₜ = 0.55 min; MS (ESIpos): m/z = 344 [M+H]⁺

### Intermediat 324

### (5S)-3-Oxo-2-{[5-(trifluormethyl)pyridin-2-yl]methyl}-2,3,5,6,7,8-hexahydro[1,2,4]triazolo[4,3-a]pyridin-5-carbonsäure

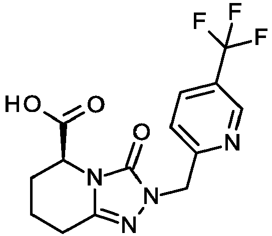

Methyl-(5S)-3-oxo-2-{[5-(trifluormethyl)pyridin-2-yl]methyl}-2,3,5,6,7,8-hexahydro[1,2,4]triazolo[4,3-a]pyridin-5-carboxylat (175 mg, 491 µmol) wurde in THF (7.0 ml) vorgelegt und Lithiumhydroxid (58.8 mg, 2.46 mmol) gelöst in Wasser zugegeben. Das Reaktionsgemisch wurde nach Rühren für 5 Stunden bei Raumtemperatur im Vakuum eingeengt und anschließend mit Wasser, 1 N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 156 mg (93 % d. Th.) der Titelverbindung erhalten.
LC-MS (Methode 3): Rₜ = 1.04 min; MS (ESIpos): m/z = 343 [M+H]⁺

### Intermediat 325

### (5S)-2-[(5-Methyl-1H-pyrazol-3-yl)methyl]-3-oxo-2,3,5,6,7,8-hexahydro[1,2,4]triazolo[4,3-a]pyridin-5-carbonsäure

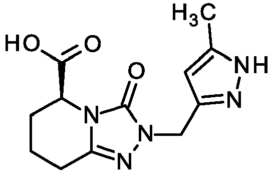

Methyl-(5RS)-2-[(5-methyl-1H-pyrazol-3-yl)methyl]-3-oxo-2,3,5,6,7,8-hexahydro[1,2,4]triazolo[4,3-a]pyridin-5-carboxylat (60.0 mg, 206 µmol) wurde in THF (3.0 ml) vorgelegt und Lithiumhydroxid (24.7 mg, 1.03 mmol) gelöst in Wasser zugegeben. Das Reaktionsgemisch wurde nach Rühren über Nacht bei Raumtemperatur im Vakuum eingeengt und anschließend mit Wasser, 1 N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 38.0 mg (67 % d. Th.) der Titelverbindung erhalten.
LC-MS (Methode 3): Rₜ = 0.57 min; MS (ESIpos): m/z = 278 [M+H]⁺

### Intermediat 326

### (5S)-2-{[3-Chlor-4-(trifluormethyl)pyridin-2-yl]methyl}-3-oxo-2,3,5,6,7,8-hexahydro[1,2,4]triazolo[4,3-a]pyridin-5-carbonsäure

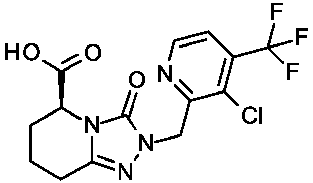

Methyl-(5S)-2-{[3-chlor-4-(trifluormethyl)pyridin-2-yl]methyl}-3-oxo-2,3,5,6,7,8-hexahydro[1,2,4]triazolo[4,3-a]pyridin-5-carboxylat (137 mg, 351 µmol) wurde in THF (2.0 ml) vorgelegt und Lithiumhydroxid (42.0 mg, 1.75 mmol) gelöst in Wasser zugegeben. Das Reaktionsgemisch wurde nach Rühren für 72 Stunden bei Raumtemperatur im Vakuum eingeengt und anschließend mit Wasser, 1 N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 107 mg (81 % d. Th.) der Titelverbindung erhalten.
LC-MS (Methode 3): Rₜ = 1.18 min; MS (ESIpos): m/z = 377 [M+H]⁺

### Intermediat 327

### (5S)-2-[4-Fluor-3-(trifluormethyl)benzyl]-3-oxo-2,3,5,6,7,8-hexahydro[1,2,4]triazolo[4,3-a]pyridin-5-carbonsäure

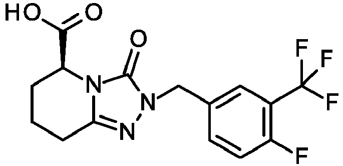

Methyl-(5S)-2-[4-fluor-3-(trifluormethyl)benzyl]-3-oxo-2,3,5,6,7,8-hexahydro[1,2,4]triazolo[4,3-a]pyridin-5-carboxylat (370 mg, 991 µmol) wurde in THF (5.0 ml) vorgelegt und Lithiumhydroxid (119 mg, 4.96 mmol) gelöst in Wasser zugegeben. Das Reaktionsgemisch wurde nach Rühren über Nacht bei Raumtemperatur mit Wasser, 1 N wässriger Salzsäure und gesättigter wässriger Natriumchlorid-Lösung versetzt. Die organische Phase wurde abgetrennt und die wässrige Phase dreimal mit Ethylacetat extrahiert. Die vereinigten organischen Phasen wurden über Natriumsulfat getrocknet, filtriert und das Filtrat eingeengt. Es wurden 133 mg (37 % d. Th.) der Titelverbindung erhalten.
LC-MS (Methode 1): Rₜ = 1.03 min; MS (ESIpos): m/z = 360 [M+H]⁺

### Intermediat 328

### (5S)-3-Oxo-2-{[6-(trifluormethyl)pyridin-3-yl]methyl}-2,3,5,6,7,8-hexahydro[1,2,4]triazolo[4,3-a]pyridin-5-carbonsäure

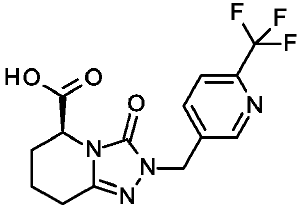

Methyl-(5S)-3-oxo-2-{[6-(trifluormethyl)pyridin-3-yl]methyl}-2,3,5,6,7,8-hexahydro[1,2,4]triazolo[4,3-a]pyridin-5-carboxylat (442 mg, 75 % Reinheit, 930 µmol) wurde in THF (2.7 ml) vorgelegt und Lithiumhydroxid (111 mg, 4.65 mmol) gelöst in Wasser zugegeben. Das Reaktionsgemisch wurde nach Rühren für 72 Stunden bei Raumtemperatur mit Wasser, 1 N wässriger Salzsäure und gesättigter wässriger Natriumchlorid-Lösung versetzt. Die organische Phase wurde abgetrennt und die wässrige Phase dreimal mit Ethylacetat extrahiert. Die vereinigten organischen Phasen wurden über Natriumsulfat getrocknet, filtriert und das Filtrat eingeengt. Es wurden 316 mg (90 % d. Th.) der Titelverbindung erhalten.
LC-MS (Methode 1): Rₜ = 0.83 min; MS (ESIpos): m/z = 343 [M+H]⁺

### Alternative Synthese:

tert-Butyl-(5S)-3-oxo-2-{[6-(trifluormethyl)pyridin-3-yl]methyl}-2,3,5,6,7,8-hexahydro[1,2,4]triazolo[4,3-a]pyridin-5-carboxylat (411 mg, 1.03 mmol) wurde in Dichlormethan (5.0 ml) gelöst und bei 0°C mit Trifluoressigsäure (1.6 ml, 21 mmol) versetzt. Nachdem das Reaktionsgemisch über Nacht bei Raumtemperatur gerührt worden war, wurde das Lösungsmittel unter Vakuum entfernt. Es wurden 798 mg (90 % Reinheit, >100 % d. Th.) der Titelverbindung erhalten.
LC-MS (Methode 4): Rₜ = 0.60 min; MS (ESIpos): m/z = 343 [M+H]⁺

### Intermediat 329

### (5S)-2-{[6-Fluor-2-(trifluormethyl)chinolin-4-yl]methyl}-3-oxo-2,3,5,6,7,8-hexahydro[1,2,4]triazolo[4,3-a]pyridin-5-carbonsäure

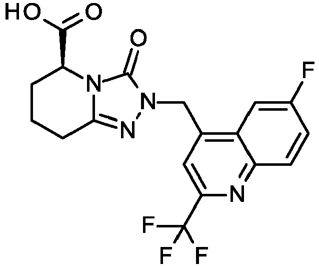

Methyl-(5S)-2-{[6-fluor-2-(trifluormethyl)chinolin-4-yl]methyl}-3-oxo-2,3,5,6,7,8-hexahydro[1,2,4]triazolo[4,3-a]pyridin-5-carboxylat (59.0 mg, 139 µmol) wurde in THF (2.0 ml) vorgelegt und Lithiumhydroxid (16.6 mg, 695 µmol) gelöst in Wasser zugegeben. Das Reaktionsgemisch wurde nach Rühren für 72 Stunden bei Raumtemperatur im Vakuum eingeengt und anschließend mit Wasser, 1 N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 44.0 mg (77 % d. Th.) der Titelverbindung erhalten.
LC-MS (Methode 3): Rₜ = 1.46 min; MS (ESIpos): m/z = 411 [M+H]⁺

### Intermediat 330

### (5S)-2-(6,7-Dihydro-5H-cyclopenta[c]pyridin-3-ylmethyl)-3-oxo-2,3,5,6,7,8-hexahydro[1,2,4]triazolo[4,3-a]pyridin-5-carbonsäure

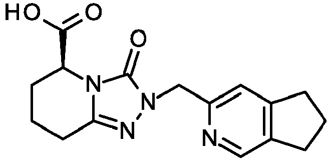

Methyl-(5S)-2-(6,7-dihydro-5H-cyclopenta[c]pyridin-3-ylmethyl)-3-oxo-2,3,5,6,7,8-hexahydro[1,2,4]triazolo[4,3-a]pyridin-5-carboxylat (109 mg, 90 % Reinheit, 299 µmol) wurde in THF (4.0 ml) vorgelegt und Lithiumhydroxid (35.8 mg, 1.50 mmol) gelöst in Wasser zugegeben. Das Reaktionsgemisch wurde nach Rühren über Nacht bei Raumtemperatur im Vakuum eingeengt und anschließend mit Wasser, 1 N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 72.0 mg (43 % Reinheit, 33 % d. Th.) der Titelverbindung erhalten.
LC-MS (Methode 3): Rₜ = 0.48 min; MS (ESIpos): m/z = 315 [M+H]⁺
¹H-NMR (400 MHz, DMSO-d6) δ [ppm]: 1.645 (7.08), 2.036 (7.44), 2.158 (4.32), 2.859 (16.00), 3.974 (4.21), 4.762 (9.00), 7.462 (5.14), 8.192 (4.78).

### Intermediat 331

### (5S)-3-Oxo-2-{[5-(trifluormethyl)pyridin-3-yl]methyl}-2,3,5,6,7,8-hexahydro[1,2,4]triazolo[4,3-a]pyridin-5-carbonsäure

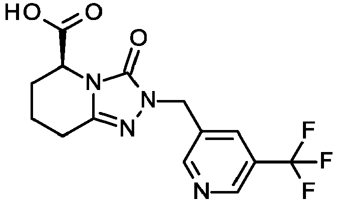

Methyl-(5S)-3-oxo-2-{[5-(trifluormethyl)pyridin-3-yl]methyl}-2,3,5,6,7,8-hexahydro[1,2,4]triazolo[4,3-a]pyridin-5-carboxylat (40.9 mg, 115 µmol) wurde in THF (3.0 ml, 37 mmol) vorgelegt und Lithiumhydroxid (13.7 mg, 574 µmol) gelöst in Wasser zugegeben. Das Reaktionsgemisch wurde nach Rühren über Nacht bei Raumtemperatur im Vakuum eingeengt und anschließend mit Wasser, 1 N wässriger Salzsäure und gesättigter wässriger Natriumchlorid-Lösung versetzt. Die wässrige Phase wurde dreimal mit Ethylacetat extrahiert. Die vereinigten organischen Phasen wurden über Natriumsulfat getrocknet, filtriert und das Filtrat eingeengt. Es wurden 35.4 mg (90 % d. Th.) der Titelverbindung erhalten.
LC-MS (Methode 3): Rₜ = 0.99 min; MS (ESIpos): m/z = 343 [M+H]⁺

### Intermediat 332

### (5S)-2-[(6-Methoxypyridin-3-yl)methyl]-3-oxo-2,3,5,6,7,8-hexahydro[1,2,4]triazolo[4,3-a]pyridin-5-carbonsäure

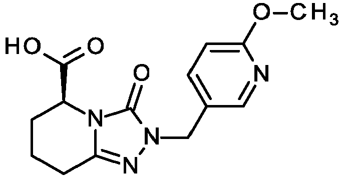

tert-Butyl-(5S)-2-[(6-methoxypyridin-3-yl)methyl]-3-oxo-2,3,5,6,7,8-hexahydro[1,2,4]triazolo[4,3-a]pyridin-5-carboxylat (761 mg, 2.11 mmol) wurde in Dichlormethan (30 ml) gelöst und bei Raumtemperatur mit Trifluoressigsäure (3.3 ml, 42 mmol) versetzt. Nachdem das Reaktionsgemisch über Nacht bei Raumtemperatur gerührt worden war, wurde das Lösungsmittel unter Vakuum entfernt. Es wurden 1.97 g (97 % d. Th.) der Titelverbindung erhalten.
LC-MS (Methode 3): Rₜ = 0.99 min; MS (ESIpos): m/z = 305 [M+H]⁺

### Intermediat 333

### (5S)-2-{[3-Fluor-2-(trifluormethyl)pyridin-4-yl]methyl}-3-oxo-2,3,5,6,7,8-hexahydro[1,2,4]triazolo[4,3-a]pyridin-5-carbonsäure

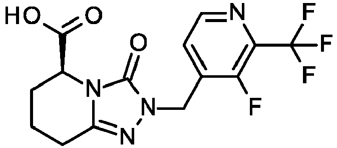

tert-Butyl-(5S)-2-{[3-fluor-2-(trifluormethyl)pyridin-4-yl]methyl}-3-oxo-2,3,5,6,7,8-hexahydro[1,2,4]triazolo[4,3-a]pyridin-5-carboxylat (1.37 g, 3.29 mmol) wurde in Dichlormethan (25 ml) gelöst und bei Raumtemperatur mit Trifluoressigsäure (5.1 ml, 66 mmol) versetzt. Nachdem das Reaktionsgemisch über Nacht bei Raumtemperatur gerührt wurde, wurde das Lösungsmittel unter Vakuum entfernt. Es wurden 2.07 g (>100% d. Th.) der Titelverbindung erhalten.
LC-MS (Methode 3): Rₜ = 1.11 min; MS (ESIpos): m/z = 361 [M+H]⁺

### Intermediat 334

### (5S)-2-[2-(4-Methylphenyl)ethyl]-3-oxo-2,3,5,6,7,8-hexahydro[1,2,4]triazolo[4,3-a]pyridin-5-carbonsäure

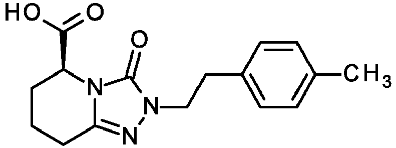

tert-Butyl-(5S)-2-[2-(4-methylphenyl)ethyl]-3-oxo-2,3,5,6,7,8-hexahydro[1,2,4]triazolo[4,3-a]pyridin-5-carboxylat (238 mg, 665 µmol) wurde in Dichlormethan (6.7 ml) gelöst und bei Raumtemperatur mit Trifluoressigsäure (800 µl, 10 mmol) versetzt. Nachdem das Reaktionsgemisch über Nacht bei Raumtemperatur gerührt worden war, wurde das Lösungsmittel unter Vakuum entfernt. Es wurden 325 mg (>100 % d. Th.) der Titelverbindung erhalten.
LC-MS (Methode 1): Rₜ = 0.98 min; MS (ESIpos): m/z = 302 [M+H]⁺
¹H-NMR (400 MHz, DMSO-d6) δ [ppm]: 0.008 (1.06), 1.111 (0.44), 1.228 (0.60), 1.534 (1.02), 1.805 (0.42), 1.816 (0.43), 2.066 (1.03), 2.072 (1.19), 2.082 (1.11), 2.094 (0.64), 2.257 (9.22), 2.524 (0.71), 2.568 (0.54), 2.580 (0.54), 2.594 (0.41), 2.633 (0.41), 2.645 (0.71), 2.656 (0.54), 2.863 (1.16), 2.882 (2.43), 2.900 (1.28), 3.766 (0.67), 3.784 (1.69), 3.804 (1.53), 3.824 (0.62), 4.390 (0.71), 4.402 (1.44), 4.415 (0.79), 7.084 (16.00), 7.093 (1.84).

### Intermediat 335

### (5S)-2-[(1R)-1-(4-Methylphenyl)ethyl]-3-oxo-2,3,5,6,7,8-hexahydro[1,2,4]triazolo[4,3-a]pyridin-5-carbonsäure (Diastereomerengemisch, 2 Isomere)

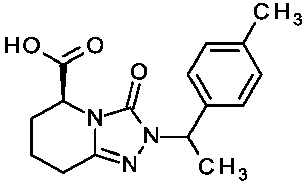

tert-Butyl-(5S)-2-[(1RS)-1-(4-methylphenyl)ethyl]-3-oxo-2,3,5,6,7,8-hexahydro[1,2,4]triazolo[4,3-a]pyridin-5-carboxylat (69.8 mg, 195 µmol) wurde in Dichlormethan (2.5 ml) gelöst und bei Raumtemperatur mit Trifluoressigsäure (300 µl, 3.9 mmol) versetzt. Nachdem das Reaktionsgemisch über Nacht bei Raumtemperatur gerührt worden war, wurde das Lösungsmittel unter Vakuum entfernt. Es wurden 73.1 mg (>100 % d. Th.) der Titelverbindung erhalten.
LC-MS (Methode 1): Rₜ = 0.98 min; MS (ESlneg): m/z = 300 [M-H]⁻
¹H-NMR (400 MHz, DMSO-d6) δ [ppm]: -0.149 (0.62), -0.008 (6.44), 0.008 (6.37), 0.146 (0.65), 1.055 (0.44), 1.291 (0.56), 1.309 (1.08), 1.326 (0.54), 1.532 (0.71), 1.584 (8.87), 1.602 (8.75), 1.799 (0.87), 1.810 (0.79), 1.822 (0.73), 1.832 (0.73), 2.058 (1.44), 2.070 (2.60), 2.081 (2.58), 2.267 (16.00), 2.280 (1.58), 2.327 (1.15), 2.366 (0.85), 2.561 (1.98), 2.576 (1.25), 2.589 (1.21), 2.603 (0.87), 2.632 (0.94), 2.643 (1.50), 2.654 (1.08), 2.669 (1.40), 2.674 (1.37), 2.709 (0.87), 4.414 (0.56), 4.426 (1.52), 4.439 (2.79), 4.450 (1.65), 5.251 (0.56), 5.268 (1.98), 5.286 (1.92), 5.303 (0.54), 7.109 (2.96), 7.129 (5.87), 7.171 (6.58), 7.191 (3.38).

### Intermediat 336

### (5S)-2-{[3-Chlor-4-(trifluormethyl)pyridin-2-yl]methyl}-3-oxo-2,3,5,6,7,8-hexahydro[1,2,4]triazolo[4,3-a]pyridin-5-carbonsäure

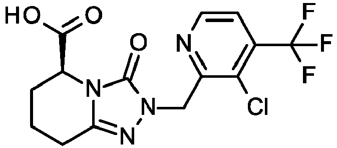

tert-Butyl-(5S)-2-{[3-chlor-4-(trifluormethyl)pyridin-2-yl]methyl}-3-oxo-2,3,5,6,7,8-hexahydro[1,2,4]triazolo[4,3-a]pyridin-5-carboxylat (794 mg, 94 % Reinheit, 1.72 mmol) wurde in Dichlormethan (28 ml) gelöst und bei Raumtemperatur mit Trifluoressigsäure (2.7 ml, 34 mmol) versetzt. Nachdem das Reaktionsgemisch für 72 Stunden bei Raumtemperatur gerührt worden war, wurde das Lösungsmittel unter Vakuum entfernt. Es wurden 800 mg (78 % Reinheit, 96 % d. Th.) der Titelverbindung erhalten.
LC-MS (Methode 1): Rₜ = 0.91 min; MS (ESIpos): m/z = 377 [M+H]⁺

### Intermediat 337

### (5S)-2-{[1-(4-Methylphenyl)cyclopropyl]methyl}-3-oxo-2,3,5,6,7,8-hexahydro[1,2,4]triazolo[4,3-a]pyridin-5-carbonsäure

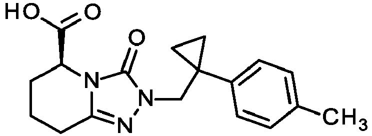

tert-Butyl-(5S)-2-{[1-(4-methylphenyl)cyclopropyl]methyl}-3-oxo-2,3,5,6,7,8-hexahydro[1,2,4]triazolo[4,3-a]pyridin-5-carboxylat (167 mg, 436 µmol) wurde in Dichlormethan (10 ml) gelöst und bei Raumtemperatur mit Trifluoressigsäure (670 µl, 8.7 mmol) versetzt. Nachdem das Reaktionsgemisch über Nacht bei Raumtemperatur gerührt worden war, wurde das Lösungsmittel unter Vakuum entfernt. Es wurden 228 mg (83 % Reinheit, >100% d. Th.) der Titelverbindung erhalten.
LC-MS (Methode 1): Rₜ = 1.06 min; MS (ESIpos): m/z = 328 [M+H]⁺
¹H-NMR (400 MHz, DMSO-d6) δ [ppm]: -0.008 (2.90), 0.008 (2.46), 0.708 (0.68), 0.729 (1.89), 0.756 (0.50), 0.783 (2.19), 0.806 (0.89), 0.978 (5.27), 1.110 (1.47), 1.175 (0.42), 1.378 (1.96), 1.406 (0.45), 1.535 (3.25), 1.782 (0.88), 1.988 (0.80), 2.051 (2.35), 2.063 (2.37), 2.074 (1.57), 2.206 (0.47), 2.235 (16.00), 2.328 (0.54), 2.524 (2.40), 2.606 (1.64), 2.615 (0.98), 2.646 (0.76), 2.671 (0.54), 3.729 (2.03), 3.765 (3.10), 3.877 (3.00), 3.913 (1.98), 4.363 (1.59), 4.374 (3.00), 4.387 (1.53), 5.754 (0.48), 7.024 (3.37), 7.044 (5.39), 7.106 (6.44), 7.126 (3.93), 11.356 (0.45).

### Intermediat 338

### (5S)-3-Oxo-2-{[2-(trifluormethyl)-1,8-naphthyridin-3-yl]methyl}-2,3,5,6,7,8-hexahydro[1,2,4]triazolo[4,3-a]pyridin-5-carbonsäure

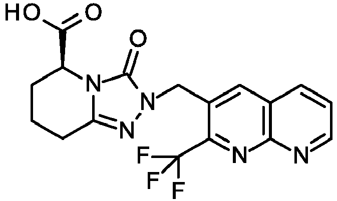

tert-Butyl-(5S)-3-oxo-2-{[2-(trifluormethyl)-1,8-naphthyridin-3-yl]methyl}-2,3,5,6,7,8-hexahydro[1,2,4]triazolo[4,3-a]pyridin-5-carboxylat (146 mg, 326 µmol) wurde in Dichlormethan (7.0 ml) gelöst und bei 0°C mit Trifluoressigsäure (500 µl, 6.5 mmol) versetzt. Nachdem das Reaktionsgemisch über Nacht bei Raumtemperatur gerührt worden war, wurde erneut Trifluoressigsäure (500 µl, 6.5 mmol) zugegeben und über Nacht bei Raumtemperatur gerührt. Das Lösungsmittel wurde anschließend unter Vakuum entfernt. Es wurden 252 mg (>100 % d. Th.) der Titelverbindung erhalten.
LC-MS (Methode 4): Rₜ = 0.56 min; MS (ESIpos): m/z = 394 [M+H]⁺
¹H-NMR (400 MHz, DMSO-d6) δ [ppm]: -0.149 (0.50), -0.008 (3.97), 0.008 (3.74), 0.146 (0.43), 1.111 (2.70), 1.228 (1.92), 1.421 (1.89), 1.535 (16.00), 1.738 (0.70), 1.856 (1.29), 1.890 (1.12), 2.073 (0.69), 2.147 (3.40), 2.160 (3.42), 2.329 (0.91), 2.367 (0.76), 2.585 (2.04), 2.599 (1.53), 2.612 (1.60), 2.627 (1.27), 2.671 (3.07), 2.711 (1.61), 4.550 (2.27), 4.560 (3.95), 4.573 (2.30), 5.243 (10.56), 7.075 (0.58), 7.815 (3.47), 7.825 (3.54), 7.835 (3.67), 7.846 (3.64), 8.380 (7.71), 8.549 (3.54), 8.554 (3.61), 8.570 (3.57), 8.575 (3.38), 9.247 (3.83), 9.252 (4.07), 9.257 (3.95), 9.262 (3.69).

### Intermediat 339

### (5S)-2-[(6-Chlorpyridin-3-yl)methyl]-3-oxo-2,3,5,6,7,8-hexahydro[1,2,4]triazolo[4,3-a]pyridin-5-carbonsäure

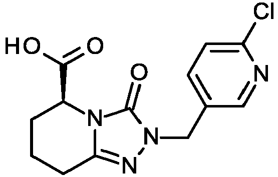

tert-Butyl-(5S)-2-[(6-chlorpyridin-3-yl)methyl]-3-oxo-2,3,5,6,7,8-hexahydro[1,2,4]triazolo[4,3-a]pyridin-5-carboxylat (509 mg, 93 % Reinheit, 1.30 mmol) wurde in Dichlormethan (20 ml) gelöst und bei 0°C mit Trifluoressigsäure (2.0 ml, 26 mmol) versetzt. Nachdem das Reaktionsgemisch über Nacht bei Raumtemperatur gerührt worden war, wurde das Lösungsmittel unter Vakuum entfernt. Es wurden 850 mg (>100% d. Th.) der Titelverbindung erhalten.
LC-MS (Methode 3): Rₜ = 0.85 min; MS (ESIpos): m/z = 309 [M+H]⁺
¹H-NMR (400 MHz, DMSO-d6) δ [ppm]: -0.008 (4.68), 0.008 (2.69), 1.030 (0.43), 1.045 (0.45), 1.110 (0.93), 1.387 (0.65), 1.505 (1.01), 1.519 (1.03), 1.534 (2.22), 1.809 (1.24), 1.819 (1.24), 1.830 (1.04), 1.841 (0.96), 2.090 (3.32), 2.100 (3.06), 2.112 (1.60), 2.328 (0.73), 2.367 (0.65), 2.519 (3.99), 2.524 (3.96), 2.568 (1.63), 2.582 (1.27), 2.614 (1.34), 2.626 (2.05), 2.636 (1.46), 2.656 (0.81), 2.666 (1.46), 2.669 (1.44), 2.710 (0.57), 4.461 (2.42), 4.473 (4.35), 4.486 (2.19), 4.827 (0.51), 4.909 (16.00), 5.067 (0.79), 5.075 (0.79), 5.088 (0.82), 5.097 (0.79), 5.445 (0.78), 7.500 (4.15), 7.510 (0.76), 7.520 (5.05), 7.703 (3.14), 7.709 (3.08), 7.723 (2.67), 7.730 (2.53), 8.309 (3.43), 8.314 (3.14).

### Intermediat 340

### (5S)-2-{[5-Chlor-6-(trifluormethyl)pyridin-3-yl]methyl}-3-oxo-2,3,5,6,7,8-hexahydro[1,2,4]triazolo[4,3-a]pyridin-5-carbonsäure

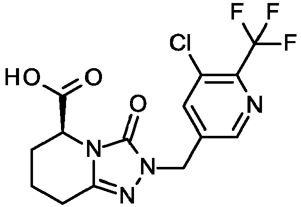

tert-Butyl-(5S)-2-{[5-chlor-6-(trifluormethyl)pyridin-3-yl]methyl}-3-oxo-2,3,5,6,7,8-hexahydro[1,2,4]triazolo[4,3-a]pyridin-5-carboxylat (156 mg, 360 µmol) wurde in Dichlormethan (3.0 ml) gelöst und bei 0°C mit Trifluoressigsäure (560 µl, 7.2 mmol) versetzt. Nachdem das Reaktionsgemisch über Nacht bei Raumtemperatur gerührt worden war, wurde das Lösungsmittel unter Vakuum entfernt. Es wurden 156 mg (66 % Reinheit, 76 % d. Th.) der Titelverbindung erhalten.
LC-MS (Methode 1): Rₜ = 0.99 min; MS (ESIpos): m/z = 377 [M+H]⁺

### Intermediat 341

### (5S)-2-(3,4-Difluorbenzyl)-3-oxo-2,3,5,6,7,8-hexahydro[1,2,4]triazolo[4,3-a]pyridin-5-carbonsäure

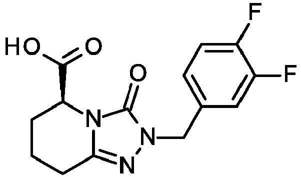

tert-Butyl-(5S)-2-(3,4-difluorbenzyl)-3-oxo-2,3,5,6,7,8-hexahydro[1,2,4]triazolo[4,3-a]pyridin-5-carboxylat (900 mg, 78 % Reinheit, 1.92 mmol) wurde in Dichlormethan (10 ml) gelöst und bei 0°C mit Trifluoressigsäure (3.0 ml, 38 mmol) versetzt. Nachdem das Reaktionsgemisch über Nacht bei Raumtemperatur gerührt worden war, wurde das Lösungsmittel unter Vakuum entfernt. Es wurden 590 mg (92 % d. Th.) der Titelverbindung erhalten.
LC-MS (Methode 3): Rₜ = 1.14 min; MS (ESIpos): m/z = 310 [M+H]⁺

### Intermediat 342

### (5S)-3-Oxo-2-(2,4,5-trifluorbenzyl)-2,3,5,6,7,8-hexahydro[1,2,4]triazolo[4,3-a]pyridin-5-carbonsäure

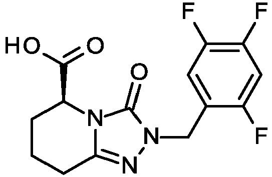

tert-Butyl-(5S)-3-oxo-2-(2,4,5-trifluorbenzyl)-2,3,5,6,7,8-hexahydro[1,2,4]triazolo[4,3-a]pyridin-5-carboxylat (523 mg, 1.36 mmol) wurde in Dichlormethan (10 ml) gelöst und bei 0°C mit Trifluoressigsäure (2.1 ml, 27 mmol) versetzt. Nachdem das Reaktionsgemisch über Nacht bei Raumtemperatur gerührt worden war, wurde das Lösungsmittel unter Vakuum entfernt. Es wurden 774 mg (>100 % d. Th.) der Titelverbindung erhalten.
LC-MS (Methode 3): Rₜ = 1.14 min; MS (ESIpos): m/z = 328 [M+H]⁺
¹H-NMR (400 MHz, DMSO-d6) δ [ppm]: -0.008 (0.82), 0.008 (0.68), 1.118 (6.81), 1.129 (0.42), 1.161 (2.13), 1.170 (0.42), 1.179 (4.32), 1.186 (0.44), 1.197 (2.17), 1.236 (1.91), 1.399 (0.56), 1.469 (0.47), 1.484 (1.04), 1.497 (1.48), 1.511 (1.77), 1.521 (2.46), 1.537 (2.89), 1.548 (2.11), 1.561 (1.23), 1.568 (1.17), 1.582 (0.67), 1.756 (0.53), 1.807 (2.09), 1.820 (2.94), 1.830 (2.93), 1.841 (2.39), 1.852 (2.37), 1.914 (1.15), 1.990 (7.81), 2.055 (0.59), 2.060 (0.54), 2.073 (1.51), 2.082 (3.23), 2.090 (5.04), 2.104 (8.58), 2.115 (7.88), 2.126 (4.11), 2.563 (3.78), 2.576 (3.69), 2.591 (2.87), 2.626 (3.11), 2.638 (4.92), 2.648 (3.42), 2.668 (1.70), 2.679 (2.33), 2.690 (1.39), 4.008 (0.60), 4.025 (1.83), 4.043 (1.80), 4.061 (0.58), 4.477 (5.29), 4.488 (10.00), 4.501 (5.14), 4.757 (1.78), 4.831 (1.52), 4.872 (16.00), 4.878 (15.58), 4.918 (1.43), 7.264 (6.52), 7.282 (7.05), 7.287 (7.36), 7.291 (7.18), 7.304 (7.27), 7.308 (7.32), 7.313 (6.92), 7.331 (6.44), 7.523 (4.17), 7.540 (4.34), 7.548 (5.42), 7.565 (5.28), 7.574 (4.10), 7.591 (3.71), 7.621 (1.61), 7.648 (1.44), 7.673 (1.15), 7.694 (1.09), 7.711 (1.06), 7.733 (1.00), 7.761 (0.87), 7.832 (0.71), 8.138 (0.92).

### Intermediat 343

### (5S)-2-{2-[3-Chlor-5-(trifluormethyl)pyridin-2-yl]ethyl}-3-oxo-2,3,5,6,7,8-hexahydro[1,2,4]triazolo[4,3-a]pyridin-5-carbonsäure

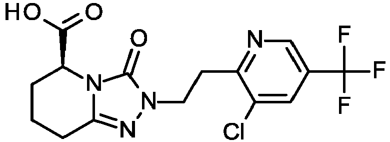

tert-Butyl-(5S)-2-{2-[3-chlor-5-(trifluormethyl)pyridin-2-yl]ethyl}-3-oxo-2,3,5,6,7,8-hexahydro[1,2,4]triazolo[4,3-a]pyridin-5-carboxylat (120 mg, 269 µmol) wurde in Dichlormethan (4.0 ml) gelöst und bei 0°C mit Trifluoressigsäure (410 µl, 5.4 mmol) versetzt. Nachdem das Reaktionsgemisch über Nacht bei Raumtemperatur gerührt worden war, wurde erneut Trifluoressigsäure (300 µl, 3.9 mmol) zugegeben und über Nacht gerührt. Das Lösungsmittel wurde anschließend unter Vakuum entfernt. Es wurden 153 mg (>100 % d. Th.) der Titelverbindung erhalten.
LC-MS (Methode 1): Rₜ = 0.99 min; MS (ESIpos): m/z = 391 [M+H]⁺
¹H-NMR (400 MHz, DMSO-d6) δ [ppm]: -0.150 (0.75), 0.146 (0.75), 0.833 (0.43), 1.038 (0.57), 1.056 (1.21), 1.073 (0.60), 1.110 (2.01), 1.157 (0.57), 1.176 (0.83), 1.236 (1.38), 1.291 (3.19), 1.309 (6.41), 1.327 (3.25), 1.390 (1.90), 1.538 (2.30), 1.566 (1.41), 1.783 (2.07), 1.793 (3.13), 1.805 (3.02), 1.815 (2.61), 1.827 (2.53), 2.047 (4.51), 2.060 (8.10), 2.076 (7.58), 2.089 (3.88), 2.328 (1.64), 2.366 (1.32), 2.522 (9.65), 2.563 (3.56), 2.606 (3.36), 2.618 (5.69), 2.629 (3.53), 2.648 (1.92), 2.660 (3.30), 2.671 (3.02), 2.710 (1.35), 3.291 (7.47), 3.309 (16.00), 3.328 (8.53), 3.432 (0.63), 3.449 (0.69), 4.004 (1.58), 4.022 (2.90), 4.039 (5.49), 4.057 (9.91), 4.081 (9.13), 4.099 (5.34), 4.116 (2.82), 4.135 (1.58), 4.384 (6.49), 4.396 (12.67), 4.408 (6.69), 4.415 (4.71), 4.433 (4.22), 4.450 (2.56), 4.729 (2.82), 8.414 (9.91), 8.881 (10.08).

### Intermediat 344

### (5S)-2-[3-Chlor-4-(trifluormethyl)benzyl]-3-oxo-2,3,5,6,7,8-hexahydro[1,2,4]triazolo[4,3-a]pyridin-5-carbonsäure

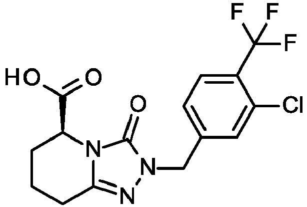

tert-Butyl-(5S)-2-[3-chlor-4-(trifluormethyl)benzyl]-3-oxo-2,3,5,6,7,8-hexahydro[1,2,4]triazolo[4,3-a]pyridin-5-carboxylat (523 mg, 1.21 mmol) wurde in Dichlormethan (10 ml) gelöst und bei 0°C mit Trifluoressigsäure (1.9 ml, 24 mmol) versetzt. Nachdem das Reaktionsgemisch über Nacht bei Raumtemperatur gerührt worden war, wurde das Lösungsmittel unter Vakuum entfernt. Es wurden 175 mg (37 % d. Th.) der Titelverbindung erhalten.
LC-MS (Methode 1): Rₜ = 1.09 min; MS (ESIpos): m/z = 376 [M+H]⁺
¹H-NMR (400 MHz, DMSO-d6) δ [ppm]: -0.008 (2.69), 0.008 (2.18), 0.018 (0.56), 0.837 (0.57), 1.227 (0.51), 1.235 (0.42), 1.460 (0.42), 1.515 (1.26), 1.527 (1.23), 1.543 (1.10), 1.822 (1.58), 1.834 (1.53), 1.844 (1.29), 1.855 (1.31), 2.108 (4.38), 2.118 (4.15), 2.130 (2.21), 2.328 (0.63), 2.366 (0.62), 2.524 (2.14), 2.558 (2.84), 2.573 (1.97), 2.586 (2.06), 2.600 (1.62), 2.631 (1.67), 2.643 (2.69), 2.653 (1.89), 2.674 (1.29), 2.683 (1.20), 2.695 (0.72), 2.710 (0.63), 4.107 (0.72), 4.322 (1.38), 4.490 (3.29), 4.501 (5.97), 4.514 (3.19), 4.588 (0.53), 4.973 (16.00), 7.391 (3.50), 7.412 (3.86), 7.561 (7.11), 7.835 (6.26), 7.855 (5.71).

### Intermediat 345

### (5S)-2-[3-Fluor-4-(trifluormethyl)benzyl]-3-oxo-2,3,5,6,7,8-hexahydro[1,2,4]triazolo[4,3-a]pyridin-5-carbonsäure

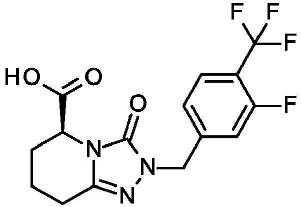

tert-Butyl-(5S)-2-[3-fluor-4-(trifluormethyl)benzyl]-3-oxo-2,3,5,6,7,8-hexahydro[1,2,4]triazolo[4,3-a]pyridin-5-carboxylat (707 mg, 1.70 mmol) wurde in Dichlormethan (10 ml) gelöst und bei 0°C mit Trifluoressigsäure (2.6 ml, 34 mmol) versetzt. Nachdem das Reaktionsgemisch über Nacht bei Raumtemperatur gerührt worden war, wurde das Lösungsmittel unter Vakuum entfernt. Es wurden 611 mg (96 % d. Th.) der Titelverbindung erhalten.
LC-MS (Methode 3): Rₜ = 1.42 min; MS (ESIpos): m/z = 360 [M+H]⁺

### Intermediat 346

### (5S)-2-{[1-(6-Chlorpyridin-2-yl)cyclopropyl]methyl}-3-oxo-2,3,5,6,7,8-hexahydro[1,2,4]triazolo[4,3-a]pyridin-5-carbonsäure

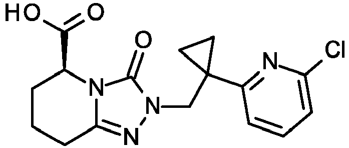

tert-Butyl-(5S)-2-{[1-(6-chlorpyridin-2-yl)cyclopropyl]methyl}-3-oxo-2,3,5,6,7,8-hexahydro[1,2,4]triazolo[4,3-a]pyridin-5-carboxylat (224 mg, 554 µmol) wurde in Dichlormethan (3.0 ml) gelöst und bei 0°C mit Trifluoressigsäure (850 µl, 11 mmol) versetzt. Nachdem das Reaktionsgemisch über Nacht bei Raumtemperatur gerührt worden war, wurde das Lösungsmittel unter Vakuum entfernt. Es wurden 330 mg (>100 % d. Th.) der Titelverbindung erhalten.
LC-MS (Methode 3): Rₜ = 1.20 min; MS (ESIpos): m/z = 349 [M+H]⁺
¹H-NMR (400 MHz, DMSO-d6) δ [ppm]: 0.008 (0.48), 1.113 (1.42), 1.143 (0.85), 1.164 (10.97), 1.176 (15.26), 1.194 (2.29), 1.231 (0.88), 1.278 (0.44), 1.299 (0.48), 1.379 (0.87), 1.450 (0.49), 1.468 (0.79), 1.485 (1.13), 1.499 (1.16), 1.513 (1.06), 1.536 (4.67), 1.545 (0.54), 1.600 (0.68), 1.762 (0.79), 1.770 (1.14), 1.779 (1.55), 1.793 (1.54), 1.803 (1.35), 1.815 (1.28), 1.825 (1.00), 1.989 (5.49), 2.063 (4.19), 2.073 (4.25), 2.470 (0.72), 2.484 (1.00), 2.590 (1.40), 2.600 (2.51), 2.612 (1.97), 2.622 (0.54), 2.631 (0.82), 2.642 (1.22), 2.654 (0.82), 3.784 (0.51), 4.005 (0.47), 4.023 (1.38), 4.040 (1.38), 4.058 (0.74), 4.091 (16.00), 4.369 (0.48), 4.381 (0.93), 4.394 (0.53), 4.419 (2.40), 4.431 (4.64), 4.444 (2.31), 7.239 (4.53), 7.259 (4.97), 7.312 (0.46), 7.582 (4.49), 7.601 (6.06), 7.696 (3.81), 7.716 (6.11), 7.735 (2.62), 11.361 (0.58), 12.173 (0.58).

### Intermediat 347

### (5S)-2-{[1-(4-Fluorphenyl)cyclopropyl]methyl}-3-oxo-2,3,5,6,7,8-hexahydro[1,2,4]triazolo[4,3-a]pyridin-5-carbonsäure

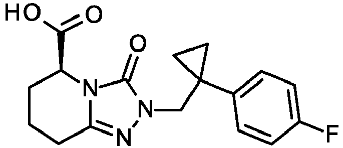

tert-Butyl-(5S)-2-{[1-(4-fluorphenyl)cyclopropyl]methyl}-3-oxo-2,3,5,6,7,8-hexahydro[1,2,4]triazolo[4,3-a]pyridin-5-carboxylat (557 mg, 1.44 mmol) wurde in Dichlormethan (10 ml) gelöst und bei 0°C mit Trifluoressigsäure (2.2 ml, 29 mmol) versetzt. Nachdem das Reaktionsgemisch über Nacht bei Raumtemperatur gerührt worden war, wurde das Lösungsmittel unter Vakuum entfernt. Es wurden 743 mg (>100 % d. Th.) der Titelverbindung erhalten.
LC-MS (Methode 4): Rₜ = 0.73 min; MS (ESIpos): m/z = 332 [M+H]⁺

### Intermediat 348

### (5S)-2-{[1-(4-Methoxyphenyl)cyclopropyl]methyl}-3-oxo-2,3,5,6,7,8-hexahydro[1,2,4]triazolo[4,3-a]pyridin-5-carbonsäure

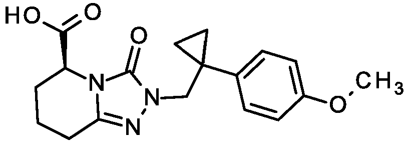

tert-Butyl-(5S)-2-{[1-(4-methoxyphenyl)cyclopropyl]methyl}-3-oxo-2,3,5,6,7,8-hexahydro[1,2,4]triazolo[4,3-a]pyridin-5-carboxylat (222 mg, 555 µmol) wurde in Dichlormethan (3.0 ml) gelöst und bei 0°C mit Trifluoressigsäure (850 µl, 11 mmol) versetzt. Nachdem das Reaktionsgemisch über Nacht bei Raumtemperatur gerührt worden war, wurde das Lösungsmittel unter Vakuum entfernt. Es wurden 373 mg (70 % Reinheit, >100 % d. Th.) der Titelverbindung erhalten.
LC-MS (Methode 1): Rₜ = 0.97 min; MS (ESIpos): m/z = 344 [M+H]⁺
¹H-NMR (400 MHz, DMSO-d6) δ [ppm]: -0.149 (0.45), -0.008 (3.69), 0.008 (3.61), 0.146 (0.45), 0.678 (1.71), 0.689 (1.42), 0.704 (5.29), 0.726 (1.23), 0.752 (5.96), 0.776 (2.07), 0.916 (1.06), 0.926 (0.88), 0.940 (3.89), 0.950 (14.15), 0.962 (3.42), 0.985 (0.74), 1.045 (0.41), 1.056 (0.73), 1.111 (5.66), 1.116 (0.71), 1.149 (0.79), 1.166 (1.71), 1.184 (0.73), 1.228 (1.91), 1.278 (1.68), 1.291 (4.42), 1.309 (8.19), 1.327 (4.14), 1.381 (0.70), 1.507 (1.73), 1.520 (1.61), 1.535 (1.64), 1.568 (0.69), 1.737 (0.63), 1.775 (1.48), 1.788 (2.14), 1.798 (2.10), 1.809 (1.73), 1.820 (1.70), 2.005 (0.41), 2.032 (2.15), 2.040 (3.48), 2.054 (5.96), 2.065 (5.63), 2.076 (3.11), 2.328 (0.71), 2.367 (0.51), 2.474 (1.41), 2.524 (2.92), 2.558 (2.48), 2.599 (2.19), 2.611 (3.64), 2.622 (2.47), 2.641 (1.28), 2.652 (1.75), 2.665 (1.41), 2.711 (0.54), 3.432 (0.50), 3.653 (0.98), 3.737 (8.71), 3.754 (1.02), 3.764 (0.54), 3.771 (0.63), 3.814 (0.58), 3.836 (8.44), 3.872 (5.20), 4.119 (0.45), 4.132 (0.45), 4.137 (0.42), 4.357 (3.69), 4.369 (6.99), 4.381 (3.73), 4.397 (1.29), 4.415 (3.38), 4.433 (3.32), 4.451 (1.21), 4.463 (0.41), 4.472 (0.41), 5.344 (2.22), 6.767 (1.50), 6.774 (13.78), 6.779 (5.45), 6.791 (5.03), 6.796 (16.00), 7.120 (1.81), 7.127 (15.91), 7.132 (5.25), 7.144 (4.97), 7.149 (14.17), 7.156 (2.15), 11.357 (0.40).

### Intermediat 349

### (5S)-3-Oxo-2-({1-[4-(trifluormethyl)phenyl]cyclopropyl}methyl)-2,3,5,6,7,8-hexahydro[1,2,4]triazolo[4,3-a]pyridin-5-carbonsäure

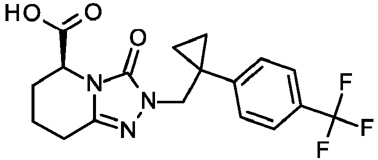

tert-Butyl-(5S)-3-oxo-2-({1 -[4-(trifluormethyl)phenyl]cyclopropyl}methyl)-2,3,5,6,7,8-hexahydro[1,2,4]triazolo[4,3-a]pyridin-5-carboxylat (113 mg, 258 µmol) wurde in Dichlormethan (1.5 ml) gelöst und bei 0°C mit Trifluoressigsäure (400 µl, 5.2 mmol) versetzt. Nachdem das Reaktionsgemisch über Nacht bei Raumtemperatur gerührt worden war, wurde das Lösungsmittel unter Vakuum entfernt. Es wurden 115 mg (88 % Reinheit, >100 % d. Th.) der Titelverbindung erhalten.
LC-MS (Methode 1): Rₜ = 1.14 min; MS (ESIpos): m/z = 382 [M+H]⁺
¹H-NMR (400 MHz, DMSO-d6) δ [ppm]: -0.150 (0.78), 0.008 (6.17), 0.146 (0.86), 0.866 (2.35), 0.893 (4.18), 0.926 (4.29), 0.951 (2.69), 1.038 (1.78), 1.055 (3.61), 1.073 (1.88), 1.110 (8.13), 1.127 (14.72), 1.227 (4.55), 1.291 (7.84), 1.309 (16.00), 1.327 (8.00), 1.358 (1.25), 1.492 (1.75), 1.736 (1.44), 1.785 (2.20), 1.795 (2.14), 1.817 (1.83), 2.052 (6.41), 2.064 (5.80), 2.327 (1.59), 2.366 (0.94), 2.465 (1.52), 2.601 (3.82), 2.612 (2.54), 2.643 (1.86), 2.670 (1.75), 2.710 (0.97), 3.414 (0.65), 3.431 (1.86), 3.449 (1.83), 3.467 (0.71), 3.859 (1.78), 3.895 (13.78), 3.903 (13.73), 3.940 (1.96), 4.371 (7.48), 4.382 (11.03), 4.396 (8.89), 4.415 (10.17), 4.433 (9.93), 4.451 (5.59), 7.438 (9.57), 7.459 (12.99), 7.569 (13.54), 7.589 (10.01).

### Intermediat 350

### (5S)-2-{[1-(2,4-Difluorphenyl)cyclopropyl]methyl}-3-oxo-2,3,5,6,7,8-hexahydro[1,2,4]triazolo[4,3-a]pyridin-5-carbonsäure

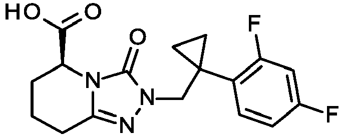

tert-Butyl-(5S)-2-{[1-(2,4-difluorphenyl)cyclopropyl]methyl}-3-oxo-2,3,5,6,7,8-hexahydro[1,2,4]triazolo[4,3-a]pyridin-5-carboxylat (256 mg, 630 µmol) wurde in Dichlormethan (3.7 ml) gelöst und bei 0°C mit Trifluoressigsäure (970 µl, 13 mmol) versetzt. Nachdem das Reaktionsgemisch über Nacht bei Raumtemperatur gerührt worden war, wurde das Lösungsmittel unter Vakuum entfernt. Es wurden 391 mg (81 % Reinheit, >100 % d. Th.) der Titelverbindung erhalten.
LC-MS (Methode 1): Rₜ = 1.01 min; MS (ESIpos): m/z = 350 [M+H]⁺

### Intermediat 351

### (5S)-2-(2,4-Difluorbenzyl)-3-oxo-2,3,5,6,7,8-hexahydro[1,2,4]triazolo[4,3-a]pyridin-5-carbonsäure

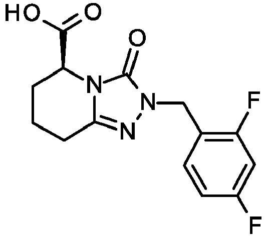

tert-Butyl-(5S)-2-(2,4-difluorbenzyl)-3-oxo-2,3,5,6,7,8-hexahydro[1,2,4]triazolo[4,3-a]pyridin-5-carboxylat (443 mg, 1.21 mmol) wurde in Dichlormethan (10 ml) gelöst und bei Raumtemperatur mit Trifluoressigsäure (1.9 ml, 24 mmol) versetzt. Nachdem das Reaktionsgemisch über Nacht bei Raumtemperatur gerührt worden war, wurde das Lösungsmittel unter Vakuum entfernt. Es wurden 530 mg (>100 % d. Th.) der Titelverbindung erhalten.
LC-MS (Methode 3): Rₜ= 1.10 min; MS (ESIpos): m/z = 310 [M+H]⁺
¹H-NMR (400 MHz, DMSO-d6) δ [ppm]: -0.149 (0.62), -0.008 (6.71), 0.008 (4.59), 0.146 (0.59), 1.038 (0.42), 1.056 (0.82), 1.110 (1.70), 1.122 (0.42), 1.175 (0.48), 1.181 (0.40), 1.227 (0.42), 1.291 (6.89), 1.309 (13.99), 1.327 (6.97), 1.392 (1.44), 1.475 (1.13), 1.488 (1.88), 1.522 (2.43), 1.534 (3.07), 1.550 (1.54), 1.569 (0.80), 1.787 (2.21), 1.799 (3.27), 1.810 (3.22), 1.821 (2.85), 1.832 (2.72), 1.988 (0.80), 2.063 (4.96), 2.075 (8.83), 2.084 (8.61), 2.092 (8.01), 2.104 (4.30), 2.328 (0.73), 2.367 (0.62), 2.570 (3.82), 2.603 (3.86), 2.614 (5.91), 2.625 (4.13), 2.645 (2.19), 2.656 (2.80), 2.668 (2.25), 2.710 (0.82), 3.432 (0.44), 3.449 (0.44), 4.397 (1.83), 4.415 (5.54), 4.433 (5.58), 4.448 (6.82), 4.460 (11.96), 4.473 (6.07), 4.517 (0.42), 4.783 (0.99), 4.805 (3.20), 4.844 (15.73), 4.860 (16.00), 4.899 (3.40), 5.097 (1.19), 5.127 (1.32), 5.475 (1.65), 5.753 (0.97), 7.047 (2.49), 7.052 (2.65), 7.069 (5.60), 7.074 (5.92), 7.090 (3.24), 7.095 (3.35), 7.221 (3.60), 7.227 (3.42), 7.246 (5.56), 7.251 (5.25), 7.271 (3.80), 7.277 (3.66), 7.290 (3.64), 7.307 (4.64), 7.311 (7.06), 7.328 (6.95), 7.349 (3.11).

### Intermediat 352

### (5S)-2-(2-Chlor-4-fluorbenzyl)-3-oxo-2,3,5,6,7,8-hexahydro[1,2,4]triazolo[4,3-a]pyridin-5-carbonsäure

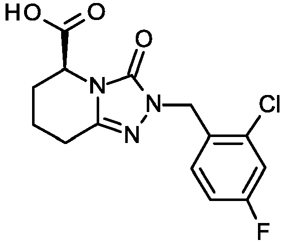

tert-Butyl-(5S)-2-(2-chlor-4-fluorbenzyl)-3-oxo-2,3,5,6,7,8-hexahydro[1,2,4]triazolo[4,3-a]pyridin-5-carboxylat (371 mg, 970 µmol) wurde in Dichlormethan (10 ml) gelöst und bei Raumtemperatur mit Trifluoressigsäure (1.5 ml, 19 mmol) versetzt. Nachdem das Reaktionsgemisch über Nacht bei Raumtemperatur gerührt worden war, wurde das Lösungsmittel unter Vakuum entfernt. Es wurden 580 mg (>100 % d. Th.) der Titelverbindung erhalten.
LC-MS (Methode 3): Rₜ= 1.19 min; MS (ESIpos): m/z = 326 [M+H]⁺
¹H-NMR (400 MHz, DMSO-d6) δ [ppm]: 1.110 (1.55), 1.157 (0.51), 1.175 (1.15), 1.192 (0.58), 1.227 (0.70), 1.404 (1.18), 1.520 (1.43), 1.534 (1.96), 1.548 (1.29), 1.561 (0.93), 1.580 (0.48), 1.802 (1.27), 1.813 (1.85), 1.824 (1.81), 1.836 (1.52), 1.847 (1.48), 1.988 (1.90), 2.073 (4.52), 2.092 (4.73), 2.099 (5.07), 2.108 (4.79), 2.119 (2.69), 2.328 (0.57), 2.366 (0.50), 2.585 (1.84), 2.617 (2.00), 2.628 (3.21), 2.639 (2.14), 2.659 (1.19), 2.670 (2.01), 2.681 (0.96), 2.710 (0.53), 4.021 (0.47), 4.038 (0.48), 4.470 (3.11), 4.482 (5.98), 4.495 (3.09), 4.857 (0.87), 4.898 (16.00), 4.940 (0.98), 5.144 (1.15), 5.158 (1.19), 5.398 (0.94), 5.504 (1.36), 5.753 (0.75), 7.190 (1.16), 7.196 (1.23), 7.211 (3.51), 7.217 (3.73), 7.232 (2.72), 7.238 (2.93), 7.250 (4.64), 7.266 (4.90), 7.287 (2.11), 7.459 (2.87), 7.465 (2.85), 7.481 (2.93), 7.487 (2.91).

### Intermediat 353

### (5S)-2-[(5-Chlor-6-methoxypyridin-3-yl)methyl]-3-oxo-2,3,5,6,7,8-hexahydro[1,2,4]triazolo[4,3-a]pyridin-5-carbonsäure

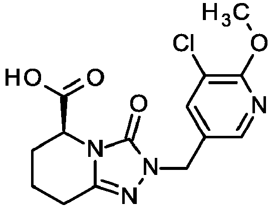

tert-Butyl-(5S)-2-[(5-chlor-6-methoxypyridin-3-yl)methyl]-3-oxo-2,3,5,6,7,8-hexahydro[1,2,4]triazolo[4,3-a]pyridin-5-carboxylat (718 mg, 1.82 mmol) wurde in Dichlormethan (10 ml) gelöst und bei Raumtemperatur mit Trifluoressigsäure (2.8 ml, 36 mmol) versetzt. Nachdem das Reaktionsgemisch über Nacht bei Raumtemperatur gerührt worden war, wurde das Lösungsmittel unter Vakuum entfernt. Es wurden 781 mg (>100 % d. Th.) der Titelverbindung erhalten.
LC-MS (Methode 3): Rₜ = 1.12 min; MS (ESIpos): m/z = 339 [M+H]⁺

### Intermediat 354

### (5S)-2-(4-Fluorbenzyl)-3-oxo-2,3,5,6,7,8-hexahydro[1,2,4]triazolo[4,3-a]pyridin-5-carbonsäure

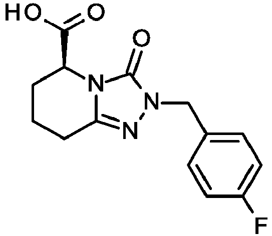

tert-Butyl-(5S)-2-(4-fluorbenzyl)-3-oxo-2,3,5,6,7,8-hexahydro[1,2,4]triazolo[4,3-a]pyridin-5-carboxylat (299 mg, 862 µmol) wurde in Dichlormethan (10 ml) gelöst und bei Raumtemperatur mit Trifluoressigsäure (1.3 ml, 17 mmol) versetzt. Nachdem das Reaktionsgemisch für 4 Stunden bei Raumtemperatur gerührt worden war, wurde das Lösungsmittel unter Vakuum entfernt. Es wurden 397 mg (>100 % d. Th.) der Titelverbindung erhalten.
LC-MS (Methode 3): Rₜ = 1.02 min; MS (ESIpos): m/z = 292 [M+H]⁺

### Intermediat 355

### (5S)-2-[(6-Chlorpyridin-2-yl)methyl]-3-oxo-2,3,5,6,7,8-hexahydro[1,2,4]triazolo[4,3-a]pyridin-5-carbonsäure

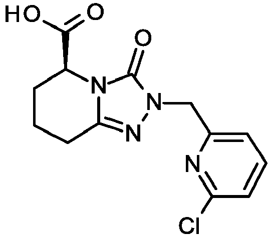

tert-Butyl-(5S)-2-[(6-chlorpyridin-2-yl)methyl]-3-oxo-2,3,5,6,7,8-hexahydro[1,2,4]triazolo[4,3-a]pyridin-5-carboxylat (372 mg, 1.02 mmol) wurde in Dichlormethan (10 ml) gelöst und bei Raumtemperatur mit Trifluoressigsäure (1.6 ml, 20 mmol) versetzt. Nachdem das Reaktionsgemisch über Nacht bei Raumtemperatur gerührt worden war, wurde das Lösungsmittel unter Vakuum entfernt. Es wurden 554 mg (>100 % d. Th.) der Titelverbindung erhalten.
LC-MS (Methode 1): Rₜ = 0.74 min; MS (ESIpos): m/z = 309 [M+H]⁺

### Intermediat 356

### (5S)-2-(4-Methoxybenzyl)-3-oxo-2,3,5,6,7,8-hexahydro[1,2,4]triazolo[4,3-a]pyridin-5-carbonsäure

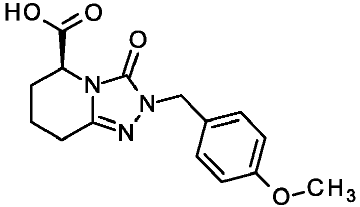

tert-Butyl-(5S)-2-(4-methoxybenzyl)-3-oxo-2,3,5,6,7,8-hexahydro[1,2,4]triazolo[4,3-a]pyridin-5-carboxylat (129 mg, 359 µmol) wurde in Dichlormethan (3.0 ml) gelöst und bei Raumtemperatur mit Trifluoressigsäure (550 µl, 7.2 mmol) versetzt. Nachdem das Reaktionsgemisch über Nacht bei Raumtemperatur gerührt worden war, wurde das Lösungsmittel unter Vakuum entfernt. Es wurden 258 mg (50 % Reinheit, >100 % d. Th.) der Titelverbindung erhalten.
LC-MS (Methode 4): Rₜ = 0.58 min; MS (ESIpos): m/z = 304 [M+H]⁺

### Intermediat 357

### (5S)-2-[(5-Chlor-3-fluorpyridin-2-yl)methyl]-3-oxo-2,3,5,6,7,8-hexahydro[1,2,4]triazolo[4,3-a]pyridin-5-carbonsäure

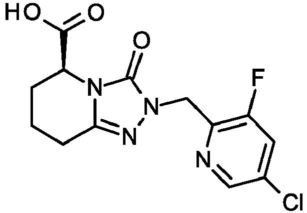

tert-Butyl-(5S)-2-[(5-chlor-3-fluorpyridin-2-yl)methyl]-3-oxo-2,3,5,6,7,8-hexahydro[1,2,4]triazolo[4,3-a]pyridin-5-carboxylat (465 mg, 1.21 mmol) wurde in Dichlormethan (10 ml) gelöst und bei Raumtemperatur mit Trifluoressigsäure (1.9 ml, 24 mmol) versetzt. Nachdem das Reaktionsgemisch über Nacht bei Raumtemperatur gerührt wurden war, wurde erneut Trifluoressigsäure (0.19 ml, 2.4 mmol) zugegeben und 3 Stunden bei Raumtemperatur gerührt. Das Lösungsmittel wurde anschließend unter Vakuum entfernt. Es wurden 813 mg (>100 % d. Th.) der Titelverbindung erhalten.
LC-MS (Methode 3): Rₜ = 0.96 min; MS (ESIpos): m/z = 327 [M+H]⁺

### Intermediat 358

### (5S)-2-[(5-Fluorpyridin-2-yl)methyl]-3-oxo-2,3,5,6,7,8-hexahydro[1,2,4]triazolo[4,3-a]pyridin-5-carbonsäure

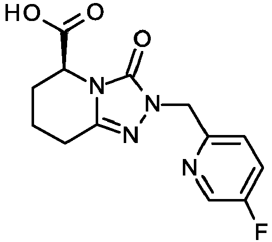

tert-Butyl-(5S)-2-[(5-fluorpyridin-2-yl)methyl]-3-oxo-2,3,5,6,7,8-hexahydro[1,2,4]triazolo[4,3-a]pyridin-5-carboxylat (345 mg, 989 µmol) wurde in Dichlormethan (10 ml) gelöst und bei Raumtemperatur mit Trifluoressigsäure (1.5 ml, 20 mmol) versetzt. Nachdem das Reaktionsgemisch über Nacht bei Raumtemperatur gerührt worden war, wurde das Lösungsmittel unter Vakuum entfernt. Es wurden 549 mg (>100 % d. Th.) der Titelverbindung erhalten.
LC-MS (Methode 4): Rₜ = 0.42 min; MS (ESIpos): m/z = 293 [M+H]⁺

### Intermediat 359

### (5S)-2-(4-Chlor-3-fluorbenzyl)-3-oxo-2,3,5,6,7,8-hexahydro[1,2,4]triazolo[4,3-a]pyridin-5-carbonsäure

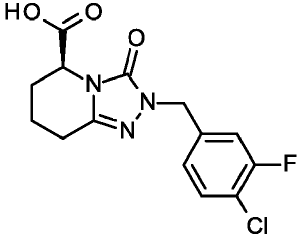

tert-Butyl-(5S)-2-(4-chlor-3-fluorbenzyl)-3-oxo-2,3,5,6,7,8-hexahydro[1,2,4]triazolo[4,3-a]pyridin-5-carboxylat (363 mg, 951 µmol) wurde in Dichlormethan (8.0 ml) gelöst und bei Raumtemperatur mit Trifluoressigsäure (1.5 ml, 19 mmol) versetzt. Nachdem das Reaktionsgemisch über Nacht bei Raumtemperatur gerührt worden war, wurde das Lösungsmittel unter Vakuum entfernt. Es wurden 454 mg (>100 % d. Th.) der Titelverbindung erhalten.
LC-MS (Methode 4): Rₜ = 0.72 min; MS (ESIpos): m/z = 326 [M+H]⁺

### Intermediat 360

### (5S)-2-[(5-Chlorpyridin-3-yl)methyl]-3-oxo-2,3,5,6,7,8-hexahydro[1,2,4]triazolo[4,3-a]pyridin-5-carbonsäure

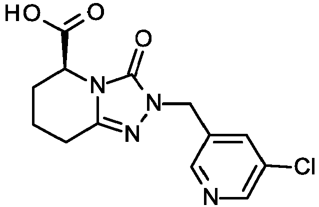

tert-Butyl-(5S)-2-[(5-chlorpyridin-3-yl)methyl]-3-oxo-2,3,5,6,7,8-hexahydro[1,2,4]triazolo[4,3-a]pyridin-5-carboxylat (450 mg, 1.23 mmol) wurde in Dichlormethan (12 ml) gelöst und bei Raumtemperatur mit Trifluoressigsäure (1.9 ml, 25 mmol) versetzt. Nachdem das Reaktionsgemisch über Nacht bei Raumtemperatur gerührt worden war, wurde das Lösungsmittel unter Vakuum entfernt. Es wurden 380 mg (100 % d. Th.) der Titelverbindung erhalten.
LC-MS (Methode 4): Rₜ = 0.5 min; MS (ESIpos): m/z = 309 [M+H]⁺

### Intermediat 361

### (5S)-2-[2-(4-Fluorphenyl)ethyl]-3-oxo-2,3,5,6,7,8-hexahydro[1,2,4]triazolo[4,3-a]pyridin-5-carbonsäure

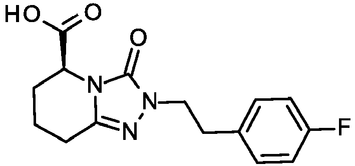

tert-Butyl-(5S)-2-[2-(4-fluorphenyl)ethyl]-3-oxo-2,3,5,6,7,8-hexahydro[1,2,4]triazolo[4,3-a]pyridin-5-carboxylat (340 mg, 941 µmol) wurde in Dichlormethan (10 ml) gelöst und bei Raumtemperatur mit Trifluoressigsäure (1.4 ml, 19 mmol) versetzt. Nachdem das Reaktionsgemisch über Nacht bei Raumtemperatur gerührt worden war, wurde das Lösungsmittel unter Vakuum entfernt. Es wurden 484 mg (>100 % d. Th.) der Titelverbindung erhalten.
LC-MS (Methode 3): Rₜ = 1.13 min; MS (ESIpos): m/z = 306 [M+H]⁺

### Intermediat 362

### (5S)-2-[(5-Methoxypyridin-2-yl)methyl]-3-oxo-2,3,5,6,7,8-hexahydro[1,2,4]triazolo[4,3-a]pyridin-5-carbonsäure

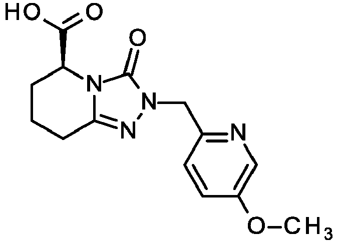

tert-Butyl-(5S)-2-[(5-methoxypyridin-2-yl)methyl]-3-oxo-2,3,5,6,7,8-hexahydro[1,2,4]triazolo[4,3-a]pyridin-5-carboxylat (196 mg, 544 µmol) wurde in Dichlormethan gelöst und bei Raumtemperatur mit Trifluoressigsäure (420 µl, 5.5 mmol) versetzt. Nachdem das Reaktionsgemisch über Nacht bei Raumtemperatur gerührt worden war, wurde erneut Trifluoressigsäure (840 µl, 11 mmol) zugegeben und für 6 Stunden gerührt. Das Lösungsmittel wurde anschließend unter Vakuum entfernt. Es wurden 302 mg (> 100 % d. Th.) der Titelverbindung erhalten.
LC-MS (Methode 1): Rₜ = 0.53 min; MS (ESIpos): m/z = 305 [M+H]⁺

### Intermediat 363

### (5S)-2-[(3,5-Dichlorpyridin-2-yl)methyl]-3-oxo-2,3,5,6,7,8-hexahydro[1,2,4]triazolo[4,3-a]pyridin-5-carbonsäure

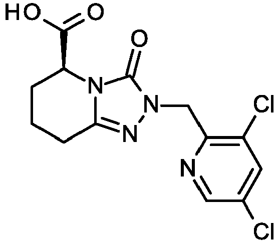

tert-Butyl-(5S)-2-[(3,5-dichlorpyridin-2-yl)methyl]-3-oxo-2,3,5,6,7,8-hexahydro[1,2,4]triazolo[4,3-a]pyridin-5-carboxylat (563 mg, 1.41 mmol) wurde in Dichlormethan (29 ml) gelöst und bei Raumtemperatur mit Trifluoressigsäure (2.2 ml, 28 mmol) versetzt. Nachdem das Reaktionsgemisch über Nacht bei Raumtemperatur gerührt worden war, wurde das Lösungsmittel unter Vakuum entfernt. Es wurden 823 mg (72 % Reinheit, >100 % d. Th.) der Titelverbindung erhalten.
LC-MS (Methode 4): Rₜ = 0.59 min; MS (ESIpos): m/z = 343 [M+H]⁺
¹H-NMR (400 MHz, DMSO-d6) δ [ppm]: -0.008 (1.94), 0.008 (2.31), 1.110 (0.45), 1.228 (0.43), 1.341 (0.41), 1.412 (16.00), 1.522 (0.93), 1.535 (1.80), 1.796 (1.19), 1.807 (1.15), 1.818 (1.05), 1.829 (1.01), 1.988 (0.57), 2.071 (2.07), 2.082 (3.10), 2.092 (2.96), 2.561 (1.34), 2.588 (1.36), 2.599 (2.22), 2.611 (1.52), 2.630 (0.75), 2.641 (1.00), 2.653 (0.60), 2.671 (0.48), 3.914 (1.27), 4.438 (0.62), 4.453 (1.95), 4.466 (3.78), 4.478 (1.88), 4.618 (0.42), 4.855 (1.24), 4.991 (1.12), 5.030 (8.30), 5.041 (9.08), 5.081 (1.19), 6.473 (0.41), 8.250 (4.12), 8.256 (5.12), 8.560 (1.18), 8.567 (5.22), 8.572 (4.55).

### Intermediat 364

### (5S)-2-[2-(4-Methoxyphenyl)ethyl]-3-oxo-2,3,5,6,7,8-hexahydro[1,2,4]triazolo[4,3-a]pyridin-5-carbonsäure

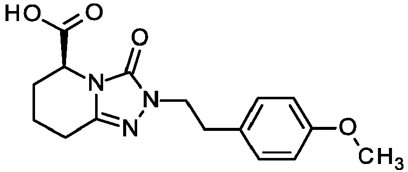

tert-Butyl-(5S)-2-[2-(4-methoxyphenyl)ethyl]-3-oxo-2,3,5,6,7,8-hexahydro[1,2,4]triazolo[4,3-a]pyridin-5-carboxylat (369 mg, 987 µmol) wurde in Dichlormethan (10 ml) gelöst und bei Raumtemperatur mit Trifluoressigsäure (1.5 ml, 20 mmol) versetzt. Nachdem das Reaktionsgemisch über Nacht bei Raumtemperatur gerührt worden war, wurde das Lösungsmittel unter Vakuum entfernt. Es wurden 489 mg (85 % Reinheit, >100 % d. Th.) der Titelverbindung erhalten.
LC-MS (Methode 3): Rₜ = 1.11 min; MS (ESIpos): m/z = 318 [M+H]⁺

### Intermediat 365

### (5S)-2-[(1-Methyl-1H-pyrazolo[3,4-b]pyridin-3-yl)methyl]-3-oxo-2,3,5,6,7,8-hexahydro[1,2,4]triazolo[4,3-a]pyridin-5-carbonsäure

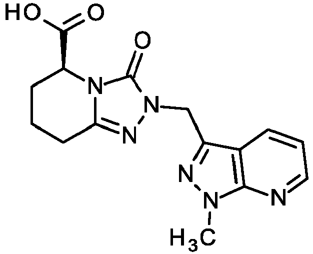

tert-Butyl-(5S)-2-[(1-methyl-1H-pyrazolo[3,4-b]pyridin-3-yl)methyl]-3-oxo-2,3,5,6,7,8-hexahydro[1,2,4]triazolo[4,3-a]pyridin-5-carboxylat (203 mg, 88 % Reinheit, 465 µmol) wurde in Dichlormethan (5.0 ml) gelöst und bei Raumtemperatur mit Trifluoressigsäure (720 µl, 9.3 mmol) versetzt. Nachdem das Reaktionsgemisch über Nacht bei Raumtemperatur gerührt worden war, wurde das Lösungsmittel unter Vakuum entfernt. Es wurden 284 mg (89 % Reinheit, >100 % d. Th.) der Titelverbindung erhalten.
LC-MS (Methode 1): Rₜ = 0.68 min; MS (ESIpos): m/z = 329 [M+H]⁺

### Intermediat 366

### (5S)-3-Oxo-2-{[3-(trifluormethyl)-1,2,4-oxadiazol-5-yl]methyl}-2,3,5,6,7,8-hexahydro[1,2,4]triazolo[4,3-a]pyridin-5-carbonsäure

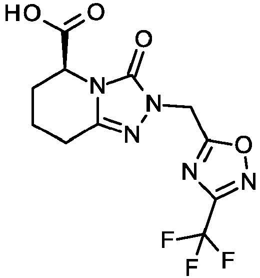

tert-Butyl-(5S)-3-oxo-2-{[3-(trifluormethyl)-1,2,4-oxadiazol-5-yl]methyl}-2,3,5,6,7,8-hexahydro[1,2,4]triazolo[4,3-a]pyridin-5-carboxylat (322 mg, 826 µmol) wurde in Dichlormethan (7.0 ml) gelöst und bei Raumtemperatur mit Trifluoressigsäure (1.3 ml, 17 mmol) versetzt. Nachdem das Reaktionsgemisch über Nacht bei Raumtemperatur gerührt worden war, wurde das Lösungsmittel unter Vakuum entfernt. Es wurden 344 mg (82 % Reinheit, >100 % d. Th.) der Titelverbindung erhalten.
LC-MS (Methode 1): Rₜ = 0.84 min; MS (ESIpos): m/z = 334 [M+H]⁺

### Intermediat 367

### (5S)-2-[(3-Chlorpyridin-2-yl)methyl]-3-oxo-2,3,5,6,7,8-hexahydro[1,2,4]triazolo[4,3-a]pyridin-5-carbonsäure

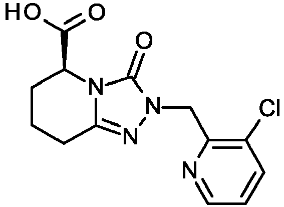

tert-Butyl-(5S)-2-[(3-chlorpyridin-2-yl)methyl]-3-oxo-2,3,5,6,7,8-hexahydro[1,2,4]triazolo[4,3-a]pyridin-5-carboxylat (268 mg, 735 µmol) wurde in Dichlormethan (6.0 ml) gelöst und bei Raumtemperatur mit Trifluoressigsäure (1.1 ml, 15 mmol) versetzt. Nachdem das Reaktionsgemisch über Nacht bei Raumtemperatur gerührt worden war, wurde das Lösungsmittel unter Vakuum entfernt. Es wurden 409 mg (>100 % d. Th.) der Titelverbindung erhalten.
LC-MS (Methode 3): Rₜ = 0.79 min; MS (ESIpos): m/z = 309 [M+H]⁺
¹H-NMR (400 MHz, DMSO-d6) δ [ppm]: -0.008 (2.47), 0.008 (2.02), 1.111 (1.99), 1.228 (0.41), 1.415 (2.49), 1.513 (1.50), 1.535 (7.93), 1.551 (1.72), 1.564 (1.47), 1.577 (1.09), 1.592 (0.52), 1.787 (1.50), 1.798 (2.36), 1.809 (2.25), 1.820 (2.06), 1.831 (1.96), 1.844 (1.39), 2.074 (3.75), 2.086 (6.76), 2.097 (6.56), 2.110 (3.53), 2.368 (0.55), 2.482 (1.65), 2.524 (4.66), 2.565 (2.75), 2.588 (2.68), 2.600 (4.31), 2.612 (2.90), 2.631 (1.42), 2.641 (1.91), 2.654 (1.08), 2.672 (0.46), 2.712 (0.64), 4.458 (4.22), 4.470 (8.12), 4.482 (4.10), 4.993 (3.18), 5.033 (15.85), 5.050 (16.00), 5.090 (3.22), 5.642 (0.42), 6.764 (0.71), 7.378 (5.76), 7.389 (5.97), 7.398 (6.17), 7.410 (6.32), 7.926 (6.28), 7.929 (6.34), 7.946 (6.01), 7.949 (5.82), 8.468 (6.23), 8.472 (6.05), 8.480 (6.31), 8.483 (5.78).

### Intermediat 368

### (5S)-2-[(3,5-Difluorpyridin-2-yl)methyl]-3-oxo-2,3,5,6,7,8-hexahydro[1,2,4]triazolo[4,3-a]pyridin-5-carbonsäure

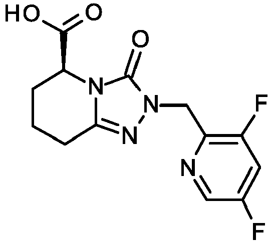

tert-Butyl-(5S)-2-[(3,5-difluorpyridin-2-yl)methyl]-3-oxo-2,3,5,6,7,8-hexahydro[1,2,4]triazolo[4,3-a]pyridin-5-carboxylat (90.1 mg, 246 µmol) wurde in Dichlormethan (2.0 ml) gelöst und bei Raumtemperatur mit Trifluoressigsäure (380 µl, 4.9 mmol) versetzt. Nachdem das Reaktionsgemisch über Nacht bei Raumtemperatur gerührt worden war, wurde das Lösungsmittel unter Vakuum entfernt. Es wurden 103 mg (90 % Reinheit, >100 % d. Th.) der Titelverbindung erhalten.
LC-MS (Methode 1): Rₜ = 0.64 min; MS (ESIpos): m/z = 311 [M+H]⁺

### Intermediat 369

### (5S)-3-Oxo-2-{[6-(trifluormethyl)pyridin-2-yl]methyl}-2,3,5,6,7,8-hexahydro[1,2,4]triazolo[4,3-a]pyridin-5-carbonsäure

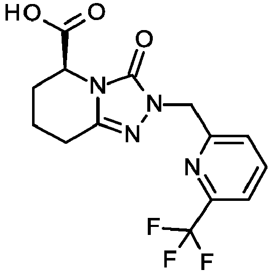

tert-Butyl-(5S)-3-oxo-2-{[6-(trifluormethyl)pyridin-2-yl]methyl}-2,3,5,6,7,8-hexahydro[1,2,4]triazolo[4,3-a]pyridin-5-carboxylat (256 mg, 89 % Reinheit, 571 µmol) wurde in Dichlormethan (5.0 ml) gelöst und bei Raumtemperatur mit Trifluoressigsäure (880 µl, 11 mmol) versetzt. Nachdem das Reaktionsgemisch über Nacht bei Raumtemperatur gerührt worden war, wurde das Lösungsmittel unter Vakuum entfernt. Es wurden 256 mg (85 % Reinheit, >100 % d. Th.) der Titelverbindung erhalten.
LC-MS (Methode 4): Rₜ = 0.61 min; MS (ESIpos): m/z = 343 [M+H]⁺

### Intermediat 370

### (5S)-3-Oxo-2-{[5-(trifluormethyl)-1,3,4-oxadiazol-2-yl]methyl}-2,3,5,6,7,8-hexahydro[1,2,4]triazolo[4,3-a]pyridin-5-carbonsäure

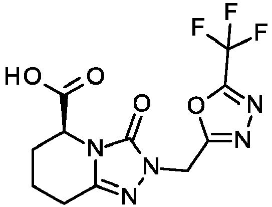

tert-Butyl-(5S)-3-oxo-2-{[5-(trifluormethyl)-1,3,4-oxadiazol-2-yl]methyl}-2,3,5,6,7,8-hexahydro[1,2,4]triazolo[4,3-a]pyridin-5-carboxylat (735 mg, 66 % Reinheit, 1.25 mmol) wurde in Dichlormethan (10 ml) gelöst und bei Raumtemperatur mit Trifluoressigsäure (1.9 ml, 25 mmol) versetzt. Nachdem das Reaktionsgemisch für 4 Tage bei Raumtemperatur gerührt worden war, wurde das Lösungsmittel unter Vakuum entfernt. Es wurden 415 mg (80 % Reinheit, 80 % d. Th.) der Titelverbindung erhalten.
LC-MS (Methode 1): Rₜ = 0.71 min; MS (ESIpos): m/z = 334 [M+H]⁺

### Intermediat 371

### (5S)-2-{[1-Ethyl-3-(trifluormethyl)-1H-pyrazol-5-yl]methyl}-3-oxo-2,3,5,6,7,8-hexahydro[1,2,4]triazolo[4,3-a]pyridin-5-carbonsäure

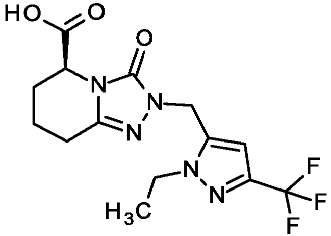

tert-Butyl-(5S)-2-{[1-ethyl-3-(trifluormethyl)-1H-pyrazol-5-yl]methyl}-3-oxo-2,3,5,6,7,8-hexahydro[1,2,4]triazolo[4,3-a]pyridin-5-carboxylat (283 mg, 89 % Reinheit, 606 µmol) wurde in Dichlormethan (5.0 ml) gelöst und bei Raumtemperatur mit Trifluoressigsäure (930 µl, 12 mmol) versetzt. Nachdem das Reaktionsgemisch über Nacht bei Raumtemperatur gerührt worden war, wurde das Lösungsmittel unter Vakuum entfernt. Es wurden 400 mg (>100 % d. Th.) der Titelverbindung erhalten.
LC-MS (Methode 4): Rₜ = 0.68 min; MS (ESIpos): m/z = 360 [M+H]⁺

### Intermediat 372

### (5S)-2-[(5-Chlorpyridin-2-yl)methyl]-3-oxo-2,3,5,6,7,8-hexahydro[1,2,4]triazolo[4,3-a]pyridin-5-carbonsäure

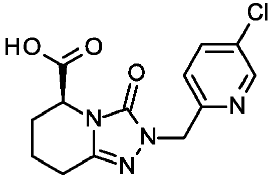

tert-Butyl-(5S)-2-[(5-chlorpyridin-2-yl)methyl]-3-oxo-2,3,5,6,7,8-hexahydro[1,2,4]triazolo[4,3-a]pyridin-5-carboxylat (450 mg, 1.23 mmol) wurde in Dichlormethan (10 ml) gelöst und bei Raumtemperatur mit Trifluoressigsäure (1.9 ml, 25 mmol) versetzt. Nachdem das Reaktionsgemisch über Nacht bei Raumtemperatur gerührt worden war, wurde das Lösungsmittel unter Vakuum entfernt. Es wurden 380 mg (66 % Reinheit, 66 % d. Th.) der Titelverbindung erhalten.
LC-MS (Methode 4): Rₜ = 0.51 min; MS (ESIpos): m/z = 309 [M+H]⁺

### Intermediat 373

### (5S)-3-Oxo-2-{[2-(trifluormethyl)-1,3-thiazol-4-yl]methyl}-2,3,5,6,7,8-hexahydro[1,2,4]triazolo[4,3-a]pyridin-5-carbonsäure

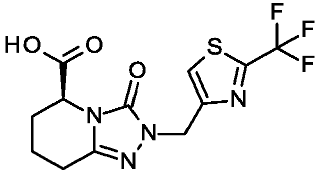

tert-Butyl-(5S)-3-oxo-2-{[2-(trifluormethyl)-1,3-thiazol-4-yl]methyl}-2,3,5,6,7,8-hexahydro[1,2,4]triazolo[4,3-a]pyridin-5-carboxylat (500 mg, 1.24 mmol) wurde in Dichlormethan (8.0 ml) gelöst und bei Raumtemperatur mit Trifluoressigsäure (1.9 ml, 25 mmol) versetzt. Nachdem das Reaktionsgemisch über Nacht bei Raumtemperatur gerührt worden war, wurde das Lösungsmittel unter Vakuum entfernt. Es wurden 430 mg (73 % Reinheit, 73 % d. Th.) der Titelverbindung erhalten.
LC-MS (Methode 4): Rₜ = 0.62 min; MS (ESIpos): m/z = 349 [M+H]⁺

### Intermediat 374

### (5S)-2-{[1-Methyl-5-(trifluormethyl)-1H-pyrazol-3-yl]methyl}-3-oxo-2,3,5,6,7,8-hexahydro[1,2,4]triazolo[4,3-a]pyridin-5-carbonsäure

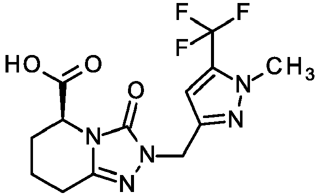

tert-Butyl-(5S)-2-{[1-methyl-5-(trifluormethyl)-1H-pyrazol-3-yl]methyl}-3-oxo-2,3,5,6,7,8-hexahydro[1,2,4]triazolo[4,3-a]pyridin-5-carboxylat (92.0 mg, 229 µmol) wurde in Dichlormethan (2.0 ml) gelöst und bei Raumtemperatur mit Trifluoressigsäure (350 µl, 4.6 mmol) versetzt. Nachdem das Reaktionsgemisch für 72 Stunden bei Raumtemperatur gerührt worden war, wurde das Lösungsmittel unter Vakuum entfernt. Es wurden 79.0 mg (Reinheit 70%, 100 % d. Th.) der Titelverbindung erhalten.
LC-MS (Methode 3): Rₜ = 1.07 min; MS (ESIpos): m/z = 346 [M+H]⁺

### Intermediat 375

### (5S)-2-{[1-Benzyl-5-(trifluormethyl)-1H-pyrazol-3-yl]methyl}-3-oxo-2,3,5,6,7,8-hexahydro[1,2,4]triazolo[4,3-a]pyridin-5-carbonsäure

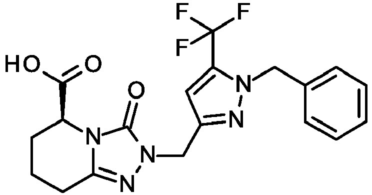

tert-Butyl-(5S)-2-{[1-benzyl-5-(trifluormethyl)-1H-pyrazol-3-yl]methyl}-3-oxo-2,3,5,6,7,8-hexahydro[1,2,4]triazolo[4,3-a]pyridin-5-carboxylat (781 mg, 75 % Reinheit, 1.23 mmol) wurde in Dichlormethan (10 ml) gelöst und bei Raumtemperatur mit Trifluoressigsäure (1.9 ml, 25 mmol) versetzt. Nachdem das Reaktionsgemisch für 72 Stunden bei Raumtemperatur gerührt worden war, wurde erneut Trifluoressigsäure (1.0 ml, 12.4 mmol) zugegeben und das Reaktionsgemisch 2 Stunden bei 40°C gerührt. Das Lösungsmittel wurde anschließend unter Vakuum entfernt. Es wurden 976 mg (70 % Reinheit,>100 % d. Th.) der Titelverbindung erhalten.
LC-MS (Methode 1): Rₜ = 1.11 min; MS (ESIpos): m/z = 422 [M+H]⁺

### Intermediat 376

### (5S)-2-[(5-Brompyridin-2-yl)methyl]-3-oxo-2,3,5,6,7,8-hexahydro[1,2,4]triazolo[4,3-a]pyridin-5-carbonsäure

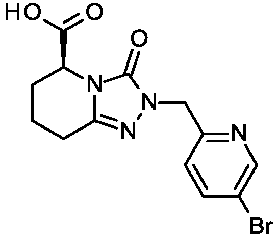

tert-Butyl-(5S)-2-[(5-brompyridin-2-yl)methyl]-3-oxo-2,3,5,6,7,8-hexahydro[1,2,4]triazolo[4,3-a]pyridin-5-carboxylat (580 mg, 1.42 mmol) wurde in Dichlormethan (91 µl) gelöst und bei Raumtemperatur mit Trifluoressigsäure (2.2 ml, 28 mmol) versetzt. Nachdem das Reaktionsgemisch über Nacht bei Raumtemperatur gerührt worden war, wurde das Lösungsmittel unter Vakuum entfernt. Es wurden 1.01 g (> 100% d. Th.) der Titelverbindung erhalten.
LC-MS (Methode 3): Rₜ = 0.90 min; MS (ESIpos): m/z = 353 [M+H]⁺
¹H-NMR (400 MHz, DMSO-d6) δ [ppm]: -0.149 (1.03), 0.146 (1.10), 1.110 (0.61), 1.176 (0.61), 1.192 (0.61), 1.227 (0.49), 1.409 (2.10), 1.535 (2.52), 1.819 (2.50), 1.830 (2.38), 1.841 (2.08), 1.852 (2.01), 1.988 (0.79), 2.098 (6.34), 2.107 (6.48), 2.328 (1.12), 2.367 (0.79), 2.564 (3.17), 2.577 (3.13), 2.591 (2.47), 2.619 (2.64), 2.630 (4.34), 2.641 (2.94), 2.661 (1.68), 2.671 (2.99), 2.711 (0.82), 4.473 (4.15), 4.485 (8.00), 4.498 (4.10), 4.528 (0.72), 4.769 (4.73), 4.867 (2.29), 4.908 (15.70), 4.919 (16.00), 4.960 (2.43), 5.224 (0.98), 5.831 (1.91), 7.162 (8.61), 7.183 (9.17), 7.532 (0.89), 7.552 (0.98), 8.024 (5.67), 8.030 (5.78), 8.045 (5.62), 8.051 (5.67), 8.102 (0.70), 8.117 (0.65), 8.654 (7.14), 8.659 (7.02), 8.697 (0.63).

### Intermediat 377

### (5S)-2-[(5-Brompyridin-3-yl)methyl]-3-oxo-2,3,5,6,7,8-hexahydro[1,2,4]triazolo[4,3-a]pyridin-5-carbonsäure

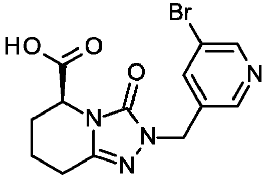

tert-Butyl-(5S)-2-[(5-brompyridin-3-yl)methyl]-3-oxo-2,3,5,6,7,8-hexahydro[1,2,4]triazolo[4,3-a]pyridin-5-carboxylat (146 mg, 357 µmol) wurde in Dichlormethan (2.9 ml) gelöst und bei Raumtemperatur mit Trifluoressigsäure (550 µl, 7.1 mmol) versetzt. Nachdem das Reaktionsgemisch über Nacht bei Raumtemperatur gerührt worden war, wurde das Lösungsmittel unter Vakuum entfernt. Es wurden 199 mg (89 % Reinheit, >100% % d. Th.) der Titelverbindung erhalten.
LC-MS (Methode 4): Rₜ = 0.52 min; MS (ESIpos): m/z = 353 [M+H]⁺
¹H-NMR (400 MHz, DMSO-d6) δ [ppm]: -0.008 (2.87), 0.008 (2.73), 1.111 (0.85), 1.226 (0.54), 1.244 (2.70), 1.260 (2.67), 1.274 (1.67), 1.391 (9.28), 1.509 (1.14), 1.522 (1.13), 1.535 (3.08), 1.815 (1.43), 1.825 (1.40), 1.836 (1.22), 1.847 (1.20), 2.093 (3.51), 2.100 (3.75), 2.109 (3.48), 2.121 (1.86), 2.328 (0.61), 2.524 (2.13), 2.568 (1.92), 2.581 (1.92), 2.595 (1.53), 2.627 (1.61), 2.639 (2.57), 2.650 (1.73), 2.670 (1.47), 2.679 (1.38), 2.692 (0.70), 4.479 (2.53), 4.491 (4.87), 4.503 (2.51), 4.893 (0.40), 4.934 (16.00), 4.974 (0.48), 5.839 (1.06), 7.703 (0.95), 7.893 (3.04), 7.897 (5.36), 7.903 (3.24), 7.919 (0.56), 8.465 (5.32), 8.469 (5.56), 8.643 (4.66), 8.649 (4.90), 9.090 (0.42).

### Intermediat 378

### (5S)-2-[2-(4-Methylphenyl)-2-oxoethyl]-3-oxo-2,3,5,6,7,8-hexahydro[1,2,4]triazolo[4,3-a]pyridin-5-carbonsäure

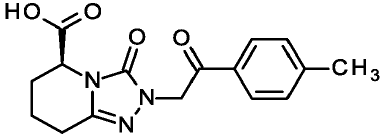

tert-Butyl-(5S)-2-[2-(4-methylphenyl)-2-oxoethyl]-3-oxo-2,3,5,6,7,8-hexahydro[1,2,4]triazolo[4,3-a]pyridin-5-carboxylat (226 mg, 99 % Reinheit, 603 µmol) wurde in Dichlormethan (9.0 ml) gelöst und bei Raumtemperatur mit Trifluoressigsäure (930 µl, 12 mmol) versetzt. Nachdem das Reaktionsgemisch über Nacht bei Raumtemperatur gerührt worden war, wurde das Lösungsmittel unter Vakuum entfernt. Es wurden 190 mg (100 % d. Th.) der Titelverbindung erhalten.
LC-MS (Methode 1): Rₜ = 0.86 min; MS (ESIpos): m/z = 316 [M+H]⁺
¹H-NMR (400 MHz, DMSO-d6) δ [ppm]: 0.008 (2.13), 1.110 (1.66), 1.419 (0.45), 1.535 (2.08), 1.566 (0.73), 1.577 (0.67), 1.828 (0.81), 1.861 (0.67), 1.988 (0.60), 2.098 (1.30), 2.109 (2.54), 2.121 (2.51), 2.328 (0.50), 2.366 (0.49), 2.398 (16.00), 2.580 (1.52), 2.594 (1.27), 2.607 (1.36), 2.621 (1.07), 2.635 (1.10), 2.647 (1.65), 2.658 (1.19), 2.687 (0.71), 2.710 (0.63), 4.466 (1.43), 4.480 (2.76), 4.491 (1.53), 5.221 (11.53), 7.359 (4.46), 7.380 (5.04), 7.912 (5.54), 7.933 (5.39).

### Intermediat 379

### (5RS,8RS)-8-Methyl-2-(4-methylbenzyl)-3-oxo-2,3,5,6,7,8-hexahydro[1,2,4]triazolo[4,3-a]pyridin-5-carbonsäure (Diastereomerengemisch; 4 Isomere)

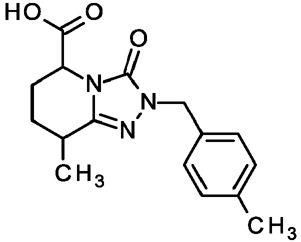

tert-Butyl-(5RS,8RS)-8-methyl-2-(4-methylbenzyl)-3-oxo-2,3,5,6,7,8-hexahydro[1,2,4]triazolo[4,3-a]pyridin-5-carboxylat (Diastereomerengemisch; 4 Isomere) (39.5 mg, 111 µmol) wurde in Dichlormethan (2.0 ml) gelöst und bei Raumtemperatur mit Trifluoressigsäure (2.0 ml, 26 mmol) versetzt. Nachdem das Reaktionsgemisch über Nacht bei Raumtemperatur gerührt worden war, wurde das Lösungsmittel unter Vakuum entfernt. Der Rückstand wurde mit Wasser und Dichlormethan versetzt. Die organische Phase wurde abgetrennt und die wässrige Phase dreimal mit Dichlormethan extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 27.2 mg (82 % d. Th.) der Titelverbindung erhalten.
LC-MS (Methode 3): Rₜ = 1.33 min; MS (ESIpos): m/z = 302 [M+H]⁺
¹H-NMR (400 MHz, DMSO-d6) δ [ppm]: -0.008 (4.03), 0.008 (2.37), 0.854 (0.43), 1.162 (6.13), 1.179 (6.09), 1.194 (1.00), 1.200 (0.78), 1.210 (0.80), 1.235 (3.03), 1.259 (1.66), 1.298 (1.05), 1.878 (0.53), 1.888 (0.41), 1.900 (0.43), 2.074 (0.43), 2.110 (0.53), 2.141 (1.26), 2.148 (1.20), 2.245 (0.73), 2.271 (11.10), 2.327 (0.46), 2.518 (2.94), 2.523 (2.66), 2.669 (0.61), 2.674 (0.46), 2.685 (0.45), 2.700 (0.64), 2.709 (0.70), 2.716 (0.77), 2.731 (0.58), 4.471 (0.92), 4.478 (1.27), 4.487 (0.82), 4.492 (0.88), 4.739 (0.98), 4.778 (2.39), 4.825 (2.35), 4.864 (1.01), 5.754 (2.62), 7.105 (0.61), 7.127 (16.00), 7.145 (0.72).

### Intermediat 380

### (5S)-2-(Cyclopropylmethyl)-3-oxo-2,3,5,6,7,8-hexahydro[1,2,4]triazolo[4,3-a]pyridin-5-carbonsäure

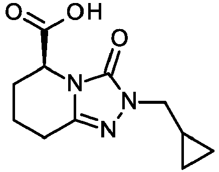

tert-Butyl-(5S)-2-(cyclopropylmethyl)-3-oxo-2,3,5,6,7,8-hexahydro[1,2,4]triazolo[4,3-a]pyridin-5-carboxylat (174 mg, 593 µmol) wurde in Dichlormethan (6.0 ml) gelöst und unter Eiskühlung mit Trifluoressigsäure (3.0 ml, 39 mmol) versetzt. Nachdem das Reaktionsgemisch über Nacht bei Raumtemperatur gerührt worden war, wurde das Lösungsmittel unter Vakuum entfernt. Es wurden 231 mg (>100 % d. Th.) der Titelverbindung erhalten.
LC-MS (Methode 3): Rₜ = 0.73 min; MS (ESIpos): m/z = 238 [M+H]⁺
¹H-NMR (400 MHz, DMSO-d6) δ [ppm]: 0.008 (2.18), 0.262 (1.91), 0.274 (8.08), 0.285 (8.53), 0.297 (2.52), 0.313 (0.57), 0.427 (1.63), 0.438 (8.30), 0.442 (7.40), 0.458 (8.82), 0.475 (1.47), 0.488 (0.45), 1.034 (0.65), 1.039 (0.94), 1.051 (1.82), 1.059 (1.78), 1.071 (2.90), 1.083 (1.70), 1.088 (1.77), 1.101 (0.92), 1.111 (3.14), 1.403 (1.07), 1.493 (0.71), 1.513 (1.50), 1.527 (1.76), 1.540 (1.95), 1.550 (1.73), 1.564 (1.44), 1.578 (1.34), 1.590 (0.60), 1.806 (2.26), 1.817 (2.13), 1.828 (2.01), 1.839 (1.90), 1.852 (1.35), 2.060 (3.96), 2.072 (7.12), 2.083 (6.87), 2.097 (3.54), 2.105 (1.98), 2.558 (4.03), 2.572 (2.98), 2.585 (3.06), 2.599 (2.54), 2.628 (2.55), 2.639 (4.08), 2.651 (2.76), 2.670 (1.62), 2.681 (1.95), 2.693 (1.10), 3.482 (16.00), 3.499 (15.83), 4.377 (0.51), 4.409 (3.82), 4.422 (7.29), 4.434 (3.82), 11.357 (0.41).

### Intermediat 381

### (5S)-2-[(E)-2-(4-Fluorphenyl)vinyl]-3-oxo-2,3,5,6,7,8-hexahydro[1,2,4]triazolo[4,3-a]pyridin-5-carbonsäure

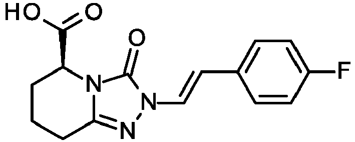

tert-Butyl-(5S)-2-[(E)-2-(4-fluorphenyl)vinyl]-3-oxo-2,3,5,6,7,8-hexahydro[1,2,4]triazolo[4,3-a]pyridin-5-carboxylat (150 mg, 417 µmol) wurde in Dichlormethan (5.0 ml) gelöst und bei Raumtemperatur mit Trifluoressigsäure (500 µl, 6.5 mmol) versetzt. Nachdem das Reaktionsgemisch über Nacht bei Raumtemperatur gerührt worden war, wurde das Lösungsmittel unter Vakuum entfernt. Es wurden 198 mg (>100 % d. Th.) der Titelverbindung erhalten.
LC-MS (Methode 3): Rₜ = 1.37 min; MS (ESIpos): m/z = 304 [M+H]⁺
¹H-NMR (400 MHz, DMSO-d6) δ [ppm]: -0.149 (0.58), -0.008 (5.47), 0.008 (4.55), 0.146 (0.64), 1.030 (0.46), 1.045 (0.49), 1.106 (2.56), 1.110 (3.05), 1.176 (0.43), 1.235 (0.52), 1.311 (2.50), 1.326 (2.02), 1.338 (2.38), 1.423 (1.31), 1.505 (0.82), 1.535 (12.52), 1.554 (1.92), 1.566 (1.80), 1.744 (0.46), 1.865 (2.23), 1.875 (2.20), 1.887 (1.98), 1.897 (1.86), 1.909 (1.40), 2.111 (3.66), 2.123 (6.38), 2.133 (6.11), 2.328 (1.16), 2.367 (1.25), 2.524 (3.18), 2.625 (1.16), 2.640 (1.50), 2.652 (1.25), 2.668 (4.21), 2.683 (2.81), 2.695 (2.90), 2.710 (3.30), 2.739 (2.38), 2.751 (3.97), 2.762 (2.63), 2.782 (1.28), 2.793 (1.83), 2.806 (0.95), 4.114 (3.30), 4.525 (5.50), 4.538 (9.65), 4.550 (5.16), 5.754 (1.40), 6.751 (8.85), 6.788 (9.83), 7.121 (7.97), 7.143 (16.00), 7.166 (8.61), 7.384 (11.48), 7.420 (10.23), 7.542 (8.09), 7.556 (9.16), 7.564 (8.52), 7.578 (7.27).

### Intermediat 382

### (5S)-2-{[6-(Difluormethyl)pyridin-3-yl]methyl}-3-oxo-2,3,5,6,7,8-hexahydro[1,2,4]triazolo[4,3-a]pyridin-5-carbonsäure

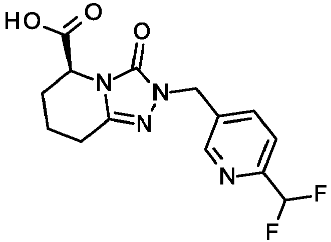

tert-Butyl-(5S)-2-{[6-(difluormethyl)pyridin-3-yl]methyl}-3-oxo-2,3,5,6,7,8-hexahydro[1,2,4]triazolo[4,3-a]pyridin-5-carboxylat (200 mg, 86 % Reinheit, 452 µmol) wurde in Dichlormethan (4.5 ml) gelöst und bei Raumtemperatur mit Trifluoressigsäure (2.0 ml, 26 mmol) versetzt. Nachdem das Reaktionsgemisch für 3.5 Stunden bei Raumtemperatur gerührt worden war, wurde das Lösungsmittel unter Vakuum entfernt. Es wurden 615 mg (25 % Reinheit, >100 % d. Th.) der Titelverbindung erhalten.
LC-MS (Methode 3): Rₜ = 0.87 min; MS (ESIpos): m/z = 325 [M+H]⁺

### Intermediat 383

### (5S)-2-(4-Brombenzyl)-3-oxo-2,3,5,6,7,8-hexahydro[1,2,4]triazolo[4,3-a]pyridin-5-carbonsäure

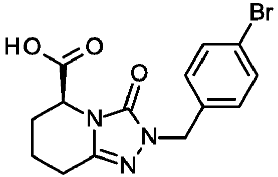

tert-Butyl-(5S)-2-(4-brombenzyl)-3-oxo-2,3,5,6,7,8-hexahydro[1,2,4]triazolo[4,3-a]pyridin-5-carboxylat (201 mg, 74 % Reinheit, 365 µmol) wurde in Dichlormethan (3.7 ml) gelöst und bei Raumtemperatur mit Trifluoressigsäure (500 µl, 6.5 mmol) versetzt. Nachdem das Reaktionsgemisch über Nacht bei Raumtemperatur gerührt worden war, wurde das Lösungsmittel unter Vakuum entfernt. Es wurden 268 mg (47 % Reinheit, 98 % d. Th.) der Titelverbindung erhalten.
LC-MS (Methode 4): Rₜ = 0.69 min; MS (ESIpos): m/z = 352 [M+H]⁺

### Intermediat 384

### (5S)-2-(3-Brombenzyl)-3-oxo-2,3,5,6,7,8-hexahydro[1,2,4]triazolo[4,3-a]pyridin-5-carbonsäure

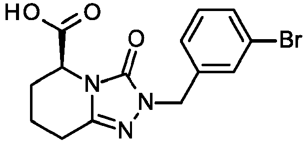

tert-Butyl-(5S)-2-(3-brombenzyl)-3-oxo-2,3,5,6,7,8-hexahydro[1,2,4]triazolo[4,3-a]pyridin-5-carboxylat (256 mg, 590 µmol) wurde in Dichlormethan (4.5 ml) gelöst und bei Raumtemperatur mit Trifluoressigsäure (500 µl, 6.5 mmol) versetzt. Nachdem das Reaktionsgemisch über Nacht bei Raumtemperatur gerührt worden war, wurde das Lösungsmittel unter Vakuum entfernt. Es wurden 346 mg (59 % Reinheit, 98 % d. Th.) der Titelverbindung erhalten.
LC-MS (Methode 4): Rₜ = 0.69 min; MS (ESIpos): m/z = 352 [M+H]⁺

### Intermediat 385

### (5S)-2-(4-Brom-2-fluorbenzyl)-3-oxo-2,3,5,6,7,8-hexahydro[1,2,4]triazolo[4,3-a]pyridin-5-carbonsäure

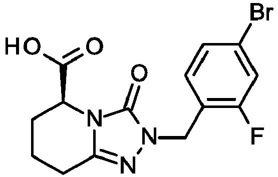

tert-Butyl-(5S)-2-(4-brom-2-fluorbenzyl)-3-oxo-2,3,5,6,7,8-hexahydro[1,2,4]triazolo[4,3-a]pyridin-5-carboxylat (135 mg, 317 µmol) wurde in Dichlormethan (3.0 ml) gelöst und bei Raumtemperatur mit Trifluoressigsäure (300 µl, 3.9 mmol) versetzt. Nachdem das Reaktionsgemisch über Nacht bei Raumtemperatur gerührt worden war, wurde das Lösungsmittel unter Vakuum entfernt. Es wurden 174 mg (>100 % d. Th.) der Titelverbindung erhalten.
LC-MS (Methode 3): Rₜ = 1.31 min; MS (ESIpos): m/z = 370 [M+H]⁺

### Intermediat 386

### (5S)-3-Oxo-2-{(1RS)-1-[6-(trifluormethyl)pyridin-3-yl]ethyl}-2,3,5,6,7,8-hexahydro[1,2,4]triazolo[4,3-a]pyridin-5-carbonsäure (Isomer 1)

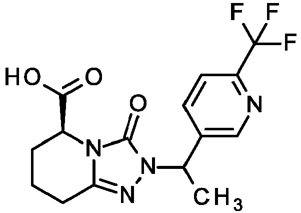

tert-Butyl-(5S)-3-oxo-2-{(1RS)-1-[6-(trifluormethyl)pyridin-3-yl]ethyl}-2,3,5,6,7,8-hexahydro[1,2,4]triazolo[4,3-a]pyridin-5-carboxylat (Isomer 1) (97.0 mg, 235 µmol) wurde in Dichlormethan (2.0 ml) gelöst und bei Raumtemperatur mit Trifluoressigsäure (200 µl, 2.6 mmol) versetzt. Nachdem das Reaktionsgemisch über Nacht bei Raumtemperatur gerührt worden war, wurde das Lösungsmittel unter Vakuum entfernt. Es wurden 154 mg (>100 % d. Th.) der Titelverbindung erhalten.
LC-MS (Methode 3): Rₜ = 1.21 min; MS (ESIpos): m/z = 357 [M+H]⁺

### Intermediat 387

### (5S)-3-Oxo-2-{(1RS)-1-[6-(trifluormethyl)pyridin-3-yl]ethyl}-2,3,5,6,7,8-hexahydro[1,2,4]triazolo[4,3-a]pyridin-5-carbonsäure (Isomer 2)

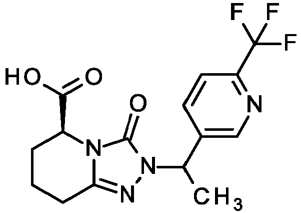

tert-Butyl-(5S)-3-oxo-2-{(1RS)-1-[6-(trifluormethyl)pyridin-3-yl]ethyl}-2,3,5,6,7,8-hexahydro[1,2,4]triazolo[4,3-a]pyridin-5-carboxylat (Isomer 2) (86.0 mg, 209 µmol) wurde in Dichlormethan (2.0 ml) gelöst und bei Raumtemperatur mit Trifluoressigsäure (200 µl, 2.6 mmol) versetzt. Nachdem das Reaktionsgemisch über Nacht bei Raumtemperatur gerührt worden war, wurde das Lösungsmittel unter Vakuum entfernt. Es wurden 133 mg (>100 % d. Th.) der Titelverbindung erhalten.
LC-MS (Methode 3): Rₜ = 1.19 min; MS (ESIpos): m/z = 357 [M+H]⁺

### Intermediat 388

### (5S)-3-Oxo-2-{[cis/trans-4-(trifluormethyl)cyclohexyl]methyl}-2,3,5,6,7,8-hexahydro[1,2,4]triazolo[4,3-a]pyridin-5-carbonsäure (Diastereomerengemisch; 2 Isomere)

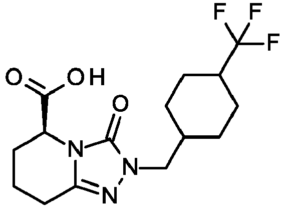

tert-Butyl-(5S)-3-oxo-2-{[cis/trans-4-(trifluormethyl)cyclohexyl]methyl}-2,3,5,6,7,8-hexahydro[1,2,4]triazolo[4,3-a]pyridin-5-carboxylat (Diastereomerengemisch, 2 Isomere) (112 mg, 264 µmol) wurde in Dichlormethan (5.0 ml) gelöst und bei Raumtemperatur mit Trifluoressigsäure (500 µl, 6.5 mmol) versetzt. Nachdem das Reaktionsgemisch über Nacht bei Raumtemperatur gerührt worden war, wurde das Lösungsmittel unter Vakuum entfernt. Es wurden 153 mg (>100 % d. Th.) der Titelverbindung erhalten.
LC-MS (Methode 3): Rₜ = 1.41 min; MS (ESIpos): m/z = 348 [M+H]⁺
¹H-NMR (400 MHz, DMSO-d6) δ [ppm]: -0.150 (1.53), -0.008 (16.00), 0.008 (13.04), 0.147 (1.83), 1.030 (2.72), 1.110 (9.48), 1.191 (2.96), 1.249 (3.95), 1.395 (3.01), 1.491 (10.32), 1.535 (8.54), 1.685 (3.56), 1.828 (5.53), 2.079 (9.98), 2.328 (2.17), 2.366 (1.78), 2.524 (6.22), 2.569 (4.20), 2.583 (3.11), 2.631 (5.33), 2.670 (3.90), 3.457 (4.64), 3.474 (4.79), 3.609 (5.04), 3.622 (5.68), 3.642 (4.54), 4.424 (8.54), 4.436 (8.69), 4.448 (4.00), 4.970 (3.75).

### Intermediat 389

### (5S)-2-{[3-Fluor-2-(trifluormethyl)pyridin-4-yl]methyl}-3-oxo-2,5,6,7-tetrahydro-3H-pyrrolo[2,1-c][1,2,4]triazol-5-carbonsäure

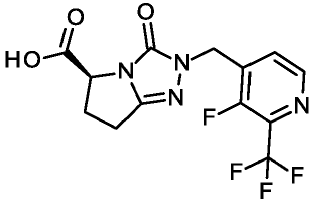

Methyl-(5S)-2-{[3-fluor-2-(trifluormethyl)pyridin-4-yl]methyl}-3-oxo-2,5,6,7-tetrahydro-3H-pyrrolo[2,1-c][1,2,4]triazol-5-carboxylat (243 mg, 75 % Reinheit, 506 µmol) wurde in THF (2.5 ml) und Wasser (2.5 ml) vorgelegt und Lithiumhydroxid (60.6 mg, 2.53 mmol) zugegeben. Das Reaktionsgemisch wurde nach Rühren für 90 min bei Raumtemperatur mit 1 N wässriger Salzsäure versetzt. Das Lösungsmittel wurde eingeengt und die wässrige Phase wurde dreimal mit Ethylacetat extrahiert. Die vereinigten organischen Phasen wurden über Natriumsulfat getrocknet, filtriert und das Filtrat eingeengt. Es wurden 264 mg (>100 % d. Th.) der Titelverbindung erhalten.
LC-MS (Methode 4): Rₜ = 0.60 min; MS (ESIpos): m/z = 347 [M+H]⁺

### Intermediat 390

### (5RS,7RS)-2-[(6-Chlorpyridin-3-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere)

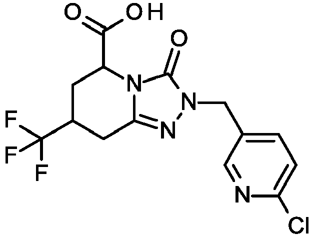

Ethyl-(5RS,7RS)-2-[(6-chlorpyridin-3-yl)methyl]-3-oxo-7-(trifluormethyl)-2,3,5,6,7,8-hexahydro[1,2,4]triazolo[4,3-a]pyridin-5-carboxylat (Diastereomerengemisch; 4 Isomere) (500 mg, 1.24 mmol) wurde in THF (10 ml) vorgelegt und bei 0°C wurden Natriumethanolat (850 µl, 21 % in Ethanol, 2.3 mmol) zugegeben. Das Reaktionsgemisch wurde für 15 min bei 0°C und über Nacht bei Raumtemperatur gerührt. Es wurde Wasser zugegeben und mit 1 N wässriger Salzsäure sauer gestellt. Die wässrige Phase wurde mit Ethylacetat extrahiert und die organische Phase wurde über Natriumsulfat getrocknet, filtriert und das Filtrat eingeengt. Es wurden 479 mg (>100 % d. Th.) der Titelverbindung erhalten.
LC-MS (Methode 4): Rₜ = 0.64 min; MS (ESIpos): m/z = 377 [M+H]⁺
¹H-NMR (400 MHz, DMSO-d6) δ [ppm]: 1.094 (0.28), 1.157 (2.06), 1.170 (1.54), 1.175 (4.26), 1.193 (2.09), 1.240 (0.19), 1.271 (0.24), 1.786 (0.19), 1.817 (0.21), 1.908 (2.01), 1.988 (7.50), 2.224 (0.42), 2.240 (0.55), 2.259 (1.12), 2.275 (1.16), 2.288 (1.30), 2.304 (1.30), 2.319 (2.92), 2.352 (1.02), 2.667 (0.88), 2.696 (3.39), 2.720 (3.41), 2.749 (0.95), 2.874 (0.20), 2.951 (0.48), 2.977 (2.16), 3.005 (1.87), 3.034 (0.37), 4.002 (0.61), 4.021 (1.81), 4.038 (1.78), 4.056 (0.60), 4.394 (0.20), 4.409 (0.23), 4.421 (0.22), 4.437 (0.19), 4.689 (2.91), 4.700 (2.65), 4.921 (1.26), 4.948 (16.00), 7.507 (4.76), 7.515 (0.69), 7.527 (5.86), 7.715 (3.26), 7.721 (3.37), 7.735 (3.05), 7.742 (3.13), 7.756 (0.28), 8.317 (4.42), 8.322 (4.42), 8.336 (0.48), 13.683 (0.28).

### Intermediat 391

### (5RS,7RS)-3-Oxo-7-(trifluormethyl)-2-{[6-(trifluormethyl)pyridin-3-yl]methyl}-2,3,5,6,7,8-hexahydro[1,2,4]triazolo[4,3-a]pyridin-5-carbonsäure (Diastereomerengemisch; 4 Isomere)

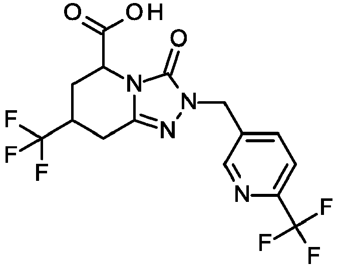

Ethyl-(5RS,7RS)-3-oxo-7-(trifluormethyl)-2-{[6-(trifluormethyl)pyridin-3-yl]methyl}-2,3,5,6,7,8-hexahydro[1,2,4]triazolo[4,3-a]pyridin-5-carboxylat (Diastereomerengemisch; 4 Isomere) (480 mg, 1.10 mmol) wurde in THF (20 ml) vorgelegt, und bei 0°C wurde Natriumethanolat (760 µl, 21 % in Ethanol, 2.0 mmol) zugegeben. Das Reaktionsgemisch wurde für 20 min bei 0°C und über Nacht bei Raumtemperatur gerührt. Es wurde Eiswasser zugegeben und mit 1 N wässriger Salzsäure sauer gestellt. Die wässrige Phase wurde mit Ethylacetat extrahiert und die organische Phase wurde über Natriumsulfat getrocknet, filtriert und das Filtrat eingeengt. Es wurden 577 mg (88% Reinheit, >100 % d. Th.) der Titelverbindung erhalten.
LC-MS (Methode 4): Rₜ = 0.73 min; MS (ESIpos): m/z = 411 [M+H]⁺
¹H-NMR (400 MHz, DMSO-d6) δ [ppm]: -0.008 (0.57), 0.008 (0.56), 1.157 (4.39), 1.169 (1.76), 1.175 (9.00), 1.192 (4.46), 1.908 (1.38), 1.988 (16.00), 2.266 (0.42), 2.283 (0.44), 2.296 (0.49), 2.311 (0.46), 2.328 (1.28), 2.366 (0.50), 2.524 (0.59), 2.675 (0.40), 2.706 (1.29), 2.729 (1.28), 2.986 (0.84), 3.014 (0.71), 4.003 (1.29), 4.021 (3.80), 4.038 (3.75), 4.056 (1.23), 4.701 (0.89), 4.706 (1.09), 4.717 (0.99), 5.047 (0.49), 5.076 (5.23), 7.900 (0.82), 7.920 (2.75), 7.933 (1.79), 7.937 (1.59), 7.954 (0.60), 7.958 (0.60), 8.659 (1.66).

### Intermediat 392

### (5RS,7RS)-2-{[3-Fluor-2-(trifluormethyl)pyridin-4-yl]methyl}-3-oxo-7-(trifluormethyl)-2,3,5,6,7,8-hexahydro[1,2,4]triazolo[4,3-a]pyridin-5-carbonsäure (Diastereomerengemisch; 4 Isomere)

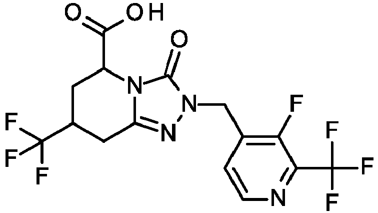

Ethyl-(5RS,7RS)-2-{[3-fluor-2-(trifluormethyl)pyridin-4-yl]methyl}-3-oxo-7-(trifluormethyl)-2,3,5,6,7,8-hexahydro[1,2,4]triazolo[4,3-a]pyridin-5-carboxylat (Diastereomerengemisch; 4 Isomere) (440 mg, 964 µmol) wurde in THF (5.0 ml) und Wasser (5.0 ml) vorgelegt und Lithiumhydroxid (69.3 mg, 2.89 mmol) wurde zugegeben. Das Reaktionsgemisch wurde nach Rühren über Nacht bei Raumtemperatur mit 1 N wässriger Salzsäure versetzt. Die wässrige Phase wurde mit Ethylacetat extrahiert und die organische Phase wurde über Natriumsulfat getrocknet, filtriert und das Filtrat eingeengt. Es wurden 372 mg (90 % d. Th.) der Titelverbindung erhalten.
LC-MS (Methode 4): Rₜ = 0.78 min; MS (ESIpos): m/z = 429 [M+H]⁺
¹H-NMR (400 MHz, DMSO-d6) δ [ppm]: -0.149 (0.91), -0.008 (8.08), 0.008 (7.23), 0.146 (0.98), 1.106 (2.33), 1.110 (1.68), 1.157 (4.28), 1.175 (8.94), 1.193 (4.49), 1.236 (0.42), 1.839 (0.46), 1.908 (14.96), 1.988 (16.00), 2.239 (0.66), 2.254 (0.89), 2.273 (1.85), 2.289 (1.87), 2.303 (2.06), 2.319 (2.18), 2.337 (4.86), 2.367 (1.97), 2.523 (2.97), 2.670 (1.10), 2.689 (1.29), 2.718 (5.76), 2.742 (5.61), 2.770 (1.75), 2.800 (0.73), 2.896 (0.48), 2.974 (0.81), 2.999 (3.66), 3.027 (3.18), 3.055 (0.71), 3.077 (0.89), 4.003 (1.29), 4.021 (3.82), 4.038 (3.78), 4.056 (1.25), 4.417 (0.48), 4.432 (0.54), 4.444 (0.52), 4.459 (0.44), 4.717 (4.76), 4.728 (4.45), 5.085 (1.45), 5.098 (2.93), 5.126 (12.20), 5.136 (12.34), 5.177 (1.39), 5.754 (0.75), 7.583 (3.57), 7.595 (6.94), 7.608 (3.91), 7.631 (0.54), 7.644 (0.87), 7.658 (0.46), 8.562 (7.88), 8.574 (8.27), 8.589 (1.12), 13.733 (0.56).

### Intermediat 393

### (5RS,7RS)-2-[(5-Chlor-3-fluorpyridin-2-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere)

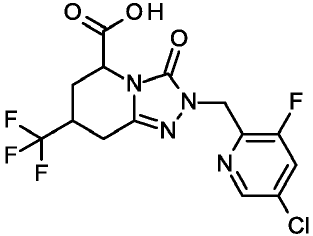

Ethyl-(5RS,7RS)-2-[(5-chlor-3-fluorpyridin-2-yl)methyl]-3-oxo-7-(trifluormethyl)-2,3,5,6,7,8-hexahydro[1,2,4]triazolo[4,3-a]pyridin-5-carboxylat (Diastereomerengemisch; 4 Isomere) (133 mg, 315 µmol) wurde in THF (1.5 ml) und Wasser (1.5 ml) vorgelegt und Lithiumhydroxid (22.6 mg, 944 µmol) wurde zugegeben. Das Reaktionsgemisch wurde nach Rühren über Nacht bei Raumtemperatur mit 1 N wässriger Salzsäure versetzt. Die wässrige Phase wurde mit Ethylacetat extrahiert und die organische Phase wurde über Natriumsulfat getrocknet, filtriert und das Filtrat eingeengt. Es wurden 113 mg (91 % d. Th.) der Titelverbindung erhalten.
LC-MS (Methode 3): Rₜ = 1.26 min; MS (ESIpos): m/z = 395 [M+H]⁺

### Intermediat 394

### (5RS,7RS)-2-[(5-Chlorpyridin-3-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere)

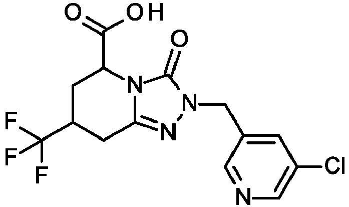

Ethyl-(5RS,7RS)-2-[(5-chlorpyridin-3-yl)methyl]-3-oxo-7-(trifluormethyl)-2,3,5,6,7,8-hexahydro[1,2,4]triazolo[4,3-a]pyridin-5-carboxylat (Diastereomerengemisch; 4 Isomere) (55.0 mg, 136 µmol) wurde in THF (1.0 ml) und Wasser (1.0 ml) vorgelegt und Lithiumhydroxid (9.76 mg, 408 µmol) zugegeben. Das Reaktionsgemisch wurde nach Rühren über Nacht bei Raumtemperatur mit 1 N wässriger Salzsäure versetzt. Die wässrige Phase wurde mit Ethylacetat extrahiert und die organische Phase wurde über Natriumsulfat getrocknet, filtriert und das Filtrat eingeengt. Es wurden 71.7 mg (75 % Reinheit, >100 % d. Th.) der Titelverbindung erhalten.
LC-MS (Methode 4): Rₜ = 0.64 min; MS (ESIpos): m/z = 377 [M+H]⁺

### Intermediat 395

### 3-Oxo-8-(trifluormethyl)-2-{[6-(trifluormethyl)pyridin-3-yl]methyl}-2,3-dihydro[1,2,4]triazolo[4,3-a]pyridin-5-carbonsäure

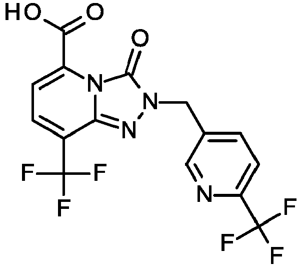

Methyl-3-oxo-8-(trifluormethyl)-2-{[6-(trifluormethyl)pyridin-3-yl]methyl}-2,3-dihydro[1,2,4]triazolo[4,3-a]pyridin-5-carboxylat (85.0 mg, 77 % Reinheit, 156 µmol) wurde in THF (1.0 ml) und Wasser (1.0 ml) vorgelegt und Lithiumhydroxid (7.46 mg, 311 µmol) zugegeben. Das Reaktionsgemisch wurde nach Rühren über Nacht bei Raumtemperatur mit 1 N wässriger Salzsäure versetzt. Die wässrige Phase wurde mit Ethylacetat extrahiert und die organische Phase wurde über Natriumsulfat getrocknet, filtriert und das Filtrat eingeengt. Es wurden 113 mg (>100 % d. Th.) der Titelverbindung erhalten.
LC-MS (Methode 1): Rₜ = 0.98 min; MS (ESIpos): m/z = 407 [M+H]⁺

### Intermediat 396

### 2-[(5-Chlor-3-fluorpyridin-2-yl)methyl]-3-oxo-8-(trifluormethyl)-2,3-dihydro[1,2,4]triazolo[4,3-a]pyridin-5-carbonsäure

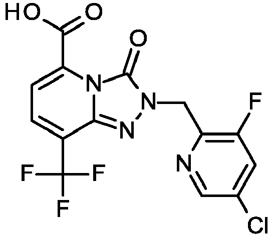

Methyl-2-[(5-chlor-3-fluorpyridin-2-yl)methyl]-3-oxo-8-(trifluormethyl)-2,3-dihydro[1,2,4]triazolo[4,3-a]pyridin-5-carboxylat (86.0 mg, 212 µmol) wurde in THF (1.0 ml) und Wasser (1.0 ml) vorgelegt und Lithiumhydroxid (10.2 mg, 425 µmol) zugegeben. Das Reaktionsgemisch wurde nach Rühren über Nacht bei Raumtemperatur mit 1 N wässriger Salzsäure versetzt. Die wässrige Phase wurde mit Ethylacetat extrahiert und die organische Phase wurde über Natriumsulfat getrocknet, filtriert und das Filtrat eingeengt. Es wurden 85.4 mg (96 % d. Th.) der Titelverbindung erhalten.
LC-MS (Methode 4): Rₜ = 0.68 min; MS (ESIpos): m/z = 391 [M+H]⁺

### Intermediat 397

### 5-{[(3R,4S)-3,4-Difluorpyrrolidin-1-yl]carbonyl}-8-(trifluormethyl)-2-{[6-(trifluormethyl)pyridin-3-yl]methyl}[1,2,4]triazolo[4,3-a]pyridin-3(2H)-on

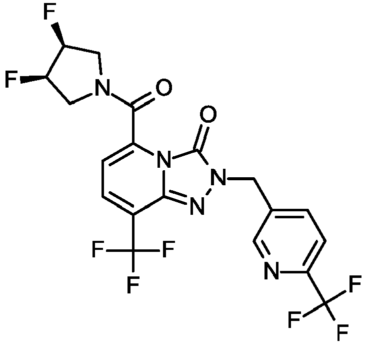

3-Oxo-8-(trifluormethyl)-2-{[6-(trifluormethyl)pyridin-3-yl]methyl}-2,3-dihydro[1,2,4]triazolo[4,3-a]pyridin-5-carbonsäure (110 mg, 249 µmol) wurde in THF (3.0 ml) bei Raumtemperatur vorgelegt. Anschließend wurden HBTU (123 mg, 324 µmol) und N,N-Diisopropylethylamin (220 µl, 1.2 mmol) zugegeben. Nach 5 min Rühren wurde (3R,4S)-3,4-Difluorpyrrolidinhydrochlorid (42.9 mg, 299 µmol) zugegeben und das Reaktionsgemisch für 4 Stunden bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über Säulenchromatographie (Kieselgel; Laufmittel:, Dichlormethan/Methanol-Gradient 1/0, 98/2) aufgereinigt. Die produkthaltigen Fraktionen wurden im Vakuum eingeengt und es wurden 23.0 mg (87 % Reinheit, 16 % d. Th.) der Titelverbindung erhalten.
LC-MS (Methode 3): Rₜ = 1.71 min; MS (ESIpos): m/z = 496 [M+H]⁺
¹H-NMR (400 MHz, DMSO-d6) δ [ppm]: -0.149 (0.99), -0.009 (8.81), 0.007 (7.55), 0.146 (0.99), 1.237 (0.90), 1.949 (0.36), 2.322 (1.80), 2.327 (2.52), 2.331 (1.80), 2.366 (0.63), 2.522 (5.30), 2.623 (0.36), 2.664 (2.16), 2.669 (2.88), 2.674 (2.25), 2.689 (16.00), 2.709 (0.90), 2.741 (0.27), 3.356 (1.08), 3.390 (0.63), 3.408 (0.54), 3.428 (0.45), 3.473 (0.45), 3.496 (0.45), 3.614 (0.45), 3.636 (0.45), 3.648 (0.45), 3.676 (0.54), 3.723 (0.45), 3.768 (0.27), 3.867 (0.36), 3.906 (0.63), 3.920 (0.54), 3.938 (0.36), 3.953 (0.36), 4.482 (0.27), 4.497 (0.27), 5.320 (1.62), 5.369 (0.27), 5.449 (0.27), 5.472 (0.27), 5.803 (0.27), 6.694 (0.27), 6.723 (0.36), 6.791 (0.36), 6.807 (0.36), 7.268 (0.45), 7.307 (0.81), 7.320 (0.36), 7.777 (0.63), 7.910 (1.08), 7.929 (1.53), 8.026 (0.81), 8.046 (0.63), 8.772 (0.99), 14.278 (0.27).

### Intermediat 398

### 5-{[(3S)-3-Fluorpyrrolidin-1-yl]carbonyl}-8-(trifluormethyl)-2-{[6-(trifluormethyl)pyridin-3-yl]methyl}[1,2,4]triazolo[4,3-a]pyridin-3(2H)-on

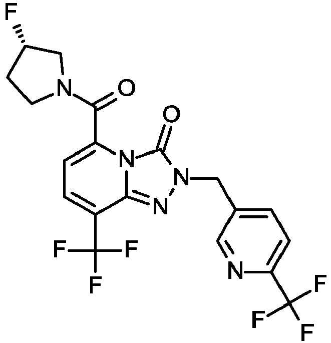

3-Oxo-8-(trifluormethyl)-2-{[6-(trifluormethyl)pyridin-3-yl]methyl}-2,3-dihydro[1,2,4]triazolo[4,3-a]pyridin-5-carbonsäure (127 mg, 300 µmol) wurde in THF (5.0 ml) bei Raumtemperatur vorgelegt. Anschließend wurden HBTU (148 mg, 390 µmol) und N,N-Diisopropylethylamin (260 µl, 1.5 mmol) zugegeben. Nach 5 min Rühren wurde (3S)-3-Fluorpyrrolidinhydrochlorid (45.2 mg, 360 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Es wurden 167 mg (60 % Reinheit, 70 % d. Th.) der Titelverbindung erhalten.
LC-MS (Methode 3): Rₜ = 1.64 min; MS (ESIpos): m/z = 478 [M+H]⁺

### Intermediat 399

### 2-[(5-Chlor-3-fluorpyridin-2-yl)methyl]-5-{[(3S)-3-fluorpyrrolidin-1-yl]carbonyl}-8-(trifluormethyl)[1,2,4]triazolo[4,3-a]pyridin-3(2H)-on

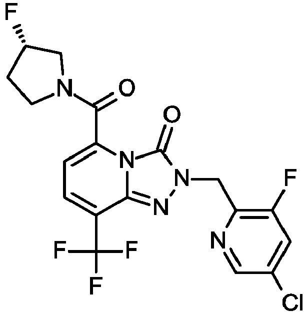

2-[(5-Chlor-3-fluorpyridin-2-yl)methyl]-3-oxo-8-(trifluormethyl)-2,3-dihydro[1,2,4]triazolo[4,3-a]pyridin-5-carbonsäure (42.0 mg, 100 µmol) wurde in THF (2.0 ml) bei Raumtemperatur vorgelegt. Anschließend wurden HBTU (49.3 mg, 130 µmol) und N,N-Diisopropylethylamin (87 µl, 500 µmol) zugegeben. Nach 5 min Rühren wurde (3S)-3-Fluorpyrrolidinhydrochlorid (15.1 mg, 120 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Es wurden 51.2 mg (62 % Reinheit, 69 % d. Th.) der Titelverbindung erhalten.
LC-MS (Methode 3): Rₜ = 1.65 min; MS (ESIpos): m/z = 462 [M+H]⁺

### Intermediat 400

### 2-[(5-Chlor-3-fluorpyridin-2-yl)methyl]-5-{[(3R,4S)-3,4-difluorpyrrolidin-1-yl]carbonyl}-8-(trifluormethyl)[1,2,4]triazolo[4,3-a]pyridin-3(2H)-on

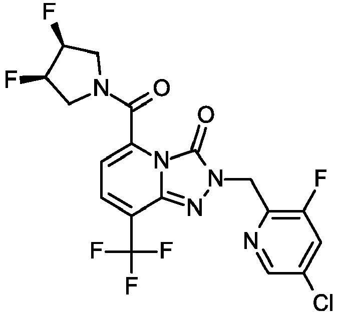

2-[(5-Chlor-3-fluorpyridin-2-yl)methyl]-3-oxo-8-(trifluormethyl)-2,3-dihydro[1,2,4]triazolo[4,3-a]pyridin-5-carbonsäure (42.0 mg, 100 µmol) wurde in THF (2.0 ml) bei Raumtemperatur vorgelegt. Anschließend wurden HBTU (49.3 mg, 130 µmol) und N,N-Diisopropylethylamin (87 µl, 500 µmol) zugegeben. Nach 5 min Rühren wurde (3R,4S)-3,4-Difluorpyrrolidinhydrochlorid (17.2 mg, 120 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Es wurden 95.2 mg (47 % Reinheit, 92 % d. Th.) der Titelverbindung erhalten.
LC-MS (Methode 3): Rₜ = 1.70 min; MS (ESIpos): m/z = 480 [M+H]⁺

### Intermediat 401

### (5S)-2-[(5-Chlorpyridin-2-yl)methyl]-5-{[(3S)-3-fluorpyrrolidin-1-yl]carbonyl}-6,7-dihydro[1,2,4]triazolo[4,3-a]pyridin-3,8(2H,5H)-dion

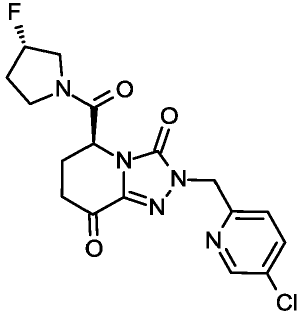

(5S,8RS)-2-[(5-Chlorpyridin-2-yl)methyl]-5-{[(3S)-3-fluorpyrrolidin-1-yl]carbonyl}-8-hydroxy-5,6,7,8-tetrahydro[1,2,4]triazolo[4,3-a]pyridin-3(2H)-on (Diastereomerengemisch; 2 Isomere) (39.0 mg, 92.6 µmol) wurde in Dichlormethan (940 µl) bei Raumtemperatur vorgelegt und das Reaktionsgemisch wurde auf 0°C abgekühlt. Anschließend wurde Dess-Martin-Periodinan (98.2 mg, 232 µmol) zugegeben und das Reaktionsgemisch für 15 min bei 0°C und über Nacht bei Raumtemperatur gerührt. Das Reaktionsgemisch wurde mit Diethylether und einer Lösung von Natriumthiosulfat (350 mg) in gesättigter wässriger Natriumhydrogencarbonat-Lösung versetzt und es wurde gerührt. Die organische Phase wurde abgetrennt und die wässrige Phase mit Diethylether extrahiert. Die vereinigten organischen Phasen wurden über Natriumsulfat getrocknet, filtriert und das Filtrat eingeengt. Es wurden 12.7 mg (90 % Reinheit, 31 % d. Th.) der Titelverbindung erhalten.
LC-MS (Methode 1): Rₜ = 0.86 min; MS (ESIpos): m/z = 394 [M+H]⁺

### Intermediat 402

### (5S)-2-[(5-Chlor-3-fluorpyridin-2-yl)methyl]-5-{[(3S)-3-fluorpyrrolidin-1-yl]carbonyl}-6,7-dihydro[1,2,4]triazolo[4,3-a]pyridin-3,8(2H,5H)-dion

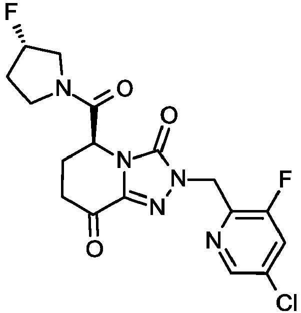

(5S,8RS)-2-[(5-Chlor-3-fluorpyridin-2-yl)methyl]-5-{[(3S)-3-fluorpyrrolidin-1-yl]carbonyl}-8-hydroxy-5,6,7,8-tetrahydro[1,2,4]triazolo[4,3-a]pyridin-3(2H)-on (Diastereomerengemisch; 2 Isomere) (51.7 mg, 86 % Reinheit, 107 µmol) wurde in Dichlormethan (1.1 ml) bei Raumtemperatur vorgelegt und das Reaktionsgemisch wurde auf 0°C abgekühlt. Anschließend wurde Dess-Martin-Periodinan (114 mg, 269 µmol) zugegeben und das Reaktionsgemisch für 15 min bei 0°C und über Nacht bei Raumtemperatur gerührt. Das Reaktionsgemisch wurde mit Diethylether und eine Lösung von Natriumthiosulfat (350 mg) in gesättigter wässriger Natriumhydrogencarbonat-Lösung versetzt und es wurde gerührt. Die organische Phase wurde abgetrennt und die wässrige Phase mit Diethylether und dreimal mit Ethylacetat extrahiert. Die vereinigten organischen Phasen wurden über Natriumsulfat getrocknet, filtriert und das Filtrat eingeengt. Es wurden 25.8 mg (75 % Reinheit, 46 % d. Th.) der Titelverbindung erhalten.
LC-MS (Methode 1): Rₜ = 0.89 min; MS (ESIpos): m/z = 412 [M+H]⁺

### Intermediat 403

### (5S)-2-[(6-Chlorpyridin-3-yl)methyl]-5-[(3,3-difluorpyrrolidin-1-yl)carbonyl]-6,7-dihydro[1,2,4]triazolo[4,3-a]pyridin-3,8(2H,5H)-dion

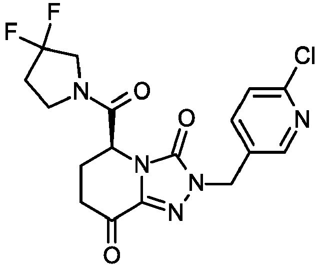

(5S,8RS)-2-[(6-Chlorpyridin-3-yl)methyl]-5-[(3,3-difluorpyrrolidin-1-yl)carbonyl]-8-hydroxy-5,6,7,8-tetrahydro[1,2,4]triazolo[4,3-a]pyridin-3(2H)-on (Diastereomerengemisch; 2 Isomere) (66.9 mg, 90 % Reinheit, 146 µmol) wurde in Dichlormethan (6.0 ml, 94 mmol) gelöst und bei Raumtemperatur Mangan(IV)-oxid (254 mg, 2.92 mmol) zugegeben. Das Reaktionsgemisch wurde über Nacht bei Raumtemperatur gerührt. Die Suspension wurde über Celite filtriert und das Filtrat unter Vakuum eingeengt. Es wurden 47.3 mg (79 % d. Th.) der Titelverbindung erhalten.
LC-MS (Methode 4): Rₜ = 0.69 min; MS (ESIpos): m/z = 412 [M+H]⁺

### Intermediat 404

### tert-Butyl-(5S)-3-oxo-2-{[2-(trifluormethyl)pyrimidin-5-yl]methyl}-2,3,5,6,7,8-hexahydro[1,2,4]triazolo[4,3-a]pyridin-5-carboxylat

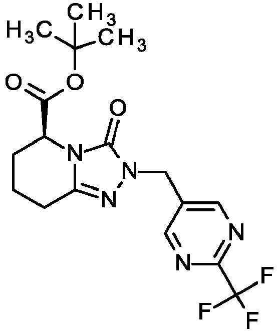

tert-Butyl-(5S)-3-oxo-2,3,5,6,7,8-hexahydro[1,2,4]triazolo[4,3-a]pyridin-5-carboxylat (16.2 mg, 67.8 µmol) wurde in Acetonitril (1.0 ml) vorgelegt. Cäsiumcarbonat (33.2 mg, 102 µmol) und 5-(Chlormethyl)-2-(trifluormethyl)pyrimidin (14.0 mg, 71.2 µmol) wurden anschließend zugegeben. Das Reaktionsgemisch wurde nach Rühren über Nacht bei Raumtemperatur Ethylacetat zugegeben. Die organische Phase wurde mit Wasser und gesättigter wässriger Natriumchlorid-Lösung gewaschen, über Natriumsulfat getrocknet, filtriert und das Filtrat eingeengt. Es wurden 22 mg (11 % Reinheit, 8 % d. Th.) der Titelverbindung erhalten.
LC-MS (Methode 3): Rₜ = 1.88 min; MS (ESIpos): m/z = 400 [M+H]⁺

### Intermediat 405

### (5S)-3-Oxo-2-{[2-(trifluormethyl)pyrimidin-5-yl]methyl}-2,3,5,6,7,8-hexahydro[1,2,4]triazolo[4,3-a]pyridin-5-carbonsäure

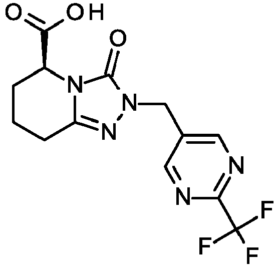

tert-Butyl-(5S)-3-oxo-2-{[2-(trifluormethyl)pyrimidin-5-yl]methyl}-2,3,5,6,7,8-hexahydro[1,2,4]triazolo[4,3-a]pyridin-5-carboxylat (22.0 mg, 11 % Reinheit, 6 µmol) wurde in 1,4-Dioxan (200 µl) gelöst und Salzsäure gelöst in 1,4-Dioxan (140 µl, 4.0 M, 550 µmol) zugegeben. Nachdem das Reaktionsgemisch über Nacht bei Raumtemperatur gerührt wurde, wurde das Lösungsmittel unter Vakuum entfernt. Es wurden 22 mg (11 % Reinheit, >100 % d. Th.) der Titelverbindung erhalten.
LC-MS (Methode 3): Rₜ = 0.98 min; MS (ESIpos): m/z = 344 [M+H]⁺

### Intermediat 406

### Ethyl-(5RS,7RS)-2-{[3-chlor-5-(trifluormethyl)pyridin-2-yl]methyl}-3-oxo-7-(trifluormethyl)-2,3,5,6,7,8-hexahydro[1,2,4]triazolo[4,3-a]pyridin-5-carboxylat (Diastereomerengemisch, 4 Isomere)

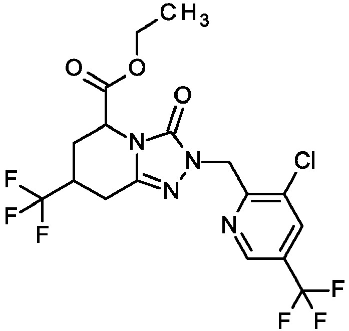

Ethyl-(5S,7R)-3-oxo-7-(trifluormethyl)-2,3,5,6,7,8-hexahydro[1,2,4]triazolo[4,3-a]pyridin-5-carboxylat (Diastereomerengemisch, 4 Isomere) (500 mg, 1.79 mmol) wurde in Acetonitril (16 ml) vorgelegt. Caesiumcarbonat (1.46 g, 4.48 mmol) und 3-Chlor-2-(chlormethyl)-5-(trifluormethyl)pyridin (432 mg, 1.88 mmol) wurden anschließend zugegeben. Das Reaktionsgemisch wurde für 72 Stunden bei Raumtemperatur gerührt und anschließend mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 859 mg (96 % d. Th.) der Titelverbindung erhalten.
LC-MS (Methode 4): Rₜ = 0.99 min; MS (ESIpos): m/z = 473 [M+H]⁺

### Intermediat 407

### tert-Butyl-(5S)-2-[(5-chlor-3-fluorpyridin-2-yl)methyl]-3-oxo-2,3,5,6,7,8-hexahydro[1,2,4]triazolo[4,3-a]pyridin-5-carboxylat

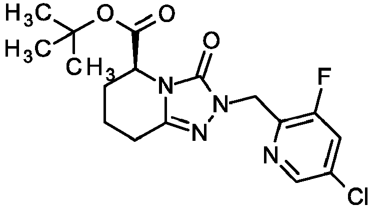

tert-Butyl-(5S)-3-oxo-2,3,5,6,7,8-hexahydro[1,2,4]triazolo[4,3-a]pyridin-5-carboxylat (500 mg, 2.09 mmol) wurde in Acetonitril (15 ml) vorgelegt. Caesiumcarbonat (1.36 g, 4.18 mmol) und 5-Chlor-2-(chlormethyl)-3-fluorpyridin (395 mg, 2.19 mmol) wurden anschließend zugegeben. Das Reaktionsgemisch wurde für 72 Stunden bei Raumtemperatur und anschließend mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 793 mg (99 % d. Th.) der Titelverbindung erhalten.
LC-MS (Methode 4): Rₜ = 0.86 min; MS (ESIpos): m/z = 383 [M+H]⁺

### Intermediat 408

### tert-Butyl-(5S)-2-[(3-chlor-5-fluorpyridin-2-yl)methyl]-3-oxo-2,3,5,6,7,8-hexahydro[1,2,4]triazolo[4,3-a]pyridin-5-carboxylat

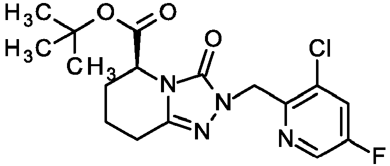

tert-Butyl-(5S)-3-oxo-2,3,5,6,7,8-hexahydro[1,2,4]triazolo[4,3-a]pyridin-5-carboxylat (452 mg, 1.89 mmol) wurde in Acetonitril (41 ml) vorgelegt. Caesiumcarbonat (1.54 g, 4.72 mmol) und 3-Chlor-2-(chlormethyl)-5-fluorpyridin (476 mg, 2.65 mmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 842 mg (78 % Reinheit, 91 % d. Th.) der Titelverbindung erhalten.
LC-MS (Methode 3): Rₜ = 1.66 min; MS (ESIpos): m/z = 383 [M+H]⁺

### Intermediat 409

### (5RS,7RS)-2-{[3-Chlor-5-(trifluormethyl)pyridin-2-yl]methyl}-3-oxo-7-(trifluormethyl)-2,3,5,6,7,8-hexahydro[1,2,4]triazolo[4,3-a]pyridin-5-carbonsäure (Diasteromeregemisch, 4 Isomere)

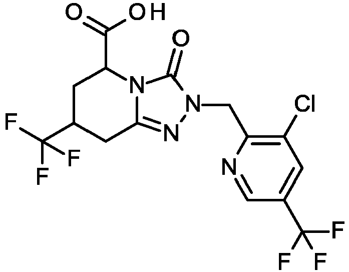

Ethyl-(5RS,7RS)-2-{[3-chlor-5-(trifluormethyl)pyridin-2-yl]methyl}-3-oxo-7-(trifluormethyl)-2,3,5,6,7,8-hexahydro[1,2,4]triazolo[4,3-a]pyridin-5-carboxylat (Diasteromeregemisch, 4 Isomere) (859 mg, 1.82 mmol) wurde in THF (21 ml) vorgelegt und Natriumethanolat-Lösung (1.09 g, 3.36 mmol, 21 Gwe.%) zugegeben. Das Reaktionsgemisch wurde für 20 min bei 0°C und über Nacht bei Raumtemperatur gerührt. Das Reaktionsgemisch wurde mit Wasser, 1 N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 846 mg (>100 % d. Th.) der Titelverbindung erhalten.
LC-MS (Methode 3): Rₜ = 1.50 min; MS (ESIpos): m/z = 445 [M+H]⁺

### Intermediat 410

### (5S)-2-[(5-Chlor-3-fluorpyridin-2-yl)methyl]-3-oxo-2,3,5,6,7,8-hexahydro[1,2,4]triazolo[4,3-a]pyridin-5-carbonsäure

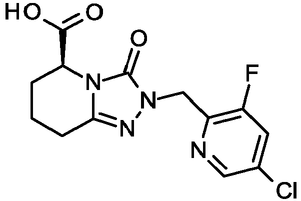

tert-Butyl-(5S)-2-[(5-chlor-3-fluorpyridin-2-yl)methyl]-3-oxo-2,3,5,6,7,8-hexahydro[1,2,4]triazolo[4,3-a]pyridin-5-carboxylat (793 mg, 2.07 mmol) wurde in Dichlormethan (15 ml) gelöst und bei Raumtemperatur mit Trifluoressigsäure (3.2 ml, 41 mmol) versetzt. Nachdem das Reaktionsgemisch über Nacht bei Raumtemperatur gerührt wurde, wurde erneut Trifluoressigsäure (0.64 ml, 8.2 mmol) zugegeben und das Reaktionsgemisch für weitere 1.5 Stunden bei Raumtemperatur gerührt. Das Lösungsmittel unter Vakuum entfernt. Es wurden 676 mg (89 % Reinheit, 89 % d. Th.) der Titelverbindung erhalten.
LC-MS (Methode 4): Rₜ = 0.56 min; MS (ESIpos): m/z = 327 [M+H]⁺

### Intermediat 411

### (5S)-2-[(3-Chlor-5-fluorpyridin-2-yl)methyl]-3-oxo-2,3,5,6,7,8-hexahydro[1,2,4]triazolo[4,3-a]pyridin-5-carbonsäure

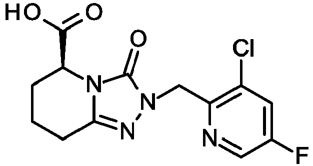

tert-Butyl-(5S)-2-[(3-chlor-5-fluorpyridin-2-yl)methyl]-3-oxo-2,3,5,6,7,8-hexahydro[1,2,4]triazolo[4,3-a]pyridin-5-carboxylat (842 mg, 2.20 mmol) wurde in Dichlormethan (14.1 ml) gelöst und bei Raumtemperatur mit Trifluoressigsäure (3.4 ml, 44 mmol) versetzt. Nachdem das Reaktionsgemisch über Nacht bei Raumtemperatur gerührt wurde, wurde das Lösungsmittel unter Vakuum entfernt. Es wurden 1.45 g (58 % Reinheit, 117 % d. Th.) der Titelverbindung erhalten.
LC-MS (Methode 4): Rₜ = 0.53 min; MS (ESIpos): m/z = 327 [M+H]⁺

### Intermediat 412

### Ethyl-(5RS,7RS)-2-[(3-chlor-5-fluorpyridin-2-yl)methyl]-3-oxo-7-(trifluormethyl)-2,3,5,6,7,8-hexahydro[1,2,4]triazolo[4,3-a]pyridin-5-carboxylat (Diastereomerengemsich, 4 Isomere)

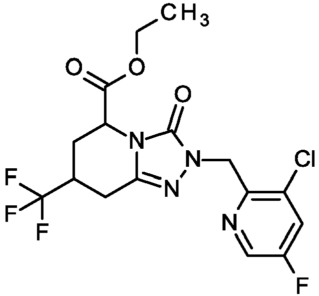

Ethyl-(5RS,7RS)-3-oxo-7-(trifluormethyl)-2,3,5,6,7,8-hexahydro[1,2,4]triazolo[4,3-a]pyridin-5-carboxylat (Diastereomerengemsich, 4 Isomere) (300 mg, 1.07 mmol) wurde in Acetonitril (10 ml) vorgelegt. Caesiumcarbonat (700 mg, 2.15 mmol) und 3-Chlor-2-(chlormethyl)-5-fluorpyridinhydrochlorid (254 mg, 96 % Reinheit, 1.13 mmol) wurden anschließend zugegeben. Das Reaktionsgemisch wurde nach Rühren über Nacht bei Raumtemperatur erneut mit 3-Chlor-2-(chlormethyl)-5-fluorpyridinhydrochlorid (254 mg, 96 % Reinheit, 1.13 mmol) versetzt und über Nacht bei 60 °C gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die Produkthaltigen Fraktionen wurden im Vakuum eingeengt und es wurden 348 mg (75 % d. Th.) der Titelverbindung erhalten.
LC-MS (Methode 3): Rₜ = 1.67 min; MS (ESIpos): m/z = 423 [M+H]⁺
¹H-NMR (400 MHz, DMSO-d6) δ [ppm]: 1.187 (1.01), 1.199 (7.45), 1.204 (2.57), 1.216 (16.00), 1.234 (7.55), 1.769 (0.74), 1.799 (1.54), 1.830 (1.61), 1.860 (0.83), 2.073 (0.42), 2.482 (0.94), 2.521 (0.84), 2.716 (1.04), 2.746 (1.26), 2.756 (1.70), 2.786 (1.98), 2.867 (1.23), 2.873 (1.39), 2.877 (1.27), 2.902 (0.68), 2.907 (0.77), 2.913 (0.75), 3.056 (0.40), 3.069 (0.55), 3.077 (0.63), 3.087 (0.58), 3.098 (0.57), 4.145 (0.51), 4.154 (1.19), 4.159 (1.36), 4.171 (3.77), 4.177 (3.81), 4.189 (3.90), 4.195 (3.66), 4.207 (1.35), 4.213 (1.28), 4.222 (0.43), 4.525 (1.71), 4.539 (1.98), 4.553 (1.89), 4.567 (1.64), 4.982 (1.78), 5.021 (4.32), 5.069 (5.20), 5.109 (1.75), 8.118 (2.64), 8.124 (2.83), 8.139 (2.69), 8.145 (2.80), 8.543 (0.91), 8.548 (5.70), 8.555 (5.07).

### Intermediat 413

### Ethyl-(5RS,7RS)-2-[(3,5-difluorpyridin-2-yl)methyl]-3-oxo-7-(trifluormethyl)-2,3,5,6,7,8-hexahydro[1,2,4]triazolo[4,3-a]pyridin-5-carboxylat (Diastereomerengemsich, 4 Isomere)

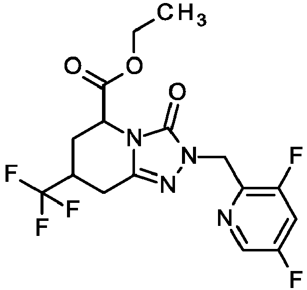

Ethyl-(5RS,7RS)-3-oxo-7-(trifluormethyl)-2,3,5,6,7,8-hexahydro[1,2,4]triazolo[4,3-a]pyridin-5-carboxylat (Diastereomerengemsich, 4 Isomere) (600 mg, 2.15 mmol) wurde in Acetonitril (10 ml) vorgelegt. Caesiumcarbonat (1.75 g, 5.37 mmol) und 2-(Chlormethyl)-3,5-difluorpyridin (387 mg, 2.36 mmol) wurden anschließend zugegeben. Das Reaktionsgemisch wurde nach Rühren über Nacht bei Raumtemperatur und anschießend über Nacht bei 60 °C gerührt. Eswurde erneut 2-(Chlormethyl)-3,5-difluorpyridin (100 mg, 0.61 mmol) zugegben und das Reaktionsgemisch für 1 Stunde bei 60 °C und für 48 Stunden bei Raumtemperatur gerührt. Das Reaktionsgeemsich wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 734 mg (79 % d. Th.) der Titelverbindung erhalten.
LC-MS (Methode 4): Rₜ = 0.82 min; MS (ESIpos): m/z = 407 [M+H]⁺

### Intermediat 414

### tert-Butyl-(5S)-3-oxo-2-{[5-(trifluormethyl)pyridin-3-yl]methyl}-2,3,5,6,7,8-hexahydro[1,2,4]triazolo[4,3-a]pyridin-5-carboxylat

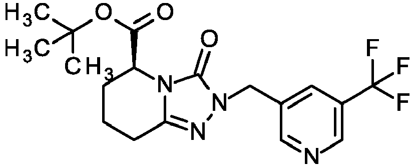

tert-Butyl-(5S)-3-oxo-2,3,5,6,7,8-hexahydro[1,2,4]triazolo[4,3-a]pyridin-5-carboxylat (154 mg, 645 µmol) wurde in Acetonitril (14 ml) vorgelegt. Caesiumcarbonat (525 mg, 1.61 mmol) und 3-(Chlormethyl)-5-(trifluormethyl)pyridin (164 mg, 838 µmol) wurden anschließend zugegeben. Das Reaktionsgemisch wurde für 5 Stunden bei 50 °C und über Nacht bei Raumtemperatur gerührt. Der Großteil an Acetonitril wurde im Vakuum entfernt und der Rückstand mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die Produkthaltigen Fraktionen wurden im Vakuum eingeengt und es wurden 26.4 mg (83 % Reinheit, 9 % d. Th.) der Titelverbindung erhalten.
LC-MS (Methode 3): Rₜ = 1.72 min; MS (ESIpos): m/z = 399 [M+H]⁺

### Intermediat 415

### (5RS,7RS)-2-[(3-Chlor-5-fluorpyridin-2-yl)methyl]-3-oxo-7-(trifluormethyl)-2,3,5,6,7,8-hexahydro[1,2,4]triazolo[4,3-a]pyridin-5-carbonsäure (Diastereomerengemsich, 4 Isomere)

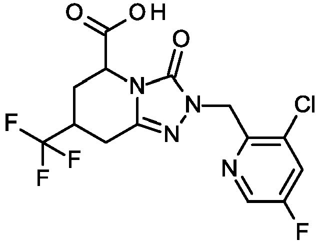

Ethyl-(5RS,7RS)-2-[(3-chlor-5-fluorpyridin-2-yl)methyl]-3-oxo-7-(trifluormethyl)-2,3,5,6,7,8-hexahydro[1,2,4]triazolo[4,3-a]pyridin-5-carboxylat (Diastereomerengemsich, 4 Isomere) (348 mg, 824 µmol) wurde in THF (10 ml) vorgelegt und Natriumethnolat (494 mg, 21 Gew-%, 1.52 mmol, in Ethanol) zugeben. Das Reaktionsgemisch wurde über Nacht bei Raumtemperatur gerührt. Das Reaktionsgemisch wurde mit Wasser, 1 N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 289 mg (87 % d. Th.) der Titelverbindung erhalten.
LC-MS (Methode 3): Rₜ = 1.14 min; MS (ESIpos): m/z = 395 [M+H]⁺

### Intermediat 416

### (5RS,7RS)-2-[(3,5-Difluorpyridin-2-yl)methyl]-3-oxo-7-(trifluormethyl)-2,3,5,6,7,8-hexahydro[1,2,4]triazolo[4,3-a]pyridin-5-carbonsäure (Diastereomerengemsich, 4 Isomere)

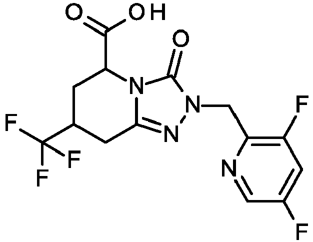

Ethyl-(5RS,7RS)-2-[(3,5-difluorpyridin-2-yl)methyl]-3-oxo-7-(trifluormethyl)-2,3,5,6,7,8-hexahydro[1,2,4]triazolo[4,3-a]pyridin-5-carboxylat (Diastereomerengemsich, 4 Isomere) (734 mg, 93 % Reinheit, 1.69 mmol) wurde in THF (20 ml) vorgelegt und Natriumethanolat-Lösung (1.01 g, 21 % Reinheit, 3.12 mmol gelöst in Ethanol) zugeben. Das Reaktionsgemisch wurde für 20 min bei 0°C und über Nacht bei Raumtemperatur gerührt. Das Reaktionsgemisch wurde mit Wasser, 1 N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 213 mg (32 % d. Th.) der Titelverbindung erhalten.
LC-MS (Methode 3): Rₜ = 1.04 min; MS (ESIpos): m/z = 379 [M+H]⁺
¹H-NMR (400 MHz, DMSO-d6) δ [ppm]: 1.309 (1.32), 1.324 (2.78), 1.341 (1.44), 1.774 (0.56), 1.804 (0.56), 2.064 (0.56), 2.072 (0.41), 2.096 (0.40), 2.169 (0.58), 2.224 (1.06), 2.240 (1.22), 2.259 (2.88), 2.275 (3.39), 2.301 (9.72), 2.332 (2.28), 2.455 (0.76), 2.636 (2.41), 2.666 (8.01), 2.701 (8.02), 2.743 (1.91), 2.785 (0.63), 2.849 (0.55), 2.949 (5.55), 2.979 (4.61), 3.848 (0.90), 4.073 (0.62), 4.090 (1.31), 4.108 (1.11), 4.113 (1.20), 4.391 (0.55), 4.405 (0.62), 4.418 (0.62), 4.433 (0.53), 4.665 (5.53), 4.671 (7.00), 4.680 (5.72), 4.685 (5.62), 4.836 (0.65), 4.857 (0.88), 4.874 (0.78), 4.898 (1.52), 4.964 (3.41), 4.998 (11.30), 5.030 (10.17), 5.066 (2.90), 7.468 (0.68), 7.495 (0.65), 7.937 (3.94), 7.943 (4.33), 7.962 (6.26), 7.966 (6.71), 7.984 (4.12), 7.990 (4.32), 8.051 (1.42), 8.420 (0.48), 8.468 (14.61), 8.473 (16.00), 13.619 (0.72).

### Intermediat 417

### (5S)-3-Oxo-2-{[5-(trifluormethyl)pyridin-3-yl]methyl}-2,3,5,6,7,8-hexahydro[1,2,4]triazolo[4,3-a]pyridin-5-carbonsäure

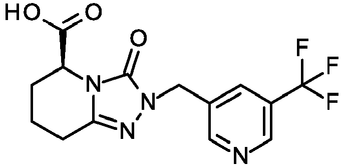

tert-Butyl-(5S)-3-oxo-2-{[5-(trifluormethyl)pyridin-3-yl]methyl}-2,3,5,6,7,8-hexahydro[1,2,4]triazolo[4,3-a]pyridin-5-carboxylat (1.13 g, 2.83 mmol) wurde in Dichlormethan (180 µl) gelöst und bei Raumtemperatur mit Trifluoressigsäure (4.4 ml, 57 mmol) versetzt. Nachdem das Reaktionsgemisch über Nacht bei Raumtemperatur gerührt wurde, wurde das Lösungsmittel unter Vakuum entfernt. Es wurden 980 mg (101 % d. Th.) der Titelverbindung erhalten.
LC-MS (Methode 3): Rₜ = 1.01 min; MS (ESIpos): m/z = 343 [M+H]⁺

### Intermediat 418

### (5S)-2-{[3-Chlor-5-(trifluormethyl)pyridin-2-yl]methyl}-5-[(3,3-difluorpyrrolidin-1-yl)carbonyl]-6,7-dihydro[1,2,4]triazolo[4,3-a]pyridin-3,8(2H,5H)-dion

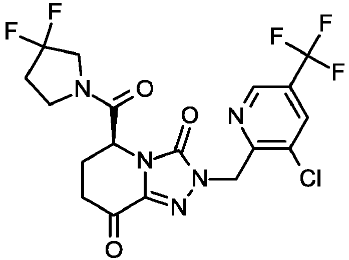

(5S,8RS)-2-{[3-Chlor-5-(trifluormethyl)pyridin-2-yl]methyl}-5-[(3,3-difluorpyrrolidin-1 - yl)carbonyl]-8-hydroxy-5,6,7,8-tetrahydro[1,2,4]triazolo[4,3-a]pyridin-3(2H)-on (Diastereomerengemisch; 2 Isomere) (58.0 mg, 120 µmol) wurde in Dichlormethan (10 ml) gelöst und bei Raumtemperatur Mangan(IV)-oxid (209 mg, 2.41 mmol) zugegeben. Das Reaktionsgemisch wurde über Nacht bei Raumtemperatur gerührt. Die Suspension wurde über Celite filtriert und das Filtrat unter Vakuum eingeengt. Es wurden 49.4 mg (86 % d. Th.) der Titelverbindung erhalten.
LC-MS (Methode 3): Rₜ = 1.55 min; MS (ESIpos): m/z = 480 [M+H]⁺

### Intermediat 419

### (5S)-2-[(5-Chlorpyridin-2-yl)methyl]-5-[(3,3-difluorpyrrolidin-1-yl)carbonyl]-6,7-dihydro[1,2,4]triazolo[4,3-a]pyridin-3,8(2H,5H)-dion

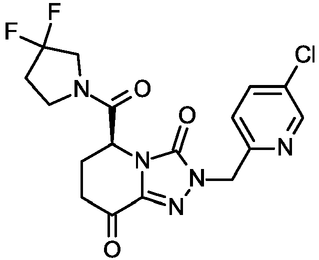

(5S,8RS)-2-[(5-Chlorpyridin-2-yl)methyl]-5-[(3,3-difluorpyrrolidin-1-yl)carbonyl]-8-hydroxy-5,6,7,8-tetrahydro[1,2,4]triazolo[4,3-a]pyridin-3(2H)-on (Diastereomerengemisch; 2 Isomere) (103 mg, 85 % Reinheit, 211 µmol) wurde in Dichlormethan (8.7 ml) gelöst und bei Raumtemperatur Mangan(IV)-oxid (366 mg, 4.21 mmol) zugegeben. Das Reaktionsgemisch wurde über Nacht bei Raumtemperatur gerührt. Die Suspension wurde über Celite filtriert und das Filtrat unter Vakuum eingeengt. Es wurden 79.4 mg (88 % Reinheit, 81 % d. Th.) der Titelverbindung erhalten.
LC-MS (Methode 3): Rₜ = 1.25 min; MS (ESIpos): m/z = 412 [M+H]⁺

### Intermediat 420

### (5S)-2-[(5-Chlorpyridin-2-yl)methyl]-5-[(3-fluorazetidin-1-yl)carbonyl]-6,7-dihydro[1,2,4]triazolo[4,3-a]pyridin-3,8(2H,5H)-dion

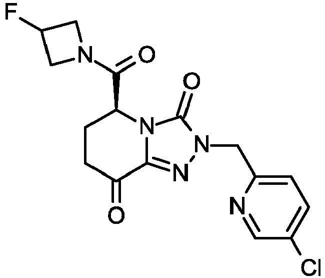

(5S,8RS)-2-[(5-Chlorpyridin-2-yl)methyl]-5-[(3-fluorazetidin-1-yl)carbonyl]-8-hydroxy-5,6,7,8-tetrahydro[1,2,4]triazolo[4,3-a]pyridin-3(2H)-on (Diastereomerengemisch; 2 Isomere) (48.1 mg, 86 % Reinheit, 108 µmol) wurde in Dichlormethan (800 µl) gelöst und bei Raumtemperatur Mangan(IV)-oxid (188 mg, 2.17 mmol) zugegeben. Das Reaktionsgemisch wurde über Nacht bei Raumtemperatur gerührt. Die Suspension wurde über Celite filtriert und das Filtrat unter Vakuum eingeengt. Es wurden 37.1 mg (63 % Reinheit, 57 % d. Th.) der Titelverbindung erhalten.
LC-MS (Methode 1): Rₜ = 0.83 min; MS (ESIpos): m/z = 380 [M+H]⁺

### Intermediat 421

### (5S)-2-[(3,5-Difluorpyridin-2-yl)methyl]-5-[(3,3-difluorpyrrolidin-1-yl)carbonyl]-6,7-dihydro[1,2,4]triazolo[4,3-a]pyridin-3,8(2H,5H)-dion

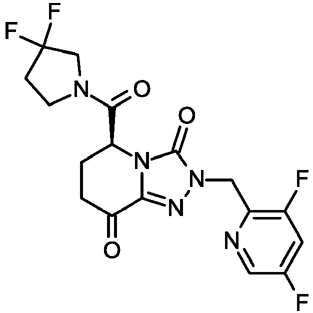

(5S,8RS)-2-[(3,5-Difluorpyridin-2-yl)methyl]-5-[(3,3-difluorpyrrolidin-1-yl)carbonyl]-8-hydroxy-5,6,7,8-tetrahydro[1,2,4]triazolo[4,3-a]pyridin-3(2H)-on (Diastereomerengemisch; 2 Isomere) (112 mg, 270 µmol) wurde in Dichlormethan (10 ml) gelöst und bei Raumtemperatur Mangan(IV)-oxid (469 mg, 5.39 mmol) zugegeben. Das Reaktionsgemisch wurde über Nacht bei Raumtemperatur gerührt. Die Suspension wurde über Celite filtriert und das Filtrat unter Vakuum eingeengt. Es wurden 61.3 mg (55 % d. Th.) der Titelverbindung erhalten.
LC-MS (Methode 4): Rₜ = 0.64 min; MS (ESIpos): m/z = 414 [M+H]⁺

### Intermediat 422

### (5S)-5-{[(3S)-3-Fluorpyrrolidin-1-yl]carbonyl}-2-{[6-(trifluormethyl)pyridin-3-yl]methyl}-6,7-dihydro[1,2,4]triazolo[4,3-a]pyridin-3,8(2H,5H)-dion (Isomer 1)

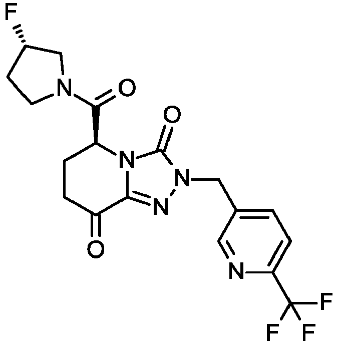

(5S,8RS)-5-{[(3S)-3-Fluorpyrrolidin-1-yl]carbonyl}-8-hydroxy-2-{[6-(trifluormethyl)pyridin-3-yl]methyl}-5,6,7,8-tetrahydro[1,2,4]triazolo[4,3-a]pyridin-3(2H)-on (Diastereomerengemisch; 2 Isomere) (30.0 mg, 69.9 µmol) wurde in Dichlormethan (180 µl) bei Raumtemperatur vorgelegt und das Reaktionsgemisch wurde auf 0°C abgekühlt. Anschließend wurde Dess-Martin-Periodinan (35.6 mg, 83.8 µmol) zugegeben und das Reaktionsgemisch für 15 min bei 0°C und für 2 Stunden bei Raumtemperatur gerührt. Es wurde erneut Dess-Martin-Periodinan (35.6 mg, 83.8 µmol) zugegeben und über Nacht gerührt. Das Reaktionsgemisch wurde mit Diethylether und einer Lösung von Natriumthiosulfat in gesättigter wässriger Natriumhydrogencarbonat-Lösung versetzt und es wurde gerührt bis die zwei Phasen homogen waren. Die organische Phase wurde über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Die produkthaltigen Fraktionen wurden im Vakuum eingeengt und es wurden 1.60 mg (5 % d. Th.) der Titelverbindung erhalten.
LC-MS (Methode 3): Rₜ = 1.28 min; MS (ESIpos): m/z = 428 [M+H]⁺
¹H-NMR (400 MHz, DMSO-d6) δ [ppm]: -0.150 (1.86), -0.044 (0.28), -0.035 (0.39), -0.033 (0.39), -0.029 (0.51), -0.027 (0.51), -0.024 (0.56), -0.022 (0.85), -0.020 (1.01), -0.016 (1.69), - 0.009 (16.00), 0.007 (14.08), 0.013 (2.08), 0.015 (1.18), 0.018 (0.85), 0.020 (0.56), 0.023 (0.56), 0.025 (0.45), 0.029 (0.34), 0.146 (1.75), 2.113 (0.34), 2.135 (0.34), 2.151 (0.39), 2.193 (0.28), 2.238 (0.45), 2.266 (0.56), 2.292 (0.39), 2.323 (1.35), 2.327 (1.86), 2.331 (1.46), 2.366 (2.37), 2.396 (0.45), 2.424 (0.23), 2.431 (0.28), 2.453 (0.34), 2.523 (4.73), 2.525 (3.66), 2.558 (1.58), 2.612 (0.68), 2.622 (0.62), 2.646 (0.28), 2.664 (1.46), 2.669 (1.80), 2.674 (1.35), 2.709 (2.08), 3.364 (0.56), 3.391 (0.34), 3.400 (0.34), 3.418 (0.28), 3.435 (0.34), 3.507 (0.28), 3.534 (0.34), 3.544 (0.34), 3.651 (0.62), 3.673 (0.56), 3.681 (0.68), 3.702 (0.51), 3.711 (0.56), 3.749 (0.34), 3.792 (0.23), 3.815 (0.34), 3.841 (0.68), 3.889 (0.39), 3.919 (0.39), 3.948 (0.34), 5.029 (0.68), 5.068 (0.45), 5.081 (0.85), 5.185 (0.51), 5.224 (2.14), 5.239 (1.52), 5.245 (1.86), 5.285 (0.73), 5.417 (0.51), 5.541 (0.28), 7.928 (0.96), 7.948 (2.93), 7.965 (1.52), 7.987 (0.51), 8.535 (0.45), 8.699 (1.75).

### Intermediat 423

### (5S)-2-{[3-Chlor-5-(trifluormethyl)pyridin-2-yl]methyl}-5-{[(3S)-3-fluorpyrrolidin-1-yl]carbonyl}-6,7-dihydro[1,2,4]triazolo[4,3-a]pyridin-3,8(2H,5H)-dion

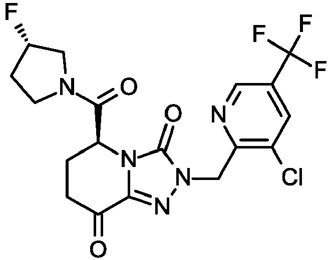

(5S,8SR)-2-{[3-Chlor-5-(trifluormethyl)pyridin-2-yl]methyl}-5-{[(3S)-3-fluorpyrrolidin-1 - yl]carbonyl}-8-hydroxy-5,6,7,8-tetrahydro[1,2,4]triazolo[4,3-a]pyridin-3(2H)-on (Diastereomerengemisch; 2 Isomere) (93.0 mg, 201 µmol) wurde in Dichlormethan (2.0 ml) gelöst und bei Raumtemperatur Mangan(IV)-oxid (349 mg, 4.01 mmol) zugegeben. Das Reaktionsgemisch wurde über Nacht bei Raumtemperatur gerührt. Die Suspension wurde über Celite filtriert und das Filtrat unter Vakuum eingeengt. Es wurden 75.5 mg (82 % d. Th.) der Titelverbindung erhalten.
LC-MS (Methode 3): Rₜ = 1.39 min; MS (ESIpos): m/z = 462 [M+H]⁺

### Ausführungsbeispiele:

### Beispiel 1

### (5RS)-5-(Pyrrolidin-1-ylcarbonyl)-2-[4-(trifluormethyl)benzyl]-5,6,7,8-tetrahydro[1,2,4]triazolo[4,3-a]pyridin-3(2H)-on (Racemat)

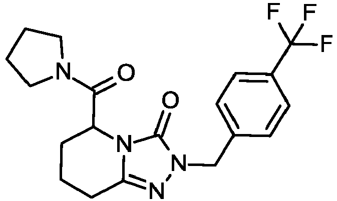

### (5RS)-5-(Pyrrolidin-1-ylcarbonyl)-5,6,7,8-tetrahydro[1,2,4]triazolo[4,3-a]pyridin-3(2H)-on

(Racemat) (70.0 mg, 296 µmol) wurde in 5.0 ml Acetonitril gelöst, dann mit Cäsiumcarbonat (145 mg, 444 µmol) und 1-(Brommethyl)-4-(trifluormethyl)benzol (85.0 mg, 356 µmol) versetzt und über Nacht unter Argon bei Raumtemperatur gerührt. Zur Aufarbeitung wurde der Ansatz mit 1 ml Wasser verrührt und über präparative HPLC getrennt (GromSil 120 ODS-4HE, 250x30mm 10µm. Flow: 50ml/min. Gradient ( A= Wasser+ 0,1% Ameisensäure, B= Acetonitril ): 0min 0% B, 6min 10%B, 27min 95% B, 38min 95 % B , 40min 0%B. Laufzeit pro Trennung 40min. Detektion: 210 nm). Die Produktfraktionen wurden vereinigt, eingeengt und lyophilisiert. Es wurden so 92 mg (79 % d. Th.) der Titelverbindung erhalten.
LC-MS (Methode 3): Rₜ = 1.52 min; MS (ESIpos): m/z = 395 [M+H]⁺
1H-NMR (400MHz, DMSO-d6): δ [ppm]= 1.61 - 1.84 (m, 4H), 1.86 - 2.11 (m, 4H), 2.45- 2.68 (m, 2H, teilweise durch Lösungsmittelsignal verdeckt), 3.21 - 3.41 (m, 2H, teilweise von Wassersignal überlagert), 3.47 (dt, 1H), 3.62 (dt, 1H), 4.75 (dd, 1H), 4.92 (s, 2H), 7.41 - 7.47 (m, 2H), 7.68 - 7.74 (m, 2H).

### Beispiel 2

### (5RS)-2-(4-tert-Butylbenzyl)-5-(pyrrolidin-1-ylcarbonyl)-5,6,7,8-tetrahydro[1,2,4]triazolo[4,3-a]pyridin-3(2H)-on (Racemat)

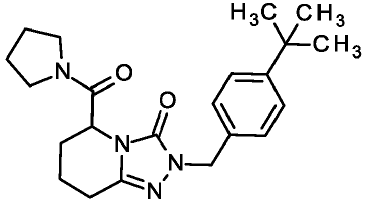

### (5RS)-5-(Pyrrolidin-1-ylcarbonyl)-5,6,7,8-tetrahydro[1,2,4]triazolo[4,3-a]pyridin-3(2H)-on

(Racemat) (50.0 mg, 212 µmol) wurde in 3.0 ml Acetonitril gelöst, dann mit Cäsiumcarbonat (103 mg, 317 pmol) und 1-(Brommethyl)-4-tert-butylbenzol (47 µl, 250 µmol) versetzt und über Nacht unter Argon bei Raumtemperatur gerührt. Zur Aufarbeitung wurde der Ansatz mit 1 ml Wasser verrührt und über präparative HPLC getrennt (GromSil 120 ODS-4HE, 250x30mm 10µm. Flow: 50ml/min. Gradient ( A= Wasser+ 0,1% Ameisensäure, B= Acetonitril ): 0min 0% B, 6min 10%B, 27min 95% B, 38min 95 % B , 40min 0%B. Laufzeit pro Trennung 40min. Detektion: 210 nm). Die Produktfraktionen wurden vereinigt, eingeengt und lyophilisiert. Es wurden so 63 mg (81 % d. Th.) der Titelverbindung erhalten.
LC-MS (Methode 3): Rₜ = 1.75 min; MS (ESIpos): m/z = 383 [M+H]⁺
¹H-NMR (400MHz, DMSO-d₆): δ [ppm]= 1.26 (s, 9H), 1.61 - 1.84 (m, 4H), 1.87 - 2.10 (m, 4H), 2.46 - 2.65 (m, 2H, teilweise durch Lösungsmittelsignal verdeckt), 3.21 - 3.41 (m, 2H, teilweise von Wassersignal überlagert), 3.47 (dt, 1H), 3.62 (dt, 1H), 4.70 - 4.73 (m, 3H), 7.13 - 7.18 (m, 2H), 7.31 - 7.37 (m, 2H).

### Beispiel 3

### (5RS)-2-[(1RS)-1-(4-Chlorphenyl)ethyl]-5-(pyrrolidin-1-ylcarbonyl)-5,6,7,8-tetrahydro[1,2,4]triazolo[4,3-a]pyridin-3(2H)-on (Diastereomerengemisch; 4 Isomere)

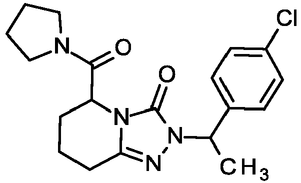

### (5RS)-5-(Pyrrolidin-1-ylcarbonyl)-5,6,7,8-tetrahydro[1,2,4]triazolo[4,3-a]pyridin-3(2H)-on

(Racemat) (50.0 mg, 212 µmol) wurde in 3.0 ml Acetonitril gelöst, dann mit Cäsiumcarbonat (103 mg, 317 µmol) und 1-[(1RS)-1-Bromethyl]-4-chlorbenzol (Racemat) (55.7 mg, 254 µmol) versetzt und über Nacht unter Argon bei Raumtemperatur gerührt. Zur Aufarbeitung wurde der Ansatz mit 1 ml Wasser verrührt und über präparative HPLC getrennt (GromSil 120 ODS-4HE, 250x30mm 10µm. Flow: 50ml/min. Gradient ( A= Wasser+ 0,1% Ameisensäure, B= Acetonitril ): 0min 0% B, 6min 10%B, 27min 95% B, 38min 95 % B , 40min 0%B. Laufzeit pro Trennung 40min. Detektion: 210 nm). Die Produktfraktionen wurden vereinigt, eingeengt und lyophilisiert. Es wurden so 56 mg (71 % d. Th.) der Titelverbindung als Diastereomerengemisch erhalten.
LC-MS (Methode 3): Rₜ = 1.52 min; MS (ESIpos): m/z = 375 [M+H]⁺
¹H-NMR (400MHz, DMSO-d₆): δ [ppm]= 1.57 - 1.64 (m, 3H), 1.64 -1.82 (m, 4H), 1.86 - 2.10 (m, 4H), 2.50 - 2.69 (m, 2H, teilweise durch Lösungsmittelsignal verdeckt), 3.18 - 3.39 (m, 2H, teilweise von Wassersignal überlagert), 3.41 - 3.50 (m, 1H), 3.56 - 3.65 (m, 1H), 4.67 - 4.75 (td, 1H), 5.25 - 5.33 (m, 1H), 7.28 - 7.41 (m, 4H).

In einem weiteren Ansatz zur gleichen Titelverbindung wurde (5RS)-2-[(1RS)-1-(4-Chlorphenyl)ethyl]-3-oxo-2,3,5,6,7,8-hexahydro[1,2,4]triazolo[4,3-a]pyridin-5-carbonsäure (Diastereomerengemisch; 4 Isomere) (392 mg, 90 % Reinheit, 1.10 mmol) in 12 ml THF gelöst. Man gab Triethylamin (310 µl, 2.2 mmol), HATU (542 mg, 1.43 mmol) und Pyrrolidin (110 µl, 1.3 mmol) zu und rührte 2h unter Argon bei Raumtemperatur. Zur Aufarbeitung wurde der Ansatz mit Wasser verdünnt und dann im Vakuum weitgehend eingeengt. Der Rückstand wurde in Ethylacetat/Wasser aufgenommen. Nach Extraktion und Abtrennung der organischen Phase wurde die wäßrige Phase noch zweimal mit Ethylacetat extrahiert. Die organischen Phasen wurden mit gesättigter wässriger Natriumchloridlösung gewaschen und über Natriumsulfat getrocknet. Der nach Einengen und Trocknen im Vakuum verbliebene Rückstand wurde in Acetonitril/Wasser gelöst und in 2 Chargen über präparative HPLC getrennt (Säule: Kromasil C18, 125x30mm, 10µm, Eluent: Acetonitril (B) / Wasser + 0,1 % TFA (A), Gradient: 0min 90% A, 6min 90% A, 18min 5%A, 20min 5%A, 21min 90%A, Fluss: 75ml/min, Detektor: 210nm). Die vereinigten produkthaltigen Fraktionen wurden im Vakuum eingeengt und getrocknet. So wurden 293 mg 56 mg (98 % Reinheit, 70 % d. Th.) der Titelverbindung als racemisches Diastereomerengemisch erhalten.

### Beispiel 4

### (5RS)-2-[(1RS)-1-(4-Chlorphenyl)ethyl]-5-(pyrrolidin-1-ylcarbonyl)-5,6,7,8-tetrahydro[1,2,4]triazolo[4,3-a]pyridin-3(2H)-on (Isomer 3)

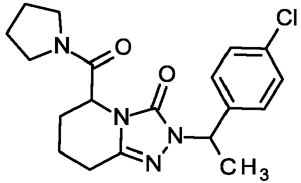

(5RS)-2-[(1RS)-1-(4-Chlorphenyl)ethyl]-5-(pyrrolidin-1-ylcarbonyl)-5,6,7,8-tetrahydro[1,2,4]triazolo[4,3-a]pyridin-3(2H)-on (Diastereomerengemisch; 4 Isomere) wurde durch zwei präparative Flüssigchromatographien an chiraler Phase getrennt. Zunächst wurde Stereoisomer 4 durch präparative Flüssigchromatographie an chiraler Phase abgetrennt [Probenvorbereitung: 392 mg gelöst in 10 ml Ethanol; Injektionsvolumen: 2.0 ml; Säule: Daicel Chiralcel^{®} OX-H 5µm, 250 x 50 mm; Laufmittel: Ethanol, Fluß: 15.0 ml/min; Temperatur 50°C; UV Detektion: 220 nm]. Man erhielt 61 mg vom zuletzt eluierenden Stereoisomer 4

Im zweiten Schritt wurden die vereinigten Fraktionen der verbliebenen drei Stereoisomeren erneut gelöst und getrennt [Probe in 10 ml Ethanol; Injektionsvolumen: 0.4 ml; Säule: Daicel Chiralcel^{®} OX-H 5µm, 250 x 50 mm; Laufmittel: Wasser/Ethanol: isokratisch 50% Ethanol; Fluß: 15.0 ml/min; Temperatur 50°C; UV Detektion: 220 nm]. Man erhielt, in der Reihenfolge der Elution, 51 mg von Isomer 1, 62 mg von Isomer 2 und 53 mg von Isomer 3.

### Isomer 3:

spez. Drehwert: -145,16 (589nm, 0,2450g / 100cm³ MeOH)
Analytische chirale HPLC: Rₜ = 4.74 min, d.e./e.e. = 100% [Säule: Daicel Chiralcel^{®} OX-H 250 x 4.6 mm; Laufmittel: Ethanol; Fluß: 1 ml/min; 50 °C UV Detektion: 220 nm].
LC-MS (Methode 4): Rₜ = 0.82 min; MS (ESIpos): m/z = 375 [M+H]⁺
¹H-NMR (500MHz, DMSO-d₆): δ [ppm]= 1.61 (d, 3H), 1.68 - 1.74 (m, 2H); 1.74 - 1.83 (m, 2H), 1.87 - 1.98 (m, 3H), 1.98 - 2.07 (m, 1H), 2.50 - 2.59 (m, 1H, teilweise durch Lösungsmittelsignal verdeckt), 2.65 (dt, 1H), 3.21 - 3.39 (m, 2H, teilweise von Wassersignal überlagert), 3.45 (dt, 1H), 3.60 (dt, 1H), 4.70 (dd, 1H), 5.28 (q, 1H), 7.30 - 7.34 (m, 2H), 7.36 - 7.40 (m, 2H).

### Beispiel 5

### (5RS)-2-[(1RS)-1-(4-Chlorphenyl)ethyl]-5-(pyrrolidin-1-ylcarbonyl)-5,6,7,8-tetrahydro[1,2,4]triazolo[4,3-a]pyridin-3(2H)-on (Isomer 4)

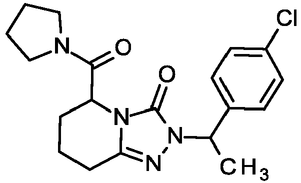

(5RS)-2-[(1RS)-1-(4-Chlorphenyl)ethyl]-5-(pyrrolidin-1-ylcarbonyl)-5,6,7,8-tetrahydro[1,2,4]triazolo[4,3-a]pyridin-3(2H)-on (Diastereomerengemisch; 4 Isomere) wurde durch zwei präparative Flüssigchromatographien an chiraler Phase getrennt.Zunächst wurde Stereoisomer 4 durch präparative Flüssigchromatographie an chiraler Phase abgetrennt [Probenvorbereitung: 392 mg gelöst in 10 ml Ethanol; Injektionsvolumen: 2.0 ml; Säule: Daicel Chiralcel^{®} OX-H 5µm, 250 x 50 mm; Laufmittel: Ethanol, Fluß: 15.0 ml/min; Temperatur 50°C; UV Detektion: 220 nm]. Man erhielt 61 mg vom zuletzt eluierenden Stereoisomer 4

Im zweiten Schritt wurden die vereinigten Fraktionen der verbliebenen drei Stereoisomeren erneut gelöst und getrennt [Probe in 10 ml Ethanol; Injektionsvolumen: 0.4 ml; Säule: Daicel Chiralcel^{®} OX-H 5µm, 250 x 50 mm; Laufmittel: Wasser/Ethanol: isokratisch 50% Ethanol; Fluß: 15.0 ml/min; Temperatur 50°C; UV Detektion: 220 nm]. Man erhielt, in der Reihenfolge der Elution, 51 mg von Isomer 1, 62 mg von Isomer 2 und 53 mg von Isomer 3.

### Isomer 4:

Analytische chirale HPLC: Rₜ = 12.6 min, d.e./e.e. = 100% [Säule: Daicel Chiralcel^{®} OX-H 250 x 4.6 mm; Laufmittel: Ethanol; Fluß: 1 ml/min; 50 °C UV Detektion: 220 nm].
spez. Drehwert: +134,80 (589nm, 0,2500g / 100cm³ MeOH)
LC-MS (Methode 4): Rₜ = 0.81 min; MS (ESIpos): m/z = 375 [M+H]⁺
1H-NMR (500MHz, DMSO-d6): δ [ppm]= 1.60 (d, 3H), 1.63 - 1.82 (m, 4H), 1.85 - 2.09 (m, 4H), 2.50 - 2.61 (m, 1H, teilweise durch Lösungsmittelsignal verdeckt), 2.65 (dt, 1H), 3.23 (dt, 1H), 3.28 - 3.36 (m, 1H, teilweise von Wassersignal überlagert), 3.45 (dt, 1H), 3.60 (dt, 1H), 4.72 (dd, 1H), 5.29 (q, 1H), 7.29 - 7.33 (m, 2H), 7.37 - 7.41 (m, 2H).

### Beispiel 6

### (5RS)-2-[4-(Methylsulfonyl)benzyl]-5-(pyrrolidin-1-ylcarbonyl)-5,6,7,8-tetrahydro[1,2,4]triazolo[4,3-a]pyridin-3(2H)-on (Racemat)

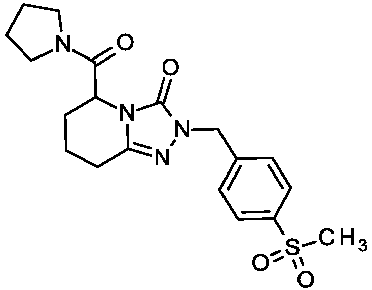

### (5RS)-5-(Pyrrolidin-1 -ylcarbonyl)-5,6,7,8-tetrahydro[1,2,4]triazolo[4,3-a]pyridin-3(2H)-on

(Racemat) (50.0 mg, 212 µmol) wurde in 3.0 ml Acetonitril gelöst, dann mit Cäsiumcarbonat (103 mg, 317 µmol) und 1-(Brommethyl)-4-(methylsulfonyl)benzol (68.5 mg, 275 pmol) versetzt und über Nacht unter Argon bei Raumtemperatur gerührt. Zur Aufarbeitung wurde der Ansatz mit 1 ml Wasser verrührt und über präparative HPLC getrennt (GromSil 120 ODS-4HE, 250x30mm 10µm. Flow: 50ml/min. Gradient ( A= Wasser+ 0,1% Ameisensäure, B= Acetonitril ): 0min 0% B, 6min 10%B, 27min 95% B, 38min 95 % B , 40min 0%B. Laufzeit pro Trennung 40min. Detektion: 210 nm). Die Produktfraktionen wurden vereinigt, eingeengt und lyophilisiert. Es wurden so 71 mg (82 % d. Th.) der Titelverbindung erhalten.
LC-MS (Methode 3): Rₜ = 0.96 min; MS (ESIpos): m/z = 405 [M+H]⁺
1H-NMR (400MHz, DMSO-d6): δ [ppm]= 1.61 - 1.84 (m, 4H), 1.87 - 2.12 (m, 4H), 2.47 - 2.69 (m, 2H, teilweise durch Lösungsmittelsignal verdeckt), 3.20 (s, 3H), 3.22 - 3.40 (m, 2H, teilweise von Wassersignal überlagert), 3.47 (dt, 1H), 3.62 (dt, 1H), 4.76 (dd, 1H), 4.94 (s, 2H), 7.45 - 7.51 (m, 2H), 7.87 - 7.92 (m, 2H).

### Beispiel 7

### (5RS)-2-[4-(Difluormethoxy)benzyl]-5-(pyrrolidin-1-ylcarbonyl)-5,6,7,8-tetrahydro[1,2,4]triazolo[4,3-a]pyridin-3(2H)-on (Racemat)

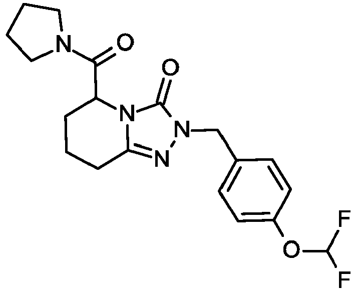

(5RS)-5-(Pyrrolidin-1-ylcarbonyl)-5,6,7,8-tetrahydro[1,2,4]triazolo[4,3-a]pyridin-3(2H)-on (Racemat) (50.0 mg, 212 µmol) wurde in 3.0 ml Acetonitril gelöst, dann mit Cäsiumcarbonat (103 mg, 317 µmol) und 1-(Brommethyl)-4-(difluormethoxy)benzol (60.2 mg, 254 µmol) versetzt und über Nacht unter Argon bei Raumtemperatur gerührt. Zur Aufarbeitung wurde der Ansatz mit 1 ml Wasser verrührt und über präparative HPLC getrennt (GromSil 120 ODS-4HE, 250x30mm 10µm. Flow: 50ml/min. Gradient ( A= Wasser+ 0,1% Ameisensäure, B= Acetonitril ): 0min 0% B, 6min 10%B, 27min 95% B, 38min 95 % B , 40min 0%B. Laufzeit pro Trennung 40min. Detektion: 210 nm). Die Produktfraktionen wurden vereinigt, eingeengt und lyophilisiert. Das erhaltene Produkt wurde ein zweites Mal durch präparative HPLC wie oben gereinigt. Es wurden so nach erneutem Einengen und Lyophilisieren 29 mg (35 % d. Th.) der Titelverbindung erhalten.
LC-MS (Methode 6): Rₜ = 1.39 min; MS (ESIpos): m/z = 393 [M+H]⁺
1H-NMR (400MHz, DMSO-d6): δ [ppm]= 1.61 - 1.84 (m, 4H), 1.87 - 2.11 (m, 4H), 2.46 - 2.66 (m, 2H, teilweise durch Lösungsmittelsignal verdeckt), 3.21 - 3.40 (m, 2H, teilweise von Wassersignal überlagert), 3.47 (dt, 1H), 3.62 (dt, 1H), 4.73 (dd, 1H), 4.80 (s, 2H), , 7.11 - 7.17 (m, 2H), 7.20 (s, 1H) 7.25 - 7.31 (m, 2H).

### Beispiel 8

### (5RS)-2-(3-Methylbenzyl)-5-(pyrrolidin-1-ylcarbonyl)-5,6,7,8-tetrahydro[1,2,4]triazolo[4,3-a]pyridin-3(2H)-on (Racemat)

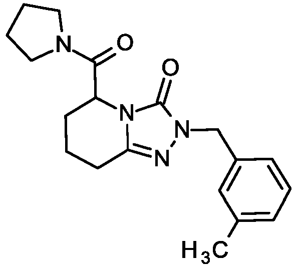

### (5RS)-5-(Pyrrolidin-1-ylcarbonyl)-5,6,7,8-tetrahydro[1,2,4]triazolo[4,3-a]pyridin-3(2H)-on

(Racemat) (45.0 mg, 190 µmol) und Cäsiumcarbonat (93.1 mg, 286 µmol) wurden in 3.0 ml Acetonitril suspendiert, dann mit 1-(Brommethyl)-3-methylbenzol (31 µl, 230 µmol) versetzt und zunächst für 2h, dann weiter über Nacht bei Raumtemperatur gerührt. Zur Aufarbeitung wurde der Ansatz mit Ethylacetat und Wasser verdünnt und die organische Phase abgetrennt. Die wässrige Phase wurde noch zweimal mit Ethylacetat extrahiert. Die vereinigten organischen Phasen wurden mit gesättigter wässriger Natriumchloridlösung gewaschen und anschließend über Natriumsulfat getrocknet. Der nach Filtration und Einengen im Vakuum erhaltene Rückstand wurde in Acteonitrl/Wasser gelöst und über präparative HPLC getrennt (Säule: Kromasil C18, 125x30mm, 10µm, Eluent: Acetonitril (B) / Wasser + 0,1 % TFA (A), Gradient: 0min 90% A, 6min 90% A, 18min 5%A, 20min 5%A, 21min 90%A, Fluss: 75ml/min, Detektor: 210nm). Die Produktfraktionen wurden vereinigt, eingeengt und lyophilisiert und im Vakuum nachgetrocknet. Es wurden so 35.1 mg (54 % d. Th.) der Titelverbindung erhalten.
LC-MS (Methode 4): Rₜ = 0.77 min; MS (ESIpos): m/z = 341 [M+H]⁺
¹H-NMR (500MHz, DMSO-d₆): δ [ppm]= 1.63 - 1.84 (m, 4H), 1.88 - 2.09 (m, 4H), 2.28 (s, 3H), 2.45 - 2.65 (m, 2H, teilweise durch Lösungsmittelsignal verdeckt), 3.26 (dt, 1H) 3.36 (dt, 1H) 3.40 - 3.66 (m, 2H, teilweise von Wassersignal überlagert), 4.70 - 4.79 (m, 3H), 6.99 - 7.10 (m, 3H), 7.21 (t, 1H).

### Beispiel 9

### (5RS)-2-[4-(3-Methyl-1,2,4-oxadiazol-5-yl)benzyl]-5-(pyrrolidin-1-ylcarbonyl)-5,6,7,8-tetrahydro[1,2,4]triazolo[4,3-a]pyridin-3(2H)-on (Racemat)

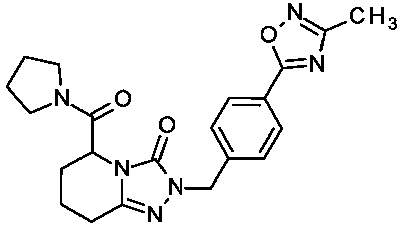

### (5RS)-5-(Pyrrolidin-1-ylcarbonyl)-5,6,7,8-tetrahydro[1,2,4]triazolo[4,3-a]pyridin-3(2H)-on

(Racemat) (55.0 mg, 233 µmol) und Cäsiumcarbonat (114 mg, 349 µmol) wurden in 3.0 ml Acetonitril suspendiert, dann mit 5-[4-(Brommethyl)phenyl]-3-methyl-1,2,4-oxadiazol (70.7 mg, 279 µmol) versetzt und zunächst für 2h, dann weiter über Nacht bei Raumtemperatur gerührt. Zur Aufarbeitung wurde der vorhandene Niederschlag abgesaugt und mit Acetonitril gewaschen. Das Filtrat wurde eingeengt, in Acetonitril/Wasser aufgenommen und über präparative HPLC getrennt (Säule: Kromasil C18, 125x30mm, 10µm, Eluent: Acetonitril (B) / Wasser + 0,1 % TFA (A), Gradient: 0min 90% A, 6min 90% A, 18min 5%A, 20min 5%A, 21min 90%A, Fluss: 75ml/min, Detektor: 210nm). Die Produktfraktionen wurden vereinigt, eingeengt und im Vakuum getrocknet. Es wurden so 42.0 mg (44 % d. Th.) der Titelverbindung erhalten.
LC-MS (Methode 3): Rₜ = 1.26 min; MS (ESIpos): m/z = 409 [M+H]⁺
¹H-NMR (400MHz, DMSO-d₆): δ [ppm]= 1.64 - 1.85 (m, 4H), 1.88 - 2.12 (m, 4H), 2.42 (s, 3H), 2.45 - 2.70 (m, 2H, teilweise durch Lösungsmittelsignal verdeckt), 3.20 - 3.57 (m, 3H, teilweise von Wassersignal überlagert), 3.63 (dt, 1H), 4.73 - 4.78 (m, 1H), 4.93 (s, 2H), 7.44 - 7.48 (m, 2H), 8.04 - 8.08 (m, 2H).

### Beispiel 10

### (5RS)-2-[4-(5-Methyl-1,2,4-oxadiazol-3-yl)benzyl]-5-(pyrrolidin-1-ylcarbonyl)-5,6,7,8-tetrahydro[1,2,4]triazolo[4,3-a]pyridin-3(2H)-on (Racemat)

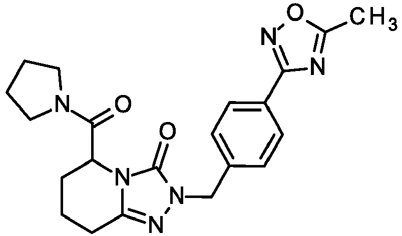

### (5RS)-5-(Pyrrolidin-1 -ylcarbonyl)-5,6,7,8-tetrahydro[1,2,4]triazolo[4,3-a]pyridin-3(2H)-on

(Racemat) (60.0 mg, 254 µmol) und Cäsiumcarbonat (124 mg, 381 µmol) wurden in 3.0 ml Acetonitril suspendiert, dann mit 5-[4-(Brommethyl)phenyl]-3-methyl-1,2,4-oxadiazol (70.7 mg, 279 µmol) versetzt und über ein Wochenende bei Raumtemperatur gerührt. Zur Aufarbeitung wurde der vorhandene Niederschlag abgesaugt und das Filtrat eingeengt. Nach Trocknung im Vakuum erfolgte chromatographische Reinigung (Instrument: Waters Prep LC/MS System, Säule: Phenomenex Kinetex C18 5µm 100x30 mm Eluent A : Wasser, Eluent B : Acetonitril, Fluß: 65 ml/min plus 5ml 2%ige Ameisensäure in Wasser, Raumtemperatur, Wellenlänge 200-400 nm, At-Säule Injektion (Komplettinjektion) Gradientenprofil: 0 bis 2 min 10% Eluent B, 2 bis 2,2 min auf 20% Eluent B, 2,2 bis 7 min auf 60% Eluent B, 7 bis 7,5 min auf 92% Eluent B, 7,5 bis 9 min bei 92% B). Die Produktfraktion wurde lyophilisiert. So wurden 57.6 mg (54 % d. Th.) der Titelverbindung erhalten.
LC-MS (Methode 3): Rₜ = 1.18 min; MS (ESIpos): m/z = 409 [M+H]⁺
¹H-NMR (400MHz, DMSO-d₆): δ [ppm]= 1.62 - 1.85 (m, 4H), 1.88 - 2.12 (m, 4H), 2.48 - 2.72 (m, 2H, teilweise durch Lösungsmittelsignal verdeckt), 2.86 (s, 3H), 3.22 - 3.41 (m, 2H, teilweise von Wassersignal überlagert), 3.47 (dt, 1H), 3.63 (dt, 1H), 4.76 (dd, 1H), 4.84 - 4.95 (m, 2H), 7.38 - 7.43 (m, 2H), 7.93 - 7.98 (m, 2H).

### Beispiel 11

### (5RS)-2-(2,3-Dihydro-1H-inden-5-ylmethyl)-5-(pyrrolidin-1-ylcarbonyl)-5,6,7,8-tetrahydro[1,2,4]triazolo[4,3-a]pyridin-3(2H)-on (Racemat)

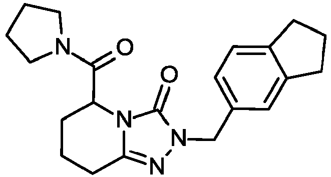

### (5RS)-5-(Pyrrolidin-1-ylcarbonyl)-5,6,7,8-tetrahydro[1,2,4]triazolo[4,3-a]pyridin-3(2H)-on

(Racemat) (75.4 mg, 316 µmol) und Cäsiumcarbonat (154 mg, 474 µmol) wurden in 3.0 ml Acetonitril suspendiert, dann mit 5-(Brommethyl)indan (100 mg, etwa 80 % Reinheit, etwa 379 µmol) versetzt, 1.5h bei Raumtemperatur gerührt und dann über ein Wochenende bei Raumtemperatur stehen gelassen. Zur weiteren Umsetzung wurden noch einmal Cäsiumcarbonat (206 mg, 632 µmol) und 5-(Brommethyl)indan (100 mg, etwa 80 % Reinheit, etwa 379 µmol) zugegeben und 4h bei Raumtemperatur gerührt. Zur Aufarbeitung wurde der vorhandene Niederschlag abgesaugt und verworfen. Das Filtrat wurde eingeengt, in Acetonitril/DMO/Wasser aufgenommen und über präparative HPLC getrennt (Säule: Kromasil C18, 250x30mm, 10µm, Eluent: Acetonitril (B) / Wasser + 0,1 % TFA (A), Gradient: 0min 90% A, 6min90% A, 27min 5%A, 38min 5%A, 38min 5%A, 39min 90% A; Fluss: 50ml/min, Detektor: 210nm). Produkthaltige Fraktionen wurden vereinigt, eingeengt und im Vakuum getrocknet. Es wurden so 77.2 mg (67 % d. Th.) der Titelverbindung erhalten.
LC-MS (Methode 4): Rₜ = 0.82 min; MS (ESIpos): m/z = 367 [M+H]⁺
¹H-NMR (400MHz, DMSO-d₆): δ [ppm]= 1.64 - 1.74 (m, 2H), 1.79 (quint; 2H) 1.88 - 2.09 (m, 6H), 2.46 - 2.64 (m, 2H, teilweise durch Lösungsmittelsignal verdeckt), 2.81 (t, 4H), 3.25 (dt, 1H), 3.36 (dt, 1 H), 3.42 - 3.68 (m, 2H, teilweise von Wassersignal überlagert), 4.67 - 4.79 (m, 3H), 6.99 (d, 1H), 7.09 (s, 1H), 7.16 (d, 1H).

### Beispiel 12

### (5RS)-2-[(5-Methyl-1,2,4-oxadiazol-3-yl)methyl]-5-(pyrrolidin-1-ylcarbonyl)-5,6,7,8-tetrahydro[1,2,4]triazolo[4,3-a]pyridin-3(2H)-on (Racemat)

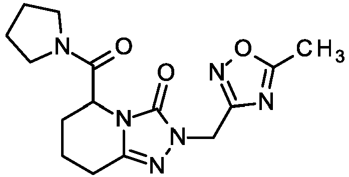

### (5RS)-5-(Pyrrolidin-1-ylcarbonyl)-5,6,7,8-tetrahydro[1,2,4]triazolo[4,3-a]pyridin-3(2H)-on

(Racemat) (30.0 mg, 127 µmol) wurde in Acetonitril (2.0 ml) vorgelegt. Cäsiumcarbonat (62.1 mg, 190 µmol) und 3-(Brommethyl)-5-methyl-1,2,4-oxadiazol (23.6 mg, 133 µmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 19.0 mg (45 % d. Th.) der Titelverbindung erhalten.
LC-MS (Methode 4): Rₜ = 0.48 min; MS (ESIpos): m/z = 333 [M+H]⁺
¹H-NMR (500 MHz, DMSO-d6) δ [ppm]: 1.701 (0.70), 1.713 (0.92), 1.717 (0.99), 1.727 (0.86), 1.776 (1.37), 1.789 (2.33), 1.803 (1.73), 1.817 (0.51), 1.899 (0.57), 1.913 (1.61), 1.926 (2.10), 1.940 (1.46), 1.952 (0.51), 1.969 (0.52), 1.979 (0.49), 2.023 (0.40), 2.040 (0.41), 2.052 (0.41), 2.518 (0.65), 2.522 (0.41), 2.568 (3.66), 2.573 (16.00), 2.590 (1.04), 2.598 (1.52), 2.866 (2.48), 3.247 (0.78), 3.256 (0.75), 3.260 (0.64), 3.270 (1.32), 3.284 (1.11), 3.310 (3.45), 3.324 (2.02), 3.338 (1.66), 3.348 (0.84), 3.352 (0.93), 3.362 (0.92), 3.376 (0.44), 3.460 (0.74), 3.466 (0.54), 3.474 (0.47), 3.480 (0.91), 3.494 (0.41), 3.599 (0.41), 3.613 (0.84), 3.619 (0.43), 3.626 (0.48), 3.633 (0.70), 4.494 (1.93), 4.720 (0.77), 4.727 (0.88), 4.732 (0.99), 4.740 (0.78), 4.857 (1.33), 4.889 (3.17), 4.928 (3.18), 4.960 (1.36).

### Beispiel 13

### (5RS)-2-[(3-Methyl-1,2-oxazol-5-yl)methyl]-5-(pyrrolidin-1-ylcarbonyl)-5,6,7,8-tetrahydro[1,2,4]triazolo[4,3-a]pyridin-3(2H)-on (Racemat)

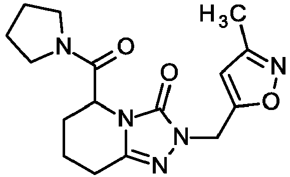

(5RS)-5-(Pyrrolidin-1-ylcarbonyl)-5,6,7,8-tetrahydro[1,2,4]triazolo[4,3-a]pyridin-3(2H)-on (Racemat) (40.0 mg, 169 µmol) wurde in Acetonitril (2.0 ml) vorgelegt. Cäsiumcarbonat (82.7 mg, 254 µmol) und 5-(Brommethyl)-3-methyl-1,2-oxazol (32.8 mg, 186 µmol) wurden anschließend zugegeben. Das Reaktionsgemisch wurde nach 2 Tagen Rühren bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 18.6 mg (33 % d. Th.) der Titelverbindung erhalten.
LC-MS (Methode 3): Rₜ = 0.89 min; MS (ESIpos): m/z = 332 [M+H]⁺
¹H-NMR (400 MHz, DMSO-d6) δ [ppm]: -0.008 (0.87), 0.008 (0.76), 1.686 (0.49), 1.709 (0.99), 1.720 (1.17), 1.732 (0.87), 1.753 (0.57), 1.772 (1.53), 1.789 (2.62), 1.806 (2.01), 1.822 (0.60), 1.892 (0.62), 1.909 (1.85), 1.925 (2.33), 1.942 (1.50), 1.959 (0.66), 1.972 (0.52), 1.983 (0.78), 1.995 (0.71), 2.004 (0.63), 2.014 (0.50), 2.019 (0.48), 2.030 (0.68), 2.039 (0.56), 2.046 (0.46), 2.055 (0.49), 2.201 (16.00), 2.215 (0.69), 2.417 (0.49), 2.523 (1.09), 2.565 (0.86), 2.580 (0.74), 2.590 (0.68), 2.602 (1.21), 2.615 (0.66), 2.643 (0.48), 3.242 (0.75), 3.254 (0.74), 3.271 (1.33), 3.288 (0.70), 3.322 (0.97), 3.339 (1.33), 3.351 (0.50), 3.357 (0.70), 3.369 (0.78), 3.454 (0.86), 3.462 (0.63), 3.471 (0.53), 3.479 (1.08), 3.496 (0.47), 3.589 (0.50), 3.606 (1.03), 3.614 (0.52), 3.623 (0.59), 3.631 (0.79), 3.703 (0.42), 4.495 (0.41), 4.510 (0.41), 4.723 (0.92), 4.732 (1.05), 4.738 (1.20), 4.747 (0.90), 4.940 (4.70), 4.982 (0.54), 6.223 (3.16).

### Beispiel 14

### (5RS)-2-[3-Fluor-4-(trifluormethyl)benzyl]-5-(pyrrolidin-1 -ylcarbonyl)-5,6,7,8-tetrahydro[1,2,4]triazolo[4,3-a]pyridin-3(2H)-on (Racemat)

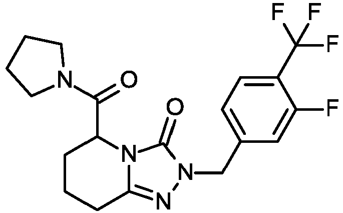

(5RS)-5-(Pyrrolidin-1-ylcarbonyl)-5,6,7,8-tetrahydro[1,2,4]triazolo[4,3-a]pyridin-3(2H)-on (Racemat) (40.0 mg, 169 µmol) wurde in Acetonitril (2.0 ml) vorgelegt. Cäsiumcarbonat (82.7 mg, 254 µmol) und 4-(Brommethyl)-2-fluor-1-(trifluormethyl)benzol (47.9 mg, 186 µmol) wurden anschließend zugegeben. Das Reaktionsgemisch wurde nach Rühren für 48 h bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Es wurden 48.9 mg (70 % d. Th.) der Titelverbindung erhalten.
LC-MS (Methode 3): Rₜ = 1.55 min; MS (ESIpos): m/z = 413 [M+H]⁺
¹H-NMR (400 MHz, DMSO-d6) δ [ppm]: -0.008 (3.00), 0.008 (1.79), 1.235 (0.49), 1.681 (1.63), 1.695 (1.81), 1.706 (1.87), 1.719 (2.35), 1.731 (2.76), 1.743 (2.21), 1.756 (2.58), 1.774 (5.53), 1.792 (8.57), 1.809 (6.54), 1.819 (2.30), 1.826 (3.06), 1.837 (3.80), 1.845 (1.54), 1.854 (1.49), 1.893 (2.04), 1.910 (5.29), 1.926 (6.37), 1.943 (3.93), 1.960 (1.38), 1.975 (1.13), 1.987 (1.15), 2.000 (1.81), 2.020 (3.43), 2.045 (1.84), 2.056 (1.54), 2.063 (1.50), 2.071 (1.41), 2.080 (0.76), 2.091 (0.52), 2.327 (0.60), 2.519 (4.26), 2.563 (2.70), 2.573 (2.53), 2.588 (2.17), 2.602 (2.17), 2.614 (3.60), 2.626 (2.07), 2.644 (1.03), 2.656 (1.47), 2.669 (1.30), 2.710 (0.51), 3.097 (1.58), 3.233 (1.25), 3.250 (2.51), 3.262 (2.74), 3.279 (4.78), 3.297 (3.97), 3.328 (2.43), 3.346 (4.21), 3.358 (1.68), 3.364 (2.26), 3.376 (2.38), 3.393 (1.08), 3.443 (1.37), 3.460 (2.81), 3.468 (2.17), 3.478 (1.79), 3.485 (3.40), 3.502 (1.52), 3.593 (1.77), 3.610 (3.23), 3.618 (1.80), 3.627 (1.95), 3.635 (2.43), 3.652 (1.15), 3.711 (0.75), 4.755 (3.09), 4.764 (3.43), 4.769 (4.01), 4.779 (2.85), 4.948 (16.00), 4.985 (0.48), 7.248 (3.78), 7.268 (4.07), 7.296 (3.67), 7.326 (3.62), 7.755 (2.93), 7.775 (5.28), 7.794 (2.72).

### Beispiel 15

### (5RS)-2-[(2-Methylpyridin-4-yl)methyl]-5-(pyrrolidin-1-ylcarbonyl)-5,6,7,8-tetrahydro[1,2,4]triazolo[4,3-a]pyridin-3(2H)-on (Racemat)

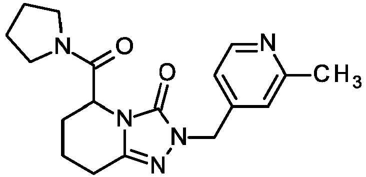

(5RS)-5-(Pyrrolidin-1-ylcarbonyl)-5,6,7,8-tetrahydro[1,2,4]triazolo[4,3-a]pyridin-3(2H)-on (40.0 mg, 169 µmol) wurde in Acetonitril (2.0 ml) vorgelegt. Cäsiumcarbonat (82.7 mg, 254 µmol) und 4-(Chlormethyl)-2-methylpyridinhydrochlorid (33.2 mg, 186 µmol) wurden anschließend zugegeben. Das Reaktionsgemisch wurde über Nacht bei Raumtemperatur und weiter 24 h bei 85 °C gerührt. Nach Abkühlen auf Raumtemperatur wurde das Reaktionsgemisch mit Wasser und Ethylacetat versetzt. Die organische Phase wurde abgetrennt und die wässrige Phase dreimal mit Ethylacetat extrahiert. Die wässrige Phasen wurde mit Natriumhydrogencarbonat auf pH 7 eingestellt. Das Lösungsmittel wurde im Vakuum entfernt und der Rückstand in Ethanol suspendiert. Das Gemisch wurde filtriert und das Filtrat eingeengt. Es wurden 51.6 mg (94 % Reinheit, 84 % d. Th.) der Titelverbindung erhalten.
LC-MS (Methode 3): Rₜ = 0.51 min; MS (ESIpos): m/z = 342 [M+H]⁺
¹H-NMR (400 MHz, DMSO-d6) δ [ppm]: -0.149 (1.03), -0.008 (8.61), 0.008 (8.84), 0.146 (0.99), 1.038 (4.77), 1.055 (9.73), 1.072 (4.87), 1.355 (0.51), 1.679 (0.92), 1.692 (1.03), 1.704 (0.90), 1.763 (1.74), 1.780 (3.17), 1.797 (5.03), 1.814 (3.79), 1.833 (1.56), 1.912 (1.91), 1.920 (2.09), 1.927 (2.48), 1.935 (2.30), 1.945 (1.86), 1.952 (1.54), 2.055 (2.57), 2.328 (1.26), 2.366 (0.94), 2.523 (4.09), 2.568 (1.54), 2.583 (1.35), 2.594 (1.42), 2.609 (1.19), 2.618 (1.26), 2.629 (1.81), 2.642 (1.29), 2.673 (16.00), 2.698 (2.66), 2.710 (1.22), 3.087 (0.44), 3.247 (0.78), 3.264 (1.52), 3.277 (1.38), 3.293 (2.27), 3.310 (1.08), 3.335 (1.19), 3.352 (2.36), 3.370 (1.38), 3.382 (1.33), 3.399 (0.76), 3.413 (1.97), 3.431 (5.39), 3.449 (5.39), 3.459 (1.01), 3.466 (2.18), 3.476 (1.77), 3.484 (1.45), 3.501 (2.20), 3.518 (1.19), 3.593 (1.65), 3.610 (2.43), 3.626 (1.86), 3.634 (2.11), 3.652 (1.56), 3.725 (1.54), 4.020 (1.93), 4.753 (0.46), 4.791 (1.58), 4.805 (2.59), 4.815 (1.52), 4.930 (0.85), 5.057 (0.48), 5.101 (4.04), 5.112 (3.93), 5.157 (0.48), 7.543 (1.58), 7.555 (1.61), 7.575 (2.75), 7.833 (0.57), 8.664 (0.57), 8.679 (0.73), 8.703 (2.85), 8.717 (2.53).

### Beispiel 16

### (5RS)-2-(3-Fluorbenzyl)-5-(pyrrolidin-1-ylcarbonyl)-5,6,7,8-tetrahydro[1,2,4]triazolo[4,3-a]pyridin-3(2H)-on (Racemat)

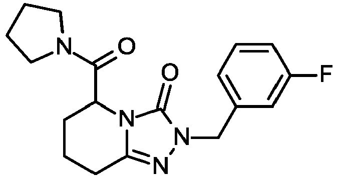

### (5RS)-5-(Pyrrolidin-1 -ylcarbonyl)-5,6,7,8-tetrahydro[1,2,4]triazolo[4,3-a]pyridin-3(2H)-on

(Racemat) (50.0 mg, 212 µmol) wurde in Acetonitril (3.0 ml) vorgelegt. Cäsiumcarbonat (103 mg, 317 µmol) und 1-(Brommethyl)-3-fluorbenzol (42.0 mg, 222 µmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 48.0 mg (66 % d. Th.) der Titelverbindung erhalten.
LC-MS (Methode 4): Rₜ = 0.68 min; MS (ESIpos): m/z = 345 [M+H]⁺
¹H-NMR (400 MHz, DMSO-d6) δ [ppm]: -0.149 (0.74), -0.008 (6.59), 0.008 (5.85), 0.146 (0.74), 1.673 (0.74), 1.685 (1.06), 1.699 (1.41), 1.709 (1.99), 1.722 (2.31), 1.733 (1.90), 1.746 (0.96), 1.755 (1.35), 1.773 (3.66), 1.791 (6.30), 1.808 (4.79), 1.824 (1.38), 1.893 (1.48), 1.910 (4.24), 1.926 (5.17), 1.943 (3.15), 1.960 (1.38), 1.983 (1.03), 1.994 (1.80), 2.005 (1.73), 2.021 (1.22), 2.036 (1.48), 2.047 (1.06), 2.053 (1.03), 2.062 (1.06), 2.072 (3.92), 2.088 (0.42), 2.322 (0.58), 2.327 (0.84), 2.332 (0.61), 2.366 (0.64), 2.518 (3.63), 2.522 (3.05), 2.564 (2.02), 2.579 (1.61), 2.593 (1.51), 2.605 (2.80), 2.617 (1.54), 2.636 (0.71), 2.647 (1.09), 2.660 (0.84), 2.665 (0.80), 2.669 (0.93), 2.674 (0.71), 2.709 (0.74), 3.230 (0.90), 3.247 (1.83), 3.259 (1.90), 3.276 (3.31), 3.294 (2.38), 3.328 (2.60), 3.346 (3.44), 3.357 (1.29), 3.364 (1.73), 3.375 (1.96), 3.393 (0.93), 3.439 (1.00), 3.457 (2.12), 3.464 (1.54), 3.474 (1.22), 3.482 (2.70), 3.499 (1.19), 3.595 (1.22), 3.611 (2.41), 3.619 (1.19), 3.628 (1.41), 3.636 (1.86), 3.653 (0.84), 4.740 (2.22), 4.750 (2.47), 4.756 (2.89), 4.765 (2.18), 4.836 (16.00), 7.005 (1.77), 7.031 (1.90), 7.036 (1.70), 7.057 (2.86), 7.076 (3.37), 7.087 (0.96), 7.103 (1.86), 7.110 (1.67), 7.124 (1.09), 7.131 (1.03), 7.356 (1.83), 7.372 (2.09), 7.376 (2.70), 7.391 (2.67), 7.396 (1.67), 7.411 (1.41).

### Beispiel 17

### (5RS)-2-(3,4-Difluorbenzyl)-5-(pyrrolidin-1-ylcarbonyl)-5,6,7,8-tetrahydro[1,2,4]triazolo[4,3-a]pyridin-3(2H)-on (Racemat)

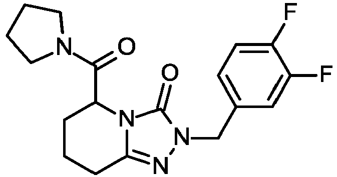

(5RS)-5-(Pyrrolidin-1 -ylcarbonyl)-5,6,7,8-tetrahydro[1,2,4]triazolo[4,3-a]pyridin-3(2H)-on (Racemat) (50.0 mg, 212 µmol) wurde in Acetonitril (3.0 ml) vorgelegt. Cäsiumcarbonat (103 mg, 317 µmol) und 4-(Brommethyl)-1,2-difluorbenzol (46.0 mg, 222 µmol) wurden anschließend zugegeben. Das Reaktionsgemisch wurde nach Rühren über Nacht bei Raumtemperatur mit Wasser, wässriger 1N Salzsäure-Lösung und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 51.4 mg (67 % d. Th.) der Titelverbindung erhalten.
LC-MS (Methode 3): Rₜ = 1.29 min; MS (ESIpos): m/z = 363 [M+H]⁺
¹H-NMR (400 MHz, DMSO-d6) δ [ppm]: -0.008 (1.10), 0.008 (0.79), 1.659 (0.87), 1.666 (0.94), 1.678 (1.26), 1.692 (1.49), 1.720 (2.53), 1.730 (1.97), 1.744 (1.22), 1.755 (1.63), 1.773 (4.14), 1.791 (6.91), 1.808 (5.24), 1.824 (1.51), 1.893 (1.74), 1.909 (4.73), 1.926 (5.77), 1.942 (3.55), 1.960 (1.63), 1.983 (1.35), 1.994 (2.27), 2.007 (2.54), 2.016 (1.71), 2.034 (1.69), 2.044 (1.27), 2.051 (1.22), 2.060 (1.18), 2.069 (0.65), 2.079 (0.40), 2.519 (2.58), 2.561 (2.09), 2.576 (1.78), 2.591 (1.72), 2.603 (3.08), 2.615 (1.71), 2.633 (0.78), 2.645 (1.17), 2.657 (0.59), 3.229 (1.01), 3.246 (2.10), 3.258 (2.34), 3.275 (4.13), 3.293 (3.31), 3.325 (2.59), 3.343 (3.64), 3.355 (1.42), 3.361 (1.92), 3.373 (2.02), 3.391 (0.93), 3.438 (1.10), 3.456 (2.33), 3.463 (1.76), 3.473 (1.45), 3.480 (2.91), 3.497 (1.27), 3.592 (1.40), 3.609 (2.74), 3.617 (1.46), 3.625 (1.61), 3.633 (2.09), 3.650 (0.96), 4.737 (2.62), 4.746 (2.87), 4.752 (3.26), 4.761 (2.48), 4.816 (16.00), 7.061 (1.51), 7.067 (1.55), 7.072 (1.61), 7.077 (1.78), 7.083 (1.77), 7.087 (1.69), 7.093 (1.61), 7.230 (1.48), 7.235 (1.43), 7.249 (1.57), 7.255 (1.72), 7.258 (1.78), 7.264 (1.49), 7.278 (1.47), 7.283 (1.38), 7.373 (1.70), 7.394 (3.23), 7.400 (1.93), 7.415 (1.77), 7.421 (3.17), 7.442 (1.48).

### Beispiel 18

### (5RS)-2-(3-Chlor-4-fluorbenzyl)-5-(pyrrolidin-1-ylcarbonyl)-5,6,7,8-tetrahydro[1,2,4]triazolo[4,3-a]pyridin-3(2H)-on (Racemat)

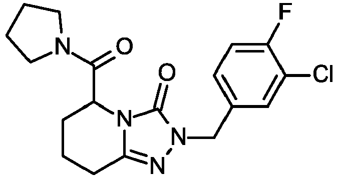

(5RS)-5-(Pyrrolidin-1 -ylcarbonyl)-5,6,7,8-tetrahydro[1,2,4]triazolo[4,3-a]pyridin-3(2H)-on (Racemat) (50.0 mg, 212 µmol) wurde in Acetonitril (3.0 ml) vorgelegt. Cäsiumcarbonat (103 mg, 317 µmol) und 4-(Brommethyl)-2-chlor-1-fluorbenzol (49.7 mg, 222 µmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 57.0 mg (70 % d. Th.) der Titelverbindung erhalten.
LC-MS (Methode 3): Rₜ = 1.43 min; MS (ESIpos): m/z = 379 [M+H]⁺
¹H-NMR (400 MHz, DMSO-d6) δ [ppm]: -0.008 (1.11), 1.666 (0.83), 1.679 (1.17), 1.692 (1.58), 1.703 (2.12), 1.717 (2.65), 1.727 (2.06), 1.755 (1.33), 1.773 (4.00), 1.790 (6.78), 1.807 (5.21), 1.824 (1.52), 1.892 (1.58), 1.909 (4.70), 1.926 (5.86), 1.942 (3.78), 1.960 (1.52), 1.978 (1.20), 1.989 (2.08), 2.001 (2.02), 2.016 (1.38), 2.031 (1.61), 2.042 (1.22), 2.049 (1.16), 2.057 (1.13), 2.067 (0.64), 2.084 (0.41), 2.328 (0.44), 2.561 (2.11), 2.576 (1.68), 2.590 (1.62), 2.602 (3.04), 2.614 (1.70), 2.632 (0.75), 2.644 (1.17), 2.656 (0.60), 2.670 (0.54), 2.710 (0.50), 3.226 (0.91), 3.243 (1.93), 3.256 (1.96), 3.273 (3.44), 3.290 (2.06), 3.326 (2.63), 3.344 (3.57), 3.356 (1.39), 3.362 (1.83), 3.374 (2.03), 3.391 (0.92), 3.436 (1.07), 3.453 (2.27), 3.461 (1.71), 3.471 (1.36), 3.478 (2.85), 3.495 (1.24), 3.591 (1.30), 3.608 (2.66), 3.616 (1.36), 3.625 (1.55), 3.633 (2.06), 3.649 (0.94), 4.737 (2.43), 4.746 (2.77), 4.752 (3.15), 4.761 (2.37), 4.818 (16.00), 7.219 (1.38), 7.225 (1.49), 7.231 (1.52), 7.241 (2.09), 7.246 (2.05), 7.252 (1.89), 7.258 (1.86), 7.371 (3.67), 7.394 (4.98), 7.415 (2.94), 7.430 (2.84), 7.435 (2.61), 7.448 (2.75), 7.453 (2.63).

### Beispiel 19

### (5RS)-5-(Pyrrolidin-1-ylcarbonyl)-2-[3-(trifluormethyl)benzyl]-5,6,7,8-tetrahydro[1,2,4]triazolo[4,3-a]pyridin-3(2H)-on (Racemat)

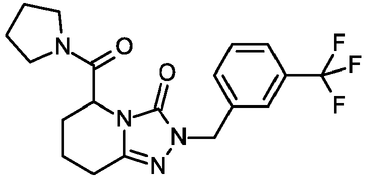

### (5RS)-5-(Pyrrolidin-1 -ylcarbonyl)-5,6,7,8-tetrahydro[1,2,4]triazolo[4,3-a]pyridin-3(2H)-on

(Racemat) (50.0 mg, 212 µmol) wurde in Acetonitril (3.0 ml) vorgelegt. Cäsiumcarbonat (103 mg, 317 µmol) und 1-(Brommethyl)-3-(trifluormethyl)benzol (53.1 mg, 222 µmol) wurden anschließend zugegeben. Das Reaktionsgemisch wurde nach Rühren über Nacht bei Raumtemperatur mit Wasser, wässriger 1 N Salzsäure-Lösung und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 60.6 mg (72 % d. Th.) der Titelverbindung erhalten.
LC-MS (Methode 3): Rₜ = 1.50 min; MS (ESIpos): m/z = 395 [M+H]⁺
¹H-NMR (600 MHz, DMSO-d6) δ [ppm]: 1.664 (0.98), 1.668 (0.95), 1.678 (1.17), 1.682 (1.23), 1.691 (0.96), 1.722 (1.76), 1.728 (1.61), 1.737 (1.14), 1.744 (0.88), 1.768 (1.18), 1.779 (4.61), 1.791 (7.62), 1.802 (5.64), 1.813 (1.54), 1.903 (1.45), 1.914 (4.15), 1.925 (5.43), 1.936 (3.45), 1.947 (0.98), 1.970 (0.90), 1.987 (1.15), 1.993 (1.84), 2.002 (1.65), 2.019 (0.96), 2.024 (1.13), 2.029 (1.12), 2.035 (1.30), 2.043 (1.30), 2.048 (1.39), 2.053 (1.22), 2.058 (0.65), 2.067 (0.45), 2.071 (0.45), 2.520 (1.58), 2.566 (1.45), 2.601 (1.51), 2.608 (2.81), 2.616 (1.71), 2.629 (0.89), 2.636 (1.43), 2.645 (0.69), 3.239 (1.12), 3.251 (2.30), 3.259 (1.97), 3.270 (3.23), 3.282 (1.50), 3.346 (3.70), 3.354 (1.55), 3.358 (1.83), 3.365 (2.29), 3.377 (1.04), 3.447 (1.17), 3.459 (2.47), 3.464 (1.70), 3.471 (1.50), 3.476 (2.81), 3.487 (1.25), 3.604 (1.37), 3.615 (2.62), 3.620 (1.48), 3.626 (1.60), 3.631 (2.16), 3.642 (1.05), 4.755 (2.69), 4.761 (2.92), 4.765 (3.15), 4.771 (2.50), 4.926 (16.00), 7.508 (2.83), 7.521 (3.83), 7.579 (2.39), 7.591 (4.65), 7.604 (2.52), 7.624 (5.77), 7.653 (3.72), 7.665 (2.53).

### Beispiel 20

### (5RS)-2-(3-Methoxybenzyl)-5-(pyrrolidin-1-ylcarbonyl)-5,6,7,8-tetrahydro[1,2,4]triazolo[4,3-a]pyridin-3(2H)-on (Racemat)

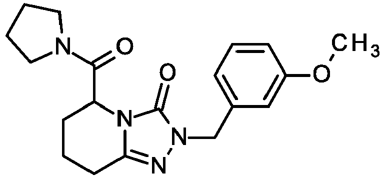

### (5RS)-5-(Pyrrolidin-1 -ylcarbonyl)-5,6,7,8-tetrahydro[1,2,4]triazolo[4,3-a]pyridin-3(2H)-on

(Racemat) (50.0 mg, 212 µmol) wurde in Acetonitril (2.0 ml) vorgelegt. Cäsiumcarbonat (72.4 mg, 222 µmol) und 1-(Brommethyl)-3-methoxybenzol (44.7 mg, 222 µmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 24.0 mg (32 % d. Th.) der Titelverbindung erhalten.
LC-MS (Methode 3): Rₜ = 1.21 min; MS (ESIpos): m/z = 357 [M+H]⁺
¹H-NMR (400 MHz, DMSO-d6) δ [ppm]: 0.008 (0.70), 1.685 (0.46), 1.717 (0.93), 1.754 (0.42), 1.772 (1.40), 1.789 (2.40), 1.806 (1.85), 1.823 (0.54), 1.893 (0.57), 1.909 (1.70), 1.926 (2.17), 1.942 (1.40), 1.960 (0.57), 1.975 (0.43), 1.987 (0.75), 1.998 (0.72), 2.015 (0.49), 2.030 (0.61), 2.040 (0.43), 2.055 (0.41), 2.568 (0.61), 2.583 (0.58), 2.594 (1.13), 2.606 (0.61), 2.635 (0.43), 3.243 (0.68), 3.256 (0.69), 3.273 (1.24), 3.290 (0.66), 3.339 (1.29), 3.351 (0.48), 3.356 (0.66), 3.368 (0.71), 3.455 (0.80), 3.463 (0.58), 3.473 (0.48), 3.480 (1.01), 3.497 (0.45), 3.595 (0.46), 3.611 (0.97), 3.620 (0.49), 3.628 (0.56), 3.637 (0.76), 3.730 (16.00), 4.732 (0.91), 4.742 (1.03), 4.747 (1.21), 4.757 (1.02), 4.770 (6.44), 6.774 (3.04), 6.777 (2.99), 6.793 (1.49), 6.820 (0.90), 6.824 (0.92), 6.842 (1.08), 6.847 (0.91), 7.218 (1.07), 7.238 (2.10), 7.258 (1.08).

### Beispiel 21

### (5RS)-2-[(1-Methyl-1H-pyrazol-3-yl)methyl]-5-(pyrrolidin-1-ylcarbonyl)-5,6,7,8-tetrahydro[1,2,4]triazolo[4,3-a]pyridin-3(2H)-on (Racemat)

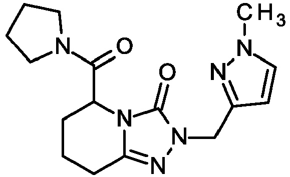

### (5RS)-5-(Pyrrolidin-1 -ylcarbonyl)-5,6,7,8-tetrahydro[1,2,4]triazolo[4,3-a]pyridin-3(2H)-on

(Racemat) (50.0 mg, 212 µmol) wurde in Acetonitril (2.0 ml) vorgelegt. Cäsiumcarbonat (72.4 mg, 222 µmol) und 3-(Brommethyl)-1-methyl-1H-pyrazol (38.9 mg, 222 µmol) wurden anschließend zugegeben. Das Reaktionsgemisch wurde über Nacht bei Raumtemperatur und 1 h bei 90°C gerührt. Nach Abkühlen auf Raumtemperatur wurde das Reaktionsgemisch mit Wasser, 1 N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und erneut über HPLC (Methode 11) getrennt. Die produkthaltigen Fraktionen wurden im Vakuum eingeengt und es wurden 8.00 mg (11 % d. Th.) der Titelverbindung erhalten.
LC-MS (Methode 4): Rₜ = 0.48 min; MS (ESIpos): m/z = 331 [M+H]⁺
¹H-NMR (400 MHz, DMSO-d6) δ [ppm]: -0.008 (3.77), 0.008 (2.98), 1.705 (1.52), 1.717 (1.23), 1.751 (0.50), 1.770 (1.66), 1.787 (2.86), 1.804 (2.19), 1.821 (0.61), 1.891 (0.73), 1.908 (2.10), 1.924 (2.77), 1.941 (1.93), 1.965 (0.96), 2.000 (0.64), 2.011 (0.70), 2.021 (0.58), 2.036 (0.55), 2.327 (1.20), 2.366 (0.96), 2.523 (4.88), 2.574 (1.52), 2.586 (0.82), 2.616 (0.53), 2.669 (1.26), 2.709 (0.85), 3.220 (0.47), 3.236 (0.91), 3.249 (0.93), 3.266 (1.66), 3.334 (2.22), 3.351 (0.99), 3.363 (0.93), 3.380 (0.44), 3.434 (0.47), 3.451 (0.96), 3.458 (0.73), 3.476 (1.23), 3.493 (0.55), 3.590 (0.58), 3.607 (1.17), 3.624 (0.64), 3.633 (0.88), 3.649 (0.41), 3.774 (16.00), 3.796 (0.53), 4.646 (0.58), 4.685 (4.26), 4.696 (4.67), 4.706 (1.49), 4.715 (1.08), 4.735 (0.64), 6.034 (2.42), 6.039 (2.48), 7.569 (2.34), 7.574 (2.34).

### Beispiel 22

### (5RS)-2-(Pyridin-2-ylmethyl)-5-(pyrrolidin-1-ylcarbonyl)-5,6,7,8-tetrahydro[1,2,4]triazolo[4,3-a]pyridin-3(2H)-on (Racemat)

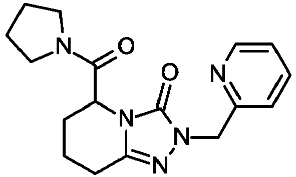

(5RS)-5-(Pyrrolidin-1-ylcarbonyl)-5,6,7,8-tetrahydro[1,2,4]triazolo[4,3-a]pyridin-3(2H)-on (50.0 mg, 212 µmol) wurde in Acetonitril (2.0 ml) vorgelegt. Cäsiumcarbonat (72.4 mg, 222 µmol) und 2-(Brommethyl)pyridin (38.2 mg, 222 µmol) wurden anschließend zugegeben. Das Reaktionsgemisch wurde nach Rühren über Nacht bei Raumtemperatur mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und erneut über präparative HPLC aufgereinigt (Chromatorex C18, 10µm, 125mm x 30mm; Laufmittel: Acetonitril/Wasser-Gradient) und es wurden 3.00 mg (4 % d. Th.) der Titelverbindung erhalten.
LC-MS (Methode 4): Rₜ = 0.43 min; MS (ESIpos): m/z = 328 [M+H]⁺
¹H-NMR (400 MHz, DMSO-d6) δ [ppm]: -0.149 (0.93), -0.008 (6.95), 0.008 (6.72), 0.146 (0.82), 1.733 (2.39), 1.758 (1.81), 1.775 (4.32), 1.792 (7.12), 1.809 (5.49), 1.825 (1.64), 1.895 (1.58), 1.912 (4.55), 1.928 (5.61), 1.945 (3.45), 1.961 (1.17), 2.019 (2.22), 2.049 (1.69), 2.073 (1.23), 2.327 (2.57), 2.332 (1.87), 2.366 (1.69), 2.523 (8.76), 2.558 (3.09), 2.568 (2.28), 2.583 (1.87), 2.595 (1.69), 2.606 (3.04), 2.620 (1.75), 2.637 (0.88), 2.648 (1.17), 2.665 (2.16), 2.669 (2.80), 2.674 (2.04), 2.709 (1.75), 3.235 (1.11), 3.252 (2.10), 3.265 (2.22), 3.281 (4.09), 3.347 (3.97), 3.365 (1.87), 3.377 (1.99), 3.395 (0.93), 3.449 (1.17), 3.467 (2.28), 3.473 (1.75), 3.491 (2.92), 3.508 (1.46), 3.600 (1.34), 3.617 (2.69), 3.634 (1.64), 3.641 (2.04), 3.659 (0.99), 4.750 (2.34), 4.760 (2.69), 4.765 (3.15), 4.774 (2.63), 4.895 (16.00), 5.114 (0.53), 5.171 (0.53), 7.121 (3.74), 7.140 (4.09), 7.278 (1.81), 7.297 (2.16), 7.311 (2.34), 7.332 (0.58), 7.748 (2.16), 7.753 (2.22), 7.767 (3.85), 7.772 (3.74), 7.787 (1.99), 7.791 (1.93), 8.506 (2.51), 8.520 (2.57).

### Beispiel 23

### (5RS)-5-(Pyrrolidin-1-ylcarbonyl)-2-{[5-(trifluormethyl)pyridin-2-yl]methyl}-5,6,7,8-tetrahydro[1,2,4]triazolo[4,3-a]pyridin-3(2H)-on (Racemat)

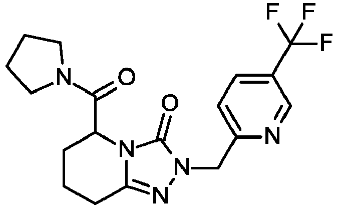

### (5RS)-5-(Pyrrolidin-1-ylcarbonyl)-5,6,7,8-tetrahydro[1,2,4]triazolo[4,3-a]pyridin-3(2H)-on

(Racemat) (40.0 mg, 169 µmol) wurde in Acetonitril (2.0 ml) vorgelegt. Cäsiumcarbonat (82.7 mg, 254 µmol) und 2-(Brommethyl)-5-(trifluormethyl)pyridin (44.7 mg, 186 µmol) wurden anschließend zugegeben. Das Reaktionsgemisch wurde nach Rühren über Nacht bei Raumtemperatur mit Wasser, 1N wässriger Salzsäure und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 44.6 mg (67 % d. Th.) der Titelverbindung erhalten.
LC-MS (Methode 3): Rₜ = 1.22 min; MS (ESIpos): m/z = 396 [M+H]⁺
¹H-NMR (400 MHz, DMSO-d6) δ [ppm]: -0.149 (0.72), 0.147 (0.71), 1.726 (2.72), 1.738 (3.10), 1.775 (4.76), 1.792 (7.48), 1.809 (5.64), 1.826 (1.77), 1.894 (1.55), 1.911 (4.76), 1.928 (5.91), 1.945 (3.79), 1.962 (1.18), 2.025 (2.84), 2.053 (1.90), 2.328 (0.88), 2.366 (0.64), 2.564 (2.98), 2.574 (2.44), 2.589 (2.03), 2.601 (2.01), 2.613 (3.47), 2.625 (1.98), 2.655 (1.38), 2.669 (1.43), 2.710 (0.77), 3.236 (0.95), 3.253 (1.97), 3.265 (2.17), 3.282 (3.78), 3.329 (2.27), 3.347 (3.67), 3.364 (2.07), 3.377 (2.12), 3.395 (1.00), 3.450 (1.06), 3.467 (2.41), 3.492 (3.06), 3.509 (1.38), 3.599 (1.44), 3.615 (2.87), 3.632 (1.84), 3.640 (2.24), 3.657 (1.00), 4.760 (2.66), 4.775 (3.67), 4.784 (2.76), 5.026 (16.00), 7.383 (4.73), 7.403 (4.96), 8.212 (3.24), 8.233 (3.24), 8.929 (5.73).

### Beispiel 24

### (5RS)-2-[(5-Methyl-1H-imidazol-4-yl)methyl]-5-(pyrrolidin-1-ylcarbonyl)-5,6,7,8-tetrahydro[1,2,4]triazolo[4,3-a]pyridin-3(2H)-on (Racemat)

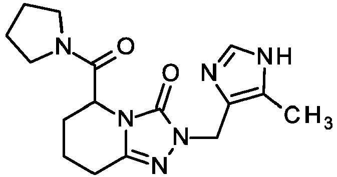

### (5RS)-5-(Pyrrolidin-1-ylcarbonyl)-5,6,7,8-tetrahydro[1,2,4]triazolo[4,3-a]pyridin-3(2H)-on

(Racemat) (30.0 mg, 127 µmol) wurde in Acetonitril (2.0 ml) vorgelegt. Cäsiumcarbonat (165 mg, 508 µmol) und 4-(Chlormethyl)-5-methyl-1H-imidazolhydrochlorid (25.5 mg, 152 µmol) wurden anschließend zugegeben. Das Reaktionsgemisch wurde nach Rühren über Nacht unter Rückfluss im Vakuum eingeengt. Der Rückstand wurde über präparative HPLC (Methode 12) aufgereinigt. Die produkthaltigen Fraktionen wurden im Vakuum eingeengt und es wurden 1.80 mg (4 % d. Th.) der Titelverbindung erhalten.
LC-MS (Methode 3): Rₜ = 0.46 min; MS (ESIpos): m/z = 331 [M+H]⁺
¹H-NMR (600 MHz, METHANOL-d4) δ [ppm]: 1.814 (0.43), 1.823 (0.63), 1.828 (0.65), 1.833 (0.66), 1.839 (0.65), 1.849 (0.45), 1.856 (0.44), 1.864 (0.53), 1.867 (0.51), 1.872 (0.54), 1.880 (0.52), 1.889 (0.64), 1.895 (0.58), 1.902 (1.23), 1.906 (1.24), 1.913 (1.77), 1.917 (1.61), 1.925 (1.40), 1.928 (1.26), 1.939 (0.67), 2.019 (0.61), 2.029 (1.55), 2.040 (2.38), 2.052 (2.28), 2.063 (1.50), 2.072 (0.63), 2.137 (0.43), 2.143 (0.44), 2.148 (0.46), 2.154 (0.67), 2.161 (0.70), 2.166 (0.75), 2.171 (0.63), 2.178 (0.46), 2.189 (0.40), 2.195 (0.45), 2.217 (0.59), 2.228 (16.00), 2.575 (0.42), 2.584 (0.47), 2.590 (0.45), 2.603 (0.80), 2.613 (0.74), 2.619 (0.77), 2.628 (0.61), 2.681 (0.63), 2.690 (1.26), 2.699 (0.63), 2.709 (0.40), 2.718 (0.74), 3.363 (0.46), 3.374 (0.82), 3.383 (0.74), 3.394 (1.07), 3.406 (0.57), 3.504 (0.62), 3.516 (1.20), 3.524 (0.65), 3.528 (0.81), 3.531 (0.72), 3.536 (1.03), 3.543 (1.03), 3.548 (1.12), 3.554 (0.65), 3.560 (1.06), 3.571 (0.53), 3.756 (0.54), 3.767 (0.99), 3.772 (0.62), 3.778 (0.65), 3.784 (0.84), 3.795 (0.44), 4.630 (0.69), 4.770 (1.70), 4.785 (1.45), 4.796 (4.48), 4.802 (1.53), 4.862 (2.68), 5.491 (5.22), 7.477 (4.91).

### Beispiel 25

### (5RS)-2-(4-Methylbenzyl)-5-(pyrrolidin-1-ylcarbonyl)-5,6,7,8-tetrahydro[1,2,4]triazolo[4,3-a]pyridin-3(2H)-on (Racemat)

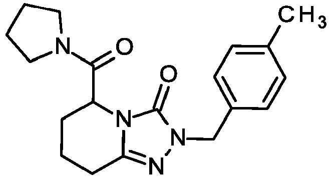

(5RS)-2-(4-Methylbenzyl)-3-oxo-2,3,5,6,7,8-hexahydro[1,2,4]triazolo[4,3-a]pyridin-5-carbonsäure (Racemat) (436 mg, 1.52 mmol) wurde in 10 ml THF gelöst. Man gab Triethylamin (420 µl, 3.0 mmol), HATU (865 mg, 2.28 mmol) und Pyrrolidin (150 µl, 1.8 mmol) zu und rührte 2h unter Argon bei Raumtemperatur. Die Reaktionsmischung wurde im Vakuum eingeengt, der Rückstand in DMSO/Acetonitril/Wasser gelöst und in 2 Chargen über präparative HPLC getrennt (Säule: Kromasil C18, 250x30mm, 10µm, Eluent: Acetonitril (B) / Wasser + 0,1 % TFA (A), Gradient: 0min 90% A, 6min90% A, 27min 5%A, 38min 5%A, 38min 5%A, 39min 90% A; Fluss: 50ml/min, Detektor: 210nm). Die vereinigten produkthaltigen Fraktionen wurden wieder eingeengt und im Vakuum getrocknet. Es wurden so 430 mg (83 % d. Th.) der Titelverbindung erhalten.
LC-MS (Methode 4): Rₜ = 0.73 min; MS (ESIpos): m/z = 341 [M+H]⁺
¹H-NMR (400MHz, DMSO-d₆): δ [ppm]= 1.61 - 1.83 (m, 4H), 1.86 - 2.10 (m, 4H), 2.27 (s, 3H), 2.42 - 2.65 (m, 2H, teilweise durch Lösungsmittelsignal verdeckt), 3.20 - 3.30 (m, 1H), 3.30 - 3.41 (m, 1H), 3.47 (dt, 1H), 3.62 (dt, 1H), 4.68 - 4.79 (m, 3H), 7.07 - 7.16 (m, 4H).

### Beispiel 26

### (5RS)-2-(4-Methylbenzyl)-5-(pyrrolidin-1-ylcarbonyl)-5,6,7,8-tetrahydro[1,2,4]triazolo[4,3-a]pyridin-3(2H)-on (Enantiomer 2)

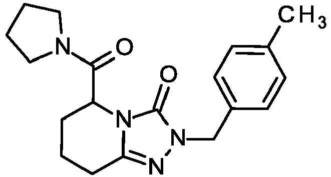

(5RS)-2-(4-Methylbenzyl)-5-(pyrrolidin-1 -ylcarbonyl)-5,6,7,8-tetrahydro[1,2,4]triazolo[4,3-a]pyridin-3(2H)-on (Racemat) wurde durch chirale präparative SFC getrennt [Probenvorbereitung: 1220 mg gelöst in 50 ml Methanol/Acetonitril; Injektionsvolumen: 2.0 ml; Säule: Daicel Chiralcel^{®} OD-H 5µm, 250 x 50 mm; Laufmittel: CO2/Ethanol: isokratisch 20% Ethanol, Fluß: 80 ml/min; Temperatur 40°C; UV Detektion: 210 nm]. Nach der Trennung wurden 494 mg von Enantiomer 1, welches zuerst eluierte, und 487 mg von Enantiomer 2, welches später eluierte, isoliert.

### Enantiomer 2:

spez. Drehwert: -25,70 (589nm, 0,4150g / 100cm³ MeOH)
Analytische chirale HPLC: Rₜ = 4.27 min, e.e. = 100% [Säule: Daicel Chiralcel^{®} ID-3 3µm 50 x 4.6 mm; Laufmittel: Isohexan/Isopropanol 1:1 ; Fluß: 1 ml/min; UV Detektion: 220 nm]
LC-MS (Methode 3): Rₜ = 1.33 min; MS (ESIpos): m/z = 341 [M+H]⁺
1H-NMR (400MHz, DMSO-d6): δ [ppm]= 1.61 - 1.83 (m, 4H), 1.87 - 2.10 (m, 4H), 2.27 (s, 3H), 2.47 - 2.70 (m, 2H, teilweise durch Lösungsmittelsignal verdeckt), 3.21 - 3.30 (m, 1H), 3.30 - 3.67 (m, 3H, teilweise von Wassersignal überlagert), 4.69 - 4.81 (m, 3H), 7.08 - 7.16 (m, 4H).

Die (5S)-Konfiguration wurde aufgrund von Kristallstrukturaufklärung für Beispiel 26 zugordnet.

### (5S)-2-(4-Methylbenzyl)-5-(pyrrolidin-1-ylcarbonyl)-5,6,7,8-tetrahydro[1,2,4]triazolo[4,3-a]pyridin-3(2H)-on

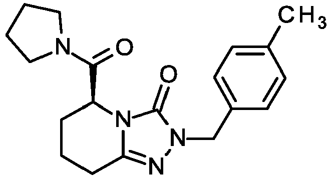

### Beispiel 27

### (5RS)-2-(4-Chlorbenzyl)-5-(pyrrolidin-1-ylcarbonyl)-5,6,7,8-tetrahydro[1,2,4]triazolo[4,3-a]pyridin-3(2H)-on (Racemat)

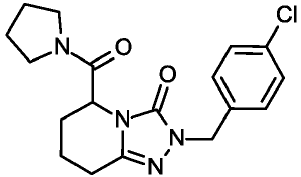

Methyl-(5RS)-2-(4-chlorbenzyl)-3-oxo-2,3,5,6,7,8-hexahydro[1,2,4]triazolo[4,3-a]pyridin-5-carboxylat (Racemat) (77.0 mg, 239 µmol) wurde in 1 ml THF/Wasser vorgelegt, mit Lithiumhydroxid (28.7 mg, 1.20 mmol) versetzt und die Reaktionsmischung über ein Wochenende bei Raumtemperatur gerührt. Zur Aufarbeitung wurde das THF im Vakuum entfernt und der wässrige Rest lyophilisiert. Man erhielt so 74 mg des Lithiumsalzes der Carbonsäure, das direkt weiter umgesetzt wurde. Dazu wurde mit wässriger Salzsäure die Carbonsäure freigesetzt und diese in 3 ml THF gelöst. Man gab Triethylamin (66 µl, 470 µmol), HATU (135 mg, 356 µmol) und Pyrrolidin (24 µl, 280 µmol) zu und rührte über Nacht unter Argon bei Raumtemperatur. Die Reaktionsmischung wurde im Vakuum eingeengt, der Rückstand über präparative HPLC getrennt (Säule: Chromatorex, 125x40mm 10µm. Flow: 50ml/min. Gradient ( A= Wasser+ 0,1% Ameisensäure , B= Acetonitril): 0min 10% B, 5min 10%B, 27min 98% B, 35min 98 % B, 35,01min 10%B , 38min 10%B. Laufzeit pro Trennung 38min. Detektion: 210 nm). Die produkthaltigen Fraktionen wurden eingeengt, lyophilisiert und im Vakuum von Lösungsmittelresten befreit. Es wurden so 64.3 mg (75 % d.Th) der Titelverbindung erhalten.
LC-MS (Methode 4): Rₜ = 0.76 min; MS (ESIpos): m/z = 361 [M+H]⁺
¹H-NMR (400MHz, DMSO-d₆): δ [ppm]= 1.61 - 1.84 (m, 4H), 1.87 - 2.10 (m, 4H), 2.46 - 2.66 (m, 2H, teilweise durch Lösungsmittelsignal verdeckt), 3.21 - 3.40 (m, 2H, teilweise von Wassersignal überlagert), 3.46 (dt, 1H), 3.62 (dt, 1H), 4.74 (dd, 1H), 4.75 - 4.85 (m, 2H), 7.22 - 7.28 (m, 2H), 7.37 - 7.43 (m, 2H).

### Beispiel 28

### tert-Butyl-4-{[(5RS)-3-oxo-5-(pyrrolidin-1-ylcarbonyl)-5,6,7,8-tetrahydro[1,2,4]triazolo[4,3-a]pyridin-2(3H)-yl]methyl}benzoat (Racemat)

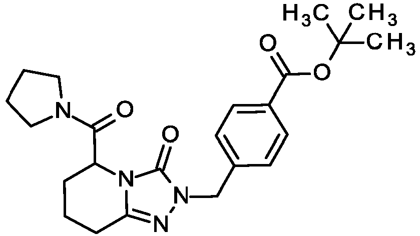

(5RS)-2-[4-(tert-Butoxycarbonyl)benzyl]-3-oxo-2,3,5,6,7,8-hexahydro[1,2,4]triazolo[4,3-a]pyridin-5-carbonsäure (Racemat) (246 mg, 88 % Reinheit, 580 µmol) wurde in 6.0 ml THF gelöst. Man gab Triethylamin (240 µl, 1.7 mmol), HATU (287 mg, 754 µmol) und Pyrrolidin (58 µl, 700 µmol) zu und rührte über Nacht unter Argon bei Raumtemperatur. Zur weiteren Umsetzung fügte man noch einmal Triethylamin (81 µl, 580 µmol), HATU (66.1 mg, 174 µmol) und Pyrrolidin (15 µl, 170 µmol) zu und rührte weitere 2.5h unter obigen Bedingungen. Zur Aufarbeitung wurde der Ansatz mit Ethylacetat/Wasser verdünnt. Nach Extraktion und Abtrennung der organischen Phase wurde die wässrige noch zweimal mit Ethylacetat extrahiert. Die organischen Phasen wurden mit gesättigter wässriger Natriumchloridlösung gewaschen und über Natriumsulfat getrocknet. Der nach Einengen im Vakuum verbliebene Rückstand wurde in DMSO/Acetonitril/Wasser gelöst und in 2 Chargen über präparative HPLC getrennt (Säule: Kromasil C18, 125x30mm, 10µm, Eluent: Acetonitril (B) / Wasser + 0,1 % TFA (A), Gradient: 0min 90% A, 6min 90% A, 18min 5%A, 20min 5%A, 21min 90%A, Fluss: 75ml/min, Detektor: 210nm). Die vereinigten produkthaltigen Fraktionen wurden eingeengt und das Rohprodukt noch einmal zwischen Wasser und Ethylacetat verteilt. Aus der organischen Phase wurden nach Trocknen, Eeinengen und Trocknen im Vakuum 255 mg (103 % der Theorie) der Titelverbindung erhalten.
LC-MS (Methode 4): Rₜ = 0.92 min; MS (ESIpos): m/z = 427 [M+H]⁺
¹H-NMR (400MHz, DMSO-d₆): δ [ppm]= 1.54 (s, 9H), 1.62 - 1.85 (m, 4H), 1.87 - 2.10 (m, 4H), 2.47 - 2.67 (m, 2H, teilweise durch Lösungsmittelsignal verdeckt), 3.22 - 3.40 (m, 2H, teilweise von Wassersignal überlagert), 3.47 (dt, 1H), 3.62 (dt, 1H), 4.75 (dd, 1H), 4.88 (s, 2H), 7.30 - 7.36 (m, 2H), 7.83 - 7.89 (m, 2H).

### Beispiel 29

### 4-{[(5RS)-3-Oxo-5-(pyrrolidin-1-ylcarbonyl)-5,6,7,8-tetrahydro[1,2,4]triazolo[4,3-a]pyridin-2(3H)-yl]methyl}benzolsulfonamid (Racemat)

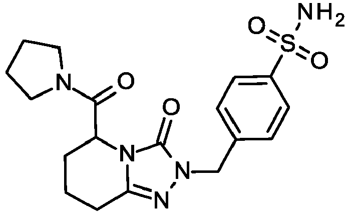

(5RS)-3-Oxo-2-(4-sulfamoylbenzyl)-2,3,5,6,7,8-hexahydro[1,2,4]triazolo[4,3-a]pyridin-5-carbonsäure (Racemat) (30.0 mg, 85.1 µmol) wurde in 2.0 ml DMF gelöst. Man gab Triethylamin (24 µl, 170 µmol), HATU (48.6 mg, 128 µmol) und Pyrrolidin (8.5 µl, 100 µmol) zu und rührte über Nacht unter Argon bei Raumtemperatur. Zur Aufarbeitung und Reinigung wurde der Ansatz direkt über präparative HPLC getrennt (Säule: Chromatorex, 125x30mm 10µm. Flow: 50ml/min. Gradient (A= Wasser+ 0,1% Ameisensäure , B= Acetonitril ). Laufzeit pro Trennung 38min. 0min 0% B, 6min 10%B, 27min 95% B, 38min 95 % B , 40min 0%B . Detektion: 210 nm). Die vereinigten produkthaltigen Fraktionen wurden lyophilisiert. So wurden 7.60 mg (22 % d. Th.) der Titelverbindung erhalten.
LC-MS (Methode 6): Rₜ = 0.99 min; MS (ESIpos): m/z = 406 [M+H]⁺
¹H-NMR (400MHz, DMSO-d₆): δ [ppm]= 1.60 - 1.85 (m, 4H), 1.85 - 2.12 (m, 4H), 2.40 - 2.69 (m, 2H, teilweise durch Lösungsmittelsignal verdeckt), 3.20 - 3.31 (m, 1H), 3.31 - 3.58 (m, 2H, teilweise von Wassersignal überlagert), 3.62 (dt, 1H), 4.75 (dd, 1H), 4.89 (s, 2H), 7.32 (s, 2H), 7.40 (d, 2H), 7.78 (d, 2H).

### Beispiel 30

### (5RS)-2-[4-(Methylsulfanyl)benzyl]-5-(pyrrolidin-1-ylcarbonyl)-5,6,7,8-tetrahydro[1,2,4]triazolo[4,3-a]pyridin-3(2H)-on (Racemat)

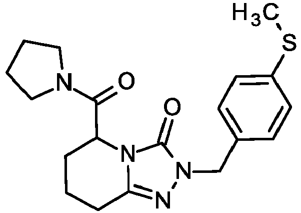

(5RS)-2-[4-(Methylsulfanyl)benzyl]-3-oxo-2,3,5,6,7,8-hexahydro[1,2,4]triazolo[4,3-a]pyridin-5-carbonsäure (Racemat) (136 mg, 95 % Reinheit, 405 µmol) wurde in 4.0 ml THF gelöst. Man gab Triethylamin (170 µl, 1.2 mmol), HATU (200 mg, 526 µmol) und Pyrrolidin (41 µl, 490 µmol) zu und rührte über ein Wochenende unter Argon bei Raumtemperatur. Die Reaktionsmischung wurde im Vakuum eingeengt, der Rückstand in Acetonitril/Wasser aufgenommen und über präparative HPLC getrennt (Säule: Kromasil C18, 125x30mm, 10µm, Eluent: Acetonitril (B) / Wasser + 0,1 % TFA (A), Gradient: 0min 90% A, 6min 90% A, 18min 5%A, 20min 5%A, 21min 90%A, Fluss: 75ml/min, Detektor: 210nm). Die produkthaltigen Fraktionen wurden vereinigt, eingeengt und im Vakuum getrocknet. Es wurden so 85.0 mg (55 % d. Th.) der Titelverbindung erhalten.
LC-MS (Methode 4): Rₜ = 0.75 min; MS (ESIpos): m/z = 373 [M+H]⁺
¹H-NMR (400MHz, DMSO-d₆): δ [ppm]= 1.61 - 1.84 (m, 4H), 1.88 - 2.10 (m, 4H), 2.45 (s, 3H), 2.47 - 2.67 (m, 2H, teilweise durch Lösungsmittelsignal verdeckt), 3.26 (dt, 1H), 3.36 (dt, 1H), 3.47 (dt, 1H), 3.65 (dt, 1H, teilweise von Wassersignal überlagert), 4.70 - 4.80 (m, 3H), 7.15 - 7.25 (m, 4H).

### Beispiel 31

### (5RS)-2-[(6-Chlorpyridin-3-yl)methyl]-5-(pyrrolidin-1-ylcarbonyl)-5,6,7,8-tetrahydro[1,2,4]triazolo[4,3-a]pyridin-3(2H)-on (Racemat)

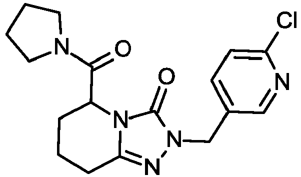

### (5RS)-2-[(6-Chlorpyridin-3-yl)methyl]-3-oxo-2,3,5,6,7,8-hexahydro[1,2,4]triazolo[4,3-a]pyridin-5-carbonsäure (Racemat)

(1.59 g, 92 % Reinheit, 4.74 mmol) wurde in 25 ml THF gelöst. Man gab Triethylamin (2.0 ml, 14 mmol), HATU (2.34 g, 6.16 mmol) und Pyrrolidin (470 µl, 5.7 mmol) zu und rührte über ein Wochenende bei Raumtemperatur. Zur Aufarbeitung wurde der Ansatz auf Wasser gegeben, das Gemisch mit Natriumchlorid gesättigt und zweimal mit Ethylacetat extrahiert. Die vereinigten organischen Phasen wurden über Magnesiumsulfat getrocknet, filitriert und eingeengt. Der Rückstand wurde durch Chromatographie an Kieselgel (PuriFlash 100g Kieselgelkartusche Dichlormethan/Methanol 20:1, Detektor: 214 nm) gereinigt . So wurden 1.11 g (65 % d. Th.) der Titelverbindung erhalten.
LC-MS (Methode 3): Rₜ = 1.04 min; MS (ESIpos): m/z = 362 [M+H]⁺
¹H-NMR (400MHz, DMSO-d₆): δ [ppm]= 1.59 - 1.86 (m, 4H), 1.86 - 2.10 (m, 4H), 2.53 - 2.71 (m, 2H, teilweise durch Lösungsmittelsignal verdeckt), 3.21 - 3.41 (m, 2H, teilweise von Wassersignal überlagert), 3.47 (dt, 1H), 3.62 (dt, 1H), 4.74 (dd, 1H), 4.88 (s, 2H), 7.51 (d, 1H), 7.70 (dd, 1H), 8.30 (d, 1H). Das Spektrum zeigt bei 1.15 ppm und bei 3.05 ppm breite Signale für Triethylammoniumionen

### Beispiel 32

### (5RS)-2-[(6-Chlorpyridin-3-yl)methyl]-5-(pyrrolidin-1-ylcarbonyl)-5,6,7,8-tetrahydro[1,2,4]triazolo[4,3-a]pyridin-3(2H)-on (Enantiomer 2)

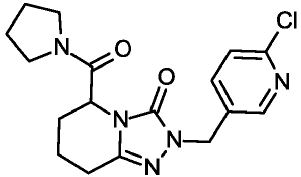

(5RS)-2-[(6-Chlorpyridin-3-yl)methyl]-5-(pyrrolidin-1-ylcarbonyl)-5,6,7,8-tetrahydro[1,2,4]triazolo[4,3-a]pyridin-3(2H)-on (Racemat) wurde durch chirale präparative SFC getrennt [Probenvorbereitung: 400 mg gelöst in 15 ml Ethanol; Injektionsvolumen: 4.0 ml; Säule: Daicel Chiralpak^{®} AD SFC 5µm, 250 x 50 mm; Laufmittel: CO2/Ethanol: isokratisch 35% Ethanol, Fluß: 80 ml/min; Temperatur 40°C; UV Detektion: 210 nm]. Nach der Trennung und Lyophilisierung der Produkte wurden 144 mg von Enantiomer 1 welches zuerst eluierte, und 138 g von Enantiomer 2, welches später eluierte, isoliert.

### Enantiomer 2:

Analytische chirale SFC: Rₜ = 3.41 min, e.e. = 100% [Säule: Daicel Chiralcel^{®} AD; Laufmittel: CO2/Ethanol 60:40 ; Fluß: 3 ml Ethanol/min; UV Detektion: 210 nm]
LC-MS (Methode 4): Rₜ = 0.60 min; MS (ESIpos): m/z = 362 [M+H]⁺
1H-NMR (400MHz, DMSO-d6): δ [ppm]= 1.59 - 1.85 (m, 4H), 1.87 - 2.10 (m, 4H), 2.47 - 2.68 (m, 2H, teilweise durch Lösungsmittelsignal verdeckt), 3.22 - 3.31 (m, 1H), 3.31 - 3.40 (m, 1H), 3.47 (dt, 1H), 3.62 (dt, 1H), 4.74 (dd, 1H), 4.88 (s, 2H), 7.51 (d, 1H), 7.70 (dd, 1H), 8.30 (d, 1H).

### Beispiel 33

### (5RS)-2-[(5-Chlorpyridin-2-yl)methyl]-5-(pyrrolidin-1 -ylcarbonyl)-5,6,7,8-tetrahydro[1,2,4]triazolo[4,3-a]pyridin-3(2H)-on (Racemat)

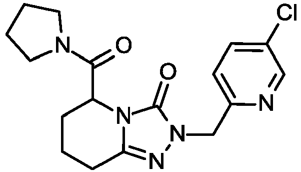

(5RS)-2-[(5-Chlorpyridin-2-yl)methyl]-3-oxo-2,3,5,6,7,8-hexahydro[1,2,4]triazolo[4,3-a]pyridin-5-carbonsäure (Racemat) (220 mg, 81 % Reinheit, 574 µmol) wurde in 3.0 ml THF gelöst. Man gab Triethylamin (400 µl, 2.9 mmol), HATU (284 mg, 746 µmol) und Pyrrolidin (57 µl, 690 µmol) zu und rührte über Nacht bei Raumtemperatur. Zur Aufarbeitung wurde der Ansatz auf Wasser gegeben, das Gemisch mit Natriumchlorid gesättigt und zweimal mit Ethylacetat extrahiert. Die vereinigten organischen Phasen wurden über Magnesiumsulfat getrocknet, filtriert und eingeengt. Der Rückstand wurde über präparative HPLC getrennt (Säule: Chromatorex, 125x30mm 10µm. Flow: 50ml/min. Gradient ( A= Wasser + 0,1% Ameisensäure , B= Acetonitril ). Laufzeit pro Trennung 38min. Detektion: 210 nm => 0min 0% B, 6min 10%B, 27min 95% B, 38min 95 % B , 40min 0%B) . So wurden 177 mg (85 % d. Th.) der Titelverbindung erhalten.
LC-MS (Methode 3): Rₜ = 1.09 min; MS (ESIpos): m/z = 362 [M+H]⁺
¹H-NMR (500MHz, DMSO-d₆): δ [ppm]= 1.64 - 1.83 (m, 4H), 1.87 - 2.11 (m, 4H), 2.46 - 2.66 (m, 2H, teilweise durch Lösungsmittelsignal verdeckt), 3.22 - 3.43 (m, 2H, teilweise von Wassersignal überlagert), 3.48 (dt, 1H), 3.62 (dt, 1H), 4.76 (dd, 1H), 4.87 - 4.95 (m, 2H), 7.21 (d, 1H), 7.92 (dd, 1H), 8.57 (d, 1H).

### Beispiel 34

### (5RS)-2-[(5-Chlorpyridin-2-yl)methyl]-5-(pyrrolidin-1-ylcarbonyl)-5,6,7,8-tetrahydro[1,2,4]triazolo[4,3-a]pyridin-3(2H)-on (Enantiomer 2)

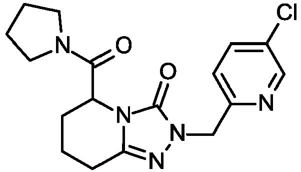

(5RS)-2-[(5-Chlorpyridin-2-yl)methyl]-5-(pyrrolidin-1-ylcarbonyl)-5,6,7,8-tetrahydro[1,2,4]triazolo[4,3-a]pyridin-3(2H)-on (Racemat) wurde durch chirale präparative Flüssigchromatographie getrennt [Probenvorbereitung: 220 mg gelöst in 12 ml warmem Isopropanol; Injektionsvolumen: 0.5 ml; Säule: Daicel Chirapak^{®} AS-H 5µm, 250 x 50 mm; Laufmittel: Isohexan/Isopropanol: isokratisch 50% Isopropanol, Fluß: 15.0 ml/min; Temperatur 25°C; UV Detektion: 210 nm]. Nach der Trennung wurden 69 mg von Enantiomer 1, welches zuerst eluierte, und 66 mg von Enantiomer 2, welches später eluierte, isoliert.

### Enantiomer 2:

Analytische chirale HPLC: Rₜ = 1.40 min, e.e. = 100% [Säule: Daicel Chiralcel^{®} AS-3 3µm 50 × 4.6 mm; Laufmittel: Isohexan/Isopropanol 1:1 ; Fluß: 1 ml/min; UV Detektion: 220 nm]
LC-MS (Methode 3): Rₜ = 1.06 min; MS (ESIpos): m/z = 362 [M+H]⁺
1H-NMR (400MHz, DMSO-d6): δ [ppm]= 1.62 - 1.86 (m, 4H), 1.86 - 2.14 (m, 4H), 2.47 - 2.69 (m, 2H, teilweise durch Lösungsmittelsignal verdeckt), 3.21 - 3.42 (m, 2H, teilweise von Wassersignal überlagert), 3.47 (dt, 1H), 3.62 (dt, 1H), 4.76 (dd, 1H), 4.86 - 4.96 (m, 2H), 7.21 (d, 1H), 7.92 (dd, 1H), 8.57 (d, 1H).

### Beispiel 35

### 5-{[(5RS)-3-Oxo-5-(pyrrolidin-1-ylcarbonyl)-5,6,7,8-tetrahydro[1,2,4]triazolo[4,3-a]pyridin-2(3H)-yl]methyl}pyridin-2-carbonitril (Racemat)

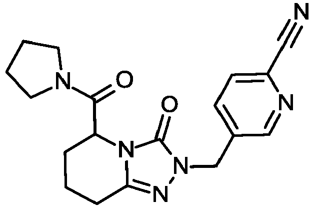

(5RS)-2-[(6-Cyanpyridin-3-yl)methyl]-3-oxo-2,3,5,6,7,8-hexahydro[1,2,4]triazolo[4,3-a]pyridin-5-carbonsäure (Racemat) (190 mg, 70 % Reinheit, 444 µmol) wurde in 4.0 ml THF gelöst. Man gab Triethylamin (190 µl, 1.3 mmol), HATU (220 mg, 578 µmol) und Pyrrolidin (45 µl, 530 µmol) zu und rührte über ein Wochenende bei Raumtemperatur. Zur Aufarbeitung wurde der Ansatz eingeengt, der resultierende Rückstand in Acetonitril/Wasser aufgenommen und über präparative HPLC getrennt (Säule: Kromasil C18, 125x30mm, 10µm, Eluent: Acetonitril (B) / Wasser (A, neutral), Gradient: 0min 90% A, 6min 90% A, 18min 5%A, 20min 5%A, 21min 90%A, Fluss: 75ml/min, Detektor: 210nm). Produkthaltige Fraktionen wurden vereinigt, eingeengt und getrocknet. So wurden 80.0 mg (51 % d. Th.) der Titelverbindung erhalten.
LC-MS (Methode 4): Rₜ = 0.55 min; MS (ESIpos): m/z = 353 [M+H]⁺
¹H-NMR (400MHz, DMSO-d₆): δ [ppm]= 1.59 - 1.84 (m, 4H), 1.86 - 2.11 (m, 4H), 2.46 - 2.69 (m, 2H, teilweise durch Lösungsmittelsignal verdeckt), 3.22 - 3.40 (m, 2H, teilweise von Wassersignal überlagert), 3.47 (dt, 1H), 3.62 (dt, 1H), 4.76 (dd, 1H), 4.95 - 5.05 (m, 2H), 7.84 (dd, 1H), 8.04 (d, 1H), 8.62 (d, 1H).

### Beispiel 36

### (5RS)-2-(4-Methoxybenzyl)-5-(pyrrolidin-1-ylcarbonyl)-5,6,7,8-tetrahydro[1,2,4]triazolo[4,3-a]pyridin-3(2H)-on (Racemat)

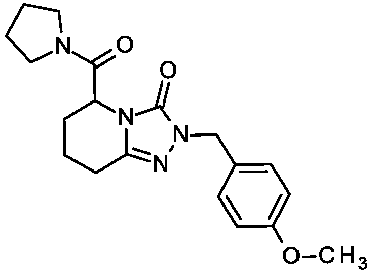

(5RS)-2-(4-Methoxybenzyl)-3-oxo-2,3,5,6,7,8-hexahydro[1,2,4]triazolo[4,3-a]pyridin-5-carbonsäure (Racemat) (1.67 g, 80 % Reinheit, 4.40 mmol) wurde in DMF (10 ml) gelöst. Man gab Triethylamin (1.2 ml, 8.8 mmol), HATU (2.51 g, 6.61 mmol) und Pyrrolidin (440 µl, 5.3 mmol) zu und rührte unter Argon über Nacht bei Raumtemperatur. Zur Aufarbeitung wurde der Ansatz mit Wasser versetzt, das Gemisch mit Natriumchlorid gesättigt und 3 × mit je 30 ml Ethylacetat extrahiert. Die vereinigten organischen Phasen wurden mit Magnesiumsulfat getrocknet, filtriert und im Vakuum eingeengt. Der verbliebene Rückstand wurde in 3 Injektionen über präparative HPLC getrennt (Säule: Chromatorex, 125x40mm 10µm. Flow: 50ml/min. Gradient ( A= Wasser+ 0,1% Ameisensäure , B= Acetonitril ). Laufzeit pro Trennung 38min. Detektion: 210 nm => 0min 0% B, 6min 10%B, 27min 95% B, 38min 95 % B , 40min 0%B =>). Durch Einengen der produkthaltigen Fraktionen wurden 830 mg (97% Reinheit, 51% d.Th.) der Titelverbindung erhalten.

Die Hauptmenge wurde ohne weitere Reinigung als Intermediat weiter verwendet.

Etwa. 50 mg wurden nochmals unter obigen Bedingungen über präparative HPLC gereinigt zu 33 mg der Titelverbindung, 100% Reinheit laut LC/MS.
LC-MS (Methode 3): Rₜ = 1.16 min; MS (ESIpos): m/z = 357 [M+H]⁺
¹H-NMR (400MHz, DMSO-d₆): δ [ppm]= 1.62 - 1.83 (m, 4H), 1.88 - 2.08 (m, 4H), 2.45 - 2.64 (m, 2H, teilweise durch Lösungsmittelsignal verdeckt), 3.21 - 3.40 (m, 2H, teilweise von Wassersignal überlagert) 3.46 (dt, 1H), 3.62 (dt, 1H), 3.73 (s, 3H), 4.66 - 4.77 (m, 3H), 6.85 - 6.91 (m, 2H), 7.13 - 7.20 (m, 2H).

### Beispiel 37

### (5RS)-2-[4-Chlor-3-(trifluormethyl)benzyl]-5-(pyrrolidin-1-ylcarbonyl)-5,6,7,8-tetrahydro[1,2,4]triazolo[4,3-a]pyridin-3(2H)-on (Racemat)

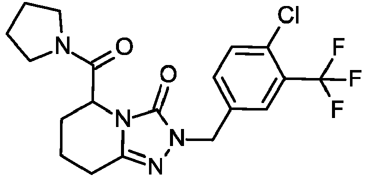

(5RS)-2-[4-Chlor-3-(trifluormethyl)benzyl]-3-oxo-2,3,5,6,7,8-hexahydro[1,2,4]triazolo[4,3-a]pyridin-5-carbonsäure (Racemat) (138 mg, 367 µmol) wurde in 4.0 ml THF gelöst. Man gab Triethylamin (110 µl, 810 µmol), HATU (182 mg, 477 µmol) und Pyrrolidin (37 µl, 440 µmol) zu und rührte über Nacht bei Raumtemperatur. Zur Aufarbeitung wurde die Reaktionsmischung eingeengt, der resultierende Rückstand in Acetonitril/Wasser aufgenommen und über präparative HPLC getrennt (Säule: Kromasil C18, 125x30mm, 10µm, Eluent: Acetonitril (B) / Wasser + 0,1 % TFA (A), Gradient: 0min 90% A, 6min 90% A, 18min 5%A, 20min 5%A, 21min 90%A, Fluss: 75ml/min, Detektor: 210nm). Produkthaltige Fraktionen wurden vereinigt, eingeengt und getrocknet. So wurden 121 mg (77 % d. Th.) der Titelverbindung erhalten.
LC-MS (Methode 3): Rₜ = 1.66 min; MS (ESIpos): m/z = 429 [M+H]⁺
¹H-NMR (400MHz, DMSO-d₆): δ [ppm]= 1.60 - 1.85 (m, 4H), 1.87 - 2.11 (m, 4H), 2.47 - 2.69 (m, 2H, teilweise durch Lösungsmittelsignal verdeckt), 3.21 - 3.57 (m, 3H, teilweise von Wassersignal überlagert), 3.62 (dt, 1H), 4.75 (dd, 1H), 4.92 (s, 2H), 7.51 (dd, 1H), 7.72 (d, 1H), 7.76 (d, 1H).

### Beispiel 38

### (5RS)-2-(3-Chlorbenzyl)-5-(pyrrolidin-1-ylcarbonyl)-5,6,7,8-tetrahydro[1,2,4]triazolo[4,3-a]pyridin-3(2H)-on (Racemat)

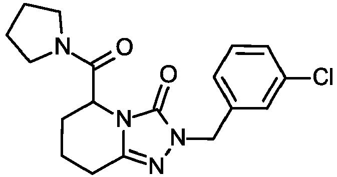

(5RS)-2-(3-Chlorbenzyl)-3-oxo-2,3,5,6,7,8-hexahydro[1,2,4]triazolo[4,3-a]pyridin-5-carbonsäure (Racemat) (110 mg, 83 % Reinheit, 297 µmol) wurde in 3.5 ml THF gelöst. Man gab Triethylamin (91 µl, 650 µmol), HATU (147 mg, 386 µmol) und Pyrrolidin (30 µl, 360 µmol) zu und rührte über Nacht bei Raumtemperatur. Zur Aufarbeitung wurde der Ansatz eingeengt, der resultierende Rückstand in Acetonitril/Wasser aufgenommen und über präparative HPLC getrennt (Säule: Kromasil C18, 125x30mm, 10µm, Eluent: Acetonitril (B) / Wasser + 0,1 % TFA (A), Gradient: 0min 90% A, 6min 90% A, 18min 5%A, 20min 5%A, 21min 90%A, Fluss: 75ml/min, Detektor: 210nm). Produkthaltige Fraktionen wurden vereinigt, eingeengt und getrocknet. So wurden 80.3 mg (75 % d. Th.) der Titelverbindung erhalten.
LC-MS (Methode 3): Rₜ = 1.39 min; MS (ESIpos): m/z = 361 [M+H]⁺
¹H-NMR (400MHz, DMSO-d₆): δ [ppm]= 1.61 - 1.84 (m, 4H), 1.88 - 2.11 (m, 4H), 2.47 - 2.68 (m, 2H, teilweise durch Lösungsmittelsignal verdeckt), 3.26 (dt, 1H), 3.36 (dt, 1H), 3.47 (dt, 1H), 3.62 (dt, 1H, teilweise von Wassersignal überlagert),, 4.75 (dd, 1H), 4.83 (s, 2H), 7.19 (br d, 1H), 7.29 (br s, 1H), 7.32 - 7.40 (m, 2H).

### Beispiel 39

### (5RS)-2-(3,4-Dichlorbenzyl)-5-(pyrrolidin-1-ylcarbonyl)-5,6,7,8-tetrahydro[1,2,4]triazolo[4,3-a]pyridin-3(2H)-on (Racemat)

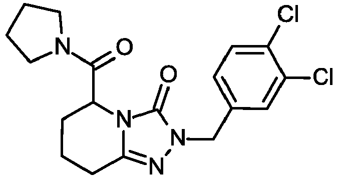

(5RS)-2-(3,4-Dichlorbenzyl)-3-oxo-2,3,5,6,7,8-hexahydro[1,2,4]triazolo[4,3-a]pyridin-5-carbonsäure (Racemat) (120 mg, 80 % Reinheit, 281 µmol) wurde in 3.0 ml THF gelöst. Man gab Triethylamin (86 µl, 620 µmol), HATU (139 mg, 365 µmol) und Pyrrolidin (28 µl, 340 µmol) zu und rührte über Nacht bei Raumtemperatur. Zur Aufarbeitung wurde der Ansatz eingeengt, der resultierende Rückstand in Acetonitril/Wasser aufgenommen und über präparative HPLC getrennt (Säule: Kromasil C18, 125x30mm, 10µm, Eluent: Acetonitril (B) / Wasser + 0,1 % TFA (A), Gradient: 0min 90% A, 6min 90% A, 18min 5%A, 20min 5%A, 21min 90%A, Fluss: 75ml/min, Detektor: 210nm). Produkthaltige Fraktionen wurden vereinigt, eingeengt und getrocknet. So wurden 113 mg (97 % d. Th.) der Titelverbindung erhalten.
LC-MS (Methode 4): Rₜ = 0.84 min; MS (ESIpos): m/z = 395 [M+H]⁺
¹H-NMR (400MHz, DMSO-d₆): δ [ppm]= 1.60 - 1.84 (m, 4H), 1.85 - 2.10 (m, 4H), 2.45 - 2.67 (m, 2H, teilweise durch Lösungsmittelsignal verdeckt), 3.21 - 3.51 (m, 3H, teilweise von Wassersignal überlagert), 3.62 (dt, 1H), 4.75 (dd, 1H), 4.83 (s, 2H), 7.22 (dd, 1H), 7.49 (d, 1H), 7.62 (d, 1H).

### Beispiel 40

### (5RS)-5-(Pyrrolidin-1-ylcarbonyl)-2-{[4-(trifluormethyl)cyclohexyl]methyl}-5,6,7,8-tetrahydro[1,2,4]triazolo[4,3-a]pyridin-3(2H)-on (Diastereomerengemisch; 4 Isomere)

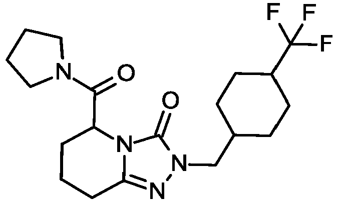

(5RS)-3-Oxo-2-{[4-(trifluormethyl)cyclohexyl]methyl}-2,3,5,6,7,8-hexahydro[1,2,4]triazolo[4,3-a]pyridin-5-carbonsäure (Diastereomerengemisch; 4 Isomere) (326 mg, 939 µmol,) wurde in 10 ml THF gelöst. Man gab Triethylamin (260 µl, 1.9 mmol), HATU (464 mg, 1.22 mmol) und Pyrrolidin (94 µl, 1.1 mmol) zu und rührte über Nacht bei Raumtemperatur. Zur Aufarbeitung wurde der Ansatz mit Wasser versetzt und dann eingeengt, der resultierende Rückstand in Acetonitril/Wasser aufgenommen und über präparative HPLC getrennt (Säule: Kromasil C18, 125x30mm, 10µm, Eluent: Acetonitril (B) / Wasser + 0,1 % TFA (A), Gradient: 0min 90% A, 6min 90% A, 18min 5%A, 20min 5%A, 21min 90%A, Fluss: 75ml/min, Detektor: 210nm). Produkthaltige Fraktionen wurden vereinigt, eingeengt und getrocknet. So wurden 90.2 mg (24 % d. Th.) der Titelverbindung als racemisches cis/trans-Gemisch erhalten.
LC-MS (Methode 4): Rₜ = 0.90 min; MS (ESIpos): m/z = 401 [M+H]⁺
¹H-NMR (400MHz, DMSO-d₆): δ [ppm]= 0.89 - 1.08 (m, 1H), 1.10 - 1.26 (m, 1H), 1.43 - 2.10 (m, 15H), 2.11 - 2.34 (m, 1H), 2.45 - 2.69 (m, 2H, teilweise durch Lösungsmittelsignal verdeckt), 3.20 - 3.29 (m, 1H), 3.29 - 3.72 (m, 5H, teilweise von Wassersignal überlagert), 4.67 - 4.74 (m, 1H).

### Beispiel 41

### (5RS)-2-[(4,4-Difluorcyclohexyl)methyl]-5-(pyrrolidin-1-ylcarbonyl)-5,6,7,8-tetrahydro[1,2,4]triazolo[4,3-a]pyridin-3(2H)-on (Racemat)

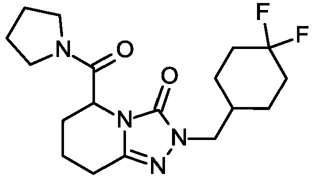

(5RS)-2-[(4,4-Difluorcyclohexyl)methyl]-3-oxo-2,3,5,6,7,8-hexahydro[1,2,4]triazolo[4,3-a]pyridin-5-carbonsäure (Racemat) (30.5 mg, 96.7 µmol) wurde in 1.0 ml THF gelöst. Man gab Triethylamin (27 µl, 1.90 µmol), HATU (47.8 mg, 126 µmol) und Pyrrolidin (9.7 µl, 120 µmol) zu und rührte über Nacht bei Raumtemperatur. Zur Aufarbeitung wurde der Ansatz mit Wasser versetzt und dann eingeengt, der resultierende Rückstand in Acetonitril/Wasser aufgenommen und über präparative HPLC getrennt (Säule: Kromasil C18, 125x30mm, 10µm, Eluent: Acetonitril (B) / Wasser + 0,1 % TFA (A), Gradient: 0min 90% A, 6min 90% A, 18min 5%A, 20min 5%A, 21min 90%A, Fluss: 75ml/min, Detektor: 210nm). Produkthaltige Fraktionen wurden vereinigt, eingeengt und getrocknet. So wurden 17.6 mg 49 % d. Th.) der Titelverbindung erhalten.
LC-MS (Methode 4): Rₜ = 0.77 min; MS (ESIpos): m/z = 369 [M+H]⁺
¹H-NMR (400MHz, DMSO-d₆): δ [ppm]= 1.12 - 1.27 (m, 2H), 1.61 - 2.09 (m, 15H), 2.47 - 2.68 (m, 2H, teilweise durch Lösungsmittelsignal verdeckt), 3.25 (dt, 1H), 3.34 (dt, 1H), 3.41 - ca. 3.70 (m, 4H, teilweise von Wassersignal überlagert), 4.70 (dd, 1H).

### Beispiel 42

### (5RS)-5-[(3,3-Difluorpyrrolidin-1 -yl)carbonyl]-2-(4-methylbenzyl)-5,6,7,8-tetrahydro[1,2,4]triazolo[4,3-a]pyridin-3(2H)-on (Racemat)

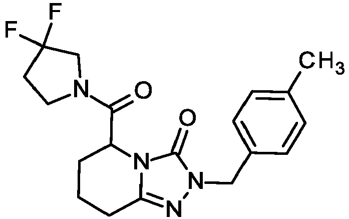

(5RS)-2-(4-Methylbenzyl)-3-oxo-2,3,5,6,7,8-hexahydro[1,2,4]triazolo[4,3-a]pyridin-5-carbonsäure (Racemat) (36.0 mg, 124 µmol) wurde in 1.3 ml THF gelöst. Man gab Triethylamin (57 µl, 410 µmol), HATU (70.7 mg, 186 µmol) und 3,3-Difluorpyrrolidinhydrochlorid (21.4 mg, 149 µmol) zu und rührte 3h bei Raumtemperatur. Zur Aufarbeitung wurde der Ansatz eingeengt, der resultierende Rückstand in DMSO/Acetonitril/Wasser gelöst und über präparative HPLC getrennt (Säule: Kromasil C18, 250x30mm, 10µm, Eluent: Acetonitril (B) / Wasser + 0,1 % TFA (A), Gradient: 0min 90% A, 6min90% A, 27min 5%A, 38min 5%A, 38min 5%A, 39min 90% A; Fluss: 50ml/min, Detektor: 210nm). Produkthaltige Fraktionen wurden vereinigt, eingeengt und getrocknet. So wurden 26.4 mg (57 % d. Th.) der Titelverbindung erhalten.
LC-MS ((Methode 3): Rₜ = 1.46 min; MS (ESIpos): m/z = 377 [M+H]⁺
¹H-NMR (400MHz, DMSO-d₆): δ [ppm]= 1.59 - 1.78 (m, 2H), 1.90 - 2.12 (m, 2H), 2.27 (s, 3H), 2.35 - 2.66 (m, 4H, teilweise durch Lösungsmittelsignal verdeckt), 3.32 - 4.24 (m, 4H, teilweise von Wassersignal überlagert), 4.69 - 4.85 (m, 3H), 7.09 - 715 (m, 4H). Es liegt ein Rotamerengemisch vor.

### Beispiel 43

### (5RS)-2-[4-Chlor-3-(trifluormethyl)benzyl]-5-[(3,3-difluorpyrrolidin-1-yl)carbonyl]-5,6,7,8-tetrahydro[1,2,4]triazolo[4,3-a]pyridin-3(2H)-on (Racemat)

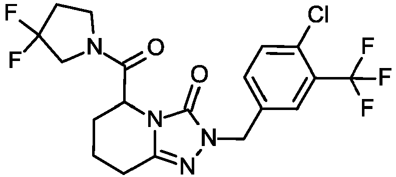

(5RS)-2-[4-Chlor-3-(trifluormethyl)benzyl]-3-oxo-2,3,5,6,7,8-hexahydro[1,2,4]triazolo[4,3-a]pyridin-5-carbonsäure (Racemat) (138 mg, 367 µmol) wurde in 4.0 ml THF gelöst. Man gab Triethylamin (160 µl, 1.2 mmol), HATU (182 mg, 477 µmol) und 3,3-Difluorpyrrolidinhydrochlorid (63.3 mg, 441 µmol) zu und rührte 3h bei Raumtemperatur. Zur Aufarbeitung wurde der Ansatz eingeengt, der resultierende Rückstand in Acetonitril/Wasser gelöst und über präparative HPLC getrennt (Säule: Kromasil C18, 125x30mm, 10µm, Eluent: Acetonitril (B) / Wasser + 0,1 % TFA (A), Gradient: 0min 90% A, 6min 90% A, 18min 5%A, 20min 5%A, 21min 90%A, Fluss: 75ml/min, Detektor: 210nm). Produkthaltige Fraktionen wurden vereinigt, eingeengt und getrocknet. So wurden 135 mg (79 % d. Th.) der Titelverbindung erhalten.
LC-MS (Methode 3): Rₜ = 1.76 min; MS (ESIpos): m/z = 465 [M+H]⁺
¹H-NMR (400MHz, DMSO-d₆): δ [ppm]= 1.58 - 1.80 (m, 2H), 1.93 - 2.14 (m, 2H), 2.34 - 2.69 (m, 4H, teilweise durch Lösungsmittelsignal verdeckt), 3.41 - 4.26 (m, 4H, teilweise von Wassersignal überlagert), 4.78 (dd, 0.5H), 4.85 (dd, 0.5H), 4.93 (s, 2H), 7.51 (dd, 1H), 7.72 (d, 1H), 7.76 (d, 1H). Es liegt ein Rotamerengemisch vor.

### Beispiel 44

### (5RS)-2-(3-Chlorbenzyl)-5-[(3,3-difluorpyrrolidin-1-yl)carbonyl]-5,6,7,8-tetrahydro[1,2,4]triazolo[4,3-a]pyridin-3(2H)-on (Racemat)

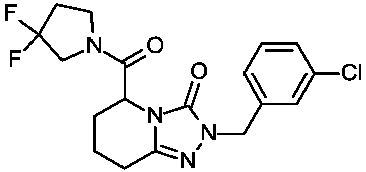

(5RS)-2-(3-Chlorbenzyl)-3-oxo-2,3,5,6,7,8-hexahydro[1,2,4]triazolo[4,3-a]pyridin-5-carbonsäure (Racemat) (110 mg, 83 % Reinheit, 297 µmol) wurde in 3.5 ml THF gelöst. Man gab Triethylamin (130 µl, 950 µmol), HATU (147 mg, 386 µmol) und 3,3-Difluorpyrrolidinhydrochlorid (51.1 mg, 356 µmol) zu und rührte über Nacht bei Raumtemperatur. Zur Aufarbeitung wurde der Ansatz eingeengt, der resultierende Rückstand in Acetonitril/Wasser gelöst und über präparative HPLC getrennt (Säule: Kromasil C18, 125x30mm, 10µm, Eluent: Acetonitril (B) / Wasser + 0,1 % TFA (A), Gradient: 0min 90% A, 6min 90% A, 18min 5%A, 20min 5%A, 21min 90%A, Fluss: 75ml/min, Detektor: 210nm). Produkthaltige Fraktionen wurden vereinigt, eingeengt und getrocknet. So wurden 89.2 mg (76 % d. Th.) der Titelverbindung erhalten.
LC-MS (Methode 3): Rₜ = 1.52 min; MS (ESIpos): m/z = 397 [M+H]⁺
¹H-NMR (400MHz, DMSO-d₆): δ [ppm]= 1.59 - 1.81 (m, 2H), 1.92 - 2.14 (m, 2H), 2.35 - 2.69 (m, 4H, teilweise durch Lösungsmittelsignal verdeckt), ca. 3.43 - 4.25 (m, 4H, teilweise von Wassersignal überlagert), 4.77 (dd, 0.5H), 4.80 - 4.89 (m, 2.5H), 7.19 (d, 1H), 7.29 (s, 1H), 7.32 - 7.42 (m, 2H). Es liegt ein Rotamerengemisch vor.

### Beispiel 45

### (5RS)-2-(3,4-Dichlorbenzyl)-5-[(3,3-difluorpyrrolidin-1 -yl)carbonyl]-5,6,7,8-tetrahydro[1,2,4]triazolo[4,3-a]pyridin-3(2H)-on (Racemat)

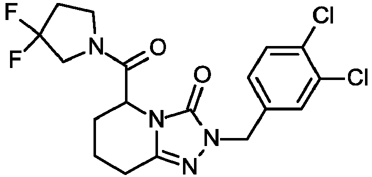

(5RS)-2-(3,4-Dichlorbenzyl)-3-oxo-2,3,5,6,7,8-hexahydro[1,2,4]triazolo[4,3-a]pyridin-5-carbonsäure (Racemat) (120 mg, 80 % Reinheit, 281 µmol) wurde in 3.5 ml THF gelöst. Man gab Triethylamin (125 µl, 900 µmol), HATU (147 mg, 386 µmol) und 3,3-Difluorpyrrolidinhydrochlorid (48.3 mg, 337 µmol) zu und rührte über Nacht bei Raumtemperatur. Zur Aufarbeitung wurde der Ansatz eingeengt, der resultierende Rückstand in Acetonitril/Wasser gelöst und über präparative HPLC getrennt (Säule: Kromasil C18, 125x30mm, 10µm, Eluent: Acetonitril (B) / Wasser + 0,1 % TFA (A), Gradient: 0min 90% A, 6min 90% A, 18min 5%A, 20min 5%A, 21min 90%A, Fluss: 75ml/min, Detektor: 210nm). Produkthaltige Fraktionen wurden vereinigt, eingeengt und getrocknet. So wurden 112 mg (90 % d. Th.) der Titelverbindung erhalten.
LC-MS (Methode 3): Rₜ = 1.68 min; MS (ESIpos): m/z = 431 [M+H]⁺
¹H-NMR (400MHz, DMSO-d₆): δ [ppm]= 1.59 - 1.80 (m, 2H), 1.92 - 2.14 (m, 2H), 2.34 - 2.69 (m, 4H, teilweise durch Lösungsmittelsignal verdeckt), 3.48 - 4.25 (m, 4H), 4.77 (dd, 0.5H) 4.81 - 4.89 (m, 2.5H), 7.22 (dd, 1H), 7.49 (d, 1H), 7.62 (d, 1H). Es liegt ein Rotamerengemisch vor.

### Beispiel 46

### (5RS)-2-(3-Chlor-4-methoxybenzyl)-5-(pyrrolidin-1-ylcarbonyl)-5,6,7,8-tetrahydro[1,2,4]triazolo[4,3-a]pyridin-3(2H)-on (Racemat)

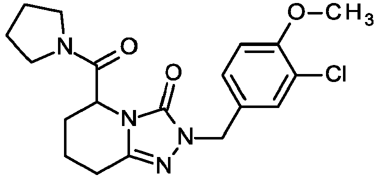

(5RS)-2-(3-Chlor-4-methoxybenzyl)-3-oxo-2,3,5,6,7,8-hexahydro[1,2,4]triazolo[4,3-a]pyridin-5-carbonsäure -trifluoressigsäure (Racemat) (70.0 mg, 155 µmol) wurde in THF (3.0 ml) vorgelegt und 1-[Bis(dimethylamino)methylen]-1H-[1,2,3]triazolo[4,5-b]pyridin-1-ium-3-oxidhexafluorophosphat (76.6 mg, 201 µmol) und Triethylamin (110 µl, 770 µmol) wurden anschließend zugegeben. Nach 15 min Rühren bei Raumtemperatur wurde Pyrrolidin (16 µl, 190 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 45.3 mg (75 % d. Th.) der Titelverbindung erhalten.
LC-MS (Methode 4): Rₜ = 0.76 min; MS (ESIpos): m/z = 391 [M+H]⁺
¹H-NMR (400 MHz, DMSO-d6) δ [ppm]: 1.399 (1.48), 1.709 (3.03), 1.756 (1.37), 1.773 (4.59), 1.790 (8.04), 1.807 (6.19), 1.824 (1.87), 1.892 (1.96), 1.909 (5.76), 1.925 (7.26), 1.942 (5.02), 1.960 (1.89), 1.977 (2.42), 1.996 (1.92), 2.007 (1.53), 2.022 (1.92), 2.032 (1.45), 2.073 (0.76), 2.328 (0.59), 2.523 (4.16), 2.564 (2.07), 2.577 (1.91), 2.590 (3.62), 2.602 (2.05), 2.621 (0.90), 2.631 (1.41), 2.669 (0.57), 2.865 (0.84), 3.224 (1.07), 3.241 (2.33), 3.254 (2.24), 3.270 (4.01), 3.287 (1.82), 3.326 (1.86), 3.344 (4.00), 3.361 (2.11), 3.373 (2.39), 3.391 (1.09), 3.433 (1.24), 3.451 (2.64), 3.458 (2.04), 3.476 (3.38), 3.493 (1.52), 3.592 (1.52), 3.609 (3.26), 3.625 (1.85), 3.633 (2.56), 3.649 (1.34), 3.729 (0.70), 3.966 (1.62), 4.696 (0.90), 4.724 (3.54), 4.735 (15.25), 4.739 (16.00), 4.779 (0.92), 7.095 (6.08), 7.116 (10.56), 7.167 (5.32), 7.172 (5.46), 7.188 (2.99), 7.193 (3.23), 7.293 (9.06), 7.298 (8.15).

### Beispiel 47

### (5RS)-5-[(3,3-Difluorpyrrolidin-1-yl)carbonyl]-2-[3-(trifluormethyl)benzyl]-5,6,7,8-tetrahydro[1,2,4]triazolo[4,3-a]pyridin-3(2H)-on (Racemat)

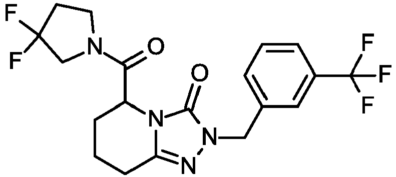

(5RS)-3-Oxo-2-[3-(trifluormethyl)benzyl]-2,3,5,6,7,8-hexahydro[1,2,4]triazolo[4,3-a]pyridin-5-carbonsäure -trifluoressigsäure (Racemat) (70.0 mg, 154 µmol) wurde in THF (2.6 ml) vorgelegt und 1-[Bis(dimethylamino)methylen]-1H-[1,2,3]triazolo[4,5-b]pyridin-1-ium-3-oxidhexafluorophosphat (76.0 mg, 200 µmol) und Triethylamin (110 µl, 770 µmol) wurden anschließend zugegeben. Nach 15 min Rühren bei Raumtemperatur wurde 3,3-Difluorpyrrolidinhydrochlorid (26.5 mg, 184 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 46.5 mg (70 % d. Th.) der Titelverbindung erhalten.
LC-MS (Methode 4): Rₜ = 0.86 min; MS (ESIpos): m/z = 431 [M+H]⁺
¹H-NMR (400 MHz, DMSO-d6) δ [ppm]: -0.150 (0.90), -0.008 (6.54), 0.008 (6.57), 0.146 (0.79), 1.730 (1.39), 2.012 (1.46), 2.327 (2.25), 2.366 (1.69), 2.523 (8.26), 2.567 (3.00), 2.590 (2.67), 2.609 (3.00), 2.670 (2.59), 2.710 (1.50), 3.537 (1.39), 3.556 (2.07), 3.567 (1.24), 3.671 (1.16), 3.703 (1.43), 3.738 (1.13), 3.770 (1.54), 3.807 (1.80), 3.894 (0.64), 3.913 (1.35), 3.938 (0.94), 3.991 (0.94), 4.180 (0.75), 4.206 (0.75), 4.766 (1.13), 4.783 (1.46), 4.791 (1.13), 4.849 (1.39), 4.931 (16.00), 7.506 (2.37), 7.525 (3.94), 7.572 (2.37), 7.591 (4.39), 7.615 (5.78), 7.648 (3.79), 7.667 (2.22).

### Beispiel 48

### (5RS)-5-[(3,3-Difluorpyrrolidin-1-yl)carbonyl]-2-(3-methoxybenzyl)-5,6,7,8-tetrahydro[1,2,4]triazolo[4,3-a]pyridin-3(2H)-on (Racemat)

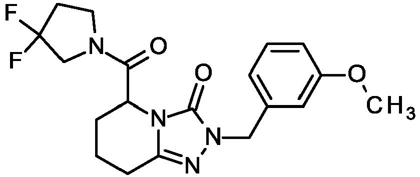

(5RS)-2-(3-Methoxybenzyl)-3-oxo-2,3,5,6,7,8-hexahydro[1,2,4]triazolo[4,3-a]pyridin-5-carbonsäure -trifluoressigsäure (Racemat) (70.0 mg, 168 µmol) wurde in THF (3.0 ml) vorgelegt und 1-[Bis(dimethylamino)methylen]-1H-[1,2,3]triazolo[4,5-b]pyridin-1-ium-3-oxidhexafluorophosphat (82.9 mg, 218 µmol) und Triethylamin (120 µl, 840 µmol) wurden anschließend zugegeben. Nach 15 min Rühren bei Raumtemperatur wurde 3,3-Difluorpyrrolidinhydrochlorid (28.9 mg, 201 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 47.1 mg (72 % d. Th.) der Titelverbindung erhalten.
LC-MS (Methode 4): Rₜ = 0.75 min; MS (ESIpos): m/z = 393 [M+H]⁺
¹H-NMR (400 MHz, DMSO-d6) δ [ppm]: 1.176 (0.43), 1.664 (0.83), 1.676 (0.96), 1.688 (1.03), 1.702 (1.17), 1.715 (1.34), 1.724 (1.33), 1.969 (0.80), 1.978 (1.09), 1.992 (1.10), 2.002 (1.22), 2.009 (1.19), 2.026 (0.96), 2.037 (0.90), 2.045 (0.92), 2.052 (0.93), 2.062 (0.88), 2.072 (0.99), 2.080 (0.69), 2.088 (0.63), 2.365 (0.44), 2.380 (0.75), 2.392 (0.57), 2.401 (0.79), 2.409 (0.90), 2.430 (0.86), 2.451 (0.65), 2.472 (0.43), 2.517 (3.02), 2.559 (2.35), 2.569 (2.87), 2.585 (2.57), 2.601 (2.46), 2.612 (1.31), 2.631 (0.48), 2.644 (0.74), 2.669 (0.40), 3.534 (3.05), 3.548 (3.83), 3.554 (3.85), 3.566 (2.82), 3.573 (2.57), 3.635 (0.72), 3.669 (1.32), 3.702 (1.44), 3.769 (1.59), 3.783 (1.02), 3.792 (0.87), 3.802 (1.67), 3.809 (1.48), 3.828 (0.66), 3.894 (0.62), 3.913 (1.29), 3.932 (0.71), 3.939 (0.91), 3.958 (0.48), 3.993 (0.80), 4.021 (0.54), 4.035 (0.71), 4.063 (0.43), 4.147 (0.47), 4.178 (0.68), 4.205 (0.69), 4.750 (1.09), 4.776 (16.00), 4.824 (1.07), 4.834 (1.23), 4.840 (1.38), 4.849 (1.03), 6.773 (6.87), 6.777 (7.30), 6.793 (3.43), 6.823 (2.14), 6.827 (2.29), 6.832 (1.64), 6.845 (2.46), 6.851 (2.14), 7.220 (2.49), 7.241 (5.00), 7.261 (2.64).

### Beispiel 49

### (5RS)-2-(3-Chlor-4-methoxybenzyl)-5-[(3,3-difluorpyrrolidin-1 -yl)carbonyl]-5,6,7,8-tetrahydro[1,2,4]triazolo[4,3-a]pyridin-3(2H)-on (Racemat)

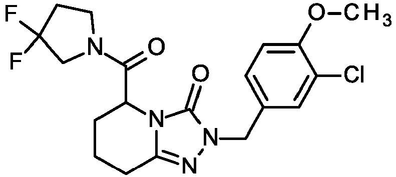

(5RS)-2-(3-Chlor-4-methoxybenzyl)-3-oxo-2,3,5,6,7,8-hexahydro[1,2,4]triazolo[4,3-a]pyridin-5-carbonsäure -trifluoressigsäure (Racemat) (70.0 mg, 155 µmol) wurde in THF (3.0 ml) vorgelegt und 1-[Bis(dimethylamino)methylen]-1H-[1,2,3]triazolo[4,5-b]pyridin-1-ium-3-oxidhexafluorophosphat (76.6 mg, 201 µmol) und Triethylamin (110 µl, 770 µmol) wurden anschließend zugegeben. Nach 15 min Rühren bei Raumtemperatur wurde 3,3-Difluorpyrrolidinhydrochlorid (26.7 mg, 186 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 36.0 mg (54 % d. Th.) der Titelverbindung erhalten.
LC-MS (Methode 4): Rₜ = 0.80 min; MS (ESIpos): m/z = 427 [M+H]⁺
¹H-NMR (400 MHz, DMSO-d6) δ [ppm]: 1.176 (0.66), 1.669 (0.43), 1.682 (0.48), 1.695 (0.55), 1.707 (0.61), 1.717 (0.61), 1.969 (0.48), 1.981 (0.50), 1.993 (0.57), 2.038 (0.43), 2.045 (0.41), 2.411 (0.41), 2.423 (0.41), 2.565 (1.07), 2.580 (1.02), 2.595 (1.07), 2.607 (0.68), 3.529 (0.46), 3.537 (0.50), 3.546 (0.75), 3.558 (0.84), 3.577 (0.41), 3.666 (0.48), 3.700 (0.55), 3.775 (0.68), 3.806 (1.00), 3.833 (16.00), 3.910 (0.55), 4.745 (4.55), 4.765 (0.50), 4.815 (0.46), 4.825 (0.55), 4.831 (0.59), 4.840 (0.43), 7.097 (1.68), 7.118 (2.94), 7.167 (1.52), 7.172 (1.59), 7.188 (0.84), 7.193 (0.93), 7.292 (2.30), 7.297 (2.12).

### Beispiel 50

### (5S)-5-(Pyrrolidin-1-ylcarbonyl)-2-{[6-(trifluormethyl)pyridin-3-yl]methyl}-2,5,6,7-tetrahydro-3H-pyrrolo[2,1-c][1,2,4]triazol-3-on (Enantiomer)

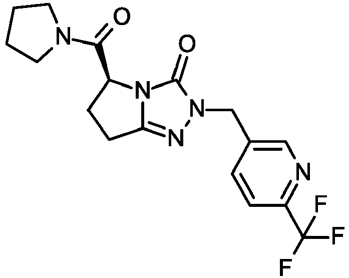

(5S)-3-Oxo-2-{[6-(trifluormethyl)pyridin-3-yl]methyl}-2,5,6,7-tetrahydro-3H-pyrrolo[2,1 - c][1,2,4]triazol-5-carbonsäure (Enantiomer) (100 mg, 305 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (151 mg, 396 µmol) und Triethylamin (170 µl, 1.2 mmol) zugegeben. Nach 15 min Rühren wurde Pyrrolidin (31 µl, 370 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 50.0 mg (43 % d. Th.) der Titelverbindung erhalten.
LC-MS (Methode 3): Rₜ = 1.13 min; MS (ESIpos): m/z = 382 [M+H]⁺
¹H-NMR (400 MHz, DMSO-d6) δ [ppm]: 1.769 (0.78), 1.784 (3.02), 1.802 (5.93), 1.819 (5.24), 1.835 (1.78), 1.889 (1.56), 1.905 (4.92), 1.922 (6.24), 1.939 (3.71), 1.957 (0.94), 2.367 (1.77), 2.389 (1.77), 2.406 (1.84), 2.416 (1.09), 2.426 (0.84), 2.651 (0.49), 2.674 (1.03), 2.692 (2.10), 2.714 (4.83), 2.724 (2.25), 2.736 (4.61), 2.744 (3.26), 2.754 (1.23), 2.780 (1.92), 2.810 (2.02), 2.832 (1.50), 3.268 (0.55), 3.285 (1.24), 3.298 (2.66), 3.334 (5.63), 3.351 (2.20), 3.364 (1.29), 3.380 (1.63), 3.397 (2.45), 3.404 (1.65), 3.415 (1.42), 3.422 (2.86), 3.439 (1.27), 3.641 (1.30), 3.657 (2.74), 3.666 (1.47), 3.674 (1.51), 3.682 (2.39), 3.699 (1.08), 4.923 (3.04), 4.930 (2.28), 4.944 (3.23), 4.950 (2.23), 5.007 (16.00), 7.902 (2.10), 7.923 (8.79), 7.933 (5.24), 7.957 (1.23), 8.673 (5.78).

Nachfolgende Analysen von Beispiel 175 haben gezeigt, dass die 3H-pyrrolo[2,1-c][1,2,4]triazol-3-on-Systeme unter Verwendung von HATU teilweise racemisieren und nicht mehr als reines Enantiomer vorliegen.

### Beispiel 51

### (5S)-2-(3-Chlor-4-fluorbenzyl)-5-(pyrrolidin-1 -ylcarbonyl)-2,5,6,7-tetrahydro-3H-pyrrolo[2,1 - c][1,2,4]triazol-3-on (Enantiomer)

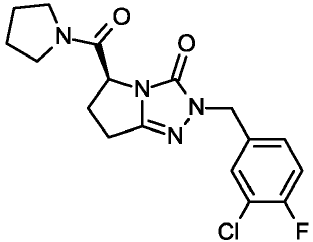

(5S)-2-(3-Chlor-4-fluorbenzyl)-3-oxo-2,5,6,7-tetrahydro-3H-pyrrolo[2,1-c][1,2,4]triazol-5-carbonsäure (Enantiomer) (100 mg, 321 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (159 mg, 417 µmol) und Triethylamin (180 µl, 1.3 mmol) zugegeben. Nach 15 min Rühren wurde Pyrrolidin (32 µl, 380 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 65.0 mg (56 % d. Th.) der Titelverbindung erhalten.
LC-MS (Methode 4): Rₜ = 0.73 min; MS (ESIpos): m/z = 365 [M+H]⁺
¹H-NMR (400 MHz, DMSO-d6) δ [ppm]: 1.768 (1.33), 1.784 (5.17), 1.800 (10.32), 1.817 (9.23), 1.833 (3.24), 1.888 (2.78), 1.904 (8.68), 1.921 (10.97), 1.938 (6.38), 1.956 (1.55), 2.327 (1.35), 2.358 (2.20), 2.365 (2.34), 2.379 (3.19), 2.385 (2.61), 2.396 (3.26), 2.407 (1.96), 2.671 (2.13), 2.690 (3.70), 2.712 (7.35), 2.721 (3.63), 2.731 (8.75), 2.739 (5.90), 2.766 (2.80), 2.773 (3.72), 2.803 (3.55), 2.825 (2.63), 2.847 (0.68), 3.265 (1.09), 3.282 (2.37), 3.331 (9.81), 3.349 (3.94), 3.361 (2.22), 3.378 (2.90), 3.395 (4.21), 3.402 (2.85), 3.419 (5.00), 3.437 (2.32), 3.640 (2.37), 3.657 (4.95), 3.665 (2.54), 3.674 (2.66), 3.681 (4.23), 3.698 (1.79), 4.778 (1.21), 4.818 (16.00), 4.823 (15.78), 4.864 (1.11), 4.916 (5.34), 4.923 (3.79), 4.937 (5.68), 4.944 (3.79), 7.250 (2.44), 7.255 (2.66), 7.262 (2.71), 7.271 (4.01), 7.276 (3.79), 7.283 (3.70), 7.289 (3.48), 7.377 (7.40), 7.401 (9.52), 7.422 (5.37), 7.453 (5.78), 7.458 (5.41), 7.471 (5.78), 7.477 (5.32).

Nachfolgende Analysen von Beispiel 175 haben gezeigt, dass die 3H-pyrrolo[2,1-c][1,2,4]triazol-3-on-Systeme unter Verwendung von HATU teilweise racemisieren und nicht mehr als reines Enantiomer vorliegen.

### Beispiel 52

### (5S)-2-[(6-Chlorpyridin-3-yl)methyl]-5-(pyrrolidin-1-ylcarbonyl)-2,5,6,7-tetrahydro-3H-pyrrolo[2,1-c][1,2,4]triazol-3-on (Enantiomer)

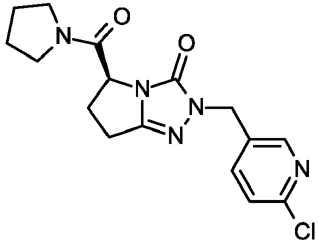

(5S)-2-[(6-Chlorpyridin-3-yl)methyl]-3-oxo-2,5,6,7-tetrahydro-3H-pyrrolo[2,1-c][1,2,4]triazol-5-carbonsäure (Enantiomer) (80.0 mg, 271 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (134 mg, 353 µmol) und Triethylamin (150 µl, 1.1 mmol) zugegeben. Nach 15 min Rühren wurde Pyrrolidin (27 µl, 330 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 30.0 mg (32 % d. Th.) der Titelverbindung erhalten.
LC-MS (Methode 3): Rₜ = 0.97 min; MS (ESIpos): m/z = 348 [M+H]⁺
¹H-NMR (400 MHz, DMSO-d6) δ [ppm]: -0.149 (0.44), 1.782 (2.91), 1.798 (5.45), 1.816 (4.87), 1.832 (1.60), 1.886 (1.48), 1.902 (4.67), 1.919 (5.91), 1.936 (3.46), 1.954 (0.86), 2.328 (0.66), 2.378 (1.73), 2.396 (1.70), 2.681 (1.98), 2.703 (4.30), 2.713 (2.17), 2.724 (4.48), 2.732 (3.11), 2.769 (1.81), 2.799 (1.89), 2.821 (1.40), 3.328 (6.29), 3.346 (2.24), 3.358 (1.33), 3.375 (1.71), 3.392 (2.30), 3.416 (2.65), 3.433 (1.15), 3.636 (1.17), 3.653 (2.62), 3.678 (2.16), 3.694 (0.97), 4.879 (16.00), 4.908 (2.88), 4.929 (3.09), 7.508 (5.17), 7.529 (6.17), 7.716 (3.34), 7.722 (3.44), 7.737 (2.73), 7.743 (2.78), 8.322 (5.32), 8.328 (5.20).

Nachfolgende Analysen von Beispiel 175 haben gezeigt, dass die 3H-pyrrolo[2,1-c][1,2,4]triazol-3-on-Systeme unter Verwendung von HATU teilweise racemisieren und nicht mehr als reines Enantiomer vorliegen.

### Beispiel 53

### (5RS)-2-[(5-Chlor-2-thienyl)methyl]-5-(pyrrolidin-1 -ylcarbonyl)-5,6,7,8-tetrahydro[1,2,4]triazolo[4,3-a]pyridin-3(2H)-on (Racemat)

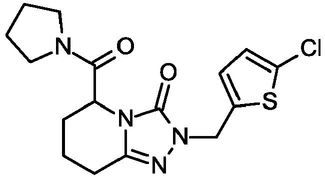

(5RS)-2-[(5-Chlor-2-thienyl)methyl]-3-oxo-2,3,5,6,7,8-hexahydro[1,2,4]triazolo[4,3-a]pyridin-5-carbonsäure -trifluoressigsäure (Racemat) (80.0 mg, 187 µmol) wurde in THF (2.0 ml) bei Raumtemperatur vorgelegt. Anschließend wurde 1-[Bis(dimethylamino)methylen]-1H-[1 ,2,3]triazolo[4,5-b]pyridin-1-ium-3-oxidhexafluorophosphat (92.4 mg, 243 µmol) und Triethylamin (130 µl, 940 µmol) zugegeben. Nach 15 min Rühren wurde Pyrrolidin (16.0 mg, 224 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 37.9 mg (55 % d. Th.) der Titelverbindung erhalten.
LC-MS (Methode 4): Rₜ = 0.75 min; MS (ESIpos): m/z = 367 [M+H]⁺
¹H-NMR (400 MHz, DMSO-d6) δ [ppm]: 0.008 (1.30), 1.667 (1.13), 1.679 (1.75), 1.702 (3.21), 1.711 (3.57), 1.723 (2.84), 1.753 (1.54), 1.769 (5.20), 1.787 (9.04), 1.804 (6.99), 1.821 (2.02), 1.889 (2.22), 1.906 (6.53), 1.923 (8.49), 1.939 (5.64), 1.958 (2.63), 1.970 (2.76), 1.982 (2.51), 1.991 (2.15), 2.001 (1.70), 2.017 (2.27), 2.027 (1.63), 2.033 (1.49), 2.042 (1.55), 2.052 (0.98), 2.068 (0.58), 2.078 (0.42), 2.327 (0.41), 2.564 (2.63), 2.579 (2.25), 2.592 (2.18), 2.604 (4.23), 2.617 (2.24), 2.634 (0.99), 2.646 (1.58), 2.659 (0.84), 2.669 (0.51), 2.865 (3.48), 3.219 (1.23), 3.236 (2.56), 3.248 (2.58), 3.265 (4.57), 3.282 (2.29), 3.333 (4.95), 3.345 (1.94), 3.350 (2.73), 3.362 (2.79), 3.380 (1.23), 3.428 (1.37), 3.445 (2.98), 3.453 (2.21), 3.462 (1.78), 3.470 (3.82), 3.487 (1.67), 3.586 (1.75), 3.602 (3.65), 3.611 (1.84), 3.619 (2.06), 3.627 (2.82), 3.644 (1.26), 4.707 (3.29), 4.717 (3.82), 4.722 (4.37), 4.732 (3.35), 4.872 (0.79), 4.914 (16.00), 4.957 (0.85), 6.898 (6.58), 6.908 (8.26), 6.973 (11.05), 6.982 (8.62).

### Beispiel 54

### (5RS)-2-[(5-Chlor-2-thienyl)methyl]-5-[(3,3-difluorpyrrolidin-1-yl)carbonyl]-5,6,7,8-tetrahydro[1,2,4]triazolo[4,3-a]pyridin-3(2H)-on (Racemat)

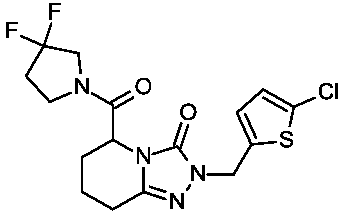

(5RS)-2-[(5-Chlor-2-thienyl)methyl]-3-oxo-2,3,5,6,7,8-hexahydro[1,2,4]triazolo[4,3-a]pyridin-5-carbonsäure -trifluoressigsäure (Racemat) (80.0 mg, 187 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (92.4 mg, 243 µmol) und Triethylamin (130 µl, 940 µmol) zugegeben. Nach 15 min Rühren wurde 3,3-Difluorpyrrolidinhydrochlorid (32.2 mg, 224 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 42.9 mg (57 % d. Th.) der Titelverbindung erhalten.
LC-MS (Methode 4): Rₜ = 0.80 min; MS (ESIpos): m/z = 403 [M+H]⁺
¹H-NMR (400 MHz, DMSO-d6) δ [ppm]: -0.008 (1.94), 1.718 (1.95), 1.977 (1.59), 1.987 (1.79), 2.032 (1.31), 2.049 (1.24), 2.068 (1.06), 2.327 (0.63), 2.359 (0.64), 2.377 (1.04), 2.406 (1.36), 2.427 (1.37), 2.447 (1.16), 2.569 (3.54), 2.580 (3.06), 2.594 (3.16), 2.610 (3.13), 2.622 (1.69), 2.653 (1.01), 2.669 (0.88), 3.528 (1.74), 3.540 (2.31), 3.548 (2.49), 3.559 (1.40), 3.567 (1.29), 3.626 (0.45), 3.660 (1.48), 3.694 (1.68), 3.731 (0.97), 3.767 (1.92), 3.800 (3.09), 3.818 (0.78), 3.884 (0.83), 3.902 (1.68), 3.928 (1.18), 3.947 (0.61), 3.984 (1.06), 4.012 (0.76), 4.025 (0.96), 4.054 (0.54), 4.138 (0.59), 4.170 (0.95), 4.195 (0.98), 4.226 (0.40), 4.725 (1.42), 4.740 (1.87), 4.749 (1.31), 4.799 (1.40), 4.815 (1.84), 4.824 (1.28), 4.880 (0.62), 4.922 (16.00), 4.964 (0.58), 6.901 (6.08), 6.911 (7.61), 6.975 (10.97), 6.984 (8.41).

### Beispiel 55

### (5S)-5-(Pyrrolidin-1-ylcarbonyl)-2-[3-(trifluormethyl)benzyl]-2,5,6,7-tetrahydro-3H-pyrrolo[2,1-c][1,2,4]triazol-3-on (Enantiomer)

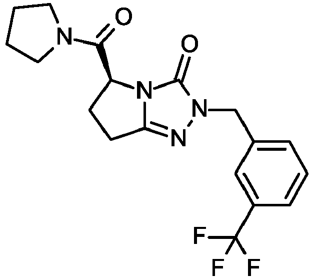

Methyl-(5S)-3-oxo-2-[3-(trifluormethyl)benzyl]-2,5,6,7-tetrahydro-3H-pyrrolo[2,1-c][1,2,4]triazol-5-carboxylat (Enantiomer) (129 mg, 378 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (187 mg, 491 µmol) und Triethylamin (160 µl, 1.1 mmol) zugegeben. Nach 15 min Rühren wurde Pyrrolidin (38 µl, 450 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 92.4 mg (64 % d. Th.) der Titelverbindung erhalten.
LC-MS (Methode 3): Rₜ = 1.42 min; MS (ESIpos): m/z = 381 [M+H]⁺
¹H-NMR (400 MHz, DMSO-d6) δ [ppm]: 1.766 (1.04), 1.782 (4.19), 1.799 (7.77), 1.816 (6.67), 1.832 (2.28), 1.887 (2.03), 1.903 (6.44), 1.920 (8.25), 1.937 (4.97), 1.954 (1.24), 2.349 (0.56), 2.360 (1.57), 2.365 (1.56), 2.380 (2.45), 2.387 (1.96), 2.397 (2.42), 2.408 (1.49), 2.417 (1.09), 2.650 (0.53), 2.672 (1.15), 2.691 (2.65), 2.712 (5.74), 2.722 (2.89), 2.733 (6.33), 2.740 (4.71), 2.749 (1.93), 2.767 (2.15), 2.774 (2.86), 2.781 (2.42), 2.797 (1.52), 2.804 (2.59), 2.818 (0.67), 2.826 (1.87), 2.848 (0.50), 3.265 (0.75), 3.282 (1.61), 3.295 (3.42), 3.330 (7.74), 3.348 (2.95), 3.360 (1.63), 3.377 (2.15), 3.394 (3.17), 3.401 (2.24), 3.412 (1.91), 3.419 (3.67), 3.436 (1.66), 3.642 (1.70), 3.659 (3.64), 3.667 (1.93), 3.676 (2.05), 3.684 (3.12), 3.701 (1.38), 4.883 (1.04), 4.923 (16.00), 4.929 (15.45), 4.946 (4.38), 4.953 (3.12), 4.969 (1.11), 7.540 (2.51), 7.559 (5.89), 7.578 (3.43), 7.597 (5.63), 7.616 (2.78), 7.637 (7.35), 7.650 (5.01), 7.669 (2.64).

Nachfolgende Analysen von Beispiel 175 haben gezeigt, dass die 3H-pyrrolo[2,1-c][1,2,4]triazol-3-on-Systeme unter Verwendung von HATU teilweise racemisieren und nicht mehr als reines Enantiomer vorliegen.

### Beispiel 56

### (5RS)-5-[(3,3-Difluorpyrrolidin-1 -yl)carbonyl]-2-(3-fluorbenzyl)-5,6,7,8-tetrahydro[1,2,4]triazolo[4,3-a]pyridin-3(2H)-on (Racemat)

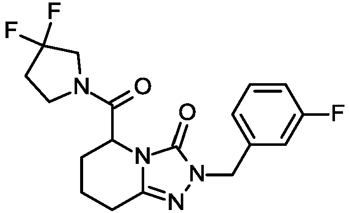

(5RS)-2-(3-Fluorbenzyl)-3-oxo-2,3,5,6,7,8-hexahydro[1,2,4]triazolo[4,3-a]pyridin-5-carbonsäurehydrochlorid (Racemat) (140 mg, 427 µmol) wurde in THF (4.4 m) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (211 mg, 555 µmol) und Triethylamin (300 µl, 2.1 mmol) zugegeben. Nach 15 min Rühren wurde 3,3-Difluorpyrrolidinhydrochlorid (73.6 mg, 513 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 103 mg (63 % d. Th.) der Titelverbindung erhalten.
LC-MS (Methode 3): Rₜ = 1.36 min; MS (ESIpos): m/z = 381 [M+H]⁺
¹H-NMR (400 MHz, DMSO-d6) δ [ppm]: -0.008 (2.16), 0.008 (2.37), 1.676 (0.97), 1.687 (0.91), 1.725 (1.22), 1.989 (1.10), 2.000 (1.15), 2.010 (1.23), 2.044 (0.92), 2.052 (0.94), 2.059 (0.96), 2.069 (0.86), 2.079 (0.71), 2.088 (0.73), 2.095 (0.65), 2.327 (0.76), 2.366 (0.86), 2.382 (0.86), 2.392 (0.62), 2.411 (0.99), 2.430 (0.96), 2.454 (0.79), 2.522 (2.93), 2.580 (2.11), 2.594 (2.22), 2.610 (2.37), 2.624 (1.23), 2.642 (0.57), 2.653 (0.79), 2.669 (1.02), 2.709 (0.62), 3.534 (1.02), 3.541 (1.18), 3.551 (1.70), 3.561 (1.83), 3.570 (1.12), 3.580 (1.01), 3.671 (1.12), 3.705 (1.23), 3.745 (0.68), 3.778 (1.44), 3.792 (0.76), 3.811 (2.30), 3.829 (0.58), 3.893 (0.57), 3.912 (1.20), 3.931 (0.68), 3.939 (0.83), 3.957 (0.42), 3.993 (0.75), 4.022 (0.50), 4.036 (0.68), 4.149 (0.44), 4.180 (0.66), 4.205 (0.68), 4.759 (1.01), 4.769 (1.15), 4.774 (1.33), 4.783 (0.99), 4.843 (16.00), 4.857 (1.28), 7.006 (1.90), 7.030 (1.98), 7.057 (2.87), 7.076 (3.29), 7.090 (1.01), 7.106 (1.95), 7.112 (1.82), 7.128 (1.09), 7.134 (1.04), 7.358 (1.62), 7.374 (1.96), 7.378 (2.58), 7.393 (2.53), 7.413 (1.28).

### Beispiel 57

### (5RS)-2-[2-(4-Methylphenyl)ethyl]-5-(pyrrolidin-1-ylcarbonyl)-5,6,7,8-tetrahydro[1,2,4]triazolo[4,3-a]pyridin-3(2H)-on (Racemat)

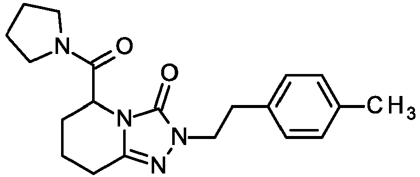

(5RS)-2-[2-(4-Methylphenyl)ethyl]-3-oxo-2,3,5,6,7,8-hexahydro[1,2,4]triazolo[4,3-a]pyridin-5-carbonsäure (Racemat) (82.0 mg, 100 % Reinheit, 272 µmol) wurde in THF (2.8 ml) vorgelegt und 1-[Bis(dimethylamino)methylen]-1H-[1,2,3]triazolo[4,5-b]pyridin-1-ium-3-oxidhexafluorophosphat (135 mg, 354 µmol) und Triethylamin (190 µl, 1.4 mmol) wurden anschließend zugegeben. Nach 15 min Rühren bei Raumtemperatur wurde Pyrrolidin (27 µl, 330 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 77.0 mg (80 % d. Th.) der Titelverbindung erhalten.
LC-MS (Methode 3): Rₜ = 1.42 min; MS (ESIpos): m/z = 355 [M+H]⁺
¹H-NMR (400 MHz, DMSO-d6) δ [ppm]: -0.150 (0.41), -0.008 (3.12), 0.008 (3.21), 1.714 (0.99), 1.765 (1.02), 1.781 (1.70), 1.798 (1.31), 1.884 (0.49), 1.899 (1.27), 1.916 (1.68), 1.934 (1.12), 1.961 (0.65), 1.998 (0.53), 2.257 (9.18), 2.327 (0.70), 2.366 (0.43), 2.613 (0.80), 2.669 (0.79), 2.709 (0.41), 2.849 (0.94), 2.868 (1.96), 2.887 (1.03), 3.233 (0.51), 3.263 (0.94), 3.328 (1.09), 3.358 (0.59), 3.440 (0.56), 3.465 (0.70), 3.588 (0.61), 3.613 (0.58), 3.729 (0.63), 3.748 (0.96), 3.766 (0.58), 3.778 (0.91), 3.798 (0.57), 4.660 (0.71), 4.675 (0.82), 4.685 (0.62), 7.085 (16.00).

### Beispiel 58

### (5RS)-2-(2-Methylbenzyl)-5-(pyrrolidin-1-ylcarbonyl)-5,6,7,8-tetrahydro[1,2,4]triazolo[4,3-a]pyridin-3(2H)-on (Racemat)

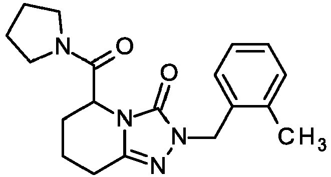

(5RS)-2-(2-Methylbenzyl)-3-oxo-2,3,5,6,7,8-hexahydro[1,2,4]triazolo[4,3-a]pyridin-5-carbonsäure (Racemat) (80.0 mg, 278 µmol) wurde in THF (3.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (138 mg, 362 µmol) und Triethylamin (78 µl, 560 µmol) zugegeben. Nach 15 min Rühren wurde Pyrrolidin (28 µl, 330 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 42.0 mg (44 % d. Th.) der Titelverbindung erhalten.
LC-MS (Methode 3): Rₜ = 1.27 min; MS (ESIpos): m/z = 341 [M+H]⁺
¹H-NMR (400 MHz, DMSO-d6) δ [ppm]: -0.008 (0.91), 0.008 (0.90), 1.110 (0.57), 1.680 (0.58), 1.703 (1.15), 1.712 (1.23), 1.723 (0.96), 1.756 (0.51), 1.772 (1.74), 1.790 (3.06), 1.807 (2.39), 1.823 (0.69), 1.892 (0.70), 1.909 (2.16), 1.925 (2.75), 1.942 (1.76), 1.959 (0.74), 1.973 (0.55), 1.984 (0.98), 1.996 (1.12), 2.008 (0.75), 2.023 (0.80), 2.034 (0.55), 2.040 (0.50), 2.049 (0.52), 2.293 (16.00), 2.475 (0.40), 2.574 (0.74), 2.585 (1.41), 2.598 (0.79), 2.627 (0.56), 3.226 (0.42), 3.243 (0.89), 3.255 (0.90), 3.272 (1.59), 3.290 (0.88), 3.321 (1.26), 3.340 (1.67), 3.352 (0.61), 3.357 (0.85), 3.369 (0.93), 3.387 (0.43), 3.436 (0.46), 3.454 (1.02), 3.461 (0.74), 3.471 (0.62), 3.479 (1.32), 3.496 (0.58), 3.594 (0.60), 3.610 (1.23), 3.619 (0.61), 3.627 (0.71), 3.635 (0.96), 3.652 (0.42), 4.727 (1.13), 4.736 (1.29), 4.742 (1.50), 4.751 (1.15), 4.778 (8.67), 7.070 (1.24), 7.088 (2.10), 7.117 (0.63), 7.125 (0.62), 7.131 (1.10), 7.139 (1.42), 7.157 (1.70), 7.166 (5.62), 7.180 (1.21), 7.184 (0.89).

### Beispiel 59

### (5RS)-2-[(2-Chlorpyridin-4-yl)methyl]-5-(pyrrolidin-1-ylcarbonyl)-5,6,7,8-tetrahydro[1,2,4]triazolo[4,3-a]pyridin-3(2H)-on (Racemat)

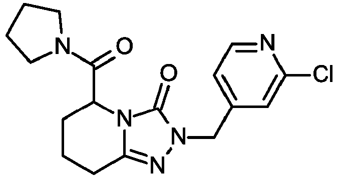

(5RS)-2-[(2-Chlorpyridin-4-yl)methyl]-3-oxo-2,3,5,6,7,8-hexahydro[1,2,4]triazolo[4,3-a]pyridin-5-carbonsäure -trifluoressigsäure (Racemat) (110 mg, 94 % Reinheit, 245 µmol) wurde in THF (2.5 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (121 mg, 318 µmol) und Triethylamin (170 µl, 1.2 mmol) zugegeben. Nach 15 min Rühren wurde Pyrrolidin (25 µl, 290 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 10.0 mg (11 % d. Th.) der Titelverbindung erhalten.
LC-MS (Methode 4): Rₜ = 0.60 min; MS (ESIpos): m/z = 362 [M+H]⁺
¹H-NMR (500 MHz, DMSO-d6) δ [ppm]: -0.120 (0.49), -0.007 (3.51), 0.006 (3.30), 0.117 (0.49), 1.499 (0.65), 1.693 (1.62), 1.740 (2.32), 1.748 (2.11), 1.766 (2.32), 1.778 (6.81), 1.792 (10.92), 1.806 (8.00), 1.820 (2.22), 1.901 (1.95), 1.912 (4.76), 1.925 (6.22), 1.939 (4.00), 1.952 (1.30), 1.997 (1.08), 2.015 (2.59), 2.033 (2.27), 2.040 (1.78), 2.046 (1.73), 2.053 (1.89), 2.062 (1.95), 2.068 (2.05), 2.361 (2.92), 2.365 (2.11), 2.439 (3.14), 2.518 (8.27), 2.522 (6.54), 2.559 (3.51), 2.571 (2.81), 2.580 (2.81), 2.592 (2.27), 2.617 (2.11), 2.627 (4.76), 2.635 (5.08), 2.650 (1.14), 2.660 (1.84), 2.865 (8.59), 3.242 (1.73), 3.256 (3.57), 3.266 (3.41), 3.280 (6.65), 3.338 (3.95), 3.353 (5.62), 3.367 (3.19), 3.376 (3.41), 3.390 (1.62), 3.449 (1.51), 3.463 (3.41), 3.469 (2.54), 3.483 (4.16), 3.497 (1.95), 3.598 (1.89), 3.611 (3.57), 3.625 (2.22), 3.631 (2.92), 3.645 (1.51), 3.700 (4.27), 4.766 (3.68), 4.772 (4.00), 4.778 (4.59), 4.785 (3.57), 4.833 (0.92), 4.878 (0.86), 4.912 (16.00), 4.948 (0.86), 7.209 (5.73), 7.219 (6.11), 7.319 (10.32), 8.342 (0.54), 8.368 (9.30), 8.378 (9.30).

### Beispiel 60

### (5RS)-2-[(4-Methyl-1,2,5-oxadiazol-3-yl)methyl]-5-(pyrrolidin-1-ylcarbonyl)-5,6,7,8-tetrahydro[1,2,4]triazolo[4,3-a]pyridin-3(2H)-on (Racemat)

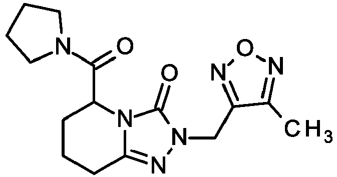

(5RS)-2-[(4-Methyl-1,2,5-oxadiazol-3-yl)methyl]-3-oxo-2,3,5,6,7,8-hexahydro[1,2,4]triazolo[4,3-a]pyridin-5-carbonsäure (Racemat) (91.0 mg, 326 µmol) wurde in THF (3.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (161 mg, 424 µmol) und Triethylamin (91 µl, 650 µmol) zugegeben. Nach 15 min Rühren wurde Pyrrolidin (33 µl, 390 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 14.0 mg (13 % d. Th.) der Titelverbindung erhalten.
LC-MS (Methode 3): Rₜ = 0.97 min; MS (ESIpos): m/z = 333 [M+H]⁺
¹H-NMR (400 MHz, DMSO-d6) δ [ppm]: -0.008 (1.20), 0.008 (1.14), 1.711 (0.58), 1.721 (0.49), 1.772 (1.12), 1.790 (2.07), 1.807 (1.67), 1.823 (0.52), 1.891 (0.52), 1.908 (1.52), 1.924 (1.83), 1.941 (1.16), 1.957 (0.46), 1.998 (0.83), 2.023 (0.48), 2.289 (16.00), 2.523 (1.52), 2.565 (0.60), 2.582 (0.53), 2.593 (0.89), 2.606 (0.52), 3.238 (0.56), 3.251 (0.62), 3.268 (1.07), 3.285 (0.55), 3.328 (1.23), 3.345 (0.59), 3.357 (0.61), 3.449 (0.70), 3.456 (0.52), 3.474 (0.90), 3.490 (0.41), 3.588 (0.43), 3.604 (0.83), 3.613 (0.44), 3.621 (0.47), 3.630 (0.66), 4.739 (0.79), 4.748 (0.87), 4.754 (1.02), 4.763 (0.76), 5.087 (4.25), 5.092 (4.25).

### Beispiel 61

### (5RS)-2-[(5-Methyl-1,2-oxazol-3-yl)methyl]-5-(pyrrolidin-1-ylcarbonyl)-5,6,7,8-tetrahydro[1,2,4]triazolo[4,3-a]pyridin-3(2H)-on (Racemat)

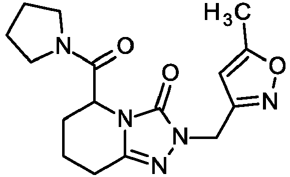

(5RS)-2-[(5-Methyl-1,2-oxazol-3-yl)methyl]-3-oxo-2,3,5,6,7,8-hexahydro[1,2,4]triazolo[4,3-a]pyridin-5-carbonsäure (Racemat) (133 mg, 94 % Reinheit, 449 µmol) wurde in THF (4.7 ml,) vorgelegt und 1-[Bis(dimethylamino)methylen]-1H-[1,2,3]triazolo[4,5-b]pyridin-1-ium-3-oxidhexafluorophosphat (222 mg, 584 µmol) und Triethylamin (310 µl, 2.2 mmol) wurden anschließend zugegeben. Nach 15 min Rühren bei Raumtemperatur wurde Pyrrolidin (45 µl, 540 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 18.0 mg (12 % d. Th.) der Titelverbindung erhalten.
LC-MS (Methode 4): Rₜ = 0.55 min; MS (ESIpos): m/z = 332 [M+H]⁺
¹H-NMR (500 MHz, DMSO-d6) δ [ppm]: -0.007 (0.94), 0.006 (0.97), 1.179 (0.41), 1.237 (0.58), 1.717 (1.18), 1.763 (0.56), 1.776 (2.23), 1.790 (3.84), 1.804 (2.83), 1.818 (0.75), 1.899 (0.86), 1.912 (2.55), 1.925 (3.24), 1.939 (2.02), 1.952 (0.90), 1.981 (0.90), 2.007 (0.47), 2.015 (0.60), 2.027 (0.77), 2.036 (0.64), 2.042 (0.66), 2.048 (0.64), 2.201 (1.16), 2.361 (1.31), 2.370 (15.57), 2.371 (16.00), 2.518 (3.75), 2.522 (3.17), 2.567 (0.92), 2.591 (0.79), 2.600 (1.50), 2.610 (0.86), 2.635 (1.63), 2.865 (1.80), 3.232 (0.54), 3.245 (1.12), 3.255 (1.05), 3.269 (1.84), 3.283 (1.07), 3.342 (2.62), 3.352 (1.14), 3.356 (1.29), 3.366 (1.44), 3.380 (0.69), 3.444 (0.60), 3.457 (1.24), 3.463 (0.90), 3.471 (0.75), 3.477 (1.59), 3.491 (0.71), 3.594 (0.69), 3.607 (1.46), 3.614 (0.77), 3.621 (0.86), 3.628 (1.16), 3.641 (0.51), 4.717 (1.24), 4.724 (1.48), 4.729 (1.63), 4.736 (1.35), 4.781 (0.66), 4.813 (5.96), 4.819 (6.01), 4.851 (0.64), 6.062 (4.18).

### Beispiel 62

### (5RS)-2-[(1-Methyl-1H-benzimidazol-2-yl)methyl]-5-(pyrrolidin-1-ylcarbonyl)-5,6,7,8-tetrahydro[1,2,4]triazolo[4,3-a]pyridin-3(2H)-on (Racemat)

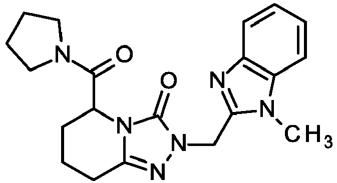

(5RS)-2-[(1-Methyl-1H-benzimidazol-2-yl)methyl]-3-oxo-2,3,5,6,7,8-hexahydro[1,2,4]triazolo[4,3-a]pyridin-5-carbonsäure (Racemat) (120 mg, 367 µmol) wurde in DMF (2.8 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (181 mg, 477 µmol) und Triethylamin (200 µl, 1.5 mmol) zugegeben. Nach 15 min Rühren wurde Pyrrolidin (37 µl, 440 µmol) zugegeben und das Reaktionsgemisch über Nacht bei Raumtemperatur gerührt. Es wurde erneut 1-[Bis(dimethylamino)methylen]-1H-[1,2,3]triazolo[4,5-b]pyridin-1-ium-3-oxidhexafluorophosphat (139 mg, 367 µmol) und Pyrrolidin (31 µl, 367 µmol) zugegeben und 2 Stunden bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 46.0 mg (33 % d. Th.) der Titelverbindung erhalten.
LC-MS (Methode 3): Rₜ = 0.83 min; MS (ESIpos): m/z = 381 [M+H]⁺
¹H-NMR (400 MHz, DMSO-d6) δ [ppm]: -0.008 (0.51), 0.008 (0.44), 1.653 (0.44), 1.667 (0.48), 1.677 (0.52), 1.690 (0.67), 1.702 (0.81), 1.712 (0.59), 1.725 (0.41), 1.777 (1.23), 1.795 (2.26), 1.812 (1.88), 1.829 (0.60), 1.897 (0.57), 1.914 (1.73), 1.931 (2.23), 1.947 (1.48), 1.964 (0.58), 1.977 (0.48), 1.987 (0.82), 1.997 (1.01), 2.006 (0.73), 2.022 (0.56), 2.033 (0.46), 2.040 (0.44), 2.048 (0.42), 2.518 (1.12), 2.566 (1.13), 2.578 (0.64), 2.608 (0.40), 3.245 (0.71), 3.258 (0.74), 3.275 (1.21), 3.292 (0.62), 3.324 (0.72), 3.342 (1.30), 3.354 (0.50), 3.360 (0.67), 3.372 (0.73), 3.459 (0.82), 3.466 (0.60), 3.476 (0.51), 3.483 (1.02), 3.501 (0.45), 3.604 (0.47), 3.620 (1.00), 3.628 (0.51), 3.637 (0.58), 3.645 (0.78), 3.755 (16.00), 4.749 (0.91), 4.758 (1.02), 4.764 (1.18), 4.772 (0.87), 5.072 (1.17), 5.111 (3.74), 5.139 (3.71), 5.178 (1.17), 7.172 (0.65), 7.175 (0.66), 7.192 (1.54), 7.209 (1.33), 7.212 (1.19), 7.233 (1.09), 7.236 (1.14), 7.253 (1.60), 7.271 (0.78), 7.273 (0.68), 7.524 (1.79), 7.544 (1.56), 7.582 (1.63), 7.602 (1.52).

### Beispiel 63

### (5RS)-2-[4-Methoxy-3-(trifluormethyl)benzyl]-5-(pyrrolidin-1-ylcarbonyl)-5,6,7,8-tetrahydro[1,2,4]triazolo[4,3-a]pyridin-3(2H)-on (Racemat)

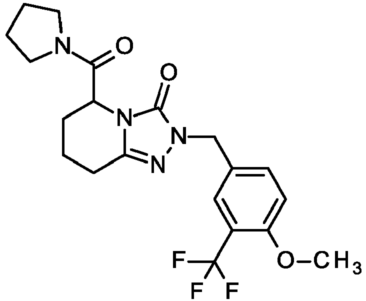

(5RS)-2-[4-Methoxy-3-(trifluormethyl)benzyl]-3-oxo-2,3,5,6,7,8-hexahydro[1,2,4]triazolo[4,3-a]pyridin-5-carbonsäure (Racemat) (75.0 mg, 202 µmol) wurde in THF (2.3 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (99.8 mg, 263 µmol) und Triethylamin (140 µl, 1.0 mmol) zugegeben. Nach 15 min Rühren wurde Pyrrolidin (20 µl, 240 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 40.0 mg (47 % d. Th.) der Titelverbindung erhalten.
LC-MS (Methode 4): Rₜ = 0.81 min; MS (ESIpos): m/z = 425 [M+H]⁺
¹H-NMR (400 MHz, DMSO-d6) δ [ppm]: -0.008 (1.02), 0.008 (0.93), 1.674 (0.45), 1.697 (0.88), 1.708 (0.99), 1.719 (0.77), 1.755 (0.43), 1.773 (1.40), 1.790 (2.47), 1.807 (1.91), 1.824 (0.55), 1.892 (0.61), 1.909 (1.78), 1.925 (2.25), 1.942 (1.58), 1.960 (0.51), 1.967 (0.47), 1.978 (0.78), 1.990 (0.76), 2.005 (0.53), 2.020 (0.62), 2.030 (0.44), 2.036 (0.41), 2.046 (0.43), 2.073 (0.94), 2.519 (1.40), 2.561 (0.63), 2.576 (0.59), 2.588 (1.12), 2.600 (0.62), 2.630 (0.44), 2.865 (0.45), 3.240 (0.73), 3.253 (0.72), 3.269 (1.28), 3.287 (0.68), 3.322 (1.06), 3.341 (1.35), 3.352 (0.53), 3.358 (0.70), 3.370 (0.77), 3.449 (0.83), 3.457 (0.61), 3.467 (0.49), 3.474 (1.05), 3.491 (0.46), 3.592 (0.48), 3.608 (1.00), 3.616 (0.50), 3.625 (0.57), 3.633 (0.77), 3.871 (16.00), 4.726 (0.91), 4.735 (1.03), 4.741 (1.21), 4.750 (0.90), 4.807 (4.91), 7.227 (1.60), 7.248 (1.89), 7.477 (1.19), 7.498 (1.06), 7.519 (2.24).

### Beispiel 64

### (5RS)-2-[(1-Methyl-1H-indazol-5-yl)methyl]-5-(pyrrolidin-1-ylcarbonyl)-5,6,7,8-tetrahydro[1,2,4]triazolo[4,3-a]pyridin-3(2H)-on (Racemat)

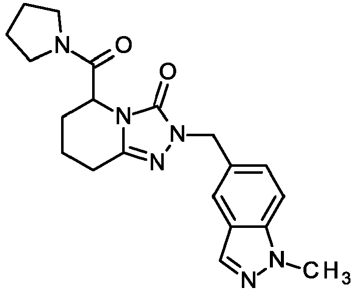

(5RS)-2-[(1-Methyl-1H-indazol-5-yl)methyl]-3-oxo-2,3,5,6,7,8-hexahydro[1,2,4]triazolo[4,3-a]pyridin-5-carbonsäure (Racemat) (65.0 mg, 199 µmol) wurde in THF (2.3 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (98.2 mg, 258 µmol) und Triethylamin (140 µl, 990 µmol) zugegeben. Nach 15 min Rühren wurde Pyrrolidin (20 µl, 240 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 26.0 mg (34 % d. Th.) der Titelverbindung erhalten.
LC-MS (Methode 3): Rₜ = 1.04 min; MS (ESIpos): m/z = 381 [M+H]⁺
¹H-NMR (400 MHz, DMSO-d6) δ [ppm]: -0.008 (1.76), 0.008 (1.67), 1.681 (0.57), 1.691 (0.92), 1.704 (1.22), 1.717 (0.94), 1.775 (1.31), 1.792 (2.33), 1.809 (1.80), 1.826 (0.53), 1.894 (0.59), 1.911 (1.69), 1.928 (2.24), 1.945 (1.49), 1.962 (0.71), 1.972 (0.74), 1.984 (0.67), 1.993 (0.54), 2.007 (0.47), 2.018 (0.55), 2.028 (0.45), 2.043 (0.42), 2.072 (0.79), 2.523 (1.39), 2.567 (0.67), 2.579 (1.17), 2.591 (0.61), 2.621 (0.45), 2.865 (0.91), 3.244 (0.73), 3.257 (0.73), 3.273 (1.25), 3.291 (0.83), 3.331 (0.80), 3.349 (1.31), 3.361 (0.51), 3.367 (0.68), 3.379 (0.74), 3.456 (0.79), 3.463 (0.57), 3.473 (0.48), 3.480 (1.00), 3.497 (0.44), 3.597 (0.45), 3.614 (0.97), 3.622 (0.48), 3.631 (0.54), 3.639 (0.75), 4.021 (16.00), 4.723 (0.86), 4.732 (0.98), 4.739 (1.17), 4.747 (0.84), 4.841 (0.57), 4.879 (2.97), 4.894 (2.98), 4.932 (0.56), 7.280 (1.34), 7.284 (1.16), 7.302 (1.36), 7.305 (1.54), 7.581 (1.86), 7.604 (4.27), 8.009 (2.76).

### Beispiel 65

### (5RS)-2-[2,5-Bis(trifluormethyl)benzyl]-5-(pyrrolidin-1-ylcarbonyl)-5,6,7,8-tetrahydro[1,2,4]triazolo[4,3-a]pyridin-3(2H)-on (Racemat)

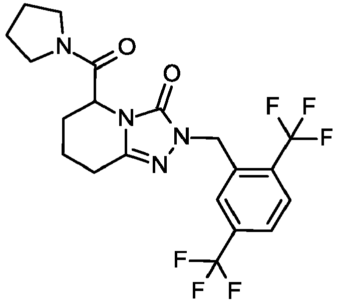

(5RS)-2-[2,5-Bis(trifluormethyl)benzyl]-3-oxo-2,3,5,6,7,8-hexahydro[1,2,4]triazolo[4,3-a]pyridin-5-carbonsäure (Racemat) (50.0 mg, 122 µmol) wurde in THF (2.0 ml) vorgelegt und 1-[Bis(dimethylamino)methylen]-1H-[1,2,3]triazolo[4,5-b]pyridin-1-ium-3-oxidhexafluorophosphat (60.4 mg, 159 µmol) und Triethylamin (51 µl, 370 µmol) wurden anschließend zugegeben. Nach 15 min Rühren bei Raumtemperatur wurde Pyrrolidin (10.4 mg, 147 µmol) zugegeben und das Reaktionsgemisch für 48 h bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 18.2 mg (32 % d. Th.) der Titelverbindung erhalten.
LC-MS (Methode 3): Rₜ = 1.72 min; MS (ESIpos): m/z = 463 [M+H]⁺
¹H-NMR (400 MHz, DMSO-d6) δ [ppm]: -0.149 (0.98), -0.008 (11.17), 0.008 (8.68), 0.146 (1.03), 1.697 (1.57), 1.707 (1.63), 1.735 (2.71), 1.756 (2.33), 1.773 (5.59), 1.790 (9.06), 1.807 (6.83), 1.824 (1.95), 1.893 (2.33), 1.909 (6.13), 1.926 (7.38), 1.943 (4.39), 1.960 (1.19), 2.025 (3.74), 2.036 (3.91), 2.078 (1.46), 2.328 (1.79), 2.366 (1.14), 2.524 (6.94), 2.566 (3.04), 2.577 (2.82), 2.592 (2.12), 2.614 (2.06), 2.625 (3.74), 2.638 (2.17), 2.670 (3.15), 2.710 (1.30), 2.865 (3.74), 3.230 (1.25), 3.247 (2.60), 3.260 (2.71), 3.276 (4.99), 3.338 (5.32), 3.356 (2.71), 3.368 (2.82), 3.385 (1.25), 3.441 (1.46), 3.458 (2.98), 3.466 (2.17), 3.483 (3.74), 3.501 (1.68), 3.594 (1.74), 3.611 (3.42), 3.627 (2.01), 3.635 (2.77), 3.652 (1.08), 3.700 (1.19), 3.730 (1.41), 4.749 (0.49), 4.785 (3.15), 4.794 (3.53), 4.800 (4.34), 4.808 (3.04), 5.077 (16.00), 7.654 (7.27), 7.917 (2.98), 7.938 (4.56), 8.013 (6.40), 8.034 (4.23).

### Beispiel 66

### (5RS)-2-[4-Fluor-3-(trifluormethyl)benzyl]-5-(pyrrolidin-1 -ylcarbonyl)-5,6,7,8-tetrahydro[1,2,4]triazolo[4,3-a]pyridin-3(2H)-on (Racemat)

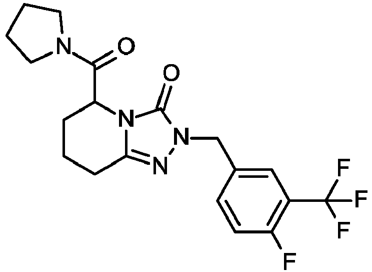

(5RS)-2-[4-Fluor-3-(trifluormethyl)benzyl]-3-oxo-2,3,5,6,7,8-hexahydro[1,2,4]triazolo[4,3-a]pyridin-5-carbonsäure (Racemat) (50.0 mg, 139 µmol) wurde in THF (2.0 ml) vorgelegt und 1-[Bis(dimethylamino)methylen]-1H-[1,2,3]triazolo[4,5-b]pyridin-1-ium-3-oxidhexafluorophosphat (68.8 mg, 181 µmol) und Triethylamin (58 µl, 420 µmol) wurden anschließend zugegeben. Nach 15 min Rühren bei Raumtemperatur wurde Pyrrolidin (11.9 mg, 167 µmol) zugegeben und das Reaktionsgemisch über Nacht bei Raumtemperatur gerührt. Es wurden erneut 1-[Bis(dimethylamino)methylen]-1H-[1,2,3]triazolo[4,5-b]pyridin-1-ium-3-oxidhexafluorophosphat (68.8 mg, 181 µmol), Pyrrolidin (11.9 mg, 167 µmol) und Triethylamin (58 µl, 420 µmol) zugegeben und 48 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 25.6 mg (45 % d. Th.) der Titelverbindung erhalten.
LC-MS (Methode 3): Rₜ = 1.53 min; MS (ESIpos): m/z = 413 [M+H]⁺
¹H-NMR (400 MHz, DMSO-d6) δ [ppm]: -0.149 (0.40), -0.008 (3.74), 0.008 (3.05), 0.146 (0.40), 1.662 (0.79), 1.673 (1.11), 1.686 (1.26), 1.707 (1.77), 1.716 (2.14), 1.726 (1.68), 1.740 (1.04), 1.754 (1.26), 1.773 (3.89), 1.790 (6.75), 1.807 (5.19), 1.824 (1.46), 1.892 (1.79), 1.909 (4.77), 1.925 (5.75), 1.942 (3.51), 1.958 (1.44), 1.982 (1.14), 1.992 (2.02), 2.004 (2.51), 2.014 (1.72), 2.030 (1.46), 2.041 (1.16), 2.048 (1.16), 2.056 (1.11), 2.065 (0.58), 2.323 (0.49), 2.327 (0.67), 2.332 (0.47), 2.366 (0.58), 2.523 (2.42), 2.558 (2.05), 2.572 (1.65), 2.588 (1.58), 2.600 (2.88), 2.613 (1.65), 2.630 (0.75), 2.642 (1.14), 2.654 (0.60), 2.665 (0.58), 2.669 (0.72), 2.674 (0.53), 2.710 (0.63), 2.865 (3.96), 3.225 (0.96), 3.242 (2.02), 3.255 (1.96), 3.272 (3.58), 3.289 (2.11), 3.340 (3.82), 3.351 (1.54), 3.357 (1.98), 3.369 (2.14), 3.387 (0.93), 3.435 (1.04), 3.452 (2.28), 3.460 (1.63), 3.470 (1.35), 3.477 (2.86), 3.494 (1.25), 3.591 (1.32), 3.607 (2.65), 3.615 (1.33), 3.624 (1.53), 3.632 (2.07), 3.649 (0.95), 4.741 (2.49), 4.750 (2.68), 4.756 (3.18), 4.765 (2.39), 4.904 (16.00), 7.478 (1.72), 7.500 (3.12), 7.526 (3.04), 7.561 (1.60), 7.566 (1.77), 7.573 (1.82), 7.579 (2.07), 7.587 (1.23), 7.595 (0.98), 7.600 (1.00), 7.669 (2.53), 7.674 (2.37), 7.687 (2.60).

### Beispiel 67

### (5RS)-2-[3-Fluor-4-(trifluormethoxy)benzyl]-5-(pyrrolidin-1-ylcarbonyl)-5,6,7,8-tetrahydro[1,2,4]triazolo[4,3-a]pyridin-3(2H)-on (Racemat)

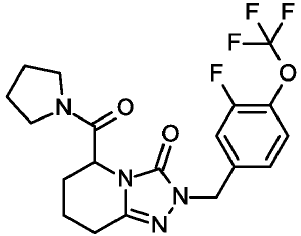

(5RS)-2-[3-Fluor-4-(trifluormethoxy)benzyl]-3-oxo-2,3,5,6,7,8-hexahydro[1,2,4]triazolo[4,3-a]pyridin-5-carbonsäure (Racemat) (80.0 mg, 213 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (105 mg, 277 µmol) und Triethylamin (89 µl, 640 µmol) zugegeben. Nach 15 min Rühren wurde Pyrrolidin (21 µl, 260 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 45.0 mg (49 % d. Th.) der Titelverbindung erhalten.
LC-MS (Methode 4): Rₜ = 0.88 min; MS (ESIpos): m/z = 429 [M+H]⁺
¹H-NMR (400 MHz, DMSO-d6) δ [ppm]: -0.008 (1.37), 0.008 (1.36), 1.662 (0.74), 1.669 (0.77), 1.681 (1.08), 1.695 (1.22), 1.704 (1.35), 1.717 (1.75), 1.728 (2.05), 1.737 (1.56), 1.757 (1.39), 1.774 (4.00), 1.791 (6.45), 1.809 (4.96), 1.818 (1.10), 1.826 (1.95), 1.836 (1.76), 1.844 (0.70), 1.854 (0.69), 1.893 (1.41), 1.910 (4.09), 1.926 (5.10), 1.943 (3.12), 1.960 (1.11), 1.979 (0.73), 1.993 (1.14), 2.003 (2.00), 2.015 (2.49), 2.024 (1.73), 2.040 (1.41), 2.051 (1.14), 2.059 (1.12), 2.066 (1.07), 2.074 (0.68), 2.559 (1.84), 2.570 (1.70), 2.584 (1.52), 2.599 (1.51), 2.611 (2.77), 2.623 (1.59), 2.641 (0.71), 2.653 (1.09), 2.665 (0.70), 3.095 (0.74), 3.232 (0.86), 3.248 (1.81), 3.261 (1.80), 3.278 (3.18), 3.295 (1.57), 3.329 (1.65), 3.346 (3.24), 3.358 (1.22), 3.364 (1.70), 3.376 (1.90), 3.394 (0.86), 3.441 (0.95), 3.459 (2.08), 3.466 (1.55), 3.476 (1.27), 3.484 (2.68), 3.501 (1.19), 3.593 (1.23), 3.610 (2.48), 3.618 (1.30), 3.626 (1.47), 3.634 (1.92), 3.651 (0.90), 4.747 (2.31), 4.756 (2.57), 4.761 (3.04), 4.771 (2.27), 4.879 (16.00), 7.160 (2.64), 7.182 (2.97), 7.303 (2.85), 7.308 (2.71), 7.331 (2.93), 7.336 (2.75), 7.537 (1.68), 7.557 (3.12), 7.577 (1.50).

### Beispiel 68

### (5RS)-2-{[3-Chlor-5-(trifluormethyl)pyridin-2-yl]methyl}-5-(pyrrolidin-1-ylcarbonyl)-5,6,7,8-tetrahydro[1,2,4]triazolo[4,3-a]pyridin-3(2H)-on (Racemat)

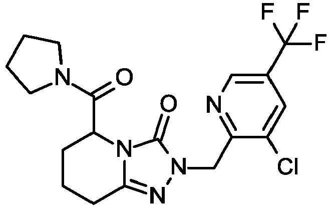

(5RS)-2-{[3-Chlor-5-(trifluormethyl)pyridin-2-yl]methyl}-3-oxo-2,3,5,6,7,8-hexahydro[1,2,4]triazolo[4,3-a]pyridin-5-carbonsäure (Racemat) (80.0 mg, 212 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (105 mg, 276 µmol) und Triethylamin (89 µl, 640 µmol) zugegeben. Nach 15 min Rühren wurde Pyrrolidin (21 µl, 250 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 62.0 mg (68 % d. Th.) der Titelverbindung erhalten.
LC-MS (Methode 4): Rₜ = 0.75 min; MS (ESIpos): m/z = 430 [M+H]⁺
¹H-NMR (400 MHz, DMSO-d6) δ [ppm]: -0.149 (0.57), -0.008 (5.51), 0.008 (4.76), 0.146 (0.58), 1.530 (0.64), 1.678 (1.30), 1.691 (2.52), 1.701 (6.30), 1.714 (8.12), 1.732 (5.69), 1.745 (2.84), 1.753 (2.43), 1.771 (8.48), 1.789 (14.82), 1.806 (11.36), 1.822 (3.36), 1.891 (3.49), 1.908 (10.40), 1.924 (12.93), 1.941 (8.24), 1.959 (3.75), 1.973 (2.58), 1.984 (4.67), 1.995 (4.19), 2.006 (3.22), 2.022 (3.48), 2.036 (2.54), 2.043 (3.25), 2.058 (2.91), 2.078 (1.33), 2.094 (0.70), 2.328 (0.87), 2.366 (0.78), 2.474 (1.94), 2.523 (3.34), 2.567 (3.64), 2.579 (7.40), 2.592 (3.66), 2.609 (1.60), 2.621 (2.75), 2.634 (1.12), 2.670 (0.91), 2.690 (0.54), 2.710 (0.57), 3.229 (1.99), 3.247 (4.27), 3.259 (4.25), 3.276 (7.72), 3.293 (4.00), 3.320 (5.40), 3.338 (7.91), 3.350 (2.84), 3.355 (4.06), 3.367 (4.42), 3.385 (1.97), 3.443 (2.22), 3.460 (4.90), 3.468 (3.60), 3.477 (2.93), 3.485 (6.22), 3.502 (2.73), 3.596 (2.81), 3.612 (5.82), 3.620 (2.96), 3.629 (3.40), 3.637 (4.55), 3.654 (2.03), 4.736 (5.46), 4.745 (6.31), 4.751 (7.34), 4.761 (5.43), 5.063 (4.87), 5.104 (16.00), 5.135 (15.54), 5.175 (4.67), 8.486 (11.75), 8.490 (11.76), 8.903 (11.49), 8.906 (11.69).

### Beispiel 69

### (5RS)-5-(Pyrrolidin-1-ylcarbonyl)-2-({5-[3-(trifluormethyl)phenyl]-1,2,4-oxadiazol-3-yl}methyl)-5,6,7,8-tetrahydro[1,2,4]triazolo[4,3-a]pyridin-3(2H)-on (Racemat)

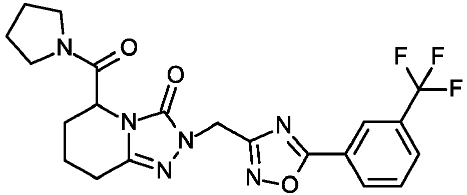

(5RS)-3-Oxo-2-({5-[3-(trifluormethyl)phenyl]-1,2,4-oxadiazol-3-yl}methyl)-2,3,5,6,7,8-hexahydro[1,2,4]triazolo[4,3-a]pyridin-5-carbonsäure (Racemat) (70.0 mg, 88 % Reinheit, 151 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (74.7 mg, 197 µmol) und Triethylamin (63 µl, 450 µmol) zugegeben. Nach 15 min Rühren wurde Pyrrolidin (12.9 mg, 181 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 27.6 mg (39 % d. Th.) der Titelverbindung erhalten.
LC-MS (Methode 3): Rₜ = 1.57 min; MS (ESIpos): m/z = 463 [M+H]⁺
¹H-NMR (400 MHz, DMSO-d6) δ [ppm]: -0.149 (1.57), -0.008 (14.76), 0.008 (12.96), 0.146 (1.57), 1.726 (4.98), 1.738 (4.23), 1.760 (1.87), 1.777 (6.00), 1.795 (10.23), 1.812 (7.87), 1.829 (2.32), 1.899 (2.40), 1.915 (7.31), 1.932 (9.52), 1.949 (6.41), 1.964 (2.55), 1.976 (1.84), 1.988 (3.04), 1.999 (2.66), 2.031 (1.99), 2.043 (2.06), 2.053 (2.02), 2.068 (1.95), 2.323 (1.39), 2.327 (1.91), 2.366 (1.12), 2.523 (7.08), 2.566 (3.60), 2.588 (2.66), 2.598 (2.59), 2.610 (5.17), 2.623 (2.62), 2.640 (1.16), 2.652 (1.84), 2.665 (2.21), 2.670 (2.21), 2.674 (1.54), 2.710 (1.16), 3.235 (1.39), 3.252 (3.04), 3.264 (2.96), 3.281 (5.43), 3.329 (2.85), 3.347 (5.47), 3.365 (2.96), 3.377 (3.19), 3.394 (1.46), 3.448 (1.57), 3.466 (3.56), 3.473 (2.51), 3.483 (2.25), 3.490 (4.42), 3.508 (1.95), 3.605 (2.02), 3.622 (4.23), 3.630 (2.10), 3.638 (2.40), 3.647 (3.26), 3.663 (1.42), 4.647 (0.86), 4.662 (0.90), 4.750 (3.78), 4.759 (4.50), 4.765 (5.13), 4.774 (3.78), 5.019 (4.95), 5.060 (16.00), 5.088 (16.00), 5.128 (5.06), 7.876 (3.37), 7.896 (7.46), 7.915 (4.31), 8.100 (5.43), 8.120 (4.53), 8.333 (8.39), 8.394 (5.43), 8.413 (5.17).

### Beispiel 70

### (5RS)-2-[(1-Ethyl-1H-imidazol-2-yl)methyl]-5-(pyrrolidin-1-ylcarbonyl)-5,6,7,8-tetrahydro[1,2,4]triazolo[4,3-a]pyridin-3(2H)-on (Racemat)

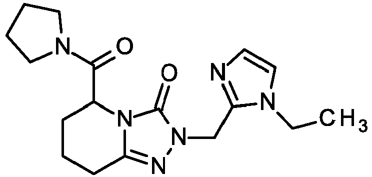

(5RS)-2-[(1-Ethyl-1H-imidazol-2-yl)methyl]-3-oxo-2,3,5,6,7,8-hexahydro[1,2,4]triazolo[4,3-a]pyridin-5-carbonsäure (Racemat) (80.0 mg, 60 % Reinheit, 165 µmol) wurde in DMF (2.0 ml) vorgelegt und 1-[Bis(dimethylamino)methylen]-1H-[1,2,3]triazolo[4,5-b]pyridin-1-ium-3-oxidhexafluorophosphat (81.4 mg, 214 µmol) und Triethylamin (92 µl, 660 µmol) wurden anschließend zugegeben. Nach 15 min Rühren bei Raumtemperatur wurde Pyrrolidin (17 µl, 200 µmol) zugegeben und das Reaktionsgemisch über Nacht bei Raumtemperatur gerührt. Es wurde erneut 1-[Bis(dimethylamino)methylen]-1H-[1,2,3]triazolo[4,5-b]pyridin-1-ium-3-oxidhexafluorophosphat (63.8 mg, 165 µmol) und Pyrrolidin (14 µl, 165 µmol) zugegeben und 2 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 6.00 mg (11 % d. Th.) der Titelverbindung erhalten.
LC-MS (Methode 9): Rₜ = 3.74 min; MS (ESIpos): m/z = 345 [M+H]⁺
¹H-NMR (400 MHz, DMSO-d6) δ [ppm]: -0.008 (3.41), 0.008 (2.88), 1.300 (7.13), 1.319 (16.00), 1.337 (7.18), 1.655 (0.66), 1.666 (0.90), 1.679 (0.96), 1.691 (0.97), 1.705 (1.24), 1.717 (1.47), 1.729 (1.20), 1.757 (1.00), 1.774 (2.92), 1.791 (4.94), 1.808 (3.77), 1.825 (1.17), 1.894 (1.99), 1.910 (3.30), 1.927 (3.65), 1.943 (2.23), 1.961 (1.03), 1.975 (0.56), 1.988 (1.00), 1.999 (2.36), 2.009 (2.34), 2.023 (1.62), 2.034 (0.90), 2.040 (0.83), 2.049 (0.76), 2.072 (0.41), 2.322 (0.52), 2.327 (0.66), 2.332 (0.51), 2.366 (0.54), 2.518 (3.41), 2.523 (2.61), 2.561 (1.55), 2.576 (1.19), 2.592 (1.19), 2.605 (2.17), 2.617 (1.20), 2.634 (0.58), 2.646 (0.89), 2.660 (0.62), 2.665 (0.63), 2.669 (0.75), 2.674 (0.55), 2.689 (0.58), 2.710 (0.54), 2.865 (13.73), 3.219 (0.78), 3.236 (1.55), 3.249 (1.78), 3.266 (3.05), 3.283 (1.95), 3.298 (2.44), 3.315 (4.95), 3.334 (6.17), 3.345 (6.63), 3.445 (0.99), 3.462 (1.79), 3.469 (1.41), 3.479 (1.11), 3.487 (2.22), 3.504 (1.00), 3.581 (1.00), 3.597 (1.93), 3.606 (1.03), 3.614 (1.16), 3.622 (1.45), 3.639 (0.68), 4.145 (1.55), 4.162 (4.11), 4.180 (3.94), 4.183 (3.94), 4.200 (1.38), 4.728 (1.76), 4.738 (2.03), 4.743 (2.55), 4.753 (1.68), 5.163 (1.06), 5.204 (6.89), 5.216 (6.69), 5.257 (1.06), 6.939 (0.66), 7.066 (0.68), 7.194 (0.65), 7.642 (3.94), 7.646 (3.98), 7.776 (4.66), 7.780 (4.28).

### Beispiel 71

### (5RS)-2-[2-Fluor-3-(trifluormethyl)benzyl]-5-(pyrrolidin-1 -ylcarbonyl)-5,6,7,8-tetrahydro[1,2,4]triazolo[4,3-a]pyridin-3(2H)-on (Racemat)

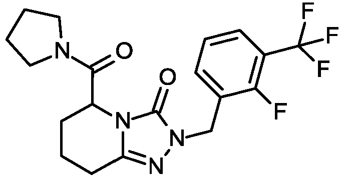

(5RS)-2-[2-Fluor-3-(trifluormethyl)benzyl]-3-oxo-2,3,5,6,7,8-hexahydro[1,2,4]triazolo[4,3-a]pyridin-5-carbonsäure (Racemat) (80.0 mg, 223 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (110 mg, 289 µmol) und Triethylamin (93 µl, 670 µmol) zugegeben. Nach 15 min Rühren wurde Pyrrolidin (22 µl, 270 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 55.0 mg (60 % d. Th.) der Titelverbindung erhalten.
LC-MS (Methode 3): Rₜ = 1.50 min; MS (ESIpos): m/z = 413 [M+H]⁺
¹H-NMR (400 MHz, DMSO-d6) δ [ppm]: -0.150 (0.92), 0.008 (8.29), 0.146 (1.03), 1.693 (3.76), 1.704 (5.42), 1.717 (6.38), 1.755 (3.06), 1.773 (9.29), 1.790 (16.00), 1.807 (12.53), 1.824 (3.65), 1.892 (3.91), 1.909 (11.28), 1.926 (14.08), 1.942 (9.14), 1.960 (3.91), 1.988 (5.09), 2.030 (3.91), 2.073 (3.21), 2.328 (2.40), 2.367 (1.18), 2.559 (5.86), 2.573 (4.20), 2.588 (4.06), 2.600 (7.48), 2.613 (4.20), 2.642 (2.91), 2.669 (2.54), 2.690 (1.25), 2.710 (1.40), 3.227 (2.43), 3.243 (4.53), 3.256 (4.57), 3.273 (8.00), 3.290 (4.39), 3.325 (5.31), 3.343 (8.41), 3.361 (4.50), 3.372 (4.94), 3.391 (2.03), 3.436 (2.43), 3.454 (5.05), 3.461 (4.02), 3.479 (6.86), 3.496 (3.02), 3.590 (3.13), 3.607 (6.30), 3.623 (3.76), 3.632 (4.90), 3.649 (2.18), 4.737 (5.82), 4.747 (6.67), 4.752 (8.04), 4.761 (5.68), 4.895 (3.50), 4.935 (15.48), 4.956 (15.48), 4.995 (3.65), 6.509 (1.29), 7.386 (4.68), 7.406 (10.43), 7.425 (6.23), 7.555 (4.72), 7.573 (7.63), 7.590 (3.65), 7.720 (4.13), 7.736 (7.82), 7.756 (3.91).

### Beispiel 72

### (5RS)-2-[3-Fluor-5-(trifluormethyl)benzyl]-5-(pyrrolidin-1 -ylcarbonyl)-5,6,7,8-tetrahydro[1,2,4]triazolo[4,3-a]pyridin-3(2H)-on (Racemat)

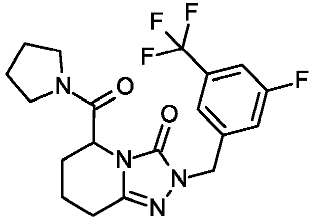

(5RS)-2-[3-Fluor-5-(trifluormethyl)benzyl]-3-oxo-2,3,5,6,7,8-hexahydro[1,2,4]triazolo[4,3-a]pyridin-5-carbonsäure (Racemat) (80.0 mg, 223 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (110 mg, 289 µmol) und Triethylamin (93 µl, 670 µmol) zugegeben. Nach 15 min Rühren wurde Pyrrolidin (22 µl, 270 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 48.0 mg (52 % d. Th.) der Titelverbindung erhalten.
LC-MS (Methode 3): Rₜ = 1.55 min; MS (ESIpos): m/z = 413 [M+H]⁺
¹H-NMR (400 MHz, DMSO-d6) δ [ppm]: -0.008 (3.15), 0.008 (2.63), 1.690 (1.16), 1.701 (1.12), 1.728 (1.86), 1.740 (1.51), 1.755 (1.38), 1.774 (4.07), 1.791 (6.54), 1.809 (5.03), 1.825 (1.42), 1.893 (1.52), 1.910 (4.33), 1.927 (5.32), 1.943 (3.23), 1.961 (1.03), 1.972 (0.74), 2.019 (2.53), 2.028 (1.84), 2.043 (1.33), 2.054 (1.16), 2.062 (1.14), 2.073 (1.44), 2.327 (0.72), 2.563 (2.11), 2.574 (1.91), 2.589 (1.64), 2.604 (1.57), 2.616 (2.72), 2.628 (1.55), 2.646 (0.75), 2.657 (1.15), 2.670 (1.25), 2.710 (0.45), 2.865 (0.60), 3.231 (0.84), 3.247 (1.83), 3.260 (1.90), 3.277 (3.31), 3.294 (1.92), 3.326 (1.93), 3.344 (3.35), 3.361 (1.78), 3.373 (1.90), 3.391 (0.87), 3.439 (0.94), 3.457 (2.17), 3.464 (1.54), 3.482 (2.73), 3.499 (1.19), 3.595 (1.24), 3.611 (2.50), 3.620 (1.31), 3.628 (1.51), 3.636 (1.92), 3.653 (0.88), 4.761 (2.42), 4.770 (2.59), 4.776 (3.10), 4.785 (2.31), 4.959 (16.00), 7.329 (2.37), 7.352 (2.34), 7.502 (5.85), 7.601 (2.38), 7.623 (2.42).

### Beispiel 73

### (5RS)-2-[3-Chlor-4-(trifluormethoxy)benzyl]-5-(pyrrolidin-1-ylcarbonyl)-5,6,7,8-tetrahydro[1,2,4]triazolo[4,3-a]pyridin-3(2H)-on (Racemat)

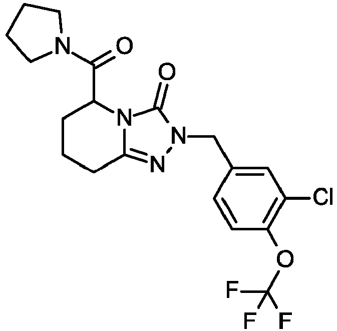

(5RS)-2-[3-Chlor-4-(trifluormethoxy)benzyl]-3-oxo-2,3,5,6,7,8-hexahydro[1,2,4]triazolo[4,3-a]pyridin-5-carbonsäure (Racemat) (75.0 mg, 95 % Reinheit, 182 µmol) wurde in THF (2.1 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (89.9 mg, 236 µmol) und Triethylamin (130 µl, 910 µmol) zugegeben. Nach 15 min Rühren wurde Pyrrolidin (18 µl, 220 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 41.0 mg (51 % d. Th.) der Titelverbindung erhalten.
LC-MS (Methode 3): Rₜ = 1.69 min; MS (ESIpos): m/z = 445 [M+H]⁺
¹H-NMR (400 MHz, DMSO-d6) δ [ppm]: -0.149 (0.80), -0.008 (6.96), 0.008 (5.94), 0.146 (0.78), 1.724 (1.73), 1.756 (1.17), 1.774 (3.10), 1.791 (5.36), 1.808 (4.08), 1.824 (1.15), 1.893 (1.26), 1.909 (3.66), 1.926 (4.36), 1.943 (2.67), 1.960 (1.19), 1.988 (1.32), 1.997 (1.60), 2.010 (1.65), 2.038 (1.17), 2.048 (0.93), 2.063 (1.06), 2.073 (16.00), 2.281 (1.30), 2.327 (1.21), 2.366 (0.71), 2.523 (3.73), 2.558 (1.89), 2.569 (1.54), 2.583 (1.30), 2.597 (1.24), 2.609 (2.28), 2.622 (1.28), 2.639 (0.58), 2.651 (0.93), 2.665 (1.28), 2.669 (1.34), 2.710 (0.59), 2.865 (0.45), 3.229 (0.69), 3.246 (1.58), 3.258 (1.52), 3.275 (2.71), 3.293 (1.56), 3.329 (1.50), 3.347 (2.73), 3.365 (1.47), 3.377 (1.61), 3.394 (0.74), 3.438 (0.78), 3.456 (1.76), 3.464 (1.28), 3.473 (1.06), 3.481 (2.25), 3.498 (0.95), 3.592 (1.02), 3.608 (2.08), 3.617 (1.06), 3.625 (1.23), 3.633 (1.58), 3.650 (0.72), 3.726 (1.63), 4.541 (0.69), 4.745 (1.93), 4.754 (2.10), 4.760 (2.52), 4.769 (1.84), 4.873 (14.14), 7.156 (0.65), 7.306 (2.49), 7.311 (2.54), 7.327 (2.99), 7.332 (3.06), 7.533 (5.10), 7.539 (4.92), 7.547 (2.99), 7.551 (2.88), 7.569 (2.47), 7.572 (2.41).

### Beispiel 74

### (5RS)-5-(Pyrrolidin-1-ylcarbonyl)-2-{[2-(trifluormethyl)pyridin-4-yl]methyl}-5,6,7,8-tetrahydro[1,2,4]triazolo[4,3-a]pyridin-3(2H)-on (Enantiomer 1)

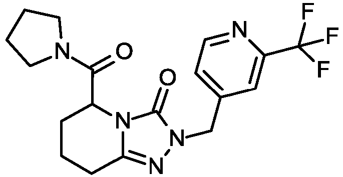

(5RS)-3-oxo-2-{[2-(trifluormethyl)pyridin-4-yl]methyl}-2,3,5,6,7,8-hexahydro[1,2,4]triazolo[4,3-a]pyridin-5-carbonsäure (Enantiomer 1) (70.0 mg, 205 µmol) wurde in THF (2 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (97.1 mg, 255 µmol) und Triethylamin (82 µl, 590 µmol) zugegeben. Nach 15 min Rühren wurde Pyrrolidin (20 µl, 240 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 66.0 mg (85 % d. Th.) der Titelverbindung erhalten.
LC-MS (Methode 3): Rₜ = 1.18 min; MS (ESIpos): m/z = 396 [M+H]⁺
¹H-NMR (400 MHz, DMSO-d6) δ [ppm]: -0.008 (1.82), 0.008 (1.91), 1.663 (0.97), 1.683 (1.29), 1.696 (1.47), 1.706 (1.28), 1.732 (1.80), 1.741 (2.17), 1.756 (2.12), 1.775 (5.55), 1.792 (8.58), 1.809 (6.36), 1.826 (1.82), 1.894 (1.68), 1.910 (4.88), 1.927 (6.01), 1.943 (3.66), 1.960 (1.07), 1.989 (0.74), 2.000 (0.78), 2.015 (1.44), 2.029 (3.22), 2.038 (3.51), 2.045 (2.41), 2.054 (1.95), 2.066 (1.46), 2.072 (1.43), 2.081 (1.30), 2.108 (0.49), 2.327 (0.55), 2.523 (1.95), 2.558 (2.98), 2.572 (2.50), 2.584 (2.37), 2.598 (1.98), 2.614 (1.90), 2.625 (3.38), 2.637 (2.01), 2.656 (0.92), 2.666 (1.65), 2.679 (0.87), 3.237 (1.07), 3.254 (2.30), 3.266 (2.34), 3.283 (4.24), 3.330 (2.25), 3.347 (4.22), 3.359 (1.53), 3.365 (2.21), 3.377 (2.39), 3.394 (1.07), 3.445 (1.23), 3.462 (2.68), 3.469 (2.01), 3.479 (1.58), 3.487 (3.47), 3.504 (1.50), 3.594 (1.62), 3.611 (3.15), 3.619 (1.64), 3.627 (1.83), 3.636 (2.39), 3.652 (1.14), 4.774 (2.95), 4.783 (3.30), 4.789 (4.07), 4.798 (2.91), 4.980 (0.51), 5.024 (16.00), 5.068 (0.51), 7.473 (4.22), 7.485 (4.38), 7.760 (8.48), 8.726 (6.15), 8.739 (6.08).

Die (5S)-Konfiguration wurde aufgrund der Kristallstrukturaufklärung für Beispiel 26, 108, 113, 157, 237, 358 und 454 zugeordnet.

### (5S)-5-(Pyrrolidin-1-ylcarbonyl)-2-{[2-(trifluormethyl)pyridin-4-yl]methyl}-5,6,7,8-tetrahydro[1,2,4]triazolo[4,3-a]pyridin-3(2H)-on

### Beispiel 75

### (5RS)-2-{[3-Fluor-2-(trifluormethyl)pyridin-4-yl]methyl}-5-(pyrrolidin-1-ylcarbonyl)-5,6,7,8-tetrahydro[1,2,4]triazolo[4,3-a]pyridin-3(2H)-on (Enantiomer 1)

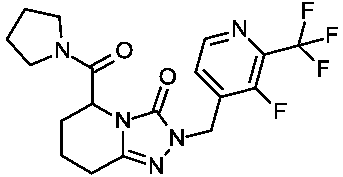

(5RS)-2-[[3-Fluoro-2-(trifluoromethyl)-4-pyridyl]methyl]-3-oxo-5,6,7,8-tetrahydro-[1,2,4]triazolo[4,3-a]pyridine-5-carbonsäure (Enantiomer 1) (80.0 mg, 214 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (106 mg, 278 µmol) und Triethylamin (89 µl, 640 µmol) zugegeben. Nach 15 min Rühren wurde Pyrrolidin (21 µl, 260 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 67.0 mg (76 % d. Th.) der Titelverbindung erhalten.
LC-MS (Methode 3): Rₜ = 1.31 min; MS (ESIpos): m/z = 414 [M+H]⁺
¹H-NMR (400 MHz, DMSO-d6) δ [ppm]: -0.150 (0.52), 0.008 (3.36), 0.146 (0.57), 1.697 (2.60), 1.708 (2.42), 1.737 (3.98), 1.758 (3.80), 1.774 (8.57), 1.792 (13.17), 1.809 (9.76), 1.825 (2.72), 1.894 (3.04), 1.909 (7.95), 1.926 (9.58), 1.942 (5.78), 1.959 (1.76), 2.025 (5.07), 2.047 (3.06), 2.060 (2.40), 2.067 (2.35), 2.075 (2.15), 2.328 (1.01), 2.366 (0.69), 2.569 (4.00), 2.580 (3.80), 2.594 (3.25), 2.609 (3.31), 2.621 (5.56), 2.633 (3.16), 2.651 (1.53), 2.664 (2.51), 2.710 (0.69), 3.235 (1.85), 3.252 (3.80), 3.264 (4.16), 3.281 (7.27), 3.329 (3.57), 3.347 (6.42), 3.364 (3.45), 3.376 (3.57), 3.394 (1.65), 3.443 (2.03), 3.460 (4.21), 3.468 (3.22), 3.486 (5.21), 3.503 (2.33), 3.592 (2.63), 3.609 (4.96), 3.626 (3.00), 3.634 (3.73), 3.650 (1.62), 4.763 (4.87), 4.772 (5.33), 4.777 (6.22), 4.787 (4.32), 5.015 (1.69), 5.057 (16.00), 5.065 (15.41), 5.107 (1.48), 7.546 (4.85), 7.559 (8.68), 7.571 (4.71), 8.561 (10.29), 8.573 (9.81).

Die (5S)-Konfiguration wurde aufgrund der Kristallstrukturaufklärung für Beispiel 26, 108, 113, 157, 237, 358 und 454 zugeordnet.

### (5S)-2-{[3-Fluor-2-(trifluormethyl)pyridin-4-yl]methyl}-5-(pyrrolidin-1-ylcarbonyl)-5,6,7,8-tetrahydro[1,2,4]triazolo[4,3-a]pyridin-3(2H)-on

### Beispiel 76

### (5RS)-5-(Pyrrolidin-1-ylcarbonyl)-2-{[2-(trifluormethyl)chinolin-4-yl]methyl}-5,6,7,8-tetrahydro[1,2,4]triazolo[4,3-a]pyridin-3(2H)-on (Enantiomer 1)

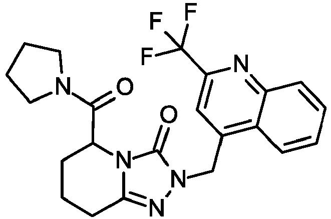

(5RS)-3-Oxo-2-{[2-(trifluormethyl)chinolin-4-yl]methyl}-2,3,5,6,7,8-hexahydro[1,2,4]triazolo[4,3-a]pyridin-5-carbonsäure (Enantiomer 1) (94.0 mg, 240 µmol) wurde in THF (2.1 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (118 mg, 311 µmol) und Triethylamin (100 µl, 720 µmol) zugegeben. Nach 15 min Rühren wurde Pyrrolidin (24 µl, 290 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 57.0 mg (53 % d. Th.) der Titelverbindung erhalten.
LC-MS (Methode 3): Rₜ = 1.55 min; MS (ESIpos): m/z = 446 [M+H]⁺
¹H-NMR (400 MHz, DMSO-d6) δ [ppm]: -0.008 (2.70), 0.008 (2.54), 1.147 (0.45), 1.165 (0.90), 1.183 (0.45), 1.653 (1.17), 1.666 (1.66), 1.678 (1.86), 1.691 (1.77), 1.704 (2.52), 1.717 (3.00), 1.728 (2.29), 1.740 (1.51), 1.751 (1.17), 1.763 (1.72), 1.781 (5.12), 1.799 (9.24), 1.816 (7.40), 1.832 (2.22), 1.899 (2.19), 1.915 (6.75), 1.932 (8.65), 1.949 (5.19), 1.966 (1.61), 1.983 (1.00), 1.998 (1.85), 2.010 (4.50), 2.019 (4.62), 2.034 (2.65), 2.046 (1.72), 2.052 (1.66), 2.061 (1.55), 2.071 (0.74), 2.087 (0.51), 2.096 (0.42), 2.328 (0.56), 2.333 (0.49), 2.342 (1.75), 2.519 (3.00), 2.525 (4.32), 2.567 (2.15), 2.594 (2.16), 2.605 (3.87), 2.617 (2.31), 2.636 (1.19), 2.647 (1.75), 2.660 (1.01), 2.670 (0.71), 3.238 (1.31), 3.255 (2.81), 3.268 (2.72), 3.285 (4.87), 3.340 (2.36), 3.357 (4.92), 3.369 (1.89), 3.375 (2.60), 3.387 (2.96), 3.405 (1.31), 3.440 (1.47), 3.457 (3.21), 3.464 (2.37), 3.474 (1.97), 3.482 (4.04), 3.499 (1.75), 3.606 (1.82), 3.622 (3.91), 3.630 (2.00), 3.639 (2.22), 3.647 (3.07), 3.664 (1.36), 3.803 (0.57), 3.818 (0.56), 4.792 (3.51), 4.801 (3.91), 4.806 (4.95), 4.815 (3.45), 5.408 (3.17), 5.449 (10.04), 5.480 (10.14), 5.521 (3.20), 5.558 (0.51), 6.510 (2.74), 7.293 (0.56), 7.386 (0.63), 7.406 (0.59), 7.750 (16.00), 7.823 (2.56), 7.826 (2.72), 7.844 (5.54), 7.862 (3.75), 7.864 (3.71), 7.943 (3.44), 7.946 (3.64), 7.964 (5.87), 7.967 (4.51), 7.982 (3.21), 7.985 (3.11), 8.214 (6.39), 8.234 (5.46), 8.370 (6.19), 8.390 (5.70).

Die (5S)-Konfiguration wurde aufgrund der Kristallstrukturaufklärung für Beispiel 26, 108, 113, 157, 237, 358 und 454 zugeordnet.

### (5S)-5-(Pyrrolidin-1-ylcarbonyl)-2-{[2-(trifluormethyl)chinolin-4-yl]methyl}-5,6,7,8-tetrahydro[1,2,4]triazolo[4,3-a]pyridin-3(2H)-on

### Beispiel 77

### (5RS)-2-{[2-Methyl-4-(trifluormethyl)-1,3-thiazol-5-yl]methyl}-5-(pyrrolidin-1-ylcarbonyl)-5,6,7,8-tetrahydro[1,2,4]triazolo[4,3-a]pyridin-3(2H)-on (Racemat)

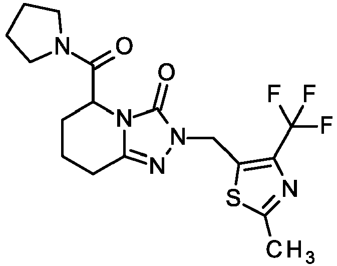

(5RS)-2-{[2-Methyl-4-(trifluormethyl)-1,3-thiazol-5-yl]methyl}-3-oxo-2,3,5,6,7,8-hexahydro[1,2,4]triazolo[4,3-a]pyridin-5-carbonsäure (Racemat) (68.0 mg, 188 µmol) wurde in THF (2.1 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (92.8 mg, 244 µmol) und Triethylamin (78 µl, 560 µmol) zugegeben. Nach 15 min Rühren wurde Pyrrolidin (19 µl, 230 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 49.0 mg (63 % d. Th.) der Titelverbindung erhalten.
LC-MS (Methode 3): Rₜ = 1.21 min; MS (ESIpos): m/z = 416 [M+H]⁺
¹H-NMR (400 MHz, DMSO-d6) δ [ppm]: -0.008 (2.56), 0.008 (2.06), 1.163 (2.87), 1.181 (6.08), 1.199 (2.98), 1.703 (0.97), 1.714 (1.06), 1.754 (0.51), 1.770 (1.54), 1.787 (2.65), 1.804 (2.06), 1.821 (0.60), 1.889 (0.63), 1.906 (1.90), 1.923 (2.45), 1.939 (1.73), 1.974 (0.84), 1.986 (0.78), 2.003 (0.52), 2.019 (0.67), 2.028 (0.50), 2.327 (0.46), 2.523 (1.51), 2.558 (0.99), 2.568 (0.83), 2.582 (0.68), 2.602 (0.66), 2.613 (1.27), 2.626 (0.75), 2.646 (16.00), 2.669 (0.74), 2.890 (0.45), 3.076 (1.10), 3.089 (1.24), 3.094 (1.13), 3.106 (1.07), 3.236 (0.78), 3.248 (0.79), 3.265 (1.40), 3.282 (0.69), 3.313 (0.78), 3.330 (1.69), 3.428 (0.64), 3.445 (0.98), 3.453 (0.73), 3.470 (1.21), 3.487 (0.56), 3.578 (0.51), 3.595 (1.09), 3.612 (0.63), 3.620 (0.80), 4.713 (0.95), 4.723 (1.09), 4.728 (1.30), 4.738 (0.95), 5.127 (2.30), 5.142 (2.33).

### Beispiel 78

### (5RS)-2-[(5-Chlorpyridin-2-yl)methyl]-5-[(3,3-difluorpyrrolidin-1-yl)carbonyl]-5,6,7,8-tetrahydro[1,2,4]triazolo[4,3-a]pyridin-3(2H)-on (Racemat)

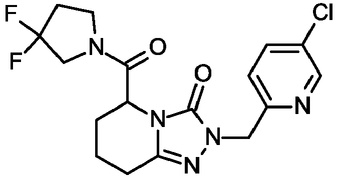

(5RS)-2-[(5-Chlorpyridin-2-yl)methyl]-3-oxo-2,3,5,6,7,8-hexahydro[1,2,4]triazolo[4,3-a]pyridin-5-carbonsäure (Racemat) (50.0 mg, 162 µmol) wurde in THF (3.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (80.1 mg, 211 µmol) und Triethylamin (68 µl, 490 µmol) zugegeben. Nach 15 min Rühren wurde 3,3-Difluorpyrrolidinhydrochlorid (27.9 mg, 194 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 25.2 mg (39 % d. Th.) der Titelverbindung erhalten.
LC-MS (Methode 4): Rt = 0.69 min; MS (ESIpos): m/z = 398 [M+H]⁺
¹H-NMR (400 MHz, DMSO-d6) δ [ppm]: -0.008 (2.72), 0.008 (2.55), 1.687 (1.41), 1.699 (1.48), 1.726 (1.92), 2.023 (1.76), 2.050 (1.24), 2.064 (1.31), 2.073 (2.50), 2.085 (1.00), 2.101 (0.87), 2.328 (0.75), 2.365 (0.71), 2.381 (1.04), 2.410 (1.31), 2.431 (1.22), 2.569 (4.02), 2.578 (3.03), 2.592 (3.63), 2.607 (3.88), 2.620 (1.88), 2.650 (1.12), 2.665 (1.04), 2.709 (0.48), 3.538 (1.74), 3.557 (2.71), 3.570 (1.44), 3.577 (1.41), 3.638 (0.51), 3.672 (1.50), 3.706 (1.75), 3.740 (1.36), 3.774 (2.31), 3.790 (1.31), 3.798 (1.21), 3.808 (2.23), 3.816 (1.89), 3.835 (0.93), 3.894 (0.74), 3.912 (1.70), 3.931 (0.99), 3.938 (1.22), 3.957 (0.58), 3.972 (0.49), 4.001 (1.06), 4.014 (0.56), 4.030 (0.71), 4.043 (0.99), 4.071 (0.60), 4.147 (0.64), 4.179 (1.00), 4.203 (1.01), 4.235 (0.43), 4.761 (1.40), 4.776 (1.83), 4.785 (1.34), 4.836 (1.45), 4.846 (1.72), 4.851 (1.89), 4.861 (1.45), 4.870 (0.73), 4.914 (16.00), 4.955 (0.69), 7.199 (6.18), 7.220 (6.60), 7.913 (4.28), 7.920 (4.38), 7.934 (4.15), 7.941 (4.23), 8.571 (5.89), 8.577 (5.93).

### Beispiel 79

### (5RS)-5-[(3,3-Difluorpyrrolidin-1-yl)carbonyl]-2-{[6-(trifluormethyl)pyridin-3-yl]methyl}-5,6,7,8-tetrahydro[1,2,4]triazolo[4,3-a]pyridin-3(2H)-on (Racemat)

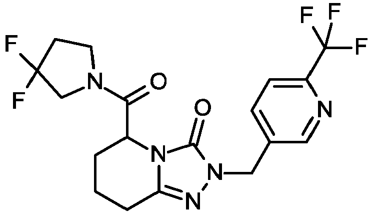

(5RS)-3-Oxo-2-{[6-(trifluormethyl)pyridin-3-yl]methyl}-2,3,5,6,7,8-hexahydro[1,2,4]triazolo[4,3-a]pyridin-5-carbonsäure -trifluoressigsäure (Racemat) (70.0 mg, 153 µmol) wurde in THF (3.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (75.8 mg, 199 µmol) und Triethylamin (110 µl, 770 µmol) zugegeben. Nach 15 min Rühren wurde 3,3-Difluorpyrrolidinhydrochlorid (26.4 mg, 184 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 37.7 mg (57 % d. Th.) der Titelverbindung erhalten.
LC-MS (Methode 4): Rₜ = 0.75 min; MS (ESIpos): m/z = 432 [M+H]⁺
¹H-NMR (400 MHz, DMSO-d6) δ [ppm]: -0.008 (3.24), 0.008 (3.00), 1.664 (1.10), 1.735 (1.37), 1.908 (1.15), 1.994 (1.34), 2.008 (1.72), 2.018 (1.67), 2.033 (1.58), 2.044 (1.35), 2.052 (1.21), 2.060 (1.13), 2.073 (1.48), 2.080 (0.80), 2.088 (0.84), 2.096 (0.75), 2.328 (0.80), 2.366 (0.81), 2.382 (0.98), 2.411 (1.16), 2.431 (1.11), 2.456 (0.84), 2.569 (3.33), 2.580 (2.48), 2.594 (2.56), 2.610 (2.91), 2.623 (1.48), 2.640 (0.60), 2.653 (0.91), 2.669 (1.06), 2.710 (0.48), 3.534 (1.23), 3.541 (1.45), 3.552 (2.00), 3.561 (2.15), 3.571 (1.27), 3.580 (1.08), 3.670 (1.29), 3.704 (1.47), 3.738 (0.63), 3.749 (0.72), 3.769 (0.56), 3.782 (1.84), 3.794 (0.96), 3.813 (2.64), 3.831 (0.71), 3.891 (0.68), 3.910 (1.45), 3.929 (0.81), 3.936 (1.06), 3.955 (0.49), 3.966 (0.43), 3.995 (0.88), 4.010 (0.45), 4.023 (0.60), 4.038 (0.82), 4.066 (0.49), 4.149 (0.51), 4.182 (0.79), 4.206 (0.79), 4.767 (1.21), 4.776 (1.43), 4.782 (1.63), 4.791 (1.15), 4.838 (1.28), 4.847 (1.44), 4.853 (1.63), 4.862 (1.17), 5.011 (14.35), 7.909 (15.74), 7.913 (16.00), 8.641 (5.64).

### Beispiel 80

### (5RS)-2-[(6-Chlorpyridin-3-yl)methyl]-5-[(3,3-difluorpyrrolidin-1-yl)carbonyl]-5,6,7,8-tetrahydro[1,2,4]triazolo[4,3-a]pyridin-3(2H)-on (Racemat)

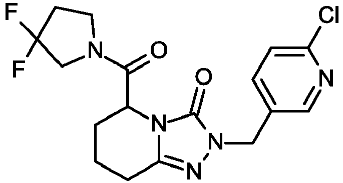

(5RS)-2-[(6-Chlorpyridin-3-yl)methyl]-3-oxo-2,3,5,6,7,8-hexahydro[1,2,4]triazolo[4,3-a]pyridin-5-carbonsäure (Racemat) (350 mg, 15 % Reinheit, 170 µmol) wurde in THF (3.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (84.1 mg, 221 µmol) und Triethylamin (71 µl, 510 µmol) zugegeben. Nach 15 min Rühren wurde 3,3-Difluorpyrrolidinhydrochlorid (29.3 mg, 204 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 46.4 mg (69 % d. Th.) der Titelverbindung erhalten.
LC-MS (Methode 4): Rₜ = 0.67 min; MS (ESIpos): m/z = 398 [M+H]⁺
¹H-NMR (400 MHz, DMSO-d6) δ [ppm]: 1.661 (1.07), 1.726 (1.27), 1.996 (1.45), 2.007 (1.54), 2.043 (1.10), 2.060 (0.88), 2.071 (0.82), 2.327 (0.70), 2.366 (0.54), 2.379 (0.89), 2.408 (1.09), 2.427 (1.04), 2.452 (0.91), 2.560 (2.42), 2.569 (2.97), 2.585 (2.82), 2.602 (2.79), 2.644 (0.79), 2.669 (0.70), 3.537 (1.17), 3.546 (1.77), 3.556 (1.91), 3.565 (1.12), 3.575 (0.94), 3.665 (1.17), 3.700 (1.30), 3.743 (0.64), 3.776 (1.61), 3.807 (2.37), 3.825 (0.62), 3.887 (0.58), 3.906 (1.29), 3.931 (0.92), 3.962 (0.43), 3.991 (0.80), 4.018 (0.57), 4.032 (0.75), 4.063 (0.47), 4.143 (0.51), 4.175 (0.74), 4.200 (0.74), 4.749 (1.05), 4.764 (1.43), 4.773 (1.05), 4.821 (1.16), 4.836 (1.48), 4.846 (1.20), 4.884 (16.00), 7.505 (4.89), 7.525 (6.06), 7.686 (3.27), 7.692 (3.34), 7.707 (2.69), 7.713 (2.73), 8.296 (5.00), 8.301 (4.81).

### Beispiel 81

### (5RS)-2-(3,5-Dichlorbenzyl)-5-(pyrrolidin-1-ylcarbonyl)-5,6,7,8-tetrahydro[1,2,4]triazolo[4,3-a]pyridin-3(2H)-on (Racemat)

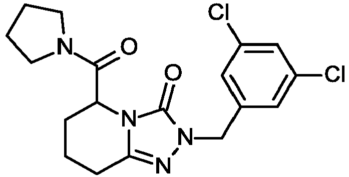

(5RS)-2-(3,5-Dichlorbenzyl)-3-oxo-2,3,5,6,7,8-hexahydro[1,2,4]triazolo[4,3-a]pyridin-5-carbonsäurehydrochlorid (Racemat) (70.0 mg, 79 % Reinheit, 145 µmol) wurde in THF (3.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (71.7 mg, 189 µmol) und Triethylamin (100 µl, 730 µmol) zugegeben. Nach 15 min Rühren wurde Pyrrolidin (12.4 mg, 174 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 21.4 mg (37 % d. Th.) der Titelverbindung erhalten.
LC-MS (Methode 3): Rₜ = 1.61 min; MS (ESIpos): m/z = 395 [M+H]⁺
¹H-NMR (400 MHz, DMSO-d6) δ [ppm]: -0.149 (0.96), -0.008 (8.42), 0.008 (7.50), 0.146 (1.00), 1.406 (0.70), 1.725 (1.96), 1.756 (1.37), 1.773 (3.77), 1.791 (6.24), 1.808 (4.80), 1.824 (1.40), 1.893 (1.44), 1.909 (4.25), 1.926 (5.36), 1.943 (3.21), 1.960 (1.44), 1.997 (1.81), 2.015 (1.55), 2.040 (1.29), 2.051 (1.07), 2.058 (1.18), 2.066 (1.07), 2.327 (1.88), 2.366 (1.40), 2.518 (8.61), 2.523 (7.02), 2.565 (2.44), 2.574 (2.00), 2.590 (1.66), 2.603 (1.55), 2.615 (2.77), 2.627 (1.66), 2.644 (0.78), 2.669 (2.48), 2.709 (1.44), 3.228 (0.89), 3.245 (1.88), 3.257 (1.85), 3.274 (3.33), 3.292 (2.25), 3.330 (2.11), 3.348 (3.29), 3.365 (1.74), 3.378 (1.88), 3.395 (0.89), 3.437 (1.00), 3.454 (2.11), 3.461 (1.55), 3.479 (2.66), 3.496 (1.15), 3.593 (1.26), 3.609 (2.40), 3.626 (1.44), 3.634 (1.92), 3.651 (0.81), 4.753 (2.25), 4.762 (2.48), 4.768 (2.77), 4.777 (2.29), 4.851 (16.00), 7.273 (11.16), 7.278 (11.31), 7.533 (2.40), 7.538 (4.25), 7.543 (2.14).

### Beispiel 82

### (5RS)-2-(3,5-Dichlorbenzyl)-5-[(3,3-difluorpyrrolidin-1-yl)carbonyl]-5,6,7,8-tetrahydro[1,2,4]triazolo[4,3-a]pyridin-3(2H)-on (Racemat)

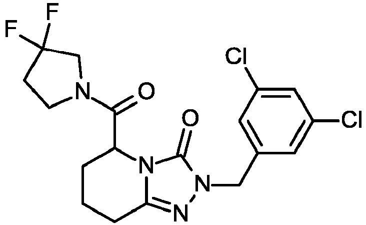

(5RS)-2-(3,5-Dichlorbenzyl)-3-oxo-2,3,5,6,7,8-hexahydro[1,2,4]triazolo[4,3-a]pyridin-5-carbonsäurehydrochlorid (Racemat) (70.0 mg, 79 % Reinheit, 145 µmol) wurde in THF (3.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (71.7 mg, 189 µmol) und Triethylamin (100 µl, 730 µmol) zugegeben. Nach 15 min Rühren wurde 3,3-Difluorpyrrolidinhydrochlorid (25.0 mg, 174 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 26.4 mg (94 % Reinheit, 40 % d. Th.) der Titelverbindung erhalten.
LC-MS (Methode 3): Rₜ = 1.73 min; MS (ESIpos): m/z = 431 [M+H]⁺
¹H-NMR (400 MHz, DMSO-d6) δ [ppm]: -0.008 (3.09), 0.008 (2.90), 1.670 (0.97), 1.734 (1.17), 1.992 (1.13), 2.004 (1.20), 2.013 (1.32), 2.048 (0.92), 2.056 (1.04), 2.063 (1.01), 2.073 (6.09), 2.084 (0.76), 2.091 (0.75), 2.100 (0.66), 2.327 (0.74), 2.366 (0.72), 2.382 (0.91), 2.412 (1.03), 2.424 (0.98), 2.453 (0.85), 2.523 (3.33), 2.565 (2.41), 2.580 (2.09), 2.590 (2.49), 2.605 (2.35), 2.622 (2.34), 2.633 (1.24), 2.665 (1.27), 2.674 (0.86), 2.709 (0.46), 3.533 (1.03), 3.541 (1.15), 3.551 (1.74), 3.562 (1.93), 3.569 (1.05), 3.581 (0.92), 3.672 (1.10), 3.705 (1.28), 3.742 (0.91), 3.764 (0.52), 3.776 (1.52), 3.790 (0.80), 3.808 (2.32), 3.827 (0.59), 3.894 (0.59), 3.912 (1.26), 3.931 (0.71), 3.939 (0.91), 3.958 (0.60), 3.989 (0.78), 4.017 (0.52), 4.032 (0.71), 4.060 (0.42), 4.149 (0.46), 4.183 (0.68), 4.206 (0.70), 4.775 (1.07), 4.784 (1.23), 4.790 (1.41), 4.799 (1.05), 4.858 (16.00), 7.271 (10.23), 7.276 (10.34), 7.536 (2.16), 7.541 (3.82), 7.546 (2.01).

### Beispiel 83

### (5RS)-2-(3,4-Difluorbenzyl)-5-[(3,3-difluorpyrrolidin-1-yl)carbonyl]-5,6,7,8-tetrahydro[1,2,4]triazolo[4,3-a]pyridin-3(2H)-on (Racemat)

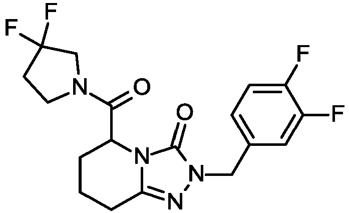

(5RS)-2-(3,4-Difluorbenzyl)-3-oxo-2,3,5,6,7,8-hexahydro[1,2,4]triazolo[4,3-a]pyridin-5-carbonsäurehydrochlorid (Racemat) (100 mg, 289 µmol) wurde in THF (3.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (143 mg, 376 µmol) und Triethylamin (200 µl, 1.4 mmol) zugegeben. Nach 15 min Rühren wurde 3,3-Difluorpyrrolidinhydrochlorid (49.8 mg, 347 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 17.0 mg (15 % d. Th.) der Titelverbindung erhalten.
LC-MS (Methode 4): Rₜ = 0.78 min; MS (ESIpos): m/z = 399 [M+H]⁺
¹H-NMR (400 MHz, DMSO-d6) δ [ppm]: -0.149 (0.64), -0.008 (5.78), 0.008 (4.12), 0.147 (0.54), 1.734 (1.51), 2.012 (1.51), 2.327 (1.54), 2.566 (2.86), 2.591 (2.58), 2.608 (2.98), 2.669 (1.40), 2.709 (0.76), 3.559 (2.11), 3.670 (1.33), 3.704 (1.54), 3.741 (0.97), 3.775 (1.59), 3.809 (2.49), 3.909 (1.33), 3.991 (0.97), 4.178 (0.97), 4.770 (1.68), 4.822 (16.00), 7.083 (1.87), 7.230 (1.68), 7.259 (2.04), 7.278 (1.80), 7.375 (1.75), 7.396 (3.57), 7.402 (2.25), 7.423 (3.55), 7.445 (1.68).

### Beispiel 84

### (5RS)-5-(Pyrrolidin-1-ylcarbonyl)-2-{[6-(trifluormethyl)pyridin-3-yl]methyl}-5,6,7,8-tetrahydro[1,2,4]triazolo[4,3-a]pyridin-3(2H)-on (Racemat)

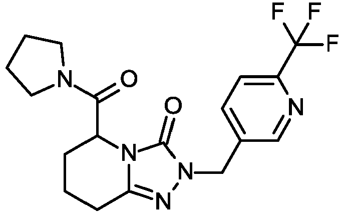

(5RS)-3-Oxo-2-{[6-(trifluormethyl)pyridin-3-yl]methyl}-2,3,5,6,7,8-hexahydro[1,2,4]triazolo[4,3-a]pyridin-5-carbonsäure -trifluoressigsäure (Racemat) (70.0 mg, 153 µmol) wurde in THF (3.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (75.8 mg, 199 µmol) und Triethylamin (110 µl, 770 µmol) zugegeben. Nach 15 min Rühren wurde Pyrrolidin (15 µl, 180 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 32.2 mg (53 % d. Th.) der Titelverbindung erhalten.
LC-MS (Methode 3): Rₜ = 1.23 min; MS (ESIpos): m/z = 396 [M+H]⁺
¹H-NMR (400 MHz, DMSO-d6) δ [ppm]: -0.008 (3.63), 0.008 (3.14), 0.146 (0.40), 1.673 (1.10), 1.686 (1.21), 1.696 (1.21), 1.710 (1.60), 1.724 (1.93), 1.736 (1.54), 1.748 (1.13), 1.758 (1.55), 1.774 (4.10), 1.791 (6.82), 1.808 (5.25), 1.825 (1.49), 1.893 (1.46), 1.909 (4.19), 1.926 (5.20), 1.942 (3.16), 1.959 (1.12), 2.013 (2.62), 2.036 (1.46), 2.048 (1.20), 2.055 (1.18), 2.063 (1.13), 2.072 (0.66), 2.327 (0.97), 2.366 (0.58), 2.523 (3.85), 2.564 (2.19), 2.579 (1.70), 2.593 (1.65), 2.605 (2.93), 2.617 (1.70), 2.635 (0.78), 2.646 (1.13), 2.660 (0.89), 2.665 (0.89), 2.669 (1.07), 2.709 (0.62), 3.231 (0.96), 3.247 (1.93), 3.260 (1.93), 3.277 (3.53), 3.295 (2.62), 3.328 (2.30), 3.346 (3.58), 3.363 (1.83), 3.375 (2.02), 3.393 (0.92), 3.440 (0.99), 3.457 (2.20), 3.464 (1.62), 3.474 (1.34), 3.482 (2.82), 3.499 (1.26), 3.592 (1.28), 3.609 (2.61), 3.617 (1.34), 3.626 (1.57), 3.633 (2.02), 3.650 (0.94), 4.747 (2.45), 4.756 (2.74), 4.762 (3.26), 4.771 (2.36), 5.005 (13.36), 7.907 (15.77), 7.910 (16.00), 8.641 (5.00).

### Beispiel 85

### (5RS)-5-(Pyrrolidin-1-ylcarbonyl)-2-{[6-(trifluormethyl)pyridin-3-yl]methyl}-5,6,7,8-tetrahydro[1,2,4]triazolo[4,3-a]pyridin-3(2H)-on (Enantiomer 2)

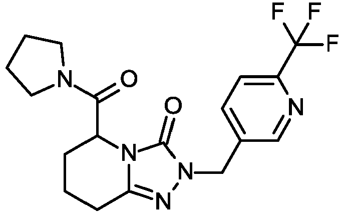

(5RS)-5-(Pyrrolidin-1 -ylcarbonyl)-2-{[6-(trifluormethyl)pyridin-3-yl]methyl}-5,6,7,8-tetrahydro[1,2,4]triazolo[4,3-a]pyridin-3(2H)-on (Racemat) wurden durch chirale präparative HPLC getrennt. [Probenvorbereitung: 75 mg gelöst in 1ml Ethanol/Acetonitril; Injektionsvolumen: 0.2 ml; Säule: Daicel Chiralpakl^{®} AD-H 5µm, 250 x 20 mm; Laufmittel: n-Heptan/Ethanol 40:60, Fluß: 20 ml/min; Temperatur 40°C; UV Detektion: 220 nm]. Nach der Trennung wurden 27 mg von Enantiomer 1, welches zuerst eluierte und 27 mg von Enantiomer 2, welches später eluierte, isoliert.

### Enantiomer 2:

Analytische chirale HPLC: Rₜ = 3.61 min, e.e. = 99.9% [Säule: Daicel Chiralcel^{®} OJ-3 3µm 50 x 4.6 mm; Laufmittel: n-Heptan/Isopropanol 1:1, 0.2% Trifluoressigsäure, 1% Wasser; Fluß: 1 ml/min; UV Detektion: 220 nm]
LC-MS (Methode 3): Rₜ = 1.22 min; MS (ESIpos): m/z = 396 [M+H]⁺
¹H-NMR (400 MHz, DMSO-d6) δ [ppm]: 1.61-1.82 (m, 4H). 1.87 - 1.95 (m, 2H), 1.95 - 2.10 (m, 2H), 2.56 - 2.72 (m, 2H), 3.22 - 3.28 (m, 1H), 3.33 - 3.39 (m, 1H), 3.47 (dt, 1H), 3.62 (dt, 1H), 4.76 (dd, 1H), 5.00 (s, 2H), 7.91 (d, 2H), 8.64 (s, 1H).

### Beispiel 86

### (5RS)-2-(3-Chlor-4-fluorbenzyl)-5-[(3,3-difluorpyrrolidin-1-yl)carbonyl]-5,6,7,8-tetrahydro[1,2,4]triazolo[4,3-a]pyridin-3(2H)-on (Racemat)

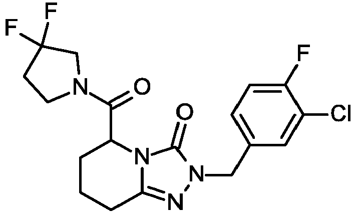

(5RS)-2-(3-Chlor-4-fluorbenzyl)-3-oxo-2,3,5,6,7,8-hexahydro[1,2,4]triazolo[4,3-a]pyridin-5-carbonsäurehydrochlorid (Racemat) (100 mg, 276 µmol) wurde in THF (3.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (136 mg, 359 µmol) und Triethylamin (190 µl, 1.4 mmol) zugegeben. Nach 15 min Rühren wurde 3,3-Difluorpyrrolidinhydrochlorid (47.6 mg, 331 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 14.0 mg (12 % d. Th.) der Titelverbindung erhalten.
LC-MS (Methode 4): Rₜ = 0.83 min; MS (ESIpos): m/z = 415 [M+H]⁺
¹H-NMR (400 MHz, DMSO-d6) δ [ppm]: -0.149 (0.46), -0.008 (3.79), 0.008 (3.46), 0.146 (0.44), 1.670 (1.08), 1.680 (1.01), 1.694 (0.93), 1.728 (1.34), 1.980 (1.24), 1.995 (1.30), 2.004 (1.45), 2.028 (1.05), 2.038 (1.04), 2.046 (1.10), 2.054 (1.05), 2.063 (0.94), 2.073 (0.88), 2.082 (0.80), 2.089 (0.70), 2.323 (0.61), 2.327 (0.83), 2.332 (0.58), 2.366 (0.83), 2.381 (0.94), 2.391 (0.67), 2.411 (1.10), 2.425 (1.04), 2.452 (0.87), 2.523 (3.60), 2.566 (2.83), 2.576 (2.48), 2.591 (2.76), 2.608 (2.96), 2.619 (1.40), 2.639 (0.57), 2.650 (0.87), 2.665 (0.91), 2.669 (0.93), 2.710 (0.53), 3.531 (1.18), 3.538 (1.34), 3.549 (1.92), 3.559 (2.12), 3.567 (1.21), 3.578 (1.04), 3.668 (1.24), 3.703 (1.42), 3.742 (0.78), 3.764 (0.56), 3.775 (1.64), 3.790 (0.88), 3.808 (2.65), 3.826 (0.68), 3.891 (0.67), 3.909 (1.41), 3.928 (0.77), 3.935 (1.02), 3.954 (0.48), 3.962 (0.46), 3.991 (0.83), 4.005 (0.44), 4.019 (0.60), 4.033 (0.77), 4.061 (0.47), 4.145 (0.53), 4.176 (0.75), 4.201 (0.77), 4.755 (1.15), 4.765 (1.38), 4.771 (1.58), 4.780 (1.20), 4.824 (16.00), 4.838 (1.79), 4.844 (1.77), 4.853 (1.32), 7.220 (1.30), 7.225 (1.47), 7.232 (1.49), 7.241 (2.05), 7.246 (2.01), 7.253 (1.84), 7.258 (1.82), 7.373 (3.89), 7.396 (5.08), 7.418 (3.09), 7.429 (2.86), 7.434 (2.69), 7.447 (2.88), 7.452 (2.65).

### Beispiel 87

### (5RS)-5-[(3,3-Difluorpyrrolidin-1-yl)carbonyl]-2-(5,6,7,8-tetrahydronaphthalin-2-ylmethyl)-5,6,7,8-tetrahydro[1,2,4]triazolo[4,3-a]pyridin-3(2H)-on (Racemat)

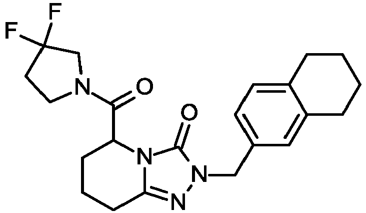

(5RS)-3-Oxo-2-(5,6,7,8-tetrahydronaphthalin-2-ylmethyl)-2,3,5,6,7,8-hexahydro[1,2,4]triazolo[4,3-a]pyridin-5-carbonsäure -trifluoressigsäure (Racemat) (100 mg, 227 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (112 mg, 295 µmol) und Triethylamin (160 µl, 1.1 mmol) zugegeben. Nach 15 min Rühren wurde 3,3-Difluorpyrrolidinhydrochlorid (39.0 mg, 272 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 44.0 mg (47 % d. Th.) der Titelverbindung erhalten.
LC-MS (Methode 1): Rₜ = 1.25 min; MS (ESIpos): m/z = 417 [M+H]⁺
¹H-NMR (400 MHz, DMSO-d6) δ [ppm]: -0.149 (1.52), -0.008 (13.87), 0.008 (12.34), 0.146 (1.37), 1.708 (13.94), 1.996 (2.59), 2.327 (4.65), 2.366 (3.20), 2.523 (15.54), 2.565 (4.80), 2.584 (4.19), 2.598 (3.73), 2.669 (16.00), 2.702 (5.18), 2.718 (5.79), 2.734 (3.05), 3.530 (2.29), 3.554 (3.20), 3.668 (1.75), 3.702 (2.06), 3.736 (1.75), 3.769 (2.13), 3.805 (3.20), 3.910 (1.68), 3.988 (1.37), 4.171 (1.22), 4.685 (5.26), 4.700 (5.64), 4.729 (7.54), 4.740 (8.46), 4.821 (1.83), 6.843 (2.67), 6.860 (3.43), 6.916 (8.15), 6.933 (3.58), 6.979 (4.72), 6.988 (4.88), 7.009 (4.19), 7.028 (4.11), 7.047 (1.60), 7.069 (1.07).

### Beispiel 88

### (5RS)-5-(Pyrrolidin-1-ylcarbonyl)-2-(5,6,7,8-tetrahydronaphthalin-2-ylmethyl)-5,6,7,8-tetrahydro[1,2,4]triazolo[4,3-a]pyridin-3(2H)-on (Racemat)

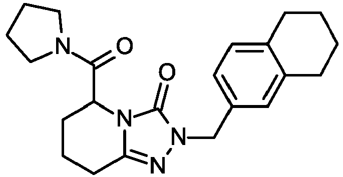

(5RS)-3-Oxo-2-(5,6,7,8-tetrahydronaphthalin-2-ylmethyl)-2,3,5,6,7,8-hexahydro[1,2,4]triazolo[4,3-a]pyridin-5-carbonsäure -trifluoressigsäure (Racemat) (100 mg, 227 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (112 mg, 295 µmol) und Triethylamin (160 µl, 1.1 mmol) zugegeben. Nach 15 min Rühren wurde Pyrrolidin (19.3 mg, 272 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 39.0 mg (45 % d. Th.) der Titelverbindung erhalten.
LC-MS (Methode 3): Rₜ = 1.60 min; MS (ESIpos): m/z = 381 [M+H]⁺
¹H-NMR (400 MHz, DMSO-d6) δ [ppm]: -0.149 (0.77), -0.008 (6.64), 0.008 (5.97), 0.146 (0.77), 1.700 (12.96), 1.708 (16.00), 1.715 (12.30), 1.754 (4.68), 1.772 (7.72), 1.789 (11.63), 1.806 (8.90), 1.822 (2.52), 1.892 (2.73), 1.908 (8.28), 1.925 (10.60), 1.942 (6.89), 1.959 (3.04), 1.983 (3.04), 1.998 (3.14), 2.014 (2.52), 2.025 (2.57), 2.041 (1.90), 2.050 (1.54), 2.061 (0.93), 2.322 (1.49), 2.327 (2.01), 2.332 (1.49), 2.366 (1.23), 2.518 (10.19), 2.523 (9.67), 2.563 (3.24), 2.578 (3.81), 2.590 (4.01), 2.604 (2.11), 2.620 (1.65), 2.632 (1.75), 2.669 (14.05), 2.686 (8.90), 2.703 (6.02), 2.718 (6.07), 2.734 (3.24), 2.865 (1.49), 3.225 (1.13), 3.243 (2.32), 3.255 (2.88), 3.272 (4.12), 3.290 (3.19), 3.339 (5.20), 3.356 (2.78), 3.368 (2.83), 3.386 (1.39), 3.435 (1.65), 3.453 (3.55), 3.460 (2.62), 3.477 (4.48), 3.494 (1.95), 3.593 (1.90), 3.609 (4.12), 3.626 (2.37), 3.634 (3.14), 3.651 (1.49), 4.639 (1.29), 4.677 (6.33), 4.695 (6.28), 4.712 (2.62), 4.722 (10.08), 4.733 (11.37), 4.744 (3.60), 4.753 (2.47), 4.772 (1.18), 6.846 (2.78), 6.862 (3.40), 6.917 (7.97), 6.932 (3.91), 6.969 (2.11), 6.978 (5.09), 6.984 (5.30), 6.998 (2.73), 7.008 (4.32), 7.027 (4.78), 7.045 (1.59).

### Beispiel 89

### (5RS)-5-(Pyrrolidin-1-ylcarbonyl)-2-(2,4,5-trifluorbenzyl)-5,6,7,8-tetrahydro[1,2,4]triazolo[4,3-a]pyridin-3(2H)-on (Racemat)

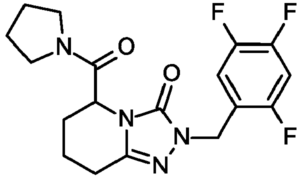

(5RS)-3-Oxo-2-(2,4,5-trifluorbenzyl)-2,3,5,6,7,8-hexahydro[1,2,4]triazolo[4,3-a]pyridin-5-carbonsäure -trifluoressigsäure (Racemat) (100 mg, 227 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (112 mg, 295 µmol) und Triethylamin (160 µl, 1.1 mmol) zugegeben. Nach 15 min Rühren wurde Pyrrolidin (19.3 mg, 272 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 51.0 mg (59 % d. Th.) der Titelverbindung erhalten.
LC-MS (Methode 4): Rₜ = 0.75 min; MS (ESIpos): m/z = 381 [M+H]⁺
¹H-NMR (400 MHz, DMSO-d6) δ [ppm]: -0.150 (0.92), -0.008 (8.02), 0.008 (7.59), 1.716 (5.33), 1.754 (3.14), 1.773 (9.33), 1.790 (15.97), 1.807 (12.47), 1.824 (3.73), 1.892 (3.99), 1.909 (11.06), 1.925 (13.71), 1.942 (8.61), 2.001 (6.15), 2.028 (3.60), 2.327 (1.80), 2.365 (1.24), 2.571 (4.25), 2.588 (4.06), 2.599 (7.26), 2.611 (4.32), 2.641 (3.21), 2.669 (1.96), 3.226 (2.19), 3.243 (4.71), 3.256 (4.38), 3.272 (8.18), 3.290 (4.32), 3.341 (8.44), 3.358 (4.84), 3.370 (4.74), 3.388 (2.32), 3.435 (2.39), 3.453 (5.37), 3.478 (6.94), 3.495 (2.98), 3.589 (3.11), 3.606 (5.92), 3.631 (4.84), 3.647 (2.32), 4.732 (5.89), 4.747 (7.69), 4.756 (5.73), 4.785 (2.65), 4.825 (16.00), 4.840 (15.90), 4.880 (2.81), 7.259 (3.34), 7.282 (4.22), 7.302 (3.96), 7.325 (3.08), 7.538 (3.66), 7.555 (3.89), 7.565 (5.04), 7.580 (5.24), 7.589 (3.96), 7.606 (3.37).

### Beispiel 90

### (5RS)-5-[(3,3-Difluorpyrrolidin-1-yl)carbonyl]-2-[(1-methyl-1H-pyrazol-3-yl)methyl]-5,6,7,8-tetrahydro[1,2,4]triazolo[4,3-a]pyridin-3(2H)-on (Racemat)

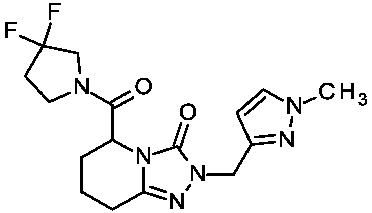

(5RS)-2-[(1-Methyl-1H-pyrazol-3-yl)methyl]-3-oxo-2,3,5,6,7,8-hexahydro[1,2,4]triazolo[4,3-a]pyridin-5-carbonsäure -trifluoressigsäure (Racemat) (170 mg, 96 % Reinheit, 417 µmol) wurde in THF (3.3 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (206 mg, 542 µmol) und Triethylamin (290 µl, 2.1 mmol) zugegeben. Nach 15 min Rühren wurde 3,3-Difluorpyrrolidinhydrochlorid (71.9 mg, 500 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 7.00 mg (4 % d. Th.) der Titelverbindung erhalten.
LC-MS (Methode 4): Rₜ = 0.54 min; MS (ESIpos): m/z = 367 [M+H]⁺
¹H-NMR (400 MHz, DMSO-d6) δ [ppm]: -0.008 (1.35), 0.008 (1.13), 1.700 (0.76), 1.982 (0.52), 2.044 (0.44), 2.429 (0.43), 2.566 (1.33), 2.580 (1.08), 3.529 (0.54), 3.549 (0.71), 3.663 (0.43), 3.696 (0.54), 3.730 (0.41), 3.774 (16.00), 3.797 (0.64), 3.908 (0.48), 4.693 (3.32), 4.702 (3.80), 4.741 (0.50), 4.783 (0.41), 4.798 (0.51), 4.807 (0.42), 6.032 (2.54), 6.038 (2.55), 7.571 (2.52), 7.577 (2.49).

### Beispiel 91

### (5RS)-5-[(3,3-Difluorpyrrolidin-1-yl)carbonyl]-2-(2,4,5-trifluorbenzyl)-5,6,7,8-tetrahydro[1,2,4]triazolo[4,3-a]pyridin-3(2H)-on (Racemat)

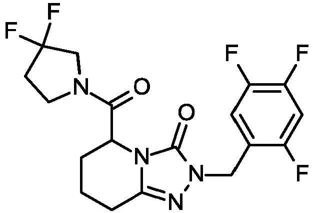

(5RS)-3-Oxo-2-(2,4,5-trifluorbenzyl)-2,3,5,6,7,8-hexahydro[1,2,4]triazolo[4,3-a]pyridin-5-carbonsäure -trifluoressigsäure (Racemat) (100 mg, 227 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (112 mg, 295 µmol) und Triethylamin (160 µl, 1.1 mmol) zugegeben. Nach 15 min Rühren wurde 3,3-Difluorpyrrolidinhydrochlorid (39.0 mg, 272 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 25.0 mg (94 % Reinheit, 25 % d. Th.) der Titelverbindung erhalten.
LC-MS (Methode 4): Rₜ = 0.78 min; MS (ESIpos): m/z = 417 [M+H]⁺
¹H-NMR (400 MHz, DMSO-d6) δ [ppm]: -0.149 (0.95), 0.008 (5.95), 1.727 (3.05), 2.007 (3.36), 2.327 (3.00), 2.366 (1.95), 2.560 (5.45), 2.570 (6.68), 2.586 (6.00), 2.604 (6.32), 2.669 (2.68), 3.536 (3.14), 3.556 (4.45), 3.669 (2.59), 3.702 (3.18), 3.738 (2.64), 3.771 (3.36), 3.805 (4.82), 3.888 (1.50), 3.907 (3.09), 3.933 (2.23), 3.989 (1.86), 4.033 (1.91), 4.175 (1.64), 4.201 (1.68), 4.765 (3.45), 4.791 (2.09), 4.831 (15.50), 4.845 (16.00), 4.885 (2.36), 7.263 (2.77), 7.286 (3.82), 7.303 (3.73), 7.330 (3.05), 7.540 (2.95), 7.557 (3.59), 7.564 (4.77), 7.582 (4.55), 7.591 (3.41), 7.608 (3.18).

### Beispiel 92

### (5RS)-2-[(6-Methoxypyridin-3-yl)methyl]-5-(pyrrolidin-1-ylcarbonyl)-5,6,7,8-tetrahydro[1,2,4]triazolo[4,3-a]pyridin-3(2H)-on (Racemat)

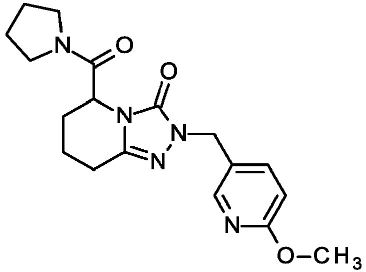

(5RS)-2-[(6-Methoxypyridin-3-yl)methyl]-3-oxo-2,3,5,6,7,8-hexahydro[1,2,4]triazolo[4,3-a]pyridin-5-carbonsäure (Racemat) (44.6 mg, 147 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (72.4 mg, 191 µmol) und Triethylamin (61 µl, 440 µmol) zugegeben. Nach 15 min Rühren wurde Pyrrolidin (12.5 mg, 176 µmol) zugegeben und das Reaktionsgemisch für 48 Stunden bei Raumtemperatur gerührt. Es wurde erneut 1-[Bis(dimethylamino)methylen]-1H-[1,2,3]triazolo[4,5-b]pyridin-1-ium-3-oxidhexafluorophosphat (72.4 mg, 191 µmol), Triethylamin (61 µl, 440 µmol) und Pyrrolidin (12.5 mg, 176 µmol) zugegeben und über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 12.7 mg (24 % d. Th.) der Titelverbindung erhalten.
LC-MS (Methode 3): Rₜ = 0.99 min; MS (ESIpos): m/z = 358 [M+H]⁺
¹H-NMR (400 MHz, DMSO-d6) δ [ppm]: -0.008 (2.07), 0.008 (1.18), 1.693 (0.72), 1.705 (0.80), 1.715 (0.62), 1.771 (1.14), 1.789 (2.00), 1.806 (1.55), 1.822 (0.43), 1.891 (0.57), 1.908 (1.48), 1.924 (1.84), 1.941 (1.24), 1.960 (0.57), 1.973 (0.63), 1.985 (0.59), 2.001 (0.43), 2.016 (0.51), 2.523 (1.70), 2.571 (0.63), 2.583 (1.01), 2.595 (0.58), 2.624 (0.41), 2.669 (0.40), 2.865 (1.35), 3.239 (0.63), 3.252 (0.63), 3.269 (1.20), 3.286 (1.14), 3.340 (1.20), 3.352 (0.55), 3.357 (0.67), 3.369 (0.68), 3.449 (0.71), 3.457 (0.53), 3.466 (0.43), 3.474 (0.86), 3.589 (0.42), 3.605 (0.82), 3.614 (0.43), 3.622 (0.47), 3.630 (0.64), 3.826 (16.00), 4.712 (0.78), 4.721 (0.89), 4.727 (1.04), 4.736 (0.82), 4.760 (5.47), 6.783 (1.59), 6.804 (1.70), 7.552 (1.12), 7.558 (1.13), 7.573 (1.08), 7.580 (1.07), 8.050 (1.33), 8.055 (1.26).

### Beispiel 93

### (5RS)-5-(Pyrrolidin-1-ylcarbonyl)-2-{[4-(trifluormethyl)pyridin-2-yl]methyl}-5,6,7,8-tetrahydro[1,2,4]triazolo[4,3-a]pyridin-3(2H)-on (Enantiomer 1)

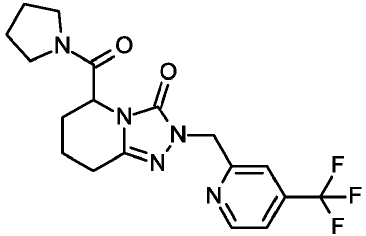

(5RS)-3-Oxo-2-{[4-(trifluormethyl)pyridin-2-yl]methyl}-2,3,5,6,7,8-hexahydro[1,2,4]triazolo[4,3-a]pyridin-5-carbonsäure (Enantiomer 1) (55.0 mg, 161 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (79.4 mg, 209 µmol) und Triethylamin (67 µl, 480 µmol) zugegeben. Nach 15 min Rühren wurde Pyrrolidin (13.7 mg, 193 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 51.0 mg (80 % d. Th.) der Titelverbindung erhalten.
LC-MS (Methode 4): Rt = 0.68 min; MS (ESIpos): m/z = 396 [M+H]⁺
¹H-NMR (400 MHz, DMSO-d6) δ [ppm]: -0.008 (2.19), 0.008 (1.87), 1.681 (1.14), 1.693 (1.55), 1.707 (2.19), 1.718 (3.14), 1.731 (3.80), 1.741 (3.09), 1.755 (2.58), 1.773 (5.88), 1.790 (9.64), 1.807 (7.28), 1.824 (2.17), 1.894 (2.32), 1.910 (6.68), 1.927 (8.29), 1.944 (4.94), 1.962 (1.50), 1.974 (1.07), 1.986 (1.05), 2.000 (1.63), 2.010 (3.01), 2.022 (3.35), 2.034 (2.19), 2.050 (2.32), 2.060 (1.68), 2.067 (1.61), 2.075 (1.61), 2.085 (0.90), 2.096 (0.54), 2.102 (0.54), 2.111 (0.45), 2.328 (0.54), 2.366 (0.41), 2.524 (2.38), 2.559 (3.20), 2.569 (2.81), 2.583 (2.36), 2.597 (2.30), 2.609 (4.19), 2.621 (2.38), 2.639 (1.03), 2.650 (1.59), 2.664 (1.03), 2.670 (0.75), 2.710 (0.49), 2.731 (0.75), 2.865 (1.20), 2.890 (0.97), 3.232 (1.33), 3.249 (2.77), 3.261 (2.75), 3.278 (5.03), 3.296 (3.41), 3.341 (5.30), 3.353 (1.98), 3.358 (2.73), 3.370 (2.83), 3.388 (1.29), 3.445 (1.44), 3.462 (3.18), 3.470 (2.32), 3.479 (1.89), 3.487 (4.06), 3.504 (1.80), 3.597 (1.87), 3.614 (3.82), 3.622 (1.93), 3.630 (2.17), 3.638 (2.94), 3.655 (1.33), 4.764 (3.44), 4.773 (3.82), 4.779 (4.51), 4.788 (3.39), 4.992 (0.86), 5.034 (15.89), 5.037 (16.00), 5.079 (0.88), 7.555 (8.10), 7.701 (4.21), 7.713 (4.40), 8.813 (5.84), 8.826 (5.80).

Die (5S)-Konfiguration wurde aufgrund der Kristallstrukturaufklärung für Beispiel 26, 108, 113, 157, 237, 358 und 454 zugeordnet.

### (5S)-5-(Pyrrolidin-1-ylcarbonyl)-2-{[4-(trifluormethyl)pyridin-2-yl]methyl}-5,6,7,8-tetrahydro[1,2,4]triazolo[4,3-a]pyridin-3(2H)-on

### Beispiel 94

### (5RS)-5-(Pyrrolidin-1-ylcarbonyl)-2-{[2-(trifluormethyl)pyrimidin-4-yl]methyl}-5,6,7,8-tetrahydro[1,2,4]triazolo[4,3-a]pyridin-3(2H)-on (Enantiomer 1)

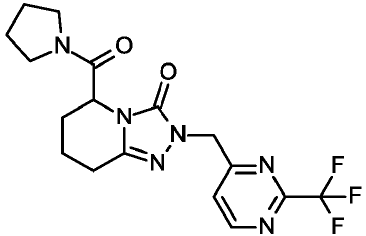

(5RS)-3-Oxo-2-{[2-(trifluormethyl)pyrimidin-4-yl]methyl}-2,3,5,6,7,8-hexahydro[1,2,4]triazolo[4,3-a]pyridin-5-carbonsäure (Enantiomer 1) (45.0 mg, 131 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (64.8 mg, 170 µmol) und Triethylamin (55 µl, 390 µmol) zugegeben. Nach 15 min Rühren wurde Pyrrolidin (11.2 mg, 157 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 31.4 mg (100 % Reinheit, 60 % d. Th.) der Titelverbindung erhalten.
LC-MS (Methode 4): Rₜ = 0.65 min; MS (ESIpos): m/z = 397 [M+H]⁺
¹H-NMR (400 MHz, DMSO-d6) δ [ppm]: -0.008 (2.47), 0.008 (2.16), 1.699 (1.16), 1.712 (1.27), 1.722 (1.22), 1.757 (2.55), 1.776 (4.88), 1.793 (7.57), 1.810 (5.49), 1.827 (1.56), 1.894 (1.25), 1.911 (3.41), 1.927 (4.31), 1.943 (2.78), 1.960 (0.86), 2.005 (0.61), 2.017 (0.64), 2.030 (1.24), 2.043 (2.93), 2.053 (3.15), 2.068 (1.74), 2.079 (1.22), 2.086 (1.16), 2.094 (1.11), 2.327 (0.73), 2.367 (0.49), 2.523 (2.15), 2.571 (2.36), 2.586 (2.09), 2.597 (2.01), 2.611 (1.69), 2.625 (1.66), 2.636 (2.90), 2.649 (1.72), 2.666 (1.24), 2.679 (1.36), 2.690 (0.68), 2.710 (0.54), 3.242 (1.01), 3.259 (1.98), 3.272 (2.00), 3.288 (3.53), 3.335 (1.77), 3.353 (3.53), 3.364 (1.28), 3.370 (1.84), 3.382 (1.99), 3.400 (0.93), 3.453 (1.01), 3.471 (2.24), 3.478 (1.66), 3.488 (1.33), 3.496 (2.90), 3.513 (1.27), 3.599 (1.36), 3.615 (2.51), 3.624 (1.36), 3.633 (1.62), 3.641 (1.96), 3.657 (0.95), 4.785 (2.52), 4.794 (2.77), 4.799 (3.42), 4.808 (2.43), 5.035 (0.44), 5.080 (16.00), 5.125 (0.42), 7.480 (5.36), 7.493 (5.54), 9.047 (6.50), 9.060 (6.45).

Die (5S)-Konfiguration wurde aufgrund der Kristallstrukturaufklärung für Beispiel 26, 108, 113, 157, 237, 358 und 454 zugeordnet.

### (5S)-5-(Pyrrolidin-1-ylcarbonyl)-2-{[2-(trifluormethyl)pyrimidin-4-yl]methyl}-5,6,7,8-tetrahydro[1,2,4]triazolo[4,3-a]pyridin-3(2H)-on

### Beispiel 95

### (5RS)-2-(3-Chlor-4-fluorbenzyl)-5-[(3-hydroxyazetidin-1-yl)carbonyl]-5,6,7,8-tetrahydro[1,2,4]triazolo[4,3-a]pyridin-3(2H)-on (Racemat)

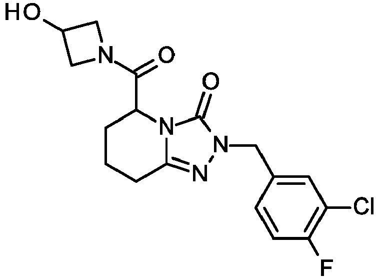

(5RS)-2-(3-Chlor-4-fluorbenzyl)-3-oxo-2,3,5,6,7,8-hexahydro[1,2,4]triazolo[4,3-a]pyridin-5-carbonsäurehydrochlorid (Racemat) (193 mg, 532 µmol) wurde in THF (5.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (263 mg, 692 µmol) und Triethylamin (300 µl, 2.1 mmol) zugegeben. Nach 15 min Rühren wurde Azetidin-3-olhydrochlorid (70.0 mg, 639 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 63.4 mg (31 % d. Th.) der Titelverbindung erhalten.
LC-MS (Methode 3): Rₜ = 1.17 min; MS (ESIpos): m/z = 381 [M+H]⁺
¹H-NMR (400 MHz, DMSO-d6) δ [ppm]: -0.008 (1.65), 0.008 (1.43), 1.677 (2.01), 1.695 (2.51), 1.709 (2.94), 1.718 (3.05), 1.902 (1.44), 1.927 (2.11), 1.938 (3.11), 1.994 (2.08), 2.004 (2.25), 2.016 (2.08), 2.073 (3.94), 2.327 (0.59), 2.366 (0.45), 2.572 (2.33), 2.580 (3.06), 2.592 (5.37), 2.605 (2.83), 2.622 (1.29), 2.634 (1.98), 2.646 (0.91), 2.669 (0.64), 2.690 (0.67), 2.710 (0.49), 3.596 (1.70), 3.603 (1.93), 3.621 (2.05), 3.629 (2.25), 3.638 (1.86), 3.649 (1.84), 3.664 (1.78), 3.674 (1.75), 3.921 (2.08), 3.932 (2.28), 3.952 (0.58), 4.021 (3.54), 4.045 (3.84), 4.055 (2.17), 4.062 (1.96), 4.105 (1.41), 4.120 (1.77), 4.131 (1.50), 4.146 (1.38), 4.339 (1.43), 4.357 (2.42), 4.378 (1.68), 4.473 (1.22), 4.485 (2.84), 4.502 (5.81), 4.520 (8.36), 4.531 (6.20), 4.546 (2.47), 4.818 (15.96), 4.823 (16.00), 5.789 (3.64), 5.802 (7.82), 5.817 (4.83), 7.223 (2.47), 7.229 (2.57), 7.244 (3.60), 7.250 (3.33), 7.256 (3.17), 7.367 (3.18), 7.371 (3.21), 7.390 (5.20), 7.411 (2.59), 7.416 (2.59), 7.434 (4.30), 7.452 (4.27).

### Beispiel 96

### (5RS)-5-[(3-Fluorazetidin-1-yl)carbonyl]-2-{[6-(trifluormethyl)pyridin-3-yl]methyl}-5,6,7,8-tetrahydro[1,2,4]triazolo[4,3-a]pyridin-3(2H)-on (Racemat)

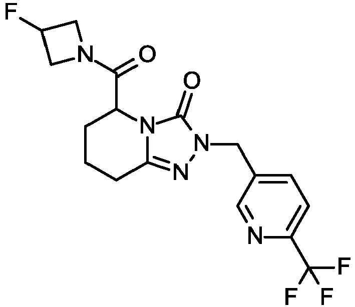

(5RS)-3-Oxo-2-{[6-(trifluormethyl)pyridin-3-yl]methyl}-2,3,5,6,7,8-hexahydro[1,2,4]triazolo[4,3-a]pyridin-5-carbonsäure (Racemat) (60.0 mg, 175 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (86.6 mg, 228 µmol) und Triethylamin (73 µl, 530 µmol) zugegeben. Nach 15 min Rühren wurde 3-Fluorazetidinhydrochlorid (23.5 mg, 210 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 30.0 mg (43 % d. Th.) der Titelverbindung erhalten.
LC-MS (Methode 3): Rₜ = 1.18 min; MS (ESIpos): m/z = 400 [M+H]⁺
¹H-NMR (400 MHz, DMSO-d6) δ [ppm]: -0.150 (0.47), -0.008 (4.45), 0.008 (3.73), 0.146 (0.42), 1.666 (1.19), 1.675 (1.54), 1.690 (1.68), 1.700 (2.17), 1.715 (2.43), 1.968 (1.63), 2.030 (1.47), 2.073 (1.68), 2.366 (0.79), 2.518 (5.13), 2.523 (4.20), 2.560 (2.38), 2.586 (3.13), 2.599 (4.31), 2.613 (2.17), 2.628 (0.84), 2.641 (1.42), 2.655 (0.68), 2.710 (0.84), 3.901 (0.56), 3.930 (1.12), 3.963 (1.21), 3.991 (1.12), 4.026 (0.68), 4.160 (0.49), 4.175 (0.54), 4.228 (1.05), 4.244 (0.98), 4.256 (0.93), 4.277 (1.24), 4.297 (1.10), 4.327 (0.82), 4.366 (0.91), 4.399 (0.72), 4.438 (0.51), 4.460 (0.70), 4.509 (0.54), 4.524 (0.68), 4.557 (2.92), 4.568 (4.36), 4.581 (2.92), 4.638 (0.47), 4.650 (0.56), 4.687 (0.54), 4.704 (0.56), 5.011 (11.69), 5.353 (0.77), 5.408 (0.72), 5.496 (0.75), 5.551 (0.72), 7.912 (16.00), 8.649 (6.27).

### Beispiel 97

### (5RS)-2-(3-Chlor-4-fluorbenzyl)-5-{[(3S)-3-hydroxypyrrolidin-1-yl]carbonyl}-5,6,7,8-tetrahydro[1,2,4]triazolo[4,3-a]pyridin-3(2H)-on (Enantiomer 1)

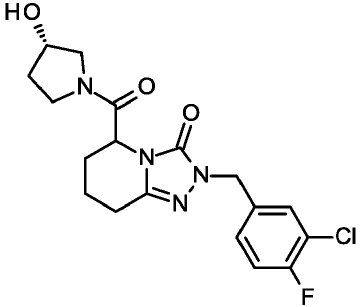

(5RS)-2-(3-Chlor-4-fluorbenzyl)-3-oxo-2,3,5,6,7,8-hexahydro[1,2,4]triazolo[4,3-a]pyridin-5-carbonsäure (Enantiomer 1) (55.0 mg, 169 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (83.5 mg, 220 µmol) und Triethylamin (71 µl, 510 µmol) zugegeben. Nach 15 min Rühren wurde (3S)-Pyrrolidin-3-olhydrochlorid (25.0 mg, 203 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 66.0 mg (97 % d. Th.) der Titelverbindung erhalten.
LC-MS (Methode 3): Rₜ = 1.19 min; MS (ESIpos): m/z = 395 [M+H]⁺
¹H-NMR (400 MHz, DMSO-d6) δ [ppm]: -0.008 (2.74), 0.008 (2.46), 1.668 (1.27), 1.681 (1.28), 1.731 (2.93), 1.741 (2.61), 1.754 (2.32), 1.763 (2.04), 1.827 (0.60), 1.839 (0.73), 1.849 (1.61), 1.860 (1.98), 1.872 (1.68), 1.882 (2.15), 1.892 (1.71), 1.904 (1.25), 1.972 (2.44), 1.983 (2.94), 1.994 (2.98), 2.005 (2.63), 2.015 (2.47), 2.023 (2.13), 2.029 (2.09), 2.038 (1.82), 2.050 (1.44), 2.058 (1.57), 2.066 (1.43), 2.073 (5.11), 2.086 (0.54), 2.327 (0.61), 2.366 (0.49), 2.523 (3.02), 2.571 (1.77), 2.580 (1.19), 2.593 (2.23), 2.604 (3.72), 2.616 (2.00), 2.634 (1.06), 2.646 (1.52), 2.660 (0.87), 2.669 (0.81), 2.710 (0.61), 2.731 (3.89), 2.877 (0.63), 2.890 (5.16), 3.199 (1.00), 3.231 (1.38), 3.298 (3.79), 3.339 (2.01), 3.375 (2.71), 3.397 (1.82), 3.415 (0.81), 3.432 (0.70), 3.442 (0.77), 3.454 (1.37), 3.461 (1.36), 3.470 (0.68), 3.483 (1.53), 3.520 (0.41), 3.545 (1.20), 3.562 (1.93), 3.569 (2.22), 3.585 (1.42), 3.640 (1.08), 3.651 (1.61), 3.667 (1.13), 3.678 (1.28), 3.734 (0.54), 3.754 (0.82), 3.774 (0.43), 4.268 (1.82), 4.362 (1.80), 4.687 (0.98), 4.695 (1.13), 4.702 (1.23), 4.710 (0.99), 4.738 (2.01), 4.745 (1.57), 4.794 (1.01), 4.817 (16.00), 4.955 (4.61), 4.964 (4.28), 5.073 (3.17), 5.075 (3.09), 5.082 (3.27), 7.222 (2.22), 7.227 (2.23), 7.238 (2.55), 7.243 (3.00), 7.249 (2.85), 7.255 (2.74), 7.362 (0.47), 7.371 (4.52), 7.394 (6.84), 7.406 (0.56), 7.416 (3.64), 7.430 (3.15), 7.448 (3.23), 7.953 (0.53).

Die (5S)-Konfiguration wurde aufgrund der Kristallstrukturaufklärung für Beispiel 26, 108, 113, 157, 237, 358 und 454 zugeordnet.

### (5S)-2-(3-Chlor-4-fluorbenzyl)-5-{[(3S)-3-hydroxypyrrolidin-1-yl]carbonyl}-5,6,7,8-tetrahydro[1,2,4]triazolo[4,3-a]pyridin-3(2H)-on

### Beispiel 98

### (5RS)-5-[(3-Hydroxyazetidin-1-yl)carbonyl]-2-[3-(trifluormethyl)benzyl]-5,6,7,8-tetrahydro[1,2,4]triazolo[4,3-a]pyridin-3(2H)-on (Enantiomer 1)

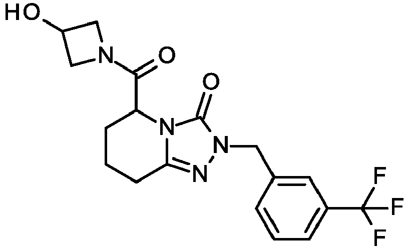

(5RS)-3-Oxo-2-[3-(trifluormethyl)benzyl]-2,3,5,6,7,8-hexahydro[1,2,4]triazolo[4,3-a]pyridin-5-carbonsäure (Enantiomer 1) (55.0 mg, 161 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (79.7 mg, 209 µmol) und Triethylamin (67 µl, 480 µmol) zugegeben. Nach 15 min Rühren wurde Azetidin-3-olhydrochlorid (21.2 mg, 193 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 7.90 mg (12 % d. Th.) der Titelverbindung erhalten.
LC-MS (Methode 3): Rₜ = 1.24 min; MS (ESIpos): m/z = 397 [M+H]⁺
¹H-NMR (500 MHz, DMSO-d6) δ [ppm]: 1.666 (2.27), 1.676 (2.26), 1.719 (3.03), 1.920 (1.91), 1.946 (3.45), 2.013 (2.68), 2.020 (2.73), 2.072 (11.94), 2.588 (2.90), 2.597 (5.00), 2.606 (2.91), 2.621 (1.59), 2.631 (2.38), 2.690 (0.66), 3.608 (2.01), 3.623 (2.15), 3.629 (2.19), 3.644 (1.80), 3.652 (1.89), 3.663 (1.89), 3.672 (1.75), 3.928 (2.17), 3.938 (2.46), 3.953 (0.71), 4.029 (3.39), 4.040 (3.81), 4.047 (4.10), 4.059 (2.67), 4.109 (1.41), 4.122 (1.81), 4.130 (1.69), 4.141 (1.49), 4.343 (1.52), 4.358 (2.55), 4.374 (1.66), 4.404 (0.44), 4.495 (3.28), 4.510 (4.92), 4.521 (7.10), 4.533 (5.97), 4.541 (4.89), 4.925 (15.15), 4.931 (16.00), 5.817 (8.02), 7.510 (3.60), 7.524 (5.29), 7.574 (2.93), 7.589 (5.35), 7.604 (3.34), 7.623 (9.33), 7.649 (6.87), 7.664 (4.72).

Die (5S)-Konfiguration wurde aufgrund der Kristallstrukturaufklärung für Beispiel 26, 108, 113, 157, 237, 358 und 454 zugeordnet.

### (5S)-5-[(3-Hydroxyazetidin-1 -yl)carbonyl]-2-[3-(trifluormethyl)benzyl]-5,6,7,8-tetrahydro[1,2,4]triazolo[4,3-a]pyridin-3(2H)-on

### Beispiel 99

### (5RS)-5-{[(3S)-3-Hydroxypyrrolidin-1-yl]carbonyl}-2-[3-(trifluormethyl)benzyl]-5,6,7,8-tetrahydro[1,2,4]triazolo[4,3-a]pyridin-3(2H)-on (Enantiomer 1)

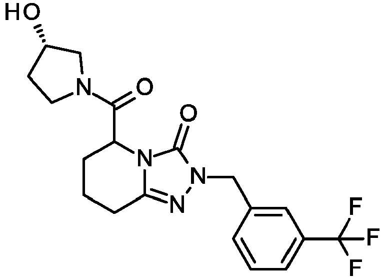

(5RS)-3-Oxo-2-[3-(trifluormethyl)benzyl]-2,3,5,6,7,8-hexahydro[1,2,4]triazolo[4,3-a]pyridin-5-carbonsäure (Enantiomer 1) (55.0 mg, 161 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (79.7 mg, 209 µmol) und Triethylamin (67 µl, 480 µmol) zugegeben. Nach 15 min Rühren wurde (3S)-Pyrrolidin-3-olhydrochlorid (23.9 mg, 193 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 10.9 mg (16 % d. Th.) der Titelverbindung erhalten.
LC-MS (Methode 3): Rₜ = 1.26 min; MS (ESIpos): m/z = 411 [M+H]⁺
¹H-NMR (500 MHz, DMSO-d6) δ [ppm]: -0.007 (2.44), 0.006 (1.71), 1.677 (1.35), 1.733 (2.92), 1.753 (2.40), 1.835 (0.67), 1.845 (0.79), 1.853 (1.67), 1.862 (2.30), 1.871 (1.79), 1.879 (2.36), 1.888 (1.98), 1.958 (1.81), 1.967 (2.10), 1.976 (2.36), 1.985 (2.86), 1.994 (2.94), 2.003 (2.57), 2.010 (2.04), 2.024 (2.04), 2.031 (2.30), 2.044 (1.74), 2.053 (1.72), 2.060 (1.78), 2.066 (1.45), 2.072 (2.04), 2.087 (0.58), 2.362 (0.62), 2.519 (2.96), 2.563 (1.74), 2.574 (0.72), 2.608 (3.40), 2.640 (1.92), 2.689 (0.42), 2.877 (0.45), 3.205 (1.08), 3.230 (1.45), 3.353 (1.74), 3.369 (2.56), 3.375 (2.51), 3.393 (2.14), 3.400 (1.78), 3.412 (0.69), 3.436 (0.87), 3.444 (0.78), 3.454 (1.01), 3.461 (1.82), 3.484 (1.52), 3.530 (0.41), 3.550 (1.21), 3.563 (2.45), 3.571 (2.19), 3.583 (1.63), 3.647 (1.29), 3.655 (1.90), 3.668 (1.25), 3.677 (1.47), 3.741 (0.57), 3.755 (0.87), 3.772 (0.49), 4.266 (2.09), 4.366 (2.09), 4.392 (0.46), 4.403 (0.66), 4.699 (1.20), 4.705 (1.32), 4.711 (1.42), 4.717 (1.14), 4.742 (1.79), 4.748 (2.20), 4.754 (1.94), 4.806 (0.98), 4.813 (1.09), 4.818 (1.14), 4.825 (0.90), 4.896 (0.42), 4.923 (16.00), 4.968 (1.22), 5.089 (1.29), 7.218 (0.52), 7.507 (2.86), 7.523 (4.44), 7.540 (0.69), 7.574 (3.29), 7.590 (6.42), 7.605 (3.61), 7.621 (6.90), 7.647 (6.52), 7.663 (4.44).

Die (5S)-Konfiguration wurde aufgrund der Kristallstrukturaufklärung für Beispiel 26, 108, 113, 157, 237, 358 und 454 zugeordnet.

### (5S)-5-{[(3S)-3-Hydroxypyrrolidin-1-yl]carbonyl}-2-[3-(trifluormethyl)benzyl]-5,6,7,8-tetrahydro[1,2,4]triazolo[4,3-a]pyridin-3(2H)-on

### Beispiel 100

### (5RS)-5-{[(3R)-3-Fluorpyrrolidin-1-yl]carbonyl}-2-{[6-(trifluormethyl)pyridin-3-yl]methyl}-5,6,7,8-tetrahydro[1,2,4]triazolo[4,3-a]pyridin-3(2H)-on (Diastereomerengemisch; 2 Isomere)

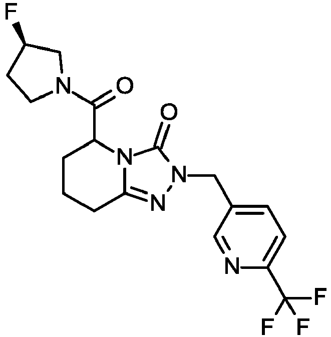

(5RS)-3-Oxo-2-{[6-(trifluormethyl)pyridin-3-yl]methyl}-2,3,5,6,7,8-hexahydro[1,2,4]triazolo[4,3-a]pyridin-5-carbonsäure (Racemat) (60.0 mg, 175 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (86.6 mg, 228 µmol) und Triethylamin (73 µl, 530 µmol) zugegeben. Nach 15 min Rühren wurde (3R)-3-Fluorpyrrolidinhydrochlorid (26.4 mg, 210 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 29.0 mg (40 % d. Th.) der Titelverbindung erhalten.
LC-MS (Methode 3): Rₜ = 1.21 min; MS (ESIpos): m/z = 414 [M+H]⁺
¹H-NMR (400 MHz, DMSO-d6) δ [ppm]: -0.149 (0.59), -0.008 (5.76), 0.008 (4.91), 0.146 (0.59), 1.658 (0.69), 1.736 (1.64), 2.005 (1.91), 2.016 (1.59), 2.067 (1.00), 2.103 (1.54), 2.140 (1.08), 2.270 (0.80), 2.327 (0.74), 2.366 (0.49), 2.518 (3.66), 2.523 (2.83), 2.571 (1.61), 2.599 (1.44), 2.611 (2.42), 2.623 (1.49), 2.653 (0.91), 2.669 (1.07), 2.710 (0.54), 3.369 (0.81), 3.387 (0.58), 3.398 (0.81), 3.524 (1.13), 3.548 (0.97), 3.573 (0.86), 3.600 (1.61), 3.636 (1.07), 3.659 (1.08), 3.681 (1.00), 3.702 (0.56), 3.726 (0.56), 3.747 (1.08), 3.775 (0.80), 3.788 (0.80), 3.856 (1.07), 3.941 (0.74), 4.009 (0.41), 4.694 (0.52), 4.703 (0.64), 4.710 (0.68), 4.719 (0.54), 4.752 (0.69), 4.760 (0.78), 4.767 (0.88), 4.776 (0.66), 4.827 (0.61), 4.839 (1.02), 4.850 (0.58), 4.861 (0.59), 4.876 (0.80), 4.885 (0.61), 5.008 (10.07), 5.261 (0.76), 5.349 (0.49), 5.392 (0.78), 5.482 (0.47), 5.512 (0.41), 7.907 (9.08), 7.911 (16.00), 8.642 (5.57).

### Beispiel 101

### (5RS)-2-[4-Fluor-3-(trifluormethyl)benzyl]-5-{[(3S)-3-hydroxypyrrolidin-1-yl]carbonyl}-5,6,7,8-tetrahydro[1,2,4]triazolo[4,3-a]pyridin-3(2H)-on (Diastereomerengemisch; 2 Isomere)

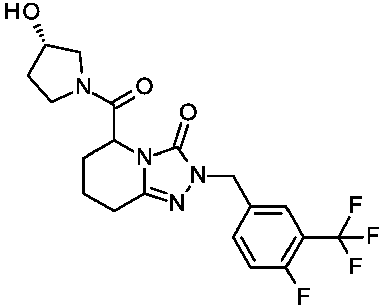

(5RS)-2-[4-Fluor-3-(trifluormethyl)benzyl]-3-oxo-2,3,5,6,7,8-hexahydro[1,2,4]triazolo[4,3-a]pyridin-5-carbonsäure (Racemat) (90.0 mg, 250 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (124 mg, 326 µmol) und Triethylamin (100 µl, 750 µmol) zugegeben. Nach 15 min Rühren wurde (3S)-Pyrrolidin-3-olhydrochlorid (37.1 mg, 301 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 34.0 mg (32 % d. Th.) der Titelverbindung erhalten.
LC-MS (Methode 3): Rₜ = 1.30 min; MS (ESIpos): m/z = 429 [M+H]⁺
¹H-NMR (400 MHz, DMSO-d6) δ [ppm]: -0.149 (1.32), -0.008 (12.62), 0.008 (10.09), 0.146 (1.26), 1.676 (1.38), 1.730 (2.98), 1.741 (2.71), 1.826 (0.65), 1.849 (1.75), 1.859 (2.09), 1.871 (1.82), 1.881 (2.34), 1.961 (2.00), 1.971 (2.58), 1.984 (3.11), 1.995 (3.23), 2.005 (3.02), 2.017 (2.65), 2.037 (1.94), 2.056 (1.60), 2.065 (1.42), 2.328 (1.54), 2.366 (0.98), 2.524 (6.71), 2.569 (2.18), 2.603 (3.88), 2.644 (1.60), 2.670 (1.85), 2.710 (0.98), 3.197 (1.05), 3.228 (1.51), 3.338 (2.77), 3.371 (3.54), 3.390 (2.34), 3.426 (0.80), 3.457 (1.72), 3.478 (1.45), 3.545 (1.29), 3.568 (2.28), 3.583 (1.54), 3.639 (1.14), 3.650 (1.72), 3.677 (1.32), 3.706 (1.51), 3.734 (0.62), 3.753 (0.89), 4.266 (2.03), 4.361 (1.97), 4.690 (1.05), 4.698 (1.20), 4.705 (1.29), 4.713 (1.08), 4.742 (2.06), 4.798 (0.92), 4.813 (1.29), 4.821 (0.98), 4.903 (16.00), 4.965 (1.29), 5.074 (1.72), 7.478 (2.49), 7.500 (4.77), 7.526 (4.46), 7.562 (2.74), 7.575 (3.29), 7.671 (3.72), 7.690 (3.82).

### Beispiel 102

### (5RS)-5-(Pyrrolidin-1-ylcarbonyl)-2-(2,4,5-trimethylbenzyl)-5,6,7,8-tetrahydro[1,2,4]triazolo[4,3-a]pyridin-3(2H)-on (Enantiomer 1)

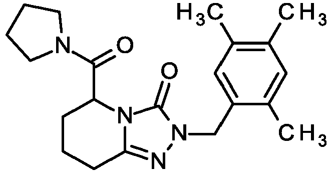

(5RS)-3-Oxo-2-(2,4,5-trimethylbenzyl)-2,3,5,6,7,8-hexahydro[1,2,4]triazolo[4,3-a]pyridin-5-carbonsäure (Enantiomer 1) (93.0 mg, 295 µmol) wurde in THF (2.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (146 mg, 383 µmol) und Triethylamin (120 µl, 880 µmol) zugegeben. Nach 15 min Rühren wurde Pyrrolidin (30 µl, 350 µmol) zugegeben und das Reaktionsgemisch für 72 Stunden bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 55.0 mg (51 % d. Th.) der Titelverbindung erhalten.
LC-MS (Methode 3): Rₜ = 1.59 min; MS (ESIpos): m/z = 369 [M+H]⁺
¹H-NMR (400 MHz, DMSO-d6) δ [ppm]: -0.008 (0.95), 0.008 (0.80), 1.661 (0.42), 1.673 (0.74), 1.687 (1.21), 1.697 (1.43), 1.710 (1.15), 1.754 (0.47), 1.772 (1.91), 1.790 (3.37), 1.807 (2.63), 1.823 (0.77), 1.892 (0.79), 1.909 (2.45), 1.926 (3.23), 1.942 (2.13), 1.961 (0.95), 1.974 (1.22), 1.985 (1.53), 1.996 (1.03), 2.010 (0.92), 2.020 (0.64), 2.026 (0.58), 2.036 (0.58), 2.046 (0.40), 2.140 (15.41), 2.144 (16.00), 2.205 (14.00), 2.457 (0.40), 2.473 (0.58), 2.523 (1.61), 2.560 (0.86), 2.571 (1.56), 2.583 (0.85), 2.613 (0.63), 3.224 (0.46), 3.240 (0.99), 3.253 (0.98), 3.270 (1.74), 3.287 (0.91), 3.321 (1.38), 3.340 (1.90), 3.352 (0.69), 3.357 (0.94), 3.369 (1.05), 3.387 (0.48), 3.433 (0.53), 3.450 (1.15), 3.458 (0.84), 3.468 (0.69), 3.475 (1.45), 3.492 (0.64), 3.591 (0.67), 3.608 (1.40), 3.616 (0.70), 3.625 (0.78), 3.633 (1.09), 3.649 (0.49), 4.684 (8.43), 4.716 (1.31), 4.725 (1.52), 4.731 (1.73), 4.740 (1.26), 6.890 (4.26), 6.914 (3.98).

Die (5S)-Konfiguration wurde aufgrund der Kristallstrukturaufklärung für Beispiel 26, 108, 113, 157, 237, 358 und 454 zugeordnet.

### (5S)-5-(Pyrrolidin-1-ylcarbonyl)-2-(2,4,5-trimethylbenzyl)-5,6,7,8-tetrahydro[1,2,4]triazolo[4,3-a]pyridin-3(2H)-on

### Beispiel 103

### (5RS)-5-(Pyrrolidin-1-ylcarbonyl)-2-[(3,5,6-trimethylpyrazin-2-yl)methyl]-5,6,7,8-tetrahydro[1,2,4]triazolo[4,3-a]pyridin-3(2H)-on (Racemat)

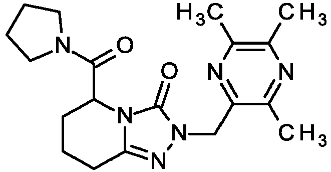

(5RS)-3-Oxo-2-[(3,5,6-trimethylpyrazin-2-yl)methyl]-2,3,5,6,7,8-hexahydro[1,2,4]triazolo[4,3-a]pyridin-5-carbonsäure (Racemat) (90.0 mg, 90 % Reinheit, 255 µmol) wurde in DMF (1.9 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (126 mg, 332 µmol) und Triethylamin (110 µl, 770 µmol) zugegeben. Nach 15 min Rühren wurde Pyrrolidin (26 µl, 310 µmol) zugegeben und das Reaktionsgemisch über Nacht bei Raumtemperatur gerührt. Es wurde erneut 1-[Bis(dimethylamino)methylen]-1H-[1,2,3]triazolo[4,5-b]pyridin-1-ium-3-oxidhexafluorophosphat (97 mg, 255 µmol) und Triethylamin (36 µl, 255 µmol) zugegeben und 1 Stunde bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 57.0 mg (60 % d. Th.) der Titelverbindung erhalten.
LC-MS (Methode 3): Rₜ = 0.93 min; MS (ESIpos): m/z = 371 [M+H]⁺
¹H-NMR (400 MHz, DMSO-d6) δ [ppm]: 1.649 (0.46), 1.662 (0.81), 1.675 (1.31), 1.685 (1.56), 1.699 (1.24), 1.756 (0.44), 1.773 (1.80), 1.790 (3.21), 1.807 (2.55), 1.824 (0.78), 1.893 (0.77), 1.909 (2.39), 1.926 (3.22), 1.943 (2.22), 1.959 (1.07), 1.969 (1.22), 1.981 (1.37), 1.993 (0.94), 2.007 (0.89), 2.018 (0.64), 2.032 (0.58), 2.384 (14.92), 2.409 (15.08), 2.417 (16.00), 2.450 (0.52), 2.458 (0.45), 2.476 (1.25), 2.574 (0.48), 2.586 (0.61), 3.222 (0.45), 3.239 (0.92), 3.252 (0.96), 3.269 (1.63), 3.286 (0.80), 3.336 (1.71), 3.354 (0.89), 3.366 (0.96), 3.383 (0.44), 3.434 (0.50), 3.451 (1.10), 3.458 (0.82), 3.476 (1.38), 3.493 (0.63), 3.594 (0.64), 3.611 (1.35), 3.619 (0.70), 3.628 (0.78), 3.636 (1.06), 3.652 (0.47), 4.717 (1.21), 4.726 (1.43), 4.732 (1.62), 4.740 (1.19), 4.868 (9.21).

### Beispiel 104

### (5RS)-5-(Pyrrolidin-1-ylcarbonyl)-2-[(3,5,6-trimethylpyrazin-2-yl)methyl]-5,6,7,8-tetrahydro[1 ,2,4]triazolo[4,3-a]pyridin-3(2H)-on (Enantiomer 2)

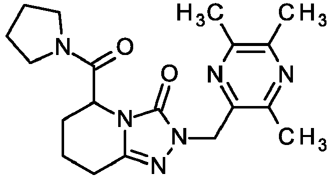

(5RS)-5-(Pyrrolidin-1-ylcarbonyl)-2-[(3,5,6-trimethylpyrazin-2-yl)methyl]-5,6,7,8-tetrahydro[1 ,2,4]triazolo[4,3-a]pyridin-3(2H)-on (Racemat) wurden durch chirale präparative HPLC getrennt [Probenvorbereitung: 46 mg gelöst in 4 ml Ethanol; Injektionsvolumen: 0.35 ml; Säule: Daicel Chiralpak^{®} AD-H 5µm, 250 x 20 mm; Laufmittel: n-Heptan/Ethanol: isokratisch 60% Ethanol, Fluß: 25 ml/min; Temperatur 25°C; UV Detektion: 210 nm]. Nach der Trennung wurden 19 mg von Enantiomer 1, welches zuerst eluierte, und 19 mg (92 % Reinheit) von Enantiomer 2, welches später eluierte, isoliert.

### Enantiomer 2:

Analytische chirale HPLC: Rₜ = 2.11 min, e.e. = 99 % [Säule: Daicel Chiralcel^{®} AD-3 3µm 50 x 4.6 mm; Laufmittel: n-Heptan/Ethanol 1:1; Fluß: 1 ml/min; UV Detektion: 220 nm].
LC-MS (Methode 4): Rₜ = 0.56 min; MS (ESIpos): m/z = 371 [M+H]⁺
¹H-NMR (400 MHz, DMSO-d6) δ [ppm]: 1.63-1.68 (m, 2H), 1.79 (quin, 2H), 1.86 - 2.07 (m, 4H), 2.38 (s, 3H), 2.41 (d, 6H), 2.44 - 2.61 (m, 2H, teilweise durch Lösungsmittelsignal verdeckt), 3.14 - 3.29 (m, 1H), 3.33 - 3.39 (m, 1H), 3.41 - 3.52 (m, 1H), 3.57 - 3.67 (m, 1H), 4.73 (dd, 1H), 4.87 (s, 2H).

### Beispiel 105

### 5(RS)-5-Methyl-2-(4-methylbenzyl)-5-(pyrrolidin-1 -ylcarbonyl)-5,6,7,8-tetrahydro[1,2,4]triazolo[4,3-a]pyridin-3(2H)-on (Racemat)

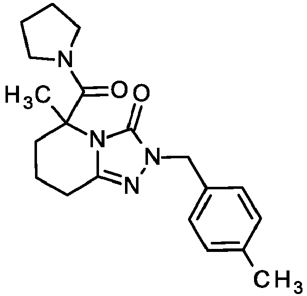

(5RS)-5-Methyl-2-(4-methylbenzyl)-3-oxo-2,3,5,6,7,8-hexahydro[1,2,4]triazolo[4,3-a]pyridin-5-carbonsäure (Racemat) (30.0 mg, 99.6 µmol) wurde in THF (1.0 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (49.2 mg, 129 µmol) und Triethylamin (42 µl, 300 µmol) zugegeben. Nach 15 min Rühren wurde Pyrrolidin (10 µl, 120 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 19.4 mg (55 % d. Th.) der Titelverbindung erhalten.
LC-MS (Methode 3): Rₜ = 1.46 min; MS (ESIpos): m/z = 355 [M+H]⁺
¹H-NMR (400 MHz, DMSO-d6) δ [ppm]: -0.008 (0.46), 0.008 (0.45), 1.155 (0.41), 1.173 (0.84), 1.192 (0.44), 1.202 (0.85), 1.218 (0.84), 1.563 (11.36), 1.715 (1.01), 1.754 (0.66), 1.767 (0.63), 1.781 (0.47), 1.817 (1.45), 1.845 (1.15), 1.958 (0.59), 1.986 (0.75), 2.269 (11.71), 2.599 (0.63), 2.611 (0.73), 2.624 (1.11), 2.635 (1.32), 3.237 (0.41), 4.664 (1.34), 4.702 (2.54), 4.780 (2.48), 4.818 (1.34), 7.127 (16.00).

### Beispiel 106

### (5RS)-2-{[3-Chlor-5-(trifluormethyl)pyridin-2-yl]methyl}-5-(pyrrolidin-1-ylcarbonyl)-5,6,7,8-tetrahydro[1,2,4]triazolo[4,3-a]pyridin-3(2H)-on (Enantiomer 1)

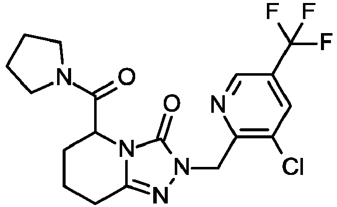

Methyl-(5RS)-2-{[3-chlor-5-(trifluormethyl)pyridin-2-yl]methyl}-3-oxo-2,3,5,6,7,8-hexahydro[1,2,4]triazolo[4,3-a]pyridin-5-carboxylat (Enantiomer 1) (80.0 mg, 205 µmol) wurde in THF (1.8 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (101 mg, 266 µmol) und Triethylamin (86 µl, 610 µmol) zugegeben. Nach 15 min Rühren wurde Pyrrolidin (21 µl, 250 µmol) zugegeben und das Reaktionsgemisch für 72 Stunden bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 4.20 mg (5 % d. Th.) der Titelverbindung erhalten.
LC-MS (Methode 3): Rₜ = 1.37 min; MS (ESIpos): m/z = 430 [M+H]⁺
¹H-NMR (400 MHz, DMSO-d6) δ [ppm]: -0.149 (1.19), -0.008 (10.74), 0.008 (9.06), 0.146 (1.10), 1.480 (0.71), 1.701 (6.28), 1.714 (8.22), 1.732 (5.79), 1.744 (2.92), 1.753 (2.52), 1.771 (8.44), 1.788 (14.81), 1.805 (11.31), 1.822 (3.31), 1.891 (3.54), 1.908 (10.25), 1.924 (12.95), 1.941 (8.27), 1.958 (3.80), 1.973 (2.70), 1.984 (4.77), 1.995 (4.33), 2.006 (3.23), 2.021 (3.54), 2.042 (3.23), 2.057 (2.92), 2.078 (1.41), 2.327 (2.43), 2.366 (1.81), 2.523 (8.75), 2.566 (4.15), 2.579 (7.65), 2.591 (3.93), 2.609 (1.81), 2.620 (2.70), 2.634 (1.24), 2.670 (2.70), 2.710 (1.77), 3.229 (2.12), 3.246 (4.33), 3.259 (4.51), 3.276 (8.18), 3.337 (8.75), 3.355 (4.33), 3.367 (4.60), 3.384 (2.08), 3.442 (2.34), 3.460 (4.99), 3.467 (3.62), 3.484 (6.32), 3.501 (2.74), 3.595 (2.87), 3.612 (5.88), 3.620 (3.05), 3.629 (3.49), 3.637 (4.69), 3.654 (2.12), 4.735 (5.44), 4.745 (6.32), 4.750 (7.25), 4.760 (5.35), 5.063 (4.82), 5.104 (16.00), 5.134 (15.43), 5.175 (4.64), 8.486 (10.96), 8.489 (11.27), 8.905 (11.09).

Die (5S)-Konfiguration wurde aufgrund der Kristallstrukturaufklärung für Beispiel 26, 108, 113, 157, 237, 358 und 454 zugeordnet.

### (5S)-2-{[3-Chlor-5-(trifluormethyl)pyridin-2-yl]methyl}-5-(pyrrolidin-1-ylcarbonyl)-5,6,7,8-tetrahydro[1,2,4]triazolo[4,3-a]pyridin-3(2H)-on

### Beispiel 107

### (5RS)-2-{[5-Chlor-4-(trifluormethyl)pyridin-2-yl]methyl}-5-(pyrrolidin-1-ylcarbonyl)-5,6,7,8-tetrahydro[1,2,4]triazolo[4,3-a]pyridin-3(2H)-on (Enantiomer 1)

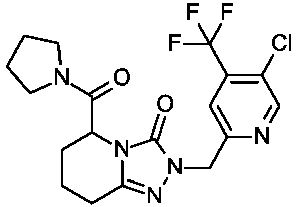

(5RS)-2-{[5-Chlor-4-(trifluormethyl)pyridin-2-yl]methyl}-3-oxo-2,3,5,6,7,8-hexahydro[1,2,4]triazolo[4,3-a]pyridin-5-carbonsäure (Enantiomer 1) (100 mg, 265 µmol) wurde in THF (3.8 ml) bei Raumtemperatur vorgelegt. Anschließend wurde 1-[Bis(dimethylamino)methylen]-1H-[1,2,3]triazolo[4,5-b]pyridin-1-ium-3-oxidhexafluorophosphat (131 mg, 345 µmol) und Triethylamin (110 µl, 800 µmol) zugegeben. Nach 15 min Rühren wurde Pyrrolidin (27 µl, 320 µmol) zugegeben und das Reaktionsgemisch für 72 Stunden bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 65.0 mg (57 % d. Th.) der Titelverbindung erhalten.
LC-MS (Methode 4): Rₜ = 0.79 min; MS (ESIpos): m/z = 430 [M+H]⁺
¹H-NMR (400 MHz, DMSO-d6) δ [ppm]: -0.008 (2.42), 0.008 (2.12), 1.711 (2.02), 1.724 (2.54), 1.735 (2.04), 1.755 (1.54), 1.772 (4.25), 1.789 (6.89), 1.806 (5.25), 1.823 (1.54), 1.891 (1.57), 1.908 (4.70), 1.925 (5.85), 1.942 (3.54), 1.959 (1.16), 2.005 (2.17), 2.016 (2.70), 2.026 (1.79), 2.043 (1.62), 2.052 (1.21), 2.067 (1.15), 2.328 (0.72), 2.366 (0.47), 2.560 (2.01), 2.575 (1.77), 2.590 (1.68), 2.602 (2.96), 2.614 (1.68), 2.632 (0.81), 2.643 (1.18), 2.670 (0.81), 2.690 (1.16), 2.710 (0.47), 2.731 (0.66), 2.890 (0.73), 3.229 (0.83), 3.246 (1.96), 3.258 (1.95), 3.275 (3.58), 3.293 (1.92), 3.335 (3.62), 3.353 (1.88), 3.365 (1.97), 3.383 (0.88), 3.441 (1.04), 3.458 (2.32), 3.465 (1.61), 3.482 (2.93), 3.500 (1.30), 3.593 (1.31), 3.609 (2.74), 3.626 (1.57), 3.634 (2.05), 3.650 (0.96), 4.755 (2.49), 4.764 (2.73), 4.770 (3.18), 4.779 (2.43), 4.992 (0.41), 5.034 (16.00), 5.076 (0.45), 7.719 (10.56), 8.902 (9.80).

Die (5S)-Konfiguration wurde aufgrund der Kristallstrukturaufklärung für Beispiel 26, 108, 113, 157, 237, 358 und 454 zugeordnet.

### (5S)-2-{[5-Chlor-4-(trifluormethyl)pyridin-2-yl]methyl}-5-(pyrrolidin-1-ylcarbonyl)-5,6,7,8-tetrahydro[1,2,4]triazolo[4,3-a]pyridin-3(2H)-on

### Beispiel 108

### (5RS)-2-(4-Methylbenzyl)-5-(1,3-thiazolidin-3-ylcarbonyl)-5,6,7,8-tetrahydro[1,2,4]triazolo[4,3-a]pyridin-3(2H)-on (Enantiomer 1)

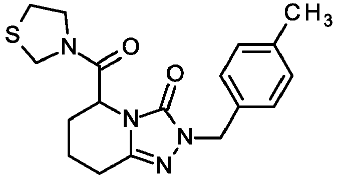

(5RS)-2-(4-Methylbenzyl)-3-oxo-2,3,5,6,7,8-hexahydro[1,2,4]triazolo[4,3-a]pyridin-5-carbonsäure (Enantiomer 1) (75.0 mg, 261 µmol) wurde in DMF (3.1 ml) und Dichlormethan (1.5 ml) bei Raumtemperatur vorgelegt. Anschließend wurde 1-[Bis(dimethylamino)methylen]-1H-benzotriazol-1-ium-3-oxidhexafluorophosphat (129 mg, 339 µmol) und N,N-Diisopropylethylamin (120 µl, 680 µmol) zugegeben. Nach 15 min Rühren wurde 1,3-Thiazolidinhydrochlorid (39.3 mg, 313 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 44.7 mg (48 % d. Th.) der Titelverbindung erhalten.
LC-MS (Methode 4): Rₜ = 0.76 min; MS (ESIpos): m/z = 359 [M+H]⁺
¹H-NMR (400 MHz, DMSO-d6) δ [ppm]: -0.008 (0.44), 1.647 (0.49), 1.721 (0.75), 1.731 (0.71), 1.952 (0.40), 1.978 (0.57), 1.987 (0.92), 2.039 (0.54), 2.049 (0.59), 2.059 (0.57), 2.272 (16.00), 2.567 (0.97), 2.576 (0.85), 2.588 (1.42), 2.600 (0.79), 2.630 (0.50), 2.999 (0.74), 3.015 (1.56), 3.031 (0.89), 3.128 (1.06), 3.144 (2.19), 3.159 (1.12), 3.624 (0.43), 3.639 (0.56), 3.688 (0.57), 3.768 (0.50), 3.779 (0.46), 3.794 (0.63), 3.941 (0.62), 3.955 (0.43), 3.968 (0.47), 4.367 (0.97), 4.392 (1.26), 4.551 (1.28), 4.577 (1.04), 4.609 (0.73), 4.632 (0.88), 4.749 (6.74), 4.801 (0.89), 4.824 (0.72), 4.845 (0.56), 4.854 (0.68), 4.860 (0.73), 4.868 (0.55), 4.928 (0.45), 4.942 (0.57), 7.101 (0.90), 7.108 (0.56), 7.123 (12.99), 7.125 (12.62), 7.147 (0.89).

Die (5S)-Konfiguration wurde aufgrund von Kristallstrukturaufklärung für Beispiel 108 zugordnet.

### (5S)-2-(4-Methylbenzyl)-5-(1,3-thiazolidin-3-ylcarbonyl)-5,6,7,8-tetrahydro[1,2,4]triazolo[4,3-a]pyridin-3(2H)-on

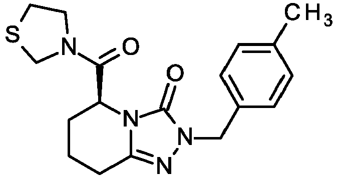

### Beispiel 109

### (2S)-1-{[(5RS)-2-(4-Methylbenzyl)-3-oxo-2,3,5,6,7,8-hexahydro[1,2,4]triazolo[4,3-a]pyridin-5-yl]carbonyl}pyrrolidin-2-carbonitril (Enantiomer 1)

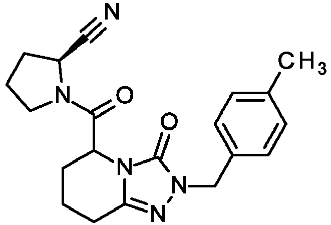

(5RS)-2-(4-Methylbenzyl)-3-oxo-2,3,5,6,7,8-hexahydro[1,2,4]triazolo[4,3-a]pyridin-5-carbonsäure (Enantiomer 1) (75.0 mg, 261 µmol) wurde in DMF (3.1 ml) und Dichlormethan (1.5 ml) bei Raumtemperatur vorgelegt. Anschließend wurde 1-[Bis(dimethylamino)methylen]-1H-benzotriazol-1-ium-3-oxidhexafluorophosphat (129 mg, 339 µmol) und N,N-Diisopropylethylamin (120 µl, 680 µmol) zugegeben. Nach 15 min Rühren wurde (2S)-Pyrrolidin-2-carbonitrilhydrochlorid (41.5 mg, 313 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 35.4 mg (36 % d. Th.) der Titelverbindung erhalten.
LC-MS (Methode 1): Rₜ = 1.02 min; MS (ESIpos): m/z = 366 [M+H]⁺
¹H-NMR (400 MHz, DMSO-d6) δ [ppm]: -0.008 (1.03), 0.008 (0.89), 1.654 (0.52), 1.769 (0.69), 2.027 (1.21), 2.042 (1.71), 2.062 (2.62), 2.078 (2.62), 2.094 (1.23), 2.104 (1.05), 2.115 (0.95), 2.136 (0.93), 2.149 (1.11), 2.159 (0.93), 2.175 (0.45), 2.185 (0.59), 2.204 (1.26), 2.224 (1.18), 2.236 (0.75), 2.244 (0.47), 2.256 (0.74), 2.273 (16.00), 2.327 (0.30), 2.366 (0.18), 2.558 (0.99), 2.569 (0.98), 2.583 (0.79), 2.603 (0.74), 2.616 (1.37), 2.627 (0.79), 2.646 (0.35), 2.658 (0.59), 2.669 (0.49), 2.709 (0.17), 3.672 (2.09), 3.688 (4.23), 3.705 (2.02), 4.749 (8.60), 4.793 (2.28), 4.803 (3.16), 4.813 (2.75), 4.824 (1.44), 7.099 (1.37), 7.120 (9.43), 7.127 (9.47), 7.148 (1.26).

Die (5S)-Konfiguration wurde aufgrund der Kristallstrukturaufklärung für Beispiel 26, 108, 113, 157, 237, 358 und 454 zugeordnet.

### (2S)-1-{[(5S)-2-(4-Methylbenzyl)-3-oxo-2,3,5,6,7,8-hexahydro[1,2,4]triazolo[4,3-a]pyridin-5-yl]carbonyl}pyrrolidin-2-carbonitril

### Beispiel 110

### (5RS)-5-{[(3S)-3-Fluorpyrrolidin-1-yl]carbonyl}-2-(4-methylbenzyl)-5,6,7,8-tetrahydro[1,2,4]triazolo[4,3-a]pyridin-3(2H)-on (Enantiomer 1)

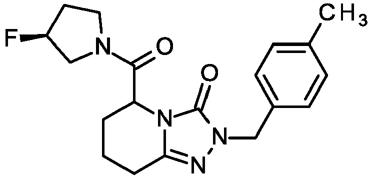

(5RS)-2-(4-Methylbenzyl)-3-oxo-2,3,5,6,7,8-hexahydro[1,2,4]triazolo[4,3-a]pyridin-5-carbonsäure (Enantiomer 1) (75.0 mg, 261 µmol) wurde in DMF (3.1 ml) und Dichlormethan (1.5 ml) bei Raumtemperatur vorgelegt. Anschließend wurde 1-[Bis(dimethylamino)methylen]-1H-benzotriazol-1-ium-3-oxidhexafluorophosphat (129 mg, 339 µmol) und N,N-Diisopropylethylamin (120 µl, 680 µmol) zugegeben. Nach 15 min Rühren wurde (3S)-3-Fluorpyrrolidinhydrochlorid (39.3 mg, 313 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 56.2 mg (60 % d. Th.) der Titelverbindung erhalten.
LC-MS (Methode 3): Rₜ = 1.29 min; MS (ESIpos): m/z = 359 [M+H]⁺
¹H-NMR (400 MHz, DMSO-d6) δ [ppm]: -0.008 (0.69), 0.008 (0.65), 1.692 (0.66), 1.706 (1.32), 1.719 (1.54), 1.737 (0.99), 1.750 (0.44), 1.993 (0.51), 2.004 (0.68), 2.017 (0.58), 2.059 (0.73), 2.073 (0.83), 2.085 (0.81), 2.108 (0.79), 2.122 (0.48), 2.133 (0.55), 2.237 (0.43), 2.272 (16.00), 2.522 (1.18), 2.562 (0.69), 2.585 (1.09), 2.590 (1.13), 2.632 (0.42), 3.287 (0.45), 3.633 (1.34), 3.652 (0.99), 3.662 (0.87), 3.679 (0.60), 3.695 (0.51), 3.720 (0.55), 3.740 (0.92), 3.768 (0.67), 3.776 (0.60), 3.784 (0.65), 3.854 (1.17), 4.665 (0.61), 4.674 (0.73), 4.681 (0.78), 4.690 (0.65), 4.698 (0.71), 4.722 (0.84), 4.737 (5.72), 4.746 (3.53), 4.788 (0.47), 5.258 (0.57), 5.381 (0.72), 5.388 (0.75), 5.510 (0.42), 7.102 (0.76), 7.119 (6.24), 7.124 (13.58), 7.146 (0.84).

Die (5S)-Konfiguration wurde aufgrund der Kristallstrukturaufklärung für Beispiel 26, 108, 113, 157, 237, 358 und 454 zugeordnet.

### (5S)-5-{[(3S)-3-Fluorpyrrolidin-1 -yl]carbonyl}-2-(4-methylbenzyl)-5,6,7,8-tetrahydro[1,2,4]triazolo[4,3-a]pyridin-3(2H)-on

### Beispiel 111

### (5RS)-5-{[(3R)-3-Fluorpyrrolidin-1-yl]carbonyl}-2-(4-methylbenzyl)-5,6,7,8-tetrahydro[1,2,4]triazolo[4,3-a]pyridin-3(2H)-on (Enantiomer 1)

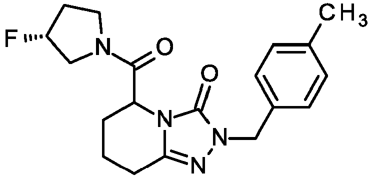

(5RS)-2-(4-Methylbenzyl)-3-oxo-2,3,5,6,7,8-hexahydro[1,2,4]triazolo[4,3-a]pyridin-5-carbonsäure (Enantiomer 1) (75.0 mg, 261 µmol) wurde in DMF (3.1 ml) und Dichlormethan (1.5 ml) bei Raumtemperatur vorgelegt. Anschließend wurde 1-[Bis(dimethylamino)methylen]-1H-benzotriazol-1-ium-3-oxidhexafluorophosphat (129 mg, 339 µmol) und N,N-Diisopropylethylamin (120 µl, 680 µmol) zugegeben. Nach 15 min Rühren wurde (3R)-3-Fluorpyrrolidinhydrochlorid (39.3 mg, 313 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 59.0 mg (63 % d. Th.) der Titelverbindung erhalten.
LC-MS (Methode 1): Rₜ = 0.98 min; MS (ESIpos): m/z = 359 [M+H]⁺
¹H-NMR (400 MHz, DMSO-d6) δ [ppm]: 0.008 (1.25), 1.715 (0.81), 1.979 (1.46), 2.021 (0.58), 2.103 (0.66), 2.135 (0.56), 2.272 (16.00), 2.573 (1.00), 2.585 (1.71), 2.598 (0.96), 2.627 (0.61), 3.359 (0.62), 3.378 (0.56), 3.388 (0.41), 3.503 (0.60), 3.522 (1.39), 3.548 (1.10), 3.569 (0.77), 3.593 (1.76), 3.920 (0.42), 3.942 (1.16), 3.965 (0.46), 4.004 (0.46), 4.746 (7.68), 4.787 (0.75), 4.801 (1.00), 4.811 (0.63), 4.825 (0.70), 4.841 (0.86), 4.850 (0.67), 5.270 (0.57), 5.347 (0.55), 5.398 (0.56), 5.478 (0.52), 7.103 (0.74), 7.125 (14.98), 7.145 (0.79).

Die (5S)-Konfiguration wurde aufgrund der Kristallstrukturaufklärung für Beispiel 26, 108, 113, 157, 237, 358 und 454 zugeordnet.

### (5S)-5-{[(3R)-3-Fluorpyrrolidin-1-yl]carbonyl}-2-(4-methylbenzyl)-5,6,7,8-tetrahydro[1,2,4]triazolo[4,3-a]pyridin-3(2H)-on

### Beispiel 112

### (5RS)-5-[(3-Fluorazetidin-1-yl)carbonyl]-2-(4-methylbenzyl)-5,6,7,8-tetrahydro[1,2,4]triazolo[4,3-a]pyridin-3(2H)-on (Racemat)

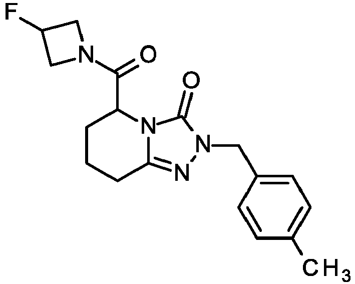

(5RS)-2-(4-Methylbenzyl)-3-oxo-2,3,5,6,7,8-hexahydro[1,2,4]triazolo[4,3-a]pyridin-5-carbonsäure (Racemat) (100 mg, 94 % Reinheit, 326 µmol) wurde mit 3-Fluorazetidinhydrochlorid (43.6 mg, 391 µmol) in Pyridin/DMF (5/1) (2.0 ml) bei Raumtemperatur vorgelegt. Anschließend wurde O-(7-Azabenzotriazol-1-yl)-N,N,N',N'-tetramethyluronium-hexafluorphosphat (161 mg, 423 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 90.8 mg (80 % d. Th.) der Titelverbindung erhalten.
LC-MS (Methode 4): Rₜ = 0.69 min; MS (ESIpos): m/z = 345 [M+H]⁺
¹H-NMR (400 MHz, DMSO-d6) δ [ppm]: 1.706 (1.68), 1.917 (0.50), 1.930 (0.68), 1.940 (0.74), 2.004 (0.68), 2.017 (0.66), 2.039 (0.48), 2.270 (16.00), 2.522 (1.08), 2.561 (1.51), 2.575 (1.97), 2.588 (0.99), 2.616 (0.62), 3.924 (0.50), 3.953 (0.54), 3.984 (0.49), 4.220 (0.45), 4.235 (0.44), 4.251 (0.59), 4.263 (0.46), 4.277 (0.45), 4.286 (0.50), 4.319 (0.49), 4.515 (1.27), 4.528 (1.95), 4.541 (1.28), 4.745 (6.20), 5.753 (0.52), 7.104 (0.55), 7.125 (15.04), 7.145 (0.54).

### Beispiel 113

### (5RS)-5-[(3-Fluorazetidin-1-yl)carbonyl]-2-(4-methylbenzyl)-5,6,7,8-tetrahydro[1,2,4]triazolo[4,3-a]pyridin-3(2H)-on (Enantiomer 2)

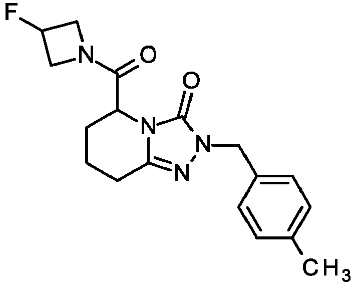

(5RS)-5-[(3-Fluorazetidin-1-yl)carbonyl]-2-(4-methylbenzyl)-5,6,7,8-tetrahydro[1,2,4]triazolo[4,3-a]pyridin-3(2H)-on (Racemat) wurde durch chirale präparative HPLC getrennt [Probenvorbereitung: 69 mg gelöst in 4 ml Ethanol und 4 ml Acetonitril; Injektionsvolumen: 0.5 ml; Säule: Daicel Chiralcel^{®} IA-H 5 µm, 250 × 20 mm; Laufmittel: n-Heptan/Ethanol 50:50; Fluß: 15 ml/min; Temperature 30°C; UV Detektion: 210 nm]. Nach der Trennung, wurden 32.3 mg von Enantiomer 1, welches zuerst eluierte, und 32.7 mg von Enantiomer 2, welches später eluierte, isoliert.

Analytische chirale HPLC: Rₜ = 2.53 min, d.e. = 100% [Säule: Daicel Chiralcel^{®} IA-3 50 × 4.6 mm; Laufmittel: n-Heptan/Ethanol 50:50; Fluß: 1 ml/min; UV Detektion: 220 nm].
LC-MS (Methode 3): Rₜ = 1.25 min; MS (ESIpos): m/z = 345 [M+H]⁺
¹H-NMR (400 MHz, DMSO-d6) δ [ppm]: 1.702 (1.92), 1.940 (0.86), 2.005 (0.77), 2.017 (0.75), 2.270 (15.01), 2.561 (1.75), 2.575 (2.00), 2.588 (1.08), 2.616 (0.66), 3.921 (0.57), 3.952 (0.62), 3.985 (0.55), 4.219 (0.51), 4.233 (0.50), 4.250 (0.64), 4.286 (0.57), 4.319 (0.52), 4.516 (1.38), 4.528 (2.10), 4.541 (1.39), 4.709 (0.41), 4.745 (6.36), 7.125 (16.00).

Die (5S)-Konfiguration wurde aufgrund von Kristallstrukturaufklärung für Beispiel 113 zugordnet.

### (5S)-5-[(3-Fluorazetidin-1-yl)carbonyl]-2-(4-methylbenzyl)-5,6,7,8-tetrahydro[1,2,4]triazolo[4,3-a]pyridin-3(2H)-on

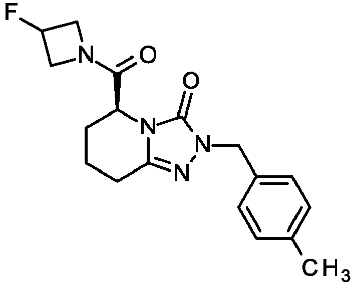

### Beispiel 114

### (5RS)-5-[(3,3-Difluorazetidin-1-yl)carbonyl]-2-(4-methylbenzyl)-5,6,7,8-tetrahydro[1,2,4]triazolo[4,3-a]pyridin-3(2H)-on (Racemat)

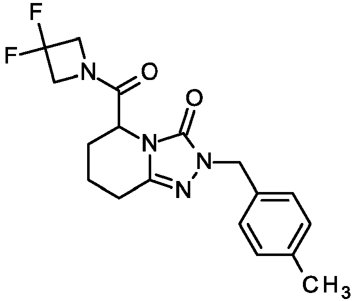

(5RS)-2-(4-Methylbenzyl)-3-oxo-2,3,5,6,7,8-hexahydro[1,2,4]triazolo[4,3-a]pyridin-5-carbonsäure (Racemat) (150 mg, 522 µmol) wurde mit 3,3-Difluorazetidinhydrochlorid (71.0 mg, 548 µmol) in Pyridin/DMF (5/1) (2.0 ml) bei Raumtemperatur vorgelegt. Anschließend wurde O-(7-Azabenzotriazol-1-yl)-N,N,N',N'-tetramethyluronium-hexafluorphosphat (218 mg, 574 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 118 mg (62 % d. Th.) der Titelverbindung erhalten.
LC-MS (Methode 3): Rₜ = 1.42 min; MS (ESIpos): m/z = 363 [M+H]⁺
¹H-NMR (400 MHz, DMSO-d6) δ [ppm]: -0.008 (1.26), 0.008 (1.16), 1.697 (0.97), 1.712 (1.59), 1.726 (1.11), 1.959 (0.43), 1.970 (0.74), 1.983 (0.68), 2.029 (0.64), 2.045 (0.67), 2.063 (0.52), 2.271 (12.02), 2.574 (0.83), 2.583 (1.39), 2.598 (0.66), 2.625 (0.40), 4.355 (0.69), 4.384 (0.70), 4.416 (0.41), 4.568 (1.00), 4.583 (1.49), 4.595 (0.96), 4.712 (0.53), 4.752 (5.61), 4.843 (0.47), 7.105 (0.42), 7.127 (16.00), 7.149 (0.40).

### Beispiel 115

### (5RS)-5-[(3,3-Difluorazetidin-1-yl)carbonyl]-2-(4-methylbenzyl)-5,6,7,8-tetrahydro[1,2,4]triazolo[4,3-a]pyridin-3(2H)-on (Enantiomer 2)

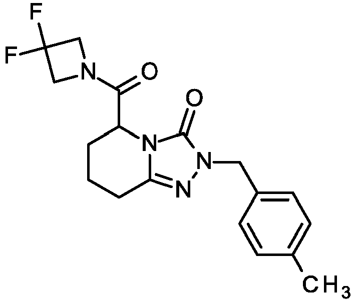

(5RS)-5-[(3,3-Difluorazetidin-1-yl)carbonyl]-2-(4-methylbenzyl)-5,6,7,8-tetrahydro[1,2,4]triazolo[4,3-a]pyridin-3(2H)-on (Racemat) wurde durch chirale präparative HPLC getrennt [Probenvorbereitung: 105 mg gelöst in 5 ml Ethanol und 5 ml Acetonitril; Injektionsvolumen: 0.5 ml; Säule: Daicel Chiralpak^{®} IB 5 µm, 250 × 20 mm; Laufmittel: n-Heptan/Ethanol 50:50; Fluß: 15 ml/min; Temperature 40°C; UV Detektion: 210 nm]. Nach der Trennung, wurden 53.3 mg von Enantiomer 1, welches zuerst eluierte, und 53.2 mg von Enantiomer 2, welches später eluierte, isoliert.

Analytische chirale HPLC: Rₜ = 3.04 min, d.e. = 99% [Säule: Daicel Chiraltek^{®} IB-3 50 × 4.6 mm; Laufmittel: i-Hexan/Ethanol 50:50; Fluß: 1 ml/min; UV Detektion: 220 nm].
LC-MS (Methode 3): Rₜ = 1.39 min; MS (ESIpos): m/z = 363 [M+H]⁺
¹H-NMR (400 MHz, DMSO-d6) δ [ppm]: -0.008 (0.65), 0.008 (0.71), 1.697 (1.02), 1.711 (1.66), 1.725 (1.18), 1.742 (0.44), 1.958 (0.42), 1.970 (0.78), 1.982 (0.72), 2.028 (0.68), 2.045 (0.73), 2.062 (0.54), 2.271 (11.98), 2.597 (0.69), 4.355 (0.74), 4.384 (0.75), 4.414 (0.42), 4.567 (0.98), 4.582 (1.51), 4.595 (0.97), 4.712 (0.53), 4.752 (5.92), 4.841 (0.48), 4.872 (0.41), 7.127 (16.00), 7.149 (0.43).

### Beispiel 116

### (5RS)-5-{[(3S)-3-Hydroxypyrrolidin-1-yl]carbonyl}-2-(4-methylbenzyl)-5,6,7,8-tetrahydro[1,2,4]triazolo[4,3-a]pyridin-3(2H)-on (Enantiomer 1)

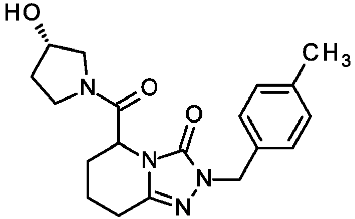

(5RS)-2-(4-Methylbenzyl)-3-oxo-2,3,5,6,7,8-hexahydro[1,2,4]triazolo[4,3-a]pyridin-5-carbonsäure (Enantiomer 1) (75.0 mg, 261 µmol) wurde in DMF (3.1 ml) und Dichlormethan (1.5 ml) bei Raumtemperatur vorgelegt. Anschließend wurde 1-[Bis(dimethylamino)methylen]-1H-benzotriazol-1-ium-3-oxidhexafluorophosphat (129 mg, 339 µmol) und N,N-Diisopropylethylamin (120 µl, 680 µmol) zugegeben. Nach 15 min Rühren wurde (3S)-Pyrrolidin-3-olhydrochlorid (38.7 mg, 313 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 54.0 mg (58 % d. Th.) der Titelverbindung erhalten.
LC-MS (Methode 1): Rₜ = 0.86 min; MS (ESIpos): m/z = 357 [M+H]⁺
¹H-NMR (400 MHz, DMSO-d6) δ [ppm]: -0.008 (0.41), 1.705 (1.37), 1.716 (1.38), 1.730 (1.13), 1.752 (0.63), 1.763 (0.58), 1.773 (0.48), 1.782 (0.43), 1.847 (0.72), 1.858 (0.82), 1.869 (0.69), 1.879 (0.90), 1.890 (0.66), 1.903 (0.57), 1.948 (0.77), 1.982 (0.83), 2.000 (1.05), 2.011 (0.90), 2.025 (0.93), 2.035 (0.75), 2.043 (0.63), 2.051 (0.61), 2.061 (0.41), 2.271 (16.00), 2.467 (0.41), 2.524 (1.22), 2.571 (0.77), 2.583 (1.42), 2.594 (0.75), 2.625 (0.59), 3.294 (2.42), 3.304 (3.08), 3.315 (14.88), 3.337 (1.33), 3.356 (0.54), 3.368 (0.86), 3.431 (0.43), 3.439 (0.46), 3.452 (0.54), 3.460 (0.69), 3.560 (0.52), 3.641 (0.79), 3.653 (1.21), 3.669 (0.82), 3.680 (0.94), 4.259 (0.66), 4.267 (0.63), 4.363 (0.76), 4.664 (0.74), 4.672 (0.85), 4.679 (0.94), 4.688 (0.77), 4.695 (0.44), 4.707 (0.69), 4.716 (0.77), 4.722 (0.98), 4.734 (3.46), 4.743 (4.98), 4.782 (0.43), 4.956 (1.20), 4.964 (1.17), 5.074 (1.13), 5.082 (1.11), 7.096 (0.75), 7.102 (0.81), 7.118 (7.66), 7.123 (13.54), 7.144 (0.90).

Die (5S)-Konfiguration wurde aufgrund der Kristallstrukturaufklärung für Beispiel 26, 108, 113, 157, 237, 358 und 454 zugeordnet.

### (5S)-5-{[(3S)-3-Hydroxypyrrolidin-1-yl]carbonyl}-2-(4-methylbenzyl)-5,6,7,8-tetrahydro[1,2,4]triazolo[4,3-a]pyridin-3(2H)-on

### Beispiel 117

### (5RS)-5-{[(cis)-6,6-Difluor-3-azabicyclo[3.1.0]hex-3-yl]carbonyl}-2-(4-methylbenzyl)-5,6,7,8-tetrahydro[1,2,4]triazolo[4,3-a]pyridin-3(2H)-on (Racemat)

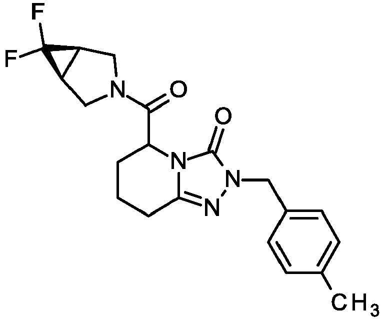

(5RS)-2-(4-Methylbenzyl)-3-oxo-2,3,5,6,7,8-hexahydro[1,2,4]triazolo[4,3-a]pyridin-5-carbonsäure (Racemat) (150 mg, 522 µmol) wurde mit (cis)-6,6-Difluor-3-azabicyclo[3.1.0]hexan (65.3 mg, 548 µmol) in Pyridin/DMF (5/1) (2.0 ml) bei Raumtemperatur vorgelegt. Anschließend wurde O-(7-Azabenzotriazol-1-yl)-N,N,N',N'-tetramethyluronium-hexafluorphosphat (218 mg, 574 µmol) zugegeben und das Reaktionsgemisch für 5 h bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 86.7 mg (43 % d. Th.) der Titelverbindung erhalten.
LC-MS (Methode 3): Rₜ = 1.48 min; MS (ESIpos): m/z = 389 [M+H]⁺
¹H-NMR (400 MHz, DMSO-d6) δ [ppm]: -0.008 (2.15), 0.008 (2.10), 1.697 (0.91), 1.707 (0.84), 1.743 (0.61), 1.980 (0.75), 2.048 (0.40), 2.270 (16.00), 2.327 (0.68), 2.518 (3.43), 2.523 (2.64), 2.563 (1.71), 2.573 (1.66), 2.585 (1.19), 2.602 (0.89), 2.617 (0.68), 2.670 (1.12), 2.709 (0.96), 3.609 (0.51), 3.641 (0.49), 3.651 (0.61), 3.705 (1.31), 3.736 (0.63), 3.773 (0.93), 3.804 (0.68), 3.893 (0.70), 3.921 (1.33), 3.934 (0.58), 3.944 (0.65), 3.957 (0.54), 4.016 (0.82), 4.044 (0.58), 4.668 (0.70), 4.678 (0.89), 4.684 (1.14), 4.694 (0.72), 4.722 (2.45), 4.737 (4.13), 4.741 (4.41), 4.779 (0.58), 7.095 (1.24), 7.117 (10.56), 7.122 (9.65), 7.144 (1.17).

### Beispiel 118

### (5RS)-2-(4-Methylbenzyl)-5-[(3-oxopyrrolidin-1 -yl)carbonyl]-5,6,7,8-tetrahydro[1,2,4]triazolo[4,3-a]pyridin-3(2H)-on (Racemat)

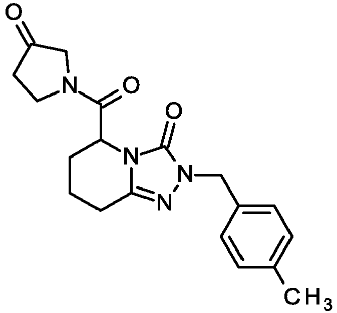

(5RS)-2-(4-Methylbenzyl)-3-oxo-2,3,5,6,7,8-hexahydro[1,2,4]triazolo[4,3-a]pyridin-5-carbonsäure (Racemat) (150 mg, 94 % Reinheit, 489 µmol) wurde mit Pyrrolidin-3-onhydrochlorid (71.3 mg, 586 µmol) in THF (3.0 ml) bei Raumtemperatur vorgelegt. Anschließend wurden Triethylamin (200 µl, 1.5 mmol) und O-(7-Azabenzotriazol-1-yl)-N,N,N',N'-tetramethyluronium-hexafluorphosphat (242 mg, 635 µmol) zugegeben und das Reaktionsgemisch über Nacht bei Raumtemperatur gerührt. Das Lösungsmittel wurde unter Vakuum entfernt und der Rückstand mit Pyridin (2.5 ml) und DMF (500 µl) aufgenommen. Nach Zugabe von (242 mg, 635 µmol) und Pyrrolidin-3-onhydrochlorid (71.3 mg, 586 µmol) wurde das Reaktionsgemisch für 2 h bei Raumtemperatur gerührt. Das Reaktionsgemisch wurde mit Wasser und Ethylacetat versetzt und mit 10%iger Zitronensäure schwach sauer gestellt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 80.7 mg (47 % d. Th.) der Titelverbindung erhalten.
LC-MS (Methode 4): Rₜ = 1.68 min; MS (ESIpos): m/z = 355 [M+H]⁺
¹H-NMR (400 MHz, DMSO-d6) δ [ppm]: 1.689 (0.93), 1.705 (1.02), 2.006 (1.17), 2.020 (1.11), 2.050 (0.88), 2.062 (0.79), 2.072 (0.66), 2.272 (16.00), 2.559 (1.50), 2.579 (2.25), 2.599 (1.83), 2.634 (0.49), 2.675 (0.58), 2.694 (0.65), 2.709 (0.82), 2.726 (0.46), 3.659 (0.71), 3.677 (0.70), 3.710 (1.38), 3.744 (1.37), 3.774 (0.70), 3.794 (0.74), 3.942 (0.40), 3.961 (0.45), 4.009 (0.77), 4.054 (1.67), 4.072 (0.42), 4.127 (1.60), 4.171 (0.71), 4.687 (0.61), 4.699 (1.26), 4.711 (0.76), 4.750 (5.78), 4.927 (0.75), 4.938 (0.44), 7.101 (0.97), 7.123 (13.94), 7.148 (0.87).

### Beispiel 119

### (5RS)-2-(4-Methylbenzyl)-5-[(3-oxopyrrolidin-1 -yl)carbonyl]-5,6,7,8-tetrahydro[1,2,4]triazolo[4,3-a]pyridin-3(2H)-on (Enantiomer 2)

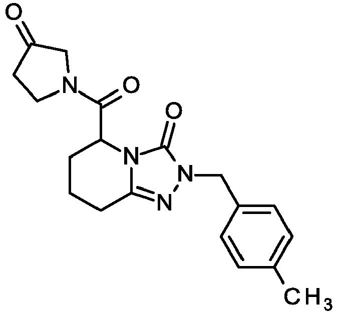

(5RS)-2-(4-Methylbenzyl)-5-[(3-oxopyrrolidin-1-yl)carbonyl]-5,6,7,8-tetrahydro[1,2,4]triazolo[4,3-a]pyridin-3(2H)-on (Racemat) wurde durch chirale präparative HPLC getrennt[Probenvorbereitung: 71 mg gelöst in 6 ml Ethanol; Injektionsvolumen: 0.5 ml; Säule: Daicel Chiralpak^{®} IC 5 µm, 250 × 20 mm; Laufmittel: Ethanol 100%; Fluß: 15 ml/min; Temperature 25°C; UV Detektion: 210 nm]. Nach der Trennung, wurden 21.8 mg von Enantiomer 1, welches zuerst eluierte, und 20.5 mg von Enantiomer 2, welches später eluierte, isoliert.

Analytische chirale HPLC: Rₜ = 2.23 min, d.e. = 99% [Säule: Daicel Chiralpak^{®} IC 50 × 4.6 mm; Laufmittel: Ethanol 100%; Fluß: 1 ml/min; UV Detektion: 220 nm].
LC-MS (Methode 3): Rₜ = 1.20 min; MS (ESIpos): m/z = 355 [M+H]⁺
¹H-NMR (400 MHz, DMSO-d6) δ [ppm]: 1.234 (0.45), 1.652 (0.44), 1.666 (0.63), 1.690 (1.15), 1.704 (1.26), 1.719 (1.03), 1.994 (0.81), 2.007 (1.49), 2.022 (1.37), 2.038 (0.99), 2.051 (1.09), 2.063 (0.98), 2.272 (16.00), 2.579 (2.68), 2.599 (2.22), 2.634 (0.56), 2.677 (0.55), 2.696 (0.79), 2.709 (0.88), 2.726 (0.50), 3.649 (0.48), 3.660 (0.84), 3.678 (0.82), 3.699 (0.51), 3.710 (1.60), 3.744 (1.61), 3.755 (0.54), 3.775 (0.85), 3.785 (0.45), 3.794 (0.86), 3.805 (0.51), 3.942 (0.45), 3.960 (0.52), 4.010 (0.86), 4.055 (1.96), 4.072 (0.56), 4.090 (0.43), 4.127 (1.81), 4.172 (0.85), 4.687 (0.72), 4.699 (1.44), 4.711 (0.88), 4.750 (6.83), 4.914 (0.54), 4.927 (0.90), 4.938 (0.57), 7.102 (0.98), 7.109 (0.75), 7.124 (14.74), 7.147 (0.92).

### Beispiel 120

### (5RS)-5-[(3-Hydroxyazetidin-1-yl)carbonyl]-2-(4-methylbenzyl)-5,6,7,8-tetrahydro[1,2,4]triazolo[4,3-a]pyridin-3(2H)-on (Racemat)

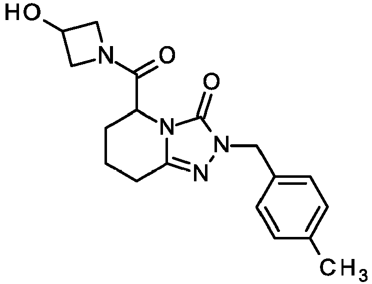

(5RS)-2-(4-Methylbenzyl)-3-oxo-2,3,5,6,7,8-hexahydro[1,2,4]triazolo[4,3-a]pyridin-5-carbonsäure (Racemat) (150 mg, 522 µmol) wurde mit Azetidin-3-olhydrochlorid (60.1 mg, 548 µmol) in DMF (1.7 ml) und Pyridin (330 µl) bei Raumtemperatur vorgelegt. Anschließend wurde O-(7-Azabenzotriazol-1-yl)-N,N,N',N'-tetramethyluronium-hexafluorphosphat (218 mg, 574 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 143 mg (80 % d. Th.) der Titelverbindung erhalten.
LC-MS (Methode 4): Rₜ = 0.62 min; MS (ESIpos): m/z = 343 [M+H]⁺
¹H-NMR (400 MHz, DMSO-d6) δ [ppm]: -0.008 (0.91), 0.008 (0.71), 1.696 (1.30), 1.706 (1.24), 1.889 (0.47), 1.902 (0.46), 1.913 (0.67), 1.925 (1.00), 1.967 (0.52), 1.980 (0.70), 1.992 (0.73), 2.001 (0.62), 2.270 (16.00), 2.522 (1.16), 2.570 (1.78), 2.582 (0.90), 2.612 (0.63), 3.598 (0.60), 3.617 (0.66), 3.624 (0.70), 3.636 (0.52), 3.648 (0.55), 3.661 (0.60), 3.673 (0.54), 3.917 (0.67), 3.928 (0.77), 4.018 (0.89), 4.032 (1.30), 4.042 (1.18), 4.056 (1.06), 4.103 (0.45), 4.118 (0.53), 4.129 (0.47), 4.144 (0.44), 4.328 (0.49), 4.348 (0.81), 4.368 (0.51), 4.481 (1.32), 4.495 (2.40), 4.504 (2.75), 4.518 (1.96), 4.740 (5.20), 4.745 (4.96), 5.800 (0.90), 7.103 (0.67), 7.123 (13.53), 7.143 (0.69).

### Beispiel 121

### (5RS)-5-[(3-Hydroxyazetidin-1 -yl)carbonyl]-2-(4-methylbenzyl)-5,6,7,8-tetrahydro[1,2,4]triazolo[4,3-a]pyridin-3(2H)-on (Enantiomer 2)

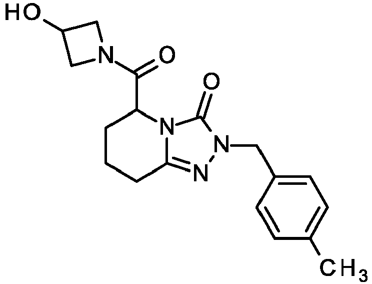

(5RS)-5-[(3-Hydroxyazetidin-1-yl)carbonyl]-2-(4-methylbenzyl)-5,6,7,8-tetrahydro[1,2,4]triazolo[4,3-a]pyridin-3(2H)-on (Racemat) wurde durch chirale präparative SFC getrennt [Probenvorbereitung: 115 mg gelöst in 15 ml Methanol. Injektionsvolumen: 0.5 ml; Säule: Daicel Chiralpak^{®} AD 5 µm, 250 × 20 mm; Laufmittel: CO₂/Methanol 80:20; Fluß: 80 ml/min; Temperature 40°C; UV Detektion: 210 nm]. Nach der Trennung, wurden 57.4 mg von Enantiomer 1, welches zuerst eluierte, und 46.4 mg von Enantiomer 2, welches später eluierte, isoliert.

Analytische chirale HPLC (SFC): Rₜ = 2.0 min, d.e. = 99% [Säule: Daicel Chiralcel^{®} AD 50 × 4.6 mm; Laufmittel: CO₂/Methanol 80:20; Fluß: 3 ml/min; UV Detektion: 220 nm].
LC-MS (Methode 3): Rₜ = 1.06 min; MS (ESIpos): m/z = 343 [M+H]⁺
¹H-NMR (400 MHz, DMSO-d6) δ [ppm]: 1.696 (1.41), 1.706 (1.35), 1.889 (0.50), 1.901 (0.50), 1.913 (0.71), 1.925 (1.07), 1.980 (0.74), 1.992 (0.78), 2.002 (0.68), 2.270 (16.00), 2.522 (1.52), 2.570 (1.94), 2.583 (1.02), 2.599 (0.46), 2.612 (0.69), 3.598 (0.65), 3.616 (0.70), 3.624 (0.74), 3.634 (0.56), 3.647 (0.59), 3.660 (0.63), 3.673 (0.57), 3.917 (0.71), 3.928 (0.83), 4.018 (0.95), 4.032 (1.37), 4.042 (1.26), 4.056 (1.11), 4.103 (0.46), 4.118 (0.56), 4.129 (0.51), 4.144 (0.49), 4.328 (0.51), 4.348 (0.85), 4.368 (0.55), 4.496 (2.50), 4.504 (3.04), 4.518 (2.20), 4.740 (5.50), 4.745 (5.33), 5.801 (3.34), 7.103 (0.68), 7.123 (13.86).

### Beispiel 122

### (5RS)-5-{[(3R)-3-Hydroxypyrrolidin-1-yl]carbonyl}-2-(4-methylbenzyl)-5,6,7,8-tetrahydro[1,2,4]triazolo[4,3-a]pyridin-3(2H)-on (Enantiomer 1)

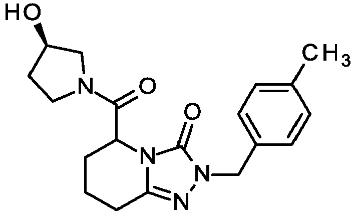

(5RS)-2-(4-Methylbenzyl)-3-oxo-2,3,5,6,7,8-hexahydro[1,2,4]triazolo[4,3-a]pyridin-5-carbonsäure (Enantiomer 1) (75.0 mg, 261 µmol) wurde in DMF (3.1 ml) und Dichlormethan (1.5 ml) bei Raumtemperatur vorgelegt. Anschließend wurde 1-[Bis(dimethylamino)methylen]-1H-benzotriazol-1-ium-3-oxidhexafluorophosphat (129 mg, 339 µmol) und N,N-Diisopropylethylamin (91 µl, 520 µmol) zugegeben. Nach 15 min Rühren wurde (3R)-Pyrrolidin-3-olhydrochlorid (38.7 mg, 313 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 58.1 mg (62 % d. Th.) der Titelverbindung erhalten.
LC-MS (Methode 1): Rₜ = 0.86 min; MS (ESIpos): m/z = 357 [M+H]⁺
¹H-NMR (400 MHz, DMSO-d6) δ [ppm]: -0.008 (0.58), 0.008 (0.62), 1.649 (0.52), 1.662 (0.50), 1.675 (0.55), 1.689 (0.69), 1.701 (0.85), 1.714 (1.02), 1.721 (1.03), 1.730 (0.92), 1.743 (0.71), 1.754 (0.61), 1.762 (0.56), 1.848 (0.52), 1.859 (0.74), 1.870 (0.56), 1.880 (0.78), 1.891 (0.73), 1.902 (0.55), 1.940 (0.57), 1.971 (1.66), 1.977 (1.57), 1.996 (0.91), 2.003 (0.74), 2.010 (0.72), 2.017 (0.69), 2.027 (0.62), 2.037 (0.43), 2.045 (0.47), 2.052 (0.40), 2.272 (16.00), 2.478 (0.56), 2.518 (1.68), 2.557 (0.61), 2.569 (0.78), 2.582 (1.40), 2.593 (0.73), 2.624 (0.54), 3.199 (0.79), 3.230 (1.12), 3.361 (1.13), 3.372 (1.78), 3.390 (1.59), 3.413 (0.60), 3.454 (0.53), 3.481 (0.81), 3.544 (0.72), 3.560 (1.02), 3.568 (1.26), 3.584 (0.76), 3.595 (0.51), 3.735 (0.41), 3.755 (0.62), 4.264 (0.76), 4.358 (0.65), 4.699 (0.78), 4.708 (0.78), 4.714 (0.96), 4.723 (0.76), 4.741 (4.48), 4.746 (5.17), 4.770 (0.77), 4.779 (0.99), 4.786 (1.01), 4.794 (0.71), 4.956 (0.76), 5.077 (0.89), 5.085 (0.87), 7.102 (0.70), 7.124 (15.25), 7.145 (0.84).

Die (5S)-Konfiguration wurde aufgrund der Kristallstrukturaufklärung für Beispiel 26, 108, 113, 157, 237, 358 und 454 zugeordnet.

### (5S)-5-{[(3R)-3-Hydroxypyrrolidin-1 -yl]carbonyl}-2-(4-methylbenzyl)-5,6,7,8-tetrahydro[1,2,4]triazolo[4,3-a]pyridin-3(2H)-on

### Beispiel 123

### (5RS)-5-{[3-(Difluormethoxy)azetidin-1-yl]carbonyl}-2-(4-methylbenzyl)-5,6,7,8-tetrahydro[1,2,4]triazolo[4,3-a]pyridin-3(2H)-on (Racemat)

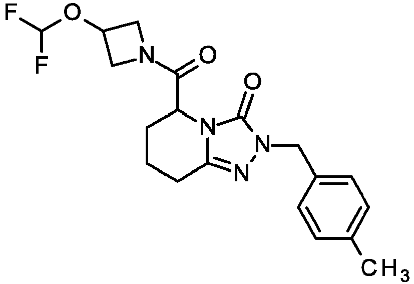

(5RS)-2-(4-Methylbenzyl)-3-oxo-2,3,5,6,7,8-hexahydro[1,2,4]triazolo[4,3-a]pyridin-5-carbonsäure (Racemat) (150 mg, 522 µmol) wurde mit 3-(Difluormethoxy)azetidin (67.5 mg, 548 µmol) in Pyridin/DMF (5/1) (3.0 ml) bei Raumtemperatur vorgelegt. Anschließend wurde O-(7-Azabenzotriazol-1-yl)-N,N,N',N'-tetramethyluronium-hexafluorphosphat (218 mg, 574 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 27.3 mg (13 % d. Th.) der Titelverbindung erhalten.
LC-MS (Methode 1): Rₜ = 1.06 min; MS (ESIpos): m/z = 393 [M+H]⁺
¹H-NMR (400 MHz, DMSO-d6) δ [ppm]: 0.008 (0.44), 1.701 (1.79), 1.714 (1.40), 1.941 (0.73), 2.003 (0.73), 2.017 (0.62), 2.271 (16.00), 2.327 (0.48), 2.366 (0.48), 2.518 (2.87), 2.559 (1.81), 2.573 (1.95), 2.587 (0.96), 2.602 (0.45), 2.615 (0.71), 2.670 (0.52), 2.710 (0.51), 3.701 (1.17), 3.840 (0.45), 3.858 (0.92), 3.867 (0.93), 3.885 (0.56), 3.895 (0.52), 4.175 (0.85), 4.189 (0.83), 4.201 (0.93), 4.253 (0.42), 4.271 (0.51), 4.281 (0.47), 4.300 (0.73), 4.327 (0.54), 4.336 (0.52), 4.530 (1.75), 4.553 (0.42), 4.651 (0.41), 4.669 (0.55), 4.691 (0.42), 4.746 (6.96), 4.774 (0.68), 5.004 (0.62), 5.050 (0.56), 6.587 (0.54), 6.600 (0.58), 6.773 (1.10), 6.786 (1.21), 6.960 (0.54), 6.972 (0.58), 7.105 (0.65), 7.126 (14.23).

### Beispiel 124

### (5RS)-2-(4-Methylbenzyl)-5-{[3-(trifluormethyl)azetidin-1-yl]carbonyl}-5,6,7,8-tetrahydro[1,2,4]triazolo[4,3-a]pyridin-3(2H)-on (Racemat)

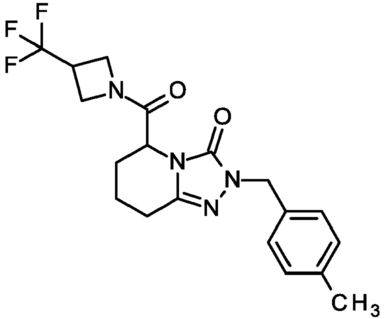

(5RS)-2-(4-Methylbenzyl)-3-oxo-2,3,5,6,7,8-hexahydro[1,2,4]triazolo[4,3-a]pyridin-5-carbonsäure (Racemat) (150 mg, 522 µmol) wurde mit 3-(Trifluormethyl)azetidinhydrochlorid (92.8 mg, 574 µmol) in Pyridin/DMF (5/1) (3.0 ml) bei Raumtemperatur vorgelegt. Anschließend wurde O-(7-Azabenzotriazol-1-yl)-N,N,N',N'-tetramethyluronium-hexafluorphosphat (208 mg, 548 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 139 mg (68 % d. Th.) der Titelverbindung erhalten.
LC-MS (Methode 1): Rₜ = 1.12 min; MS (ESIpos): m/z = 395 [M+H]⁺
¹H-NMR (400 MHz, DMSO-d6) δ [ppm]: 1.694 (1.05), 1.707 (1.44), 1.720 (1.08), 2.003 (0.41), 2.014 (0.44), 2.027 (0.47), 2.271 (12.27), 2.501 (8.74), 2.567 (0.81), 2.575 (0.93), 3.845 (0.41), 3.871 (0.47), 3.934 (0.49), 3.948 (0.43), 4.081 (0.45), 4.105 (0.66), 4.160 (0.44), 4.184 (0.67), 4.207 (0.70), 4.219 (0.41), 4.230 (0.45), 4.386 (0.53), 4.401 (0.48), 4.430 (0.50), 4.453 (0.73), 4.522 (0.44), 4.536 (0.73), 4.548 (0.83), 4.560 (0.69), 4.572 (0.48), 4.584 (0.42), 4.607 (0.74), 4.750 (5.64), 7.104 (0.48), 7.125 (16.00), 7.147 (0.48).

### Beispiel 125

### (5RS)-5-[(3-Methoxyazetidin-1 -yl)carbonyl]-2-(4-methylbenzyl)-5,6,7,8-tetrahydro[1,2,4]triazolo[4,3-a]pyridin-3(2H)-on (Racemat)

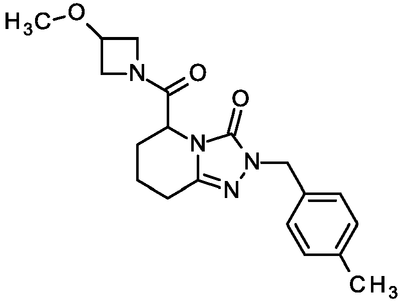

(5RS)-2-(4-Methylbenzyl)-3-oxo-2,3,5,6,7,8-hexahydro[1,2,4]triazolo[4,3-a]pyridin-5-carbonsäure (Racemat) (150 mg, 522 µmol) wurde mit 3-Methoxyazetidin (47.8 mg, 548 µmol) in Pyridin/DMF (5/1) (3.0 ml) bei Raumtemperatur vorgelegt. Anschließend wurde O-(7-Azabenzotriazol-1-yl)-N,N,N',N'-tetramethyluronium-hexafluorphosphat (218 mg, 574 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 123 mg (66 % d. Th.) der Titelverbindung erhalten.
LC-MS (Methode 3): Rₜ = 1.25 min; MS (ESIpos): m/z = 357 [M+H]⁺
¹H-NMR (400 MHz, DMSO-d6) δ [ppm]: -0.008 (1.40), 0.008 (1.18), 1.674 (0.74), 1.686 (1.12), 1.697 (1.34), 1.928 (0.77), 1.989 (0.59), 1.999 (0.64), 2.024 (0.45), 2.270 (14.99), 2.522 (1.34), 2.571 (1.48), 2.584 (0.75), 2.613 (0.47), 3.223 (14.06), 3.663 (0.43), 3.671 (0.43), 3.688 (0.50), 3.700 (0.80), 3.712 (0.47), 3.729 (0.51), 3.737 (0.51), 4.007 (0.82), 4.027 (0.89), 4.051 (0.44), 4.087 (0.43), 4.104 (0.57), 4.115 (0.77), 4.128 (0.73), 4.148 (0.57), 4.216 (0.43), 4.226 (0.61), 4.242 (0.49), 4.252 (0.47), 4.259 (0.44), 4.269 (0.62), 4.341 (0.53), 4.364 (0.53), 4.509 (1.78), 4.525 (0.92), 4.742 (6.60), 7.102 (0.59), 7.124 (16.00), 7.145 (0.52).

### Beispiel 126

### (5RS)-5-[(cis)-3-Azabicyclo[3.1.0]hex-3-ylcarbonyl]-2-(4-methylbenzyl)-5,6,7,8-tetrahydro[1,2,4]triazolo[4,3-a]pyridin-3(2H)-on (Racemat)

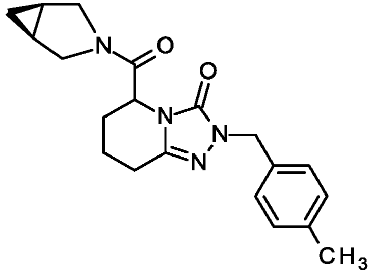

(5RS)-2-(4-Methylbenzyl)-3-oxo-2,3,5,6,7,8-hexahydro[1,2,4]triazolo[4,3-a]pyridin-5-carbonsäure (Racemat) (150 mg, 522 µmol) wurde mit (cis)-3-Azabicyclo[3.1.0]hexanhydrochlorid (65.6 mg, 548 µmol) in Pyridin/DMF (5/1) (3.0 ml) bei Raumtemperatur vorgelegt. Anschließend wurde O-(7-Azabenzotriazol-1-yl)-N,N,N',N'-tetramethyluronium-hexafluorphosphat (218 mg, 574 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 133 mg (72 % d. Th.) der Titelverbindung erhalten.
LC-MS (Methode 4): Rₜ = 0.78 min; MS (ESIpos): m/z = 353 [M+H]⁺
¹H-NMR (400 MHz, DMSO-d6) δ [ppm]: 0.049 (0.40), 0.060 (1.02), 0.071 (1.06), 0.081 (0.51), 0.096 (0.86), 0.107 (0.86), 0.689 (0.74), 0.701 (0.76), 0.707 (0.73), 0.713 (0.71), 0.725 (0.66), 1.515 (0.51), 1.524 (0.73), 1.534 (0.65), 1.543 (0.64), 1.550 (0.53), 1.559 (0.62), 1.568 (0.49), 1.612 (0.56), 1.621 (0.73), 1.630 (0.94), 1.639 (0.89), 1.649 (0.99), 1.659 (1.14), 1.674 (1.73), 1.684 (1.79), 1.695 (1.24), 1.922 (0.50), 1.935 (0.45), 1.976 (0.46), 1.994 (0.46), 2.002 (0.51), 2.019 (0.42), 2.270 (16.00), 2.518 (1.36), 2.559 (1.36), 2.571 (0.70), 2.601 (0.44), 3.203 (0.62), 3.214 (0.65), 3.232 (0.74), 3.243 (0.72), 3.335 (1.10), 3.346 (0.84), 3.497 (1.56), 3.526 (1.22), 3.533 (0.66), 3.544 (0.63), 3.560 (0.73), 3.570 (0.68), 3.659 (2.48), 3.667 (1.15), 3.692 (1.81), 3.722 (1.49), 3.840 (1.36), 3.867 (1.16), 4.618 (0.59), 4.627 (0.72), 4.633 (0.74), 4.643 (0.58), 4.679 (0.57), 4.718 (2.71), 4.729 (2.94), 4.738 (4.39), 4.778 (0.70), 7.094 (1.14), 7.116 (9.92), 7.121 (9.92), 7.142 (1.16).

### Beispiel 127

### 2-(4-Methylbenzyl)- (5RS)-{[(2S)-2-methylpyrrolidin-1-yl]carbonyl}-5,6,7,8-tetrahydro[1,2,4]triazolo[4,3-a]pyridin-3(2H)-on (Enantiomer 1)

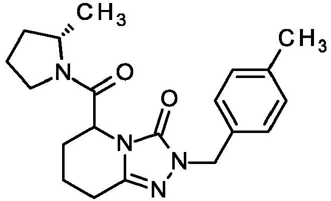

(5RS)-2-(4-Methylbenzyl)-3-oxo-2,3,5,6,7,8-hexahydro[1,2,4]triazolo[4,3-a]pyridin-5-carbonsäure (Enantiomer 1) (75.0 mg, 261 µmol) wurde in Dichlormethan (1.5 ml) und DMF (3.0 ml) bei Raumtemperatur vorgelegt. Anschließend wurde 1-[Bis(dimethylamino)methylen]-1H-benzotriazol-1-ium-3-oxidhexafluorophosphat (129 mg, 339 µmol) und N,N-Diisopropylethylamin (64 µl, 370 µmol) zugegeben. Nach 15 min Rühren wurde (2S)-2-Methylpyrrolidin (26.7 mg, 313 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 56.3 mg (61 % d. Th.) der Titelverbindung erhalten.
LC-MS (Methode 3): Rₜ = 1.45 min; MS (ESIpos): m/z = 355 [M+H]⁺
¹H-NMR (400 MHz, DMSO-d6) δ [ppm]: 1.083 (4.23), 1.098 (4.29), 1.166 (1.19), 1.182 (1.20), 1.515 (0.43), 1.522 (0.45), 1.544 (0.43), 1.676 (0.49), 1.688 (0.88), 1.702 (1.05), 1.718 (0.90), 1.730 (0.42), 1.862 (0.51), 1.870 (0.56), 1.887 (0.78), 1.905 (1.14), 1.924 (0.88), 1.939 (1.13), 1.950 (1.08), 1.969 (0.74), 1.995 (0.83), 2.015 (0.71), 2.031 (0.56), 2.270 (12.19), 2.524 (0.87), 2.569 (1.14), 2.581 (0.54), 3.432 (0.49), 3.456 (0.49), 3.669 (0.44), 3.676 (0.51), 3.992 (0.54), 4.669 (0.86), 4.679 (1.59), 4.684 (0.95), 4.694 (0.68), 4.706 (0.71), 4.717 (1.84), 4.746 (0.81), 4.777 (1.77), 4.816 (0.82), 7.122 (16.00).

Die (5S)-Konfiguration wurde aufgrund der Kristallstrukturaufklärung für Beispiel 26, 108, 113, 157, 237, 358 und 454 zugeordnet.

### 2-(4-Methylbenzyl)-(5S)-{[(2S)-2-methylpyrrolidin-1-yl]carbonyl}-5,6,7,8-tetrahydro[1,2,4]triazolo[4,3-a]pyridin-3(2H)-on

### Beispiel 128

### (5RS)-{[(3RS)-1,1-Difluor-5-azaspiro[2.4]hept-5-yl]carbonyl}-2-(4-methylbenzyl)-5,6,7,8-tetrahydro[1,2,4]triazolo[4,3-a]pyridin-3(2H)-on (Diastereomerengemisch; 2 Isomere)

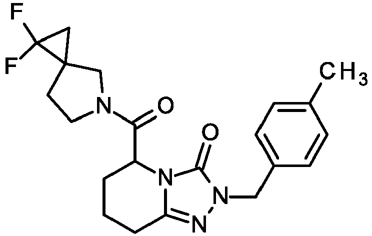

(5RS)-2-(4-Methylbenzyl)-3-oxo-2,3,5,6,7,8-hexahydro[1,2,4]triazolo[4,3-a]pyridin-5-carbonsäure (Enantiomer 1) (200 mg, 696 µmol) wurde in Dichlormethan (4.0 ml) und DMF (8.0 ml) bei Raumtemperatur vorgelegt. Anschließend wurde 1-[Bis(dimethylamino)methylen]-1H-benzotriazol-1-ium-3-oxidhexafluorophosphat (343 mg, 905 µmol) und N,N-Diisopropylethylamin (320 µl, 1.8 mmol) zugegeben. Nach 15 min Rühren wurde (3RS)-1,1-Difluor-5-azaspiro[2.4]heptanhydrochlorid (142 mg, 835 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 218 mg (78 % d. Th.) der Titelverbindung erhalten.
LC-MS (Methode 3): Rₜ = 1.56 min; MS (ESIpos): m/z = 403 [M+H]⁺
¹H-NMR (400 MHz, DMSO-d6) δ [ppm]: -0.008 (1.41), 0.008 (1.27), 1.069 (0.81), 1.159 (0.77), 1.601 (0.42), 1.614 (0.52), 1.648 (1.24), 1.668 (1.35), 1.694 (1.64), 1.710 (1.47), 2.008 (1.15), 2.099 (0.56), 2.115 (0.57), 2.161 (0.53), 2.271 (15.89), 2.572 (0.75), 2.584 (1.31), 2.625 (0.49), 3.455 (1.29), 3.497 (0.60), 3.552 (0.59), 3.582 (0.47), 3.640 (0.47), 3.666 (0.52), 3.718 (0.52), 3.751 (0.73), 3.764 (0.69), 3.805 (0.46), 4.710 (0.65), 4.719 (0.61), 4.742 (3.92), 4.794 (0.69), 7.101 (0.69), 7.125 (16.00), 7.146 (0.68). (Mischung von Diastereomeren)

Die (5S)-Konfiguration wurde aufgrund der Kristallstrukturaufklärung für Beispiel 26, 108, 113, 157, 237, 358 und 454 zugeordnet.

### (5S)-{[(3RS)-1,1-Difluor-5-azaspiro[2.4]hept-5-yl]carbonyl}-2-(4-methylbenzyl)-5,6,7,8-tetrahydro[1,2,4]triazolo[4,3-a]pyridin-3(2H)-on (Diastereomerengemisch; 2 Isomere)

### Beispiel 129

### (5RS)-{[(3RS)-1,1-Difluor-5-azaspiro[2.4]hept-5-yl]carbonyl}-2-(4-methylbenzyl)-5,6,7,8-tetrahydro[1,2,4]triazolo[4,3-a]pyridin-3(2H)-on (Isomer 2)

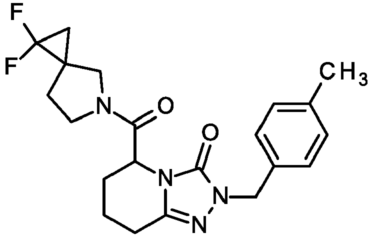

(5RS)-{[(3RS)-1,1-Difluor-5-azaspiro[2.4]hept-5-yl]carbonyl}-2-(4-methylbenzyl)-5,6,7,8-tetrahydro[1,2,4]triazolo[4,3-a]pyridin-3(2H)-on (Diastereomerengemisch; 2 Isomere) wurde durch chirale präparative HPLC getrennt [Probenvorbereitung: 210 mg gelöst in 4 ml Acetonitril; Injektionsvolumen: 0.2 ml; Säule: Daicel Chiralpak^{®} ID 5 µm, 250 × 20 mm; Laufmittel: Methyl tert-butylether/Acetonitril 50:50; Fluß: 15 ml/min; Temperature 40°C; UV Detektion: 210 nm]. Nach der Trennung, wurden 99.0 mg von Enantiomer 1, welches zuerst eluierte, und 101.0 mg von Enantiomer 2, welches später eluierte, isoliert.

Analytische chirale HPLC: Rₜ = 10.49 min, d.e. = 99% [Säule: Daicel Chiralcel^{®} ID 250 × 4.6 mm; Laufmittel: Methyl tert-butylether/Acetonitril 50:50; Fluß: 1 ml/min; UV Detektion: 220 nm].
LC-MS (Methode 4): Rₜ = 0.84 min; MS (ESIpos): m/z = 403 [M+H]⁺
¹H-NMR (400 MHz, DMSO-d6) δ [ppm]: -0.008 (1.53), 0.008 (1.55), 1.604 (0.44), 1.617 (0.64), 1.637 (0.89), 1.654 (1.26), 1.669 (1.51), 1.689 (1.60), 1.702 (1.64), 1.710 (1.61), 1.721 (1.32), 2.008 (1.66), 2.025 (1.55), 2.051 (0.73), 2.070 (0.51), 2.084 (0.52), 2.102 (0.51), 2.115 (0.48), 2.165 (0.55), 2.181 (0.52), 2.272 (15.03), 2.561 (0.81), 2.572 (0.83), 2.584 (1.54), 2.596 (0.83), 2.626 (0.57), 3.455 (2.27), 3.478 (0.59), 3.486 (0.60), 3.497 (1.15), 3.515 (0.61), 3.640 (0.76), 3.647 (0.80), 3.665 (0.96), 3.804 (0.82), 3.831 (0.61), 3.881 (0.48), 3.895 (0.42), 4.710 (0.54), 4.725 (0.81), 4.743 (5.21), 4.750 (2.79), 4.783 (0.76), 4.798 (0.93), 4.808 (0.62), 7.101 (0.75), 7.123 (16.00), 7.147 (0.72).

### Beispiel 130

### (5RS)-{[(3S)-3-Methoxypyrrolidin-1-yl]carbonyl}-2-(4-methylbenzyl)-5,6,7,8-tetrahydro[1,2,4]triazolo[4,3-a]pyridin-3(2H)-on (Enantiomer 1)

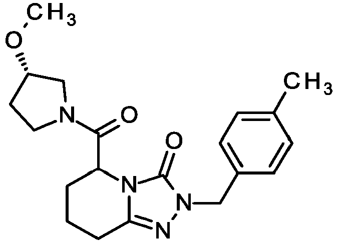

(5RS)-2-(4-Methylbenzyl)-3-oxo-2,3,5,6,7,8-hexahydro[1,2,4]triazolo[4,3-a]pyridin-5-carbonsäure (Enantiomer 1) (75.0 mg, 261 µmol) wurde in DMF (3.1 ml) und Dichlormethan (1.5 ml) bei Raumtemperatur vorgelegt. Anschließend wurde 1-[Bis(dimethylamino)methylen]-1H-benzotriazol-1-ium-3-oxidhexafluorophosphat (129 mg, 339 µmol) und N,N-Diisopropylethylamin (120 µl, 680 µmol) zugegeben. Nach 15 min Rühren wurde (3S)-3-Methoxypyrrolidinhydrochlorid (43.1 mg, 313 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 40.8 mg (42 % d. Th.) der Titelverbindung erhalten.
LC-MS (Methode 1): Rₜ = 0.96 min; MS (ESIpos): m/z = 371 [M+H]⁺
¹H-NMR (400 MHz, DMSO-d6) δ [ppm]: -0.008 (2.98), 0.008 (2.66), 1.644 (0.58), 1.656 (0.64), 1.668 (0.67), 1.702 (0.97), 1.712 (0.97), 1.723 (0.75), 1.860 (0.43), 1.884 (0.68), 1.894 (0.70), 1.906 (0.55), 1.918 (0.89), 1.931 (0.91), 1.940 (0.92), 1.981 (1.60), 1.993 (1.41), 2.003 (1.20), 2.017 (0.89), 2.038 (0.66), 2.065 (0.57), 2.272 (16.00), 2.567 (0.89), 2.578 (1.51), 2.590 (0.88), 2.621 (0.54), 3.235 (12.99), 3.269 (13.24), 3.362 (1.64), 3.384 (0.55), 3.392 (0.75), 3.405 (1.07), 3.416 (1.20), 3.438 (0.53), 3.449 (0.86), 3.455 (0.70), 3.473 (0.65), 3.556 (0.51), 3.567 (0.57), 3.585 (0.78), 3.596 (0.75), 3.709 (0.94), 3.737 (0.69), 3.768 (0.42), 3.787 (0.61), 3.956 (0.89), 4.044 (0.83), 4.742 (6.80), 4.765 (0.83), 4.779 (1.64), 4.788 (1.54), 4.797 (0.73), 7.100 (0.86), 7.123 (14.91), 7.145 (0.90).

Die (5S)-Konfiguration wurde aufgrund der Kristallstrukturaufklärung für Beispiel 26, 108, 113, 157, 237, 358 und 454 zugeordnet.

### (5S)-{[(3S)-3-Methoxypyrrolidin-1-yl]carbonyl}-2-(4-methylbenzyl)-5,6,7,8-tetrahydro[1,2,4]triazolo[4,3-a]pyridin-3(2H)-on

### Beispiel 131

### (5RS)-{[3-Hydroxy-3-(trifluormethyl)azetidin-1-yl]carbonyl}-2-(4-methylbenzyl)-5,6,7,8-tetrahydro[1,2,4]triazolo[4,3-a]pyridin-3(2H)-on (Racemat)

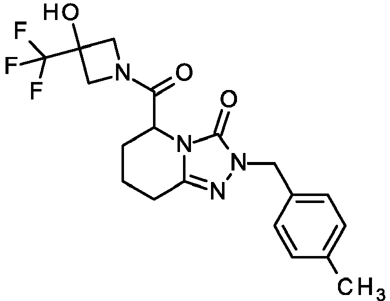

(5RS)-2-(4-Methylbenzyl)-3-oxo-2,3,5,6,7,8-hexahydro[1,2,4]triazolo[4,3-a]pyridin-5-carbonsäure (Racemat) (150 mg, 522 µmol) wurde mit 3-(Trifluormethyl)azetidin-3-olhydrochlorid (97.3 mg, 548 µmol) in Pyridin/DMF (5/1) (3.0 ml) bei Raumtemperatur vorgelegt. Anschließend wurde O-(7-Azabenzotriazol-1-yl)-N,N,N',N'-tetramethyluronium-hexafluorphosphat (218 mg, 574 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 147 mg (68 % d. Th.) der Titelverbindung erhalten.
LC-MS (Methode 4): Rₜ = 0.76 min; MS (ESIpos): m/z = 411 [M+H]⁺
¹H-NMR (400 MHz, DMSO-d6) δ [ppm]: -0.008 (0.49), 0.008 (0.45), 1.704 (1.35), 1.715 (1.20), 2.019 (0.55), 2.027 (0.58), 2.036 (0.57), 2.271 (16.00), 2.518 (0.75), 2.567 (1.09), 2.575 (1.21), 2.587 (1.01), 2.598 (0.45), 3.857 (0.57), 3.885 (0.67), 3.933 (0.61), 3.961 (0.86), 4.081 (1.14), 4.108 (0.81), 4.164 (0.94), 4.192 (1.19), 4.222 (0.65), 4.351 (0.56), 4.376 (0.89), 4.454 (1.17), 4.479 (0.74), 4.558 (0.60), 4.573 (0.90), 4.585 (0.67), 4.593 (0.61), 4.609 (0.77), 4.621 (1.19), 4.647 (0.77), 4.741 (2.29), 4.747 (2.82), 4.754 (4.40), 7.103 (0.62), 7.125 (13.60), 7.145 (0.56), 7.545 (1.60).

### Beispiel 132

### (5RS)-2-(4-Methylbenzyl)-{[(2R)-2-methylpyrrolidin-1-yl]carbonyl}-5,6,7,8-tetrahydro[1,2,4]triazolo[4,3-a]pyridin-3(2H)-on (Enantiomer 1)

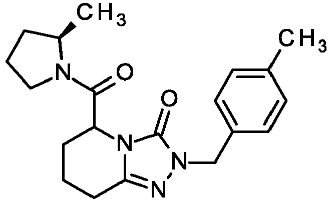

(5RS)-2-(4-Methylbenzyl)-3-oxo-2,3,5,6,7,8-hexahydro[1,2,4]triazolo[4,3-a]pyridin-5-carbonsäure (Enantiomer 1) (75.0 mg, 261 µmol) wurde in Dichlormethan (1.3 ml) und DMF (2.9 ml) bei Raumtemperatur vorgelegt. Anschließend wurde 1-[Bis(dimethylamino)methylen]-1H-benzotriazol-1-ium-3-oxidhexafluorophosphat (129 mg, 339 µmol) und N,N-Diisopropylethylamin (64 µl, 370 µmol) zugegeben. Nach 15 min Rühren wurde (2R)-2-Methylpyrrolidin (26.7 mg, 313 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 46.4 mg (50 % d. Th.) der Titelverbindung erhalten.
LC-MS (Methode 3): Rₜ = 1.47 min; MS (ESIpos): m/z = 355 [M+H]⁺
¹H-NMR (400 MHz, DMSO-d6) δ [ppm]: -0.008 (1.50), 0.008 (1.44), 1.057 (6.00), 1.073 (6.07), 1.265 (2.61), 1.281 (2.63), 1.497 (0.42), 1.508 (0.57), 1.517 (0.66), 1.526 (0.56), 1.650 (0.63), 1.659 (0.58), 1.679 (0.60), 1.694 (0.82), 1.714 (1.10), 1.724 (0.98), 1.859 (0.74), 1.870 (0.72), 1.876 (0.73), 1.886 (0.73), 1.897 (1.06), 1.915 (1.11), 1.924 (0.87), 1.933 (1.01), 1.945 (1.27), 1.963 (1.63), 1.977 (1.11), 1.997 (0.92), 2.005 (0.85), 2.015 (0.70), 2.025 (0.74), 2.033 (0.69), 2.042 (0.64), 2.062 (0.48), 2.272 (16.00), 2.583 (1.13), 2.595 (0.65), 2.625 (0.44), 3.247 (0.43), 3.379 (0.41), 3.516 (0.46), 3.523 (0.68), 3.541 (0.68), 3.552 (0.42), 3.566 (0.43), 3.584 (0.85), 3.602 (0.48), 3.609 (0.46), 4.022 (0.62), 4.030 (0.60), 4.038 (0.47), 4.107 (0.46), 4.691 (0.89), 4.700 (1.13), 4.707 (1.26), 4.715 (1.20), 4.739 (6.57), 7.099 (1.02), 7.120 (11.38), 7.124 (11.23), 7.145 (0.99).

Die (5S)-Konfiguration wurde aufgrund der Kristallstrukturaufklärung für Beispiel 26, 108, 113, 157, 237, 358 und 454 zugeordnet.

### (5S)-2-(4-Methylbenzyl)-{[(2R)-2-methylpyrrolidin-1-yl]carbonyl}-5,6,7,8-tetrahydro[1,2,4]triazolo[4,3-a]pyridin-3(2H)-on

### Beispiel 133

### (5RS)-2-(4-Methylbenzyl)-5-[(3-oxoazetidin-1-yl)carbonyl]-5,6,7,8-tetrahydro[1,2,4]triazolo[4,3-a]pyridin-3(2H)-on (Racemat)

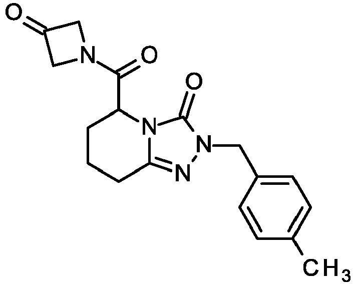

(5RS)-2-(4-Methylbenzyl)-3-oxo-2,3,5,6,7,8-hexahydro[1,2,4]triazolo[4,3-a]pyridin-5-carbonsäure (Racemat) (150 mg, 522 µmol) wurde mit Azetidin-3-on (39.0 mg, 548 µmol) in Pyridin/DMF (5/1) (3.0 ml) bei Raumtemperatur vorgelegt. Anschließend wurde O-(7-Azabenzotriazol-1-yl)-N,N,N',N'-tetramethyluronium-hexafluorphosphat (218 mg, 574 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 72.2 mg (41 % d. Th.) der Titelverbindung erhalten.
LC-MS (Methode 1): Rₜ = 0.89 min; MS (ESIpos): m/z = 341 [M+H]⁺
¹H-NMR (400 MHz, DMSO-d6) δ [ppm]: 1.710 (0.41), 1.721 (0.65), 1.731 (0.63), 1.743 (0.59), 2.059 (0.86), 2.072 (1.04), 2.085 (0.67), 2.271 (10.99), 2.517 (0.67), 2.559 (0.57), 2.590 (0.46), 2.603 (0.95), 2.616 (0.48), 4.662 (0.54), 4.675 (1.04), 4.688 (0.55), 4.743 (0.87), 4.758 (4.04), 4.766 (1.64), 7.128 (16.00).

### Beispiel 134

### (5RS,7RS)-5-[(3-Hydroxyazetidin-1-yl)carbonyl]-7-methyl-2-{[6-(trifluormethyl)pyridin-3-yl]methyl}-5,6,7,8-tetrahydro[1,2,4]triazolo[4,3-a]pyridin-3(2H)-on (Diastereomerengemisch; 4 Isomere)

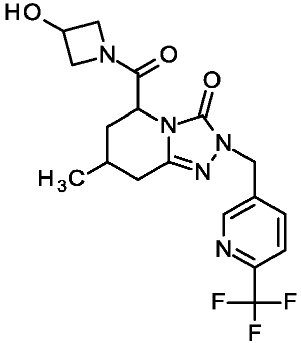

(5RS,7RS)-7-Methyl-3-oxo-2-{[6-(trifluormethyl)pyridin-3-yl]methyl}-2,3,5,6,7,8-hexahydro[1,2,4]triazolo[4,3-a]pyridin-5-carbonsäure (Diastereomerengemisch; 4 Isomere) (100 mg, 281 µmol) wurde in Dichlormethan (1.0 ml) und DMF (2.0 ml) bei Raumtemperatur vorgelegt. Anschließend wurde 1-[Bis(dimethylamino)methylen]-1H-benzotriazol-1-ium-3-oxidhexafluorophosphat (138 mg, 365 µmol) und N,N-Diisopropylethylamin (130 µl, 730 µmol) zugegeben. Nach 15 min Rühren wurde Azetidin-3-olhydrochlorid (36.9 mg, 337 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 85.4 mg (74 % d. Th.) der Titelverbindung erhalten.
LC-MS (Methode 1): Rₜ = 0.87 min; MS (ESIpos): m/z = 412 [M+H]⁺
¹H-NMR (400 MHz, DMSO-d6) δ [ppm]: -0.008 (2.42), 0.008 (2.61), 1.010 (8.92), 1.020 (11.30), 1.026 (11.21), 1.036 (9.74), 1.308 (0.59), 1.339 (1.81), 1.370 (2.83), 1.400 (2.12), 1.433 (0.76), 1.876 (1.61), 2.142 (3.55), 2.174 (4.35), 2.206 (1.62), 2.213 (1.69), 2.327 (0.64), 2.366 (0.41), 2.622 (2.64), 2.669 (2.68), 2.690 (0.68), 2.710 (0.45), 2.731 (6.01), 2.890 (7.03), 3.578 (1.58), 3.585 (1.51), 3.604 (1.66), 3.643 (1.32), 3.655 (1.35), 3.668 (1.47), 3.680 (1.39), 3.903 (1.92), 3.914 (1.76), 4.002 (1.25), 4.021 (1.65), 4.045 (2.41), 4.058 (1.45), 4.067 (1.50), 4.079 (1.42), 4.112 (1.16), 4.126 (1.43), 4.137 (1.26), 4.153 (1.15), 4.325 (1.22), 4.344 (2.16), 4.355 (1.88), 4.370 (3.12), 4.387 (2.44), 4.398 (2.75), 4.414 (1.49), 4.507 (4.97), 4.521 (3.48), 4.986 (16.00), 5.754 (1.00), 5.779 (4.75), 5.793 (4.89), 7.899 (1.61), 7.919 (11.72), 7.951 (1.52), 8.669 (7.52).

### Beispiel 135

### (5RS,7RS)-5-[(3-Hydroxyazetidin-1-yl)carbonyl]-7-methyl-2-{[6-(trifluormethyl)pyridin-3-yl]methyl}-5,6,7,8-tetrahydro[1,2,4]triazolo[4,3-a]pyridin-3(2H)-on (Isomer 1)

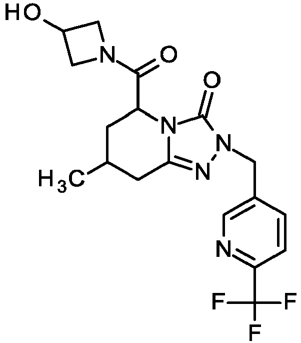

(5RS,7RS)-5-[(3-Hydroxyazetidin-1-yl)carbonyl]-7-methyl-2-{[6-(trifluormethyl)pyridin-3-yl]methyl}-5,6,7,8-tetrahydro[1,2,4]triazolo[4,3-a]pyridin-3(2H)-on (Diastereomerengemisch; 4 Isomere) wurde durch chirale präparative HPLC getrennt [Probenvorbereitung: 80 mg gelöst in 4 ml Ethanol; Injektionsvolumen: 0.3 ml; Säule: Daicel Chiralpak^{®} IC 5 µm, 250 × 20 mm; Laufmittel: n-Heptan/Ethanol 50:50; Fluß: 15 ml/min; Temperature 40°C; UV Detektion: 220 nm]. Nach der Trennung, wurden 32.0 mg von Isomer 1, welches zuerst eluierte, und 35.0 mg von Isomer 2, welches später eluierte, isoliert.

### Isomer 1:

Analytische chirale HPLC: Rₜ = 5.13 min, d.e. = 95% [Säule: Daicel Chiralcel^{®} IAC 250 × 4.6 mm; Laufmittel: i-Hexan/Ethanol 50:50; Fluß: 1 ml/min; UV Detektion: 220 nm].
LC-MS (Methode 4): Rₜ = 0.63 min; MS (ESIpos): m/z = 412 [M+H]⁺
¹H-NMR (600 MHz, DMSO-d6) δ [ppm]: 0.005 (0.61), 1.011 (8.14), 1.022 (16.00), 1.033 (8.42), 1.320 (0.60), 1.340 (1.30), 1.354 (0.89), 1.361 (1.38), 1.374 (1.45), 1.381 (0.86), 1.394 (1.38), 1.414 (0.70), 1.872 (1.32), 1.876 (1.34), 2.136 (1.59), 2.142 (1.78), 2.148 (2.62), 2.156 (2.85), 2.168 (2.59), 2.176 (2.80), 2.183 (1.75), 2.195 (1.42), 2.203 (1.39), 2.517 (0.80), 2.521 (0.80), 2.524 (0.64), 2.615 (0.44), 2.630 (2.25), 2.633 (2.12), 2.654 (2.01), 2.657 (2.01), 2.660 (1.79), 3.584 (1.49), 3.589 (1.37), 3.601 (1.52), 3.607 (1.32), 3.649 (1.18), 3.657 (1.22), 3.666 (1.31), 3.674 (1.27), 3.907 (1.65), 3.915 (1.54), 4.011 (1.14), 4.023 (1.42), 4.027 (1.24), 4.040 (1.18), 4.051 (1.14), 4.059 (1.27), 4.066 (1.30), 4.074 (1.23), 4.119 (1.10), 4.129 (1.22), 4.135 (1.21), 4.147 (1.04), 4.335 (1.09), 4.347 (1.87), 4.363 (2.11), 4.373 (1.76), 4.381 (3.04), 4.391 (3.07), 4.399 (1.69), 4.410 (1.48), 4.496 (2.30), 4.507 (4.00), 4.518 (2.41), 4.531 (0.73), 4.986 (10.70), 4.991 (10.02), 5.802 (6.92), 5.812 (6.99), 7.905 (1.16), 7.911 (1.30), 7.919 (6.21), 7.924 (8.62), 8.671 (4.90).

### Beispiel 136

### (5RS,7RS)-5-{[(3S)-3-Fluorpyrrolidin-1-yl]carbonyl}-7-methyl-2-{[6-(trifluormethyl)pyridin-3-yl]methyl}-5,6,7,8-tetrahydro[1,2,4]triazolo[4,3-a]pyridin-3(2H)-on (Diastereomerengemisch; 4 Isomere)

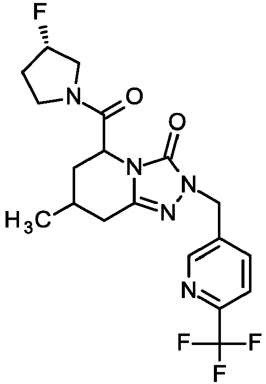

(5RS,7RS)-7-Methyl-3-oxo-2-{[6-(trifluormethyl)pyridin-3-yl]methyl}-2,3,5,6,7,8-hexahydro[1,2,4]triazolo[4,3-a]pyridin-5-carbonsäure (Diastereomerengemisch; 4 Isomere) (100 mg, 281 µmol) wurde in Dichlormethan (1.0 ml) und DMF (2.0 ml) bei Raumtemperatur vorgelegt. Anschließend wurde 1-[Bis(dimethylamino)methylen]-1H-benzotriazol-1-ium-3-oxidhexafluorophosphat (138 mg, 365 µmol) und N,N-Diisopropylethylamin (130 µl, 730 µmol) zugegeben. Nach 15 min Rühren wurde (3S)-3-Fluorpyrrolidinhydrochlorid (42.3 mg, 337 µmol) zugegeben und das Reaktionsgemisch 2 h bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 93.0 mg (78 % d. Th.) der Titelverbindung erhalten.
LC-MS (Methode 3): Rₜ = 1.31 min; MS (ESIpos): m/z = 428 [M+H]⁺
¹H-NMR (400 MHz, DMSO-d6) δ [ppm]: -0.149 (0.54), -0.008 (4.36), 0.008 (4.13), 0.146 (0.54), 1.019 (5.78), 1.027 (14.45), 1.035 (7.38), 1.043 (14.30), 1.303 (0.65), 1.333 (1.76), 1.363 (2.31), 1.392 (1.65), 1.411 (0.81), 1.894 (1.42), 2.092 (1.16), 2.115 (1.14), 2.150 (3.60), 2.181 (2.75), 2.191 (3.83), 2.222 (4.36), 2.250 (2.41), 2.328 (0.92), 2.366 (0.52), 2.645 (2.55), 2.680 (2.36), 2.710 (0.54), 3.340 (0.76), 3.353 (0.71), 3.370 (0.92), 3.381 (0.80), 3.408 (0.50), 3.496 (0.64), 3.505 (0.60), 3.550 (1.42), 3.584 (1.94), 3.611 (1.31), 3.648 (1.20), 3.685 (1.45), 3.743 (0.52), 3.759 (0.86), 3.784 (1.49), 3.823 (1.07), 3.888 (1.62), 3.927 (1.07), 3.951 (1.11), 3.988 (0.54), 4.021 (0.40), 4.576 (0.71), 4.592 (0.89), 4.603 (0.84), 4.618 (0.72), 4.641 (0.87), 4.657 (1.35), 4.668 (1.09), 4.674 (0.99), 4.685 (1.27), 4.702 (0.70), 4.722 (0.84), 4.737 (1.00), 4.749 (1.01), 4.764 (0.85), 4.984 (16.00), 5.268 (0.89), 5.340 (0.62), 5.391 (1.04), 5.474 (0.61), 5.513 (0.60), 7.899 (1.19), 7.918 (15.34), 7.942 (0.74), 8.134 (0.60), 8.664 (7.69). (Mischung von Diastereomeren)

### Beispiel 137

### (5RS,7RS)-5-{[(3S)-3-Fluorpyrrolidin-1 -yl]carbonyl}-7-methyl-2-{[6-(trifluormethyl)pyridin-3-yl]methyl}-5,6,7,8-tetrahydro[1,2,4]triazolo[4,3-a]pyridin-3(2H)-on (Isomer 1)

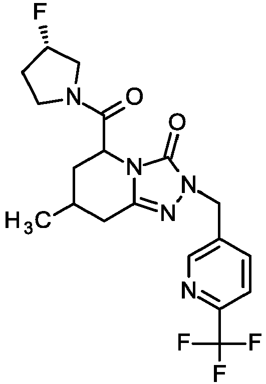

(5RS,7RS)-5-{[(3S)-3-Fluorpyrrolidin-1-yl]carbonyl}-7-methyl-2-{[6-(trifluormethyl)pyridin-3-yl]methyl}-5,6,7,8-tetrahydro[1,2,4]triazolo[4,3-a]pyridin-3(2H)-on (Diastereomerengemisch; 4 Isomere) wurde durch chirale präparative HPLC getrennt [Probenvorbereitung: 87 mg gelöst in 3 ml Ethanol; Injektionsvolumen: 0.1 ml; Säule: Daicel Chiralcel^{®} OX-H 5 µm, 250 × 20 mm; Laufmittel: n-Heptan/Ethanol 50:50; Fluß: 20 ml/min; Temperature 25°C; UV Detektion: 215 nm]. Nach der Trennung, wurden 30.1 mg von Isomer 1, welches zuerst eluierte, und 37.4 mg von Isomer 2, welches später eluierte, isoliert.

### Isomer 1:

Analytische chirale HPLC: Rₜ = 2.07 min, d.e. = 100% [Säule: Daicel Chiralcel^{®} OX-3 50 × 4.6 mm; Laufmittel: n-Heptan/Ethanol 50:50; Fluß: 1 ml/min; UV Detektion: 220 nm].
LC-MS (Methode 4): Rₜ = 0.75 min; MS (ESIpos): m/z = 428 [M+H]⁺
¹H-NMR (500 MHz, DMSO-d6) δ [ppm]: 0.862 (0.42), 1.021 (8.42), 1.030 (11.85), 1.033 (10.67), 1.043 (9.64), 1.236 (0.53), 1.312 (0.68), 1.336 (1.44), 1.363 (2.05), 1.390 (1.65), 1.414 (0.83), 1.896 (1.37), 1.968 (0.43), 1.990 (0.47), 2.055 (0.44), 2.080 (0.57), 2.103 (1.00), 2.116 (0.94), 2.138 (1.19), 2.159 (2.57), 2.184 (2.56), 2.192 (2.90), 2.214 (3.67), 2.240 (2.57), 2.252 (2.56), 2.278 (1.16), 2.648 (2.43), 2.681 (2.11), 3.333 (1.13), 3.347 (1.49), 3.356 (2.24), 3.370 (2.60), 3.379 (2.36), 3.392 (2.36), 3.467 (0.90), 3.474 (0.87), 3.496 (1.25), 3.503 (1.23), 3.558 (2.68), 3.574 (3.46), 3.580 (3.36), 3.607 (2.29), 3.635 (0.61), 3.701 (0.58), 3.708 (0.66), 3.726 (0.80), 3.733 (0.69), 3.778 (0.56), 3.785 (0.59), 3.803 (0.74), 3.810 (0.68), 3.932 (1.39), 3.949 (2.22), 3.967 (1.42), 3.987 (0.98), 4.012 (0.73), 4.665 (1.30), 4.677 (1.47), 4.686 (1.41), 4.698 (1.25), 4.727 (1.56), 4.739 (1.77), 4.748 (1.71), 4.761 (1.51), 4.986 (16.00), 5.289 (1.44), 5.354 (1.14), 5.394 (1.40), 5.460 (1.13), 7.902 (1.50), 7.918 (12.17), 7.940 (0.91), 8.665 (6.94).

### Beispiel 138

### (5RS,7RS)-5-{[(3S)-3-Fluorpyrrolidin-1-yl]carbonyl}-7-methyl-2-{[6-(trifluormethyl)pyridin-3-yl]methyl}-5,6,7,8-tetrahydro[1,2,4]triazolo[4,3-a]pyridin-3(2H)-on (Isomer 2)

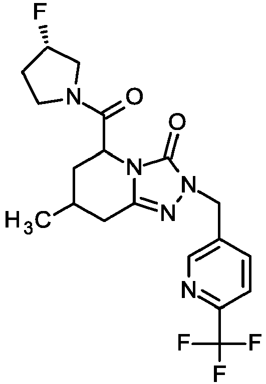

(5RS,7RS)-5-{[(3S)-3-Fluorpyrrolidin-1-yl]carbonyl}-7-methyl-2-{[6-(trifluormethyl)pyridin-3-yl]methyl}-5,6,7,8-tetrahydro[1,2,4]triazolo[4,3-a]pyridin-3(2H)-on (Diastereomerengemisch; 4 Isomere) wurde durch chirale präparative HPLC getrennt [Probenvorbereitung: 87 mg gelöst in 3 ml Ethanol; Injektionsvolumen: 0.1 ml; Säule: Daicel Chiralcel^{®} OX-H 5 µm, 250 × 20 mm; Laufmittel: n-Heptan/Ethanol 50:50; Fluß: 20 ml/min; Temperature 25°C; UV Detektion: 215 nm]. Nach der Trennung, wurden 30.1 mg von Isomer 1, welches zuerst eluierte, und 37.4 mg von Isomer 2, welches später eluierte, isoliert.

### Isomer 2:

Analytische chirale HPLC: Rₜ = 2.43 min, d.e. = 100% [Säule: Daicel Chiralcel^{®} OX-3 50 × 4.6 mm; Laufmittel: n-Heptan/Ethanol 50:50; Fluß: 1 ml/min; UV Detektion: 220 nm].
LC-MS (Methode 4): Rₜ = 0.74 min; MS (ESIpos): m/z = 428 [M+H]⁺
¹H-NMR (500 MHz, DMSO-d6) δ [ppm]: 0.847 (0.50), 0.861 (0.56), 1.028 (15.91), 1.041 (16.00), 1.056 (0.79), 1.088 (0.43), 1.101 (0.58), 1.235 (0.87), 1.314 (0.86), 1.336 (1.78), 1.360 (2.56), 1.384 (1.62), 1.406 (0.75), 1.896 (1.43), 1.908 (1.51), 1.963 (0.52), 1.984 (0.55), 2.048 (0.49), 2.070 (0.63), 2.109 (0.83), 2.122 (1.06), 2.158 (3.53), 2.182 (3.07), 2.190 (3.79), 2.214 (3.63), 2.233 (1.72), 2.259 (1.72), 2.273 (1.57), 2.286 (1.33), 2.300 (1.19), 2.313 (0.87), 2.645 (2.47), 2.649 (2.45), 2.677 (2.20), 2.681 (2.08), 3.273 (1.17), 3.288 (1.58), 3.296 (2.22), 3.311 (2.69), 3.319 (2.69), 3.333 (3.51), 3.347 (3.48), 3.387 (2.00), 3.394 (1.81), 3.415 (1.45), 3.423 (1.35), 3.434 (0.69), 3.448 (0.59), 3.465 (0.88), 3.472 (0.89), 3.493 (1.06), 3.501 (0.98), 3.639 (0.74), 3.653 (1.90), 3.658 (1.96), 3.673 (2.34), 3.682 (1.99), 3.694 (2.41), 3.703 (1.33), 3.714 (1.05), 3.733 (0.91), 3.765 (1.34), 3.782 (2.43), 3.801 (1.78), 3.831 (1.27), 3.855 (0.63), 3.880 (2.40), 4.581 (1.40), 4.594 (1.59), 4.603 (1.53), 4.615 (1.36), 4.646 (1.58), 4.658 (1.80), 4.668 (1.73), 4.680 (1.53), 4.982 (14.44), 5.273 (1.35), 5.379 (1.41), 5.387 (1.38), 5.394 (1.28), 5.500 (1.11), 7.901 (1.80), 7.916 (12.89), 7.940 (0.85), 8.664 (6.94).

### Beispiel 139

### (5RS,6RS)-6-Methyl-2-(4-methylbenzyl)-5-(pyrrolidin-1-ylcarbonyl)-5,6,7,8-tetrahydro[1,2,4]triazolo[4,3-a]pyridin-3(2H)-on (Isomer 2)

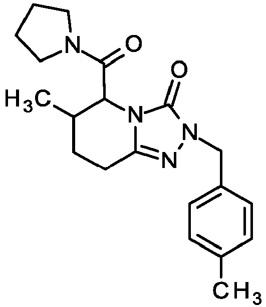

(5RS,6RS)-6-Methyl-2-(4-methylbenzyl)-3-oxo-2,3,5,6,7,8-hexahydro[1,2,4]triazolo[4,3-a]pyridin-5-carbonsäure (Diastereomerengemisch) (95.0 mg, 315 µmol) wurde mit Pyrrolidin (28 µl, 330 µmol) in Pyridin/DMF (5/1) (3.0 ml) bei Raumtemperatur vorgelegt. Anschließend wurde O-(7-Azabenzotriazol-1-yl)-N,N,N',N'-tetramethyluronium-hexafluorphosphat (132 mg, 347 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und direkt einer chiralen präparative HPLC-Trennung unterzogen [Probenvorbereitung: 35 mg gelöst in 3 ml Ethanol; Injektionsvolumen: 0.5 ml; Säule: Daicel Chiralcel^{®} IC 5 µm, 250 × 20 mm; Laufmittel: n-Heptan/Ethanol 40:60; Fluß: 15 ml/min; Temperature 50°C; UV Detektion: 220 nm]. Nach der Trennung wurden 10 mg von Isomer 1, welches als erstes eluierte, und 11 mg von Isomer 2, welches als zweites eluierte, isoliert.

### Isomer 2:

Analytische chirale HPLC: Rₜ = 12.49 min, d.e. = 99% [Säule: Daicel Chiralcel^{®} IC 250 × 4.6 mm; Laufmittel: i-Hexan/Ethanol 40:60; Fluß: 1 ml/min; UV Detektion: 220 nm].
LC-MS (Methode 3): Rₜ = 1.48 min; MS (ESIpos): m/z = 355 [M+H]⁺
¹H-NMR (400 MHz, DMSO-d6) δ [ppm]= 0.99 (d, 3H), 1.49 - 1.62 (m, 1H), 1.70 - 1.84 (m, 2H), 1.84 - 2.10 (m, 3H), 2.20 (dtd, 1H), 2.27 (s, 3H), 2.55 - 2.60 (m, 1H), 2.62 - 2.71 (m, 1H), 3.21 - 3.29 (m, 1H), 3.32 - 3.39 (m, 1H), 3.41 - 3.41 (m, 1H), 3.57 (dt, 1H), 3.77 (dt, 1H), 4.62 - 4.83 (m, 3H), 7.07 - 7.19 (m, 4H).

### Beispiel 140

### (5RS)-5-{[(3S,4S)-3,4-Difluorpyrrolidin-1-yl]carbonyl}-2-(4-methylbenzyl)-5,6,7,8-tetrahydro[1,2,4]triazolo[4,3-a]pyridin-3(2H)-on (Enantiomer 1)

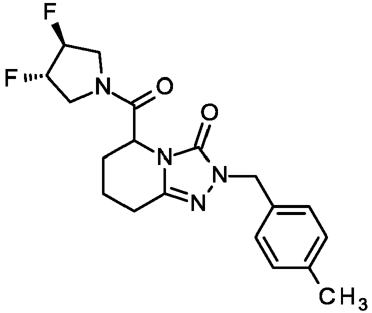

(5RS)-2-(4-Methylbenzyl)-3-oxo-2,3,5,6,7,8-hexahydro[1,2,4]triazolo[4,3-a]pyridin-5-carbonsäure (Enantiomer 1) (75.0 mg, 261 µmol) wurde in DMF (2.0 ml) und Dichlormethan (1.0 ml) bei Raumtemperatur vorgelegt. Anschließend wurde 1-[Bis(dimethylamino)methylen]-1H-benzotriazol-1-ium-3-oxidhexafluorophosphat (129 mg, 339 µmol) und N,N-Diisopropylethylamin (120 µl, 680 µmol) zugegeben. Nach 15 min Rühren wurde (3S,4S)-3,4-Difluorpyrrolidin hydrochlorid (45.0 mg, 313 µmol) zugegeben und das Reaktionsgemisch für 2 h bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 53.7 mg (55 % d. Th.) der Titelverbindung erhalten.
LC-MS (Methode 4): Rₜ = 0.74 min; MS (ESIpos): m/z = 377 [M+H]⁺
¹H-NMR (600 MHz, DMSO-d6) δ [ppm]: 1.624 (0.44), 1.629 (0.48), 1.632 (0.47), 1.637 (0.44), 1.642 (0.54), 1.646 (0.55), 1.651 (0.53), 1.722 (0.68), 1.729 (0.74), 1.738 (0.57), 1.744 (0.43), 1.952 (0.45), 1.957 (0.48), 1.962 (0.44), 1.969 (0.54), 1.976 (0.78), 1.980 (0.81), 1.985 (0.70), 2.022 (0.42), 2.027 (0.51), 2.032 (0.49), 2.037 (0.60), 2.040 (0.52), 2.045 (0.67), 2.051 (0.69), 2.056 (0.65), 2.273 (16.00), 2.518 (0.67), 2.563 (0.77), 2.584 (0.76), 2.592 (1.47), 2.600 (0.80), 2.612 (0.47), 2.620 (0.70), 3.647 (2.34), 3.697 (1.29), 3.706 (0.58), 3.711 (0.90), 3.716 (0.50), 3.830 (0.50), 3.853 (0.57), 3.899 (0.49), 3.922 (0.57), 4.152 (0.84), 4.175 (0.78), 4.195 (0.88), 4.217 (0.74), 4.749 (5.23), 4.752 (5.07), 4.873 (1.32), 4.879 (1.49), 4.883 (1.59), 4.890 (1.27), 5.336 (0.63), 5.346 (0.57), 5.429 (0.99), 5.516 (0.64), 7.109 (1.69), 7.123 (9.34), 7.129 (8.38), 7.143 (1.51).

Die (5S)-Konfiguration wurde aufgrund der Kristallstrukturaufklärung für Beispiel 26, 108, 113, 157, 237, 358 und 454 zugeordnet.

### (5S)-5-{[(3S,4S)-3,4-Difluorpyrrolidin-1 -yl]carbonyl}-2-(4-methylbenzyl)-5,6,7,8-tetrahydro[1,2,4]triazolo[4,3-a]pyridin-3(2H)-on

### Beispiel 141

### (5RS)-2-(4-Methylbenzyl)-5-{[(1RS)-1-oxido-1,3-thiazolidin-3-yl]carbonyl}-5,6,7,8-tetrahydro[1,2,4]triazolo[4,3-a]pyridin-3(2H)-on (Diastereomerengemisch; 2 Isomere)

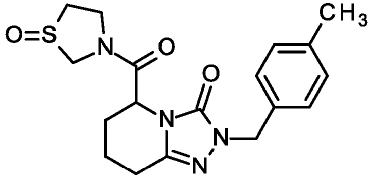

(5RS)-2-(4-Methylbenzyl)-5-(1,3-thiazolidin-3-ylcarbonyl)-5,6,7,8-tetrahydro[1,2,4]triazolo[4,3-a]pyridin-3(2H)-on (Enantiomer 1) (80.0 mg, 223 µmol) wurde in Dichlormethan (3.0 ml) vorgelegt und mit 3-Chlorbenzolcarboperoxosäure (55.0 mg, 70 % Reinheit, 223 µmol) versetzt. Nach 4 h bei Raumtemperatur wurde mit gesättigter wässriger Natriumhydrogencarbonat-Lösung versetzt und mit Dichlormethan extrahiert. Die vereinigten organischen Phasen wurden über Magnesiumsulfat getrocknet, filtriert und das Filtrat eingeengt. Der Rückstand wurde über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 40.0 mg (48 % d. Th.) der Titelverbindung erhalten.
LC-MS (Methode 1): Rₜ = 0.85 min; MS (ESIpos): m/z = 375 [M+H]⁺
¹H-NMR (500 MHz, DMSO-d6) δ [ppm]: 1.627 (0.41), 1.740 (0.62), 1.762 (0.45), 1.987 (0.63), 1.995 (0.76), 2.004 (0.69), 2.022 (0.56), 2.084 (0.42), 2.274 (16.00), 2.514 (0.79), 2.518 (0.79), 2.521 (0.60), 2.568 (0.47), 2.591 (0.73), 2.599 (1.07), 2.634 (0.57), 3.058 (0.50), 3.077 (0.45), 3.097 (0.45), 3.125 (0.46), 3.175 (0.50), 3.262 (0.59), 3.274 (0.75), 3.966 (0.48), 3.974 (0.70), 3.987 (0.64), 3.994 (0.60), 4.088 (0.42), 4.109 (0.64), 4.121 (0.44), 4.287 (0.64), 4.308 (0.49), 4.313 (0.64), 4.342 (0.74), 4.368 (0.88), 4.421 (0.66), 4.446 (0.72), 4.489 (0.59), 4.513 (0.64), 4.610 (0.76), 4.614 (0.82), 4.636 (0.60), 4.640 (0.66), 4.755 (5.93), 4.859 (0.41), 4.864 (0.49), 4.983 (0.64), 4.995 (0.55), 5.002 (0.52), 5.008 (0.55), 5.016 (0.87), 5.020 (0.92), 5.045 (0.78), 5.753 (0.47), 7.105 (0.99), 7.122 (5.96), 7.130 (11.47), 7.145 (0.98). (Mischung von Diastereomeren)

Die (5S)-Konfiguration wurde aufgrund der Kristallstrukturaufklärung für Beispiel 26, 108, 113, 157, 237, 358 und 454 zugeordnet.

### (5S)-2-(4-Methylbenzyl)-5-{[(1RS)-1-oxido-1,3-thiazolidin-3-yl]carbonyl}-5,6,7,8-tetrahydro[1,2,4]triazolo[4,3-a]pyridin-3(2H)-on (Diastereomerengemisch; 2 Isomere)

### Beispiel 142

### (5RS)-5-[(1,1-Dioxido-1,3-thiazolidin-3-yl)carbonyl]-2-(4-methylbenzyl)-5,6,7,8-tetrahydro[1,2,4]triazolo[4,3-a]pyridin-3(2H)-on (Enantiomer 1)

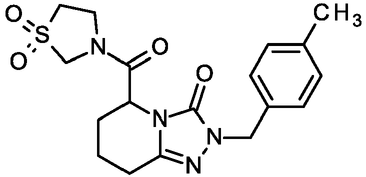

(5RS)-2-(4-Methylbenzyl)-5-(1,3-thiazolidin-3-ylcarbonyl)-5,6,7,8-tetrahydro[1,2,4]triazolo[4,3-a]pyridin-3(2H)-on (Enantiomer 1) (32.0 mg, 89.3 µmol) wurde in Dichlormethan (2.4 ml) vorgelegt und mit 3-Chlorbenzolcarboperoxosäure (77.0 mg, 70 % Reinheit, 312 µmol) versetzt. Nach 4 h bei Raumtemperatur wurde mit gesättigter wässriger Natriumhydrogencarbonat-Lösung versetzt und mit Dichlormethan extrahiert. Die vereinigten organischen Phasen wurden über Magnesiumsulfat getrocknet, filtriert und das Filtrat eingeengt. Der Rückstand wurde über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 25.5 mg (73 % d. Th.) der Titelverbindung erhalten.
LC-MS (Methode 4): Rₜ = 0.69 min; MS (ESIpos): m/z = 391 [M+H]⁺
¹H-NMR (500 MHz, DMSO-d6) δ [ppm]: 1.583 (0.40), 1.730 (0.58), 1.741 (0.60), 1.962 (0.42), 1.990 (0.89), 1.996 (0.83), 2.015 (0.42), 2.272 (16.00), 2.514 (0.74), 2.518 (0.59), 2.521 (0.48), 2.558 (0.75), 2.569 (0.62), 2.595 (1.12), 2.628 (0.49), 3.423 (0.53), 3.439 (0.95), 3.449 (0.62), 3.455 (0.75), 3.461 (0.76), 3.476 (0.70), 3.580 (0.60), 3.593 (1.14), 3.607 (0.63), 3.850 (0.41), 3.859 (0.62), 3.874 (1.06), 3.884 (0.72), 3.889 (0.69), 3.896 (0.71), 3.901 (0.67), 3.912 (0.60), 4.158 (0.41), 4.247 (0.41), 4.470 (0.46), 4.495 (0.88), 4.544 (0.89), 4.569 (0.47), 4.755 (8.51), 4.844 (1.10), 4.868 (2.16), 4.874 (0.98), 4.880 (0.97), 4.886 (0.71), 4.982 (1.43), 5.006 (1.07), 5.020 (0.41), 5.027 (0.47), 5.032 (0.47), 5.752 (4.50), 7.105 (1.46), 7.122 (9.73), 7.127 (9.02), 7.144 (1.29).

Die (5S)-Konfiguration wurde aufgrund der Kristallstrukturaufklärung für Beispiel 26, 108, 113, 157, 237, 358 und 454 zugeordnet.

### (5S)-5-[(1,1-Dioxido-1,3-thiazolidin-3-yl)carbonyl]-2-(4-methylbenzyl)-5,6,7,8-tetrahydro[1,2,4]triazolo[4,3-a]pyridin-3(2H)-on

### Beispiel 143

### (5RS)-5-{[(3S)-3-(Difluormethyl)pyrrolidin-1 -yl]carbonyl}-2-(4-methylbenzyl)-5,6,7,8-tetrahydro[1,2,4]triazolo[4,3-a]pyridin-3(2H)-on (Enantiomer 1)

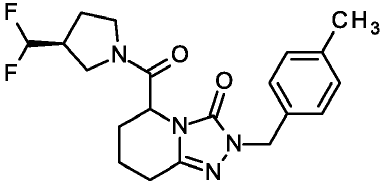

(5RS)-2-(4-Methylbenzyl)-3-oxo-2,3,5,6,7,8-hexahydro[1,2,4]triazolo[4,3-a]pyridin-5-carbonsäure (Enantiomer 1) (100 mg, 348 µmol) wurde in DMF (4.0 ml) und Dichlormethan (2.0 ml) bei Raumtemperatur vorgelegt. Anschließend wurde 1-[Bis(dimethylamino)methylen]-1H-benzotriazol-1-ium-3-oxidhexafluorophosphat (172 mg, 452 µmol) und N,N-Diisopropylethylamin (160 µl, 900 µmol) zugegeben. Nach 15 min Rühren wurde (3S)-3-(Difluormethyl)pyrrolidinhydrochlorid (65.8 mg, 418 µmol) zugegeben und das Reaktionsgemisch für 2 h bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 78.6 mg (58 % d. Th.) der Titelverbindung erhalten.
LC-MS (Methode 3): Rₜ = 1.47 min; MS (ESIpos): m/z = 391 [M+H]⁺
¹H-NMR (500 MHz, DMSO-d6) δ [ppm]: 1.665 (0.43), 1.676 (0.55), 1.709 (1.13), 1.829 (0.41), 1.934 (0.46), 1.943 (0.53), 1.950 (0.75), 1.959 (0.76), 1.984 (0.90), 1.994 (0.95), 2.005 (0.70), 2.013 (0.90), 2.026 (0.70), 2.034 (0.69), 2.041 (0.55), 2.046 (0.49), 2.053 (0.41), 2.115 (0.44), 2.271 (16.00), 2.517 (1.18), 2.574 (0.72), 2.583 (1.40), 2.593 (0.75), 2.617 (0.64), 3.270 (0.49), 3.286 (0.47), 3.295 (0.60), 3.341 (0.43), 3.437 (0.44), 3.454 (0.41), 3.479 (0.49), 3.516 (0.79), 3.533 (0.62), 3.542 (0.59), 3.610 (0.48), 3.647 (0.44), 4.707 (0.48), 4.715 (0.44), 4.722 (0.57), 4.728 (0.96), 4.738 (3.89), 4.745 (3.45), 4.760 (0.44), 4.775 (0.87), 4.780 (0.80), 4.787 (0.40), 6.130 (0.51), 6.139 (0.54), 6.162 (0.52), 7.104 (1.25), 7.120 (10.70), 7.125 (10.23), 7.141 (1.15).

Die (5S)-Konfiguration wurde aufgrund der Kristallstrukturaufklärung für Beispiel 26, 108, 113, 157, 237, 358 und 454 zugeordnet.

### (5S)-5-{[(3S)-3-(Difluormethyl)pyrrolidin-1 -yl]carbonyl}-2-(4-methylbenzyl)-5,6,7,8-tetrahydro[1,2,4]triazolo[4,3-a]pyridin-3(2H)-on

### Beispiel 144

### (5RS)-5-[(cis)-2-Azabicyclo[3.1.0]hex-2-ylcarbonyl]-2-(4-methylbenzyl)-5,6,7,8-tetrahydro[1,2,4]triazolo[4,3-a]pyridin-3(2H)-on (Diastereomerengemisch; 2 Isomere)

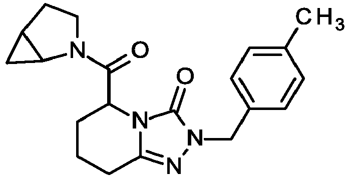

(5RS)-2-(4-Methylbenzyl)-3-oxo-2,3,5,6,7,8-hexahydro[1,2,4]triazolo[4,3-a]pyridin-5-carbonsäure (Enantiomer 1) (100 mg, 348 µmol) wurde in DMF (4.0 ml) und Dichlormethan (2.0 ml) bei Raumtemperatur vorgelegt. Anschließend wurde 1-[Bis(dimethylamino)methylen]-1H-benzotriazol-1-ium-3-oxidhexafluorophosphat (172 mg, 452 µmol) und N,N-Diisopropylethylamin (160 µl, 900 µmol) zugegeben. Nach 15 min Rühren wurde cis-2-Azabicyclo[3.1.0]hexanhydrochlorid (Racemat) (49.9 mg, 418 µmol) zugegeben und das Reaktionsgemisch für 2 h bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 92.9 mg (76 % d. Th.) der Titelverbindung erhalten.
LC-MS (Methode 3): Rₜ = 1.41 min; MS (ESIpos): m/z = 353 [M+H]⁺
¹H-NMR (500 MHz, DMSO-d6) δ [ppm]: 0.618 (0.56), 0.623 (0.56), 0.628 (0.79), 0.633 (0.69), 0.639 (0.64), 0.644 (0.61), 0.650 (0.49), 0.654 (0.45), 0.811 (0.53), 0.828 (0.52), 1.567 (0.43), 1.744 (0.75), 1.755 (0.95), 1.767 (1.00), 1.772 (0.92), 1.784 (0.62), 1.860 (0.57), 1.866 (0.43), 1.877 (0.51), 1.884 (0.64), 1.890 (0.41), 1.902 (0.42), 1.908 (0.42), 2.001 (0.55), 2.012 (0.48), 2.019 (0.70), 2.032 (0.79), 2.039 (0.78), 2.047 (0.76), 2.065 (0.53), 2.078 (0.41), 2.145 (0.74), 2.150 (0.68), 2.272 (15.30), 2.518 (0.58), 2.522 (0.77), 2.586 (1.07), 2.596 (0.62), 2.620 (0.47), 2.959 (0.55), 2.984 (0.56), 3.154 (0.42), 3.495 (0.42), 3.499 (0.40), 3.699 (0.42), 3.704 (0.67), 3.711 (0.88), 3.716 (0.65), 3.724 (0.71), 3.729 (0.78), 4.738 (1.92), 4.743 (2.32), 4.753 (3.33), 4.934 (0.45), 4.940 (0.46), 5.070 (0.56), 5.076 (0.65), 5.083 (0.61), 5.089 (0.52), 7.109 (0.79), 7.127 (16.00), 7.144 (0.66).

Die (5S)-Konfiguration wurde aufgrund der Kristallstrukturaufklärung für Beispiel 26, 108, 113, 157, 237, 358 und 454 zugeordnet.

### (5S)-5-[(cis)-2-Azabicyclo[3.1.0]hex-2-ylcarbonyl]-2-(4-methylbenzyl)-5,6,7,8-tetrahydro[1,2,4]triazolo[4,3-a]pyridin-3(2H)-on (Diastereomerengemisch; 2 Isomere)

### Beispiel 145

### (5RS)-2-(4-Methylbenzyl)-5-[(3,3,4,4-tetrafluorpyrrolidin-1-yl)carbonyl]-5,6,7,8-tetrahydro[1,2,4]triazolo[4,3-a]pyridin-3(2H)-on (Enantiomer 1)

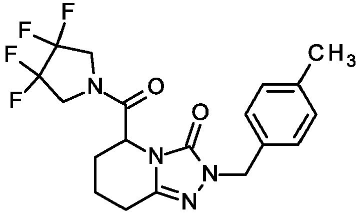

(5RS)-2-(4-Methylbenzyl)-3-oxo-2,3,5,6,7,8-hexahydro[1,2,4]triazolo[4,3-a]pyridin-5-carbonsäure (Enantiomer 1) (50.0 mg, 174 µmol) wurde in DMF (4.0 ml) und Dichlormethan (2.0 ml) bei Raumtemperatur vorgelegt. Anschließend wurde 1-[Bis(dimethylamino)methylen]-1H-benzotriazol-1-ium-3-oxidhexafluorophosphat (85.8 mg, 226 µmol) und N,N-Diisopropylethylamin (79 µl, 450 µmol) zugegeben. Nach 15 min Rühren wurde 3,3,4,4-Tetrafluorpyrrolidinhydrochlorid (37.5 mg, 209 µmol) zugegeben und das Reaktionsgemisch für 2 h bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 11.0 mg (15 % d. Th.) der Titelverbindung erhalten.
LC-MS (Methode 3): Rₜ = 1.68 min; MS (ESIpos): m/z = 413 [M+H]⁺
¹H-NMR (500 MHz, DMSO-d6) δ [ppm]: -0.007 (0.56), 1.141 (2.17), 1.236 (0.42), 1.658 (0.47), 1.665 (0.57), 1.678 (0.56), 1.686 (0.71), 1.697 (0.83), 1.704 (0.92), 1.716 (0.72), 1.726 (0.43), 1.952 (0.43), 1.958 (0.45), 1.964 (0.44), 1.972 (0.62), 1.980 (0.69), 1.986 (0.74), 1.993 (0.62), 2.048 (0.50), 2.054 (0.48), 2.061 (0.69), 2.069 (0.65), 2.076 (0.65), 2.082 (0.64), 2.090 (0.45), 2.116 (0.98), 2.271 (16.00), 2.481 (0.93), 2.514 (0.53), 2.518 (0.54), 2.562 (1.05), 2.568 (1.04), 2.580 (1.52), 2.591 (1.69), 2.601 (0.83), 2.624 (0.53), 4.007 (0.64), 4.037 (0.76), 4.105 (0.74), 4.132 (0.59), 4.411 (0.54), 4.440 (0.52), 4.620 (0.73), 4.646 (0.65), 4.716 (0.43), 4.747 (4.79), 4.752 (4.59), 4.783 (0.41), 4.830 (1.29), 4.838 (1.55), 4.842 (1.66), 4.851 (1.23), 7.104 (1.48), 7.109 (0.84), 7.116 (1.66), 7.121 (10.49), 7.126 (9.73), 7.143 (1.20).

Die (5S)-Konfiguration wurde aufgrund der Kristallstrukturaufklärung für Beispiel 26, 108, 113, 157, 237, 358 und 454 zugeordnet.

### (5S)-2-(4-Methylbenzyl)-5-[(3,3,4,4-tetrafluorpyrrolidin-1-yl)carbonyl]-5,6,7,8-tetrahydro[1,2,4]triazolo[4,3-a]pyridin-3(2H)-on

### Beispiel 146

### (5RS)-5-{[3-(Fluormethyl)azetidin-1-yl]carbonyl}-2-(4-methylbenzyl)-5,6,7,8-tetrahydro[1,2,4]triazolo[4,3-a]pyridin-3(2H)-on (Enantiomer 1)

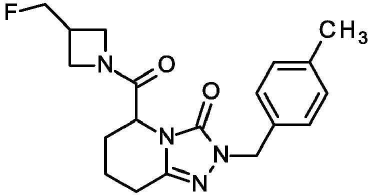

(5RS)-2-(4-Methylbenzyl)-3-oxo-2,3,5,6,7,8-hexahydro[1,2,4]triazolo[4,3-a]pyridin-5-carbonsäure (Enantiomer 1) (50.0 mg, 174 µmol) wurde in DMF (2.0 ml) und Dichlormethan (1.0 ml) bei Raumtemperatur vorgelegt. Anschließend wurde 1-[Bis(dimethylamino)methylen]-1H-benzotriazol-1-ium-3-oxidhexafluorophosphat (85.8 mg, 226 µmol) und N,N-Diisopropylethylamin (79 µl, 450 µmol) zugegeben. Nach 15 min Rühren wurde 3-(Fluoromethyl)azetidintrifluoroacetat (42.4 mg, 209 µmol) zugegeben und das Reaktionsgemisch für 2 h bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 23.2 mg (37 % d. Th.) der Titelverbindung erhalten.
LC-MS (Methode 3): Rₜ = 1.31 min; MS (ESIpos): m/z = 359 [M+H]⁺
¹H-NMR (500 MHz, DMSO-d6) δ [ppm]: -0.007 (0.41), 1.683 (0.67), 1.694 (1.11), 1.705 (1.36), 1.715 (1.05), 1.725 (0.44), 1.911 (0.49), 1.917 (0.52), 1.923 (0.43), 1.986 (0.51), 1.993 (0.63), 2.001 (0.62), 2.005 (0.47), 2.014 (0.63), 2.270 (16.00), 2.514 (0.90), 2.518 (0.75), 2.521 (0.42), 2.526 (0.57), 2.563 (0.66), 2.573 (1.35), 2.584 (0.65), 2.607 (0.56), 3.639 (0.50), 3.650 (0.50), 3.659 (0.56), 3.670 (0.52), 3.704 (0.47), 3.716 (0.48), 3.724 (0.56), 3.735 (0.52), 3.921 (0.43), 3.939 (0.70), 3.963 (0.59), 3.974 (0.57), 3.980 (0.62), 3.991 (0.87), 4.007 (0.80), 4.110 (0.43), 4.122 (0.48), 4.128 (0.56), 4.139 (0.50), 4.252 (0.44), 4.269 (0.79), 4.399 (0.45), 4.416 (0.86), 4.488 (0.61), 4.497 (1.30), 4.500 (1.08), 4.505 (1.00), 4.509 (1.33), 4.518 (1.67), 4.530 (1.10), 4.545 (1.22), 4.556 (1.11), 4.613 (1.09), 4.624 (1.10), 4.639 (1.18), 4.651 (1.12), 4.744 (6.33), 7.107 (0.78), 7.125 (13.27), 7.137 (0.43), 7.142 (0.70).

Die (5S)-Konfiguration wurde aufgrund der Kristallstrukturaufklärung für Beispiel 26, 108, 113, 157, 237, 358 und 454 zugeordnet.

### (5S)-5-{[3-(Fluormethyl)azetidin-1-yl]carbonyl}-2-(4-methylbenzyl)-5,6,7,8-tetrahydro[1,2,4]triazolo[4,3-a]pyridin-3(2H)-on

### Beispiel 147

### (5RS)-5-{[(3RS)-3-Fluor-3-(hydroxymethyl)pyrrolidin-1-yl]carbonyl}-2-(4-methylbenzyl)-5,6,7,8-tetrahydro[1,2,4]triazolo[4,3-a]pyridin-3(2H)-on (Diastereomerengemisch; 2 Isomere)

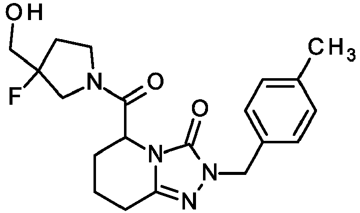

(5RS)-2-(4-Methylbenzyl)-3-oxo-2,3,5,6,7,8-hexahydro[1,2,4]triazolo[4,3-a]pyridin-5-carbonsäure (Enantiomer 1) (100 mg, 348 µmol) wurde in DMF (4.0 ml) und Dichlormethan (2.0 ml) bei Raumtemperatur vorgelegt. Anschließend wurde 1-[Bis(dimethylamino)methylen]-1H-benzotriazol-1-ium-3-oxidhexafluorophosphat (172 mg, 452 µmol) und N,N-Diisopropylethylamin (160 µl, 900 µmol) zugegeben. Nach 15 min Rühren wurde [(3RS)-3-Fluorpyrrolidin-3-yl]methanolhydrochlorid (Racemat) (65.0 mg, 418 µmol) zugegeben und das Reaktionsgemisch für 2 h bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 62.8 mg (46 % d. Th.) der Titelverbindung erhalten.
LC-MS (Methode 3): Rₜ = 1.17 min; MS (ESIpos): m/z = 389 [M+H]⁺
¹H-NMR (500 MHz, DMSO-d6) δ [ppm]: -0.007 (0.53), 1.670 (0.41), 1.676 (0.41), 1.685 (0.45), 1.696 (0.56), 1.705 (0.77), 1.716 (1.02), 1.723 (1.05), 1.733 (0.76), 1.973 (0.72), 1.994 (0.76), 2.010 (0.86), 2.023 (0.60), 2.040 (0.73), 2.052 (0.91), 2.064 (0.73), 2.073 (0.74), 2.121 (0.50), 2.132 (0.55), 2.136 (0.56), 2.272 (16.00), 2.514 (0.86), 2.518 (0.81), 2.521 (0.89), 2.577 (0.67), 2.586 (1.31), 2.595 (0.76), 2.619 (0.58), 3.367 (0.42), 3.395 (0.47), 3.437 (0.52), 3.464 (0.64), 3.488 (0.64), 3.516 (0.43), 3.556 (0.83), 3.569 (0.40), 3.584 (0.63), 3.600 (0.63), 3.620 (1.19), 3.656 (1.64), 3.675 (0.69), 3.681 (0.94), 3.697 (0.57), 3.703 (0.49), 3.724 (0.53), 3.737 (0.53), 3.750 (0.57), 3.759 (0.61), 3.773 (0.41), 4.656 (0.40), 4.705 (0.49), 4.725 (0.57), 4.736 (3.09), 4.744 (4.56), 4.753 (1.96), 4.760 (0.52), 4.766 (0.52), 4.773 (0.46), 4.832 (0.44), 4.837 (0.47), 5.241 (0.50), 7.103 (0.89), 7.108 (1.04), 7.120 (7.69), 7.125 (11.68), 7.142 (0.91).

Die (5S)-Konfiguration wurde aufgrund der Kristallstrukturaufklärung für Beispiel 26, 108, 113, 157, 237, 358 und 454 zugeordnet.

### (5S)-5-{[(3RS)-3-Fluor-3-(hydroxymethyl)pyrrolidin-1-yl]carbonyl}-2-(4-methylbenzyl)-5,6,7,8-tetrahydro[1,2,4]triazolo[4,3-a]pyridin-3(2H)-on (Diastereomerengemisch; 2 Isomere)

### Beispiel 148

### (5RS)-5-{[(2S)-2-Methylazetidin-1-yl]carbonyl}-2-(4-methylbenzyl)-5,6,7,8-tetrahydro[1,2,4]triazolo[4,3-a]pyridin-3(2H)-on (Enantiomer 1)

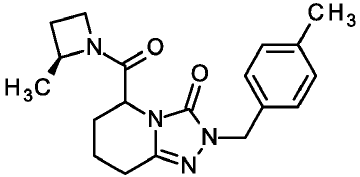

(5RS)-2-(4-Methylbenzyl)-3-oxo-2,3,5,6,7,8-hexahydro[1,2,4]triazolo[4,3-a]pyridin-5-carbonsäure (Enantiomer 1) (100 mg, 348 µmol) wurde in DMF (4.0 m) und Dichlormethan (2.0 ml) bei Raumtemperatur vorgelegt. Anschließend wurde 1-[Bis(dimethylamino)methylen]-1H-benzotriazol-1-ium-3-oxidhexafluorophosphat (172 mg, 452 µmol) und N,N-Diisopropylethylamin (160 µl, 900 µmol) zugegeben. Nach 15 min Rühren wurde (2S)-2-Methylazetidinhydrochlorid (44.9 mg, 418 µmol) zugegeben und das Reaktionsgemisch für 2 h bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 70.3 mg (59 % d. Th.) der Titelverbindung erhalten.
LC-MS (Methode 3): Rₜ = 1.36 min; MS (ESIpos): m/z = 341 [M+H]⁺
¹H-NMR (500 MHz, DMSO-d6) δ [ppm]: 1.314 (3.24), 1.326 (3.25), 1.349 (1.64), 1.361 (1.62), 1.450 (1.44), 1.462 (1.41), 1.520 (1.03), 1.533 (1.02), 1.706 (1.29), 1.797 (0.63), 1.803 (0.73), 1.815 (0.86), 1.819 (0.85), 1.826 (0.82), 1.838 (0.72), 1.849 (0.51), 1.917 (0.49), 1.924 (0.51), 1.933 (0.42), 1.987 (0.52), 2.001 (0.52), 2.008 (0.53), 2.017 (0.46), 2.270 (16.00), 2.395 (0.54), 2.401 (0.60), 2.413 (0.61), 2.418 (0.61), 2.434 (0.49), 2.517 (0.78), 2.522 (0.97), 2.557 (0.73), 2.567 (1.38), 2.576 (0.76), 2.601 (0.57), 4.120 (0.46), 4.132 (0.46), 4.207 (0.60), 4.220 (0.64), 4.238 (0.42), 4.329 (0.43), 4.340 (0.59), 4.357 (0.58), 4.370 (0.61), 4.423 (0.90), 4.431 (1.03), 4.435 (1.11), 4.443 (0.76), 4.681 (0.41), 4.697 (0.42), 4.712 (0.94), 4.728 (1.99), 4.743 (3.47), 4.756 (0.78), 4.776 (0.69), 7.107 (0.87), 7.126 (13.90), 7.142 (0.72).

Die (5S)-Konfiguration wurde aufgrund der Kristallstrukturaufklärung für Beispiel 26, 108, 113, 157, 237, 358 und 454 zugeordnet.

### (5S)-5-{[(2S)-2-Methylazetidin-1-yl]carbonyl}-2-(4-methylbenzyl)-5,6,7,8-tetrahydro[1,2,4]triazolo[4,3-a]pyridin-3(2H)-on

### Beispiel 149

### (5RS)-5-{[3-(Difluormethyl)azetidin-1-yl]carbonyl}-2-(4-methylbenzyl)-5,6,7,8-tetrahydro[1,2,4]triazolo[4,3-a]pyridin-3(2H)-on (Enantiomer 1)

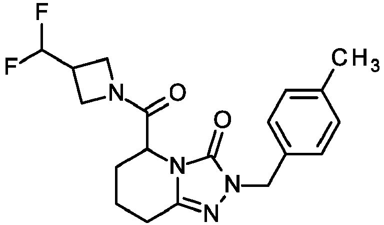

(5RS)-2-(4-Methylbenzyl)-3-oxo-2,3,5,6,7,8-hexahydro[1,2,4]triazolo[4,3-a]pyridin-5-carbonsäure (Enantiomer 1) (50.0 mg, 174 µmol) wurde in DMF (4.0 ml) und Dichlormethan (2.0 ml) bei Raumtemperatur vorgelegt. Anschließend wurde 1-[Bis(dimethylamino)methylen]-1H-benzotriazol-1-ium-3-oxidhexafluorophosphat (85.8 mg, 226 µmol) und N,N-Diisopropylethylamin (79 µl, 450 µmol) zugegeben. Nach 15 min Rühren wurde 3-(Difluormethyl)azetidinhydrochlorid (30.0 mg, 209 µmol) zugegeben und das Reaktionsgemisch für 2 h bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 32.3 mg (48 % d. Th.) der Titelverbindung erhalten.
LC-MS (Methode 4): Rₜ = 0.76 min; MS (ESIpos): m/z = 377 [M+H]⁺
¹H-NMR (500 MHz, DMSO-d6) δ [ppm]: 1.676 (0.59), 1.696 (1.28), 1.705 (1.35), 1.714 (1.08), 1.912 (0.46), 1.949 (0.44), 1.984 (0.48), 1.996 (0.65), 2.004 (0.71), 2.012 (0.62), 2.016 (0.60), 2.024 (0.53), 2.270 (16.00), 2.519 (1.23), 2.568 (0.69), 2.577 (1.28), 2.586 (0.71), 2.610 (0.51), 2.689 (1.41), 2.889 (0.42), 3.178 (0.43), 3.186 (0.44), 3.196 (0.46), 3.204 (0.40), 3.775 (0.53), 3.786 (0.55), 3.796 (0.64), 3.806 (0.59), 3.846 (0.43), 3.858 (0.47), 3.866 (0.64), 3.878 (0.59), 3.941 (0.56), 3.959 (0.84), 4.017 (0.59), 4.036 (0.95), 4.055 (0.48), 4.111 (0.55), 4.122 (0.60), 4.129 (0.64), 4.139 (0.56), 4.272 (1.16), 4.275 (1.15), 4.284 (0.93), 4.293 (0.92), 4.434 (0.55), 4.452 (1.03), 4.470 (0.47), 4.518 (1.08), 4.528 (1.66), 4.538 (1.06), 4.746 (7.75), 6.314 (0.51), 6.322 (0.52), 6.352 (0.57), 6.361 (0.56), 7.106 (1.00), 7.124 (14.14), 7.141 (0.85).

Die (5S)-Konfiguration wurde aufgrund der Kristallstrukturaufklärung für Beispiel 26, 108, 113, 157, 237, 358 und 454 zugeordnet.

### (5S)-5-{[3-(Difluormethyl)azetidin-1-yl]carbonyl}-2-(4-methylbenzyl)-5,6,7,8-tetrahydro[1,2,4]triazolo[4,3-a]pyridin-3(2H)-on

### Beispiel 150

### (5RS)-5-{[cis-3,4-Difluorpyrrolidin-1-yl]carbonyl}-2-(4-methylbenzyl)-5,6,7,8-tetrahydro[1,2,4]triazolo[4,3-a]pyridin-3(2H)-on (Enantiomer 1)

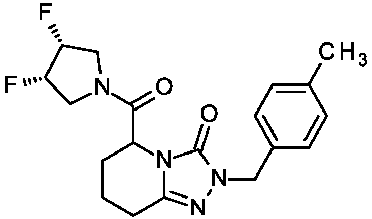

(5RS)-2-(4-Methylbenzyl)-3-oxo-2,3,5,6,7,8-hexahydro[1,2,4]triazolo[4,3-a]pyridin-5-carbonsäure (Enantiomer 1) (100 mg, 348 µmol) wurde in DMF (8.0 ml) und Dichlormethan (4.0 ml) bei Raumtemperatur vorgelegt. Anschließend wurde 1-[Bis(dimethylamino)methylen]-1H-benzotriazol-1-ium-3-oxidhexafluorophosphat (172 mg, 452 µmol) und N,N-Diisopropylethylamin (160 µl, 900 µmol) zugegeben. Nach 15 min Rühren wurde cis-3,4-Difluorpyrrolidinhydrochlorid (60.0 mg, 418 µmol) zugegeben und das Reaktionsgemisch für 2 h bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 97.6 mg (75 % d. Th.) der Titelverbindung erhalten.
LC-MS (Methode 1): Rₜ = 1.03 min; MS (ESIpos): m/z = 377 [M+H]⁺
¹H-NMR (400 MHz, DMSO-d6) δ [ppm]: 0.008 (0.52), 1.636 (0.49), 1.643 (0.54), 1.668 (0.75), 1.678 (0.74), 1.695 (0.84), 1.707 (1.04), 1.717 (0.98), 1.730 (0.79), 1.949 (0.69), 1.957 (0.59), 1.974 (0.44), 1.996 (0.88), 2.005 (0.65), 2.022 (0.54), 2.031 (0.62), 2.039 (0.67), 2.047 (0.64), 2.056 (0.56), 2.066 (0.49), 2.074 (0.53), 2.082 (0.46), 2.272 (16.00), 2.522 (1.21), 2.558 (0.95), 2.571 (1.24), 2.585 (1.60), 2.597 (0.90), 2.628 (0.53), 3.485 (0.56), 3.493 (0.47), 3.506 (0.41), 3.514 (0.43), 3.523 (0.49), 3.533 (0.69), 3.541 (0.64), 3.575 (0.43), 3.612 (0.45), 3.626 (0.52), 3.668 (0.51), 3.681 (0.72), 3.688 (0.61), 3.701 (0.94), 3.714 (0.56), 3.724 (0.66), 3.733 (0.53), 3.753 (0.60), 3.766 (0.54), 3.865 (0.45), 3.924 (0.44), 3.938 (0.50), 3.973 (0.48), 3.987 (0.48), 4.172 (0.59), 4.703 (0.41), 4.743 (7.59), 4.750 (3.74), 4.768 (1.29), 4.779 (1.99), 4.789 (1.52), 5.254 (0.49), 5.265 (0.49), 5.275 (0.55), 5.284 (0.44), 5.338 (0.40), 5.349 (0.42), 5.375 (0.53), 5.387 (0.60), 5.398 (0.49), 5.407 (0.49), 5.415 (0.44), 5.457 (0.42), 5.470 (0.40), 5.479 (0.42), 7.096 (0.75), 7.102 (0.98), 7.117 (6.91), 7.123 (13.48), 7.144 (1.02).

Die (5S)-Konfiguration wurde aufgrund der Kristallstrukturaufklärung für Beispiel 26, 108, 113, 157, 237, 358 und 454 zugeordnet.

### (5S)-5-{[cis-3,4-Difluorpyrrolidin-1-yl]carbonyl}-2-(4-methylbenzyl)-5,6,7,8-tetrahydro[1,2,4]triazolo[4,3-a]pyridin-3(2H)-on

### Beispiel 151

### (5RS)-5-{[(3RS)-3-Fluor-3-methylpyrrolidin-1 -yl]carbonyl}-2-(4-methylbenzyl)-5,6,7,8-tetrahydro[1,2,4]triazolo[4,3-a]pyridin-3(2H)-on (Diastereomerengemisch; 2 Isomere)

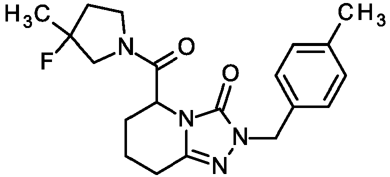

(5RS)-2-(4-Methylbenzyl)-3-oxo-2,3,5,6,7,8-hexahydro[1,2,4]triazolo[4,3-a]pyridin-5-carbonsäure (Enantiomer 1) (100 mg, 348 µmol) wurde in DMF (8.0 ml) und Dichlormethan (4.0 ml) bei Raumtemperatur vorgelegt. Anschließend wurde 1-[Bis(dimethylamino)methylen]-1H-benzotriazol-1-ium-3-oxidhexafluorophosphat (172 mg, 452 µmol) und N,N-Diisopropylethylamin (160 µl, 900 µmol) zugegeben. Nach 15 min Rühren wurde (3RS)-3-Fluor-3-methylpyrrolidinhydrochlorid (Racemat) (58.3 mg, 418 µmol) zugegeben und das Reaktionsgemisch für 2 h bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 97.9 mg (75 % d. Th.) der Titelverbindung erhalten.
LC-MS (Methode 1): Rₜ = 1.06 min; MS (ESIpos): m/z = 373 [M+H]⁺
¹H-NMR (400 MHz, DMSO-d6) δ [ppm]: -0.008 (0.81), 0.008 (0.67), 1.487 (2.19), 1.496 (2.37), 1.505 (2.23), 1.521 (2.02), 1.539 (2.25), 1.548 (2.39), 1.557 (2.22), 1.573 (1.99), 1.717 (1.18), 1.971 (0.64), 1.983 (0.87), 1.994 (0.92), 2.041 (0.65), 2.065 (0.84), 2.102 (0.67), 2.199 (0.44), 2.222 (0.52), 2.272 (16.00), 2.558 (0.72), 2.583 (1.28), 2.625 (0.46), 3.335 (0.59), 3.354 (0.54), 3.370 (0.45), 3.525 (0.49), 3.552 (0.51), 3.572 (0.69), 3.613 (0.67), 3.631 (0.55), 3.643 (0.63), 3.660 (0.57), 3.679 (0.57), 3.729 (0.64), 3.750 (0.40), 3.951 (0.55), 3.973 (0.40), 4.718 (0.47), 4.724 (0.59), 4.745 (6.15), 4.765 (0.40), 4.824 (0.41), 4.831 (0.44), 7.101 (0.69), 7.124 (13.52), 7.145 (0.82).

Die (5S)-Konfiguration wurde aufgrund der Kristallstrukturaufklärung für Beispiel 26, 108, 113, 157, 237, 358 und 454 zugeordnet.

### (5S)-5-{[(3RS)-3-Fluor-3-methylpyrrolidin-1-yl]carbonyl}-2-(4-methylbenzyl)-5,6,7,8-tetrahydro[1,2,4]triazolo[4,3-a]pyridin-3(2H)-on (Diastereomerengemisch; 2 Isomere)

### Beispiel 152

### (5RS)-5-{[(3RS)-3-Hydroxy-3-(trifluormethyl)pyrrolidin-1-yl]carbonyl}-2-(4-methylbenzyl)-5,6,7,8-tetrahydro[1,2,4]triazolo[4,3-a]pyridin-3(2H)-on (Diastereomerengemisch; 2 Isomere)

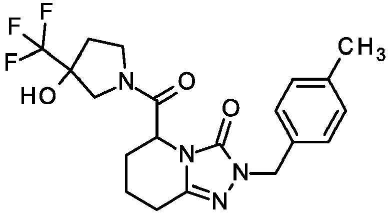

(5RS)-2-(4-Methylbenzyl)-3-oxo-2,3,5,6,7,8-hexahydro[1,2,4]triazolo[4,3-a]pyridin-5-carbonsäure (Enantiomer 1) (100 mg, 348 µmol) wurde in DMF (8.0 ml) und Dichlormethan (4.0 ml) bei Raumtemperatur vorgelegt. Anschließend wurde 1-[Bis(dimethylamino)methylen]-1H-benzotriazol-1-ium-3-oxidhexafluorophosphat (172 mg, 452 µmol) und N,N-Diisopropylethylamin (160 µl, 900 µmol) zugegeben. Nach 15 min Rühren wurde (3RS)-3-(Trifluormethyl)pyrrolidin-3-ol (Racemat) (64.8 mg, 418 µmol) zugegeben und das Reaktionsgemisch für 2 h bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 110 mg (73 % d. Th.) der Titelverbindung erhalten.
LC-MS (Methode 1): Rₜ = 1.06 min; MS (ESIpos): m/z = 425 [M+H]⁺
¹H-NMR (400 MHz, DMSO-d6) δ [ppm]: -0.008 (0.70), 0.008 (0.56), 1.664 (0.42), 1.674 (0.50), 1.687 (0.58), 1.719 (1.06), 1.979 (0.80), 1.997 (0.55), 2.015 (0.85), 2.031 (1.20), 2.060 (0.60), 2.077 (0.73), 2.101 (0.91), 2.111 (0.73), 2.124 (0.84), 2.243 (0.48), 2.272 (16.00), 2.299 (0.41), 2.523 (0.93), 2.577 (0.98), 2.588 (1.35), 2.600 (0.67), 2.630 (0.49), 3.444 (0.70), 3.476 (1.14), 3.492 (0.90), 3.539 (1.03), 3.572 (0.71), 3.585 (0.70), 3.617 (0.50), 3.678 (0.77), 3.707 (0.78), 3.733 (0.51), 3.753 (0.43), 3.761 (0.78), 3.778 (0.56), 3.802 (0.94), 3.840 (0.65), 3.868 (0.45), 4.743 (5.23), 4.748 (5.82), 4.769 (0.84), 4.779 (0.40), 4.836 (0.45), 4.842 (0.49), 6.549 (1.70), 6.564 (1.56), 6.615 (1.15), 6.631 (1.25), 7.099 (0.87), 7.124 (12.86), 7.146 (0.94).

Die (5S)-Konfiguration wurde aufgrund der Kristallstrukturaufklärung für Beispiel 26, 108, 113, 157, 237, 358 und 454 zugeordnet.

### (5S)-5-{[(3RS)-3-Hydroxy-3-(trifluormethyl)pyrrolidin-1-yl]carbonyl}-2-(4-methylbenzyl)-5,6,7,8-tetrahydro[1,2,4]triazolo[4,3-a]pyridin-3(2H)-on (Diastereomerengemisch; 2 Isomere)

### Beispiel 153

### (5RS)-5-(Pyrrolidin-1-ylcarbonyl)-2-{[6-(2,2,2-trifluorethoxy)pyridin-3-yl]methyl}-5,6,7,8-tetrahydro[1,2,4]triazolo[4,3-a]pyridin-3(2H)-on (Racemat)

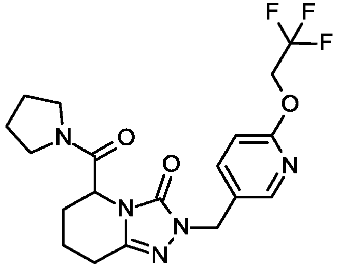

Unter Argon wurden 2,2,2-Trifluorethanol (180 µl, 2.5 mmol) in 3 ml DMF (getrocknet über Molsieb) gelöst. Bei 0°C wurden Natriumhydrid (101 mg, 60 % Reinheit, 2.52 mmol) zugegeben und 30min bei 0°C nachgerührt. Nach Zugabe von (5RS)-2-[(6-Chlorpyridin-3-yl)methyl]-5-(pyrrolidin-1-ylcarbonyl)-5,6,7,8-tetrahydro[1,2,4]triazolo[4,3-a]pyridin-3(2H)-on (Racemat) (210 mg, 87 % Reinheit, 505 µmol), gelöst in 1 ml DMF, wurde 3 Tage bei 60°C gerührt.

Zur weiteren Umsetzung wurden zunächst weiteres 2,2,2-Trifluorethanol (180 µl, 2.5 mmol) in 1mL DMF gelöst, dann bei 0°C Natriumhydrid (101 mg, 60 % Reinheit, 2.52 mmol) zugegeben und die Mischung bei 30min bei 0°C nachgerührt. Die so entstandene Reagenzlösung wurde dann der Haupt-Ansatzlösung zugefügt und diese weitere 48h bei 60°C gerührt.

Zur Aufarbeitung wurde der abgekühlte Ansatz mit Wasser versetzt, mit Natriumchlorid gesättigt und mehrmals mit Ethylacetat extrahiert. Die vereinigten organischen Phasen wurden über Magnesiumsulfat getrocknet, filtriert und eingeengt. Der verbliebene Rückstand wurde über präparative HPLC getrennt (Säule: Chromatorex, 125x30mm 10µm. Flow: 50ml/min. Gradient ( A= Wasser + 0,1% Ameisensäure , B= Acetonitril ). Laufzeit pro Trennung 38min. Detektion: 210 nm => 0min 0% B, 6min 10%B, 27min 95% B, 38min 95 % B , 40min 0%B =>). Durch Lyophilisierung der produkthaltigen Fraktionen erhielt man 24.4 mg (11 % d. Th.) der Titelverbindung.
LC-MS (Methode 3): Rₜ = 1.47 min; MS (ESIpos): m/z = 426 [M+H]⁺
¹H-NMR (400MHz, DMSO-d₆): δ [ppm]= 1.59 - 1.84 (m, 4H), 1.86 - 2.10 (m, 4H), 2.45 - 2.65 (m, 2H, teilweise durch Lösungsmittelsignal verdeckt), 3.20 - 3.41 (m, 2H, teilweise von Wassersignal überlagert), 3.46 (dt, 1H), 3.62 (dt, 1H), 4.73 (dd, 1H), 4.81 (s, 2H), 4.98 (q, 2H), 6.97 (d, 1H), 7.67 (dd, 1H), 8.08 (d, 1H).

### Beispiel 154

### 4-{[(5RS)-3-Oxo-5-(pyrrolidin-1-ylcarbonyl)-5,6,7,8-tetrahydro[1,2,4]triazolo[4,3-a]pyridin-2(3H)-yl]methyl}benzoesäure (Racemat)

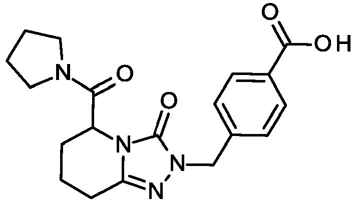

tert-Butyl-4-{[(5RS)-3-oxo-5-(pyrrolidin-1-ylcarbonyl)-5,6,7,8-tetrahydro[1,2,4]triazolo[4,3-a]pyridin-2(3H)-yl]methyl}benzoat (Racemat) (245 mg, 574 µmol) wurde in 5 ml Dichlormethan gelöst. Nach Zugabe von 750 µl (9.7 mmol) Trifluoressigsäure wurde die Reaktionsmischung über Nacht bei Raumtemperatur gerührt. Zur Aufarbeitung wurde der Ansatz unter Eisbadkühlung und kräftigem Rühren mit 3N wässriger Natronlauge versetzt und so auf pH 3 gestellt. Nach Verdünnen mit Dichlormethan/Wasser und Extraktion wurde die organische Phase abgetrennt, anschließend die wässrige noch zweimal mit Dichlormethan extrahiert. Die vereinigten organischen Phasen wurden mit gesättigter Natriumchloridlösung gewaschen, über Natriumsulfat getrocknet, eingeengt und im Vakuum von RestLösungsmittel befreit. So wurden 195 mg (92 % d. Th.) der Titelverbindung erhalten.
LC-MS (Methode 4): Rₜ = 0.58 min; MS (ESIpos): m/z = 371 [M+H]⁺
¹H-NMR (400MHz, DMSO-d₆): δ [ppm]= 1.62 - 1.84 (m, 4H), 1.89 - 2.10 (m, 4H), 2.52 - 2.67 (m, 2H, teilweise durch Lösungsmittelsignal verdeckt), 3.22 - 3.41 (m, 2H, teilweise von Wassersignal überlagert), 3.47 (dt, 1H), 3.62 (dt, 1H), 4.75 (dd, 1H), 4.84 - 4.94 (m, 2H), 7.31 - 7.36 (m, 2H), 7.88 - 7.93 (m, 2H), 12.92 (br s, 1H).

### Beispiel 155

### N-Methyl-4-{[(5RS)-3-oxo-5-(pyrrolidin-1-ylcarbonyl)-5,6,7,8-tetrahydro[1,2,4]triazolo[4,3-a]pyridin-2(3H)-yl]methyl}benzamid (Racemat)

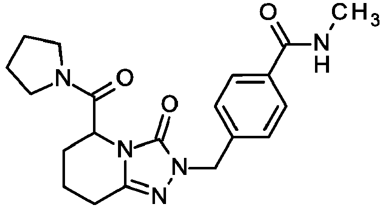

4-{[(5RS)-3-Oxo-5-(pyrrolidin-1-ylcarbonyl)-5,6,7,8-tetrahydro[1,2,4]triazolo[4,3-a]pyridin-2(3H)-yl]methyl}benzoesäure (Racemat) (78.0 mg, 211 µmol) wurde in 2.5 ml Dichlormethan vorgelegt, sodann HATU (160 mg, 421 µmol), N,N-Diisopropylethylamin (73 µl, 420 µmol) und Methylamin, 33%ig in Ethanol (31 µl, 250 µmol) zugegeben. Der Ansatz wurde über Nacht bei Raumtemperatur gerührt. Man fügte weiteres Methylamin, 33%ig in Ethanol (13 µl, 110 µmol) zu und ließ weitere 5h bei Raumtemperatur rühren. Zur Aufarbeitung wurde der Ansatz mit Dichlormethan/Wasser verdünnt, mit 1N wässriger Salzsäure sauer gestellt, extrahiert und die organische Phase abgetrennt. Die wässrige Phase wurde noch zweimal mit Dichlormethan extrahiert, anschließend wurden die vereinigten organischen Phasen mit gesättigter Natriumchloridlösung gewaschen, über Natriumsulfat getrocknet, eingeengt und RestLösungsmittel im Vakuum entfernt. Das Rohprodukt wurde durch präparative HPLC getrennt (Instrument: Waters Prep LC/MS System, Säule: Phenomenex Kinetex C18 5µm 100x30 mm Eluent A : Wasser, Eluent B : Acetonitril, Fluß: 65 ml/min plus 5ml 2%ige Ameisensäure in Wasser, Raumtemperatur, Wellenlänge 200-400 nm, At-Säule Injektion (Komplettinjektion) Gradientenprofil: 0 bis 2 min 10% Eluent B, 2 bis 2,2 min auf 20% Eluent B, 2,2 bis 7 min auf 60% Eluent B, 7 bis 7,5 min auf 92% Eluent B, 7,5 bis 9 min bei 92% B). Durch Vereinigung der produkthaltigen, lyophilisierten Fraktionen erhielt man 2.00 mg (2.5 % d. Th.) der Titelverbindung.
LC-MS (Methode 12): Rₜ = 1.00 min; MS (ESIpos): m/z = 384 [M+H]⁺
¹H-NMR (500MHz, DMSO-d₆): δ [ppm]= 1.62 - 1.83 (m, 4H), 1.87 - 2.11 (m, 4H), 2.47 - 2.67 (m, 2H, teilweise durch Lösungsmittelsignal verdeckt), 2.62 (dt, 1H), 2.77 (d, 3H), 3.23 - 3.40 (m, 2H, teilweise von Wassersignal überlagert), 3.47 (dt, 1H), 3.62 (dt, 1H), 4.75 (dd, 1H), 4.85 (s, 2H), 7.28 (d, 2H), 7.78 (d, 2H), 8.39 (br q, 1H).

### Beispiel 156

### N,N-Dimethyl-4-{[(5RS)-3-oxo-5-(pyrrolidin-1-ylcarbonyl)-5,6,7,8-tetrahydro[1,2,4]triazolo[4,3-a]pyridin-2(3H)-yl]methyl}benzamid (Racemat)

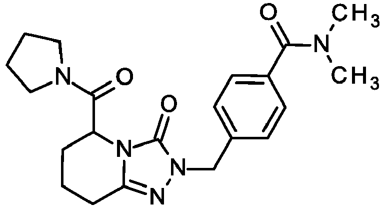

4-{[(5RS)-3-Oxo-5-(pyrrolidin-1-ylcarbonyl)-5,6,7,8-tetrahydro[1,2,4]triazolo[4,3-a]pyridin-2(3H)-yl]methyl}benzoesäure (Racemat) (78.0 mg, 211 µmol) wurde in 2.5 ml Dichlormethan vorgelegt, sodann HATU (160 mg, 421 µmol), N,N-Diisopropylethylamin (73 µl, 420 µmol) und Dimethylamin, 2M in THF (126 µl, 252 µmol) zugegeben. Der Ansatz wurde zunächst 4h, dann über ein Wochenende bei Raumtemperatur gerührt. Man fügte weiteres Dimethylamin, 2M in THF (105 µl, 210 µmol) zu und ließ weitere 54h bei Raumtemperatur rühren. Zur Aufarbeitung wurde der Ansatz mit Dichlormethan/Wasser verdünnt, mit 1N wässriger Salzsäure sauer gestellt, extrahiert und die organische Phase abgetrennt. Die wässrige Phase wurde noch zweimal mit Dichlormethan extrahiert, anschließend wurden die vereinigten organischen Phasen mit gesättigter Natriumchloridlösung gewaschen, über Natriumsulfat getrocknet, eingeengt und RestLösungsmittel im Vakuum entfernt. Das Rohprodukt wurde in Acetonitril/Wasser gelöst und durch präparative HPLC getrennt (Säule: Kromasil C18, 125x30mm, 10µm, Eluent: Acetonitril (B) / Wasser + 0,1 % TFA (A), Gradient: 0min 90% A, 6min 90% A, 18min 5%A, 20min 5%A, 21min 90%A, Fluss: 75ml/min, Detektor: 210nm). Durch Vereinigung der produkthaltigen Fraktionen und Entfernen der Lösungsmittel im Vakuum erhielt man 36.7 mg (42 % d. Th.)der Titelverbindung.
LC-MS (Methode 3): Rₜ = 0.95 min; MS (ESIpos): m/z = 398 [M+H]⁺
¹H-NMR (400MHz, DMSO-d₆): δ [ppm]= 1.62 - 1.84 (m, 4H), 1.87 - 2.11 (m, 4H), 2.46 - 2.69 (m, 2H, teilweise durch Lösungsmittelsignal verdeckt), 2.90 (br s, 3H), 2.97 (br s, 3H), 3.26 (dt, 1H), 3.36 (dt, 1H), 3.47 (dt, 1H), 3.62 (dt, 1H, teilweise von Wassersignal überlagert), 4.75 (dd, 1H), 4.85 (s, 2H), 7.24 - 7.29 (m, 2H), 7.34 - 7.39 (m, 2H).

### Beispiel 157

### (5S)-2-(4-Methylbenzyl)-5-(pyrrolidin-1-ylcarbonyl)-2,5,6,7-tetrahydro-3H-pyrrolo[2,1-c][1,2,4]triazol-3-on (Enantiomer)

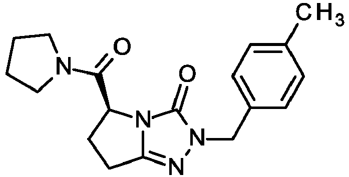

(5S)-2-(4-Methylbenzyl)-3-oxo-2,5,6,7-tetrahydro-3H-pyrrolo[2,1-c][1,2,4]triazol-5-carbonsäure (Enantiomer) (50.0 mg, 183 µmol) wurde in DMF (2.2 ml) und Dichlormethan (1.1 ml) bei Raumtemperatur vorgelegt. Anschließend wurde 1-[Bis(dimethylamino)methylen]-1H-benzotriazol-1-ium-3-oxidhexafluorophosphat (90.2 mg, 238 µmol) und N,N-Diisopropylethylamin (64 µl, 370 µmol) zugegeben. Nach 15 min Rühren wurde Pyrrolidin (15.6 mg, 220 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt. Der Rückstand wurde durch chirale präparative HPLC weiter aufgereinigt [Probenvorbereitung: 37 mg gelöst in 1 ml Acetonitril; Injektionsvolumen: 0.20 ml; Säule: Daicel Chiralpak^{®} AD-H 5µm, 250 × 20 mm; Laufmittel: n-Heptan/Ethanol: 30:70; Fluß: 20 ml/min; Temperatur 23°C; UV Detektion: 220 nm]. Es wurden 19 mg (31 % d. Th.) der Titelverbindung erhalten.

Analytische chirale HPLC: Rₜ = 3.49 min, e.e. = 99.9 % [Säule: Daicel Chiralcel^{®} AD-3 3µm 50 × 4.6 mm; Laufmittel: n-Heptan/iPropanol 1:1, 0.2 % Trifluoressigsäure, 1 % Wasser; Fluß: 1 ml/min; UV Detektion: 220 nm
LC-MS (Methode 4): Rₜ = 0.70 min; MS (ESIpos): m/z = 327 [M+H]⁺
¹H-NMR (400 MHz, DMSO-d6) δ [ppm]: -0.008 (2.92), 0.008 (2.77), 1.781 (0.76), 1.798 (1.57), 1.816 (1.43), 1.832 (0.52), 1.887 (0.43), 1.903 (1.35), 1.919 (1.69), 1.936 (1.00), 2.274 (9.31), 2.327 (0.50), 2.366 (0.66), 2.377 (0.48), 2.388 (0.45), 2.524 (1.50), 2.670 (1.06), 2.685 (0.85), 2.693 (1.02), 2.706 (1.21), 2.715 (1.32), 2.748 (0.45), 2.755 (0.48), 2.786 (0.55), 2.808 (0.43), 3.291 (0.62), 3.309 (1.49), 3.328 (1.53), 3.345 (0.62), 3.396 (0.72), 3.403 (0.49), 3.414 (0.42), 3.421 (0.84), 3.438 (0.42), 3.527 (1.79), 3.646 (2.87), 3.663 (3.01), 3.688 (2.02), 3.705 (1.07), 4.698 (0.41), 4.736 (2.51), 4.748 (2.52), 4.897 (0.83), 4.905 (0.63), 4.919 (0.97), 4.925 (0.62), 7.137 (16.00).

Die (5S)-Konfiguration wurde aufgrund von Kristallstrukturaufklärung für Beispiel 157zugeordnet.

### Beispiel 158

### (5RS)-5-(3,6-Dihydropyridin-1(2H)-ylcarbonyl)-2-(4-methylbenzyl)-5,6,7,8-tetrahydro[1,2,4]triazolo[4,3-a]pyridin-3(2H)-on (Racemat)

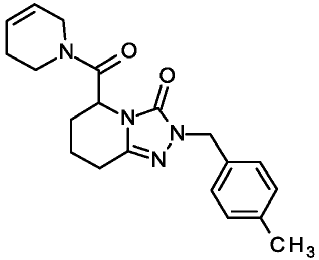

1,2,3,6-Tetrahydropyridin (8.3 mg, 0.10 mmol) wurde in einer Kavität einer 96er Multititerplatte vorgelegt und mit (5RS)-2-(4-Methylbenzyl)-3-oxo-2,3,5,6,7,8-hexahydro[1,2,4]triazolo[4,3-a]pyridin-5-carbonsäure (Racemat) (28.7 mg, 0.10 mmol) und mit HATU (49.4 mg, 0.13 mmol), gelöst in 0.8 ml DMF, versetzt. Zu der Mischung wurden 50 µl 4-Methylmorpholin gegeben, die Mikrotiterplatte verschlossen und bei Raumtemperatur über Nacht geschüttelt. Anschließend wurde filtriert und das Filtrat per präparativer LC-MS getrennt.

(Instrument MS: Waters, Instrument HPLC: Waters (Säule Waters X-Bridge C18, 19 mm × 50 mm, 5 µm, Eluent A: Wasser, Eluent B: Acetonitril (ULC) mit Gradient; Fluss: 38.5 ml/min; Modifier: aq. Ammoniak 5%, Fluß: 1.5 ml/min; UV-Detektion: DAD; 210 - 400 nm)
bzw.:
Instrument MS: Waters, Instrument HPLC: Waters (Säule Phenomenex Luna 5µ C18(2) 100A, AXIA Tech. 50 × 21.2 mm, Eluent A: Wasser, Eluent B: Acetonitril (ULC), mit Gradient; Fluss: 38.5 ml/min; Modifier: aq. Ameisensäure 10%, Fluß: 1.5 ml/min; UV-Detektion: DAD; 210 - 400 nm).

Die produktenthaltenden Fraktionen wurden per Zentrifugaltrockner eingedampft. Der Rückstand aller Produktfraktionen wurde in insgesamt 1.8 ml DMSO gelöst, vereint und erneut eingedampft. Es wurden so 19.8 mg (56% d. Th.) der Titelverbindung erhalten.

LC/MS (Methode 13): Rₜ = 0.95 min, MS (ESIpos): m/z = 353 [M+H]+.

In Analogie zu Beispiel 158 wurden die in Tabelle 1 gezeigten Beispielverbindungen hergestellt.

**Tabelle 1**

| Beispiel | IUPAC-Name / Struktur (Ausbeute) | Analytische Daten |
|---|---|---|
| **Beispiel 159** | (5RS)-2-(4-Methylbenzyl)-5-(morpholin-4-ylcarbonyl)-5,6,7,8-tetrahydro[1,2,4]triazolo[4,3-a]pyridin-3(2H)-on (Racemat) | LC-MS (Methode 13): |
| | | Rₜ = 0.85 min |
| | | MS (ESIpos): m/z = 357 [M+H]⁺ |
| | 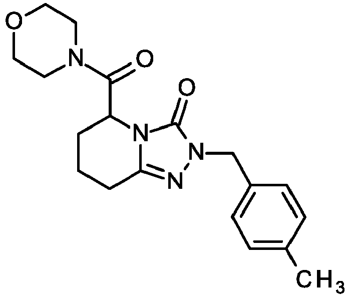 | |
| | (19.9 mg, 56% d. Th.) | |
| **Beispiel 160** | (5RS)-2-(4-Methylbenzyl)-5-[(4-methylpiperidin-1-yl)carbonyl]-5,6,7,8-tetrahydro[1,2,4]triazolo[4,3-a]pyridin-3(2H)-on (Racemat) | LC-MS (Methode 13): |
| | | Rₜ = 1.02 min |
| | | MS (ESIpos): m/z = 369 [M+H]⁺ |
| | 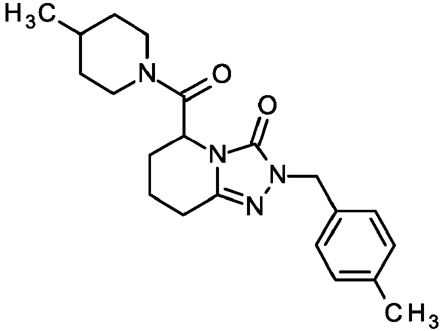 | |
| | (19.9 mg, 54% d. Th.) | |
| **Beispiel 161** | (5RS)-2-(4-Methylbenzyl)-5-(piperidin-1-ylcarbonyl)-5,6,7,8-tetrahydro[1,2,4]triazolo[4,3-a]pyridin-3(2H)-on (Racemat) | LC-MS (Methode 13): |
| | | Rₜ = 0.96 min |
| | | MS (ESIpos): m/z = 355 [M+H]⁺ |
| | 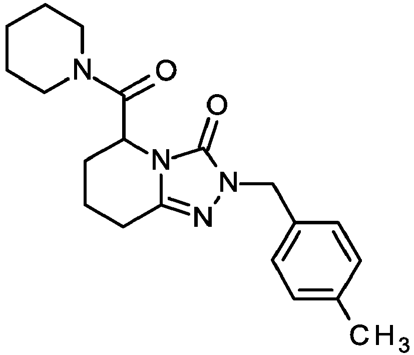 | |
| | (22.1 mg, 62% d. Th.) | |
| **Beispiel 162** | (5RS)-2-(4-Methylbenzyl)-5-[2(RS)-(2-methylpyrrolidin-1-yl)carbonyl]-5,6,7,8-tetrahydro[1,2,4]triazolo[4,3-a]pyridin-3(2H)-on (4 Stereoisomere) | LC-MS (Methode 13): |
| | | Rt = 0.95 min |
| | | MS (ESIpos): m/z = 355 [M+H]⁺ |
| | 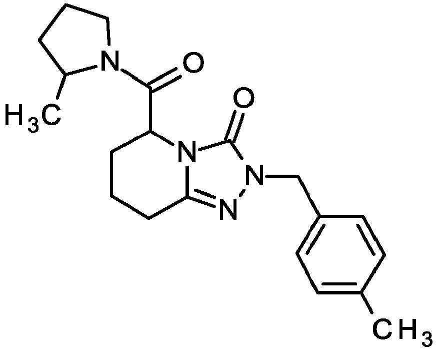 | |
| | (19.4 mg, 55% d. Th.) | |

### Beispiel 163

### (5RS)-2-[(6-Chlorpyridin-3-yl)methyl]-5-{[(3S)-3-fluorpyrrolidin-1-yl]carbonyl}-2,5,6,7-tetrahydro-3H-pyrrolo[2,1-c][1,2,4]triazol-3-on (Diastereomerengemisch; 2 Isomere)

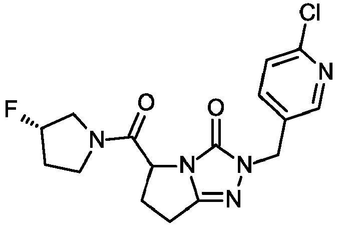

(5S)-2-[(6-Chlorpyridin-3-yl)methyl]-3-oxo-2,5,6,7-tetrahydro-3H-pyrrolo[2,1-c][1,2,4]triazol-5-carbonsäure (75.0 mg, 78 % Reinheit, 199 µmol) wurde in THF (2.1 ml) bei Raumtemperatur vorgelegt. Anschließend wurden (3S)-3-Fluorpyrrolidinhydrochlorid (29.9 mg, 238 µmol), HATU (98.1 mg, 258 µmol) und Triethylamin (170 µl, 1.2 mmol) zugegeben. Das Reaktionsgemisch wurde über Nacht bei Raumtemperatur gerührt. Es wurden erneut Triethylamin (55 µl, 400 µmol), (3S)-3-Fluorpyrrolidinhydrochlorid (12.5 mg, 99.3 µmol) und HATU (37.7 mg, 99.3 µmol) zugegeben und für weitere 90 Minuten gerührt. Das Reaktionsgemisch wurde mit Wasser versetzt und im Vakuum eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 5.91 mg (8 % d. Th.) der Titelverbindung erhalten.
LC-MS (Methode 3): Rₜ = 0.94 min; MS (ESIpos): m/z = 366 [M+H]⁺
¹H-NMR (400 MHz, DMSO-d6) δ [ppm]: -0.008 (1.89), 0.008 (1.61), 2.100 (1.11), 2.115 (1.07), 2.137 (1.07), 2.147 (1.03), 2.161 (0.86), 2.217 (0.84), 2.234 (0.96), 2.264 (1.15), 2.288 (0.52), 2.301 (0.58), 2.307 (0.66), 2.319 (0.78), 2.326 (0.84), 2.334 (0.82), 2.342 (0.82), 2.349 (0.56), 2.357 (0.66), 2.367 (0.62), 2.375 (0.60), 2.383 (0.74), 2.390 (1.01), 2.396 (1.11), 2.403 (0.98), 2.411 (1.03), 2.420 (1.15), 2.430 (0.78), 2.441 (0.66), 2.450 (0.48), 2.671 (1.15), 2.690 (1.41), 2.711 (3.40), 2.719 (3.04), 2.731 (4.98), 2.743 (2.57), 2.752 (4.06), 2.771 (0.86), 2.785 (0.78), 2.792 (1.25), 2.806 (0.64), 2.817 (1.35), 2.825 (1.37), 2.838 (1.27), 2.848 (1.59), 2.859 (1.03), 2.870 (1.01), 2.881 (0.68), 3.342 (1.25), 3.369 (0.74), 3.384 (0.56), 3.393 (0.78), 3.419 (0.66), 3.428 (0.70), 3.468 (0.40), 3.482 (0.72), 3.491 (0.86), 3.516 (1.11), 3.525 (0.74), 3.563 (0.56), 3.573 (0.52), 3.600 (1.29), 3.624 (2.33), 3.646 (1.45), 3.664 (1.89), 3.669 (1.89), 3.685 (2.33), 3.700 (1.55), 3.710 (1.21), 3.726 (1.75), 3.758 (0.58), 3.790 (0.72), 3.832 (0.54), 3.840 (0.62), 3.927 (0.54), 3.935 (0.56), 3.966 (0.74), 3.988 (0.68), 4.013 (0.58), 4.885 (16.00), 4.901 (1.47), 4.939 (1.43), 4.946 (1.55), 4.961 (1.61), 4.967 (1.35), 5.001 (0.46), 5.008 (0.60), 5.018 (0.88), 5.024 (1.17), 5.039 (0.76), 5.045 (0.56), 5.277 (1.37), 5.363 (0.78), 5.372 (0.92), 5.410 (1.37), 5.504 (0.90), 7.511 (6.01), 7.531 (7.46), 7.722 (3.92), 7.743 (3.34), 8.325 (4.96), 8.330 (5.23).

### Beispiel 164

### (5RS)-5-{[(3S)-3-Fluorpyrrolidin-1-yl]carbonyl}-2-{[6-(trifluormethyl)pyridin-3-yl]methyl}-2,5,6,7-tetrahydro-3H-pyrrolo[2,1-c][1,2,4]triazol-3-on (Diastereomerengemisch; 2 Isomere)

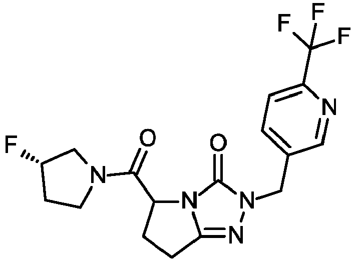

(5S)-3-Oxo-2-{[6-(trifluormethyl)pyridin-3-yl]methyl}-2,5,6,7-tetrahydro-3H-pyrrolo[2,1-c][1,2,4]triazol-5-carbonsäure (75.0 mg, 228 µmol) wurde in THF (2.4 ml) bei Raumtemperatur vorgelegt. Anschließend wurden (3S)-3-Fluorpyrrolidinhydrochlorid (34.4 mg, 274 µmol), HATU (113 mg, 297 µmol) und Triethylamin (190 µl, 1.4 mmol) zugegeben. Das Reaktionsgemisch wurde für 20 Stunden bei Raumtemperatur gerührt. Es wurden erneut HATU (113 mg, 297 µmol), (3S)-3-Fluorpyrrolidinhydrochlorid (17.2 mg, 137 µmol) und Triethylamin (190 µl, 1.4 mmol) zugegeben und über Nacht gerührt. Das Reaktionsgemisch wurde mit Wasser versetzt und im Vakuum eingeengt. Es wurden erneut HATU (113 mg, 297 µmol), (3S)-3-Fluorpyrrolidinhydrochlorid (17.2 mg, 137 µmol) und Triethylamin (190 µl, 1.4 mmol) zugegeben und bei 40°C über Nacht gerührt. Das Reaktionsgemisch wurde mit Wasser versetzt und im Vakuum eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 39.4 mg (43 % d. Th.) der Titelverbindung erhalten.
LC-MS (Methode 4): Rₜ = 0.63 min; MS (ESIpos): m/z = 400 [M+H]⁺
¹H-NMR (400 MHz, DMSO-d6) δ [ppm]: -0.008 (2.09), 0.008 (1.78), 1.994 (0.41), 2.073 (0.50), 2.101 (1.23), 2.119 (1.08), 2.140 (1.10), 2.150 (1.06), 2.165 (0.94), 2.220 (0.88), 2.238 (1.01), 2.266 (1.21), 2.327 (0.97), 2.333 (0.76), 2.343 (0.61), 2.351 (0.76), 2.358 (0.67), 2.366 (1.12), 2.385 (0.86), 2.393 (0.90), 2.407 (1.42), 2.413 (1.23), 2.422 (0.97), 2.431 (1.21), 2.440 (0.76), 2.451 (0.58), 2.665 (0.68), 2.680 (1.14), 2.700 (1.42), 2.721 (3.08), 2.729 (3.64), 2.741 (5.50), 2.762 (3.84), 2.772 (1.50), 2.782 (1.32), 2.795 (0.92), 2.803 (1.51), 2.827 (1.32), 2.835 (1.30), 2.848 (1.23), 2.858 (1.50), 2.870 (0.94), 2.881 (0.88), 2.893 (0.59), 3.372 (1.39), 3.398 (1.01), 3.424 (0.68), 3.432 (0.72), 3.472 (0.70), 3.488 (0.86), 3.496 (1.10), 3.518 (1.44), 3.541 (0.49), 3.567 (0.92), 3.578 (0.83), 3.605 (2.02), 3.630 (2.74), 3.651 (1.28), 3.669 (1.78), 3.674 (1.66), 3.688 (2.11), 3.704 (1.37), 3.714 (1.06), 3.729 (1.77), 3.761 (0.50), 3.794 (0.61), 3.834 (0.47), 3.843 (0.50), 3.930 (0.45), 3.938 (0.49), 3.969 (0.88), 3.991 (1.12), 4.017 (0.81), 4.053 (0.47), 4.079 (0.56), 4.110 (0.45), 4.886 (0.94), 4.893 (1.03), 4.908 (1.06), 4.915 (0.92), 4.953 (1.19), 4.960 (1.32), 4.975 (1.41), 4.982 (1.23), 5.012 (16.00), 5.032 (1.64), 5.039 (2.07), 5.054 (1.35), 5.060 (0.95), 5.279 (1.44), 5.365 (0.95), 5.374 (0.94), 5.412 (1.44), 5.497 (0.92), 5.506 (0.90), 7.904 (2.50), 7.925 (10.38), 7.937 (6.31), 7.958 (1.66), 8.675 (6.00).

### Beispiel 165

### (5RS)-2-(3-Chlor-4-fluorbenzyl)-5-{[(3S)-3-fluorpyrrolidin-1-yl]carbonyl}-2,5,6,7-tetrahydro-3H-pyrrolo[2,1-c][1,2,4]triazol-3-on (Diastereomerengemisch; 2 Isomere)

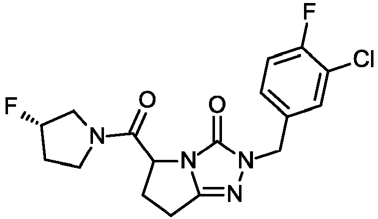

(5S)-2-(3-Chlor-4-fluorbenzyl)-3-oxo-2,5,6,7-tetrahydro-3H-pyrrolo[2,1-c][1,2,4]triazol-5-carbonsäure (75.0 mg, 87 % Reinheit, 209 µmol) wurde in THF (2.2 ml) bei Raumtemperatur vorgelegt. Anschließend wurden (3S)-3-Fluorpyrrolidinhydrochlorid (31.5 mg, 251 µmol), HATU (103 mg, 272 µmol) und Triethylamin (180 µl, 1.3 mmol) zugegeben. Das Reaktionsgemisch wurde über Nacht bei Raumtemperatur gerührt. Es wurden erneut (3S)-3-Fluorpyrrolidinhydrochlorid (15.8 mg, 126 µmol), HATU (103 mg, 272 µmol) und Triethylamin (180 µl, 1.3 mmol) zugegeben und bei 70°C für 4 Stunden gerührt. Das Reaktionsgemisch wurde mit Wasser versetzt und im Vakuum eingeengt. Der Rückstand wurde über präparative HPLC aufgereinigt (Chromatorex C18, 10µm, 125mm × 30mm; Laufmittel: Acetonitril/Wasser mit 0.1% Ameisensäure-Gradient). Die produkthaltigen Fraktionen wurden im Vakuum eingeengt und es wurden 16.4 mg (20 % d. Th.) der Titelverbindung erhalten.
LC-MS (Methode 3): Rₜ = 1.37 min; MS (ESIpos): m/z = 383 [M+H]⁺
¹H-NMR (400 MHz, DMSO-d6) δ [ppm]: -0.149 (1.85), -0.008 (16.00), 0.008 (13.87), 0.146 (1.80), 2.098 (0.73), 2.266 (0.79), 2.327 (1.91), 2.366 (1.63), 2.396 (0.90), 2.421 (0.84), 2.523 (4.55), 2.670 (1.57), 2.698 (0.90), 2.711 (1.91), 2.727 (2.19), 2.738 (3.59), 2.758 (2.64), 2.799 (0.90), 2.827 (0.95), 2.851 (1.07), 2.874 (0.67), 3.372 (0.79), 3.423 (0.51), 3.495 (0.62), 3.515 (0.90), 3.602 (1.29), 3.628 (1.68), 3.666 (1.07), 3.685 (1.35), 3.727 (4.55), 3.970 (0.56), 3.991 (0.67), 4.015 (0.56), 4.826 (7.92), 4.881 (0.79), 4.903 (0.73), 4.947 (0.79), 4.954 (0.79), 4.970 (0.84), 5.029 (1.01), 5.047 (0.79), 5.277 (0.90), 5.364 (0.56), 5.411 (0.90), 5.503 (0.56), 6.922 (0.45), 7.276 (1.96), 7.288 (1.74), 7.379 (2.86), 7.402 (4.38), 7.424 (2.13), 7.458 (2.19), 7.473 (2.19).

### Beispiel 166

### (5RS)-2-[(6-Chlorpyridin-3-yl)methyl]-5-[(3,3-difluorpyrrolidin-1-yl)carbonyl]-2,5,6,7-tetrahydro-3H-pyrrolo[2,1-c][1,2,4]triazol-3-on (Racemat)

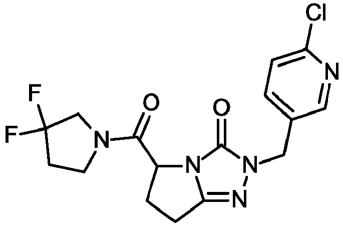

(5S)-2-[(6-Chlorpyridin-3-yl)methyl]-3-oxo-2,5,6,7-tetrahydro-3H-pyrrolo[2,1-c][1,2,4]triazol-5-carbonsäure (75.0 mg, 78 % Reinheit, 199 µmol) wurde in THF (2.1 ml) bei Raumtemperatur vorgelegt. Anschließend wurden 3,3-Difluorpyrrolidinhydrochlorid (34.2 mg, 238 µmol), HATU (98.1 mg, 258 µmol) und Triethylamin (170 µl, 1.2 mmol) zugegeben. Das Reaktionsgemisch wurde für 20 Stunden bei Raumtemperatur gerührt. Es wurden erneut 3,3-Difluorpyrrolidinhydrochlorid (17.1 mg, 119 µmol), HATU (98.1 mg, 258 µmol) und Triethylamin (55 µl, 400 µmol) zugegeben und über Nacht gerührt. Das Reaktionsgemisch wurde mit Wasser versetzt und im Vakuum eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 21.2 mg (28 % d. Th.) der Titelverbindung erhalten.
LC-MS (Methode 3): Rₜ = 1.11 min; MS (ESIpos): m/z = 384 [M+H]⁺
¹H-NMR (400 MHz, DMSO-d6) δ [ppm]: -0.149 (1.41), -0.008 (11.99), 0.008 (10.88), 0.146 (1.35), 2.327 (0.96), 2.366 (0.96), 2.391 (1.02), 2.412 (2.04), 2.424 (2.13), 2.440 (2.52), 2.450 (1.77), 2.459 (1.59), 2.523 (3.45), 2.561 (1.41), 2.578 (0.78), 2.596 (0.42), 2.669 (1.20), 2.675 (1.14), 2.686 (1.05), 2.694 (1.20), 2.716 (2.52), 2.736 (2.79), 2.743 (3.48), 2.751 (1.65), 2.777 (1.92), 2.791 (1.44), 2.820 (1.29), 2.842 (0.75), 3.550 (2.13), 3.569 (3.63), 3.588 (1.86), 3.682 (0.54), 3.699 (1.80), 3.729 (2.31), 3.744 (1.20), 3.763 (0.75), 3.774 (1.65), 3.808 (1.20), 3.895 (0.48), 3.925 (0.96), 3.962 (1.08), 3.976 (1.29), 3.995 (1.08), 4.002 (1.05), 4.021 (0.51), 4.230 (0.48), 4.259 (1.05), 4.291 (1.05), 4.886 (16.00), 4.927 (1.44), 4.934 (1.17), 4.948 (1.53), 5.011 (1.47), 5.018 (1.14), 5.032 (1.59), 5.039 (1.02), 7.510 (4.82), 7.531 (5.96), 7.718 (2.61), 7.723 (2.67), 7.739 (2.25), 7.744 (2.22), 8.324 (4.01), 8.329 (3.93).

### Beispiel 167

### (5RS)-5-[(3,3-Difluorpyrrolidin-1-yl)carbonyl]-2-{[6-(trifluormethyl)pyridin-3-yl]methyl}-2,5,6,7-tetrahydro-3H-pyrrolo[2,1-c][1,2,4]triazol-3-on (Racemat)

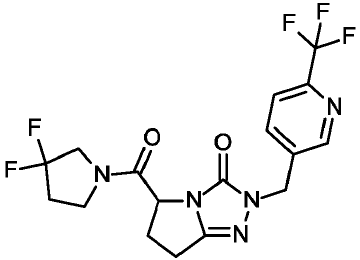

(5S)-3-Oxo-2-{[6-(trifluormethyl)pyridin-3-yl]methyl}-2,5,6,7-tetrahydro-3H-pyrrolo[2,1-c][1,2,4]triazol-5-carbonsäure (75.0 mg, 228 µmol) wurde in THF (2.4 ml) bei Raumtemperatur vorgelegt. Anschließend wurden 3,3-Difluorpyrrolidinhydrochlorid (39.4 mg, 274 µmol), HATU (113 mg, 297 µmol) und Triethylamin (190 µl, 1.4 mmol) zugegeben. Das Reaktionsgemisch wurde für 20 Stunden bei Raumtemperatur gerührt. Es wurden erneut 3,3-Difluorpyrrolidinhydrochlorid (19.7 mg, 137 µmol), HATU (113 mg, 297 µmol) und Triethylamin (64 µl, 460 µmol) zugegeben und über Nacht gerührt. Das Reaktionsgemisch wurde mit Wasser versetzt und im Vakuum eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 15.4 mg (16 % d. Th.) der Titelverbindung erhalten.
LC-MS (Methode 3): Rₜ = 1.30 min; MS (ESIpos): m/z = 418 [M+H]⁺
¹H-NMR (400 MHz, DMSO-d6) δ [ppm]: -0.150 (1.95), -0.034 (0.54), -0.009 (16.00), 0.007 (14.34), 0.146 (1.95), 2.327 (1.41), 2.331 (1.16), 2.365 (1.04), 2.408 (0.83), 2.421 (1.16), 2.442 (1.16), 2.460 (1.33), 2.523 (3.69), 2.581 (0.46), 2.669 (1.53), 2.710 (1.08), 2.725 (1.08), 2.747 (1.20), 2.753 (1.37), 2.762 (0.79), 2.787 (0.83), 2.801 (0.58), 2.830 (0.50), 3.556 (0.75), 3.573 (1.41), 3.592 (0.66), 3.702 (0.75), 3.732 (0.87), 3.748 (0.46), 3.770 (0.33), 3.779 (0.62), 3.812 (0.50), 3.929 (0.37), 3.964 (0.46), 3.979 (0.46), 3.998 (0.41), 4.006 (0.41), 4.263 (0.46), 4.293 (0.46), 4.948 (0.46), 4.963 (0.66), 5.012 (5.68), 5.025 (0.79), 5.046 (0.62), 5.053 (0.50), 7.904 (0.75), 7.924 (3.07), 7.935 (1.82), 7.958 (0.54), 8.673 (2.03).

### Beispiel 168

### (5RS)-2-(3-Chlor-4-fluorbenzyl)-5-[(3,3-difluorpyrrolidin-1-yl)carbonyl]-2,5,6,7-tetrahydro-3H-pyrrolo[2,1-c][1,2,4]triazol-3-on (Racemat)

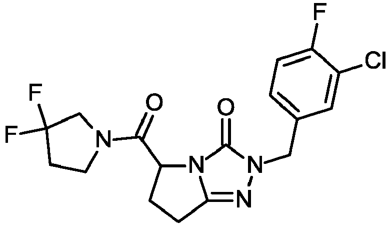

(5S)-2-(3-Chlor-4-fluorbenzyl)-3-oxo-2,5,6,7-tetrahydro-3H-pyrrolo[2,1-c][1,2,4]triazol-5-carbonsäure (75.0 mg, 87 % Reinheit, 209 µmol) wurde in THF (2.2 ml) bei Raumtemperatur vorgelegt. Anschließend wurden 3,3-Difluorpyrrolidinhydrochlorid (36.1 mg, 251 µmol), HATU (103 mg, 272 µmol) und Triethylamin (180 µl, 1.3 mmol) zugegeben. Das Reaktionsgemisch wurde über Nacht bei Raumtemperatur gerührt. Das Reaktionsgemisch wurde mit Wasser versetzt und im Vakuum eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 64.0 mg (71 % d. Th.) der Titelverbindung erhalten.
LC-MS (Methode 4): Rₜ = 0.79 min; MS (ESIpos): m/z = 401 [M+H]⁺
¹H-NMR (400 MHz, DMSO-d6) δ [ppm]: -0.150 (1.35), 0.008 (10.27), 0.146 (1.18), 2.327 (2.69), 2.366 (2.86), 2.387 (1.85), 2.410 (3.37), 2.430 (3.54), 2.440 (3.87), 2.460 (2.86), 2.523 (9.60), 2.578 (1.85), 2.669 (3.03), 2.710 (3.54), 2.724 (3.87), 2.744 (5.56), 2.750 (5.73), 2.781 (3.71), 2.797 (2.53), 2.825 (2.02), 2.847 (1.35), 3.552 (3.37), 3.571 (5.73), 3.590 (3.03), 3.701 (3.20), 3.727 (7.58), 3.745 (1.85), 3.776 (2.69), 3.809 (2.02), 3.896 (0.67), 3.926 (1.52), 3.962 (2.02), 3.979 (2.02), 3.996 (1.68), 4.024 (0.84), 4.231 (0.84), 4.260 (1.68), 4.290 (1.68), 4.322 (0.51), 4.785 (1.01), 4.825 (16.00), 4.868 (0.84), 4.934 (1.85), 4.941 (1.85), 4.954 (2.36), 5.018 (2.36), 5.026 (1.68), 5.040 (2.36), 7.270 (3.20), 7.379 (5.22), 7.402 (7.24), 7.424 (3.87), 7.455 (3.87), 7.472 (3.87).

### Beispiel 169

### (5RS)-2-[(6-Chlorpyridin-3-yl)methyl]-5-[(3-fluorazetidin-1-yl)carbonyl]-2,5,6,7-tetrahydro-3H-pyrrolo[2,1-c][1,2,4]triazol-3-on (Racemat)

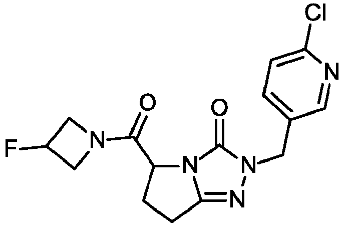

(5S)-2-[(6-Chlorpyridin-3-yl)methyl]-3-oxo-2,5,6,7-tetrahydro-3H-pyrrolo[2,1-c][1,2,4]triazol-5-carbonsäure (75.0 mg, 78 % Reinheit, 199 µmol) wurde in THF (2.1 ml) bei Raumtemperatur vorgelegt. Anschließend wurden 3-Fluorazetidinhydrochlorid (26.6 mg, 238 µmol), HATU (98.1 mg, 258 µmol) und Triethylamin (170 µl, 1.2 mmol) zugegeben. Das Reaktionsgemisch wurde über Nacht bei Raumtemperatur gerührt. Es wurden erneut 3-Fluorazetidinhydrochlorid (13.3 mg, 119 µmol), HATU (98.1 mg, 258 µmol) und Triethylamin (55 µl, 400 µmol) zugegeben und für weitere 5 Stunden gerührt. Das Reaktionsgemisch wurde mit Wasser versetzt und im Vakuum eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 15.4 mg (21 % d. Th.) der Titelverbindung erhalten.
LC-MS (Methode 4): Rₜ = 0.52 min; MS (ESIpos): m/z = 352 [M+H]⁺
¹H-NMR (400 MHz, DMSO-d6) δ [ppm]: -0.149 (0.73), -0.008 (6.62), 0.008 (5.33), 0.146 (0.69), 2.328 (0.95), 2.367 (0.90), 2.390 (0.90), 2.422 (4.13), 2.453 (1.25), 2.524 (2.58), 2.670 (1.38), 2.675 (1.55), 2.690 (1.68), 2.723 (12.26), 3.724 (0.60), 3.929 (1.25), 3.962 (1.55), 3.995 (1.25), 4.024 (1.42), 4.193 (0.69), 4.207 (0.77), 4.225 (1.20), 4.253 (1.72), 4.277 (2.02), 4.293 (1.33), 4.310 (1.46), 4.321 (1.25), 4.343 (0.86), 4.429 (0.60), 4.455 (1.12), 4.489 (1.29), 4.514 (1.55), 4.544 (1.03), 4.557 (1.12), 4.582 (0.39), 4.664 (0.65), 4.679 (0.77), 4.695 (0.77), 4.719 (3.23), 4.731 (4.39), 4.757 (0.69), 4.834 (0.65), 4.877 (16.00), 4.917 (0.77), 5.093 (0.60), 5.343 (0.82), 5.350 (0.99), 5.358 (0.77), 5.374 (0.56), 5.390 (0.77), 5.398 (0.95), 5.486 (0.82), 5.493 (0.99), 5.500 (0.82), 5.532 (0.77), 5.540 (0.90), 7.495 (1.08), 7.506 (6.37), 7.516 (1.55), 7.526 (7.87), 7.720 (4.22), 7.741 (3.61), 8.326 (6.06).

### Beispiel 170

### (5RS)-5-[(3-Fluorazetidin-1-yl)carbonyl]-2-{[6-(trifluormethyl)pyridin-3-yl]methyl}-2,5,6,7-tetrahydro-3H-pyrrolo[2,1-c][1,2,4]triazol-3-on (Racemat)

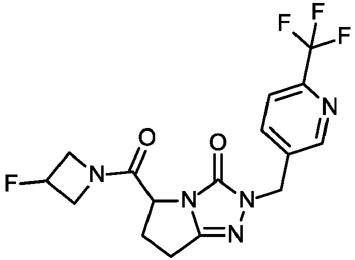

(5S)-3-Oxo-2-{[6-(trifluormethyl)pyridin-3-yl]methyl}-2,5,6,7-tetrahydro-3H-pyrrolo[2,1 - c][1,2,4]triazol-5-carbonsäure (75.0 mg, 228 µmol) wurde in THF (2.4 ml) bei Raumtemperatur vorgelegt. Anschließend wurden 3-Fluorazetidinhydrochlorid (30.6 mg, 274 µmol), HATU (113 mg, 297 µmol) und Triethylamin (190 µl, 1.4 mmol) zugegeben. Das Reaktionsgemisch wurde für 20 Stunden bei Raumtemperatur gerührt. Das Reaktionsgemisch wurde mit Wasser versetzt und im Vakuum eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 5.30 mg (6 % d. Th.) der Titelverbindung erhalten.
LC-MS (Methode 3): Rₜ = 1.12 min; MS (ESIpos): m/z = 386 [M+H]⁺
¹H-NMR (400 MHz, DMSO-d6) δ [ppm]: -0.008 (3.13), 0.008 (3.01), 2.328 (0.70), 2.366 (0.52), 2.405 (0.67), 2.432 (3.56), 2.464 (1.22), 2.523 (2.04), 2.670 (0.85), 2.734 (10.77), 3.729 (1.73), 3.938 (1.10), 3.966 (1.37), 3.999 (1.13), 4.028 (1.28), 4.200 (0.64), 4.215 (0.67), 4.230 (1.03), 4.257 (1.52), 4.284 (1.83), 4.296 (1.28), 4.314 (1.16), 4.326 (1.19), 4.348 (0.73), 4.434 (0.46), 4.459 (0.94), 4.494 (1.13), 4.518 (1.37), 4.547 (0.82), 4.561 (0.79), 4.669 (0.55), 4.684 (0.64), 4.746 (3.95), 4.960 (0.43), 5.003 (13.17), 5.043 (0.46), 5.352 (0.88), 5.392 (0.70), 5.400 (0.85), 5.495 (0.88), 5.503 (0.70), 5.543 (0.82), 5.754 (16.00), 7.899 (2.74), 7.919 (9.79), 7.934 (6.11), 7.955 (1.83), 8.674 (6.48).

### Beispiel 171

### (5RS)-2-(3-Chlor-4-fluorbenzyl)-5-[(3-fluorazetidin-1-yl)carbonyl]-2,5,6,7-tetrahydro-3H-pyrrolo[2,1-c][1,2,4]triazol-3-on (Racemat)

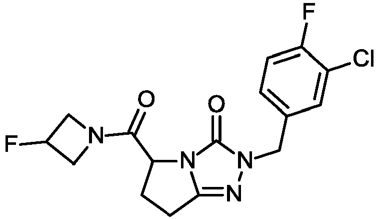

(5S)-2-(3-Chlor-4-fluorbenzyl)-3-oxo-2,5,6,7-tetrahydro-3H-pyrrolo[2,1-c][1,2,4]triazol-5-carbonsäure (75.0 mg, 87 % Reinheit, 209 µmol) wurde in THF (2.2 ml) bei Raumtemperatur vorgelegt. Anschließend wurden 3-Fluorazetidinhydrochlorid (28.0 mg, 251 µmol), HATU (103 mg, 272 µmol) und Triethylamin (180 µl, 1.3 mmol) zugegeben. Das Reaktionsgemisch wurde über das Wochenende bei Raumtemperatur gerührt. Es wurden erneut 3-Fluorazetidinhydrochlorid (14.0 mg, 126 µmol), HATU (103 mg, 272 µmol) und Triethylamin (180 µl, 1.3 mmol) zugegeben und bei 70°C für 4 Stunden gerührt. Das Reaktionsgemisch wurde mit Wasser versetzt und im Vakuum eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 31.2 mg (40 % d. Th.) der Titelverbindung erhalten.
LC-MS (Methode 3): Rₜ = 1.34 min; MS (ESIpos): m/z = 369 [M+H]⁺
¹H-NMR (400 MHz, DMSO-d6) δ [ppm]: -0.149 (1.75), -0.008 (16.00), 0.146 (1.81), 2.328 (1.81), 2.366 (1.17), 2.423 (4.50), 2.454 (1.58), 2.670 (1.99), 2.731 (14.31), 3.935 (1.46), 3.965 (1.87), 3.996 (1.46), 4.023 (1.87), 4.227 (1.46), 4.254 (2.10), 4.281 (2.57), 4.297 (1.69), 4.309 (1.75), 4.430 (0.70), 4.455 (1.34), 4.487 (1.58), 4.516 (1.81), 4.543 (1.11), 4.557 (1.05), 4.737 (4.96), 4.775 (1.40), 4.816 (12.67), 4.862 (0.93), 5.351 (1.23), 5.398 (1.11), 5.494 (1.11), 5.540 (1.05), 5.753 (1.69), 7.273 (3.62), 7.374 (5.26), 7.397 (7.82), 7.419 (3.68), 7.453 (4.67), 7.470 (4.67).

### Beispiel 172

### (5RS)-2-[(6-Chlorpyridin-3-yl)methyl]-5-{[(3R,4S)-3,4-difluorpyrrolidin-1-yl]carbonyl}-2,5,6,7-tetrahydro-3H-pyrrolo[2,1-c][1,2,4]triazol-3-on (Racemat)

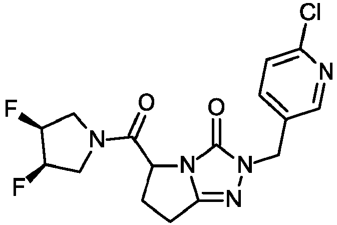

(5S)-2-[(6-Chlorpyridin-3-yl)methyl]-3-oxo-2,5,6,7-tetrahydro-3H-pyrrolo[2,1-c][1,2,4]triazol-5-carbonsäure (75.0 mg, 78 % Reinheit, 199 µmol) wurde in THF (2.0 ml) bei Raumtemperatur vorgelegt. Anschließend wurden (3R,4S)-3,4-Difluorpyrrolidinhydrochlorid (34.2 mg, 238 µmol), HATU (98.1 mg, 258 µmol) und Triethylamin (170 µl, 1.2 mmol) zugegeben. Das Reaktionsgemisch wurde für 20 Stunden bei Raumtemperatur gerührt. Es wurden erneut HATU (98.1 mg, 258 µmol), Triethylamin (55 µl, 400 µmol) und (3R,4S)-3,4-Difluorpyrrolidinhydrochlorid (17.1 mg, 119 µmol) zugegeben und über Nacht bei Raumtemperatur gerührt. Das Reaktionsgemisch wurde mit Wasser versetzt und im Vakuum eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 45.7 mg (59 % d. Th.) der Titelverbindung erhalten.
LC-MS (Methode 3): Rₜ = 1.01 min; MS (ESIpos): m/z = 384 [M+H]⁺
¹H-NMR (400 MHz, DMSO-d6) δ [ppm]: 2.327 (0.78), 2.350 (0.62), 2.358 (0.83), 2.366 (1.14), 2.384 (1.04), 2.401 (1.27), 2.409 (1.25), 2.418 (1.33), 2.447 (1.17), 2.633 (0.44), 2.674 (1.90), 2.694 (2.26), 2.706 (1.74), 2.722 (2.89), 2.728 (4.29), 2.748 (5.23), 2.783 (2.50), 2.806 (2.06), 2.818 (1.01), 2.828 (1.61), 2.839 (1.27), 2.852 (0.60), 2.862 (0.96), 3.478 (1.04), 3.487 (0.99), 3.512 (1.46), 3.521 (1.59), 3.532 (1.30), 3.567 (1.27), 3.652 (1.35), 3.666 (1.72), 3.687 (1.67), 3.700 (1.87), 3.722 (1.51), 3.734 (0.83), 3.752 (1.27), 3.763 (0.88), 3.784 (0.62), 3.797 (0.62), 3.849 (0.47), 3.862 (0.52), 3.877 (0.94), 3.892 (1.17), 3.926 (1.67), 3.940 (1.40), 3.966 (0.88), 4.181 (0.68), 4.196 (0.78), 4.209 (0.70), 4.223 (1.27), 4.237 (0.81), 4.249 (0.73), 4.265 (0.68), 4.885 (16.00), 4.961 (1.80), 4.968 (1.93), 4.983 (3.69), 4.990 (3.17), 5.004 (2.24), 5.094 (0.60), 5.262 (1.04), 5.274 (1.07), 5.284 (1.04), 5.322 (0.96), 5.344 (0.81), 5.356 (0.86), 5.367 (0.86), 5.383 (0.91), 5.394 (0.96), 5.407 (1.09), 5.420 (1.01), 5.430 (0.99), 5.450 (0.94), 5.458 (0.78), 5.473 (0.86), 5.486 (0.88), 7.511 (5.36), 7.531 (6.56), 7.715 (2.39), 7.720 (4.14), 7.726 (2.60), 7.741 (3.54), 7.746 (2.13), 8.324 (5.18).

### Beispiel 173

### (5RS)-5-{[(3R,4S)-3,4-Difluorpyrrolidin-1-yl]carbonyl}-2-{[6-(trifluormethyl)pyridin-3-yl]methyl}-2,5,6,7-tetrahydro-3H-pyrrolo[2,1-c][1,2,4]triazol-3-on (Racemat)

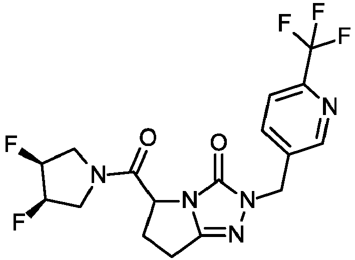

(5S)-3-Oxo-2-{[6-(trifluormethyl)pyridin-3-yl]methyl}-2,5,6,7-tetrahydro-3H-pyrrolo[2,1-c][1,2,4]triazol-5-carbonsäure (75.0 mg, 228 µmol) wurde in THF (2.4 ml) bei Raumtemperatur vorgelegt. Anschließend wurden (3R,4S)-3,4-Difluorpyrrolidinhydrochlorid (39.4 mg, 274 µmol), HATU (113 mg, 297 µmol) und Triethylamin (190 µl, 1.4 mmol) zugegeben. Das Reaktionsgemisch wurde über Nacht bei Raumtemperatur gerührt. Das Reaktionsgemisch wurde mit Wasser versetzt und im Vakuum eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Die produkthaltigen Fraktionen wurden im Vakuum eingeengt und es wurden 80.3 mg (84 % d. Th.) der Titelverbindung erhalten.
LC-MS (Methode 3): Rₜ = 1.24 min; MS (ESIpos): m/z = 418 [M+H]⁺
¹H-NMR (400 MHz, DMSO-d6) δ [ppm]: -0.149 (0.65), -0.008 (6.58), 0.008 (5.20), 0.146 (0.66), 2.327 (0.78), 2.366 (0.98), 2.428 (0.72), 2.457 (0.73), 2.670 (0.88), 2.682 (0.76), 2.703 (1.20), 2.710 (0.90), 2.715 (0.88), 2.731 (1.59), 2.738 (2.23), 2.758 (2.66), 2.794 (1.35), 2.816 (1.01), 2.838 (0.82), 2.850 (0.69), 2.872 (0.50), 3.364 (16.00), 3.482 (0.98), 3.517 (0.94), 3.525 (0.95), 3.572 (0.75), 3.656 (0.69), 3.670 (0.91), 3.692 (0.87), 3.705 (1.00), 3.726 (0.82), 3.745 (0.44), 3.756 (0.65), 3.768 (0.51), 3.882 (0.50), 3.896 (0.60), 3.930 (0.79), 3.944 (0.72), 3.971 (0.44), 4.199 (0.44), 4.227 (0.66), 4.240 (0.41), 4.975 (0.98), 4.982 (1.03), 4.997 (2.25), 5.012 (8.25), 5.264 (0.53), 5.286 (0.56), 5.324 (0.51), 5.369 (0.44), 5.387 (0.44), 5.409 (0.54), 5.489 (0.48), 7.904 (1.09), 7.924 (4.96), 7.935 (3.27), 7.956 (0.75), 8.672 (3.24).

### Beispiel 174

### (5RS)-2-(3-Chlor-4-fluorbenzyl)-5-{[(3R,4S)-3,4-difluorpyrrolidin-1-yl]carbonyl}-2,5,6,7-tetrahydro-3H-pyrrolo[2,1-c][1,2,4]triazol-3-on (Racemat)

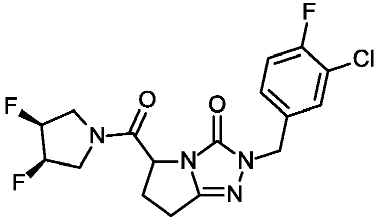

(5S)-2-(3-Chlor-4-fluorbenzyl)-3-oxo-2,5,6,7-tetrahydro-3H-pyrrolo[2,1-c][1,2,4]triazol-5-carbonsäure (75.0 mg, 87 % Reinheit, 209 µmol) wurde in THF (2.2 ml) bei Raumtemperatur vorgelegt. Anschließend wurden (3R,4S)-3,4-Difluorpyrrolidinhydrochlorid (36.1 mg, 251 µmol), HATU (103 mg, 272 µmol) und Triethylamin (180 µl, 1.3 mmol) zugegeben. Das Reaktionsgemisch wurde über Nacht bei Raumtemperatur gerührt. Das Reaktionsgemisch wurde mit Wasser versetzt und im Vakuum eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Die produkthaltigen Fraktionen wurden im Vakuum eingeengt und es wurden 62.9 mg (74 % d. Th.) der Titelverbindung erhalten.
LC-MS (Methode 3): Rₜ = 1.40 min; MS (ESIpos): m/z = 401 [M+H]⁺
¹H-NMR (400 MHz, DMSO-d6) δ [ppm]: -0.150 (0.49), -0.008 (4.42), 0.008 (3.97), 0.146 (0.49), 2.073 (1.28), 2.328 (0.88), 2.351 (0.74), 2.359 (1.01), 2.366 (1.55), 2.378 (1.10), 2.384 (1.24), 2.396 (1.31), 2.402 (1.46), 2.410 (1.42), 2.418 (1.49), 2.435 (0.81), 2.446 (1.28), 2.455 (0.79), 2.524 (2.73), 2.642 (0.58), 2.664 (1.38), 2.670 (1.24), 2.683 (1.76), 2.703 (2.73), 2.713 (2.19), 2.730 (3.31), 2.736 (5.21), 2.745 (3.81), 2.755 (6.60), 2.766 (1.38), 2.773 (1.40), 2.789 (3.58), 2.803 (1.13), 2.810 (2.52), 2.822 (1.20), 2.832 (1.94), 2.844 (1.62), 2.856 (0.74), 2.866 (1.24), 3.480 (1.28), 3.490 (1.29), 3.514 (1.73), 3.523 (1.89), 3.532 (1.56), 3.570 (1.60), 3.655 (1.51), 3.668 (2.03), 3.689 (2.10), 3.703 (2.28), 3.711 (1.82), 3.727 (4.12), 3.738 (1.04), 3.744 (1.01), 3.755 (1.49), 3.766 (1.10), 3.787 (0.79), 3.801 (0.76), 3.851 (0.59), 3.866 (0.68), 3.880 (1.19), 3.894 (1.42), 3.929 (2.01), 3.943 (1.51), 3.968 (1.08), 4.184 (0.83), 4.198 (1.01), 4.212 (0.84), 4.225 (1.55), 4.239 (0.95), 4.252 (0.84), 4.267 (0.77), 4.785 (0.79), 4.826 (16.00), 4.868 (0.67), 4.969 (2.05), 4.977 (2.23), 4.991 (4.24), 4.998 (3.85), 5.011 (2.70), 5.018 (1.64), 5.252 (0.76), 5.261 (1.19), 5.274 (1.24), 5.284 (1.20), 5.323 (1.24), 5.332 (0.97), 5.346 (0.99), 5.355 (1.01), 5.368 (1.06), 5.384 (1.11), 5.395 (1.13), 5.408 (1.28), 5.422 (1.17), 5.445 (0.99), 5.451 (1.11), 5.461 (0.92), 5.475 (1.06), 5.489 (0.99), 5.498 (0.68), 7.247 (1.13), 7.252 (2.10), 7.258 (2.23), 7.264 (2.55), 7.269 (2.79), 7.274 (3.33), 7.280 (3.02), 7.286 (2.98), 7.291 (1.74), 7.380 (4.01), 7.402 (6.63), 7.425 (2.97), 7.455 (3.85), 7.473 (3.87), 8.385 (0.41).

### Beispiel 175

### (5RS)-5-{[(3R,4S)-3,4-Difluorpyrrolidin-1-yl]carbonyl}-2-{[3-fluor-2-(trifluormethyl)pyridin-4-yl]methyl}-2,5,6,7-tetrahydro-3H-pyrrolo[2,1-c][1,2,4]triazol-3-on (Racemat)

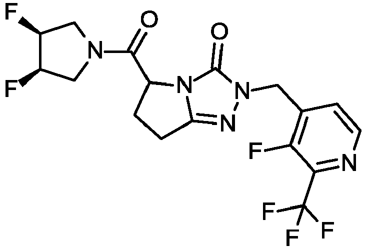

(5S)-2-{[3-Fluor-2-(trifluormethyl)pyridin-4-yl]methyl}-3-oxo-2,5,6,7-tetrahydro-3H-pyrrolo[2,1-c][1,2,4]triazol-5-carbonsäure (66.0 mg, 183 µmol) wurde in THF (1.9 ml) bei Raumtemperatur vorgelegt. Anschließend wurden (3R,4S)-3,4-Difluorpyrrolidinhydrochlorid (31.5 mg, 220 µmol), HATU (90.5 mg, 238 µmol) und Triethylamin (150 µl, 1.1 mmol) zugegeben. Das Reaktionsgemisch wurde über Nacht bei Raumtemperatur gerührt. Es wurden erneut HATU (90.5 mg, 238 µmol), Triethylamin (150 µl, 1.1 mmol) und (3R,4S)-3,4-Difluorpyrrolidinhydrochlorid (52.5 mg, 366 µmol) zugegeben und über Nacht bei Raumtemperatur gerührt. Das Reaktionsgemisch wurde mit Wasser versetzt und im Vakuum eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Die produkthaltigen Fraktionen wurden im Vakuum eingeengt und es wurden 53.0 mg (63 % d. Th.) der Titelverbindung erhalten.
LC-MS (Methode 4): Rₜ = 0.71 min; MS (ESIpos): m/z = 436 [M+H]⁺
¹H-NMR (400 MHz, DMSO-d6) δ [ppm]: -0.149 (1.77), -0.008 (15.84), 0.008 (16.00), 0.146 (1.77), 0.854 (1.39), 1.236 (6.21), 1.410 (2.19), 2.327 (2.41), 2.366 (2.62), 2.438 (1.87), 2.670 (2.94), 2.700 (2.09), 2.710 (2.78), 2.719 (3.10), 2.732 (2.62), 2.755 (5.83), 2.775 (6.85), 2.808 (4.76), 2.827 (2.57), 2.850 (2.03), 2.862 (1.93), 2.884 (1.50), 3.487 (1.50), 3.530 (1.98), 3.578 (2.30), 3.675 (2.35), 3.695 (2.52), 3.707 (2.68), 3.730 (2.09), 3.772 (1.55), 3.793 (0.96), 3.805 (0.96), 3.886 (1.44), 3.900 (1.61), 3.936 (2.30), 3.973 (1.34), 4.198 (1.28), 4.225 (1.93), 4.239 (1.28), 4.991 (2.41), 4.999 (2.78), 5.014 (5.24), 5.020 (5.83), 5.035 (3.05), 5.062 (11.99), 5.073 (10.70), 5.113 (1.28), 5.288 (1.50), 5.388 (1.55), 7.607 (3.80), 7.620 (7.44), 7.632 (4.12), 8.573 (8.51), 8.585 (8.56), 10.193 (1.07).

### Beispiel 176

### (5RS)-5-{[(3R,4S)-3,4-Difluorpyrrolidin-1-yl]carbonyl}-2-{[3-fluor-2-(trifluormethyl)pyridin-4-yl]methyl}-2,5,6,7-tetrahydro-3H-pyrrolo[2,1-c][1,2,4]triazol-3-on (Enantiomer 1)

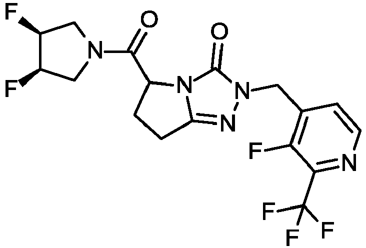

(5RS)-5-{[(3R,4S)-3,4-Difluorpyrrolidin-1-yl]carbonyl}-2-{[3-fluor-2-(trifluormethyl)pyridin-4-yl]methyl}-2,5,6,7-tetrahydro-3H-pyrrolo[2,1-c][1,2,4]triazol-3-on (Racemat) wurden durch chirale präparative HPLC getrennt [Probenvorbereitung: 53 mg gelöst in 3 ml Acetonitril und 1 ml Ethanol (warm); Injektionsvolumen: 0.25 ml; Säule: Daicel Chiralpak^{®} AS-H 5 µm, 250 x 20 mm; Laufmittel: n-Heptan/Ethanol 50:50; Fluss: 15 ml/min; Temperatur 25°C; UVDetektion: 210 nm]. Nach der Trennung, wurden 9.2 mg von Enantiomer 1, welches zuerst eluiert, und 12.4 mg von Enantiomer 2, welches später eluiert, isoliert.

### Enantiomer 1:

Analytische chirale HPLC: Rₜ = 2.22 min, e.e. = 99% [Säule: Daicel Chiraltek^{®} AS, 50 × 4.6 mm; Laufmittel: i-Hexan/Ethanol 50:50; Fluss: 1 ml/min; UV-Detektion: 220 nm].
LC-MS (Methode 4): Rₜ = 0.70 min; MS (ESIpos): m/z = 436 [M+H]⁺
¹H-NMR (400 MHz, DMSO-d6) δ [ppm]: -0.149 (7.43), -0.059 (1.78), -0.045 (2.59), -0.029 (4.69), 0.019 (5.82), 0.146 (7.43), 1.238 (2.26), 2.328 (8.08), 2.365 (8.40), 2.425 (4.04), 2.433 (4.36), 2.559 (4.53), 2.670 (7.92), 2.709 (7.11), 2.731 (2.75), 2.753 (7.11), 2.775 (8.08), 2.805 (5.01), 2.825 (2.91), 2.848 (2.26), 3.524 (2.59), 3.580 (2.75), 3.673 (3.39), 3.694 (3.07), 3.708 (3.23), 3.716 (3.07), 3.726 (2.26), 3.760 (2.10), 3.896 (1.94), 3.934 (2.75), 3.980 (1.78), 4.224 (2.10), 4.998 (3.72), 5.017 (7.11), 5.035 (3.88), 5.063 (16.00), 5.114 (2.10), 5.277 (2.26), 5.326 (1.94), 5.373 (2.10), 5.397 (2.26), 5.411 (2.26), 5.454 (1.94), 7.606 (4.69), 7.619 (9.05), 7.631 (4.85), 8.572 (9.70), 8.583 (9.05).

### Beispiel 177

### (5RS)-5-{[(3R,4S)-3,4-Difluorpyrrolidin-1-yl]carbonyl}-2-{[3-fluor-2-(trifluormethyl)pyridin-4-yl]methyl}-2,5,6,7-tetrahydro-3H-pyrrolo[2,1-c][1,2,4]triazol-3-on (Enantiomer 2)

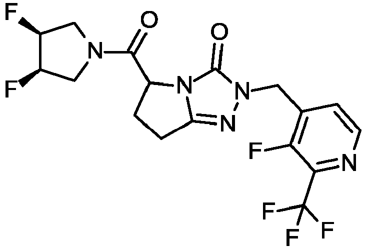

(5RS)-5-{[(3R,4S)-3,4-Difluorpyrrolidin-1-yl]carbonyl}-2-{[3-fluor-2-(trifluormethyl)pyridin-4-yl]methyl}-2,5,6,7-tetrahydro-3H-pyrrolo[2,1-c][1,2,4]triazol-3-on (Racemat) wurden durch chirale präparative HPLC getrennt [Probenvorbereitung: 53 mg gelöst in 3 ml Acetonitril und 1 ml Ethanol (warm); Injektionsvolumen: 0.25 ml; Säule: Daicel Chiralpak^{®} AS-H 5 µm, 250 x 20 mm; Laufmittel: n-Heptan/Ethanol 50:50; Fluss: 15 ml/min; Temperatur 25°C; UV-Detektion: 210 nm]. Nach der Trennung, wurden 9.2 mg von Enantiomer 1, welches zuerst eluiert, und 12.4 mg von Enantiomer 2, welches später eluiert, isoliert.

### Enantiomer 2:

Analytische chiral HPLC: Rₜ = 3.86 min, e.e. = 98% [column: Daicel Chiraltek^{®} AS, 50 x 4.6 mm; Laufmittel: i-Hexan/Ethanol 40:60; Fluss: 1 ml/min; UV detektion: 220 nm].
LC-MS (Methode 4): Rₜ = 0.71 min; MS (ESIpos): m/z = 436 [M+H]⁺
¹H-NMR (400 MHz, DMSO-d6) δ [ppm]: -0.149 (1.89), -0.027 (0.41), -0.025 (0.54), -0.022 (0.63), -0.019 (0.99), -0.016 (1.17), -0.015 (1.44), -0.008 (16.00), 0.008 (15.19), 0.014 (1.98), 0.016 (1.26), 0.019 (0.90), 0.021 (0.72), 0.024 (0.50), 0.030 (0.45), 0.146 (1.89), 2.327 (0.90), 2.332 (0.68), 2.366 (0.95), 2.523 (2.52), 2.560 (0.72), 2.669 (1.04), 2.710 (0.99), 2.718 (0.41), 2.755 (0.72), 2.775 (0.90), 2.808 (0.63), 5.021 (0.77), 5.036 (0.41), 5.063 (1.58), 5.072 (1.35), 7.607 (0.50), 7.620 (0.95), 7.632 (0.50), 8.572 (1.08), 8.585 (1.04).

### Beispiel 178

### (5RS)-5-{[(3S)-3-Fluorpyrrolidin-1-yl]carbonyl}-2-{[3-fluor-2-(trifluormethyl)pyridin-4-yl]methyl}-2,5,6,7-tetrahydro-3H-pyrrolo[2,1-c][1,2,4]triazol-3-on (Diastereomerengemisch; 2 Isomere)

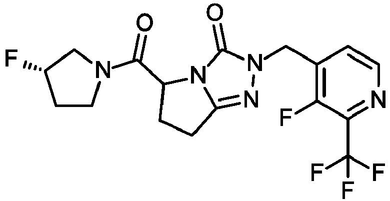

(5S)-2-{[3-Fluor-2-(trifluormethyl)pyridin-4-yl]methyl}-3-oxo-2,5,6,7-tetrahydro-3H-pyrrolo[2,1 - c][1,2,4]triazol-5-carbonsäure (66.0 mg, 182 µmol) wurde in THF (1.9 ml) bei Raumtemperatur vorgelegt. Anschließend wurden (3S)-3-Fluorpyrrolidinhydrochlorid (27.5 mg, 219 µmol), HATU (90.2 mg, 237 µmol) und Triethylamin (150 µl, 1.1 mmol) zugegeben. Das Reaktionsgemisch wurde über Nacht bei Raumtemperatur gerührt. Es wurden erneut (3S)-3-Fluorpyrrolidinhydrochlorid (13.7 mg, 109 µmol), HATU (90.2 mg, 237 µmol) und Triethylamin (150 µl, 1.1 mmol) zugegeben und über Nacht bei Raumtemperatur gerührt. Das Reaktionsgemisch wurde mit Wasser versetzt und im Vakuum eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 60.9 mg (78 % d. Th.) der Titelverbindung erhalten.
LC-MS (Methode 4): Rₜ = 0.69 min; MS (ESIpos): m/z = 418 [M+H]⁺
¹H-NMR (400 MHz, DMSO-d6) δ [ppm]: -0.149 (0.93), 0.008 (7.50), 0.146 (0.99), 2.121 (2.67), 2.267 (2.36), 2.327 (3.16), 2.367 (3.29), 2.670 (2.60), 2.698 (2.11), 2.716 (2.91), 2.738 (6.08), 2.745 (5.95), 2.758 (9.67), 2.779 (6.82), 2.815 (2.48), 2.837 (2.36), 2.859 (2.17), 2.868 (2.85), 2.882 (2.29), 3.352 (2.29), 3.376 (1.43), 3.437 (1.18), 3.500 (1.80), 3.519 (2.17), 3.609 (3.04), 3.634 (4.16), 3.654 (2.60), 3.679 (3.47), 3.691 (3.53), 3.732 (3.04), 3.798 (1.18), 3.843 (0.99), 3.938 (1.05), 3.970 (1.49), 3.991 (1.61), 4.014 (0.93), 4.080 (0.81), 4.701 (1.12), 4.715 (0.99), 4.902 (2.05), 4.909 (2.11), 4.924 (2.29), 4.931 (1.80), 4.970 (2.42), 4.977 (2.60), 4.992 (2.79), 5.020 (2.11), 5.061 (15.01), 5.070 (16.00), 5.113 (1.55), 5.281 (2.42), 5.378 (1.74), 5.414 (2.42), 5.500 (1.55), 5.730 (0.68), 7.610 (5.40), 7.622 (9.67), 7.635 (5.21), 8.573 (11.22), 8.585 (10.60).

### Beispiel 179

### (5RS)-5-{[(3S)-3-Fluorpyrrolidin-1-yl]carbonyl}-2-{[3-fluor-2-(trifluormethyl)pyridin-4-yl]methyl}-2,5,6,7-tetrahydro-3H-pyrrolo[2,1-c][1,2,4]triazol-3-on (Isomer 1)

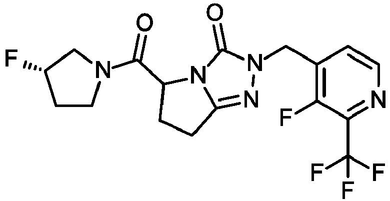

(5RS)-5-{[(3S)-3-Fluorpyrrolidin-1 -yl]carbonyl}-2-{[3-fluor-2-(trifluormethyl)pyridin-4-yl]methyl}-2,5,6,7-tetrahydro-3H-pyrrolo[2,1-c][1,2,4]triazol-3-on (Diastereomerengemisch; 2 Isomere) wurden durch chirale präparative HPLC getrennt [Probenvorbereitung: 60 mg gelöst in 3 ml Acetonitril und 1 ml Ethanol; Injektionsvolumen: 0.25 ml; Säule: Daicel Chiralpak^{®} AS-H 5 µm, 250 × 20 mm; Laufmittel: n-Heptan/Ethanol 50:50; Fluss: 15 ml/min; Temperatur 25°C; UV-Detektion: 210 nm]. Nach der Trennung, wurden 10.7 mg von Isomer 1, welches zuerst eluiert, und 15.6 mg von Isomer 2, welches später eluiert, isoliert.

### Isomer 1:

Analytische chirale HPLC: Rₜ = 2.20 min, d.e. = 99% [Säule: Daicel Chiraltek^{®} AS, 50 × 4.6 mm; Laufmittel: i-Hexan/Ethanol 50:50; Fluss: 1 ml/min; UV-Detektion: 220 nm].
LC-MS (Methode 4): Rₜ = 0.68 min; MS (ESIpos): m/z = 418 [M+H]⁺
¹H-NMR (400 MHz, DMSO-d6) δ [ppm]: -0.149 (1.73), -0.008 (15.96), 0.008 (16.00), 0.146 (1.78), 1.100 (0.61), 1.118 (0.61), 1.235 (0.65), 2.104 (0.74), 2.155 (0.56), 2.257 (0.69), 2.327 (1.26), 2.366 (1.47), 2.396 (0.91), 2.417 (1.13), 2.441 (0.65), 2.523 (3.77), 2.665 (1.04), 2.670 (1.39), 2.698 (0.65), 2.710 (1.26), 2.746 (2.12), 2.758 (3.25), 2.764 (2.25), 2.775 (2.60), 2.792 (1.30), 2.814 (0.95), 2.844 (0.78), 2.866 (0.56), 3.345 (0.65), 3.356 (0.82), 3.375 (0.78), 3.386 (0.52), 3.403 (0.43), 3.477 (0.56), 3.502 (0.78), 3.520 (1.21), 3.545 (0.43), 3.572 (0.87), 3.586 (0.74), 3.609 (1.86), 3.635 (1.73), 3.700 (0.39), 3.722 (0.48), 3.969 (0.56), 3.991 (1.04), 4.017 (0.69), 4.058 (0.48), 4.083 (0.52), 4.111 (0.43), 5.023 (0.52), 5.071 (5.46), 5.116 (0.43), 5.278 (0.74), 5.367 (0.61), 5.412 (0.74), 5.498 (0.69), 7.610 (1.21), 7.623 (2.38), 7.635 (1.30), 8.574 (2.95), 8.586 (2.99).

### Beispiel 180

### (5RS)-5-{[(3S)-3-Fluorpyrrolidin-1-yl]carbonyl}-2-{[3-fluor-2-(trifluormethyl)pyridin-4-yl]methyl}-2,5,6,7-tetrahydro-3H-pyrrolo[2,1-c][1,2,4]triazol-3-on (Isomer 2)

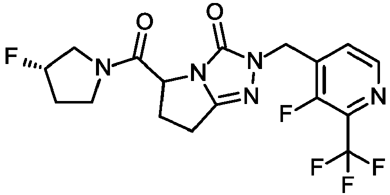

(5RS)-5-{[(3S)-3-Fluorpyrrolidin-1 -yl]carbonyl}-2-{[3-fluor-2-(trifluormethyl)pyridin-4-yl]methyl}-2,5,6,7-tetrahydro-3H-pyrrolo[2,1-c][1,2,4]triazol-3-on (Diastereomerengemisch; 2 Isomere) wurden durch chirale präparative HPLC getrennt [Probenvorbereitung: 60 mg gelöst in 3 ml Acetonitril und 1 ml Ethanol; Injektionsvolumen: 0.25 ml; Säule: Daicel Chiralpak^{®} AS-H 5 µm, 250 x 20 mm; Laufmittel: n-Heptan/Ethanol 50:50; Fluss: 15 ml/min; Temperatur 25°C; UV-Detektion: 210 nm]. Nach der Trennung, wurden 10.7 mg von Isomer 1, welches zuerst eluiert, und 15.6 mg von Isomer 2, welches später eluiert, isoliert.

### Isomer 2:

Analytische chirale HPLC: Rₜ = 3.51 min, d.e. = 97.4% [Säule: Daicel Chiraltek^{®} AS, 50 × 4.6 mm; Laufmittel: i-Hexan/Ethanol 50:50; Fluss: 1 ml/min; UV-Detektion: 220 nm].
LC-MS (Methode 4): Rₜ = 0.67 min; MS (ESIpos): m/z = 418 [M+H]⁺
¹H-NMR (400 MHz, DMSO-d6) δ [ppm]: -0.149 (1.64), -0.008 (16.00), 0.008 (14.04), 0.146 (1.71), 0.858 (0.44), 1.100 (0.65), 1.118 (0.58), 1.236 (0.91), 1.984 (0.40), 2.010 (0.44), 2.118 (1.20), 2.146 (1.16), 2.215 (0.87), 2.237 (0.98), 2.266 (1.13), 2.327 (1.78), 2.337 (1.13), 2.344 (0.80), 2.354 (0.87), 2.366 (1.53), 2.410 (0.87), 2.433 (1.16), 2.441 (1.31), 2.451 (0.87), 2.463 (1.05), 2.523 (4.33), 2.670 (1.38), 2.674 (1.35), 2.697 (0.95), 2.710 (1.49), 2.716 (1.60), 2.738 (3.67), 2.748 (2.15), 2.759 (4.51), 2.771 (2.80), 2.779 (4.98), 2.818 (1.53), 2.828 (0.84), 2.838 (1.45), 2.847 (1.60), 2.859 (1.82), 2.869 (2.07), 2.882 (1.53), 2.891 (1.31), 2.904 (0.95), 3.333 (2.25), 3.352 (1.56), 3.361 (1.02), 3.379 (0.84), 3.393 (0.80), 3.401 (0.87), 3.428 (0.98), 3.436 (1.05), 3.499 (0.76), 3.525 (1.05), 3.534 (0.91), 3.630 (2.04), 3.655 (2.00), 3.679 (2.73), 3.690 (2.69), 3.706 (2.40), 3.716 (1.78), 3.732 (2.15), 3.739 (1.78), 3.769 (0.80), 3.800 (1.05), 3.844 (0.87), 3.866 (0.55), 3.875 (0.55), 3.931 (0.76), 3.939 (0.84), 3.962 (0.58), 4.902 (1.60), 4.909 (1.75), 4.924 (1.82), 4.931 (1.56), 4.970 (1.85), 4.977 (2.04), 4.992 (2.25), 4.998 (1.82), 5.018 (0.95), 5.061 (7.31), 5.071 (7.53), 5.113 (0.98), 5.282 (1.38), 5.377 (1.09), 5.415 (1.35), 5.510 (1.02), 7.609 (2.69), 7.622 (5.16), 7.635 (2.95), 8.573 (5.60), 8.585 (5.67).

### Beispiel 181

### (5RS)-5-[(3,3-Difluorpyrrolidin-1-yl)carbonyl]-2-{[3-fluor-2-(trifluormethyl)pyridin-4-yl]methyl}-2,5,6,7-tetrahydro-3H-pyrrolo[2,1-c][1,2,4]triazol-3-on (Racemat)

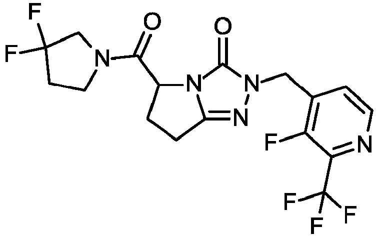

(5S)-2-{[3-Fluor-2-(trifluormethyl)pyridin-4-yl]methyl}-3-oxo-2,5,6,7-tetrahydro-3H-pyrrolo[2,1 - c][1,2,4]triazol-5-carbonsäure (66.0 mg, 182 µmol) wurde in THF (1.9 ml) bei Raumtemperatur vorgelegt. Anschließend wurden 3,3-Difluorpyrrolidinhydrochlorid (31.4 mg, 219 µmol), HATU (90.2 mg, 237 µmol) und Triethylamin (150 µl, 1.1 mmol) zugegeben. Das Reaktionsgemisch wurde über Nacht bei Raumtemperatur gerührt. Es wurden erneut 3,3-Difluorpyrrolidinhydrochlorid (15.7 mg, 109 µmol), HATU (90.2 mg, 237 µmol) und Triethylamin (150 µl, 1.1 mmol) zugegeben und über Nacht bei Raumtemperatur gerührt. Das Reaktionsgemisch wurde mit Wasser versetzt und im Vakuum eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 38.9 mg (49 % d. Th.) der Titelverbindung erhalten.
LC-MS (Methode 4): Rₜ = 0.75 min; MS (ESIpos): m/z = 436 [M+H]⁺
¹H-NMR (400 MHz, DMSO-d6) δ [ppm]: -0.149 (0.93), -0.008 (7.61), 0.008 (6.67), 0.146 (0.86), 1.410 (1.15), 2.328 (1.87), 2.366 (2.22), 2.392 (1.51), 2.411 (2.51), 2.432 (4.52), 2.440 (3.30), 2.451 (3.66), 2.461 (3.95), 2.469 (3.09), 2.524 (5.96), 2.565 (3.01), 2.583 (1.94), 2.600 (1.08), 2.670 (2.30), 2.694 (1.00), 2.701 (1.08), 2.711 (3.52), 2.720 (2.22), 2.733 (2.44), 2.742 (4.52), 2.749 (3.01), 2.760 (4.66), 2.765 (5.24), 2.771 (5.67), 2.779 (3.52), 2.804 (3.37), 2.813 (3.23), 2.818 (2.94), 2.842 (2.51), 2.864 (1.58), 3.559 (4.09), 3.578 (6.82), 3.597 (3.66), 3.673 (0.65), 3.692 (1.08), 3.708 (3.66), 3.740 (3.73), 3.752 (1.94), 3.782 (3.16), 3.816 (2.15), 3.849 (0.50), 3.904 (0.86), 3.934 (1.72), 3.963 (2.22), 3.973 (1.87), 3.982 (2.51), 4.001 (2.08), 4.007 (2.08), 4.027 (1.00), 4.233 (0.86), 4.262 (1.87), 4.293 (1.79), 4.324 (0.65), 4.957 (2.15), 4.965 (2.22), 4.978 (2.58), 5.021 (1.65), 5.042 (2.73), 5.049 (2.37), 5.063 (16.00), 5.073 (14.28), 5.115 (1.72), 7.610 (2.58), 7.621 (5.09), 7.632 (2.80), 8.572 (8.18), 8.584 (8.18).

### Beispiel 182

### (5RS)-5-[(3-Fluorazetidin-1-yl)carbonyl]-2-{[3-fluor-2-(trifluormethyl)pyridin-4-yl]methyl}-2,5,6,7-tetrahydro-3H-pyrrolo[2,1-c][1,2,4]triazol-3-on (Racemat)

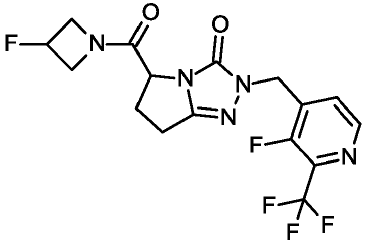

(5S)-2-{[3-Fluor-2-(trifluormethyl)pyridin-4-yl]methyl}-3-oxo-2,5,6,7-tetrahydro-3H-pyrrolo[2,1-c][1,2,4]triazol-5-carbonsäure (66.0 mg, 182 µmol) wurde in THF (1.9 ml) bei Raumtemperatur vorgelegt. Anschließend wurden 3-Fluorazetidinhydrochlorid (24.4 mg, 219 µmol), HATU (90.2 mg, 237 µmol) und Triethylamin (150 µl, 1.1 mmol) zugegeben. Das Reaktionsgemisch wurde über Nacht bei Raumtemperatur gerührt. Es wurden erneut 3-Fluorazetidinhydrochlorid (12.2 mg, 109 µmol), HATU (90.2 mg, 237 µmol) und Triethylamin (150 µl, 1.1 mmol) zugegeben und über Nacht bei Raumtemperatur gerührt. Das Reaktionsgemisch wurde mit Wasser versetzt und im Vakuum eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 39.0 mg (52 % d. Th.) der Titelverbindung erhalten.
LC-MS (Methode 4): Rₜ = 0.67 min; MS (ESIpos): m/z = 404 [M+H]⁺
¹H-NMR (400 MHz, DMSO-d6) δ [ppm]: 0.146 (0.43), 2.328 (1.09), 2.366 (0.75), 2.412 (1.14), 2.444 (4.98), 2.670 (1.11), 2.710 (1.84), 2.749 (16.00), 3.942 (1.73), 3.970 (2.16), 4.003 (1.75), 4.033 (2.11), 4.204 (0.91), 4.221 (1.02), 4.235 (1.70), 4.250 (1.86), 4.263 (2.36), 4.274 (2.30), 4.287 (2.57), 4.300 (2.39), 4.331 (1.86), 4.355 (1.09), 4.434 (0.70), 4.460 (1.45), 4.499 (1.61), 4.515 (2.30), 4.552 (1.16), 4.567 (1.16), 4.668 (0.77), 4.701 (1.64), 4.715 (2.16), 4.750 (4.27), 4.761 (5.57), 5.011 (1.55), 5.053 (11.95), 5.108 (1.18), 5.355 (1.34), 5.401 (1.20), 5.498 (1.27), 5.544 (1.23), 5.733 (0.75), 7.612 (4.61), 7.625 (8.73), 7.638 (4.84), 7.870 (0.66), 8.567 (9.82), 8.579 (9.68).

### Beispiel 183

### (5S)-5-{[(3R,4R)-3-Fluor-4-hydroxypyrrolidin-1 -yl]carbonyl}-2-(4-methylbenzyl)-5,6,7,8-tetrahydro[1,2,4]triazolo[4,3-a]pyridin-3(2H)-on

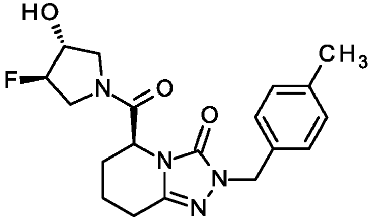

(5S)-2-(4-Methylbenzyl)-3-oxo-2,3,5,6,7,8-hexahydro[1,2,4]triazolo[4,3-a]pyridin-5-carbonsäure (100 mg, 327 µmol) wurde in THF (5.0 ml) bei Raumtemperatur vorgelegt. Anschließend wurden HBTU (161 mg, 425 µmol) und N,N-Diisopropylethylamin (280 µl, 1.6 mmol) zugegeben. Nach 15 min Rühren wurde (3R,4R)-4-Fluorpyrrolidin-3-olhydrochlorid (55.6 mg, 393 µmol) zugeben und das Reaktionsgemisch über Nacht bei Raumtemperatur gerührt. Das Reaktionsgemisch wurde im Vakuum eingeengt. Der Rückstand wurde über präparative HPLC aufgereinigt (Methode 10). Die produkthaltigen Fraktionen wurden im Vakuum eingeengt und es wurden 27.0 mg (22 % d. Th.) der Titelverbindung erhalten.
LC-MS (Methode 3): Rₜ = 1.19 min; MS (ESIpos): m/z = 375 [M+H]⁺
¹H-NMR (400 MHz, DMSO-d6) δ [ppm]: -0.008 (1.05), 0.008 (0.98), 1.721 (0.88), 1.913 (0.46), 1.950 (0.66), 2.040 (0.52), 2.047 (0.53), 2.056 (0.46), 2.273 (16.00), 2.518 (1.63), 2.522 (1.63), 2.562 (0.77), 2.585 (1.21), 2.628 (0.44), 3.350 (0.47), 3.381 (0.72), 3.457 (0.51), 3.474 (0.47), 3.531 (0.80), 3.557 (0.57), 3.601 (0.59), 3.651 (0.61), 3.690 (1.43), 3.757 (0.46), 4.230 (0.42), 4.338 (0.47), 4.748 (5.25), 4.794 (0.41), 4.809 (0.78), 4.818 (0.72), 4.825 (0.49), 4.903 (0.56), 5.032 (0.53), 5.571 (0.87), 5.580 (0.87), 5.595 (0.49), 5.603 (0.47), 5.662 (0.68), 5.671 (0.71), 5.703 (0.94), 5.712 (0.92), 7.101 (0.88), 7.124 (13.88), 7.147 (0.89).

### Beispiel 184

### (5S)-2-(Cyclopropylmethyl)-5-{[(3R,4S)-3,4-difluorpyrrolidin-1-yl]carbonyl}-5,6,7,8-tetrahydro[1,2,4]triazolo[4,3-a]pyridin-3(2H)-on

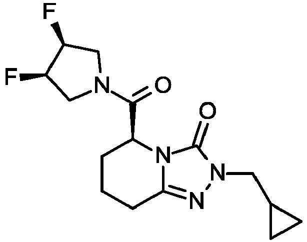

Unter Argon: (5S)-2-(Cyclopropylmethyl)-3-oxo-2,3,5,6,7,8-hexahydro[1,2,4]triazolo[4,3-a]pyridin-5-carbonsäure (115 mg, 460 µmol) wurde in THF (8.5 ml) bei Raumtemperatur vorgelegt. Anschließend wurden HATU (228 mg, 599 µmol) und N,N-Diisopropylethylamin (240 µl, 1.4 mmol) zugegeben. Nach 15 min Rühren wurde (3R,4S)-3,4-Difluorpyrrolidinhydrochlorid (79.3 mg, 553 µmol) zugeben und das Reaktionsgemisch über Nacht bei Raumtemperatur gerührt. Das Reaktionsgemisch wurde im Vakuum eingeengt und der Rückstand wurde mit Wasser und Acetonitril gelöst und über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 53.3 mg (35 % d. Th.) der Titelverbindung erhalten.
LC-MS (Methode 4): Rₜ = 0.56 min; MS (ESIpos): m/z = 327 [M+H]⁺
¹H-NMR (400 MHz, DMSO-d6) δ [ppm]: 0.261 (12.07), 0.267 (12.21), 0.273 (12.25), 0.437 (10.80), 0.455 (10.86), 1.058 (3.69), 1.715 (5.00), 1.724 (4.78), 1.901 (1.20), 1.936 (2.51), 1.975 (2.53), 1.996 (2.05), 2.005 (1.59), 2.023 (2.13), 2.031 (2.37), 2.040 (2.55), 2.047 (2.47), 2.057 (2.29), 2.067 (1.95), 2.074 (1.97), 2.082 (1.71), 2.328 (0.60), 2.351 (0.50), 2.366 (0.78), 2.567 (3.73), 2.578 (4.58), 2.602 (5.04), 2.612 (6.20), 2.625 (3.47), 2.655 (1.93), 2.669 (1.41), 2.709 (0.42), 3.418 (0.94), 3.454 (14.61), 3.471 (16.00), 3.497 (2.67), 3.510 (2.61), 3.521 (2.53), 3.531 (2.55), 3.540 (1.59), 3.554 (1.24), 3.563 (1.65), 3.604 (1.57), 3.617 (1.89), 3.637 (1.35), 3.651 (1.65), 3.660 (2.07), 3.674 (3.47), 3.690 (3.05), 3.708 (2.67), 3.723 (2.69), 3.740 (2.27), 3.752 (2.17), 3.772 (1.39), 3.786 (1.06), 3.854 (1.63), 3.895 (1.18), 3.921 (1.51), 3.935 (1.67), 3.949 (1.12), 3.969 (1.67), 3.984 (1.63), 3.998 (1.04), 4.013 (0.94), 4.119 (1.06), 4.133 (1.22), 4.147 (1.18), 4.161 (2.13), 4.175 (1.37), 4.189 (1.16), 4.204 (1.02), 4.729 (4.68), 4.741 (6.85), 4.752 (4.56), 5.248 (1.77), 5.259 (1.83), 5.269 (2.07), 5.279 (1.73), 5.290 (1.26), 5.323 (1.55), 5.331 (1.57), 5.344 (1.71), 5.358 (1.65), 5.368 (1.99), 5.381 (2.29), 5.392 (1.87), 5.401 (1.93), 5.434 (1.30), 5.466 (1.55), 5.474 (1.55), 5.487 (1.22), 5.753 (11.24).

### Beispiel 185

### (5S)-5-(Pyrrolidin-1-ylcarbonyl)-2-{[trans/cis-4-(trifluormethyl)cyclohexyl]methyl}-5,6,7,8-tetrahydro[1,2,4]triazolo[4,3-a]pyridin-3(2H)-on (Isomer 1)

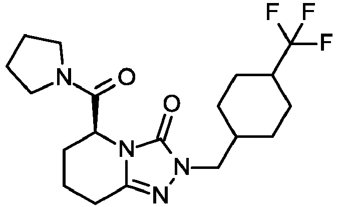

(5S)-3-Oxo-2-{[cis/trans-4-(trifluormethyl)cyclohexyl]methyl}-2,3,5,6,7,8-hexahydro[1,2,4]triazolo[4,3-a]pyridin-5-carbonsäure (Diastereomerengemisch, 2 Isomere) (445 mg, 1.26 mmol) wurde in THF (13 ml) bei Raumtemperatur vorgelegt. Anschließend wurden Pyrrolidin (120 µl, 1.5 mmol), HATU (621 mg, 1.63 mmol) und Triethylamin (880 µl, 6.3 mmol) zugegeben und das Reaktionsgemisch über das Wochenende bei Raumtemperatur gerührt. Das Reaktionsgemisch wurde mit Wasser versetzt und im Vakuum eingeengt. Der Rückstand wurde über präparative HPLC aufgereinigt (Chromatorex C18, 10µm, 125mm x 40mm; Laufmittel: (Acetonitril/Wasser mit 0.1% Ameisensäure)-Gradient). Die produkthaltigen Fraktionen wurden im Vakuum eingeengt und es wurden 158 mg (31 % d. Th.) des Diastereomerengemisches (2 Isomere) erhalten.

(5S)-5-(Pyrrolidin-1-ylcarbonyl)-2-{[trans/cis-4-(trifluormethyl)cyclohexyl]methyl}-5,6,7,8-tetrahydro[1,2,4]triazolo[4,3-a]pyridin-3(2H)-on (Diastereomerengemisch; 2 Isomere) wurden durch chirale präparative HPLC getrennt [Probenvorbereitung: 158 mg gelöst in 15 ml Methanol; Injektionsvolumen: 1 ml; Säule: Daicel Chiralpak^{®} IB 5 µm, 250 x 30 mm; Laufmittel: Kohlendioxid/Ethanol 70:30; Fluss: 80 ml/min; Temperatur 40°C; UV-Detektion: 210 nm]. Nach der Trennung wurden 56 mg von Isomer 1, welches zuerst eluiert, und 54 mg von Isomer 2, welches später eluiert, isoliert.

### Isomer 1:

Analytische chirale HPLC: Rₜ = 0.85 min, d.e. = 99% [Säule: Daicel Chiralpak^{®} IB, 50 x 4.6 mm; Laufmittel: n-Heptan/Ethanol 70:30; Fluss: 3 ml/min; UV-Detektion: 210 nm].
LC-MS (Methode 3): Rₜ = 1.53 min; MS (ESIpos): m/z = 401 [M+H]⁺
¹H-NMR (600 MHz, DMSO-d6) δ [ppm]: 1.465 (2.98), 1.473 (3.40), 1.480 (3.84), 1.487 (4.67), 1.490 (4.53), 1.502 (2.02), 1.507 (1.91), 1.513 (1.33), 1.517 (1.11), 1.524 (1.67), 1.532 (1.52), 1.539 (1.17), 1.549 (1.06), 1.554 (0.73), 1.586 (1.97), 1.591 (2.58), 1.598 (2.08), 1.605 (1.53), 1.612 (1.40), 1.619 (0.96), 1.666 (0.68), 1.679 (0.82), 1.684 (0.90), 1.688 (0.91), 1.693 (0.73), 1.702 (0.78), 1.707 (1.03), 1.711 (1.20), 1.717 (1.36), 1.723 (1.20), 1.733 (0.80), 1.763 (0.70), 1.775 (3.05), 1.786 (5.27), 1.798 (4.12), 1.809 (1.20), 1.899 (1.02), 1.910 (3.13), 1.921 (4.16), 1.932 (2.69), 1.944 (0.83), 1.945 (0.83), 1.954 (0.68), 1.971 (0.80), 1.977 (1.36), 1.986 (1.26), 1.999 (0.71), 2.005 (0.80), 2.009 (0.82), 2.015 (1.00), 2.017 (0.86), 2.023 (0.93), 2.028 (1.06), 2.033 (0.95), 2.047 (0.72), 2.051 (1.08), 2.057 (1.44), 2.064 (1.42), 2.071 (1.20), 2.077 (0.81), 2.265 (0.75), 2.273 (0.73), 2.281 (0.74), 2.512 (0.95), 2.520 (0.72), 2.558 (1.03), 2.603 (1.01), 2.610 (1.93), 2.618 (1.12), 2.630 (0.67), 2.638 (1.12), 3.226 (0.77), 3.237 (1.55), 3.246 (1.41), 3.249 (1.09), 3.257 (2.33), 3.269 (1.10), 3.312 (1.21), 3.324 (2.53), 3.331 (1.48), 3.337 (16.00), 3.343 (1.86), 3.355 (0.76), 3.439 (0.82), 3.450 (1.78), 3.455 (1.23), 3.462 (1.08), 3.467 (2.08), 3.478 (0.95), 3.567 (0.52), 3.580 (0.64), 3.590 (4.31), 3.596 (3.89), 3.604 (4.32), 3.610 (3.99), 3.618 (1.89), 3.629 (0.84), 3.633 (0.57), 4.706 (1.96), 4.711 (2.09), 4.716 (2.32), 4.721 (1.86), 5.759 (13.81).

### Beispiel 186

### (5S)-5-(Pyrrolidin-1-ylcarbonyl)-2-{[trans/cis-4-(trifluormethyl)cyclohexyl]methyl}-5,6,7,8-tetrahydro[1,2,4]triazolo[4,3-a]pyridin-3(2H)-on (Isomer 2)

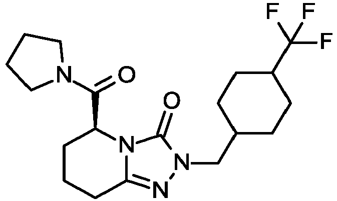

(5S)-5-(Pyrrolidin-1-ylcarbonyl)-2-{[trans/cis-4-(trifluormethyl)cyclohexyl]methyl}-5,6,7,8-tetrahydro[1,2,4]triazolo[4,3-a]pyridin-3(2H)-on (Diastereomerengemisch; 2 Isomere) wurden durch chirale präparative HPLC getrennt [Probenvorbereitung: 158 mg gelöst in 15 ml Methanol; Injektionsvolumen: 1 ml; Säule: Daicel Chiralpak^{®} IB 5 µm, 250 x 30 mm; Laufmittel: Kohlendioxid/Ethanol 70:30; Fluss: 80 ml/min; Temperatur 40°C; UV-Detektion: 210 nm]. Nach der Trennung wurden 56 mg von Isomer 1, welches zuerst eluiert, und 54 mg von Isomer 2, welches später eluiert, isoliert.

### Isomer 2:

Analytische chirale HPLC: Rₜ = 1.84 min, d.e. = 99% [Säule: Daicel Chiralpak^{®} IB, 50 x 4.6 mm; Laufmittel: n-Heptan/Ethanol 70:30; Fluss: 3 ml/min; UV-Detektion: 210 nm].
LC-MS (Methode 3): Rₜ = 1.55 min; MS (ESIpos): m/z = 401 [M+H]⁺
¹H-NMR (400 MHz, DMSO-d6) δ [ppm]: 0.957 (1.26), 0.991 (4.36), 1.023 (5.17), 1.043 (2.00), 1.053 (2.17), 1.147 (2.40), 1.155 (2.66), 1.179 (6.23), 1.186 (6.72), 1.211 (5.99), 1.218 (6.21), 1.243 (2.34), 1.621 (0.98), 1.637 (2.09), 1.647 (2.92), 1.681 (10.46), 1.693 (11.63), 1.706 (11.51), 1.747 (2.77), 1.765 (7.80), 1.782 (12.99), 1.799 (10.09), 1.817 (4.65), 1.832 (7.41), 1.863 (6.74), 1.885 (3.67), 1.902 (9.17), 1.918 (11.87), 1.935 (8.62), 1.952 (3.10), 1.960 (2.94), 1.971 (5.00), 1.983 (5.77), 1.995 (4.03), 2.010 (3.59), 2.020 (2.67), 2.035 (2.39), 2.045 (1.37), 2.057 (0.91), 2.070 (0.68), 2.152 (1.28), 2.160 (1.36), 2.182 (2.42), 2.192 (1.94), 2.204 (2.33), 2.235 (1.10), 2.568 (2.93), 2.588 (3.12), 2.600 (6.14), 2.612 (3.25), 2.629 (1.51), 2.642 (2.49), 2.653 (1.16), 3.212 (1.80), 3.229 (3.63), 3.242 (4.02), 3.258 (6.37), 3.275 (3.12), 3.325 (7.13), 3.343 (3.58), 3.355 (3.72), 3.373 (1.75), 3.390 (0.75), 3.407 (1.01), 3.427 (13.83), 3.443 (16.00), 3.468 (5.79), 3.485 (2.63), 3.578 (2.60), 3.595 (5.41), 3.603 (2.82), 3.611 (3.18), 3.619 (4.08), 3.636 (1.81), 4.682 (4.94), 4.692 (5.90), 4.697 (6.35), 4.706 (4.55), 5.754 (5.05).

### Beispiel 187

### (5S)-5-(2-Azabicyclo[2.1.1]hex-2-ylcarbonyl)-2-{[3-fluor-2-(trifluormethyl)pyridin-4-yl]methyl}-5,6,7,8-tetrahydro[1,2,4]triazolo[4,3-a]pyridin-3(2H)-on

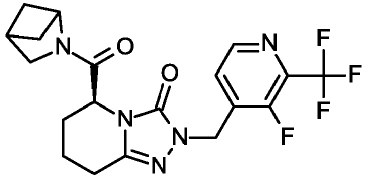

(5S)-2-{[3-Fluor-2-(trifluormethyl)pyridin-4-yl]methyl}-3-oxo-2,3,5,6,7,8-hexahydro[1,2,4]triazolo[4,3-a]pyridin-5-carbonsäure (126 mg, 319 µmol) wurde in THF (10 ml) bei Raumtemperatur vorgelegt. Anschließend wurden HBTU (157 mg, 414 µmol) und N,N-Diisopropylethylamin (560 µl, 3.2 mmol) zugegeben. Nach 10 min Rühren wurde 2-3-Azabicyclo[2.1.1]hexane-trifluoressigsäure (75.4 mg, 382 µmol) zugeben und das Reaktionsgemisch für 30 min bei Raumtemperatur gerührt. Das Reaktionsgemisch wurde mit Wasser und Ethylacetat versetzt und die organische Phase wurde abgetrennt. Die organische Phase wurde mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/ mit 0.1% Ameisensäure-Gradient). Die produkthaltigen Fraktionen wurden im Vakuum eingeengt und es wurden 23.6 mg (17 % d. Th.) der Titelverbindung erhalten.
LC-MS (Methode 3): Rₜ = 1.31 min; MS (ESIpos): m/z = 426 [M+H]⁺
¹H-NMR (400 MHz, DMSO-d6) δ [ppm]: -0.149 (1.09), -0.008 (10.21), 0.008 (7.21), 0.146 (0.87), 0.942 (1.20), 0.958 (1.04), 1.216 (1.37), 1.233 (1.75), 1.241 (2.51), 1.259 (2.68), 1.305 (3.39), 1.323 (2.84), 1.331 (2.57), 1.357 (5.57), 1.368 (7.10), 1.379 (5.19), 1.405 (1.15), 1.658 (1.80), 1.757 (3.66), 1.768 (3.39), 1.825 (1.91), 1.866 (1.97), 1.932 (3.39), 2.041 (6.44), 2.270 (0.82), 2.327 (2.57), 2.366 (2.35), 2.523 (8.41), 2.558 (4.15), 2.573 (3.71), 2.587 (4.15), 2.608 (4.15), 2.620 (5.84), 2.634 (3.66), 2.665 (3.55), 2.669 (3.49), 2.710 (2.35), 2.861 (2.84), 2.870 (2.78), 2.951 (1.86), 3.234 (3.06), 3.258 (5.41), 3.518 (2.29), 3.538 (3.06), 3.630 (2.84), 3.649 (2.18), 4.557 (2.62), 4.575 (2.57), 4.650 (1.69), 4.665 (2.57), 4.681 (4.04), 4.697 (3.66), 4.912 (2.62), 4.920 (3.11), 4.928 (3.22), 4.935 (2.73), 5.050 (6.23), 5.070 (16.00), 7.128 (0.82), 7.547 (2.57), 7.560 (5.95), 7.574 (5.24), 7.589 (2.02), 8.383 (0.44), 8.564 (9.67), 8.576 (9.72).

### Beispiel 188

### (5S)-5-[(1SR,5RS)-2-Azabicyclo[3.1.0]hex-2-ylcarbonyl]-2-(4-methylbenzyl)-5,6,7,8-tetrahydro[1,2,4]triazolo[4,3-a]pyridin-3(2H)-on (Isomer 1)

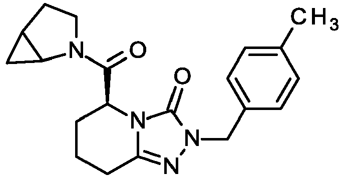

(5S)-5-[(cis)-2-Azabicyclo[3.1.0]hex-2-ylcarbonyl]-2-(4-methylbenzyl)-5,6,7,8-tetrahydro[1,2,4]triazolo[4,3-a]pyridin-3(2H)-on (Diastereomerengemisch; 2 Isomere) wurden durch chirale präparative HPLC getrennt [Probenvorbereitung: 85 mg gelöst in 5 ml Ethanol; Injektionsvolumen: 0.4 ml; Säule: Daicel Chiralpak^{®} AY-H 5 µm, 250 x 20 mm; Laufmittel: n-Heptan/Ethanol 20:80; Fluss: 20 ml/min; Temperatur 25°C; UV-Detektion: 220 nm]. Nach der Trennung wurden 32.5 mg von Isomer 1, welches zuerst eluiert, und 38.2 mg von Isomer 2, welches später eluiert, isoliert.

### Isomer 1:

Analytische chirale HPLC: Rₜ = 4.02 min, d.e. = 100% [Säule: Daicel Chiraltrek^{®} AY-3-3 µm 50 x 6 mm; Laufmittel: n-Heptan/Ethanol 20:80; Fluss: 1 ml/min; UV-Detection: 220 nm].
LC-MS (Methode 3): Rₜ = 1.40 min; MS (ESIpos): m/z = 353 [M+H]⁺
¹H-NMR (400 MHz, DMSO-d6) δ [ppm]: 0.615 (0.46), 0.622 (0.54), 0.629 (0.93), 0.635 (0.92), 0.641 (0.61), 0.648 (0.51), 0.809 (0.76), 0.817 (0.50), 0.823 (0.47), 0.831 (0.78), 1.567 (0.43), 1.572 (0.41), 1.588 (0.43), 1.714 (0.42), 1.738 (0.72), 1.752 (0.82), 1.767 (0.82), 1.772 (0.81), 1.788 (0.56), 1.876 (0.40), 1.884 (0.65), 1.892 (0.58), 1.907 (0.57), 1.914 (0.54), 2.006 (0.50), 2.019 (0.71), 2.027 (0.71), 2.039 (0.88), 2.047 (0.75), 2.055 (0.78), 2.062 (0.61), 2.072 (0.55), 2.085 (0.41), 2.147 (0.65), 2.273 (12.38), 2.521 (1.08), 2.563 (0.61), 2.575 (0.60), 2.585 (0.88), 2.597 (0.58), 2.956 (0.78), 2.965 (0.46), 2.978 (0.41), 2.987 (0.84), 3.697 (0.82), 3.703 (0.88), 3.712 (0.99), 3.718 (0.98), 3.729 (1.15), 3.755 (0.41), 4.754 (5.01), 5.069 (0.77), 5.077 (0.94), 5.085 (0.87), 5.092 (0.76), 7.105 (0.41), 7.127 (16.00), 7.149 (0.41).

### Beispiel 189

### (5S)-5-[(1 SR,5RS)-2-Azabicyclo[3.1.0]hex-2-ylcarbonyl]-2-(4-methylbenzyl)-5,6,7,8-tetrahydro[1,2,4]triazolo[4,3-a]pyridin-3(2H)-on (Isomer 2)

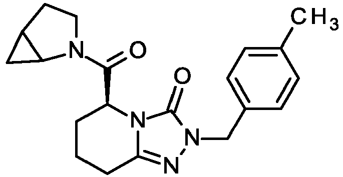

(5S)-5-[(cis)-2-Azabicyclo[3.1.0]hex-2-ylcarbonyl]-2-(4-methylbenzyl)-5,6,7,8-tetrahydro[1,2,4]triazolo[4,3-a]pyridin-3(2H)-on (Diastereomerengemisch; 2 Isomere) wurden durch chirale präparative HPLC getrennt [Probenvorbereitung: 85 mg gelöst in 5 ml Ethanol; Injektionsvolumen: 0.4 ml; Säule: Daicel Chiralpak^{®} AY-H 5 µm, 250 x 20 mm; Laufmittel: n-Heptan/Ethanol 20:80; Fluss: 20 ml/min; Temperatur 25°C; UV-Detektion: 220 nm]. Nach der Trennung wurden 32.5 mg von Isomer 1, welches zuerst eluiert, und 38.2 mg von Isomer 2, welches später eluiert, isoliert.

### Isomer 2:

Analytische chirale HPLC: Rₜ = 5.66 min, d.e. = 100% [column: Daicel Chiraltrek^{®} AY-3-3 µm 50 x 6 mm; Laufmittel: n-Heptan/Ethanol 20:80; Fluss: 1 ml/min; UV-Detektion: 220 nm].
LC-MS (Methode 3): Rₜ = 1.40 min; MS (ESIpos): m/z = 353 [M+H]⁺
¹H-NMR (400 MHz, DMSO-d6) δ [ppm]: 0.614 (0.50), 0.620 (0.50), 0.626 (0.41), 0.635 (0.65), 0.641 (0.60), 0.649 (1.02), 0.655 (1.00), 0.662 (0.63), 0.668 (0.58), 0.882 (0.78), 0.890 (0.52), 0.896 (0.49), 0.904 (0.80), 1.650 (0.44), 1.663 (0.49), 1.675 (0.55), 1.686 (0.65), 1.699 (0.66), 1.711 (0.49), 1.717 (0.46), 1.738 (0.71), 1.752 (1.01), 1.766 (0.91), 1.773 (0.80), 1.787 (0.63), 1.849 (0.41), 1.859 (0.77), 1.869 (0.51), 1.881 (0.53), 1.891 (0.53), 1.931 (0.50), 1.940 (0.52), 1.957 (0.47), 1.974 (0.44), 1.984 (0.56), 1.990 (0.56), 2.008 (0.63), 2.029 (0.60), 2.043 (0.80), 2.057 (0.46), 2.070 (0.43), 2.083 (0.41), 2.117 (0.70), 2.125 (0.46), 2.133 (0.56), 2.145 (0.56), 2.153 (0.61), 2.161 (0.55), 2.272 (15.63), 2.523 (0.89), 2.569 (0.89), 2.584 (1.31), 2.596 (0.68), 2.625 (0.46), 3.130 (0.70), 3.151 (1.03), 3.161 (0.58), 3.171 (0.50), 3.181 (0.88), 3.202 (0.43), 3.480 (0.51), 3.486 (0.56), 3.495 (0.90), 3.501 (0.87), 3.510 (0.57), 3.516 (0.48), 3.578 (0.46), 3.588 (0.49), 3.603 (0.58), 3.612 (0.61), 3.619 (0.51), 3.634 (0.43), 3.677 (0.56), 3.683 (0.53), 3.944 (0.41), 4.710 (0.44), 4.719 (0.51), 4.725 (0.68), 4.742 (5.01), 4.750 (1.90), 4.925 (0.82), 4.934 (0.96), 4.941 (1.03), 4.949 (0.79), 7.104 (0.56), 7.126 (16.00), 7.147 (0.61).

### Beispiel 190

### (5S)-5-{[(2S)-2-Glycoloylpyrrolidin-1 -yl]carbonyl}-2-(4-methylbenzyl)-5,6,7,8-tetrahydro[1,2,4]triazolo[4,3-a]pyridin-3(2H)-on

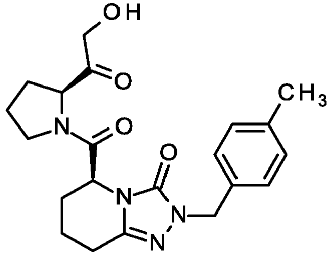

2-[(2S)-1-{[(5S)-2-(4-Methylbenzyl)-3-oxo-2,3,5,6,7,8-hexahydro[1,2,4]triazolo[4,3-a]pyridin-5-yl]carbonyl}pyrrolidin-2-yl]-2-oxoethyl-acetat (124 mg, 282 µmol) wurde in THF (1.5 ml) und Wasser (1.5 ml) vorgelegt und Lithiumhydroxid (16.9 mg, 706 µmol) gelöst in Wasser wurde zugegeben. Das Reaktionsgemisch wurde nach 30 min Rühren bei Raumtemperatur mit Wasser, 1 N wässriger Salzsäure und gesättigter wässriger Natriumchlorid-Lösung versetzt. Die organische Phase wurde abgetrennt und die wässrige Phase dreimal mit Ethylacetat extrahiert. Die vereinigten organischen Phasen wurden über Natriumsulfat getrocknet, filtriert und das Filtrat eingeengt. Der Rückstand wurde über präparative HPLC aufgereinigt (Methode 11). Die produkthaltigen Fraktionen wurden im Vakuum eingeengt und es wurden 21.7 mg (19 % d. Th.) der Titelverbindung erhalten.
LC-MS (Methode 3): Rₜ = 1.16 min; MS (ESIpos): m/z = 399 [M+H]⁺
¹H-NMR (400 MHz, DMSO-d6) δ [ppm]: -0.008 (2.58), 0.008 (2.09), 1.569 (0.46), 1.595 (0.59), 1.606 (0.60), 1.632 (0.43), 1.697 (0.48), 1.715 (1.13), 1.730 (1.83), 1.746 (1.66), 1.753 (1.01), 1.762 (1.38), 1.778 (0.83), 1.892 (0.57), 1.910 (1.44), 1.922 (1.77), 1.927 (1.91), 1.939 (1.34), 1.945 (1.40), 2.036 (1.22), 2.047 (2.10), 2.058 (2.01), 2.123 (0.47), 2.140 (0.76), 2.161 (0.86), 2.172 (0.80), 2.193 (0.71), 2.269 (16.00), 2.467 (0.53), 2.523 (1.88), 2.559 (1.48), 2.573 (1.07), 2.601 (0.45), 3.540 (0.50), 3.558 (1.07), 3.565 (0.78), 3.575 (0.71), 3.582 (1.28), 3.600 (0.60), 3.740 (0.62), 3.756 (1.21), 3.765 (0.70), 3.773 (0.74), 3.781 (0.96), 3.797 (0.45), 4.153 (3.51), 4.170 (3.94), 4.559 (1.33), 4.573 (1.48), 4.580 (1.52), 4.595 (1.23), 4.731 (5.31), 4.735 (5.29), 4.808 (1.25), 4.820 (2.25), 4.831 (1.09), 5.177 (1.33), 5.192 (2.83), 5.207 (1.25), 7.090 (1.34), 7.112 (9.45), 7.118 (9.08), 7.125 (4.11), 7.139 (1.22).

### Beispiel 191

### (5S)-5-[(1,1 -Difluor-5-azaspiro[2.3]hex-5-yl)carbonyl]-2-(4-methylbenzyl)-5,6,7,8-tetrahydro[1,2,4]triazolo[4,3-a]pyridin-3(2H)-on

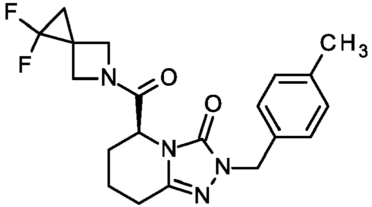

(5S)-2-(4-Methylbenzyl)-3-oxo-2,3,5,6,7,8-hexahydro[1,2,4]triazolo[4,3-a]pyridin-5-carbonsäure (50.0 mg, 174 µmol) wurde in Dichlormethan (4.0 ml) und DMF (7.0 ml) bei Raumtemperatur vorgelegt. Anschließend wurden 1-[Bis(dimethylamino)methylen]-1H-benzotriazol-1-ium-3-oxidhexafluorophosphat (85.8 mg, 226 µmol) und N,N-Diisopropylethylamin (42 µl, 240 µmol) zugegeben. Nach 15 min Rühren wurde 1,1-Difluor-5-azaspiro[2.3]hexanhydrochlorid (32.5 mg, 209 µmol) zugegeben und das Reaktionsgemisch für 2h bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 22.0 mg (30 % d. Th.) der Titelverbindung erhalten.
LC-MS (Methode 1): Rₜ = 1.10 min; MS (ESIpos): m/z = 389 [M+H]⁺
¹H-NMR (400 MHz, DMSO-d6) δ [ppm]: 1.702 (1.18), 1.715 (1.79), 1.728 (1.34), 1.745 (0.60), 1.766 (1.06), 1.787 (1.43), 1.812 (1.15), 1.970 (0.63), 1.982 (0.82), 1.995 (0.85), 2.012 (0.82), 2.029 (0.80), 2.045 (0.53), 2.271 (12.65), 2.567 (1.06), 2.580 (1.02), 3.983 (0.66), 4.007 (1.20), 4.056 (1.17), 4.079 (0.63), 4.363 (0.81), 4.385 (0.86), 4.481 (0.52), 4.510 (0.93), 4.531 (1.39), 4.544 (0.53), 4.574 (0.46), 4.588 (0.69), 4.600 (0.40), 4.753 (5.83), 5.753 (0.46), 7.107 (0.42), 7.128 (16.00), 7.149 (0.45).

### Beispiel 192

### (5S)-5-[(3-Methylazetidin-1 -yl)carbonyl]-2-(4-methylbenzyl)-5,6,7,8-tetrahydro[1,2,4]triazolo[4,3-a]pyridin-3(2H)-on

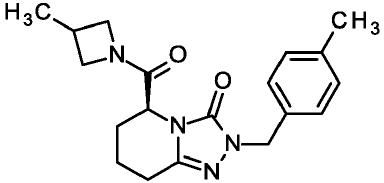

(5S)-2-(4-Methylbenzyl)-3-oxo-2,3,5,6,7,8-hexahydro[1,2,4]triazolo[4,3-a]pyridin-5-carbonsäure (50.0 mg, 174 µmol) wurde in DMF (4.0 ml) und Dichlormethan (2.0 ml) bei Raumtemperatur vorgelegt. Anschließend wurden 1-[Bis(dimethylamino)methylen]-1H-benzotriazol-1-ium-3-oxidhexafluorophosphat (85.8 mg, 226 µmol) und N,N-Diisopropylethylamin (79 µl, 450 µmol) zugegeben. Nach 15 min Rühren wurde 3-Methylazetidinhydrochlorid (22.5 mg, 209 µmol) zugegeben und das Reaktionsgemisch für 2 h bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 39.3 mg (63 % d. Th.) der Titelverbindung erhalten.
LC-MS (Methode 1): Rₜ = 1.02 min; MS (ESIpos): m/z = 341 [M+H]⁺
¹H-NMR (400 MHz, DMSO-d6) δ [ppm]: -0.008 (0.45), 0.008 (0.43), 1.183 (4.30), 1.191 (4.27), 1.201 (4.62), 1.208 (4.13), 1.688 (1.31), 1.700 (1.87), 1.715 (1.47), 1.728 (0.65), 1.890 (0.48), 1.902 (0.53), 1.913 (0.79), 1.925 (0.90), 1.935 (0.57), 1.974 (0.74), 1.988 (0.69), 1.994 (0.69), 2.009 (0.68), 2.270 (14.75), 2.568 (1.78), 2.581 (0.90), 2.610 (0.61), 2.688 (0.48), 2.709 (0.62), 2.719 (0.54), 2.733 (0.45), 3.399 (0.56), 3.413 (0.56), 3.423 (0.61), 3.437 (0.57), 3.454 (0.58), 3.469 (0.59), 3.478 (0.65), 3.493 (0.60), 3.696 (0.54), 3.710 (0.60), 3.716 (0.64), 3.730 (0.55), 3.844 (0.53), 3.859 (0.61), 3.865 (0.65), 3.879 (0.56), 3.937 (0.60), 3.959 (0.95), 3.981 (0.54), 4.002 (0.61), 4.024 (0.99), 4.047 (0.54), 4.258 (0.58), 4.278 (1.15), 4.299 (0.52), 4.387 (0.55), 4.408 (1.09), 4.429 (0.52), 4.451 (0.63), 4.464 (1.38), 4.478 (1.38), 4.489 (0.61), 4.741 (6.73), 7.103 (0.54), 7.124 (16.00), 7.145 (0.55).

### Beispiel 193

### (5S)-2-(4-Methylbenzyl)-5-{[(1RS)-1-oxido-1,3-thiazolidin-3-yl]carbonyl}-5,6,7,8-tetrahydro[1,2,4]triazolo[4,3-a]pyridin-3(2H)-on (Isomer 1)

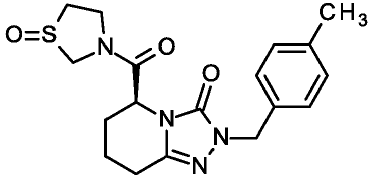

(5S)-2-(4-Methylbenzyl)-5-{[(1RS)-1-oxido-1,3-thiazolidin-3-yl]carbonyl}-5,6,7,8-tetrahydro[1,2,4]triazolo[4,3-a]pyridin-3(2H)-on (Diastereomerengemisch; 2 Isomere) wurde durch chirale präparative HPLC getrennt [Probenvorbereitung: 24.8 mg gelöst in 1.5 ml Ethanol; Injektionsvolumen: 1.5 ml; Säule: Daicel Chiralpak^{®} IA 5 µm, 250 × 20 mm; Laufmittel: Ethanol; Fluss: 15 ml/min; Temperature 60°C; UV-Detektion: 220 nm]. Nach der Trennung wurden 8.0 mg von Isomer 1 welches zuerst eluiert, und 10 mg von Isomer 2 welches später eluiert, isoliert.

### Isomer 1:

Analytische chirale HPLC: Rₜ = 9.22 min, d.e. = 100% [Säule: Daicel Chiralpak^{®} IA 5 µm, 250 × 20 mm; Laufmittel: Ethanol; Fluss: 15 ml/min; UV-Detektion: 220 nm].
LC-MS (Methode 3): Rₜ = 1.05 min; MS (ESIpos): m/z = 375 [M+H]⁺
¹H-NMR (400 MHz, DMSO-d6) δ [ppm]: 1.617 (0.54), 1.629 (0.55), 1.641 (0.49), 1.703 (0.50), 1.712 (0.48), 1.727 (0.47), 1.736 (0.52), 2.022 (1.00), 2.033 (1.04), 2.044 (0.82), 2.078 (0.73), 2.088 (0.68), 2.274 (16.00), 2.557 (0.73), 2.576 (0.73), 2.589 (1.02), 2.601 (0.86), 2.617 (0.53), 2.630 (0.43), 3.037 (0.51), 3.060 (1.07), 3.082 (1.14), 3.093 (0.85), 3.100 (0.84), 3.117 (0.44), 3.125 (0.49), 3.755 (0.42), 3.770 (0.42), 3.778 (0.77), 3.785 (0.54), 3.800 (0.46), 3.807 (0.42), 4.063 (0.41), 4.082 (0.56), 4.086 (0.53), 4.093 (0.44), 4.105 (0.47), 4.112 (0.48), 4.117 (0.44), 4.162 (0.48), 4.174 (0.85), 4.191 (0.74), 4.198 (0.79), 4.284 (0.75), 4.317 (0.90), 4.418 (1.33), 4.449 (1.48), 4.608 (0.60), 4.613 (0.60), 4.641 (0.49), 4.646 (0.50), 4.753 (7.88), 4.850 (0.71), 4.864 (1.03), 4.874 (0.71), 4.898 (0.46), 4.912 (0.61), 4.921 (0.44), 5.018 (0.94), 5.046 (0.85), 7.103 (0.85), 7.128 (12.94), 7.150 (1.01).

### Beispiel 194

### (5S)-2-(4-Methylbenzyl)-5-{[(1RS)-1-oxido-1,3-thiazolidin-3-yl]carbonyl}-5,6,7,8-tetrahydro[1,2,4]triazolo[4,3-a]pyridin-3(2H)-on (Isomer 2)

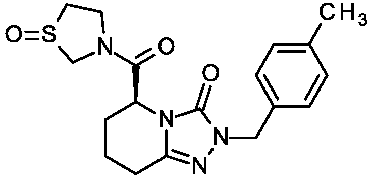

(5S)-2-(4-Methylbenzyl)-5-{[(1RS)-1-oxido-1,3-thiazolidin-3-yl]carbonyl}-5,6,7,8-tetrahydro[1,2,4]triazolo[4,3-a]pyridin-3(2H)-on (Diastereomerengemisch; 2 Isomere) wurde durch chirale präparative HPLC getrennt [Probenvorbereitung: 24.8 mg gelöst in 1.5 ml Ethanol; Injektionsvolumen: 1.5 ml; Säule: Daicel Chiralpak^{®} IA 5 µm, 250 × 20 mm; Laufmittel: Ethanol; Fluss: 15 ml/min; Temperature 60°C; UV-Detektion: 220 nm]. Nach der Trennung wurden 8.0 mg von Isomer 1 welches zuerst eluiert, und 10 mg von Isomer 2 welches später eluiert, isoliert.

### Isomer 2:

Analytische chirale HPLC: Rₜ = 11.44 min, d.e. = 100% [Säule: Daicel Chiralpak^{®} IA 5 µm, 250 × 20 mm; Laufmittel: Ethanol; Fluss: 15 ml/min; UVDetektion: 220 nm].
LC-MS (Methode 3): Rₜ = 1.05 min; MS (ESIpos): m/z = 375 [M+H]⁺
¹H-NMR (400 MHz, DMSO-d6) δ [ppm]: 1.739 (0.77), 1.994 (1.34), 2.114 (0.42), 2.274 (16.00), 2.560 (1.09), 2.574 (0.86), 2.598 (1.53), 2.610 (0.98), 2.640 (0.61), 2.938 (0.54), 2.972 (0.59), 3.132 (0.43), 3.160 (0.56), 3.181 (0.77), 3.209 (0.40), 3.273 (0.59), 3.972 (1.19), 3.989 (1.18), 3.997 (1.06), 4.108 (0.72), 4.122 (0.72), 4.135 (0.43), 4.286 (0.52), 4.308 (0.71), 4.338 (1.47), 4.371 (1.58), 4.486 (1.01), 4.516 (1.12), 4.606 (1.00), 4.611 (0.98), 4.644 (0.79), 4.756 (6.31), 4.985 (1.16), 4.994 (1.28), 5.010 (0.98), 5.019 (1.39), 5.050 (0.71), 7.130 (14.05).

### Beispiel 195

### (5S)-5-[(3-Fluor-3-methylazetidin-1-yl)carbonyl]-2-(4-methylbenzyl)-5,6,7,8-tetrahydro[1,2,4]triazolo[4,3-a]pyridin-3(2H)-on

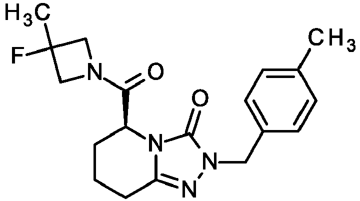

(5S)-2-(4-Methylbenzyl)-3-oxo-2,3,5,6,7,8-hexahydro[1,2,4]triazolo[4,3-a]pyridin-5-carbonsäure (50.0 mg, 174 µmol) wurde in DMF (2.0 ml) und N,N-Diisopropylethylamin (79 µl, 450 µmol) bei Raumtemperatur vorgelegt. Anschließend wurden 1-[Bis(dimethylamino)methylen]-1H-benzotriazol-1-ium-3-oxidhexafluorophosphat (85.8 mg, 226 µmol) und Dichlormethan (1.0 ml) zugegeben. Nach 15 min Rühren wurde 3-Fluor-3-methylazetidinhydrochlorid (24.0 mg, 191 µmol) zugegeben und das Reaktionsgemisch für 2.5h bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 23.9 mg (38 % d. Th.) der Titelverbindung erhalten.
LC-MS (Methode 3): Rₜ = 1.42 min; MS (ESIpos): m/z = 359 [M+H]⁺
¹H-NMR (400 MHz, DMSO-d6) δ [ppm]: 1.569 (3.12), 1.577 (3.06), 1.624 (3.10), 1.632 (3.04), 1.712 (1.77), 1.941 (0.71), 2.011 (0.74), 2.024 (0.68), 2.271 (13.50), 2.577 (1.67), 2.620 (0.50), 3.933 (0.54), 3.951 (0.47), 3.980 (1.33), 3.998 (0.90), 4.028 (0.93), 4.043 (0.99), 4.068 (0.41), 4.253 (0.47), 4.301 (0.51), 4.337 (0.49), 4.389 (0.52), 4.403 (0.55), 4.424 (0.65), 4.448 (0.83), 4.474 (0.70), 4.503 (0.81), 4.519 (1.19), 4.533 (1.19), 4.748 (7.16), 7.126 (16.00).

### Beispiel 196

### (2S)-1-{[(5S)-2-(4-Methylbenzyl)-3-oxo-2,3,5,6,7,8-hexahydro[1,2,4]triazolo[4,3-a]pyridin-5-yl]carbonyl}pyrrolidin-2-carbaldehyd

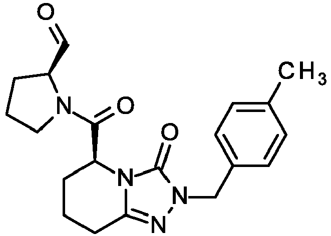

Oxalsäuredichlorid (20 µl, 230 µmol) wurde in Dichlormethan (2 ml) vorgelgt und auf -78°C gekühlt. Anschließend wurde eine Lösung aus Dimethylsulfoxid (36 µl, 500 µmol) und Dichlormethan (1 ml) zugetropft und für 15 min bei -78°C nachgerührt. (5S)-5-{[(2S)-2-(Hydroxymethyl)pyrrolidin-1-yl]carbonyl}-2-(4-methylbenzyl)-5,6,7,8-tetrahydro[1,2,4]triazolo[4,3-a]pyridin-3(2H)-on (77.8 mg, 210 µmol) wurde gelöst in Dichlormethan (2 ml) zugetropft und das Reaktionsgemisch wurde für weitere 60 min bei - 78°C nachgerührt. Triethylamin (150 µl, 1.1 mmol) wurde zugetropft und es wurde für weitere 20 min bei -78°C nachgerührt. Das Reaktionsgemisch wurde auf Raumtemperatur gebracht Das Reaktionsgemisch wurde mit Wasser versetzt. Die organische Phase wurde abgetrennt und die wässrige Phase dreimal mit Dichlormethan extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über Säulenchromatographie aufgereinigt (SiO₂; Laufmittel:, Methanol/Dichlormethan 10/90). Die produkthaltigen Fraktionen wurden im Vakuum eingeengt und es wurden 48.9 mg (57 % d. Th.) der Titelverbindung erhalten.
LC-MS (Methode 3): Rₜ = 1.35 min; MS (ESIpos): m/z = 369 [M+H]⁺
¹H-NMR (400 MHz, DMSO-d6) δ [ppm]: -0.008 (3.81), 0.008 (2.35), 1.013 (0.57), 1.091 (0.63), 1.235 (0.83), 1.645 (0.74), 1.657 (0.74), 1.670 (0.86), 1.691 (0.85), 1.705 (0.93), 1.724 (0.92), 1.735 (1.12), 1.748 (1.29), 1.760 (1.25), 1.771 (1.10), 1.782 (0.89), 1.826 (0.52), 1.842 (0.89), 1.858 (1.06), 1.875 (1.07), 1.883 (1.06), 1.892 (1.32), 1.906 (1.40), 1.922 (1.29), 1.937 (1.22), 1.949 (1.42), 1.966 (1.33), 1.985 (1.17), 1.996 (1.25), 2.011 (1.16), 2.020 (1.35), 2.037 (1.48), 2.049 (1.29), 2.064 (1.15), 2.078 (1.20), 2.086 (1.68), 2.099 (1.92), 2.110 (1.86), 2.122 (0.92), 2.233 (0.40), 2.268 (16.00), 2.327 (0.42), 2.518 (2.56), 2.567 (1.27), 2.575 (1.43), 2.587 (1.83), 2.602 (1.80), 2.616 (0.59), 2.628 (0.66), 2.669 (0.42), 3.184 (1.05), 3.211 (1.19), 3.239 (0.57), 3.248 (0.79), 3.266 (1.32), 3.401 (0.42), 3.425 (0.46), 3.576 (0.44), 3.593 (0.86), 3.600 (0.69), 3.618 (1.06), 3.634 (0.49), 3.745 (0.60), 3.763 (0.86), 3.771 (0.74), 3.785 (0.64), 4.323 (0.62), 4.332 (0.73), 4.338 (1.13), 4.344 (0.76), 4.354 (0.60), 4.359 (0.59), 4.709 (0.52), 4.740 (9.55), 4.780 (0.79), 4.792 (0.69), 4.801 (0.52), 4.838 (1.16), 4.849 (1.96), 4.862 (0.99), 5.000 (0.52), 5.007 (0.50), 5.686 (0.85), 5.701 (0.80), 5.753 (1.65), 5.816 (0.77), 5.832 (0.74), 7.090 (1.40), 7.097 (1.38), 7.112 (9.05), 7.118 (13.08), 7.139 (1.38), 9.344 (3.27), 9.349 (3.19).

### Beispiel 197

### (5S)-5-[(1RS,5SR)-2-Azabicyclo[3.1.0]hex-2-ylcarbonyl]-2-[(6-chlorpyridin-3-yl)methyl]-5,6,7,8-tetrahydro[1,2,4]triazolo[4,3-a]pyridin-3(2H)-on (Isomer 1)

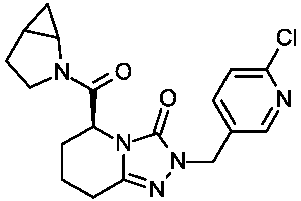

(5S)-2-[(6-Chlorpyridin-3-yl)methyl]-3-oxo-2,3,5,6,7,8-hexahydro[1,2,4]triazolo[4,3-a]pyridin-5-carbonsäure (68.0 mg, 220 µmol) wurde in THF (3.7 ml) bei Raumtemperatur vorgelegt. Anschließend wurden HATU (109 mg, 286 µmol), (1RS,5SR)-2-Azabicyclo[3.1.0]hexanhydrochlorid (31.6 mg, 264 µmol) und N,N-Diisopropylethylamin (120 µl, 660 µmol) zugegeben. Das Reaktionsgemisch wurde über Nacht bei Raumtemperatur gerührt. Das Reaktionsgemisch wurde im Vakuum eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Die produkthaltigen Fraktionen wurden im Vakuum eingeengt und es wurden 63.0 mg (77 % d. Th.) eines Diastereomerengemisches (2 Isomere) erhalten.

Das Diastereomerengemisch (2 Isomere) wurde durch chirale präparative HPLC getrennt [Probenvorbereitung: 63 mg gelöst in 2 ml Acetonitril; Injektionsvolumen: 0.25 ml; Säule: Daicel Chiralpak^{®} IF 5 µm, 250 × 20 mm; Laufmittel: n-Heptan/Ethanol 20:80; Fluss: 20 ml/min; Temperatur 30°C; UV-Detektion: 220 nm]. Nach der Trennung wurden 15.3 mg von Isomer 1, welches zuerst eluiert, und 17.2 mg von Isomer 2, welches später eluiert, isoliert.

### Isomer 1:

Analytische chirale HPLC: Rₜ = 2.91 min, d.e. > 99% [Säule: Daicel Chiralpak^{®} IF, 50 × 4.6 mm; Laufmittel: n-Heptan/Ethanol 50:50; Fluss: 1 ml/min; UV-Detektion: 220 nm].
LC-MS (Methode 3): Rₜ = 1.14 min; MS (ESIpos): m/z = 374 [M+H]⁺
¹H-NMR (400 MHz, DMSO-d6) δ [ppm]: -0.149 (1.50), -0.008 (13.97), 0.008 (12.20), 0.146 (1.59), 0.643 (2.92), 0.903 (2.39), 1.755 (2.56), 1.860 (1.94), 1.955 (2.03), 2.044 (2.30), 2.327 (3.18), 2.366 (2.65), 2.523 (9.72), 2.600 (3.54), 2.665 (2.30), 2.670 (3.09), 2.674 (2.56), 2.710 (2.39), 3.148 (2.56), 3.177 (2.56), 3.506 (2.39), 3.610 (1.59), 3.679 (1.50), 3.936 (1.33), 4.743 (1.50), 4.877 (16.00), 4.885 (8.66), 4.934 (2.39), 4.950 (2.74), 4.959 (2.21), 7.504 (6.63), 7.524 (8.49), 7.687 (3.71), 7.693 (5.04), 7.708 (2.74), 7.714 (3.89), 8.296 (5.04), 8.302 (6.72).

### Beispiel 198

### (5S)-5-[(1SR,5RS)-2-Azabicyclo[3.1.0]hex-2-ylcarbonyl]-2-[(6-chlorpyridin-3-yl)methyl]-5,6,7,8-tetrahydro[1,2,4]triazolo[4,3-a]pyridin-3(2H)-on (Isomer 2)

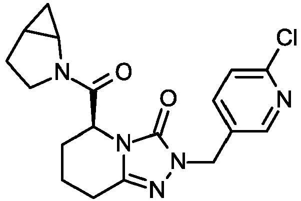

(5S)-5-[(1RS,5SR)-2-Azabicyclo[3.1.0]hex-2-ylcarbonyl]-2-[(6-chlorpyridin-3-yl)methyl]-5,6,7,8-tetrahydro[1,2,4]triazolo[4,3-a]pyridin-3(2H)-on (Diastereomerengemisch; 2 Isomere) wurde durch chirale präparative HPLC getrennt [Probenvorbereitung: 63 mg gelöst in 2 ml Acetonitril; Injektionsvolumen: 0.25 ml; Säule: Daicel Chiralpak^{®} IF 5 µm, 250 × 20 mm; Laufmittel: n-Heptan/Ethanol 20:80; Fluss: 20 ml/min; Temperatur 30°C; UV-Detektion: 220 nm]. Nach der Trennung wurden 15.3 mg von Isomer 1, welches zuerst eluiert, und 17.2 mg von Isomer 2, welches später eluiert, isoliert.

### Isomer 2:

Analytische chirale HPLC: Rₜ = 4.28 min, d.e. = 98% [column: Daicel Chiralpak^{®} IF, 50 × 4.6 mm; Laufmittel: n-Heptan/Ethanol 50:50; Fluss: 1 ml/min; UV-Detektion: 220 nm].
LC-MS (Methode 3): Rₜ = 1.15 min; MS (ESIpos): m/z = 374 [M+H]⁺
¹H-NMR (400 MHz, DMSO-d6) δ [ppm]: -0.149 (1.33), -0.008 (16.00), 0.008 (9.33), 0.146 (1.24), 0.562 (0.89), 0.619 (2.22), 0.625 (2.49), 0.632 (4.00), 0.638 (3.91), 0.645 (2.58), 0.651 (2.13), 0.728 (0.80), 0.796 (1.69), 0.811 (3.38), 0.818 (2.22), 0.834 (3.38), 0.847 (1.60), 1.578 (2.04), 1.756 (3.91), 1.791 (2.93), 1.854 (1.42), 1.862 (1.51), 1.876 (2.13), 1.885 (2.76), 1.894 (2.31), 1.908 (2.22), 1.915 (2.13), 2.028 (3.11), 2.038 (3.02), 2.048 (3.38), 2.086 (1.69), 2.163 (2.58), 2.322 (1.69), 2.327 (2.22), 2.332 (1.69), 2.366 (2.04), 2.518 (12.36), 2.523 (11.82), 2.566 (4.18), 2.580 (3.29), 2.600 (4.09), 2.612 (2.76), 2.641 (1.60), 2.665 (1.87), 2.669 (2.31), 2.674 (1.69), 2.709 (2.13), 2.934 (1.51), 2.955 (3.38), 2.964 (2.04), 2.977 (1.78), 2.986 (3.56), 3.008 (1.42), 3.541 (1.24), 3.702 (4.09), 3.711 (4.80), 3.717 (4.18), 3.726 (4.71), 3.732 (5.24), 3.757 (1.87), 3.764 (1.69), 3.802 (0.98), 4.653 (1.07), 4.850 (1.33), 4.882 (7.20), 4.890 (14.22), 4.896 (13.07), 4.936 (1.07), 5.087 (3.38), 5.094 (4.09), 5.102 (3.73), 5.110 (3.20), 7.505 (7.20), 7.525 (8.98), 7.690 (5.96), 7.697 (5.87), 7.711 (4.98), 7.717 (4.80), 8.300 (6.76), 8.306 (6.49).

### Beispiel 199

### (5S)-5-(2-Azabicyclo[2.1.1]hex-2-ylcarbonyl)-2-{[6-(trifluormethyl)pyridin-3-yl]methyl}-5,6,7,8-tetrahydro[1,2,4]triazolo[4,3-a]pyridin-3(2H)-on

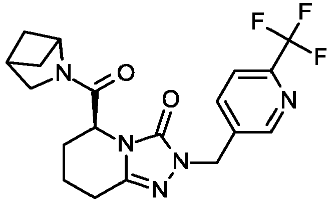

(5S)-3-Oxo-2-{[6-(trifluormethyl)pyridin-3-yl]methyl}-2,3,5,6,7,8-hexahydro[1,2,4]triazolo[4,3-a]pyridin-5-carbonsäure (112 mg, 327 µmol) wurde in THF (5.0 ml) bei Raumtemperatur vorgelegt. Anschließend wurden HATU (161 mg, 425 µmol) und N,N-Diisopropylethylamin (570 µl, 3.3 mmol) zugegeben. Nach 10 min Rühren wurde 2-3-Azabicyclo[2.1.1]hexane-trifluoressigsäure (77.4 mg, 393 µmol) zugegeben und das Reaktionsgemisch für 60 min bei Raumtemperatur gerührt. Das Reaktionsgemisch wurde mit Wasser und Ethylacetat versetzt und die organische Phase wurde abgetrennt. Die organische Phase wurde mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Die produkthaltigen Fraktionen wurden im Vakuum eingeengt und es wurden 24.2 mg (18 % d. Th.) der Titelverbindung erhalten.
LC-MS (Methode 4): Rₜ = 0.69 min; MS (ESIpos): m/z = 408 [M+H]⁺
¹H-NMR (400 MHz, DMSO-d6) δ [ppm]: -0.008 (3.67), 0.008 (3.09), 1.223 (0.73), 1.241 (0.84), 1.249 (1.36), 1.267 (1.47), 1.304 (1.61), 1.321 (1.54), 1.330 (1.47), 1.355 (3.02), 1.369 (4.75), 1.383 (2.69), 1.410 (1.26), 1.647 (0.82), 1.744 (1.96), 1.758 (1.82), 1.815 (0.96), 1.856 (1.05), 1.931 (1.87), 2.036 (3.56), 2.072 (0.98), 2.327 (0.98), 2.366 (0.98), 2.558 (3.13), 2.571 (2.48), 2.593 (2.60), 2.605 (3.58), 2.618 (2.08), 2.635 (0.84), 2.647 (1.24), 2.669 (1.26), 2.710 (1.15), 2.861 (1.59), 2.869 (1.57), 2.877 (1.64), 2.932 (1.01), 2.949 (1.05), 3.228 (1.66), 3.252 (2.90), 3.514 (1.43), 3.534 (1.87), 3.634 (1.78), 3.654 (1.26), 4.555 (1.47), 4.573 (1.50), 4.636 (1.03), 4.651 (1.47), 4.663 (1.10), 4.676 (2.08), 4.693 (2.01), 4.896 (1.50), 4.904 (1.78), 4.911 (1.80), 4.919 (1.47), 4.966 (0.73), 5.001 (7.72), 5.018 (5.08), 5.059 (0.70), 7.913 (16.00), 8.645 (5.01).

### Beispiel 200

### (5S)-5-[(1RS,5SR)-2-Azabicyclo[3.1.0]hex-2-ylcarbonyl]-2-{[6-(trifluormethyl)pyridin-3-yl]methyl}-5,6,7,8-tetrahydro[1,2,4]triazolo[4,3-a]pyridin-3(2H)-on (Isomer 1)

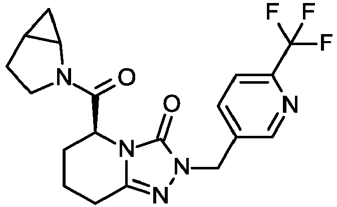

(5S)-3-Oxo-2-{[6-(trifluormethyl)pyridin-3-yl]methyl}-2,3,5,6,7,8-hexahydro[1,2,4]triazolo[4,3-a]pyridin-5-carbonsäure (251 mg, 733 µmol) wurde in THF (12 ml) bei Raumtemperatur vorgelegt. Anschließend wurden HATU (362 mg, 953 µmol), (1R,5S)-2-Azabicyclo[3.1.0]hexanhydrochlorid (105 mg, 880 µmol) und N,N-Diisopropylethylamin (380 µl, 2.2 mmol) zugegeben. Das Reaktionsgemisch wurde über Nacht bei Raumtemperatur gerührt. Es wurden erneut HATU (279 mg, 733 µmol), (1RS,5SR)-2-Azabicyclo[3.1.0]hexanhydrochlorid (88 mg, 733 µmol) und N,N-Diisopropylethylamin (127 µl, 1.7 mmol) zugegeben und für weitere 20 Stunden gerührt. Das Reaktionsgemisch wurde im Vakuum eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 54.0 mg (18 % d. Th.) eines Diastereomerengemisches (2 Isomere) erhalten.

Das Diastereomerengemisch (2 Isomere) wurde durch chirale präparative HPLC getrennt [Probenvorbereitung: 54 mg gelöst in 20 ml Methanol; Injektionsvolumen: 1 ml; Säule: Daicel Chiralpak^{®} IE 5 µm, 250 × 20 mm; Laufmittel: Kohlendioxid/Methanol 80:20; Fluss: 80 ml/min; Temperatur 40°C; UV-Detektion: 210 nm]. Nach der Trennung wurden 20.2 mg von Isomer 1, welches zuerst eluiert, und 21.3 mg von Isomer 2, welches später eluiert, isoliert.

### Isomer 1:

Analytische chirale HPLC: Rₜ = 5.41 min, d.e. > 99.5% [Säule: Daicel Chiralpak^{®} IE, 50 x 4.6 mm; Laufmittel: n-Heptan/Methanol 80:20; Fluss: 3 ml/min; UV-Detektion: 210 nm].
LC-MS (Methode 3): Rₜ = 1.32 min; MS (ESIpos): m/z = 408 [M+H]⁺
¹H-NMR (400 MHz, DMSO-d6) δ [ppm]: -0.149 (0.66), -0.008 (5.58), 0.008 (5.50), 0.146 (0.59), 0.630 (1.98), 0.637 (1.98), 0.644 (2.57), 0.650 (2.13), 0.657 (1.32), 0.663 (1.17), 0.691 (0.81), 0.711 (0.81), 0.870 (0.81), 0.884 (1.61), 0.906 (1.69), 0.920 (0.73), 1.567 (0.66), 1.651 (0.95), 1.773 (2.13), 1.839 (0.73), 1.862 (1.54), 1.871 (0.95), 1.885 (0.88), 1.894 (0.81), 1.958 (1.47), 1.976 (1.32), 2.029 (1.17), 2.062 (1.83), 2.154 (1.10), 2.323 (1.03), 2.327 (1.39), 2.332 (1.10), 2.366 (1.03), 2.523 (5.43), 2.567 (2.06), 2.578 (1.83), 2.593 (1.98), 2.610 (2.79), 2.651 (1.03), 2.665 (1.47), 2.670 (1.76), 2.674 (1.25), 2.710 (1.17), 3.132 (1.32), 3.152 (1.76), 3.162 (1.69), 3.172 (0.95), 3.183 (1.98), 3.203 (0.81), 3.491 (1.10), 3.497 (1.25), 3.506 (1.91), 3.521 (1.25), 3.584 (1.03), 3.593 (1.10), 3.609 (1.25), 3.619 (1.32), 3.624 (1.10), 3.641 (0.95), 3.649 (0.81), 3.677 (0.73), 3.687 (1.25), 3.693 (1.17), 3.703 (0.73), 3.920 (0.51), 3.943 (0.88), 3.970 (0.51), 4.761 (1.17), 4.769 (0.81), 4.951 (1.69), 4.959 (2.06), 4.965 (2.20), 4.974 (1.76), 5.004 (9.69), 7.908 (10.94), 7.912 (16.00), 8.642 (3.82).

### Beispiel 201

### (5S)-5-[(1SR,5RS)-2-Azabicyclo[3.1.0]hex-2-ylcarbonyl]-2-{[6-(trifluormethyl)pyridin-3-yl]methyl}-5,6,7,8-tetrahydro[1,2,4]triazolo[4,3-a]pyridin-3(2H)-on (Isomer 2)

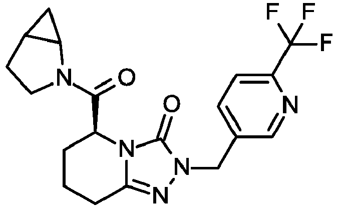

(5S)-5-[(1RS,5SR)-2-Azabicyclo[3.1.0]hex-2-ylcarbonyl]-2-{[6-(trifluormethyl)pyridin-3-yl]methyl}-5,6,7,8-tetrahydro[1,2,4]triazolo[4,3-a]pyridin-3(2H)-on (Diastereomerengemisch; 2 Isomere) wurde durch chirale präparative HPLC getrennt [Probenvorbereitung: 54 mg gelöst in 20 ml Methanol; Injektionsvolumen: 1 ml; Säule: Daicel Chiralpak^{®} IE 5 µm, 250 × 20 mm; Laufmittel: Kohlendioxid/Methanol 80:20; Fluss: 80 ml/min; Temperatur 40°C; UV-Detektion: 210 nm]. Nach der Trennung wurden 20.2 mg von Isomer 1, welches zuerst eluiert, und 21.3 mg von Isomer 2, welches später eluiert, isoliert.

### Isomer 2:

Analytische chirale HPLC: Rₜ = 6.82 min, d.e. > 99.5% [column: Daicel Chiralpak^{®} IE, 50 x 4.6 mm; Laufmittel: n-Heptan/Methanol 80:20; Fluss: 3 ml/min; UV-Detektion: 210 nm].
LC-MS (Methode 3): Rₜ = 1.33 min; MS (ESIpos): m/z = 408 [M+H]⁺
¹H-NMR (400 MHz, DMSO-d6) δ [ppm]: -0.149 (0.51), -0.008 (4.45), 0.008 (4.11), 0.146 (0.51), 0.559 (0.56), 0.625 (1.18), 0.630 (1.41), 0.638 (2.42), 0.644 (2.42), 0.651 (1.63), 0.657 (1.30), 0.731 (0.45), 0.751 (0.51), 0.803 (0.96), 0.816 (2.03), 0.838 (2.03), 0.852 (0.96), 1.579 (1.18), 1.745 (1.35), 1.759 (2.37), 1.773 (2.42), 1.794 (2.03), 1.865 (0.90), 1.879 (1.01), 1.889 (1.69), 1.896 (1.35), 1.911 (1.30), 1.918 (1.18), 1.992 (0.90), 2.031 (1.80), 2.039 (1.58), 2.047 (1.58), 2.059 (1.75), 2.185 (1.52), 2.220 (0.90), 2.327 (1.13), 2.332 (0.85), 2.366 (0.79), 2.523 (4.39), 2.563 (2.37), 2.576 (2.03), 2.590 (1.80), 2.609 (2.42), 2.622 (1.63), 2.650 (0.96), 2.665 (1.35), 2.670 (1.41), 2.674 (1.01), 2.710 (0.90), 2.938 (0.90), 2.960 (2.08), 2.970 (1.24), 2.982 (1.01), 2.992 (2.14), 3.014 (0.85), 3.241 (0.51), 3.548 (0.73), 3.554 (0.73), 3.709 (2.42), 3.718 (2.93), 3.725 (2.54), 3.733 (2.76), 3.740 (3.21), 3.764 (1.13), 3.808 (0.51), 4.675 (0.68), 4.975 (0.90), 5.015 (7.21), 5.026 (6.42), 5.067 (0.90), 5.105 (2.03), 5.112 (2.48), 5.120 (2.31), 5.128 (1.97), 7.912 (15.94), 7.915 (16.00), 8.646 (5.41).

### Beispiel 202

### (5S)-5-[(1RS,5SR)-2-Azabicyclo[3.1.0]hex-2-ylcarbonyl]-2-(3-chlor-4-fluorbenzyl)-5,6,7,8-tetrahydro[1,2,4]triazolo[4,3-a]pyridin-3(2H)-on (Isomer 1)

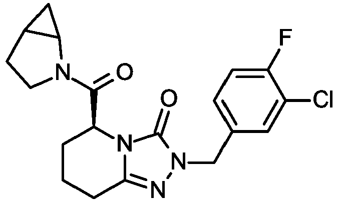

(5S)-2-(3-Chlor-4-fluorbenzyl)-3-oxo-2,3,5,6,7,8-hexahydro[1,2,4]triazolo[4,3-a]pyridin-5-carbonsäure (533 mg, 1.64 mmol) wurde in THF (1.5 ml) bei Raumtemperatur vorgelegt. Anschließend wurden HATU (809 mg, 2.13 mmol), (1RS,5SR)-2-Azabicyclo[3.1.0]hexanhydrochlorid (235 mg, 1.96 mmol) und N,N-Diisopropylethylamin (860 µl, 4.9 mmol) zugegeben. Es wurden erneut HATU (622 mg, 1.64 mmol), (1RS,5SR)-2-Azabicyclo[3.1.0]hexanhydrochlorid (196 mg, 1.63 mmol) und N,N-Diisopropylethylamin (287 µl, 1.6 mmol) zugegeben und für weitere 20 Stunden gerührt. Das Reaktionsgemisch wurde im Vakuum eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 112 mg (18 % d. Th.) eines Diastereomerengemisches (2 Isomere) erhalten.

Das Diastereomerengemisch (2 Isomere) wurde durch chirale präparative HPLC getrennt [Probenvorbereitung: 112 mg gelöst in 2 ml Ethanol; Injektionsvolumen: 0.35 ml; Säule: Daicel Chiralpak^{®} AY-H 5 µm, 250 × 20 mm; Laufmittel: n-Heptan/Ethanol 40:60; Fluss: 15 ml/min; Temperatur 40°C; UV-Detektion: 220 nm]. Nach der Trennung wurden 42.1 mg von Isomer 1, welches zuerst eluiert, und 63.6 mg von Isomer 2, welches später eluiert, isoliert.

### Isomer 1:

Analytische chirale HPLC: Rₜ = 7.43 min, d.e. = 99% [column: Daicel Chiralpak^{®} AY-H 5 µm, 250 x 4.6 mm; Laufmittel: n-Heptan/Ethanol 50:50; Fluss: 1 ml/min; UV-Detektion: 220 nm].
LC-MS (Methode 3): Rₜ = 1.49 min; MS (ESIpos): m/z = 391 [M+H]⁺
¹H-NMR (400 MHz, DMSO-d6) δ [ppm]: -0.149 (0.75), -0.008 (6.67), 0.008 (5.91), 0.146 (0.75), 0.626 (1.68), 0.633 (2.55), 0.639 (2.55), 0.646 (3.07), 0.652 (3.01), 0.659 (1.86), 0.665 (1.62), 0.676 (0.75), 0.693 (1.22), 0.713 (1.22), 0.727 (0.52), 0.870 (1.16), 0.885 (2.26), 0.892 (1.51), 0.906 (2.38), 0.920 (0.99), 1.548 (0.81), 1.569 (0.99), 1.583 (0.87), 1.657 (1.45), 1.692 (1.51), 1.711 (1.39), 1.756 (3.19), 1.770 (2.84), 1.791 (2.09), 1.830 (0.99), 1.840 (1.04), 1.851 (1.16), 1.861 (2.26), 1.871 (1.51), 1.882 (1.45), 1.893 (1.33), 1.946 (1.68), 1.955 (2.09), 1.988 (1.51), 2.008 (1.91), 2.025 (1.51), 2.051 (2.55), 2.059 (2.43), 2.085 (1.22), 2.131 (1.57), 2.139 (1.39), 2.148 (1.62), 2.159 (1.57), 2.168 (1.45), 2.328 (1.04), 2.366 (0.87), 2.523 (4.52), 2.564 (2.61), 2.575 (2.38), 2.591 (2.67), 2.606 (4.00), 2.619 (2.09), 2.635 (0.99), 2.648 (1.51), 2.665 (1.16), 2.670 (1.28), 2.710 (0.93), 3.106 (0.46), 3.131 (2.09), 3.151 (2.96), 3.161 (1.91), 3.172 (1.39), 3.182 (2.67), 3.202 (1.28), 3.483 (1.57), 3.489 (1.74), 3.499 (2.72), 3.504 (2.67), 3.514 (1.74), 3.519 (1.45), 3.582 (1.33), 3.591 (1.45), 3.607 (1.74), 3.616 (1.86), 3.622 (1.57), 3.638 (1.28), 3.648 (1.16), 3.664 (0.81), 3.670 (0.93), 3.680 (1.74), 3.686 (1.62), 3.696 (0.93), 3.918 (0.70), 3.944 (1.28), 3.967 (0.64), 4.736 (1.16), 4.751 (1.68), 4.760 (1.16), 4.817 (16.00), 4.825 (9.39), 4.942 (2.43), 4.950 (2.84), 4.957 (3.01), 4.966 (2.32), 7.228 (2.03), 7.234 (2.20), 7.243 (2.67), 7.248 (3.01), 7.255 (2.84), 7.260 (2.61), 7.372 (5.62), 7.395 (7.59), 7.417 (4.35), 7.435 (3.71), 7.453 (3.71).

### Beispiel 203

### (5S)-5-[(1SR,5RS)-2-Azabicyclo[3.1.0]hex-2-ylcarbonyl]-2-(3-chlor-4-fluorbenzyl)-5,6,7,8-tetrahydro[1,2,4]triazolo[4,3-a]pyridin-3(2H)-on (Isomer 2)

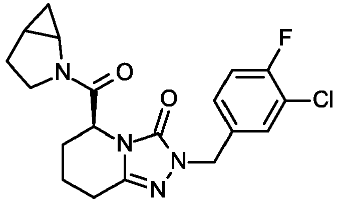

(5S)-5-[(1SR,5RS)-2-Azabicyclo[3.1.0]hex-2-ylcarbonyl]-2-(3-chlor-4-fluorbenzyl)-5,6,7,8-tetrahydro[1,2,4]triazolo[4,3-a]pyridin-3(2H)-on (Diastereomerengemisch; 2 Isomere) wurde durch chirale präparative HPLC getrennt [Probenvorbereitung: 112 mg gelöst in 2 ml Ethanol; Injektionsvolumen: 0.35 ml; Säule: Daiceandrel Chiralpak^{®} AY-H 5 µm, 250 × 20 mm; Laufmittel: n-Heptan/Ethanol 40:60; Fluss: 15 ml/min; Temperatur 40°C; UV-Detektion: 220 nm]. Nach der Trennung wurden 42.1 mg von Isomer 1, welches zuerst eluiert, und 63.6 mg von Isomer 2, welches später eluiert, isoliert.

### Isomer 2:

Analytische chirale HPLC: Rₜ = 10.06 min, d.e. = 99% [Säule: Daicel Chiralpak^{®} AY-H 5 µm, 250 x 4.6 mm; Laufmittel: n-Heptan/Ethanol 50:50; Fluss: 1 ml/min; UV-Detektion: 220 nm].
LC-MS (Methode 3): Rₜ = 1.50 min; MS (ESIpos): m/z = 391 [M+H]⁺
¹H-NMR (400 MHz, DMSO-d6) δ [ppm]: -0.008 (4.82), 0.008 (3.02), 0.564 (0.72), 0.577 (0.47), 0.619 (1.70), 0.625 (1.95), 0.632 (3.12), 0.638 (3.02), 0.645 (2.02), 0.651 (1.67), 0.729 (0.60), 0.750 (0.60), 0.798 (1.29), 0.813 (2.58), 0.820 (1.70), 0.826 (1.61), 0.834 (2.55), 0.848 (1.13), 1.554 (1.32), 1.567 (1.48), 1.575 (1.42), 1.590 (1.51), 1.726 (1.32), 1.741 (2.27), 1.755 (3.06), 1.770 (2.93), 1.776 (2.83), 1.791 (2.30), 1.806 (0.94), 1.855 (1.07), 1.862 (1.20), 1.877 (1.45), 1.886 (2.14), 1.894 (1.83), 1.909 (1.83), 1.915 (1.73), 1.973 (0.57), 1.994 (1.10), 2.007 (1.39), 2.018 (2.30), 2.026 (2.30), 2.032 (2.30), 2.041 (2.39), 2.053 (2.61), 2.061 (2.39), 2.084 (1.39), 2.099 (1.01), 2.161 (2.08), 2.195 (1.20), 2.328 (0.60), 2.366 (0.54), 2.524 (3.59), 2.559 (2.80), 2.572 (2.46), 2.586 (2.14), 2.597 (2.14), 2.606 (2.93), 2.619 (1.98), 2.638 (0.88), 2.647 (1.07), 2.661 (0.82), 2.670 (0.69), 2.710 (0.50), 2.935 (1.23), 2.957 (2.65), 2.966 (1.57), 2.979 (1.45), 2.989 (2.77), 3.010 (1.13), 3.243 (0.76), 3.528 (0.47), 3.534 (0.50), 3.544 (0.88), 3.549 (0.85), 3.559 (0.47), 3.705 (2.93), 3.714 (3.46), 3.721 (3.31), 3.733 (3.69), 3.758 (1.42), 3.765 (1.23), 3.778 (0.44), 3.805 (0.66), 4.649 (0.69), 4.658 (0.79), 4.664 (0.82), 4.674 (0.60), 4.821 (4.98), 4.833 (16.00), 5.093 (2.68), 5.100 (3.18), 5.108 (2.87), 5.115 (2.46), 7.226 (2.02), 7.232 (2.11), 7.238 (2.20), 7.244 (2.52), 7.248 (2.83), 7.253 (2.68), 7.260 (2.52), 7.265 (2.14), 7.372 (4.63), 7.396 (6.17), 7.417 (3.62), 7.437 (3.94), 7.442 (3.75), 7.455 (3.87), 7.460 (3.53).

### Beispiel 204

### (5S)-2-[(E)-2-(4-Fluorphenyl)vinyl]-5-(pyrrolidin-1-ylcarbonyl)-5,6,7,8-tetrahydro[1,2,4]triazolo[4,3-a]pyridin-3(2H)-on

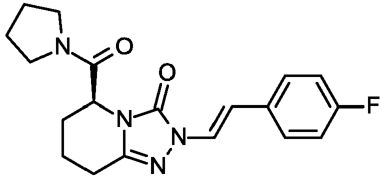

(5S)-2-[(E)-2-(4-Fluorphenyl)vinyl]-3-oxo-2,3,5,6,7,8-hexahydro[1,2,4]triazolo[4,3-a]pyridin-5-carbonsäure (195 mg, 643 µmol) wurde in THF (6.0 ml) bei Raumtemperatur vorgelegt. Anschließend wurden HBTU (317 mg, 836 µmol) und N,N-Diisopropylethylamin (560 µl, 3.2 mmol) zugegeben. Nach 15 min Rühren wurde Pyrrolidin (64 µl, 770 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm × 40mm; Laufmittel: Acetonitril/ mit 0.1% Ameisensäure-Gradient). Die produkthaltigen Fraktionen wurden im Vakuum eingeengt und es wurden 68.5 mg (30 % d. Th.) der Titelverbindung erhalten.
LC-MS (Methode 3): Rₜ = 1.58 min; MS (ESIpos): m/z = 357 [M+H]⁺
¹H-NMR (400 MHz, DMSO-d6) δ [ppm]: -0.149 (0.95), -0.008 (8.77), 0.008 (8.91), 0.146 (1.02), 1.288 (0.66), 1.732 (1.97), 1.743 (1.83), 1.768 (4.16), 1.783 (8.84), 1.800 (12.20), 1.817 (9.42), 1.832 (3.00), 1.893 (1.17), 1.905 (2.63), 1.922 (7.96), 1.938 (10.08), 1.955 (6.06), 1.973 (1.75), 1.988 (1.61), 2.014 (2.05), 2.024 (3.14), 2.047 (2.56), 2.056 (1.97), 2.062 (1.97), 2.072 (2.41), 2.085 (3.43), 2.091 (2.19), 2.099 (1.97), 2.327 (1.39), 2.366 (1.39), 2.523 (5.11), 2.613 (1.32), 2.627 (1.46), 2.638 (1.17), 2.655 (3.58), 2.670 (4.75), 2.680 (3.58), 2.695 (3.00), 2.704 (3.00), 2.716 (5.11), 2.728 (2.92), 2.746 (1.24), 2.758 (1.83), 2.771 (0.88), 3.238 (1.39), 3.256 (3.21), 3.268 (3.51), 3.285 (6.94), 3.344 (7.67), 3.356 (3.21), 3.361 (3.87), 3.374 (3.73), 3.391 (1.90), 3.457 (1.83), 3.474 (3.80), 3.482 (2.85), 3.491 (2.34), 3.499 (4.89), 3.516 (2.19), 3.611 (2.26), 3.627 (4.53), 3.635 (2.34), 3.644 (2.70), 3.652 (3.65), 3.669 (1.61), 4.791 (4.09), 4.800 (4.68), 4.806 (5.33), 4.815 (3.95), 5.754 (1.61), 6.747 (8.77), 6.783 (9.94), 7.121 (7.67), 7.144 (16.00), 7.166 (8.62), 7.330 (11.69), 7.367 (10.37), 7.519 (8.18), 7.533 (9.28), 7.541 (8.99), 7.555 (7.67).

### Beispiel 205

### (5S)-5-[(1RS,5SR)-2-Azabicyclo[3.1.0]hex-2-ylcarbonyl]-2-{[3-fluor-2-(trifluormethyl)pyridin-4-yl]methyl}-5,6,7,8-tetrahydro[1,2,4]triazolo[4,3-a]pyridin-3(2H)-on (Diastereomerengemisch; 2 Isomere)

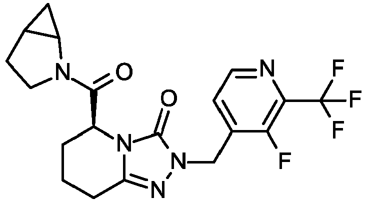

(5S)-2-{[3-Fluor-2-(trifluormethyl)pyridin-4-yl]methyl}-3-oxo-2,3,5,6,7,8-hexahydro[1,2,4]triazolo[4,3-a]pyridin-5-carbonsäure (52.0 mg, 144 µmol) wurde in THF (130 µl) bei Raumtemperatur vorgelegt. Anschließend wurden HATU (71.3 mg, 188 µmol), N,N-Diisopropylethylamin (76 µl, 430 µmol) und (1RS,5SR)-2-Azabicyclo[3.1.0]hexanhydrochlorid (20.7 mg, 173 µmol) zugegeben. Das Reaktionsgemisch wurde über Nacht bei Raumtemperatur gerührt. Das Reaktionsgemisch wurde im Vakuum eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 35.0 mg (57 % d. Th.) der Titelverbindung erhalten.
LC-MS (Methode 3): Rₜ = 1.40 min; MS (ESIpos): m/z = 426 [M+H]⁺
¹H-NMR (400 MHz, DMSO-d6) δ [ppm]: -0.149 (1.74), -0.008 (16.00), 0.008 (14.61), 0.146 (1.74), 0.562 (0.63), 0.640 (2.99), 0.728 (0.49), 0.805 (0.90), 0.820 (1.88), 0.842 (1.88), 0.856 (0.83), 0.907 (0.56), 1.595 (1.25), 1.773 (3.20), 1.795 (2.43), 1.861 (1.11), 1.889 (1.67), 1.912 (1.25), 1.991 (1.32), 2.071 (2.02), 2.192 (1.67), 2.327 (2.23), 2.366 (2.16), 2.523 (7.51), 2.563 (2.92), 2.578 (2.37), 2.592 (2.09), 2.625 (2.92), 2.670 (3.27), 2.710 (2.16), 2.942 (0.90), 2.965 (1.81), 2.974 (1.04), 2.996 (2.09), 3.016 (0.83), 3.151 (0.56), 3.512 (0.63), 3.556 (0.63), 3.715 (2.23), 3.740 (2.99), 3.765 (1.04), 3.814 (0.49), 4.682 (0.63), 4.777 (0.42), 4.977 (0.63), 5.076 (9.25), 5.122 (2.23), 5.129 (2.30), 5.138 (2.16), 7.549 (2.50), 7.562 (4.52), 7.575 (2.71), 8.562 (4.94), 8.574 (5.08).

### Beispiel 206

### (5S)-2-{[6-(Difluormethyl)pyridin-3-yl]methyl}-5-{[(3S)-3-fluorpyrrolidin-1 -yl]carbonyl}-5,6,7,8-tetrahydro[1,2,4]triazolo[4,3-a]pyridin-3(2H)-on

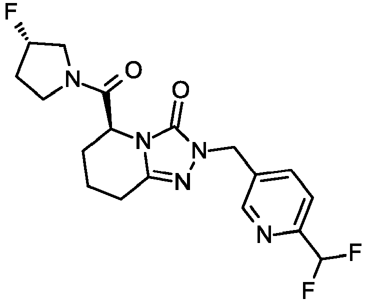

(5S)-2-{[6-(Difluormethyl)pyridin-3-yl]methyl}-3-oxo-2,3,5,6,7,8-hexahydro[1,2,4]triazolo[4,3-a]pyridin-5-carbonsäure (100 mg, 25 % Reinheit, 77.1 µmol) wurde in THF (800 µl) bei Raumtemperatur vorgelegt. Anschließend wurden (3S)-3-Fluorpyrrolidin (8.24 mg, 92.5 µmol), HATU (38.1 mg, 100 µmol) und Triethylamin (54 µl, 390 µmol) zugegeben. Das Reaktionsgemisch wurde über Nacht bei Raumtemperatur gerührt. Das Reaktionsgemisch wurde mit Wasser versetzt und im Vakuum eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 15.8 mg (52 % d. Th.) der Titelverbindung erhalten.
LC-MS (Methode 3): Rₜ = 1.05 min; MS (ESIpos): m/z = 396 [M+H]⁺
¹H-NMR (400 MHz, DMSO-d6) δ [ppm]: -0.149 (0.69), 0.146 (0.74), 0.950 (0.66), 1.007 (0.42), 1.732 (3.05), 1.874 (0.54), 1.914 (0.71), 1.998 (2.72), 2.026 (1.96), 2.090 (2.37), 2.106 (2.49), 2.137 (1.90), 2.268 (1.44), 2.327 (1.00), 2.366 (0.80), 2.565 (3.31), 2.605 (4.05), 2.648 (1.58), 2.665 (1.37), 2.710 (0.88), 3.273 (1.54), 3.302 (2.23), 3.320 (2.42), 3.368 (3.38), 3.396 (3.86), 3.406 (3.83), 3.468 (4.74), 3.494 (5.02), 3.523 (5.54), 3.546 (4.81), 3.572 (4.14), 3.599 (4.61), 3.636 (4.12), 3.654 (3.67), 3.679 (3.24), 3.705 (2.27), 3.725 (2.15), 3.745 (3.12), 3.775 (2.34), 3.786 (2.18), 3.857 (2.61), 3.918 (0.97), 3.943 (1.47), 4.008 (0.83), 4.039 (0.69), 4.688 (0.97), 4.698 (1.25), 4.704 (1.28), 4.713 (1.00), 4.746 (1.23), 4.761 (1.63), 4.769 (1.26), 4.818 (0.85), 4.831 (1.23), 4.870 (1.11), 4.955 (16.00), 5.261 (1.33), 5.350 (0.68), 5.392 (1.59), 5.481 (0.69), 5.515 (0.81), 6.807 (4.02), 6.944 (7.57), 7.081 (3.72), 7.682 (5.61), 7.702 (7.55), 7.819 (4.74), 7.839 (3.67), 8.563 (6.89).

### Beispiel 207

### (5S)-2-{[6-(Difluormethyl)pyridin-3-yl]methyl}-5-{[(3R,4S)-3,4-difluorpyrrolidin-1 -yl]carbonyl}-5,6,7,8-tetrahydro[1,2,4]triazolo[4,3-a]pyridin-3(2H)-on

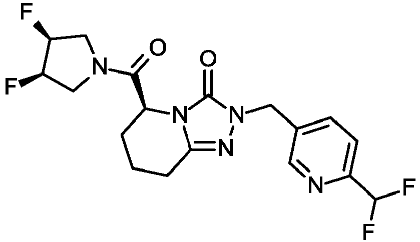

(5S)-2-{[6-(Difluormethyl)pyridin-3-yl]methyl}-3-oxo-2,3,5,6,7,8-hexahydro[1,2,4]triazolo[4,3-a]pyridin-5-carbonsäure (100 mg, 25 % Reinheit, 77.1 µmol) wurde in THF (800 µl) bei Raumtemperatur vorgelegt. Anschließend wurden (3R,4S)-3,4-Difluorpyrrolidin (9.91 mg, 92.5 µmol), HATU (38.1 mg, 100 µmol) und Triethylamin (54 µl, 390 µmol) zugegeben. Das Reaktionsgemisch wurde über Nacht bei Raumtemperatur gerührt. Das Reaktionsgemisch wurde mit Wasser versetzt und im Vakuum eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 16.4 mg (49 % d. Th.) der Titelverbindung erhalten.
LC-MS (Methode 3): Rₜ = 1.13 min; MS (ESIpos): m/z = 414 [M+H]⁺
¹H-NMR (400 MHz, DMSO-d6) δ [ppm]: -0.154 (1.88), -0.013 (16.00), 0.141 (1.83), 0.941 (0.39), 1.657 (0.55), 1.729 (0.61), 1.769 (0.33), 1.952 (0.39), 1.966 (0.55), 2.013 (0.66), 2.032 (0.50), 2.055 (0.55), 2.067 (0.50), 2.076 (0.50), 2.089 (0.44), 2.125 (0.33), 2.322 (2.33), 2.327 (1.77), 2.361 (2.05), 2.518 (5.92), 2.561 (1.38), 2.572 (0.94), 2.585 (0.89), 2.598 (1.33), 2.660 (1.83), 2.664 (2.44), 2.705 (1.94), 3.347 (0.39), 3.448 (0.33), 3.486 (0.39), 3.530 (0.50), 3.612 (0.33), 3.665 (0.39), 3.681 (0.55), 3.698 (0.66), 3.723 (0.55), 3.750 (0.44), 3.763 (0.39), 3.921 (0.39), 3.966 (0.33), 3.983 (0.39), 4.170 (0.39), 4.789 (0.89), 4.801 (1.27), 4.952 (5.04), 5.242 (0.39), 5.250 (0.39), 5.282 (0.33), 5.327 (0.44), 5.344 (0.39), 5.359 (0.33), 5.386 (0.44), 5.395 (0.50), 5.412 (0.39), 5.425 (0.33), 5.453 (0.39), 5.491 (0.39), 6.802 (0.94), 6.939 (2.05), 7.076 (1.00), 7.676 (1.38), 7.697 (1.88), 7.812 (1.27), 7.833 (1.00), 8.556 (2.10).

### Beispiel 208

### (5S)-2-{[6-(Difluormethyl)pyridin-3-yl]methyl}-5-[(3,3-difluorpyrrolidin-1-yl)carbonyl]-5,6,7,8-tetrahydro[1,2,4]triazolo[4,3-a]pyridin-3(2H)-on

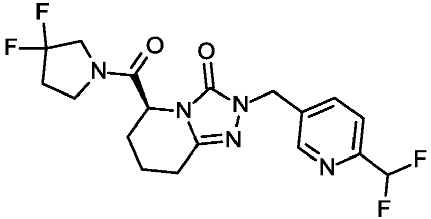

(5S)-2-{[6-(Difluormethyl)pyridin-3-yl]methyl}-3-oxo-2,3,5,6,7,8-hexahydro[1,2,4]triazolo[4,3-a]pyridin-5-carbonsäure (100 mg, 25 % Reinheit, 77.1 µmol) wurde in THF (800 µl) bei Raumtemperatur vorgelegt. Anschließend wurden 3,3-Difluorpyrrolidin (9.91 mg, 92.5 µmol), HATU (38.1 mg, 100 µmol) und Triethylamin (54 µl, 390 µmol) zugegeben. Das Reaktionsgemisch wurde über Nacht bei Raumtemperatur gerührt. Das Reaktionsgemisch wurde mit Wasser versetzt und im Vakuum eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Die produkthaltigen Fraktionen wurden im Vakuum eingeengt und es wurden 24.3 mg (74 % d. Th.) der Titelverbindung erhalten.
LC-MS (Methode 3): Rₜ = 1.20 min; MS (ESIpos): m/z = 414 [M+H]⁺
¹H-NMR (400 MHz, DMSO-d6) δ [ppm]: -0.149 (1.38), 0.146 (1.30), 1.041 (1.65), 1.673 (1.23), 1.731 (1.69), 2.002 (1.76), 2.012 (1.88), 2.047 (1.38), 2.327 (1.50), 2.366 (1.73), 2.381 (1.04), 2.410 (1.27), 2.431 (1.11), 2.563 (3.80), 2.573 (3.95), 2.588 (3.80), 2.605 (3.84), 2.648 (1.30), 2.670 (2.00), 2.710 (1.92), 2.884 (1.00), 3.533 (1.38), 3.541 (1.61), 3.550 (2.19), 3.560 (2.42), 3.580 (1.19), 3.636 (0.50), 3.668 (1.46), 3.703 (1.69), 3.747 (0.88), 3.782 (2.30), 3.811 (2.57), 3.829 (0.81), 3.891 (0.77), 3.909 (1.61), 3.935 (1.15), 3.953 (0.50), 3.993 (0.96), 4.021 (0.77), 4.037 (0.92), 4.065 (0.61), 4.148 (0.58), 4.179 (0.92), 4.206 (0.92), 4.759 (1.27), 4.774 (1.88), 4.784 (1.38), 4.831 (1.27), 4.846 (1.76), 4.856 (1.27), 4.957 (16.00), 6.806 (3.72), 6.943 (7.10), 7.081 (3.41), 7.682 (4.87), 7.702 (6.71), 7.820 (3.84), 7.840 (2.95), 8.157 (0.81), 8.563 (5.14).

### Beispiel 209

### (5S)-2-{[6-(Difluormethyl)pyridin-3-yl]methyl}-5-[(3-fluorazetidin-1-yl)carbonyl]-5,6,7,8-tetrahydro[1,2,4]triazolo[4,3-a]pyridin-3(2H)-on

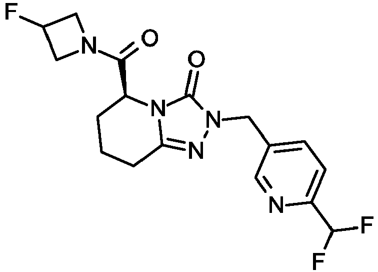

(5S)-2-{[6-(Difluormethyl)pyridin-3-yl]methyl}-3-oxo-2,3,5,6,7,8-hexahydro[1,2,4]triazolo[4,3-a]pyridin-5-carbonsäure (100 mg, 25 % Reinheit, 77.1 µmol) wurde in THF (800 µl) bei Raumtemperatur vorgelegt. Anschließend wurden 3-Fluorazetidin (6.95 mg, 92.5 µmol), HATU (38.1 mg, 100 µmol) und Triethylamin (54 µl, 390 µmol) zugegeben. Das Reaktionsgemisch wurde über Nacht bei Raumtemperatur gerührt. Das Reaktionsgemisch wurde mit Wasser versetzt und im Vakuum eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 23.2 mg (77 % d. Th.) der Titelverbindung erhalten.
LC-MS (Methode 3): Rₜ = 1.01 min; MS (ESIpos): m/z = 382 [M+H]⁺
¹H-NMR (400 MHz, DMSO-d6) δ [ppm]: -0.149 (1.89), -0.031 (0.40), -0.026 (0.55), -0.024 (0.65), -0.020 (0.84), -0.018 (1.09), -0.015 (1.39), -0.008 (16.00), -0.007 (12.42), 0.008 (15.60), 0.015 (1.74), 0.018 (0.94), 0.021 (0.70), 0.026 (0.45), 0.028 (0.40), 0.033 (0.35), 0.146 (1.94), 0.990 (0.55), 1.701 (1.19), 1.709 (1.24), 1.939 (0.60), 1.949 (0.75), 1.965 (0.80), 1.975 (0.80), 2.008 (0.70), 2.323 (1.64), 2.327 (2.43), 2.332 (1.84), 2.366 (1.79), 2.445 (0.45), 2.464 (0.70), 2.523 (5.42), 2.526 (4.02), 2.558 (2.09), 2.562 (1.94), 2.566 (1.44), 2.569 (1.19), 2.572 (1.19), 2.579 (1.84), 2.594 (2.34), 2.606 (1.29), 2.622 (0.75), 2.636 (0.80), 2.649 (0.45), 2.665 (1.79), 2.670 (2.34), 2.674 (1.84), 2.709 (1.74), 3.930 (0.55), 3.963 (0.60), 3.994 (0.50), 4.023 (0.40), 4.173 (0.40), 4.216 (0.35), 4.225 (0.50), 4.238 (0.50), 4.255 (0.50), 4.273 (0.65), 4.296 (0.55), 4.326 (0.45), 4.367 (0.45), 4.393 (0.35), 4.432 (0.40), 4.459 (0.45), 4.504 (0.40), 4.521 (0.35), 4.548 (1.44), 4.561 (2.14), 4.573 (1.49), 4.688 (0.35), 4.715 (0.35), 4.958 (5.47), 5.351 (0.35), 5.407 (0.40), 5.496 (0.40), 6.805 (1.64), 6.942 (3.03), 7.080 (1.59), 7.680 (1.84), 7.700 (2.48), 7.823 (1.74), 7.842 (1.34), 8.568 (2.68).

### Beispiel 210

### (5S)-2-(4-Brombenzyl)-5-{[(3R,4S)-3,4-difluorpyrrolidin-1 -yl]carbonyl}-5,6,7,8-tetrahydro[1,2,4]triazolo[4,3-a]pyridin-3(2H)-on

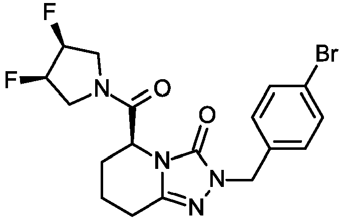

### (5S)-2-(4-Brombenzyl)-3-oxo-2,3,5,6,7,8-hexahydro[1,2,4]triazolo[4,3-a]pyridin-5-carbonsäure

(134 mg, 47 % Reinheit, 179 µmol) wurde in THF (2.6 ml) bei Raumtemperatur vorgelegt. Anschließend wurden HBTU (88.1 mg, 232 µmol) und N,N-Diisopropylethylamin (250 µl, 1.4 mmol) zugegeben. Nach 5 min Rühren wurde (3R,4S)-3,4-Difluorpyrrolidinhydrochlorid (30.8 mg, 214 µmol) zugeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 54.3 mg (69 % d. Th.) der Titelverbindung erhalten.
LC-MS (Methode 3): Rₜ = 1.47 min; MS (ESIpos): m/z = 441 [M+H]⁺
¹H-NMR (400 MHz, DMSO-d6) δ [ppm]: 1.174 (0.60), 1.664 (1.05), 1.725 (1.31), 1.925 (0.44), 1.959 (1.00), 1.988 (1.71), 2.004 (1.29), 2.029 (0.82), 2.038 (0.89), 2.046 (0.97), 2.054 (0.92), 2.063 (0.77), 2.073 (0.69), 2.081 (0.76), 2.089 (0.65), 2.328 (0.44), 2.558 (2.09), 2.569 (1.44), 2.583 (1.90), 2.596 (2.49), 2.608 (1.43), 2.628 (0.55), 2.639 (0.85), 2.669 (0.53), 3.452 (0.51), 3.486 (0.85), 3.507 (0.62), 3.524 (0.76), 3.533 (1.02), 3.542 (0.96), 3.566 (0.44), 3.575 (0.63), 3.586 (0.41), 3.612 (0.67), 3.626 (0.77), 3.645 (0.46), 3.659 (0.54), 3.668 (0.75), 3.682 (1.08), 3.701 (1.39), 3.715 (0.85), 3.724 (0.88), 3.733 (0.89), 3.753 (0.84), 3.764 (0.80), 3.786 (0.45), 3.799 (0.43), 3.863 (0.66), 3.905 (0.49), 3.925 (0.65), 3.939 (0.74), 3.953 (0.43), 3.974 (0.70), 3.988 (0.71), 4.003 (0.56), 4.020 (0.50), 4.127 (0.45), 4.142 (0.52), 4.156 (0.50), 4.169 (0.84), 4.183 (0.56), 4.196 (0.49), 4.211 (0.46), 4.789 (16.00), 5.254 (0.70), 5.264 (0.73), 5.275 (0.83), 5.285 (0.70), 5.296 (0.53), 5.310 (0.50), 5.327 (0.60), 5.336 (0.57), 5.349 (0.66), 5.364 (0.57), 5.377 (0.74), 5.387 (0.87), 5.398 (0.73), 5.406 (0.76), 5.416 (0.71), 5.428 (0.49), 5.439 (0.50), 5.448 (0.55), 5.472 (0.61), 5.479 (0.63), 5.492 (0.48), 7.173 (4.16), 7.180 (4.90), 7.194 (5.08), 7.201 (5.37), 7.527 (7.08), 7.545 (5.88), 7.548 (5.94).

### Beispiel 211

### (5S)-2-(3-Brombenzyl)-5-{[(3R,4S)-3,4-difluorpyrrolidin-1 -yl]carbonyl}-5,6,7,8-tetrahydro[1,2,4]triazolo[4,3-a]pyridin-3(2H)-on

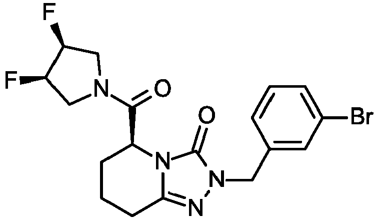

### (5S)-2-(3-Brombenzyl)-3-oxo-2,3,5,6,7,8-hexahydro[1,2,4]triazolo[4,3-a]pyridin-5-carbonsäure

(128 mg, 59 % Reinheit, 215 µmol) wurde in THF (3.1 ml) bei Raumtemperatur vorgelegt. Anschließend wurden HBTU (106 mg, 279 µmol) und N,N-Diisopropylethylamin (300 µl, 1.7 mmol) zugegeben. Nach 5 min Rühren wurde (3R,4S)-3,4-Difluorpyrrolidinhydrochlorid (37.0 mg, 258 µmol) zugeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 23.2 mg (24 % d. Th.) der Titelverbindung erhalten.
LC-MS (Methode 3): Rₜ = 1.50 min; MS (ESIpos): m/z = 441 [M+H]⁺
¹H-NMR (400 MHz, DMSO-d6) δ [ppm]: 1.670 (1.28), 1.695 (0.91), 1.730 (1.61), 1.928 (0.54), 1.962 (1.20), 2.011 (1.55), 2.019 (1.18), 2.036 (0.94), 2.046 (1.09), 2.054 (1.18), 2.062 (1.15), 2.073 (1.00), 2.081 (0.85), 2.089 (0.96), 2.096 (0.81), 2.327 (0.54), 2.560 (1.83), 2.571 (2.22), 2.582 (1.65), 2.596 (2.31), 2.608 (3.03), 2.620 (1.72), 2.638 (0.65), 2.650 (1.00), 2.665 (0.85), 3.455 (0.57), 3.489 (1.00), 3.510 (0.74), 3.517 (0.80), 3.526 (0.87), 3.537 (1.22), 3.545 (1.15), 3.580 (0.80), 3.616 (0.78), 3.630 (0.91), 3.649 (0.54), 3.671 (0.89), 3.685 (1.24), 3.704 (1.54), 3.718 (0.98), 3.726 (1.29), 3.740 (0.89), 3.757 (1.05), 3.769 (1.07), 3.791 (0.50), 3.803 (0.52), 3.870 (0.80), 3.925 (0.81), 3.939 (0.85), 3.954 (0.50), 3.975 (0.85), 3.989 (0.81), 4.004 (0.52), 4.017 (0.46), 4.133 (0.54), 4.148 (0.59), 4.161 (0.57), 4.175 (0.96), 4.189 (0.65), 4.201 (0.57), 4.217 (0.52), 4.795 (2.35), 4.807 (3.61), 4.823 (16.00), 4.868 (0.41), 5.255 (0.83), 5.266 (0.87), 5.276 (0.98), 5.286 (0.81), 5.299 (0.61), 5.329 (0.70), 5.339 (0.70), 5.351 (0.78), 5.366 (0.68), 5.377 (0.91), 5.389 (1.05), 5.400 (0.83), 5.408 (0.89), 5.417 (0.83), 5.429 (0.59), 5.442 (0.59), 5.459 (0.74), 5.472 (0.72), 5.481 (0.74), 5.495 (0.57), 7.221 (1.92), 7.237 (3.02), 7.288 (2.18), 7.292 (2.61), 7.308 (3.75), 7.311 (4.42), 7.327 (1.78), 7.331 (2.05), 7.435 (5.42), 7.476 (3.16), 7.495 (2.57).

### Beispiel 212

### (5S)-2-(4-Brombenzyl)-5-{[(3S)-3-fluorpyrrolidin-1-yl]carbonyl}-5,6,7,8-tetrahydro[1,2,4]triazolo[4,3-a]pyridin-3(2H)-on

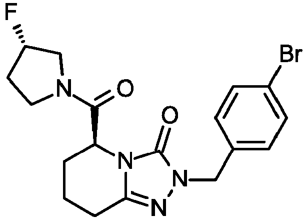

### (5S)-2-(4-Brombenzyl)-3-oxo-2,3,5,6,7,8-hexahydro[1,2,4]triazolo[4,3-a]pyridin-5-carbonsäure

(134 mg, 47 % Reinheit, 179 µmol) wurde in THF (2.6 ml) bei Raumtemperatur vorgelegt. Anschließend wurden HBTU (88.1 mg, 232 µmol) und N,N-Diisopropylethylamin (250 µl, 1.4 mmol) zugegeben. Nach 5 min Rühren wurde (3S)-3-Fluorpyrrolidinhydrochlorid (26.9 mg, 214 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 51.7 mg (66 % d. Th.) der Titelverbindung erhalten.
LC-MS (Methode 3): Rₜ = 1.40 min; MS (ESIpos): m/z = 423 [M+H]⁺
¹H-NMR (400 MHz, DMSO-d6) δ [ppm]: -0.149 (1.20), -0.008 (9.55), 0.008 (10.75), 0.146 (1.20), 0.940 (0.85), 0.956 (0.80), 1.728 (4.00), 1.913 (0.90), 2.027 (1.60), 2.085 (2.35), 2.133 (1.70), 2.267 (1.45), 2.327 (1.60), 2.366 (0.85), 2.558 (3.25), 2.573 (2.10), 2.600 (3.30), 2.642 (1.30), 2.670 (1.75), 2.709 (0.90), 3.268 (0.95), 3.287 (2.35), 3.344 (1.60), 3.367 (1.10), 3.392 (1.15), 3.464 (0.90), 3.491 (1.10), 3.606 (0.70), 3.633 (3.60), 3.651 (2.55), 3.657 (2.65), 3.675 (1.95), 3.700 (1.40), 3.722 (1.45), 3.742 (2.65), 3.765 (2.00), 3.786 (1.55), 3.855 (3.20), 4.674 (1.50), 4.684 (1.85), 4.690 (1.95), 4.699 (1.55), 4.731 (1.90), 4.746 (3.25), 4.756 (1.90), 4.785 (16.00), 4.830 (0.70), 5.258 (1.70), 5.389 (2.05), 5.510 (1.10), 7.175 (6.10), 7.181 (8.15), 7.196 (8.05), 7.202 (8.55), 7.520 (1.90), 7.526 (13.45), 7.547 (12.20).

### Beispiel 213

### (5S)-2-(3-Brombenzyl)-5-{[(3S)-3-fluorpyrrolidin-1 -yl]carbonyl}-5,6,7,8-tetrahydro[1,2,4]triazolo[4,3-a]pyridin-3(2H)-on

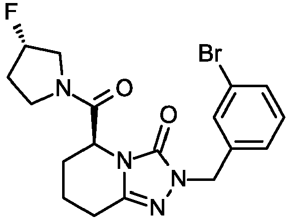

### (5S)-2-(3-Brombenzyl)-3-oxo-2,3,5,6,7,8-hexahydro[1,2,4]triazolo[4,3-a]pyridin-5-carbonsäure

(128 mg, 59 % Reinheit, 215 µmol) wurde in THF (3.1 ml) bei Raumtemperatur vorgelegt. Anschließend wurden HBTU (106 mg, 279 µmol) und N,N-Diisopropylethylamin (300 µl, 1.7 mmol) zugegeben. Nach 5 min Rühren wurde (3S)-3-Fluorpyrrolidinhydrochlorid (32.4 mg, 258 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 55.5 mg (61 % d. Th.) der Titelverbindung erhalten.
LC-MS (Methode 3): Rₜ = 1.40 min; MS (ESIpos): m/z = 423 [M+H]⁺
¹H-NMR (400 MHz, DMSO-d6) δ [ppm]: -0.149 (0.62), 0.146 (0.65), 1.719 (3.49), 1.732 (4.21), 1.871 (0.78), 1.881 (0.75), 1.906 (1.01), 1.995 (1.21), 2.008 (1.21), 2.021 (1.76), 2.064 (1.67), 2.089 (2.48), 2.101 (2.74), 2.126 (1.76), 2.138 (2.02), 2.220 (1.08), 2.242 (1.08), 2.269 (1.47), 2.327 (0.95), 2.366 (0.69), 2.561 (2.45), 2.570 (2.78), 2.587 (2.12), 2.613 (3.46), 2.654 (1.34), 2.669 (1.50), 2.710 (0.82), 3.273 (0.85), 3.347 (1.21), 3.369 (1.14), 3.397 (1.18), 3.405 (1.14), 3.458 (0.85), 3.466 (0.88), 3.494 (1.14), 3.503 (1.08), 3.613 (0.69), 3.638 (3.89), 3.655 (2.61), 3.663 (2.81), 3.680 (2.06), 3.704 (1.50), 3.722 (1.60), 3.742 (3.23), 3.770 (2.06), 3.785 (2.06), 3.856 (3.33), 4.691 (1.53), 4.700 (1.86), 4.706 (1.99), 4.715 (1.57), 4.747 (1.96), 4.756 (2.25), 4.762 (2.58), 4.772 (1.99), 4.819 (16.00), 4.866 (0.88), 5.260 (1.63), 5.390 (2.22), 5.512 (1.21), 7.222 (2.94), 7.241 (4.70), 7.291 (4.70), 7.311 (7.93), 7.330 (3.69), 7.436 (7.09), 7.475 (4.34), 7.495 (3.56).

### Beispiel 214

### (5S)-2-(4-Brom-2-fluorbenzyl)-5-{[(3S)-3-fluorpyrrolidin-1-yl]carbonyl}-5,6,7,8-tetrahydro[1,2,4]triazolo[4,3-a]pyridin-3(2H)-on

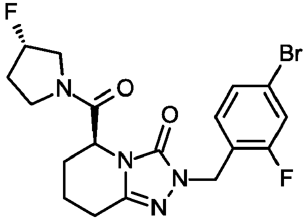

(5S)-2-(4-Brom-2-fluorbenzyl)-3-oxo-2,3,5,6,7,8-hexahydro[1,2,4]triazolo[4,3-a]pyridin-5-carbonsäure (85.0 mg, 223 µmol) wurde in THF (2.0 ml) bei Raumtemperatur vorgelegt. Anschließend wurden HBTU (110 mg, 290 µmol) und N,N-Diisopropylethylamin (190 µl, 1.1 mmol) zugegeben. Nach 5 min Rühren wurde (3S)-3-Fluorpyrrolidinhydrochlorid (33.6 mg, 267 µmol) zugegeben und das Reaktionsgemisch über das Wochenende bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Die produkthaltigen Fraktionen wurden im Vakuum eingeengt und es wurden 49.1 mg (50 % d. Th.) der Titelverbindung erhalten.
LC-MS (Methode 3): Rₜ = 1.47 min; MS (ESIpos): m/z = 441 [M+H]⁺
¹H-NMR (400 MHz, DMSO-d6) δ [ppm]: -0.149 (2.07), -0.008 (15.92), 0.008 (15.04), 0.146 (1.91), 1.722 (6.61), 1.861 (1.35), 1.895 (1.67), 2.021 (2.87), 2.087 (3.82), 2.135 (2.63), 2.265 (2.31), 2.327 (3.26), 2.366 (2.55), 2.524 (9.47), 2.569 (3.42), 2.597 (5.17), 2.639 (1.99), 2.670 (3.34), 2.710 (2.47), 3.364 (1.67), 3.391 (1.83), 3.462 (1.27), 3.488 (1.83), 3.604 (1.19), 3.630 (6.05), 3.655 (4.38), 3.673 (3.18), 3.693 (2.31), 3.718 (2.55), 3.740 (4.38), 3.764 (3.42), 3.782 (2.79), 3.851 (5.25), 4.669 (2.55), 4.678 (3.34), 4.684 (3.50), 4.693 (2.71), 4.726 (3.18), 4.735 (3.58), 4.741 (4.22), 4.750 (3.18), 4.772 (3.90), 4.811 (16.00), 4.837 (10.27), 4.877 (2.71), 5.257 (2.63), 5.388 (3.42), 5.509 (1.83), 7.188 (3.98), 7.204 (7.08), 7.208 (8.68), 7.224 (4.06), 7.229 (4.70), 7.403 (8.36), 7.408 (8.60), 7.428 (7.24), 7.543 (7.56), 7.547 (7.16), 7.567 (7.72), 7.572 (7.24), 8.559 (0.64).

### Beispiel 215

### (5S)-2-(4-Brom-2-fluorbenzyl)-5-{[(3R,4S)-3,4-difluorpyrrolidin-1-yl]carbonyl}-5,6,7,8-tetrahydro[1,2,4]triazolo[4,3-a]pyridin-3(2H)-on

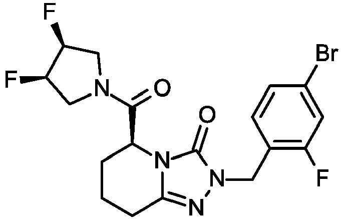

(5S)-2-(4-Brom-2-fluorbenzyl)-3-oxo-2,3,5,6,7,8-hexahydro[1,2,4]triazolo[4,3-a]pyridin-5-carbonsäure (85.0 mg, 223 µmol) wurde in THF (2.0 ml) bei Raumtemperatur vorgelegt. Anschließend wurden HBTU (110 mg, 290 µmol) und N,N-Diisopropylethylamin (190 µl, 1.1 mmol) zugegeben. Nach 5 min Rühren wurde (3R,4S)-3,4-Difluorpyrrolidinhydrochlorid (38.4 mg, 267 µmol) zugegeben und das Reaktionsgemisch über das Wochenende bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 50.6 mg (49 % d. Th.) der Titelverbindung erhalten.
LC-MS (Methode 3): Rₜ = 1.54 min; MS (ESIpos): m/z = 459 [M+H]⁺
¹H-NMR (400 MHz, DMSO-d6) δ [ppm]: -0.149 (0.70), -0.008 (5.71), 0.008 (5.79), 0.146 (0.63), 1.636 (2.03), 1.661 (2.64), 1.670 (2.35), 1.685 (1.84), 1.699 (2.42), 1.711 (2.93), 1.720 (3.34), 1.734 (2.81), 1.911 (1.09), 1.953 (2.49), 1.962 (2.18), 1.978 (1.62), 1.998 (3.20), 2.022 (2.03), 2.032 (2.28), 2.040 (2.49), 2.048 (2.42), 2.056 (1.91), 2.067 (1.82), 2.073 (2.20), 2.083 (1.69), 2.327 (0.99), 2.366 (0.68), 2.577 (4.65), 2.591 (6.34), 2.603 (3.56), 2.621 (1.36), 2.633 (2.18), 2.646 (1.14), 2.665 (0.92), 2.670 (1.19), 2.674 (0.94), 2.710 (0.80), 3.448 (1.23), 3.457 (0.77), 3.483 (2.13), 3.504 (1.55), 3.511 (1.57), 3.521 (1.84), 3.530 (2.59), 3.538 (2.37), 3.563 (1.09), 3.573 (1.60), 3.583 (1.02), 3.611 (1.67), 3.624 (1.96), 3.643 (1.19), 3.657 (1.33), 3.666 (1.86), 3.680 (2.74), 3.698 (3.46), 3.713 (2.13), 3.722 (2.13), 3.731 (2.32), 3.751 (2.06), 3.761 (2.03), 3.783 (1.16), 3.796 (1.14), 3.825 (0.75), 3.861 (1.65), 3.902 (1.23), 3.921 (1.62), 3.935 (1.89), 3.950 (1.14), 3.971 (1.79), 3.985 (1.84), 4.000 (1.14), 4.013 (1.04), 4.124 (1.14), 4.138 (1.31), 4.152 (1.26), 4.166 (2.15), 4.179 (1.38), 4.192 (1.26), 4.208 (1.14), 4.778 (7.46), 4.785 (7.24), 4.795 (4.72), 4.816 (16.00), 4.830 (9.08), 4.838 (9.27), 4.869 (1.48), 4.877 (2.25), 5.243 (1.14), 5.252 (1.79), 5.265 (1.79), 5.274 (2.03), 5.283 (1.69), 5.295 (1.31), 5.327 (1.48), 5.336 (1.50), 5.348 (1.60), 5.363 (1.43), 5.375 (1.89), 5.386 (2.25), 5.398 (1.86), 5.406 (1.91), 5.414 (1.69), 5.425 (1.23), 5.439 (1.23), 5.456 (1.52), 5.470 (1.57), 5.478 (1.62), 5.492 (1.21), 5.501 (0.80), 7.180 (3.00), 7.188 (3.53), 7.200 (6.70), 7.208 (7.53), 7.221 (3.87), 7.228 (4.16), 7.405 (8.08), 7.425 (6.80), 7.545 (7.07), 7.549 (4.96), 7.569 (7.21), 7.573 (4.99).

### Beispiel 216

### (5S)-5-{[(3S)-3-Fluorpyrrolidin-1-yl]carbonyl}-2-{(1RS)-1-[6-(trifluormethyl)pyridin-3-yl]ethyl}-5,6,7,8-tetrahydro[1,2,4]triazolo[4,3-a]pyridin-3(2H)-on (Isomer 1)

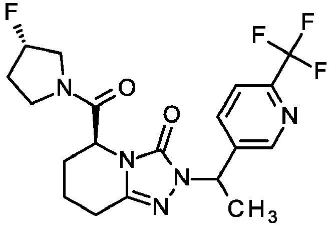

(5S)-3-Oxo-2-{(1RS)-1-[6-(trifluormethyl)pyridin-3-yl]ethyl}-2,3,5,6,7,8-hexahydro[1,2,4]triazolo[4,3-a]pyridin-5-carbonsäure (Isomer 1) (75.0 mg, 210 µmol) wurde in THF (2.0 ml) bei Raumtemperatur vorgelegt. Anschließend wurden HBTU (104 mg, 274 µmol) und N,N-Diisopropylethylamin (180 µl, 1.1 mmol) zugegeben. Nach 5 min Rühren wurde (3S)-3-Fluorpyrrolidinhydrochlorid (31.7 mg, 253 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Die produkthaltigen Fraktionen wurden im Vakuum eingeengt und es wurden 32.5 mg (36 % d. Th.) der Titelverbindung erhalten.
LC-MS (Methode 3): Rₜ = 1.36 min; MS (ESIpos): m/z = 428 [M+H]⁺
¹H-NMR (400 MHz, DMSO-d6) δ [ppm]: -0.149 (0.57), -0.008 (4.90), 0.008 (5.76), 0.146 (0.57), 1.681 (15.40), 1.699 (16.00), 1.723 (3.24), 1.735 (4.02), 1.747 (3.10), 1.851 (0.68), 1.861 (0.66), 1.887 (0.87), 1.960 (0.90), 1.972 (0.80), 1.985 (1.13), 1.995 (1.46), 2.009 (1.24), 2.061 (1.64), 2.075 (1.83), 2.092 (2.11), 2.126 (1.21), 2.153 (0.79), 2.210 (0.88), 2.231 (0.83), 2.259 (1.15), 2.322 (0.79), 2.327 (1.06), 2.366 (0.60), 2.523 (3.39), 2.569 (1.87), 2.586 (1.53), 2.593 (2.30), 2.609 (1.29), 2.648 (2.28), 2.656 (2.47), 2.669 (2.14), 2.689 (1.02), 2.697 (1.10), 2.710 (1.06), 3.273 (0.63), 3.348 (0.74), 3.361 (0.69), 3.370 (0.74), 3.397 (0.79), 3.405 (0.82), 3.459 (0.61), 3.467 (0.66), 3.494 (0.93), 3.503 (0.87), 3.590 (0.57), 3.616 (1.31), 3.638 (2.46), 3.661 (1.94), 3.689 (0.83), 3.704 (2.09), 3.728 (2.05), 3.750 (1.64), 3.769 (1.31), 3.838 (2.57), 4.644 (1.23), 4.654 (1.51), 4.659 (1.64), 4.669 (1.23), 4.703 (1.53), 4.713 (1.84), 4.719 (2.05), 4.728 (1.56), 5.256 (1.31), 5.388 (1.32), 5.468 (1.95), 5.477 (2.49), 5.486 (2.09), 5.495 (2.76), 7.883 (4.74), 7.903 (6.80), 7.999 (3.75), 8.019 (2.72), 8.705 (5.28).

### Beispiel 217

### (5S)-5-{[(3S)-3-Fluorpyrrolidin-1-yl]carbonyl}-2-{(1RS)-1-[6-(trifluormethyl)pyridin-3-yl]ethyl}-5,6,7,8-tetrahydro[1,2,4]triazolo[4,3-a]pyridin-3(2H)-on (Isomer 2)

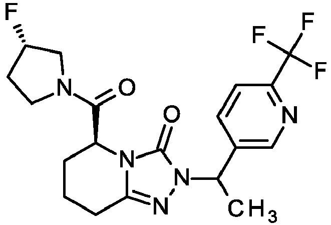

(5S)-3-Oxo-2-{(1RS)-1-[6-(trifluormethyl)pyridin-3-yl]ethyl}-2,3,5,6,7,8-hexahydro[1,2,4]triazolo[4,3-a]pyridin-5-carbonsäure (Isomer 2) (65.0 mg, 177 µmol) wurde in THF (2.0 ml) bei Raumtemperatur vorgelegt. Anschließend wurden HBTU (87.2 mg, 230 µmol) und N,N-Diisopropylethylamin (150 µl, 880 µmol) zugegeben. Nach 5 min Rühren wurde (3S)-3-Fluorpyrrolidinhydrochlorid (26.7 mg, 212 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Die produkthaltigen Fraktionen wurden im Vakuum eingeengt und es wurden 30.2 mg (40 % d. Th.) der Titelverbindung erhalten.
LC-MS (Methode 3): Rₜ = 1.35 min; MS (ESIpos): m/z = 428 [M+H]⁺
¹H-NMR (400 MHz, DMSO-d6) δ [ppm]: -0.149 (0.56), -0.008 (4.61), 0.008 (4.37), 0.146 (0.49), 1.677 (15.68), 1.694 (16.00), 1.746 (1.65), 1.884 (0.49), 1.924 (0.75), 2.010 (0.61), 2.084 (1.67), 2.104 (1.72), 2.259 (1.02), 2.327 (1.12), 2.367 (0.53), 2.586 (1.62), 2.602 (1.34), 2.612 (1.51), 2.626 (1.26), 2.655 (1.99), 2.665 (1.94), 2.669 (1.96), 2.697 (0.78), 2.710 (0.80), 2.882 (0.51), 3.247 (0.49), 3.275 (0.90), 3.371 (0.65), 3.379 (0.68), 3.441 (0.53), 3.468 (0.77), 3.618 (1.89), 3.634 (1.74), 3.646 (1.36), 3.676 (0.97), 3.715 (1.48), 3.738 (1.51), 3.759 (1.17), 3.777 (1.00), 3.842 (2.06), 4.689 (1.02), 4.698 (1.24), 4.704 (1.33), 4.713 (0.99), 4.746 (1.26), 4.754 (1.46), 4.761 (1.69), 4.770 (1.26), 5.245 (1.06), 5.377 (1.46), 5.489 (1.69), 5.498 (2.54), 5.506 (2.30), 5.515 (2.09), 7.900 (3.88), 7.920 (6.69), 7.974 (2.72), 7.994 (1.62), 8.663 (4.90).

### Beispiel 218

### (5S)-5-{[(3R,4S)-3,4-Difluorpyrrolidin-1-yl]carbonyl}-2-{(1RS)-1-[6-(trifluormethyl)pyridin-3-yl]ethyl}-5,6,7,8-tetrahydro[1,2,4]triazolo[4,3-a]pyridin-3(2H)-on (Isomer 1)

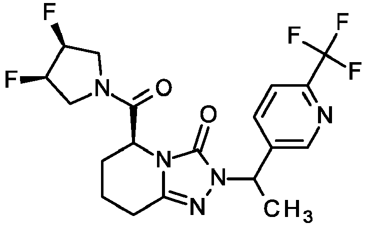

(5S)-3-Oxo-2-{(1R)-1-[6-(trifluormethyl)pyridin-3-yl]ethyl}-2,3,5,6,7,8-hexahydro[1,2,4]triazolo[4,3-a]pyridin-5-carbonsäure (Isomer 1) (75.0 mg, 210 µmol) wurde in THF (2.0 ml) bei Raumtemperatur vorgelegt. Anschließend wurden HBTU (104 mg, 274 µmol) und N,N-Diisopropylethylamin (180 µl, 1.1 mmol) zugegeben. Nach 5 min Rühren wurde (3R,4S)-3,4-Difluorpyrrolidinhydrochlorid (36.3 mg, 253 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Die produkthaltigen Fraktionen wurden im Vakuum eingeengt und es wurden 32.4 mg (35 % d. Th.) der Titelverbindung erhalten.
LC-MS (Methode 3): Rₜ = 1.43 min; MS (ESIpos): m/z = 446 [M+H]⁺
¹H-NMR (400 MHz, DMSO-d6) δ [ppm]: -0.149 (1.11), -0.008 (9.41), 0.008 (9.45), 0.146 (1.07), 1.406 (0.97), 1.679 (15.86), 1.697 (16.00), 1.719 (2.58), 1.943 (1.32), 1.968 (0.93), 1.976 (1.29), 1.990 (1.29), 2.022 (1.15), 2.041 (1.25), 2.048 (1.29), 2.058 (1.22), 2.077 (1.00), 2.322 (1.65), 2.327 (2.29), 2.332 (1.75), 2.523 (6.59), 2.569 (2.04), 2.584 (2.18), 2.595 (2.61), 2.606 (1.93), 2.619 (1.32), 2.652 (2.79), 2.665 (3.15), 2.669 (3.04), 2.674 (2.51), 2.694 (1.36), 2.709 (1.32), 3.491 (1.18), 3.511 (0.89), 3.528 (1.04), 3.537 (1.43), 3.545 (1.32), 3.579 (0.93), 3.617 (0.97), 3.630 (1.11), 3.673 (1.15), 3.687 (1.61), 3.703 (1.79), 3.722 (1.57), 3.736 (1.07), 3.753 (1.25), 3.766 (1.15), 3.801 (0.93), 3.842 (1.04), 3.882 (0.79), 3.915 (0.93), 3.928 (1.15), 3.965 (1.04), 3.978 (1.00), 4.116 (0.79), 4.131 (0.75), 4.146 (1.00), 4.158 (0.79), 4.744 (2.72), 4.758 (3.69), 4.768 (2.51), 5.251 (1.04), 5.262 (1.00), 5.272 (1.07), 5.303 (0.79), 5.312 (0.79), 5.319 (0.89), 5.329 (0.86), 5.342 (0.89), 5.365 (0.89), 5.376 (1.07), 5.385 (1.07), 5.396 (1.07), 5.404 (1.04), 5.418 (1.04), 5.427 (0.79), 5.452 (1.36), 5.470 (2.86), 5.485 (3.19), 5.500 (2.76), 5.516 (0.82), 7.884 (5.37), 7.904 (7.48), 8.002 (4.22), 8.023 (3.11), 8.705 (6.59).

### Beispiel 219

### (5S)-5-{[(3R,4S)-3,4-Difluorpyrrolidin-1-yl]carbonyl}-2-{(1RS)-1-[6-(trifluormethyl)pyridin-3-yl]ethyl}-5,6,7,8-tetrahydro[1,2,4]triazolo[4,3-a]pyridin-3(2H)-on (Isomer 2)

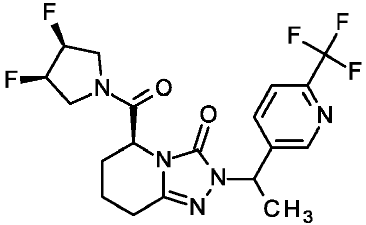

(5S)-3-Oxo-2-{(1RS)-1-[6-(trifluormethyl)pyridin-3-yl]ethyl}-2,3,5,6,7,8-hexahydro[1,2,4]triazolo[4,3-a]pyridin-5-carbonsäure (Isomer 2) (65.0 mg, 177 µmol) wurde in THF (2.0 ml) bei Raumtemperatur vorgelegt. Anschließend wurden HBTU (87.2 mg, 230 µmol) und N,N-Diisopropylethylamin (150 µl, 880 µmol) zugegeben. Nach 5 min Rühren wurde (3R,4S)-3,4-Difluorpyrrolidinhydrochlorid (30.5 mg, 212 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Die produkthaltigen Fraktionen wurden im Vakuum eingeengt und es wurden 11.6 mg (15 % d. Th.) der Titelverbindung erhalten.
LC-MS (Methode 3): Rₜ = 1.43 min; MS (ESIpos): m/z = 446 [M+H]⁺
¹H-NMR (400 MHz, DMSO-d6) δ [ppm]: -0.149 (1.97), -0.050 (0.62), -0.008 (16.00), 0.008 (15.32), 0.018 (0.68), 0.146 (1.66), 1.616 (0.94), 1.652 (1.25), 1.677 (13.66), 1.695 (13.30), 1.741 (1.19), 1.779 (0.83), 1.976 (0.99), 2.017 (1.51), 2.027 (1.51), 2.065 (1.04), 2.093 (0.78), 2.101 (0.78), 2.322 (2.86), 2.327 (3.79), 2.332 (2.81), 2.366 (1.66), 2.392 (0.57), 2.404 (0.62), 2.523 (7.69), 2.567 (0.83), 2.583 (1.09), 2.594 (1.14), 2.609 (1.56), 2.620 (1.14), 2.633 (1.40), 2.651 (2.08), 2.661 (2.44), 2.665 (3.48), 2.669 (4.00), 2.674 (3.01), 2.692 (0.68), 2.710 (1.51), 3.462 (0.73), 3.483 (0.68), 3.501 (0.68), 3.508 (0.94), 3.517 (0.99), 3.605 (0.78), 3.645 (0.68), 3.660 (1.19), 3.673 (0.94), 3.695 (0.99), 3.720 (0.83), 3.737 (0.83), 3.758 (0.62), 3.859 (0.57), 3.897 (0.57), 3.916 (0.78), 3.929 (0.83), 3.958 (0.57), 3.966 (0.78), 3.980 (0.62), 4.145 (0.57), 4.157 (0.78), 4.186 (0.57), 4.796 (1.97), 4.810 (2.49), 4.820 (1.82), 5.247 (0.83), 5.255 (0.78), 5.264 (0.68), 5.311 (0.73), 5.342 (0.73), 5.357 (0.94), 5.368 (0.94), 5.391 (0.88), 5.441 (0.73), 5.476 (1.04), 5.493 (1.66), 5.501 (1.82), 5.511 (1.56), 5.520 (1.66), 7.897 (1.77), 7.901 (2.08), 7.916 (3.32), 7.920 (3.58), 7.966 (1.71), 7.979 (2.18), 7.993 (1.19), 8.551 (0.78), 8.660 (4.26).

### Beispiel 220

### (5RS,8RS)-5-{[(3S)-3-Fluorpyrrolidin-1-yl]carbonyl}-8-methyl-2-{[6-(trifluormethyl)pyridin-3-yl]methyl}-5,6,7,8-tetrahydro[1,2,4]triazolo[4,3-a]pyridin-3(2H)-on (Diastereomerengemisch; 4 Isomere)

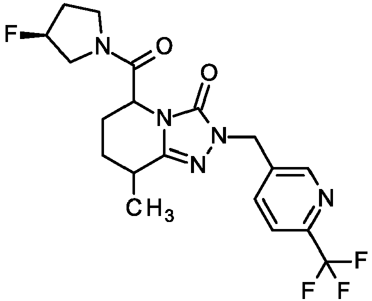

(5RS,8RS)-8-Methyl-3-oxo-2-{[6-(trifluormethyl)pyridin-3-yl]methyl}octahydro[1,2,4]triazolo[4,3-a]pyridin-5-carbonsäure (Diastereomerengemisch; 4 Isomere) (100 mg, 279 µmol) wurde in Dichlormethan (2.0 ml) und DMF (4.0 ml) bei Raumtemperatur vorgelegt. Anschließend wurden 1-[Bis(dimethylamino)methylen]-1H-benzotriazol-1-ium-3-oxidhexafluorophosphat (138 mg, 363 µmol) und N,N-Diisopropylethylamin (120 µl, 670 µmol) zugegeben. Nach 15 min Rühren wurde (3S)-3-Fluorpyrrolidinhydrochlorid (38.5 mg, 307 µmol) zugegeben und das Reaktionsgemisch für 30 min bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 76.0 mg (64 % d. Th.) der Titelverbindung als Diastereomerengemisch erhalten.
LC-MS (Methode 3): Rₜ = 1.39 min; MS (ESIpos): m/z = 428 [M+H]⁺
¹H-NMR (400 MHz, DMSO-d6) δ [ppm]: -0.008 (0.95), 0.008 (0.84), 1.175 (11.85), 1.181 (12.64), 1.192 (12.80), 1.198 (12.36), 1.345 (0.79), 1.375 (1.07), 1.409 (1.13), 1.442 (0.85), 1.804 (1.52), 1.814 (1.61), 1.824 (1.65), 1.833 (1.39), 1.965 (0.69), 1.999 (1.14), 2.033 (0.60), 2.040 (0.61), 2.078 (3.30), 2.088 (3.09), 2.103 (3.06), 2.111 (3.23), 2.126 (2.92), 2.135 (2.51), 2.153 (1.32), 2.172 (0.58), 2.227 (0.92), 2.237 (0.95), 2.258 (1.26), 2.524 (0.86), 2.707 (1.11), 2.722 (1.74), 2.737 (2.13), 2.752 (1.75), 2.766 (1.08), 3.276 (0.43), 3.357 (0.89), 3.367 (1.05), 3.387 (0.89), 3.397 (0.83), 3.414 (0.46), 3.463 (0.43), 3.490 (0.58), 3.498 (0.58), 3.520 (1.78), 3.543 (1.38), 3.550 (1.09), 3.569 (1.18), 3.599 (2.26), 3.625 (1.34), 3.641 (1.96), 3.648 (1.65), 3.671 (1.54), 3.700 (0.76), 3.714 (0.73), 3.736 (1.85), 3.758 (1.07), 3.766 (1.05), 3.776 (1.17), 3.844 (1.52), 3.910 (0.54), 3.932 (0.99), 3.952 (0.72), 3.980 (0.44), 4.006 (0.53), 4.038 (0.41), 4.725 (0.97), 4.738 (1.03), 4.773 (0.95), 4.782 (1.50), 4.793 (0.96), 4.854 (0.84), 4.860 (1.12), 4.872 (0.97), 4.890 (0.89), 4.902 (1.20), 4.909 (0.81), 5.002 (0.58), 5.052 (13.40), 5.092 (0.56), 5.262 (1.23), 5.269 (1.12), 5.349 (0.69), 5.395 (1.28), 5.481 (0.68), 5.511 (0.56), 7.884 (0.66), 7.908 (16.00), 7.928 (0.87), 8.633 (6.30).

### Beispiel 221

### (5RS,8RS)-5-{[(3S)-3-Fluorpyrrolidin-1-yl]carbonyl}-8-methyl-2-{[6-(trifluormethyl)pyridin-3-yl]methyl}-5,6,7,8-tetrahydro[1,2,4]triazolo[4,3-a]pyridin-3(2H)-on (Isomer 1)

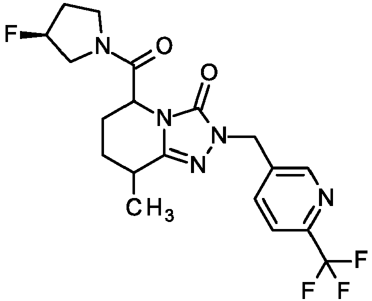

(5RS,8RS)-5-{[(3S)-3-Fluorpyrrolidin-1-yl]carbonyl}-8-methyl-2-{[6-(trifluormethyl)pyridin-3-yl]methyl}-5,6,7,8-tetrahydro[1,2,4]triazolo[4,3-a]pyridin-3(2H)-on (Diastereomerengemisch; 4 Isomere) wurde durch chirale präparative HPLC getrennt [Probenvorbereitung: 60 mg gelöst in 1 ml Ethanol und 1 ml Acetonitril; Injektionsvolumen: 0.25 ml; Säule: Daicel Chiralpak^{®} IA 5 µm, 250 × 20 mm; Laufmittel: n-Heptan/Ethanol 50:50; Fluss: 20 ml/min; Temperatur 40°C; UV-Detektion: 220 nm]. Nach der Trennung wurden 18.4 mg von Isomer 1, welches zuerst eluiert, und 18.5 mg von Isomer 2, welches später eluiert, isoliert.

### Isomer 1:

Analytische chirale HPLC: Rₜ = 1.95 min, d.e. = 100% [Säule: Daicel Chiralcel^{®} ID-3 50 x 4.6 mm; Laufmittel: n-Heptan/Ethanol 70:30; Fluss: 1 ml/min; UV-Detektion: 220 nm].
LC-MS (Methode 3): Rₜ = 1.40 min; MS (ESIpos): m/z = 428 [M+H]⁺
¹H-NMR (400 MHz, DMSO-d6) δ [ppm]: -0.008 (2.11), 0.008 (1.73), 1.175 (15.58), 1.191 (16.00), 1.316 (0.40), 1.345 (1.10), 1.373 (1.14), 1.401 (0.52), 1.797 (0.87), 1.821 (1.22), 1.842 (0.80), 1.856 (0.67), 2.003 (0.50), 2.076 (3.92), 2.138 (1.06), 2.153 (0.81), 2.207 (0.68), 2.229 (0.70), 2.245 (0.70), 2.257 (0.97), 2.328 (0.43), 2.670 (0.47), 2.720 (1.03), 2.735 (1.32), 2.744 (1.35), 2.774 (0.60), 3.338 (0.78), 3.356 (0.70), 3.367 (1.10), 3.385 (1.09), 3.395 (0.76), 3.413 (0.61), 3.519 (2.55), 3.542 (1.99), 3.567 (1.70), 3.598 (2.78), 3.647 (0.57), 3.671 (0.60), 3.679 (0.60), 3.745 (0.48), 3.768 (0.62), 3.776 (0.61), 3.909 (0.78), 3.931 (1.41), 3.951 (1.04), 3.978 (0.66), 4.005 (0.77), 4.036 (0.62), 4.858 (1.59), 4.872 (1.35), 4.889 (1.29), 4.901 (1.72), 4.909 (1.15), 5.051 (13.02), 5.269 (0.97), 5.348 (0.97), 5.399 (0.99), 5.480 (0.97), 7.885 (0.47), 7.908 (12.03), 7.928 (0.69), 8.632 (5.68).

### Beispiel 222

### (5RS,8RS)-5-{[(3S)-3-Fluorpyrrolidin-1-yl]carbonyl}-8-methyl-2-{[6-(trifluormethyl)pyridin-3-yl]methyl}-5,6,7,8-tetrahydro[1,2,4]triazolo[4,3-a]pyridin-3(2H)-on (Isomer 2)

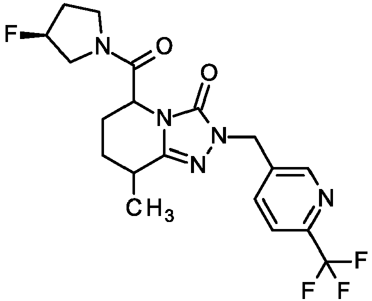

(5RS,8RS)-5-{[(3S)-3-Fluorpyrrolidin-1-yl]carbonyl}-8-methyl-2-{[6-(trifluormethyl)pyridin-3-yl]methyl}-5,6,7,8-tetrahydro[1,2,4]triazolo[4,3-a]pyridin-3(2H)-on (Diastereomerengemisch; 4 Isomere) wurde durch chirale präparative HPLC getrennt [Probenvorbereitung: 60 mg gelöst in 1 ml Ethanol und 1 ml Acetonitril; Injektionsvolumen: 0.25 ml; Säule: Daicel Chiralpak^{®} IA 5 µm, 250 × 20 mm; Laufmittel: n-Heptan/Ethanol 50:50; Fluss: 20 ml/min; Temperatur 40°C; UV-Detektion: 220 nm]. Nach der Trennung wurden 18.4 mg von Isomer 1, welches zuerst eluiert, und 18.5 mg von Isomer 2, welches später eluiert, isoliert.

### Isomer 2:

Analytische chirale HPLC: Rₜ = 2.22 min, d.e. = 100% [column: Daicel Chiralcel^{®} ID-3 50 x 4.6 mm; Laufmittel: n-Heptan/Ethanol 70:30; Fluss: 1 ml/min; UV detection: 220 nm].
LC-MS (Methode 3): Rₜ = 1.39 min; MS (ESIpos): m/z = 428 [M+H]⁺
¹H-NMR (400 MHz, DMSO-d6) δ [ppm]: -0.008 (1.59), 0.008 (1.32), 1.120 (0.68), 1.181 (15.77), 1.198 (16.00), 1.377 (0.51), 1.391 (0.56), 1.408 (1.21), 1.441 (1.17), 1.464 (0.45), 1.803 (1.39), 1.813 (1.24), 1.824 (1.15), 1.837 (1.13), 1.964 (0.73), 1.999 (1.22), 2.102 (2.42), 2.115 (3.09), 2.126 (3.14), 2.135 (2.50), 2.146 (1.27), 2.170 (0.54), 2.227 (0.65), 2.236 (0.75), 2.271 (0.93), 2.282 (0.84), 2.524 (0.99), 2.707 (1.01), 2.722 (1.52), 2.737 (1.78), 2.752 (1.45), 2.767 (0.89), 3.275 (0.76), 3.294 (1.15), 3.303 (1.69), 3.391 (1.53), 3.400 (1.29), 3.453 (0.67), 3.462 (0.68), 3.489 (0.84), 3.497 (0.79), 3.599 (0.51), 3.624 (1.85), 3.641 (2.31), 3.670 (1.69), 3.698 (1.02), 3.714 (1.01), 3.735 (2.25), 3.757 (1.43), 3.774 (1.16), 3.843 (2.15), 4.724 (1.36), 4.737 (1.44), 4.772 (1.34), 4.781 (2.09), 4.792 (1.37), 5.001 (0.77), 5.042 (8.22), 5.049 (6.72), 5.091 (0.65), 5.260 (1.10), 5.391 (1.28), 5.510 (0.78), 7.883 (0.53), 7.907 (12.15), 7.927 (0.85), 8.632 (4.85).

### Beispiel 223

### (5RS,8RS)-8-Methyl-2-(4-methylbenzyl)-5-(pyrrolidin-1-ylcarbonyl)-5,6,7,8-tetrahydro[1,2,4]triazolo[4,3-a]pyridin-3(2H)-on (Diastereomerengemisch; 4 Isomere)

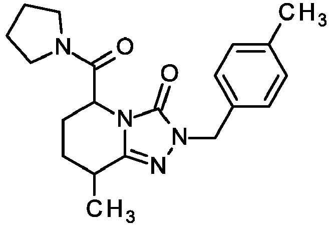

(5RS,8RS)-8-Methyl-2-(4-methylbenzyl)-3-oxo-2,3,5,6,7,8-hexahydro[1,2,4]triazolo[4,3-a]pyridin-5-carbonsäure (Diastereomerengemisch; 4 Isomere) (27.3 mg, 90.6 µmol) wurde in DMF (1.5 ml) und Dichlormethan (750 µl) bei Raumtemperatur vorgelegt. Anschließend wurden 1-[Bis(dimethylamino)methylen]-1H-benzotriazol-1-ium-3-oxidhexafluorophosphat (44.7 mg, 118 µmol) und N,N-Diisopropylethylamin (22 µl, 130 µmol) zugegeben. Nach 15 min Rühren wurde Pyrrolidin (9.1 µl, 110 µmol) zugeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 18.0 mg (55 % d. Th.) der Titelverbindung erhalten als Mischung von Diastereomeren.
LC-MS (Methode 1): Rₜ = 1.10 min; MS (ESIpos): m/z = 355 [M+H]⁺
¹H-NMR (400 MHz, DMSO-d6) δ [ppm]: -0.008 (0.89), 0.008 (0.80), 1.166 (8.74), 1.183 (9.03), 1.380 (0.42), 1.396 (1.31), 1.411 (0.71), 1.432 (0.51), 1.442 (0.50), 1.528 (1.03), 1.755 (1.12), 1.770 (2.91), 1.787 (4.30), 1.804 (3.36), 1.821 (0.86), 1.892 (0.84), 1.908 (2.40), 1.925 (2.98), 1.942 (1.80), 1.958 (0.48), 2.040 (1.84), 2.049 (2.25), 2.058 (1.75), 2.271 (16.00), 2.523 (0.60), 2.663 (0.69), 2.677 (0.96), 2.693 (1.06), 2.708 (0.93), 2.723 (0.55), 3.220 (0.49), 3.237 (1.05), 3.250 (1.04), 3.267 (1.83), 3.284 (0.98), 3.335 (2.01), 3.347 (0.81), 3.353 (1.05), 3.364 (1.12), 3.382 (0.52), 3.428 (0.55), 3.445 (1.19), 3.453 (0.91), 3.463 (0.75), 3.470 (1.52), 3.487 (0.68), 3.578 (0.69), 3.595 (1.41), 3.603 (0.76), 3.611 (0.84), 3.619 (1.12), 3.636 (0.52), 4.707 (1.64), 4.738 (1.56), 4.746 (5.65), 4.757 (1.42), 4.799 (3.57), 4.839 (1.59), 7.097 (1.28), 7.118 (10.61), 7.124 (9.75), 7.145 (1.25).

### Beispiel 224

### (5RS,7RS)-7-Methyl-2-(4-methylbenzyl)-5-(pyrrolidin-1-ylcarbonyl)-5,6,7,8-tetrahydro[1,2,4]triazolo[4,3-a]pyridin-3(2H)-on (Diastereomerengemisch; 4 Isomere)

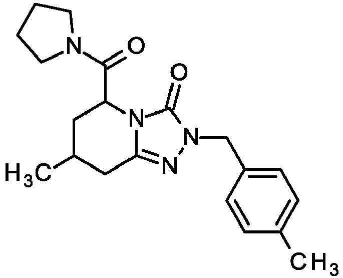

(5RS,7RS)-7-Methyl-2-(4-methylbenzyl)-3-oxo-2,3,5,6,7,8-hexahydro[1,2,4]triazolo[4,3-a]pyridin-5-carbonsäure (60.0 mg, 199 µmol) wurde mit Pyrrolidin (25 µl, 300 µmol) in Pyridin/DMF (5/1) (3.0 mL) bei Raumtemperatur vorgelegt. Anschließend wurde O-(7-Azabenzotriazol-1-yl)-*N,N,N',N*'-tetramethyluronium-hexafluorphosphat (121 mg, 319 µmol) zugegeben und das Reaktionsgemisch für 5 h bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 23.0 mg (29 % d. Th.) der Titelverbindung als Diastereomerengemisch erhalten.
LC-MS (Methode 1): Rₜ = 1.06 min; MS (ESIpos): m/z = 355 [M+H]⁺
¹H-NMR (400 MHz, DMSO-d6) δ [ppm]: 1.013 (3.80), 1.030 (3.91), 1.106 (7.23), 1.329 (0.57), 1.359 (0.58), 1.771 (1.01), 1.788 (1.80), 1.805 (1.54), 1.814 (0.95), 1.860 (0.50), 1.882 (0.68), 1.899 (1.42), 1.916 (1.58), 1.931 (0.91), 2.111 (0.74), 2.141 (0.57), 2.151 (0.79), 2.181 (0.84), 2.270 (10.66), 2.611 (0.49), 2.616 (0.51), 2.646 (0.40), 2.651 (0.45), 2.657 (0.43), 3.076 (2.31), 3.226 (0.41), 3.243 (0.65), 3.256 (0.57), 3.273 (0.92), 3.290 (0.45), 3.339 (0.47), 3.356 (0.93), 3.374 (0.50), 3.386 (0.56), 3.468 (0.59), 3.476 (0.46), 3.493 (0.74), 3.634 (0.73), 3.651 (0.41), 3.659 (0.56), 4.601 (0.62), 4.617 (0.73), 4.628 (0.71), 4.643 (0.61), 4.718 (4.78), 7.128 (16.00).

### Beispiel 225

### (5RS,7RS)-2-(4-Methylbenzyl)-5-(pyrrolidin-1-ylcarbonyl)-7-(trifluormethyl)-5,6,7,8-tetrahydro[1,2,4]triazolo[4,3-a]pyridin-3(2H)-on (Diastereomer 1, Racemat)

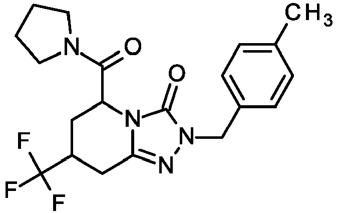

_(5RS,7RS)-2-(4-Methylbenzyl)-3-oxo-7-(trifluormethyl)-2,3,5,6,7,8-hexahydro[1,2,4]triazolo[4,3-a]pyridin-5-carbonsäure (Diastereomerengemisch; 4 Isomere) (98.0 mg, 276 µmol) wurde mit Pyrrolidin (25 µl, 300 µmol) in Pyridin/DMF (5/1) (2.0 mL) bei Raumtemperatur vorgelegt. Anschließend wurde *O*-(7-Azabenzotriazol-1-yl)-*N,N*,*N*',*N*'-tetramethyluronium-hexafluorphosphat (115 mg, 303 µmol) zugegeben und das Reaktionsgemisch für 2 h bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 37.4 mg (33 % d. Th.) Diastereomer 1 (Racemat) und 8.7 mg (8 % d. Th.) Diastereomer 2 (Racemat) der Titelverbindung erhalten.
LC-MS (Methode 3): Rₜ = 1.69 min; MS (ESIpos): m/z = 409 [M+H]⁺
¹H-NMR (400 MHz, DMSO-d6) δ [ppm]: -0.008 (0.44), 0.008 (0.45), 1.777 (0.99), 1.794 (1.60), 1.806 (1.64), 1.822 (1.20), 1.839 (0.52), 1.907 (0.75), 1.924 (2.35), 1.941 (3.00), 1.958 (1.73), 1.975 (0.43), 2.167 (0.74), 2.182 (0.75), 2.197 (1.28), 2.210 (2.06), 2.272 (15.46), 2.523 (0.50), 2.643 (0.56), 2.676 (1.24), 2.687 (1.24), 2.719 (0.94), 2.949 (1.79), 2.983 (1.24), 2.993 (0.80), 3.248 (0.42), 3.265 (0.82), 3.277 (0.98), 3.295 (1.74), 3.332 (1.01), 3.349 (1.49), 3.366 (0.94), 3.379 (0.80), 3.396 (0.43), 3.534 (0.43), 3.551 (0.96), 3.559 (0.83), 3.568 (0.62), 3.576 (1.44), 3.593 (0.64), 3.631 (0.66), 3.648 (1.38), 3.655 (0.63), 3.665 (0.77), 3.673 (0.92), 4.723 (0.92), 4.762 (4.01), 4.782 (3.97), 4.820 (0.94), 4.937 (1.52), 4.947 (1.32), 7.104 (0.72), 7.112 (0.47), 7.127 (16.00), 7.150 (0.70).

### Beispiel 226

### (5RS,7RS)-2-(4-Methylbenzyl)-5-(pyrrolidin-1-ylcarbonyl)-7-(trifluormethyl)-5,6,7,8-tetrahydro[1,2,4]triazolo[4,3-a]pyridin-3(2H)-on (Diastereomer 2, Racemat)

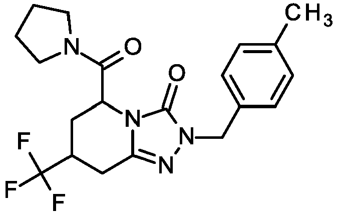

(5RS,7RS)-2-(4-Methylbenzyl)-3-oxo-7-(trifluormethyl)-2,3,5,6,7,8-hexahydro[1,2,4]triazolo[4,3-a]pyridin-5-carbonsäure (Diastereomerengemisch; 4 Isomere) (98.0 mg, 276 µmol) wurde mit Pyrrolidin (25 µl, 300 µmol) in Pyridin/DMF (5/1) (2.0 mL) bei Raumtemperatur vorgelegt. Anschließend wurde *O*-(7-Azabenzotriazol-1-yl)-*N*,*N*,*N*',*N*'-tetramethyluronium-hexafluorphosphat (115 mg, 303 µmol) zugegeben und das Reaktionsgemisch für 2 h bei Raumtemperatur gerührt. Das Lösungsmittel wurde unter Vakuum entfernt und der Rückstand über präparative HPLC aufgereinigt (Chromatorex C18, 10µm), 125mm x 30mm; Laufmittel: Acetonitril/Wasser-Gradient). Die produkthaltigen Fraktionen wurden im Vakuum eingeengt und es wurden 37.4 mg (33 % d. Th.) Diastereomer 1 (Racemat) und 8.7 mg (8 % d. Th.) Diastereomer 2 (Racemat) der Titelverbindung erhalten.
LC-MS (Methode 3): Rₜ = 1.62 min; MS (ESIpos): m/z = 409 [M+H]⁺
¹H-NMR (400 MHz, DMSO-d6) δ [ppm]: -0.008 (0.69), 0.008 (0.74), 1.665 (0.67), 1.692 (0.67), 1.779 (0.59), 1.785 (0.66), 1.797 (0.98), 1.802 (1.02), 1.817 (0.88), 1.910 (1.28), 1.927 (1.65), 1.944 (0.95), 2.274 (9.27), 2.634 (0.46), 2.665 (0.67), 2.674 (0.77), 2.706 (0.77), 2.863 (0.57), 3.263 (0.51), 3.276 (0.51), 3.293 (0.94), 3.354 (0.43), 3.371 (0.81), 3.389 (0.49), 3.401 (0.51), 3.555 (0.51), 3.562 (0.47), 3.579 (0.82), 3.637 (0.80), 3.654 (0.44), 3.661 (0.50), 4.710 (0.65), 4.724 (0.78), 4.745 (4.83), 7.141 (16.00).

### Beispiel 227

### (5RS,7RS)-2-[(6-Chlorpyridin-3-yl)methyl]-5-(pyrrolidin-1-ylcarbonyl)-7-(trifluormethyl)-5,6,7,8-tetrahydro[1,2,4]triazolo[4,3-a]pyridin-3(2H)-on (Diastereomer 1, Racemat)

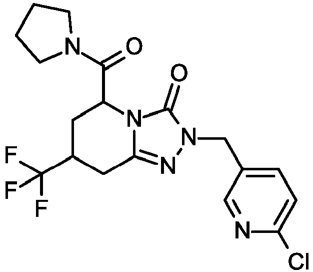

(5RS,7RS)-2-[(6-Chlorpyridin-3-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere) (88.0 mg, 234 µmol) wurde in THF (3.0 ml) bei Raumtemperatur vorgelegt. Anschließend wurden HBTU (115 mg, 304 µmol) und N,N-Diisopropylethylamin (200 µl, 1.2 mmol) zugegeben. Nach 15 min Rühren wurde Pyrrolidin (23 µl, 280 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Nach der Trennung wurden 10.2 mg (10 % d. Th.) von Diastereomer 1 (Racemat), welches zuerst eluiert, und 43.7 mg (44 % d. Th.) von Diastereomer 2 (Racemat), welches später eluiert, isoliert.

### Diastereomer 1, Racemat:

LC-MS (Methode 3): Rₜ = 1.37 min; MS (ESIpos): m/z = 430 [M+H]⁺
¹H-NMR (400 MHz, DMSO-d6) δ [ppm]: -0.149 (1.19), -0.008 (9.63), 0.008 (8.89), 0.146 (1.11), 1.635 (1.26), 1.666 (2.81), 1.693 (2.74), 1.726 (1.48), 1.778 (2.37), 1.785 (2.74), 1.795 (3.93), 1.802 (4.00), 1.818 (3.19), 1.893 (1.56), 1.909 (5.48), 1.926 (6.89), 1.944 (4.00), 2.327 (2.07), 2.366 (1.85), 2.523 (8.67), 2.657 (2.15), 2.669 (2.37), 2.688 (2.52), 2.697 (3.04), 2.710 (2.30), 2.729 (3.41), 2.876 (2.44), 2.911 (1.56), 3.012 (1.19), 3.264 (2.44), 3.276 (2.59), 3.351 (2.52), 3.369 (3.70), 3.386 (2.30), 3.399 (2.37), 3.417 (1.26), 3.569 (2.15), 3.586 (3.63), 3.611 (2.00), 3.627 (3.63), 3.652 (1.93), 4.711 (2.74), 4.725 (3.04), 4.738 (2.81), 4.753 (2.44), 4.884 (16.00), 7.516 (6.22), 7.536 (7.63), 7.720 (4.52), 7.727 (4.44), 7.741 (3.85), 7.747 (3.85), 8.328 (6.00), 8.334 (5.70).

### Beispiel 228

### (5RS,7RS)-2-[(6-Chlorpyridin-3-yl)methyl]-5-(pyrrolidin-1-ylcarbonyl)-7-(trifluormethyl)-5,6,7,8-tetrahydro[1,2,4]triazolo[4,3-a]pyridin-3(2H)-on (Diastereomer 2, Racemat)

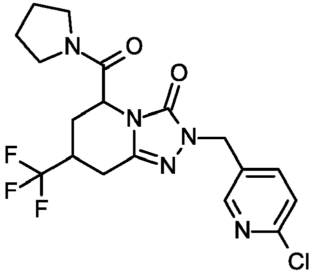

(5RS,7RS)-2-[(6-Chlorpyridin-3-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere) (88.0 mg, 234 µmol) wurde in THF (3.0 ml) bei Raumtemperatur vorgelegt. Anschließend wurden HBTU (115 mg, 304 µmol) und N,N-Diisopropylethylamin (200 µl, 1.2 mmol) zugegeben. Nach 15 min Rühren wurde Pyrrolidin (23 µl, 280 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Nach der Trennung wurden 10.2 mg (10 % d. Th.) von Diastereomer 1 (Racemat), welches zuerst eluiert, und 43.7 mg (44 % d. Th.) von Diastereomer 2 (Racemat), welches später eluiert, isoliert.

### Diastereomer 2, Racemat:

LC-MS (Methode 3): Rₜ = 1.45 min; MS (ESIpos): m/z = 430 [M+H]⁺
¹H-NMR (400 MHz, DMSO-d6) δ [ppm]: -0.149 (0.54), -0.008 (4.44), 0.008 (3.96), 0.146 (0.54), 1.778 (2.01), 1.795 (3.23), 1.805 (3.35), 1.822 (2.59), 1.906 (1.50), 1.923 (4.73), 1.939 (5.81), 1.957 (3.32), 2.173 (1.47), 2.189 (1.53), 2.203 (1.92), 2.218 (4.02), 2.327 (1.02), 2.366 (0.70), 2.523 (3.03), 2.667 (1.95), 2.694 (2.49), 2.705 (2.59), 2.734 (2.08), 2.962 (3.00), 3.000 (1.92), 3.010 (1.47), 3.250 (0.86), 3.268 (1.72), 3.280 (2.27), 3.297 (5.91), 3.331 (3.03), 3.349 (3.32), 3.366 (2.17), 3.377 (1.72), 3.395 (0.99), 3.532 (0.93), 3.549 (1.98), 3.557 (1.79), 3.574 (3.10), 3.591 (1.31), 3.625 (1.44), 3.641 (2.87), 3.658 (1.63), 3.666 (1.85), 3.683 (0.93), 4.914 (16.00), 4.949 (3.26), 4.964 (2.78), 7.510 (5.78), 7.530 (7.41), 7.692 (4.12), 7.699 (4.12), 7.713 (3.32), 7.719 (3.29), 8.299 (5.72), 8.305 (5.52).

### Beispiel 229

### (5RS,7RS)-2-[(6-Chlorpyridin-3-yl)methyl]-5-(pyrrolidin-1-ylcarbonyl)-7-(trifluormethyl)-5,6,7,8-tetrahydro[1,2,4]triazolo[4,3-a]pyridin-3(2H)-on (Enantiomer 1)

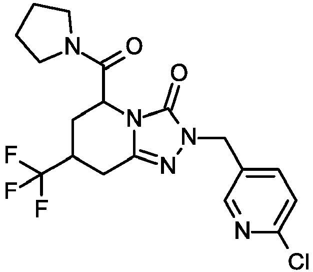

(5RS,7RS)-2-[(6-Chlorpyridin-3-yl)methyl]-5-(pyrrolidin-1-ylcarbonyl)-7-(trifluormethyl)-5,6,7,8-tetrahydro[1,2,4]triazolo[4,3-a]pyridin-3(2H)-on (Diastereomer 2, Racemat) wurde durch chirale präparative HPLC getrennt [Probenvorbereitung: 42 mg gelöst in 2 ml Ethanol und 2 ml n-Heptan; Injektionsvolumen: 0.40 ml; Säule: Daicel Chiralpak^{®} IB 5 µm, 250 x 20 mm; Laufmittel: n-Heptan/Ethanol 50:50; Fluss: 15 ml/min; Temperatur 40°C; UV-Detektion: 220 nm]. Nach der Trennung wurden 14.7 mg von Enantiomer 1, welches zuerst eluiert, und 16.1 mg von Enantiomer 2, welches später eluiert, isoliert.

### Enantiomer 1:

Analytische chirale HPLC: Rₜ = 4.99 min, e.e. = 99% [Säule: Daicel Chiralpak^{®} IB, 250 x 4.6 mm; Laufmittel: i-Hexan/Ethanol 50:50; Fluss: 1 ml/min; UVDetektion: 220 nm].
LC-MS (Methode 3): Rₜ = 1.47 min; MS (ESIpos): m/z = 430 [M+H]⁺
¹H-NMR (400 MHz, DMSO-d6) δ [ppm]: -0.149 (1.30), -0.008 (11.16), 0.008 (10.55), 0.146 (1.30), 1.233 (0.61), 1.778 (1.99), 1.794 (3.20), 1.806 (3.29), 1.822 (2.51), 1.906 (1.47), 1.923 (4.58), 1.940 (5.62), 1.957 (3.29), 2.172 (1.47), 2.188 (1.56), 2.202 (1.73), 2.217 (4.06), 2.328 (1.82), 2.366 (1.64), 2.523 (6.49), 2.669 (2.85), 2.694 (2.42), 2.705 (2.51), 2.710 (2.25), 2.734 (2.16), 2.962 (3.11), 2.998 (1.90), 3.010 (1.47), 3.250 (1.12), 3.267 (2.34), 3.279 (2.77), 3.348 (4.67), 3.365 (2.59), 3.377 (2.08), 3.396 (1.21), 3.531 (0.95), 3.549 (2.08), 3.557 (1.73), 3.574 (3.03), 3.591 (1.30), 3.623 (1.38), 3.640 (2.77), 3.658 (1.64), 3.666 (1.99), 3.682 (0.95), 4.914 (16.00), 4.950 (3.20), 4.961 (3.03), 7.510 (4.93), 7.530 (6.23), 7.692 (3.37), 7.698 (3.63), 7.713 (2.85), 7.719 (2.94), 8.298 (4.15), 8.304 (4.24).

### Beispiel 230

### (5RS,7RS)-2-[(6-Chlorpyridin-3-yl)methyl]-5-[(3,3-difluorpyrrolidin-1-yl)carbonyl]-7-(trifluormethyl)-5,6,7,8-tetrahydro[1,2,4]triazolo[4,3-a]pyridin-3(2H)-on (Diastereomer 2, Racemat)

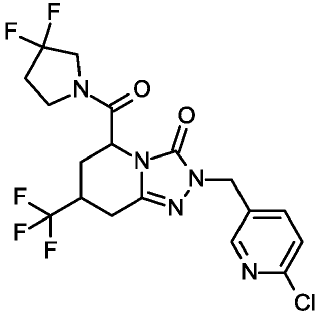

(5RS,7RS)-2-[(6-Chlorpyridin-3-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere) (88.0 mg, 234 µmol) wurde in THF (3.0 ml) bei Raumtemperatur vorgelegt. Anschließend wurden HBTU (115 mg, 304 µmol) und N,N-Diisopropylethylamin (200 µl, 1.2 mmol) zugegeben. Nach 15 min Rühren wurde 3,3-Difluorpyrrolidinhydrochlorid (40.2 mg, 280 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Nach der Trennung wurden 10.2 mg von Diastereomer 1 (Racemat) (9 % d. Th.), welches zuerst eluiert, und 48 mg (44 % d. Th.) von Diastereomer 2 (Racemat)., welches später eluiert, isoliert.

### Diastereomer 2 (Racemat):

LC-MS (Methode 3): Rₜ = 1.56 min; MS (ESIpos): m/z = 466 [M+H]⁺
¹H-NMR (400 MHz, DMSO-d6) δ [ppm]: -0.008 (2.62), 0.008 (2.46), 2.112 (0.44), 2.129 (0.51), 2.148 (1.03), 2.164 (1.01), 2.179 (1.06), 2.189 (1.30), 2.205 (0.97), 2.220 (0.94), 2.236 (0.90), 2.259 (1.65), 2.278 (1.76), 2.311 (0.92), 2.327 (0.61), 2.366 (0.55), 2.374 (0.59), 2.394 (1.16), 2.412 (1.03), 2.424 (1.45), 2.440 (1.32), 2.461 (0.88), 2.523 (1.85), 2.563 (1.83), 2.581 (1.41), 2.601 (0.99), 2.619 (0.50), 2.678 (1.36), 2.686 (1.10), 2.707 (1.76), 2.716 (2.61), 2.725 (1.52), 2.747 (1.89), 2.755 (1.78), 2.901 (1.23), 2.970 (2.55), 3.010 (1.85), 3.534 (0.50), 3.545 (1.25), 3.565 (2.55), 3.578 (1.94), 3.596 (1.23), 3.609 (0.42), 3.710 (1.16), 3.750 (1.54), 3.779 (1.76), 3.814 (1.21), 3.889 (0.72), 3.897 (1.17), 3.914 (2.90), 3.932 (3.03), 3.951 (1.12), 4.130 (1.03), 4.157 (0.84), 4.173 (1.56), 4.200 (1.63), 4.234 (0.97), 4.878 (0.59), 4.920 (16.00), 4.951 (2.06), 4.964 (2.22), 5.029 (1.98), 5.042 (1.91), 7.512 (6.06), 7.532 (7.69), 7.695 (3.71), 7.701 (3.78), 7.715 (3.16), 7.722 (3.16), 8.299 (5.17), 8.305 (5.08).

### Beispiel 231

### (5RS,7RS)-2-[(6-Chlorpyridin-3-yl)methyl]-5-[(3,3-difluorpyrrolidin-1-yl)carbonyl]-7-(trifluormethyl)-5,6,7,8-tetrahydro[1,2,4]triazolo[4,3-a]pyridin-3(2H)-on (Enantiomer 1)

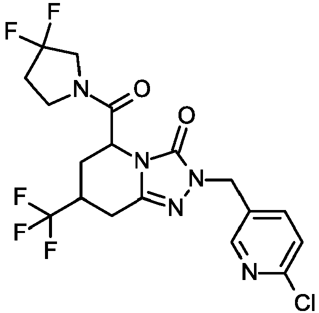

(5RS,7RS)-2-[(6-Chlorpyridin-3-yl)methyl]-5-[(3,3-difluorpyrrolidin-1-yl)carbonyl]-7-(trifluormethyl)-5,6,7,8-tetrahydro[1,2,4]triazolo[4,3-a]pyridin-3(2H)-on (Diastereomer 2, Racemat) wurde durch chirale präparative HPLC getrennt [Probenvorbereitung: 48 mg gelöst in 2 ml Ethanol und 1.5 ml n-Heptan; Injektionsvolumen: 0.5 ml; Säule: Daicel Chiralpak^{®} IE 5 µm, 250 x 20 mm; Laufmittel: n-Heptan/Ethanol 40:60; Fluss: 15 ml/min; Temperatur 40°C; UV-Detektion: 220 nm]. Nach der Trennung wurden 16.7 mg von Enantiomer 1, welches zuerst eluiert, und 17.5 mg von Enantiomer 2, welches später eluiert, isoliert.

### Enantiomer 1:

Analytische chirale HPLC: Rₜ = 5.80 min, e.e. = 99% [Säule: Daicel Chiralpak^{®} IE 5 µm, 250 x 4.6 mm; Laufmittel: i-Hexan/Ethanol 40:60; Fluss: 1 ml/min; UV-Detektion: 220 nm].
LC-MS (Methode 3): Rₜ = 1.56 min; MS (ESIpos): m/z = 466 [M+H]⁺
¹H-NMR (400 MHz, DMSO-d6) δ [ppm]: -0.149 (1.25), -0.008 (10.99), 0.008 (10.43), 0.146 (1.25), 1.235 (0.77), 2.113 (0.49), 2.128 (0.49), 2.147 (0.97), 2.178 (1.04), 2.190 (1.25), 2.220 (0.97), 2.236 (0.83), 2.257 (1.67), 2.281 (1.74), 2.323 (1.25), 2.328 (1.53), 2.366 (1.32), 2.395 (1.11), 2.424 (1.46), 2.441 (1.25), 2.523 (4.87), 2.563 (2.37), 2.582 (1.67), 2.600 (1.18), 2.665 (1.25), 2.670 (1.67), 2.679 (1.74), 2.687 (1.18), 2.710 (2.50), 2.716 (2.71), 2.726 (1.60), 2.747 (1.88), 2.755 (1.81), 2.911 (1.18), 2.971 (2.50), 3.010 (1.81), 3.545 (1.32), 3.565 (2.64), 3.580 (2.02), 3.596 (1.32), 3.676 (0.42), 3.709 (1.18), 3.751 (1.60), 3.780 (1.81), 3.814 (1.25), 3.896 (1.18), 3.914 (2.92), 3.932 (3.13), 3.951 (1.18), 4.130 (1.04), 4.157 (0.83), 4.173 (1.60), 4.199 (1.60), 4.233 (0.90), 4.879 (0.63), 4.921 (16.00), 4.952 (2.09), 4.964 (2.23), 5.029 (2.02), 5.042 (2.02), 7.512 (6.26), 7.533 (8.00), 7.695 (3.83), 7.701 (3.83), 7.716 (3.20), 7.722 (3.20), 8.299 (5.29), 8.305 (5.08).

### Beispiel 232

### (5RS,7RS)-2-[(6-Chlorpyridin-3-yl)methyl]-5-[(3,3-difluorpyrrolidin-1-yl)carbonyl]-7-(trifluormethyl)-5,6,7,8-tetrahydro[1,2,4]triazolo[4,3-a]pyridin-3(2H)-on (Enantiomer 2)

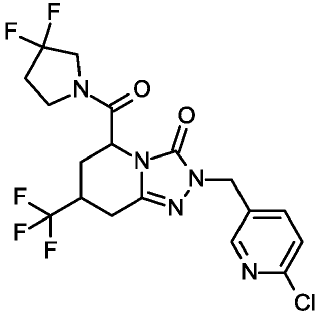

(5RS,7RS)-2-[(6-Chlorpyridin-3-yl)methyl]-5-[(3,3-difluorpyrrolidin-1-yl)carbonyl]-7-(trifluormethyl)-5,6,7,8-tetrahydro[1,2,4]triazolo[4,3-a]pyridin-3(2H)-on (Diastereomer 2, Racemat) wurde durch chirale präparative HPLC getrennt [Probenvorbereitung: 48 mg gelöst in 2 ml Ethanol und 1.5 ml n-Heptan; Injektionsvolumen: 0.5 ml; Säule: Daicel Chiralpak^{®} IE 5 µm, 250 × 20 mm; Laufmittel: n-Heptan/Ethanol 40:60; Fluss: 15 ml/min; Temperatur 40°C; UV-Detektion: 220 nm]. Nach der Trennung wurden 16.7 mg von Enantiomer 1, welches zuerst eluiert, und 17.5 mg von Enantiomer 2, welches später eluiert, isoliert.

### Enantiomer 2:

Analytische chirale HPLC: Rₜ = 6.78 min, e.e. = 99% [Säule: Daicel Chiralpak^{®} IE 5 µm, 250 x 4.6 mm; Laufmittel: i-Hexan/Ethanol 40:60; Fluss: 1 ml/min; UV-Detektion: 220 nm].
LC-MS (Methode 3): Rₜ = 1.56 min; MS (ESIpos): m/z = 466 [M+H]⁺
¹H-NMR (400 MHz, DMSO-d6) δ [ppm]: -0.149 (1.77), -0.008 (16.00), 0.008 (12.99), 0.146 (1.77), 1.234 (0.83), 2.189 (0.94), 2.278 (1.25), 2.323 (1.56), 2.328 (1.97), 2.332 (1.35), 2.366 (2.08), 2.424 (1.14), 2.519 (7.90), 2.523 (6.75), 2.580 (1.25), 2.665 (1.66), 2.670 (2.29), 2.675 (1.87), 2.710 (2.81), 2.716 (1.87), 2.747 (1.35), 2.755 (1.25), 2.970 (1.77), 3.009 (1.25), 3.545 (0.94), 3.565 (1.77), 3.580 (1.35), 3.709 (0.83), 3.751 (0.94), 3.779 (1.35), 3.814 (0.94), 3.896 (0.94), 3.913 (1.97), 3.932 (2.18), 3.950 (0.83), 4.129 (0.73), 4.174 (1.14), 4.199 (1.14), 4.920 (10.91), 4.952 (1.35), 4.964 (1.56), 5.030 (1.25), 5.041 (1.35), 7.512 (4.47), 7.533 (5.82), 7.695 (2.70), 7.701 (2.70), 7.716 (2.29), 7.722 (2.29), 8.299 (3.95), 8.305 (3.84).

### Beispiel 233

### (5RS,7RS)-5-[(3-Hydroxyazetidin-1-yl)carbonyl]-7-(trifluormethyl)-2-{[6-(trifluormethyl)pyridin-3-yl]methyl}-5,6,7,8-tetrahydro[1,2,4]triazolo[4,3-a]pyridin-3(2H)-on (Diastereomer 1; 2 Isomere)

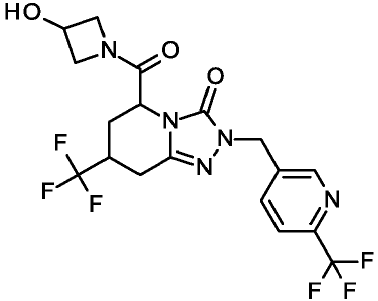

(5RS,7RS)-3-Oxo-7-(trifluormethyl)-2-{[6-(trifluormethyl)pyridin-3-yl]methyl}-2,3,5,6,7,8-hexahydro[1,2,4]triazolo[4,3-a]pyridin-5-carbonsäure (Diastereomerengemisch; 4 Isomere) (53.0 mg, 129 µmol) wurde in Dichlormethan (1.0 ml) und DMF (2.0 m) bei 0°C vorgelegt. Anschließend wurden 1-[Bis(dimethylamino)methylen]-1H-benzotriazol-1-ium-3-oxidhexafluorophosphat (63.7 mg, 168 µmol) und N,N-Diisopropylethylamin (59 µl, 340 µmol) zugegeben. Nach 60 min Rühren bei 0°C wurde Azetidin-3-olhydrochlorid (17.0 mg, 155 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 20.5 mg (34 % d. Th.) der Titelverbindung erhalten.
LC-MS (Methode 3): Rₜ = 1.32 min; MS (ESIpos): m/z = 466 [M+H]⁺
¹H-NMR (400 MHz, DMSO-d6) δ [ppm]: -0.008 (2.82), 0.008 (2.65), 1.236 (0.44), 2.073 (0.95), 2.126 (0.46), 2.145 (1.11), 2.163 (1.78), 2.177 (3.07), 2.190 (4.53), 2.199 (4.11), 2.231 (0.66), 2.328 (0.52), 2.523 (1.78), 2.657 (1.32), 2.666 (1.33), 2.686 (1.72), 2.695 (2.51), 2.704 (1.73), 2.726 (1.98), 2.875 (0.84), 2.905 (1.25), 2.966 (3.44), 2.975 (2.41), 3.005 (2.56), 3.017 (2.01), 3.650 (2.51), 3.659 (1.87), 3.669 (1.95), 3.677 (2.75), 4.027 (1.52), 4.040 (1.57), 4.074 (2.71), 4.089 (2.90), 4.114 (1.38), 4.129 (1.25), 4.144 (1.26), 4.153 (1.29), 4.170 (1.07), 4.461 (0.99), 4.479 (2.15), 4.498 (3.22), 4.520 (2.29), 4.542 (3.24), 4.562 (1.86), 4.579 (0.71), 4.728 (2.11), 4.741 (3.29), 4.749 (2.58), 4.760 (1.45), 5.018 (1.29), 5.043 (10.99), 5.049 (11.28), 5.787 (0.60), 5.801 (0.60), 5.830 (3.51), 5.835 (3.85), 5.844 (4.03), 5.850 (3.07), 7.917 (16.00), 7.948 (0.54), 8.645 (5.51), 8.681 (0.49).

### Beispiel 234

### (5RS,7RS)-5-[(3-Hydroxyazetidin-1-yl)carbonyl]-7-(trifluormethyl)-2-{[6-(trifluormethyl)pyridin-3-yl]methyl}-5,6,7,8-tetrahydro[1,2,4]triazolo[4,3-a]pyridin-3(2H)-on (Isomer 2)

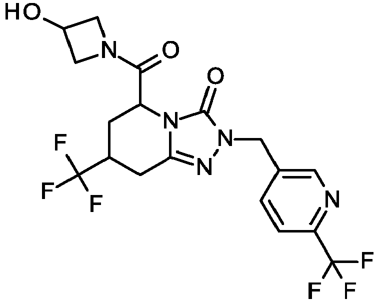

(5RS,7RS)-5-[(3-Hydroxyazetidin-1-yl)carbonyl]-7-(trifluormethyl)-2-{[6-(trifluormethyl)pyridin-3-yl]methyl}-5,6,7,8-tetrahydro[1,2,4]triazolo[4,3-a]pyridin-3(2H)-on (Diastereomer 1; 2 Isomere) wurde durch chirale präparative HPLC getrennt [Probenvorbereitung: 20.5 mg gelöst in 1 ml Ethanol und 1 ml iso-Propanol; Injektionsvolumen: 0.5 ml; Säule: Daicel Chiralpak^{®} IB 5 µm, 250 x 20 mm; Laufmittel: n-Heptan/Ethanol 50:50; Fluss: 20 ml/min; Temperatur 25°C; UV-Detektion: 220 nm]. Nach der Trennung wurden 6.1 mg von Isomer 1, welches zuerst eluiert, und 7.4 mg von Isomer 2, welches später eluiert, isoliert.

### Isomer 2:

Analytische chirale HPLC: Rₜ = 2.34 min, d.e. = 100% [Säule: Daicel Chiralpak^{®} IB-3-3 µm 50 × 4.6 mm; Laufmittel: i-Hexan/i-Propanol 50:50; Fluss: 1 ml/min; UV-Detektion: 220 nm].
LC-MS (Methode 3): Rₜ = 1.31 min; MS (ESIpos): m/z = 466 [M+H]⁺
¹H-NMR (400 MHz, DMSO-d6) δ [ppm]: 1.456 (0.65), 2.145 (1.08), 2.162 (1.77), 2.189 (4.32), 2.230 (0.66), 2.328 (0.77), 2.366 (0.54), 2.657 (1.22), 2.665 (1.38), 2.694 (2.29), 2.704 (1.66), 2.726 (1.80), 2.916 (1.19), 2.965 (3.27), 2.994 (1.08), 3.005 (2.36), 3.017 (1.85), 3.650 (2.33), 3.659 (1.80), 3.677 (2.57), 4.026 (1.47), 4.039 (1.45), 4.073 (2.54), 4.089 (2.75), 4.113 (1.31), 4.129 (1.19), 4.144 (1.21), 4.153 (1.22), 4.170 (1.01), 4.460 (0.89), 4.478 (2.03), 4.498 (3.01), 4.521 (2.19), 4.542 (3.08), 4.561 (1.75), 4.578 (0.70), 4.728 (2.01), 4.740 (3.10), 5.019 (1.22), 5.042 (10.30), 5.049 (10.63), 5.784 (0.61), 5.798 (0.56), 5.831 (4.09), 5.841 (4.20), 7.917 (16.00), 8.645 (6.64), 8.682 (0.65).

### Beispiel 235

### (5RS,7RS)-5-{[(3S)-3-Fluorpyrrolidin-1-yl]carbonyl}-7-(trifluormethyl)-2-{[6-(trifluormethyl)pyridin-3-yl]methyl}-5,6,7,8-tetrahydro[1,2,4]triazolo[4,3-a]pyridin-3(2H)-on (Diastereomerengemisch 1; 2 Isomere)

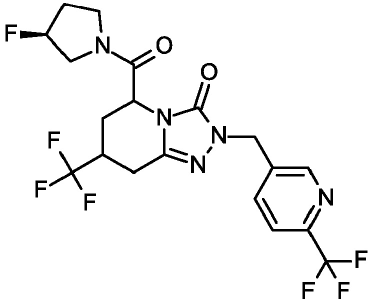

(5RS,7RS)-3-Oxo-7-(trifluormethyl)-2-{[6-(trifluormethyl)pyridin-3-yl]methyl}-2,3,5,6,7,8-hexahydro[1,2,4]triazolo[4,3-a]pyridin-5-carbonsäure (Diastereomerengemisch; 4 Isomere) (100 mg, 244 µmol) und (3S)-3-Fluorpyrrolidinhydrochlorid (33.7 mg, 268 µmol) wurden in Dichlormethan (2.0 ml) und DMF (4.0 ml) bei Raumtemperatur vorgelegt. Anschließend wurden N,N-Diisopropylethylamin (100 µl, 580 µmol) und HBTU (120 mg, 317 µmol) zugegeben. Nach 30 min Rühren bei Raumtemperatur wurden gesättigte wässrige Ammoniumchlorid Lösung und Diethylether zugegeben. Die organische Phase wurde abgetrennt und die wässrige Phase zweimal mit Diethylether extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 9.5 mg (8 % d. Th.) Diastereomerengemisch 1; (2 Isomere), welches zuerst eluiert und 81.0 mg (69 % d. Th.) Diastereomerengemisch 2; (2 Isomere), welches später eluiert, isoliert.

### Diastereomerengemisch 1 (2 Isomere):

LC-MS (Methode 3): Rₜ = 1.52 min; MS (ESIpos): m/z = 482 [M+H]⁺
¹H-NMR (400 MHz, DMSO-d6) δ [ppm]: 0.146 (0.42), 1.629 (0.89), 1.660 (1.92), 1.689 (2.14), 1.739 (1.24), 2.096 (1.31), 2.156 (1.24), 2.230 (1.52), 2.260 (1.60), 2.327 (1.24), 2.366 (0.89), 2.679 (2.18), 2.711 (3.02), 2.719 (3.47), 2.751 (3.40), 2.891 (3.33), 2.930 (2.23), 3.027 (1.55), 3.137 (0.61), 3.400 (0.80), 3.440 (0.66), 3.509 (0.80), 3.564 (0.92), 3.611 (1.71), 3.661 (1.60), 3.687 (2.09), 3.714 (1.57), 3.747 (1.20), 3.828 (0.82), 3.855 (0.84), 3.877 (1.66), 3.900 (1.67), 3.932 (1.62), 3.955 (1.78), 3.980 (0.98), 4.011 (0.71), 4.044 (0.52), 4.675 (0.85), 4.689 (0.85), 4.702 (0.96), 4.716 (0.89), 4.726 (0.92), 4.741 (2.04), 4.754 (2.02), 4.768 (1.95), 4.782 (1.10), 4.808 (0.89), 4.822 (0.98), 4.834 (1.01), 4.849 (0.80), 5.015 (16.00), 5.269 (1.05), 5.349 (0.71), 5.402 (1.38), 5.481 (0.75), 5.527 (0.75), 7.908 (3.10), 7.928 (10.34), 7.944 (6.14), 7.963 (1.78), 8.112 (0.42), 8.677 (8.03).

### Beispiel 236

### (5RS,7RS)-5-{[(3S)-3-Fluorpyrrolidin-1-yl]carbonyl}-7-(trifluormethyl)-2-{[6-(trifluormethyl)pyridin-3-yl]methyl}-5,6,7,8-tetrahydro[1,2,4]triazolo[4,3-a]pyridin-3(2H)-on (Diastereomerengemisch 2; 2 Isomere)

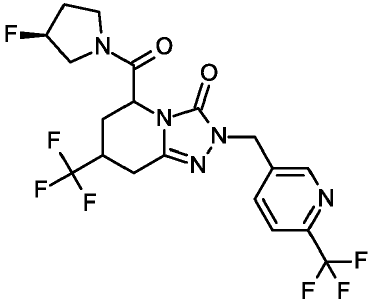

(5RS,7RS)-3-Oxo-7-(trifluormethyl)-2-{[6-(trifluormethyl)pyridin-3-yl]methyl}-2,3,5,6,7,8-hexahydro[1,2,4]triazolo[4,3-a]pyridin-5-carbonsäure (Diastereomerengemisch; 4 Isomere) (100 mg, 244 µmol) und (3S)-3-Fluorpyrrolidinhydrochlorid (33.7 mg, 268 µmol) wurden in Dichlormethan (2.0 ml) und DMF (4.0 ml) bei Raumtemperatur vorgelegt. Anschließend wurden N,N-Diisopropylethylamin (100 µl, 580 µmol) und 1-[Bis(dimethylamino)methylen]-1H-benzotriazol-1-ium-3-oxidhexafluorophosphat (120 mg, 317 µmol) zugegeben. Nach 30 min Rühren bei Raumtemperatur wurden gesättigte wässrige Ammoniumchlorid Lösung und Diethylether zugegeben. Die organische Phase wurde abgetrennt und die wässrige Phase zweimal mit Diethylether extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 9.5 mg (8 % d. Th.) Diastereomerengemisch 1; (2 Isomere), welches zuerst eluiert und 81.0 mg (69 % d. Th.) Diastereomerengemisch 2; (2 Isomere), welches später eluiert, isoliert.

### Diastereomerengemisch 2 (2 Isomere):

LC-MS (Methode 3): Rₜ = 1.59 min; MS (ESIpos): m/z = 482 [M+H]⁺
¹H-NMR (400 MHz, DMSO-d6) δ [ppm]: -0.008 (1.65), 0.008 (1.33), 2.125 (0.90), 2.141 (1.33), 2.160 (1.40), 2.171 (1.70), 2.205 (1.37), 2.215 (1.80), 2.227 (2.15), 2.236 (2.62), 2.248 (2.33), 2.277 (1.60), 2.293 (1.58), 2.328 (0.94), 2.366 (0.44), 2.518 (1.76), 2.523 (1.38), 2.670 (0.51), 2.675 (0.45), 2.686 (0.78), 2.695 (0.80), 2.710 (1.42), 2.724 (2.01), 2.734 (1.28), 2.755 (1.22), 2.763 (1.34), 2.890 (0.98), 2.925 (0.89), 2.981 (2.29), 3.019 (1.52), 3.354 (0.76), 3.380 (0.82), 3.408 (0.70), 3.434 (0.43), 3.443 (0.41), 3.519 (0.48), 3.530 (0.61), 3.541 (0.44), 3.570 (0.90), 3.592 (0.74), 3.620 (1.36), 3.643 (1.74), 3.672 (1.58), 3.689 (0.92), 3.700 (0.92), 3.738 (0.40), 3.765 (0.48), 3.796 (0.63), 3.831 (0.52), 3.852 (1.09), 3.884 (0.51), 3.892 (0.68), 3.930 (0.43), 3.964 (0.61), 3.990 (1.28), 4.017 (0.63), 4.054 (0.48), 4.922 (0.72), 4.937 (0.76), 4.971 (1.02), 4.986 (1.01), 5.017 (0.52), 5.048 (12.85), 5.073 (0.76), 5.084 (1.10), 5.269 (0.96), 5.358 (0.56), 5.403 (1.14), 5.490 (0.53), 5.524 (0.47), 7.919 (16.00), 8.644 (4.92).

### Beispiel 237

### (5S,7R)-5-{[(3S)-3-Fluorpyrrolidin-1-yl]carbonyl}-7-(trifluormethyl)-2-{[6-(trifluormethyl)pyridin-3-yl]methyl}-5,6,7,8-tetrahydro[1,2,4]triazolo[4,3-a]pyridin-3(2H)-on (Isomer 1)

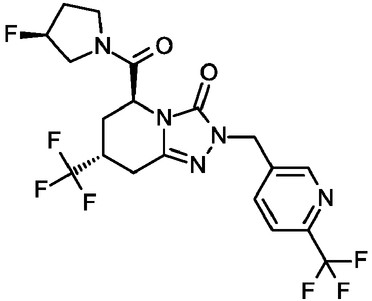

(5RS,7RS)-5-{[(3S)-3-Fluorpyrrolidin-1-yl]carbonyl}-7-(trifluormethyl)-2-{[6-(trifluormethyl)pyridin-3-yl]methyl}-5,6,7,8-tetrahydro[1,2,4]triazolo[4,3-a]pyridin-3(2H)-on Diastereomerengemisch 2 (2 Isomere) wurden durch chirale präparative HPLC getrennt [Probenvorbereitung: 66 mg gelöst in 6 ml iso-Propanol; Injektionsvolumen: 0.5 ml; Säule: Daicel Chiralpak^{®} IB 5 µm, 250 x 20 mm; Laufmittel: n-Heptan/iso-Propanol 40:60; Fluss: 15 ml/min; Temperatur 25°C; UV-Detektion: 210 nm]. Nach der Trennung wurden 28.8 mg von (5S,7R)-5-{[(3S)-3-Fluorpyrrolidin-1-yl]carbonyl}-7-(trifluormethyl)-2-{[6-(trifluormethyl)pyridin-3-yl]methyl}-5,6,7,8-tetrahydro[1,2,4]triazolo[4,3-a]pyridin-3(2H)-on (Isomer 1), welches zuerst eluiert, und 28.8 mg von Isomer 2, welches später eluiert, isoliert.

### Isomer 1:

spez. Drehwert: +42.51 (589nm, 0.5450g / 100cm³ MeOH)
Analytische chirale HPLC: Rₜ = 1.38 min, d.e. = 100% [Säule: Daicel Chiralpak^{®} IB-3-3 µm 50 x 4.6 mm; Laufmittel: i-Hexan/i-Propanol 50:50; Fluss: 1 ml/min; UV-Detektion: 220 nm].
LC-MS (Methode 3): Rₜ = 1.59 min; MS (ESIpos): m/z = 482 [M+H]⁺
¹H-NMR (400 MHz, DMSO-d6) δ [ppm]: 0.008 (2.13), 1.261 (0.42), 2.108 (0.76), 2.120 (0.83), 2.143 (1.30), 2.172 (2.16), 2.204 (1.57), 2.215 (1.43), 2.235 (1.93), 2.250 (1.89), 2.295 (2.57), 2.328 (1.22), 2.523 (1.13), 2.682 (1.10), 2.697 (0.67), 2.710 (1.91), 2.721 (2.02), 2.735 (1.15), 2.750 (1.64), 2.764 (1.04), 2.925 (1.09), 2.934 (1.13), 2.946 (1.01), 2.954 (0.95), 2.981 (3.14), 3.018 (1.94), 3.029 (1.47), 3.346 (0.77), 3.356 (1.00), 3.375 (0.96), 3.384 (0.72), 3.402 (0.97), 3.434 (0.80), 3.443 (0.80), 3.497 (0.57), 3.505 (0.62), 3.533 (0.87), 3.541 (0.80), 3.623 (0.85), 3.646 (2.19), 3.674 (2.40), 3.691 (1.42), 3.700 (1.79), 3.716 (0.76), 3.738 (0.78), 3.766 (0.41), 3.774 (0.45), 3.799 (0.64), 3.806 (0.65), 3.831 (0.97), 3.853 (1.74), 3.877 (0.84), 3.893 (0.73), 3.901 (0.69), 3.930 (0.83), 3.960 (0.62), 3.987 (0.87), 4.019 (0.51), 4.924 (1.35), 4.937 (1.45), 4.972 (2.03), 4.986 (2.00), 5.004 (0.50), 5.016 (0.79), 5.045 (14.11), 5.269 (1.26), 5.394 (1.60), 5.401 (1.65), 5.524 (0.88), 7.919 (16.00), 7.943 (0.44), 8.644 (5.11).

### Beispiel 238

### (5RS,7RS)-2-[(6-Chlorpyridin-3-yl)methyl]-5-[(3-fluorazetidin-1-yl)carbonyl]-7-(trifluormethyl)-5,6,7,8-tetrahydro[1,2,4]triazolo[4,3-a]pyridin-3(2H)-on (Diastereomer 1, Racemat)

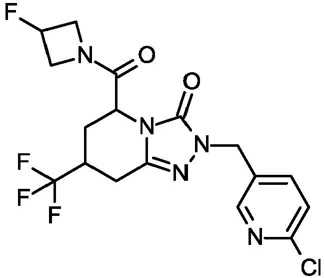

(5RS,7RS)-2-[(6-Chlorpyridin-3-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere) (88.0 mg, 234 µmol) wurde in THF (3.0 ml) bei Raumtemperatur vorgelegt. Anschließend wurden HBTU (115 mg, 304 µmol) und N,N-Diisopropylethylamin (200 µl, 1.2 mmol) zugegeben. Nach 15 min Rühren wurde 3-Fluorazetidinhydrochlorid (31.3 mg, 280 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Nach der Trennung wurden 7.90 mg (8 % d. Th.) von Diastereomer 1 (Racemat), welches zuerst eluiert, und 31 mg (30 % d. Th.) mg von Diastereomer 2 (Racemat), welches später eluiert, isoliert.

### Diastereomer 1, Racemat:

LC-MS (Methode 4): Rₜ = 0.72 min; MS (ESIpos): m/z = 434 [M+H]⁺
¹H-NMR (400 MHz, DMSO-d6) δ [ppm]: -0.149 (1.51), -0.008 (16.00), 0.008 (12.99), 0.146 (1.62), 1.679 (1.16), 1.708 (1.86), 1.739 (1.28), 2.323 (1.86), 2.328 (2.20), 2.332 (1.62), 2.366 (2.43), 2.456 (1.39), 2.523 (7.77), 2.665 (1.97), 2.670 (2.55), 2.675 (2.09), 2.710 (3.94), 2.752 (2.32), 2.867 (2.32), 2.902 (1.28), 2.992 (0.93), 3.969 (0.70), 4.450 (2.67), 4.463 (2.55), 4.478 (2.90), 4.492 (2.09), 4.669 (0.70), 4.902 (6.96), 5.382 (0.70), 5.513 (0.70), 7.517 (3.13), 7.538 (4.06), 7.734 (2.32), 7.754 (1.97), 8.340 (3.36).

### Beispiel 239

### (5RS,7RS)-2-[(6-Chlorpyridin-3-yl)methyl]-5-[(3-fluorazetidin-1-yl)carbonyl]-7-(trifluormethyl)-5,6,7,8-tetrahydro[1,2,4]triazolo[4,3-a]pyridin-3(2H)-on (Diastereomer 2, Racemat)

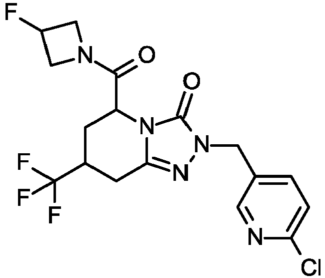

(5RS,7RS)-2-[(6-Chlorpyridin-3-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere) (88.0 mg, 234 µmol) wurde in THF (3.0 ml) bei Raumtemperatur vorgelegt. Anschließend wurden HBTU (115 mg, 304 µmol) und N,N-Diisopropylethylamin (200 µl, 1.2 mmol) zugegeben. Nach 15 min Rühren wurde 3-Fluorazetidinhydrochlorid (31.3 mg, 280 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Nach der Trennung wurden 7.90 mg (8 % d. Th.) von Diastereomer 1 (Racemat), welches zuerst eluiert, und 31 mg (30 % d. Th.) mg von Diastereomer 2 (Racemat), welches später eluiert, isoliert.

### Diastereomer 2, Racemat:

LC-MS (Methode 4): Rₜ = 0.74 min; MS (ESIpos): m/z = 434 [M+H]⁺
¹H-NMR (400 MHz, DMSO-d6) δ [ppm]: -0.150 (1.12), -0.008 (9.92), 0.008 (7.26), 0.146 (0.94), 2.187 (2.13), 2.221 (2.54), 2.253 (3.31), 2.327 (1.83), 2.366 (1.59), 2.524 (5.08), 2.670 (2.24), 2.684 (3.19), 2.696 (3.25), 2.710 (1.83), 2.725 (2.60), 2.968 (5.90), 3.003 (3.66), 3.017 (2.36), 3.978 (1.59), 4.039 (1.83), 4.287 (1.71), 4.427 (2.13), 4.460 (1.83), 4.488 (2.07), 4.737 (5.90), 4.751 (5.49), 4.919 (16.00), 5.358 (1.30), 5.502 (1.24), 7.510 (6.85), 7.531 (8.56), 7.701 (5.73), 7.708 (3.31), 7.721 (4.84), 8.307 (9.03).

### Beispiel 240

### (5RS,7RS)-2-[(6-Chlorpyridin-3-yl)methyl]-5-[(3-fluorazetidin-1-yl)carbonyl]-7-(trifluormethyl)-5,6,7,8-tetrahydro[1,2,4]triazolo[4,3-a]pyridin-3(2H)-on (Enantiomer 1)

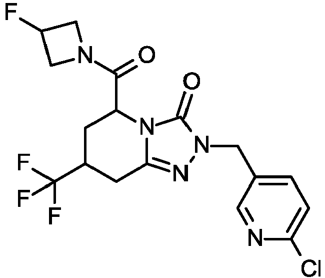

(5RS,7RS)-2-[(6-Chlorpyridin-3-yl)methyl]-5-[(3-fluorazetidin-1-yl)carbonyl]-7-(trifluormethyl)-5,6,7,8-tetrahydro[1,2,4]triazolo[4,3-a]pyridin-3(2H)-on (Diastereomer 2, Racemat) wurde durch chirale präparative HPLC getrennt [Probenvorbereitung: 30 mg gelöst in 2.5 ml Ethanol und 1.5 ml n-Heptan; Injektionsvolumen: 0.7 ml; Säule: Daicel Chiralpak^{®} IB 5 µm, 250 x 20 mm; Laufmittel: n-Heptan/Ethanol 40:60; Fluss: 15 ml/min; Temperatur 40°C; UV-Detektion: 220 nm]. Nach der Trennung wurden 8 mg von Enantiomer 1, welches zuerst eluiert, und 8.8 mg von Enantiomer 2, welches später eluiert, isoliert.

### Enantiomer 1:

Analytische chirale HPLC: Rₜ = 4.56 min, e.e. = 99% [column: Daicel Chiralpak^{®} IB 5 µm, 250 x 4.6 mm; Laufmittel: i-Hexan/Ethanol 50:50; Fluss: 1 ml/min; UV-Detektion: 220 nm].
LC-MS (Methode 3): Rₜ = 1.36 min; MS (ESIpos): m/z = 434 [M+H]⁺
¹H-NMR (400 MHz, DMSO-d6) δ [ppm]: -0.150 (1.23), 0.008 (9.60), 0.146 (0.98), 1.234 (0.80), 2.104 (0.74), 2.121 (0.98), 2.138 (1.85), 2.155 (2.34), 2.169 (2.22), 2.186 (2.15), 2.223 (2.58), 2.252 (3.32), 2.283 (1.23), 2.327 (2.28), 2.366 (2.15), 2.523 (9.23), 2.610 (0.49), 2.669 (2.71), 2.685 (3.38), 2.695 (3.38), 2.709 (2.65), 2.725 (2.58), 2.934 (1.91), 2.943 (2.09), 2.968 (5.97), 3.002 (3.57), 3.013 (2.40), 3.374 (0.68), 3.402 (0.55), 3.950 (1.29), 3.977 (1.60), 4.006 (1.48), 4.039 (1.91), 4.207 (0.92), 4.219 (0.98), 4.235 (1.60), 4.251 (1.78), 4.261 (1.54), 4.279 (1.42), 4.287 (1.72), 4.304 (1.54), 4.333 (0.68), 4.398 (1.66), 4.423 (2.15), 4.459 (1.72), 4.484 (2.03), 4.621 (0.80), 4.633 (0.98), 4.645 (0.86), 4.671 (1.42), 4.687 (1.60), 4.713 (1.60), 4.736 (6.09), 4.751 (5.29), 4.881 (0.68), 4.919 (16.00), 4.967 (0.68), 5.336 (0.49), 5.359 (1.23), 5.401 (0.92), 5.503 (1.23), 5.550 (1.11), 7.511 (6.46), 7.532 (8.25), 7.701 (5.54), 7.721 (4.49), 7.727 (2.77), 8.307 (8.31).

### Beispiel 241

### (5RS,7RS)-2-[(6-Chlorpyridin-3-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on (Diastereomerengemisch 1; 2 Isomere)

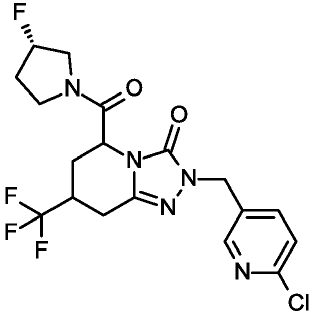

(5RS,7RS)-2-[(6-Chlorpyridin-3-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere) (88.0 mg, 234 µmol) wurde in THF (3.0 ml) bei Raumtemperatur vorgelegt. Anschließend wurden HBTU (115 mg, 304 µmol) und N,N-Diisopropylethylamin (200 µl, 1.2 mmol) zugegeben. Nach 15 min Rühren wurde (3S)-3-Fluorpyrrolidinhydrochlorid (35.2 mg, 280 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Nach der Trennung wurden 11.2 mg (30 % d. Th.) von Diastereomerengemisch 1 (2 Isomere), welches zuerst eluiert, und 54 mg (30 % d. Th.) von Diastereomerengemisch 2 (2 Isomere), welches später eluiert, isoliert.

### Diastereomerengemisch 1 (2 Isomere):

LC-MS (Methode 4): Rₜ = 0.74 min; MS (ESIpos): m/z = 448 [M+H]⁺
¹H-NMR (400 MHz, DMSO-d6) δ [ppm]: -0.149 (1.86), -0.033 (0.47), -0.025 (0.56), -0.022 (0.65), -0.008 (16.00), 0.008 (14.19), 0.018 (0.98), 0.146 (1.86), 1.620 (0.42), 1.651 (1.07), 1.664 (0.37), 1.681 (1.07), 1.695 (0.70), 1.704 (0.74), 1.711 (0.60), 1.731 (0.65), 2.051 (0.33), 2.094 (0.65), 2.111 (0.65), 2.124 (0.65), 2.156 (0.56), 2.211 (0.60), 2.226 (0.70), 2.259 (0.79), 2.323 (1.21), 2.327 (1.58), 2.332 (1.16), 2.366 (1.35), 2.523 (5.30), 2.578 (0.60), 2.589 (0.56), 2.594 (0.56), 2.670 (2.70), 2.702 (1.49), 2.710 (3.26), 2.742 (1.86), 2.882 (1.77), 2.922 (1.21), 2.999 (0.74), 3.019 (0.93), 3.043 (0.60), 3.271 (0.51), 3.351 (0.88), 3.368 (0.74), 3.379 (0.47), 3.399 (0.51), 3.426 (0.42), 3.436 (0.37), 3.506 (0.42), 3.514 (0.42), 3.525 (0.42), 3.534 (0.37), 3.556 (0.56), 3.583 (0.47), 3.608 (0.98), 3.622 (0.74), 3.636 (0.65), 3.649 (0.74), 3.656 (0.88), 3.682 (1.07), 3.690 (0.70), 3.707 (0.70), 3.718 (0.60), 3.745 (0.60), 3.792 (0.37), 3.824 (0.60), 3.832 (0.37), 3.849 (0.47), 3.871 (0.74), 3.894 (0.84), 3.910 (0.47), 3.919 (0.70), 3.927 (0.88), 3.954 (0.88), 3.974 (0.51), 4.005 (0.37), 4.664 (0.42), 4.678 (0.51), 4.691 (0.47), 4.706 (0.51), 4.714 (0.60), 4.729 (1.07), 4.743 (1.02), 4.756 (1.07), 4.771 (0.56), 4.796 (0.51), 4.810 (0.56), 4.823 (0.56), 4.838 (0.60), 4.890 (9.02), 4.930 (0.37), 5.269 (0.51), 5.277 (0.56), 5.347 (0.42), 5.400 (0.70), 5.478 (0.37), 5.526 (0.37), 7.516 (3.53), 7.536 (4.42), 7.719 (1.49), 7.726 (2.47), 7.733 (1.81), 7.739 (1.40), 7.746 (2.09), 7.754 (1.44), 8.332 (3.49).

### Beispiel 242

### (5RS,7RS)-2-[(6-Chlorpyridin-3-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on (Diastereomerengemisch 2; 2 Isomere)

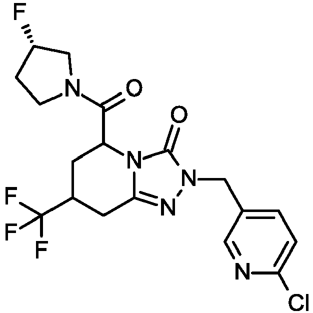

(5RS,7RS)-2-[(6-Chlorpyridin-3-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere) (88.0 mg, 234 µmol) wurde in THF (3.0 ml) bei Raumtemperatur vorgelegt. Anschließend wurden HBTU (115 mg, 304 µmol) und N,N-Diisopropylethylamin (200 µl, 1.2 mmol) zugegeben. Nach 15 min Rühren wurde (3S)-3-Fluorpyrrolidinhydrochlorid (35.2 mg, 280 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Nach der Trennung wurden 11.2 mg (30 % d. Th.) von Diastereomerengemisch 1 (2 Isomere), welches zuerst eluiert, und 54 mg (30 % d. Th.) von Diastereomerengemisch 2 (2 Isomere), welches später eluiert, isoliert.

### Diastereomerengemisch 2 (2 Isomere):

LC-MS (Methode 4): Rₜ = 0.77 min; MS (ESIpos): m/z = 448 [M+H]⁺
¹H-NMR (400 MHz, DMSO-d6) δ [ppm]: -0.149 (0.48), -0.008 (4.34), 0.008 (3.69), 0.146 (0.48), 2.132 (1.45), 2.150 (1.72), 2.161 (2.14), 2.196 (1.69), 2.205 (2.00), 2.226 (3.10), 2.239 (2.31), 2.278 (2.03), 2.322 (1.03), 2.327 (1.07), 2.332 (0.72), 2.366 (0.52), 2.523 (2.31), 2.674 (1.48), 2.685 (0.86), 2.714 (2.38), 2.724 (1.38), 2.744 (1.41), 2.753 (1.45), 2.908 (1.03), 2.973 (2.66), 3.011 (1.69), 3.356 (1.03), 3.373 (1.00), 3.402 (0.83), 3.437 (0.55), 3.512 (0.55), 3.520 (0.62), 3.564 (1.07), 3.586 (0.86), 3.613 (1.62), 3.631 (1.83), 3.667 (1.90), 3.695 (1.14), 3.728 (0.45), 3.761 (0.52), 3.793 (0.66), 3.824 (0.55), 3.847 (1.21), 3.866 (0.62), 3.889 (0.72), 3.925 (0.45), 3.958 (0.59), 3.986 (1.45), 4.012 (0.76), 4.050 (0.55), 4.875 (0.45), 4.921 (16.00), 4.957 (1.34), 4.969 (1.21), 5.013 (1.00), 5.025 (0.97), 5.067 (1.07), 5.265 (1.03), 5.355 (0.62), 5.399 (1.31), 5.487 (0.59), 5.522 (0.52), 7.511 (6.45), 7.532 (8.07), 7.691 (2.24), 7.698 (4.55), 7.705 (2.69), 7.712 (2.00), 7.719 (3.76), 7.725 (2.14), 8.304 (5.21).

### Beispiel 243

### (5RS,7RS)-2-[(6-Chlorpyridin-3-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on (Isomer 1)

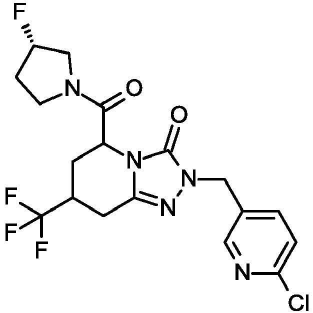

### (5RS,7RS)-2-[(6-Chlorpyridin-3-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on

(Diastereomerengemisch 2; 2 Isomere) wurde durch chirale präparative HPLC getrennt [Probenvorbereitung: 54 mg gelöst in 1 ml Ethanol und 1 ml Acetonitril; Injektionsvolumen: 0.01 ml; Säule: Daicel Chiralpak^{®} IE 5 µm, 250 x 20 mm; Laufmittel: n-Heptan/Ethanol 50:50; Fluss: 20 ml/min; Temperatur 30°C; UV-Detektion: 220 nm]. Nach der Trennung wurden 15.1 mg von Isomer 1, welches zuerst eluiert, und 15.9 mg von Isomer 2, welches später eluiert, isoliert.

### Isomer 1:

Analytische chirale HPLC: Rₜ = 2.65 min, d.e. = 99% [Säule: Daicel Chiralpak^{®} IE, 50 x 4.6 mm; Laufmittel: n-Heptan/Ethanol 50:50; Fluss: 1 ml/min; UV-Detektion: 220 nm].
LC-MS (Methode 3): Rₜ = 1.44 min; MS (ESIpos): m/z = 448 [M+H]⁺
¹H-NMR (400 MHz, DMSO-d6) δ [ppm]: -0.149 (1.26), -0.008 (10.67), 0.008 (11.41), 0.147 (1.43), 0.857 (0.97), 0.877 (0.92), 2.118 (1.43), 2.135 (1.78), 2.165 (3.38), 2.196 (2.24), 2.207 (2.29), 2.225 (2.58), 2.242 (2.70), 2.263 (1.84), 2.282 (3.78), 2.322 (3.50), 2.327 (3.56), 2.332 (2.87), 2.366 (2.29), 2.523 (10.27), 2.670 (4.07), 2.688 (1.26), 2.701 (2.01), 2.710 (4.65), 2.726 (1.72), 2.740 (2.24), 2.755 (1.55), 2.877 (0.92), 2.897 (1.26), 2.940 (1.72), 2.972 (4.36), 3.008 (2.70), 3.020 (2.18), 3.368 (2.12), 3.397 (1.66), 3.427 (1.26), 3.492 (0.97), 3.526 (1.26), 3.537 (1.09), 3.615 (1.26), 3.637 (2.81), 3.667 (3.67), 3.693 (2.92), 3.713 (1.09), 3.732 (1.03), 3.802 (1.03), 3.825 (1.66), 3.845 (2.41), 3.866 (1.55), 3.889 (1.26), 3.896 (1.09), 3.921 (1.09), 3.953 (1.15), 3.979 (1.43), 4.876 (0.97), 4.915 (16.00), 4.957 (3.21), 4.970 (2.92), 5.266 (1.72), 5.391 (2.47), 5.409 (1.38), 5.524 (1.38), 7.511 (9.00), 7.532 (10.67), 7.692 (2.87), 7.698 (6.02), 7.705 (3.73), 7.712 (2.29), 7.719 (5.16), 7.725 (2.92), 8.300 (7.80).

### Beispiel 244

### (5RS,7RS)-2-[(6-Chlorpyridin-3-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on (Isomer 2)

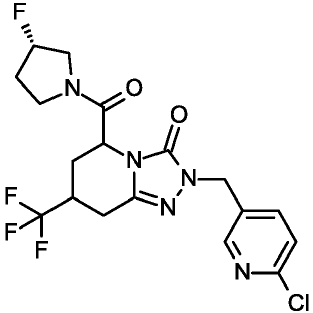

### (5RS,7RS)-2-[(6-Chlorpyridin-3-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on

(Diastereomerengemisch 2; 2 Isomere) wurde durch chirale präparative HPLC getrennt [Probenvorbereitung: 54 mg gelöst in 1 ml Ethanol und 1 ml Acetonitril; Injektionsvolumen: 0.01 ml; Säule: Daicel Chiralpak^{®} IE 5 µm, 250 x 20 mm; Laufmittel: n-Heptan/Ethanol 50:50; Fluss: 20 ml/min; Temperatur 30°C; UV Detektion: 220 nm]. Nach der Trennung wurden 15.1 mg von Isomer 1, welches zuerst eluiert, und 15.9 mg von Isomer 2, welches später eluiert, isoliert.

### Isomer 2:

Analytische chiral HPLC: Rₜ = 3.15 min, d.e. = 93% [column: Daicel Chiralpak^{®} IE, 50 x 4.6 mm; Laufmittel: n-Heptan/Ethanol 50:50; Fluss: 1 ml/min; UV detection: 220 nm].
LC-MS (Methode 3): Rₜ = 1.45 min; MS (ESIpos): m/z = 448 [M+H]⁺
¹H-NMR (400 MHz, DMSO-d6) δ [ppm]: -0.008 (2.36), 0.008 (2.14), 2.116 (1.14), 2.132 (1.25), 2.150 (1.89), 2.166 (1.40), 2.181 (1.17), 2.196 (1.36), 2.205 (1.97), 2.226 (3.32), 2.239 (2.34), 2.274 (1.40), 2.327 (0.75), 2.366 (0.56), 2.523 (2.11), 2.670 (0.81), 2.675 (1.30), 2.684 (0.93), 2.705 (1.33), 2.715 (2.12), 2.724 (1.17), 2.745 (1.53), 2.753 (1.41), 2.895 (0.95), 2.964 (2.30), 2.975 (1.56), 3.003 (1.68), 3.014 (1.31), 3.355 (2.49), 3.373 (1.63), 3.384 (1.05), 3.401 (0.76), 3.512 (0.76), 3.521 (0.79), 3.564 (1.60), 3.587 (1.26), 3.614 (1.91), 3.630 (1.82), 3.656 (1.02), 3.666 (0.92), 3.682 (0.50), 3.761 (0.51), 3.782 (0.56), 3.791 (0.58), 3.848 (0.48), 3.880 (0.64), 3.888 (0.58), 3.965 (0.74), 3.988 (1.92), 4.012 (0.76), 4.049 (0.84), 4.078 (0.56), 4.922 (16.00), 5.014 (1.53), 5.027 (1.50), 5.067 (1.64), 5.076 (0.99), 5.274 (0.94), 5.356 (0.94), 5.406 (0.90), 5.487 (0.90), 7.512 (4.52), 7.532 (5.71), 7.691 (1.70), 7.698 (3.25), 7.705 (1.96), 7.712 (1.54), 7.719 (2.69), 7.726 (1.59), 8.304 (3.85).

### Beispiel 245

### (5RS,7RS)-2-[(6-Chlorpyridin-3-yl)methyl]-5-{[(3R)-3-fluorpyrrolidin-1-yl]carbonyl}-7-(trifluormethyl)-5,6,7,8-tetrahydro[1,2,4]triazolo[4,3-a]pyridin-3(2H)-on (Diastereomerengemisch 2; 2 Isomere)

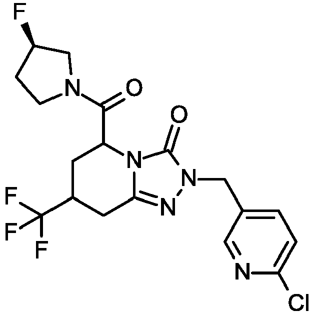

(5RS,7RS)-2-[(6-Chlorpyridin-3-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere) (88.0 mg, 234 µmol) wurde in THF (3.0 ml) bei Raumtemperatur vorgelegt. Anschließend wurden HBTU (115 mg, 304 µmol) und N,N-Diisopropylethylamin (200 µl, 1.2 mmol) zugegeben. Nach 15 min Rühren wurde (3R)-3-Fluorpyrrolidinhydrochlorid (35.2 mg, 280 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Nach der Trennung wurden 20.1 mg (10 % d. Th.) von Diastereomerengemisch 1 (2 Isomere), welches zuerst eluiert, und 46 mg (44 % d. Th.) von Diastereomerengemisch 2 (2 Isomere), welches später eluiert, isoliert.

### Diastereomerengemisch 2 (2 Isomere):

LC-MS (Methode 4): Rₜ = 0.77 min; MS (ESIpos): m/z = 448 [M+H]⁺
¹H-NMR (400 MHz, DMSO-d6) δ [ppm]: -0.150 (1.12), -0.008 (10.52), 0.008 (7.16), 0.146 (1.12), 2.115 (1.37), 2.162 (2.24), 2.181 (1.43), 2.205 (2.05), 2.227 (3.24), 2.272 (1.99), 2.323 (1.99), 2.328 (2.18), 2.337 (0.93), 2.366 (1.06), 2.523 (4.54), 2.670 (2.30), 2.711 (2.49), 2.724 (1.43), 2.741 (1.43), 2.754 (1.49), 2.895 (1.18), 2.972 (2.61), 3.011 (1.74), 3.372 (1.31), 3.400 (0.93), 3.437 (0.62), 3.487 (0.81), 3.512 (0.75), 3.524 (0.81), 3.564 (1.18), 3.586 (1.00), 3.613 (1.74), 3.633 (1.87), 3.667 (1.99), 3.696 (1.18), 3.713 (0.68), 3.734 (0.62), 3.763 (0.56), 3.793 (0.87), 3.825 (0.56), 3.848 (1.25), 3.867 (0.75), 3.889 (0.68), 3.917 (0.56), 3.966 (0.68), 3.986 (1.56), 4.018 (0.75), 4.047 (0.81), 4.082 (0.56), 4.921 (16.00), 4.958 (1.31), 4.969 (1.25), 5.011 (1.25), 5.026 (1.25), 5.064 (1.06), 5.273 (1.06), 5.345 (0.68), 5.353 (0.75), 5.401 (1.56), 5.493 (0.68), 5.521 (0.68), 7.511 (6.91), 7.532 (8.53), 7.692 (2.24), 7.698 (4.61), 7.705 (2.86), 7.712 (2.12), 7.719 (3.98), 7.725 (2.49), 8.305 (6.16).

### Beispiel 246

### (5RS,7RS)-2-[(6-Chlorpyridin-3-yl)methyl]-5-{[(3R)-3-fluorpyrrolidin-1-yl]carbonyl}-7-(trifluormethyl)-5,6,7,8-tetrahydro[1,2,4]triazolo[4,3-a]pyridin-3(2H)-on (Isomer 1)

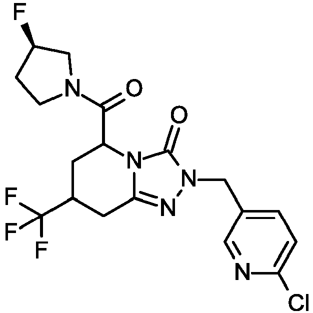

### (5RS,7RS)-2-[(6-Chlorpyridin-3-yl)methyl]-5-{[(3R)-3-fluorpyrrolidin-1-yl]carbonyl}-7-(trifluormethyl)-5,6,7,8-tetrahydro[1,2,4]triazolo[4,3-a]pyridin-3(2H)-on

(Diastereomerengemisch 2, 2 Isomere) wurde durch chirale präparative HPLC getrennt [Probenvorbereitung: 45.8 mg gelöst in 2.5 ml Ethanol, 2.5 ml n-Heptan und 1 ml Dichlormethan; Injektionsvolumen: 0.4 ml; Säule: Daicel Chiralpak^{®} IE 5 µm, 250 x 20 mm; Laufmittel: n-Heptan/Ethanol 50:50; Fluss: 15 ml/min; Temperatur 40°C; UV-Detektion: 220 nm]. Nach der Trennung wurden 19.8 mg von Isomer 1, welches zuerst eluiert, und 16 mg von Isomer 2, welches später eluiert, isoliert.

### Isomer 1:

Analytische chirale HPLC: Rₜ = 7.02 min, d.e. = 99% [Säule: Daicel Chiralpak^{®} IE 5 µm, 250 x 4.6 mm; Laufmittel: n-Heptan/Ethanol 50:50; Fluss: 1 ml/min; UV-Detektion: 220 nm].
LC-MS (Methode 3): Rₜ = 1.42 min; MS (ESIpos): m/z = 448 [M+H]⁺
¹H-NMR (400 MHz, DMSO-d6) δ [ppm]: -0.149 (0.66), -0.008 (6.74), 0.008 (5.83), 0.146 (0.71), 0.846 (0.63), 0.862 (0.58), 1.086 (0.66), 1.102 (0.79), 1.236 (0.76), 2.115 (1.06), 2.131 (1.22), 2.150 (1.80), 2.181 (1.09), 2.204 (1.85), 2.226 (3.25), 2.273 (1.27), 2.327 (1.01), 2.332 (0.71), 2.366 (0.91), 2.523 (3.17), 2.665 (0.89), 2.670 (1.19), 2.674 (1.47), 2.684 (1.01), 2.714 (2.21), 2.723 (1.27), 2.745 (1.62), 2.753 (1.47), 2.887 (0.96), 2.963 (2.33), 3.003 (1.70), 3.014 (1.34), 3.355 (1.09), 3.373 (1.09), 3.383 (0.74), 3.401 (0.61), 3.512 (0.76), 3.520 (0.79), 3.564 (1.62), 3.586 (1.27), 3.614 (1.90), 3.630 (1.83), 3.657 (1.01), 3.665 (0.94), 3.683 (0.51), 3.759 (0.51), 3.783 (0.66), 3.857 (0.48), 3.880 (0.63), 3.964 (0.76), 3.986 (1.93), 4.020 (0.74), 4.046 (0.86), 4.080 (0.61), 4.922 (16.00), 5.014 (1.55), 5.026 (1.52), 5.067 (1.67), 5.274 (0.94), 5.357 (0.91), 5.407 (0.89), 5.489 (0.89), 7.512 (4.74), 7.532 (6.03), 7.691 (1.85), 7.698 (3.37), 7.705 (2.03), 7.712 (1.70), 7.719 (2.87), 7.726 (1.67), 8.304 (4.13).

### Beispiel 247

### (5RS,7RS)-2-[(6-Chlorpyridin-3-yl)methyl]-5-{[(3R)-3-fluorpyrrolidin-1-yl]carbonyl}-7-(trifluormethyl)-5,6,7,8-tetrahydro[1,2,4]triazolo[4,3-a]pyridin-3(2H)-on (Isomer 2)

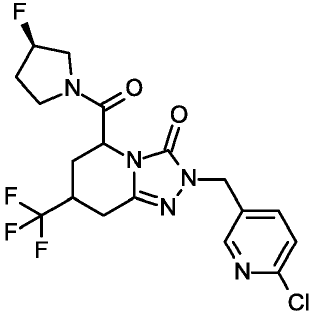

### (5RS,7RS)-2-[(6-Chlorpyridin-3-yl)methyl]-5-{[(3R)-3-fluorpyrrolidin-1-yl]carbonyl}-7-(trifluormethyl)-5,6,7,8-tetrahydro[1,2,4]triazolo[4,3-a]pyridin-3(2H)-on

(Diastereomerengemisch 2, 2 Isomere) wurde durch chirale präparative HPLC getrennt [Probenvorbereitung: 45.8 mg gelöst in 2.5 ml Ethanol, 2.5 ml n-Heptan und 1 ml Dichlormethan; Injektionsvolumen: 0.4 ml; Säule: Daicel Chiralpak^{®} IE 5 µm, 250 x 20 mm; Laufmittel: n-Heptan/Ethanol 50:50; Fluss: 15 ml/min; Temperatur 40°C; UV-Detektion: 220 nm]. Nach der Trennung wurden 19.8 mg von Isomer 1, welches zuerst eluiert, und 16 mg von Isomer 2, welches später eluiert, isoliert.

### Isomer 2:

Analytische chirale HPLC: Rₜ = 9.14 min, d.e. = 99% [Säule: Daicel Chiralpak^{®} IE 5 µm, 250 x 4.6 mm; Laufmittel: n-Heptan/Ethanol 50:50; Fluss: 1 ml/min; UV-Detektion: 220 nm].
LC-MS (Methode 3): Rₜ = 1.42 min; MS (ESIpos): m/z = 448 [M+H]⁺
¹H-NMR (400 MHz, DMSO-d6) δ [ppm]: -0.149 (1.57), 0.008 (11.20), 0.146 (1.25), 0.857 (0.52), 1.021 (0.73), 1.102 (0.59), 1.234 (1.22), 1.976 (0.70), 2.003 (0.70), 2.082 (0.63), 2.108 (1.22), 2.133 (1.91), 2.167 (3.17), 2.194 (2.37), 2.225 (2.23), 2.233 (2.40), 2.241 (2.71), 2.282 (3.83), 2.327 (2.57), 2.366 (1.46), 2.590 (1.08), 2.672 (3.41), 2.687 (1.22), 2.701 (2.30), 2.711 (3.83), 2.725 (1.81), 2.741 (2.26), 2.755 (1.57), 2.933 (1.67), 2.970 (4.52), 3.010 (2.92), 3.021 (2.23), 3.339 (1.53), 3.350 (1.70), 3.369 (1.53), 3.395 (1.60), 3.427 (1.46), 3.437 (1.25), 3.499 (0.97), 3.526 (1.29), 3.536 (1.32), 3.618 (1.18), 3.641 (2.89), 3.668 (3.65), 3.694 (2.78), 3.712 (1.04), 3.730 (1.43), 3.768 (0.70), 3.795 (0.94), 3.822 (1.46), 3.847 (2.54), 3.869 (1.39), 3.888 (1.01), 3.921 (1.36), 3.956 (0.80), 3.980 (1.36), 4.011 (0.97), 4.875 (1.01), 4.916 (16.00), 4.957 (3.23), 4.971 (3.06), 5.268 (1.74), 5.398 (2.64), 5.521 (1.22), 7.512 (8.10), 7.532 (10.33), 7.698 (5.46), 7.704 (3.72), 7.719 (4.49), 7.725 (2.96), 8.301 (7.48), 17.633 (0.52).

### Beispiel 248

### (5RS,7RS)-2-[(6-Chlorpyridin-3-yl)methyl]-5-{[(3R,4S)-3,4-difluorpyrrolidin-1-yl]carbonyl}-7-(trifluormethyl)-5,6,7,8-tetrahydro[1,2,4]triazolo[4,3-a]pyridin-3(2H)-on (Diastereomer 1, Racemat)

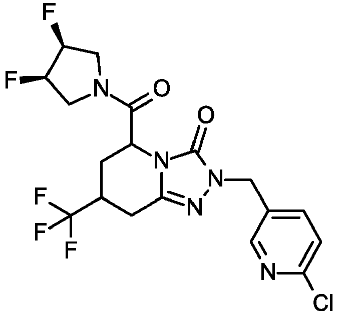

(5RS,7RS)-2-[(6-Chlorpyridin-3-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere) (88.0 mg, 234 µmol) wurde in THF (3.0 ml) bei Raumtemperatur vorgelegt. Anschließend wurden HBTU (115 mg, 304 µmol) und N,N-Diisopropylethylamin (200 µl, 1.2 mmol) zugegeben. Nach 15 min Rühren wurde (3R,4S)-3,4-Difluorpyrrolidinhydrochlorid (40.2 mg, 280 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Nach der Trennung wurden 15.5 mg (13 % d. Th.) von Diastereomer 1 (Racemat), welches zuerst eluiert, und 59 mg (54 % d. Th.) von Diastereomer 2 (Racemat), welches später eluiert, isoliert.

### Diastereomer 1, Racemat:

LC-MS (Methode 4): Rₜ = 0.77 min; MS (ESIpos): m/z = 466 [M+H]⁺
¹H-NMR (400 MHz, DMSO-d6) δ [ppm]: -0.150 (1.14), -0.008 (10.54), 0.007 (8.83), 0.145 (1.14), 1.635 (1.97), 1.663 (2.16), 1.697 (1.84), 1.728 (1.65), 2.327 (1.84), 2.366 (1.40), 2.522 (7.49), 2.559 (3.05), 2.669 (2.16), 2.679 (2.60), 2.711 (4.06), 2.719 (3.43), 2.752 (3.94), 2.884 (3.43), 2.924 (2.41), 2.992 (1.65), 3.002 (1.78), 3.011 (1.84), 3.507 (1.27), 3.517 (1.52), 3.551 (1.71), 3.559 (1.84), 3.570 (1.27), 3.605 (1.33), 3.614 (1.02), 3.638 (1.52), 3.651 (1.46), 3.671 (1.14), 3.684 (1.02), 3.696 (1.40), 3.709 (1.33), 3.729 (1.40), 3.743 (1.40), 3.766 (1.08), 3.779 (1.40), 3.792 (1.59), 3.805 (1.71), 3.818 (1.40), 3.826 (1.46), 3.839 (1.40), 3.867 (1.21), 3.906 (1.08), 4.083 (1.14), 4.097 (1.27), 4.110 (1.65), 4.125 (2.10), 4.143 (1.33), 4.153 (1.97), 4.169 (1.52), 4.709 (2.03), 4.723 (2.60), 4.735 (2.79), 4.751 (2.29), 4.760 (1.59), 4.890 (16.00), 4.920 (3.68), 5.245 (1.27), 5.273 (1.40), 5.283 (1.27), 5.306 (0.95), 5.319 (1.02), 5.325 (1.02), 5.340 (1.08), 5.368 (1.27), 5.378 (1.52), 5.391 (1.40), 5.404 (1.46), 5.413 (1.21), 5.426 (0.89), 5.435 (0.95), 5.449 (1.14), 5.458 (1.02), 5.469 (1.02), 5.483 (0.95), 7.514 (6.16), 7.535 (7.43), 7.698 (0.89), 7.716 (3.05), 7.723 (3.11), 7.727 (3.43), 7.734 (3.75), 7.743 (2.48), 7.748 (2.73), 7.754 (2.54), 8.302 (1.21), 8.325 (3.62), 8.331 (6.92), 8.338 (4.00).

### Beispiel 249

### (5RS,7RS)-2-[(6-Chlorpyridin-3-yl)methyl]-5-{[(3R,4S)-3,4-difluorpyrrolidin-1-yl]carbonyl}-7-(trifluormethyl)-5,6,7,8-tetrahydro[1,2,4]triazolo[4,3-a]pyridin-3(2H)-on (Diastereomer 2, Racemat)

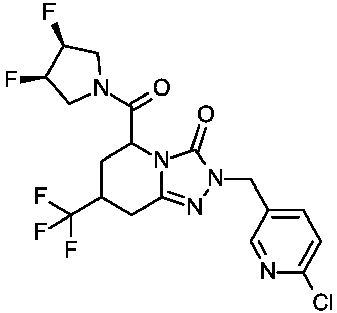

(5RS,7RS)-2-[(6-Chlorpyridin-3-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere) (88.0 mg, 234 µmol) wurde in THF (3.0 ml) bei Raumtemperatur vorgelegt. Anschließend wurden HBTU (115 mg, 304 µmol) und N,N-Diisopropylethylamin (200 µl, 1.2 mmol) zugegeben. Nach 15 min Rühren wurde (3R,4S)-3,4-Difluorpyrrolidinhydrochlorid (40.2 mg, 280 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Nach der Trennung wurden 15.5 mg (13 % d. Th.) von Diastereomer 1 (Racemat), welches zuerst eluiert, und 59 mg (54 % d. Th.) von Diastereomer 2 (Racemat), welches später eluiert, isoliert.

### Diastereomer 2, Racemat:

LC-MS (Methode 4): Rₜ = 0.80 min; MS (ESIpos): m/z = 466 [M+H]⁺
¹H-NMR (400 MHz, DMSO-d6) δ [ppm]: -0.008 (3.00), 0.008 (2.78), 1.030 (0.77), 1.045 (0.77), 2.122 (0.58), 2.138 (0.63), 2.156 (1.24), 2.172 (1.13), 2.187 (1.27), 2.203 (2.42), 2.210 (2.40), 2.219 (2.23), 2.232 (1.38), 2.274 (1.85), 2.305 (1.07), 2.328 (0.55), 2.366 (0.44), 2.670 (0.72), 2.676 (1.60), 2.689 (1.05), 2.706 (1.90), 2.717 (2.84), 2.728 (1.35), 2.746 (2.23), 2.757 (1.62), 2.881 (1.27), 2.963 (3.19), 2.975 (2.18), 3.003 (2.37), 3.015 (1.87), 3.492 (0.83), 3.501 (0.91), 3.534 (1.38), 3.542 (1.27), 3.584 (1.35), 3.643 (0.94), 3.656 (0.85), 3.676 (0.72), 3.689 (0.74), 3.700 (1.57), 3.714 (1.43), 3.733 (1.07), 3.748 (0.99), 3.764 (0.72), 3.777 (0.74), 3.797 (0.55), 3.810 (0.55), 3.840 (0.66), 3.853 (0.94), 3.868 (1.24), 3.888 (1.18), 3.907 (0.99), 4.041 (0.72), 4.055 (0.99), 4.069 (0.63), 4.088 (0.99), 4.103 (0.96), 4.117 (0.66), 4.131 (0.69), 4.152 (0.58), 4.167 (0.63), 4.180 (0.58), 4.195 (1.16), 4.211 (0.69), 4.224 (0.58), 4.240 (0.55), 4.878 (0.61), 4.918 (16.00), 4.963 (0.66), 4.990 (3.00), 5.004 (4.43), 5.259 (0.83), 5.267 (0.94), 5.286 (0.94), 5.294 (0.94), 5.307 (0.55), 5.325 (0.74), 5.341 (0.74), 5.358 (0.74), 5.367 (0.77), 5.383 (1.18), 5.397 (1.10), 5.405 (0.94), 5.415 (1.05), 5.426 (0.96), 5.439 (0.66), 5.455 (0.74), 5.469 (0.74), 5.489 (0.80), 7.512 (5.43), 7.533 (6.88), 7.688 (1.96), 7.695 (2.45), 7.698 (2.97), 7.705 (2.86), 7.709 (2.01), 7.719 (2.51), 7.725 (2.26), 8.295 (2.73), 8.301 (5.34), 8.307 (3.33).

### Beispiel 250

### (5RS,7RS)-2-[(6-Chlorpyridin-3-yl)methyl]-5-{[(3R,4S)-3,4-difluorpyrrolidin-1-yl]carbonyl}-7-(trifluormethyl)-5,6,7,8-tetrahydro[1,2,4]triazolo[4,3-a]pyridin-3(2H)-on (Enantiomer 1)

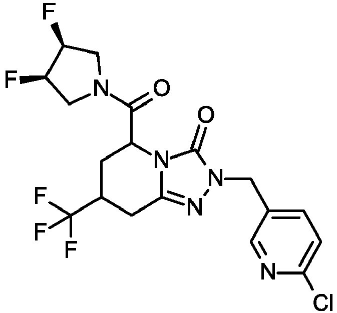

(5RS,7RS)-2-[(6-Chlorpyridin-3-yl)methyl]-5-{[(3R,4S)-3,4-difluorpyrrolidin-1-yl]carbonyl}-7-(trifluormethyl)-5,6,7,8-tetrahydro[1,2,4]triazolo[4,3-a]pyridin-3(2H)-on (Diastereomer 2, Racemat) wurde durch chirale präparative HPLC getrennt [Probenvorbereitung: 59 mg gelöst in 3 ml Ethanol; Injektionsvolumen: 0.05 ml; Säule: Daicel Chiralpak^{®} IE 5 µm, 250 x 20 mm; Laufmittel: n-Heptan/Ethanol 50:50; Fluss: 20 ml/min; Temperatur 30°C; UV-Detektion: 220 nm]. Nach der Trennung wurden 13.8 mg von Enantiomer 1, welches zuerst eluiert, und 15.8 mg von Enantiomer 2, welches später eluiert, isoliert.

### Enantiomer 1:

Analytische chirale HPLC: Rₜ = 1.89 min, e.e. = 99% [Säule: Daicel Chiralpak^{®} IE, 50 x 4.6 mm; Laufmittel: n-Heptan/Ethanol 50:50; Fluss: 1 ml/min; UV-Detektion: 220 nm].
LC-MS (Methode 3): Rₜ = 1.48 min; MS (ESIpos): m/z = 466 [M+H]⁺
¹H-NMR (400 MHz, DMSO-d6) δ [ppm]: -0.149 (0.93), -0.008 (9.80), 0.008 (7.71), 0.146 (0.93), 0.859 (0.96), 1.135 (1.58), 1.153 (3.15), 1.171 (1.61), 2.156 (1.16), 2.171 (1.23), 2.187 (1.37), 2.203 (2.50), 2.209 (2.47), 2.218 (2.36), 2.271 (2.06), 2.307 (1.16), 2.327 (1.61), 2.366 (1.37), 2.523 (5.31), 2.669 (1.85), 2.674 (2.02), 2.688 (1.13), 2.709 (2.47), 2.716 (2.81), 2.727 (1.54), 2.746 (2.33), 2.757 (1.68), 2.891 (1.64), 2.910 (2.09), 2.928 (1.51), 2.964 (3.22), 2.976 (2.09), 3.003 (2.50), 3.014 (1.92), 3.491 (0.96), 3.499 (0.99), 3.533 (1.44), 3.582 (1.30), 3.641 (0.93), 3.656 (0.96), 3.700 (1.54), 3.712 (1.44), 3.732 (1.03), 3.745 (1.03), 3.840 (0.89), 3.868 (1.20), 3.888 (1.30), 4.056 (0.99), 4.085 (0.99), 4.102 (1.06), 4.195 (1.23), 4.918 (16.00), 4.990 (3.05), 5.003 (4.52), 5.272 (0.99), 5.283 (1.03), 5.295 (0.99), 5.340 (0.86), 5.384 (1.16), 5.404 (1.03), 5.425 (1.13), 5.504 (0.89), 7.512 (6.17), 7.532 (7.57), 7.688 (2.09), 7.698 (3.19), 7.704 (3.25), 7.719 (2.81), 7.725 (2.57), 8.301 (7.02).

### Beispiel 251

### (5RS,7RS)-5-[(3,3-Difluorpyrrolidin-1-yl)carbonyl]-2-{[3-fluor-2-(trifluormethyl)pyridin-4-yl]methyl}-7-(trifluormethyl)-5,6,7,8-tetrahydro[1,2,4]triazolo[4,3-a]pyridin-3(2H)-on (Diastereomer 2, Racemat)

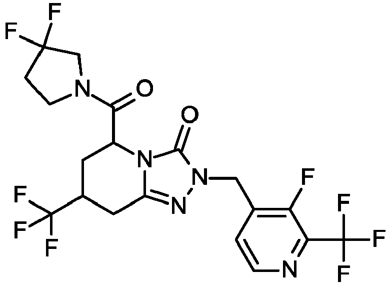

(5RS,7RS)-2-{[3-Fluor-2-(trifluormethyl)pyridin-4-yl]methyl}-3-oxo-7-(trifluormethyl)-2,3,5,6,7,8-hexahydro[1,2,4]triazolo[4,3-a]pyridin-5-carbonsäure (Diastereomerengemisch; 4 Isomere) (93.0 mg, 217 µmol) wurde in THF (2.0 ml) bei Raumtemperatur vorgelegt. Anschließend wurden HBTU (107 mg, 282 µmol) und N,N-Diisopropylethylamin (190 µl, 1.1 mmol) zugegeben. Nach 15 min Rühren wurde 3,3-Difluorpyrrolidinhydrochlorid (37.4 mg, 261 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Nach der Trennung wurden 9.3 mg (6 % d. Th.) von Diastereomer 1 (Racemat), welches zuerst eluiert, und 75.8 mg (66 % d. Th.) von Diastereomer 2 (Racemat), welches später eluiert, isoliert.
LC-MS (Methode 3): Rₜ = 1.91 min; MS (ESIpos): m/z = 518 [M+H]⁺
¹H-NMR (400 MHz, DMSO-d6) δ [ppm]: -0.008 (2.21), 0.008 (2.19), 1.030 (0.81), 1.045 (0.81), 2.073 (0.62), 2.130 (0.89), 2.146 (1.06), 2.165 (2.14), 2.171 (1.67), 2.177 (1.87), 2.182 (2.05), 2.196 (2.25), 2.206 (2.48), 2.212 (2.38), 2.222 (1.95), 2.237 (1.87), 2.252 (1.71), 2.279 (3.19), 2.300 (3.44), 2.328 (2.21), 2.332 (2.35), 2.366 (0.89), 2.377 (1.24), 2.397 (2.11), 2.427 (2.79), 2.443 (2.48), 2.462 (1.67), 2.473 (1.67), 2.523 (2.87), 2.565 (3.40), 2.583 (2.73), 2.603 (1.84), 2.622 (0.92), 2.665 (0.62), 2.670 (0.78), 2.674 (0.62), 2.702 (2.40), 2.709 (2.86), 2.731 (3.35), 2.740 (4.75), 2.749 (3.10), 2.771 (3.90), 2.778 (3.67), 2.920 (2.35), 2.993 (4.94), 3.025 (2.56), 3.033 (3.68), 3.041 (2.21), 3.520 (0.62), 3.539 (1.16), 3.550 (2.35), 3.569 (6.00), 3.589 (4.68), 3.604 (2.37), 3.618 (0.90), 3.636 (0.49), 3.685 (0.67), 3.718 (2.17), 3.755 (3.76), 3.788 (4.03), 3.817 (2.33), 3.851 (0.54), 3.876 (0.54), 3.894 (1.25), 3.902 (2.44), 3.919 (4.70), 3.934 (5.06), 3.953 (2.29), 3.960 (1.38), 3.979 (0.54), 4.105 (0.75), 4.135 (1.98), 4.147 (0.81), 4.163 (1.70), 4.178 (3.27), 4.204 (3.16), 4.236 (1.84), 4.267 (0.52), 4.984 (3.75), 4.997 (3.71), 5.048 (4.57), 5.062 (3.98), 5.074 (4.49), 5.089 (15.44), 5.117 (16.00), 5.158 (4.49), 7.561 (5.02), 7.573 (9.68), 7.586 (5.37), 8.568 (11.97), 8.580 (12.08).

### Beispiel 252

### (5RS,7RS)-5-[(3,3-Difluorpyrrolidin-1-yl)carbonyl]-2-{[3-fluor-2-(trifluormethyl)pyridin-4-yl]methyl}-7-(trifluormethyl)-5,6,7,8-tetrahydro[1,2,4]triazolo[4,3-a]pyridin-3(2H)-on (Enantiomer 2)

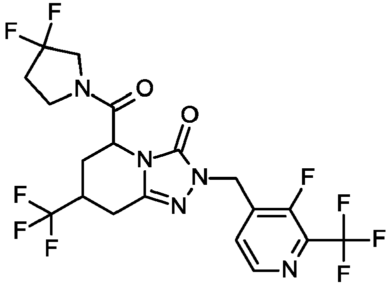

### (5RS,7RS)-5-[(3,3-Difluorpyrrolidin-1-yl)carbonyl]-2-{[3-fluor-2-(trifluormethyl)pyridin-4-yl]methyl}-7-(trifluormethyl)-5,6,7,8-tetrahydro[1,2,4]triazolo[4,3-a]pyridin-3(2H)-on

(Diastereomer 2, Racemat) wurde durch chirale präparative HPLC getrennt [Probenvorbereitung: 75.8 mg in 3 ml Isopropanol im Ultraschallbad gelöst, dann 3 ml n-Heptan zugegeben; Injektionsvolumen: 0.8 ml; Säule: Daicel Chiralpak^{®} IA 5 µm, 250 x 20 mm; Laufmittel: n-Heptan/Isopropanol 50:50; Fluss: 15 ml/min; Temperatur 30°C; UV-Detektion: 220 nm]. Nach der Trennung wurden 11.4 mg von Enantiomer 1, welches zuerst eluiert, und 13.3 mg von Enantiomer 2, welches später eluiert, isoliert.

### Enantiomer 2:

Analytische chirale HPLC: Rₜ = 7.07 min, e.e. = 99% [Säule: Daicel Chiralpak^{®} IA 5 µm, 250 x 4.6 mm; Laufmittel: i-Hexan/Isopropanol 50:50; Fluss: 1 ml/min; UV-Detektion: 220 nm].
LC-MS (Methode 3): Rₜ = 1.77 min; MS (ESIpos): m/z = 518 [M+H]⁺
¹H-NMR (400 MHz, DMSO-d6) δ [ppm]: -0.149 (1.82), -0.008 (16.00), 0.008 (15.76), 0.146 (1.87), 1.260 (0.77), 2.165 (0.72), 2.180 (0.62), 2.205 (0.86), 2.252 (0.57), 2.281 (1.01), 2.296 (1.15), 2.322 (1.87), 2.327 (2.44), 2.332 (1.96), 2.366 (1.72), 2.397 (0.72), 2.409 (0.67), 2.426 (0.96), 2.444 (1.01), 2.523 (5.99), 2.665 (1.58), 2.670 (2.11), 2.674 (1.58), 2.701 (0.81), 2.710 (2.30), 2.731 (1.10), 2.739 (1.49), 2.770 (1.20), 2.778 (1.15), 2.928 (0.81), 2.993 (1.63), 3.033 (1.15), 3.550 (0.77), 3.569 (1.96), 3.588 (1.58), 3.604 (0.77), 3.717 (0.77), 3.756 (1.15), 3.787 (1.29), 3.818 (0.72), 3.903 (0.72), 3.919 (1.49), 3.935 (1.58), 3.951 (0.72), 4.135 (0.62), 4.178 (1.01), 4.204 (1.01), 4.983 (1.25), 4.996 (1.20), 5.047 (1.53), 5.061 (1.20), 5.074 (1.39), 5.089 (4.84), 5.116 (5.08), 5.158 (1.49), 7.560 (1.68), 7.573 (3.16), 7.586 (1.72), 8.568 (4.02), 8.580 (3.98).

### Beispiel 253

### (5RS,7RS)-5-[(3-Fluorazetidin-1-yl)carbonyl]-2-{[3-fluor-2-(trifluormethyl)pyridin-4-yl]methyl}-7-(trifluormethyl)-5,6,7,8-tetrahydro[1,2,4]triazolo[4,3-a]pyridin-3(2H)-on (Diastereomer 1, Racemat)

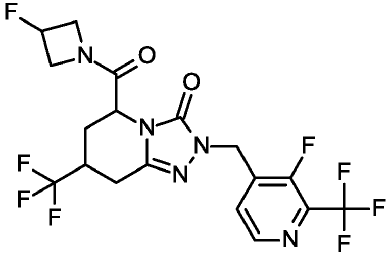

(5RS,7RS)-2-{[3-Fluor-2-(trifluormethyl)pyridin-4-yl]methyl}-3-oxo-7-(trifluormethyl)-2,3,5,6,7,8-hexahydro[1,2,4]triazolo[4,3-a]pyridin-5-carbonsäure (Diastereomerengemisch; 4 Isomere) (93.0 mg, 217 µmol) wurde in THF (2.0 ml) bei Raumtemperatur vorgelegt. Anschließend wurden HBTU (107 mg, 282 µmol) und N,N-Diisopropylethylamin (190 µl, 1.1 mmol) zugegeben. Nach 15 min Rühren wurde 3-Fluorazetidinhydrochlorid (29.1 mg, 261 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Nach der Trennung wurden 6.7 mg (6 % d. Th.) von Diastereomer 1 (Racemat), welches zuerst eluiert, und 65.5 mg (62 % d. Th.) von Diastereomer 2 (Racemat), welches später eluiert, isoliert.

### Diastereomer 1, Racemat:

LC-MS (Methode 3): Rₜ = 1.70 min; MS (ESIpos): m/z = 486 [M+H]⁺
¹H-NMR (400 MHz, DMSO-d6) δ [ppm]: -0.150 (1.81), -0.009 (15.15), 0.007 (16.00), 0.146 (1.64), 1.667 (0.62), 1.698 (1.81), 1.728 (2.83), 1.757 (2.04), 1.790 (0.79), 2.327 (2.83), 2.365 (2.54), 2.457 (2.09), 2.522 (8.37), 2.664 (1.87), 2.669 (2.49), 2.709 (2.94), 2.737 (2.37), 2.745 (2.49), 2.776 (2.77), 2.888 (3.11), 2.917 (1.70), 2.928 (1.87), 2.968 (0.62), 3.004 (1.36), 3.017 (1.36), 3.246 (0.51), 3.905 (0.62), 3.943 (1.07), 3.971 (1.36), 4.006 (1.02), 4.041 (0.79), 4.164 (0.73), 4.178 (0.62), 4.190 (0.62), 4.211 (0.79), 4.230 (0.96), 4.255 (0.96), 4.282 (0.73), 4.307 (0.73), 4.321 (0.51), 4.338 (0.62), 4.355 (0.85), 4.384 (1.30), 4.412 (1.24), 4.443 (1.58), 4.471 (3.39), 4.485 (3.00), 4.498 (2.88), 4.512 (2.54), 4.587 (0.57), 4.602 (0.73), 4.639 (1.13), 4.658 (1.24), 4.696 (0.62), 4.727 (0.68), 4.742 (0.51), 5.070 (9.78), 5.079 (10.06), 5.380 (1.24), 5.517 (1.24), 7.635 (3.45), 7.649 (6.05), 7.661 (3.39), 8.579 (5.82), 8.590 (5.48).

### Beispiel 254

### (5RS,7RS)-5-[(3-Fluorazetidin-1-yl)carbonyl]-2-{[3-fluor-2-(trifluormethyl)pyridin-4-yl]methyl}-7-(trifluormethyl)-5,6,7,8-tetrahydro[1,2,4]triazolo[4,3-a]pyridin-3(2H)-on (Diastereomer 2, Racemat)

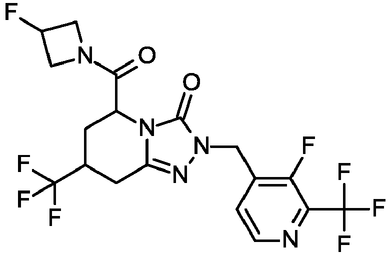

(5RS,7RS)-2-{[3-Fluor-2-(trifluormethyl)pyridin-4-yl]methyl}-3-oxo-7-(trifluormethyl)-2,3,5,6,7,8-hexahydro[1,2,4]triazolo[4,3-a]pyridin-5-carbonsäure (Diastereomerengemisch; 4 Isomere) (93.0 mg, 217 µmol) wurde in THF (2.0 ml) bei Raumtemperatur vorgelegt. Anschließend wurden HBTU (107 mg, 282 µmol) und N,N-Diisopropylethylamin (190 µl, 1.1 mmol) zugegeben. Nach 15 min Rühren wurde 3-Fluorazetidinhydrochlorid (29.1 mg, 261 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Nach der Trennung wurden 6.7 mg (6 % d. Th.) von Diastereomer 1 (Racemat), welches zuerst eluiert, und 65.5 mg (62 % d. Th.) von Diastereomer 2 (Racemat), welches später eluiert, isoliert.

### Diastereomer 2, Racemat:

LC-MS (Methode 3): Rₜ = 1.75 min; MS (ESIpos): m/z = 486 [M+H]⁺
¹H-NMR (400 MHz, DMSO-d6) δ [ppm]: -0.149 (0.51), -0.008 (3.67), 0.008 (3.01), 1.030 (0.61), 1.045 (0.71), 2.073 (0.61), 2.137 (1.68), 2.156 (2.90), 2.173 (3.62), 2.187 (3.57), 2.203 (3.16), 2.219 (1.73), 2.240 (3.67), 2.273 (5.10), 2.308 (1.89), 2.328 (1.43), 2.366 (1.12), 2.524 (3.72), 2.679 (2.85), 2.709 (5.76), 2.718 (4.89), 2.748 (4.38), 2.989 (8.76), 3.027 (5.04), 3.036 (3.62), 3.970 (2.55), 4.001 (2.04), 4.031 (2.24), 4.213 (1.27), 4.241 (2.04), 4.270 (2.29), 4.286 (2.70), 4.335 (1.12), 4.403 (2.34), 4.428 (2.75), 4.463 (2.50), 4.490 (3.01), 4.626 (1.17), 4.644 (1.43), 4.671 (2.24), 4.683 (2.50), 4.698 (2.34), 4.710 (2.09), 4.722 (2.19), 4.770 (8.36), 4.785 (7.85), 5.052 (3.72), 5.093 (16.00), 5.110 (9.17), 5.121 (9.27), 5.151 (1.94), 5.163 (2.45), 5.362 (1.78), 5.408 (1.73), 5.505 (1.78), 5.551 (1.68), 7.567 (5.61), 7.579 (10.90), 7.592 (5.91), 8.568 (11.87), 8.580 (11.87).

### Beispiel 255

### (5RS,7RS)-5-[(3-Fluorazetidin-1-yl)carbonyl]-2-{[3-fluor-2-(trifluormethyl)pyridin-4-yl]methyl}-7-(trifluormethyl)-5,6,7,8-tetrahydro[1,2,4]triazolo[4,3-a]pyridin-3(2H)-on (Enantiomer 2)

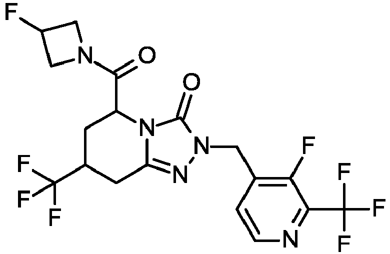

(5RS,7RS)-5-[(3-Fluorazetidin-1-yl)carbonyl]-2-{[3-fluor-2-(trifluormethyl)pyridin-4-yl]methyl}-7-(trifluormethyl)-5,6,7,8-tetrahydro[1,2,4]triazolo[4,3-a]pyridin-3(2H)-on (Diastereomer 2, Racemat) wurde durch chirale präparative HPLC getrennt [Probenvorbereitung: 65.5 mg in 3 ml Isopropanol gelöst, dann 1 ml Dichlormethan und 2 ml n-Heptan zugegeben; Injektionsvolumen: 0.4 ml; Säule: Daicel Chiralpak^{®} IA 5 µm, 250 x 20 mm; Laufmittel: n-Heptan/Isopropanol 50:50; Fluss: 15 ml/min; Temperatur 30°C; UV-Detektion: 220 nm]. Nach der Trennung wurden 5.7 mg von Enantiomer 1, welches zuerst eluiert, und 5.4 mg von Enantiomer 2, welches später eluiert, isoliert.

### Enantiomer 2:

Analytische chirale HPLC: Rₜ = 7.09 min, e.e. = 99% [Säule: Daicel Chiralpak^{®} IA 5 µm, 250 x 4.6 mm; Laufmittel: n-Heptan/Isopropanol 50:50; Fluss: 1 ml/min; UV-Detektion: 220 nm].
LC-MS (Methode 3): Rₜ = 1.64 min; MS (ESIpos): m/z = 486 [M+H]⁺
¹H-NMR (400 MHz, DMSO-d6) δ [ppm]: -0.150 (1.04), -0.009 (16.00), 0.007 (7.79), 0.017 (0.39), 0.146 (1.01), 0.986 (0.27), 1.002 (0.30), 1.234 (0.42), 2.136 (0.27), 2.155 (0.44), 2.172 (0.54), 2.185 (0.54), 2.201 (0.52), 2.218 (0.32), 2.241 (0.62), 2.272 (0.74), 2.308 (0.30), 2.317 (0.30), 2.322 (0.49), 2.327 (0.57), 2.331 (0.44), 2.366 (0.59), 2.523 (3.72), 2.561 (0.27), 2.563 (0.27), 2.664 (0.57), 2.669 (0.76), 2.674 (0.71), 2.709 (1.26), 2.717 (0.71), 2.747 (0.64), 2.951 (0.44), 2.958 (0.47), 2.988 (1.28), 3.025 (0.74), 3.035 (0.52), 3.967 (0.37), 3.999 (0.32), 4.030 (0.32), 4.053 (0.25), 4.212 (0.25), 4.241 (0.30), 4.256 (0.37), 4.268 (0.37), 4.281 (0.42), 4.308 (0.27), 4.401 (0.35), 4.431 (0.47), 4.461 (0.42), 4.490 (0.42), 4.670 (0.37), 4.683 (0.39), 4.694 (0.42), 4.707 (0.37), 4.721 (0.35), 4.738 (0.27), 4.768 (1.21), 4.783 (1.08), 5.052 (0.54), 5.092 (2.29), 5.109 (1.38), 5.120 (1.33), 5.151 (0.32), 5.161 (0.37), 5.361 (0.27), 5.408 (0.25), 5.505 (0.30), 5.753 (1.97), 7.565 (0.89), 7.578 (1.53), 7.591 (0.84), 8.567 (1.73), 8.579 (1.65).

### Beispiel 256

### (5RS,7RS)-5-{[(3S)-3-Fluorpyrrolidin-1-yl]carbonyl}-2-{[3-fluor-2-(trifluormethyl)pyridin-4-yl]methyl}-7-(trifluormethyl)-5,6,7,8-tetrahydro[1,2,4]triazolo[4,3-a]pyridin-3(2H)-on (Diastereomerengemisch 2; 2 Isomere)

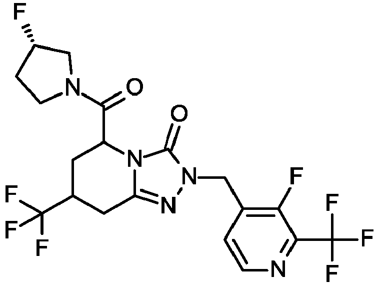

(5RS,7RS)-2-{[3-Fluor-2-(trifluormethyl)pyridin-4-yl]methyl}-3-oxo-7-(trifluormethyl)-2,3,5,6,7,8-hexahydro[1,2,4]triazolo[4,3-a]pyridin-5-carbonsäure (Diastereomerengemisch; 4 Isomere) (93.0 mg, 217 µmol) wurde in THF (2.0 ml) bei Raumtemperatur vorgelegt. Anschließend wurden HBTU (107 mg, 282 µmol) und N,N-Diisopropylethylamin (190 µl, 1.1 mmol) zugegeben. Nach 15 min Rühren wurde (3S)-3-Fluorpyrrolidinhydrochlorid (32.7 mg, 261 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Nach der Trennung wurden 8.8 mg (7 % d. Th.) von Diastereomerengemisch 1 (2 Isomere), welches zuerst eluiert, und 68.3 mg (63 % d. Th.) von Diastereomerengemisch 2 (2 Isomere), welches später eluiert, isoliert.

### Diastereomerengemisch 2, 2 Isomere:

LC-MS (Methode 3): Rₜ = 1.76 min; MS (ESIpos): m/z = 500 [M+H]⁺
¹H-NMR (400 MHz, DMSO-d6) δ [ppm]: -0.149 (0.68), -0.008 (5.73), 0.008 (6.83), 0.146 (0.68), 2.000 (0.94), 2.117 (2.71), 2.149 (4.64), 2.167 (4.38), 2.183 (4.43), 2.213 (4.27), 2.224 (6.57), 2.234 (6.88), 2.245 (8.96), 2.282 (4.59), 2.323 (2.24), 2.328 (2.87), 2.333 (2.76), 2.366 (2.29), 2.523 (7.35), 2.670 (2.35), 2.675 (1.77), 2.700 (2.55), 2.710 (4.59), 2.730 (3.65), 2.738 (6.41), 2.749 (4.07), 2.768 (3.96), 2.778 (4.48), 2.927 (2.97), 2.995 (7.71), 3.033 (5.00), 3.163 (14.80), 3.173 (14.85), 3.360 (8.08), 3.378 (5.73), 3.388 (4.07), 3.406 (3.75), 3.436 (2.14), 3.446 (2.03), 3.508 (1.41), 3.527 (1.93), 3.572 (3.07), 3.596 (2.55), 3.621 (4.74), 3.643 (6.41), 3.662 (3.44), 3.671 (5.32), 3.687 (3.18), 3.696 (3.07), 3.713 (1.30), 3.736 (1.30), 3.763 (1.72), 3.794 (2.14), 3.832 (1.62), 3.853 (3.70), 3.891 (2.24), 3.928 (1.30), 3.963 (2.14), 3.990 (4.38), 4.023 (1.88), 4.087 (4.38), 4.938 (2.29), 4.950 (2.35), 4.986 (3.23), 4.999 (3.13), 5.045 (5.16), 5.087 (11.41), 5.095 (14.85), 5.117 (15.84), 5.159 (4.12), 5.269 (3.13), 5.358 (1.77), 5.402 (3.75), 5.489 (1.72), 5.524 (1.41), 7.562 (5.73), 7.574 (10.32), 7.587 (5.37), 8.136 (13.29), 8.568 (16.00), 8.580 (16.00).

### Beispiel 257

### (5RS,7RS)-5-{[(3S)-3-Fluorpyrrolidin-1-yl]carbonyl}-2-{[3-fluor-2-(trifluormethyl)pyridin-4-yl]methyl}-7-(trifluormethyl)-5,6,7,8-tetrahydro[1,2,4]triazolo[4,3-a]pyridin-3(2H)-on (Isomer 1)

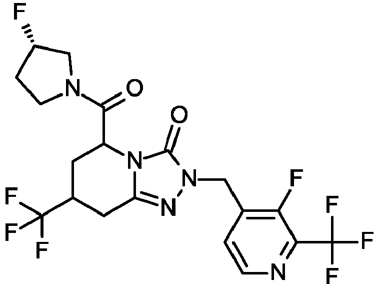

### (5RS,7RS)-5-{[(3S)-3-Fluorpyrrolidin-1-yl]carbonyl}-2-{[3-fluor-2-(trifluormethyl)pyridin-4-yl]methyl}-7-(trifluormethyl)-5,6,7,8-tetrahydro[1,2,4]triazolo[4,3-a]pyridin-3(2H)-on

(Diastereomerengemisch 2; 2 Isomere) wurde durch chirale präparative HPLC getrennt [Probenvorbereitung: 68.3 mg gelöst in 3 ml Acetonitril und 2 ml Ethanol (warm); Injektionsvolumen: 0.25 ml; Säule: Daicel Chiralpak^{®} IB 5 µm, 250 x 20 mm; Laufmittel: n-Heptan/Isopropanol 50:50; Fluss: 15 ml/min; Temperatur 25°C; UV-Detektion: 210 nm]. Nach der Trennung wurden 30.7 mg von Isomer 1, welches zuerst eluiert, und 27.2 mg von Isomer 2, welches später eluiert, isoliert.

### Isomer 1:

Analytische chirale HPLC: Rₜ = 2.17 min, d.e. = 99% [Säule: Daicel Chiraltek^{®} IB 3 µm, 50 x 4.6 mm; Laufmittel: i-Hexan/Ethanol 50:50; Fluss: 1 ml/min; UV-Detektion: 220 nm].
LC-MS (Methode 4): Rₜ = 0.89 min; MS (ESIpos): m/z = 500 [M+H]⁺
¹H-NMR (400 MHz, DMSO-d6) δ [ppm]: -0.149 (1.69), -0.008 (15.64), 0.008 (16.00), 0.145 (1.87), 1.235 (1.60), 2.189 (4.18), 2.207 (3.56), 2.243 (4.62), 2.302 (5.60), 2.327 (3.82), 2.332 (3.91), 2.366 (3.56), 2.523 (11.82), 2.669 (4.00), 2.696 (2.93), 2.710 (5.69), 2.724 (4.00), 2.736 (5.24), 2.749 (3.11), 2.765 (4.18), 2.779 (2.84), 2.994 (8.18), 3.032 (4.80), 3.045 (3.64), 3.330 (4.98), 3.350 (2.67), 3.359 (3.38), 3.378 (2.76), 3.406 (2.76), 3.445 (2.22), 3.507 (1.69), 3.534 (2.31), 3.543 (2.13), 3.621 (2.22), 3.640 (4.98), 3.670 (5.96), 3.686 (3.47), 3.696 (4.62), 3.735 (2.13), 3.802 (1.69), 3.832 (2.40), 3.855 (4.80), 3.929 (1.96), 3.962 (1.60), 3.988 (2.31), 4.938 (3.47), 4.950 (3.56), 4.985 (5.16), 4.999 (4.98), 5.042 (4.00), 5.086 (13.60), 5.118 (11.82), 5.159 (3.73), 5.270 (3.29), 5.400 (4.18), 5.523 (2.31), 7.561 (4.89), 7.573 (9.87), 7.587 (5.42), 8.568 (13.42), 8.580 (13.60).

### Beispiel 258

### (5RS,7RS)-5-{[(3S)-3-Fluorpyrrolidin-1-yl]carbonyl}-2-{[3-fluor-2-(trifluormethyl)pyridin-4-yl]methyl}-7-(trifluormethyl)-5,6,7,8-tetrahydro[1,2,4]triazolo[4,3-a]pyridin-3(2H)-on (Isomer 2)

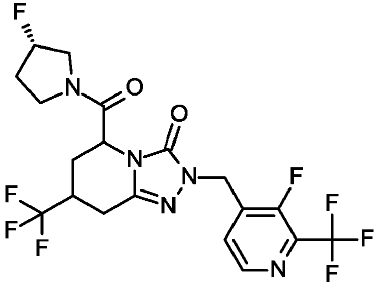

### (5RS,7RS)-5-{[(3S)-3-Fluorpyrrolidin-1-yl]carbonyl}-2-{[3-fluor-2-(trifluormethyl)pyridin-4-yl]methyl}-7-(trifluormethyl)-5,6,7,8-tetrahydro[1,2,4]triazolo[4,3-a]pyridin-3(2H)-on

(Diastereomerengemisch 2; 2 Isomere) wurde durch chirale präparative HPLC getrennt [Probenvorbereitung: 68.3 mg gelöst in 3 ml Acetonitril und 2 ml Ethanol (warm); Injektionsvolumen: 0.25 ml; Säule: Daicel Chiralpak^{®} IB 5 µm, 250 x 20 mm; Laufmittel: n-Heptan/Isopropanol 50:50; Fluss: 15 ml/min; Temperatur 25°C; UV-Detektion: 210 nm]. Nach der Trennung wurden 30.7 mg von Isomer 1, welches zuerst eluiert, und 27.2 mg von Isomer 2, welches später eluiert, isoliert.

### Isomer 2:

Analytische chirale HPLC: Rₜ = 3.68 min, d.e. = 97.8% [Säule: Daicel Chiraltek^{®} IB 3 µm, 50 x 4.6 mm; Laufmittel: i-Hexan/Ethanol 50:50; Fluss: 1 ml/min; UV-Detektion: 220 nm].
LC-MS (Methode 4): Rₜ = 0.89 min; MS (ESIpos): m/z = 500 [M+H]⁺
¹H-NMR (400 MHz, DMSO-d6) δ [ppm]: -0.149 (1.67), -0.008 (14.33), 0.008 (12.71), 0.146 (1.67), 1.234 (1.12), 2.148 (3.10), 2.168 (3.16), 2.179 (2.17), 2.197 (2.17), 2.223 (4.71), 2.233 (5.33), 2.244 (6.88), 2.281 (2.91), 2.323 (2.23), 2.327 (2.98), 2.332 (2.29), 2.366 (2.36), 2.523 (8.06), 2.665 (2.05), 2.670 (2.91), 2.674 (2.17), 2.700 (2.11), 2.710 (4.03), 2.730 (2.73), 2.738 (4.22), 2.749 (2.42), 2.770 (3.22), 2.778 (3.10), 2.910 (1.98), 2.987 (4.65), 3.025 (3.41), 3.037 (2.85), 3.361 (2.36), 3.379 (2.29), 3.408 (1.18), 3.518 (1.67), 3.571 (3.16), 3.595 (2.79), 3.620 (3.72), 3.642 (3.72), 3.661 (2.05), 3.764 (1.12), 3.795 (1.24), 3.884 (1.43), 3.965 (1.61), 3.992 (3.60), 4.013 (1.43), 4.053 (1.80), 4.087 (1.24), 5.053 (4.40), 5.095 (16.00), 5.114 (10.67), 5.159 (1.98), 5.275 (2.05), 5.359 (1.98), 5.407 (1.86), 5.490 (1.86), 7.556 (2.67), 7.569 (5.33), 7.576 (5.52), 7.588 (2.79), 8.569 (9.92), 8.581 (9.67).

### Beispiel 259

### (5RS,7RS)-5-{[(3R,4S)-3,4-Difluorpyrrolidin-1-yl]carbonyl}-2-{[3-fluor-2-(trifluormethyl)pyridin-4-yl]methyl}-7-(trifluormethyl)-5,6,7,8-tetrahydro[1,2,4]triazolo[4,3-a]pyridin-3(2H)-on (Diastereomer 1, Racemat)

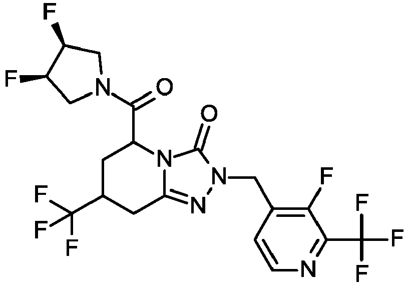

(5RS,7RS)-2-{[3-Fluor-2-(trifluormethyl)pyridin-4-yl]methyl}-3-oxo-7-(trifluormethyl)-2,3,5,6,7,8-hexahydro[1,2,4]triazolo[4,3-a]pyridin-5-carbonsäure (Diastereomerengemisch; 4 Isomere) (93.0 mg, 217 µmol) wurde in THF (2.0 ml) bei Raumtemperatur vorgelegt. Anschließend wurden HBTU (107 mg, 282 µmol) und N,N-Diisopropylethylamin (190 µl, 1.1 mmol) zugegeben. Nach 15 min Rühren wurde (3R,4S)-3,4-Difluorpyrrolidinhydrochlorid (37.4 mg, 261 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Nach der Trennung wurden 8.5 mg (8 % d. Th.) von Diastereomer 1 (Racemat), welches zuerst eluiert, und 72 mg (64 % d. Th.) von Diastereomer 2 (Racemat), welches später eluiert, isoliert.

### Diastereomer 1, Racemat:

LC-MS (Methode 3): Rₜ = 1.75 min; MS (ESIpos): m/z = 518 [M+H]⁺
¹H-NMR (400 MHz, DMSO-d6) δ [ppm]: -0.150 (2.18), -0.009 (16.00), 0.007 (13.45), 0.145 (2.18), 1.652 (1.09), 1.682 (1.09), 1.713 (1.09), 1.747 (1.09), 2.322 (4.36), 2.327 (6.18), 2.331 (4.36), 2.365 (4.00), 2.412 (1.09), 2.444 (1.82), 2.450 (1.82), 2.523 (14.18), 2.558 (3.27), 2.560 (1.82), 2.564 (2.18), 2.566 (1.82), 2.571 (1.45), 2.576 (1.82), 2.581 (1.45), 2.585 (1.45), 2.592 (1.45), 2.596 (1.45), 2.664 (4.73), 2.669 (5.82), 2.673 (4.00), 2.689 (3.27), 2.709 (4.36), 2.737 (1.82), 2.776 (2.18), 2.906 (2.18), 2.947 (1.45), 2.989 (1.45), 3.024 (1.45), 3.180 (0.73), 3.189 (0.73), 3.201 (1.09), 3.218 (1.82), 3.223 (1.45), 3.234 (1.82), 3.246 (2.55), 3.251 (2.18), 3.358 (0.73), 3.520 (0.73), 3.565 (1.09), 3.639 (0.73), 3.699 (0.73), 3.730 (0.73), 3.788 (1.09), 3.802 (0.73), 3.823 (0.73), 4.139 (1.09), 4.744 (1.45), 4.757 (1.45), 4.764 (1.09), 4.772 (1.45), 5.070 (8.00), 5.249 (0.73), 5.256 (0.73), 5.361 (0.73), 5.394 (1.09), 5.450 (0.73), 5.470 (0.73), 5.753 (13.09), 7.623 (1.82), 7.637 (4.00), 7.650 (2.18), 8.578 (3.64), 8.589 (3.27).

### Beispiel 260

### (5RS,7RS)-5-{[(3R,4S)-3,4-Difluorpyrrolidin-1-yl]carbonyl}-2-{[3-fluor-2-(trifluormethyl)pyridin-4-yl]methyl}-7-(trifluormethyl)-5,6,7,8-tetrahydro[1,2,4]triazolo[4,3-a]pyridin-3(2H)-on (Diastereomer 2, Racemat)

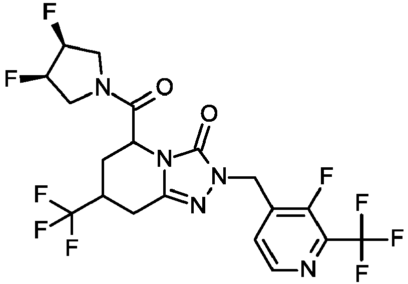

(5RS,7RS)-2-{[3-Fluor-2-(trifluormethyl)pyridin-4-yl]methyl}-3-oxo-7-(trifluormethyl)-2,3,5,6,7,8-hexahydro[1,2,4]triazolo[4,3-a]pyridin-5-carbonsäure (Diastereomerengemisch; 4 Isomere) (93.0 mg, 217 µmol) wurde in THF (2.0 ml) bei Raumtemperatur vorgelegt. Anschließend wurden HBTU (107 mg, 282 µmol) und N,N-Diisopropylethylamin (190 µl, 1.1 mmol) zugegeben. Nach 15 min Rühren wurde (3R,4S)-3,4-Difluorpyrrolidinhydrochlorid (37.4 mg, 261 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Nach der Trennung wurden 8.5 mg (8 % d. Th.) von Diastereomer 1 (Racemat), welches zuerst eluiert, und 72 mg (64 % d. Th.) von Diastereomer 2 (Racemat), welches später eluiert, isoliert.

### Diastereomer 2, Racemat:

LC-MS (Methode 3): Rₜ = 1.81 min; MS (ESIpos): m/z = 518 [M+H]⁺
¹H-NMR (400 MHz, DMSO-d6) δ [ppm]: -0.149 (0.52), -0.008 (6.19), 0.008 (4.22), 0.146 (0.52), 2.137 (1.22), 2.154 (1.44), 2.173 (2.56), 2.189 (2.30), 2.204 (2.67), 2.220 (5.11), 2.230 (4.56), 2.238 (4.56), 2.294 (3.59), 2.328 (3.52), 2.366 (1.59), 2.519 (9.30), 2.524 (8.48), 2.665 (1.37), 2.670 (1.78), 2.675 (1.41), 2.700 (2.89), 2.711 (3.30), 2.729 (3.85), 2.740 (5.56), 2.751 (2.78), 2.769 (4.56), 2.780 (3.37), 2.898 (2.70), 2.986 (6.33), 2.997 (4.70), 3.025 (4.78), 3.036 (3.78), 3.168 (16.00), 3.493 (2.07), 3.502 (3.11), 3.526 (2.63), 3.536 (4.07), 3.545 (3.41), 3.556 (2.52), 3.566 (1.93), 3.588 (2.93), 3.599 (2.07), 3.650 (2.04), 3.665 (1.93), 3.684 (1.70), 3.697 (1.93), 3.707 (3.44), 3.720 (3.26), 3.740 (2.70), 3.754 (2.30), 3.767 (1.56), 3.780 (1.59), 3.801 (1.22), 3.814 (1.22), 3.843 (1.81), 3.871 (2.85), 3.884 (2.52), 3.891 (2.78), 4.046 (1.89), 4.060 (2.70), 4.074 (2.41), 4.091 (3.07), 4.108 (2.52), 4.122 (1.67), 4.136 (1.63), 4.150 (1.26), 4.166 (1.44), 4.180 (1.37), 4.195 (2.37), 4.210 (1.52), 4.224 (1.22), 4.238 (1.19), 5.023 (6.78), 5.034 (8.93), 5.045 (5.93), 5.087 (11.19), 5.110 (8.48), 5.120 (9.63), 5.151 (1.78), 5.161 (3.00), 5.260 (1.96), 5.270 (2.15), 5.285 (2.07), 5.296 (2.07), 5.329 (1.67), 5.337 (1.74), 5.350 (1.81), 5.384 (2.52), 5.407 (2.04), 5.417 (2.19), 5.430 (2.11), 5.440 (1.37), 5.457 (1.74), 5.471 (1.67), 5.479 (1.70), 5.491 (1.85), 7.554 (3.15), 7.566 (6.74), 7.576 (7.70), 7.588 (3.63), 8.167 (0.89), 8.569 (11.81), 8.581 (11.26).

### Beispiel 261

### (5RS,7RS)-5-{[(3R,4S)-3,4-Difluorpyrrolidin-1-yl]carbonyl}-2-{[3-fluor-2-(trifluormethyl)pyridin-4-yl]methyl}-7-(trifluormethyl)-5,6,7,8-tetrahydro[1,2,4]triazolo[4,3-a]pyridin-3(2H)-on (Enantiomer 1)

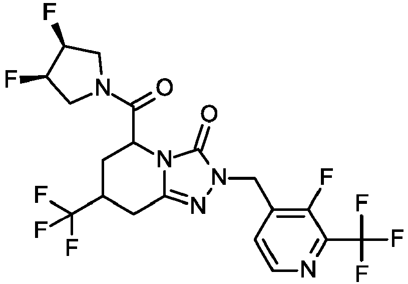

### (5RS,7RS)-5-{[(3R,4S)-3,4-Difluorpyrrolidin-1-yl]carbonyl}-2-{[3-fluor-2-(trifluormethyl)pyridin-4-yl]methyl}-7-(trifluormethyl)-5,6,7,8-tetrahydro[1,2,4]triazolo[4,3-a]pyridin-3(2H)-on

(Diastereomer 2, Racemat) wurde durch chirale präparative HPLC getrennt [Probenvorbereitung: 72.2 mg gelöst in 3 ml Acetonitril und 2 ml Ethanol (warm); Injektionsvolumen: 0.25 ml; Säule: Daicel Chiralpak^{®} IB 5 µm, 250 x 20 mm; Laufmittel: n-Heptan/Ethanol 50:50; Fluss: 15 ml/min; Temperatur 25°C; UV-Detektion: 210 nm]. Nach der Trennung wurden 31.3 mg von Enantiomer 1, welches zuerst eluiert, und 31.2 mg von Enantiomer 2, welches später eluiert, isoliert.

### Enantiomer 1:

Analytische chirale HPLC: Rₜ = 2.08 min, e.e. = 99% [Säule: Daicel Chiraltek^{®} IB, 3 µm, 50 x 4.6 mm; Laufmittel: i-Hexan/Ethanol 50:50; Fluss: 1 ml/min; UV-Detektion: 220 nm].
LC-MS (Methode 4): Rₜ = 0.90 min; MS (ESIpos): m/z = 518 [M+H]⁺
¹H-NMR (400 MHz, DMSO-d6) δ [ppm]: -0.149 (1.73), -0.008 (16.00), 0.008 (12.80), 0.146 (1.73), 0.852 (0.61), 1.234 (1.30), 2.138 (0.78), 2.153 (0.78), 2.173 (1.56), 2.185 (1.38), 2.204 (1.47), 2.219 (3.03), 2.229 (2.94), 2.238 (2.94), 2.291 (2.42), 2.327 (3.29), 2.366 (2.68), 2.523 (6.66), 2.665 (1.73), 2.670 (2.25), 2.674 (1.64), 2.699 (1.82), 2.710 (3.89), 2.729 (2.42), 2.740 (3.63), 2.752 (1.64), 2.769 (3.03), 2.781 (2.25), 2.898 (1.64), 2.986 (3.98), 2.997 (2.85), 3.025 (3.03), 3.037 (2.51), 3.502 (1.56), 3.535 (2.25), 3.545 (1.99), 3.587 (1.73), 3.650 (1.30), 3.663 (1.12), 3.682 (0.86), 3.707 (2.08), 3.719 (1.99), 3.740 (1.64), 3.753 (1.38), 3.780 (0.86), 3.814 (0.69), 3.872 (1.73), 3.891 (1.64), 4.046 (0.95), 4.059 (1.30), 4.090 (1.30), 4.108 (1.21), 4.137 (0.86), 4.195 (1.47), 4.210 (0.86), 4.224 (0.78), 4.239 (0.69), 5.022 (4.15), 5.034 (5.62), 5.045 (3.72), 5.087 (7.18), 5.110 (5.36), 5.120 (6.49), 5.152 (1.04), 5.161 (1.99), 5.270 (1.47), 5.297 (1.30), 5.329 (0.95), 5.351 (1.04), 5.383 (1.47), 5.417 (1.30), 5.429 (1.30), 5.440 (0.78), 5.470 (1.04), 7.554 (1.73), 7.566 (4.06), 7.576 (4.93), 7.589 (2.25), 8.569 (7.26), 8.581 (7.26).

### Beispiel 262

### (5RS,7RS)-5-{[(3R,4S)-3,4-Difluorpyrrolidin-1-yl]carbonyl}-2-{[3-fluor-2-(trifluormethyl)pyridin-4-yl]methyl}-7-(trifluormethyl)-5,6,7,8-tetrahydro[1,2,4]triazolo[4,3-a]pyridin-3(2H)-on (Enantiomer 2)

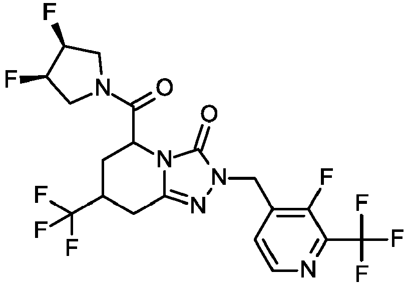

### (5RS,7RS)-5-{[(3R,4S)-3,4-Difluorpyrrolidin-1-yl]carbonyl}-2-{[3-fluor-2-(trifluormethyl)pyridin-4-yl]methyl}-7-(trifluormethyl)-5,6,7,8-tetrahydro[1,2,4]triazolo[4,3-a]pyridin-3(2H)-on

(Diastereomer 2, Racemat) wurde durch chirale präparative HPLC getrennt [Probenvorbereitung: 72.2 mg gelöst in 3 ml Acetonitril und 2 ml Ethanol (warm); Injektionsvolumen: 0.25 ml; Säule: Daicel Chiralpak^{®} IB 5 µm, 250 x 20 mm; Laufmittel: n-Heptan/Ethanol 50:50; Fluss: 15 ml/min; Temperatur 25°C; UV-Detektion: 210 nm]. Nach der Trennung, wurden 31.3 mg von Enantiomer 1, welches zuerst eluiert, und 31.2 mg von Enantiomer 2, welches später eluiert, isoliert.

### Enantiomer 2:

Analytische chirale HPLC: Rₜ = 4.11 min, e.e. = 99% [Säule: Daicel Chiraltek^{®} IB 3 µm, 50 x 4.6 mm; Laufmittel: i-Hexan/Ethanol 50:50; Fluss: 1 ml/min; UV-Detektion: 220 nm].
LC-MS (Methode 4): Rₜ = 0.90 min; MS (ESIpos): m/z = 518 [M+H]⁺
¹H-NMR (400 MHz, DMSO-d6) δ [ppm]: -0.149 (1.71), 0.008 (15.86), 0.147 (1.71), 0.853 (0.96), 1.234 (2.39), 2.138 (1.78), 2.153 (1.78), 2.173 (3.49), 2.189 (3.21), 2.204 (3.42), 2.221 (6.97), 2.229 (6.63), 2.239 (6.50), 2.293 (5.40), 2.327 (5.40), 2.366 (2.80), 2.519 (10.60), 2.523 (8.27), 2.670 (2.53), 2.700 (3.56), 2.711 (4.79), 2.730 (5.06), 2.741 (7.52), 2.751 (3.76), 2.769 (6.02), 2.781 (4.51), 2.915 (3.42), 2.986 (8.62), 2.997 (6.22), 3.026 (6.56), 3.038 (5.20), 3.502 (3.28), 3.526 (2.67), 3.536 (4.85), 3.546 (4.03), 3.556 (3.01), 3.590 (3.69), 3.650 (2.53), 3.664 (2.46), 3.684 (1.98), 3.707 (4.51), 3.720 (4.44), 3.740 (3.35), 3.753 (3.01), 3.767 (1.98), 3.780 (1.85), 3.802 (1.37), 3.814 (1.44), 3.870 (3.69), 3.892 (3.56), 4.048 (1.85), 4.061 (2.67), 4.075 (1.71), 4.091 (3.01), 4.109 (2.53), 4.122 (1.71), 4.137 (1.78), 4.151 (1.50), 4.166 (1.71), 4.180 (1.64), 4.195 (3.08), 4.210 (1.98), 4.223 (1.50), 4.239 (1.57), 5.023 (9.23), 5.035 (12.51), 5.045 (8.41), 5.087 (15.52), 5.110 (11.56), 5.120 (13.81), 5.152 (2.39), 5.161 (4.31), 5.261 (2.60), 5.271 (2.94), 5.298 (2.87), 5.329 (2.12), 5.350 (2.39), 5.384 (3.35), 5.408 (2.67), 5.418 (2.94), 5.430 (2.80), 5.441 (1.71), 5.457 (2.19), 5.480 (2.26), 5.492 (2.32), 7.555 (3.83), 7.566 (9.37), 7.577 (10.87), 7.589 (5.20), 8.569 (16.00), 8.581 (15.93).

### Beispiel 263

### (5RS,7RS)-2-[(5-Chlor-3-fluorpyridin-2-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on

(Diastereomerengemisch 1; 2 Isomere)

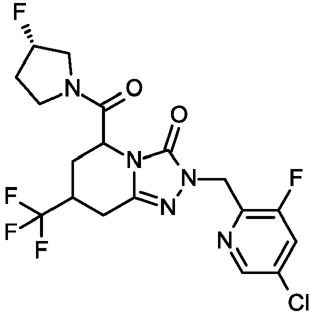

(5RS,7RS)-2-[(5-Chlor-3-fluorpyridin-2-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere) (55.0 mg, 139 µmol) wurde in THF (2.0 ml) bei Raumtemperatur vorgelegt. Anschließend wurden HBTU (68.7 mg, 181 µmol) und N,N-Diisopropylethylamin (120 µl, 700 µmol) zugegeben. Nach 5 min Rühren wurde (3S)-3-Fluorpyrrolidinhydrochlorid (21.0 mg, 167 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Nach der Trennung wurden 10.5 mg (16 % d. Th.) von Diastereomerengemisch 1 (2 Isomere), welches zuerst eluiert, und 37 mg (51 % d. Th.) von Diastereomerengemisch 2 (2 Isomere), welches später eluiert, isoliert.

### Diastereomerengemisch 1, 2 Isomere:

LC-MS (Methode 1): Rₜ = 1.06 min; MS (ESIpos): m/z = 466 [M+H]⁺
¹H-NMR (400 MHz, DMSO-d6) δ [ppm]: -0.149 (1.70), -0.033 (0.53), -0.022 (0.95), -0.008 (14.62), 0.008 (16.00), 0.019 (0.95), 0.024 (0.53), 0.026 (0.42), 0.146 (1.70), 1.653 (0.42), 1.682 (0.53), 1.713 (0.53), 1.739 (0.53), 2.097 (0.32), 2.109 (0.32), 2.131 (0.32), 2.212 (0.32), 2.231 (0.32), 2.257 (0.32), 2.323 (1.70), 2.327 (2.33), 2.332 (1.80), 2.366 (1.91), 2.460 (0.32), 2.523 (7.31), 2.559 (2.44), 2.574 (1.27), 2.581 (0.85), 2.586 (0.74), 2.598 (0.74), 2.604 (0.53), 2.637 (0.95), 2.665 (2.44), 2.670 (3.60), 2.674 (2.75), 2.710 (3.18), 2.760 (0.21), 2.862 (1.06), 2.898 (0.74), 2.987 (0.42), 3.011 (0.42), 3.359 (1.59), 3.435 (0.74), 3.468 (0.53), 3.499 (0.64), 3.534 (0.42), 3.554 (0.53), 3.588 (0.53), 3.619 (0.64), 3.636 (0.64), 3.657 (0.64), 3.674 (0.74), 3.703 (0.53), 3.721 (0.42), 3.743 (0.32), 3.787 (0.32), 3.795 (0.32), 3.826 (0.42), 3.859 (0.53), 3.887 (0.42), 3.931 (0.64), 3.957 (0.74), 3.986 (0.42), 4.677 (0.32), 4.690 (0.42), 4.704 (0.32), 4.716 (0.42), 4.731 (0.42), 4.742 (0.42), 4.755 (0.42), 4.767 (0.42), 4.781 (0.32), 4.798 (0.42), 4.812 (0.32), 4.825 (0.32), 4.971 (4.77), 5.284 (0.32), 5.354 (0.32), 5.396 (0.53), 5.417 (0.32), 5.481 (0.32), 5.524 (0.32), 8.104 (1.17), 8.109 (1.27), 8.129 (1.17), 8.133 (1.27), 8.489 (1.70).

### Beispiel 264

### (5RS,7RS)-2-[(5-Chlor-3-fluorpyridin-2-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on (Diastereomerengemisch 2; 2 Isomere)

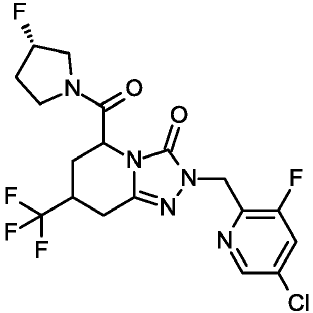

(5RS,7RS)-2-[(5-Chlor-3-fluorpyridin-2-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere) (55.0 mg, 139 µmol) wurde in THF (2.0 ml) bei Raumtemperatur vorgelegt. Anschließend wurden HBTU (68.7 mg, 181 µmol) und N,N-Diisopropylethylamin (120 µl, 700 µmol) zugegeben. Nach 5 min Rühren wurde (3S)-3-Fluorpyrrolidinhydrochlorid (21.0 mg, 167 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Nach der Trennung wurden 10.5 mg (16 % d. Th.) von Diastereomerengemisch 1 (2 Isomere), welches zuerst eluiert, und 37 mg (51 % d. Th.) von Diastereomerengemisch 2 (2 Isomere), welches später eluiert, isoliert.

### Diastereomerengemisch 2, 2 Isomere:

LC-MS (Methode 1): Rₜ = 1.10 min; MS (ESIpos): m/z = 466 [M+H]⁺
¹H-NMR (400 MHz, DMSO-d6) δ [ppm]: -1.950 (1.23), -1.643 (1.23), -1.565 (0.92), -1.154 (1.23), -0.149 (5.54), -0.039 (1.85), 0.146 (5.23), 1.883 (1.23), 1.954 (1.23), 1.989 (1.54), 1.999 (1.54), 2.039 (1.23), 2.120 (4.31), 2.149 (7.38), 2.186 (6.77), 2.199 (6.46), 2.216 (6.15), 2.268 (5.54), 2.297 (3.69), 2.327 (10.46), 2.367 (6.15), 2.413 (1.54), 2.610 (2.15), 2.635 (3.38), 2.670 (15.38), 2.710 (10.15), 2.801 (1.23), 2.815 (1.23), 2.883 (1.54), 2.948 (9.85), 2.982 (7.08), 3.025 (1.23), 3.084 (1.23), 3.119 (1.23), 3.204 (1.54), 3.361 (5.85), 3.388 (3.38), 3.426 (2.46), 3.474 (1.54), 3.499 (1.85), 3.510 (2.15), 3.524 (1.85), 3.534 (2.15), 3.567 (4.00), 3.597 (4.00), 3.605 (3.08), 3.628 (4.92), 3.668 (4.62), 3.691 (4.00), 3.726 (1.85), 3.762 (1.23), 3.790 (2.46), 3.798 (1.85), 3.820 (2.46), 3.843 (3.08), 3.865 (2.15), 3.887 (2.15), 3.919 (1.85), 3.976 (2.77), 3.996 (3.08), 4.019 (2.77), 4.049 (1.85), 4.076 (1.54), 4.904 (2.46), 4.931 (5.85), 4.970 (16.00), 4.995 (3.69), 5.017 (9.54), 5.056 (6.15), 5.265 (3.08), 5.303 (1.23), 5.355 (2.15), 5.398 (3.69), 5.490 (2.46), 5.522 (1.54), 7.947 (0.92), 8.103 (10.15), 8.107 (10.77), 8.127 (9.85), 8.131 (10.77), 8.201 (1.23), 8.214 (1.54), 8.483 (15.08), 8.698 (0.92), 8.965 (1.23), 10.344 (1.23), 11.406 (0.92), 12.388 (1.23), 12.839 (0.92), 12.989 (1.23), 13.493 (1.23), 15.243 (0.92), 16.225 (0.92).

### Beispiel 265

### (5RS,7RS)-2-[(5-Chlor-3-fluorpyridin-2-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on (Isomer 2)

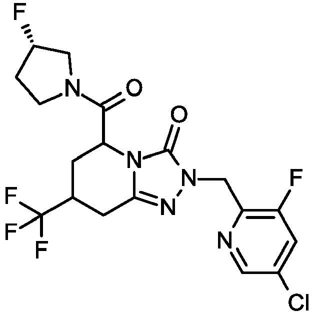

### (5RS,7RS)-2-[(5-Chlor-3-fluorpyridin-2-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on

(Diastereomerengemisch 2, 2 Isomere) wurde durch chirale präparative HPLC getrennt [Probenvorbereitung: 37 mg gelöst in 2 ml n-Heptan und 2 ml Ethanol; Injektionsvolumen: 0.9 ml; Säule: Daicel Chiralpak^{®} IC 5 µm, 250 x 20 mm; Laufmittel: n-Heptan/Ethanol 50:50; Fluss: 15 ml/min; Temperatur 50°C; UV-Detektion: 220 nm]. Nach der Trennung wurden 14.3 mg von Isomer 1, welches zuerst eluiert, und 15.1 mg von Isomer 2, welches später eluiert, isoliert.

### Isomer 2:

Analytische chirale HPLC: Rₜ = 6.52 min, d.e. = 99% [Säule: Daicel Chiralpak^{®} IC 5 µm, 250 x 4.6 mm; Laufmittel: i-Hexan/Ethanol 50:50; Fluss: 1 ml/min; UV-Detektion: 220 nm].
LC-MS (Methode 3): Rₜ = 1.49 min; MS (ESIpos): m/z = 466 [M+H]⁺
¹H-NMR (400 MHz, DMSO-d6) δ [ppm]: -0.149 (1.56), -0.008 (16.00), 0.008 (15.94), 0.146 (1.50), 1.235 (0.88), 1.971 (0.50), 2.115 (1.50), 2.151 (2.88), 2.185 (2.25), 2.200 (2.12), 2.216 (1.94), 2.231 (1.94), 2.267 (3.25), 2.296 (2.00), 2.327 (2.37), 2.366 (0.69), 2.669 (3.75), 2.678 (3.44), 2.692 (1.62), 2.710 (2.25), 2.948 (4.63), 2.982 (3.75), 3.362 (1.75), 3.390 (1.62), 3.426 (1.25), 3.497 (0.81), 3.523 (1.19), 3.648 (1.81), 3.667 (2.63), 3.692 (2.94), 3.721 (1.25), 3.784 (0.69), 3.818 (1.75), 3.845 (2.25), 3.868 (1.25), 3.916 (1.37), 3.952 (0.75), 3.976 (1.19), 4.009 (0.69), 4.906 (1.75), 4.922 (3.25), 4.966 (8.44), 5.021 (4.12), 5.060 (1.88), 5.266 (1.56), 5.394 (2.37), 5.525 (1.12), 8.102 (4.75), 8.107 (4.81), 8.126 (4.81), 8.131 (4.94), 8.483 (6.12).

### Beispiel 266

### (5RS,7RS)-2-[(5-Chlor-3-fluorpyridin-2-yl)methyl]-5-{[(3R,4S)-3,4-difluorpyrrolidin-1-yl]carbonyl}-7-(trifluormethyl)-5,6,7,8-tetrahydro[1,2,4]triazolo[4,3-a]pyridin-3(2H)-on (Diastereomer 2, Racemat)

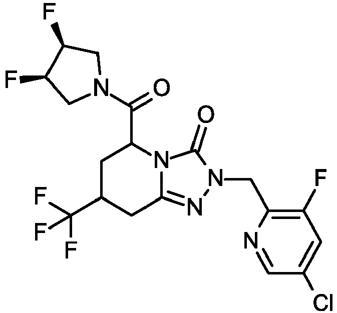

(5RS,7RS)-2-[(5-Chlor-3-fluorpyridin-2-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere) (55.0 mg, 139 µmol) wurde in THF (2.0 ml) bei Raumtemperatur vorgelegt. Anschließend wurden HBTU (68.7 mg, 181 µmol) und N,N-Diisopropylethylamin (120 µl, 700 µmol) zugegeben. Nach 5 min Rühren wurde (3R,4S)-3,4-Difluorpyrrolidinhydrochlorid (24.0 mg, 167 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Nach der Trennung wurden 23.9 mg (35 % d. Th.) von Diastereomer 2 (Racemat), welches später eluiert, isoliert.
LC-MS (Methode 1): Rₜ = 1.13 min; MS (ESIpos): m/z = 484 [M+H]⁺
¹H-NMR (400 MHz, DMSO-d6) δ [ppm]: -0.150 (1.73), -0.031 (0.56), -0.008 (16.00), 0.008 (13.67), 0.146 (1.63), 2.073 (0.42), 2.145 (0.47), 2.161 (0.47), 2.193 (1.17), 2.256 (0.84), 2.295 (0.56), 2.322 (1.45), 2.327 (1.96), 2.332 (1.45), 2.366 (0.56), 2.439 (0.42), 2.648 (0.42), 2.665 (1.77), 2.669 (2.33), 2.684 (1.21), 2.695 (0.65), 2.710 (0.84), 2.728 (0.56), 2.942 (1.77), 2.976 (1.17), 2.988 (0.75), 3.491 (0.37), 3.527 (0.61), 3.535 (0.56), 3.578 (0.61), 3.640 (0.37), 3.653 (0.33), 3.698 (0.56), 3.709 (0.51), 3.729 (0.42), 3.746 (0.37), 3.851 (0.42), 3.870 (0.51), 3.888 (0.51), 3.897 (0.47), 4.058 (0.37), 4.088 (0.47), 4.102 (0.33), 4.201 (0.47), 4.933 (0.89), 4.971 (2.47), 4.993 (1.77), 5.008 (1.21), 5.024 (1.40), 5.028 (1.35), 5.047 (0.47), 5.064 (0.56), 5.267 (0.42), 5.288 (0.47), 5.342 (0.37), 5.382 (0.56), 5.394 (0.47), 5.403 (0.42), 5.426 (0.37), 5.454 (0.33), 5.486 (0.37), 8.103 (1.40), 8.107 (1.49), 8.126 (1.49), 8.131 (1.45), 8.477 (2.19).

### Beispiel 267

### (5RS,7RS)-2-[(5-Chlor-3-fluorpyridin-2-yl)methyl]-5-{[(3R,4S)-3,4-difluorpyrrolidin-1-yl]carbonyl}-7-(trifluormethyl)-5,6,7,8-tetrahydro[1,2,4]triazolo[4,3-a]pyridin-3(2H)-on (Enantiomer 1)

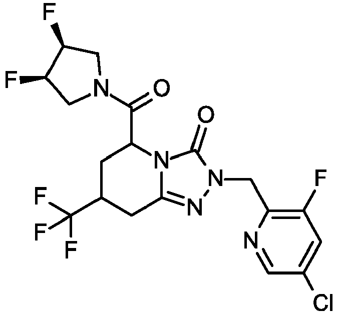

### (5RS,7RS)-2-[(5-Chlor-3-fluorpyridin-2-yl)methyl]-5-{[(3R,4S)-3,4-difluorpyrrolidin-1-yl]carbonyl}-7-(trifluormethyl)-5,6,7,8-tetrahydro[1,2,4]triazolo[4,3-a]pyridin-3(2H)-on

(Diastereomer 2, Racemat) wurde durch chirale präparative HPLC getrennt [Probenvorbereitung: 23.9 mg gelöst in 1.5 ml n-Heptan und 2 ml Ethanol; Injektionsvolumen: 1.2 ml; Säule: Daicel Chiralpak^{®} IE 5 µm, 250 x 20 mm; Laufmittel: n-Heptan/Ethanol 50:50; Fluss: 15 ml/min; Temperatur 40°C; UV-Detektion: 220 nm]. Nach der Trennung, wurden 8.6 mg von Enantiomer 1, welches zuerst eluiert, und 9.4 mg von Enantiomer 2, welches später eluiert, isoliert.

### Enantiomer 1:

Analytische chirale HPLC: Rₜ = 1.86 min, e.e. = 99% [Säule: Daicel Chiralpak^{®} IE, 3 µm, 50 x 4.6 mm; Laufmittel: n-Heptan/Ethanol 50:50; Fluss: 1 ml/min; UV-Detektion: 220 nm].
LC-MS (Methode 3): Rₜ = 1.55 min; MS (ESIpos): m/z = 484 [M+H]⁺
¹H-NMR (400 MHz, DMSO-d6) δ [ppm]: -0.149 (1.69), -0.008 (16.00), 0.008 (14.89), 0.146 (1.85), 2.128 (0.41), 2.144 (0.82), 2.162 (0.82), 2.193 (2.14), 2.259 (1.40), 2.294 (0.99), 2.323 (1.48), 2.327 (2.18), 2.332 (1.60), 2.366 (1.07), 2.651 (0.70), 2.674 (2.71), 2.683 (2.14), 2.696 (1.07), 2.711 (1.69), 2.941 (3.08), 2.976 (2.06), 2.988 (1.32), 3.488 (0.58), 3.530 (0.95), 3.577 (0.95), 3.641 (0.70), 3.653 (0.58), 3.673 (0.58), 3.697 (0.99), 3.712 (0.99), 3.732 (0.78), 3.746 (0.66), 3.759 (0.45), 3.773 (0.45), 3.837 (0.58), 3.852 (0.78), 3.867 (0.95), 3.911 (0.70), 3.916 (0.70), 4.044 (0.49), 4.056 (0.70), 4.072 (0.53), 4.087 (0.82), 4.104 (0.58), 4.117 (0.45), 4.133 (0.45), 4.170 (0.45), 4.201 (0.78), 4.216 (0.49), 4.231 (0.41), 4.933 (1.69), 4.972 (4.36), 4.977 (3.78), 4.993 (3.13), 5.008 (2.06), 5.024 (2.34), 5.028 (2.39), 5.052 (0.82), 5.064 (1.11), 5.067 (1.03), 5.263 (0.74), 5.292 (0.70), 5.384 (0.95), 5.394 (0.86), 5.414 (0.78), 5.422 (0.66), 5.454 (0.49), 5.474 (0.70), 5.484 (0.66), 8.103 (2.67), 8.108 (2.80), 8.127 (2.76), 8.132 (2.76), 8.477 (4.40), 8.481 (4.40).

### Beispiel 268

### (5RS,7RS)-2-[(5-Chlorpyridin-3-yl)methyl]-5-{[(3R,4S)-3,4-difluorpyrrolidin-1-yl]carbonyl}-7-(trifluormethyl)-5,6,7,8-tetrahydro[1,2,4]triazolo[4,3-a]pyridin-3(2H)-on (Diastereomer 1, Racemat)

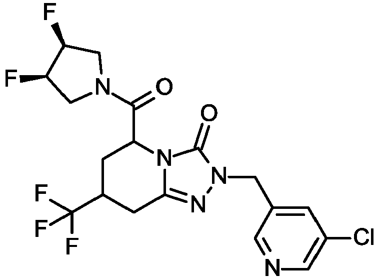

(5RS,7RS)-2-[(5-Chlorpyridin-3-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere) (70.0 mg, 75 % Reinheit, 139 µmol) wurde in THF (2.0 ml) bei Raumtemperatur vorgelegt. Anschließend wurden HBTU (68.7 mg, 181 µmol) und N,N-Diisopropylethylamin (120 µl, 700 µmol) zugegeben. Nach 5 min Rühren wurde (3R,4S)-3,4-Difluorpyrrolidinhydrochlorid (24.0 mg, 167 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Nach der Trennung wurden 12.6 mg (19 % d. Th.) von Diastereomer 1 (Racemat), welches zuerst eluiert, und 9 mg (14 % d. Th.) von Diastereomer 2 (Racemat), welches später eluiert, isoliert.

### Diastereomer 1, Racemat:

LC-MS (Methode 3): Rₜ = 1.41 min; MS (ESIpos): m/z = 466 [M+H]⁺
¹H-NMR (400 MHz, DMSO-d6) δ [ppm]: -0.149 (1.45), -0.020 (0.85), -0.017 (1.09), -0.008 (12.36), 0.008 (16.00), 0.146 (1.58), 1.642 (1.27), 1.670 (1.39), 1.703 (1.27), 1.735 (1.03), 2.323 (1.27), 2.327 (1.76), 2.332 (1.39), 2.366 (1.21), 2.523 (5.52), 2.558 (2.48), 2.564 (2.12), 2.572 (1.76), 2.665 (1.58), 2.669 (2.12), 2.674 (1.64), 2.697 (1.45), 2.710 (1.52), 2.730 (2.00), 2.737 (2.24), 2.769 (2.61), 2.901 (2.18), 2.940 (1.58), 2.996 (1.03), 3.016 (1.09), 3.509 (0.85), 3.521 (0.97), 3.555 (1.15), 3.564 (1.21), 3.572 (0.85), 3.608 (0.85), 3.642 (0.85), 3.655 (1.03), 3.700 (1.03), 3.713 (1.03), 3.735 (0.97), 3.748 (1.21), 3.783 (0.91), 3.797 (0.97), 3.809 (0.97), 3.830 (1.09), 3.844 (0.97), 3.864 (1.03), 3.895 (0.91), 3.910 (0.85), 3.951 (0.79), 4.114 (0.91), 4.130 (1.45), 4.144 (0.97), 4.160 (1.45), 4.174 (1.03), 4.723 (1.21), 4.732 (1.39), 4.737 (1.76), 4.750 (1.70), 4.765 (1.45), 4.773 (1.03), 4.923 (7.94), 4.952 (1.45), 5.248 (0.91), 5.261 (0.85), 5.276 (0.85), 5.287 (0.73), 5.322 (0.73), 5.342 (0.91), 5.354 (0.85), 5.364 (0.91), 5.371 (0.97), 5.383 (0.97), 5.392 (0.97), 5.406 (0.91), 5.415 (0.79), 5.465 (0.79), 5.472 (0.85), 7.796 (3.33), 7.801 (3.64), 8.425 (0.73), 8.446 (2.61), 8.453 (4.12), 8.458 (3.15), 8.576 (3.88), 8.581 (3.45), 9.265 (3.45).

### Beispiel 269

### (5RS,7RS)-2-[(5-Chlorpyridin-3-yl)methyl]-5-{[(3R,4S)-3,4-difluorpyrrolidin-1-yl]carbonyl}-7-(trifluormethyl)-5,6,7,8-tetrahydro[1,2,4]triazolo[4,3-a]pyridin-3(2H)-on (Diastereomer 2, Racemat)

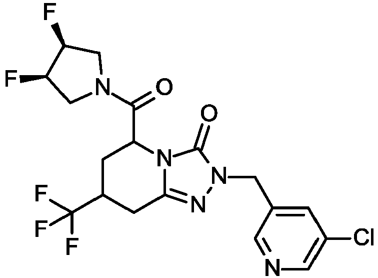

(5RS,7RS)-2-[(5-Chlorpyridin-3-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere) (70.0 mg, 75 % Reinheit, 139 µmol) wurde in THF (2.0 ml) bei Raumtemperatur vorgelegt. Anschließend wurden HBTU (68.7 mg, 181 µmol) und N,N-Diisopropylethylamin (120 µl, 700 µmol) zugegeben. Nach 5 min Rühren wurde (3R,4S)-3,4-Difluorpyrrolidinhydrochlorid (24.0 mg, 167 µmol) zugegeben und das Reaktionsgemisch über Nacht bei Raumtemperatur gerührt. Das Reaktionsgemisch wurde mit Wasser und Ethylacetat versetzt und die organische Phase wurde abgetrennt. Die organische Phase wurde mit 10% wässriger Citronensäure-Lösung und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Nach der Trennung, wurden 12.6 mg (19 % d. Th.) von Diastereomer 1 (Racemat), welches zuerst eluiert, und 9 mg (14 % d. Th.) von Diastereomer 2 (Racemat), welches später eluiert, isoliert.

### Diastereomer 2, Racemat:

LC-MS (Methode 3): Rₜ = 1.46 min; MS (ESIpos): m/z = 466 [M+H]⁺
¹H-NMR (400 MHz, DMSO-d6) δ [ppm]: -0.149 (1.95), -0.008 (16.00), 0.008 (14.42), 0.146 (1.86), 2.166 (1.21), 2.182 (1.16), 2.197 (1.44), 2.208 (2.27), 2.218 (2.69), 2.228 (2.13), 2.241 (1.11), 2.277 (1.95), 2.312 (1.16), 2.323 (1.90), 2.327 (2.64), 2.332 (1.90), 2.366 (1.81), 2.523 (5.43), 2.665 (2.04), 2.670 (2.64), 2.674 (1.95), 2.693 (1.44), 2.703 (1.21), 2.710 (2.09), 2.723 (1.95), 2.733 (2.97), 2.743 (1.44), 2.762 (2.37), 2.772 (1.81), 2.889 (1.30), 2.904 (1.25), 2.977 (3.11), 2.988 (2.27), 3.016 (2.27), 3.027 (1.81), 3.500 (1.02), 3.533 (1.58), 3.542 (1.44), 3.552 (1.25), 3.563 (0.74), 3.578 (1.07), 3.586 (1.58), 3.596 (0.97), 3.646 (0.97), 3.660 (0.79), 3.678 (0.79), 3.695 (0.79), 3.703 (1.44), 3.718 (1.53), 3.738 (1.07), 3.752 (1.07), 3.766 (0.79), 3.780 (0.74), 3.800 (0.65), 3.847 (0.83), 3.866 (1.34), 3.895 (1.34), 3.930 (0.70), 4.043 (0.74), 4.057 (0.93), 4.071 (0.65), 4.087 (1.11), 4.104 (0.97), 4.119 (0.70), 4.134 (0.70), 4.157 (0.70), 4.173 (0.74), 4.201 (1.21), 4.230 (0.70), 4.911 (0.74), 4.952 (13.22), 5.007 (3.06), 5.020 (4.31), 5.273 (1.02), 5.296 (1.07), 5.328 (0.65), 5.351 (0.74), 5.386 (1.30), 5.397 (1.16), 5.407 (1.02), 5.418 (1.07), 5.427 (1.07), 5.439 (0.65), 5.458 (0.83), 5.480 (0.83), 5.490 (0.93), 7.750 (1.81), 7.763 (4.50), 8.425 (7.14), 8.429 (4.73), 8.570 (4.87).

### Beispiel 270

### (5RS,8RS)-5-{[(3R,4S)-3,4-Difluorpyrrolidin-1-yl]carbonyl}-8-(trifluormethyl)-2-{[6-(trifluormethyl)pyridin-3-yl]methyl}-5,6,7,8-tetrahydro[1,2,4]triazolo[4,3-a]pyridin-3(2H)-on (Diastereomerengemisch; 4 Isomere)

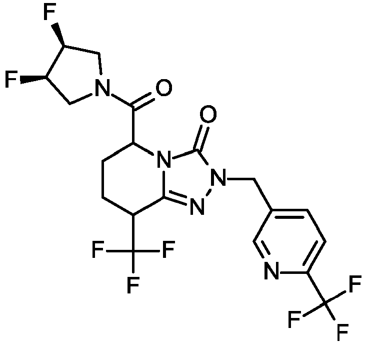

Unter Argon: 5-{[(3RS,4RS)-3,4-Difluorpyrrolidin-1-yl]carbonyl}-8-(trifluormethyl)-2-{[6-(trifluormethyl)pyridin-3-yl]methyl}[1,2,4]triazolo[4,3-a]pyridin-3(2H)-on (23.0 mg, 46.4 µmol) und Palladium auf Kohle (50.0 mg, 10% Palladium) wurden in Ethanol (5.0 ml) suspendiert und über Nacht bei Raumtemperatur in einer Wasserstoffatmosphäre (1 atm) gerührt. Das Reaktionsgemisch wurde über Celite filtriert. Das Filtrat wurde eingeengt und der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Die produkthaltigen Fraktionen wurden im Vakuum eingeengt und es wurden 10.6 mg (46 % d. Th.) der Titelverbindung erhalten.
LC-MS (Methode 4): Rₜ = 0.85 min; MS (ESIpos): m/z = 500 [M+H]⁺
¹H-NMR (400 MHz, DMSO-d6) δ [ppm]: -0.149 (1.36), -0.008 (12.38), 0.008 (12.98), 0.146 (1.36), 1.724 (0.83), 1.755 (1.81), 1.781 (1.89), 1.807 (0.83), 2.067 (1.81), 2.084 (2.34), 2.120 (1.66), 2.167 (3.40), 2.229 (0.83), 2.323 (2.11), 2.327 (2.87), 2.332 (2.19), 2.366 (1.36), 2.665 (2.04), 2.669 (2.72), 2.674 (2.26), 2.710 (1.36), 3.477 (0.98), 3.486 (0.91), 3.498 (0.83), 3.511 (1.58), 3.520 (1.74), 3.532 (1.96), 3.541 (1.21), 3.567 (1.43), 3.574 (1.43), 3.632 (1.06), 3.645 (1.21), 3.666 (0.75), 3.690 (1.43), 3.704 (1.89), 3.726 (1.51), 3.736 (1.58), 3.757 (1.66), 3.771 (1.36), 3.794 (1.06), 3.804 (0.98), 3.840 (0.53), 3.873 (1.06), 3.892 (0.60), 3.909 (0.91), 3.935 (1.06), 3.949 (1.21), 3.964 (0.91), 3.986 (1.21), 4.000 (1.06), 4.015 (0.68), 4.028 (0.60), 4.104 (1.81), 4.136 (1.51), 4.152 (1.13), 4.166 (0.91), 4.179 (1.21), 4.191 (0.98), 4.205 (0.83), 4.220 (0.68), 4.892 (3.92), 4.901 (4.91), 5.065 (2.11), 5.106 (10.57), 5.120 (5.81), 5.126 (6.34), 5.167 (1.43), 5.257 (1.28), 5.270 (1.28), 5.280 (1.43), 5.289 (1.28), 5.300 (0.75), 5.318 (0.91), 5.335 (1.06), 5.346 (0.98), 5.357 (1.06), 5.370 (1.06), 5.381 (1.43), 5.393 (1.43), 5.402 (1.21), 5.412 (1.28), 5.421 (1.13), 5.433 (0.83), 5.447 (0.91), 5.455 (0.91), 5.464 (0.91), 5.479 (0.98), 5.486 (1.06), 5.500 (0.83), 7.922 (16.00), 7.941 (1.28), 8.634 (7.09).

### Beispiel 271

### (5RS,8RS)-5-{[(3S)-3-Fluorpyrrolidin-1-yl]carbonyl}-8-(trifluormethyl)-2-{[6-(trifluormethyl)pyridin-3-yl]methyl}-5,6,7,8-tetrahydro[1,2,4]triazolo[4,3-a]pyridin-3(2H)-on (Diastereomerengemisch; 4 Isomere)

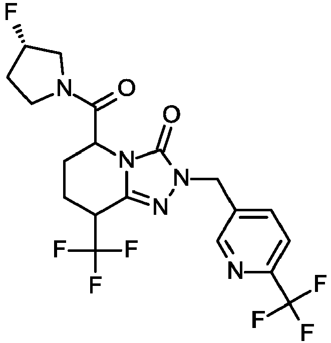

Unter Argon: 5-{[(3S)-3-Fluorpyrrolidin-1-yl]carbonyl}-8-(trifluormethyl)-2-{[6-(trifluormethyl)pyridin-3-yl]methyl}[1,2,4]triazolo[4,3-a]pyridin-3(2H)-on (165 mg, 346 µmol) und Palladium auf Kohle (50.0 mg, 10% Palladium) wurden in Ethanol (29 ml) suspendiert und über Nacht bei Raumtemperatur in einer Wasserstoffatmosphäre (1 atm) gerührt. Das Reaktionsgemisch wurde über Celite filtriert. Das Filtrat wurde eingeengt und der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Die produkthaltigen Fraktionen wurden im Vakuum eingeengt und es wurden 21.3 mg (74 % d. Th.) der Titelverbindung erhalten.

### Diastereomerengemisch (4 Isomere):

LC-MS (Methode 3): Rₜ = 1.51 min; MS (ESIpos): m/z = 482 [M+H]⁺
¹H-NMR (400 MHz, DMSO-d6) δ [ppm]: -0.150 (1.89), -0.034 (0.38), -0.009 (16.00), 0.007 (15.87), 0.146 (2.02), 1.742 (0.63), 1.772 (0.76), 1.799 (0.63), 1.827 (0.50), 2.078 (1.51), 2.104 (1.76), 2.152 (1.39), 2.178 (1.76), 2.222 (1.26), 2.251 (0.76), 2.322 (3.15), 2.327 (4.16), 2.331 (3.02), 2.365 (2.39), 2.522 (10.20), 2.587 (0.88), 2.639 (0.50), 2.664 (3.28), 2.669 (4.54), 2.674 (3.28), 2.700 (0.38), 2.709 (2.27), 2.731 (0.50), 2.890 (0.50), 3.366 (1.51), 3.384 (1.39), 3.396 (0.88), 3.412 (1.01), 3.424 (0.50), 3.439 (0.38), 3.467 (0.50), 3.520 (0.76), 3.535 (1.26), 3.559 (1.01), 3.588 (0.88), 3.609 (1.64), 3.629 (1.13), 3.656 (1.01), 3.676 (0.76), 3.737 (0.76), 3.757 (0.76), 3.779 (0.88), 3.804 (0.50), 3.869 (0.88), 3.926 (0.50), 3.948 (0.63), 3.964 (0.63), 4.022 (0.50), 4.053 (0.63), 4.091 (1.01), 4.097 (1.01), 4.778 (0.50), 4.791 (0.63), 4.837 (0.63), 4.846 (0.63), 4.921 (0.76), 4.933 (0.76), 4.956 (0.88), 5.059 (0.63), 5.069 (0.50), 5.099 (2.52), 5.110 (3.02), 5.122 (4.28), 5.162 (0.88), 5.268 (0.88), 5.355 (0.50), 5.402 (0.88), 5.490 (0.63), 7.897 (0.50), 7.921 (9.57), 7.941 (0.63), 8.635 (3.78).

### Beispiel 272

### (5RS,8RS)-5-{[(3S)-3-Fluorpyrrolidin-1-yl]carbonyl}-8-(trifluormethyl)-2-{[6-(trifluormethyl)pyridin-3-yl]methyl}-5,6,7,8-tetrahydro[1,2,4]triazolo[4,3-a]pyridin-3(2H)-on (Isomer 2)

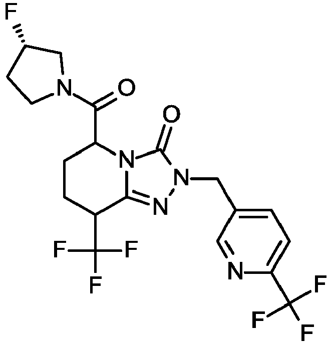

(5RS,8RS)-5-{[(3S)-3-Fluorpyrrolidin-1 -yl]carbonyl}-8-(trifluormethyl)-2-{[6-(trifluormethyl)pyridin-3-yl]methyl}-5,6,7,8-tetrahydro[1,2,4]triazolo[4,3-a]pyridin-3(2H)-on (Diastereomerengemisch; 4 Isomere) wurde durch chirale präparative HPLC getrennt [Probenvorbereitung: 93 mg wurden in 3 ml Ethanol und 3 ml n-Heptan gelöst; Injektionsvolumen: 0.58 ml; Säule: Daicel Chiralpak^{®} IA 5 µm, 250 x 20 mm; Laufmittel: n-Heptan/Ethanol 60:40; Fluss: 15 ml/min; Temperatur 40°C; UV-Detektion: 220 nm]. Nach der Trennung wurden 46 mg von Isomer 1, welches zuerst eluiert, und 32.7 mg von Isomer 2, welches später eluiert, isoliert.

### Isomer 2:

Analytische chirale HPLC: Rₜ = 1.95 min, d.e. = 98.4% [Säule: Daicel Chiralpak^{®} IA, 3 µm, 50 x 4.6 mm; Laufmittel: n-Heptan/Ethanol 50:50; Fluss: 1 ml/min; UV-Detektion: 220 nm].
LC-MS (Methode 3): Rₜ = 1.54 min; MS (ESIpos): m/z = 482 [M+H]⁺
¹H-NMR (400 MHz, DMSO-d6) δ [ppm]: -0.149 (0.71), -0.008 (6.13), 0.008 (5.87), 0.146 (0.71), 1.806 (1.01), 1.827 (1.52), 1.850 (1.06), 2.078 (2.58), 2.095 (2.28), 2.105 (3.14), 2.142 (2.03), 2.152 (1.92), 2.177 (1.77), 2.192 (1.52), 2.223 (2.38), 2.248 (1.47), 2.274 (1.32), 2.327 (1.77), 2.332 (1.32), 2.367 (1.37), 2.665 (1.16), 2.670 (1.57), 2.674 (1.16), 2.710 (1.27), 3.342 (1.42), 3.360 (0.71), 3.370 (0.81), 3.379 (0.91), 3.406 (0.86), 3.415 (0.81), 3.468 (0.66), 3.475 (0.71), 3.504 (0.81), 3.514 (0.81), 3.633 (2.94), 3.651 (2.18), 3.676 (1.57), 3.699 (1.22), 3.736 (2.03), 3.761 (1.92), 3.781 (1.57), 3.806 (1.16), 3.869 (2.38), 4.089 (1.37), 4.099 (1.37), 4.780 (1.42), 4.791 (1.57), 4.838 (2.18), 4.848 (1.82), 5.059 (1.87), 5.100 (7.90), 5.123 (5.77), 5.164 (1.42), 5.268 (1.42), 5.399 (1.62), 5.520 (0.91), 7.921 (16.00), 7.941 (0.96), 8.637 (7.04).

### Beispiel 273

### (5RS,8RS)-2-[(3-Fluorpyridin-2-yl)methyl]-5-{[(3S)-3-fluorpyrrolidin-1-yl]carbonyl}-8-(trifluormethyl)-5,6,7,8-tetrahydro[1,2,4]triazolo[4,3-a]pyridin-3(2H)-on (Diastereomerengemisch; 4 Isomere)

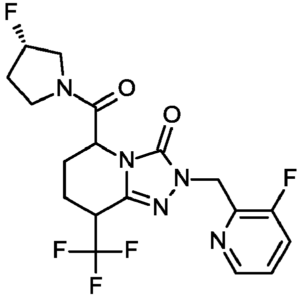

Unter Argon, 2-[(5-Chlor-3-fluorpyridin-2-yl)methyl]-5-{[(3S)-3-fluorpyrrolidin-1-yl]carbonyl}-8-(trifluormethyl)[1,2,4]triazolo[4,3-a]pyridin-3(2H)-on (50.0 mg, 62 % Reinheit, 67.1 µmol) und Palladium auf Kohle (10.0 mg, 10% Palladium) wurden in Ethanol (5.0 ml) suspendiert und über das Wochenende bei Raumtemperatur in einer Wasserstoffatmosphäre (1 atm) gerührt. Das Reaktionsgemisch wurde über Celite filtriert. Das Filtrat wurde eingeengt und der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Die produkthaltigen Fraktionen wurden im Vakuum eingeengt und es wurden 0.5 mg (1.7 % d. Th.) der Titelverbindung erhalten.
LC-MS (Methode 3): Rₜ = 1.18 min; MS (ESIpos): m/z = 432 [M+H]⁺
¹H-NMR (500 MHz, DMSO-d6) δ [ppm]: -0.120 (1.33), -0.009 (5.54), -0.007 (16.00), 0.007 (11.08), 0.117 (1.28), 1.330 (0.87), 1.343 (0.82), 1.768 (1.90), 1.796 (2.56), 1.815 (1.79), 1.823 (2.10), 1.856 (2.36), 1.880 (2.10), 1.901 (0.77), 2.010 (2.82), 2.019 (3.54), 2.027 (3.85), 2.037 (5.03), 2.050 (6.10), 2.072 (4.97), 2.081 (4.26), 2.101 (3.49), 2.121 (4.51), 2.152 (9.44), 2.164 (5.90), 2.182 (4.36), 2.230 (3.18), 2.258 (2.36), 2.271 (2.31), 2.354 (1.18), 2.358 (2.31), 2.361 (3.08), 2.365 (2.21), 2.369 (1.08), 2.518 (5.38), 2.522 (3.79), 2.628 (1.03), 2.631 (2.15), 2.635 (2.97), 2.639 (2.10), 2.643 (0.87), 3.348 (6.56), 3.358 (4.92), 3.371 (3.74), 3.380 (3.13), 3.390 (2.36), 3.411 (1.74), 3.418 (1.69), 3.461 (1.18), 3.468 (1.23), 3.488 (1.95), 3.495 (1.74), 3.516 (1.90), 3.522 (1.95), 3.543 (4.67), 3.563 (4.87), 3.577 (3.03), 3.585 (3.28), 3.590 (3.18), 3.598 (5.03), 3.615 (1.23), 3.633 (3.79), 3.650 (3.95), 3.656 (2.97), 3.670 (2.92), 3.686 (2.72), 3.711 (1.33), 3.738 (2.62), 3.746 (1.54), 3.756 (3.38), 3.764 (2.05), 3.772 (2.05), 3.783 (1.38), 3.789 (1.33), 3.812 (1.59), 3.837 (0.77), 3.861 (3.33), 3.936 (1.59), 3.954 (2.87), 3.972 (2.51), 3.994 (1.28), 4.015 (1.44), 4.041 (1.74), 4.079 (3.95), 4.772 (2.00), 4.776 (2.26), 4.785 (2.36), 4.825 (2.62), 4.830 (3.33), 4.838 (2.72), 4.842 (2.51), 4.896 (2.51), 4.900 (2.97), 4.908 (2.82), 4.912 (2.62), 4.936 (2.51), 4.943 (3.85), 4.953 (2.62), 4.965 (3.90), 4.970 (8.05), 4.973 (7.33), 4.997 (5.59), 5.001 (11.64), 5.005 (10.67), 5.114 (4.26), 5.118 (7.54), 5.122 (6.82), 5.127 (7.74), 5.131 (4.36), 5.146 (2.87), 5.149 (4.97), 5.154 (4.46), 5.159 (5.13), 5.163 (2.77), 5.279 (3.13), 5.285 (2.97), 5.368 (2.05), 5.385 (3.23), 5.393 (3.44), 5.474 (1.85), 5.507 (1.33), 7.422 (6.10), 7.431 (11.08), 7.439 (12.26), 7.448 (12.77), 7.457 (7.08), 7.711 (8.15), 7.713 (6.41), 7.729 (12.10), 7.733 (6.97), 7.748 (7.23), 8.360 (8.05), 8.363 (13.28), 8.369 (8.67), 8.372 (12.87), 8.524 (5.03).

### Beispiel 274

### (5RS,8RS)-5-{[(3R,4S)-3,4-Difluorpyrrolidin-1-yl]carbonyl}-2-[(3-fluorpyridin-2-yl)methyl]-8-(trifluormethyl)-5,6,7,8-tetrahydro[1,2,4]triazolo[4,3-a]pyridin-3(2H)-on (Diastereomerengemisch; 4 Isomere)

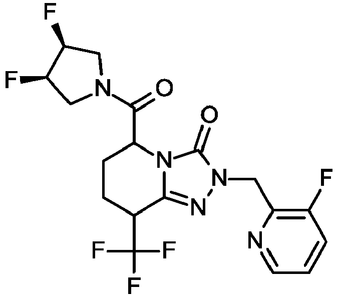

Unter Argon: 2-[(5-Chlor-3-fluorpyridin-2-yl)methyl]-5-{[(3R,4S)-3,4-difluorpyrrolidin-1-yl]carbonyl}-8-(trifluormethyl)[1,2,4]triazolo[4,3-a]pyridin-3(2H)-on (95.0 mg, 47 % Reinheit, 93.1 µmol) und Palladium auf Kohle (10.0 mg, 10% Palladium) wurden in Ethanol (5.0 ml) suspendiert und über Nacht bei Raumtemperatur in einer Wasserstoffatmosphäre (1 atm) gerührt. Das Reaktionsgemisch wurde über Celite filtriert. Das Filtrat wurde eingeengt und der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Die produkthaltigen Fraktionen wurden im Vakuum eingeengt und es wurden 17 mg (41 % d. Th.) der Titelverbindung erhalten.
LC-MS (Methode 3): Rₜ = 1.25 min; MS (ESIpos): m/z = 450 [M+H]⁺
¹H-NMR (400 MHz, DMSO-d6) δ [ppm]: -0.149 (2.00), 1.747 (1.83), 1.780 (4.50), 1.808 (5.17), 1.839 (2.50), 2.024 (4.67), 2.034 (5.67), 2.049 (5.67), 2.067 (4.67), 2.094 (2.83), 2.133 (9.50), 2.141 (9.33), 2.327 (6.00), 2.366 (3.83), 2.669 (6.17), 2.710 (3.67), 3.471 (3.00), 3.480 (2.83), 3.493 (2.67), 3.504 (4.67), 3.515 (5.50), 3.525 (5.33), 3.534 (3.67), 3.559 (4.17), 3.567 (4.50), 3.626 (2.83), 3.641 (3.33), 3.660 (2.17), 3.674 (2.67), 3.686 (4.17), 3.698 (4.83), 3.720 (4.83), 3.731 (4.50), 3.751 (5.17), 3.765 (3.83), 3.785 (3.33), 3.798 (3.17), 3.855 (2.67), 3.867 (3.00), 3.904 (2.33), 3.933 (2.83), 3.947 (3.00), 3.962 (2.00), 3.983 (3.50), 3.997 (3.17), 4.011 (2.00), 4.026 (1.83), 4.087 (4.83), 4.094 (4.83), 4.102 (5.00), 4.134 (3.67), 4.148 (3.17), 4.164 (2.67), 4.176 (3.67), 4.191 (2.50), 4.204 (2.17), 4.880 (11.50), 4.890 (14.33), 4.971 (9.67), 5.010 (16.00), 5.065 (3.00), 5.114 (9.33), 5.127 (10.00), 5.154 (4.83), 5.168 (6.00), 5.247 (2.50), 5.257 (3.50), 5.268 (3.67), 5.285 (3.50), 5.324 (2.33), 5.337 (3.17), 5.345 (3.00), 5.354 (3.00), 5.366 (3.17), 5.380 (4.17), 5.392 (4.17), 5.403 (3.67), 5.415 (3.67), 5.425 (2.83), 5.443 (2.33), 5.451 (2.33), 5.463 (2.83), 5.475 (3.17), 5.486 (2.83), 5.502 (2.00), 7.419 (5.67), 7.429 (10.67), 7.440 (12.17), 7.450 (12.83), 7.461 (7.00), 7.706 (8.50), 7.731 (13.17), 7.753 (7.83), 8.360 (13.00), 8.371 (12.83).

### Beispiel 275

### (5S,8RS)-5-{[(3S)-3-Fluorpyrrolidin-1-yl]carbonyl}-8-hydroxy-2-{[6-(trifluormethyl)pyridin-3-yl]methyl}-5,6,7,8-tetrahydro[1,2,4]triazolo[4,3-a]pyridin-3(2H)-on (Diastereomerengemisch; 2 Isomere)

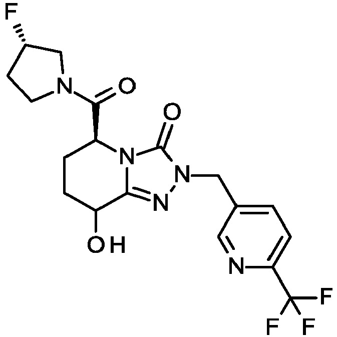

(5S)-5-{[(3S)-3-Fluorpyrrolidin-1 -yl]carbonyl}-2-{[6-(trifluormethyl)pyridin-3-yl]methyl}-5,6,7,8-tetrahydro[1,2,4]triazolo[4,3-a]pyridin-3(2H)-on (150 mg, 363 µmol) wurde unter Argon in Dichlormethan (1.5 ml) bei Raumtemperatur vorgelegt. Anschließend wurde bei 0°C Hydrogentetrafluoroborat (Ethylether-Addukt) (55 µl, 400 µmol) zugetropft und das Reaktionsgemisch für 30 min bei 0°C und 60 min bei Raumtemperatur gerührt. Das Reaktionsgemisch wurde eingeengt und über Nacht am Hochvakuum getrocknet. Der Rückstand wurde in Acetonitril (600 µl) gelöst und bei Raumtemperatur eine Lösung aus Eisen(II)-(*S*,*S*-(2-({(*S*)-2-[(*S*)-1-(pyridin-2-ylmethyl)pyrrolidin-2-yl]pyrrolidin-1-yl}methyl)pyridin)(bis-acetonitril) hexafluorantimonat (16.9 mg, 18.1 µmol) und Essigsäure (10 µl, 180 µmol) in Acetonitril (0.36 ml) langsam zugetropft. Anschließend wurde bei Raumtemperatur Wasserstoffperoxid (27 µl, 50 % Lösung in Wasser, 440 µmol) in Acetonitril (3.3 ml) langsam zugetropft und für 10 min gerührt. Es wurde erneut Wasserstoffperoxid (27 µl, 50 Gew.% Lösung in Wasser, 440 µmol) in Acetontiril (3.3 ml) zugetropft und für weitere 10 Minuten gerührt. Nochmal wurde Wasserstoffperoxid (27 µl, 50 % Lösung in Wasser, 440 µmol) in Acetonitril (3.3 ml) zugetropft und für weitere 40 Minuten bei Raumtemperatur gerührt. Das Reaktionsgemisch wurde mit 1 N wässriger Natriumhydroxid-Lösung versetzt und für 20 min bei Raumtemperatur stark gerührt. Es wurde erneut mit 1 N wässriger Natriumhydroxid-Lösung verdünnt und die wässrige Phase mit Dichlormethan extrahiert. Die organische Phase wurde mit gesättigter wässriger Natriumchlorid-Lösung gewaschen, über Natriumsulfat getrocknet, filtriert und das Filtrat eingeengt. Der Rückstand wurde über präparative HPLC (Methode 11) aufgereinigt. Die produkthaltigen Fraktionen wurden im Vakuum eingeengt und es wurden 15.0 mg (9 % d. Th.) der Titelverbindung erhalten.
LC-MS (Methode 3): Rₜ = 1.09 min; MS (ESIpos): m/z = 430 [M+H]⁺
¹H-NMR (400 MHz, DMSO-d6) δ [ppm]: -0.149 (1.25), -0.008 (16.00), 0.008 (8.87), 0.015 (0.77), 0.018 (0.60), 0.025 (0.32), 0.146 (1.17), 1.728 (0.85), 1.740 (1.01), 1.751 (1.41), 1.765 (1.49), 1.778 (1.61), 1.800 (0.97), 1.818 (1.17), 1.852 (0.77), 1.884 (0.36), 1.912 (0.52), 1.948 (0.60), 1.999 (0.40), 2.021 (0.36), 2.037 (0.24), 2.105 (1.49), 2.224 (0.60), 2.265 (0.85), 2.323 (1.25), 2.327 (1.53), 2.331 (1.21), 2.366 (2.10), 2.389 (0.97), 2.405 (1.01), 2.425 (0.77), 2.523 (7.70), 2.525 (6.85), 2.558 (1.21), 2.561 (0.77), 2.564 (0.69), 2.566 (0.64), 2.569 (0.56), 2.572 (0.56), 2.576 (0.52), 2.582 (0.40), 2.587 (0.28), 2.594 (0.28), 2.598 (0.28), 2.612 (0.24), 2.651 (0.28), 2.665 (1.29), 2.669 (1.69), 2.673 (1.17), 2.709 (1.69), 3.238 (0.28), 3.361 (0.69), 3.373 (0.40), 3.386 (0.64), 3.397 (0.48), 3.419 (0.32), 3.457 (0.36), 3.484 (0.48), 3.494 (0.52), 3.508 (0.28), 3.516 (0.32), 3.620 (0.85), 3.629 (1.29), 3.636 (1.37), 3.643 (1.45), 3.649 (1.37), 3.667 (1.25), 3.690 (0.85), 3.711 (0.64), 3.730 (1.33), 3.755 (1.21), 3.769 (0.97), 3.776 (1.01), 3.796 (0.60), 3.834 (0.32), 3.861 (1.21), 4.475 (0.36), 4.487 (0.69), 4.501 (0.64), 4.510 (0.77), 4.527 (2.02), 4.538 (1.93), 4.547 (0.77), 4.599 (0.36), 4.610 (0.44), 4.624 (0.24), 4.655 (0.36), 4.670 (0.64), 4.682 (0.40), 4.751 (0.77), 4.762 (0.69), 4.801 (0.89), 4.806 (0.93), 4.817 (0.93), 5.044 (3.83), 5.060 (5.96), 5.101 (0.24), 5.258 (0.93), 5.389 (1.21), 5.513 (0.64), 5.772 (2.66), 5.783 (3.51), 5.796 (1.41), 7.896 (0.60), 7.920 (7.58), 8.650 (2.66), 8.663 (1.37).

### Alternative Methode:

(5S)-5-{[(3S)-3-Fluorpyrrolidin-1-yl]carbonyl}-2-{[6-(trifluormethyl)pyridin-3-yl]methyl}-5,6,7,8-tetrahydro[1,2,4]triazolo[4,3-a]pyridin-3(2H)-on (400 mg, 968 µmol) und Cer(IV)sulfat (1.29 g, 3.87 mmol) wurden in tert-Butanol (1.3 ml) bei Raumtemperatur suspendiert. Anschließend wurde 1 N wässrige Schwefelsäure (1.3 ml, 24 mmol) zugegeben und das Reaktionsgemisch über Nacht bei 70°C gerührt. Das Reaktionsgemisch wurde auf Raumtemperatur abgekühlt und mit 2 N wässriger Natriumhydroxid-Lösung auf pH 9 gestellt. Die Suspension wurde filtriert und das Filtrat dreimal mit Dichlormethan extrahiert. Die vereinigten organischen Phasen wurden über Natriumsulfat getrocknet, filtriert und das Filtrat eingeengt. Es wurden 270 mg (59 % d. Th.) der Titelverbindung erhalten.
LC-MS (Methode 3): Rₜ = 1.03 min; MS (ESIpos): m/z = 430 [M+H]⁺

### Beispiel 276

### (5S)-5-{[(3S)-3-Fluorpyrrolidin-1-yl]carbonyl}-2-{[6-(trifluormethyl)pyridin-3-yl]methyl}-5,6-dihydro[1,2,4]triazolo[4,3-a]pyridin-3(2H)-on

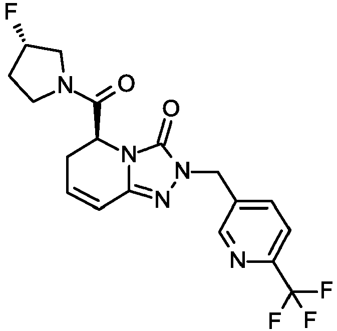

(5S,8RS)-5-{[(3S)-3-Fluorpyrrolidin-1 -yl]carbonyl}-8-hydroxy-2-{[6-(trifluormethyl)pyridin-3-yl]methyl}-5,6,7,8-tetrahydro[1,2,4]triazolo[4,3-a]pyridin-3(2H)-on (Diastereomerengemisch; 2 Isomere) (200 mg, 70 % Reinheit, 326 µmol) wurde in THF (4.8 ml) bei Raumtemperatur vorgelegt. Anschließend wurde 3,3,3-Triethyl-1-(methoxycarbonyl)diazathian-3-ium-1-id-2,2-dioxid (233 mg, 978 µmol) zugegeben und für 45 min bei 90°C in der Mikrowelle gerührt. Es wurden erneut 3,3,3-Triethyl-1-(methoxycarbonyl)diazathian-3-ium-1-id-2,2-dioxid (78 mg, 326 µmol) zugegeben und für weitere 2 Stunden bei 90°C in der Mikrowelle gerührt. Das Reaktionsgemisch wurde im Vakuum eingeengt und der Rückstand wurde über präparative HPLC aufgereinigt (Chromatorex C18, 10µm, 125mm x 40mm; Laufmittel: Acetonitril/Wasser mit 0.1% Ameisensäure-Gradient). Die produkthaltigen Fraktionen wurden im Vakuum eingeengt und es wurden 9.60 mg (7 % d. Th.) der Titelverbindung erhalten.
LC-MS (Methode 3): Rₜ = 1.28 min; MS (ESIpos): m/z = 412 [M+H]⁺
¹H-NMR (400 MHz, DMSO-d6) δ [ppm]: -0.149 (1.80), -0.032 (0.48), -0.027 (0.60), -0.024 (0.72), -0.019 (0.96), -0.016 (1.20), -0.009 (16.00), 0.007 (15.52), 0.018 (0.96), 0.022 (0.60), 0.027 (0.60), 0.146 (1.80), 2.091 (0.60), 2.116 (0.72), 2.125 (0.72), 2.165 (0.48), 2.239 (0.48), 2.270 (0.60), 2.322 (2.77), 2.327 (3.85), 2.332 (2.77), 2.366 (3.85), 2.394 (0.36), 2.414 (0.36), 2.420 (0.36), 2.434 (0.48), 2.440 (0.48), 2.447 (0.60), 2.451 (0.60), 2.521 (8.66), 2.523 (8.54), 2.526 (6.50), 2.557 (3.61), 2.560 (2.65), 2.562 (2.17), 2.564 (1.80), 2.567 (1.80), 2.569 (1.56), 2.572 (1.32), 2.574 (1.32), 2.577 (1.20), 2.579 (1.20), 2.582 (0.84), 2.584 (0.84), 2.586 (1.08), 2.589 (0.84), 2.601 (0.84), 2.606 (0.96), 2.621 (0.84), 2.628 (0.60), 2.648 (0.48), 2.653 (0.48), 2.665 (3.25), 2.669 (4.33), 2.674 (3.13), 2.709 (4.33), 2.725 (0.60), 2.747 (0.48), 2.753 (0.84), 2.770 (0.60), 2.913 (0.36), 2.943 (0.36), 2.978 (0.60), 2.994 (0.60), 3.008 (0.72), 3.032 (0.48), 3.047 (0.36), 3.055 (0.48), 3.208 (0.36), 3.220 (0.36), 3.246 (0.72), 3.255 (0.72), 3.357 (0.72), 3.372 (0.60), 3.423 (0.48), 3.470 (0.72), 3.496 (0.84), 3.637 (1.08), 3.661 (0.96), 3.688 (0.60), 3.728 (0.96), 3.774 (0.96), 3.847 (0.96), 4.949 (0.60), 4.970 (0.72), 5.004 (1.20), 5.024 (0.96), 5.080 (6.02), 5.259 (0.60), 5.390 (0.84), 5.511 (0.48), 6.248 (0.84), 6.262 (0.96), 6.278 (0.60), 6.307 (1.68), 6.315 (1.80), 6.334 (0.84), 6.340 (0.84), 7.922 (7.58), 8.559 (0.36), 8.664 (2.41), 17.706 (0.36).

### Beispiel 278

### (5S)-8,8-Difluor-5-{[(3S)-3-fluorpyrrolidin-1-yl]carbonyl}-2-{[6-(trifluormethyl)pyridin-3-yl]methyl}-5,6,7,8-tetrahydro[1,2,4]triazolo[4,3-a]pyridin-3(2H)-on

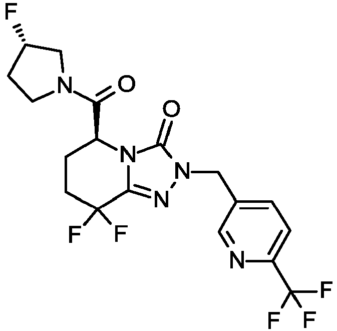

(5S)-5-{[(3S)-3-Fluorpyrrolidin-1-yl]carbonyl}-2-{[6-(trifluormethyl)pyridin-3-yl]methyl}-6,7-dihydro[1,2,4]triazolo[4,3-a]pyridin-3,8(2H,5H)-dion (Isomer 1) (33.5 mg, 62 % Reinheit, 48.6 µmol) wurde unter Argon in Dichlormethan (5.0 ml) bei Raumtemperatur vorgelegt. Anschließend wurde Diethylaminoschwefeltrifluorid (19 µl, 150 µmol) zugegeben und bei 40°C über Nacht gerührt. Das Reaktionsgemisch wurde mit Wasser und gesättigter wässriger Natriumhydrogencarbonat-Lösung versetzt. Die wässrige Phase wurde viermal mit Dichlormethan extrahiert und die vereinigten organischen Phasen wurden über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Die produkthaltigen Fraktionen wurden im Vakuum eingeengt und es wurden 7.10 mg (31 % d. Th.) der Titelverbindung erhalten.
LC-MS (Methode 3): Rₜ = 1.52 min; MS (ESIpos): m/z = 450 [M+H]⁺
¹H-NMR (400 MHz, DMSO-d6) δ [ppm]: -0.149 (1.80), -0.008 (15.78), 0.008 (16.00), 0.027 (0.58), 0.146 (1.66), 1.096 (1.30), 1.113 (3.17), 1.131 (1.51), 2.119 (0.94), 2.148 (1.37), 2.169 (1.01), 2.177 (0.94), 2.237 (1.15), 2.284 (2.02), 2.323 (3.24), 2.327 (3.68), 2.332 (3.10), 2.352 (1.73), 2.366 (3.17), 2.381 (1.37), 2.407 (0.94), 2.435 (1.30), 2.446 (1.08), 2.523 (6.92), 2.568 (0.58), 2.591 (0.58), 2.665 (1.95), 2.670 (2.67), 2.674 (1.95), 2.710 (2.31), 3.184 (0.58), 3.201 (0.65), 3.243 (0.72), 3.346 (1.51), 3.357 (1.08), 3.375 (0.65), 3.395 (0.65), 3.420 (0.58), 3.428 (0.58), 3.518 (0.58), 3.527 (0.65), 3.614 (0.58), 3.655 (1.59), 3.679 (1.66), 3.707 (1.15), 3.758 (0.94), 3.780 (1.37), 3.801 (1.15), 3.825 (0.79), 3.890 (1.51), 4.864 (0.72), 4.878 (1.15), 4.940 (1.51), 5.172 (9.87), 5.277 (1.08), 5.407 (1.15), 5.531 (0.58), 7.946 (14.49), 7.949 (9.37), 8.677 (4.25).

### Beispiel 279

### (5S,8RS)-2-[(5-Chlorpyridin-3-yl)methyl]-5-{[(3S)-3-fluorpyrrolidin-1-yl]carbonyl}-8-hydroxy-5,6,7,8-tetrahydro[1,2,4]triazolo[4,3-a]pyridin-3(2H)-on (Diastereomerengemisch; 2 Isomere)

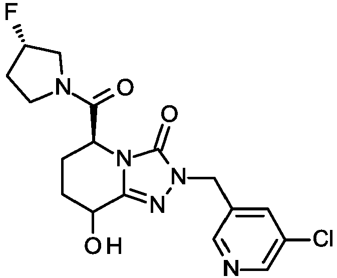

(5S)-2-[(5-Chlorpyridin-3-yl)methyl]-5-{[(3S)-3-fluorpyrrolidin-1-yl]carbonyl}-5,6,7,8-tetrahydro[1,2,4]triazolo[4,3-a]pyridin-3(2H)-on (15.2 mg, 38.8 µmol) und Cer(IV)sulfat (51.6 mg, 155 µmol) wurden in tert-Butanol (52 µl) bei Raumtemperatur suspendiert. Anschließend wurde 1 N wässrige Schwefelsäure (52 µl, 970 µmol) zugegeben und das Reaktionsgemisch über Nacht bei 70°C gerührt. Es wurde erneut Cer(IV)sulfat (25.8 mg, 77.6 µmol) zugegeben und ein weiteres Mal über Nacht bei 70°C gerührt. Das Reaktionsgemisch wurde auf Raumtemperatur abgekühlt und mit 2 N wässriger Natriumhydroxid-Lösung auf pH 9 gestellt. Der Feststoff wurde abgesaugt und das Filtrat dreimal mit Dichlormethan extrahiert. Die vereinigten organischen Phasen wurden über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Die produkthaltigen Fraktionen wurden im Vakuum eingeengt und es wurden 7.10 mg (46 % d. Th.) der Titelverbindung erhalten.
LC-MS (Methode 4): Rₜ = 0.48 min; MS (ESIpos): m/z = 396 [M+H]⁺
¹H-NMR (600 MHz, DMSO-d6) δ [ppm]: 1.443 (2.53), 1.446 (2.51), 1.727 (1.43), 1.743 (2.23), 1.749 (2.12), 1.757 (2.29), 1.791 (1.45), 1.796 (1.26), 1.811 (2.25), 1.829 (1.28), 1.854 (1.39), 1.907 (2.21), 1.937 (0.93), 1.996 (0.93), 2.012 (1.02), 2.071 (0.87), 2.085 (1.04), 2.103 (2.12), 2.108 (2.12), 2.132 (1.99), 2.156 (1.21), 2.235 (1.28), 2.266 (1.28), 2.383 (2.38), 2.612 (1.34), 3.300 (15.81), 3.310 (16.00), 3.377 (2.90), 3.400 (2.40), 3.423 (1.52), 3.442 (1.30), 3.448 (1.39), 3.465 (1.54), 3.471 (1.45), 3.488 (1.04), 3.626 (1.56), 3.644 (3.44), 3.655 (2.51), 3.662 (2.68), 3.686 (1.65), 3.707 (1.97), 3.725 (1.60), 3.733 (1.52), 3.751 (1.80), 3.766 (1.08), 3.784 (1.95), 3.803 (1.19), 3.830 (1.15), 3.847 (2.25), 4.502 (1.39), 4.537 (3.66), 4.611 (1.00), 4.659 (0.82), 4.669 (1.34), 4.677 (0.89), 4.749 (1.21), 4.757 (1.19), 4.803 (1.34), 4.806 (1.39), 4.813 (1.52), 4.947 (6.52), 4.964 (10.72), 5.280 (1.80), 5.367 (1.75), 5.403 (1.19), 5.490 (1.21), 5.783 (1.47), 7.763 (3.66), 7.782 (2.77), 8.355 (1.36), 8.431 (4.78), 8.446 (3.55), 8.569 (7.77), 8.572 (7.90).

### Beispiel 280

### (5RS,6RS)-5-{[(3S)-3-Fluorpyrrolidin-1-yl]carbonyl}-2-{[3-fluor-2-(trifluormethyl)pyridin-4-yl]methyl}-6-(trifluormethyl)-5,6,7,8-tetrahydro[1,2,4]triazolo[4,3-a]pyridin-3(2H)-on (Diasteromeregemisch, 2 Isomere)

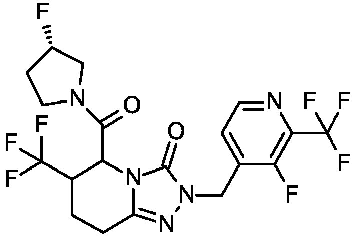

(5RS,6RS)-2-{[3-Fluor-2-(trifluormethyl)pyridin-4-yl]methyl}-3-oxo-6-(trifluormethyl)-2,3,5,6,7,8-hexahydro[1,2,4]triazolo[4,3-a]pyridin-5-carbonsäure (Diastereomerengemisch; 4 Isomere) (96.0 mg, 224 µmol) wurde in THF (2.0 ml) vorgelegt und HBTU (111 mg, 291 µmol) und N,N-Diisopropylethylamin (120 µl, 670 µmol) wurden anschließend zugegeben. Nach 15 min Rühren bei Raumtemperatur wurde (3S)-3-Fluorpyrrolidinhydrochlorid (33.8 mg, 269 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 72.0 mg (64 % d. Th.) der Titelverbindung erhalten.
LC-MS (Methode 3): Rₜ = 1.64 min; MS (ESIpos): m/z = 500 [M+H]⁺
¹H-NMR (400 MHz, DMSO-d6) δ [ppm]: -0.149 (1.82), -0.008 (16.00), 0.008 (13.07), 0.146 (1.70), 2.043 (1.47), 2.248 (0.64), 2.328 (2.23), 2.366 (2.34), 2.523 (6.33), 2.670 (2.81), 2.710 (2.81), 2.782 (1.17), 2.823 (0.70), 3.513 (0.70), 3.538 (1.41), 3.619 (0.70), 3.668 (0.94), 3.768 (0.76), 4.135 (0.70), 4.840 (0.82), 4.855 (0.76), 4.898 (1.11), 4.909 (1.05), 4.942 (0.88), 4.951 (0.82), 4.972 (1.00), 4.986 (0.88), 5.082 (4.10), 5.097 (3.81), 5.278 (0.76), 5.412 (0.88), 7.546 (0.88), 7.558 (1.88), 7.573 (1.70), 7.587 (1.11), 8.572 (3.52), 8.584 (3.40).

### Beispiel 281

### (5RS,6RS)-5-{[(3S)-3-Fluorpyrrolidin-1-yl]carbonyl}-2-{[3-fluor-2-(trifluormethyl)pyridin-4-yl]methyl}-6-(trifluormethyl)-5,6,7,8-tetrahydro[1,2,4]triazolo[4,3-a]pyridin-3(2H)-on (Isomer 1)

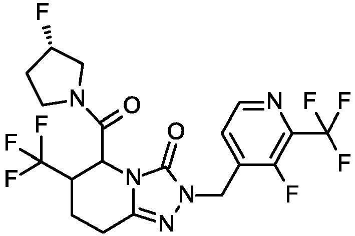

(5RS,6RS)-5-{[(3S)-3-Fluorpyrrolidin-1 -yl]carbonyl}-2-{[3-fluor-2-(trifluormethyl)pyridin-4-yl]methyl}-6-(trifluormethyl)-5,6,7,8-tetrahydro[1,2,4]triazolo[4,3-a]pyridin-3(2H)-on (Diasteromerengemisch; 2 Isomere) wurde durch chirale präparative HPLC getrennt [Probenvorbereitung: 68 mg gelöst in 6 ml Acetonitril/Ethanol (2:1); Injektionsvolumen: 0.4 ml; Säule: Daicel Chiralcel^{®} OD-H 5 µm, 250 x 20 mm; Laufmittel: n-Heptan/Ethanol 70:30; Fluss: 15 ml/min; Temperatur 25°C; UV Detektion: 210 nm]. Nach der Trennung wurden 26.8 mg von Isomer 1, welches zuerst eluierte, und 27.2 mg von Isomer 2, welches später eluierte, isoliert.

### Isomer 1:

Analytische chirale HPLC: Rₜ = 10.33 min, d.e. = 100% [Säule: Daicel Chiralcel^{®} OD-3 50 x 4.6 mm; Laufmittel: n-Heptan/Ethanol 90:10; Fluss: 1 ml/min; UV-Detektion: 220 nm].
LC-MS (Methode 4): Rₜ = 0.86 min; MS (ESIpos): m/z = 500 [M+H]⁺
¹H-NMR (500 MHz, DMSO-d6) δ [ppm]: -0.120 (0.91), -0.007 (10.14), 0.006 (7.74), 0.117 (0.91), 0.845 (0.57), 1.234 (1.37), 1.961 (2.73), 1.978 (2.79), 1.989 (2.33), 2.036 (3.36), 2.047 (6.89), 2.062 (6.89), 2.073 (3.93), 2.095 (2.11), 2.133 (2.96), 2.159 (2.28), 2.265 (1.82), 2.290 (1.88), 2.361 (2.39), 2.365 (1.71), 2.518 (5.98), 2.522 (4.56), 2.631 (3.87), 2.635 (3.13), 2.649 (2.16), 2.665 (3.42), 2.681 (2.51), 2.703 (1.54), 2.716 (2.22), 2.769 (1.71), 2.785 (3.30), 2.797 (4.61), 2.820 (2.22), 2.831 (2.79), 3.331 (3.70), 3.345 (2.68), 3.394 (1.77), 3.415 (1.77), 3.423 (2.05), 3.465 (1.77), 3.471 (2.16), 3.492 (3.59), 3.500 (3.76), 3.509 (2.51), 3.649 (3.07), 3.667 (4.84), 3.679 (4.38), 3.704 (2.51), 3.724 (5.24), 3.742 (3.07), 3.755 (2.05), 3.785 (1.71), 3.806 (2.73), 3.820 (2.79), 3.840 (1.31), 4.018 (1.37), 4.042 (1.31), 4.092 (1.20), 4.110 (1.14), 4.840 (6.32), 4.853 (6.32), 4.899 (9.00), 4.907 (8.71), 5.034 (1.77), 5.054 (2.28), 5.067 (8.54), 5.082 (10.65), 5.087 (13.04), 5.098 (12.19), 5.115 (1.82), 5.132 (1.94), 5.293 (2.96), 5.399 (3.02), 5.521 (1.99), 5.946 (0.51), 7.549 (3.87), 7.559 (7.12), 7.569 (4.21), 7.575 (3.25), 7.585 (5.41), 7.596 (2.73), 8.572 (16.00), 8.582 (15.43).

### Beispiel 282

### (5RS,6RS)-2-[(3,5-Dichlorpyridin-2-yl)methyl]-5-{[(3S)-3-fluorpyrrolidin-1 -yl]carbonyl}-6-(trifluormethyl)-5,6,7,8-tetrahydro[1,2,4]triazolo[4,3-a]pyridin-3(2H)-on (Diasteromerengemisch; 2 Isomere)

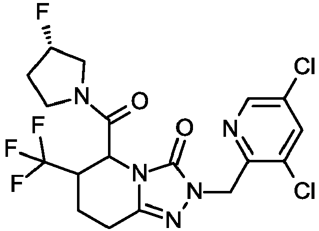

(5RS,6RS)-2-[(3,5-Dichlorpyridin-2-yl)methyl]-3-oxo-6-(trifluormethyl)-2,3,5,6,7,8-hexahydro[1,2,4]triazolo[4,3-a]pyridin-5-carbonsäure (Diastereomerengemisch; 4 Isomere) (50.0 mg, 122 µmol) wurde in THF (2.0 ml) vorgelegt und HBTU (60.0 mg, 158 µmol) und N,N-Diisopropylethylamin (64 µl, 360 µmol) wurden anschließend zugegeben. Nach 15 min Rühren bei Raumtemperatur wurde (3S)-3-Fluorpyrrolidinhydrochlorid (18.3 mg, 146 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 46.4 mg (79 % d. Th.) der Titelverbindung erhalten.
LC-MS (Methode 4): Rₜ = 0.84 min; MS (ESIpos): m/z = 482 [M+H]⁺
¹H-NMR (400 MHz, DMSO-d6) δ [ppm]: -0.149 (0.64), -0.008 (5.30), 0.008 (4.77), 0.146 (0.64), 1.038 (0.42), 1.055 (0.95), 1.073 (0.53), 1.944 (1.80), 1.964 (1.80), 1.978 (1.48), 2.003 (1.80), 2.026 (2.23), 2.041 (2.75), 2.055 (2.23), 2.073 (5.93), 2.110 (2.23), 2.144 (1.38), 2.244 (1.27), 2.286 (1.27), 2.323 (1.80), 2.327 (2.44), 2.366 (1.70), 2.523 (5.93), 2.575 (1.06), 2.620 (1.91), 2.665 (2.86), 2.670 (3.18), 2.674 (2.65), 2.690 (0.95), 2.710 (2.01), 2.736 (2.75), 2.779 (1.48), 3.347 (2.65), 3.358 (2.01), 3.376 (2.01), 3.405 (1.70), 3.467 (1.17), 3.502 (1.38), 3.530 (3.07), 3.567 (1.17), 3.607 (2.12), 3.652 (1.48), 3.669 (1.91), 3.689 (1.80), 3.714 (1.59), 3.733 (1.38), 3.775 (0.95), 3.803 (0.85), 3.819 (0.95), 3.845 (0.42), 4.004 (0.53), 4.038 (0.42), 4.089 (1.06), 4.122 (1.59), 4.146 (1.80), 4.170 (0.74), 4.816 (2.01), 4.831 (1.91), 4.862 (2.54), 4.871 (2.54), 4.882 (2.23), 4.891 (2.01), 4.930 (2.44), 4.944 (2.33), 4.981 (1.80), 4.987 (1.06), 5.021 (10.91), 5.026 (16.00), 5.045 (6.46), 5.067 (0.74), 5.085 (1.59), 5.279 (1.70), 5.409 (2.01), 5.509 (0.85), 5.945 (0.42), 8.254 (8.26), 8.259 (8.90), 8.558 (6.15), 8.560 (6.78), 8.563 (6.68).

### Beispiel 283

### (5RS,6RS)-2-[(3,5-Dichlorpyridin-2-yl)methyl]-5-{[(3S)-3-fluorpyrrolidin-1-yl]carbonyl}-6-(trifluormethyl)-5,6,7,8-tetrahydro[1,2,4]triazolo[4,3-a]pyridin-3(2H)-on (Isomer 1)

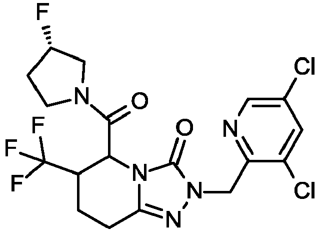

(5RS,6RS)-2-[(3,5-Dichlorpyridin-2-yl)methyl]-5-{[(3S)-3-fluorpyrrolidin-1 -yl]carbonyl}-6-(trifluormethyl)-5,6,7,8-tetrahydro[1,2,4]triazolo[4,3-a]pyridin-3(2H)-on (Diasteromerengemisch; 2 Isomere) wurde durch chirale präparative HPLC getrennt [Probenvorbereitung: 43 mg gelöst in 5 ml Acetonitril/Isopropanol (3:2); Injektionsvolumen: 0.5 ml; Säule: Daicel Chiralcel^{®} OD-H 5 µm, 250 x 20 mm; Laufmittel: n-Heptan/Isopropanol 50:50; Fluss: 15 ml/min; Temperatur 25°C; UV-Detektion: 210 nm]. Nach der Trennung wurden 19.2 mg von Isomer 1, welches zuerst eluierte, und 18.3 mg von Isomer 2, welches später eluierte, isoliert.

### Isomer 1:

Analytische chirale HPLC: Rₜ = 3.97 min, d.e. = 100% [Säule: Daicel Chiralcel^{®} ID-3 50 x 4.6 mm; Laufmittel: n-Heptan/Isopropanol 50:50; Fluss: 1 ml/min; UV-Detektion: 220 nm].
LC-MS (Methode 3): Rₜ = 1.61 min; MS (ESIpos): m/z = 482 [M+H]⁺
¹H-NMR (400 MHz, DMSO-d6) δ [ppm]: -0.149 (1.20), -0.008 (14.40), 0.008 (9.18), 0.146 (1.16), 1.234 (0.96), 1.909 (0.80), 1.922 (1.36), 1.944 (1.80), 1.957 (1.28), 2.026 (2.85), 2.041 (3.45), 2.055 (2.45), 2.078 (1.88), 2.112 (2.41), 2.147 (1.80), 2.254 (1.28), 2.288 (1.20), 2.327 (1.72), 2.366 (1.44), 2.524 (7.14), 2.576 (1.48), 2.602 (1.72), 2.620 (1.88), 2.670 (2.57), 2.674 (2.73), 2.710 (1.92), 2.745 (2.85), 2.758 (1.84), 2.788 (1.60), 3.404 (1.76), 3.473 (2.25), 3.501 (1.96), 3.509 (2.01), 3.656 (2.81), 3.668 (3.05), 3.690 (3.53), 3.714 (2.93), 3.733 (2.09), 3.749 (2.09), 3.776 (1.16), 3.802 (1.76), 3.820 (1.76), 3.844 (0.88), 4.005 (0.92), 4.036 (0.76), 4.099 (0.88), 4.122 (0.80), 4.816 (3.61), 4.831 (3.61), 4.861 (4.77), 4.871 (4.61), 4.981 (3.09), 5.020 (16.00), 5.027 (8.82), 5.046 (8.82), 5.067 (1.08), 5.085 (2.49), 5.277 (1.80), 5.408 (2.29), 5.529 (1.36), 8.254 (6.50), 8.259 (6.74), 8.557 (6.14), 8.562 (7.06), 8.566 (4.81).

### Beispiel 284

### (5RS,6RS)-5-{[(3S)-3-Fluorpyrrolidin-1-yl]carbonyl}-6-(trifluormethyl)-2-{[6-(trifluormethyl)pyridin-3-yl]methyl}-5,6,7,8-tetrahydro[1,2,4]triazolo[4,3-a]pyridin-3(2H)-on (Diastereomerengemisch; 2 Isomere)

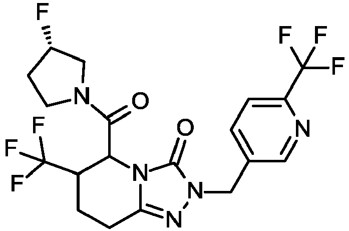

(5RS,6RS)-3-Oxo-6-(trifluormethyl)-2-{[6-(trifluormethyl)pyridin-3-yl]methyl}-2,3,5,6,7,8-hexahydro[1,2,4]triazolo[4,3-a]pyridin-5-carbonsäure (Diastereomerengemisch; 4 Isomere) (54.0 mg, 132 µmol) wurde in THF (2.0 ml) vorgelegt und HBTU (64.9 mg, 171 µmol) und N,N-Diisopropylethylamin (69 µl, 390 µmol) wurden anschließend zugegeben. Nach 15 min Rühren bei Raumtemperatur wurde (3S)-3-Fluorpyrrolidinhydrochlorid (19.8 mg, 158 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 51.4 mg (81 % d. Th.) der Titelverbindung erhalten.
LC-MS (Methode 3): Rₜ = 1.55 min; MS (ESIpos): m/z = 482 [M+H]⁺

### Beispiel 285

### (5RS,6RS)-5-{[(3S)-3-Fluorpyrrolidin-1-yl]carbonyl}-6-(trifluormethyl)-2-{[6-(trifluormethyl)pyridin-3-yl]methyl}-5,6,7,8-tetrahydro[1,2,4]triazolo[4,3-a]pyridin-3(2H)-on (Isomer 1)

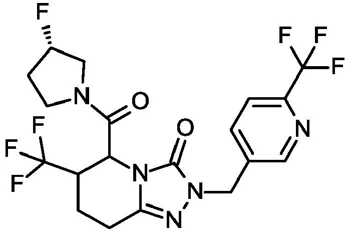

(5RS,6RS)-5-{[(3S)-3-Fluorpyrrolidin-1-yl]carbonyl}-6-(trifluormethyl)-2-{[6-(trifluormethyl)pyridin-3-yl]methyl}-5,6,7,8-tetrahydro[1,2,4]triazolo[4,3-a]pyridin-3(2H)-on (Diastereomerengemisch; 2 Isomere) wurde durch chirale präparative HPLC getrennt [Probenvorbereitung: 200 mg gelöst in 5 ml Acetonitril/Isopropanol (1:1); Injektionsvolumen: 0.15 ml; Säule: Daicel Chiralcel^{®} IB 5 µm, 250 x 30 mm; Laufmittel: n-Heptan/Isopropanol 50:50; Fluss: 60 ml/min; Temperatur 28°C; UV-Detektion: 220 nm]. Nach der Trennung wurden 69.8 mg von Isomer 1, welches zuerst eluierte, und 71.7 mg von Isomer 2, welches später eluierte, isoliert.

### Isomer 1:

Analytische chirale HPLC: Rₜ = 1.79 min, d.e. = 100% [Säule: Daicel Chiralcel^{®} IB-3 50 x 4.6 mm; Laufmittel: n-Heptan/Isopropanol 50:50; Fluss: 1 ml/min; UV-Detektion: 220 nm].
LC-MS (Methode 3): Rₜ = 1.54 min; MS (ESIpos): m/z = 482 [M+H]⁺
¹H-NMR (400 MHz, DMSO-d6) δ [ppm]: -0.149 (1.40), -0.008 (16.00), 0.008 (9.77), 0.146 (1.40), 2.052 (1.18), 2.327 (2.15), 2.366 (1.93), 2.669 (2.36), 2.710 (2.15), 2.781 (0.75), 3.509 (0.64), 3.666 (1.07), 4.831 (0.97), 4.847 (0.97), 4.889 (1.29), 4.899 (1.29), 5.018 (2.36), 5.035 (4.08), 5.278 (0.64), 5.405 (0.75), 7.919 (7.30), 8.643 (1.83).

### Beispiel 286

### (5RS,6RS)-5-{[(3S)-3-Fluorpyrrolidin-1-yl]carbonyl}-6-(trifluormethyl)-2-{[6-(trifluormethyl)pyridin-3-yl]methyl}-5,6,7,8-tetrahydro[1,2,4]triazolo[4,3-a]pyridin-3(2H)-on (Isomer 2)

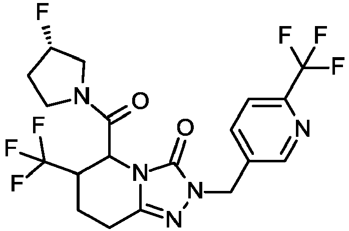

(5RS,6RS)-5-{[(3S)-3-Fluorpyrrolidin-1-yl]carbonyl}-6-(trifluormethyl)-2-{[6-(trifluormethyl)pyridin-3-yl]methyl}-5,6,7,8-tetrahydro[1,2,4]triazolo[4,3-a]pyridin-3(2H)-on (Diastereomerengemisch; 2 Isomere) wurde durch chirale präparative HPLC getrennt [Probenvorbereitung: 200 mg gelöst in 5 ml Acetonitril/Isopropanol (1:1); Injektionsvolumen: 0.15 ml; Säule: Daicel Chiralcel^{®} IB 5 µm, 250 x 30 mm; Laufmittel: n-Heptan/Isopropanol 50:50; Fluss: 60 ml/min; Temperatur 28°C; UV-Detektion: 220 nm]. Nach der Trennung wurden 69.8 mg von Isomer 1, welches zuerst eluierte, und 71.7 mg von Isomer 2, welches später eluierte, isoliert.

### Isomer 2:

Analytische chirale HPLC: Rₜ = 2.10 min, d.e. = 100% [Säule: Daicel Chiralcel^{®} IB-3 50 x 4.6 mm; Laufmittel: n-Heptan/Isopropanol 50:50; Fluss: 1 ml/min; UV Detektion: 220 nm].
LC-MS (Methode 3): Rₜ = 1.54 min; MS (ESIpos): m/z = 482 [M+H]⁺
¹H-NMR (400 MHz, DMSO-d6) δ [ppm]: -0.149 (1.85), -0.008 (16.00), 0.008 (14.32), 0.146 (1.85), 1.967 (1.18), 2.028 (2.36), 2.044 (2.02), 2.327 (3.03), 2.366 (2.36), 2.524 (6.74), 2.587 (0.84), 2.669 (3.87), 2.710 (3.03), 2.767 (1.85), 3.376 (2.19), 3.394 (2.02), 3.422 (1.35), 3.535 (3.54), 3.561 (1.68), 3.589 (1.52), 3.607 (1.68), 3.766 (0.67), 4.099 (0.84), 4.121 (1.18), 4.145 (1.68), 4.187 (0.67), 4.930 (2.36), 4.939 (2.36), 4.963 (2.86), 4.977 (2.69), 5.024 (8.76), 5.036 (4.72), 5.280 (1.01), 5.383 (1.01), 5.413 (1.01), 5.514 (1.01), 7.918 (15.16), 7.921 (16.00), 8.644 (4.04).

### Beispiel 287

### (5RS,6RS)-2-[(3,5-Dichlorpyridin-2-yl)methyl]-5-{[(3R,4S)-3,4-difluorpyrrolidin-1-yl]carbonyl}-6-(trifluormethyl)-5,6,7,8-tetrahydro[1,2,4]triazolo[4,3-a]pyridin-3(2H)-on (Diastereomer 1; Racemat)

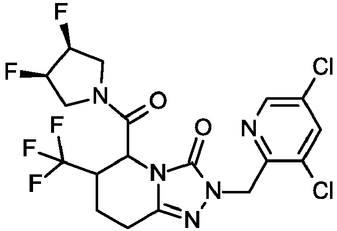

(5RS,6RS)-2-[(3,5-Dichlorpyridin-2-yl)methyl]-3-oxo-6-(trifluormethyl)-2,3,5,6,7,8-hexahydro[1,2,4]triazolo[4,3-a]pyridin-5-carbonsäure (Diastereomerengemisch; 4 Isomere) (50.0 mg, 122 µmol) wurde in THF (2.0 ml) vorgelegt und HBTU (60.0 mg, 158 µmol) und N,N-Diisopropylethylamin (64 µl, 360 µmol) wurden anschließend zugegeben. Nach 15 min Rühren bei Raumtemperatur wurde (3R,4S)-3,4-Difluorpyrrolidinhydrochlorid (20.9 mg, 146 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 41.0 mg (67 % d. Th.) der Titelverbindung erhalten.
LC-MS (Methode 3): Rₜ = 1.66 min; MS (ESIpos): m/z = 500 [M+H]⁺
¹H-NMR (400 MHz, DMSO-d6) δ [ppm]: -0.149 (1.74), -0.008 (16.00), 0.008 (14.26), 0.146 (1.74), 1.937 (1.09), 2.021 (2.29), 2.073 (2.61), 2.328 (3.16), 2.366 (3.16), 2.603 (1.31), 2.625 (1.52), 2.669 (4.03), 2.710 (3.48), 2.740 (2.29), 2.756 (1.52), 3.503 (1.20), 3.650 (1.20), 3.699 (1.09), 3.716 (1.31), 3.818 (1.09), 3.847 (0.87), 4.057 (0.76), 4.398 (0.87), 4.918 (4.14), 4.932 (2.72), 4.989 (1.41), 5.027 (14.59), 5.047 (5.33), 5.087 (1.31), 5.255 (0.98), 5.356 (1.20), 5.462 (0.87), 8.255 (6.10), 8.260 (6.64), 8.557 (5.22).

### Beispiel 288

### (5RS,6RS)-2-[(3,5-Dichlorpyridin-2-yl)methyl]-5-{[(3R,4S)-3,4-difluorpyrrolidin-1-yl]carbonyl}-6-(trifluormethyl)-5,6,7,8-tetrahydro[1,2,4]triazolo[4,3-a]pyridin-3(2H)-on (Enantiomer 2)

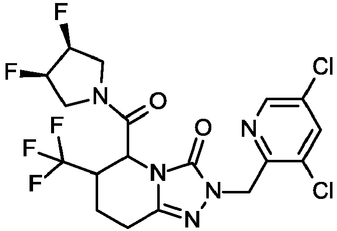

(5RS,6RS)-2-[(3,5-Dichlorpyridin-2-yl)methyl]-5-{[(3R,4S)-3,4-difluorpyrrolidin-1-yl]carbonyl}-6-(trifluormethyl)-5,6,7,8-tetrahydro[1,2,4]triazolo[4,3-a]pyridin-3(2H)-on (Diastereomer 1; Racemat) wurde durch chirale präparative HPLC getrennt [Probenvorbereitung: 40 mg gelöst in 3.5 ml Ethanol (im Ultraschallbad); Injektionsvolumen: 0.80 ml; Säule: Daicel Chiralcel^{®} OX-H 5 µm, 250 x 20 mm; Laufmittel: Ethanol + 0.2 % Diethylamin; Fluss: 15 ml/min; Temperatur 55°C; UV Detektion: 220 nm]. Nach der Trennung wurden 19.5 mg von Enantiomer 1, welches zuerst eluierte, und 18.4 mg von Enantiomer 2, welches später eluierte, isoliert.

### Enantiomer 2:

Analytische chirale HPLC: Rₜ = 8.73 min, e.e. = 100% [Säule: Daicel Chiralcel^{®} OX-H-3 250 x 4.6 mm; Laufmittel: Ethanol + 0.2 % Diethylamin; Fluss: 1 ml/min; UV-Detektion: 220 nm].
LC-MS (Methode 3): Rₜ = 1.70 min; MS (ESIpos): m/z = 500 [M+H]⁺
¹H-NMR (400 MHz, DMSO-d6) δ [ppm]: -0.150 (0.79), 0.146 (0.79), 1.234 (0.93), 1.936 (1.08), 2.006 (2.14), 2.022 (2.59), 2.077 (1.22), 2.327 (1.45), 2.366 (1.24), 2.605 (1.53), 2.626 (1.90), 2.670 (2.67), 2.710 (1.61), 2.741 (2.78), 2.756 (1.72), 2.784 (1.48), 3.504 (1.59), 3.524 (1.43), 3.586 (1.24), 3.632 (1.19), 3.651 (1.45), 3.699 (1.27), 3.717 (1.48), 3.732 (1.40), 3.783 (0.90), 3.830 (1.24), 3.848 (0.95), 3.901 (0.85), 4.102 (0.85), 4.396 (1.00), 4.918 (5.32), 4.933 (3.09), 4.989 (1.22), 5.027 (16.00), 5.047 (5.71), 5.086 (1.22), 5.264 (1.19), 5.355 (1.48), 5.447 (1.06), 8.255 (6.88), 8.260 (7.17), 8.559 (6.72).

### Beispiel 289

### (5RS,6RS)-2-[(3,5-Dichlorpyridin-2-yl)methyl]-5-[(3,3-difluorpyrrolidin-1-yl)carbonyl]-6-(trifluormethyl)-5,6,7,8-tetrahydro[1,2,4]triazolo[4,3-a]pyridin-3(2H)-on (Diastereomer 1, Racemat)

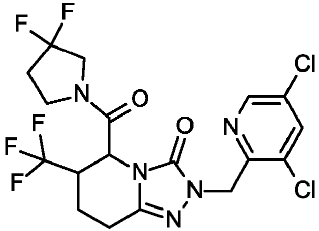

(5RS,6RS)-2-[(3,5-Dichlorpyridin-2-yl)methyl]-3-oxo-6-(trifluormethyl)-2,3,5,6,7,8-hexahydro[1,2,4]triazolo[4,3-a]pyridin-5-carbonsäure (Diastereomerengemisch; 4 Isomere) (50.0 mg, 122 µmol) wurde in THF (2.0 ml) vorgelegt und HBTU (60.0 mg, 158 µmol) und N,N-Diisopropylethylamin (64 µl, 360 µmol) wurden anschließend zugegeben. Nach 15 min Rühren bei Raumtemperatur wurde 3,3-Difluorpyrrolidinhydrochlorid (20.9 mg, 146 µmol) zugeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 43.0 mg (71 % d. Th.) der Titelverbindung erhalten.
LC-MS (Methode 3): Rₜ = 1.75 min; MS (ESIpos): m/z = 500 [M+H]⁺
¹H-NMR (400 MHz, DMSO-d6) δ [ppm]: -0.149 (1.23), -0.008 (10.09), 0.008 (9.95), 0.146 (1.15), 1.939 (1.51), 1.957 (2.16), 1.973 (2.52), 1.991 (2.31), 2.008 (1.59), 2.073 (4.40), 2.328 (2.16), 2.366 (2.52), 2.387 (1.51), 2.416 (1.80), 2.436 (1.73), 2.457 (1.66), 2.573 (2.81), 2.591 (2.45), 2.609 (2.38), 2.638 (2.31), 2.655 (3.10), 2.670 (3.75), 2.710 (2.16), 2.745 (2.81), 2.791 (1.51), 3.406 (2.16), 3.554 (1.66), 3.571 (3.24), 3.588 (3.82), 3.607 (1.59), 3.640 (0.86), 3.675 (3.03), 3.694 (2.16), 3.707 (2.88), 3.720 (2.23), 3.740 (1.73), 3.768 (1.01), 3.801 (2.59), 3.834 (2.31), 3.869 (1.95), 3.909 (1.37), 3.938 (0.72), 4.100 (0.94), 4.119 (2.02), 4.144 (1.87), 4.161 (0.86), 4.365 (0.79), 4.392 (1.30), 4.423 (1.15), 4.453 (0.58), 4.897 (3.82), 4.910 (3.68), 4.948 (4.25), 4.961 (4.18), 4.991 (1.95), 5.031 (16.00), 5.039 (13.91), 5.080 (1.73), 5.489 (0.50), 8.256 (7.86), 8.261 (8.65), 8.557 (9.01), 8.562 (8.86).

### Beispiel 290

### (5RS,6RS)-2-[(3,5-Dichlorpyridin-2-yl)methyl]-5-[(3,3-difluorpyrrolidin-1-yl)carbonyl]-6-(trifluormethyl)-5,6,7,8-tetrahydro[1,2,4]triazolo[4,3-a]pyridin-3(2H)-on (Enantiomer 2)

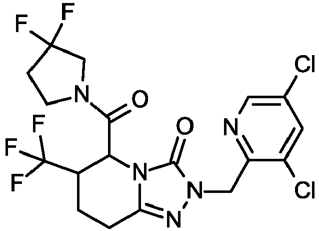

(5RS,6RS)-2-[(3,5-Dichlorpyridin-2-yl)methyl]-5-[(3,3-difluorpyrrolidin-1-yl)carbonyl]-6-(trifluormethyl)-5,6,7,8-tetrahydro[1,2,4]triazolo[4,3-a]pyridin-3(2H)-on (Diastereomer 1, Racemat) wurde durch chirale präparative HPLC getrennt [Probenvorbereitung: 42 mg gelöst in 3.5 ml Ethanol (im Ultraschallbad); Injektionsvolumen: 0.80 ml; Säule: Daicel Chiralcel^{®} OX-H 5 µm, 250 x 20 mm; Laufmittel: Ethanol + 0.2 % Diethylamin; Fluss: 15 ml/min; Temperatur 55°C; UV Detektion: 220 nm]. Nach der Trennung wurden 17.9 mg von Enantiomer 1, welches zuerst eluierte, und 17.0 mg von Enantiomer 2, welches später eluierte, isoliert.

### Enantiomer 2:

Analytische chirale HPLC: Rₜ = 9.89 min, e.e. = 100% [Säule: Daicel Chiralcel^{®} OZ-H-3 250 x 4.6 mm; Laufmittel: n-Heptan/Ethanol 70:30 + 0.2 % Diethylamin; Fluss: 1 ml/min; UV-Detektion: 220 nm].
LC-MS (Methode 3): Rₜ = 1.74 min; MS (ESIpos): m/z = 500 [M+H]⁺
¹H-NMR (400 MHz, DMSO-d6) δ [ppm]: -0.149 (1.99), 0.008 (16.00), 0.146 (2.21), 1.235 (1.40), 1.955 (2.06), 1.974 (2.36), 1.991 (2.36), 2.007 (1.84), 2.057 (2.21), 2.327 (3.24), 2.366 (3.61), 2.573 (2.80), 2.594 (2.29), 2.610 (2.29), 2.637 (2.29), 2.669 (4.87), 2.710 (3.17), 2.742 (2.58), 3.407 (2.14), 3.571 (3.32), 3.588 (3.69), 3.606 (1.55), 3.674 (3.10), 3.707 (2.95), 3.739 (1.84), 3.767 (1.18), 3.800 (2.51), 3.835 (2.43), 3.867 (1.92), 3.909 (1.33), 4.117 (1.92), 4.143 (1.92), 4.395 (1.25), 4.897 (3.47), 4.910 (3.61), 4.947 (4.13), 4.961 (4.13), 4.991 (2.06), 5.030 (15.48), 5.039 (13.20), 5.079 (1.47), 8.256 (8.85), 8.261 (9.00), 8.557 (9.88), 8.562 (9.51).

### Beispiel 291

### (5RS,6RS)-2-[(5-Chlor-3-fluorpyridin-2-yl)methyl]-5-{[(3R,4S)-3,4-difluorpyrrolidin-1-yl]carbonyl}-6-(trifluormethyl)-5,6,7,8-tetrahydro[1,2,4]triazolo[4,3-a]pyridin-3(2H)-on (Diastereomer 1, Racemat)

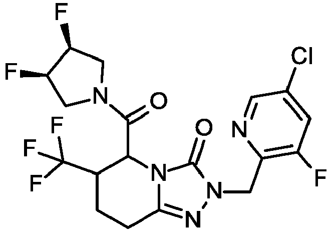

(5RS,6RS)-2-[(5-Chlor-3-fluorpyridin-2-yl)methyl]-3-oxo-6-(trifluormethyl)-2,3,5,6,7,8-hexahydro[1,2,4]triazolo[4,3-a]pyridin-5-carbonsäure (Diastereomerengemisch; 4 Isomere) (50.0 mg, 127 µmol) wurde in THF (2.0 ml) vorgelegt und HBTU (62.5 mg, 165 µmol) und N,N-Diisopropylethylamin (66 µl, 380 µmol) wurden anschließend zugegeben. Nach 15 min Rühren bei Raumtemperatur wurde (3R,4S)-3,4-Difluorpyrrolidinhydrochlorid (21.8 mg, 152 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 45.0 mg (73 % d. Th.) der Titelverbindung erhalten.
LC-MS (Methode 3): Rₜ = 1.56 min; MS (ESIpos): m/z = 484 [M+H]⁺
¹H-NMR (400 MHz, DMSO-d6) δ [ppm]: -0.149 (1.28), -0.008 (16.00), 0.008 (8.30), 0.146 (1.22), 1.903 (1.42), 1.925 (1.69), 1.938 (1.22), 1.961 (1.01), 1.996 (2.90), 2.012 (3.31), 2.038 (1.35), 2.073 (5.00), 2.327 (2.03), 2.366 (2.36), 2.524 (9.18), 2.573 (1.49), 2.597 (1.42), 2.619 (2.16), 2.635 (1.69), 2.665 (2.70), 2.669 (2.84), 2.674 (2.84), 2.710 (3.38), 2.733 (3.31), 2.748 (2.23), 2.776 (1.76), 3.398 (1.55), 3.466 (1.08), 3.501 (1.69), 3.547 (1.96), 3.599 (1.49), 3.626 (1.22), 3.648 (1.69), 3.662 (1.69), 3.696 (1.62), 3.713 (1.42), 3.736 (1.55), 3.758 (1.01), 3.771 (1.01), 3.789 (1.15), 3.802 (1.22), 3.824 (1.62), 3.839 (1.42), 3.854 (1.08), 3.865 (0.88), 3.880 (0.95), 3.893 (1.15), 3.908 (0.95), 4.054 (0.95), 4.091 (0.95), 4.356 (0.88), 4.372 (1.08), 4.384 (1.15), 4.396 (1.35), 4.410 (1.01), 4.421 (0.88), 4.437 (0.88), 4.902 (5.20), 4.909 (6.01), 4.925 (4.39), 4.940 (1.62), 4.979 (13.37), 4.998 (4.93), 5.002 (4.59), 5.037 (1.28), 5.086 (0.47), 5.260 (1.49), 5.269 (1.49), 5.362 (1.89), 5.392 (1.62), 5.401 (1.62), 5.451 (1.22), 5.480 (1.22), 8.098 (5.60), 8.103 (5.74), 8.122 (5.67), 8.127 (5.60), 8.476 (6.35).

### Beispiel 292

### (5RS,6RS)-2-[(5-Chlor-3-fluorpyridin-2-yl)methyl]-5-{[(3R,4S)-3,4-difluorpyrrolidin-1-yl]carbonyl}-6-(trifluormethyl)-5,6,7,8-tetrahydro[1,2,4]triazolo[4,3-a]pyridin-3(2H)-on Enantiomer 1)

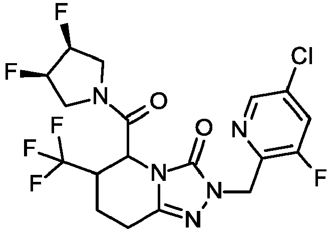

(5RS,6RS)-2-[(5-Chlor-3-fluorpyridin-2-yl)methyl]-5-{[(3R,4S)-3,4-difluorpyrrolidin-1-yl]carbonyl}-6-(trifluormethyl)-5,6,7,8-tetrahydro[1,2,4]triazolo[4,3-a]pyridin-3(2H)-on (Diastereomer 1, Racemat) wurde durch chirale präparative HPLC getrennt [Probenvorbereitung: 44 mg gelöst in 2 ml Acetonitril/Ethanol (1:1) + 0.2% Diethylamin; Injektionsvolumen: 0.15 ml; Säule: Daicel Chiralcel^{®} IB 5 µm, 250 x 20 mm; Laufmittel: n-Heptan/Ethanol 50:50; Fluss: 20 ml/min; Temperatur 30°C; UV-Detektion: 220 nm]. Nach der Trennung wurden 9.9 mg von Enantiomer 1, welches zuerst eluierte, und 11.9 mg von Enantiomer 2, welches später eluierte, isoliert.

### Enantiomer 1:

Analytische chirale HPLC: Rₜ = 1.66 min, e.e. = 100% [Säule: Daicel Chiralcel^{®} IE-3 50 x 4.6 mm; Laufmittel: n-Heptan/Ethanol 50:50 + 0.2% Diethylamin; Fluss: 1 ml/min; UV-Detektion: 220 nm].
LC-MS (Methode 3): Rₜ = 1.55 min; MS (ESIpos): m/z = 484 [M+H]⁺
¹H-NMR (400 MHz, DMSO-d6) δ [ppm]: -0.149 (1.91), 0.008 (16.00), 0.146 (1.91), 1.109 (0.41), 1.236 (0.81), 1.904 (0.93), 1.926 (1.04), 1.961 (0.64), 1.997 (1.86), 2.013 (2.20), 2.057 (0.99), 2.327 (1.86), 2.366 (1.86), 2.596 (0.87), 2.620 (1.33), 2.635 (1.10), 2.670 (2.38), 2.710 (2.55), 2.734 (2.14), 2.749 (1.39), 2.777 (1.22), 3.269 (0.70), 3.399 (0.87), 3.468 (0.58), 3.501 (1.04), 3.556 (1.10), 3.600 (0.93), 3.628 (0.75), 3.650 (1.04), 3.663 (1.10), 3.698 (1.04), 3.713 (0.87), 3.736 (0.93), 3.770 (0.64), 3.790 (0.75), 3.826 (0.99), 3.840 (0.87), 3.854 (0.64), 3.881 (0.58), 3.895 (0.64), 3.908 (0.58), 4.054 (0.64), 4.100 (0.58), 4.356 (0.46), 4.372 (0.70), 4.398 (0.87), 4.410 (0.70), 4.438 (0.52), 4.903 (3.19), 4.909 (4.06), 4.925 (2.84), 4.941 (0.87), 4.979 (9.10), 5.002 (3.19), 5.037 (0.64), 5.260 (0.99), 5.362 (1.16), 5.401 (1.04), 5.481 (0.81), 8.098 (3.36), 8.103 (3.59), 8.122 (3.65), 8.127 (3.54), 8.477 (4.29).

### Beispiel 293

### (5RS,6RS)-2-[(5-Chlor-3-fluorpyridin-2-yl)methyl]-5-{[(3R,4S)-3,4-difluorpyrrolidin-1-yl]carbonyl}-6-(trifluormethyl)-5,6,7,8-tetrahydro[1,2,4]triazolo[4,3-a]pyridin-3(2H)-on (Enantiomer 2)

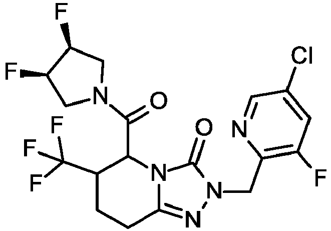

(5RS,6RS)-2-[(5-Chlor-3-fluorpyridin-2-yl)methyl]-5-{[(3R,4S)-3,4-difluorpyrrolidin-1-yl]carbonyl}-6-(trifluormethyl)-5,6,7,8-tetrahydro[1,2,4]triazolo[4,3-a]pyridin-3(2H)-on (Diastereomer 1, Racemat) wurde durch chirale präparative HPLC getrennt [Probenvorbereitung: 44 mg gelöst in 2 ml Acetonitril/Ethanol (1:1) + 0.2% Diethylamin; Injektionsvolumen: 0.15 ml; Säule: Daicel Chiralcel^{®} IB 5 µm, 250 x 20 mm; Laufmittel: n-Heptan/Ethanol 50:50; Fluss: 20 ml/min; Temperatur 30°C; UV-Detektion: 220 nm]. Nach der Trennung wurden 9.9 mg von Eantiomer 1, welches zuerst eluierte, und 11.9 mg von Enantiomer 2, welches später eluierte, isoliert.

### Enantiomer 2:

Analytische chirale HPLC: Rₜ = 1.92 min, e.e. = 97% [Säule: Daicel Chiralcel^{®} IE-3 50 x 4.6 mm; Laufmittel: n-Heptan/Ethanol 50:50 + 0.2% Diethylamin; Fluss: 1 ml/min; UV-Detektion: 220 nm].
LC-MS (Methode 3): Rₜ = 1.55 min; MS (ESIpos): m/z = 484 [M+H]⁺
¹H-NMR (400 MHz, DMSO-d6) δ [ppm]: -0.149 (1.80), -0.008 (16.00), 0.008 (14.88), 0.146 (1.80), 1.074 (1.91), 1.091 (3.99), 1.110 (2.08), 1.233 (0.90), 1.904 (1.12), 1.925 (1.46), 1.961 (0.90), 1.997 (2.30), 2.013 (2.75), 2.057 (1.12), 2.327 (1.91), 2.366 (2.36), 2.599 (1.35), 2.619 (1.80), 2.637 (1.40), 2.669 (2.81), 2.710 (3.31), 2.734 (3.14), 2.750 (2.41), 2.776 (2.25), 3.466 (0.95), 3.510 (1.35), 3.556 (1.52), 3.599 (1.35), 3.624 (1.12), 3.649 (1.35), 3.662 (1.40), 3.697 (1.35), 3.713 (1.18), 3.736 (1.35), 3.759 (0.90), 3.770 (0.90), 3.790 (0.90), 3.802 (0.95), 3.825 (1.40), 3.840 (1.18), 3.894 (0.95), 3.908 (0.84), 4.054 (0.79), 4.092 (0.79), 4.356 (0.84), 4.396 (1.07), 4.903 (4.15), 4.912 (5.16), 4.925 (3.59), 4.945 (1.07), 4.979 (11.51), 4.998 (4.10), 5.038 (0.95), 5.270 (1.35), 5.363 (1.52), 5.402 (1.40), 5.481 (1.07), 8.098 (4.94), 8.103 (5.16), 8.122 (5.00), 8.127 (5.33), 8.478 (7.02).

### Beispiel 294

### (5RS,6RS)-2-[(5-Chlor-3-fluorpyridin-2-yl)methyl]-5-{[(3S)-3-fluorpyrrolidin-1-yl]carbonyl}-6-(trifluormethyl)-5,6,7,8-tetrahydro[1,2,4]triazolo[4,3-a]pyridin-3(2H)-on (Diastereomerengemisch; 2 Isomere)

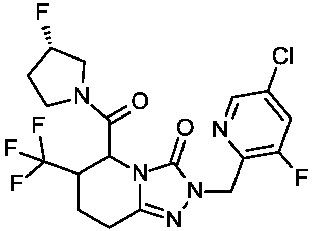

(5RS,6RS)-2-[(5-Chlor-3-fluorpyridin-2-yl)methyl]-3-oxo-6-(trifluormethyl)-2,3,5,6,7,8-hexahydro[1,2,4]triazolo[4,3-a]pyridin-5-carbonsäure (Diastereomerengemisch; 4 Isomere) (50.0 mg, 127 µmol) wurde in THF (2.0 ml) vorgelegt und HBTU (62.5 mg, 165 µmol) und N,N-Diisopropylethylamin (66 µl, 380 µmol) wurden anschließend zugegeben. Nach 15 min Rühren bei Raumtemperatur wurde (3S)-3-Fluorpyrrolidinhydrochlorid (19.1 mg, 152 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 39.0 mg (66 % d. Th.) der Titelverbindung erhalten.
LC-MS (Methode 3): Rₜ = 1.50 min; MS (ESIpos): m/z = 466 [M+H]⁺
¹H-NMR (400 MHz, DMSO-d6) δ [ppm]: -0.149 (0.51), -0.008 (4.55), 0.008 (4.09), 0.146 (0.51), 1.250 (1.15), 1.257 (0.72), 1.266 (1.29), 1.271 (1.23), 1.288 (1.07), 1.899 (0.63), 1.913 (1.04), 1.933 (1.73), 1.954 (1.76), 1.968 (1.44), 1.994 (1.69), 2.010 (2.01), 2.018 (2.34), 2.033 (2.50), 2.047 (2.01), 2.068 (1.69), 2.082 (1.73), 2.107 (1.96), 2.148 (1.23), 2.228 (0.88), 2.242 (1.23), 2.288 (1.15), 2.327 (1.18), 2.366 (0.76), 2.524 (2.43), 2.570 (0.90), 2.592 (1.07), 2.614 (1.67), 2.670 (1.94), 2.690 (5.80), 2.710 (1.96), 2.728 (2.56), 2.741 (1.94), 2.770 (1.29), 2.783 (1.02), 3.269 (0.62), 3.298 (1.46), 3.315 (1.66), 3.333 (1.37), 3.343 (1.18), 3.362 (1.53), 3.378 (1.67), 3.391 (1.22), 3.409 (1.37), 3.471 (1.22), 3.498 (1.43), 3.528 (2.82), 3.550 (1.25), 3.565 (1.97), 3.619 (16.00), 3.663 (12.83), 3.793 (1.11), 3.802 (1.18), 3.819 (1.13), 3.845 (0.62), 4.006 (0.48), 4.038 (0.42), 4.090 (0.88), 4.123 (1.48), 4.146 (1.83), 4.170 (0.81), 4.809 (1.87), 4.825 (1.80), 4.854 (2.38), 4.864 (2.40), 4.874 (1.99), 4.884 (1.90), 4.922 (2.36), 4.937 (3.28), 4.976 (11.24), 4.997 (4.44), 5.018 (0.83), 5.034 (1.25), 5.278 (1.60), 5.379 (0.90), 5.411 (1.94), 5.511 (0.78), 5.533 (0.74), 8.097 (5.67), 8.102 (6.01), 8.121 (5.76), 8.126 (6.04), 8.478 (7.30).

### Beispiel 295

### (5RS,6RS)-2-[(5-Chlor-3-fluorpyridin-2-yl)methyl]-5-{[(3S)-3-fluorpyrrolidin-1-yl]carbonyl}-6-(trifluormethyl)-5,6,7,8-tetrahydro[1,2,4]triazolo[4,3-a]pyridin-3(2H)-on (Isomer 1)

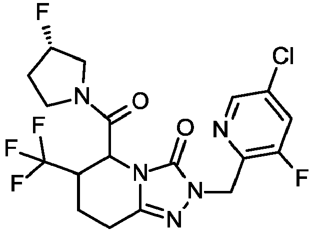

### (5RS,6RS)-2-[(5-Chlor-3-fluorpyridin-2-yl)methyl]-5-{[(3S)-3-fluorpyrrolidin-1-yl]carbonyl}-6-(trifluormethyl)-5,6,7,8-tetrahydro[1,2,4]triazolo[4,3-a]pyridin-3(2H)-on

(Diastereomerengemisch; 2 Isomere) wurde durch chirale präparative HPLC getrennt [Probenvorbereitung: 38 mg gelöst in 2 ml Acetonitril/Ethanol (1:1); Injektionsvolumen: 0.15 ml; Säule: Daicel Chiralcel^{®} ID 5 µm, 250 x 20 mm; Laufmittel: n-Heptan/Ethanol 35:65 + 0.2% Diethylamin; Fluss: 20 ml/min; Temperatur 25°C; UV Detektion: 220 nm]. Nach der Trennung wurden 11.3 mg von Isomer 1, welches zuerst eluierte, und 13.0 mg von Isomer 2, welches später eluierte, isoliert.

### Isomer 1:

Analytische chirale HPLC: Rₜ = 1.57 min, d.e. = 100% [Säule: Daicel Chiralcel^{®} ID-3 50 x 4.6 mm; Laufmittel: n-Heptan/Ethanol 50:50 + 0.2% Diethylamin; Fluss: 1 ml/min; UV Detektion: 220 nm].
LC-MS (Methode 3): Rₜ = 1.50 min; MS (ESIpos): m/z = 466 [M+H]⁺
¹H-NMR (400 MHz, DMSO-d6) δ [ppm]: -0.149 (1.91), -0.008 (16.00), 0.008 (14.39), 0.146 (1.76), 0.866 (0.88), 0.886 (1.17), 0.904 (0.73), 1.233 (1.61), 1.912 (1.76), 1.933 (2.20), 1.947 (1.91), 2.003 (1.76), 2.018 (4.40), 2.033 (5.14), 2.108 (3.08), 2.242 (1.91), 2.290 (1.61), 2.327 (4.11), 2.366 (3.96), 2.523 (9.39), 2.569 (3.23), 2.595 (2.94), 2.614 (3.23), 2.670 (6.75), 2.710 (5.72), 2.738 (3.96), 2.782 (2.35), 3.366 (2.50), 3.411 (2.35), 3.471 (3.08), 3.497 (2.50), 3.662 (5.87), 3.684 (3.52), 3.717 (3.96), 3.740 (4.55), 3.802 (2.50), 3.819 (2.79), 3.846 (1.32), 3.998 (1.76), 4.039 (1.32), 4.056 (0.73), 4.091 (1.47), 4.124 (1.17), 4.155 (0.88), 4.809 (5.58), 4.824 (5.58), 4.854 (7.05), 4.864 (7.05), 4.932 (2.79), 4.971 (14.39), 4.975 (14.39), 4.996 (7.49), 5.000 (7.49), 5.015 (1.32), 5.039 (2.50), 5.277 (2.79), 5.410 (3.67), 5.532 (1.91), 8.097 (7.93), 8.102 (8.37), 8.121 (8.22), 8.126 (8.51), 8.478 (8.51).

### Beispiel 296

### (5RS,6RS)-2-[(5-Chlor-3-fluorpyridin-2-yl)methyl]-5-[(3,3-difluorpyrrolidin-1-yl)carbonyl]-6-(trifluormethyl)-5,6,7,8-tetrahydro[1,2,4]triazolo[4,3-a]pyridin-3(2H)-on (Diastereomer 1, Racemat)

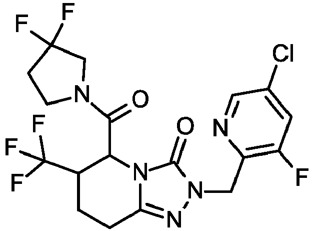

(5RS,6RS)-2-[(5-Chlor-3-fluorpyridin-2-yl)methyl]-3-oxo-6-(trifluormethyl)-2,3,5,6,7,8-hexahydro[1,2,4]triazolo[4,3-a]pyridin-5-carbonsäure (Diastereomerengemisch; 4 Isomere) (50.0 mg, 127 µmol) wurde in THF (1.6 ml) vorgelegt und HBTU (62.5 mg, 165 µmol) und N,N-Diisopropylethylamin (66 µl, 380 µmol) wurden anschließend zugegeben. Nach 15 min Rühren bei Raumtemperatur wurde 3,3-Difluorpyrrolidinhydrochlorid (21.8 mg, 152 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 48.0 mg (78 % d. Th.) der Titelverbindung erhalten.
LC-MS (Methode 4): Rₜ = 0.86 min; MS (ESIpos): m/z = 484 [M+H]⁺
¹H-NMR (400 MHz, DMSO-d6) δ [ppm]: -0.149 (0.59), -0.008 (4.82), 0.008 (4.82), 0.146 (0.57), 1.253 (13.91), 1.269 (16.00), 1.279 (10.17), 1.294 (8.34), 1.928 (0.59), 1.947 (0.89), 1.963 (0.97), 1.981 (0.93), 1.997 (0.65), 2.049 (0.91), 2.073 (2.59), 2.328 (0.75), 2.367 (0.93), 2.389 (0.67), 2.409 (0.63), 2.419 (0.79), 2.439 (0.73), 2.460 (0.69), 2.523 (2.71), 2.576 (1.07), 2.591 (1.01), 2.608 (0.89), 2.632 (0.93), 2.649 (1.24), 2.665 (1.24), 2.670 (1.22), 2.711 (1.01), 2.736 (1.15), 2.781 (0.61), 3.114 (1.28), 3.131 (1.44), 3.143 (1.30), 3.160 (0.43), 3.539 (0.41), 3.552 (0.67), 3.570 (1.66), 3.591 (1.92), 3.603 (1.62), 3.613 (1.76), 3.630 (1.07), 3.672 (1.24), 3.688 (0.97), 3.707 (1.52), 3.733 (0.55), 3.771 (0.47), 3.805 (1.15), 3.838 (1.03), 3.863 (0.67), 3.880 (0.55), 3.904 (0.59), 4.100 (0.43), 4.118 (0.83), 4.144 (0.81), 4.390 (0.49), 4.422 (0.45), 4.888 (1.58), 4.902 (1.56), 4.940 (2.21), 4.953 (1.96), 4.984 (4.23), 4.992 (4.19), 5.031 (0.67), 7.050 (1.40), 7.177 (1.40), 7.304 (1.38), 8.099 (2.31), 8.104 (2.47), 8.123 (2.35), 8.128 (2.53), 8.477 (2.98), 8.481 (2.84).

### Beispiel 297

### (5RS,6RS)-2-[(5-Chlor-3-fluorpyridin-2-yl)methyl]-5-[(3,3-difluorpyrrolidin-1-yl)carbonyl]-6-(trifluormethyl)-5,6,7,8-tetrahydro[1,2,4]triazolo[4,3-a]pyridin-3(2H)-on (Enantiomer 2)

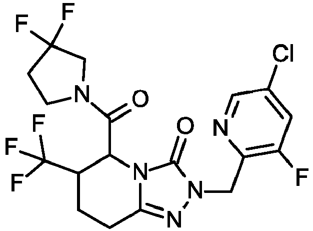

(5RS,6RS)-2-[(5-Chlor-3-fluorpyridin-2-yl)methyl]-5-[(3,3-difluorpyrrolidin-1-yl)carbonyl]-6-(trifluormethyl)-5,6,7,8-tetrahydro[1,2,4]triazolo[4,3-a]pyridin-3(2H)-on (Diastereomer 1, Racemat) wurde durch chirale präparative HPLC getrennt [Probenvorbereitung: 48 mg gelöst in 2 ml Acetonitril/Methanol (1:1); Injektionsvolumen: 0.20 ml; Säule: Daicel Chiralcel^{®} IC 5 µm, 250 x 20 mm; Laufmittel: n-Heptan/Ethanol 50:50; Fluss: 20 ml/min; Temperatur 30°C; UV-Detektion: 220 nm]. Nach der Trennung wurden 13.9 mg von Enantiomer 1, welches zuerst eluierte, und 16.2 mg von Enantiomer 2, welches später eluierte, isoliert.

### Enantiomer 2:

Analytische chirale HPLC: Rₜ = 1.67 min, e.e. = 100% [Säule: Daicel Chiralcel^{®} IC-3 50 x 4.6 mm; Laufmittel: n-Heptan/Ethanol 50:50; Fluss: 1 ml/min; UV-Detektion: 220 nm].
LC-MS (Methode 3): Rₜ = 1.64 min; MS (ESIpos): m/z = 484 [M+H]⁺
¹H-NMR (400 MHz, DMSO-d6) δ [ppm]: -0.150 (3.80), 0.146 (2.40), 0.866 (1.00), 0.885 (1.40), 0.904 (0.80), 1.235 (1.00), 1.313 (0.80), 1.946 (1.80), 1.961 (1.80), 1.980 (1.80), 1.998 (1.60), 2.064 (2.20), 2.327 (12.60), 2.365 (16.00), 2.590 (3.00), 2.605 (2.80), 2.626 (2.60), 2.669 (6.80), 2.709 (7.60), 2.736 (2.00), 2.781 (1.20), 3.397 (2.60), 3.569 (2.20), 3.591 (2.00), 3.611 (1.40), 3.640 (1.00), 3.672 (2.00), 3.706 (2.60), 3.773 (1.00), 3.804 (1.80), 3.836 (1.80), 3.861 (1.40), 3.903 (1.20), 3.998 (1.20), 4.117 (1.40), 4.143 (1.40), 4.391 (1.20), 4.415 (1.00), 4.888 (2.60), 4.902 (2.60), 4.941 (3.80), 4.953 (2.60), 4.985 (6.80), 5.031 (1.00), 8.103 (3.00), 8.127 (3.20), 8.482 (3.40).

### Beispiel 298

### (5RS,6RS)-2-[(5-Chlorpyridin-2-yl)methyl]-5-{[(3S)-3-fluorpyrrolidin-1-yl]carbonyl}-6-(trifluormethyl)-5,6,7,8-tetrahydro[1,2,4]triazolo[4,3-a]pyridin-3(2H)-on (Diastereomerengemisch; 2 Isomere)

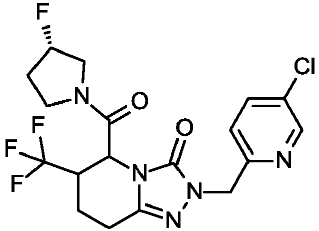

(5RS,6RS)-2-[(5-Chlorpyridin-2-yl)methyl]-3-oxo-6-(trifluormethyl)-2,3,5,6,7,8-hexahydro[1,2,4]triazolo[4,3-a]pyridin-5-carbonsäure (Diastereomerengemisch; 4 Isomere) (47.0 mg, 125 µmol) wurde in THF (2.0 ml) vorgelegt und HBTU (61.5 mg, 162 µmol) und N,N-Diisopropylethylamin (65 µl, 370 µmol) wurden anschließend zugegeben. Nach 15 min Rühren bei Raumtemperatur wurde (3S)-3-Fluorpyrrolidinhydrochlorid (18.8 mg, 150 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 31.0 mg (55 % d. Th.) der Titelverbindung erhalten.
LC-MS (Methode 3): Rₜ = 1.44 min; MS (ESIpos): m/z = 448 [M+H]⁺
¹H-NMR (400 MHz, DMSO-d6) δ [ppm]: -0.149 (1.01), -0.008 (8.66), 0.008 (8.56), 0.146 (1.01), 1.385 (1.47), 1.946 (1.67), 1.968 (2.28), 2.007 (1.67), 2.045 (3.59), 2.060 (3.29), 2.073 (3.14), 2.110 (2.53), 2.144 (1.72), 2.245 (1.52), 2.290 (1.42), 2.328 (2.58), 2.366 (1.87), 2.611 (1.06), 2.669 (3.29), 2.710 (2.73), 2.762 (2.84), 2.805 (1.42), 3.369 (2.28), 3.468 (1.16), 3.509 (1.57), 3.535 (3.34), 3.562 (1.22), 3.615 (1.82), 3.648 (1.57), 3.670 (2.58), 3.740 (1.87), 3.769 (1.42), 3.805 (0.96), 3.824 (1.06), 4.099 (0.96), 4.126 (1.52), 4.150 (1.97), 4.175 (0.86), 4.831 (2.08), 4.847 (2.13), 4.883 (2.94), 4.893 (3.85), 4.915 (6.73), 4.923 (16.00), 4.934 (14.53), 4.954 (3.80), 4.969 (3.24), 4.994 (0.61), 5.142 (0.61), 5.279 (1.82), 5.411 (2.18), 5.513 (0.96), 5.947 (0.51), 7.206 (4.66), 7.218 (3.29), 7.227 (7.80), 7.239 (3.34), 7.249 (3.09), 7.920 (5.57), 7.926 (6.38), 7.941 (5.42), 7.947 (6.08), 8.574 (9.52), 8.580 (8.46).

### Beispiel 299

### (5RS,6RS)-2-[(5-Chlorpyridin-2-yl)methyl]-5-{[(3S)-3-fluorpyrrolidin-1-yl]carbonyl}-6-(trifluormethyl)-5,6,7,8-tetrahydro[1,2,4]triazolo[4,3-a]pyridin-3(2H)-on (Isomer 2)

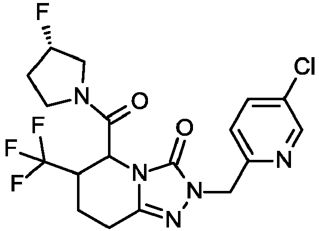

### (5RS,6RS)-2-[(5-Chlorpyridin-2-yl)methyl]-5-{[(3S)-3-fluorpyrrolidin-1-yl]carbonyl}-6-(trifluormethyl)-5,6,7,8-tetrahydro[1,2,4]triazolo[4,3-a]pyridin-3(2H)-on

(Diastereomerengemisch; 2 Isomere) wurde durch chirale präparative HPLC getrennt [Probenvorbereitung: 30 mg gelöst in 3 ml Methanol/tertButylmethylether (2:1); Injektionsvolumen: 1.00 ml; Säule: Daicel Chiralcel^{®} IB 5 µm, 250 x 20 mm; Laufmittel: Methanol/tert-Butylmethylether 10:90; Fluss: 20 ml/min; Temperatur 30°C; UV Detektion: 270 nm]. Nach der Trennung wurden 11.1 mg von Isomer 1, welches zuerst eluierte, und 11.3 mg von Isomer 2, welches später eluierte, isoliert.

### Isomer 2:

Analytische chirale HPLC: Rₜ = 6.44 min, d.e. = 100% [Säule: Daicel Chiralcel^{®} IB 50 x 4.6 mm; Laufmittel: Methanol/tert-Butylmethylether 10:90; Fluss: 1 ml/min; UV Detektion: 220 nm].
LC-MS (Methode 4): Rₜ = 0.77 min; MS (ESIpos): m/z = 448 [M+H]⁺
¹H-NMR (400 MHz, DMSO-d6) δ [ppm]: -0.149 (1.82), -0.008 (16.00), 0.008 (15.36), 0.146 (1.87), 1.186 (8.95), 1.427 (2.97), 1.946 (1.36), 1.968 (1.36), 1.981 (1.15), 2.030 (1.40), 2.046 (3.27), 2.060 (3.18), 2.077 (1.91), 2.110 (1.82), 2.126 (1.53), 2.146 (1.49), 2.257 (1.10), 2.292 (1.02), 2.327 (1.49), 2.366 (1.19), 2.524 (2.97), 2.592 (0.64), 2.610 (0.98), 2.633 (1.06), 2.655 (1.87), 2.670 (1.91), 2.689 (0.89), 2.710 (1.78), 2.760 (1.82), 2.775 (2.08), 2.803 (1.02), 2.819 (1.23), 3.370 (1.02), 3.406 (0.98), 3.416 (1.02), 3.469 (1.40), 3.504 (1.70), 3.648 (1.78), 3.670 (3.44), 3.705 (1.70), 3.726 (1.53), 3.744 (2.04), 3.767 (1.32), 3.805 (1.36), 3.824 (1.36), 3.849 (0.59), 4.013 (0.72), 4.036 (0.59), 4.107 (0.68), 4.131 (0.64), 4.831 (3.10), 4.846 (3.06), 4.883 (4.20), 4.892 (4.37), 4.915 (6.49), 4.921 (6.88), 4.932 (11.50), 4.946 (2.38), 4.962 (0.51), 4.974 (0.42), 5.278 (1.53), 5.409 (1.99), 5.532 (1.06), 7.207 (4.20), 7.228 (7.60), 7.249 (3.44), 7.922 (4.12), 7.928 (4.07), 7.943 (3.95), 7.949 (3.90), 8.573 (5.26), 8.579 (5.22).

### Beispiel 300

### (5RS,6RS)-2-[(5-Chlorpyridin-2-yl)methyl]-5-{[(3R,4S)-3,4-difluorpyrrolidin-1-yl]carbonyl}-6-(trifluormethyl)-5,6,7,8-tetrahydro[1,2,4]triazolo[4,3-a]pyridin-3(2H)-on (Diastereomer 1; Racemat)

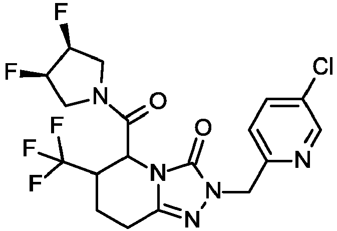

(5RS,6RS)-2-[(5-Chlorpyridin-2-yl)methyl]-3-oxo-6-(trifluormethyl)-2,3,5,6,7,8-hexahydro[1,2,4]triazolo[4,3-a]pyridin-5-carbonsäure (Diastereomerengemisch; 4 Isomere) (47.0 mg, 125 µmol) wurde in THF (2.0 ml) vorgelegt und HBTU (61.5 mg, 162 µmol) und N,N-Diisopropylethylamin (65 µl, 370 µmol) wurden anschließend zugegeben. Nach 15 min Rühren bei Raumtemperatur wurde (3R,4S)-3,4-Difluorpyrrolidinhydrochlorid (21.5 mg, 150 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 34.0 mg (59 % d. Th.) der Titelverbindung erhalten.
LC-MS (Methode 3): Rₜ = 1.50 min; MS (ESIpos): m/z = 466 [M+H]⁺
¹H-NMR (400 MHz, DMSO-d6) δ [ppm]: -0.149 (0.67), -0.008 (6.04), 0.008 (5.59), 0.146 (0.67), 1.924 (0.61), 1.938 (1.03), 1.960 (1.34), 1.973 (0.97), 2.006 (1.18), 2.021 (2.58), 2.038 (2.61), 2.053 (1.58), 2.067 (0.94), 2.327 (1.21), 2.366 (1.34), 2.523 (3.25), 2.593 (0.61), 2.613 (0.91), 2.635 (0.91), 2.655 (1.67), 2.669 (2.16), 2.696 (0.94), 2.709 (2.58), 2.725 (0.79), 2.767 (2.37), 2.782 (1.58), 2.811 (1.28), 2.825 (0.70), 3.399 (0.97), 3.411 (1.00), 3.423 (1.00), 3.436 (0.97), 3.473 (0.88), 3.508 (1.28), 3.553 (1.43), 3.563 (1.40), 3.592 (1.15), 3.604 (1.15), 3.633 (0.88), 3.653 (1.24), 3.667 (1.18), 3.686 (0.76), 3.702 (1.21), 3.718 (1.03), 3.727 (1.03), 3.738 (1.31), 3.761 (0.85), 3.774 (0.91), 3.792 (0.85), 3.805 (0.82), 3.828 (1.06), 3.844 (0.85), 3.859 (0.76), 3.871 (0.61), 3.885 (0.64), 3.900 (0.76), 3.914 (0.76), 4.060 (0.67), 4.098 (0.67), 4.365 (0.70), 4.380 (0.76), 4.392 (0.76), 4.403 (0.85), 4.418 (0.76), 4.430 (0.70), 4.446 (0.64), 4.903 (1.03), 4.923 (11.69), 4.935 (16.00), 4.944 (4.98), 4.950 (4.43), 4.978 (0.67), 5.120 (0.49), 5.134 (0.43), 5.244 (0.97), 5.256 (1.09), 5.265 (1.28), 5.273 (1.00), 5.311 (0.76), 5.322 (0.91), 5.358 (1.34), 5.389 (1.09), 5.397 (1.09), 5.462 (0.91), 5.478 (0.91), 7.210 (3.28), 7.225 (3.89), 7.231 (3.83), 7.245 (4.01), 7.923 (3.73), 7.930 (3.58), 7.945 (3.64), 7.948 (3.46), 8.573 (4.61), 8.579 (4.37).

### Beispiel 301

### (5RS,6RS)-2-[(5-Chlorpyridin-2-yl)methyl]-5-{[(3R,4S)-3,4-difluorpyrrolidin-1-yl]carbonyl}-6-(trifluormethyl)-5,6,7,8-tetrahydro[1,2,4]triazolo[4,3-a]pyridin-3(2H)-on (Enantiomer 2)

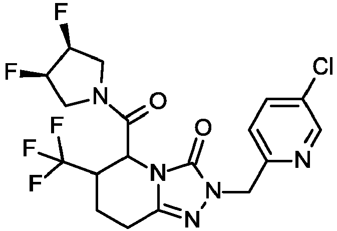

(5RS,6RS)-2-[(5-Chlorpyridin-2-yl)methyl]-3-oxo-6-(trifluormethyl)-2,3,5,6,7,8-hexahydro[1,2,4]triazolo[4,3-a]pyridin-5-carbonsäure (Diastereomer 1; Racemat) wurde durch chirale präparative HPLC getrennt [Probenvorbereitung: 34 mg gelöst in 3 ml Methanol/tert-Butylmethylether (3:1); Injektionsvolumen: 1.00 ml; Säule: Daicel Chiralcel^{®} IB 5 µm, 250 x 20 mm; Laufmittel: Methanol/tertButylmethylether 10:90; Fluss: 15 ml/min; Temperatur 30°C; UV-Detektion: 270 nm]. Nach der Trennung wurden 15.3 mg von Enantiomer 1, welches zuerst eluierte, und 14.3mg von Enantiomer 2, welches später eluierte, isoliert.

### Enantiomer 2:

Analytische chirale HPLC: Rₜ = 5.58 min, e.e. = 100% [Säule: Daicel Chiralcel^{®} IB 50 x 4.6 mm; Laufmittel: Methanol/tert-Butylmethylether 10:90; Fluss: 1 ml/min; UV-Detektion: 220 nm].
LC-MS (Methode 4): Rₜ = 0.80 min; MS (ESIpos): m/z = 466 [M+H]⁺
¹H-NMR (400 MHz, DMSO-d6) δ [ppm]: -0.149 (1.57), -0.008 (13.32), 0.008 (12.83), 0.146 (1.53), 1.186 (13.39), 1.427 (0.94), 1.925 (0.52), 1.938 (0.87), 1.960 (1.15), 1.973 (0.80), 2.007 (1.08), 2.022 (2.54), 2.038 (2.57), 2.053 (1.57), 2.078 (0.97), 2.327 (1.11), 2.366 (0.90), 2.593 (0.56), 2.612 (0.90), 2.636 (0.90), 2.655 (1.60), 2.670 (2.02), 2.695 (0.90), 2.710 (2.26), 2.769 (2.19), 2.783 (1.43), 2.812 (1.18), 3.423 (0.97), 3.437 (0.90), 3.472 (0.80), 3.508 (1.18), 3.555 (1.25), 3.593 (1.04), 3.632 (0.87), 3.653 (1.18), 3.668 (1.18), 3.687 (0.70), 3.702 (1.11), 3.718 (0.94), 3.727 (0.80), 3.739 (1.22), 3.761 (0.63), 3.774 (0.70), 3.793 (0.80), 3.805 (0.80), 3.828 (1.01), 3.846 (0.80), 3.860 (0.73), 3.885 (0.63), 3.900 (0.80), 3.914 (0.63), 4.061 (0.63), 4.098 (0.66), 4.367 (0.56), 4.380 (0.70), 4.392 (0.70), 4.408 (0.87), 4.418 (0.73), 4.430 (0.70), 4.446 (0.56), 4.923 (11.65), 4.935 (16.00), 4.946 (6.99), 5.244 (0.90), 5.256 (0.97), 5.266 (1.08), 5.322 (0.77), 5.358 (1.25), 5.389 (0.97), 5.440 (0.66), 5.463 (0.87), 5.479 (0.83), 7.211 (3.41), 7.225 (3.58), 7.231 (3.90), 7.246 (3.62), 7.924 (3.44), 7.927 (3.41), 7.930 (3.34), 7.945 (3.27), 7.949 (3.23), 7.951 (3.20), 8.573 (5.39), 8.579 (5.25).

### Beispiel 302

### (5RS,6RS)-5-[(3-Fluorazetidin-1-yl)carbonyl]-2-{[3-fluor-2-(trifluormethyl)pyridin-4-yl]methyl}-6-(trifluormethyl)-5,6,7,8-tetrahydro[1,2,4]triazolo[4,3-a]pyridin-3(2H)-on (Diastereomer 1, Racemat)

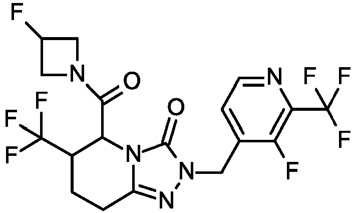

(5RS,6RS)-2-{[3-Fluor-2-(trifluormethyl)pyridin-4-yl]methyl}-3-oxo-6-(trifluormethyl)-2,3,5,6,7,8-hexahydro[1,2,4]triazolo[4,3-a]pyridin-5-carbonsäure (Diastereomerengemisch; 4 Isomere) (96.0 mg, 224 µmol) wurde in THF (2.0 ml) vorgelegt und HBTU (111 mg, 291 µmol) und N,N-Diisopropylethylamin (120 µl, 670 µmol) wurden anschließend zugegeben. Nach 15 min Rühren bei Raumtemperatur wurde 3-Fluorazetidinhydrochlorid (30.0 mg, 269 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 25.0 mg (23 % d. Th.) der Titelverbindung erhalten.
LC-MS (Methode 3): Rₜ = 1.61 min; MS (ESIpos): m/z = 486 [M+H]⁺
¹H-NMR (400 MHz, DMSO-d6) δ [ppm]: -0.149 (1.95), -0.008 (16.00), 0.008 (14.87), 0.146 (2.03), 1.995 (1.95), 2.073 (2.18), 2.327 (3.15), 2.366 (3.00), 2.523 (7.21), 2.670 (3.68), 2.710 (3.31), 2.767 (2.18), 2.783 (1.20), 2.812 (1.05), 3.379 (1.13), 3.973 (1.13), 4.000 (1.20), 4.032 (1.05), 4.229 (1.13), 4.252 (1.43), 4.560 (0.90), 4.654 (2.85), 4.666 (4.36), 4.677 (2.63), 4.867 (0.68), 5.086 (6.91), 5.100 (5.93), 5.371 (0.83), 5.560 (0.90), 7.566 (2.70), 7.579 (5.18), 7.592 (2.70), 8.571 (5.18), 8.583 (5.11).

### Beispiel 303

### (5RS,6RS)-5-[(3-Fluorazetidin-1-yl)carbonyl]-2-{[3-fluor-2-(trifluormethyl)pyridin-4-yl]methyl}-6-(trifluormethyl)-5,6,7,8-tetrahydro[1,2,4]triazolo[4,3-a]pyridin-3(2H)-on (Enantiomer 1)

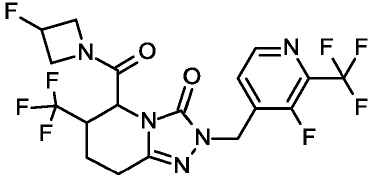

(5RS,6RS)-5-[(3-Fluorazetidin-1-yl)carbonyl]-2-{[3-fluor-2-(trifluormethyl)pyridin-4-yl]methyl}-6-(trifluormethyl)-5,6,7,8-tetrahydro[1,2,4]triazolo[4,3-a]pyridin-3(2H)-on (Diastereomer1, Racemat) wurde durch chirale präparative HPLC getrennt [Probenvorbereitung: 23 mg gelöst in Isopropanol/Dichlormethan/n-Heptan (3:1:2); Injektionsvolumen: 0.50 ml; Säule: Daicel Chiralcel^{®} IB 5 µm, 250 x 30 mm; Laufmittel: n-Heptan/Isopropanol 60:40; Fluss: 60 ml/min; Temperatur 40°C; UV-Detektion: 220 nm]. Nach der Trennung wurden 10.5 mg von Enantiomer 1, welches zuerst eluierte, und 11.8 mg von Enantiomer 2, welches später eluierte, isoliert.

### Enantiomer 1:

Analytische chirale HPLC: Rₜ = 4.70 min, e.e. = 100% [Säule: Daicel Chiralcel^{®} IB-3 50 x 4.6 mm; Laufmittel: n-Heptan/Isopropanol 60:40; Fluss: 1 ml/min; UV-Detektion: 220 nm].
LC-MS (Methode 3): Rₜ = 1.59 min; MS (ESIpos): m/z = 486 [M+H]⁺
¹H-NMR (400 MHz, DMSO-d6) δ [ppm]: -0.149 (1.52), -0.008 (16.00), 0.146 (1.68), 1.994 (2.48), 2.047 (2.08), 2.327 (2.96), 2.366 (3.04), 2.670 (4.24), 2.710 (3.76), 2.767 (2.96), 2.811 (1.68), 3.968 (1.44), 3.999 (1.52), 4.030 (1.44), 4.229 (1.60), 4.252 (2.08), 4.567 (1.12), 4.625 (1.04), 4.654 (4.16), 4.666 (5.92), 4.679 (3.44), 4.815 (0.80), 5.087 (9.44), 5.100 (8.16), 5.369 (1.12), 5.560 (0.96), 7.566 (3.52), 7.579 (6.40), 7.592 (3.84), 8.571 (6.48), 8.583 (6.40).

### Beispiel 304

### (5RS,6RS)-5-[(3-Fluorazetidin-1-yl)carbonyl]-6-(trifluormethyl)-2-{[6-(trifluormethyl)pyridin-3-yl]methyl}-5,6,7,8-tetrahydro[1,2,4]triazolo[4,3-a]pyridin-3(2H)-on (Diastereomer 1, Racemat)

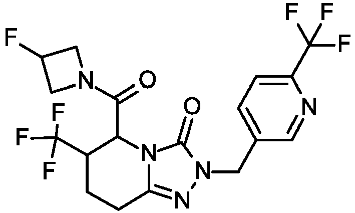

(5RS,6RS)-3-Oxo-6-(trifluormethyl)-2-{[6-(trifluormethyl)pyridin-3-yl]methyl}-2,3,5,6,7,8-hexahydro[1,2,4]triazolo[4,3-a]pyridin-5-carbonsäure (Diastereomerengemisch; 4 Isomere) (54.0 mg, 132 µmol) wurde in THF (2.0 ml) vorgelegt und HBTU (64.9 mg, 171 µmol) und N,N-Diisopropylethylamin (69 µl, 390 µmol) wurden anschließend zugegeben. Nach 15 min Rühren bei Raumtemperatur wurde 3-Fluorazetidinhydrochlorid (17.6 mg, 158 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 47.9 mg (78 % d. Th.) der Titelverbindung erhalten.
LC-MS (Methode 3): Rₜ = 1.52 min; MS (ESIpos): m/z = 468 [M+H]⁺
¹H-NMR (400 MHz, DMSO-d6) δ [ppm]: -0.149 (1.77), -0.008 (16.00), 0.008 (12.72), 0.146 (1.77), 0.886 (0.42), 1.969 (1.43), 1.983 (1.77), 2.040 (1.52), 2.073 (1.43), 2.327 (2.78), 2.366 (3.54), 2.523 (7.75), 2.669 (4.04), 2.710 (3.12), 2.756 (1.85), 2.771 (1.18), 2.797 (0.93), 3.965 (0.93), 3.999 (0.93), 4.028 (0.84), 4.065 (0.67), 4.224 (1.01), 4.246 (1.09), 4.272 (1.18), 4.304 (1.09), 4.569 (0.67), 4.642 (2.53), 4.656 (3.87), 4.666 (2.61), 4.821 (0.51), 5.030 (7.33), 5.044 (7.49), 5.418 (0.67), 5.560 (0.67), 7.921 (12.46), 8.652 (4.63).

### Beispiel 305

### (5RS,6RS)-5-[(3-Fluorazetidin-1-yl)carbonyl]-6-(trifluormethyl)-2-{[6-(trifluormethyl)pyridin-3-yl]methyl}-5,6,7,8-tetrahydro[1,2,4]triazolo[4,3-a]pyridin-3(2H)-on (Enantiomer 2)

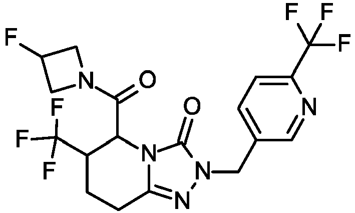

(5RS,6RS)-5-[(3-Fluorazetidin-1-yl)carbonyl]-6-(trifluormethyl)-2-{[6-(trifluormethyl)pyridin-3-yl]methyl}-5,6,7,8-tetrahydro[1,2,4]triazolo[4,3-a]pyridin-3(2H)-on (Diastereomer 1; Racemat) wurde durch chirale präparative HPLC getrennt [Probenvorbereitung: 46 mg gelöst in Acetonitril/Ethanol (3:1); Injektionsvolumen: 0.30 ml; Säule: Daicel Chiralcel^{®} IE 5 µm, 250 x 30 mm; Laufmittel: n-Heptan/Ethanol 50:50; Fluss: 15 ml/min; Temperatur 30°C; UV-Detektion: 210 nm]. Nach der Trennung wurden 16.9 mg von Isomer 1, welches zuerst eluierte, und 21.7 mg von Isomer 2, welches später eluierte, isoliert.

### Enantiomer 2:

Analytische chirale HPLC: Rₜ = 2.09 min, e.e. = 100% [Säule: Daicel Chiralcel^{®} IE-3 50 x 4.6 mm; Laufmittel: n-Heptan/IEthanol 50:50; Fluss: 1 ml/min; UV Detektion: 220 nm].
LC-MS (Methode 4): Rₜ = 0.80 min; MS (ESIpos): m/z = 468 [M+H]⁺
¹H-NMR (400 MHz, DMSO-d6) δ [ppm]: -0.149 (0.84), 0.008 (5.48), 0.146 (0.75), 1.100 (0.66), 1.235 (1.33), 1.950 (1.51), 1.968 (1.99), 1.984 (2.44), 2.001 (1.63), 2.040 (1.99), 2.327 (1.45), 2.366 (1.36), 2.524 (6.48), 2.670 (3.22), 2.710 (1.51), 2.738 (1.60), 2.754 (2.71), 2.771 (1.66), 2.797 (1.33), 2.813 (0.69), 3.936 (0.75), 3.967 (1.27), 3.998 (1.36), 4.030 (1.18), 4.061 (0.84), 4.224 (1.39), 4.244 (1.54), 4.274 (1.66), 4.304 (1.51), 4.471 (0.75), 4.485 (0.78), 4.526 (0.72), 4.540 (0.69), 4.567 (0.96), 4.595 (0.72), 4.643 (3.59), 4.655 (5.06), 4.666 (3.28), 4.821 (0.72), 4.849 (0.72), 4.873 (0.66), 5.030 (9.94), 5.045 (9.76), 5.372 (0.90), 5.418 (0.87), 5.515 (0.90), 5.561 (0.87), 7.922 (16.00), 8.653 (6.24).

### Beispiel 306

### (5RS,6RS)-2-[(3,5-Dichlorpyridin-2-yl)methyl]-5-[(3-fluorazetidin-1-yl)carbonyl]-6-(trifluormethyl)-5,6,7,8-tetrahydro[1,2,4]triazolo[4,3-a]pyridin-3(2H)-on (Diastereomer 1; Racemat)

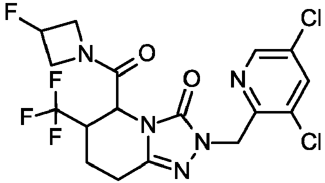

(5RS,6RS)-2-[(3,5-Dichlorpyridin-2-yl)methyl]-3-oxo-6-(trifluormethyl)-2,3,5,6,7,8-hexahydro[1,2,4]triazolo[4,3-a]pyridin-5-carbonsäure (Diastereomerengemisch; 4 Isomere) (50.0 mg, 122 µmol) wurde in THF (2.0 ml) vorgelegt und HBTU (60.0 mg, 158 µmol) und N,N-Diisopropylethylamin (64 µl, 360 µmol) wurden anschließend zugegeben. Nach 15 min Rühren bei Raumtemperatur wurde 3-Fluorazetidinhydrochlorid (16.3 mg, 146 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 45.3 mg (80 % d. Th.) der Titelverbindung erhalten.
LC-MS (Methode 4): Rₜ = 0.82 min; MS (ESIpos): m/z = 468 [M+H]⁺

### Beispiel 307

### (5RS,6RS)-2-[(3,5-Dichlorpyridin-2-yl)methyl]-5-[(3-fluorazetidin-1-yl)carbonyl]-6-(trifluormethyl)-5,6,7,8-tetrahydro[1,2,4]triazolo[4,3-a]pyridin-3(2H)-on (Enantiomer 1)

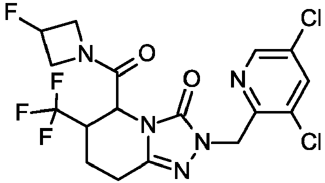

(5RS,6RS)-2-[(3,5-Dichlorpyridin-2-yl)methyl]-5-[(3-fluorazetidin-1-yl)carbonyl]-6-(trifluormethyl)-5,6,7,8-tetrahydro[1,2,4]triazolo[4,3-a]pyridin-3(2H)-on (Diastereomer 1; Racemat) wurde durch chirale präparative HPLC getrennt [Probenvorbereitung: 40 mg gelöst in Acetonitril/Ethanol (3:1); Injektionsvolumen: 0.40 ml; Säule: Daicel Chiralcel^{®} IE 5 µm, 250 x 30 mm; Laufmittel: n-Heptan/Ethanol 50:50; Fluss: 15 ml/min; Temperatur 25°C; UV-Detektion: 210 nm]. Nach der Trennung wurden 17.6 mg von Enantiomer 1, welches zuerst eluierte, und 18.1 mg von Enantiomer 2, welches später eluierte, isoliert.

### Enantiomer 1:

Analytische chirale HPLC: Rₜ = 2.65 min, e.e. = 100% [Säule: Daicel Chiralcel^{®} IE-3 50 x 4.6 mm; Laufmittel: n-Heptan/IEthanol 50:50; Fluss: 1 ml/min; UV Detektion: 220 nm].
LC-MS (Methode 4): Rₜ = 0.82 min; MS (ESIpos): m/z = 468 [M+H]⁺
¹H-NMR (400 MHz, DMSO-d6) δ [ppm]: -0.149 (1.90), -0.008 (16.00), 0.008 (14.18), 0.146 (1.90), 1.235 (0.78), 1.964 (1.56), 2.064 (1.56), 2.327 (3.46), 2.366 (3.29), 2.523 (7.87), 2.669 (3.37), 2.710 (4.06), 2.728 (2.25), 2.745 (1.21), 2.772 (1.21), 3.958 (1.12), 3.984 (1.04), 4.018 (1.04), 4.243 (1.73), 4.543 (0.86), 4.609 (3.03), 4.623 (3.55), 4.631 (2.77), 4.800 (0.69), 5.037 (9.51), 5.046 (7.26), 5.411 (0.78), 5.548 (0.69), 8.258 (5.97), 8.263 (6.31), 8.556 (4.06).

### Beispiel 308

### (5RS,6RS)-2-[(5-Chlor-3-fluorpyridin-2-yl)methyl]-5-[(3-fluorazetidin-1-yl)carbonyl]-6-(trifluormethyl)-5,6,7,8-tetrahydro[1,2,4]triazolo[4,3-a]pyridin-3(2H)-on (Diastereomer 1; Racemat)

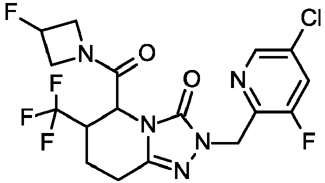

(5RS,6RS)-2-[(5-Chlor-3-fluorpyridin-2-yl)methyl]-3-oxo-6-(trifluormethyl)-2,3,5,6,7,8-hexahydro[1,2,4]triazolo[4,3-a]pyridin-5-carbonsäure (Diastereomerengemisch; 4 Isomere) (50.0 mg, 127 µmol) wurde in THF (2.0 ml) vorgelegt und HBTU (62.5 mg, 165 µmol) und N,N-Diisopropylethylamin (66 µl, 380 µmol) wurden anschließend zugegeben. Nach 15 min Rühren bei Raumtemperatur wurde 3-Fluorazetidinhydrochlorid (17.0 mg, 152 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 37.0 mg (65 % d. Th.) der Titelverbindung erhalten.
LC-MS (Methode 3): Rₜ = 1.46 min; MS (ESIpos): m/z = 452 [M+H]⁺
¹H-NMR (400 MHz, DMSO-d6) δ [ppm]: -0.149 (1.64), -0.008 (15.18), 0.008 (15.69), 0.146 (1.85), 1.921 (2.87), 1.942 (3.28), 1.956 (4.00), 2.053 (3.28), 2.327 (3.08), 2.366 (2.56), 2.589 (2.36), 2.635 (3.79), 2.650 (4.00), 2.669 (4.31), 2.710 (4.51), 2.720 (5.54), 2.735 (3.49), 2.764 (2.56), 3.925 (1.54), 3.957 (2.46), 3.987 (2.46), 4.017 (2.26), 4.048 (1.44), 4.215 (2.36), 4.240 (2.87), 4.268 (2.77), 4.297 (2.87), 4.465 (1.44), 4.555 (1.74), 4.601 (6.67), 4.610 (8.62), 4.616 (8.82), 4.766 (1.13), 4.779 (1.33), 4.804 (1.44), 4.841 (1.33), 4.856 (1.44), 4.990 (16.00), 5.000 (14.46), 5.415 (1.85), 5.549 (1.74), 8.104 (8.31), 8.129 (8.41), 8.476 (11.59).

### Beispiel 309

### (5RS,6RS)-2-[(5-Chlor-3-fluorpyridin-2-yl)methyl]-5-[(3-fluorazetidin-1-yl)carbonyl]-6-(trifluormethyl)-5,6,7,8-tetrahydro[1,2,4]triazolo[4,3-a]pyridin-3(2H)-on (Enantiomer 2)

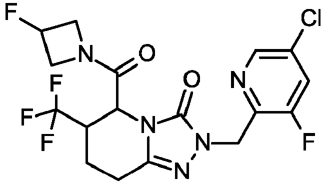

(5RS,6RS)-2-[(5-Chlor-3-fluorpyridin-2-yl)methyl]-5-[(3-fluorazetidin-1-yl)carbonyl]-6-(trifluormethyl)-5,6,7,8-tetrahydro[1,2,4]triazolo[4,3-a]pyridin-3(2H)-on (Diastereomer 1; Racemat) wurde durch chirale präparative HPLC getrennt [Probenvorbereitung: 36 mg gelöst in 3 ml Ethanol; Injektionsvolumen: 0.80 ml; Säule: Daicel Chiralcel^{®} OX-H 5 µm, 250 x 20 mm; Laufmittel: Ethanol + 0.2% Diethylamin; Fluss: 15 ml/min; Temperatur 55°C; UV-Detektion: 235 nm]. Nach der Trennung wurden 13.7 mg von Enantiomer 1, welches zuerst eluierte, und 11.7 mg von Enantiomer 2, welches später eluierte, isoliert.

### Enantiomer 2:

Analytische chirale HPLC: Rₜ = 7.56 min, e.e. = 100% [Säule: Daicel Chiralcel^{®} OZ-H 50 x 4.6 mm; Laufmittel: n-Heptan/Ethanol 70:30 + 0.2% Diethylamin; Fluss: 1 ml/min; UV Detektion: 220 nm].
LC-MS (Methode 3): Rₜ = 1.46 min; MS (ESIpos): m/z = 452 [M+H]⁺
¹H-NMR (400 MHz, DMSO-d6) δ [ppm]: -0.149 (1.44), -0.008 (13.25), 0.146 (1.40), 1.236 (0.90), 1.922 (2.61), 1.941 (3.15), 1.956 (3.65), 1.972 (2.25), 2.052 (3.34), 2.327 (2.03), 2.366 (1.94), 2.590 (2.03), 2.634 (3.61), 2.650 (3.70), 2.669 (3.02), 2.703 (2.93), 2.710 (3.65), 2.719 (5.18), 2.735 (3.11), 2.763 (2.52), 2.778 (1.31), 3.870 (0.41), 3.927 (1.22), 3.959 (2.34), 3.986 (2.52), 4.018 (2.16), 4.055 (1.44), 4.172 (1.08), 4.184 (1.26), 4.214 (2.12), 4.238 (2.66), 4.266 (2.75), 4.295 (2.66), 4.314 (1.26), 4.343 (0.90), 4.467 (1.31), 4.481 (1.35), 4.518 (1.26), 4.533 (1.31), 4.552 (1.62), 4.583 (1.08), 4.601 (6.40), 4.610 (8.34), 4.616 (8.88), 4.622 (7.44), 4.790 (0.99), 4.805 (1.17), 4.832 (1.17), 4.856 (1.17), 4.884 (0.95), 4.990 (16.00), 5.000 (14.38), 5.371 (1.53), 5.414 (1.58), 5.513 (1.53), 5.556 (1.49), 8.104 (6.81), 8.128 (6.99), 8.476 (8.16).

### Beispiel 310

### (5RS,6RS)-2-[(5-Chlorpyridin-2-yl)methyl]-5-[(3-fluorazetidin-1-yl)carbonyl]-6-(trifluormethyl)-5,6,7,8-tetrahydro[1,2,4]triazolo[4,3-a]pyridin-3(2H)-on (Diastereomer 1; Racemat)

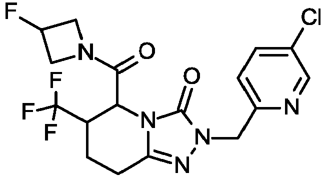

(5RS,6RS)-2-[(5-Chlorpyridin-2-yl)methyl]-3-oxo-6-(trifluormethyl)-2,3,5,6,7,8-hexahydro[1,2,4]triazolo[4,3-a]pyridin-5-carbonsäure (Diastereomerengemisch; 4 Isomere) (47.0 mg, 125 µmol) wurde in THF (2.0 ml) vorgelegt und HBTU (61.5 mg, 162 µmol) und N,N-Diisopropylethylamin (65 µl, 370 µmol) wurden anschließend zugegeben. Nach 15 min Rühren bei Raumtemperatur wurde 3-Fluorazetidinhydrochlorid (16.7 mg, 150 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 30.0 mg (55 % d. Th.) der Titelverbindung erhalten.
LC-MS (Methode 3): Rₜ = 1.40 min; MS (ESIpos): m/z = 434 [M+H]⁺
¹H-NMR (400 MHz, DMSO-d6) δ [ppm]: -0.149 (2.09), -0.008 (15.63), 0.008 (16.00), 0.146 (2.02), 1.953 (1.79), 1.973 (2.09), 1.986 (2.54), 2.073 (3.44), 2.327 (2.32), 2.366 (2.09), 2.670 (4.34), 2.710 (2.32), 2.734 (1.87), 2.751 (3.29), 2.768 (2.17), 2.794 (1.72), 2.811 (0.97), 3.935 (0.90), 3.965 (1.57), 3.994 (1.64), 4.023 (1.50), 4.060 (0.82), 4.192 (0.97), 4.224 (1.64), 4.247 (2.09), 4.282 (1.64), 4.304 (1.57), 4.487 (0.90), 4.526 (0.90), 4.542 (0.90), 4.567 (1.20), 4.633 (4.26), 4.642 (4.93), 4.647 (5.23), 4.655 (4.56), 4.780 (1.35), 4.794 (1.72), 4.879 (1.20), 4.931 (13.23), 4.943 (13.08), 4.989 (0.97), 5.412 (1.20), 5.553 (1.12), 7.226 (9.05), 7.248 (9.87), 7.928 (4.64), 7.944 (4.26), 8.575 (7.03), 8.581 (3.96).

### Beispiel 311

### (5RS,6RS)-2-[(5-Chlorpyridin-2-yl)methyl]-5-[(3-fluorazetidin-1-yl)carbonyl]-6-(trifluormethyl)-5,6,7,8-tetrahydro[1,2,4]triazolo[4,3-a]pyridin-3(2H)-on (Enantiomer 2)

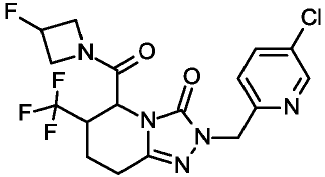

(5RS,6RS)-2-[(5-Chlorpyridin-2-yl)methyl]-5-[(3-fluorazetidin-1-yl)carbonyl]-6-(trifluormethyl)-5,6,7,8-tetrahydro[1,2,4]triazolo[4,3-a]pyridin-3(2H)-on (Diastereomer 1; Racemat) wurde durch chirale präparative HPLC getrennt [Probenvorbereitung: 30 mg gelöst in 3 ml Ethanol/Acetonirtril (1:2); Injektionsvolumen: 0.25 ml; Säule: Daicel Chiralcel^{®} IC 5 µm, 250 x 20 mm; Laufmittel: n-Heptan/Ethanol (50:50); Fluss: 15 ml/min; Temperatur 25°C; UV-Detektion: 210 nm]. Nach der Trennung wurden 12.30 mg von Enantiomer 1, welches zuerst eluierte, und 12.1 mg von Enantiomer 2, welches später eluierte, isoliert.

### Enantiomer 2:

Analytische chirale HPLC: Rₜ = 2.51 min, e.e. = 100% [Säule: Daicel Chiralcel^{®} IC-3 50 x 4.6 mm; Laufmittel: n-Heptan/Ethanol 50:50; Fluss: 1 ml/min; UV-Detektion: 220 nm].
LC-MS (Methode 3): Rₜ = 140.00 min; MS (ESIpos): m/z = 433 [M+H]⁺
¹H-NMR (400 MHz, DMSO-d6) δ [ppm]: -0.149 (0.76), -0.008 (7.47), 0.008 (6.94), 0.146 (0.88), 1.234 (0.53), 1.953 (1.94), 1.971 (2.29), 1.986 (2.82), 2.073 (2.94), 2.327 (2.00), 2.366 (1.53), 2.670 (4.88), 2.710 (1.94), 2.735 (2.18), 2.751 (3.94), 2.767 (2.41), 2.794 (1.94), 3.932 (0.94), 3.964 (1.88), 3.995 (2.06), 4.023 (1.82), 4.058 (1.18), 4.192 (1.00), 4.223 (2.00), 4.247 (2.47), 4.278 (1.82), 4.303 (1.88), 4.475 (1.00), 4.489 (1.06), 4.528 (1.00), 4.563 (1.24), 4.633 (5.24), 4.647 (6.41), 4.655 (5.59), 4.880 (0.94), 4.931 (15.94), 4.943 (16.00), 5.414 (1.18), 5.555 (1.24), 7.227 (10.59), 7.248 (11.29), 7.927 (5.18), 7.947 (4.94), 8.575 (9.65).

### Beispiel 312

### (5RS,7RS)-5-[(3,3-Difluorpyrrolidin-1-yl)carbonyl]-7-(trifluormethyl)-2-{[6-(trifluormethyl)pyridin-3-yl]methyl}-5,6,7,8-tetrahydro[1,2,4]triazolo[4,3-a]pyridin-3(2H)-on (Diastereomer 1; Racemat)

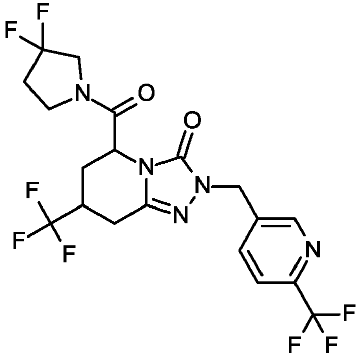

(5RS,7RS)-3-Oxo-7-(trifluormethyl)-2-{[6-(trifluormethyl)pyridin-3-yl]methyl}-2,3,5,6,7,8-hexahydro[1,2,4]triazolo[4,3-a]pyridin-5-carbonsäure (Diastereomerengemisch; 4 Isomere) (100 mg, 90 % Reinheit, 219 µmol) wurde in THF (3.0 ml) vorgelegt und HBTU (108 mg, 285 µmol) und N,N-Diisopropylethylamin (110 µl, 660 µmol) wurden anschließend zugegeben. Nach 15 min Rühren bei Raumtemperatur wurde 3,3-Difluorpyrrolidinhydrochlorid (37.7 mg, 263 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient) und das Diastereomer 1 isoliert. Die produkthaltigen Fraktionen wurden im Vakuum eingeengt und es wurden 78.2 mg (71 % d. Th.) der Titelverbindung erhalten.
LC-MS (Methode 3): Rₜ = 1.69 min; MS (ESIpos): m/z = 500 [M+H]⁺
¹H-NMR (400 MHz, DMSO-d6) δ [ppm]: -0.149 (0.71), -0.008 (5.74), 0.146 (0.71), 2.138 (0.40), 2.156 (0.79), 2.173 (0.82), 2.198 (0.97), 2.229 (0.73), 2.245 (0.68), 2.270 (1.30), 2.290 (1.46), 2.327 (1.37), 2.366 (0.71), 2.376 (0.51), 2.397 (0.97), 2.427 (1.24), 2.444 (1.13), 2.462 (0.79), 2.565 (1.92), 2.583 (1.48), 2.602 (1.02), 2.670 (0.88), 2.688 (1.06), 2.696 (1.02), 2.717 (1.41), 2.726 (2.30), 2.735 (1.48), 2.757 (1.63), 2.764 (1.68), 2.895 (1.15), 2.927 (1.08), 2.979 (2.12), 3.019 (1.61), 3.541 (0.44), 3.552 (1.04), 3.571 (2.21), 3.586 (2.16), 3.603 (1.28), 3.714 (0.99), 3.756 (1.28), 3.786 (1.52), 3.821 (1.17), 3.901 (1.04), 3.919 (2.36), 3.936 (2.58), 3.955 (1.06), 4.135 (0.88), 4.163 (0.73), 4.178 (1.28), 4.205 (1.35), 4.238 (0.90), 4.968 (1.70), 4.982 (1.63), 5.019 (1.74), 5.048 (16.00), 7.920 (15.23), 7.943 (0.93), 8.642 (5.16), 8.678 (0.77).

### Beispiel 313

### (5RS,7RS)-5-[(3,3-Difluorpyrrolidin-1-yl)carbonyl]-7-(trifluormethyl)-2-{[6-(trifluormethyl)pyridin-3-yl]methyl}-5,6,7,8-tetrahydro[1,2,4]triazolo[4,3-a]pyridin-3(2H)-on (Enantiomer 1)

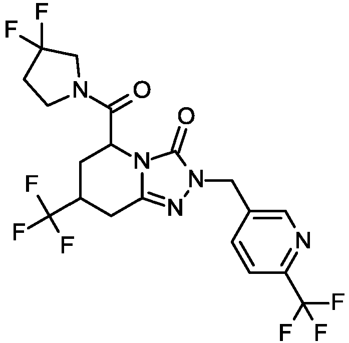

(5RS,7RS)-5-[(3,3-Difluorpyrrolidin-1-yl)carbonyl]-7-(trifluormethyl)-2-{[6-(trifluormethyl)pyridin-3-yl]methyl}-5,6,7,8-tetrahydro[1,2,4]triazolo[4,3-a]pyridin-3(2H)-on (Diastereomer 1; Racemat) wurde durch chirale präparative HPLC getrennt [Probenvorbereitung: 77 mg gelöst in 2 ml Ethanol; Injektionsvolumen: 0.50 ml; Säule: Daicel Chiralcel^{®} OX-H 5 µm, 250 x 20 mm; Laufmittel: n-Heptan/Ethanol (50:50); Fluss: 20 ml/min; Temperatur 40°C; UV-Detektion: 220 nm]. Nach der Trennung wurden 24.0 mg von Isomer 1, welches zuerst eluierte, und 28.8 mg von Isomer 2, welches später eluierte, isoliert.

### Isomer 1:

Analytische chirale HPLC: Rₜ = 1.33 min, e.e. = 100% [Säule: Daicel Chiralcel^{®} IE-3 50 x 4.6 mm; Laufmittel: n-Heptan/Ethanol 50:50; Fluss: 1 ml/min; UV-Detektion: 220 nm].
LC-MS (Methode 3): Rₜ = 1.69 min; MS (ESIpos): m/z = 500 [M+H]⁺
¹H-NMR (400 MHz, DMSO-d6) δ [ppm]: -0.150 (0.83), -0.008 (6.23), 0.008 (6.13), 0.146 (0.65), 2.156 (0.86), 2.197 (0.99), 2.228 (0.77), 2.244 (0.74), 2.268 (1.33), 2.287 (1.39), 2.327 (2.87), 2.366 (1.20), 2.396 (0.92), 2.425 (1.26), 2.444 (1.26), 2.522 (6.17), 2.565 (1.97), 2.582 (1.29), 2.669 (2.50), 2.674 (1.94), 2.687 (1.05), 2.710 (1.45), 2.716 (1.42), 2.725 (2.16), 2.734 (1.33), 2.756 (1.66), 2.764 (1.45), 2.902 (1.02), 2.979 (2.00), 3.018 (1.48), 3.551 (1.02), 3.570 (2.03), 3.584 (1.54), 3.602 (1.02), 3.714 (0.92), 3.754 (1.11), 3.785 (1.33), 3.820 (1.02), 3.901 (0.96), 3.918 (2.19), 3.936 (2.40), 3.954 (0.92), 4.135 (0.77), 4.163 (0.71), 4.179 (1.17), 4.205 (1.29), 4.237 (0.77), 4.968 (1.54), 4.982 (1.57), 5.047 (16.00), 7.917 (14.49), 7.920 (15.35), 8.642 (5.15).

### Beispiel 314

### (5RS,7RS)-2-[(5-Chlorpyridin-2-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on (Diastereomerengemisch; 4 Isomere)

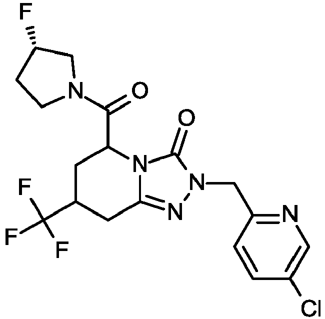

(5RS,7RS)-2-[(5-Chlorpyridin-2-yl)methyl]-3-oxo-7-(trifluormethyl)-2,3,5,6,7,8-hexahydro[1 ,2,4]triazolo[4,3-a]pyridin-5-carbonsäure (Diastereomerengemisch; 4 Isomere) (206 mg, 546 µmol) wurde in THF (8.0 ml) vorgelegt und HBTU (269 mg, 710 µmol) und N,N-Diisopropylethylamin (290 µl, 1.6 mmol) wurden anschließend zugegeben. Nach 15 min Rühren bei Raumtemperatur wurde (3S)-3-Fluorpyrrolidinhydrochlorid (82.3 mg, 656 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 221 mg (90 % d. Th.) der Titelverbindung erhalten.
LC-MS (Methode 3): Rₜ = 1.44 min; MS (ESIpos): m/z = 448 [M+H]⁺
¹H-NMR (400 MHz, DMSO-d6) δ [ppm]: -0.150 (1.37), -0.009 (12.42), 0.007 (13.00), 0.146 (1.37), 1.055 (0.81), 2.072 (16.00), 2.175 (2.57), 2.240 (4.40), 2.291 (2.67), 2.327 (1.82), 2.689 (2.15), 2.709 (2.80), 2.719 (3.42), 2.730 (1.99), 2.748 (2.05), 2.976 (4.40), 3.009 (2.90), 3.353 (1.86), 3.371 (1.79), 3.400 (1.30), 3.442 (0.91), 3.512 (0.81), 3.572 (1.73), 3.618 (1.96), 3.638 (2.84), 3.673 (2.93), 3.691 (1.89), 3.802 (0.98), 3.854 (1.66), 3.897 (1.21), 3.995 (1.99), 4.021 (1.34), 4.890 (1.37), 4.932 (7.20), 4.940 (10.49), 4.947 (10.33), 4.951 (10.20), 4.974 (1.76), 4.991 (2.90), 5.025 (1.43), 5.038 (1.40), 5.081 (1.50), 5.267 (1.60), 5.358 (0.91), 5.401 (1.96), 5.488 (0.88), 7.221 (5.70), 7.242 (6.19), 7.286 (0.85), 7.919 (6.19), 7.925 (6.62), 7.940 (6.00), 7.946 (6.45), 8.574 (6.65), 8.580 (6.71).

### Beispiel 315

### (5RS,7RS)-2-[(5-Chlorpyridin-2-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on (Isomer 2)

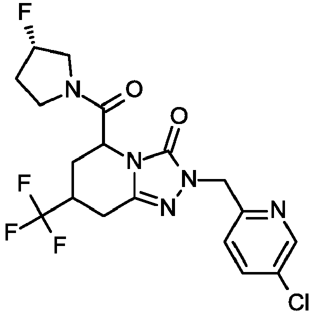

### (5RS,7RS)-2-[(5-Chlorpyridin-2-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on

(Diastereomerengemisch; 4 Isomere) wurde durch chirale präparative HPLC getrennt [Probenvorbereitung: 218 mg gelöst in 5 ml Ethanol/nHeptan (3:2); Injektionsvolumen: 0.23 ml; Säule: Daicel Chiralcel^{®} IC 5 µm, 250 x 20 mm; Laufmittel: n-Heptan/Ethanol (50:50); Fluss: 15 ml/min; Temperatur 40°C; UV-Detektion: 220 nm]. Nach der Trennung wurden 100 mg von Isomer 1, welches zuerst eluierte, und 90 mg von Isomer 2, welches später eluierte, isoliert.

### Isomer 2:

Analytische chirale HPLC: Rₜ = 4.14 min, d.e. = 100% [Säule: Daicel Chiralcel^{®} IC-3 50 x 4.6 mm; Laufmittel: n-Heptan/Ethanol 50:50; Fluss: 1 ml/min; UV-Detektion: 220 nm].
LC-MS (Methode 3): Rₜ = 1.44 min; MS (ESIpos): m/z = 448 [M+H]⁺
¹H-NMR (400 MHz, DMSO-d6) δ [ppm]: -0.149 (1.94), -0.008 (16.00), 0.008 (15.60), 0.146 (1.94), 1.234 (0.71), 2.002 (0.62), 2.140 (1.85), 2.175 (3.31), 2.211 (2.03), 2.245 (2.95), 2.295 (3.92), 2.327 (3.31), 2.366 (1.41), 2.670 (2.64), 2.709 (2.95), 2.718 (2.95), 2.732 (1.98), 2.748 (1.85), 2.764 (1.45), 2.977 (5.55), 3.011 (3.57), 3.022 (2.25), 3.343 (1.50), 3.355 (1.72), 3.372 (1.59), 3.382 (1.32), 3.399 (1.50), 3.434 (1.32), 3.442 (1.28), 3.495 (0.97), 3.532 (1.28), 3.622 (1.28), 3.647 (2.82), 3.673 (3.83), 3.699 (2.95), 3.718 (1.10), 3.732 (1.19), 3.772 (0.71), 3.812 (1.01), 3.833 (1.45), 3.854 (2.56), 3.876 (1.63), 3.899 (1.15), 3.930 (1.23), 3.964 (0.84), 3.986 (1.32), 4.020 (0.84), 4.891 (2.42), 4.932 (12.52), 4.948 (8.42), 4.952 (9.92), 4.975 (3.31), 4.988 (4.32), 5.268 (1.94), 5.399 (2.56), 5.524 (1.37), 7.222 (6.39), 7.242 (6.96), 7.919 (7.10), 7.926 (7.23), 7.940 (6.74), 7.947 (6.92), 8.574 (7.54), 8.580 (7.58).

### Beispiel 316

### (5RS,7RS)-2-[(5-Chlorpyridin-3-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on (Diastereomerengemisch; 2 Isomere)

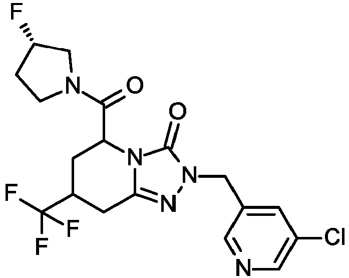

(5RS,7RS)-2-[(5-Chlorpyridin-3-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere) (625 mg, 1.66 mmol) wurde in THF (3.0 ml) vorgelegt und HBTU (818 mg, 2.16 mmol) und N,N-Diisopropylethylamin (870 µl, 5.0 mmol) wurden anschließend zugegeben. Nach 15 min Rühren bei Raumtemperatur wurde (3S)-3-Fluorpyrrolidinhydrochlorid (250 mg, 1.99 mmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 296 mg (38 % d. Th.) der Titelverbindung erhalten.
LC-MS (Methode 3): Rₜ = 1.44 min; MS (ESIpos): m/z = 448 [M+H]⁺
¹H-NMR (400 MHz, DMSO-d6) δ [ppm]: -0.149 (1.08), -0.008 (8.89), 0.008 (9.13), 0.146 (1.10), 2.004 (0.47), 2.073 (1.57), 2.171 (2.52), 2.190 (1.50), 2.212 (2.41), 2.235 (3.70), 2.284 (2.39), 2.323 (1.44), 2.327 (1.52), 2.366 (0.68), 2.670 (1.15), 2.691 (1.15), 2.700 (1.08), 2.710 (0.97), 2.729 (2.70), 2.740 (1.60), 2.761 (1.73), 2.769 (1.78), 2.914 (1.23), 2.984 (2.99), 3.023 (2.02), 3.357 (1.10), 3.378 (1.15), 3.406 (0.97), 3.443 (0.63), 3.495 (0.55), 3.505 (0.55), 3.530 (0.89), 3.568 (1.31), 3.590 (1.13), 3.614 (1.78), 3.640 (2.23), 3.674 (1.99), 3.700 (1.36), 3.718 (0.63), 3.733 (0.66), 3.761 (0.58), 3.793 (0.63), 3.829 (0.73), 3.849 (1.50), 3.872 (0.89), 3.896 (0.63), 3.923 (0.58), 3.969 (0.63), 3.994 (1.47), 4.015 (0.81), 4.055 (0.63), 4.087 (0.47), 4.909 (0.68), 4.924 (1.44), 4.956 (16.00), 4.986 (1.39), 5.031 (1.15), 5.043 (1.10), 5.084 (1.18), 5.269 (1.29), 5.358 (0.81), 5.402 (1.55), 5.488 (0.71), 5.524 (0.58), 7.761 (5.30), 7.766 (3.83), 8.428 (6.82), 8.454 (0.50), 8.568 (5.72), 8.574 (5.77).

### Beispiel 317

### (5RS,7RS)-2-[(5-Chlorpyridin-3-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on (Isomer 1)

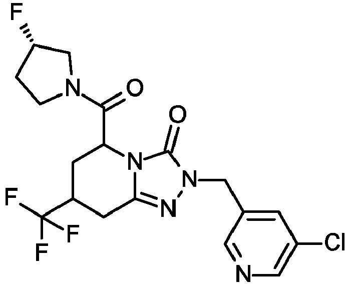

### (5RS,7RS)-2-[(5-Chlorpyridin-3-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on

(Diastereomerengemisch; 2 Isomere) wurde durch chirale präparative HPLC getrennt [Probenvorbereitung: 296 mg gelöst in 9 ml Ethanol/nHeptan (7:2); Injektionsvolumen: 0.20 ml; Säule: Daicel Chiralcel^{®} IE 5 µm, 250 x 20 mm; Laufmittel: n-Heptan/Ethanol (30:70); Fluss: 15 ml/min; Temperatur 40°C; UV-Detektion: 220 nm]. Nach der Trennung wurden 110 mg von Isomer 1, welches zuerst eluierte, und 101 mg von Isomer 2, welches später eluierte, isoliert.

### Isomer 1:

Analytische chirale HPLC: Rₜ = 3.16 min, d.e. = 100% [Säule: Daicel Chiralcel^{®} IE 50 x 4.6 mm; Laufmittel: n-Heptan/Ethanol 50:50; Fluss: 1 ml/min; UV-Detektion: 220 nm].
LC-MS (Methode 3): Rₜ = 1.40 min; MS (ESIpos): m/z = 448 [M+H]⁺
¹H-NMR (400 MHz, DMSO-d6) δ [ppm]: -0.149 (1.59), -0.008 (12.81), 0.008 (13.77), 0.146 (1.59), 1.236 (0.64), 2.005 (0.64), 2.137 (2.17), 2.170 (4.08), 2.205 (2.49), 2.235 (3.19), 2.250 (3.19), 2.284 (4.40), 2.327 (3.44), 2.366 (2.49), 2.669 (2.68), 2.689 (1.98), 2.710 (3.12), 2.717 (2.74), 2.729 (3.57), 2.740 (2.10), 2.757 (2.87), 2.770 (1.98), 2.934 (1.85), 2.985 (5.23), 3.023 (3.12), 3.033 (2.42), 3.355 (2.04), 3.373 (1.85), 3.399 (1.85), 3.442 (1.47), 3.504 (1.15), 3.540 (1.40), 3.622 (1.40), 3.647 (2.93), 3.675 (4.21), 3.700 (3.19), 3.718 (1.27), 3.736 (1.34), 3.777 (0.83), 3.809 (1.08), 3.829 (1.66), 3.849 (2.80), 3.871 (1.85), 3.895 (1.15), 3.924 (1.34), 3.954 (0.96), 3.981 (1.59), 4.014 (0.83), 4.908 (1.34), 4.923 (2.55), 4.949 (16.00), 4.972 (3.76), 4.986 (3.44), 5.268 (2.17), 5.400 (2.87), 5.524 (1.47), 7.756 (6.25), 7.761 (7.33), 8.426 (8.67), 8.568 (6.82), 8.574 (6.95).

### Beispiel 318

### (5RS,7RS)-2-[(5-Chlorpyridin-3-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on (Isomer 2)

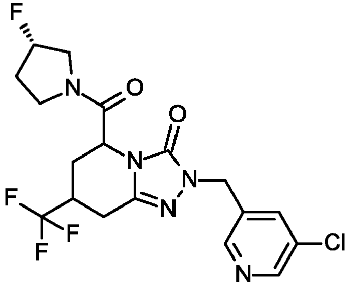

### (5RS,7RS)-2-[(5-Chlorpyridin-3-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on

(Diastereomerengemisch; 2 Isomere) wurde durch chirale präparative HPLC getrennt [Probenvorbereitung: 296 mg gelöst in 9 ml Ethanol/nHeptan (7:2); Injektionsvolumen: 0.20 ml; Säule: Daicel Chiralcel^{®} IE 5 µm, 250 x 20 mm; Laufmittel: n-Heptan/Ethanol (30:70); Fluss: 15 ml/min; Temperatur 40°C; UV-Detektion: 220 nm]. Nach der Trennung wurden 110 mg von Isomer 1, welches zuerst eluierte, und 101 mg von Isomer 2, welches später eluierte, isoliert.

### Isomer 1:

Analytische chirale HPLC: Rₜ = 4.32 min, d.e. = 100% [Säule: Daicel Chiralcel^{®} IE 50 x 4.6 mm; Laufmittel: n-Heptan/Ethanol 50:50; Fluss: 1 ml/min; UV-Detektion: 220 nm].
LC-MS (Methode 3): Rₜ = 1.40 min; MS (ESIpos): m/z = 448 [M+H]⁺
¹H-NMR (400 MHz, DMSO-d6) δ [ppm]: -0.149 (1.14), -0.008 (8.84), 0.008 (8.27), 0.146 (1.03), 2.019 (0.50), 2.159 (1.87), 2.191 (1.18), 2.211 (2.29), 2.233 (3.47), 2.274 (1.45), 2.327 (1.79), 2.366 (1.33), 2.669 (1.71), 2.692 (0.99), 2.699 (0.99), 2.710 (1.60), 2.722 (1.37), 2.731 (2.02), 2.739 (1.30), 2.761 (1.68), 2.768 (1.64), 2.891 (1.03), 2.977 (2.29), 3.016 (1.71), 3.360 (1.14), 3.378 (1.14), 3.406 (0.69), 3.490 (0.46), 3.526 (0.84), 3.567 (1.75), 3.589 (1.37), 3.614 (2.06), 3.633 (1.87), 3.658 (1.03), 3.791 (0.65), 3.858 (0.50), 3.889 (0.69), 3.968 (0.76), 3.994 (1.98), 4.017 (0.76), 4.057 (0.95), 4.087 (0.69), 4.956 (16.00), 5.029 (1.64), 5.044 (1.64), 5.084 (1.75), 5.274 (0.99), 5.358 (0.99), 5.408 (1.03), 5.488 (1.03), 7.761 (4.38), 7.766 (4.50), 8.428 (5.94), 8.568 (4.42), 8.574 (4.42).

### Beispiel 319

### (5RS,8RS)-2-(3-Chlor-4-fluorbenzyl)-5-{[(3R,4S)-3,4-difluorpyrrolidin-1-yl]carbonyl}-8-methyl-5,6,7,8-tetrahydro[1,2,4]triazolo[4,3-a]pyridin-3(2H)-on (Diastereomerengemisch; 4 Isomere)

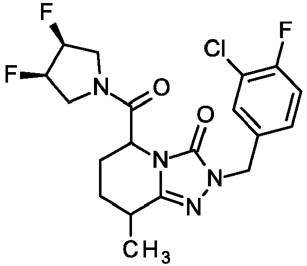

(5RS,8RS)-2-(3-Chlor-4-fluorbenzyl)-8-methyl-3-oxo-2,3,5,6,7,8-hexahydro[1,2,4]triazolo[4,3-a]pyridin-5-carbonsäure (Diastereomerengemisch; 4 Isomere) (100 mg, 79 % Reinheit, 233 µmol) wurde in THF (2.0 ml) vorgelegt und HBTU (115 mg, 302 µmol) und N,N-Diisopropylethylamin (120 µl, 700 µmol) wurden anschließend zugegeben. Nach 15 min Rühren bei Raumtemperatur wurde (3R,4S)-3,4-Difluorpyrrolidinhydrochlorid (40.1 mg, 279 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 44.0 mg (44 % d. Th.) der Titelverbindung erhalten.
LC-MS (Methode 4): Rₜ = 0.87 min; MS (ESIpos): m/z = 429 [M+H]⁺
¹H-NMR (400 MHz, DMSO-d6) δ [ppm]: -0.149 (0.92), -0.008 (7.38), 0.008 (7.15), 0.146 (0.83), 1.173 (15.59), 1.190 (16.00), 1.310 (0.55), 1.341 (1.43), 1.370 (1.57), 1.809 (1.52), 2.014 (0.65), 2.053 (2.35), 2.077 (2.90), 2.327 (1.29), 2.366 (0.88), 2.670 (1.48), 2.710 (1.94), 2.729 (1.80), 2.742 (1.66), 3.453 (0.74), 3.487 (1.20), 3.510 (1.38), 3.534 (1.01), 3.542 (1.15), 3.554 (0.83), 3.565 (0.92), 3.611 (0.92), 3.625 (1.11), 3.644 (0.69), 3.666 (1.24), 3.681 (1.98), 3.695 (1.34), 3.713 (1.94), 3.728 (1.06), 3.745 (1.15), 3.758 (1.15), 3.778 (0.55), 3.792 (0.55), 3.862 (0.92), 3.909 (1.34), 3.922 (1.01), 3.937 (0.60), 3.958 (0.97), 3.973 (1.01), 3.988 (0.60), 4.002 (0.55), 4.125 (0.55), 4.141 (0.69), 4.153 (0.69), 4.168 (0.97), 4.179 (0.74), 4.194 (0.65), 4.208 (0.60), 4.802 (1.61), 4.826 (3.92), 4.842 (7.93), 4.865 (4.29), 4.875 (4.52), 4.906 (0.92), 4.915 (1.52), 5.265 (0.97), 5.276 (1.11), 5.288 (0.92), 5.298 (0.69), 5.314 (0.69), 5.329 (0.78), 5.352 (0.88), 5.366 (0.78), 5.389 (1.15), 5.409 (1.06), 5.429 (0.78), 5.482 (0.78), 7.228 (2.26), 7.234 (2.44), 7.242 (2.31), 7.374 (3.00), 7.395 (4.80), 7.419 (2.72), 7.429 (3.46), 7.447 (3.37).

### Beispiel 320

### (5RS,8RS)-2-(3-Chlor-4-fluorbenzyl)-5-{[(3S)-3-fluorpyrrolidin-1-yl]carbonyl}-8-methyl-5,6,7,8-tetrahydro[1,2,4]triazolo[4,3-a]pyridin-3(2H)-on (Diastereomerengemisch; 4 Isomere)

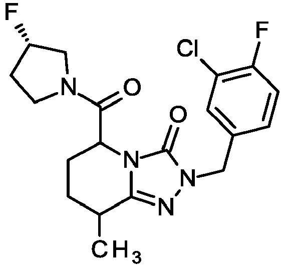

(5RS,8RS)-2-(3-Chlor-4-fluorbenzyl)-8-methyl-3-oxo-2,3,5,6,7,8-hexahydro[1,2,4]triazolo[4,3-a]pyridin-5-carbonsäure (Diastereomerengemisch; 4 Isomere) (100 mg, 79 % Reinheit, 233 µmol) wurde in THF (2.0 ml) vorgelegt und HBTU (115 mg, 302 µmol) und N,N-Diisopropylethylamin (120 µl, 700 µmol) wurden anschließend zugegeben. Nach 15 min Rühren bei Raumtemperatur wurde (3S)-3-Fluorpyrrolidinhydrochlorid (35.0 mg, 279 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 55.0 mg (58 % d. Th.) der Titelverbindung erhalten.
LC-MS (Methode 3): Rₜ = 1.57 min; MS (ESIpos): m/z = 411 [M+H]⁺
¹H-NMR (400 MHz, DMSO-d6) δ [ppm]: -0.149 (1.83), -0.008 (16.00), 0.008 (13.96), 0.146 (1.88), 1.175 (7.42), 1.180 (7.54), 1.192 (7.79), 1.197 (7.46), 1.380 (0.75), 1.813 (1.04), 2.105 (2.08), 2.270 (0.79), 2.327 (2.29), 2.366 (1.33), 2.670 (2.37), 2.710 (2.00), 3.516 (1.17), 3.597 (1.33), 3.643 (1.17), 3.667 (1.04), 3.731 (0.96), 3.840 (0.96), 3.930 (0.63), 4.776 (0.92), 4.799 (0.92), 4.839 (3.08), 4.848 (3.54), 4.871 (3.58), 5.266 (0.71), 5.391 (0.79), 7.245 (1.67), 7.373 (2.58), 7.395 (4.00), 7.418 (2.04), 7.431 (2.04), 7.448 (2.21).

### Beispiel 321

### (5RS,8RS)-2-(3-Chlor-4-fluorbenzyl)-5-[(3-fluorazetidin-1-yl)carbonyl]-8-methyl-5,6,7,8-tetrahydro[1,2,4]triazolo[4,3-a]pyridin-3(2H)-on (Diastereomerengemisch; 4 Isomere)

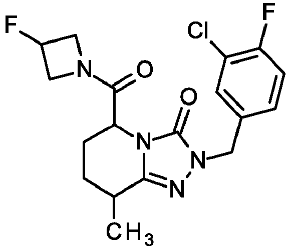

(5RS,8RS)-2-(3-Chlor-4-fluorbenzyl)-8-methyl-3-oxo-2,3,5,6,7,8-hexahydro[1,2,4]triazolo[4,3-a]pyridin-5-carbonsäure (Diastereomerengemisch; 4 Isomere) (100 mg, 79 % Reinheit, 233 µmol) wurde in THF (2.0 ml) vorgelegt und HBTU (115 mg, 302 µmol) und N,N-Diisopropylethylamin (120 µl, 700 µmol) wurden anschließend zugegeben. Nach 15 min Rühren bei Raumtemperatur wurde 3-Fluorazetidinhydrochlorid (31.1 mg, 279 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 48.0 mg (52 % d. Th.) der Titelverbindung erhalten.
LC-MS (Methode 3): Rₜ = 1.53 min; MS (ESIpos): m/z = 397 [M+H]⁺
¹H-NMR (400 MHz, DMSO-d6) δ [ppm]: -0.149 (0.45), -0.008 (4.06), 0.008 (3.98), 0.146 (0.44), 1.168 (15.72), 1.185 (16.00), 1.244 (0.42), 1.258 (0.43), 1.329 (0.42), 1.358 (0.84), 1.384 (1.02), 1.401 (0.96), 1.431 (0.51), 1.802 (1.14), 1.812 (1.16), 2.037 (2.52), 2.047 (2.73), 2.328 (0.47), 2.523 (1.47), 2.670 (0.54), 2.675 (0.53), 2.695 (0.80), 2.710 (1.63), 2.725 (1.48), 2.738 (1.25), 2.755 (0.72), 3.925 (0.72), 3.987 (0.81), 4.013 (0.55), 4.175 (0.44), 4.216 (0.79), 4.230 (0.84), 4.245 (0.71), 4.266 (0.96), 4.299 (0.86), 4.332 (0.49), 4.357 (0.87), 4.381 (0.59), 4.417 (0.45), 4.444 (0.60), 4.508 (0.40), 4.523 (0.46), 4.562 (0.46), 4.579 (0.51), 4.596 (2.31), 4.605 (3.55), 4.616 (2.39), 4.636 (0.47), 4.667 (0.44), 4.687 (0.44), 4.803 (1.87), 4.843 (6.36), 4.868 (2.98), 4.876 (2.92), 4.916 (0.87), 5.348 (0.55), 5.411 (0.55), 5.490 (0.54), 5.553 (0.53), 7.237 (1.82), 7.371 (2.19), 7.394 (3.62), 7.416 (1.76), 7.433 (2.20), 7.451 (2.21).

### Beispiel 322

### (5RS,8RS)-5-{[(3R,4S)-3,4-Difluorpyrrolidin-1-yl]carbonyl}-2-{[3-fluor-2-(trifluormethyl)pyridin-4-yl]methyl}-8-methyl-5,6,7,8-tetrahydro[1,2,4]triazolo[4,3-a]pyridin-3(2H)-on (Diastereomerengemisch; 4 Isomere)

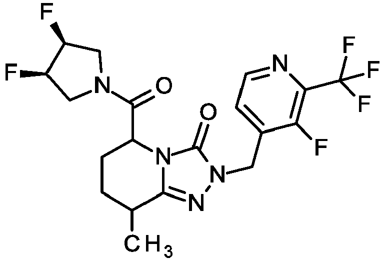

(5RS,8RS)-2-{[3-Fluor-2-(trifluormethyl)pyridin-4-yl]methyl}-8-methyl-3-oxo-2,3,5,6,7,8-hexahydro[1,2,4]triazolo[4,3-a]pyridin-5-carbonsäure (Diastereomerengemisch; 4 Isomere) (80.0 mg, 214 µmol) wurde in THF (2.0 ml) vorgelegt und HBTU (105 mg, 278 µmol) und N,N-Diisopropylethylamin (110 µl, 640 µmol) wurden anschließend zugegeben. Nach 15 min Rühren bei Raumtemperatur wurde (3R,4S)-3,4-Difluorpyrrolidinhydrochlorid (36.8 mg, 256 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 38.0 mg (38 % d. Th.) der Titelverbindung erhalten.
LC-MS (Methode 4): Rₜ = 0.82 min; MS (ESIpos): m/z = 464 [M+H]⁺
¹H-NMR (400 MHz, DMSO-d6) δ [ppm]: -0.149 (1.67), -0.008 (16.00), 0.008 (12.74), 0.146 (1.71), 1.178 (7.16), 1.194 (7.28), 1.345 (0.56), 1.377 (0.60), 1.839 (0.56), 2.107 (1.07), 2.327 (1.19), 2.332 (0.84), 2.366 (1.15), 2.518 (4.34), 2.523 (3.34), 2.665 (0.92), 2.670 (1.23), 2.674 (0.84), 2.710 (1.19), 2.755 (0.68), 2.766 (0.68), 3.496 (0.44), 3.507 (0.44), 3.518 (0.56), 3.526 (0.48), 3.674 (0.48), 3.687 (0.64), 3.720 (0.72), 3.750 (0.44), 3.763 (0.44), 3.913 (0.52), 4.853 (1.43), 4.867 (1.31), 5.048 (0.80), 5.091 (2.75), 5.116 (1.75), 5.124 (1.79), 5.166 (0.56), 7.509 (1.19), 7.518 (1.31), 7.530 (0.64), 8.561 (1.83), 8.573 (1.79).

### Beispiel 323

### (5RS,8RS)-2-(3-Chlor-4-fluorbenzyl)-5-[(3,3-difluorpyrrolidin-1-yl)carbonyl]-8-methyl-5,6,7,8-tetrahydro[1,2,4]triazolo[4,3-a]pyridin-3(2H)-on (Diastereomerengemisch; 4 Isomere)

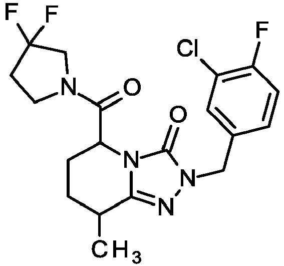

(5RS,8RS)-2-(3-Chlor-4-fluorbenzyl)-8-methyl-3-oxo-2,3,5,6,7,8-hexahydro[1,2,4]triazolo[4,3-a]pyridin-5-carbonsäure (Diastereomerengemisch; 4 Isomere) (100 mg, 79 % Reinheit, 233 µmol) wurde in THF vorgelegt und HBTU (115 mg, 302 µmol) und N,N-Diisopropylethylamin (120 µl, 700 µmol) wurden anschließend zugegeben. Nach 15 min Rühren bei Raumtemperatur wurde 3,3-Difluorpyrrolidinhydrochlorid (40.1 mg, 279 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 14.0 mg (14 % d. Th.) der Titelverbindung erhalten.
LC-MS (Methode 3): Rₜ = 1.72 min; MS (ESIpos): m/z = 429 [M+H]⁺
¹H-NMR (400 MHz, DMSO-d6) δ [ppm]: -0.149 (1.90), -0.008 (16.00), 0.008 (14.19), 0.146 (1.90), 1.175 (13.05), 1.192 (13.33), 1.370 (1.14), 1.794 (1.62), 2.072 (2.38), 2.327 (2.48), 2.366 (2.29), 2.669 (2.67), 2.710 (2.29), 2.720 (1.52), 2.735 (2.00), 2.751 (1.62), 3.527 (1.52), 3.549 (2.19), 3.665 (1.14), 3.699 (1.43), 3.734 (1.24), 3.769 (2.38), 3.797 (2.00), 3.900 (1.52), 3.969 (0.86), 4.178 (0.86), 4.201 (0.95), 4.802 (3.05), 4.844 (5.71), 4.871 (7.33), 4.912 (1.62), 7.234 (2.19), 7.246 (1.90), 7.373 (3.90), 7.396 (5.33), 7.418 (3.24), 7.427 (3.24), 7.450 (2.95).

### Beispiel 324

### (5RS,8RS)-2-(2,4-Difluorbenzyl)-5-{[(3R,4S)-3,4-difluorpyrrolidin-1-yl]carbonyl}-8-methyl-5,6,7,8-tetrahydro[1,2,4]triazolo[4,3-a]pyridin-3(2H)-on (Diastereomerengemisch; 4 Isomere)

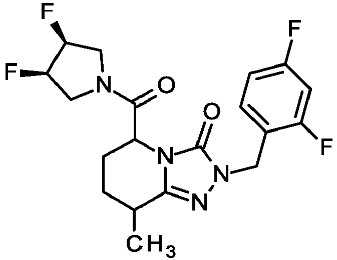

(5RS,8RS)-2-(2,4-Difluorbenzyl)-8-methyl-3-oxo-2,3,5,6,7,8-hexahydro[1,2,4]triazolo[4,3-a]pyridin-5-carbonsäure (Diastereomerengemisch; 4 Isomere) (80.0 mg, 247 µmol) wurde in THF (2.0 ml) vorgelegt und HBTU (122 mg, 322 µmol) und N,N-Diisopropylethylamin (130 µl, 740 µmol) wurden anschließend zugegeben. Nach 15 min Rühren bei Raumtemperatur wurde (3R,4S)-3,4-Difluorpyrrolidinhydrochlorid (42.6 mg, 297 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 46.0 mg (45 % d. Th.) der Titelverbindung erhalten.
LC-MS (Methode 4): Rₜ = 0.80 min; MS (ESIpos): m/z = 413 [M+H]⁺
¹H-NMR (400 MHz, DMSO-d6) δ [ppm]: -0.149 (0.68), -0.008 (6.34), 0.008 (6.42), 0.146 (0.75), 1.165 (15.62), 1.182 (16.00), 1.337 (1.36), 1.370 (1.51), 1.398 (0.68), 1.810 (1.36), 2.041 (2.19), 2.072 (2.72), 2.328 (1.28), 2.366 (0.98), 2.523 (4.60), 2.670 (1.74), 2.700 (1.36), 2.710 (2.42), 2.726 (1.58), 2.742 (1.13), 3.381 (0.83), 3.398 (0.53), 3.448 (0.68), 3.482 (1.13), 3.508 (1.13), 3.519 (1.13), 3.529 (1.06), 3.538 (1.13), 3.562 (0.83), 3.607 (0.83), 3.622 (0.98), 3.640 (0.68), 3.663 (1.13), 3.677 (1.74), 3.695 (1.36), 3.710 (1.66), 3.725 (0.98), 3.742 (1.06), 3.755 (1.13), 3.775 (0.53), 3.856 (0.83), 3.905 (1.28), 3.919 (0.98), 3.934 (0.53), 3.955 (0.91), 3.970 (0.91), 3.985 (0.60), 3.998 (0.53), 4.119 (0.60), 4.135 (0.68), 4.148 (0.60), 4.162 (0.98), 4.174 (0.68), 4.188 (0.68), 4.203 (0.60), 4.788 (2.34), 4.805 (3.25), 4.827 (5.96), 4.884 (3.02), 4.894 (3.40), 4.924 (1.36), 4.934 (1.66), 5.275 (1.06), 5.349 (0.83), 5.385 (1.06), 5.407 (0.91), 5.428 (0.68), 5.440 (0.68), 5.479 (0.75), 7.059 (1.28), 7.081 (2.79), 7.102 (1.58), 7.222 (1.28), 7.251 (3.47), 7.262 (1.89), 7.272 (3.25), 7.289 (1.96), 7.310 (0.91).

### Beispiel 325

### (5RS,8RS)-5-{[(3R,4S)-3,4-Difluorpyrrolidin-1-yl]carbonyl}-8-methyl-2-{[6-(trifluormethyl)pyridin-3-yl]methyl}-5,6,7,8-tetrahydro[1,2,4]triazolo[4,3-a]pyridin-3(2H)-on (Diastereomerengemisch; 4 Isomere)

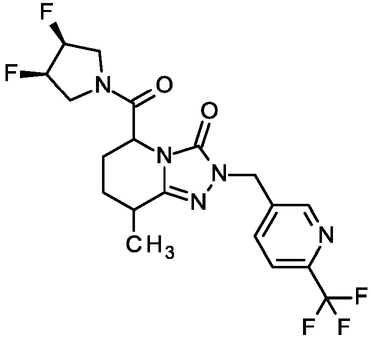

(5RS,8RS)-8-Methyl-3-oxo-2-{[6-(trifluormethyl)pyridin-3-yl]methyl}-2,3,5,6,7,8-hexahydro[1,2,4]triazolo[4,3-a]pyridin-5-carbonsäure (Diastereomerengemisch; 4 Isomere) (80.0 mg, 225 µmol) wurde in THF (2.0 ml) vorgelegt und HBTU (111 mg, 292 µmol) und N,N-Diisopropylethylamin (120 µl, 670 µmol) wurden anschließend zugegeben. Nach 15 min Rühren bei Raumtemperatur wurde (3R,4S)-3,4-Difluorpyrrolidinhydrochlorid (38.7 mg, 269 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 70.0 mg (70 % d. Th.) der Titelverbindung erhalten.
LC-MS (Methode 4): Rₜ = 0.78 min; MS (ESIpos): m/z = 446 [M+H]⁺
¹H-NMR (400 MHz, DMSO-d6) δ [ppm]: -0.150 (0.54), -0.008 (4.71), 0.008 (5.18), 0.146 (0.58), 1.173 (15.46), 1.190 (16.00), 1.225 (1.08), 1.244 (5.33), 1.259 (6.38), 1.274 (3.52), 1.305 (0.54), 1.337 (1.35), 1.347 (1.04), 1.369 (1.47), 1.399 (0.66), 1.799 (1.00), 1.819 (1.35), 2.072 (2.36), 2.096 (2.67), 2.328 (0.77), 2.366 (0.58), 2.523 (2.67), 2.670 (0.85), 2.690 (0.77), 2.709 (1.12), 2.721 (1.20), 2.737 (1.62), 2.748 (1.55), 2.776 (0.62), 3.129 (0.46), 3.139 (0.50), 3.158 (0.46), 3.457 (0.66), 3.491 (1.04), 3.515 (1.08), 3.527 (1.00), 3.538 (0.93), 3.547 (1.08), 3.560 (0.73), 3.570 (0.77), 3.579 (0.62), 3.616 (1.28), 3.630 (1.31), 3.648 (0.77), 3.663 (0.81), 3.671 (1.16), 3.684 (1.74), 3.696 (1.12), 3.718 (1.74), 3.731 (0.97), 3.750 (1.04), 3.761 (1.04), 3.783 (0.54), 3.795 (0.50), 3.867 (0.85), 3.913 (1.24), 3.926 (0.97), 3.941 (0.50), 3.964 (0.89), 3.977 (0.89), 3.992 (0.54), 4.006 (0.46), 4.128 (0.50), 4.142 (0.58), 4.156 (0.62), 4.167 (0.85), 4.183 (0.66), 4.195 (0.62), 4.210 (0.58), 4.835 (3.32), 4.842 (2.74), 4.849 (3.09), 5.006 (0.54), 5.047 (12.83), 5.093 (0.46), 5.266 (0.93), 5.276 (0.97), 5.288 (0.93), 5.314 (0.70), 5.329 (0.73), 5.353 (0.77), 5.390 (0.97), 5.409 (0.93), 5.420 (0.93), 5.432 (0.62), 5.456 (0.73), 5.464 (0.77), 5.476 (0.73), 7.904 (9.12), 7.908 (11.32), 7.926 (1.00), 8.628 (5.80).

### Beispiel 326

### (5RS,8RS)-2-(2,4-Difluorbenzyl)-5-{[(3S)-3-fluorpyrrolidin-1-yl]carbonyl}-8-methyl-5,6,7,8-tetrahydro[1,2,4]triazolo[4,3-a]pyridin-3(2H)-on (Diastereomerengemisch; 4 Isomere)

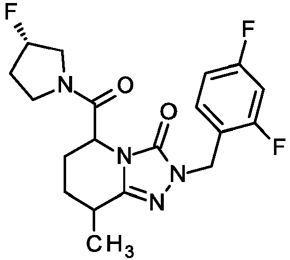

(5RS,8RS)-2-(2,4-Difluorbenzyl)-8-methyl-3-oxo-2,3,5,6,7,8-hexahydro[1,2,4]triazolo[4,3-a]pyridin-5-carbonsäure (Diastereomerengemisch; 4 Isomere) (80.0 mg, 247 µmol) wurde in THF vorgelegt und HBTU (122 mg, 322 µmol) und N,N-Diisopropylethylamin (130 µl, 740 µmol) wurden anschließend zugegeben. Nach 15 min Rühren bei Raumtemperatur wurde (3S)-3-Fluorpyrrolidinhydrochlorid (37.3 mg, 297 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 50.0 mg (51 % d. Th.) der Titelverbindung erhalten.
LC-MS (Methode 3): Rₜ = 1.43 min; MS (ESIpos): m/z = 395 [M+H]⁺
¹H-NMR (400 MHz, DMSO-d6) δ [ppm]: -0.149 (0.66), -0.008 (6.12), 0.008 (5.45), 0.146 (0.66), 1.168 (15.16), 1.173 (15.96), 1.184 (16.00), 1.190 (15.82), 1.228 (0.49), 1.245 (1.99), 1.261 (2.84), 1.277 (1.42), 1.349 (1.02), 1.380 (1.46), 1.412 (1.51), 1.443 (1.02), 1.787 (1.86), 1.796 (1.95), 1.805 (1.91), 1.934 (0.58), 1.975 (1.06), 2.052 (3.28), 2.092 (3.63), 2.102 (3.59), 2.130 (1.82), 2.221 (1.11), 2.256 (1.42), 2.328 (0.84), 2.366 (0.49), 2.669 (1.24), 2.690 (1.46), 2.700 (2.04), 2.714 (2.53), 2.730 (2.53), 2.743 (1.29), 2.890 (0.80), 3.267 (0.49), 3.356 (1.73), 3.374 (1.20), 3.384 (1.33), 3.402 (0.71), 3.447 (0.58), 3.455 (0.62), 3.481 (0.71), 3.490 (0.80), 3.515 (1.99), 3.542 (1.60), 3.560 (1.37), 3.592 (2.61), 3.619 (1.73), 3.640 (2.13), 3.664 (1.95), 3.687 (0.93), 3.704 (0.93), 3.726 (1.99), 3.752 (1.29), 3.762 (1.55), 3.836 (1.82), 3.904 (0.66), 3.926 (1.24), 3.949 (0.66), 3.969 (0.62), 3.995 (0.66), 4.026 (0.53), 4.697 (1.11), 4.711 (1.20), 4.745 (1.15), 4.754 (1.86), 4.766 (1.20), 4.784 (2.17), 4.792 (2.30), 4.824 (5.67), 4.832 (6.12), 4.872 (1.51), 4.890 (5.81), 4.930 (2.66), 5.258 (1.42), 5.346 (0.84), 5.391 (1.60), 5.477 (0.80), 5.509 (0.66), 5.943 (0.44), 7.054 (1.91), 7.060 (2.04), 7.075 (4.25), 7.081 (4.61), 7.097 (2.39), 7.103 (2.48), 7.220 (2.39), 7.227 (2.39), 7.245 (5.32), 7.252 (5.27), 7.269 (5.36), 7.274 (4.70), 7.284 (2.70), 7.290 (3.50), 7.305 (1.11), 7.312 (1.20).

### Beispiel 327

### (5RS,8RS)-2-(2,4-Difluorbenzyl)-5-[(3,3-difluorpyrrolidin-1-yl)carbonyl]-8-methyl-5,6,7,8-tetrahydro[1,2,4]triazolo[4,3-a]pyridin-3(2H)-on (Diastereomerengemisch; 4 Isomere)

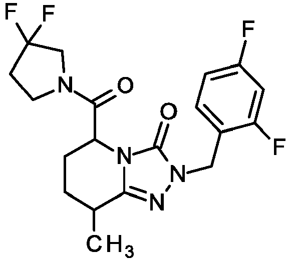

(5RS,8RS)-2-(2,4-Difluorbenzyl)-8-methyl-3-oxo-2,3,5,6,7,8-hexahydro[1,2,4]triazolo[4,3-a]pyridin-5-carbonsäure (Diastereomerengemisch; 4 Isomere) (80.0 mg, 247 µmol) wurde in THF (2.0 ml) vorgelegt und HBTU (122 mg, 322 µmol) und N,N-Diisopropylethylamin (130 µl, 740 µmol) wurden anschließend zugegeben. Nach 15 min Rühren bei Raumtemperatur wurde 3,3-Difluorpyrrolidinhydrochlorid (42.6 mg, 297 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 30.0 mg (29 % d. Th.) der Titelverbindung erhalten.
LC-MS (Methode 3): Rₜ = 1.57 min; MS (ESIpos): m/z = 413 [M+H]⁺
¹H-NMR (400 MHz, DMSO-d6) δ [ppm]: -0.150 (1.37), -0.008 (11.75), 0.008 (10.16), 0.146 (1.30), 1.166 (15.78), 1.183 (16.00), 1.370 (1.30), 1.787 (1.73), 1.820 (1.51), 2.055 (2.67), 2.082 (2.45), 2.092 (2.31), 2.327 (1.66), 2.366 (1.59), 2.377 (1.08), 2.405 (1.37), 2.425 (1.37), 2.447 (1.23), 2.569 (1.87), 2.586 (1.23), 2.670 (1.87), 2.690 (1.44), 2.708 (2.59), 2.720 (2.38), 2.735 (1.80), 2.750 (1.15), 3.525 (1.87), 3.543 (2.59), 3.558 (1.30), 3.626 (0.43), 3.660 (1.51), 3.693 (1.66), 3.731 (1.23), 3.766 (2.67), 3.793 (2.38), 3.812 (0.86), 3.876 (0.79), 3.894 (1.51), 3.921 (1.15), 3.940 (0.94), 3.970 (1.01), 4.013 (1.01), 4.043 (0.65), 4.138 (0.58), 4.168 (0.94), 4.193 (0.94), 4.788 (4.18), 4.828 (6.13), 4.847 (2.45), 4.890 (6.05), 4.930 (2.81), 7.059 (1.59), 7.081 (3.32), 7.102 (1.87), 7.221 (2.09), 7.227 (1.87), 7.247 (4.68), 7.269 (4.97), 7.276 (2.52), 7.285 (3.39), 7.306 (1.37).

### Beispiel 328

### (5RS,8RS)-5-[(3-Fluorazetidin-1 -yl)carbonyl]-8-methyl-2-{[6-(trifluormethyl)pyridin-3-yl]methyl}-5,6,7,8-tetrahydro[1,2,4]triazolo[4,3-a]pyridin-3(2H)-on (Diastereomerengemisch; 4 Isomere)

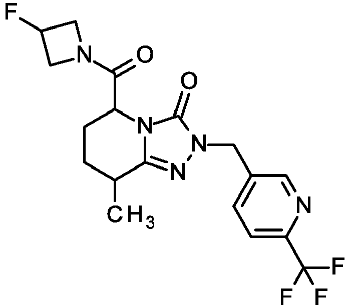

(5RS,8RS)-8-Methyl-3-oxo-2-{[6-(trifluormethyl)pyridin-3-yl]methyl}-2,3,5,6,7,8-hexahydro[1,2,4]triazolo[4,3-a]pyridin-5-carbonsäure (Diastereomerengemisch; 4 Isomere) (50.0 mg, 140 µmol) wurde in THF (500 µl) vorgelegt und HBTU (69.2 mg, 182 µmol) und N,N-Diisopropylethylamin (73 µl, 420 µmol) wurden anschließend zugegeben. Nach 15 min Rühren bei Raumtemperatur wurde 3-Fluorazetidinhydrochlorid (18.8 mg, 168 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 35.0 mg (60 % d. Th.) der Titelverbindung erhalten.
LC-MS (Methode 3): Rₜ = 1.34 min; MS (ESIpos): m/z = 414 [M+H]⁺
¹H-NMR (400 MHz, DMSO-d6) δ [ppm]: -0.008 (3.37), 0.008 (3.14), 0.146 (0.42), 1.168 (15.69), 1.185 (16.00), 1.325 (0.46), 1.365 (0.98), 1.385 (1.02), 1.396 (1.08), 1.425 (0.50), 1.813 (1.06), 1.822 (1.08), 2.057 (2.54), 2.328 (0.66), 2.332 (0.52), 2.366 (0.56), 2.523 (2.02), 2.670 (0.75), 2.700 (0.79), 2.711 (1.35), 2.730 (1.41), 2.744 (1.21), 2.759 (0.69), 3.908 (0.44), 3.930 (0.77), 3.958 (0.75), 3.991 (0.83), 4.017 (0.52), 4.177 (0.44), 4.220 (0.71), 4.234 (0.71), 4.275 (0.79), 4.303 (0.77), 4.341 (0.48), 4.363 (0.79), 4.386 (0.56), 4.423 (0.44), 4.448 (0.58), 4.530 (0.44), 4.569 (0.44), 4.584 (0.48), 4.619 (3.03), 4.673 (0.44), 5.004 (0.56), 5.046 (7.13), 5.096 (0.39), 5.349 (0.52), 5.411 (0.52), 5.493 (0.52), 5.554 (0.52), 7.906 (11.32), 8.635 (4.70).

### Beispiel 329

### (5RS,8RS)-2-(2,4-Difluorbenzyl)-5-[(3-fluorazetidin-1-yl)carbonyl]-8-methyl-5,6,7,8-tetrahydro[1,2,4]triazolo[4,3-a]pyridin-3(2H)-on (Diastereomerengemisch; 4 Isomere)

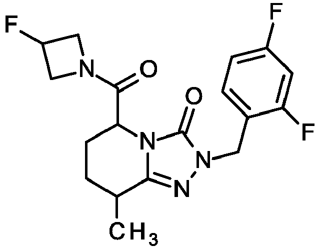

(5RS,8RS)-2-(2,4-Difluorbenzyl)-8-methyl-3-oxo-2,3,5,6,7,8-hexahydro[1,2,4]triazolo[4,3-a]pyridin-5-carbonsäure (Diastereomerengemisch; 4 Isomere) (80.0 mg, 247 µmol) wurde in THF (2.0 ml) vorgelegt und HBTU (122 mg, 322 µmol) und N,N-Diisopropylethylamin (130 µl, 740 µmol) wurden anschließend zugegeben. Nach 15 min Rühren bei Raumtemperatur wurde 3-Fluorazetidinhydrochlorid (33.1 mg, 297 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 55.0 mg (58 % d. Th.) der Titelverbindung erhalten.
LC-MS (Methode 3): Rₜ = 1.46 min; MS (ESIpos): m/z = 381 [M+H]⁺
¹H-NMR (400 MHz, DMSO-d6) δ [ppm]: -0.149 (1.04), -0.008 (13.04), 0.008 (7.58), 0.146 (1.08), 1.160 (16.00), 1.177 (16.00), 1.334 (0.54), 1.393 (1.18), 1.434 (0.61), 1.805 (1.28), 2.037 (2.96), 2.327 (1.01), 2.366 (0.74), 2.670 (1.55), 2.696 (1.48), 2.710 (2.36), 3.920 (0.91), 3.951 (0.84), 3.984 (0.94), 4.008 (0.61), 4.227 (0.88), 4.263 (1.01), 4.295 (0.94), 4.351 (0.94), 4.410 (0.51), 4.439 (0.64), 4.518 (0.54), 4.583 (3.60), 4.790 (2.49), 4.830 (5.36), 4.886 (2.59), 4.934 (1.21), 5.346 (0.64), 5.408 (0.57), 5.489 (0.57), 5.552 (0.57), 7.057 (1.21), 7.077 (2.39), 7.095 (1.35), 7.226 (1.25), 7.251 (3.20), 7.272 (3.40), 7.290 (2.36), 7.311 (0.94).

### Beispiel 330

### (5RS,8RS)-5-[(3,3-Difluorpyrrolidin-1-yl)carbonyl]-8-methyl-2-{[6-(trifluormethyl)pyridin-3-yl]methyl}-5,6,7,8-tetrahydro[1,2,4]triazolo[4,3-a]pyridin-3(2H)-on (Diastereomerengemisch; 4 Isomere)

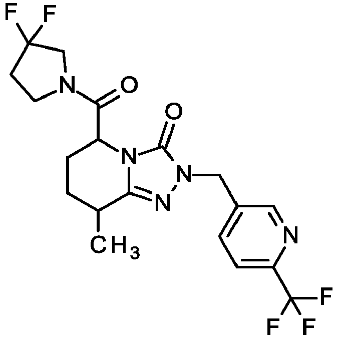

(5RS,8RS)-8-Methyl-3-oxo-2-{[6-(trifluormethyl)pyridin-3-yl]methyl}-2,3,5,6,7,8-hexahydro[1,2,4]triazolo[4,3-a]pyridin-5-carbonsäure (Diastereomerengemisch; 4 Isomere) (100 mg, 281 µmol) wurde in THF (2.0 ml) vorgelegt und HBTU (138 mg, 365 µmol) und N,N-Diisopropylethylamin (150 µl, 840 µmol) wurden anschließend zugegeben. Nach 15 min Rühren bei Raumtemperatur wurde 3,3-Difluorpyrrolidinhydrochlorid (48.4 mg, 337 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 60.0 mg (48 % d. Th.) der Titelverbindung erhalten.
LC-MS (Methode 4): Rₜ = 0.81 min; MS (ESIpos): m/z = 446 [M+H]⁺
¹H-NMR (400 MHz, DMSO-d6) δ [ppm]: -0.008 (3.55), 0.008 (3.57), 0.146 (0.46), 1.175 (15.46), 1.191 (16.00), 1.317 (0.44), 1.335 (0.95), 1.349 (1.15), 1.364 (1.25), 1.379 (1.25), 1.395 (0.88), 1.407 (0.64), 1.805 (1.66), 1.817 (1.52), 1.827 (1.39), 1.839 (1.39), 2.073 (3.67), 2.105 (2.42), 2.115 (2.28), 2.328 (0.61), 2.366 (0.76), 2.381 (0.95), 2.390 (0.76), 2.408 (1.25), 2.427 (1.22), 2.451 (0.78), 2.518 (4.01), 2.572 (1.81), 2.589 (1.27), 2.607 (0.66), 2.670 (0.71), 2.696 (0.42), 2.711 (1.52), 2.728 (1.91), 2.743 (2.30), 2.758 (1.81), 2.773 (1.17), 3.533 (1.79), 3.552 (2.69), 3.566 (1.42), 3.573 (1.37), 3.633 (0.46), 3.667 (1.49), 3.701 (1.74), 3.736 (1.03), 3.742 (0.95), 3.756 (0.66), 3.775 (2.98), 3.801 (1.96), 3.807 (1.76), 3.818 (0.91), 3.841 (0.44), 3.882 (0.78), 3.901 (1.64), 3.920 (0.95), 3.927 (1.22), 3.947 (0.93), 3.977 (0.98), 3.992 (0.56), 4.005 (0.71), 4.021 (0.91), 4.048 (0.59), 4.151 (0.61), 4.183 (0.93), 4.206 (0.93), 4.238 (0.46), 4.805 (1.37), 4.812 (1.96), 4.824 (1.49), 4.868 (1.39), 4.875 (2.15), 4.887 (1.47), 5.006 (0.59), 5.050 (12.84), 5.093 (0.66), 7.881 (0.71), 7.906 (15.24), 7.927 (1.35), 8.629 (5.70).

### Beispiel 331

### (5RS,7RS)-2-(3-Chlor-4-fluorbenzyl)-5-[(3,3-difluorpyrrolidin-1-yl)carbonyl]-7-methyl-5,6,7,8-tetrahydro[1,2,4]triazolo[4,3-a]pyridin-3(2H)-on (Diastereomerengemisch; 4 Isomere)

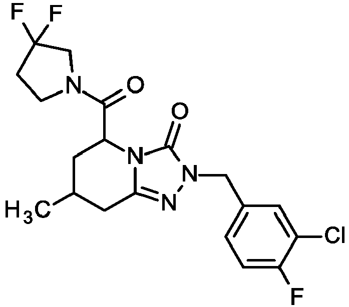

(5RS,7RS)-2-(3-Chlor-4-fluorbenzyl)-7-methyl-3-oxo-2,3,5,6,7,8-hexahydro[1,2,4]triazolo[4,3-a]pyridin-5-carbonsäure (Diastereomerengemisch; 4 Isomere) (70.0 mg, 206 µmol) wurde in THF (2.0 ml) vorgelegt und HBTU (102 mg, 268 µmol) und N,N-Diisopropylethylamin (110 µl, 620 µmol) wurden anschließend zugegeben. Nach 15 min Rühren bei Raumtemperatur wurde 3,3-Difluorpyrrolidinhydrochlorid (35.5 mg, 247 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 47.8 mg (54 % d. Th.) der Titelverbindung erhalten.
LC-MS (Methode 3): Rₜ = 1.69 min; MS (ESIpos): m/z = 429 [M+H]⁺
¹H-NMR (400 MHz, DMSO-d6) δ [ppm]: -0.149 (1.30), -0.008 (11.60), 0.008 (9.50), 0.146 (1.20), 1.025 (12.50), 1.041 (12.80), 1.343 (1.20), 1.371 (1.30), 1.873 (1.00), 2.073 (0.90), 2.158 (2.40), 2.188 (2.00), 2.198 (2.80), 2.228 (3.60), 2.327 (2.40), 2.366 (1.70), 2.413 (1.60), 2.651 (2.00), 2.669 (2.40), 2.710 (1.40), 3.555 (2.00), 3.570 (2.20), 3.679 (1.40), 3.712 (1.40), 3.739 (0.90), 3.771 (1.60), 3.803 (1.30), 3.836 (1.70), 3.915 (1.50), 3.941 (1.00), 4.043 (1.00), 4.081 (0.90), 4.110 (1.20), 4.141 (1.20), 4.170 (0.90), 4.591 (1.20), 4.607 (1.40), 4.619 (1.40), 4.633 (1.20), 4.705 (1.20), 4.719 (1.30), 4.730 (1.20), 4.746 (1.10), 4.802 (16.00), 7.240 (1.70), 7.256 (2.50), 7.262 (2.40), 7.268 (2.20), 7.376 (4.10), 7.399 (5.60), 7.421 (3.10), 7.443 (3.20), 7.448 (3.30), 7.461 (3.20), 7.466 (3.10).

### Beispiel 332

### (5RS,7RS)-5-[(3,3-Difluorpyrrolidin-1-yl)carbonyl]-7-methyl-2-{[6-(trifluormethyl)pyridin-3-yl]methyl}-5,6,7,8-tetrahydro[1,2,4]triazolo[4,3-a]pyridin-3(2H)-on (Diastereomerengemisch; 4 Isomere)

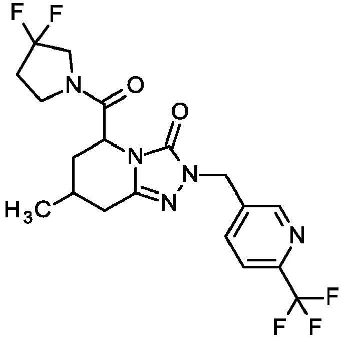

(5RS,7RS)-7-Methyl-3-oxo-2-{[6-(trifluormethyl)pyridin-3-yl]methyl}-2,3,5,6,7,8-hexahydro[1,2,4]triazolo[4,3-a]pyridin-5-carbonsäure (Diastereomerengemisch; 4 Isomere) (100 mg, 281 µmol) wurde in THF (2.0 ml) vorgelegt und HBTU (138 mg, 365 µmol) und N,N-Diisopropylethylamin (150 µl, 840 µmol) wurden anschließend zugegeben. Nach 15 min Rühren bei Raumtemperatur wurde 3,3-Difluorpyrrolidinhydrochlorid (48.4 mg, 337 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 106 mg (85 % d. Th.) der Titelverbindung erhalten.
LC-MS (Methode 4): Rₜ = 0.79 min; MS (ESIpos): m/z = 446 [M+H]⁺
¹H-NMR (400 MHz, DMSO-d6) δ [ppm]: -0.149 (0.75), -0.008 (7.11), 0.008 (6.34), 0.146 (0.75), 1.027 (12.01), 1.043 (12.42), 1.318 (0.55), 1.332 (0.57), 1.349 (1.14), 1.361 (1.21), 1.379 (1.21), 1.391 (1.23), 1.410 (0.93), 1.422 (0.62), 1.900 (0.89), 2.073 (0.50), 2.160 (2.26), 2.190 (1.94), 2.201 (2.60), 2.231 (2.85), 2.270 (1.30), 2.328 (1.00), 2.366 (1.09), 2.382 (0.96), 2.400 (0.98), 2.414 (1.39), 2.433 (1.09), 2.453 (0.78), 2.523 (4.67), 2.577 (1.18), 2.595 (0.61), 2.651 (1.98), 2.670 (1.32), 2.686 (1.76), 2.710 (0.93), 3.539 (1.19), 3.557 (2.05), 3.571 (2.33), 3.590 (1.05), 3.648 (0.43), 3.681 (1.25), 3.714 (1.50), 3.740 (0.84), 3.773 (1.53), 3.805 (1.34), 3.818 (1.03), 3.825 (0.96), 3.843 (1.59), 3.861 (0.71), 3.893 (0.73), 3.912 (1.50), 3.938 (1.02), 3.957 (0.43), 4.051 (1.00), 4.089 (0.98), 4.108 (0.73), 4.138 (1.10), 4.170 (0.93), 4.594 (1.10), 4.609 (1.35), 4.621 (1.25), 4.636 (1.10), 4.705 (1.14), 4.720 (1.35), 4.732 (1.23), 4.747 (1.16), 4.988 (16.00), 7.900 (1.03), 7.920 (14.02), 7.946 (0.73), 8.665 (5.02).

### Beispiel 333

### (5RS,7RS)-5-{[(3R,4S)-3,4-Difluorpyrrolidin-1-yl]carbonyl}-2-{[3-fluor-2-(trifluormethyl)pyridin-4-yl]methyl}-7-methyl-5,6,7,8-tetrahydro[1,2,4]triazolo[4,3-a]pyridin-3(2H)-on (Diastereomerengemisch; 4 Isomere)

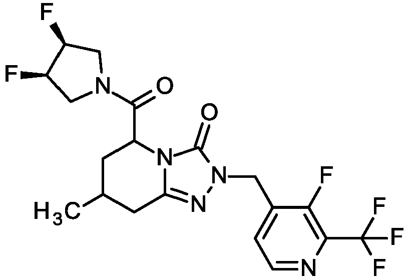

(5RS,7RS)-2-{[3-Fluor-2-(trifluormethyl)pyridin-4-yl]methyl}-7-methyl-3-oxo-2,3,5,6,7,8-hexahydro[1,2,4]triazolo[4,3-a]pyridin-5-carbonsäure (Diastereomerengemisch; 4 Isomere) (81.4 mg, 90 % Reinheit, 196 µmol) wurde in THF (2.0 ml) vorgelegt und HBTU (96.5 mg, 254 µmol) und N,N-Diisopropylethylamin (100 µl, 590 µmol) wurden anschließend zugegeben. Nach 15 min Rühren bei Raumtemperatur wurde (3R,4S)-3,4-Difluorpyrrolidinhydrochlorid (33.7 mg, 235 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 41.5 mg (46 % d. Th.) der Titelverbindung erhalten.
LC-MS (Methode 3): Rₜ = 1.49 min; MS (ESIpos): m/z = 464 [M+H]⁺
¹H-NMR (400 MHz, DMSO-d6) δ [ppm]: -0.149 (1.08), -0.008 (9.90), 0.008 (8.58), 0.146 (1.08), 1.029 (12.29), 1.033 (12.77), 1.045 (13.13), 1.050 (12.49), 1.301 (0.76), 1.332 (1.72), 1.364 (2.63), 1.393 (1.84), 1.427 (0.84), 1.892 (1.76), 2.073 (2.95), 2.171 (3.51), 2.201 (2.95), 2.212 (4.07), 2.242 (4.15), 2.259 (1.96), 2.276 (1.72), 2.291 (1.76), 2.327 (1.20), 2.366 (0.88), 2.523 (3.99), 2.661 (3.15), 2.665 (3.27), 2.690 (3.87), 2.697 (2.51), 3.463 (0.84), 3.472 (0.60), 3.497 (1.32), 3.508 (1.28), 3.517 (1.16), 3.533 (0.84), 3.545 (1.48), 3.552 (1.60), 3.597 (1.08), 3.617 (0.96), 3.631 (1.24), 3.652 (0.64), 3.665 (0.88), 3.675 (0.96), 3.690 (1.36), 3.707 (1.52), 3.728 (1.28), 3.741 (1.40), 3.756 (1.52), 3.770 (1.72), 3.792 (1.28), 3.803 (1.60), 3.823 (1.08), 3.872 (0.68), 3.987 (0.84), 4.002 (0.84), 4.015 (0.88), 4.032 (1.20), 4.048 (0.84), 4.061 (0.84), 4.076 (0.60), 4.108 (0.76), 4.123 (0.84), 4.136 (0.80), 4.151 (1.56), 4.167 (0.96), 4.180 (0.84), 4.195 (0.72), 4.662 (2.11), 4.672 (2.39), 4.677 (2.47), 4.684 (2.43), 4.689 (2.31), 4.699 (2.11), 5.040 (16.00), 5.106 (0.44), 5.250 (1.16), 5.260 (1.24), 5.269 (1.20), 5.284 (1.12), 5.306 (0.84), 5.321 (1.04), 5.341 (1.08), 5.356 (0.84), 5.373 (1.20), 5.406 (1.16), 5.436 (0.88), 5.449 (1.04), 5.459 (1.04), 5.471 (0.96), 5.485 (0.76), 5.494 (0.56), 7.589 (3.31), 7.602 (6.26), 7.615 (3.55), 8.568 (5.87), 8.579 (5.67).

### Beispiel 334

### (5RS,7RS)-5-[(3,3-Difluorpyrrolidin-1 -yl)carbonyl]-2-{[3-fluor-2-(trifluormethyl)pyridin-4-yl]methyl}-7-methyl-5,6,7,8-tetrahydro[1,2,4]triazolo[4,3-a]pyridin-3(2H)-on (Diastereomerengemisch; 4 Isomere)

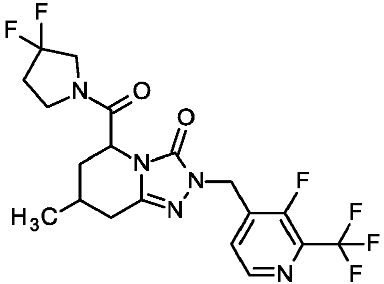

(5RS,7RS)-2-{[3-Fluor-2-(trifluormethyl)pyridin-4-yl]methyl}-7-methyl-3-oxo-2,3,5,6,7,8-hexahydro[1,2,4]triazolo[4,3-a]pyridin-5-carbonsäure (Diastereomerengemisch; 4 Isomere) (81.4 mg, 90 % Reinheit, 196 µmol) wurde in THF (2.0 ml) vorgelegt und HBTU (96.5 mg, 254 µmol) und N,N-Diisopropylethylamin (100 µl, 590 µmol) wurden anschließend zugegeben. Nach 15 min Rühren bei Raumtemperatur wurde 3,3-Difluorpyrrolidinhydrochlorid (33.7 mg, 235 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 36.3 mg (40 % d. Th.) der Titelverbindung erhalten.
LC-MS (Methode 3): Rₜ = 1.56 min; MS (ESIpos): m/z = 464 [M+H]⁺
¹H-NMR (400 MHz, DMSO-d6) δ [ppm]: -0.149 (0.84), -0.008 (7.15), 0.008 (6.47), 0.146 (0.84), 1.033 (11.98), 1.049 (12.26), 1.327 (0.53), 1.342 (0.59), 1.357 (1.11), 1.372 (1.21), 1.387 (1.21), 1.403 (1.21), 1.418 (0.65), 1.432 (0.65), 1.891 (1.21), 2.174 (2.35), 2.204 (2.01), 2.215 (2.75), 2.245 (3.03), 2.283 (1.27), 2.327 (0.99), 2.366 (0.90), 2.381 (0.99), 2.401 (0.99), 2.414 (1.36), 2.432 (1.21), 2.454 (0.87), 2.524 (3.78), 2.559 (1.70), 2.576 (1.05), 2.596 (0.59), 2.665 (2.48), 2.709 (1.83), 3.541 (1.15), 3.550 (1.33), 3.558 (1.98), 3.570 (2.10), 3.589 (1.02), 3.683 (1.21), 3.717 (1.42), 3.738 (0.77), 3.751 (0.68), 3.770 (1.58), 3.803 (1.58), 3.823 (0.93), 3.831 (0.96), 3.848 (1.55), 3.866 (0.68), 3.888 (0.74), 3.908 (1.58), 3.933 (0.96), 3.952 (0.43), 4.056 (0.99), 4.094 (1.02), 4.131 (1.21), 4.165 (0.93), 4.603 (1.15), 4.618 (1.36), 4.630 (1.27), 4.645 (1.11), 4.713 (1.21), 4.728 (1.36), 4.740 (1.30), 4.755 (1.15), 5.042 (16.00), 7.591 (2.54), 7.603 (4.80), 7.616 (2.72), 8.568 (5.29), 8.580 (5.20).

### Beispiel 335

### (5RS,7RS)-5-[(3-Fluorazetidin-1 -yl)carbonyl]-2-{[3-fluor-2-(trifluormethyl)pyridin-4-yl]methyl}-7-methyl-5,6,7,8-tetrahydro[1,2,4]triazolo[4,3-a]pyridin-3(2H)-on (Diastereomerengemisch 1; Racemat)

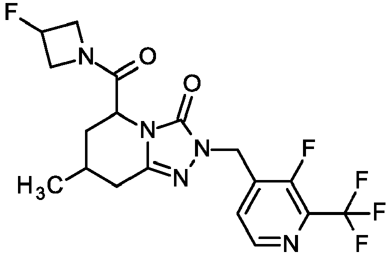

(5RS,7RS)-2-{[3-Fluor-2-(trifluormethyl)pyridin-4-yl]methyl}-7-methyl-3-oxo-2,3,5,6,7,8-hexahydro[1,2,4]triazolo[4,3-a]pyridin-5-carbonsäure (Diastereomerengemisch; 4 Isomere) (81.4 mg, 90 % Reinheit, 196 µmol) wurde in THF (2.0 ml) vorgelegt und HBTU (96.5 mg, 254 µmol) und N,N-Diisopropylethylamin (100 µl, 590 µmol) wurden anschließend zugegeben. Nach 15 min Rühren bei Raumtemperatur wurde 3-Fluorazetidinhydrochlorid (26.2 mg, 235 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 38.9 mg (55 % d. Th.) der Titelverbindung erhalten.
LC-MS (Methode 4): Rₜ = 0.75 min; MS (ESIpos): m/z = 432 [M+H]⁺
¹H-NMR (400 MHz, DMSO-d6) δ [ppm]: -0.149 (1.79), -0.008 (16.00), 0.008 (13.81), 0.146 (1.79), 1.025 (13.71), 1.041 (14.11), 1.388 (1.39), 1.417 (1.59), 1.435 (1.49), 1.467 (0.70), 1.894 (2.09), 2.170 (3.88), 2.200 (2.88), 2.210 (3.78), 2.240 (2.39), 2.327 (2.09), 2.366 (2.19), 2.523 (6.86), 2.648 (2.78), 2.670 (2.39), 2.679 (2.88), 2.710 (2.19), 2.857 (1.29), 3.103 (1.09), 3.880 (0.70), 3.966 (1.09), 4.025 (0.80), 4.153 (0.70), 4.221 (1.09), 4.248 (1.39), 4.273 (1.29), 4.305 (1.29), 4.337 (0.89), 4.396 (3.98), 4.411 (4.37), 4.424 (3.88), 4.439 (3.78), 4.465 (0.80), 4.510 (0.70), 4.561 (0.70), 4.651 (0.70), 5.042 (10.93), 5.107 (0.50), 5.380 (1.19), 5.523 (1.09), 7.603 (3.48), 7.616 (6.56), 7.628 (3.78), 8.567 (6.06), 8.579 (5.96).

### Beispiel 336

### (5RS,7RS)-5-{[(3R,4S)-3,4-Difluorpyrrolidin-1-yl]carbonyl}-7-methyl-2-{[6-(trifluormethyl)pyridin-3-yl]methyl}-5,6,7,8-tetrahydro[1,2,4]triazolo[4,3-a]pyridin-3(2H)-on (Diastereomerengemisch 1; Racemat)

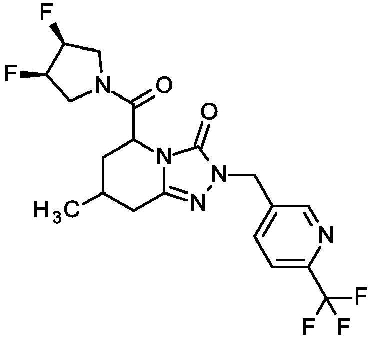

(5RS,7RS)-7-Methyl-3-oxo-2-{[6-(trifluormethyl)pyridin-3-yl]methyl}-2,3,5,6,7,8-hexahydro[1,2,4]triazolo[4,3-a]pyridin-5-carbonsäure (Diastereomerengemisch; 4 Isomere) (78.8 mg, 221 µmol) wurde in THF (2.0 ml) vorgelegt und HBTU (109 mg, 288 µmol) und N,N-Diisopropylethylamin (150 µl, 880 µmol) wurden anschließend zugegeben. Nach 15 min Rühren bei Raumtemperatur wurde (3R,4S)-3,4-Difluorpyrrolidinhydrochlorid (38.1 mg, 265 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 78.9 mg (80 % d. Th.) der Titelverbindung erhalten.
LC-MS (Methode 4): Rₜ = 0.76 min; MS (ESIpos): m/z = 446 [M+H]⁺
¹H-NMR (400 MHz, DMSO-d6) δ [ppm]: -0.149 (0.58), 0.146 (0.52), 1.023 (10.88), 1.027 (11.14), 1.039 (11.23), 1.290 (0.61), 1.322 (1.50), 1.354 (2.20), 1.385 (1.56), 1.417 (0.75), 1.874 (3.65), 2.157 (2.89), 2.187 (2.52), 2.197 (3.39), 2.228 (3.41), 2.246 (1.76), 2.260 (1.56), 2.278 (1.62), 2.327 (0.78), 2.366 (0.46), 2.642 (2.43), 2.683 (2.20), 2.710 (0.61), 3.461 (0.93), 3.495 (1.24), 3.504 (1.33), 3.513 (1.33), 3.548 (1.68), 3.598 (1.04), 3.617 (0.90), 3.631 (1.13), 3.651 (0.72), 3.664 (0.90), 3.674 (0.98), 3.687 (1.13), 3.697 (1.16), 3.709 (1.42), 3.721 (1.10), 3.731 (1.24), 3.744 (1.22), 3.772 (1.71), 3.807 (1.36), 3.842 (0.81), 3.884 (0.69), 3.982 (0.75), 3.997 (0.84), 4.010 (0.72), 4.027 (1.07), 4.043 (0.72), 4.056 (0.64), 4.070 (0.55), 4.115 (0.67), 4.130 (0.81), 4.143 (0.69), 4.158 (1.36), 4.174 (0.81), 4.187 (0.78), 4.202 (0.78), 4.648 (1.68), 4.654 (1.82), 4.663 (2.05), 4.671 (2.34), 4.682 (1.91), 4.690 (1.74), 4.696 (1.56), 4.986 (16.00), 5.054 (3.27), 5.249 (1.27), 5.260 (1.27), 5.270 (1.30), 5.282 (1.13), 5.322 (1.10), 5.340 (1.04), 5.384 (1.36), 5.407 (1.30), 5.460 (1.16), 5.486 (0.81), 6.088 (0.95), 7.917 (12.88), 8.663 (6.37).

### Beispiel 337

### (5S,7S)-5-{[(3R,4S)-3,4-Difluorpyrrolidin-1-yl]carbonyl}-7-methyl-2-{[6-(trifluormethyl)pyridin-3-yl]methyl}-5,6,7,8-tetrahydro[1,2,4]triazolo[4,3-a]pyridin-3(2H)-on (Enantiomer 1)

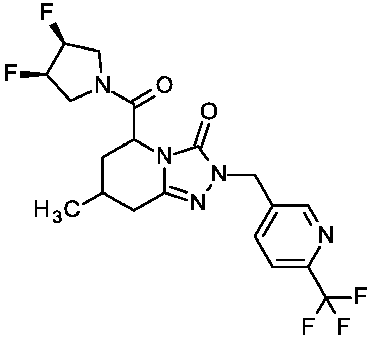

(5RS,7RS)-5-{[(3R,4S)-3,4-Difluorpyrrolidin-1-yl]carbonyl}-7-methyl-2-{[6-(trifluormethyl)pyridin-3-yl]methyl}-5,6,7,8-tetrahydro[1,2,4]triazolo[4,3-a]pyridin-3(2H)-on (Diastereomer 1; Racemat) wurde durch chirale präparative HPLC getrennt [Probenvorbereitung: 66.7 mg gelöst in 7 ml Ethanol; Injektionsvolumen: 0.15 ml; Säule: Daicel Chiralcel^{®} OD-H 5 µm, 250 x 20 mm; Laufmittel: n-Heptan/Ethanol (60:40); Fluss: 15 ml/min; Temperatur 25°C; UV-Detektion: 220 nm]. Nach der Trennung wurden 21.7 mg von Enantiomer 1, welches zuerst eluierte, und 26.4 mg von Enantiomer 2, welches später eluierte, isoliert.

### Enantiomer 1:

Analytische chirale HPLC: Rₜ = 2.78 min, e.e. = 100% [Säule: Daicel Chiralcel^{®} IB-3 50 x 4.6 mm; Laufmittel: n-Heptan/Ethanol 50:50; Fluss: 1 ml/min; UV-Detektion: 220 nm].
LC-MS (Methode 15): Rₜ = 1.31 min; MS (ESIpos): m/z = 446 [M+H]⁺
¹H-NMR (400 MHz, DMSO-d6) δ [ppm]: -0.149 (1.09), -0.008 (10.72), 0.008 (11.52), 0.146 (1.20), 1.023 (10.64), 1.028 (11.23), 1.039 (11.52), 1.044 (11.08), 1.236 (1.17), 1.290 (0.77), 1.322 (1.49), 1.354 (2.22), 1.386 (1.57), 1.418 (0.73), 1.875 (3.75), 2.157 (2.95), 2.187 (2.51), 2.198 (3.43), 2.228 (3.43), 2.245 (1.71), 2.263 (1.57), 2.279 (1.57), 2.328 (1.35), 2.643 (2.48), 2.670 (1.86), 2.679 (2.41), 2.710 (0.62), 3.461 (0.95), 3.495 (1.28), 3.504 (1.28), 3.514 (1.31), 3.548 (1.71), 3.598 (1.17), 3.616 (0.95), 3.630 (1.13), 3.650 (0.80), 3.664 (0.95), 3.674 (0.95), 3.688 (1.13), 3.697 (1.13), 3.709 (1.38), 3.721 (1.13), 3.731 (1.24), 3.744 (1.28), 3.772 (1.64), 3.807 (1.38), 3.841 (0.77), 3.934 (0.40), 3.982 (0.80), 3.997 (0.84), 4.010 (0.73), 4.027 (1.13), 4.043 (0.77), 4.056 (0.69), 4.070 (0.51), 4.115 (0.69), 4.130 (0.80), 4.143 (0.69), 4.159 (1.35), 4.173 (0.84), 4.186 (0.80), 4.202 (0.84), 4.648 (1.64), 4.655 (1.75), 4.663 (2.00), 4.670 (2.26), 4.682 (1.97), 4.690 (1.79), 4.697 (1.57), 4.986 (16.00), 5.055 (3.46), 5.249 (1.24), 5.260 (1.28), 5.271 (1.24), 5.322 (1.02), 5.340 (0.98), 5.406 (1.28), 5.461 (1.02), 6.086 (0.98), 7.916 (12.46), 7.919 (12.28), 8.664 (5.94).

### Beispiel 338

### (5RS,7RS)-5-{[(3R,4S)-3,4-Difluorpyrrolidin-1-yl]carbonyl}-7-methyl-2-{[6-(trifluormethyl)pyridin-3-yl]methyl}-5,6,7,8-tetrahydro[1,2,4]triazolo[4,3-a]pyridin-3(2H)-on (Enantiomer 2)

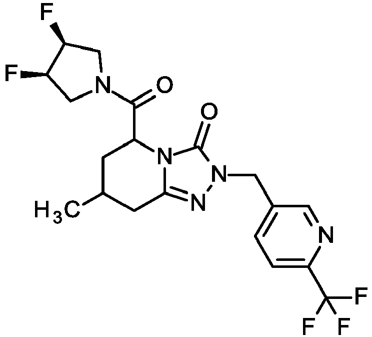

(5RS,7RS)-5-{[(3R,4S)-3,4-Difluorpyrrolidin-1-yl]carbonyl}-7-methyl-2-{[6-(trifluormethyl)pyridin-3-yl]methyl}-5,6,7,8-tetrahydro[1,2,4]triazolo[4,3-a]pyridin-3(2H)-on (Diastereomer 1) wurde durch chirale präparative HPLC getrennt [Probenvorbereitung: 66.7 mg gelöst in 7 ml Ethanol; Injektionsvolumen: 0.15 ml; Säule: Daicel Chiralcel^{®} OD-H 5 µm, 250 x 20 mm; Laufmittel: n-Heptan/Ethanol (60:40); Fluss: 15 ml/min; Temperatur 25°C; UV-Detektion: 220 nm]. Nach der Trennung wurden 21.7 mg von Enantiomer 1, welches zuerst eluierte, und 26.4 mg von Enantiomer 2, welches später eluierte, isoliert.

### Enantiomer 2:

Analytische chirale HPLC: Rₜ = 2.98 min, e.e. = 100% [Säule: Daicel Chiralcel^{®} IB-3 50 x 4.6 mm; Laufmittel: n-Heptan/Ethanol 50:50; Fluss: 1 ml/min; UV-Detektion: 220 nm].
LC-MS (Methode 4): Rₜ = 0.76 min; MS (ESIpos): m/z = 446 [M+H]⁺
¹H-NMR (400 MHz, DMSO-d6) δ [ppm]: -0.149 (1.26), -0.008 (10.77), 0.008 (10.65), 0.146 (1.26), 1.023 (10.81), 1.028 (11.26), 1.039 (11.65), 1.044 (11.19), 1.235 (1.03), 1.290 (0.76), 1.320 (1.49), 1.355 (2.14), 1.388 (1.53), 1.418 (0.73), 1.883 (1.49), 2.157 (3.17), 2.187 (2.60), 2.198 (3.63), 2.228 (3.55), 2.246 (1.76), 2.279 (1.57), 2.327 (1.45), 2.366 (0.42), 2.648 (2.41), 2.670 (1.79), 2.679 (2.33), 2.709 (0.50), 3.460 (0.80), 3.495 (0.99), 3.505 (1.11), 3.514 (1.15), 3.548 (1.53), 3.597 (0.92), 3.617 (0.84), 3.630 (0.99), 3.652 (0.57), 3.674 (0.84), 3.687 (1.03), 3.697 (0.92), 3.709 (1.22), 3.721 (1.03), 3.732 (0.99), 3.744 (0.99), 3.772 (1.41), 3.808 (1.26), 3.983 (0.76), 3.997 (0.80), 4.010 (0.76), 4.028 (1.07), 4.043 (0.76), 4.056 (0.69), 4.072 (0.53), 4.115 (0.65), 4.130 (0.76), 4.144 (0.69), 4.158 (1.26), 4.174 (0.73), 4.187 (0.69), 4.201 (0.65), 4.648 (1.57), 4.655 (1.76), 4.663 (2.06), 4.674 (2.33), 4.681 (2.02), 4.690 (1.76), 4.697 (1.60), 4.986 (16.00), 5.249 (1.07), 5.259 (1.07), 5.270 (1.11), 5.307 (0.76), 5.322 (0.84), 5.372 (1.03), 5.384 (1.11), 5.407 (1.07), 5.459 (0.99), 7.901 (0.95), 7.919 (12.18), 8.663 (6.07).

### Beispiel 339

### (5RS,7RS)-2-(3-Chlor-4-fluorbenzyl)-5-{[(3R,4S)-3,4-difluorpyrrolidin-1-yl]carbonyl}-7-methyl-5,6,7,8-tetrahydro[1,2,4]triazolo[4,3-a]pyridin-3(2H)-on (Diastereomerengemisch; 4 Isomere)

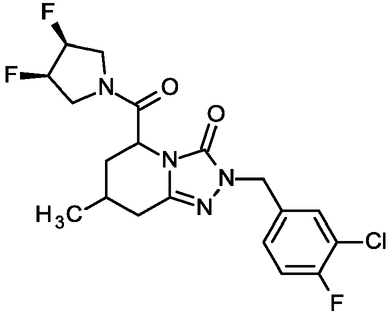

(5RS,7RS)-2-(3-Chlor-4-fluorbenzyl)-7-methyl-3-oxo-2,3,5,6,7,8-hexahydro[1,2,4]triazolo[4,3-a]pyridin-5-carbonsäure (Diastereomerengemisch; 4 Isomere) (70.0 mg, 206 µmol) wurde in THF (2.0 ml) vorgelegt und HBTU (85.9 mg, 227 µmol) und N,N-Diisopropylethylamin (110 µl, 620 µmol) wurden anschließend zugegeben. Nach 15 min Rühren bei Raumtemperatur wurde (3R,4S)-3,4-Difluorpyrrolidinhydrochlorid (35.5 mg, 247 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 54.2 mg (61 % d. Th.) der Titelverbindung erhalten.
LC-MS (Methode 3): Rₜ = 1.62 min; MS (ESIpos): m/z = 429 [M+H]⁺
¹H-NMR (400 MHz, DMSO-d6) δ [ppm]: -0.149 (1.33), 0.008 (11.11), 0.146 (1.21), 1.026 (10.32), 1.038 (10.57), 1.317 (1.39), 1.348 (1.69), 1.385 (1.39), 1.414 (0.78), 1.885 (1.39), 2.073 (3.68), 2.154 (2.84), 2.185 (2.23), 2.195 (3.26), 2.226 (3.62), 2.328 (2.29), 2.367 (1.39), 2.648 (2.23), 2.670 (2.66), 2.710 (1.51), 3.512 (1.21), 3.548 (1.39), 3.629 (1.03), 3.710 (1.33), 3.759 (1.57), 3.806 (1.27), 3.851 (0.91), 4.020 (1.03), 4.162 (1.33), 4.653 (1.69), 4.673 (2.17), 4.801 (16.00), 5.270 (1.03), 5.406 (1.09), 5.458 (0.97), 7.256 (2.35), 7.377 (2.96), 7.398 (4.35), 7.418 (2.11), 7.444 (3.50), 7.462 (3.50).

### Beispiel 340

### (5RS,7RS)-2-(3-Chlor-4-fluorbenzyl)-5-[(3-fluorazetidin-1-yl)carbonyl]-7-methyl-5,6,7,8-tetrahydro[1,2,4]triazolo[4,3-a]pyridin-3(2H)-on (Diastereomerengemisch; 4 Isomere)

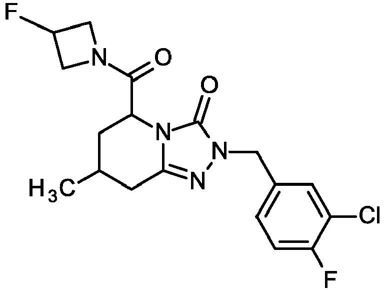

(5RS,7RS)-2-(3-Chlor-4-fluorbenzyl)-7-methyl-3-oxo-2,3,5,6,7,8-hexahydro[1,2,4]triazolo[4,3-a]pyridin-5-carbonsäure (Diastereomerengemisch; 4 Isomere) (70.0 mg, 206 µmol) wurde in THF (2.0 ml) vorgelegt und HBTU (85.9 mg, 227 µmol) und N,N-Diisopropylethylamin (110 µl, 620 µmol) wurden anschließend zugegeben. Nach 15 min Rühren bei Raumtemperatur wurde 3-Fluorazetidinhydrochlorid (27.6 mg, 247 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 57.2 mg (69 % d. Th.) der Titelverbindung erhalten.
LC-MS (Methode 3): Rₜ = 1.53 min; MS (ESIpos): m/z = 397 [M+H]⁺
¹H-NMR (400 MHz, DMSO-d6) δ [ppm]: 0.008 (2.93), 1.017 (15.46), 1.033 (16.00), 1.340 (0.54), 1.370 (1.55), 1.400 (1.84), 1.420 (1.72), 1.451 (0.70), 1.876 (1.86), 2.072 (4.68), 2.119 (1.08), 2.152 (4.88), 2.182 (3.65), 2.192 (4.50), 2.223 (2.93), 2.327 (0.62), 2.366 (0.44), 2.630 (3.05), 2.636 (3.11), 2.670 (3.23), 2.709 (0.56), 3.878 (0.75), 3.906 (0.97), 3.936 (1.30), 3.965 (1.55), 3.997 (0.71), 4.026 (0.85), 4.136 (0.63), 4.149 (0.72), 4.165 (0.58), 4.185 (0.85), 4.201 (0.82), 4.233 (1.94), 4.251 (1.38), 4.262 (1.43), 4.291 (1.59), 4.319 (1.46), 4.385 (4.37), 4.400 (4.82), 4.413 (5.10), 4.428 (3.90), 4.448 (0.69), 4.480 (1.31), 4.496 (0.86), 4.533 (0.78), 4.549 (0.82), 4.573 (0.46), 4.644 (0.59), 4.661 (0.68), 4.686 (0.63), 4.698 (0.69), 4.713 (0.73), 4.738 (0.54), 4.806 (14.39), 5.377 (1.22), 5.519 (1.21), 7.264 (3.03), 7.375 (3.36), 7.398 (5.54), 7.420 (2.62), 7.452 (3.55), 7.470 (3.56).

### Beispiel 341

### (5RS,7RS)-5-[(3-Fluorazetidin-1-yl)carbonyl]-7-methyl-2-{[6-(trifluormethyl)pyridin-3-yl]methyl}-5,6,7,8-tetrahydro[1,2,4]triazolo[4,3-a]pyridin-3(2H)-on (Diastereomerengemisch; 4 Isomere)

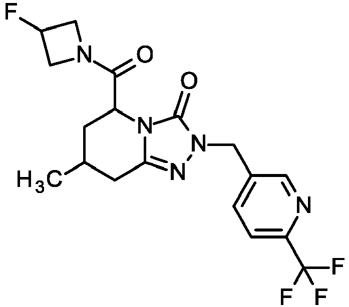

(5RS,7RS)-7-Methyl-3-oxo-2-{[6-(trifluormethyl)pyridin-3-yl]methyl}-2,3,5,6,7,8-hexahydro[1,2,4]triazolo[4,3-a]pyridin-5-carbonsäure (Diastereomerengemisch; 4 Isomere) (39.8 mg, 112 µmol) wurde in THF (2.0 ml) vorgelegt und HBTU (55.1 mg, 145 µmol) und N,N-Diisopropylethylamin (78 µl, 450 µmol) wurden anschließend zugegeben. Nach 15 min Rühren bei Raumtemperatur wurde 3-Fluorazetidinhydrochlorid (15.0 mg, 134 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 7.20 mg (16 % d. Th.) der Titelverbindung erhalten.
LC-MS (Methode 4): Rₜ = 0.71 min; MS (ESIpos): m/z = 414 [M+H]⁺
¹H-NMR (400 MHz, DMSO-d6) δ [ppm]: -0.149 (0.80), -0.008 (6.70), 0.008 (6.25), 0.146 (0.88), 1.019 (15.47), 1.035 (16.00), 1.377 (1.52), 1.397 (1.79), 1.407 (1.83), 1.426 (1.71), 1.456 (0.72), 1.883 (1.79), 2.156 (4.65), 2.185 (3.31), 2.195 (4.50), 2.226 (2.93), 2.327 (1.30), 2.366 (0.99), 2.524 (3.89), 2.636 (3.24), 2.671 (3.92), 2.710 (1.14), 3.881 (0.80), 3.913 (0.95), 3.938 (1.26), 3.966 (1.49), 3.994 (0.72), 4.027 (0.84), 4.152 (0.80), 4.185 (0.91), 4.220 (1.22), 4.237 (1.75), 4.271 (1.60), 4.303 (1.41), 4.322 (1.22), 4.388 (4.84), 4.403 (5.60), 4.416 (5.52), 4.430 (4.27), 4.477 (0.99), 4.506 (0.88), 4.541 (0.80), 4.556 (0.80), 4.656 (0.72), 4.712 (0.76), 4.990 (13.22), 5.381 (1.26), 5.515 (1.26), 7.900 (1.98), 7.920 (11.85), 7.949 (1.49), 8.673 (7.35).

### Beispiel 342

### (5S)-2-{2-[3-Chlor-5-(trifluormethyl)pyridin-2-yl]ethyl}-5-{[(3S)-3-fluorpyrrolidin-1-yl]carbonyl}-5,6,7,8-tetrahydro[1,2,4]triazolo[4,3-a]pyridin-3(2H)-on

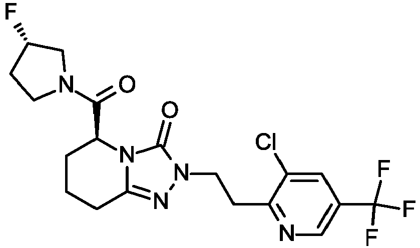

(5S)-2-{2-[3-Chlor-5-(trifluormethyl)pyridin-2-yl]ethyl}-3-oxo-2,3,5,6,7,8-hexahydro[1,2,4]triazolo[4,3-a]pyridin-5-carbonsäure (75.0 mg, 72 % Reinheit, 138 µmol) wurde in THF (1.5 ml) vorgelegt und HBTU (99.6 mg, 263 µmol) und N,N-Diisopropylethylamin (110 µl, 620 µmol) wurden anschließend zugegeben. Nach 15 min Rühren bei Raumtemperatur wurde (3S)-3-Fluorpyrrolidinhydrochlorid (29.5 mg, 235 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die Produkthaltigen Fraktionen wurden im Vakuum eingeengt und es wurden 50.9 mg (80 % d. Th.) der Titelverbindung erhalten.
LC-MS (Methode 3): Rₜ = 1.45 min; MS (ESIpos): m/z = 462 [M+H]⁺
¹H-NMR (400 MHz, DMSO-d6) δ [ppm]: -0.149 (0.83), -0.008 (7.76), 0.008 (7.06), 0.146 (0.83), 1.723 (3.81), 1.876 (0.90), 1.989 (1.39), 2.060 (2.08), 2.073 (16.00), 2.129 (1.11), 2.256 (1.04), 2.327 (2.15), 2.366 (1.59), 2.523 (7.83), 2.594 (2.35), 2.602 (2.63), 2.645 (1.25), 2.669 (2.29), 2.710 (1.59), 3.282 (5.13), 3.383 (0.97), 3.453 (0.69), 3.480 (0.90), 3.580 (0.69), 3.606 (2.98), 3.631 (2.15), 3.705 (1.87), 3.727 (1.87), 3.751 (1.59), 3.769 (1.25), 3.836 (2.56), 3.975 (0.83), 3.995 (1.45), 4.011 (2.63), 4.028 (4.29), 4.056 (3.39), 4.075 (2.08), 4.091 (1.04), 4.110 (0.62), 4.599 (1.32), 4.609 (1.66), 4.615 (1.66), 4.625 (1.32), 4.657 (1.66), 4.666 (1.87), 4.673 (2.01), 4.681 (1.45), 5.250 (1.25), 5.380 (1.52), 5.502 (0.97), 5.934 (0.48), 8.410 (5.96), 8.877 (5.96).

### Beispiel 343

### (5S)-2-{2-[3-Chlor-5-(trifluormethyl)pyridin-2-yl]ethyl}-5-[(3,3-difluorpyrrolidin-1-yl)carbonyl]-5,6,7,8-tetrahydro[1,2,4]triazolo[4,3-a]pyridin-3(2H)-on

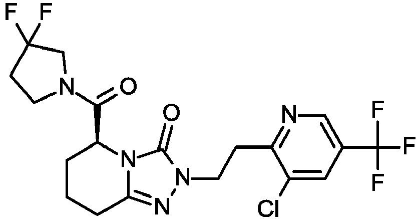

(5S)-2-{2-[3-Chlor-5-(trifluormethyl)pyridin-2-yl]ethyl}-3-oxo-2,3,5,6,7,8-hexahydro[1,2,4]triazolo[4,3-a]pyridin-5-carbonsäure (65.0 mg, 166 µmol) wurde in THF (2.5 ml) bei Raumtemperatur vorgelegt. Anschließend wurden HBTU (82.0 mg, 216 µmol) und N,N-Diisopropylethylamin (87 µl, 500 µmol) zugegeben. Nach 15 min Rühren wurde 3,3-Difluorpyrrolidinhydrochlorid (28.7 mg, 200 µmol) zugegeben und das Reaktionsgemisch über Nacht bei Raumtemperatur gerührt. Es wurde erneut HBTU (82.0 mg, 216 µmol) und 3,3-Difluorpyrrolidinhydrochlorid (28.7 mg, 200 µmol) zugegeben und über Nacht gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 33.3 mg (42 % d. Th.) der Titelverbindung erhalten.
LC-MS (Methode 3): Rₜ = 1.59 min; MS (ESIpos): m/z = 480 [M+H]⁺
¹H-NMR (400 MHz, DMSO-d6) δ [ppm]: -0.149 (1.01), 0.008 (7.50), 0.146 (0.97), 1.225 (1.31), 1.243 (7.46), 1.258 (8.43), 1.273 (4.85), 1.689 (5.30), 1.702 (7.20), 1.714 (6.68), 1.915 (2.16), 1.947 (3.24), 1.960 (3.10), 1.973 (4.07), 1.988 (3.13), 2.004 (2.39), 2.013 (2.65), 2.028 (2.87), 2.038 (2.91), 2.047 (2.28), 2.063 (2.20), 2.073 (16.00), 2.328 (1.75), 2.350 (1.19), 2.367 (3.32), 2.398 (2.76), 2.418 (2.65), 2.438 (1.79), 2.523 (11.00), 2.565 (5.41), 2.573 (4.03), 2.593 (6.97), 2.639 (2.39), 2.670 (1.94), 2.710 (1.57), 3.130 (0.71), 3.140 (0.75), 3.159 (0.67), 3.280 (8.47), 3.511 (5.45), 3.530 (9.44), 3.549 (5.00), 3.618 (1.42), 3.652 (3.24), 3.686 (3.54), 3.705 (2.01), 3.721 (1.79), 3.738 (3.88), 3.769 (4.92), 3.793 (3.69), 3.811 (1.83), 3.856 (1.83), 3.875 (3.58), 3.895 (2.05), 3.901 (2.46), 3.920 (1.12), 3.953 (1.04), 3.981 (3.66), 4.000 (3.28), 4.017 (6.94), 4.035 (11.93), 4.056 (11.75), 4.075 (5.93), 4.092 (2.80), 4.103 (1.75), 4.131 (2.24), 4.161 (2.09), 4.193 (0.90), 4.656 (2.87), 4.672 (4.03), 4.681 (2.87), 4.735 (3.06), 4.750 (3.99), 4.760 (2.91), 4.823 (0.71), 6.509 (1.19), 8.410 (11.82), 8.875 (12.16).

### Beispiel 344

### (5S)-5-{[(3R,4S)-3,4-Difluorpyrrolidin-1-yl]carbonyl}-2-[2-(4-fluorphenyl)ethyl]-5,6,7,8-tetrahydro[1,2,4]triazolo[4,3-a]pyridin-3(2H)-on

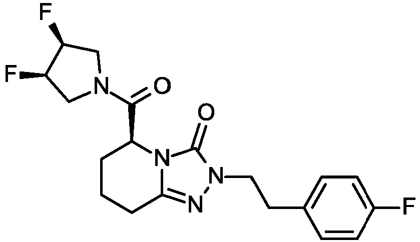

(5S)-2-[2-(4-Fluorphenyl)ethyl]-3-oxo-2,3,5,6,7,8-hexahydro[1,2,4]triazolo[4,3-a]pyridin-5-carbonsäure (73.1 mg, 240 µmol) wurde in THF (2.0 ml) vorgelegt und HBTU (118 mg, 311 µmol) und N,N-Diisopropylethylamin (130 µl, 720 µmol) wurden anschließend zugegeben. Nach 15 min Rühren bei Raumtemperatur wurde (3R,4S)-3,4-Difluorpyrrolidinhydrochlorid (41.3 mg, 287 µmol) zugegeben und das Reaktionsgemisch über Nacht bei Raumtemperatur gerührt. Es wurde erneut HBTU (90 mg, 240 µmol), (3R,4S)-3,4-Difluorpyrrolidinhydrochlorid (34.4 mg, 239 µmol) und N,N-Diisopropylethylamin (43 µl, 240 µmol) zugegeben und über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 62.3 mg (66 % d. Th.) der Titelverbindung erhalten.
LC-MS (Methode 4): Rₜ = 0.76 min; MS (ESIpos): m/z = 395 [M+H]⁺
¹H-NMR (400 MHz, DMSO-d6) δ [ppm]: -0.149 (1.11), -0.008 (9.50), 0.008 (9.19), 0.146 (1.15), 1.664 (1.94), 1.674 (2.46), 1.687 (3.33), 1.700 (4.32), 1.712 (4.95), 1.723 (4.83), 1.737 (3.13), 1.900 (1.11), 1.940 (2.42), 1.965 (1.74), 1.983 (3.29), 2.008 (2.26), 2.016 (2.22), 2.023 (2.46), 2.031 (2.26), 2.040 (2.02), 2.050 (1.74), 2.057 (1.90), 2.073 (13.15), 2.327 (1.11), 2.366 (1.47), 2.523 (4.55), 2.526 (3.92), 2.557 (4.87), 2.571 (4.36), 2.580 (3.52), 2.605 (3.21), 2.616 (5.98), 2.627 (3.33), 2.647 (1.39), 2.659 (2.46), 2.670 (2.30), 2.710 (1.58), 2.895 (7.80), 2.913 (16.00), 2.931 (8.28), 3.367 (0.59), 3.443 (1.27), 3.453 (0.75), 3.477 (2.14), 3.487 (1.58), 3.498 (2.06), 3.506 (2.26), 3.515 (1.90), 3.524 (1.98), 3.533 (1.98), 3.549 (1.19), 3.558 (1.62), 3.568 (0.99), 3.603 (1.74), 3.617 (1.94), 3.636 (1.27), 3.650 (1.39), 3.659 (2.02), 3.671 (3.13), 3.681 (2.34), 3.691 (2.02), 3.704 (2.77), 3.715 (2.77), 3.732 (3.45), 3.749 (4.00), 3.766 (7.92), 3.784 (12.55), 3.801 (8.75), 3.807 (7.49), 3.817 (4.04), 3.826 (4.24), 3.833 (2.77), 3.841 (3.09), 3.851 (1.47), 3.859 (1.47), 3.880 (1.43), 3.913 (1.62), 3.927 (1.94), 3.942 (1.27), 3.962 (1.90), 3.976 (1.90), 3.990 (1.23), 4.005 (1.15), 4.103 (1.19), 4.118 (1.39), 4.131 (1.27), 4.145 (2.30), 4.160 (1.43), 4.173 (1.27), 4.188 (1.15), 4.706 (5.54), 4.717 (6.65), 4.721 (7.29), 4.731 (5.27), 5.254 (1.86), 5.266 (2.06), 5.276 (1.70), 5.287 (1.31), 5.300 (1.47), 5.314 (1.47), 5.323 (1.35), 5.340 (1.50), 5.358 (1.66), 5.370 (1.86), 5.388 (1.70), 5.397 (1.98), 5.407 (1.82), 5.419 (1.23), 5.429 (1.35), 5.443 (1.54), 5.451 (1.58), 5.462 (1.50), 7.060 (5.35), 7.064 (7.29), 7.082 (13.35), 7.086 (14.61), 7.104 (9.23), 7.109 (8.63), 7.198 (6.34), 7.212 (9.74), 7.221 (10.69), 7.229 (8.44), 7.243 (5.54).

### Beispiel 345

### (5S)-5-[(3,3-Difluorazetidin-1-yl)carbonyl]-2-[2-(4-fluorphenyl)ethyl]-5,6,7,8-tetrahydro[1,2,4]triazolo[4,3-a]pyridin-3(2H)-on

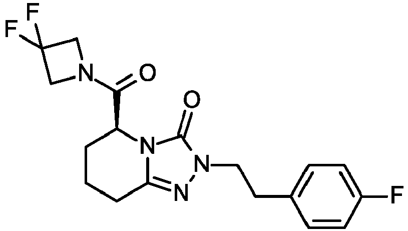

(5S)-2-[2-(4-Fluorphenyl)ethyl]-3-oxo-2,3,5,6,7,8-hexahydro[1,2,4]triazolo[4,3-a]pyridin-5-carbonsäure (73.1 mg, 240 µmol) wurde in THF (2.0 ml) vorgelegt und HBTU (118 mg, 311 µmol) und N,N-Diisopropylethylamin (130 µl, 720 µmol) wurden anschließend zugegeben. Nach 15 min Rühren bei Raumtemperatur wurde 3,3-Difluorazetidinhydrochlorid (37.2 mg, 287 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 56.0 mg (61 % d. Th.) der Titelverbindung erhalten.
LC-MS (Methode 3): Rₜ = 1.40 min; MS (ESIpos): m/z = 381 [M+H]⁺
¹H-NMR (400 MHz, DMSO-d6) δ [ppm]: -0.149 (1.79), 0.146 (1.90), 1.709 (4.36), 1.721 (6.60), 1.735 (5.71), 1.950 (2.35), 1.961 (3.58), 1.974 (3.36), 1.997 (1.79), 2.012 (2.69), 2.032 (3.02), 2.046 (2.24), 2.073 (6.60), 2.327 (2.80), 2.366 (2.35), 2.523 (10.85), 2.564 (4.36), 2.584 (4.92), 2.600 (5.82), 2.613 (6.83), 2.627 (3.36), 2.642 (1.23), 2.656 (2.24), 2.670 (3.58), 2.710 (2.46), 2.900 (6.94), 2.918 (13.87), 2.937 (7.61), 3.788 (6.60), 3.803 (10.74), 3.808 (11.19), 3.822 (5.26), 4.352 (4.03), 4.499 (4.81), 4.513 (7.94), 4.526 (4.70), 4.662 (2.35), 4.694 (2.69), 4.770 (1.12), 4.799 (2.46), 4.830 (2.24), 7.062 (7.16), 7.084 (16.00), 7.106 (10.29), 7.207 (9.40), 7.221 (10.85), 7.228 (9.17), 7.242 (7.05).

### Beispiel 346

### (5S)-5-[(3,3-Difluorpyrrolidin-1-yl)carbonyl]-2-[2-(4-methoxyphenyl)ethyl]-5,6,7,8-tetrahydro[1,2,4]triazolo[4,3-a]pyridin-3(2H)-on

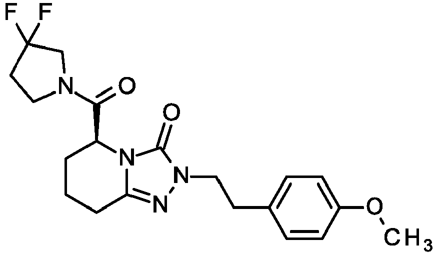

(5S)-2-[2-(4-Methoxyphenyl)ethyl]-3-oxo-2,3,5,6,7,8-hexahydro[1,2,4]triazolo[4,3-a]pyridin-5-carbonsäure (92.0 mg, 290 µmol) wurde in THF (2.0 ml) vorgelegt und HBTU (143 mg, 377 µmol) und N,N-Diisopropylethylamin (150 µl, 870 µmol) wurden anschließend zugegeben. Nach 15 min Rühren bei Raumtemperatur wurde 3,3-Difluorpyrrolidinhydrochlorid (49.9 mg, 348 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 51.6 mg (92 % Reinheit, 40 % d. Th.) der Titelverbindung erhalten.
LC-MS (Methode 4): Rₜ = 0.75 min; MS (ESIpos): m/z = 407 [M+H]⁺
¹H-NMR (400 MHz, DMSO-d6) δ [ppm]: 0.008 (2.58), 1.701 (2.94), 1.713 (4.02), 1.724 (3.77), 1.909 (0.73), 1.957 (1.87), 1.970 (1.77), 1.981 (2.06), 1.995 (2.06), 2.005 (1.63), 2.015 (1.54), 2.031 (1.60), 2.041 (1.55), 2.051 (1.14), 2.066 (1.03), 2.073 (1.41), 2.085 (0.51), 2.328 (0.87), 2.354 (0.71), 2.371 (1.17), 2.403 (1.47), 2.421 (1.42), 2.442 (1.09), 2.523 (4.27), 2.573 (3.78), 2.580 (3.40), 2.596 (2.90), 2.609 (2.10), 2.620 (3.47), 2.631 (1.84), 2.664 (1.65), 2.690 (0.57), 2.710 (0.63), 2.731 (0.98), 2.830 (5.18), 2.849 (10.41), 2.867 (5.46), 2.890 (1.30), 3.519 (15.11), 3.539 (11.03), 3.557 (6.77), 3.659 (1.93), 3.691 (2.71), 3.724 (5.71), 3.743 (6.41), 3.761 (4.83), 3.766 (6.69), 3.787 (4.86), 3.821 (1.36), 3.869 (0.93), 3.887 (1.96), 3.913 (1.36), 3.932 (0.62), 3.956 (0.62), 3.984 (1.22), 3.997 (0.65), 4.013 (0.85), 4.025 (1.11), 4.055 (0.62), 4.119 (0.74), 4.150 (1.12), 4.178 (1.12), 4.208 (0.51), 4.464 (0.46), 4.671 (1.50), 4.687 (2.17), 4.696 (1.57), 4.749 (1.55), 4.758 (1.90), 4.764 (2.10), 4.773 (1.54), 6.826 (13.56), 6.848 (16.00), 7.100 (14.23), 7.121 (12.19).

### Beispiel 347

### (5S)-5-[(3-Fluorazetidin-1 -yl)carbonyl]-2-[2-(4-methoxyphenyl)ethyl]-5,6,7,8-tetrahydro[1,2,4]triazolo[4,3-a]pyridin-3(2H)-on

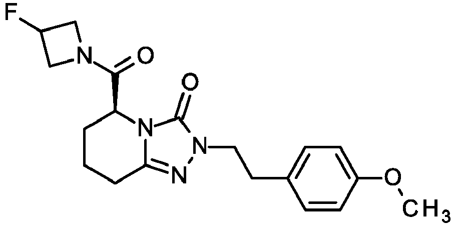

(5S)-2-[2-(4-Methoxyphenyl)ethyl]-3-oxo-2,3,5,6,7,8-hexahydro[1,2,4]triazolo[4,3-a]pyridin-5-carbonsäure (92.0 mg, 290 µmol) wurde in THF (2.0 ml) vorgelegt und HBTU (143 mg, 377 µmol) und N,N-Diisopropylethylamin (150 µl, 870 µmol) wurden anschließend zugegeben. Nach 15 min Rühren bei Raumtemperatur wurde 3-Fluorazetidinhydrochlorid (38.8 mg, 348 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 58.3 mg (54 % d. Th.) der Titelverbindung erhalten.
LC-MS (Methode 4): Rₜ = 0.67 min; MS (ESIpos): m/z = 375 [M+H]⁺
¹H-NMR (400 MHz, DMSO-d6) δ [ppm]: -0.149 (0.49), 0.146 (0.54), 1.717 (7.02), 1.909 (1.85), 1.922 (2.69), 1.934 (2.88), 1.943 (2.71), 1.955 (2.23), 1.977 (2.25), 1.995 (3.16), 2.012 (2.77), 2.029 (1.85), 2.047 (0.94), 2.072 (7.93), 2.327 (0.82), 2.366 (0.75), 2.572 (5.35), 2.594 (3.84), 2.610 (5.84), 2.624 (3.07), 2.639 (1.24), 2.652 (2.00), 2.665 (1.61), 2.710 (0.97), 2.834 (5.71), 2.852 (11.15), 2.871 (6.06), 3.368 (1.09), 3.531 (6.66), 3.751 (6.68), 3.761 (8.12), 3.769 (6.74), 3.780 (4.30), 3.891 (1.38), 3.906 (1.68), 3.935 (1.59), 3.970 (1.85), 3.994 (1.27), 4.138 (0.90), 4.154 (1.01), 4.168 (0.80), 4.225 (2.17), 4.252 (2.10), 4.282 (1.85), 4.293 (1.55), 4.316 (1.82), 4.345 (1.31), 4.384 (0.88), 4.407 (1.31), 4.430 (1.05), 4.450 (6.06), 4.465 (8.68), 4.476 (6.14), 4.498 (1.18), 4.523 (0.71), 4.601 (0.80), 4.616 (0.94), 4.654 (0.90), 4.670 (0.94), 4.680 (0.88), 4.694 (0.69), 5.327 (1.14), 5.387 (1.14), 5.469 (1.18), 5.530 (1.16), 6.827 (13.64), 6.848 (16.00), 7.105 (10.12), 7.121 (8.80).

### Beispiel 348

### (5S)-2-{[1-(6-Chlorpyridin-2-yl)cyclopropyl]methyl}-5-[(3-fluorazetidin-1-yl)carbonyl]-5,6,7,8-tetrahydro[1,2,4]triazolo[4,3-a]pyridin-3(2H)-on

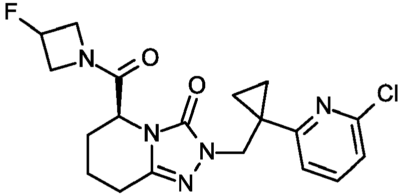

(5S)-2-{[1-(6-Chlorpyridin-2-yl)cyclopropyl]methyl}-3-oxo-2,3,5,6,7,8-hexahydro[1,2,4]triazolo[4,3-a]pyridin-5-carbonsäure (60.0 mg, 172 µmol) wurde in THF (3.0 ml) vorgelegt und HBTU (84.8 mg, 224 µmol) und N,N-Diisopropylethylamin (90 µl, 520 µmol) wurden anschließend zugegeben. Nach 15 min Rühren bei Raumtemperatur wurde 3-Fluorazetidinhydrochlorid (23.0 mg, 206 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 12.1 mg (17 % d. Th.) der Titelverbindung erhalten.
LC-MS (Methode 3): Rₜ = 1.35 min; MS (ESIpos): m/z = 406 [M+H]⁺
¹H-NMR (400 MHz, DMSO-d6) δ [ppm]: -0.149 (0.72), -0.008 (6.41), 0.008 (5.61), 0.146 (0.68), 1.107 (0.87), 1.124 (1.74), 1.140 (4.78), 1.166 (16.00), 1.679 (2.92), 1.929 (1.71), 1.991 (1.40), 2.327 (1.21), 2.366 (0.99), 2.523 (5.99), 2.565 (3.94), 2.578 (2.01), 2.594 (0.83), 2.607 (1.33), 2.670 (1.36), 2.690 (0.49), 2.710 (0.99), 3.883 (0.57), 3.914 (1.06), 3.947 (1.18), 3.988 (2.24), 4.033 (3.45), 4.091 (2.73), 4.116 (2.58), 4.129 (1.52), 4.154 (1.90), 4.211 (1.06), 4.227 (1.48), 4.257 (1.21), 4.290 (0.95), 4.310 (0.91), 4.345 (0.49), 4.373 (0.68), 4.410 (0.49), 4.433 (0.68), 4.504 (3.68), 4.623 (0.57), 4.663 (0.53), 5.340 (0.68), 5.401 (0.64), 5.483 (0.61), 5.543 (0.61), 7.243 (5.95), 7.262 (6.60), 7.571 (2.43), 7.578 (2.50), 7.590 (3.30), 7.598 (3.15), 7.699 (1.63), 7.710 (1.78), 7.718 (2.84), 7.729 (2.65), 7.738 (1.33), 7.749 (1.10).

### Beispiel 349

### (5S)-2-{[1-(6-Chlorpyridin-2-yl)cyclopropyl]methyl}-5-[(3,3-difluorpyrrolidin-1-yl)carbonyl]-5,6,7,8-tetrahydro[1,2,4]triazolo[4,3-a]pyridin-3(2H)-on

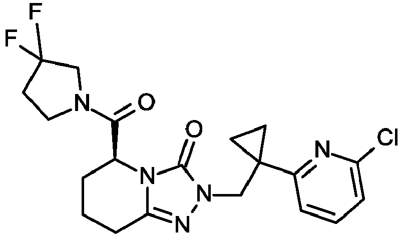

(5S)-2-{[1-(6-Chlorpyridin-2-yl)cyclopropyl]methyl}-3-oxo-2,3,5,6,7,8-hexahydro[1,2,4]triazolo[4,3-a]pyridin-5-carbonsäure (60.0 mg, 172 µmol) wurde in THF (3.0 ml) vorgelegt und HBTU (84.8 mg, 224 µmol) und N,N-Diisopropylethylamin (90 µl, 520 µmol) wurden anschließend zugegeben. Nach 15 min Rühren bei Raumtemperatur wurde 3,3-Difluorpyrrolidinhydrochlorid (29.6 mg, 206 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 19.6 mg (26 % d. Th.) der Titelverbindung erhalten.
LC-MS (Methode 4): Rₜ = 0.81 min; MS (ESIpos): m/z = 438 [M+H]⁺
¹H-NMR (400 MHz, DMSO-d6) δ [ppm]: -0.149 (1.50), -0.008 (14.75), 0.008 (12.25), 0.146 (1.75), 1.110 (3.75), 1.128 (5.00), 1.148 (3.50), 1.168 (16.00), 1.699 (2.25), 1.961 (2.25), 2.327 (4.25), 2.366 (3.25), 2.407 (1.75), 2.523 (15.00), 2.563 (5.25), 2.575 (5.25), 2.617 (1.75), 2.670 (4.50), 2.710 (3.25), 3.533 (3.25), 3.544 (3.25), 3.653 (2.00), 3.687 (2.25), 3.729 (1.50), 3.760 (4.00), 3.787 (2.50), 3.899 (2.00), 3.923 (1.50), 3.941 (1.50), 3.965 (5.50), 4.002 (7.75), 4.130 (6.00), 4.163 (4.75), 4.724 (2.50), 4.796 (2.50), 7.242 (8.75), 7.262 (9.75), 7.569 (7.75), 7.589 (10.25), 7.701 (7.00), 7.721 (11.50), 7.740 (5.00).

### Beispiel 350

### (5S)-2-{[1-(6-Chlorpyridin-2-yl)cyclopropyl]methyl}-5-{[(3S)-3-fluorpyrrolidin-1-yl]carbonyl}-5,6,7,8-tetrahydro[1,2,4]triazolo[4,3-a]pyridin-3(2H)-on

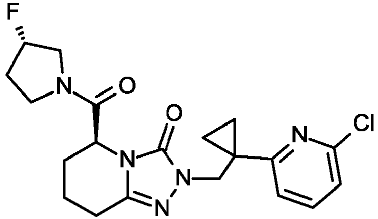

(5S)-2-{[1-(6-Chlorpyridin-2-yl)cyclopropyl]methyl}-3-oxo-2,3,5,6,7,8-hexahydro[1,2,4]triazolo[4,3-a]pyridin-5-carbonsäure (60.0 mg, 172 µmol) wurde in THF (3.0 ml) vorgelegt und HBTU (84.8 mg, 224 µmol) und N,N-Diisopropylethylamin (90 µl, 520 µmol) wurden anschließend zugegeben. Nach 15 min Rühren bei Raumtemperatur wurde (3S)-3-Fluorpyrrolidinhydrochlorid (25.9 mg, 206 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 22.5 mg (31 % d. Th.) der Titelverbindung erhalten.
LC-MS (Methode 4): Rₜ = 0.74 min; MS (ESIpos): m/z = 420 [M+H]⁺
¹H-NMR (400 MHz, DMSO-d6) δ [ppm]: -0.149 (1.70), -0.008 (14.64), 0.008 (12.26), 0.146 (1.70), 1.061 (2.04), 1.094 (1.70), 1.108 (4.43), 1.128 (5.45), 1.147 (3.74), 1.167 (12.94), 1.699 (4.43), 1.869 (1.36), 1.997 (2.72), 2.102 (2.04), 2.133 (2.04), 2.261 (1.70), 2.327 (4.43), 2.366 (3.74), 2.395 (0.68), 2.523 (16.00), 2.575 (4.09), 2.620 (1.70), 2.670 (4.77), 2.710 (3.74), 3.233 (1.02), 3.260 (1.70), 3.383 (2.72), 3.453 (1.36), 3.480 (1.70), 3.617 (3.74), 3.642 (3.74), 3.669 (2.38), 3.683 (2.04), 3.703 (2.04), 3.722 (3.40), 3.736 (3.06), 3.764 (3.06), 3.840 (3.74), 3.942 (4.09), 3.958 (3.06), 3.979 (5.45), 3.996 (4.77), 4.122 (4.43), 4.144 (5.79), 4.159 (3.06), 4.182 (4.09), 4.639 (2.04), 4.655 (2.38), 4.665 (1.70), 4.697 (2.38), 4.712 (3.06), 4.722 (2.38), 5.255 (2.04), 5.384 (2.38), 5.506 (1.36), 7.242 (9.53), 7.261 (10.21), 7.344 (0.68), 7.568 (4.09), 7.587 (5.79), 7.602 (6.47), 7.699 (3.74), 7.705 (4.43), 7.719 (5.79), 7.724 (7.49), 7.738 (2.72), 7.744 (3.06).

### Beispiel 351

### (5S)-5-[(3-Fluorazetidin-1-yl)carbonyl]-2-{[1-(4-fluorphenyl)cyclopropyl]methyl}-5,6,7,8-tetrahydro[1,2,4]triazolo[4,3-a]pyridin-3(2H)-on

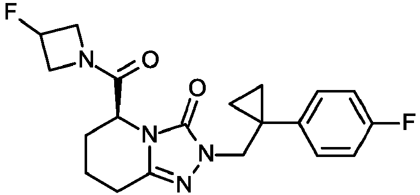

(5S)-2-{[1-(4-Fluorphenyl)cyclopropyl]methyl}-3-oxo-2,3,5,6,7,8-hexahydro[1,2,4]triazolo[4,3-a]pyridin-5-carbonsäure (80.0 mg, 241 µmol) wurde in THF (4.0 ml) vorgelegt und HBTU (119 mg, 314 µmol) und N,N-Diisopropylethylamin (130 µl, 720 µmol) wurden anschließend zugegeben. Nach 15 min Rühren bei Raumtemperatur wurde 3-Fluorazetidinhydrochlorid (32.3 mg, 290 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 40.8 mg (44 % d. Th.) der Titelverbindung erhalten.
LC-MS (Methode 3): Rₜ = 1.43 min; MS (ESIpos): m/z = 389 [M+H]⁺
¹H-NMR (400 MHz, DMSO-d6) δ [ppm]: -0.149 (1.23), -0.008 (16.00), 0.008 (9.89), 0.146 (1.12), 0.767 (3.30), 0.800 (3.41), 0.825 (1.29), 0.995 (7.97), 1.692 (3.30), 1.931 (1.86), 2.327 (1.66), 2.366 (1.35), 2.524 (8.89), 2.571 (2.64), 2.615 (0.92), 2.670 (1.38), 2.710 (1.09), 3.698 (3.56), 3.735 (5.53), 3.830 (2.12), 3.850 (2.01), 3.865 (1.69), 3.886 (1.81), 3.955 (0.95), 4.182 (1.49), 4.212 (1.35), 4.441 (3.27), 4.618 (0.57), 5.310 (0.66), 5.455 (0.66), 7.015 (2.78), 7.035 (5.30), 7.057 (3.07), 7.229 (3.73), 7.243 (4.79), 7.260 (2.72).

### Beispiel 352

### (5S)-2-{[1-(4-Fluorphenyl)cyclopropyl]methyl}-5-{[(3S)-3-fluorpyrrolidin-1-yl]carbonyl}-5,6,7,8-tetrahydro[1,2,4]triazolo[4,3-a]pyridin-3(2H)-on

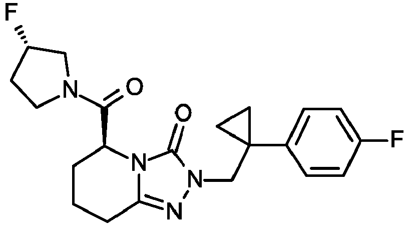

(5S)-2-{[1-(4-Fluorphenyl)cyclopropyl]methyl}-3-oxo-2,3,5,6,7,8-hexahydro[1,2,4]triazolo[4,3-a]pyridin-5-carbonsäure (80.0 mg, 241 µmol) wurde in THF (4.0 ml) vorgelegt und HBTU (119 mg, 314 µmol) und N,N-Diisopropylethylamin (130 µl, 720 µmol) wurden anschließend zugegeben. Nach 15 min Rühren bei Raumtemperatur wurde (3S)-3-Fluorpyrrolidinhydrochlorid (36.4 mg, 290 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 44.6 mg (46 % d. Th.) der Titelverbindung erhalten.
LC-MS (Methode 3): Rₜ = 1.47 min; MS (ESIpos): m/z = 403 [M+H]⁺
¹H-NMR (400 MHz, DMSO-d6) δ [ppm]: -0.149 (1.34), -0.008 (16.00), 0.008 (9.57), 0.146 (1.23), 0.723 (1.95), 0.750 (3.55), 0.778 (0.77), 0.811 (3.65), 0.835 (1.80), 0.983 (3.09), 0.998 (8.13), 1.710 (4.06), 1.838 (0.82), 1.873 (1.18), 1.997 (2.83), 2.008 (2.42), 2.019 (2.11), 2.033 (2.01), 2.048 (1.80), 2.073 (1.85), 2.125 (1.34), 2.249 (1.39), 2.327 (1.65), 2.366 (1.18), 2.519 (8.64), 2.524 (7.36), 2.586 (3.09), 2.628 (1.34), 2.669 (1.39), 2.710 (1.03), 3.379 (0.98), 3.389 (1.03), 3.450 (0.77), 3.476 (1.03), 3.486 (0.98), 3.563 (0.77), 3.598 (2.62), 3.616 (2.32), 3.633 (2.26), 3.649 (4.63), 3.659 (4.12), 3.685 (6.07), 3.695 (5.30), 3.732 (1.95), 3.753 (1.44), 3.782 (0.57), 3.818 (3.04), 3.859 (3.76), 3.880 (4.32), 3.895 (2.78), 3.916 (3.09), 4.586 (1.59), 4.595 (1.90), 4.602 (1.95), 4.610 (1.59), 4.644 (2.01), 4.654 (2.11), 4.659 (2.57), 4.668 (1.70), 5.247 (1.44), 5.368 (1.44), 5.491 (1.13), 7.015 (5.66), 7.037 (11.63), 7.058 (6.38), 7.221 (4.73), 7.228 (6.23), 7.234 (6.48), 7.242 (8.95), 7.250 (5.86), 7.257 (4.17), 7.264 (4.22).

### Beispiel 353

### (5S)-5-[(3,3-Difluorpyrrolidin-1-yl)carbonyl]-2-{[1-(4-fluorphenyl)cyclopropyl]methyl}-5,6,7,8-tetrahydro[1,2,4]triazolo[4,3-a]pyridin-3(2H)-on

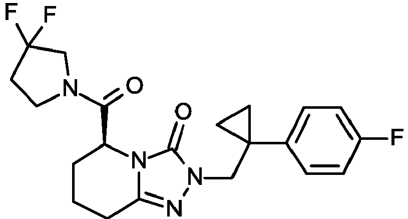

(5RS)-2-{[1-(4-Fluorphenyl)cyclopropyl]methyl}-3-oxo-2,3,5,6,7,8-hexahydro[1,2,4]triazolo[4,3-a]pyridin-5-carbonsäure (80.0 mg, 241 µmol) wurde in THF (4.0 ml) vorgelegt und HBTU (119 mg, 314 µmol) und N,N-Diisopropylethylamin (130 µl, 720 µmol) wurden anschließend zugegeben. Nach 15 min Rühren bei Raumtemperatur wurde 3,3-Difluorpyrrolidinhydrochlorid (41.6 mg, 290 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 46.1 mg (45 % d. Th.) der Titelverbindung erhalten.
LC-MS (Methode 3): Rₜ = 1.61 min; MS (ESIpos): m/z = 421 [M+H]⁺
¹H-NMR (400 MHz, DMSO-d6) δ [ppm]: -0.149 (0.60), -0.008 (5.39), 0.008 (4.70), 0.146 (0.62), 0.717 (1.43), 0.726 (2.10), 0.753 (4.13), 0.780 (1.04), 0.808 (4.45), 0.833 (2.33), 0.842 (1.75), 0.957 (1.50), 0.966 (1.34), 0.980 (3.94), 0.996 (11.00), 1.013 (3.41), 1.024 (1.20), 1.037 (1.04), 1.649 (1.59), 1.662 (2.05), 1.695 (3.09), 1.705 (3.02), 1.949 (2.90), 1.959 (2.95), 1.974 (3.18), 1.986 (3.07), 2.012 (1.64), 2.021 (1.24), 2.037 (1.08), 2.072 (3.37), 2.327 (0.65), 2.345 (0.81), 2.366 (1.89), 2.385 (1.52), 2.394 (1.68), 2.414 (1.64), 2.434 (1.20), 2.455 (0.71), 2.469 (1.57), 2.568 (3.07), 2.582 (5.12), 2.594 (2.49), 2.612 (1.11), 2.624 (1.78), 2.636 (0.88), 2.670 (0.69), 2.710 (0.71), 3.507 (3.46), 3.526 (5.76), 3.545 (2.88), 3.615 (0.55), 3.648 (2.14), 3.662 (5.37), 3.682 (2.65), 3.699 (7.52), 3.716 (1.41), 3.724 (1.11), 3.738 (3.00), 3.750 (1.54), 3.769 (4.10), 3.787 (1.13), 3.803 (0.55), 3.845 (1.13), 3.870 (8.00), 3.883 (1.54), 3.890 (1.84), 3.906 (5.42), 3.926 (0.76), 3.954 (1.52), 3.968 (0.71), 3.983 (1.06), 3.996 (1.34), 4.024 (0.83), 4.096 (0.85), 4.127 (1.38), 4.154 (1.34), 4.185 (0.55), 4.653 (1.87), 4.667 (2.67), 4.677 (1.84), 4.726 (1.91), 4.735 (2.24), 4.741 (2.58), 4.750 (1.91), 7.014 (7.26), 7.019 (2.61), 7.036 (16.00), 7.058 (9.18), 7.066 (1.08), 7.218 (1.11), 7.226 (9.22), 7.231 (4.54), 7.239 (10.44), 7.247 (8.88), 7.256 (3.53), 7.261 (7.56).

### Beispiel 354

### (5S)-5-[(3-Hydroxyazetidin-1-yl)carbonyl]-2-{[1-(4-methylphenyl)cyclopropyl]methyl}-5,6,7,8-tetrahydro[1,2,4]triazolo[4,3-a]pyridin-3(2H)-on

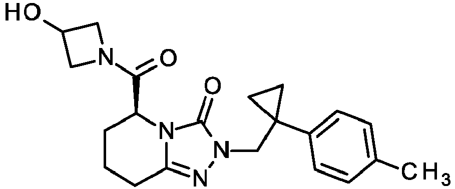

(5S)-2-{[1-(4-Methylphenyl)cyclopropyl]methyl}-3-oxo-2,3,5,6,7,8-hexahydro[1,2,4]triazolo[4,3-a]pyridin-5-carbonsäure (45.0 mg, 137 µmol) wurde in THF (2.0 ml) vorgelegt und HBTU (67.8 mg, 179 µmol) und N,N-Diisopropylethylamin (72 µl, 410 µmol) wurden anschließend zugegeben. Nach 15 min Rühren bei Raumtemperatur wurde Azetidin-3-olhydrochlorid (18.1 mg, 165 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 19.1 mg (36 % d. Th.) der Titelverbindung erhalten.
LC-MS (Methode 3): Rₜ = 1.32 min; MS (ESIpos): m/z = 383 [M+H]⁺
¹H-NMR (400 MHz, DMSO-d6) δ [ppm]: 0.008 (1.07), 0.711 (0.96), 0.777 (1.56), 0.802 (0.80), 0.930 (0.49), 0.944 (0.73), 0.969 (2.84), 0.984 (1.24), 0.994 (0.48), 1.681 (1.53), 1.691 (1.56), 1.878 (0.47), 1.903 (1.19), 1.914 (1.15), 1.944 (0.79), 1.954 (0.79), 2.236 (16.00), 2.451 (0.40), 2.565 (1.96), 2.577 (1.01), 2.594 (0.50), 2.607 (0.81), 3.572 (0.54), 3.582 (0.58), 3.606 (1.21), 3.618 (0.65), 3.631 (0.67), 3.643 (0.72), 3.654 (1.10), 3.692 (1.94), 3.730 (1.42), 3.879 (1.86), 3.900 (1.83), 3.915 (1.14), 3.936 (1.03), 3.956 (0.55), 3.968 (0.60), 3.979 (0.65), 3.992 (1.11), 4.009 (0.68), 4.036 (0.52), 4.076 (0.51), 4.092 (0.65), 4.116 (0.47), 4.242 (0.53), 4.261 (0.84), 4.281 (0.54), 4.395 (0.64), 4.410 (1.18), 4.418 (1.24), 4.430 (1.31), 4.440 (1.31), 4.456 (1.31), 4.476 (1.35), 5.779 (1.94), 5.793 (1.32), 7.026 (3.34), 7.045 (5.44), 7.103 (3.59), 7.110 (3.97), 7.123 (2.43), 7.130 (2.29).

### Beispiel 355

### (5S)-5-[(3-Fluorazetidin-1-yl)carbonyl]-2-{[1-(4-methylphenyl)cyclopropyl]methyl}-5,6,7,8-tetrahydro[1,2,4]triazolo[4,3-a]pyridin-3(2H)-on

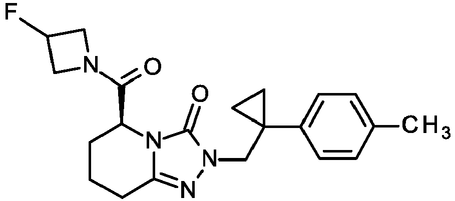

(5S)-2-{[1-(4-Methylphenyl)cyclopropyl]methyl}-3-oxo-2,3,5,6,7,8-hexahydro[1,2,4]triazolo[4,3-a]pyridin-5-carbonsäure (45.0 mg, 137 µmol) wurde in THF (2.0 ml) vorgelegt und HBTU (67.8 mg, 179 µmol) und N,N-Diisopropylethylamin (72 µl, 410 µmol) wurden anschließend zugegeben. Nach 15 min Rühren bei Raumtemperatur wurde 3-Fluorazetidinhydrochlorid (1:1) (18.4 mg, 165 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 17.3 mg (33 % d. Th.) der Titelverbindung erhalten.
LC-MS (Methode 3): Rₜ = 1.57 min; MS (ESIpos): m/z = 385 [M+H]⁺
¹H-NMR (400 MHz, DMSO-d6) δ [ppm]: -0.008 (2.15), 0.008 (1.85), 0.734 (1.36), 0.773 (1.76), 0.798 (0.79), 0.926 (0.52), 0.935 (0.45), 0.966 (3.73), 0.982 (1.21), 0.993 (0.45), 1.690 (1.97), 1.931 (1.03), 1.967 (0.82), 1.982 (0.70), 2.002 (0.48), 2.234 (16.00), 2.327 (0.70), 2.366 (0.52), 2.523 (3.12), 2.570 (1.58), 2.614 (0.58), 2.669 (0.76), 2.709 (0.55), 3.693 (1.12), 3.735 (1.61), 3.874 (2.30), 3.911 (1.61), 3.957 (0.58), 4.187 (0.67), 4.213 (0.70), 4.243 (0.52), 4.255 (0.55), 4.282 (0.67), 4.429 (1.52), 4.443 (2.30), 4.455 (1.55), 7.025 (2.88), 7.044 (4.61), 7.109 (3.64), 7.124 (2.42).

### Beispiel 356

### (5S)-5-{[(3S)-3-Fluorpyrrolidin-1-yl]carbonyl}-2-({1-[4-(trifluormethyl)phenyl]cyclopropyl}methyl)-5,6,7,8-tetrahydro[1,2,4]triazolo[4,3-a]pyridin-3(2H)-on

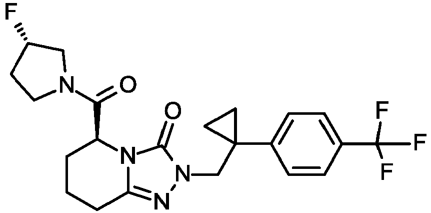

(5S)-3-Oxo-2-({1-[4-(trifluormethyl)phenyl]cyclopropyl}methyl)-2,3,5,6,7,8-hexahydro[1,2,4]triazolo[4,3-a]pyridin-5-carbonsäure (57.4 mg, 151 µmol) wurde in THF (1.4 ml) vorgelegt und HBTU (74.2 mg, 196 µmol) und N,N-Diisopropylethylamin (79 µl, 450 µmol) wurden anschließend zugegeben. Nach 15 min Rühren bei Raumtemperatur wurde (3S)-3-Fluorpyrrolidinhydrochlorid (1:1) (22.7 mg, 181 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 38.5 mg (55 % d. Th.) der Titelverbindung erhalten.
LC-MS (Methode 4): Rₜ = 0.94 min; MS (ESIpos): m/z = 453 [M+H]⁺
¹H-NMR (400 MHz, DMSO-d6) δ [ppm]: -0.149 (1.70), -0.008 (16.00), 0.146 (1.70), 0.858 (2.30), 0.939 (1.53), 1.112 (4.68), 1.709 (1.79), 1.871 (0.51), 1.999 (1.28), 2.073 (1.45), 2.251 (0.60), 2.327 (2.64), 2.366 (2.21), 2.589 (1.45), 2.669 (2.81), 2.710 (2.30), 3.491 (0.51), 3.592 (1.45), 3.615 (1.19), 3.688 (0.94), 3.707 (1.02), 3.732 (0.85), 3.763 (1.45), 3.799 (2.21), 3.807 (2.04), 3.920 (1.62), 3.935 (1.87), 3.957 (1.11), 3.971 (1.19), 4.607 (0.77), 4.665 (1.11), 5.247 (0.68), 5.380 (0.68), 5.492 (0.51), 7.430 (2.81), 7.449 (3.66), 7.571 (4.77), 7.592 (3.66).

### Beispiel 357

### (5S)-5-[(3-Fluorazetidin-1-yl)carbonyl]-2-{[1-(4-methoxyphenyl)cyclopropyl]methyl}-5,6,7,8-tetrahydro[1,2,4]triazolo[4,3-a]pyridin-3(2H)-on

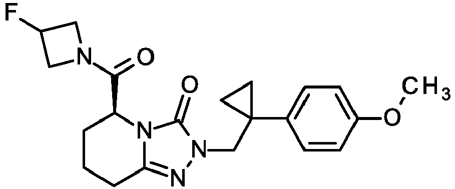

(5S)-2-{[1-(4-Methoxyphenyl)cyclopropyl]methyl}-3-oxo-2,3,5,6,7,8-hexahydro[1,2,4]triazolo[4,3-a]pyridin-5-carbonsäure (93.2 mg, 50 % Reinheit, 136 µmol) wurde in THF (1.2 ml) vorgelegt und HBTU (134 mg, 353 µmol) und N,N-Diisopropylethylamin (140 µl, 810 µmol) wurden anschließend zugegeben. Nach 15 min Rühren bei Raumtemperatur wurde 3-Fluorazetidinhydrochlorid (18.2 mg, 163 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 38.1 mg (70 % d. Th.) der Titelverbindung erhalten.
LC-MS (Methode 4): Rₜ = 0.77 min; MS (ESIpos): m/z = 401 [M+H]⁺
¹H-NMR (400 MHz, DMSO-d6) δ [ppm]: -0.149 (0.69), -0.008 (6.04), 0.008 (5.43), 0.147 (0.65), 0.705 (0.82), 0.742 (1.16), 0.938 (2.29), 1.696 (1.21), 1.934 (0.65), 2.072 (0.73), 2.328 (1.55), 2.366 (0.99), 2.523 (4.36), 2.670 (1.38), 2.710 (0.99), 3.674 (0.60), 3.700 (16.00), 3.834 (1.29), 3.869 (1.08), 4.281 (0.47), 4.426 (0.95), 4.440 (1.51), 4.451 (0.99), 6.775 (2.20), 6.796 (2.46), 7.131 (1.73), 7.146 (1.60).

### Beispiel 358

### (5S)-2-{[1-(2,4-Difluorphenyl)cyclopropyl]methyl}-5-{[(3S)-3-fluorpyrrolidin-1-yl]carbonyl}-5,6,7,8-tetrahydro[1,2,4]triazolo[4,3-a]pyridin-3(2H)-on

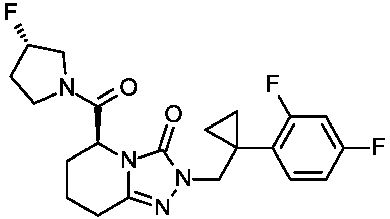

(5S)-2-{[1-(2,4-Difluorphenyl)cyclopropyl]methyl}-3-oxo-2,3,5,6,7,8-hexahydro[1,2,4]triazolo[4,3-a]pyridin-5-carbonsäure (78.0 mg, 223 µmol) wurde in THF (2.0 ml) vorgelegt und HBTU (110 mg, 290 µmol) und N,N-Diisopropylethylamin (120 µl, 670 µmol) wurden anschließend zugegeben. Nach 15 min Rühren bei Raumtemperatur wurde (3S)-3-Fluorpyrrolidinhydrochlorid (28.0 mg, 223 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 42.3 mg (45 % d. Th.) der Titelverbindung erhalten.
LC-MS (Methode 3): Rₜ = 1.52 min; MS (ESIpos): m/z = 421 [M+H]⁺
¹H-NMR (400 MHz, DMSO-d6) δ [ppm]: -0.149 (1.76), -0.008 (16.00), 0.008 (12.28), 0.146 (1.76), 0.732 (2.71), 0.765 (2.65), 1.001 (5.42), 1.690 (2.08), 1.995 (1.26), 2.073 (1.39), 2.327 (2.65), 2.366 (1.45), 2.669 (2.83), 2.710 (1.76), 3.606 (2.20), 3.632 (2.65), 3.700 (1.76), 3.805 (3.15), 3.823 (1.95), 3.859 (1.45), 4.626 (1.26), 6.910 (2.08), 7.112 (1.76), 7.134 (1.83).

### Beispiel 359

### (5S)-2-{[1-(2,4-Difluorphenyl)cyclopropyl]methyl}-5-[(3,3-difluorpyrrolidin-1-yl)carbonyl]-5,6,7,8-tetrahydro[1,2,4]triazolo[4,3-a]pyridin-3(2H)-on

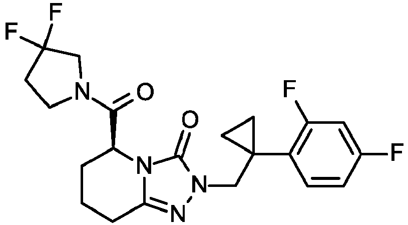

(5S)-2-{[1-(2,4-Difluorphenyl)cyclopropyl]methyl}-3-oxo-2,3,5,6,7,8-hexahydro[1,2,4]triazolo[4,3-a]pyridin-5-carbonsäure (78.0 mg, 100 % Reinheit, 223 µmol) wurde in THF (2.0 ml) vorgelegt und HBTU (110 mg, 290 µmol) und N,N-Diisopropylethylamin (120 µl, 670 µmol) wurden anschließend zugegeben. Nach 15 min Rühren bei Raumtemperatur wurde 3,3-Difluorpyrrolidinhydrochlorid (32.1 mg, 223 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 42.2 mg (43 % d. Th.) der Titelverbindung erhalten.
LC-MS (Methode 3): Rₜ = 1.66 min; MS (ESIpos): m/z = 439 [M+H]⁺
¹H-NMR (400 MHz, DMSO-d6) δ [ppm]: -0.149 (1.72), -0.008 (16.00), 0.008 (12.30), 0.146 (1.63), 0.712 (1.03), 0.735 (3.44), 0.764 (3.78), 0.788 (1.12), 1.001 (7.83), 1.679 (1.81), 1.969 (1.63), 2.073 (8.09), 2.327 (2.41), 2.366 (2.67), 2.431 (1.20), 2.523 (9.29), 2.591 (1.20), 2.670 (2.49), 2.710 (2.24), 3.493 (1.63), 3.512 (3.10), 3.530 (1.55), 3.604 (1.89), 3.613 (1.72), 3.640 (2.92), 3.649 (2.58), 3.670 (1.20), 3.719 (1.38), 3.731 (1.03), 3.752 (1.46), 3.808 (2.41), 3.819 (2.32), 3.844 (2.15), 3.855 (2.15), 3.946 (0.77), 3.986 (0.69), 4.106 (0.69), 4.138 (0.60), 4.634 (1.29), 4.707 (1.29), 6.889 (1.03), 6.910 (2.24), 6.924 (1.12), 7.085 (1.20), 7.091 (1.20), 7.112 (1.89), 7.134 (1.81), 7.150 (2.06), 7.171 (1.98), 7.189 (0.77).

### Beispiel 360

### (5S)-2-{[1-Ethyl-3-(trifluormethyl)-1H-pyrazol-5-yl]methyl}-5-{[(3S)-3-fluorpyrrolidin-1 yl]carbonyl}-5,6,7,8-tetrahydro[1,2,4]triazolo[4,3-a]pyridin-3(2H)-on

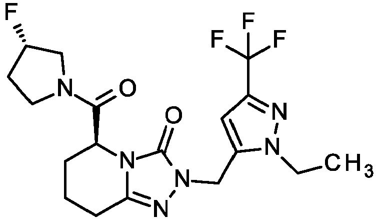

(5S)-2-{[1-Ethyl-3-(trifluormethyl)-1H-pyrazol-5-yl]methyl}-3-oxo-2,3,5,6,7,8-hexahydro[1,2,4]triazolo[4,3-a]pyridin-5-carbonsäure (99.9 mg, 91 % Reinheit, 254 µmol) wurde in THF (2.0 ml) vorgelegt und HBTU (125 mg, 330 µmol) und N,N-Diisopropylethylamin (130 µl, 760 µmol) wurden anschließend zugegeben. Nach 15 min Rühren bei Raumtemperatur wurde (3S)-3-Fluorpyrrolidinhydrochlorid (1:1) (38.2 mg, 304 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 49.2 mg (45 % d. Th.) der Titelverbindung erhalten.
LC-MS (Methode 3): Rₜ = 1.39 min; MS (ESIpos): m/z = 431 [M+H]⁺
¹H-NMR (400 MHz, DMSO-d6) δ [ppm]: -0.008 (2.11), 0.008 (1.84), 1.352 (7.67), 1.370 (16.00), 1.387 (7.75), 1.716 (2.42), 1.729 (2.81), 1.739 (2.10), 1.863 (0.55), 1.874 (0.50), 1.897 (0.67), 1.968 (0.55), 2.002 (0.92), 2.011 (1.11), 2.038 (0.79), 2.054 (1.06), 2.064 (1.33), 2.073 (5.60), 2.084 (1.60), 2.099 (1.51), 2.134 (1.15), 2.170 (0.42), 2.218 (0.70), 2.239 (0.78), 2.267 (0.94), 2.327 (0.51), 2.366 (0.50), 2.565 (1.79), 2.581 (1.33), 2.608 (2.16), 2.650 (0.82), 2.664 (0.58), 2.669 (0.55), 2.710 (0.51), 3.269 (0.51), 3.287 (0.87), 3.343 (0.65), 3.356 (0.58), 3.365 (0.60), 3.392 (0.70), 3.401 (0.73), 3.454 (0.51), 3.463 (0.55), 3.490 (0.74), 3.498 (0.72), 3.623 (1.74), 3.635 (1.32), 3.652 (1.93), 3.662 (1.55), 3.677 (1.11), 3.685 (1.06), 3.721 (1.60), 3.744 (1.68), 3.768 (1.41), 3.776 (1.09), 3.785 (1.18), 3.854 (2.16), 4.186 (2.45), 4.204 (7.35), 4.222 (7.28), 4.240 (2.37), 4.665 (1.06), 4.674 (1.28), 4.681 (1.40), 4.690 (1.09), 4.723 (1.42), 4.732 (1.58), 4.739 (1.87), 4.747 (2.37), 4.787 (10.29), 4.792 (7.03), 4.796 (7.44), 4.835 (0.85), 5.257 (1.08), 5.389 (1.47), 5.511 (0.79), 6.626 (7.19).

### Beispiel 361

### (5S)-5-{[(3R,4S)-3,4-Difluorpyrrolidin-1-yl]carbonyl}-2-{[1-ethyl-3-(trifluormethyl)-1H-pyrazol-5-yl]methyl}-5,6,7,8-tetrahydro[1,2,4]triazolo[4,3-a]pyridin-3(2H)-on

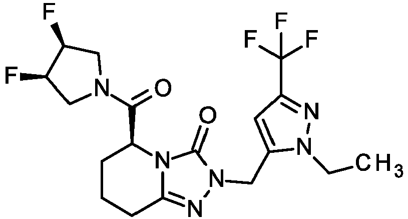

(5S)-2-{[1-Ethyl-3-(trifluormethyl)-1H-pyrazol-5-yl]methyl}-3-oxo-2,3,5,6,7,8-hexahydro[1,2,4]triazolo[4,3-a]pyridin-5-carbonsäure (99.9 mg, 91 % Reinheit, 254 µmol) wurde in THF (2.0 ml) vorgelegt und HBTU (125 mg, 330 µmol) und N,N-Diisopropylethylamin (130 µl, 760 µmol) wurden anschließend zugegeben. Nach 15 min Rühren bei Raumtemperatur wurde (3R,4S)-3,4-Difluorpyrrolidinhydrochlorid (43.7 mg, 304 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 45.8 mg (40 % d. Th.) der Titelverbindung erhalten.
LC-MS (Methode 3): Rₜ = 1.46 min; MS (ESIpos): m/z = 449 [M+H]⁺
¹H-NMR (400 MHz, DMSO-d6) δ [ppm]: 0.008 (3.10), 1.244 (0.66), 1.259 (0.70), 1.274 (0.45), 1.351 (7.19), 1.369 (14.93), 1.387 (7.30), 1.669 (1.28), 1.705 (1.30), 1.717 (1.51), 1.726 (1.67), 1.921 (0.54), 1.956 (1.24), 2.002 (1.57), 2.026 (0.97), 2.036 (1.10), 2.044 (1.18), 2.051 (1.10), 2.060 (0.95), 2.071 (0.85), 2.079 (0.97), 2.086 (0.81), 2.327 (0.64), 2.366 (0.85), 2.524 (2.40), 2.566 (2.56), 2.576 (1.78), 2.591 (2.40), 2.603 (3.14), 2.615 (1.76), 2.634 (0.68), 2.645 (1.07), 2.670 (0.79), 2.710 (0.91), 3.484 (2.25), 3.505 (1.69), 3.515 (1.65), 3.524 (1.36), 3.533 (1.30), 3.557 (0.83), 3.566 (1.01), 3.610 (0.97), 3.623 (1.05), 3.643 (0.70), 3.656 (0.76), 3.666 (0.99), 3.679 (1.80), 3.690 (1.16), 3.699 (1.10), 3.712 (1.51), 3.724 (1.36), 3.743 (1.07), 3.755 (1.10), 3.775 (0.89), 3.789 (0.60), 3.865 (0.79), 3.902 (0.60), 3.928 (0.79), 3.941 (0.89), 3.956 (0.58), 3.977 (0.91), 3.991 (0.91), 4.005 (0.62), 4.020 (0.52), 4.128 (0.54), 4.143 (0.62), 4.156 (0.68), 4.169 (1.07), 4.185 (2.79), 4.204 (7.26), 4.222 (7.15), 4.240 (2.40), 4.751 (1.01), 4.767 (2.34), 4.791 (16.00), 4.798 (7.81), 4.838 (0.79), 5.254 (0.87), 5.265 (0.87), 5.274 (1.01), 5.284 (0.97), 5.296 (0.64), 5.311 (0.60), 5.329 (0.74), 5.337 (0.74), 5.351 (0.77), 5.364 (0.74), 5.374 (1.01), 5.387 (1.12), 5.398 (0.87), 5.406 (0.89), 5.427 (0.60), 5.440 (0.62), 5.458 (0.74), 5.472 (0.74), 5.480 (0.77), 5.494 (0.60), 6.619 (5.44), 6.624 (6.20).

### Beispiel 362

### (5S)-5-[(3,3-Difluorpyrrolidin-1-yl)carbonyl]-2-{[1-ethyl-3-(trifluormethyl)-1H-pyrazol-5-yl]methyl}-5,6,7,8-tetrahydro[1,2,4]triazolo[4,3-a]pyridin-3(2H)-on

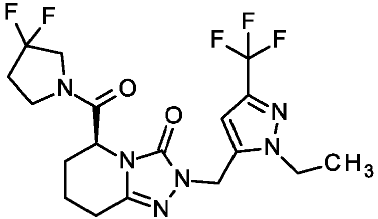

(5S)-2-{[1-Ethyl-3-(trifluormethyl)-1H-pyrazol-5-yl]methyl}-3-oxo-2,3,5,6,7,8-hexahydro[1,2,4]triazolo[4,3-a]pyridin-5-carbonsäure (99.9 mg, 91 % Reinheit, 254 µmol) wurde in THF (2.0 ml) vorgelegt und HBTU (125 mg, 330 µmol) und N,N-Diisopropylethylamin (130 µl, 760 µmol) wurden anschließend zugegeben. Nach 15 min Rühren bei Raumtemperatur wurde 3,3-Difluorpyrrolidinhydrochlorid (43.7 mg, 304 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 49.8 mg (43 % d. Th.) der Titelverbindung erhalten.
LC-MS (Methode 3): Rₜ = 1.53 min; MS (ESIpos): m/z = 449 [M+H]⁺
¹H-NMR (400 MHz, DMSO-d6) δ [ppm]: -0.008 (3.74), 0.008 (1.80), 1.350 (7.76), 1.368 (16.00), 1.386 (7.57), 1.685 (0.98), 1.701 (1.16), 1.714 (1.30), 1.722 (1.30), 1.971 (1.06), 1.984 (1.07), 1.995 (1.23), 2.020 (0.91), 2.030 (0.86), 2.039 (0.91), 2.046 (0.91), 2.056 (0.86), 2.066 (0.68), 2.074 (0.66), 2.082 (0.58), 2.327 (0.54), 2.366 (0.66), 2.379 (0.73), 2.408 (0.88), 2.429 (0.89), 2.563 (2.10), 2.572 (2.36), 2.587 (2.57), 2.604 (2.36), 2.615 (1.11), 2.646 (0.69), 2.670 (0.52), 2.710 (0.45), 3.368 (1.56), 3.527 (1.26), 3.546 (1.89), 3.562 (0.89), 3.568 (0.88), 3.666 (0.91), 3.700 (1.05), 3.731 (0.76), 3.762 (1.37), 3.782 (0.84), 3.793 (1.22), 3.808 (1.07), 3.826 (0.73), 3.889 (0.49), 3.907 (1.08), 3.926 (0.62), 3.934 (0.74), 3.992 (0.69), 4.021 (0.49), 4.034 (0.60), 4.141 (0.43), 4.186 (2.33), 4.204 (6.79), 4.222 (6.20), 4.240 (1.95), 4.731 (0.93), 4.746 (1.32), 4.791 (6.98), 4.797 (8.50), 4.815 (1.25), 4.820 (1.29), 4.830 (0.96), 4.836 (0.88), 6.622 (6.40).

### Beispiel 363

### (5S)-2-{[1-Ethyl-3-(trifluormethyl)-1H-pyrazol-5-yl]methyl}-5-[(3-fluorazetidin-1-yl)carbonyl]-5,6,7,8-tetrahydro[1,2,4]triazolo[4,3-a]pyridin-3(2H)-on

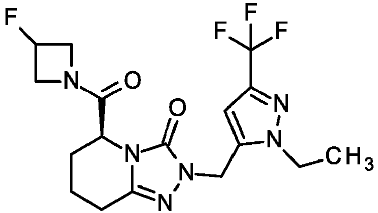

(5S)-2-{[1-Ethyl-3-(trifluormethyl)-1H-pyrazol-5-yl]methyl}-3-oxo-2,3,5,6,7,8-hexahydro[1,2,4]triazolo[4,3-a]pyridin-5-carbonsäure (99.9 mg, 91 % Reinheit, 254 µmol) wurde in THF (2.0 ml) vorgelegt und HBTU (125 mg, 330 µmol) und N,N-Diisopropylethylamin (130 µl, 760 µmol) wurden anschließend zugegeben. Nach 15 min Rühren bei Raumtemperatur wurde 3-Fluorazetidinhydrochlorid (33.9 mg, 304 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 50.6 mg (48 % d. Th.) der Titelverbindung erhalten.
LC-MS (Methode 3): Rₜ = 1.36 min; MS (ESIpos): m/z = 417 [M+H]⁺
¹H-NMR (400 MHz, DMSO-d6) δ [ppm]: -0.149 (0.83), -0.008 (6.74), 0.008 (7.66), 0.146 (0.87), 1.349 (7.70), 1.366 (16.00), 1.384 (8.39), 1.708 (3.48), 1.948 (1.60), 2.022 (1.47), 2.072 (0.69), 2.327 (1.24), 2.366 (1.70), 2.522 (4.08), 2.526 (3.26), 2.580 (3.62), 2.594 (4.58), 2.607 (2.34), 2.622 (1.01), 2.637 (1.51), 2.650 (0.73), 2.665 (1.28), 2.670 (1.60), 2.710 (1.93), 3.922 (1.05), 3.951 (0.96), 3.985 (1.15), 4.008 (0.69), 4.155 (0.64), 4.185 (2.89), 4.203 (7.70), 4.220 (7.89), 4.239 (3.21), 4.257 (1.47), 4.281 (1.19), 4.311 (0.92), 4.346 (0.73), 4.391 (0.73), 4.429 (0.50), 4.455 (0.69), 4.517 (2.70), 4.529 (4.17), 4.541 (2.57), 4.568 (0.60), 4.624 (0.50), 4.636 (0.60), 4.673 (0.55), 4.691 (0.60), 4.715 (0.50), 4.795 (15.08), 5.351 (0.73), 5.403 (0.69), 5.493 (0.69), 5.546 (0.64), 6.632 (7.61).

### Beispiel 364

### (5S)-5-[(3,3-Difluorpyrrolidin-1 -yl)carbonyl]-2-{[2-(trifluormethyl)-1,3-thiazol-4-yl]methyl}-5,6,7,8-tetrahydro[1,2,4]triazolo[4,3-a]pyridin-3(2H)-on

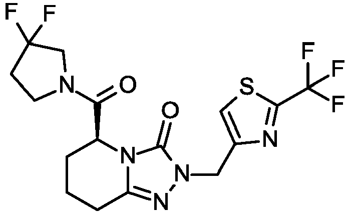

(5S)-3-Oxo-2-{[2-(trifluormethyl)-1,3-thiazol-4-yl]methyl}-2,3,5,6,7,8-hexahydro[1,2,4]triazolo[4,3-a]pyridin-5-carbonsäure (50.0 mg, 144 µmol) wurde in THF (2.0 ml) vorgelegt und HBTU (70.8 mg, 187 µmol) und N,N-Diisopropylethylamin (75 µl, 430 µmol) wurden anschließend zugegeben. Nach 15 min Rühren bei Raumtemperatur wurde 3,3-Difluorpyrrolidinhydrochlorid (24.7 mg, 172 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 12.0 mg (19 % d. Th.) der Titelverbindung erhalten.
LC-MS (Methode 3): Rₜ = 1.43 min; MS (ESIpos): m/z = 438 [M+H]⁺
¹H-NMR (400 MHz, DMSO-d6) δ [ppm]: -0.149 (1.35), -0.008 (11.66), 0.008 (10.84), 0.146 (1.41), 1.236 (0.59), 1.684 (2.05), 1.733 (2.64), 1.986 (2.23), 2.001 (2.23), 2.012 (2.58), 2.035 (1.99), 2.045 (1.82), 2.054 (1.93), 2.061 (1.93), 2.073 (3.69), 2.081 (1.41), 2.089 (1.41), 2.097 (1.29), 2.327 (1.23), 2.366 (1.99), 2.381 (1.47), 2.410 (1.76), 2.430 (1.64), 2.451 (1.11), 2.573 (6.33), 2.583 (5.16), 2.598 (5.74), 2.612 (5.63), 2.625 (2.99), 2.642 (1.35), 2.654 (1.88), 2.669 (2.17), 2.710 (1.88), 3.538 (2.70), 3.550 (3.69), 3.558 (3.93), 3.568 (2.29), 3.577 (2.17), 3.637 (0.76), 3.670 (2.34), 3.704 (2.75), 3.741 (1.93), 3.774 (3.22), 3.789 (1.99), 3.797 (1.82), 3.807 (3.46), 3.814 (3.05), 3.833 (1.35), 3.895 (1.29), 3.914 (2.64), 3.932 (1.52), 3.940 (1.93), 3.958 (0.94), 3.971 (0.82), 4.000 (1.64), 4.013 (0.94), 4.028 (1.17), 4.042 (1.58), 4.070 (0.88), 4.151 (1.00), 4.182 (1.52), 4.208 (1.47), 4.238 (0.70), 4.758 (2.11), 4.772 (2.93), 4.782 (2.17), 4.831 (2.23), 4.846 (2.87), 4.856 (2.17), 4.957 (2.23), 4.997 (14.01), 5.011 (16.00), 5.051 (2.70), 7.889 (14.07).

### Beispiel 365

### (5S)-5-{[(3S)-3-Fluorpyrrolidin-1-yl]carbonyl}-2-{[2-(trifluormethyl)-1,3-thiazol-4-yl]methyl}-5,6,7,8-tetrahydro[1,2,4]triazolo[4,3-a]pyridin-3(2H)-on

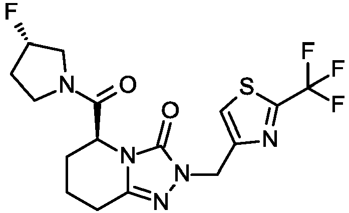

(5S)-3-Oxo-2-{[2-(trifluormethyl)-1,3-thiazol-4-yl]methyl}-2,3,5,6,7,8-hexahydro[1,2,4]triazolo[4,3-a]pyridin-5-carbonsäure (100 mg, 287 µmol) wurde in THF (2.0 ml) vorgelegt und HBTU (142 mg, 373 µmol) und N,N-Diisopropylethylamin (150 µl, 860 µmol) wurden anschließend zugegeben. Nach 15 min Rühren bei Raumtemperatur wurde (3S)-3-Fluorpyrrolidinhydrochlorid (43.3 mg, 345 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 28.0 mg (23 % d. Th.) der Titelverbindung erhalten.
LC-MS (Methode 3): Rₜ = 1.29 min; MS (ESIpos): m/z = 420 [M+H]⁺
¹H-NMR (400 MHz, DMSO-d6) δ [ppm]: -0.149 (0.75), -0.008 (6.39), 0.008 (6.48), 0.146 (0.77), 1.717 (2.91), 1.727 (4.25), 1.740 (5.22), 1.880 (0.95), 1.890 (0.97), 1.913 (1.23), 1.968 (0.71), 1.994 (1.54), 2.003 (1.43), 2.029 (2.36), 2.086 (3.06), 2.105 (3.66), 2.122 (2.23), 2.137 (2.42), 2.172 (0.75), 2.220 (1.37), 2.240 (1.39), 2.270 (1.85), 2.327 (0.93), 2.366 (0.84), 2.525 (2.78), 2.567 (2.80), 2.577 (3.33), 2.593 (2.73), 2.616 (4.25), 2.659 (1.70), 2.669 (1.48), 2.710 (0.86), 3.274 (1.26), 3.348 (1.26), 3.362 (1.15), 3.370 (1.19), 3.397 (1.34), 3.406 (1.34), 3.459 (0.97), 3.468 (1.04), 3.495 (1.39), 3.504 (1.34), 3.614 (0.90), 3.638 (3.94), 3.657 (4.08), 3.665 (2.84), 3.683 (2.51), 3.698 (1.81), 3.729 (2.27), 3.749 (3.24), 3.774 (2.60), 3.793 (2.12), 3.825 (0.51), 3.862 (4.19), 4.691 (1.98), 4.700 (2.45), 4.707 (2.60), 4.716 (2.03), 4.748 (2.58), 4.757 (3.02), 4.763 (3.37), 4.772 (2.45), 4.953 (2.95), 4.993 (16.00), 5.011 (10.53), 5.050 (1.98), 5.258 (2.01), 5.384 (2.73), 5.390 (2.80), 5.517 (1.48), 5.944 (0.64), 7.887 (12.65).

### Beispiel 366

### (5S)-5-{[(3R,4S)-3,4-Difluorpyrrolidin-1-yl]carbonyl}-2-{[2-(trifluormethyl)-1,3-thiazol-4-yl]methyl}-5,6,7,8-tetrahydro[1,2,4]triazolo[4,3-a]pyridin-3(2H)-on

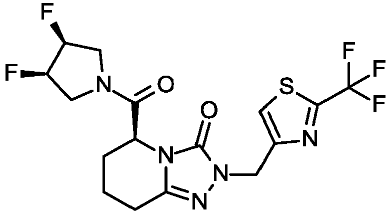

(5S)-3-Oxo-2-{[2-(trifluormethyl)-1,3-thiazol-4-yl]methyl}-2,3,5,6,7,8-hexahydro[1,2,4]triazolo[4,3-a]pyridin-5-carbonsäure (100 mg, 287 µmol) wurde in THF (2.0 ml) vorgelegt und HBTU (142 mg, 373 µmol) und N,N-Diisopropylethylamin (150 µl, 860 µmol) wurden anschließend zugegeben. Nach 15 min Rühren bei Raumtemperatur wurde (3R,4S)-3,4-Difluorpyrrolidinhydrochlorid (49.5 mg, 345 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 25.0 mg (20 % d. Th.) der Titelverbindung erhalten.
LC-MS (Methode 3): Rₜ = 1.37 min; MS (ESIpos): m/z = 438 [M+H]⁺
¹H-NMR (400 MHz, DMSO-d6) δ [ppm]: -0.149 (1.19), -0.008 (10.41), 0.008 (9.40), 0.146 (1.23), 1.673 (1.94), 1.739 (2.42), 1.972 (1.83), 2.017 (2.39), 2.041 (1.53), 2.051 (1.72), 2.058 (1.90), 2.066 (1.79), 2.086 (1.34), 2.094 (1.49), 2.102 (1.27), 2.327 (1.31), 2.366 (1.34), 2.523 (4.21), 2.566 (3.10), 2.577 (3.84), 2.600 (3.92), 2.612 (5.22), 2.624 (3.02), 2.642 (1.08), 2.654 (1.75), 2.669 (1.94), 2.710 (1.49), 3.457 (1.01), 3.482 (1.16), 3.490 (1.94), 3.501 (1.42), 3.512 (1.49), 3.522 (1.53), 3.535 (1.75), 3.545 (1.94), 3.554 (1.12), 3.577 (1.23), 3.616 (1.31), 3.630 (1.49), 3.650 (0.86), 3.663 (1.08), 3.672 (1.38), 3.687 (2.35), 3.702 (2.01), 3.720 (2.09), 3.735 (2.05), 3.752 (1.60), 3.765 (1.75), 3.785 (1.34), 3.799 (0.82), 3.873 (1.27), 3.909 (0.97), 3.932 (1.31), 3.946 (1.45), 3.961 (0.86), 3.981 (1.31), 3.995 (1.34), 4.010 (0.86), 4.024 (0.75), 4.135 (0.90), 4.150 (0.97), 4.164 (0.97), 4.178 (1.57), 4.191 (1.12), 4.204 (1.01), 4.220 (0.90), 4.807 (5.48), 4.957 (2.50), 4.997 (16.00), 5.008 (8.80), 5.013 (9.10), 5.054 (1.60), 5.255 (1.31), 5.266 (1.34), 5.277 (1.57), 5.286 (1.27), 5.297 (1.01), 5.331 (1.16), 5.338 (1.08), 5.352 (1.27), 5.366 (1.08), 5.377 (1.60), 5.389 (1.72), 5.408 (1.49), 5.429 (0.97), 5.474 (1.16), 5.482 (1.23), 5.496 (0.97), 7.880 (8.13), 7.889 (9.14).

### Beispiel 367

### (2S)-1-{[(5S)-3-Oxo-2-{[2-(trifluormethyl)-1,3-thiazol-4-yl]methyl}-2,3,5,6,7,8-hexahydro[1,2,4]triazolo[4,3-a]pyridin-5-yl]carbonyl}pyrrolidin-2-carbonitril

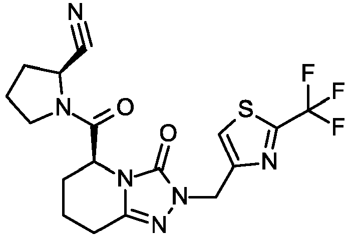

(5S)-3-Oxo-2-{[2-(trifluormethyl)-1,3-thiazol-4-yl]methyl}-2,3,5,6,7,8-hexahydro[1,2,4]triazolo[4,3-a]pyridin-5-carbonsäure (50.0 mg, 144 µmol) wurde in THF (2.0 ml) vorgelegt und HBTU (70.8 mg, 187 µmol) und N,N-Diisopropylethylamin (75 µl, 430 µmol) wurden anschließend zugegeben. Nach 15 min Rühren bei Raumtemperatur wurde (2S)-Pyrrolidin-2-carbonitrilhydrochlorid (22.8 mg, 172 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 20.0 mg (33 % d. Th.) der Titelverbindung erhalten.
LC-MS (Methode 3): Rₜ = 1.36 min; MS (ESIpos): m/z = 427 [M+H]⁺
¹H-NMR (400 MHz, DMSO-d6) δ [ppm]: -0.149 (0.78), -0.008 (6.45), 0.008 (6.32), 0.146 (0.70), 1.245 (0.52), 1.260 (0.70), 1.396 (0.83), 1.665 (1.66), 1.791 (2.18), 1.805 (1.66), 2.048 (6.84), 2.061 (8.33), 2.080 (8.28), 2.096 (4.45), 2.107 (3.27), 2.117 (3.57), 2.138 (3.05), 2.152 (4.23), 2.163 (3.40), 2.178 (1.44), 2.187 (1.92), 2.207 (4.10), 2.219 (1.40), 2.226 (3.88), 2.238 (2.44), 2.246 (1.35), 2.258 (1.96), 2.278 (0.78), 2.328 (1.57), 2.366 (1.61), 2.523 (3.97), 2.574 (3.62), 2.589 (3.10), 2.600 (3.36), 2.614 (2.57), 2.632 (2.40), 2.644 (4.40), 2.657 (2.66), 2.674 (2.27), 2.686 (1.79), 2.710 (1.79), 3.512 (0.39), 3.679 (6.67), 3.696 (13.47), 3.712 (6.63), 4.788 (4.80), 4.799 (5.06), 4.809 (5.84), 4.819 (6.37), 4.824 (5.01), 4.830 (5.32), 4.839 (3.71), 4.964 (1.40), 5.004 (16.00), 5.010 (15.83), 5.051 (1.35), 7.880 (13.34).

### Beispiel 368

### (5S)-5-[(3,3-Difluorpyrrolidin-1-yl)carbonyl]-2-{[6-(trifluormethyl)pyridin-2-yl]methyl}-5,6,7,8-tetrahydro[1,2,4]triazolo[4,3-a]pyridin-3(2H)-on

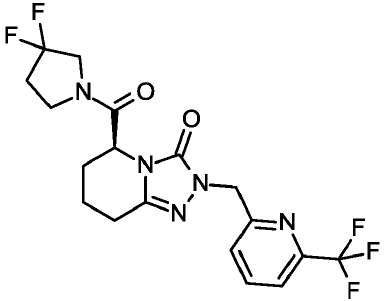

(5S)-3-Oxo-2-{[6-(trifluormethyl)pyridin-2-yl]methyl}-2,3,5,6,7,8-hexahydro[1,2,4]triazolo[4,3-a]pyridin-5-carbonsäure (63.9 mg, 187 µmol) wurde in THF (2.0 ml) vorgelegt und HBTU (92.0 mg, 243 µmol) und N,N-Diisopropylethylamin (98 µl, 560 µmol) wurden anschließend zugegeben. Nach 15 min Rühren bei Raumtemperatur wurde 3,3-Difluorpyrrolidinhydrochlorid (32.2 mg, 224 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 35.5 mg (44 % d. Th.) der Titelverbindung erhalten.
LC-MS (Methode 3): Rₜ = 1.43 min; MS (ESIpos): m/z = 432 [M+H]⁺
¹H-NMR (400 MHz, DMSO-d6) δ [ppm]: -0.149 (0.90), -0.008 (8.08), 0.008 (7.97), 0.146 (0.87), 1.695 (1.03), 1.753 (1.26), 1.916 (0.49), 1.981 (0.72), 2.037 (1.38), 2.328 (1.10), 2.366 (1.33), 2.384 (0.82), 2.411 (1.00), 2.431 (0.97), 2.570 (2.74), 2.584 (2.10), 2.594 (2.21), 2.609 (2.18), 2.628 (2.23), 2.669 (1.92), 2.710 (1.00), 3.546 (1.15), 3.557 (1.62), 3.566 (1.72), 3.576 (1.05), 3.585 (0.95), 3.678 (1.08), 3.712 (1.28), 3.749 (0.85), 3.783 (1.44), 3.795 (0.95), 3.821 (1.56), 3.840 (0.56), 3.899 (0.54), 3.918 (1.23), 3.944 (0.87), 3.977 (0.41), 4.006 (0.79), 4.049 (0.69), 4.076 (0.41), 4.156 (0.49), 4.188 (0.74), 4.213 (0.67), 4.788 (1.00), 4.803 (1.41), 4.812 (0.97), 4.859 (0.97), 4.874 (1.41), 4.884 (0.95), 5.012 (16.00), 7.404 (3.54), 7.424 (3.67), 7.829 (4.00), 7.848 (4.72), 8.081 (2.38), 8.101 (4.18), 8.121 (2.05).

### Beispiel 369

### (5S)-5-[(3-Fluorazetidin-1-yl)carbonyl]-2-{[6-(trifluormethyl)pyridin-2-yl]methyl}-5,6,7,8-tetrahydro[1,2,4]triazolo[4,3-a]pyridin-3(2H)-on

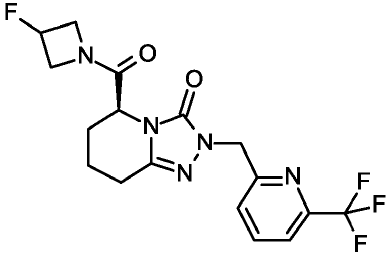

(5S)-3-Oxo-2-{[6-(trifluormethyl)pyridin-2-yl]methyl}-2,3,5,6,7,8-hexahydro[1,2,4]triazolo[4,3-a]pyridin-5-carbonsäure (63.9 mg, 187 µmol) wurde in THF (2.0 ml) vorgelegt und HBTU (92.0 mg, 243 µmol) und N,N-Diisopropylethylamin (98 µl, 560 µmol) wurden anschließend zugegeben. Nach 15 min Rühren bei Raumtemperatur wurde 3-Fluorazetidinhydrochlorid (25.0 mg, 224 µmol) zugegeben und das Reaktionsgemisch über Nacht bei Raumtemperatur gerührt. Es wurde erneut N,N-Diisopropylethylamin (30 µl, 171 µmol) und 3-Fluorazetidinhydrochlorid (10.0 mg, 90 µmol) zugegeben und für 2 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 48.0 mg (64 % d. Th.) der Titelverbindung erhalten.
LC-MS (Methode 4): Rₜ = 0.67 min; MS (ESIpos): m/z = 400 [M+H]⁺
¹H-NMR (400 MHz, DMSO-d6) delta [ppm]: -0.149 (0.59), -0.008 (5.08), 0.146 (0.62), 1.689 (1.49), 1.699 (2.17), 1.723 (3.40), 1.733 (3.51), 1.989 (2.24), 2.001 (2.24), 2.050 (1.98), 2.073 (2.86), 2.327 (0.73), 2.366 (0.61), 2.559 (2.89), 2.577 (2.84), 2.603 (3.43), 2.617 (5.09), 2.630 (2.65), 2.647 (0.99), 2.660 (1.92), 2.670 (1.45), 2.710 (0.69), 3.910 (0.73), 3.936 (1.52), 3.965 (1.55), 3.997 (1.55), 4.026 (0.88), 4.166 (0.65), 4.180 (0.75), 4.194 (0.62), 4.231 (1.23), 4.245 (1.23), 4.281 (1.49), 4.312 (1.53), 4.344 (0.86), 4.371 (1.52), 4.396 (1.02), 4.431 (0.73), 4.460 (0.93), 4.516 (0.70), 4.530 (0.83), 4.572 (4.49), 4.586 (6.34), 4.598 (4.31), 4.637 (0.69), 4.654 (0.77), 4.688 (0.73), 4.705 (0.73), 4.730 (0.59), 5.015 (16.00), 5.354 (0.97), 5.405 (0.94), 5.497 (0.96), 5.548 (0.96), 6.957 (0.24), 7.086 (0.29), 7.213 (0.26), 7.419 (6.28), 7.439 (6.67), 7.827 (6.31), 7.847 (7.44), 8.079 (3.43), 8.099 (6.16), 8.118 (2.92), 9.029 (0.16), 9.156 (0.19).

### Beispiel 370

### (5S)-5-{[(3S)-3-Fluorpyrrolidin-1-yl]carbonyl}-2-{[6-(trifluormethyl)pyridin-2-yl]methyl}-5,6,7,8-tetrahydro[1,2,4]triazolo[4,3-a]pyridin-3(2H)-on

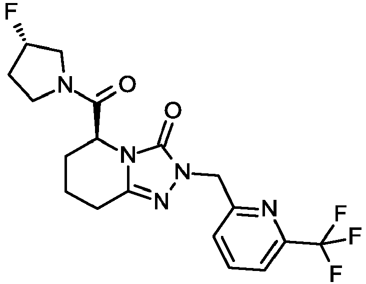

(5S)-3-Oxo-2-{[6-(trifluormethyl)pyridin-2-yl]methyl}-2,3,5,6,7,8-hexahydro[1,2,4]triazolo[4,3-a]pyridin-5-carbonsäure (63.9 mg, 187 µmol) wurde in THF (2.0 ml) vorgelegt und HBTU (92.0 mg, 243 µmol) und N,N-Diisopropylethylamin (98 µl, 560 µmol) wurden anschließend zugegeben. Nach 15 min Rühren bei Raumtemperatur wurde (3S)-3-Fluorpyrrolidinhydrochlorid (28.1 mg, 224 µmol) zugegeben und das Reaktionsgemisch über Nacht bei Raumtemperatur gerührt. Es wurde erneut N,N-Diisopropylethylamin (30 µl, 171 µmol) und 3-Fluorazetidinhydrochlorid (10.0 mg, 90 µmol) zugegeben und für 2 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 45.8 mg (58 % d. Th.) der Titelverbindung erhalten.
LC-MS (Methode 4): Rₜ = 0.69 min; MS (ESIpos): m/z = 414 [M+H]⁺
¹H-NMR (400 MHz, DMSO-d6) δ [ppm]: -0.149 (1.00), -0.008 (8.75), 0.008 (7.10), 0.146 (0.96), 1.751 (2.34), 1.901 (0.50), 1.941 (0.73), 2.006 (0.58), 2.063 (1.27), 2.073 (2.26), 2.086 (1.61), 2.106 (1.76), 2.121 (1.61), 2.271 (0.96), 2.327 (1.27), 2.366 (1.50), 2.524 (2.92), 2.562 (1.84), 2.578 (1.46), 2.588 (1.61), 2.603 (1.34), 2.630 (2.30), 2.670 (1.88), 2.710 (1.42), 3.356 (0.54), 3.377 (0.58), 3.404 (0.61), 3.413 (0.65), 3.476 (0.50), 3.502 (0.73), 3.511 (0.65), 3.616 (0.50), 3.641 (2.34), 3.659 (1.76), 3.684 (1.34), 3.705 (0.88), 3.739 (1.34), 3.758 (1.53), 3.782 (1.34), 3.801 (1.04), 3.867 (2.07), 4.715 (1.04), 4.725 (1.30), 4.732 (1.34), 4.740 (1.00), 4.773 (1.34), 4.782 (1.46), 4.788 (1.73), 4.796 (1.27), 5.008 (16.00), 5.261 (1.00), 5.384 (1.34), 5.392 (1.34), 5.514 (0.77), 7.405 (3.84), 7.425 (4.14), 7.828 (4.37), 7.848 (5.06), 8.081 (2.76), 8.101 (4.95), 8.121 (2.34).

### Beispiel 371

### (5S)-5-{[(3R,4S)-3,4-Difluorpyrrolidin-1-yl]carbonyl}-2-{[6-(trifluormethyl)pyridin-2-yl]methyl}-5,6,7,8-tetrahydro[1,2,4]triazolo[4,3-a]pyridin-3(2H)-on

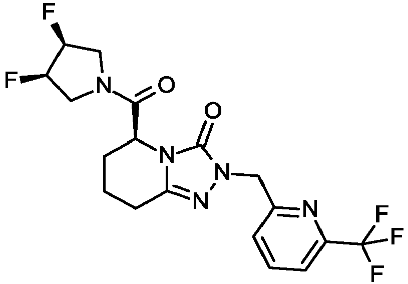

(5S)-3-Oxo-2-{[6-(trifluormethyl)pyridin-2-yl]methyl}-2,3,5,6,7,8-hexahydro[1,2,4]triazolo[4,3-a]pyridin-5-carbonsäure (63.9 mg, 187 µmol) wurde in THF (2.0 ml) vorgelegt und HBTU (92.0 mg, 243 µmol) und N,N-Diisopropylethylamin (98 µl, 560 µmol) wurden anschließend zugegeben. Nach 15 min Rühren bei Raumtemperatur wurde (3R,4S)-3,4-Difluorpyrrolidinhydrochlorid (32.2 mg, 224 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 35.4 mg (44 % d. Th.) der Titelverbindung erhalten.
LC-MS (Methode 4): Rₜ = 0.72 min; MS (ESIpos): m/z = 432 [M+H]⁺
¹H-NMR (400 MHz, DMSO-d6) δ [ppm]: -0.151 (0.30), -0.010 (2.83), 0.006 (2.53), 0.144 (0.31), 1.680 (0.68), 1.757 (16.00), 1.994 (0.65), 2.041 (1.03), 2.072 (3.14), 2.084 (0.76), 2.327 (0.35), 2.365 (0.46), 2.560 (0.94), 2.575 (0.99), 2.586 (1.23), 2.597 (0.93), 2.612 (1.29), 2.624 (1.74), 2.637 (1.02), 2.668 (0.83), 2.709 (0.47), 3.461 (0.31), 3.496 (0.66), 3.518 (0.57), 3.527 (0.57), 3.551 (0.57), 3.581 (0.40), 3.624 (0.44), 3.638 (0.51), 3.679 (0.48), 3.694 (0.89), 3.709 (0.66), 3.727 (0.71), 3.741 (0.69), 3.760 (0.58), 3.772 (0.56), 3.876 (0.42), 3.938 (0.41), 3.952 (0.47), 3.988 (0.46), 4.002 (0.46), 4.180 (0.57), 4.207 (0.37), 4.611 (2.51), 4.833 (2.42), 5.010 (9.06), 5.276 (0.52), 5.409 (0.53), 5.457 (0.44), 7.401 (2.26), 7.421 (2.43), 7.827 (2.30), 7.846 (2.69), 8.081 (1.33), 8.100 (2.38), 8.120 (1.12).

### Beispiel 372

### (5S)-2-{[5-Chlor-4-(trifluormethyl)pyridin-2-yl]methyl}-5-{[(3R,4S)-3,4-difluorpyrrolidin-1-yl]carbonyl}-5,6,7,8-tetrahydro[1,2,4]triazolo[4,3-a]pyridin-3(2H)-on

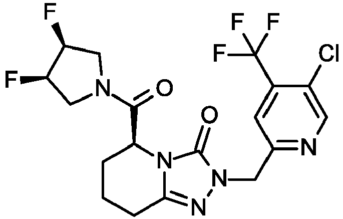

(5S)-2-{[5-Chlor-4-(trifluormethyl)pyridin-2-yl]methyl}-3-oxo-2,3,5,6,7,8-hexahydro[1,2,4]triazolo[4,3-a]pyridin-5-carbonsäure (70.0 mg, 186 µmol) wurde in THF (3.0 ml) vorgelegt und HBTU (91.6 mg, 242 µmol) und N,N-Diisopropylethylamin (97 µl, 560 µmol) wurden anschließend zugegeben. Nach 15 min Rühren bei Raumtemperatur wurde (3R,4S)-3,4-Difluorpyrrolidinhydrochlorid (32.0 mg, 223 µmol) zugegeben und das Reaktionsgemisch für 6 Tage bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 53.4 mg (62 % d. Th.) der Titelverbindung erhalten.
LC-MS (Methode 3): Rₜ = 1.51 min; MS (ESIpos): m/z = 466 [M+H]⁺
¹H-NMR (400 MHz, DMSO-d6) δ [ppm]: -0.149 (0.41), -0.008 (3.04), 0.008 (3.60), 1.175 (0.45), 1.669 (1.31), 1.737 (1.59), 1.988 (1.92), 2.028 (1.81), 2.073 (10.98), 2.327 (1.40), 2.366 (0.71), 2.569 (2.50), 2.579 (1.72), 2.593 (2.39), 2.607 (3.34), 2.619 (1.90), 2.649 (1.21), 2.670 (1.51), 2.690 (1.53), 2.710 (0.73), 3.490 (1.03), 3.512 (1.31), 3.554 (0.93), 3.616 (0.86), 3.629 (0.95), 3.672 (1.55), 3.685 (1.90), 3.705 (1.36), 3.719 (1.42), 3.750 (1.21), 3.771 (0.95), 3.869 (0.88), 3.930 (0.90), 3.943 (0.99), 3.979 (0.97), 3.993 (0.84), 4.008 (0.60), 4.021 (0.67), 4.147 (0.67), 4.173 (1.01), 4.187 (0.75), 4.200 (0.62), 4.215 (0.65), 4.810 (2.63), 4.825 (3.36), 4.835 (2.28), 5.040 (16.00), 5.274 (1.01), 5.351 (0.82), 5.388 (1.03), 5.455 (0.82), 7.718 (9.09), 8.901 (8.66).

### Beispiel 373

### (5S)-2-{[5-Chlor-4-(trifluormethyl)pyridin-2-yl]methyl}-5-[(3-hydroxyazetidin-1-yl)carbonyl]-5,6,7,8-tetrahydro[1,2,4]triazolo[4,3-a]pyridin-3(2H)-on

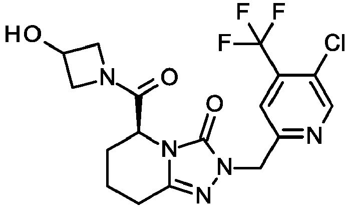

(5S)-2-{[5-Chlor-4-(trifluormethyl)pyridin-2-yl]methyl}-3-oxo-2,3,5,6,7,8-hexahydro[1 ,2,4]triazolo[4,3-a]pyridin-5-carbonsäure (70.0 mg, 186 µmol) wurde in THF (3.0 ml) vorgelegt und HBTU (91.6 mg, 242 µmol) und N,N-Diisopropylethylamin (97 µl, 560 µmol) wurden anschließend zugegeben. Nach 15 min Rühren bei Raumtemperatur wurde Azetidin-3-olhydrochlorid (24.4 mg, 223 µmol) zugegeben und das Reaktionsgemisch für 6 Tage bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 46.0 mg (57 % d. Th.) der Titelverbindung erhalten.
LC-MS (Methode 3): Rₜ = 1.20 min; MS (ESIpos): m/z = 432 [M+H]⁺
¹H-NMR (400 MHz, DMSO-d6) δ [ppm]: -0.149 (1.32), -0.008 (11.98), 0.008 (10.40), 0.146 (1.38), 1.725 (2.70), 1.919 (1.25), 1.953 (2.77), 2.005 (1.78), 2.015 (1.98), 2.327 (2.50), 2.366 (2.44), 2.523 (9.02), 2.569 (2.30), 2.581 (2.57), 2.594 (4.48), 2.606 (2.44), 2.624 (1.12), 2.636 (1.65), 2.669 (2.63), 2.710 (2.57), 3.606 (1.65), 3.627 (2.90), 3.652 (1.65), 3.663 (1.51), 3.934 (1.65), 3.947 (1.98), 4.021 (2.44), 4.042 (3.29), 4.066 (1.45), 4.091 (1.25), 4.107 (1.58), 4.131 (1.25), 4.349 (1.19), 4.366 (2.04), 4.386 (1.51), 4.495 (4.08), 4.509 (4.48), 4.531 (4.67), 4.546 (4.41), 5.037 (16.00), 5.802 (4.94), 7.731 (14.22), 8.898 (5.99).

### Beispiel 374

### (5S)-2-{[5-Chlor-4-(trifluormethyl)pyridin-2-yl]methyl}-5-{[(3S)-3-hydroxypyrrolidin-1-yl]carbonyl}-5,6,7,8-tetrahydro[1,2,4]triazolo[4,3-a]pyridin-3(2H)-on

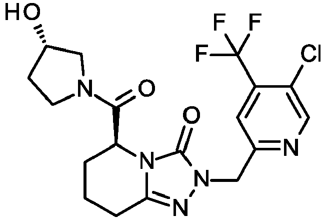

(5S)-2-{[5-Chlor-4-(trifluormethyl)pyridin-2-yl]methyl}-3-oxo-2,3,5,6,7,8-hexahydro[1,2,4]triazolo[4,3-a]pyridin-5-carbonsäure (70.0 mg, 186 µmol) wurde in THF (3.0 ml) vorgelegt und HBTU (91.6 mg, 242 µmol) und N,N-Diisopropylethylamin (97 µl, 560 µmol) wurden anschließend zugegeben. Nach 15 min Rühren bei Raumtemperatur wurde (3S)-Pyrrolidin-3-olhydrochlorid (27.6 mg, 223 µmol) zugegeben und das Reaktionsgemisch für 6 Tage bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 47.2 mg (57 % d. Th.) der Titelverbindung erhalten.
LC-MS (Methode 3): Rₜ = 1.22 min; MS (ESIpos): m/z = 446 [M+H]⁺
¹H-NMR (400 MHz, DMSO-d6) δ [ppm]: -0.149 (0.73), -0.008 (6.82), 0.008 (5.51), 0.146 (0.70), 1.406 (0.61), 1.739 (3.21), 1.848 (1.56), 1.858 (2.05), 1.879 (2.11), 1.973 (2.36), 1.983 (2.48), 1.995 (3.00), 2.006 (3.18), 2.017 (2.69), 2.068 (1.68), 2.327 (1.38), 2.366 (1.01), 2.523 (5.84), 2.605 (4.10), 2.647 (1.71), 2.669 (1.65), 2.709 (1.13), 2.874 (0.92), 3.201 (1.01), 3.232 (1.44), 3.342 (2.63), 3.368 (3.24), 3.386 (2.36), 3.421 (0.92), 3.431 (0.89), 3.453 (1.62), 3.482 (1.53), 3.549 (1.25), 3.564 (2.17), 3.573 (2.39), 3.588 (1.71), 3.654 (1.96), 3.680 (1.47), 3.697 (0.49), 3.755 (0.92), 4.268 (1.99), 4.360 (2.02), 4.704 (1.16), 4.713 (1.25), 4.720 (1.44), 4.727 (1.10), 4.756 (2.23), 4.762 (1.87), 4.826 (1.22), 4.953 (4.10), 4.961 (3.46), 5.034 (16.00), 5.073 (5.54), 5.082 (5.38), 7.714 (6.70), 7.720 (8.08), 8.902 (11.96).

### Beispiel 375

### (5S)-2-{[5-Chlor-4-(trifluormethyl)pyridin-2-yl]methyl}-5-[(3-fluorazetidin-1-yl)carbonyl]-5,6,7,8-tetrahydro[1,2,4]triazolo[4,3-a]pyridin-3(2H)-on

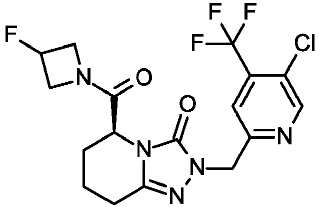

(5S)-2-{[5-Chlor-4-(trifluormethyl)pyridin-2-yl]methyl}-3-oxo-2,3,5,6,7,8-hexahydro[1,2,4]triazolo[4,3-a]pyridin-5-carbonsäure (70.0 mg, 186 µmol) wurde in THF (3.0 ml) vorgelegt und HBTU (91.6 mg, 242 µmol) und N,N-Diisopropylethylamin (97 µl, 560 µmol) wurden anschließend zugegeben. Nach 15 min Rühren bei Raumtemperatur wurde 3-Fluorazetidinhydrochlorid (24.9 mg, 223 µmol) zugegeben und das Reaktionsgemisch für 6 Tage bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 42.0 mg (52 % d. Th.) der Titelverbindung erhalten.
LC-MS (Methode 3): Rₜ = 1.41 min; MS (ESIpos): m/z = 434 [M+H]^{+ 1}H-NMR (400 MHz, DMSO-d6) δ [ppm]: -0.149 (0.64), -0.008 (6.01), 0.008 (5.20), 0.146 (0.71), 1.691 (1.93), 1.715 (3.51), 1.970 (1.70), 2.038 (1.49), 2.327 (1.08), 2.366 (0.53), 2.560 (2.77), 2.585 (3.14), 2.598 (4.47), 2.612 (2.34), 2.628 (0.96), 2.641 (1.56), 2.669 (1.24), 2.710 (0.66), 3.908 (0.99), 3.937 (1.21), 3.999 (1.08), 4.174 (0.64), 4.211 (1.05), 4.226 (1.12), 4.259 (1.10), 4.278 (1.21), 4.305 (1.15), 4.335 (0.64), 4.365 (1.17), 4.392 (0.76), 4.431 (0.57), 4.455 (0.71), 4.522 (0.64), 4.557 (2.73), 4.569 (4.40), 4.581 (2.84), 4.639 (0.60), 4.677 (0.60), 5.044 (16.00), 5.355 (0.71), 5.402 (0.73), 5.498 (0.76), 5.546 (0.76), 7.737 (10.15), 8.891 (4.17), 8.902 (4.15).

### Beispiel 376

### (5S)-2-{[5-Chlor-4-(trifluormethyl)pyridin-2-yl]methyl}-5-{[(3S)-3-fluorpyrrolidin-1-yl]carbonyl}-5,6,7,8-tetrahydro[1,2,4]triazolo[4,3-a]pyridin-3(2H)-on

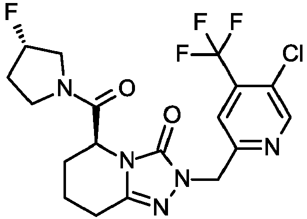

(5S)-2-{[5-Chlor-4-(trifluormethyl)pyridin-2-yl]methyl}-3-oxo-2,3,5,6,7,8-hexahydro[1,2,4]triazolo[4,3-a]pyridin-5-carbonsäure (70.0 mg, 186 µmol) wurde in THF (3.0 ml) vorgelegt und HBTU (91.6 mg, 242 µmol) und N,N-Diisopropylethylamin (97 µl, 560 µmol) wurden anschließend zugegeben. Nach 15 min Rühren bei Raumtemperatur wurde (3S)-3-Fluorpyrrolidinhydrochlorid (28.0 mg, 223 µmol) zugegeben und das Reaktionsgemisch für 6 Tage bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 50.3 mg (60 % d. Th.) der Titelverbindung erhalten.
LC-MS (Methode 3): Rₜ = 1.44 min; MS (ESIpos): m/z = 448 [M+H]⁺
¹H-NMR (400 MHz, DMSO-d6) δ [ppm]: -0.149 (1.07), -0.008 (10.68), 0.008 (7.72), 0.146 (1.11), 1.736 (2.73), 1.929 (0.59), 2.006 (2.25), 2.017 (2.00), 2.073 (4.25), 2.087 (2.03), 2.106 (2.07), 2.137 (1.59), 2.255 (1.15), 2.327 (1.59), 2.366 (1.22), 2.523 (5.73), 2.567 (2.44), 2.583 (1.81), 2.597 (2.11), 2.608 (3.55), 2.620 (2.18), 2.650 (1.33), 2.665 (1.81), 2.670 (1.92), 2.709 (1.33), 3.352 (1.37), 3.371 (1.33), 3.398 (1.00), 3.468 (0.67), 3.495 (1.18), 3.504 (1.22), 3.525 (1.52), 3.554 (1.37), 3.593 (1.92), 3.628 (1.26), 3.652 (1.63), 3.686 (1.15), 3.725 (1.22), 3.747 (1.52), 3.776 (1.29), 3.856 (1.66), 3.920 (0.55), 3.943 (1.03), 3.985 (0.52), 4.009 (0.55), 4.705 (0.85), 4.714 (0.96), 4.721 (1.03), 4.730 (0.78), 4.764 (1.07), 4.779 (1.22), 4.788 (0.92), 4.833 (0.78), 4.846 (1.26), 4.856 (0.74), 4.869 (0.85), 4.884 (1.03), 4.893 (0.81), 5.039 (16.00), 5.258 (1.07), 5.349 (0.70), 5.389 (1.18), 5.481 (0.63), 5.515 (0.63), 7.717 (8.46), 8.903 (7.94).

### Beispiel 377

### (5S)-2-[(5-Chlorpyridin-2-yl)methyl]-5-[(3,3-difluorpyrrolidin-1-yl)carbonyl]-5,6,7,8-tetrahydro[1,2,4]triazolo[4,3-a]pyridin-3(2H)-on

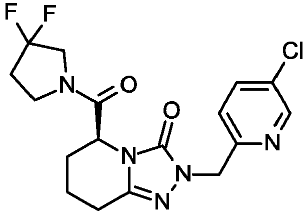

(5S)-2-[(5-Chlorpyridin-2-yl)methyl]-3-oxo-2,3,5,6,7,8-hexahydro[1,2,4]triazolo[4,3-a]pyridin-5-carbonsäure (100 mg, 324 µmol) wurde in THF (2.0 ml) vorgelegt und HBTU (160 mg, 421 µmol) und N,N-Diisopropylethylamin (170 µl, 970 µmol) wurden anschließend zugegeben. Nach 15 min Rühren bei Raumtemperatur wurde 3,3-Difluorpyrrolidinhydrochlorid (55.8 mg, 389 µmol) zugegeben und das Reaktionsgemisch über Nacht bei Raumtemperatur gerührt. Es wurde erneut N,N-Diisopropylethylamin (170 µl, 970 µmol) zugegeben und für 2 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 47.0 mg (36 % d. Th.) der Titelverbindung erhalten.
LC-MS (Methode 3): Rₜ = 1.24 min; MS (ESIpos): m/z = 398 [M+H]⁺
¹H-NMR (400 MHz, DMSO-d6) δ [ppm]: -0.149 (0.69), -0.008 (5.97), 0.008 (5.29), 0.146 (0.69), 1.248 (1.05), 1.265 (1.41), 1.284 (0.90), 1.411 (1.30), 1.699 (1.41), 1.714 (1.52), 1.727 (1.81), 1.917 (0.69), 1.981 (1.63), 1.992 (1.52), 2.021 (1.70), 2.049 (1.23), 2.065 (1.34), 2.074 (1.38), 2.086 (1.12), 2.093 (1.05), 2.328 (1.19), 2.366 (1.67), 2.381 (1.01), 2.411 (1.27), 2.430 (1.27), 2.452 (0.98), 2.569 (4.05), 2.578 (3.08), 2.593 (3.62), 2.608 (3.80), 2.620 (1.85), 2.651 (1.09), 2.665 (1.34), 2.670 (1.38), 2.710 (1.34), 3.538 (1.88), 3.556 (2.82), 3.570 (1.52), 3.577 (1.48), 3.640 (0.51), 3.672 (1.52), 3.705 (1.74), 3.740 (1.38), 3.774 (2.28), 3.791 (1.30), 3.808 (2.14), 3.817 (1.85), 3.835 (0.94), 3.894 (0.83), 3.912 (1.77), 3.931 (0.98), 3.938 (1.27), 3.957 (0.58), 3.973 (0.51), 4.001 (1.09), 4.030 (0.76), 4.044 (1.01), 4.072 (0.62), 4.148 (0.65), 4.180 (0.94), 4.204 (0.94), 4.234 (0.43), 4.761 (1.41), 4.776 (1.92), 4.786 (1.41), 4.836 (1.45), 4.852 (1.92), 4.861 (1.48), 4.914 (16.00), 4.947 (0.47), 4.955 (0.76), 7.199 (6.12), 7.220 (6.66), 7.914 (4.38), 7.920 (4.52), 7.935 (4.24), 7.941 (4.34), 8.572 (5.39), 8.578 (5.29).

### Beispiel 378

### (5S)-2-[(5-Chlorpyridin-2-yl)methyl]-5-{[(3R,4S)-3,4-difluorpyrrolidin-1-yl]carbonyl}-5,6,7,8-tetrahydro[1,2,4]triazolo[4,3-a]pyridin-3(2H)-on

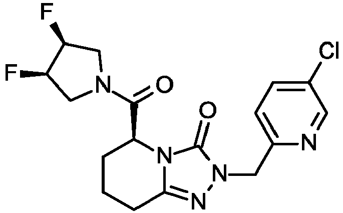

(5S)-2-[(5-Chlorpyridin-2-yl)methyl]-3-oxo-2,3,5,6,7,8-hexahydro[1,2,4]triazolo[4,3-a]pyridin-5-carbonsäure (100 mg, 324 µmol) wurde in THF (2.0 ml) vorgelegt und HBTU (160 mg, 421 µmol) und N,N-Diisopropylethylamin (170 µl, 970 µmol) wurden anschließend zugegeben. Nach 15 min Rühren bei Raumtemperatur wurde (3R,4S)-3,4-Difluorpyrrolidinhydrochlorid (55.8 mg, 389 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 10.0 mg (8 % d. Th.) der Titelverbindung erhalten.
LC-MS (Methode 4): Rₜ = 0.64 min; MS (ESIpos): m/z = 398 [M+H]⁺
¹H-NMR (400 MHz, DMSO-d6) δ [ppm]: -0.008 (1.28), 0.008 (1.25), 1.656 (0.94), 1.669 (1.15), 1.682 (1.23), 1.692 (1.08), 1.707 (0.89), 1.718 (1.18), 1.729 (1.39), 1.740 (1.56), 1.753 (1.30), 1.936 (0.50), 1.943 (0.52), 1.978 (1.16), 1.986 (1.06), 2.000 (0.81), 2.021 (1.50), 2.045 (0.97), 2.055 (1.06), 2.063 (1.17), 2.073 (2.34), 2.090 (0.84), 2.098 (0.92), 2.106 (0.78), 2.582 (1.56), 2.596 (2.30), 2.608 (3.05), 2.620 (1.71), 2.639 (0.60), 2.650 (0.98), 2.663 (0.53), 3.322 (2.23), 3.449 (0.59), 3.459 (0.81), 3.468 (0.57), 3.483 (0.84), 3.493 (1.38), 3.502 (1.01), 3.514 (1.18), 3.524 (1.17), 3.535 (1.04), 3.547 (1.13), 3.555 (0.73), 3.568 (0.60), 3.578 (0.81), 3.588 (0.54), 3.619 (0.83), 3.633 (0.93), 3.652 (0.59), 3.666 (0.67), 3.674 (0.91), 3.688 (1.62), 3.701 (1.11), 3.707 (1.12), 3.722 (1.44), 3.736 (1.26), 3.753 (1.04), 3.765 (1.10), 3.786 (0.86), 3.799 (0.54), 3.852 (0.50), 3.864 (0.63), 3.871 (0.79), 3.900 (0.51), 3.907 (0.56), 3.912 (0.56), 3.919 (0.49), 3.934 (0.75), 3.947 (0.88), 3.962 (0.53), 3.977 (0.62), 3.984 (0.83), 3.997 (0.85), 4.012 (0.55), 4.026 (0.49), 4.133 (0.53), 4.148 (0.61), 4.162 (0.58), 4.175 (0.98), 4.189 (0.67), 4.202 (0.59), 4.217 (0.54), 4.798 (2.25), 4.811 (3.22), 4.821 (2.12), 4.870 (0.57), 4.911 (16.00), 4.958 (0.47), 5.256 (0.79), 5.265 (0.86), 5.276 (0.95), 5.287 (0.84), 5.299 (0.55), 5.314 (0.65), 5.322 (0.61), 5.329 (0.68), 5.338 (0.66), 5.344 (0.62), 5.353 (0.76), 5.366 (0.68), 5.381 (0.83), 5.388 (1.01), 5.398 (0.80), 5.408 (0.91), 5.418 (0.84), 5.429 (0.64), 5.443 (0.61), 5.451 (0.67), 5.457 (0.67), 5.465 (0.71), 5.475 (0.66), 5.482 (0.62), 5.495 (0.55), 7.197 (3.48), 7.200 (3.78), 7.218 (3.76), 7.220 (4.05), 7.912 (2.52), 7.915 (3.07), 7.918 (2.95), 7.921 (2.92), 7.933 (2.46), 7.935 (2.96), 7.939 (2.85), 7.941 (2.78), 8.215 (0.62), 8.571 (3.84), 8.573 (3.84), 8.577 (3.84).

### Beispiel 379

### (5S)-2-[(5-Chlorpyridin-2-yl)methyl]-5-[(3-fluorazetidin-1-yl)carbonyl]-5,6,7,8-tetrahydro[1,2,4]triazolo[4,3-a]pyridin-3(2H)-on

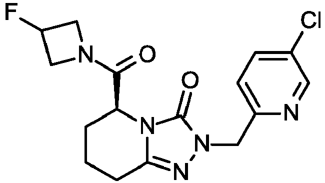

(5S)-2-[(5-Chlorpyridin-2-yl)methyl]-3-oxo-2,3,5,6,7,8-hexahydro[1,2,4]triazolo[4,3-a]pyridin-5-carbonsäure (100 mg, 324 µmol) wurde in THF (2.0 ml) vorgelegt und HBTU (160 mg, 421 µmol) und N,N-Diisopropylethylamin (170 µl, 970 µmol) wurden anschließend zugegeben. Nach 15 min Rühren bei Raumtemperatur wurde 3-Fluorazetidinhydrochlorid (43.4 mg, 389 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und der Rückstand über präparative HPLC (Methode 11) gereinigt. Die produkthaltigen Fraktionen wurden im Vakuum eingeengt und es wurden 1.00 mg (1 % d. Th.) der Titelverbindung erhalten.
LC-MS (Methode 3): Rₜ = 1.04 min; MS (ESIpos): m/z = 366 [M+H]⁺
¹H-NMR (400 MHz, DMSO-d6) δ [ppm]: -0.149 (1.02), -0.008 (8.51), 0.008 (5.75), 0.146 (0.64), 1.722 (4.09), 1.969 (2.04), 2.029 (1.71), 2.040 (1.76), 2.328 (1.76), 2.366 (1.97), 2.564 (3.17), 2.584 (3.86), 2.598 (4.65), 2.611 (2.35), 2.626 (0.92), 2.640 (1.46), 2.653 (0.72), 2.670 (0.82), 2.710 (0.66), 3.904 (0.77), 3.929 (1.38), 3.957 (1.30), 3.990 (1.33), 4.017 (0.77), 4.159 (0.69), 4.173 (0.77), 4.237 (1.15), 4.287 (1.38), 4.316 (1.20), 4.343 (0.84), 4.368 (1.35), 4.397 (0.95), 4.432 (0.74), 4.459 (0.89), 4.513 (0.87), 4.559 (4.75), 4.647 (0.74), 4.683 (0.69), 4.700 (0.69), 4.724 (0.54), 4.915 (16.00), 5.356 (0.84), 5.404 (0.87), 5.499 (0.84), 5.546 (0.84), 7.212 (7.00), 7.234 (7.44), 7.912 (3.55), 7.918 (3.65), 7.933 (3.35), 7.938 (3.35), 8.571 (3.40).

### Beispiel 380

### (5S)-5-{[(3R,4S)-3,4-Difluorpyrrolidin-1-yl]carbonyl}-2-[(5-methoxypyridin-2-yl)methyl]-5,6,7,8-tetrahydro[1,2,4]triazolo[4,3-a]pyridin-3(2H)-on

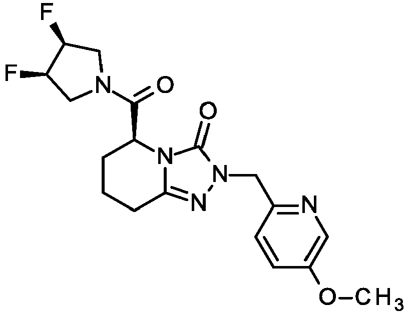

(5S)-2-[(5-Methoxypyridin-2-yl)methyl]-3-oxo-2,3,5,6,7,8-hexahydro[1,2,4]triazolo[4,3-a]pyridin-5-carbonsäure (45.0 mg, 148 µmol) wurde in THF (2.0 ml) vorgelegt und HBTU (72.9 mg, 192 µmol) und N,N-Diisopropylethylamin (77 µl, 440 µmol) wurden anschließend zugegeben. Nach 15 min Rühren bei Raumtemperatur wurde (3R,4S)-3,4-Difluorpyrrolidinhydrochlorid (25.5 mg, 177 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 11.0 mg (19 % d. Th.) der Titelverbindung erhalten.
LC-MS (Methode 3): Rₜ = 1.15 min; MS (ESIpos): m/z = 394 [M+H]⁺
¹H-NMR (400 MHz, DMSO-d6) δ [ppm]: 0.008 (1.72), 1.679 (0.66), 1.720 (0.83), 1.732 (0.84), 1.742 (0.80), 1.968 (0.60), 2.011 (0.73), 2.037 (0.49), 2.047 (0.54), 2.055 (0.57), 2.062 (0.53), 2.071 (0.45), 2.089 (0.43), 2.559 (1.35), 2.569 (0.98), 2.583 (1.23), 2.595 (1.50), 2.607 (0.84), 2.637 (0.50), 3.491 (0.61), 3.501 (0.46), 3.514 (0.51), 3.523 (0.51), 3.535 (0.51), 3.546 (0.56), 3.619 (0.41), 3.632 (0.45), 3.674 (0.46), 3.688 (0.77), 3.704 (0.64), 3.721 (0.66), 3.736 (0.72), 3.754 (0.65), 3.766 (0.66), 3.787 (0.68), 3.810 (16.00), 3.871 (0.41), 3.947 (0.42), 3.983 (0.41), 4.178 (0.46), 4.787 (1.48), 4.797 (1.73), 4.827 (6.88), 5.256 (0.42), 5.267 (0.43), 5.277 (0.49), 5.287 (0.41), 5.378 (0.46), 5.388 (0.53), 5.409 (0.46), 5.418 (0.41), 7.102 (1.50), 7.106 (1.54), 7.124 (1.77), 7.128 (1.82), 7.353 (1.37), 7.357 (1.33), 7.375 (1.17), 7.379 (1.11), 8.209 (1.99), 8.216 (1.83).

### Beispiel 381

### (5S)-5-[(3,3-Difluorpyrrolidin-1-yl)carbonyl]-2-[(5-methoxypyridin-2-yl)methyl]-5,6,7,8-tetrahydro[1,2,4]triazolo[4,3-a]pyridin-3(2H)-on

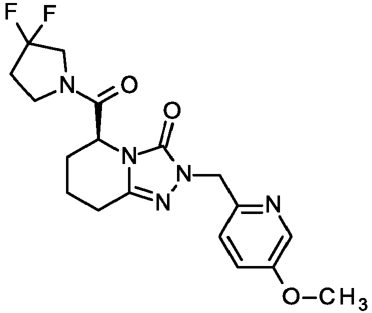

(5S)-2-[(5-Methoxypyridin-2-yl)methyl]-3-oxo-2,3,5,6,7,8-hexahydro[1,2,4]triazolo[4,3-a]pyridin-5-carbonsäure (45.0 mg, 148 µmol) wurde in THF (2.0 ml) vorgelegt und HBTU (72.9 mg, 192 µmol) und N,N-Diisopropylethylamin (77 µl, 440 µmol) wurden anschließend zugegeben. Nach 15 min Rühren bei Raumtemperatur wurde 3,3-Difluorpyrrolidinhydrochlorid (25.5 mg, 177 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 14.0 mg (23 % d. Th.) der Titelverbindung erhalten.
LC-MS (Methode 4): Rₜ = 0.57 min; MS (ESIpos): m/z = 394 [M+H]⁺
¹H-NMR (400 MHz, DMSO-d6) delta [ppm]: -0.008 (0.99), 0.008 (0.92), 1.675 (0.28), 1.722 (0.51), 1.914 (0.21), 1.981 (0.48), 1.998 (0.36), 2.015 (0.40), 2.031 (0.33), 2.042 (0.30), 2.051 (0.30), 2.057 (0.31), 2.067 (0.31), 2.073 (0.49), 2.085 (0.24), 2.093 (0.21), 2.366 (0.24), 2.382 (0.26), 2.412 (0.29), 2.432 (0.29), 2.452 (0.21), 2.566 (0.80), 2.580 (0.84), 2.597 (0.86), 2.608 (0.55), 2.626 (0.19), 2.639 (0.26), 2.670 (0.19), 2.710 (0.18), 2.893 (0.51), 3.540 (0.40), 3.552 (0.54), 3.560 (0.59), 3.571 (0.34), 3.579 (0.31), 3.673 (0.37), 3.706 (0.40), 3.742 (0.32), 3.774 (0.51), 3.790 (0.33), 3.810 (16.00), 3.835 (0.26), 3.897 (0.20), 3.916 (0.41), 3.935 (0.23), 3.942 (0.29), 4.002 (0.25), 4.031 (0.18), 4.043 (0.23), 4.180 (0.24), 4.207 (0.23), 4.748 (0.34), 4.757 (0.39), 4.763 (0.47), 4.772 (0.33), 4.787 (0.21), 4.827 (3.23), 4.831 (3.75), 4.849 (0.40), 4.871 (0.23), 7.106 (1.41), 7.127 (1.69), 7.352 (1.06), 7.359 (1.08), 7.373 (0.91), 7.381 (0.93), 8.209 (1.75), 8.217 (1.73).

### Beispiel 382

### (5S)-5-[(3-Fluorazetidin-1 -yl)carbonyl]-2-[(5-methoxypyridin-2-yl)methyl]-5,6,7,8-tetrahydro[1,2,4]triazolo[4,3-a]pyridin-3(2H)-on

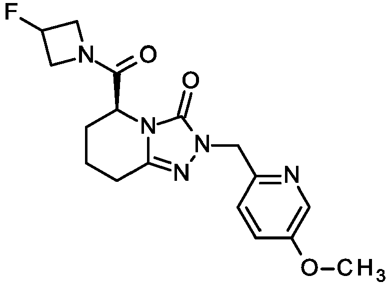

(5S)-2-[(5-Methoxypyridin-2-yl)methyl]-3-oxo-2,3,5,6,7,8-hexahydro[1,2,4]triazolo[4,3-a]pyridin-5-carbonsäure (45.0 mg, 148 µmol) wurde in THF (2.0 ml) vorgelegt und HBTU (72.9 mg, 192 µmol) und N,N-Diisopropylethylamin (77 µl, 440 µmol) wurden anschließend zugegeben. Nach 15 min Rühren bei Raumtemperatur wurde 3-Fluorazetidinhydrochlorid (19.8 mg, 177 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 14.0 mg (26 % d. Th.) der Titelverbindung erhalten.
LC-MS (Methode 4): Rₜ = 0.48 min; MS (ESIpos): m/z = 362 [M+H]⁺
¹H-NMR (400 MHz, DMSO-d6) delta [ppm]: -0.149 (0.34), -0.008 (3.04), 0.008 (2.32), 0.146 (0.30), 1.720 (1.27), 1.960 (0.60), 2.021 (0.51), 2.032 (0.53), 2.327 (0.48), 2.366 (0.51), 2.523 (1.43), 2.572 (1.15), 2.585 (1.45), 2.598 (0.71), 2.614 (0.27), 2.627 (0.42), 2.641 (0.18), 2.670 (0.46), 2.710 (0.44), 3.809 (16.00), 3.901 (0.21), 3.928 (0.41), 3.956 (0.41), 3.988 (0.46), 4.017 (0.23), 4.157 (0.18), 4.172 (0.21), 4.223 (0.32), 4.239 (0.34), 4.252 (0.30), 4.274 (0.35), 4.305 (0.37), 4.340 (0.21), 4.368 (0.39), 4.395 (0.25), 4.430 (0.18), 4.457 (0.25), 4.534 (0.90), 4.545 (1.45), 4.559 (0.88), 4.577 (0.23), 4.648 (0.21), 4.676 (0.19), 4.701 (0.19), 4.831 (5.32), 5.357 (0.25), 5.405 (0.27), 5.500 (0.25), 5.547 (0.25), 7.118 (2.30), 7.140 (2.76), 7.350 (1.17), 7.358 (1.15), 7.372 (0.97), 7.379 (0.99), 8.210 (1.66).

### Beispiel 383

### (5S)-2-[(6-Chlorpyridin-2-yl)methyl]-5-[(3,3-difluorpyrrolidin-1-yl)carbonyl]-5,6,7,8-tetrahydro[1,2,4]triazolo[4,3-a]pyridin-3(2H)-on

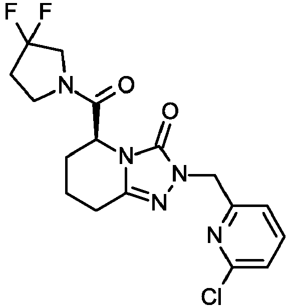

(5S)-2-[(6-Chlorpyridin-2-yl)methyl]-3-oxo-2,3,5,6,7,8-hexahydro[1,2,4]triazolo[4,3-a]pyridin-5-carbonsäure (62.9 mg, 204 µmol) wurde in THF (2.0 ml) vorgelegt und HBTU (100 mg, 265 µmol) und N,N-Diisopropylethylamin (110 µl, 610 µmol) wurden anschließend zugegeben. Nach 15 min Rühren bei Raumtemperatur wurde 3,3-Difluorpyrrolidinhydrochlorid (35.1 mg, 244 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 52.6 mg (65 % d. Th.) der Titelverbindung erhalten.
LC-MS (Methode 3): Rₜ = 1.24 min; MS (ESIpos): m/z = 398 [M+H]⁺
¹H-NMR (400 MHz, DMSO-d6) δ [ppm]: -0.149 (1.19), -0.008 (10.77), 0.008 (8.41), 0.146 (1.17), 1.483 (6.37), 1.687 (1.00), 1.746 (1.22), 1.980 (0.72), 2.028 (1.32), 2.056 (0.97), 2.072 (1.69), 2.082 (0.85), 2.327 (1.17), 2.366 (1.07), 2.382 (0.85), 2.411 (1.02), 2.430 (0.97), 2.452 (0.80), 2.523 (4.21), 2.566 (2.51), 2.580 (2.14), 2.591 (2.49), 2.605 (2.71), 2.620 (2.34), 2.632 (1.29), 2.665 (1.44), 2.670 (1.47), 2.710 (0.85), 3.541 (1.24), 3.553 (1.74), 3.562 (1.84), 3.572 (1.07), 3.581 (0.97), 3.674 (1.12), 3.707 (1.32), 3.744 (0.87), 3.778 (1.47), 3.791 (0.95), 3.799 (0.82), 3.811 (1.59), 3.836 (0.62), 3.896 (0.60), 3.915 (1.27), 3.941 (0.90), 3.960 (0.42), 4.000 (0.77), 4.030 (0.50), 4.043 (0.72), 4.072 (0.45), 4.153 (0.42), 4.182 (0.72), 4.208 (0.67), 4.776 (1.00), 4.791 (1.37), 4.801 (1.02), 4.848 (1.09), 4.864 (1.52), 4.873 (1.19), 4.892 (16.00), 7.134 (4.18), 7.153 (4.45), 7.433 (3.76), 7.453 (4.18), 7.840 (3.04), 7.860 (5.42), 7.879 (2.69).

### Beispiel 384

### (5S)-2-[(6-Chlorpyridin-2-yl)methyl]-5-[(3-fluorazetidin-1 -yl)carbonyl]-5,6,7,8-tetrahydro[1,2,4]triazolo[4,3-a]pyridin-3(2H)-on

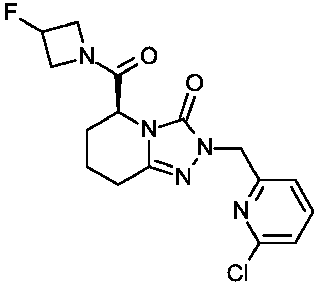

(5S)-2-[(6-Chlorpyridin-2-yl)methyl]-3-oxo-2,3,5,6,7,8-hexahydro[1,2,4]triazolo[4,3-a]pyridin-5-carbonsäure (62.9 mg, 204 µmol) wurde in THF (2.0 ml) vorgelegt und HBTU (100 mg, 265 µmol) und N,N-Diisopropylethylamin (110 µl, 610 µmol) wurden anschließend zugegeben. Nach 15 min Rühren bei Raumtemperatur wurde 3-Fluorazetidinhydrochlorid (27.3 mg, 244 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 45.5 mg (61 % d. Th.) der Titelverbindung erhalten.
LC-MS (Methode 3): Rₜ = 1.04 min; MS (ESIpos): m/z = 366 [M+H]⁺
¹H-NMR (400 MHz, DMSO-d6) δ [ppm]: -0.149 (1.89), -0.008 (16.00), 0.008 (15.16), 0.146 (1.89), 1.722 (3.70), 1.980 (2.17), 2.044 (1.96), 2.327 (1.75), 2.366 (1.26), 2.574 (3.42), 2.597 (4.33), 2.611 (5.03), 2.625 (2.59), 2.653 (1.68), 2.670 (2.52), 2.710 (1.33), 3.904 (0.84), 3.932 (1.61), 3.962 (1.54), 3.993 (1.54), 4.019 (0.98), 4.175 (0.84), 4.280 (1.33), 4.308 (1.40), 4.368 (1.47), 4.427 (0.77), 4.456 (0.98), 4.559 (4.75), 4.574 (6.57), 4.585 (4.96), 4.652 (0.84), 4.686 (0.77), 4.705 (0.77), 4.893 (15.02), 4.898 (15.09), 5.355 (0.98), 5.405 (0.91), 5.497 (0.98), 5.549 (0.91), 7.150 (4.61), 7.169 (4.89), 7.433 (5.66), 7.452 (6.29), 7.839 (3.42), 7.858 (6.50), 7.877 (3.14).

### Beispiel 385

### (5S)-2-[(6-Chlorpyridin-2-yl)methyl]-5-{[(3R,4S)-3,4-difluorpyrrolidin-1-yl]carbonyl}-5,6,7,8-tetrahydro[1,2,4]triazolo[4,3-a]pyridin-3(2H)-on

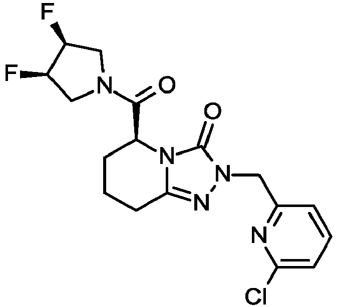

(5S)-2-[(6-Chlorpyridin-2-yl)methyl]-3-oxo-2,3,5,6,7,8-hexahydro[1,2,4]triazolo[4,3-a]pyridin-5-carbonsäure (62.9 mg, 204 µmol) wurde in THF (2.0 ml) vorgelegt und HBTU (100 mg, 265 µmol) und N,N-Diisopropylethylamin (110 µl, 610 µmol) wurden anschließend zugegeben. Nach 15 min Rühren bei Raumtemperatur wurde (3R,4S)-3,4-Difluorpyrrolidinhydrochlorid (35.1 mg, 244 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 60.4 mg (75 % d. Th.) der Titelverbindung erhalten.
LC-MS (Methode 3): Rₜ = 1.17 min; MS (ESIpos): m/z = 398 [M+H]⁺
¹H-NMR (400 MHz, DMSO-d6) δ [ppm]: -0.149 (0.90), -0.008 (7.33), 0.008 (6.93), 0.146 (0.97), 1.677 (1.17), 1.747 (1.40), 1.988 (1.07), 2.029 (1.43), 2.073 (5.97), 2.105 (0.87), 2.327 (0.83), 2.366 (0.83), 2.523 (2.77), 2.558 (1.87), 2.573 (1.70), 2.584 (2.30), 2.608 (2.33), 2.619 (3.17), 2.631 (1.77), 2.665 (1.30), 2.670 (1.30), 2.710 (0.97), 3.458 (0.60), 3.493 (1.23), 3.503 (0.90), 3.516 (1.00), 3.524 (1.00), 3.538 (0.97), 3.548 (1.03), 3.579 (0.77), 3.622 (0.80), 3.635 (0.97), 3.654 (0.63), 3.676 (0.90), 3.691 (1.57), 3.707 (1.33), 3.725 (1.20), 3.738 (1.17), 3.757 (1.00), 3.770 (1.03), 3.789 (0.67), 3.804 (0.57), 3.875 (0.77), 3.915 (0.57), 3.934 (0.77), 3.946 (0.87), 3.961 (0.53), 3.983 (0.80), 3.998 (0.83), 4.011 (0.53), 4.026 (0.50), 4.136 (0.53), 4.151 (0.60), 4.165 (0.67), 4.179 (0.97), 4.193 (0.70), 4.206 (0.60), 4.220 (0.53), 4.824 (3.23), 4.891 (16.00), 5.266 (0.83), 5.276 (0.90), 5.288 (0.83), 5.299 (0.53), 5.314 (0.67), 5.353 (0.80), 5.390 (0.97), 5.409 (0.93), 5.419 (0.87), 5.430 (0.63), 5.452 (0.70), 7.134 (3.47), 7.153 (3.70), 7.433 (4.50), 7.453 (5.13), 7.838 (2.67), 7.842 (2.93), 7.858 (4.63), 7.861 (5.10), 7.878 (2.37), 7.881 (2.57).

### Beispiel 386

### (5S)-2-[(6-Chlorpyridin-2-yl)methyl]-5-{[(3S)-3-fluorpyrrolidin-1-yl]carbonyl}-5,6,7,8-tetrahydro[1,2,4]triazolo[4,3-a]pyridin-3(2H)-on

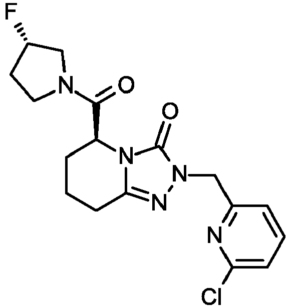

(5S)-2-[(6-Chlorpyridin-2-yl)methyl]-3-oxo-2,3,5,6,7,8-hexahydro[1,2,4]triazolo[4,3-a]pyridin-5-carbonsäure (62.9 mg, 204 µmol) wurde in THF (2.0 ml) vorgelegt und HBTU (100 mg, 265 µmol) und N,N-Diisopropylethylamin (110 µl, 610 µmol) wurden anschließend zugegeben. Nach 15 min Rühren bei Raumtemperatur wurde (3S)-3-Fluorpyrrolidinhydrochlorid (30.7 mg, 244 µmol) zugegeben und das Reaktionsgemisch über Nacht bei Raumtemperatur gerührt. Es wurde erneut HBTU (38 mg, 102 µmol) und N,N-Diisopropylethylamin (18 µl, 102 µmol) zugegeben und über Nacht gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 53.3 mg (69 % d. Th.) der Titelverbindung erhalten.
LC-MS (Methode 3): Rₜ = 1.09 min; MS (ESIpos): m/z = 380 [M+H]⁺
¹H-NMR (400 MHz, DMSO-d6) δ [ppm]: -0.149 (0.48), -0.008 (4.53), 0.008 (3.45), 0.146 (0.46), 1.747 (2.81), 1.890 (0.58), 1.932 (0.79), 1.968 (0.40), 2.005 (0.79), 2.041 (1.27), 2.073 (2.00), 2.090 (1.79), 2.108 (2.12), 2.139 (1.47), 2.222 (0.79), 2.270 (1.06), 2.327 (0.58), 2.366 (0.46), 2.560 (1.85), 2.575 (1.52), 2.584 (1.79), 2.602 (1.62), 2.622 (2.47), 2.665 (1.25), 2.710 (0.42), 3.278 (0.71), 3.333 (1.04), 3.352 (0.58), 3.365 (0.62), 3.374 (0.66), 3.401 (0.71), 3.409 (0.73), 3.462 (0.56), 3.471 (0.58), 3.498 (0.79), 3.507 (0.73), 3.613 (0.54), 3.638 (2.72), 3.656 (2.06), 3.682 (1.33), 3.701 (1.02), 3.733 (1.35), 3.753 (1.75), 3.774 (1.45), 3.795 (1.18), 3.863 (2.43), 4.706 (1.23), 4.716 (1.66), 4.722 (1.47), 4.732 (1.14), 4.763 (1.47), 4.772 (1.70), 4.778 (1.85), 4.787 (1.37), 4.888 (16.00), 5.260 (1.14), 5.383 (1.50), 5.390 (1.54), 5.514 (0.83), 6.519 (0.96), 7.135 (4.61), 7.154 (4.90), 7.433 (4.78), 7.453 (5.32), 7.841 (3.97), 7.860 (7.07), 7.879 (3.45).

### Beispiel 387

### (5S)-2-{[3-Chlor-4-(trifluormethyl)pyridin-2-yl]methyl}-5-[(3,3-difluorpyrrolidin-1-yl)carbonyl]-5,6,7,8-tetrahydro[1,2,4]triazolo[4,3-a]pyridin-3(2H)-on

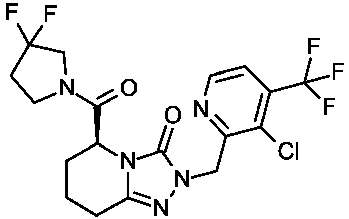

(5S)-2-{[3-Chlor-4-(trifluormethyl)pyridin-2-yl]methyl}-3-oxo-2,3,5,6,7,8-hexahydro[1,2,4]triazolo[4,3-a]pyridin-5-carbonsäure (47.0 mg, 125 µmol) wurde in THF (2.0 ml) bei Raumtemperatur vorgelegt. Anschließend wurde HBTU (61.5 mg, 162 µmol) und N,N-Diisopropylethylamin (65 µl, 370 µmol) zugegeben. Nach 15 min Rühren wurde 3,3-Difluorpyrrolidinhydrochlorid (21.5 mg, 150 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 18.5 mg (32 % d. Th.) der Titelverbindung erhalten.
LC-MS (Methode 3): Rₜ = 1.47 min; MS (ESIpos): m/z = 466 [M+H]⁺
¹H-NMR (400 MHz, DMSO-d6) δ [ppm]: -0.149 (1.17), 0.008 (8.40), 0.146 (1.07), 1.711 (4.48), 1.978 (2.57), 2.328 (2.38), 2.366 (2.47), 2.576 (5.46), 2.589 (6.16), 2.603 (3.31), 2.631 (1.54), 2.670 (2.05), 2.710 (1.91), 3.533 (3.17), 3.552 (5.22), 3.573 (2.47), 3.671 (2.33), 3.705 (2.61), 3.736 (2.01), 3.769 (3.27), 3.802 (2.61), 3.813 (2.57), 3.832 (1.73), 3.911 (2.47), 3.939 (1.77), 3.997 (1.73), 4.039 (1.45), 4.143 (1.03), 4.176 (1.45), 4.201 (1.59), 4.769 (2.89), 4.847 (2.71), 4.858 (2.15), 5.104 (3.50), 5.144 (13.62), 5.166 (16.00), 5.207 (3.64), 7.844 (10.78), 7.856 (10.87), 8.735 (9.19), 8.748 (9.00).

### Beispiel 388

### (5S)-2-{[3-Chlor-4-(trifluormethyl)pyridin-2-yl]methyl}-5-{[(3R,4S)-3,4-difluorpyrrolidin-1-yl]carbonyl}-5,6,7,8-tetrahydro[1,2,4]triazolo[4,3-a]pyridin-3(2H)-on

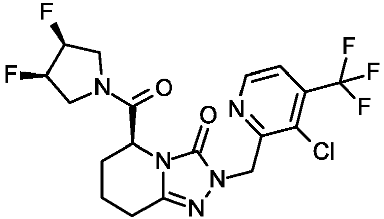

(5S)-2-{[3-Chlor-4-(trifluormethyl)pyridin-2-yl]methyl}-3-oxo-2,3,5,6,7,8-hexahydro[1,2,4]triazolo[4,3-a]pyridin-5-carbonsäure (50.0 mg, 133 µmol) wurde in THF (3.1 ml) vorgelegt und HBTU (65.4 mg, 173 µmol) und N,N-Diisopropylethylamin (69 µl, 400 µmol) wurden anschließend zugegeben. Nach 15 min Rühren bei Raumtemperatur wurde (3R,4S)-3,4-Difluorpyrrolidinhydrochlorid (22.9 mg, 159 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 37.0 mg (60 % d. Th.) der Titelverbindung erhalten.
LC-MS (Methode 3): Rₜ = 1.40 min; MS (ESIpos): m/z = 466 [M+H]⁺
¹H-NMR (400 MHz, DMSO-d6) δ [ppm]: -0.149 (1.23), -0.008 (15.35), 0.008 (9.72), 0.146 (1.36), 1.703 (5.38), 1.715 (5.89), 1.725 (5.77), 1.917 (1.30), 1.958 (2.85), 1.996 (2.91), 2.008 (2.91), 2.040 (2.59), 2.049 (2.79), 2.057 (2.91), 2.065 (2.85), 2.073 (3.50), 2.084 (2.07), 2.092 (2.20), 2.100 (1.94), 2.327 (2.01), 2.332 (1.49), 2.366 (2.20), 2.519 (13.80), 2.524 (12.89), 2.565 (5.51), 2.578 (6.41), 2.589 (8.16), 2.602 (4.66), 2.619 (1.88), 2.632 (2.66), 2.665 (1.94), 2.670 (2.46), 2.674 (1.81), 2.710 (2.46), 3.456 (1.49), 3.490 (2.91), 3.499 (2.07), 3.511 (2.66), 3.520 (2.79), 3.530 (2.40), 3.545 (2.33), 3.562 (1.43), 3.573 (1.88), 3.583 (1.30), 3.616 (2.01), 3.630 (2.20), 3.649 (1.49), 3.663 (1.75), 3.672 (2.40), 3.685 (3.50), 3.695 (2.91), 3.705 (2.66), 3.717 (3.50), 3.731 (2.59), 3.747 (2.59), 3.759 (2.46), 3.780 (2.07), 3.793 (1.30), 3.869 (2.01), 3.905 (1.43), 3.931 (1.88), 3.945 (2.14), 3.959 (1.43), 3.980 (2.20), 3.994 (2.14), 4.009 (1.36), 4.023 (1.23), 4.128 (1.36), 4.143 (1.62), 4.156 (1.49), 4.170 (2.53), 4.184 (1.68), 4.197 (1.49), 4.212 (1.36), 4.791 (6.87), 4.801 (8.10), 4.807 (8.74), 4.816 (6.35), 5.101 (4.28), 5.142 (15.35), 5.146 (14.77), 5.157 (12.96), 5.172 (12.50), 5.198 (2.01), 5.213 (3.95), 5.254 (2.07), 5.262 (2.14), 5.273 (2.46), 5.284 (2.01), 5.297 (1.49), 5.311 (1.68), 5.326 (1.75), 5.335 (1.75), 5.350 (2.07), 5.364 (1.94), 5.387 (2.59), 5.406 (2.33), 5.415 (2.07), 5.426 (1.62), 5.440 (1.55), 5.449 (1.75), 5.463 (1.75), 5.472 (1.68), 5.493 (1.43), 7.844 (15.74), 7.856 (16.00), 8.735 (10.62), 8.748 (10.11).

### Beispiel 389

### (5S)-2-{[3-Chlor-4-(trifluormethyl)pyridin-2-yl]methyl}-5-[(3-fluorazetidin-1-yl)carbonyl]-5,6,7,8-tetrahydro[1,2,4]triazolo[4,3-a]pyridin-3(2H)-on

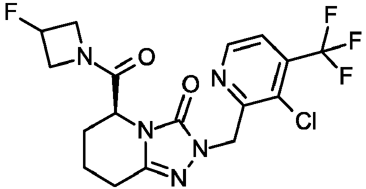

(5S)-2-{[3-Chlor-4-(trifluormethyl)pyridin-2-yl]methyl}-3-oxo-2,3,5,6,7,8-hexahydro[1,2,4]triazolo[4,3-a]pyridin-5-carbonsäure (50.0 mg, 133 µmol) wurde in THF (3.1 ml) vorgelegt und HBTU (65.4 mg, 173 µmol) und N,N-Diisopropylethylamin (69 µl, 400 µmol) wurden anschließend zugegeben. Nach 15 min Rühren bei Raumtemperatur wurde 3-Fluorazetidinhydrochlorid (17.8 mg, 159 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 30.0 mg (52 % d. Th.) der Titelverbindung erhalten.
LC-MS (Methode 3): Rₜ = 1.30 min; MS (ESIpos): m/z = 434 [M+H]⁺
¹H-NMR (400 MHz, DMSO-d6) δ [ppm]: -0.008 (1.41), 0.008 (1.30), 1.721 (4.73), 1.934 (1.28), 1.946 (1.80), 1.957 (1.88), 1.967 (1.75), 1.979 (1.27), 1.991 (0.90), 2.008 (1.32), 2.026 (1.85), 2.044 (1.66), 2.063 (1.20), 2.073 (0.76), 2.080 (0.64), 2.524 (1.40), 2.572 (3.52), 2.586 (5.17), 2.599 (2.48), 2.614 (0.89), 2.628 (1.52), 2.642 (0.62), 3.895 (0.63), 3.923 (1.28), 3.953 (1.26), 3.985 (1.30), 4.012 (0.73), 4.155 (0.55), 4.169 (0.62), 4.184 (0.51), 4.234 (0.97), 4.286 (1.22), 4.314 (1.22), 4.348 (0.73), 4.368 (1.16), 4.389 (0.84), 4.426 (0.57), 4.453 (0.76), 4.509 (0.60), 4.526 (2.15), 4.537 (3.77), 4.551 (3.73), 4.561 (2.07), 4.576 (0.70), 4.604 (0.47), 4.620 (0.56), 4.635 (0.63), 4.646 (0.55), 4.662 (0.58), 4.671 (0.59), 4.686 (0.62), 4.699 (0.55), 4.713 (0.49), 5.118 (1.66), 5.159 (16.00), 5.171 (5.56), 5.213 (0.81), 5.355 (0.81), 5.370 (0.60), 5.381 (0.64), 5.389 (0.73), 5.396 (0.82), 5.497 (0.78), 5.505 (0.70), 5.512 (0.62), 5.532 (0.71), 5.539 (0.81), 7.845 (8.15), 7.857 (8.51), 8.722 (2.50), 8.734 (4.62), 8.747 (2.37).

### Beispiel 390

### (5S)-2-{[3-Chlor-4-(trifluormethyl)pyridin-2-yl]methyl}-5-[(3-hydroxyazetidin-1-yl)carbonyl]-5,6,7,8-tetrahydro[1,2,4]triazolo[4,3-a]pyridin-3(2H)-on

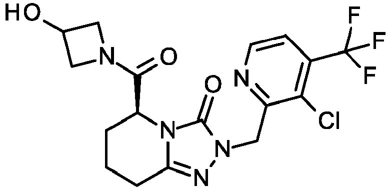

(5S)-2-{[3-Chlor-4-(trifluormethyl)pyridin-2-yl]methyl}-3-oxo-2,3,5,6,7,8-hexahydro[1,2,4]triazolo[4,3-a]pyridin-5-carbonsäure (50.0 mg, 133 µmol) wurde in THF (3.1 ml) vorgelegt und HBTU (65.4 mg, 173 µmol) und N,N-Diisopropylethylamin (69 µl, 400 µmol) wurden anschließend zugegeben. Nach 15 min Rühren bei Raumtemperatur wurde Azetidin-3-olhydrochlorid (17.4 mg, 159 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 26.0 mg (45 % d. Th.) der Titelverbindung erhalten.
LC-MS (Methode 3): Rₜ = 1.11 min; MS (ESIpos): m/z = 432 [M+H]⁺
¹H-NMR (400 MHz, DMSO-d6) δ [ppm]: -0.149 (1.15), 0.008 (8.71), 0.146 (1.10), 1.706 (7.23), 1.719 (7.18), 1.903 (2.41), 1.915 (2.47), 1.938 (4.55), 1.989 (2.30), 2.001 (3.23), 2.014 (3.01), 2.023 (3.01), 2.038 (2.30), 2.328 (2.30), 2.366 (1.81), 2.523 (9.81), 2.564 (5.48), 2.579 (7.07), 2.592 (3.45), 2.607 (1.48), 2.621 (2.25), 2.670 (2.19), 2.710 (1.75), 3.593 (2.47), 3.601 (2.79), 3.627 (3.78), 3.643 (2.58), 3.658 (2.52), 3.668 (2.47), 3.943 (2.74), 3.954 (3.18), 4.020 (4.82), 4.043 (4.93), 4.061 (2.52), 4.097 (1.97), 4.113 (2.68), 4.137 (1.86), 4.347 (1.92), 4.366 (3.23), 4.387 (2.41), 4.508 (13.21), 5.111 (1.97), 5.152 (16.00), 5.157 (13.97), 5.164 (12.00), 5.204 (1.64), 5.803 (5.42), 7.844 (13.70), 7.857 (14.19), 8.733 (11.29), 8.745 (11.01).

### Beispiel 391

### (5S)-2-{[3-Chlor-4-(trifluormethyl)pyridin-2-yl]methyl}-5-{[(3S)-3-hydroxypyrrolidin-1-yl]carbonyl}-5,6,7,8-tetrahydro[1,2,4]triazolo[4,3-a]pyridin-3(2H)-on

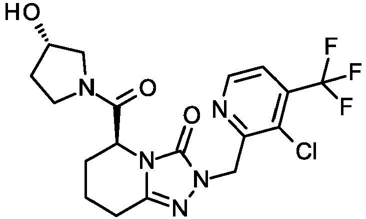

(5S)-2-{[3-Chlor-4-(trifluormethyl)pyridin-2-yl]methyl}-3-oxo-2,3,5,6,7,8-hexahydro[1,2,4]triazolo[4,3-a]pyridin-5-carbonsäure (50.0 mg, 133 µmol) wurde in THF (2.0 ml) vorgelegt und HBTU (65.4 mg, 173 µmol) und N,N-Diisopropylethylamin (69 µl, 400 µmol) wurden anschließend zugegeben. Nach 15 min Rühren bei Raumtemperatur wurde (3S)-Pyrrolidin-3-olhydrochlorid (19.7 mg, 159 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 11.0 mg (19 % d. Th.) der Titelverbindung erhalten.
LC-MS (Methode 3): Rₜ = 1.12 min; MS (ESIpos): m/z = 446 [M+H]⁺
¹H-NMR (400 MHz, DMSO-d6) δ [ppm]: -0.150 (1.86), -0.008 (16.00), 0.008 (12.93), 0.146 (1.86), 1.243 (2.08), 1.257 (2.30), 1.728 (6.03), 1.846 (2.63), 1.855 (2.74), 1.877 (3.07), 1.959 (2.85), 2.004 (3.07), 2.044 (3.07), 2.327 (4.60), 2.366 (3.29), 2.585 (5.92), 2.626 (2.63), 2.669 (5.04), 2.689 (1.86), 2.709 (3.51), 3.375 (3.95), 3.435 (2.08), 3.456 (2.85), 3.566 (1.75), 3.644 (3.07), 3.654 (4.49), 3.682 (3.40), 4.262 (2.63), 4.363 (2.85), 4.706 (3.62), 4.714 (2.74), 4.746 (2.52), 5.007 (1.86), 5.036 (1.75), 5.091 (3.95), 5.132 (12.05), 5.159 (10.52), 5.199 (3.07), 7.842 (10.85), 7.855 (11.18), 7.910 (2.30), 8.738 (8.44), 8.750 (8.00).

### Beispiel 392

### (5S)-2-{[3-Chlor-4-(trifluormethyl)pyridin-2-yl]methyl}-5-{[(3S)-3-fluorpyrrolidin-1-yl]carbonyl}-5,6,7,8-tetrahydro[1,2,4]triazolo[4,3-a]pyridin-3(2H)-on

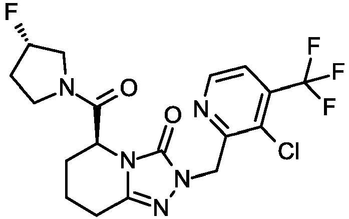

(5S)-2-{[3-Chlor-4-(trifluormethyl)pyridin-2-yl]methyl}-3-oxo-2,3,5,6,7,8-hexahydro[1,2,4]triazolo[4,3-a]pyridin-5-carbonsäure (53.0 mg, 141 µmol) wurde in THF (2.0 ml) bei Raumtemperatur vorgelegt. Anschließend wurden HBTU (69.4 mg, 183 µmol) und N,N-Diisopropylethylamin (74 µl, 420 µmol) zugegeben. Nach 15 min Rühren wurde (3S)-3-Fluorpyrrolidin (15.0 mg, 169 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und der Rückstand wurde über präparative HPLC aufgereinigt (Methode 10). Die produkthaltigen Fraktionen wurden im Vakuum eingeengt und es wurden 11.0 mg (17 % d. Th.) der Titelverbindung erhalten.
LC-MS (Methode 3): Rₜ = 1.33 min; MS (ESIpos): m/z = 448 [M+H]⁺
¹H-NMR (400 MHz, DMSO-d6) δ [ppm]: -0.149 (1.89), -0.008 (16.00), 0.008 (13.03), 0.146 (1.80), 1.734 (6.56), 1.901 (1.62), 1.990 (1.98), 2.016 (2.43), 2.089 (4.22), 2.105 (4.22), 2.122 (2.79), 2.137 (2.61), 2.265 (2.07), 2.327 (3.96), 2.366 (2.61), 2.523 (13.30), 2.570 (3.87), 2.592 (4.85), 2.634 (1.80), 2.669 (4.04), 2.709 (2.52), 3.371 (1.71), 3.406 (1.44), 3.469 (1.08), 3.496 (1.71), 3.629 (4.22), 3.655 (4.31), 3.682 (2.79), 3.692 (2.07), 3.728 (3.33), 3.747 (3.60), 3.771 (2.70), 3.793 (2.25), 3.862 (4.67), 4.693 (2.16), 4.702 (2.70), 4.708 (2.79), 4.717 (2.16), 4.750 (2.70), 4.758 (3.15), 4.765 (3.51), 4.774 (2.61), 5.096 (3.87), 5.137 (12.85), 5.160 (8.54), 5.170 (9.62), 5.200 (2.07), 5.211 (3.24), 5.257 (2.25), 5.388 (2.97), 5.508 (1.62), 7.844 (11.51), 7.856 (12.04), 8.738 (9.17), 8.751 (8.90).

### Beispiel 393

### (5S)-2-[(3-Chlorpyridin-2-yl)methyl]-5-{[(3S)-3-fluorpyrrolidin-1-yl]carbonyl}-5,6,7,8-tetrahydro[1,2,4]triazolo[4,3-a]pyridin-3(2H)-on

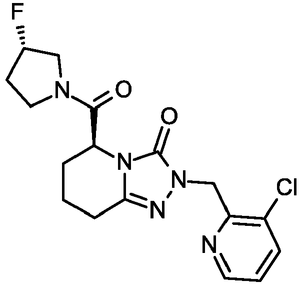

(5S)-2-[(3-Chlorpyridin-2-yl)methyl]-3-oxo-2,3,5,6,7,8-hexahydro[1,2,4]triazolo[4,3-a]pyridin-5-carbonsäure (102 mg, 330 µmol) wurde in THF (4.0 ml) vorgelegt und HBTU (163 mg, 430 µmol) und N,N-Diisopropylethylamin (170 µl, 990 µmol) wurden anschließend zugegeben. Nach 15 min Rühren bei Raumtemperatur wurde (3S)-3-Fluorpyrrolidinhydrochlorid (49.8 mg, 396 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 58.1 mg (46 % d. Th.) der Titelverbindung erhalten.
LC-MS (Methode 3): Rₜ = 0.95 min; MS (ESIpos): m/z = 380 [M+H]⁺
¹H-NMR (400 MHz, DMSO-d6) δ [ppm]: -0.149 (1.01), -0.008 (8.13), 0.008 (8.46), 0.146 (1.01), 1.722 (9.21), 1.732 (9.63), 1.861 (1.59), 1.898 (2.43), 1.907 (2.26), 1.974 (2.09), 2.009 (3.60), 2.018 (3.52), 2.045 (2.18), 2.064 (4.10), 2.082 (6.20), 2.098 (5.78), 2.135 (3.69), 2.217 (2.18), 2.237 (2.26), 2.268 (2.85), 2.327 (1.84), 2.366 (1.76), 2.561 (6.45), 2.581 (7.20), 2.624 (2.43), 2.670 (1.93), 2.690 (9.47), 2.710 (1.76), 3.274 (2.01), 3.348 (2.01), 3.363 (1.68), 3.372 (1.93), 3.398 (2.01), 3.407 (2.18), 3.460 (1.51), 3.469 (1.68), 3.496 (2.26), 3.504 (2.09), 3.633 (5.86), 3.642 (3.85), 3.658 (6.03), 3.669 (4.69), 3.685 (3.85), 3.729 (4.69), 3.751 (5.11), 3.776 (4.10), 3.797 (3.35), 3.828 (0.92), 3.865 (6.70), 4.685 (3.35), 4.695 (4.02), 4.701 (4.19), 4.710 (3.27), 4.741 (4.19), 4.750 (4.69), 4.756 (5.36), 4.765 (4.10), 4.947 (6.95), 4.987 (16.00), 4.990 (15.50), 5.034 (11.81), 5.043 (13.82), 5.073 (4.52), 5.083 (6.20), 5.258 (3.18), 5.382 (4.27), 5.389 (4.44), 5.515 (2.43), 5.944 (0.67), 7.374 (10.05), 7.386 (10.39), 7.395 (10.81), 7.406 (11.14), 7.923 (11.23), 7.926 (11.39), 7.943 (10.72), 7.946 (10.47), 8.468 (11.39), 8.479 (11.39).

### Beispiel 394

### (5S)-2-[(3-Chlorpyridin-2-yl)methyl]-5-[(3,3-difluorazetidin-1-yl)carbonyl]-5,6,7,8-tetrahydro[1,2,4]triazolo[4,3-a]pyridin-3(2H)-on

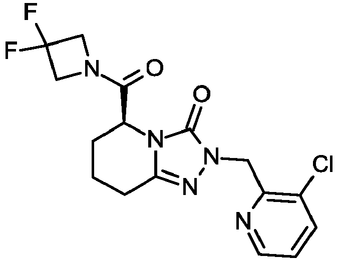

(5S)-2-[(3-Chlorpyridin-2-yl)methyl]-3-oxo-2,3,5,6,7,8-hexahydro[1,2,4]triazolo[4,3-a]pyridin-5-carbonsäure (102 mg, 330 µmol) wurde in THF (4.0 ml) vorgelegt und HBTU (163 mg, 430 µmol) und N,N-Diisopropylethylamin (170 µl, 990 µmol) wurden anschließend zugegeben. Nach 15 min Rühren bei Raumtemperatur wurde 3,3-Difluorazetidinhydrochlorid (51.4 mg, 396 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 75.6 mg (60 % d. Th.) der Titelverbindung erhalten.
LC-MS (Methode 3): Rₜ = 1.07 min; MS (ESIpos): m/z = 384 [M+H]⁺
¹H-NMR (400 MHz, DMSO-d6) δ [ppm]: -0.149 (0.48), -0.008 (4.34), 0.008 (4.02), 0.146 (0.48), 1.710 (3.05), 1.726 (4.50), 1.736 (4.27), 1.750 (3.05), 1.770 (1.19), 1.953 (1.29), 1.968 (1.83), 1.979 (2.44), 1.990 (2.25), 2.005 (1.09), 2.030 (1.25), 2.046 (1.83), 2.057 (1.93), 2.066 (2.25), 2.079 (1.83), 2.089 (1.25), 2.104 (0.87), 2.114 (0.58), 2.327 (0.80), 2.366 (0.71), 2.562 (4.56), 2.578 (4.02), 2.588 (6.20), 2.602 (2.96), 2.616 (1.09), 2.630 (1.67), 2.644 (0.74), 2.670 (0.93), 2.710 (0.80), 4.326 (1.73), 4.356 (2.92), 4.378 (2.92), 4.408 (1.80), 4.568 (4.21), 4.583 (6.97), 4.596 (4.18), 4.700 (0.64), 4.728 (1.83), 4.758 (2.22), 4.802 (1.12), 4.834 (2.12), 4.863 (1.80), 4.986 (2.92), 5.026 (16.00), 5.041 (16.00), 5.081 (2.92), 7.378 (5.59), 7.390 (5.78), 7.399 (6.07), 7.410 (6.23), 7.929 (6.71), 7.933 (7.07), 7.949 (6.43), 7.953 (6.43), 8.451 (6.52), 8.455 (6.81), 8.463 (6.68), 8.466 (6.52).

### Beispiel 395

### (5S)-2-[(3-Chlorpyridin-2-yl)methyl]-5-{[(3R,4S)-3,4-difluorpyrrolidin-1 -yl]carbonyl}-5,6,7,8-tetrahydro[1,2,4]triazolo[4,3-a]pyridin-3(2H)-on

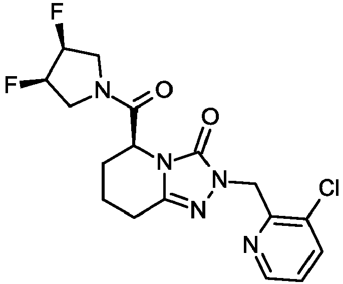

(5S)-2-[(3-Chlorpyridin-2-yl)methyl]-3-oxo-2,3,5,6,7,8-hexahydro[1,2,4]triazolo[4,3-a]pyridin-5-carbonsäure (102 mg, 330 µmol) wurde in THF (4.0 ml) vorgelegt und HBTU (163 mg, 430 µmol) und N,N-Diisopropylethylamin (170 µl, 990 µmol) wurden anschließend zugegeben. Nach 15 min Rühren bei Raumtemperatur wurde (3R,4S)-3,4-Difluorpyrrolidinhydrochlorid (56.9 mg, 396 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 61.2 mg (47 % d. Th.) der Titelverbindung erhalten.
LC-MS (Methode 3): Rₜ = 1.05 min; MS (ESIpos): m/z = 398 [M+H]⁺
¹H-NMR (400 MHz, DMSO-d6) δ [ppm]: -0.150 (1.35), -0.008 (11.87), 0.008 (10.29), 0.146 (1.35), 1.710 (8.41), 1.721 (7.59), 1.733 (5.11), 1.908 (1.73), 1.952 (3.46), 1.989 (3.61), 2.002 (3.53), 2.017 (2.48), 2.032 (3.08), 2.042 (3.15), 2.049 (3.23), 2.057 (3.38), 2.067 (3.53), 2.072 (7.14), 2.084 (2.63), 2.092 (2.48), 2.102 (1.73), 2.327 (1.65), 2.366 (1.73), 2.518 (9.84), 2.567 (7.66), 2.577 (10.22), 2.589 (5.78), 2.606 (2.10), 2.620 (3.23), 2.632 (1.73), 2.670 (2.10), 2.709 (1.95), 2.890 (0.75), 3.455 (1.95), 3.479 (2.03), 3.488 (3.83), 3.498 (2.70), 3.510 (3.38), 3.520 (3.46), 3.529 (2.93), 3.542 (3.00), 3.563 (1.73), 3.572 (2.40), 3.582 (1.50), 3.616 (2.55), 3.630 (2.85), 3.649 (1.80), 3.663 (2.10), 3.671 (2.78), 3.685 (4.58), 3.696 (3.46), 3.705 (3.08), 3.718 (4.43), 3.730 (2.78), 3.748 (2.85), 3.760 (3.00), 3.781 (2.63), 3.794 (1.65), 3.835 (1.13), 3.870 (2.40), 3.911 (1.73), 3.932 (2.33), 3.946 (2.70), 3.961 (1.65), 3.981 (2.63), 3.995 (2.63), 4.010 (1.65), 4.024 (1.65), 4.131 (1.58), 4.146 (1.80), 4.160 (1.80), 4.174 (3.15), 4.188 (2.18), 4.201 (1.80), 4.216 (1.65), 4.781 (7.96), 4.791 (9.31), 4.797 (10.37), 4.806 (7.51), 4.952 (6.84), 4.959 (4.88), 4.992 (16.00), 4.998 (15.47), 5.029 (14.50), 5.044 (15.02), 5.068 (4.51), 5.083 (6.38), 5.242 (1.80), 5.253 (2.48), 5.263 (2.63), 5.274 (2.93), 5.283 (2.40), 5.296 (1.88), 5.311 (1.73), 5.327 (2.18), 5.336 (2.18), 5.350 (2.48), 5.363 (2.33), 5.375 (2.78), 5.385 (3.23), 5.397 (2.55), 5.406 (2.78), 5.415 (2.85), 5.426 (2.10), 5.440 (1.80), 5.448 (2.10), 5.464 (2.10), 5.472 (2.10), 5.480 (2.18), 5.493 (1.88), 5.502 (1.20), 7.374 (10.44), 7.385 (10.97), 7.394 (11.42), 7.406 (11.72), 7.924 (13.00), 7.944 (12.24), 8.465 (12.85), 8.476 (12.85).

### Beispiel 396

### (5S)-2-[(3-Chlorpyridin-2-yl)methyl]-5-[(3-fluorazetidin-1-yl)carbonyl]-5,6,7,8-tetrahydro[1,2,4]triazolo[4,3-a]pyridin-3(2H)-on

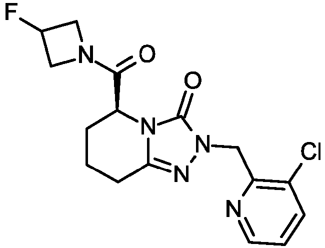

(5S)-2-[(3-Chlorpyridin-2-yl)methyl]-3-oxo-2,3,5,6,7,8-hexahydro[1,2,4]triazolo[4,3-a]pyridin-5-carbonsäure (102 mg, 330 µmol) wurde in THF (4.0 ml) vorgelegt und HBTU (163 mg, 430 µmol) und N,N-Diisopropylethylamin (170 µl, 990 µmol) wurden anschließend zugegeben. Nach 15 min Rühren bei Raumtemperatur wurde 3-Fluorazetidinhydrochlorid (44.2 mg, 396 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 44.7 mg (37 % d. Th.) der Titelverbindung erhalten.
LC-MS (Methode 3): Rₜ = 0.95 min; MS (ESIpos): m/z = 366 [M+H]⁺
¹H-NMR (400 MHz, DMSO-d6) δ [ppm]: -0.008 (1.57), 0.008 (1.11), 1.718 (5.92), 1.928 (1.66), 1.939 (2.29), 1.951 (2.40), 1.961 (2.25), 1.972 (1.69), 1.985 (1.20), 2.002 (1.67), 2.021 (2.41), 2.039 (2.28), 2.056 (1.75), 2.073 (16.00), 2.524 (3.36), 2.561 (4.73), 2.575 (6.55), 2.588 (3.16), 2.603 (1.16), 2.617 (1.86), 2.631 (0.77), 3.894 (0.84), 3.923 (1.64), 3.953 (1.64), 3.984 (1.63), 4.013 (0.91), 4.154 (0.71), 4.168 (0.80), 4.183 (0.68), 4.219 (1.26), 4.233 (1.28), 4.248 (1.10), 4.262 (1.00), 4.286 (1.40), 4.296 (1.38), 4.315 (1.43), 4.359 (1.19), 4.385 (1.41), 4.429 (0.76), 4.454 (0.96), 4.513 (2.81), 4.525 (5.12), 4.538 (4.60), 4.549 (2.31), 4.563 (0.89), 4.579 (0.85), 4.590 (0.66), 4.607 (0.64), 4.618 (0.74), 4.635 (0.82), 4.644 (0.71), 4.662 (0.76), 4.671 (0.81), 4.686 (0.85), 4.697 (0.74), 4.713 (0.61), 4.975 (3.42), 5.015 (15.16), 5.031 (6.48), 5.041 (5.56), 5.071 (1.15), 5.081 (1.50), 5.355 (1.02), 5.362 (0.91), 5.370 (0.85), 5.380 (0.89), 5.388 (0.96), 5.395 (1.04), 5.498 (1.07), 5.513 (0.91), 5.522 (0.87), 5.531 (0.96), 5.539 (1.02), 7.375 (5.78), 7.386 (5.99), 7.395 (6.11), 7.407 (6.15), 7.925 (6.95), 7.929 (6.74), 7.945 (6.47), 7.949 (6.04), 8.453 (3.20), 8.463 (5.44), 8.474 (2.85).

### Beispiel 397

### (5S)-2-[(3,5-Dichlorpyridin-2-yl)methyl]-5-[(3,3-difluorazetidin-1-yl)carbonyl]-5,6,7,8-tetrahydro[1,2,4]triazolo[4,3-a]pyridin-3(2H)-on

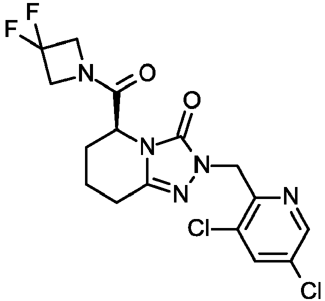

(5S)-2-[(3,5-Dichlorpyridin-2-yl)methyl]-3-oxo-2,3,5,6,7,8-hexahydro[1,2,4]triazolo[4,3-a]pyridin-5-carbonsäure (42.1 mg, 123 µmol) wurde in THF (1.0 ml) vorgelegt und HBTU (93.1 mg, 245 µmol) und N,N-Diisopropylethylamin (85 µl, 490 µmol) wurden anschließend zugegeben. Nach 15 min Rühren bei Raumtemperatur wurde 3,3-Difluorazetidinhydrochlorid (23.8 mg, 184 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 43.8 mg (85 % d. Th.) der Titelverbindung erhalten.
LC-MS (Methode 3): Rₜ = 1.32 min; MS (ESIpos): m/z = 418 [M+H]⁺
¹H-NMR (400 MHz, DMSO-d6) δ [ppm]: -0.149 (1.71), -0.008 (16.00), 0.008 (14.02), 0.146 (1.78), 1.720 (2.63), 1.732 (2.50), 1.976 (1.45), 1.990 (1.32), 2.059 (1.25), 2.073 (0.99), 2.327 (2.17), 2.366 (1.98), 2.523 (6.85), 2.569 (2.57), 2.582 (3.42), 2.597 (1.78), 2.625 (0.99), 2.669 (2.17), 2.710 (2.04), 4.356 (1.58), 4.375 (1.65), 4.569 (2.24), 4.584 (3.56), 4.596 (2.04), 4.718 (1.05), 4.747 (1.12), 4.833 (1.19), 4.863 (0.86), 4.980 (1.25), 5.020 (8.63), 5.031 (8.76), 5.071 (1.38), 8.256 (4.81), 8.261 (4.81), 8.552 (5.33), 8.557 (5.20).

### Beispiel 398

### (5S)-2-[(3,5-Dichlorpyridin-2-yl)methyl]-5-[(3-fluorazetidin-1-yl)carbonyl]-5,6,7,8-tetrahydro[1,2,4]triazolo[4,3-a]pyridin-3(2H)-on

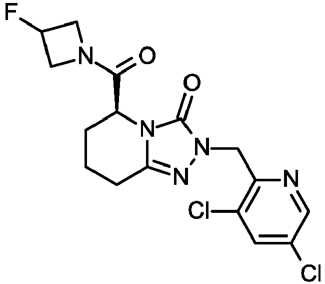

(5S)-2-[(3,5-Dichlorpyridin-2-yl)methyl]-3-oxo-2,3,5,6,7,8-hexahydro[1,2,4]triazolo[4,3-a]pyridin-5-carbonsäure (42.1 mg, 123 µmol) wurde in THF (1.0 ml) vorgelegt und HBTU (93.1 mg, 245 µmol) und N,N-Diisopropylethylamin (85 µl, 490 µmol) wurden anschließend zugegeben. Nach 15 min Rühren bei Raumtemperatur wurde 3-Fluorazetidinhydrochlorid (20.5 mg, 184 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 37.5 mg (76 % d. Th.) der Titelverbindung erhalten.
LC-MS (Methode 3): Rₜ = 1.20 min; MS (ESIpos): m/z = 400 [M+H]⁺
¹H-NMR (400 MHz, DMSO-d6) δ [ppm]: 1.713 (6.00), 1.937 (2.24), 1.948 (2.38), 1.957 (2.23), 1.997 (1.64), 2.016 (2.36), 2.034 (2.18), 2.052 (1.51), 2.069 (0.81), 2.328 (0.45), 2.366 (0.45), 2.558 (4.75), 2.572 (5.93), 2.586 (2.94), 2.601 (1.13), 2.614 (1.73), 2.628 (0.76), 2.670 (0.50), 2.710 (0.47), 3.892 (0.81), 3.920 (1.61), 3.950 (1.58), 3.982 (1.66), 4.009 (0.93), 4.152 (0.66), 4.167 (0.78), 4.181 (0.66), 4.224 (1.23), 4.279 (1.56), 4.308 (1.43), 4.338 (0.95), 4.362 (1.56), 4.385 (1.03), 4.423 (0.73), 4.449 (0.98), 4.527 (4.92), 4.571 (0.86), 4.597 (0.58), 4.617 (0.68), 4.633 (0.80), 4.669 (0.76), 4.686 (0.76), 4.710 (0.60), 4.971 (2.86), 5.011 (16.00), 5.021 (6.85), 5.031 (5.96), 5.061 (0.91), 5.071 (1.28), 5.355 (0.98), 5.396 (1.03), 5.498 (0.98), 5.539 (1.00), 8.253 (7.23), 8.258 (7.31), 8.553 (3.19), 8.564 (3.14).

### Beispiel 399

### (5S)-2-[(3,5-Dichlorpyridin-2-yl)methyl]-5-[(3,3-difluorpyrrolidin-1-yl)carbonyl]-5,6,7,8-tetrahydro[1,2,4]triazolo[4,3-a]pyridin-3(2H)-on

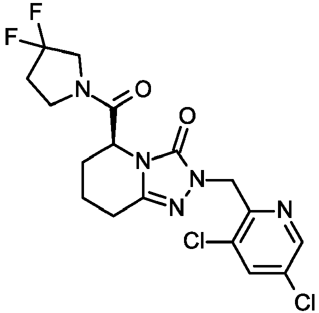

(5S)-2-[(3,5-Dichlorpyridin-2-yl)methyl]-3-oxo-2,3,5,6,7,8-hexahydro[1,2,4]triazolo[4,3-a]pyridin-5-carbonsäure (42.1 mg, 123 µmol) wurde in THF (1.0 ml) vorgelegt und HBTU (93.1 mg, 245 µmol) und N,N-Diisopropylethylamin (85 µl, 490 µmol) wurden anschließend zugegeben. Nach 15 min Rühren bei Raumtemperatur wurde 3,3-Difluorpyrrolidinhydrochlorid (26.4 mg, 184 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 34.3 mg (65 % d. Th.) der Titelverbindung erhalten.
LC-MS (Methode 4): Rₜ = 0.73 min; MS (ESIpos): m/z = 432 [M+H]⁺
¹H-NMR (400 MHz, DMSO-d6) δ [ppm]: -0.149 (1.92), -0.008 (16.00), 0.008 (15.60), 0.146 (1.84), 1.704 (4.24), 1.982 (2.24), 2.008 (2.40), 2.046 (1.84), 2.058 (1.92), 2.327 (3.12), 2.366 (2.88), 2.427 (2.16), 2.523 (10.96), 2.562 (5.68), 2.578 (5.36), 2.605 (1.52), 2.620 (1.60), 2.669 (2.96), 2.709 (2.40), 3.530 (2.88), 3.549 (4.64), 3.568 (2.08), 3.667 (2.00), 3.700 (2.32), 3.733 (1.84), 3.764 (3.12), 3.797 (2.40), 3.809 (2.16), 3.829 (1.36), 3.890 (1.04), 3.909 (2.16), 3.934 (1.52), 3.993 (1.36), 4.035 (1.28), 4.064 (0.80), 4.140 (0.88), 4.171 (1.36), 4.196 (1.28), 4.740 (1.84), 4.755 (2.56), 4.764 (1.76), 4.818 (1.84), 4.834 (2.56), 4.844 (1.92), 4.955 (4.56), 4.995 (13.92), 5.027 (15.92), 5.067 (5.28), 8.250 (10.96), 8.255 (11.12), 8.563 (11.76), 8.568 (11.36).

### Beispiel 400

### (5S)-2-[(3,5-Dichlorpyridin-2-yl)methyl]-5-{[(3R,4S)-3,4-difluorpyrrolidin-1-yl]carbonyl}-5,6,7,8-tetrahydro[1,2,4]triazolo[4,3-a]pyridin-3(2H)-on

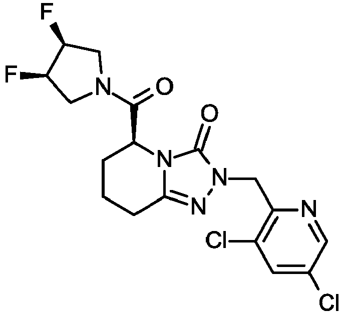

(5S)-2-[(3,5-Dichlorpyridin-2-yl)methyl]-3-oxo-2,3,5,6,7,8-hexahydro[1,2,4]triazolo[4,3-a]pyridin-5-carbonsäure (42.1 mg, 123 µmol) wurde in THF (1.0 ml) vorgelegt und HBTU (93.1 mg, 245 µmol) und N,N-Diisopropylethylamin (85 µl, 490 µmol) wurden anschließend zugegeben. Nach 15 min Rühren bei Raumtemperatur wurde (3R,4S)-3,4-Difluorpyrrolidinhydrochlorid (26.4 mg, 184 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 46.2 mg (87 % d. Th.) der Titelverbindung erhalten.
LC-MS (Methode 4): Rₜ = 0.70 min; MS (ESIpos): m/z = 432 [M+H]⁺
¹H-NMR (400 MHz, DMSO-d6) δ [ppm]: -0.149 (1.86), -0.008 (16.00), 0.008 (12.91), 0.146 (1.78), 1.695 (4.33), 1.706 (4.95), 1.718 (4.71), 1.950 (2.24), 1.989 (2.47), 2.000 (2.63), 2.027 (2.09), 2.045 (2.24), 2.052 (2.24), 2.062 (2.16), 2.073 (10.59), 2.328 (2.32), 2.366 (2.24), 2.523 (8.66), 2.564 (5.49), 2.576 (6.72), 2.589 (3.79), 2.607 (1.55), 2.620 (2.24), 2.670 (2.78), 2.710 (2.63), 3.452 (1.31), 3.486 (2.24), 3.507 (2.16), 3.516 (2.16), 3.526 (1.78), 3.541 (1.93), 3.560 (1.24), 3.568 (1.55), 3.613 (1.70), 3.627 (1.86), 3.645 (1.16), 3.660 (1.31), 3.668 (1.86), 3.681 (2.78), 3.691 (2.32), 3.701 (2.01), 3.714 (2.86), 3.726 (2.09), 3.744 (2.01), 3.756 (2.01), 3.776 (1.78), 3.789 (1.08), 3.865 (1.55), 3.902 (1.16), 3.927 (1.47), 3.940 (1.78), 3.956 (1.08), 3.976 (1.70), 3.990 (1.70), 4.005 (1.08), 4.019 (1.08), 4.125 (1.08), 4.139 (1.16), 4.153 (1.08), 4.168 (2.24), 4.182 (1.39), 4.195 (1.16), 4.210 (1.08), 4.778 (5.18), 4.787 (6.34), 4.793 (6.96), 4.802 (5.10), 4.952 (4.17), 4.958 (2.94), 4.992 (11.52), 4.997 (11.21), 5.018 (10.36), 5.033 (10.67), 5.058 (2.55), 5.073 (4.17), 5.152 (0.46), 5.252 (1.78), 5.273 (1.93), 5.295 (1.16), 5.326 (1.55), 5.335 (1.47), 5.349 (1.47), 5.362 (1.55), 5.372 (1.86), 5.385 (2.09), 5.405 (1.78), 5.425 (1.24), 5.479 (1.39), 5.492 (1.24), 7.368 (0.54), 8.251 (8.58), 8.255 (8.50), 8.561 (6.65), 8.564 (8.97), 8.570 (7.42).

### Beispiel 401

### (5S)-2-{[3-Chlor-5-(trifluormethyl)pyridin-2-yl]methyl}-5-{[(3R)-3-fluorpyrrolidin-1-yl]carbonyl}-5,6,7,8-tetrahydro[1,2,4]triazolo[4,3-a]pyridin-3(2H)-on

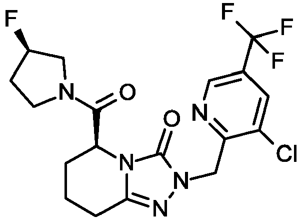

(5S)-2-{[3-Chlor-5-(trifluormethyl)pyridin-2-yl]methyl}-3-oxo-2,3,5,6,7,8-hexahydro[1,2,4]triazolo[4,3-a]pyridin-5-carbonsäure (45.0 mg, 119 µmol) wurde in THF (2.0 ml) bei Raumtemperatur vorgelegt. Anschließend wurden HATU (59.0 mg, 155 µmol) und Triethylamin (50 µl, 360 µmol) zugegeben. Nach 15 min Rühren wurde (3R)-3-Fluorpyrrolidinhydrochlorid (18.0 mg, 143 µmol) zugegeben und das Reaktionsgemisch für 72 Stunden bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 27.0 mg (50 % d. Th.) der Titelverbindung erhalten.
LC-MS (Methode 3): Rₜ = 1.35 min; MS (ESIpos): m/z = 448 [M+H]⁺
¹H-NMR (400 MHz, DMSO-d6) δ [ppm]: -0.149 (1.64), -0.008 (16.00), 0.008 (12.00), 0.146 (1.33), 1.726 (4.85), 1.990 (5.82), 2.098 (3.27), 2.256 (2.42), 2.327 (3.76), 2.366 (2.30), 2.572 (5.21), 2.585 (7.27), 2.599 (4.36), 2.627 (2.73), 2.669 (4.18), 2.709 (1.88), 3.352 (2.73), 3.370 (2.73), 3.504 (2.36), 3.529 (5.39), 3.554 (4.91), 3.596 (6.36), 3.657 (1.64), 3.682 (1.76), 3.776 (1.58), 3.947 (4.12), 4.009 (1.88), 4.040 (1.33), 4.823 (3.52), 4.861 (3.58), 5.073 (4.06), 5.113 (16.00), 5.133 (9.58), 5.143 (8.67), 5.183 (2.67), 5.268 (2.24), 5.349 (2.18), 5.401 (2.36), 5.482 (2.18), 8.487 (14.30), 8.905 (13.88).

### Beispiel 402

### (5S)-2-{[3-Chlor-5-(trifluormethyl)pyridin-2-yl]methyl}-5-[(3-fluorazetidin-1-yl)carbonyl]-5,6,7,8-tetrahydro[1,2,4]triazolo[4,3-a]pyridin-3(2H)-on

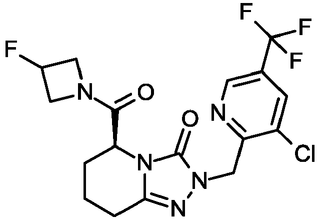

(5S)-2-{[3-Chlor-5-(trifluormethyl)pyridin-2-yl]methyl}-3-oxo-2,3,5,6,7,8-hexahydro[1,2,4]triazolo[4,3-a]pyridin-5-carbonsäure (45.0 mg, 119 µmol) wurde in THF (2.0 ml) bei Raumtemperatur vorgelegt. Anschließend wurden HATU (59.0 mg, 155 µmol) und Triethylamin (50 µl, 360 µmol) zugegeben. Nach 15 min Rühren wurde 3-Fluorazetidinhydrochlorid (16.0 mg, 143 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 14.0 mg (27 % d. Th.) der Titelverbindung erhalten.
LC-MS (Methode 4): Rₜ = 0.72 min; MS (ESIpos): m/z = 434 [M+H]⁺
¹H-NMR (400 MHz, DMSO-d6) δ [ppm]: -0.149 (1.02), -0.008 (10.73), 0.008 (8.00), 0.146 (1.02), 0.976 (1.48), 0.994 (2.62), 1.719 (3.94), 1.804 (1.71), 1.814 (1.93), 1.822 (4.47), 1.830 (1.90), 1.839 (1.67), 1.906 (16.00), 1.945 (1.55), 1.956 (1.67), 2.023 (1.59), 2.040 (1.40), 2.060 (0.99), 2.327 (1.63), 2.366 (1.55), 2.518 (6.60), 2.523 (6.07), 2.568 (3.72), 2.582 (5.23), 2.595 (3.00), 2.624 (1.59), 2.665 (1.29), 2.670 (1.71), 2.674 (1.21), 2.689 (0.87), 2.710 (1.36), 2.731 (1.18), 2.866 (0.87), 2.890 (1.67), 3.056 (1.78), 3.073 (3.98), 3.091 (1.59), 3.893 (0.53), 3.920 (1.06), 3.950 (1.02), 3.984 (1.06), 4.011 (0.57), 4.167 (0.49), 4.226 (0.87), 4.281 (0.99), 4.312 (0.95), 4.369 (0.99), 4.424 (0.45), 4.451 (0.61), 4.534 (3.22), 4.548 (3.03), 4.575 (0.57), 4.633 (0.53), 4.671 (0.53), 4.687 (0.53), 5.089 (1.10), 5.130 (11.11), 5.183 (0.61), 5.356 (0.68), 5.539 (0.68), 6.273 (1.25), 6.510 (1.44), 7.276 (0.53), 8.491 (5.80), 8.494 (5.76), 8.884 (2.62), 8.901 (2.46).

### Beispiel 403

### (5RS)-2-{[3-Chlor-5-(trifluormethyl)pyridin-2-yl]methyl}-5-[(3-hydroxyazetidin-1-yl)carbonyl]-5,6,7,8-tetrahydro[1,2,4]triazolo[4,3-a]pyridin-3(2H)-on (Racemat)

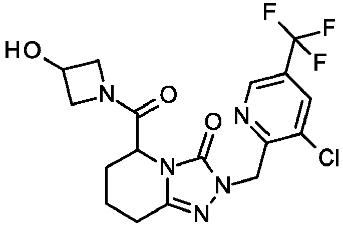

(5RS)-2-{[3-Chlor-5-(trifluormethyl)pyridin-2-yl]methyl}-3-oxo-2,3,5,6,7,8-hexahydro[1,2,4]triazolo[4,3-a]pyridin-5-carbonsäure (Racemat) (45.0 mg, 119 µmol) wurde in THF (2.0 ml) bei Raumtemperatur vorgelegt. Anschließend wurden HATU (59.0 mg, 155 µmol) und Triethylamin (50 µl, 360 µmol) zugegeben. Nach 15 min Rühren wurde Azetidin-3-olhydrochlorid (15.7 mg, 143 µmol) zugegeben und das Reaktionsgemisch für 72 Stunden bei Raumtemperatur gerührt. Es wurde erneut HATU (59.0 mg, 155 µmol) und Triethylamin (50 µl, 360 µmol) zugegeben und 3 Stunden bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und der Rückstand wurde über präparative HPLC aufgereinigt (Methode 10). Die produkthaltigen Fraktionen wurden im Vakuum eingeengt und es wurden 800 µg (2 % d. Th.) der Titelverbindung erhalten.
LC-MS (Methode 3): Rₜ = 1.12 min; MS (ESIpos): m/z = 432 [M+H]⁺
¹H-NMR (400 MHz, DMSO-d6) δ [ppm]: -0.150 (1.80), -0.009 (16.00), 0.007 (12.68), 0.146 (1.63), 1.723 (1.16), 1.937 (0.93), 2.327 (3.61), 2.365 (2.04), 2.522 (10.53), 2.669 (3.32), 2.709 (2.04), 3.597 (0.76), 3.962 (0.70), 4.022 (0.87), 4.365 (0.64), 4.505 (1.75), 5.081 (0.70), 5.121 (2.44), 5.802 (1.57), 8.487 (2.21), 8.896 (2.44).

### Beispiel 404

### (5S)-2-{[3-Chlor-5-(trifluormethyl)pyridin-2-yl]methyl}-5-[(3,3-difluorpyrrolidin-1-yl)carbonyl]-5,6,7,8-tetrahydro[1,2,4]triazolo[4,3-a]pyridin-3(2H)-on

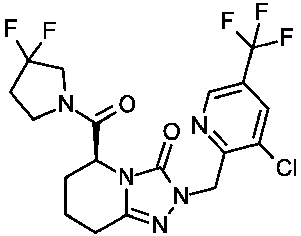

(5S)-2-{[3-Chlor-5-(trifluormethyl)pyridin-2-yl]methyl}-3-oxo-2,3,5,6,7,8-hexahydro[1,2,4]triazolo[4,3-a]pyridin-5-carbonsäure (35.0 mg, 92.9 µmol) wurde in THF (1.0 ml) vorgelegt und HBTU (45.8 mg, 121 µmol) und N,N-Diisopropylethylamin (49 µl, 280 µmol) wurden anschließend zugegeben. Nach 15 min Rühren bei Raumtemperatur wurde 3,3-Difluorpyrrolidinhydrochlorid (16.0 mg, 111 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 33.8 mg (78 % d. Th.) der Titelverbindung erhalten.
LC-MS (Methode 3): Rₜ = 1.50 min; MS (ESIpos): m/z = 466 [M+H]⁺
¹H-NMR (400 MHz, DMSO-d6) δ [ppm]: -0.149 (0.47), -0.008 (4.42), 0.008 (2.91), 1.252 (14.54), 1.268 (16.00), 1.276 (11.71), 1.293 (10.07), 1.686 (2.47), 1.699 (3.85), 1.711 (5.07), 1.724 (4.66), 1.932 (0.91), 1.967 (1.93), 1.982 (2.71), 1.991 (2.60), 2.003 (2.78), 2.016 (2.65), 2.029 (1.98), 2.040 (1.95), 2.055 (2.11), 2.066 (2.11), 2.076 (1.61), 2.091 (1.40), 2.101 (0.83), 2.328 (1.12), 2.366 (1.53), 2.379 (1.77), 2.408 (2.13), 2.429 (2.08), 2.453 (1.85), 2.471 (1.93), 2.524 (6.48), 2.571 (6.61), 2.587 (7.49), 2.600 (3.56), 2.617 (1.20), 2.629 (1.87), 2.642 (0.83), 2.670 (1.14), 2.710 (1.14), 2.895 (9.55), 3.115 (1.48), 3.126 (1.56), 3.133 (1.61), 3.144 (1.53), 3.425 (1.72), 3.444 (1.93), 3.463 (1.20), 3.532 (4.14), 3.551 (6.56), 3.571 (3.43), 3.587 (1.17), 3.604 (1.53), 3.621 (1.82), 3.636 (1.53), 3.649 (1.51), 3.670 (2.63), 3.704 (2.86), 3.736 (2.13), 3.767 (3.30), 3.789 (2.21), 3.798 (3.59), 3.815 (2.89), 3.833 (1.90), 3.870 (0.49), 3.892 (1.33), 3.910 (2.73), 3.930 (1.51), 3.936 (1.95), 3.955 (0.86), 3.970 (0.83), 3.998 (1.74), 4.012 (0.91), 4.028 (1.20), 4.040 (1.64), 4.069 (0.96), 4.142 (1.04), 4.174 (1.61), 4.201 (1.56), 4.231 (0.65), 4.750 (2.19), 4.761 (2.71), 4.766 (3.12), 4.776 (2.26), 4.830 (2.24), 4.840 (2.71), 4.845 (3.10), 4.855 (2.21), 5.076 (3.28), 5.116 (13.61), 5.139 (14.33), 5.166 (0.68), 5.180 (3.51), 8.492 (9.52), 8.902 (9.42), 8.987 (0.62).

### Beispiel 405

### (5S)-2-{[3-Chlor-5-(trifluormethyl)pyridin-2-yl]methyl}-5-{[(3S)-3-fluorpyrrolidin-1-yl]carbonyl}-5,6,7,8-tetrahydro[1,2,4]triazolo[4,3-a]pyridin-3(2H)-on

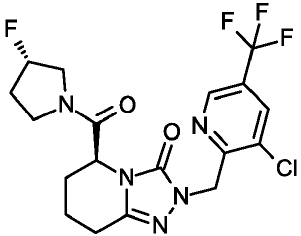

(5S)-2-{[3-Chlor-5-(trifluormethyl)pyridin-2-yl]methyl}-3-oxo-2,3,5,6,7,8-hexahydro[1,2,4]triazolo[4,3-a]pyridin-5-carbonsäure (35.0 mg, 92.9 µmol) wurde in THF (1.0 ml) vorgelegt und HBTU (45.8 mg, 121 µmol) und N,N-Diisopropylethylamin (49 µl, 280 µmol) wurden anschließend zugegeben. Nach 15 min Rühren bei Raumtemperatur wurde (3S)-3-Fluorpyrrolidinhydrochlorid (14.0 mg, 111 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 25.0 mg (61 % d. Th.) der Titelverbindung erhalten.
LC-MS (Methode 3): Rₜ = 1.36 min; MS (ESIpos): m/z = 448 [M+H]⁺
¹H-NMR (400 MHz, DMSO-d6) δ [ppm]: -0.149 (1.09), -0.008 (13.31), 0.008 (8.11), 0.146 (1.09), 1.725 (8.34), 1.734 (8.63), 1.868 (1.37), 1.904 (2.11), 1.913 (1.89), 1.990 (2.91), 2.016 (3.49), 2.069 (4.23), 2.084 (5.66), 2.102 (5.26), 2.120 (3.66), 2.136 (3.37), 2.217 (2.06), 2.265 (2.51), 2.327 (2.46), 2.523 (15.49), 2.566 (4.86), 2.586 (6.51), 2.632 (2.34), 2.669 (2.63), 2.710 (2.11), 3.348 (1.77), 3.371 (1.83), 3.398 (1.94), 3.406 (1.83), 3.460 (1.37), 3.468 (1.43), 3.495 (2.06), 3.504 (2.00), 3.628 (5.26), 3.653 (5.14), 3.680 (3.37), 3.689 (2.69), 3.729 (4.00), 3.749 (4.23), 3.776 (3.37), 3.795 (2.69), 3.828 (0.91), 3.861 (5.43), 4.690 (2.80), 4.699 (3.31), 4.705 (3.43), 4.715 (2.69), 4.747 (3.66), 4.755 (4.06), 4.762 (4.51), 4.771 (3.26), 5.068 (4.69), 5.109 (16.00), 5.133 (9.49), 5.143 (10.46), 5.174 (2.46), 5.184 (3.43), 5.258 (2.74), 5.388 (3.66), 5.511 (2.06), 5.944 (0.86), 8.491 (12.97), 8.904 (13.03).

### Beispiel 406

### (5S)-2-[(5-Chlor-3-fluorpyridin-2-yl)methyl]-5-[(3,3-difluorpyrrolidin-1-yl)carbonyl]-5,6,7,8-tetrahydro[1,2,4]triazolo[4,3-a]pyridin-3(2H)-on

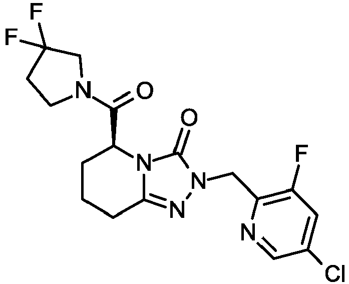

(5S)-2-[(5-Chlor-3-fluorpyridin-2-yl)methyl]-3-oxo-2,3,5,6,7,8-hexahydro[1,2,4]triazolo[4,3-a]pyridin-5-carbonsäure (79.3 mg, 243 µmol) wurde in THF (2.0 ml) vorgelegt und HBTU (120 mg, 316 µmol) und N,N-Diisopropylethylamin (130 µl, 730 µmol) wurden anschließend zugegeben. Nach 15 min Rühren bei Raumtemperatur wurde 3,3-Difluorpyrrolidinhydrochlorid (41.8 mg, 291 µmol) zugegeben und das Reaktionsgemisch über Nacht bei Raumtemperatur gerührt. Es wurde erneut HBTU (120 mg, 316 µmol), 3,3-Difluorpyrrolidinhydrochlorid (41.8 mg, 291 µmol) und N,N-Diisopropylethylamin (130 µl, 730 µmol) zugegeben und über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 71.0 mg (70 % d. Th.) der Titelverbindung erhalten.
LC-MS (Methode 3): Rₜ = 1.32 min; MS (ESIpos): m/z = 416 [M+H]⁺
¹H-NMR (400 MHz, DMSO-d6) δ [ppm]: -0.150 (1.61), -0.008 (16.00), 0.008 (12.15), 0.146 (1.57), 1.668 (2.08), 1.693 (4.13), 1.910 (1.34), 1.958 (2.12), 1.970 (2.24), 1.980 (2.75), 1.996 (2.00), 2.012 (1.57), 2.023 (1.77), 2.038 (1.81), 2.048 (1.89), 2.073 (7.90), 2.327 (1.69), 2.366 (2.04), 2.378 (1.61), 2.407 (1.97), 2.429 (1.89), 2.449 (1.77), 2.523 (7.23), 2.568 (6.96), 2.583 (3.50), 2.610 (1.61), 2.669 (1.69), 2.709 (1.61), 3.528 (2.59), 3.548 (3.97), 3.563 (1.97), 3.630 (0.51), 3.665 (1.93), 3.698 (2.20), 3.733 (1.93), 3.765 (2.79), 3.779 (1.69), 3.788 (1.57), 3.798 (2.95), 3.805 (2.44), 3.824 (1.14), 3.888 (0.98), 3.906 (2.32), 3.931 (1.53), 3.952 (0.67), 3.962 (0.67), 3.990 (1.42), 4.020 (0.94), 4.033 (1.30), 4.061 (0.86), 4.137 (0.86), 4.168 (1.26), 4.194 (1.30), 4.227 (0.59), 4.724 (1.89), 4.740 (2.52), 4.750 (1.89), 4.804 (1.97), 4.814 (2.36), 4.820 (2.56), 4.829 (1.85), 4.901 (2.91), 4.939 (7.55), 4.984 (7.90), 4.988 (7.98), 5.023 (3.07), 5.027 (3.07), 8.089 (5.07), 8.094 (5.27), 8.113 (5.31), 8.118 (5.35), 8.476 (6.64), 8.480 (6.41).

### Beispiel 407

### (5S)-2-[(5-Chlor-3-fluorpyridin-2-yl)methyl]-5-[(3-fluorazetidin-1-yl)carbonyl]-5,6,7,8-tetrahydro[1,2,4]triazolo[4,3-a]pyridin-3(2H)-on

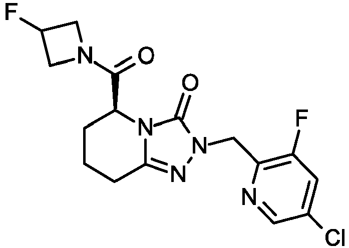

(5S)-2-[(5-Chlor-3-fluorpyridin-2-yl)methyl]-3-oxo-2,3,5,6,7,8-hexahydro[1,2,4]triazolo[4,3-a]pyridin-5-carbonsäure (79.3 mg, 243 µmol) wurde in THF (2.0 ml) vorgelegt und HBTU (120 mg, 316 µmol) und N,N-Diisopropylethylamin (130 µl, 730 µmol) wurden anschließend zugegeben. Nach 15 min Rühren bei Raumtemperatur wurde 3-Fluorazetidinhydrochlorid (32.5 mg, 291 µmol) zugegeben und das Reaktionsgemisch über Nacht bei Raumtemperatur gerührt. Es wurde erneut HBTU (120 mg, 316 µmol), 3-Fluorazetidinhydrochlorid (32.5 mg, 291 µmol) und N,N-Diisopropylethylamin (130 µl, 730 µmol) zugegeben und über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 64.1 mg (69 % d. Th.) der Titelverbindung erhalten.
LC-MS (Methode 3): Rₜ = 1.13 min; MS (ESIpos): m/z = 384 [M+H]⁺
¹H-NMR (400 MHz, DMSO-d6) δ [ppm]: -0.149 (0.90), -0.008 (8.03), 0.008 (7.55), 0.146 (0.90), 1.406 (0.80), 1.702 (10.07), 1.911 (2.84), 1.924 (3.75), 1.935 (3.96), 1.945 (3.69), 1.968 (1.91), 1.986 (2.79), 2.005 (3.99), 2.021 (3.59), 2.040 (2.58), 2.059 (1.44), 2.073 (6.46), 2.327 (0.98), 2.366 (0.90), 2.561 (10.79), 2.575 (5.21), 2.590 (1.91), 2.603 (3.19), 2.617 (1.38), 2.670 (1.12), 2.690 (0.98), 2.710 (1.01), 3.892 (1.36), 3.919 (2.74), 3.951 (2.71), 3.982 (2.71), 4.009 (1.59), 4.150 (1.17), 4.165 (1.28), 4.180 (1.06), 4.216 (2.10), 4.229 (2.15), 4.244 (1.91), 4.265 (2.71), 4.295 (2.63), 4.311 (1.38), 4.327 (1.59), 4.355 (2.50), 4.387 (1.67), 4.421 (1.25), 4.446 (1.65), 4.517 (8.93), 4.550 (1.49), 4.566 (1.46), 4.594 (0.93), 4.617 (1.14), 4.634 (1.33), 4.670 (1.25), 4.687 (1.36), 4.697 (1.22), 4.711 (1.01), 4.915 (4.60), 4.919 (4.62), 4.954 (16.00), 4.958 (15.95), 4.981 (8.43), 4.987 (8.05), 5.026 (2.39), 5.353 (1.67), 5.398 (1.70), 5.496 (1.65), 5.541 (1.67), 8.091 (8.53), 8.095 (8.96), 8.115 (8.77), 8.119 (9.09), 8.469 (5.93), 8.478 (5.95).

### Beispiel 408

### (5S)-2-[(5-Chlor-3-fluorpyridin-2-yl)methyl]-5-{[(3R,4S)-3,4-difluorpyrrolidin-1-yl]carbonyl}-5,6,7,8-tetrahydro[1,2,4]triazolo[4,3-a]pyridin-3(2H)-on

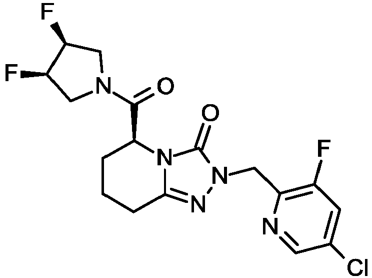

(5S)-2-[(5-Chlor-3-fluorpyridin-2-yl)methyl]-3-oxo-2,3,5,6,7,8-hexahydro[1,2,4]triazolo[4,3-a]pyridin-5-carbonsäure (79.3 mg, 243 µmol) wurde in THF (2.0 ml) vorgelegt und HBTU (120 mg, 316 µmol) und N,N-Diisopropylethylamin (130 µl, 730 µmol) wurden anschließend zugegeben. Nach 15 min Rühren bei Raumtemperatur wurde (3R,4S)-3,4-Difluorpyrrolidinhydrochlorid (41.8 mg, 291 µmol) zugegeben und das Reaktionsgemisch über Nacht bei Raumtemperatur gerührt. Es wurde erneut HBTU (120 mg, 316 µmol), (3R,4S)-3,4-Difluorpyrrolidinhydrochlorid (41.8 mg, 291 µmol) und N,N-Diisopropylethylamin (130 µl, 730 µmol) zugegeben und über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 61.7 mg (61 % d. Th.) der Titelverbindung erhalten.
LC-MS (Methode 3): Rₜ = 1.25 min; MS (ESIpos): m/z = 416 [M+H]⁺
¹H-NMR (400 MHz, DMSO-d6) δ [ppm]: -0.150 (0.54), -0.008 (5.15), 0.008 (5.00), 0.146 (0.58), 1.405 (0.52), 1.650 (1.84), 1.660 (2.36), 1.675 (3.11), 1.685 (4.35), 1.697 (5.00), 1.708 (4.91), 1.911 (1.80), 1.922 (1.29), 1.939 (2.40), 1.966 (1.56), 1.979 (2.94), 1.990 (2.55), 2.003 (1.82), 2.019 (2.17), 2.028 (2.27), 2.036 (2.38), 2.044 (2.38), 2.053 (2.21), 2.063 (1.95), 2.073 (16.00), 2.088 (1.14), 2.327 (0.60), 2.366 (0.67), 2.465 (0.99), 2.567 (6.16), 2.580 (3.43), 2.598 (1.22), 2.610 (1.93), 2.623 (0.97), 2.670 (0.73), 2.690 (0.88), 2.710 (0.71), 2.885 (0.86), 3.440 (0.66), 3.450 (1.18), 3.459 (0.79), 3.475 (1.42), 3.484 (2.55), 3.494 (1.95), 3.506 (1.99), 3.516 (2.06), 3.528 (2.14), 3.538 (2.38), 3.547 (1.44), 3.560 (1.07), 3.570 (1.56), 3.580 (0.97), 3.611 (1.61), 3.625 (1.84), 3.644 (1.18), 3.658 (1.37), 3.666 (1.87), 3.681 (3.58), 3.695 (2.36), 3.714 (2.90), 3.727 (2.66), 3.744 (2.14), 3.757 (2.21), 3.778 (1.70), 3.791 (1.11), 3.827 (0.77), 3.843 (1.05), 3.862 (1.63), 3.903 (1.14), 3.925 (1.54), 3.938 (1.78), 3.953 (1.11), 3.973 (1.74), 3.987 (1.72), 4.002 (1.12), 4.016 (1.01), 4.124 (1.09), 4.139 (1.24), 4.152 (1.22), 4.166 (2.15), 4.180 (1.39), 4.194 (1.24), 4.209 (1.09), 4.764 (5.23), 4.778 (6.95), 4.788 (4.87), 4.902 (4.40), 4.941 (11.19), 4.977 (5.60), 4.981 (5.96), 4.989 (6.24), 4.993 (6.07), 5.015 (1.99), 5.020 (2.08), 5.028 (2.66), 5.032 (2.59), 5.242 (1.22), 5.251 (1.87), 5.263 (1.82), 5.273 (1.93), 5.294 (1.20), 5.308 (1.11), 5.316 (1.20), 5.328 (1.50), 5.336 (1.50), 5.348 (1.61), 5.362 (1.52), 5.374 (2.12), 5.385 (2.19), 5.396 (1.82), 5.405 (1.67), 5.410 (1.65), 5.439 (1.09), 5.446 (1.20), 5.457 (1.56), 5.470 (1.50), 5.479 (1.56), 5.492 (1.18), 5.502 (0.75), 8.090 (6.39), 8.114 (6.56), 8.478 (9.27).

### Beispiel 409

### (5S)-2-[(5-Chlor-3-fluorpyridin-2-yl)methyl]-5-[(3,3-difluorazetidin-1-yl)carbonyl]-5,6,7,8-tetrahydro[1,2,4]triazolo[4,3-a]pyridin-3(2H)-on

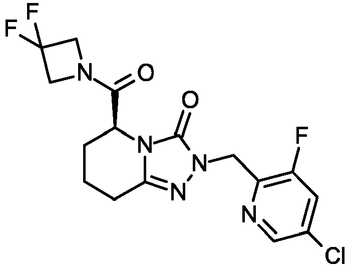

(5S)-2-[(5-Chlor-3-fluorpyridin-2-yl)methyl]-3-oxo-2,3,5,6,7,8-hexahydro[1,2,4]triazolo[4,3-a]pyridin-5-carbonsäure (79.3 mg, 243 µmol) wurde in THF (2.0 ml) vorgelegt und HBTU (120 mg, 316 µmol) und N,N-Diisopropylethylamin (130 µl, 730 µmol) wurden anschließend zugegeben. Nach 15 min Rühren bei Raumtemperatur wurde 3,3-Difluorazetidinhydrochlorid (37.7 mg, 291 µmol) zugegeben und das Reaktionsgemisch über Nacht bei Raumtemperatur gerührt. Es wurde erneut HBTU (120 mg, 316 µmol), 3,3-Difluorazetidinhydrochlorid (37.7 mg, 291 µmol) und N,N-Diisopropylethylamin (130 µl, 730 µmol) zugegeben und über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 78.3 mg (80 % d. Th.) der Titelverbindung erhalten
LC-MS (Methode 3): Rₜ = 1.19 min; MS (ESIpos): m/z = 402 [M+H]⁺
¹H-NMR (400 MHz, DMSO-d6) δ [ppm]: -0.149 (1.72), -0.008 (16.00), 0.008 (14.28), 0.146 (1.61), 1.405 (0.77), 1.695 (5.71), 1.708 (8.45), 1.722 (7.26), 1.952 (2.97), 1.964 (4.40), 1.976 (4.04), 2.014 (1.96), 2.029 (3.27), 2.049 (4.10), 2.063 (3.03), 2.073 (6.48), 2.327 (2.14), 2.366 (1.96), 2.557 (7.26), 2.571 (9.99), 2.586 (4.88), 2.599 (1.78), 2.613 (2.68), 2.669 (2.44), 2.709 (2.02), 2.891 (0.48), 4.324 (2.97), 4.355 (4.82), 4.376 (4.76), 4.407 (2.86), 4.557 (6.72), 4.572 (10.83), 4.585 (6.42), 4.713 (3.03), 4.741 (3.45), 4.772 (1.61), 4.805 (1.61), 4.833 (3.39), 4.861 (2.91), 4.926 (3.51), 4.931 (3.39), 4.965 (14.16), 4.969 (13.86), 4.987 (14.22), 4.991 (14.28), 5.026 (3.33), 5.030 (3.39), 8.094 (9.16), 8.099 (9.52), 8.118 (9.40), 8.123 (9.64), 8.469 (12.61), 8.473 (12.97).

### Beispiel 410

### (5S)-2-[(3,5-Difluorpyridin-2-yl)methyl]-5-[(3-fluorazetidin-1 -yl)carbonyl]-5,6,7,8-tetrahydro[1,2,4]triazolo[4,3-a]pyridin-3(2H)-on

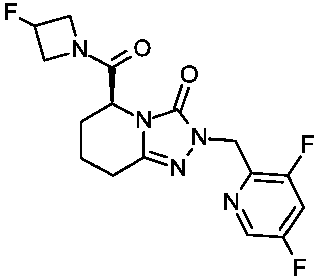

(5S)-2-[(3,5-Difluorpyridin-2-yl)methyl]-3-oxo-2,3,5,6,7,8-hexahydro[1,2,4]triazolo[4,3-a]pyridin-5-carbonsäure (34.2 mg, 90 % Reinheit, 99.2 µmol) wurde in THF (1.0 ml) vorgelegt und HBTU (48.9 mg, 129 µmol) und N,N-Diisopropylethylamin (52 µl, 300 µmol) wurden anschließend zugegeben. Nach 15 min Rühren bei Raumtemperatur wurde 3-Fluorazetidinhydrochlorid (13.3 mg, 119 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 25.9 mg (71 % d. Th.) der Titelverbindung erhalten.
LC-MS (Methode 3): Rₜ = 0.95 min; MS (ESIpos): m/z = 368 [M+H]⁺
¹H-NMR (400 MHz, DMSO-d6) δ [ppm]: -0.149 (2.23), 0.146 (2.23), 1.699 (11.85), 1.933 (4.77), 2.003 (4.62), 2.021 (4.46), 2.327 (4.54), 2.365 (3.85), 2.559 (12.31), 2.572 (5.54), 2.586 (2.08), 2.602 (3.00), 2.669 (4.31), 2.709 (3.31), 3.826 (1.15), 3.844 (1.69), 3.892 (1.85), 3.921 (3.15), 3.949 (3.23), 3.981 (3.15), 4.009 (2.08), 4.262 (3.46), 4.292 (2.92), 4.353 (2.69), 4.390 (2.00), 4.425 (2.08), 4.449 (2.15), 4.515 (10.92), 4.636 (1.69), 4.906 (4.54), 4.945 (16.00), 4.971 (9.31), 5.016 (2.62), 5.354 (2.08), 5.495 (2.08), 6.950 (6.23), 7.078 (6.69), 7.206 (6.31), 7.926 (4.38), 7.932 (4.69), 7.952 (8.23), 7.974 (4.69), 8.458 (9.00).

### Beispiel 411

### (5S)-2-[(3,5-Difluorpyridin-2-yl)methyl]-5-{[(3R,4S)-3,4-difluorpyrrolidin-1 -yl]carbonyl}-5,6,7,8-tetrahydro[1,2,4]triazolo[4,3-a]pyridin-3(2H)-on

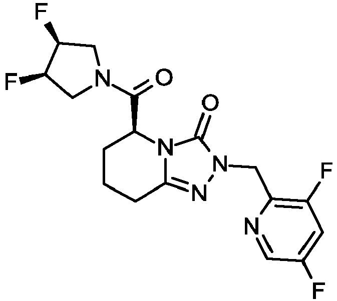

(5S)-2-[(3,5-Difluorpyridin-2-yl)methyl]-3-oxo-2,3,5,6,7,8-hexahydro[1,2,4]triazolo[4,3-a]pyridin-5-carbonsäure (34.2 mg, 90 % Reinheit, 99.2 µmol) wurde in THF (1.0 ml) vorgelegt und HBTU (48.9 mg, 129 µmol) und N,N-Diisopropylethylamin (52 µl, 300 µmol) wurden anschließend zugegeben. Nach 15 min Rühren bei Raumtemperatur wurde (3R,4S)-3,4-Difluorpyrrolidinhydrochlorid (17.1 mg, 119 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 19.7 mg (50 % d. Th.) der Titelverbindung erhalten.
LC-MS (Methode 3): Rₜ = 1.06 min; MS (ESIpos): m/z = 400 [M+H]⁺
¹H-NMR (400 MHz, DMSO-d6) δ [ppm]: -0.149 (1.87), -0.008 (16.00), 0.008 (14.04), 0.146 (1.77), 1.695 (3.14), 1.707 (2.94), 1.936 (1.37), 1.977 (1.57), 2.018 (1.37), 2.035 (1.37), 2.327 (2.85), 2.366 (2.65), 2.563 (4.91), 2.609 (1.57), 2.669 (3.24), 2.710 (2.94), 3.449 (1.18), 3.483 (1.77), 3.495 (1.37), 3.505 (1.47), 3.528 (1.57), 3.538 (1.57), 3.570 (1.08), 3.610 (1.18), 3.625 (1.28), 3.644 (0.88), 3.666 (1.18), 3.681 (2.26), 3.695 (1.77), 3.714 (1.77), 3.727 (1.67), 3.745 (1.47), 3.756 (1.37), 3.778 (1.18), 3.845 (1.87), 3.926 (0.98), 3.938 (1.08), 3.973 (1.08), 3.988 (1.08), 4.168 (1.47), 4.182 (0.88), 4.195 (0.79), 4.762 (3.04), 4.777 (3.93), 4.787 (2.75), 4.893 (2.45), 4.932 (5.99), 4.968 (3.14), 4.980 (3.34), 5.020 (1.47), 5.274 (1.18), 5.348 (0.98), 5.374 (1.37), 5.457 (0.98), 7.928 (1.67), 7.950 (3.14), 7.973 (1.57), 8.465 (5.60).

### Beispiel 412

### (5S)-2-[(3,5-Difluorpyridin-2-yl)methyl]-5-[(3,3-difluorpyrrolidin-1-yl)carbonyl]-5,6,7,8-tetrahydro[1,2,4]triazolo[4,3-a]pyridin-3(2H)-on

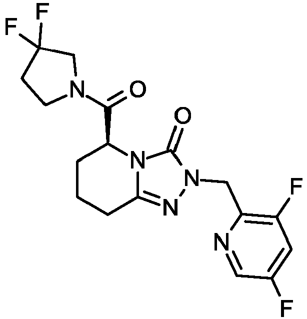

(5S)-2-[(3,5-Difluorpyridin-2-yl)methyl]-3-oxo-2,3,5,6,7,8-hexahydro[1,2,4]triazolo[4,3-a]pyridin-5-carbonsäure (34.2 mg, 90 % Reinheit, 99.2 µmol) wurde in THF (1.0 ml) vorgelegt und HBTU (48.9 mg, 129 µmol) und N,N-Diisopropylethylamin (52 µl, 300 µmol) wurden anschließend zugegeben. Nach 15 min Rühren bei Raumtemperatur wurde 3,3-Difluorpyrrolidinhydrochlorid (17.1 mg, 119 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 19.9 mg (50 % d. Th.) der Titelverbindung erhalten.
spez. Drehwert: -19.10 (589nm, 0.5200g / 100cm³ Chloroform)
Analytische chirale HPLC: Rₜ = 3.77 min, e.e. = 98.5% [Säule: Daicel Chiralpak^{®} AZ-3 3 ym 50 x 4.6 mm; Laufmittel: i-Hexan/i-Propanol 50:50; Fluss: 1 ml/min; UV-Detektion: 220 nm].
LC-MS (Methode 3): Rₜ = 1.14 min; MS (ESIpos): m/z = 400 [M+H]⁺
¹H-NMR (400 MHz, DMSO-d6) δ [ppm]: -0.149 (1.80), 0.008 (16.00), 0.146 (1.69), 1.693 (3.83), 1.957 (2.03), 2.086 (3.61), 2.328 (3.27), 2.366 (4.06), 2.566 (6.20), 2.670 (3.27), 2.710 (3.49), 3.530 (2.25), 3.550 (3.27), 3.666 (1.69), 3.700 (1.92), 3.734 (1.69), 3.765 (2.37), 3.800 (2.48), 3.845 (1.46), 3.890 (0.90), 3.907 (1.92), 3.933 (1.46), 3.991 (1.24), 4.033 (1.13), 4.168 (1.24), 4.198 (1.13), 4.739 (2.37), 4.749 (1.58), 4.819 (2.14), 4.895 (2.59), 4.933 (6.42), 4.978 (6.31), 5.015 (2.48), 7.925 (2.37), 7.930 (2.37), 7.949 (3.72), 7.972 (2.25), 7.978 (2.25), 8.463 (7.89), 8.469 (7.66).

### Beispiel 413

### (5S)-5-[(3,3-Difluorpyrrolidin-1-yl)carbonyl]-2-[(1-methyl-1H-pyrazolo[3,4-b]pyridin-3-yl)methyl]-5,6,7,8-tetrahydro[1,2,4]triazolo[4,3-a]pyridin-3(2H)-on

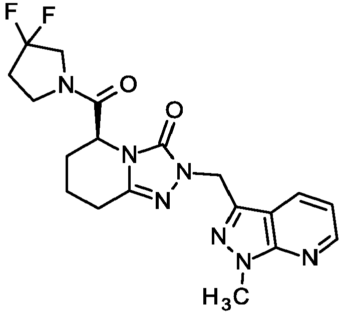

(5S)-2-[(1-Methyl-1H-pyrazolo[3,4-b]pyridin-3-yl)methyl]-3-oxo-2,3,5,6,7,8-hexahydro[1,2,4]triazolo[4,3-a]pyridin-5-carbonsäure (38.0 mg, 116 µmol) wurde in THF (2.0 ml) vorgelegt und HBTU (57.1 mg, 150 µmol) und N,N-Diisopropylethylamin (60 µl, 350 µmol) wurden anschließend zugegeben. Nach 15 min Rühren bei Raumtemperatur wurde 3,3-Difluorpyrrolidinhydrochlorid (19.9 mg, 139 µmol) zugegeben und das Reaktionsgemisch über Nacht bei Raumtemperatur gerührt. Es wurde erneut HBTU (57.1 mg, 150 µmol) und 3,3-Difluorpyrrolidinhydrochlorid (19.9 mg, 139 µmol) zugegeben. Nach 6 Stunden Rühren wurde N,N-Diisopropylethylamin (60 µl, 350 µmol) zugegeben und über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 38.9 mg (93 % Reinheit, 75 % d. Th.) der Titelverbindung erhalten.
LC-MS (Methode 4): Rₜ = 0.61 min; MS (ESIpos): m/z = 418 [M+H]⁺
¹H-NMR (400 MHz, DMSO-d6) δ [ppm]: -0.149 (0.81), -0.008 (6.99), 0.008 (5.93), 0.146 (0.77), 1.160 (3.00), 1.178 (6.33), 1.196 (3.08), 1.405 (1.06), 1.686 (0.59), 1.983 (0.62), 2.327 (1.35), 2.366 (1.50), 2.523 (4.58), 2.670 (1.32), 2.710 (1.43), 3.064 (0.51), 3.083 (1.72), 3.095 (1.76), 3.101 (1.68), 3.113 (1.65), 3.131 (0.51), 3.549 (0.84), 3.567 (0.99), 3.670 (0.48), 3.703 (0.55), 3.783 (0.81), 3.807 (0.55), 3.928 (0.51), 4.027 (16.00), 4.767 (0.66), 4.828 (0.48), 4.836 (0.55), 5.111 (7.91), 7.186 (1.61), 7.197 (1.68), 7.206 (1.79), 7.217 (1.76), 8.151 (1.50), 8.170 (1.46), 8.544 (1.72), 8.548 (1.76), 8.555 (1.83), 8.559 (1.68).

### Beispiel 414

### (5S)-5-{[(3R,4S)-3,4-Difluorpyrrolidin-1-yl]carbonyl}-2-[(1-methyl-1H-pyrazolo[3,4-b]pyridin-3-yl)methyl]-5,6,7,8-tetrahydro[1,2,4]triazolo[4,3-a]pyridin-3(2H)-on

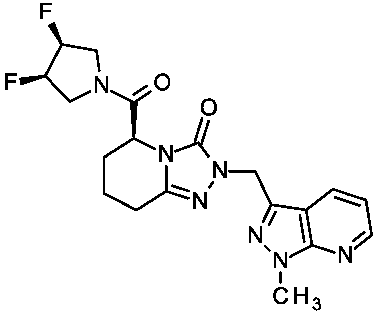

(5S)-2-[(1-Methyl-1H-pyrazolo[3,4-b]pyridin-3-yl)methyl]-3-oxo-2,3,5,6,7,8-hexahydro[1,2,4]triazolo[4,3-a]pyridin-5-carbonsäure (38.0 mg, 116 µmol) wurde in THF (2.0 ml) vorgelegt und HBTU (57.1 mg, 150 µmol) und N,N-Diisopropylethylamin (60 µl, 350 µmol) wurden anschließend zugegeben. Nach 15 min Rühren bei Raumtemperatur wurde (3R,4S)-3,4-Difluorpyrrolidinhydrochlorid (19.9 mg, 139 µmol) zugegeben und das Reaktionsgemisch über Nacht bei Raumtemperatur gerührt. Es wurden erneut HBTU (57.1 mg, 150 µmol) und 3,3-Difluorpyrrolidinhydrochlorid (19.9 mg, 139 µmol) zugegeben. Nach 6 Stunden Rühren wurde N,N-Diisopropylethylamin (60 µl, 350 µmol) zugegeben und über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 33.6 mg (70 % d. Th.) der Titelverbindung erhalten.
LC-MS (Methode 4): Rₜ = 0.58 min; MS (ESIpos): m/z = 418 [M+H]⁺
¹H-NMR (400 MHz, DMSO-d6) δ [ppm]: -0.149 (1.77), -0.008 (16.00), 0.008 (9.91), 0.146 (1.20), 1.699 (0.68), 1.989 (0.80), 2.327 (3.59), 2.366 (4.10), 2.523 (14.98), 2.670 (2.39), 2.710 (1.88), 3.545 (0.74), 3.700 (0.80), 3.928 (0.74), 4.028 (13.27), 4.185 (0.57), 4.798 (1.37), 5.111 (6.78), 5.289 (0.51), 7.182 (1.02), 7.188 (1.20), 7.193 (1.08), 7.199 (1.37), 7.208 (1.37), 7.213 (1.08), 7.219 (1.14), 8.147 (1.08), 8.153 (1.20), 8.167 (1.08), 8.173 (1.14), 8.548 (1.94), 8.555 (1.99), 8.559 (1.94).

### Beispiel 415

### (5S)-5-[(3,3-Difluorazetidin-1-yl)carbonyl]-2-[(1-methyl-1H-pyrazolo[3,4-b]pyridin-3-yl)methyl]-5,6,7,8-tetrahydro[1,2,4]triazolo[4,3-a]pyridin-3(2H)-on

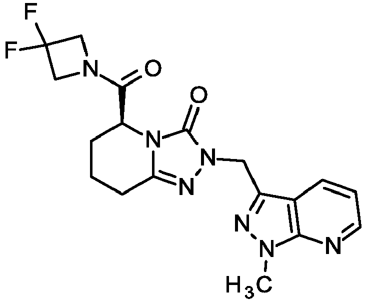

(5S)-2-[(1-Methyl-1H-pyrazolo[3,4-b]pyridin-3-yl)methyl]-3-oxo-2,3,5,6,7,8-hexahydro[1,2,4]triazolo[4,3-a]pyridin-5-carbonsäure (38.0 mg, 116 µmol) wurde in THF (2.0 ml) vorgelegt und HBTU (57.1 mg, 150 µmol) und N,N-Diisopropylethylamin (60 µl, 350 µmol) wurden anschließend zugegeben. Nach 15 min Rühren bei Raumtemperatur wurde 3,3-Difluorazetidinhydrochlorid (18.0 mg, 139 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 11.8 mg (25 % d. Th.) der Titelverbindung erhalten.
LC-MS (Methode 3): Rₜ = 1.05 min; MS (ESIpos): m/z = 404 [M+H]⁺
¹H-NMR (400 MHz, DMSO-d6) δ [ppm]: -0.149 (0.90), 0.008 (6.58), 0.146 (0.85), 1.689 (1.45), 1.968 (0.80), 2.033 (0.65), 2.366 (1.55), 2.523 (4.93), 2.559 (2.04), 2.602 (0.45), 2.710 (1.50), 4.026 (16.00), 4.368 (0.80), 4.395 (0.75), 4.595 (1.00), 4.609 (1.50), 4.621 (1.05), 4.717 (0.50), 4.744 (0.55), 4.861 (0.60), 5.122 (7.13), 7.191 (1.60), 7.202 (1.74), 7.211 (1.69), 7.222 (1.64), 8.153 (1.74), 8.157 (1.79), 8.173 (1.74), 8.177 (1.60), 8.545 (1.79), 8.549 (1.79), 8.556 (1.74), 8.560 (1.64).

### Beispiel 416

### (5RS)-5-{[(3S)-3-Fluorpyrrolidin-1-yl]carbonyl}-2-[(3-methyl-1,2-oxazol-5-yl)methyl]-5,6,7,8-tetrahydro[1,2,4]triazolo[4,3-a]pyridin-3(2H)-on (Racemat)

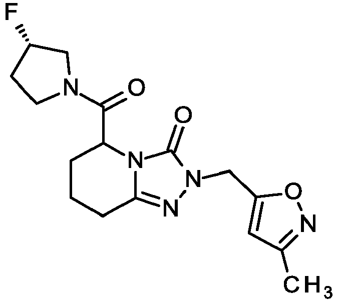

(5RS)-2-[(3-Methyl-1,2-oxazol-5-yl)methyl]-3-oxo-2,3,5,6,7,8-hexahydro[1,2,4]triazolo[4,3-a]pyridin-5-carbonsäure (Racemat) (60.0 mg, 216 µmol) wurde in THF (3.0 ml) bei Raumtemperatur vorgelegt. Anschließend wurden HBTU (123 mg, 323 µmol) und Triethylamin (90 µl, 650 µmol) zugegeben. Nach 15 min Rühren wurde (3S)-3-Fluorpyrrolidin (28.8 mg, 323 µmol) zugegeben und das Reaktionsgemisch für 48 Stunden bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 24.3 mg (32 % d. Th.) der Titelverbindung erhalten.
LC-MS (Methode 3): Rₜ = 0.88 min; MS (ESIpos): m/z = 350 [M+H]⁺
¹H-NMR (400 MHz, DMSO-d6) δ [ppm]: -0.008 (1.93), 0.008 (1.11), 1.721 (0.98), 1.733 (1.10), 1.988 (1.00), 2.040 (0.46), 2.073 (1.80), 2.104 (0.82), 2.136 (0.64), 2.203 (16.00), 2.238 (0.46), 2.268 (0.50), 2.300 (0.64), 2.303 (0.68), 2.327 (0.41), 2.523 (1.89), 2.571 (1.07), 2.595 (1.09), 2.608 (1.43), 2.620 (0.83), 2.650 (0.49), 2.665 (0.50), 2.669 (0.49), 3.345 (0.40), 3.357 (0.54), 3.501 (0.52), 3.508 (0.55), 3.522 (0.69), 3.550 (0.56), 3.569 (0.49), 3.595 (0.91), 3.632 (0.66), 3.653 (0.72), 3.677 (0.52), 3.743 (0.66), 3.775 (0.51), 3.783 (0.46), 3.854 (0.61), 3.940 (0.52), 4.739 (0.43), 4.745 (0.46), 4.812 (0.49), 4.845 (0.42), 4.851 (0.45), 4.946 (5.75), 5.259 (0.41), 5.389 (0.47), 6.226 (2.65).

### Beispiel 417

### (5RS)-5-[(3-Fluorazetidin-1-yl)carbonyl]-2-[(3-methyl-1,2-oxazol-5-yl)methyl]-5,6,7,8-tetrahydro[1,2,4]triazolo[4,3-a]pyridin-3(2H)-on (Racemat)

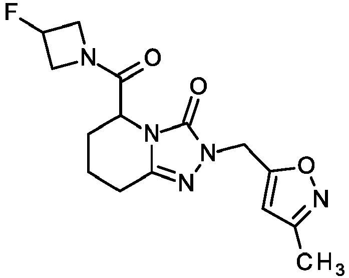

(5RS)-2-[(3-Methyl-1,2-oxazol-5-yl)methyl]-3-oxo-2,3,5,6,7,8-hexahydro[1,2,4]triazolo[4,3-a]pyridin-5-carbonsäure (Racemat) (60.0 mg, 216 µmol) wurde in THF (3.0 ml) bei Raumtemperatur vorgelegt. Anschließend wurden HBTU (123 mg, 323 µmol) und Triethylamin (90 µl, 650 µmol) zugegeben. Nach 15 min Rühren wurde 3-Fluorazetidinhydrochlorid (36.1 mg, 323 µmol) zugegeben und das Reaktionsgemisch für 48 Stunden bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 19.2 mg (27 % d. Th.) der Titelverbindung erhalten.
LC-MS (Methode 3): Rₜ = 0.83 min; MS (ESIpos): m/z = 336 [M+H]⁺
¹H-NMR (400 MHz, DMSO-d6) δ [ppm]: 1.714 (1.68), 1.949 (0.76), 2.029 (0.69), 2.072 (0.96), 2.201 (16.00), 2.293 (1.37), 2.585 (1.67), 2.598 (2.00), 2.612 (1.02), 2.639 (0.61), 2.670 (0.45), 3.928 (0.55), 3.954 (0.54), 3.988 (0.53), 4.269 (0.58), 4.300 (0.50), 4.364 (0.45), 4.528 (1.44), 4.541 (2.10), 4.554 (1.49), 4.950 (5.39), 6.234 (3.50), 6.924 (0.44).

### Beispiel 418

### (5S)-5-[(3,3-Difluorpyrrolidin-1-yl)carbonyl]-2-{[2-(trifluormethyl)-1,8-naphthyridin-3-yl]methyl}-5,6,7,8-tetrahydro[1,2,4]triazolo[4,3-a]pyridin-3(2H)-on

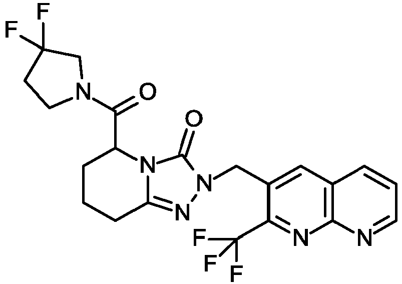

(5S)-3-Oxo-2-{[2-(trifluormethyl)-1,8-naphthyridin-3-yl]methyl}-2,3,5,6,7,8-hexahydro[1,2,4]triazolo[4,3-a]pyridin-5-carbonsäure (50.0 mg, 127 µmol) wurde in THF (3.0 ml) vorgelegt und HBTU (62.7 mg, 165 µmol) und N,N-Diisopropylethylamin (66 µl, 380 µmol) wurden anschließend zugegeben. Nach 15 min Rühren bei Raumtemperatur wurde 3,3-Difluorpyrrolidinhydrochlorid (21.9 mg, 153 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 23.1 mg (38 % d. Th.) der Titelverbindung erhalten.
LC-MS (Methode 3): Rₜ = 1.28 min; MS (ESIpos): m/z = 483 [M+H]⁺
¹H-NMR (400 MHz, DMSO-d6) δ [ppm]: -0.149 (1.63), -0.008 (16.00), 0.008 (12.96), 0.146 (1.63), 1.690 (1.01), 1.783 (1.30), 2.060 (1.95), 2.073 (2.17), 2.092 (2.06), 2.327 (1.23), 2.366 (1.27), 2.445 (1.16), 2.523 (4.45), 2.564 (2.39), 2.584 (2.79), 2.599 (2.57), 2.612 (1.92), 2.625 (1.41), 2.639 (1.48), 2.650 (2.46), 2.664 (1.88), 2.690 (1.09), 2.709 (1.23), 3.578 (1.85), 3.597 (3.00), 3.616 (1.48), 3.722 (1.16), 3.756 (1.34), 3.790 (0.76), 3.813 (2.28), 3.839 (1.52), 3.913 (0.58), 3.931 (1.30), 3.957 (0.87), 3.993 (0.43), 4.020 (0.76), 4.064 (0.69), 4.092 (0.47), 4.174 (0.47), 4.202 (0.76), 4.230 (0.80), 4.855 (1.05), 4.868 (1.70), 4.877 (1.01), 4.922 (1.12), 4.937 (1.67), 4.946 (1.01), 5.218 (10.28), 7.815 (2.68), 7.825 (2.79), 7.835 (2.75), 7.846 (2.79), 8.377 (4.16), 8.385 (4.02), 8.560 (3.40), 8.564 (3.44), 8.580 (3.29), 8.585 (3.11), 9.242 (3.73), 9.247 (3.95), 9.252 (3.87), 9.257 (3.55).

### Beispiel 419

### (5S)-5-{[(3S)-3-Fluorpyrrolidin-1-yl]carbonyl}-2-{[2-(trifluormethyl)-1,8-naphthyridin-3-yl]methyl}-5,6,7,8-tetrahydro[1,2,4]triazolo[4,3-a]pyridin-3(2H)-on

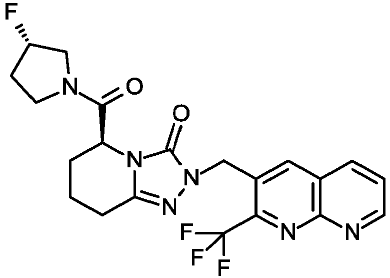

(5S)-3-Oxo-2-{[2-(trifluormethyl)-1,8-naphthyridin-3-yl]methyl}-2,3,5,6,7,8-hexahydro[1,2,4]triazolo[4,3-a]pyridin-5-carbonsäure (50.0 mg, 127 µmol) wurde in THF (3.0 ml) vorgelegt und HBTU (62.7 mg, 165 µmol) und N,N-Diisopropylethylamin (66 µl, 380 µmol) wurden anschließend zugegeben. Nach 15 min Rühren bei Raumtemperatur wurde (3S)-3-Fluorpyrrolidinhydrochlorid (1:1) (19.2 mg, 153 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 36.2 mg (61 % d. Th.) der Titelverbindung erhalten.
LC-MS (Methode 3): Rₜ = 1.14 min; MS (ESIpos): m/z = 465 [M+H]⁺
¹H-NMR (400 MHz, DMSO-d6) δ [ppm]: -0.149 (1.73), -0.008 (16.00), 0.008 (12.57), 0.146 (1.73), 1.782 (0.41), 2.109 (0.72), 2.323 (0.90), 2.327 (1.17), 2.366 (1.13), 2.518 (4.56), 2.523 (3.76), 2.581 (0.45), 2.669 (1.51), 2.710 (1.24), 3.453 (11.33), 3.669 (0.72), 3.780 (0.53), 3.880 (0.53), 4.849 (0.49), 5.217 (2.30), 7.817 (0.90), 7.827 (0.90), 7.838 (0.87), 7.848 (0.90), 8.368 (1.69), 8.570 (0.79), 8.590 (0.72), 9.242 (0.87), 9.246 (0.90), 9.252 (0.87), 9.257 (0.79).

### Beispiel 420

### (5S)-5-[(3-Fluorazetidin-1-yl)carbonyl]-2-{[2-(trifluormethyl)-1,8-naphthyridin-3-yl]methyl}-5,6,7,8-tetrahydro[1,2,4]triazolo[4,3-a]pyridin-3(2H)-on

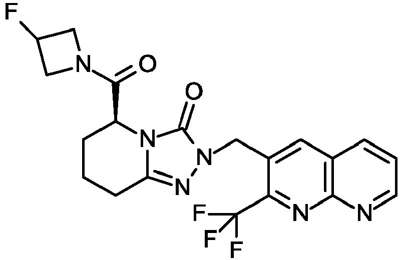

(5S)-3-Oxo-2-{[2-(trifluormethyl)-1,8-naphthyridin-3-yl]methyl}-2,3,5,6,7,8-hexahydro[1,2,4]triazolo[4,3-a]pyridin-5-carbonsäure (50.0 mg, 127 µmol) wurde in THF (3.0 ml) vorgelegt und HBTU (62.7 mg, 165 µmol) und N,N-Diisopropylethylamin (66 µl, 380 µmol) wurden anschließend zugegeben. Nach 15 min Rühren bei Raumtemperatur wurde 3-Fluorazetidinhydrochlorid (17.0 mg, 153 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 20.0 mg (35 % d. Th.) der Titelverbindung erhalten.
LC-MS (Methode 3): Rₜ = 1.09 min; MS (ESIpos): m/z = 451 [M+H]⁺
¹H-NMR (400 MHz, DMSO-d6) δ [ppm]: -0.149 (1.80), -0.008 (16.00), 0.008 (14.79), 0.146 (1.84), 1.759 (1.34), 2.045 (1.72), 2.328 (1.59), 2.366 (1.55), 2.639 (2.64), 2.651 (1.51), 2.669 (2.14), 2.710 (1.51), 3.976 (0.96), 4.280 (0.88), 4.640 (2.26), 4.654 (3.35), 4.665 (2.18), 5.217 (8.00), 5.406 (0.63), 5.549 (0.63), 7.814 (3.18), 7.824 (3.18), 7.835 (3.35), 7.845 (3.31), 8.384 (3.77), 8.558 (2.35), 8.579 (2.30), 9.241 (3.39), 9.246 (3.64), 9.252 (3.73), 9.257 (3.35).

### Beispiel 421

### (5S)-2-[(6-Chlorpyridin-3-yl)methyl]-5-[(3-fluorazetidin-1 -yl)carbonyl]-5,6,7,8-tetrahydro[1,2,4]triazolo[4,3-a]pyridin-3(2H)-on

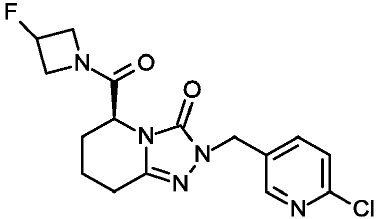

(5S)-2-[(6-Chlorpyridin-3-yl)methyl]-3-oxo-2,3,5,6,7,8-hexahydro[1,2,4]triazolo[4,3-a]pyridin-5-carbonsäure (50.0 mg, 162 µmol) wurde in THF (3.0 ml) vorgelegt und HBTU (79.8 mg, 211 µmol) und N,N-Diisopropylethylamin (85 µl, 490 µmol) wurden anschließend zugegeben. Nach 15 min Rühren bei Raumtemperatur wurde 3-Fluorazetidinhydrochlorid (21.7 mg, 194 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 21.0 mg (35 % d. Th.) der Titelverbindung erhalten.
LC-MS (Methode 3): Rₜ = 0.99 min; MS (ESIpos): m/z = 366 [M+H]⁺
¹H-NMR (400 MHz, DMSO-d6) δ [ppm]: -0.149 (0.64), -0.008 (6.53), 0.008 (4.72), 0.146 (0.64), 1.648 (1.11), 1.662 (1.43), 1.672 (2.04), 1.696 (3.22), 1.707 (3.33), 1.719 (3.12), 1.956 (2.12), 1.966 (1.95), 2.002 (1.74), 2.012 (1.71), 2.023 (1.87), 2.327 (0.49), 2.523 (1.99), 2.576 (3.91), 2.590 (5.43), 2.604 (2.71), 2.619 (1.02), 2.632 (1.76), 2.646 (0.72), 2.670 (0.52), 3.897 (0.69), 3.926 (1.46), 3.957 (1.54), 3.989 (1.43), 4.020 (0.85), 4.154 (0.63), 4.169 (0.72), 4.185 (0.58), 4.208 (0.80), 4.223 (1.29), 4.238 (1.27), 4.253 (1.21), 4.270 (1.58), 4.291 (1.46), 4.322 (1.18), 4.360 (1.07), 4.394 (0.93), 4.429 (0.66), 4.456 (0.89), 4.503 (0.67), 4.518 (0.83), 4.538 (3.62), 4.551 (5.54), 4.564 (3.58), 4.598 (0.52), 4.632 (0.61), 4.646 (0.69), 4.659 (0.63), 4.684 (0.69), 4.700 (0.72), 4.724 (0.53), 4.883 (16.00), 5.351 (0.91), 5.406 (0.93), 5.494 (0.89), 5.541 (0.77), 5.548 (0.89), 6.515 (0.69), 7.503 (5.54), 7.523 (6.98), 7.694 (4.39), 7.714 (3.70), 8.305 (5.65).

### Beispiel 422

### (5S)-2-[(6-Chlorpyridin-3-yl)methyl]-5-{[(3S)-3-fluorpyrrolidin-1-yl]carbonyl}-5,6,7,8-tetrahydro[1,2,4]triazolo[4,3-a]pyridin-3(2H)-on

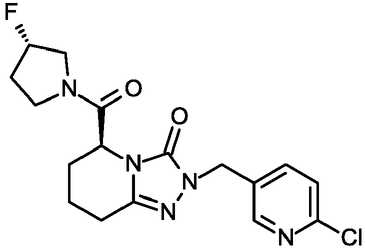

(5S)-2-[(6-Chlorpyridin-3-yl)methyl]-3-oxo-2,3,5,6,7,8-hexahydro[1,2,4]triazolo[4,3-a]pyridin-5-carbonsäure (50.0 mg, 162 µmol) wurde in THF (3.0 ml) vorgelegt und HBTU (79.8 mg, 211 µmol) und N,N-Diisopropylethylamin (85 µl, 490 µmol) wurden anschließend zugegeben. Nach 15 min Rühren bei Raumtemperatur wurde (3S)-3-Fluorpyrrolidinhydrochlorid (24.4 mg, 194 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 32.1 mg (52 % d. Th.) der Titelverbindung erhalten.
LC-MS (Methode 3): Rₜ = 1.05 min; MS (ESIpos): m/z = 380 [M+H]⁺
¹H-NMR (400 MHz, DMSO-d6) δ [ppm]: 0.008 (1.05), 1.720 (2.57), 1.727 (2.64), 1.870 (0.64), 1.894 (0.61), 1.904 (0.81), 1.968 (0.58), 1.993 (1.05), 2.020 (1.59), 2.042 (1.32), 2.058 (1.52), 2.074 (1.86), 2.085 (2.16), 2.094 (2.00), 2.101 (2.00), 2.121 (1.39), 2.135 (1.42), 2.217 (0.95), 2.237 (0.88), 2.266 (1.15), 2.519 (3.96), 2.524 (4.19), 2.563 (1.96), 2.578 (1.66), 2.602 (2.47), 2.646 (0.91), 2.670 (0.41), 3.344 (1.01), 3.357 (0.85), 3.365 (0.81), 3.393 (0.81), 3.401 (0.78), 3.454 (0.64), 3.464 (0.64), 3.490 (0.85), 3.499 (0.74), 3.606 (0.74), 3.632 (2.77), 3.649 (2.27), 3.655 (2.06), 3.675 (1.76), 3.697 (1.22), 3.721 (1.32), 3.740 (2.10), 3.764 (1.69), 3.783 (1.22), 3.850 (2.40), 4.678 (1.32), 4.687 (1.52), 4.694 (1.56), 4.703 (1.25), 4.736 (1.69), 4.745 (1.83), 4.751 (1.89), 4.760 (1.35), 4.880 (16.00), 5.257 (1.18), 5.380 (1.52), 5.388 (1.49), 5.509 (0.88), 7.504 (4.87), 7.524 (5.92), 7.683 (2.23), 7.690 (3.92), 7.696 (2.44), 7.704 (1.83), 7.710 (3.11), 7.717 (1.76), 8.296 (4.06), 8.302 (3.55).

### Beispiel 423

### (5S)-2-[(6-Chlorpyridin-3-yl)methyl]-5-[(3,3-difluorpyrrolidin-1-yl)carbonyl]-5,6,7,8-tetrahydro[1,2,4]triazolo[4,3-a]pyridin-3(2H)-on

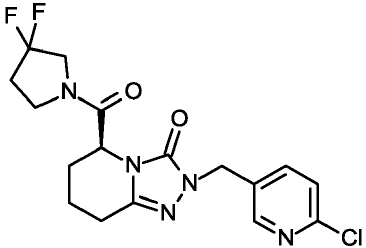

(5S)-2-[(6-Chlorpyridin-3-yl)methyl]-3-oxo-2,3,5,6,7,8-hexahydro[1,2,4]triazolo[4,3-a]pyridin-5-carbonsäure (50.0 mg, 162 µmol) wurde in THF (3.0 ml) vorgelegt und HBTU (79.8 mg, 211 µmol) und N,N-Diisopropylethylamin (85 µl, 490 µmol) wurden anschließend zugegeben. Nach 15 min Rühren bei Raumtemperatur wurde 3,3-Difluorpyrrolidinhydrochlorid (27.9 mg, 194 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 32.2 mg (50 % d. Th.) der Titelverbindung erhalten.
LC-MS (Methode 4): Rₜ = 0.66 min; MS (ESIpos): m/z = 398 [M+H]⁺
¹H-NMR (400 MHz, DMSO-d6) δ [ppm]: -0.149 (0.50), -0.008 (4.74), 0.008 (4.04), 0.146 (0.47), 1.660 (1.02), 1.727 (1.22), 1.996 (1.41), 2.007 (1.46), 2.043 (1.07), 2.050 (1.07), 2.059 (0.97), 2.073 (7.86), 2.328 (0.72), 2.366 (0.64), 2.380 (0.87), 2.409 (1.04), 2.424 (0.99), 2.451 (0.82), 2.560 (2.51), 2.569 (3.00), 2.585 (2.75), 2.602 (2.68), 2.645 (0.77), 2.670 (0.69), 2.710 (0.47), 3.529 (1.07), 3.535 (1.14), 3.546 (1.74), 3.556 (1.89), 3.565 (1.12), 3.575 (0.99), 3.665 (1.14), 3.699 (1.29), 3.742 (0.62), 3.776 (1.54), 3.807 (2.41), 3.825 (0.60), 3.886 (0.57), 3.906 (1.24), 3.931 (0.87), 3.951 (0.45), 3.990 (0.79), 4.018 (0.52), 4.034 (0.72), 4.061 (0.42), 4.144 (0.47), 4.175 (0.72), 4.200 (0.72), 4.749 (1.07), 4.764 (1.44), 4.773 (1.04), 4.821 (1.12), 4.836 (1.46), 4.845 (1.19), 4.884 (16.00), 7.505 (4.99), 7.525 (6.28), 7.686 (3.32), 7.692 (3.40), 7.707 (2.70), 7.713 (2.73), 8.296 (4.94), 8.302 (4.79).

### Beispiel 424

### (5S)-2-[(6-Chlorpyridin-3-yl)methyl]-5-{[(3R,4S)-3,4-difluorpyrrolidin-1-yl]carbonyl}-5,6,7,8-tetrahydro[1,2,4]triazolo[4,3-a]pyridin-3(2H)-on

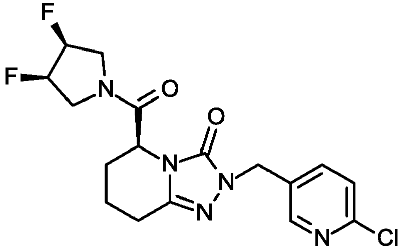

(5S)-2-[(6-Chlorpyridin-3-yl)methyl]-3-oxo-2,3,5,6,7,8-hexahydro[1,2,4]triazolo[4,3-a]pyridin-5-carbonsäure (70.0 mg, 227 µmol) wurde in THF (4.0 ml) vorgelegt und HBTU (112 mg, 295 µmol) und N,N-Diisopropylethylamin (120 µl, 680 µmol) wurden anschließend zugegeben. Nach 15 min Rühren bei Raumtemperatur wurde (3R,4S)-3,4-Difluorpyrrolidinhydrochlorid (39.1 mg, 272 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 66.6 mg (74 % d. Th.) der Titelverbindung erhalten.
LC-MS (Methode 3): Rₜ = 1.10 min; MS (ESIpos): m/z = 398 [M+H]⁺
¹H-NMR (400 MHz, DMSO-d6) δ [ppm]: -0.149 (1.29), -0.008 (12.08), 0.008 (10.65), 0.146 (1.38), 1.656 (1.21), 1.729 (1.51), 1.968 (1.25), 2.013 (1.73), 2.073 (3.54), 2.327 (1.60), 2.366 (1.12), 2.563 (2.59), 2.588 (2.33), 2.601 (2.93), 2.644 (1.08), 2.669 (1.73), 2.710 (1.25), 3.532 (1.25), 3.577 (0.82), 3.626 (0.99), 3.683 (1.51), 3.700 (1.51), 3.731 (1.29), 3.752 (1.21), 3.866 (0.78), 3.937 (0.95), 3.973 (0.86), 3.988 (0.91), 4.170 (0.91), 4.797 (3.19), 4.884 (16.00), 5.274 (0.95), 5.349 (0.91), 5.389 (1.16), 7.506 (4.27), 7.526 (5.13), 7.680 (1.94), 7.688 (3.15), 7.695 (2.42), 7.709 (2.59), 7.716 (1.90), 8.297 (5.65).

### Beispiel 425

### (5S)-5-{[(3S)-3-Hydroxypyrrolidin-1-yl]carbonyl}-2-{[6-(trifluormethyl)pyridin-3-yl]methyl}-5,6,7,8-tetrahydro[1,2,4]triazolo[4,3-a]pyridin-3(2H)-on

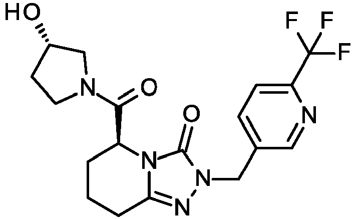

(5S)-3-Oxo-2-{[6-(trifluormethyl)pyridin-3-yl]methyl}-2,3,5,6,7,8-hexahydro[1,2,4]triazolo[4,3-a]pyridin-5-carbonsäure (60.0 mg, 175 µmol) wurde in THF (2.0 ml) vorgelegt und HATU (86.6 mg, 228 µmol) und Triethylamin (73 µl, 530 µmol) wurden anschließend zugegeben. Nach 15 min Rühren bei Raumtemperatur wurde (3S)-Pyrrolidin-3-olhydrochlorid (26.0 mg, 210 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und der Rückstand wurde über präparative HPLC aufgereinigt (Methode 10). Die produkthaltigen Fraktionen wurden im Vakuum eingeengt und es wurden 1.00 mg (92 % Reinheit, 1 % d. Th.) der Titelverbindung erhalten.
LC-MS (Methode 3): Rₜ = 0.99 min; MS (ESIpos): m/z = 412 [M+H]⁺
¹H-NMR (400 MHz, DMSO-d6) δ [ppm]: -0.008 (0.69), 0.008 (0.58), 1.637 (0.77), 1.644 (0.75), 1.650 (0.83), 1.663 (1.03), 1.677 (0.98), 1.689 (0.86), 1.736 (2.05), 1.751 (1.94), 1.774 (1.47), 1.783 (1.06), 1.828 (0.46), 1.840 (0.61), 1.851 (1.28), 1.862 (1.57), 1.873 (1.33), 1.883 (1.65), 1.894 (1.33), 1.905 (0.93), 1.939 (0.53), 1.973 (1.71), 1.985 (1.87), 1.997 (2.24), 2.007 (2.18), 2.019 (2.06), 2.029 (1.92), 2.036 (1.50), 2.045 (1.31), 2.057 (1.10), 2.065 (1.19), 2.073 (1.20), 2.523 (1.16), 2.561 (1.75), 2.575 (1.29), 2.597 (1.55), 2.608 (2.72), 2.619 (1.46), 2.638 (0.72), 2.650 (1.08), 2.661 (0.60), 3.204 (0.72), 3.236 (1.03), 3.343 (1.75), 3.369 (1.82), 3.379 (2.23), 3.397 (1.40), 3.419 (0.57), 3.434 (0.59), 3.443 (0.61), 3.457 (1.07), 3.464 (1.17), 3.473 (0.55), 3.485 (1.17), 3.550 (0.84), 3.567 (1.42), 3.573 (1.63), 3.589 (1.08), 3.599 (0.79), 3.640 (0.94), 3.651 (1.41), 3.667 (0.92), 3.678 (1.09), 3.756 (0.58), 4.270 (1.41), 4.363 (1.43), 4.696 (0.83), 4.705 (0.97), 4.712 (1.06), 4.720 (0.84), 4.739 (1.24), 4.752 (1.60), 4.761 (1.16), 4.806 (0.64), 4.815 (0.73), 4.821 (0.85), 4.830 (0.66), 4.964 (2.61), 5.005 (10.49), 5.078 (2.55), 5.087 (2.46), 7.909 (16.00), 8.640 (4.92).

### Beispiel 426

### (5S)-5-[(3 ,3-Difluorpyrrolidin-1-yl)carbonyl]-2-{[6-(trifluormethyl)pyridin-3-yl] methyl}-5,6, 7 ,8-tetrahydro[1,2,4]triazolo[4,3-a]pyridin-3(2H)-on

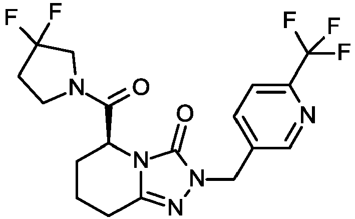

(5S)-3-Oxo-2-{[6-(trifluormethyl)pyridin-3-yl]methyl}-2,3,5,6,7,8-hexahydro[1,2,4]triazolo[4,3-a]pyridin-5-carbonsäure (100 mg, 85 % Reinheit, 248 µmol) wurde in THF (2.0 ml) vorgelegt und HBTU (122 mg, 323 µmol) und N,N-Diisopropylethylamin (130 µl, 750 µmol) wurden anschließend zugegeben. Nach 15 min Rühren bei Raumtemperatur wurde 3,3-Difluorpyrrolidinhydrochlorid (42.8 mg, 298 µmol) zugegeben und das Reaktionsgemisch über Nacht bei Raumtemperatur gerührt. Es wurden erneut HBTU (122 mg, 323 µmol) und 3,3-Difluorpyrrolidinhydrochlorid (42.8 mg, 298 µmol) zugegeben. Nach Rühren für 48 Stunden bei Raumtemperatur wurde N,N-Diisopropylethylamin (130 µl, 750 µmol) zugegeben und über Nacht gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 79.6 mg (72 % d. Th.) der Titelverbindung erhalten.
LC-MS (Methode 1): Rₜ = 1.01 min; MS (ESIpos): m/z = 432 [M+H]⁺
¹H-NMR (400 MHz, DMSO-d6) δ [ppm]: -0.150 (0.48), -0.008 (6.41), 0.008 (3.51), 0.146 (0.45), 1.375 (0.48), 1.405 (1.49), 1.663 (1.24), 1.673 (1.15), 1.736 (1.52), 1.753 (1.60), 1.879 (0.96), 1.973 (1.38), 2.008 (1.88), 2.018 (1.83), 2.033 (1.72), 2.043 (1.49), 2.051 (1.35), 2.059 (1.24), 2.073 (1.86), 2.087 (0.90), 2.327 (0.59), 2.366 (0.93), 2.382 (1.12), 2.410 (1.77), 2.430 (2.33), 2.524 (3.77), 2.569 (3.63), 2.579 (2.76), 2.593 (2.87), 2.610 (3.09), 2.623 (1.63), 2.641 (0.70), 2.653 (1.01), 2.665 (0.96), 2.710 (0.53), 3.534 (1.38), 3.541 (1.55), 3.551 (2.11), 3.561 (2.17), 3.570 (1.29), 3.580 (1.07), 3.637 (0.45), 3.670 (1.32), 3.704 (1.52), 3.738 (0.79), 3.747 (0.93), 3.782 (1.97), 3.813 (2.59), 3.830 (0.70), 3.847 (0.42), 3.891 (0.76), 3.910 (1.49), 3.928 (0.84), 3.936 (1.04), 3.954 (0.53), 3.965 (0.51), 3.994 (0.93), 4.009 (0.51), 4.023 (0.70), 4.038 (0.84), 4.065 (0.51), 4.149 (0.59), 4.180 (0.84), 4.205 (0.87), 4.767 (1.35), 4.781 (1.69), 4.791 (1.21), 4.837 (1.32), 4.847 (1.52), 4.853 (1.63), 4.862 (1.15), 4.970 (0.56), 5.011 (14.23), 7.909 (16.00), 7.912 (15.38), 8.641 (5.34).

### Beispiel 427

### (5S)-5-{[(3S)-3-Fluorpyrrolidin-1 -yl]carbonyl}-2-{[6-(trifluormethyl)pyridin-3-yl]methyl}-5,6,7,8-tetrahydro[1,2,4]triazolo[4,3-a]pyridin-3(2H)-on

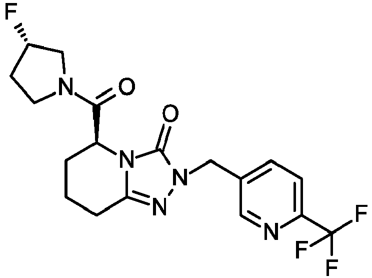

(5S)-3-Oxo-2-{[6-(trifluormethyl)pyridin-3-yl]methyl}-2,3,5,6,7,8-hexahydro[1,2,4]triazolo[4,3-a]pyridin-5-carbonsäure (1000 mg, 2.92 mmol) wurde in THF (18 ml) bei Raumtemperatur vorgelegt. Anschließend wurden HBTU (1.44 g, 3.80 mmol) und N,N-Diisopropylethylamin (1.5 ml, 8.8 mmol) zugegeben. Nach 15 min Rühren wurde (3S)-3-Fluorpyrrolidinhydrochlorid (404 mg, 3.21 mmol) zugegeben und das Reaktionsgemisch über Nacht bei Raumtemperatur gerührt. Es wurde erneut N,N-Diisopropylethylamin (1.5 ml, 8.8 mmol) zugegeben und erneut über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 797 mg (66 % d. Th.) der Titelverbindung erhalten.
LC-MS (Methode 3): Rₜ = 1.23 min; MS (ESIpos): m/z = 414 [M+H]⁺
¹H-NMR (400 MHz, DMSO-d6) δ [ppm]: -0.008 (1.38), 0.008 (1.26), 1.701 (1.17), 1.712 (1.38), 1.726 (2.00), 1.736 (2.23), 1.883 (0.48), 1.906 (0.51), 1.923 (0.69), 1.997 (0.78), 2.005 (0.71), 2.022 (0.83), 2.032 (1.29), 2.053 (1.08), 2.073 (2.09), 2.088 (1.68), 2.104 (2.05), 2.124 (1.15), 2.139 (1.40), 2.173 (0.41), 2.221 (0.76), 2.243 (0.69), 2.256 (0.74), 2.270 (1.03), 2.327 (0.53), 2.366 (0.55), 2.519 (2.48), 2.523 (2.09), 2.562 (1.54), 2.566 (1.52), 2.572 (1.59), 2.588 (1.33), 2.614 (2.30), 2.657 (0.90), 2.670 (0.87), 2.710 (0.53), 3.275 (0.69), 3.350 (0.62), 3.362 (0.60), 3.371 (0.64), 3.398 (0.74), 3.407 (0.74), 3.459 (0.55), 3.468 (0.57), 3.495 (0.78), 3.504 (0.74), 3.610 (0.51), 3.637 (2.16), 3.654 (1.86), 3.661 (1.86), 3.681 (1.54), 3.703 (0.97), 3.727 (1.06), 3.746 (1.89), 3.769 (1.36), 3.775 (1.40), 3.789 (1.15), 3.857 (2.28), 4.694 (1.10), 4.703 (1.33), 4.710 (1.38), 4.719 (1.10), 4.752 (1.43), 4.761 (1.56), 4.767 (1.82), 4.776 (1.36), 5.008 (14.51), 5.260 (1.13), 5.383 (1.33), 5.391 (1.40), 5.511 (0.78), 7.911 (16.00), 7.914 (10.30), 8.642 (4.76).

### Beispiel 428

### (5S)-5-[(3-Hydroxyazetidin-1-yl)carbonyl]-2-{[6-(trifluormethyl)pyridin-3-yl]methyl}-5,6,7,8-tetrahydro[1,2,4]triazolo[4,3-a]pyridin-3(2H)-on

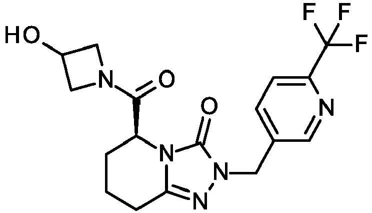

(5S)-3-Oxo-2-{[6-(trifluormethyl)pyridin-3-yl]methyl}-2,3,5,6,7,8-hexahydro[1,2,4]triazolo[4,3-a]pyridin-5-carbonsäure (65.0 mg, 190 µmol) wurde in THF bei Raumtemperatur vorgelegt. Anschließend wurden HATU (93.9 mg, 247 µmol) und Triethylamin (79 µl, 570 µmol) zugegeben. Nach 15 min Rühren wurde Azetidin-3-olhydrochlorid (25.0 mg, 228 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und der Rückstand wurde über präparative HPLC aufgereinigt (Methode 10). Die produkthaltigen Fraktionen wurden im Vakuum eingeengt und es wurden 1.00 mg (1 % d. Th.) der Titelverbindung erhalten.
LC-MS (Methode 3): Rₜ = 0.98 min; MS (ESIpos): m/z = 398 [M+H]⁺
¹H-NMR (400 MHz, DMSO-d6) δ [ppm]: -0.149 (2.09), -0.008 (15.61), 0.008 (16.00), 0.146 (2.16), 2.327 (4.71), 2.366 (2.16), 2.669 (4.41), 2.709 (2.40), 4.534 (2.09), 5.010 (4.48), 5.803 (1.93), 7.908 (5.87), 8.644 (3.25).

### Beispiel 429

### (5S)-5-[(3-Fluorazetidin-1-yl)carbonyl]-2-{[6-(trifluormethyl)pyridin-3-yl]methyl}-5,6,7,8-tetrahydro[1,2,4]triazolo[4,3-a]pyridin-3(2H)-on

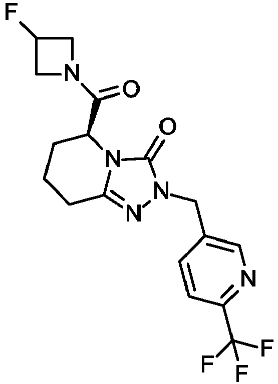

(5S)-3-Oxo-2-{[6-(trifluormethyl)pyridin-3-yl]methyl}-2,3,5,6,7,8-hexahydro[1,2,4]triazolo[4,3-a]pyridin-5-carbonsäure (100 mg, 292 µmol) wurde in THF (2.0 ml) vorgelegt und HBTU (144 mg, 380 µmol) und N,N-Diisopropylethylamin (150 µl, 880 µmol) wurden anschließend zugegeben. Nach 15 min Rühren bei Raumtemperatur wurde 3-Fluorazetidinhydrochlorid (39.1 mg, 351 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 74.0 mg (63 % d. Th.) der Titelverbindung erhalten.
LC-MS (Methode 1): Rₜ = 0.90 min; MS (ESIpos): m/z = 400 [M+H]⁺
¹H-NMR (400 MHz, DMSO-d6) δ [ppm]: 1.658 (1.45), 1.670 (1.85), 1.683 (2.02), 1.694 (2.48), 1.708 (2.77), 1.961 (1.98), 1.975 (1.90), 2.004 (1.65), 2.025 (1.68), 2.359 (0.41), 2.579 (3.40), 2.593 (4.36), 2.606 (2.24), 2.622 (0.89), 2.635 (1.39), 2.648 (0.64), 2.662 (0.41), 3.898 (0.65), 3.925 (1.33), 3.956 (1.38), 3.986 (1.29), 4.017 (0.76), 4.153 (0.58), 4.168 (0.66), 4.181 (0.58), 4.206 (0.78), 4.222 (1.20), 4.236 (1.17), 4.250 (1.09), 4.269 (1.43), 4.290 (1.27), 4.321 (0.95), 4.359 (1.03), 4.393 (0.82), 4.429 (0.61), 4.455 (0.79), 4.505 (0.69), 4.518 (0.83), 4.550 (3.29), 4.562 (4.78), 4.575 (3.11), 4.597 (0.48), 4.630 (0.56), 4.646 (0.65), 4.681 (0.63), 4.699 (0.62), 4.724 (0.46), 5.004 (12.79), 5.347 (0.79), 5.401 (0.77), 5.489 (0.79), 5.543 (0.75), 7.905 (16.00), 8.642 (6.38).

### Beispiel 430

### (5S)-5-{[(3S)-3-Fluorpyrrolidin-1-yl]carbonyl}-2-[(6-methoxypyridin-3-yl)methyl]-5,6,7,8-tetrahydro[1,2,4]triazolo[4,3-a]pyridin-3(2H)-on

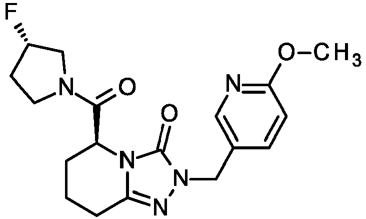

(5S)-2-[(6-Methoxypyridin-3-yl)methyl]-3-oxo-2,3,5,6,7,8-hexahydro[1,2,4]triazolo[4,3-a]pyridin-5-carbonsäure (390 mg, 1.28 mmol) wurde in THF (5.0 ml) vorgelegt und HBTU (632 mg, 1.67 mmol) und N,N-Diisopropylethylamin (670 µl, 3.8 mmol) wurden anschließend zugegeben. Nach 15 min Rühren bei Raumtemperatur wurde (3S)-3-Fluorpyrrolidinhydrochlorid (193 mg, 1.54 mmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 98.9 mg (20 % d. Th.) der Titelverbindung erhalten.
LC-MS (Methode 4): Rₜ = 0.57 min; MS (ESIpos): m/z = 376 [M+H]⁺
¹H-NMR (400 MHz, DMSO-d6) δ [ppm]: -0.008 (1.34), 0.008 (1.26), 1.706 (0.98), 1.718 (1.14), 2.003 (0.53), 2.048 (0.45), 2.073 (0.60), 2.083 (0.66), 2.108 (0.58), 2.134 (0.43), 2.524 (1.18), 2.565 (0.60), 2.593 (0.91), 3.632 (1.01), 3.650 (0.75), 3.677 (0.48), 3.718 (0.42), 3.736 (0.77), 3.765 (0.54), 3.781 (0.53), 3.827 (16.00), 3.851 (0.91), 4.661 (0.43), 4.671 (0.50), 4.677 (0.55), 4.686 (0.45), 4.719 (0.57), 4.727 (0.67), 4.734 (0.76), 4.743 (0.60), 4.762 (5.88), 5.258 (0.42), 5.389 (0.56), 6.784 (2.23), 6.806 (2.39), 7.551 (0.72), 7.557 (1.53), 7.563 (0.93), 7.572 (0.71), 7.578 (1.46), 7.584 (0.86), 8.054 (1.92).

### Beispiel 431

### (5S)-5-{[(3R,4S)-3,4-Difluorpyrrolidin-1-yl]carbonyl}-2-[(6-methoxypyridin-3-yl)methyl]-5,6,7,8-tetrahydro[1,2,4]triazolo[4,3-a]pyridin-3(2H)-on

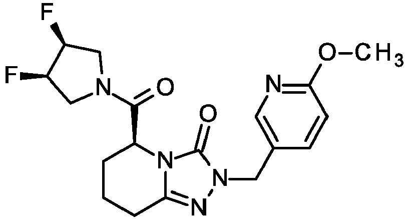

(5S)-2-[(6-Methoxypyridin-3-yl)methyl]-3-oxo-2,3,5,6,7,8-hexahydro[1,2,4]triazolo[4,3-a]pyridin-5-carbonsäure (390 mg, 1.28 mmol) wurde in THF (5.0 ml) vorgelegt und HBTU (632 mg, 1.67 mmol) und N,N-Diisopropylethylamin (670 µl, 3.8 mmol) wurden anschließend zugegeben. Nach 15 min Rühren bei Raumtemperatur wurde (3R,4S)-3,4-Difluorpyrrolidinhydrochlorid (221 mg, 1.54 mmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 111 mg (22 % d. Th.) der Titelverbindung erhalten.
LC-MS (Methode 4): Rₜ = 0.61 min; MS (ESIpos): m/z = 394 [M+H]⁺
¹H-NMR (400 MHz, DMSO-d6) δ [ppm]: -0.008 (0.88), 0.008 (0.85), 1.657 (0.55), 1.695 (0.52), 1.707 (0.63), 1.716 (0.71), 1.730 (0.60), 1.948 (0.53), 1.956 (0.47), 1.994 (0.68), 2.003 (0.53), 2.018 (0.42), 2.029 (0.48), 2.037 (0.53), 2.045 (0.50), 2.052 (0.41), 2.073 (0.81), 2.525 (0.94), 2.575 (0.99), 2.588 (1.28), 2.599 (0.72), 2.630 (0.43), 3.482 (0.43), 3.491 (0.41), 3.532 (0.52), 3.539 (0.47), 3.625 (0.42), 3.666 (0.41), 3.679 (0.56), 3.686 (0.47), 3.699 (0.72), 3.713 (0.46), 3.722 (0.55), 3.752 (0.47), 3.764 (0.45), 3.825 (14.71), 3.828 (16.00), 3.935 (0.40), 4.169 (0.44), 4.766 (8.26), 5.275 (0.42), 5.376 (0.42), 5.386 (0.47), 5.398 (0.40), 6.785 (1.86), 6.806 (2.01), 7.547 (0.88), 7.555 (1.43), 7.562 (1.08), 7.569 (0.92), 7.576 (1.40), 7.583 (1.01), 8.054 (2.30).

### Beispiel 432

### (2S)-1-({(5S)-2-[(6-Methoxypyridin-3-yl)methyl]-3-oxo-2,3,5,6,7,8-hexahydro[1,2,4]triazolo[4,3-a]pyridin-5-yl}carbonyl)pyrrolidin-2-carbonitril

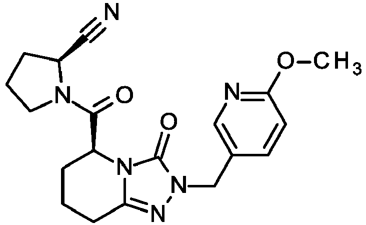

(5S)-2-[(6-Methoxypyridin-3-yl)methyl]-3-oxo-2,3,5,6,7,8-hexahydro[1,2,4]triazolo[4,3-a]pyridin-5-carbonsäure (390 mg, 1.28 mmol) wurde in THF (5.0 ml) vorgelegt und HBTU (632 mg, 1.67 mmol) und N,N-Diisopropylethylamin (670 µl, 3.8 mmol) wurden anschließend zugegeben. Nach 15 min Rühren bei Raumtemperatur wurde (2S)-Pyrrolidin-2-carbonitrilhydrochlorid (204 mg, 1.54 mmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 109 mg (18 % d. Th.) der Titelverbindung erhalten.
LC-MS (Methode 4): Rₜ = 0.60 min; MS (ESIpos): m/z = 383 [M+H]⁺
¹H-NMR (400 MHz, DMSO-d6) δ [ppm]: -0.008 (0.93), 0.008 (0.87), 1.988 (0.50), 2.027 (0.70), 2.045 (0.98), 2.059 (1.43), 2.076 (1.42), 2.093 (0.73), 2.104 (0.58), 2.115 (0.48), 2.135 (0.50), 2.148 (0.61), 2.159 (0.53), 2.206 (0.71), 2.225 (0.67), 2.237 (0.42), 2.562 (0.56), 2.573 (0.59), 2.587 (0.46), 2.608 (0.41), 2.620 (0.75), 2.632 (0.43), 2.899 (0.83), 3.670 (1.14), 3.686 (2.31), 3.703 (1.10), 3.828 (16.00), 4.767 (3.05), 4.772 (2.80), 4.786 (0.78), 4.793 (1.36), 4.803 (1.54), 4.813 (1.53), 4.823 (0.80), 6.788 (1.71), 6.810 (1.83), 7.550 (1.02), 7.556 (1.09), 7.571 (1.03), 7.577 (1.07), 8.051 (1.46), 8.056 (1.52).

### Beispiel 433

### (5S)-5-[(3-Fluorazetidin-1-yl)carbonyl]-2-[(6-methoxypyridin-3-yl)methyl]-5,6,7,8-tetrahydro[1,2,4]triazolo[4,3-a]pyridin-3(2H)-on

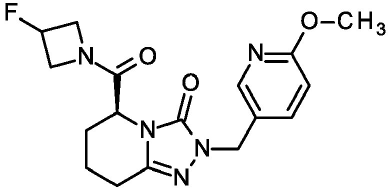

(5S)-2-[(6-Methoxypyridin-3-yl)methyl]-3-oxo-2,3,5,6,7,8-hexahydro[1,2,4]triazolo[4,3-a]pyridin-5-carbonsäure (390 mg, 1.28 mmol) wurde in THF (5.0 ml) vorgelegt und HBTU (632 mg, 1.67 mmol) und N,N-Diisopropylethylamin (670 µl, 3.8 mmol) wurden anschließend zugegeben. Nach 15 min Rühren bei Raumtemperatur wurde 3-Fluorazetidinhydrochlorid (172 mg, 1.54 mmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 82.9 mg (18 % d. Th.) der Titelverbindung erhalten.
LC-MS (Methode 4): Rₜ = 0.55 min; MS (ESIpos): m/z = 362 [M+H]⁺
¹H-NMR (400 MHz, DMSO-d6) δ [ppm]: -0.008 (0.77), 0.008 (0.79), 1.697 (1.16), 1.940 (0.55), 2.002 (0.46), 2.013 (0.50), 2.524 (1.06), 2.564 (1.20), 2.578 (1.49), 2.591 (0.77), 2.620 (0.48), 3.825 (16.00), 3.955 (0.41), 4.252 (0.44), 4.517 (0.94), 4.529 (1.44), 4.542 (0.94), 4.769 (4.35), 6.783 (1.53), 6.804 (1.66), 7.561 (1.13), 7.582 (1.11), 8.061 (1.73).

### Beispiel 434

### (5S)-5-[(3,3-Difluorpyrrolidin-1-yl)carbonyl]-2-[(6-methoxypyridin-3-yl)methyl]-5,6,7,8-tetrahydro[1,2,4]triazolo[4,3-a]pyridin-3(2H)-on

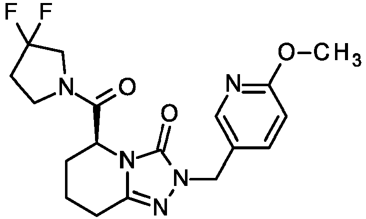

(5S)-2-[(6-Methoxypyridin-3-yl)methyl]-3-oxo-2,3,5,6,7,8-hexahydro[1,2,4]triazolo[4,3-a]pyridin-5-carbonsäure (390 mg, 1.28 mmol) wurde in THF (5.0 ml) vorgelegt und HBTU (632 mg, 1.67 mmol) und N,N-Diisopropylethylamin (670 µl, 3.8 mmol) wurden anschließend zugegeben. Nach 15 min Rühren bei Raumtemperatur wurde 3,3-Difluorpyrrolidinhydrochlorid (221 mg, 1.54 mmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 113 mg (22 % d. Th.) der Titelverbindung erhalten.
LC-MS (Methode 4): Rₜ = 0.64 min; MS (ESIpos): m/z = 394 [M+H]⁺
¹H-NMR (400 MHz, DMSO-d6) δ [ppm]: -0.008 (0.54), 0.008 (0.53), 1.688 (0.41), 1.711 (0.48), 1.970 (0.42), 1.978 (0.42), 1.988 (0.48), 1.996 (0.44), 2.003 (0.43), 2.571 (1.03), 2.587 (1.08), 2.602 (0.57), 3.535 (0.42), 3.544 (0.64), 3.555 (0.70), 3.663 (0.41), 3.697 (0.46), 3.776 (0.65), 3.805 (0.80), 3.826 (16.00), 3.906 (0.47), 4.728 (0.41), 4.737 (0.47), 4.743 (0.54), 4.752 (0.49), 4.766 (6.00), 4.802 (0.43), 4.812 (0.47), 4.817 (0.53), 6.784 (1.80), 6.806 (1.94), 7.553 (1.14), 7.559 (1.17), 7.574 (1.11), 7.580 (1.14), 8.052 (1.50), 8.058 (1.50).

### Beispiel 435

### (5S)-2-[(5-Chlorpyridin-3-yl)methyl]-5-[(3,3-difluorpyrrolidin-1-yl)carbonyl]-5,6,7,8-tetrahydro[1,2,4]triazolo[4,3-a]pyridin-3(2H)-on

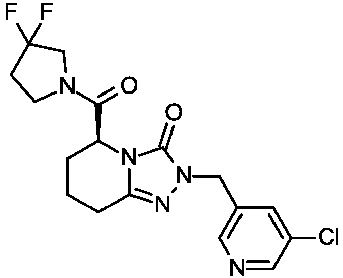

(5S)-2-[(5-Chlorpyridin-3-yl)methyl]-3-oxo-2,3,5,6,7,8-hexahydro[1,2,4]triazolo[4,3-a]pyridin-5-carbonsäure (80.3 mg, 260 µmol) wurde in THF (2.0 ml) vorgelegt und HBTU (128 mg, 338 µmol) und N,N-Diisopropylethylamin (140 µl, 780 µmol) wurden anschließend zugegeben. Nach 15 min Rühren bei Raumtemperatur wurde 3,3-Difluorpyrrolidinhydrochlorid (44.8 mg, 312 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 32.0 mg (31 % d. Th.) der Titelverbindung erhalten.
LC-MS (Methode 4): Rₜ = 0.64 min; MS (ESIpos): m/z = 398 [M+H]⁺
¹H-NMR (400 MHz, DMSO-d6) δ [ppm]: -0.149 (1.81), -0.008 (16.00), 0.008 (13.78), 0.146 (1.81), 1.672 (0.93), 1.733 (1.14), 1.882 (1.08), 1.975 (1.60), 2.072 (1.08), 2.327 (2.12), 2.366 (2.01), 2.572 (2.84), 2.583 (2.22), 2.598 (2.01), 2.616 (2.22), 2.669 (2.43), 2.709 (1.91), 3.550 (1.55), 3.561 (1.65), 3.670 (0.98), 3.704 (1.19), 3.744 (0.72), 3.777 (1.34), 3.809 (2.01), 3.893 (0.62), 3.911 (1.19), 3.937 (0.83), 3.990 (0.72), 4.033 (0.72), 4.182 (0.72), 4.204 (0.72), 4.765 (0.98), 4.780 (1.34), 4.852 (1.29), 4.917 (13.42), 7.757 (4.34), 8.421 (4.44), 8.562 (4.03), 8.568 (3.77).

### Beispiel 436

### (5S)-2-[(5-Chlorpyridin-3-yl)methyl]-5-[(3-fluorazetidin-1 -yl)carbonyl]-5,6,7,8-tetrahydro[1,2,4]triazolo[4,3-a]pyridin-3(2H)-on

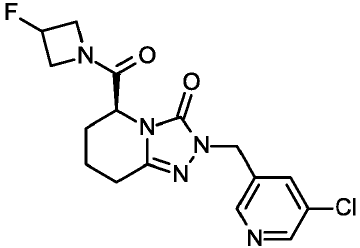

(5S)-2-[(5-Chlorpyridin-3-yl)methyl]-3-oxo-2,3,5,6,7,8-hexahydro[1,2,4]triazolo[4,3-a]pyridin-5-carbonsäure (80.3 mg, 260 µmol) wurde in THF (2.0 ml) vorgelegt und HBTU (128 mg, 338 µmol) und N,N-Diisopropylethylamin (140 µl, 780 µmol) wurden anschließend zugegeben. Nach 15 min Rühren bei Raumtemperatur wurde 3-Fluorazetidinhydrochlorid (34.8 mg, 312 µmol) zugegeben und das Reaktionsgemisch über Nacht bei Raumtemperatur gerührt. Es wurden erneut HBTU (49 mg, 130 µmol) und N,N-Diisopropylethylamin (23 µl, 125 µmol) zugegeben und über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 49.1 mg (52 % d. Th.) der Titelverbindung erhalten.
LC-MS (Methode 4): Rₜ = 0.57 min; MS (ESIpos): m/z = 366 [M+H]⁺
¹H-NMR (400 MHz, DMSO-d6) δ [ppm]: -0.008 (0.83), 0.008 (0.80), 1.651 (0.61), 1.665 (0.79), 1.675 (1.03), 1.690 (1.16), 1.700 (1.51), 1.714 (1.68), 1.723 (1.58), 1.909 (0.49), 1.953 (0.99), 1.963 (1.10), 1.972 (1.01), 1.986 (0.90), 1.995 (0.74), 2.012 (0.88), 2.022 (0.90), 2.032 (0.99), 2.057 (0.66), 2.074 (16.00), 2.520 (1.07), 2.524 (0.97), 2.566 (1.41), 2.573 (1.38), 2.591 (2.03), 2.605 (2.84), 2.618 (1.45), 2.634 (0.55), 2.646 (0.92), 2.660 (0.43), 2.690 (0.60), 3.932 (0.79), 3.962 (0.80), 3.994 (0.79), 4.023 (0.46), 4.215 (0.43), 4.228 (0.70), 4.243 (0.67), 4.257 (0.64), 4.274 (0.83), 4.297 (0.77), 4.327 (0.60), 4.366 (0.55), 4.400 (0.50), 4.461 (0.51), 4.524 (0.43), 4.556 (2.02), 4.568 (2.95), 4.581 (2.13), 4.921 (8.65), 5.192 (0.68), 5.347 (0.77), 5.354 (0.83), 5.402 (0.67), 5.410 (0.72), 5.490 (0.53), 5.497 (0.60), 5.545 (0.50), 5.552 (0.56), 7.775 (2.69), 8.432 (4.55), 8.572 (3.67), 8.577 (3.79).

### Beispiel 437

### (5S)-2-[(5-Chlorpyridin-3-yl)methyl]-5-[(3,3-difluorazetidin-1-yl)carbonyl]-5,6,7,8-tetrahydro[1,2,4]triazolo[4,3-a]pyridin-3(2H)-on

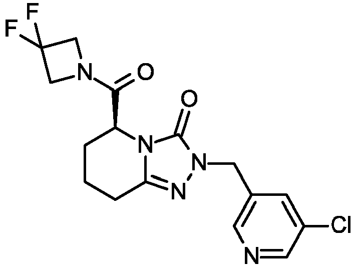

(5S)-2-[(5-Chlorpyridin-3-yl)methyl]-3-oxo-2,3,5,6,7,8-hexahydro[1,2,4]triazolo[4,3-a]pyridin-5-carbonsäure (80.3 mg, 260 µmol) wurde in THF (2.0 ml) vorgelegt und HBTU (128 mg, 338 µmol) und N,N-Diisopropylethylamin (140 µl, 780 µmol) wurden anschließend zugegeben. Nach 15 min Rühren bei Raumtemperatur wurde 3,3-Difluorazetidinhydrochlorid (40.4 mg, 312 µmol) zugegeben und das Reaktionsgemisch für 72 Stunden bei Raumtemperatur gerührt. Es wurden erneut HBTU (49 mg, 130 µmol) und N,N-Diisopropylethylamin (23 µl, 125 µmol) zugegeben und über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 46.8 mg (47 % d. Th.) der Titelverbindung erhalten.
LC-MS (Methode 3): Rₜ = 1.31 min; MS (ESIpos): m/z = 384 [M+H]⁺
¹H-NMR (400 MHz, DMSO-d6) δ [ppm]: -0.149 (0.59), -0.008 (5.47), 0.008 (4.69), 0.146 (0.60), 1.695 (1.59), 1.710 (2.35), 1.720 (2.72), 1.734 (1.99), 1.958 (0.72), 1.970 (0.76), 1.981 (1.13), 1.995 (1.53), 2.027 (0.83), 2.036 (1.05), 2.051 (1.54), 2.061 (1.18), 2.073 (1.96), 2.087 (0.63), 2.327 (0.67), 2.366 (0.54), 2.523 (2.30), 2.565 (1.83), 2.580 (2.04), 2.587 (2.00), 2.600 (2.59), 2.613 (3.34), 2.626 (1.60), 2.641 (0.58), 2.654 (0.92), 2.669 (1.03), 2.710 (0.51), 4.339 (1.04), 4.367 (1.75), 4.390 (1.76), 4.421 (1.05), 4.607 (2.34), 4.622 (3.64), 4.634 (2.33), 4.728 (1.10), 4.758 (1.26), 4.790 (0.59), 4.822 (0.57), 4.851 (1.25), 4.882 (1.12), 4.924 (16.00), 7.768 (5.18), 7.773 (3.13), 8.428 (5.44), 8.432 (5.42), 8.565 (4.90), 8.571 (4.84).

### Beispiel 438

### (5S)-2-[(5-Chlorpyridin-3-yl)methyl]-5-{[(3S)-3-fluorpyrrolidin-1-yl]carbonyl}-5,6,7,8-tetrahydro[1,2,4]triazolo[4,3-a]pyridin-3(2H)-on

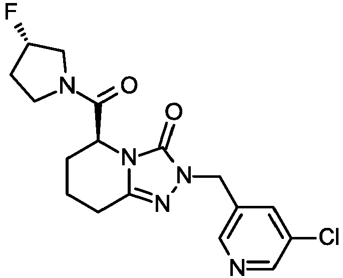

(5S)-2-[(5-Chlorpyridin-3-yl)methyl]-3-oxo-2,3,5,6,7,8-hexahydro[1,2,4]triazolo[4,3-a]pyridin-5-carbonsäure (80.3 mg, 260 µmol) wurde in THF (2.0 ml) vorgelegt und HBTU (128 mg, 338 µmol) und N,N-Diisopropylethylamin (140 µl, 780 µmol) wurden anschließend zugegeben. Nach 15 min Rühren bei Raumtemperatur wurde (3S)-3-Fluorpyrrolidinhydrochlorid (39.2 mg, 312 µmol) zugegeben und das Reaktionsgemisch über Nacht bei Raumtemperatur gerührt. Es wurden erneut HBTU (49 mg, 130 µmol) und N,N-Diisopropylethylamin (23 µl, 125 µmol) zugegeben und für 72 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 45.6 mg (46 % d. Th.) der Titelverbindung erhalten.
LC-MS (Methode 3): Rₜ = 1.16 min; MS (ESIpos): m/z = 380 [M+H]⁺
¹H-NMR (400 MHz, DMSO-d6) δ [ppm]: -0.008 (1.20), 0.008 (0.99), 1.177 (0.44), 1.410 (0.46), 1.700 (1.25), 1.711 (1.50), 1.725 (2.19), 1.734 (2.40), 1.877 (0.52), 1.911 (0.70), 1.997 (0.86), 2.029 (1.30), 2.051 (0.98), 2.067 (1.28), 2.078 (1.62), 2.088 (1.79), 2.103 (2.22), 2.138 (1.51), 2.221 (0.81), 2.242 (0.75), 2.270 (1.07), 2.524 (1.52), 2.566 (1.62), 2.570 (1.54), 2.576 (1.72), 2.592 (1.47), 2.619 (2.43), 2.661 (1.02), 2.670 (0.75), 2.891 (0.42), 3.274 (0.51), 3.293 (0.65), 3.303 (0.97), 3.321 (1.00), 3.331 (0.68), 3.349 (0.59), 3.362 (0.61), 3.371 (0.69), 3.398 (0.80), 3.406 (0.81), 3.460 (0.67), 3.468 (0.69), 3.495 (0.93), 3.504 (0.91), 3.613 (0.95), 3.637 (3.39), 3.655 (3.06), 3.660 (3.02), 3.681 (3.39), 3.705 (3.85), 3.723 (4.42), 3.743 (5.52), 3.770 (4.46), 3.778 (4.07), 3.816 (2.19), 3.855 (3.39), 4.695 (1.16), 4.704 (1.42), 4.710 (1.48), 4.719 (1.18), 4.752 (1.50), 4.761 (1.71), 4.768 (1.94), 4.776 (1.44), 4.915 (16.00), 5.260 (1.18), 5.384 (1.48), 5.391 (1.55), 5.512 (0.86), 7.756 (4.66), 7.761 (5.11), 8.422 (6.31), 8.564 (5.64), 8.569 (5.61).

### Beispiel 439

### (5S)-2-[(5-Chlorpyridin-3-yl)methyl]-5-{[(3R,4S)-3,4-difluorpyrrolidin-1-yl]carbonyl}-5,6,7,8-tetrahydro[1,2,4]triazolo[4,3-a]pyridin-3(2H)-on

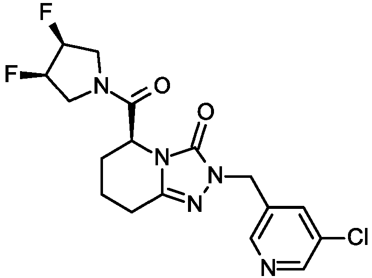

(5S)-2-[(5-Chlorpyridin-3-yl)methyl]-3-oxo-2,3,5,6,7,8-hexahydro[1,2,4]triazolo[4,3-a]pyridin-5-carbonsäure (80.3 mg, 260 µmol) wurde in THF (2.0 ml) vorgelegt und HBTU (128 mg, 338 µmol) und N,N-Diisopropylethylamin (140 µl, 780 µmol) wurden anschließend zugegeben. Nach 15 min Rühren bei Raumtemperatur wurde (3R,4S)-3,4-Difluorpyrrolidinhydrochlorid (44.8 mg, 312 µmol) zugegeben und das Reaktionsgemisch über Nacht bei Raumtemperatur gerührt. Es wurden erneut HBTU (49 mg, 130 µmol) und N,N-Diisopropylethylamin (23 µl, 125 µmol) zugegeben und für 72 Stunden bei Raumtemperatur gerührt. Es wurden erneut 1-[Bis(dimethylamino)methylen]-1H-benzotriazol-1-ium-3-oxidhexa-fluoro-phosphat (49 mg, 130 µmol), N,N-Diisopropylethylamin (23 µl, 125 µmol) und (3R,4S)-3,4-Difluorpyrrolidinhydrochlorid (19 mg, 130 µmol) zugegeben und über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 15.1 mg (15 % d. Th.) der Titelverbindung erhalten.
LC-MS (Methode 3): Rₜ = 1.18 min; MS (ESIpos): m/z = 398 [M+H]⁺
¹H-NMR (400 MHz, DMSO-d6) δ [ppm]: -0.149 (0.42), -0.008 (4.20), 0.008 (3.95), 0.146 (0.45), 1.659 (1.27), 1.685 (0.73), 1.732 (1.72), 1.938 (0.60), 1.972 (1.30), 1.994 (1.06), 2.014 (1.71), 2.038 (1.11), 2.049 (1.17), 2.056 (1.27), 2.064 (1.29), 2.073 (3.97), 2.084 (0.91), 2.091 (0.96), 2.099 (0.83), 2.327 (0.49), 2.366 (0.60), 2.523 (2.56), 2.561 (1.97), 2.565 (1.88), 2.576 (2.48), 2.586 (1.71), 2.591 (1.64), 2.600 (2.40), 2.614 (3.24), 2.626 (1.90), 2.645 (0.71), 2.656 (1.13), 2.669 (1.04), 2.709 (0.60), 2.829 (0.45), 2.849 (0.88), 2.867 (0.47), 3.456 (0.56), 3.481 (0.65), 3.490 (1.14), 3.501 (0.87), 3.524 (1.00), 3.536 (1.37), 3.545 (1.36), 3.555 (0.84), 3.568 (0.57), 3.578 (0.75), 3.588 (0.49), 3.618 (0.81), 3.631 (0.91), 3.650 (0.57), 3.664 (0.71), 3.673 (0.92), 3.687 (1.51), 3.704 (1.64), 3.714 (6.37), 3.725 (1.80), 3.741 (1.11), 3.756 (1.34), 3.768 (1.56), 3.789 (0.84), 3.802 (0.68), 3.871 (0.84), 3.890 (0.49), 3.913 (0.73), 3.925 (0.89), 3.939 (0.91), 3.954 (0.54), 3.975 (0.88), 3.989 (0.94), 4.004 (0.58), 4.018 (0.54), 4.133 (0.54), 4.148 (0.71), 4.161 (0.62), 4.175 (1.03), 4.188 (0.69), 4.201 (0.58), 4.216 (0.57), 4.801 (2.22), 4.813 (3.34), 4.823 (2.17), 4.917 (16.00), 5.255 (0.84), 5.266 (0.88), 5.276 (1.00), 5.287 (0.83), 5.298 (0.62), 5.312 (0.61), 5.330 (0.73), 5.338 (0.71), 5.351 (0.76), 5.366 (0.69), 5.379 (0.91), 5.389 (1.06), 5.399 (0.85), 5.408 (0.95), 5.417 (0.85), 5.429 (0.65), 5.442 (0.64), 5.451 (0.68), 5.458 (0.71), 5.473 (0.76), 5.481 (0.75), 5.495 (0.62), 6.826 (1.08), 6.847 (1.30), 7.099 (1.17), 7.121 (1.00), 7.753 (3.97), 7.758 (4.19), 8.419 (5.98), 8.564 (4.00).

### Beispiel 440

### (5S)-2-{[5-Chlor-6-(trifluormethyl)pyridin-3-yl]methyl}-5-{[(3R,4S)-3,4-difluorpyrrolidin-1-yl]carbonyl}-5,6,7,8-tetrahydro[1,2,4]triazolo[4,3-a]pyridin-3(2H)-on

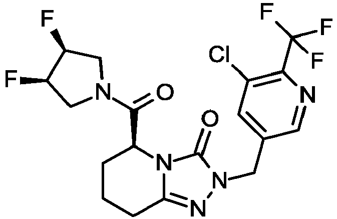

(5S)-2-{[5-Chlor-6-(trifluormethyl)pyridin-3-yl]methyl}-3-oxo-2,3,5,6,7,8-hexahydro[1,2,4]triazolo[4,3-a]pyridin-5-carbonsäure (35.0 mg, 92.9 µmol) wurde in THF (2.0 ml) vorgelegt und HBTU (45.8 mg, 121 µmol) und N,N-Diisopropylethylamin (49 µl, 280 µmol) wurden anschließend zugegeben. Nach 15 min Rühren bei Raumtemperatur wurde (3R,4S)-3,4-Difluorpyrrolidinhydrochlorid (16.0 mg, 111 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 25.0 mg (58 % d. Th.) der Titelverbindung erhalten.
LC-MS (Methode 3): Rₜ = 1.53 min; MS (ESIpos): m/z = 466 [M+H]⁺
¹H-NMR (400 MHz, DMSO-d6) δ [ppm]: -0.150 (1.49), -0.008 (13.42), 0.008 (10.58), 0.146 (1.49), 1.640 (1.49), 1.752 (1.90), 2.032 (2.58), 2.073 (2.31), 2.328 (4.34), 2.366 (2.98), 2.523 (12.20), 2.583 (2.85), 2.622 (3.39), 2.670 (4.88), 2.710 (3.12), 3.494 (1.63), 3.621 (1.22), 3.691 (2.17), 3.930 (1.08), 3.979 (1.08), 4.176 (1.22), 4.826 (3.80), 5.041 (16.00), 5.390 (1.36), 7.035 (9.63), 8.066 (6.10), 8.538 (8.14).

### Beispiel 441

### (5S)-2-{[5-Chlor-6-(trifluormethyl)pyridin-3-yl]methyl}-5-[(3,3-difluorpyrrolidin-1-yl)carbonyl]-5,6,7,8-tetrahydro[1,2,4]triazolo[4,3-a]pyridin-3(2H)-on

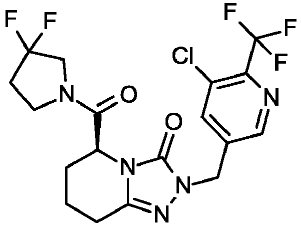

(5S)-2-{[5-Chlor-6-(trifluormethyl)pyridin-3-yl]methyl}-3-oxo-2,3,5,6,7,8-hexahydro[1,2,4]triazolo[4,3-a]pyridin-5-carbonsäure (35.0 mg, 92.9 µmol) wurde in THF (2.0 ml) vorgelegt und HBTU (45.8 mg, 121 µmol) und N,N-Diisopropylethylamin (49 µl, 280 µmol) wurden anschließend zugegeben. Nach 15 min Rühren bei Raumtemperatur wurde 3,3-Difluorpyrrolidinhydrochlorid (16.0 mg, 111 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 27.0 mg (62 % d. Th.) der Titelverbindung erhalten.
LC-MS (Methode 3): Rₜ = 1.60 min; MS (ESIpos): m/z = 466 [M+H]⁺
¹H-NMR (400 MHz, DMSO-d6) δ [ppm]: -0.149 (1.15), -0.008 (10.01), 0.008 (8.29), 0.146 (1.04), 1.656 (1.27), 1.746 (1.61), 2.020 (2.07), 2.327 (2.65), 2.366 (3.57), 2.383 (1.15), 2.411 (1.50), 2.432 (1.50), 2.452 (1.38), 2.523 (11.28), 2.564 (4.14), 2.579 (3.22), 2.590 (3.68), 2.604 (2.99), 2.623 (3.34), 2.665 (3.11), 2.669 (3.34), 2.709 (2.99), 3.542 (1.84), 3.563 (2.65), 3.573 (1.50), 3.582 (1.38), 3.676 (1.61), 3.709 (1.73), 3.745 (1.38), 3.778 (1.96), 3.813 (2.99), 3.831 (0.81), 3.894 (0.92), 3.912 (1.73), 3.939 (1.15), 3.996 (1.15), 4.038 (1.04), 4.067 (0.69), 4.154 (0.58), 4.186 (0.92), 4.210 (0.92), 4.242 (0.46), 4.781 (1.38), 4.795 (1.96), 4.805 (1.38), 4.849 (1.50), 4.864 (1.96), 4.873 (1.38), 5.040 (16.00), 8.068 (5.99), 8.538 (5.87).

### Beispiel 442

### (5S)-2-[(5-Chlor-6-methoxypyridin-3-yl)methyl]-5-{[(3R,4S)-3,4-difluorpyrrolidin-1-yl]carbonyl}-5,6,7,8-tetrahydro[1,2,4]triazolo[4,3-a]pyridin-3(2H)-on

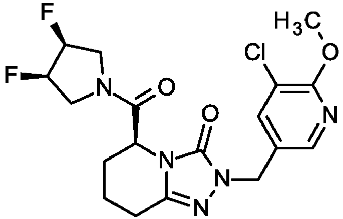

(5S)-2-[(5-Chlor-6-methoxypyridin-3-yl)methyl]-3-oxo-2,3,5,6,7,8-hexahydro[1,2,4]triazolo[4,3-a]pyridin-5-carbonsäure (80.0 mg, 236 µmol) wurde in THF (2.0 ml) vorgelegt und HBTU (116 mg, 307 µmol) und N,N-Diisopropylethylamin (120 µl, 710 µmol) wurden anschließend zugegeben. Nach 15 min Rühren bei Raumtemperatur wurde (3R,4S)-3,4-Difluorpyrrolidinhydrochlorid (40.7 mg, 283 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 37.0 mg (37 % d. Th.) der Titelverbindung erhalten.
LC-MS (Methode 4): Rₜ = 0.72 min; MS (ESIpos): m/z = 428 [M+H]⁺
¹H-NMR (400 MHz, DMSO-d6) δ [ppm]: -0.008 (2.46), 0.008 (2.19), 1.654 (0.57), 1.723 (0.70), 1.956 (0.53), 2.001 (0.70), 2.024 (0.45), 2.035 (0.49), 2.043 (0.55), 2.051 (0.51), 2.060 (0.43), 2.073 (2.65), 2.562 (1.13), 2.572 (0.79), 2.586 (1.04), 2.599 (1.36), 2.611 (0.79), 2.642 (0.49), 2.670 (0.45), 3.486 (0.49), 3.523 (0.43), 3.532 (0.57), 3.541 (0.57), 3.626 (0.43), 3.668 (0.43), 3.682 (0.62), 3.700 (0.74), 3.721 (0.62), 3.734 (0.42), 3.751 (0.49), 3.765 (0.51), 3.924 (14.47), 3.926 (16.00), 3.972 (0.42), 3.985 (0.43), 4.171 (0.45), 4.792 (1.87), 4.804 (8.38), 5.254 (0.42), 5.266 (0.43), 5.276 (0.47), 5.375 (0.45), 5.388 (0.51), 5.399 (0.42), 5.407 (0.40), 7.739 (1.63), 7.745 (2.99), 7.750 (1.89), 8.015 (1.65), 8.021 (3.08), 8.026 (1.76).

### Beispiel 443

### (5S)-2-[(5-Chlor-6-methoxypyridin-3-yl)methyl]-5-[(3,3-difluorpyrrolidin-1-yl)carbonyl]-5,6,7,8-tetrahydro[1,2,4]triazolo[4,3-a]pyridin-3(2H)-on

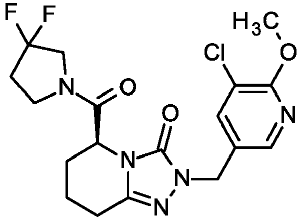

(5S)-2-[(5-Chlor-6-methoxypyridin-3-yl)methyl]-3-oxo-2,3,5,6,7,8-hexahydro[1,2,4]triazolo[4,3-a]pyridin-5-carbonsäure (80.0 mg, 236 µmol) wurde in THF (2.0 ml) vorgelegt und HBTU (116 mg, 307 µmol) und N,N-Diisopropylethylamin (120 µl, 710 µmol) wurden anschließend zugegeben. Nach 15 min Rühren bei Raumtemperatur wurde 3,3-Difluorpyrrolidinhydrochlorid (40.7 mg, 283 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 35.0 mg (35 % d. Th.) der Titelverbindung erhalten.
LC-MS (Methode 3): Rₜ = 1.41 min; MS (ESIpos): m/z = 428 [M+H]⁺
¹H-NMR (400 MHz, DMSO-d6) δ [ppm]: -0.008 (2.15), 0.008 (1.91), 1.722 (0.45), 1.972 (0.71), 1.996 (0.50), 2.073 (0.45), 2.328 (0.43), 2.524 (1.63), 2.569 (1.20), 2.586 (1.03), 2.601 (1.03), 2.670 (0.49), 3.528 (0.41), 3.537 (0.45), 3.546 (0.65), 3.557 (0.73), 3.667 (0.43), 3.700 (0.49), 3.775 (0.64), 3.805 (0.84), 3.909 (0.58), 3.925 (16.00), 4.759 (0.54), 4.769 (0.41), 4.805 (6.06), 4.832 (0.60), 4.842 (0.49), 7.744 (2.13), 7.749 (2.24), 8.020 (1.96), 8.025 (1.94).

### Beispiel 444

### (5S)-5-{[(3S)-3-Fluorpyrrolidin-1-yl]carbonyl}-2-[3-(trifluormethyl)benzyl]-5,6,7,8-tetrahydro[1,2,4]triazolo[4,3-a]pyridin-3(2H)-on

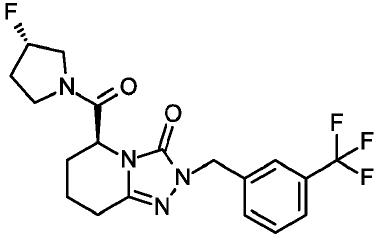

(5S)-3-Oxo-2-[3-(trifluormethyl)benzyl]-2,3,5,6,7,8-hexahydro[1,2,4]triazolo[4,3-a]pyridin-5-carbonsäure (70.0 mg, 205 µmol) wurde in THF (3.0 ml) vorgelegt und HATU (101 mg, 267 µmol) und Triethylamin (86 µl, 620 µmol) wurden anschließend zugegeben. Nach 15 min Rühren bei Raumtemperatur wurde (3S)-3-Fluorpyrrolidinhydrochlorid (30.9 mg, 246 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 36.9 mg (44 % d. Th.) der Titelverbindung erhalten.
LC-MS (Methode 3): Rₜ = 1.49 min; MS (ESIpos): m/z = 413 [M+H]⁺
¹H-NMR (400 MHz, DMSO-d6) δ [ppm]: -0.008 (1.84), 0.008 (1.64), 1.163 (0.63), 1.633 (0.62), 1.642 (0.77), 1.655 (0.92), 1.667 (1.04), 1.681 (0.99), 1.707 (1.53), 1.719 (2.14), 1.731 (2.70), 1.741 (2.21), 1.909 (0.49), 1.968 (0.74), 1.997 (2.63), 2.027 (1.41), 2.037 (1.44), 2.049 (1.05), 2.065 (1.42), 2.072 (3.24), 2.080 (1.67), 2.089 (1.84), 2.101 (2.01), 2.137 (1.51), 2.174 (0.50), 2.220 (0.91), 2.257 (1.10), 2.269 (1.15), 2.327 (0.55), 2.366 (0.43), 2.523 (2.43), 2.567 (2.31), 2.584 (1.52), 2.597 (1.92), 2.609 (3.44), 2.621 (2.07), 2.639 (0.82), 2.650 (1.26), 2.665 (0.98), 2.710 (0.48), 3.274 (0.47), 3.352 (0.69), 3.363 (1.15), 3.381 (0.87), 3.393 (0.71), 3.407 (0.80), 3.468 (0.52), 3.494 (0.59), 3.505 (1.14), 3.524 (1.79), 3.552 (1.39), 3.570 (1.09), 3.596 (2.27), 3.613 (0.45), 3.638 (1.50), 3.655 (1.92), 3.681 (1.22), 3.696 (0.75), 3.724 (0.78), 3.744 (1.50), 3.772 (1.20), 3.784 (1.18), 3.855 (1.53), 3.922 (0.58), 3.946 (1.15), 3.969 (0.53), 3.983 (0.47), 4.011 (0.55), 4.043 (0.44), 4.698 (0.75), 4.706 (0.90), 4.713 (0.96), 4.722 (0.74), 4.754 (0.99), 4.763 (1.08), 4.770 (1.21), 4.779 (0.91), 4.824 (0.79), 4.838 (1.28), 4.848 (0.82), 4.861 (0.92), 4.869 (1.02), 4.876 (1.13), 4.885 (1.03), 4.930 (16.00), 5.261 (1.04), 5.270 (1.03), 5.348 (0.69), 5.393 (1.16), 5.481 (0.68), 5.512 (0.56), 7.508 (2.41), 7.527 (4.04), 7.572 (2.77), 7.591 (5.41), 7.620 (5.71), 7.648 (4.73), 7.667 (2.61).

### Beispiel 445

### (5S)-5-{[(3R,4S)-3,4-Difluorpyrrolidin-1-yl]carbonyl}-2-[3-(trifluormethyl)benzyl]-5,6,7,8-tetrahydro[1,2,4]triazolo[4,3-a]pyridin-3(2H)-on

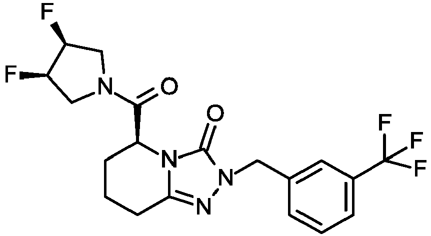

(5S)-3-Oxo-2-[3-(trifluormethyl)benzyl]-2,3,5,6,7,8-hexahydro[1,2,4]triazolo[4,3-a]pyridin-5-carbonsäure (60.0 mg, 176 µmol) wurde in THF (3.0 ml) vorgelegt und HBTU (86.7 mg, 229 µmol) und N,N-Diisopropylethylamin (92 µl, 530 µmol) wurden anschließend zugegeben. Nach 15 min Rühren bei Raumtemperatur wurde (3R,4S)-3,4-Difluorpyrrolidinhydrochlorid (30.3 mg, 211 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 41.9 mg (55 % d. Th.) der Titelverbindung erhalten.
LC-MS (Methode 4): Rₜ = 0.85 min; MS (ESIpos): m/z = 431 [M+H]⁺
¹H-NMR (400 MHz, DMSO-d6) δ [ppm]: -0.149 (0.53), -0.008 (5.75), 0.008 (4.69), 0.146 (0.53), 1.244 (0.79), 1.259 (0.86), 1.274 (0.53), 1.667 (1.25), 1.731 (1.52), 1.937 (0.51), 1.971 (1.17), 2.012 (1.57), 2.036 (1.01), 2.054 (1.16), 2.063 (1.11), 2.073 (2.67), 2.090 (0.92), 2.327 (0.74), 2.366 (0.63), 2.523 (3.53), 2.570 (2.56), 2.580 (1.83), 2.594 (2.36), 2.608 (3.14), 2.620 (1.85), 2.651 (1.14), 2.665 (1.16), 2.669 (1.07), 2.710 (0.78), 3.033 (0.43), 3.403 (0.58), 3.455 (0.63), 3.490 (1.27), 3.499 (0.92), 3.512 (1.11), 3.521 (1.12), 3.532 (0.99), 3.544 (1.06), 3.565 (0.56), 3.574 (0.79), 3.616 (0.86), 3.630 (0.96), 3.649 (0.56), 3.671 (0.94), 3.685 (1.75), 3.699 (1.24), 3.719 (1.37), 3.732 (1.22), 3.751 (0.99), 3.762 (1.02), 3.785 (0.59), 3.797 (0.51), 3.871 (0.81), 3.926 (0.79), 3.941 (0.89), 3.955 (0.56), 3.976 (0.89), 3.990 (0.88), 4.005 (0.53), 4.018 (0.48), 4.134 (0.54), 4.149 (0.61), 4.162 (0.59), 4.176 (0.99), 4.190 (0.68), 4.202 (0.63), 4.218 (0.58), 4.803 (2.28), 4.816 (3.22), 4.825 (2.15), 4.930 (16.00), 5.266 (0.86), 5.277 (0.99), 5.298 (0.64), 5.330 (0.73), 5.339 (0.71), 5.352 (0.79), 5.366 (0.71), 5.376 (0.92), 5.390 (1.06), 5.400 (0.86), 5.409 (0.94), 5.443 (0.63), 5.473 (0.73), 5.482 (0.76), 5.495 (0.59), 7.505 (1.73), 7.524 (2.86), 7.572 (1.90), 7.591 (3.72), 7.614 (6.46), 7.648 (3.85), 7.668 (2.18).

### Beispiel 446

### (5S)-5-[(3,3-Difluorpyrrolidin-1-yl)carbonyl]-2-[3-(trifluormethyl)benzyl]-5,6,7,8-tetrahydro[1,2,4]triazolo[4,3-a]pyridin-3(2H)-on

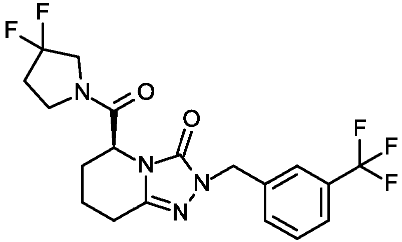

(5S)-3-Oxo-2-[3-(trifluormethyl)benzyl]-2,3,5,6,7,8-hexahydro[1,2,4]triazolo[4,3-a]pyridin-5-carbonsäure (60.0 mg, 176 µmol) wurde in THF (3.0 ml) vorgelegt und HBTU (86.7 mg, 229 µmol) und N,N-Diisopropylethylamin (92 µl, 530 µmol) wurden anschließend zugegeben. Nach 15 min Rühren bei Raumtemperatur wurde 3,3-Difluorpyrrolidinhydrochlorid (30.3 mg, 211 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 38.0 mg (50 % d. Th.) der Titelverbindung erhalten.
LC-MS (Methode 3): Rₜ = 1.62 min; MS (ESIpos): m/z = 431 [M+H]⁺
¹H-NMR (400 MHz, DMSO-d6) δ [ppm]: -0.008 (3.35), 0.008 (3.36), 0.146 (0.40), 1.672 (1.05), 1.681 (0.96), 1.695 (0.89), 1.720 (1.32), 1.731 (1.28), 1.872 (0.59), 1.972 (1.05), 1.986 (1.17), 2.001 (1.31), 2.013 (1.47), 2.041 (1.03), 2.049 (1.03), 2.057 (1.00), 2.066 (0.92), 2.072 (0.98), 2.085 (0.76), 2.092 (0.66), 2.327 (0.56), 2.366 (0.78), 2.381 (0.85), 2.410 (1.01), 2.430 (0.96), 2.452 (0.79), 2.523 (2.83), 2.576 (2.41), 2.591 (2.53), 2.608 (2.78), 2.620 (1.36), 2.640 (0.54), 2.652 (0.84), 2.665 (0.79), 2.710 (0.57), 3.537 (1.29), 3.549 (1.82), 3.557 (1.97), 3.568 (1.11), 3.576 (1.01), 3.648 (1.56), 3.670 (1.23), 3.703 (1.36), 3.738 (1.12), 3.772 (1.54), 3.783 (1.03), 3.791 (0.84), 3.808 (1.83), 3.828 (0.65), 3.839 (0.48), 3.894 (0.62), 3.913 (1.56), 3.932 (0.75), 3.939 (0.96), 3.958 (0.61), 3.991 (0.82), 4.005 (0.42), 4.019 (0.56), 4.033 (0.75), 4.061 (0.45), 4.149 (0.46), 4.181 (0.73), 4.208 (0.71), 4.767 (1.12), 4.776 (1.31), 4.782 (1.45), 4.792 (1.06), 4.840 (1.14), 4.850 (1.29), 4.855 (1.47), 4.865 (1.09), 4.931 (16.00), 7.506 (2.20), 7.525 (3.60), 7.572 (2.11), 7.591 (4.15), 7.616 (5.26), 7.648 (3.33), 7.668 (1.87).

### Beispiel 447

### (5S)-5-[(3-Fluorazetidin-1 -yl)carbonyl]-2-[3-(trifluormethyl)benzyl]-5,6,7,8-tetrahydro[1,2,4]triazolo[4,3-a]pyridin-3(2H)-on

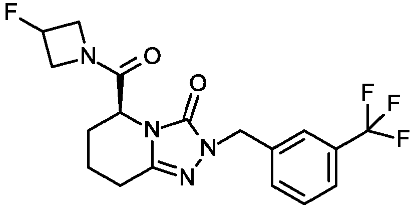

(5S)-3-Oxo-2-[3-(trifluormethyl)benzyl]-2,3,5,6,7,8-hexahydro[1,2,4]triazolo[4,3-a]pyridin-5-carbonsäure (60.0 mg, 176 µmol) wurde in THF (3.0 ml) vorgelegt und HBTU (86.7 mg, 229 µmol) und N,N-Diisopropylethylamin (92 µl, 530 µmol) wurden anschließend zugegeben. Nach 15 min Rühren bei Raumtemperatur wurde 3-Fluorazetidinhydrochlorid (23.5 mg, 211 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 30.5 mg (44 % d. Th.) der Titelverbindung erhalten.
LC-MS (Methode 4): Rₜ = 0.80 min; MS (ESIpos): m/z = 399 [M+H]⁺
¹H-NMR (400 MHz, DMSO-d6) δ [ppm]: -0.149 (1.79), -0.008 (16.00), 0.008 (13.70), 0.146 (1.87), 1.708 (2.94), 1.961 (1.58), 2.327 (2.73), 2.366 (2.51), 2.523 (9.47), 2.584 (3.52), 2.599 (4.52), 2.613 (2.44), 2.641 (1.65), 2.669 (3.16), 2.709 (2.80), 3.928 (1.15), 3.959 (1.22), 3.990 (1.22), 4.266 (1.29), 4.290 (1.22), 4.463 (0.72), 4.554 (2.87), 4.566 (4.23), 4.931 (12.77), 5.353 (0.79), 5.495 (0.79), 7.511 (2.44), 7.529 (4.23), 7.572 (2.22), 7.590 (4.30), 7.621 (5.88), 7.649 (4.66), 7.669 (2.65).

### Beispiel 448

### (5S)-5-{[(3S)-3-Fluorpyrrolidin-1-yl]carbonyl}-2-[4-fluor-3-(trifluormethyl)benzyl]-5,6,7,8-tetrahydro[1,2,4]triazolo[4,3-a]pyridin-3(2H)-on

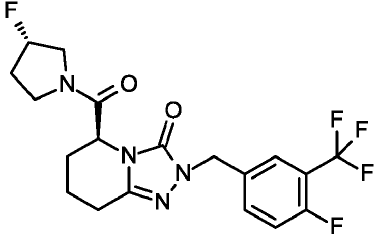

(5S)-2-[4-Fluor-3-(trifluormethyl)benzyl]-3-oxo-2,3,5,6,7,8-hexahydro[1,2,4]triazolo[4,3-a]pyridin-5-carbonsäure (61.0 mg, 170 µmol) wurde in THF (2.1 ml) bei Raumtemperatur vorgelegt. Anschließend wurden HATU (83.9 mg, 221 µmol) und Triethylamin (71 µl, 510 µmol) zugegeben. Nach 15 min Rühren wurde (3S)-3-Fluorpyrrolidinhydrochlorid (25.6 mg, 204 µmol) zugegeben und das Reaktionsgemisch für 72 Stunden bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 38.0 mg (52 % d. Th.) der Titelverbindung erhalten.
LC-MS (Methode 3): Rₜ = 1.53 min; MS (ESIpos): m/z = 431 [M+H]⁺
¹H-NMR (400 MHz, DMSO-d6) δ [ppm]: -0.150 (1.93), -0.008 (16.00), 0.008 (15.16), 0.146 (2.01), 1.727 (1.51), 2.077 (1.26), 2.327 (5.70), 2.366 (3.43), 2.523 (15.58), 2.606 (1.76), 2.669 (5.78), 2.710 (2.85), 3.652 (1.09), 3.852 (0.84), 4.908 (7.71), 5.400 (0.84), 7.481 (1.26), 7.502 (2.18), 7.528 (1.93), 7.577 (1.42), 7.685 (1.68).

### Beispiel 449

### (5S)-2-[4-Fluor-3-(trifluormethyl)benzyl]-5-[(3-hydroxyazetidin-1-yl)carbonyl]-5,6,7,8-tetrahydro[1,2,4]triazolo[4,3-a]pyridin-3(2H)-on

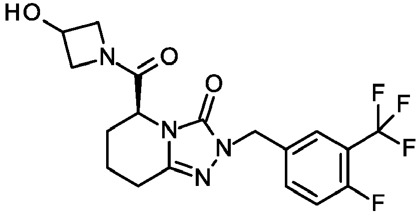

(5S)-2-[4-Fluor-3-(trifluormethyl)benzyl]-3-oxo-2,3,5,6,7,8-hexahydro[1,2,4]triazolo[4,3-a]pyridin-5-carbonsäure (60.0 mg, 167 µmol) wurde in THF bei Raumtemperatur vorgelegt. Anschließend wurden HBTU (82.3 mg, 217 µmol) und N,N-Diisopropylethylamin (87 µl, 500 µmol) zugegeben. Nach 15 min Rühren wurde Azetidin-3-olhydrochlorid (22.0 mg, 200 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 32.0 mg (46 % d. Th.) der Titelverbindung erhalten.
LC-MS (Methode 3): Rₜ = 1.29 min; MS (ESIpos): m/z = 415 [M+H]⁺
¹H-NMR (400 MHz, DMSO-d6) δ [ppm]: -0.008 (2.18), 0.008 (1.77), 1.662 (1.86), 1.710 (2.59), 1.719 (2.65), 1.732 (2.18), 1.906 (1.39), 1.919 (1.36), 1.931 (2.08), 1.942 (3.09), 1.954 (2.81), 1.964 (1.67), 1.993 (1.96), 2.003 (2.15), 2.012 (1.89), 2.029 (1.51), 2.328 (0.66), 2.367 (0.50), 2.519 (3.50), 2.524 (3.94), 2.566 (2.08), 2.580 (2.81), 2.592 (5.24), 2.605 (2.75), 2.621 (1.23), 2.634 (1.93), 2.646 (0.85), 2.670 (0.73), 2.675 (0.57), 2.710 (0.54), 3.596 (1.61), 3.603 (1.89), 3.621 (2.05), 3.630 (2.65), 3.646 (1.86), 3.660 (1.74), 3.671 (1.74), 3.920 (2.05), 3.932 (2.34), 3.952 (0.57), 4.021 (3.63), 4.045 (3.85), 4.054 (2.08), 4.062 (1.93), 4.101 (1.39), 4.116 (1.74), 4.127 (1.45), 4.142 (1.33), 4.339 (1.45), 4.357 (2.43), 4.378 (1.67), 4.485 (2.62), 4.502 (5.08), 4.515 (6.28), 4.526 (6.94), 4.537 (5.24), 4.551 (2.21), 4.905 (16.00), 4.910 (15.78), 5.791 (2.78), 5.803 (6.75), 5.817 (3.47), 7.476 (1.86), 7.501 (4.51), 7.525 (3.22), 7.565 (2.97), 7.570 (3.16), 7.577 (3.50), 7.598 (1.80), 7.674 (4.45), 7.691 (4.45).

### Beispiel 450

### (5S)-5-{[(3S)-3-Fluorpyrrolidin-1-yl]carbonyl}-2-{[2-(trifluormethyl)pyridin-4-yl]methyl}-5,6,7,8-tetrahydro[1,2,4]triazolo[4,3-a]pyridin-3(2H)-on

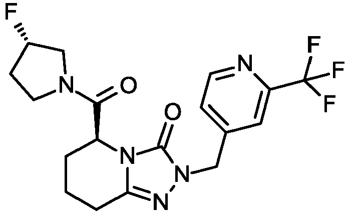

(5S)-3-Oxo-2-{[2-(trifluormethyl)pyridin-4-yl]methyl}-2,3,5,6,7,8-hexahydro[1,2,4]triazolo[4,3-a]pyridin-5-carbonsäure (100 mg, 292 µmol) wurde in DMF (3.3 ml) bei Raumtemperatur vorgelegt. Anschließend wurden HBTU (144 mg, 380 µmol) und N,N-Diisopropylethylamin (150 µl, 880 µmol) zugegeben. Nach 15 min Rühren wurde (3S)-3-Fluorpyrrolidinhydrochlorid (44.0 mg, 351 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 65.8 mg (54 % d. Th.) der Titelverbindung erhalten.
LC-MS (Methode 3): Rₜ = 1.18 min; MS (ESIpos): m/z = 414 [M+H]⁺
¹H-NMR (400 MHz, DMSO-d6) δ [ppm]: -0.149 (1.51), -0.008 (16.00), 0.008 (10.48), 0.146 (1.36), 1.751 (1.65), 1.952 (0.65), 2.104 (1.79), 2.271 (1.00), 2.323 (3.01), 2.328 (3.80), 2.366 (3.16), 2.518 (15.50), 2.523 (12.84), 2.565 (1.79), 2.580 (1.29), 2.607 (1.15), 2.634 (2.01), 2.670 (4.16), 2.710 (2.94), 3.413 (0.72), 3.474 (0.43), 3.502 (0.65), 3.637 (1.79), 3.654 (1.72), 3.679 (1.22), 3.703 (0.79), 3.753 (1.29), 3.777 (1.22), 3.790 (0.93), 3.857 (1.87), 4.722 (0.93), 4.731 (1.22), 4.780 (1.15), 4.795 (1.51), 5.027 (12.27), 5.261 (1.00), 5.383 (1.15), 5.512 (0.72), 7.486 (2.44), 7.752 (5.31), 8.729 (3.87), 8.741 (3.95).

### Beispiel 451

### (5S)-5-[(3,3-Difluorpyrrolidin-1-yl)carbonyl]-2-(4-fluorbenzyl)-5,6,7,8-tetrahydro[1,2,4]triazolo[4,3-a]pyridin-3(2H)-on

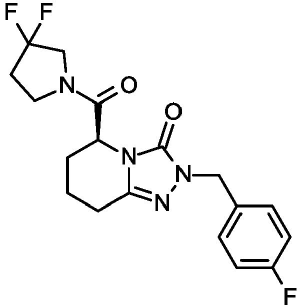

(5S)-2-(4-Fluorbenzyl)-3-oxo-2,3,5,6,7,8-hexahydro[1,2,4]triazolo[4,3-a]pyridin-5-carbonsäure (62.8 mg, 215 µmol) wurde in THF (2.0 ml, 25 mmol) vorgelegt und HBTU (106 mg, 280 µmol) und N,N-Diisopropylethylamin (110 µl, 650 µmol) wurden anschließend zugegeben. Nach 15 min Rühren bei Raumtemperatur wurde 3,3-Difluorpyrrolidinhydrochlorid (37.1 mg, 259 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 34.6 mg (42 % d. Th.) der Titelverbindung erhalten.
LC-MS (Methode 3): Rₜ = 1.38 min; MS (ESIpos): m/z = 381 [M+H]⁺
¹H-NMR (400 MHz, DMSO-d6) δ [ppm]: -0.149 (1.79), -0.008 (16.00), 0.008 (12.60), 0.146 (1.70), 1.710 (1.61), 1.995 (1.52), 2.042 (1.07), 2.327 (1.97), 2.366 (1.70), 2.380 (0.98), 2.410 (1.16), 2.565 (3.40), 2.580 (2.86), 2.597 (2.86), 2.638 (1.07), 2.670 (2.23), 2.709 (1.61), 3.536 (1.34), 3.547 (2.06), 3.557 (2.15), 3.575 (1.07), 3.665 (1.25), 3.700 (1.43), 3.743 (0.72), 3.775 (1.70), 3.807 (2.59), 3.889 (0.72), 3.907 (1.52), 3.934 (1.07), 3.991 (0.89), 4.035 (0.80), 4.063 (0.54), 4.172 (0.80), 4.198 (0.80), 4.738 (1.16), 4.747 (1.43), 4.798 (15.28), 4.828 (1.61), 4.838 (1.43), 7.141 (4.11), 7.163 (9.56), 7.185 (6.08), 7.256 (5.27), 7.270 (5.90), 7.277 (4.38), 7.291 (3.66).

### Beispiel 452

### (5S)-5-{[(3R,4S)-3,4-Difluorpyrrolidin-1-yl]carbonyl}-2-(4-fluorbenzyl)-5,6,7,8-tetrahydro[1,2,4]triazolo[4,3-a]pyridin-3(2H)-on

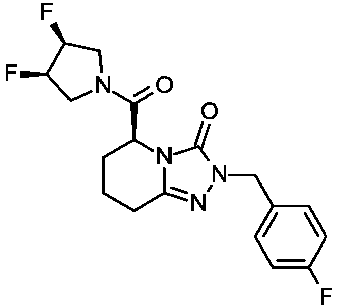

(5S)-2-(4-Fluorbenzyl)-3-oxo-2,3,5,6,7,8-hexahydro[1,2,4]triazolo[4,3-a]pyridin-5-carbonsäure (62.8 mg, 215 µmol) wurde in THF (2.0 ml) vorgelegt und HBTU (106 mg, 280 µmol) und N,N-Diisopropylethylamin (110 µl, 650 µmol) wurden anschließend zugegeben. Nach 15 min Rühren bei Raumtemperatur wurde (3R,4S)-3,4-Difluorpyrrolidinhydrochlorid (37.1 mg, 259 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 63.3 mg (77% d. Th.) der Titelverbindung erhalten.
LC-MS (Methode 3): Rₜ = 1.30 min; MS (ESIpos): m/z = 381 [M+H]⁺
¹H-NMR (400 MHz, DMSO-d6) delta [ppm]: -0.149 (1.91), -0.008 (16.00), 0.008 (12.70), 0.146 (1.74), 1.722 (1.04), 2.002 (1.04), 2.072 (2.43), 2.327 (2.61), 2.366 (1.91), 2.523 (7.65), 2.582 (1.91), 2.595 (2.43), 2.638 (0.87), 2.670 (2.78), 2.710 (1.91), 3.533 (1.04), 3.576 (0.70), 3.626 (0.70), 3.669 (0.70), 3.681 (1.04), 3.701 (1.22), 3.724 (0.87), 3.865 (0.70), 3.940 (0.70), 3.973 (0.70), 3.987 (0.70), 4.171 (0.70), 4.796 (13.22), 5.273 (0.70), 5.388 (0.87), 7.142 (2.43), 7.164 (5.39), 7.183 (3.30), 7.251 (2.26), 7.258 (2.96), 7.264 (3.30), 7.272 (4.35), 7.279 (2.43), 7.287 (1.91), 7.293 (1.74).

### Beispiel 453

### (5S)-5-{[(3R,4S)-3,4-Difluorpyrrolidin-1-yl]carbonyl}-2-(4-methoxybenzyl)-5,6,7,8-tetrahydro[1,2,4]triazolo[4,3-a]pyridin-3(2H)-on

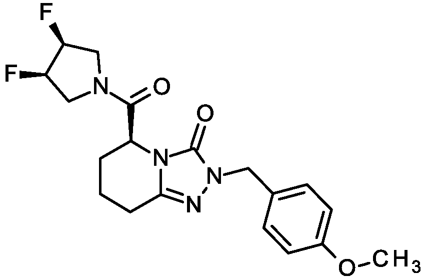

(5S)-2-(4-Methoxybenzyl)-3-oxo-2,3,5,6,7,8-hexahydro[1,2,4]triazolo[4,3-a]pyridin-5-carbonsäure (258 mg, 50 % Reinheit, 425 µmol) wurde in THF (5.0 ml) vorgelegt und HBTU (210 mg, 553 µmol) und N,N-Diisopropylethylamin (220 µl, 1.3 mmol) wurden anschließend zugegeben. Nach 15 min Rühren bei Raumtemperatur wurde (3R,4S)-3,4-Difluorpyrrolidinhydrochlorid (73.3 mg, 510 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 45.0 mg (27 % d. Th.) der Titelverbindung erhalten.
LC-MS (Methode 4): Rₜ = 0.70 min; MS (ESIpos): m/z = 393 [M+H]⁺
¹H-NMR (400 MHz, DMSO-d6) δ [ppm]: -0.008 (1.34), 0.008 (1.31), 1.251 (0.81), 1.261 (0.45), 1.268 (0.91), 1.274 (0.75), 1.291 (0.69), 1.642 (1.22), 1.666 (1.72), 1.676 (1.72), 1.692 (1.93), 1.704 (2.38), 1.714 (2.23), 1.728 (1.80), 1.909 (1.09), 1.927 (0.81), 1.944 (1.55), 1.970 (0.99), 1.980 (1.58), 1.993 (1.98), 2.001 (1.45), 2.018 (1.24), 2.028 (1.43), 2.036 (1.54), 2.043 (1.48), 2.052 (1.30), 2.063 (1.13), 2.071 (1.21), 2.079 (1.07), 2.088 (0.60), 2.519 (2.90), 2.570 (2.85), 2.583 (3.73), 2.595 (2.10), 2.615 (0.77), 2.626 (1.27), 2.637 (0.61), 2.689 (1.56), 3.441 (0.48), 3.450 (0.85), 3.459 (0.56), 3.483 (1.47), 3.493 (1.43), 3.505 (1.30), 3.514 (1.58), 3.567 (7.32), 3.576 (7.76), 3.586 (7.42), 3.612 (8.16), 3.625 (7.94), 3.644 (6.37), 3.666 (4.04), 3.681 (3.25), 3.688 (2.58), 3.701 (2.92), 3.714 (2.10), 3.754 (1.71), 3.766 (1.50), 3.787 (0.85), 3.800 (0.81), 3.816 (0.49), 3.829 (0.56), 3.835 (0.54), 3.845 (0.73), 3.858 (0.89), 3.864 (1.10), 3.883 (0.53), 3.893 (0.71), 3.906 (1.04), 3.925 (1.06), 3.938 (1.20), 3.953 (0.74), 3.967 (0.87), 3.974 (1.15), 3.988 (1.16), 4.002 (0.75), 4.017 (0.68), 4.130 (0.73), 4.144 (0.83), 4.158 (0.79), 4.171 (1.38), 4.186 (0.90), 4.198 (0.79), 4.214 (0.73), 4.679 (1.11), 4.719 (16.00), 4.727 (8.38), 4.766 (3.94), 4.772 (4.71), 4.777 (3.27), 5.244 (0.73), 5.254 (1.15), 5.266 (1.15), 5.276 (1.25), 5.285 (1.01), 5.296 (0.78), 5.309 (0.68), 5.318 (0.74), 5.329 (0.93), 5.338 (0.94), 5.350 (0.98), 5.365 (0.88), 5.377 (1.23), 5.387 (1.39), 5.399 (1.14), 5.408 (1.13), 5.414 (1.01), 5.427 (0.75), 5.439 (0.71), 5.448 (0.78), 5.458 (0.98), 5.467 (0.94), 5.471 (0.95), 5.480 (1.00), 5.494 (0.74), 5.503 (0.49), 6.871 (7.75), 6.875 (9.87), 6.893 (10.64), 6.896 (10.38), 7.023 (0.51), 7.150 (8.24), 7.158 (9.24), 7.172 (7.28), 7.179 (7.47), 7.278 (0.53).

### Beispiel 454

### (5S)-2-(3-Chlor-4-fluorbenzyl)-5-{[(3S)-3-fluorpyrrolidin-1 -yl]carbonyl}-5,6,7,8-tetrahydro[1,2,4]triazolo[4,3-a]pyridin-3(2H)-on

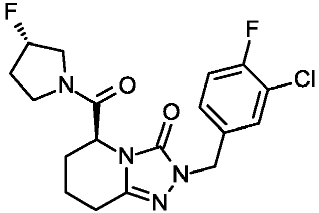

(5S)-2-(3-Chlor-4-fluorbenzyl)-3-oxo-2,3,5,6,7,8-hexahydro[1,2,4]triazolo[4,3-a]pyridin-5-carbonsäure (60.0 mg, 184 µmol) wurde in THF (2.0 ml) vorgelegt und HBTU (90.8 mg, 239 µmol) und N,N-Diisopropylethylamin (96 µl, 550 µmol) wurden anschließend zugegeben. Nach 15 min Rühren bei Raumtemperatur wurde (3S)-3-Fluorpyrrolidinhydrochlorid (27.8 mg, 221 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 39.0 mg (53 % d. Th.) der Titelverbindung erhalten.
LC-MS (Methode 3): Rₜ = 1.44 min; MS (ESIpos): m/z = 397 [M+H]⁺
¹H-NMR (400 MHz, DMSO-d6) δ [ppm]: -0.149 (1.96), -0.008 (16.00), 0.008 (13.73), 0.146 (1.86), 1.728 (2.22), 1.906 (0.57), 2.088 (1.34), 2.266 (0.77), 2.327 (2.74), 2.366 (2.01), 2.523 (8.52), 2.609 (2.12), 2.669 (3.05), 2.710 (1.96), 3.395 (0.72), 3.493 (0.67), 3.635 (1.91), 3.652 (1.55), 3.719 (0.98), 3.742 (1.50), 3.768 (1.14), 3.853 (1.81), 4.702 (1.08), 4.759 (1.50), 4.820 (10.48), 5.260 (0.88), 5.390 (1.14), 5.515 (0.77), 7.245 (1.70), 7.251 (1.75), 7.373 (2.84), 7.396 (4.34), 7.417 (2.32), 7.431 (2.48), 7.449 (2.43).

### Beispiel 455

### (5S)-2-(3-Chlor-4-fluorbenzyl)-5-[(3,3-difluorpyrrolidin-1-yl)carbonyl]-5,6,7,8-tetrahydro[1,2,4]triazolo[4,3-a]pyridin-3(2H)-on

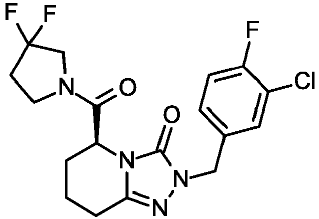

(5S)-2-(3-Chlor-4-fluorbenzyl)-3-oxo-2,3,5,6,7,8-hexahydro[1,2,4]triazolo[4,3-a]pyridin-5-carbonsäure (60.0 mg, 184 µmol) wurde in THF (2.0 ml) vorgelegt und HBTU (90.8 mg, 239 µmol) und N,N-Diisopropylethylamin (96 µl, 550 µmol) wurden anschließend zugegeben. Nach 15 min Rühren bei Raumtemperatur wurde 3,3-Difluorpyrrolidinhydrochlorid (31.7 mg, 221 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 58.0 mg (76 % d. Th.) der Titelverbindung erhalten.
LC-MS (Methode 3): Rₜ = 1.58 min; MS (ESIpos): m/z = 415 [M+H]⁺
¹H-NMR (400 MHz, DMSO-d6) δ [ppm]: -0.008 (2.55), 0.008 (1.32), 1.670 (1.23), 1.680 (1.17), 1.692 (1.09), 1.718 (1.53), 1.728 (1.49), 1.980 (1.43), 1.995 (1.50), 2.005 (1.65), 2.011 (1.56), 2.028 (1.19), 2.039 (1.16), 2.047 (1.23), 2.054 (1.17), 2.064 (1.10), 2.073 (2.24), 2.082 (0.88), 2.090 (0.75), 2.366 (0.60), 2.381 (1.02), 2.392 (0.77), 2.410 (1.20), 2.430 (1.27), 2.453 (1.50), 2.524 (3.13), 2.576 (2.70), 2.591 (2.97), 2.607 (3.10), 2.620 (1.50), 2.637 (0.64), 2.650 (0.89), 2.664 (0.57), 3.519 (0.50), 3.531 (1.37), 3.538 (1.52), 3.549 (2.11), 3.559 (2.19), 3.567 (1.25), 3.578 (1.06), 3.669 (1.29), 3.702 (1.46), 3.742 (0.87), 3.764 (0.74), 3.775 (1.80), 3.790 (1.00), 3.808 (2.63), 3.826 (0.68), 3.891 (0.72), 3.910 (1.44), 3.928 (0.83), 3.936 (1.00), 3.954 (0.54), 3.961 (0.49), 3.990 (0.91), 4.005 (0.49), 4.019 (0.64), 4.033 (0.81), 4.061 (0.48), 4.145 (0.56), 4.177 (0.81), 4.202 (0.81), 4.756 (1.32), 4.765 (1.56), 4.771 (1.70), 4.780 (1.35), 4.824 (16.00), 4.838 (1.92), 4.844 (1.80), 4.853 (1.29), 7.220 (1.53), 7.226 (1.67), 7.232 (1.69), 7.241 (2.18), 7.247 (2.07), 7.253 (1.88), 7.258 (1.76), 7.373 (3.79), 7.396 (5.05), 7.418 (3.12), 7.429 (3.04), 7.434 (2.80), 7.447 (2.95), 7.452 (2.59).

### Beispiel 456

### (5S)-2-(3-Chlor-4-fluorbenzyl)-5-{[(3R,4S)-3,4-difluorpyrrolidin-1-yl]carbonyl}-5,6,7,8-tetrahydro[1,2,4]triazolo[4,3-a]pyridin-3(2H)-on

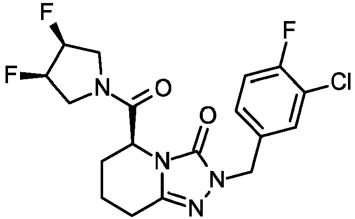

(5S)-2-(3-Chlor-4-fluorbenzyl)-3-oxo-2,3,5,6,7,8-hexahydro[1,2,4]triazolo[4,3-a]pyridin-5-carbonsäure (60.0 mg, 184 µmol) wurde in THF (2.0 ml) vorgelegt und HBTU (90.8 mg, 239 µmol) und N,N-Diisopropylethylamin (96 µl, 550 µmol) wurden anschließend zugegeben. Nach 15 min Rühren bei Raumtemperatur wurde (3R,4S)-3,4-Difluorpyrrolidinhydrochlorid (31.7 mg, 221 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 53.0 mg (69 % d. Th.) der Titelverbindung erhalten.
LC-MS (Methode 3): Rₜ = 1.47 min; MS (ESIpos): m/z = 415 [M+H]⁺
¹H-NMR (400 MHz, DMSO-d6) δ [ppm]: -0.150 (0.55), -0.008 (5.05), 0.008 (4.28), 0.146 (0.63), 1.665 (1.23), 1.729 (1.60), 1.971 (1.16), 2.008 (1.55), 2.051 (1.26), 2.073 (4.71), 2.327 (0.99), 2.366 (0.67), 2.569 (2.20), 2.594 (2.20), 2.607 (2.83), 2.649 (0.94), 2.669 (1.09), 2.709 (0.70), 3.489 (1.14), 3.535 (1.14), 3.545 (1.11), 3.577 (0.70), 3.614 (0.85), 3.629 (0.89), 3.684 (1.33), 3.702 (1.48), 3.723 (1.28), 3.755 (1.01), 3.868 (0.84), 3.926 (0.78), 3.939 (0.87), 3.974 (0.78), 3.989 (0.85), 4.172 (0.92), 4.198 (0.67), 4.804 (3.62), 4.823 (16.00), 5.276 (1.02), 5.388 (1.06), 7.235 (2.20), 7.241 (2.32), 7.370 (2.59), 7.374 (2.87), 7.395 (4.42), 7.419 (2.37), 7.430 (3.75), 7.448 (3.63).

### Beispiel 457

### (5S)-2-(3-Chlor-4-fluorbenzyl)-5-[(3-fluorazetidin-1-yl)carbonyl]-5,6,7,8-tetrahydro[1,2,4]triazolo[4,3-a]pyridin-3(2H)-on

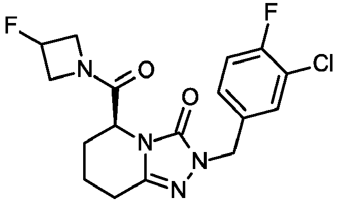

(5S)-2-(3-Chlor-4-fluorbenzyl)-3-oxo-2,3,5,6,7,8-hexahydro[1,2,4]triazolo[4,3-a]pyridin-5-carbonsäure (60.0 mg, 184 µmol) wurde in THF (2.0 ml) vorgelegt und HBTU (90.8 mg, 239 µmol) und N,N-Diisopropylethylamin (96 µl, 550 µmol) wurden anschließend zugegeben. Nach 15 min Rühren bei Raumtemperatur wurde 3-Fluorazetidinhydrochlorid (24.7 mg, 221 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 38.0 mg (54 % d. Th.) der Titelverbindung erhalten.
LC-MS (Methode 3): Rₜ = 1.37 min; MS (ESIpos): m/z = 383 [M+H]⁺
¹H-NMR (400 MHz, DMSO-d6) δ [ppm]: -0.149 (0.57), 0.008 (3.84), 0.146 (0.49), 1.258 (0.93), 1.681 (2.36), 1.704 (3.76), 1.957 (2.08), 2.028 (1.83), 2.327 (0.80), 2.366 (0.82), 2.523 (4.91), 2.558 (3.01), 2.583 (3.84), 2.597 (5.04), 2.610 (2.50), 2.626 (0.97), 2.639 (1.59), 2.653 (0.69), 2.670 (0.82), 2.710 (0.77), 3.901 (0.73), 3.928 (1.44), 3.958 (1.46), 3.990 (1.41), 4.018 (0.80), 4.158 (0.62), 4.172 (0.71), 4.185 (0.60), 4.225 (1.30), 4.239 (1.30), 4.257 (1.41), 4.267 (1.53), 4.290 (1.46), 4.323 (1.22), 4.352 (0.88), 4.395 (0.88), 4.433 (0.66), 4.457 (0.86), 4.504 (0.75), 4.519 (0.91), 4.544 (3.87), 4.557 (5.50), 4.570 (3.62), 4.597 (0.49), 4.634 (0.62), 4.647 (0.75), 4.684 (0.71), 4.701 (0.64), 4.727 (0.51), 4.824 (16.00), 5.353 (0.88), 5.406 (0.88), 5.496 (0.86), 5.550 (0.82), 7.248 (2.87), 7.372 (3.29), 7.394 (5.26), 7.417 (2.67), 7.437 (3.31), 7.455 (3.12).

### Beispiel 458

### (5S)-2-[3-Chlor-4-(trifluormethyl)benzyl]-5-{[(3R,4S)-3,4-difluorpyrrolidin-1-yl]carbonyl}-5,6,7,8-tetrahydro[1,2,4]triazolo[4,3-a]pyridin-3(2H)-on

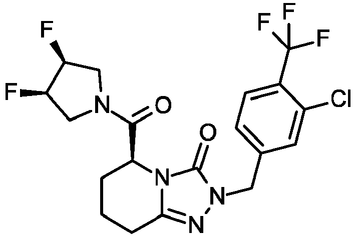

(5S)-2-[3-Chlor-4-(trifluormethyl)benzyl]-3-oxo-2,3,5,6,7,8-hexahydro[1,2,4]triazolo[4,3-a]pyridin-5-carbonsäure (43.8 mg, 117 µmol) wurde in THF (2.0 ml) vorgelegt und HBTU (57.5 mg, 152 µmol) und N,N-Diisopropylethylamin (61 µl, 350 µmol) wurden anschließend zugegeben. Nach 15 min Rühren bei Raumtemperatur wurde (3R,4S)-3,4-Difluorpyrrolidinhydrochlorid (20.1 mg, 140 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es wurden 34.5 mg (64 % d. Th.) der Titelverbindung erhalten.
LC-MS (Methode 4): Rₜ = 0.93 min; MS (ESIpos): m/z = 465 [M+H]⁺
¹H-NMR (400 MHz, DMSO-d6) δ [ppm]: -0.149 (0.42), -0.008 (3.58), 0.008 (3.09), 1.226 (0.51), 1.244 (2.93), 1.259 (3.14), 1.274 (1.91), 1.644 (1.11), 1.668 (1.31), 1.689 (0.71), 1.741 (1.61), 1.908 (0.42), 1.947 (0.57), 1.982 (1.26), 2.003 (0.99), 2.020 (1.73), 2.027 (1.86), 2.045 (1.14), 2.062 (1.35), 2.073 (10.23), 2.089 (0.92), 2.098 (1.01), 2.106 (0.86), 2.328 (0.72), 2.366 (0.69), 2.523 (2.61), 2.570 (1.65), 2.581 (2.21), 2.591 (1.59), 2.605 (2.33), 2.618 (3.14), 2.630 (1.74), 2.649 (0.63), 2.661 (1.28), 2.670 (1.10), 2.690 (0.47), 2.710 (0.62), 3.459 (1.13), 3.484 (1.37), 3.493 (1.94), 3.504 (1.68), 3.516 (1.88), 3.527 (2.12), 3.538 (2.52), 3.549 (2.94), 3.558 (2.70), 3.581 (3.49), 3.590 (3.41), 3.619 (4.21), 3.633 (4.46), 3.652 (4.12), 3.666 (4.31), 3.674 (4.64), 3.689 (5.20), 3.705 (4.91), 3.721 (4.10), 3.756 (3.02), 3.769 (2.58), 3.790 (1.77), 3.803 (1.52), 3.831 (1.04), 3.871 (1.29), 3.909 (0.90), 3.929 (1.08), 3.943 (1.17), 3.958 (0.75), 3.979 (1.08), 3.994 (1.05), 4.008 (0.68), 4.022 (0.63), 4.133 (0.65), 4.148 (0.69), 4.161 (0.68), 4.175 (1.07), 4.188 (0.77), 4.201 (0.69), 4.216 (0.62), 4.810 (2.43), 4.822 (3.53), 4.832 (2.28), 4.944 (16.00), 5.266 (0.95), 5.276 (1.04), 5.288 (0.89), 5.299 (0.62), 5.314 (0.71), 5.330 (0.74), 5.338 (0.74), 5.353 (0.83), 5.367 (0.75), 5.389 (1.10), 5.400 (0.87), 5.409 (0.99), 5.419 (0.92), 5.430 (0.71), 5.443 (0.68), 5.452 (0.72), 5.466 (0.74), 5.475 (0.72), 5.482 (0.69), 5.496 (0.60), 7.372 (2.48), 7.391 (2.67), 7.563 (5.75), 7.841 (4.19), 7.861 (3.82).

### Beispiel 459

### (5S)-2-[3-Chlor-4-(trifluormethyl)benzyl]-5-[(3-fluorazetidin-1 -yl)carbonyl]-5,6,7,8-tetrahydro[1,2,4]triazolo[4,3-a]pyridin-3(2H)-on

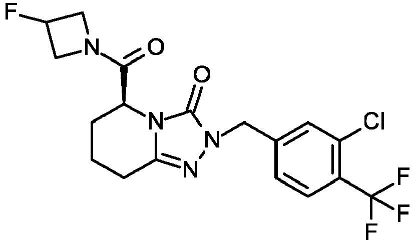

(5S)-2-[3-Chlor-4-(trifluormethyl)benzyl]-3-oxo-2,3,5,6,7,8-hexahydro[1,2,4]triazolo[4,3-a]pyridin-5-carbonsäure (43.8 mg, 117 µmol) wurde in THF (2.0 ml) vorgelegt und HBTU (57.5 mg, 152 µmol) und N,N-Diisopropylethylamin (61 µl, 350 µmol) wurden anschließend zugegeben. Nach 15 min Rühren bei Raumtemperatur wurde 3-Fluorazetidinhydrochlorid (15.6 mg, 140 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 30.2 mg (58 % d. Th.) der Titelverbindung erhalten.
LC-MS (Methode 4): Rₜ = 0.89 min; MS (ESIpos): m/z = 433 [M+H]⁺
¹H-NMR (400 MHz, DMSO-d6) δ [ppm]: -0.149 (0.53), 0.146 (0.51), 1.708 (3.04), 1.722 (3.28), 1.971 (2.07), 2.038 (1.83), 2.073 (2.00), 2.327 (0.95), 2.366 (0.68), 2.576 (2.89), 2.594 (3.71), 2.608 (5.08), 2.621 (2.63), 2.637 (1.10), 2.650 (1.74), 2.665 (1.38), 2.710 (0.77), 3.907 (0.79), 3.933 (1.42), 3.963 (1.48), 3.995 (1.47), 4.020 (0.91), 4.175 (0.70), 4.228 (1.29), 4.275 (1.60), 4.295 (1.38), 4.324 (0.95), 4.366 (1.12), 4.400 (0.97), 4.437 (0.73), 4.462 (0.95), 4.524 (0.79), 4.563 (3.93), 4.577 (5.79), 4.589 (3.84), 4.651 (0.74), 4.704 (0.74), 4.945 (16.00), 5.354 (0.91), 5.409 (0.92), 5.497 (0.91), 5.550 (0.92), 7.376 (4.42), 7.396 (4.84), 7.569 (8.14), 7.838 (5.78), 7.859 (5.38).

### Beispiel 460

### (5S)-2-[3-Chlor-4-(trifluormethyl)benzyl]-5-[(3,3-difluorpyrrolidin-1-yl)carbonyl]-5,6,7,8-tetrahydro[1,2,4]triazolo[4,3-a]pyridin-3(2H)-on

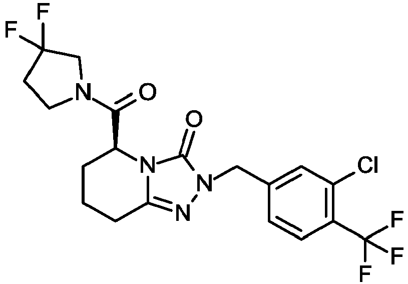

(5S)-2-[3-Chlor-4-(trifluormethyl)benzyl]-3-oxo-2,3,5,6,7,8-hexahydro[1,2,4]triazolo[4,3-a]pyridin-5-carbonsäure (43.8 mg, 117 µmol) wurde in THF (2.0 ml) vorgelegt und HBTU (57.5 mg, 152 µmol) und N,N-Diisopropylethylamin (61 µl, 350 µmol) wurden anschließend zugegeben. Nach 15 min Rühren bei Raumtemperatur wurde 3,3-Difluorpyrrolidinhydrochlorid (20.1 mg, 140 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 35.4 mg (65 % d. Th.) der Titelverbindung erhalten.
LC-MS (Methode 4): Rₜ = 0.96 min; MS (ESIpos): m/z = 465 [M+H]⁺
¹H-NMR (400 MHz, DMSO-d6) δ [ppm]: -0.008 (2.40), 0.008 (2.34), 1.244 (0.81), 1.259 (0.99), 1.273 (0.51), 1.673 (1.17), 1.682 (1.09), 1.696 (0.85), 1.740 (1.46), 1.997 (1.38), 2.012 (1.69), 2.022 (1.79), 2.037 (1.61), 2.049 (1.29), 2.057 (1.23), 2.064 (1.17), 2.073 (1.49), 2.085 (0.85), 2.093 (0.87), 2.100 (0.77), 2.281 (0.61), 2.328 (0.48), 2.366 (0.80), 2.382 (0.99), 2.393 (0.73), 2.411 (1.18), 2.430 (1.12), 2.454 (0.87), 2.519 (3.66), 2.562 (2.91), 2.576 (2.85), 2.587 (3.07), 2.602 (2.51), 2.619 (2.89), 2.631 (1.54), 2.650 (0.62), 2.661 (1.10), 2.670 (0.87), 2.690 (0.52), 2.710 (0.48), 2.885 (1.28), 3.535 (1.31), 3.542 (1.47), 3.553 (2.09), 3.562 (2.29), 3.571 (1.29), 3.582 (1.16), 3.639 (0.45), 3.673 (1.35), 3.707 (1.54), 3.726 (0.90), 3.745 (0.94), 3.779 (1.78), 3.786 (1.32), 3.795 (0.96), 3.812 (2.75), 3.831 (0.74), 3.893 (0.70), 3.911 (1.53), 3.930 (0.84), 3.938 (1.09), 3.956 (0.52), 3.966 (0.48), 3.995 (0.94), 4.009 (0.48), 4.023 (0.66), 4.037 (0.85), 4.065 (0.52), 4.150 (0.56), 4.182 (0.85), 4.207 (0.83), 4.541 (0.47), 4.776 (1.25), 4.785 (1.47), 4.791 (1.72), 4.800 (1.24), 4.847 (1.27), 4.856 (1.53), 4.862 (1.75), 4.871 (1.39), 4.945 (16.00), 7.371 (3.29), 7.392 (3.62), 7.564 (5.98), 7.839 (6.03), 7.860 (5.60).

### Beispiel 461

### (5S)-2-[3-Chlor-4-(trifluormethyl)benzyl]-5-{[(3S)-3-fluorpyrrolidin-1 -yl]carbonyl}-5,6,7,8-tetrahydro[1,2,4]triazolo[4,3-a]pyridin-3(2H)-on

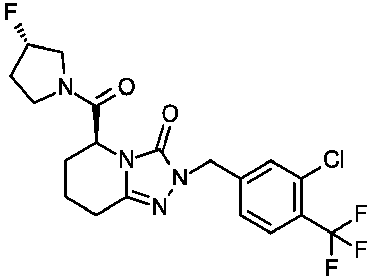

(5S)-2-[3-Chlor-4-(trifluormethyl)benzyl]-3-oxo-2,3,5,6,7,8-hexahydro[1,2,4]triazolo[4,3-a]pyridin-5-carbonsäure (43.8 mg, 117 µmol) wurde in THF (2.0 ml) bei Raumtemperatur vorgelegt. Anschließend wurden HATU (57.6 mg, 152 µmol) und Triethylamin (49 µl, 350 µmol) zugegeben. Nach 15 min Rühren wurde (3S)-3-Fluorpyrrolidinhydrochlorid (17.6 mg, 140 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 44.7 mg (86 % d. Th.) der Titelverbindung erhalten.
LC-MS (Methode 4): Rₜ = 0.90 min; MS (ESIpos): m/z = 447 [M+H]⁺
¹H-NMR (400 MHz, DMSO-d6) δ [ppm]: 0.008 (2.72), 1.733 (2.50), 1.741 (2.71), 1.885 (0.64), 1.920 (0.84), 1.970 (0.43), 2.005 (0.84), 2.036 (1.45), 2.047 (1.38), 2.073 (5.69), 2.085 (2.04), 2.108 (2.42), 2.125 (1.59), 2.140 (1.69), 2.222 (0.93), 2.242 (0.83), 2.256 (0.88), 2.270 (1.25), 2.327 (0.64), 2.366 (0.49), 2.523 (2.19), 2.570 (1.75), 2.580 (1.91), 2.595 (1.62), 2.621 (2.69), 2.665 (1.42), 2.710 (0.48), 3.276 (0.75), 3.351 (0.78), 3.363 (0.72), 3.372 (0.77), 3.398 (0.88), 3.407 (0.88), 3.460 (0.65), 3.469 (0.68), 3.496 (0.90), 3.505 (0.85), 3.611 (0.59), 3.637 (2.74), 3.655 (2.24), 3.661 (2.19), 3.681 (1.85), 3.703 (1.17), 3.726 (1.26), 3.747 (2.22), 3.770 (1.72), 3.788 (1.39), 3.857 (2.72), 4.703 (1.29), 4.712 (1.56), 4.719 (1.59), 4.728 (1.26), 4.761 (1.69), 4.769 (1.88), 4.776 (2.09), 4.785 (1.56), 4.941 (16.00), 5.260 (1.32), 5.391 (1.77), 5.512 (0.96), 7.372 (2.79), 7.393 (3.03), 7.564 (5.99), 7.839 (5.89), 7.859 (5.50).

### Beispiel 462

### (5S)-2-(4-Chlor-3-fluorbenzyl)-5-{[(3R,4S)-3,4-difluorpyrrolidin-1-yl]carbonyl}-5,6,7,8-tetrahydro[1,2,4]triazolo[4,3-a]pyridin-3(2H)-on

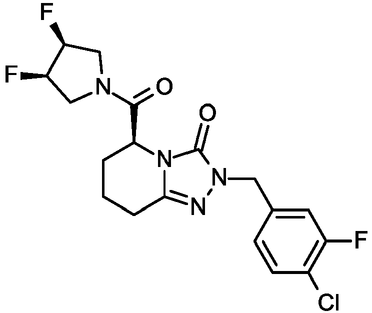

(5S)-2-(4-Chlor-3-fluorbenzyl)-3-oxo-2,3,5,6,7,8-hexahydro[1,2,4]triazolo[4,3-a]pyridin-5-carbonsäure (80.0 mg, 233 µmol) wurde in THF (2.0 ml) vorgelegt und HBTU (115 mg, 303 µmol) und N,N-Diisopropylethylamin (120 µl, 700 µmol) wurden anschließend zugegeben. Nach 15 min Rühren bei Raumtemperatur wurde (3R,4S)-3,4-Difluorpyrrolidinhydrochlorid (40.2 mg, 280 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 58.0 mg (60 % d. Th.) der Titelverbindung erhalten.
LC-MS (Methode 3): Rₜ = 1.48 min; MS (ESIpos): m/z = 415 [M+H]⁺
¹H-NMR (400 MHz, DMSO-d6) δ [ppm]: -0.149 (0.47), -0.008 (4.35), 0.008 (4.00), 0.146 (0.45), 1.665 (1.17), 1.699 (0.65), 1.736 (1.42), 1.940 (0.53), 1.973 (1.13), 2.020 (1.52), 2.038 (0.97), 2.048 (1.03), 2.056 (1.13), 2.063 (1.09), 2.073 (1.78), 2.083 (0.83), 2.091 (0.87), 2.099 (0.77), 2.328 (0.69), 2.366 (0.87), 2.523 (2.79), 2.558 (1.86), 2.572 (2.14), 2.582 (1.44), 2.597 (2.04), 2.610 (2.73), 2.621 (1.50), 2.640 (0.57), 2.652 (0.91), 2.665 (0.91), 2.710 (0.81), 3.490 (3.44), 3.500 (2.45), 3.512 (2.10), 3.522 (1.82), 3.533 (1.58), 3.545 (1.54), 3.567 (0.83), 3.576 (0.99), 3.586 (0.75), 3.617 (0.91), 3.630 (1.01), 3.650 (0.63), 3.664 (0.73), 3.672 (0.99), 3.686 (1.56), 3.702 (1.40), 3.719 (1.13), 3.734 (1.11), 3.755 (1.01), 3.765 (0.91), 3.788 (0.55), 3.800 (0.51), 3.868 (0.75), 3.904 (0.57), 3.927 (0.73), 3.941 (0.87), 3.955 (0.49), 3.977 (0.81), 3.991 (0.83), 4.005 (0.51), 4.019 (0.47), 4.131 (0.53), 4.146 (0.59), 4.159 (0.57), 4.172 (0.89), 4.186 (0.63), 4.199 (0.57), 4.214 (0.51), 4.806 (3.42), 4.848 (16.00), 5.265 (0.83), 5.276 (0.93), 5.287 (0.81), 5.298 (0.55), 5.312 (0.63), 5.329 (0.67), 5.337 (0.65), 5.351 (0.75), 5.365 (0.65), 5.388 (0.99), 5.407 (0.91), 5.417 (0.81), 5.428 (0.63), 5.441 (0.59), 5.464 (0.65), 5.472 (0.63), 5.482 (0.65), 5.494 (0.53), 6.947 (0.61), 7.075 (2.08), 7.081 (2.65), 7.101 (2.89), 7.202 (0.65), 7.219 (2.81), 7.244 (2.93), 7.547 (1.88), 7.551 (2.10), 7.567 (3.46), 7.571 (3.84), 7.587 (1.78), 7.591 (2.00).

### Beispiel 463

### (5S)-2-(4-Chlor-3-fluorbenzyl)-5-[(3-fluorazetidin-1-yl)carbonyl]-5,6,7,8-tetrahydro[1,2,4]triazolo[4,3-a]pyridin-3(2H)-on

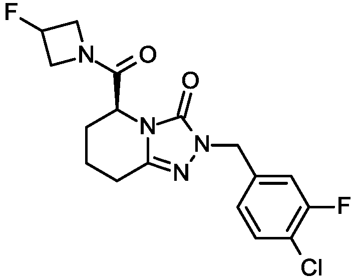

(5S)-2-(4-Chlor-3-fluorbenzyl)-3-oxo-2,3,5,6,7,8-hexahydro[1,2,4]triazolo[4,3-a]pyridin-5-carbonsäure (80.0 mg, 233 µmol) wurde in THF (2.0 ml) vorgelegt und HBTU (115 mg, 303 µmol) und N,N-Diisopropylethylamin (120 µl, 700 µmol) wurden anschließend zugegeben. Nach 15 min Rühren bei Raumtemperatur wurde 3-Fluorazetidinhydrochlorid (31.2 mg, 280 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 36.0 mg (40 % d. Th.) der Titelverbindung erhalten.
LC-MS (Methode 3): Rₜ = 1.38 min; MS (ESIpos): m/z = 383 [M+H]⁺
¹H-NMR (400 MHz, DMSO-d6) δ [ppm]: -0.149 (0.92), -0.008 (8.11), 0.146 (0.92), 1.680 (1.99), 1.704 (3.16), 1.715 (3.24), 1.964 (2.03), 2.029 (1.81), 2.327 (1.35), 2.366 (1.49), 2.523 (5.80), 2.560 (3.34), 2.585 (3.95), 2.599 (5.08), 2.612 (2.70), 2.627 (1.03), 2.641 (1.64), 2.669 (1.49), 2.710 (1.49), 3.899 (0.71), 3.932 (1.42), 3.961 (1.46), 3.989 (1.42), 4.019 (0.85), 4.158 (0.64), 4.172 (0.71), 4.224 (1.21), 4.241 (1.24), 4.271 (1.60), 4.292 (1.42), 4.322 (1.14), 4.360 (0.96), 4.395 (0.85), 4.433 (0.64), 4.459 (0.89), 4.548 (3.52), 4.561 (5.48), 4.573 (3.70), 4.676 (0.75), 4.701 (0.68), 4.849 (16.00), 5.352 (0.89), 5.406 (0.85), 5.495 (0.89), 5.548 (0.89), 7.069 (0.57), 7.086 (4.05), 7.106 (4.41), 7.197 (0.46), 7.226 (3.80), 7.252 (3.95), 7.548 (2.74), 7.568 (5.23), 7.588 (2.60).

### Beispiel 464

### (5S)-2-(4-Chlor-3-fluorbenzyl)-5-[(3,3-difluorpyrrolidin-1-yl)carbonyl]-5,6,7,8-tetrahydro[1,2,4]triazolo[4,3-a]pyridin-3(2H)-on

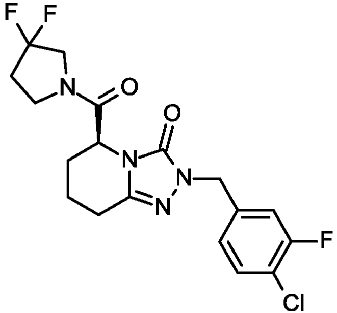

(5S)-2-(4-Chlor-3-fluorbenzyl)-3-oxo-2,3,5,6,7,8-hexahydro[1,2,4]triazolo[4,3-a]pyridin-5-carbonsäure (80.0 mg, 246 µmol) wurde in THF vorgelegt und HBTU (121 mg, 319 µmol) und N,N-Diisopropylethylamin (130 µl, 740 µmol) wurden anschließend zugegeben. Nach 15 min Rühren bei Raumtemperatur wurde 3,3-Difluorpyrrolidinhydrochlorid (42.3 mg, 295 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 32.0 mg (31 % d. Th.) der Titelverbindung erhalten.
LC-MS (Methode 3): Rₜ = 1.56 min; MS (ESIpos): m/z = 415 [M+H]⁺
¹H-NMR (400 MHz, DMSO-d6) δ [ppm]: -0.149 (0.96), 0.146 (0.93), 1.736 (1.42), 2.013 (1.56), 2.366 (1.99), 2.593 (2.02), 2.609 (2.29), 2.709 (1.46), 3.557 (1.72), 3.670 (1.16), 3.703 (1.19), 3.742 (0.80), 3.774 (1.49), 3.809 (2.12), 3.909 (1.16), 3.935 (0.86), 3.992 (0.86), 4.035 (0.70), 4.178 (0.80), 4.202 (0.73), 4.768 (1.36), 4.848 (16.00), 7.081 (2.72), 7.102 (3.08), 7.217 (2.82), 7.243 (2.78), 7.549 (2.78), 7.569 (5.04), 7.589 (2.45).

### Beispiel 465

### (5S)-2-(4-Chlor-3-fluorbenzyl)-5-[(3,3-difluorazetidin-1-yl)carbonyl]-5,6,7,8-tetrahydro[1,2,4]triazolo[4,3-a]pyridin-3(2H)-on

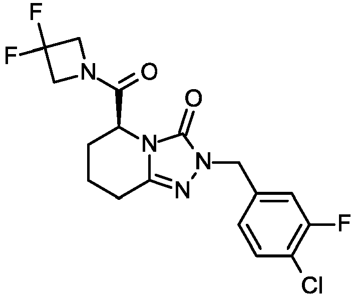

(5S)-2-(4-Chlor-3-fluorbenzyl)-3-oxo-2,3,5,6,7,8-hexahydro[1,2,4]triazolo[4,3-a]pyridin-5-carbonsäure (80.0 mg, 246 µmol) wurde in THF vorgelegt und HBTU (121 mg, 319 µmol) und N,N-Diisopropylethylamin (130 µl, 740 µmol) wurden anschließend zugegeben. Nach 15 min Rühren bei Raumtemperatur wurde 3,3-Difluorazetidinhydrochlorid (38.2 mg, 295 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 69.0 mg (70 % d. Th.) der Titelverbindung erhalten.
LC-MS (Methode 3): Rₜ = 1.51 min; MS (ESIpos): m/z = 401 [M+H]⁺
¹H-NMR (400 MHz, DMSO-d6) δ [ppm]: 1.701 (1.56), 1.711 (2.31), 1.723 (2.92), 1.736 (2.21), 1.960 (0.70), 1.971 (0.73), 1.982 (1.08), 1.995 (1.51), 2.008 (1.34), 2.019 (0.71), 2.026 (0.80), 2.037 (1.03), 2.051 (1.34), 2.061 (1.12), 2.075 (1.16), 2.096 (0.45), 2.561 (1.86), 2.577 (2.07), 2.582 (2.02), 2.596 (2.35), 2.608 (3.23), 2.621 (1.55), 2.635 (0.56), 2.650 (0.89), 2.664 (0.59), 4.336 (0.99), 4.365 (1.68), 4.388 (1.71), 4.419 (1.03), 4.601 (2.22), 4.616 (3.49), 4.628 (2.18), 4.725 (1.04), 4.755 (1.19), 4.785 (0.54), 4.817 (0.56), 4.854 (16.00), 4.875 (1.28), 7.091 (2.53), 7.112 (2.77), 7.228 (2.62), 7.233 (2.47), 7.254 (2.69), 7.258 (2.54), 7.550 (2.83), 7.570 (5.03), 7.590 (2.63).

### Beispiel 466

### (5S)-2-(3,4-Difluorbenzyl)-5-{[(3S)-3-fluorpyrrolidin-1-yl]carbonyl}-5,6,7,8-tetrahydro[1,2,4]triazolo[4,3-a]pyridin-3(2H)-on

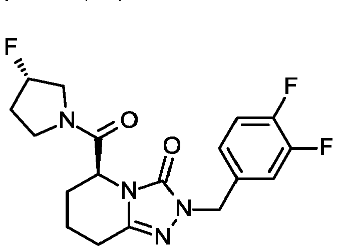

(5S)-2-(3,4-Difluorbenzyl)-3-oxo-2,3,5,6,7,8-hexahydro[1,2,4]triazolo[4,3-a]pyridin-5-carbonsäure (60.0 mg, 194 µmol) wurde in THF (2.0 ml) vorgelegt und HBTU (95.6 mg, 252 µmol) und N,N-Diisopropylethylamin (100 µl, 580 µmol) wurden anschließend zugegeben. Nach 15 min Rühren bei Raumtemperatur wurde (3S)-3-Fluorpyrrolidinhydrochlorid (29.2 mg, 233 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 42.0 mg (57 % d. Th.) der Titelverbindung erhalten.
LC-MS (Methode 4): Rₜ = 0.74 min; MS (ESIpos): m/z = 381 [M+H]⁺
¹H-NMR (400 MHz, DMSO-d6) δ [ppm]: -0.008 (1.32), 0.008 (1.00), 1.705 (1.55), 1.731 (2.78), 1.874 (0.56), 1.909 (0.73), 1.969 (0.40), 1.995 (0.87), 2.023 (1.36), 2.048 (1.03), 2.064 (1.30), 2.074 (1.68), 2.090 (1.82), 2.100 (2.07), 2.126 (1.27), 2.136 (1.45), 2.220 (0.82), 2.269 (1.10), 2.328 (0.48), 2.523 (1.88), 2.560 (1.65), 2.570 (1.83), 2.585 (1.51), 2.613 (2.42), 2.654 (0.91), 2.665 (0.74), 3.273 (0.64), 3.292 (0.87), 3.302 (1.28), 3.320 (1.58), 3.330 (1.65), 3.458 (0.74), 3.466 (0.76), 3.493 (0.93), 3.502 (0.85), 3.610 (0.57), 3.634 (2.77), 3.653 (2.15), 3.678 (1.49), 3.697 (1.09), 3.724 (1.18), 3.745 (1.94), 3.768 (1.62), 3.785 (1.30), 3.855 (2.49), 4.686 (1.18), 4.695 (1.42), 4.701 (1.48), 4.710 (1.16), 4.743 (1.56), 4.752 (1.74), 4.758 (1.95), 4.767 (1.44), 4.819 (16.00), 5.259 (1.23), 5.382 (1.53), 5.390 (1.58), 5.511 (0.86), 7.075 (1.88), 7.080 (1.96), 7.091 (1.90), 7.233 (1.60), 7.257 (2.00), 7.281 (1.62), 7.375 (1.67), 7.396 (3.27), 7.402 (1.91), 7.417 (1.81), 7.423 (3.19), 7.444 (1.51).

### Beispiel 467

### (5S)-2-(3,4-Difluorbenzyl)-5-{[(3R,4S)-3,4-difluorpyrrolidin-1-yl]carbonyl}-5,6,7,8-tetrahydro[1,2,4]triazolo[4,3-a]pyridin-3(2H)-on

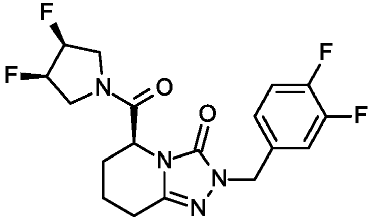

(5S)-2-(3,4-Difluorbenzyl)-3-oxo-2,3,5,6,7,8-hexahydro[1,2,4]triazolo[4,3-a]pyridin-5-carbonsäure (60.0 mg, 194 µmol) wurde in THF (2.0 ml) vorgelegt und HBTU (95.6 mg, 252 µmol) und N,N-Diisopropylethylamin (100 µl, 580 µmol) wurden anschließend zugegeben. Nach 15 min Rühren bei Raumtemperatur wurde (3R,4S)-3,4-Difluorpyrrolidinhydrochlorid (33.4 mg, 233 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 65.0 mg (84 % d. Th.) der Titelverbindung erhalten.
LC-MS (Methode 4): Rₜ = 0.77 min; MS (ESIpos): m/z = 399 [M+H]⁺
¹H-NMR (400 MHz, DMSO-d6) δ [ppm]: -0.150 (0.90), -0.008 (10.40), 0.008 (5.42), 0.146 (0.81), 1.652 (1.36), 1.731 (1.54), 1.977 (1.36), 2.010 (2.08), 2.054 (1.54), 2.327 (2.62), 2.366 (3.34), 2.523 (11.66), 2.570 (2.98), 2.580 (2.17), 2.594 (2.53), 2.608 (3.16), 2.650 (1.27), 2.670 (2.44), 2.710 (1.90), 3.456 (0.90), 3.490 (1.45), 3.513 (1.18), 3.545 (1.27), 3.576 (0.90), 3.616 (0.99), 3.630 (0.99), 3.685 (1.63), 3.701 (1.63), 3.724 (1.36), 3.754 (1.18), 3.868 (0.90), 3.940 (0.99), 3.976 (0.90), 3.990 (0.99), 4.174 (0.99), 4.802 (4.07), 4.822 (16.00), 5.276 (0.99), 5.388 (1.18), 7.073 (1.90), 7.230 (1.45), 7.251 (1.81), 7.279 (1.54), 7.377 (1.18), 7.398 (2.53), 7.421 (2.35), 7.447 (0.99).

### Beispiel 468

### (5S)-2-(3,4-Difluorbenzyl)-5-[(3,3-difluorpyrrolidin-1-yl)carbonyl]-5,6,7,8-tetrahydro[1,2,4]triazolo[4,3-a]pyridin-3(2H)-on

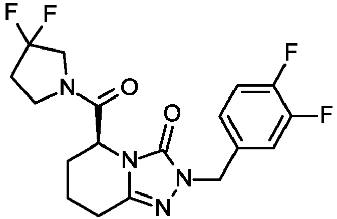

(5S)-2-(3,4-Difluorbenzyl)-3-oxo-2,3,5,6,7,8-hexahydro[1,2,4]triazolo[4,3-a]pyridin-5-carbonsäure (60.0 mg, 194 µmol) wurde in THF (2.0 ml) vorgelegt und HBTU (95.6 mg, 252 µmol) und N,N-Diisopropylethylamin (100 µl, 580 µmol) wurden anschließend zugegeben. Nach 15 min Rühren bei Raumtemperatur wurde 3,3-Difluorpyrrolidinhydrochlorid (33.4 mg, 233 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 45.0 mg (58 % d. Th.) der Titelverbindung erhalten.
LC-MS (Methode 4): Rₜ = 0.81 min; MS (ESIpos): m/z = 399 [M+H]⁺
¹H-NMR (400 MHz, DMSO-d6) δ [ppm]: -0.149 (0.73), 0.146 (0.65), 1.243 (1.06), 1.258 (1.14), 1.273 (0.65), 1.670 (1.14), 1.731 (1.39), 2.012 (1.55), 2.328 (1.80), 2.366 (2.29), 2.380 (0.98), 2.408 (1.14), 2.426 (1.14), 2.523 (7.59), 2.567 (3.76), 2.591 (3.02), 2.609 (3.18), 2.669 (2.20), 2.710 (2.12), 3.538 (1.47), 3.559 (2.20), 3.578 (1.14), 3.635 (0.57), 3.670 (1.39), 3.703 (1.55), 3.743 (0.98), 3.774 (1.80), 3.809 (2.61), 3.826 (0.73), 3.889 (0.73), 3.909 (1.47), 3.935 (1.06), 3.954 (0.57), 3.992 (0.98), 4.035 (0.82), 4.061 (0.57), 4.145 (0.65), 4.177 (0.82), 4.203 (0.82), 4.700 (0.41), 4.770 (1.71), 4.779 (1.22), 4.822 (16.00), 4.852 (1.39), 7.082 (1.80), 7.230 (1.39), 7.257 (1.80), 7.283 (1.47), 7.376 (1.47), 7.396 (2.86), 7.423 (2.94), 7.445 (1.39).

### Beispiel 469

### (5S)-2-(3,4-Difluorbenzyl)-5-[(3-fluorazetidin-1-yl)carbonyl]-5,6,7,8-tetrahydro[1,2,4]triazolo[4,3-a]pyridin-3(2H)-on

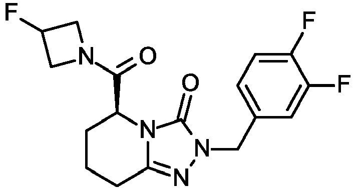

(5S)-2-(3,4-Difluorbenzyl)-3-oxo-2,3,5,6,7,8-hexahydro[1,2,4]triazolo[4,3-a]pyridin-5-carbonsäure (60.0 mg, 194 µmol) wurde in THF (2.0 ml) vorgelegt und HBTU (95.6 mg, 252 µmol) und N,N-Diisopropylethylamin (100 µl, 580 µmol) wurden anschließend zugegeben. Nach 15 min Rühren bei Raumtemperatur wurde 3-Fluorazetidinhydrochlorid (26.0 mg, 233 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 46.0 mg (65 % d. Th.) der Titelverbindung erhalten.
LC-MS (Methode 3): Rₜ = 1.28 min; MS (ESIpos): m/z = 367 [M+H]⁺
¹H-NMR (400 MHz, DMSO-d6) δ [ppm]: 1.658 (1.01), 1.671 (1.34), 1.682 (1.98), 1.705 (3.24), 1.715 (3.29), 1.961 (1.99), 1.972 (1.82), 2.008 (1.60), 2.019 (1.65), 2.030 (1.77), 2.073 (1.65), 2.559 (2.65), 2.584 (3.64), 2.598 (4.93), 2.611 (2.51), 2.627 (0.98), 2.640 (1.60), 2.653 (0.71), 2.690 (0.79), 3.900 (0.66), 3.931 (1.37), 3.961 (1.45), 3.991 (1.40), 4.023 (0.80), 4.159 (0.60), 4.175 (0.66), 4.188 (0.54), 4.213 (0.76), 4.227 (1.24), 4.241 (1.20), 4.257 (1.32), 4.270 (1.45), 4.293 (1.44), 4.324 (1.20), 4.355 (0.84), 4.372 (0.70), 4.397 (0.86), 4.434 (0.62), 4.460 (0.86), 4.507 (0.64), 4.522 (0.75), 4.546 (3.49), 4.559 (5.42), 4.571 (3.51), 4.601 (0.49), 4.635 (0.58), 4.649 (0.71), 4.685 (0.64), 4.702 (0.66), 4.713 (0.59), 4.728 (0.52), 4.824 (16.00), 5.354 (0.85), 5.399 (0.75), 5.406 (0.83), 5.497 (0.84), 5.549 (0.83), 7.086 (2.60), 7.239 (1.90), 7.263 (2.53), 7.288 (1.94), 7.374 (1.50), 7.396 (3.14), 7.421 (3.10), 7.443 (1.35).

### Beispiel 470

### (5S)-5-[(3,3-Difluorpyrrolidin-1-yl)carbonyl]-2-[3-fluor-4-(trifluormethyl)benzyl]-5,6,7,8-tetrahydro[1,2,4]triazolo[4,3-a]pyridin-3(2H)-on

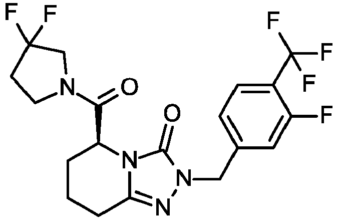

(5S)-2-[3-Fluor-4-(trifluormethyl)benzyl]-3-oxo-2,3,5,6,7,8-hexahydro[1,2,4]triazolo[4,3-a]pyridin-5-carbonsäure (60.0 mg, 167 µmol) wurde in THF (2.0 ml) vorgelegt und HBTU (82.3 mg, 217 µmol) und N,N-Diisopropylethylamin (87 µl, 500 µmol) wurden anschließend zugegeben. Nach 15 min Rühren bei Raumtemperatur wurde 3,3-Difluorpyrrolidinhydrochlorid (28.8 mg, 200 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 37.0 mg (49 % d. Th.) der Titelverbindung erhalten.
LC-MS (Methode 4): Rₜ = 0.92 min; MS (ESIpos): m/z = 449 [M+H]⁺
¹H-NMR (400 MHz, DMSO-d6) δ [ppm]: -0.149 (0.99), 0.008 (7.37), 0.147 (0.91), 1.672 (1.29), 1.743 (1.60), 2.015 (2.03), 2.027 (1.90), 2.051 (1.51), 2.328 (1.55), 2.366 (2.33), 2.383 (1.12), 2.410 (1.38), 2.431 (1.29), 2.563 (3.54), 2.578 (3.11), 2.588 (3.54), 2.604 (3.02), 2.620 (3.19), 2.665 (2.11), 2.670 (2.11), 2.710 (1.90), 3.541 (1.68), 3.561 (2.46), 3.572 (1.38), 3.581 (1.29), 3.641 (0.39), 3.674 (1.42), 3.708 (1.68), 3.744 (1.12), 3.779 (1.98), 3.789 (1.29), 3.813 (2.59), 3.833 (0.82), 3.893 (0.73), 3.912 (1.64), 3.937 (1.12), 3.956 (0.52), 3.997 (0.99), 4.025 (0.73), 4.041 (0.91), 4.069 (0.56), 4.151 (0.65), 4.182 (0.91), 4.206 (0.95), 4.237 (0.43), 4.775 (1.42), 4.789 (1.81), 4.799 (1.38), 4.845 (1.34), 4.861 (1.81), 4.870 (1.29), 4.954 (16.00), 7.248 (3.62), 7.268 (4.05), 7.296 (3.62), 7.326 (3.62), 7.759 (2.72), 7.778 (5.18), 7.798 (2.63).

### Beispiel 471

### (5S)-5-{[(3S)-3-Fluorpyrrolidin-1-yl]carbonyl}-2-[3-fluor-4-(trifluormethyl)benzyl]-5,6,7,8-tetrahydro[1,2,4]triazolo[4,3-a]pyridin-3(2H)-on

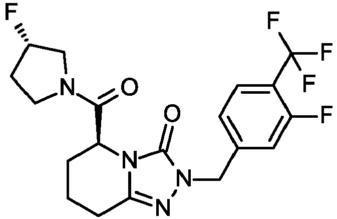

(5S)-2-[3-Fluor-4-(trifluormethyl)benzyl]-3-oxo-2,3,5,6,7,8-hexahydro[1,2,4]triazolo[4,3-a]pyridin-5-carbonsäure (60.0 mg, 167 µmol) wurde in THF (2.0 ml) vorgelegt und HBTU (82.3 mg, 217 µmol) und N,N-Diisopropylethylamin (87 µl, 500 µmol) wurden anschließend zugegeben. Nach 15 min Rühren bei Raumtemperatur wurde (3S)-3-Fluorpyrrolidinhydrochlorid (25.2 mg, 200 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 44.0 mg (61 % d. Th.) der Titelverbindung erhalten.
LC-MS (Methode 4): Rₜ = 0.86 min; MS (ESIpos): m/z = 431 [M+H]⁺
¹H-NMR (400 MHz, DMSO-d6) δ [ppm]: -0.149 (0.49), -0.008 (4.64), 0.008 (3.55), 0.146 (0.49), 1.709 (1.39), 1.733 (2.38), 1.744 (2.67), 1.890 (0.58), 1.929 (0.83), 1.970 (0.41), 2.005 (0.85), 2.031 (1.00), 2.040 (1.46), 2.075 (1.75), 2.086 (2.02), 2.104 (2.33), 2.110 (2.33), 2.139 (1.65), 2.222 (0.92), 2.271 (1.22), 2.327 (0.92), 2.366 (0.80), 2.523 (2.92), 2.575 (1.68), 2.582 (1.90), 2.597 (1.63), 2.623 (2.65), 2.666 (1.60), 2.710 (0.75), 3.279 (0.83), 3.353 (0.66), 3.364 (0.68), 3.373 (0.75), 3.400 (0.85), 3.409 (0.85), 3.461 (0.63), 3.471 (0.66), 3.497 (0.88), 3.506 (0.85), 3.611 (0.56), 3.637 (2.72), 3.654 (2.26), 3.681 (1.85), 3.702 (1.12), 3.729 (1.31), 3.751 (1.92), 3.776 (1.68), 3.791 (1.31), 3.858 (2.65), 4.702 (1.24), 4.711 (1.53), 4.718 (1.58), 4.727 (1.26), 4.760 (1.63), 4.769 (1.92), 4.775 (2.14), 4.784 (1.53), 4.950 (16.00), 5.261 (1.29), 5.391 (1.70), 5.512 (0.95), 7.248 (2.87), 7.269 (3.14), 7.297 (3.45), 7.326 (3.48), 7.758 (2.72), 7.778 (5.06), 7.797 (2.55).

### Beispiel 472

### (5S)-5-{[(3R,4S)-3,4-Difluorpyrrolidin-1-yl]carbonyl}-2-[3-fluor-4-(trifluormethyl)benzyl]-5,6,7,8-tetrahydro[1,2,4]triazolo[4,3-a]pyridin-3(2H)-on

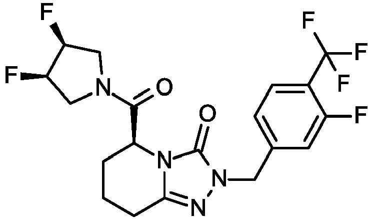

(5S)-2-[3-Fluor-4-(trifluormethyl)benzyl]-3-oxo-2,3,5,6,7,8-hexahydro[1,2,4]triazolo[4,3-a]pyridin-5-carbonsäure (60.0 mg, 167 µmol) wurde in THF (2.0 ml) vorgelegt und HBTU (82.3 mg, 217 µmol) und N,N-Diisopropylethylamin (87 µl, 500 µmol) wurden anschließend zugegeben. Nach 15 min Rühren bei Raumtemperatur wurde (3R,4S)-3,4-Difluorpyrrolidinhydrochlorid (28.8 mg, 200 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 43.0 mg (57 % d. Th.) der Titelverbindung erhalten.
LC-MS (Methode 4): Rₜ = 0.86 min; MS (ESIpos): m/z = 449 [M+H]⁺
¹H-NMR (400 MHz, DMSO-d6) δ [ppm]: -0.149 (1.82), -0.008 (15.01), 0.008 (13.66), 0.146 (1.71), 1.655 (1.45), 1.743 (1.71), 1.982 (1.30), 2.022 (2.03), 2.031 (2.13), 2.064 (1.35), 2.099 (1.14), 2.327 (1.92), 2.366 (2.29), 2.523 (6.13), 2.567 (2.03), 2.582 (2.44), 2.592 (1.71), 2.606 (2.55), 2.619 (3.38), 2.631 (1.92), 2.665 (2.18), 2.670 (2.34), 2.710 (2.18), 3.461 (0.62), 3.495 (1.40), 3.504 (0.94), 3.517 (1.19), 3.526 (1.30), 3.549 (1.09), 3.578 (0.83), 3.620 (0.88), 3.633 (1.04), 3.653 (0.62), 3.675 (1.04), 3.689 (1.97), 3.703 (1.45), 3.723 (1.45), 3.736 (1.40), 3.755 (1.09), 3.766 (1.09), 3.790 (0.62), 3.873 (0.94), 3.908 (0.73), 3.931 (0.88), 3.945 (1.04), 3.960 (0.57), 3.981 (0.94), 3.995 (0.94), 4.010 (0.57), 4.023 (0.57), 4.134 (0.62), 4.149 (0.73), 4.162 (0.68), 4.175 (1.14), 4.189 (0.73), 4.201 (0.68), 4.216 (0.68), 4.821 (3.84), 4.954 (16.00), 5.266 (1.04), 5.276 (1.19), 5.288 (0.99), 5.353 (0.88), 5.389 (1.14), 5.408 (1.14), 5.420 (1.04), 5.431 (0.73), 5.465 (0.83), 7.246 (2.96), 7.267 (3.27), 7.295 (3.27), 7.325 (3.27), 7.760 (2.18), 7.779 (4.05), 7.798 (1.92).

### Beispiel 473

### (5S)-5-[(3-Fluorazetidin-1-yl)carbonyl]-2-[3-fluor-4-(trifluormethyl)benzyl]-5,6,7,8-tetrahydro[1,2,4]triazolo[4,3-a]pyridin-3(2H)-on

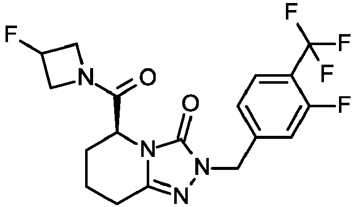

(5S)-2-[3-Fluor-4-(trifluormethyl)benzyl]-3-oxo-2,3,5,6,7,8-hexahydro[1,2,4]triazolo[4,3-a]pyridin-5-carbonsäure (60.0 mg, 167 µmol) wurde in THF (2.0 ml) vorgelegt und HBTU (82.3 mg, 217 µmol) und N,N-Diisopropylethylamin (87 µl, 500 µmol) wurden anschließend zugegeben. Nach 15 min Rühren bei Raumtemperatur wurde 3-Fluorazetidinhydrochlorid (22.4 mg, 200 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 29.0 mg (42 % d. Th.) der Titelverbindung erhalten.
LC-MS (Methode 4): Rₜ = 0.82 min; MS (ESIpos): m/z = 417 [M+H]⁺
¹H-NMR (400 MHz, DMSO-d6) δ [ppm]: -0.149 (1.06), 0.146 (1.06), 1.674 (1.48), 1.698 (2.17), 1.708 (2.70), 1.723 (3.07), 1.977 (2.12), 2.019 (1.87), 2.041 (1.87), 2.086 (4.07), 2.327 (1.23), 2.366 (1.28), 2.570 (3.04), 2.595 (3.93), 2.609 (5.35), 2.622 (2.79), 2.637 (1.09), 2.651 (1.78), 2.665 (1.64), 2.710 (1.45), 3.908 (0.75), 3.935 (1.45), 3.964 (1.48), 3.995 (1.53), 4.023 (0.92), 4.176 (0.75), 4.230 (1.23), 4.243 (1.20), 4.258 (1.09), 4.276 (1.59), 4.306 (1.37), 4.330 (0.89), 4.367 (1.25), 4.400 (0.98), 4.434 (0.67), 4.463 (0.95), 4.515 (0.67), 4.528 (0.81), 4.565 (3.93), 4.578 (5.71), 4.590 (3.71), 4.654 (0.70), 4.689 (0.72), 4.706 (0.72), 4.732 (0.61), 4.954 (16.00), 5.354 (0.89), 5.408 (0.92), 5.498 (0.92), 5.551 (0.86), 7.252 (4.52), 7.272 (4.88), 7.303 (4.29), 7.332 (4.40), 7.757 (2.70), 7.776 (5.16), 7.796 (2.62).

### Beispiel 474

### (5S)-2-(2-Chlor-4-fluorbenzyl)-5-{[(3R,4S)-3,4-difluorpyrrolidin-1-yl]carbonyl}-5,6,7,8-tetrahydro[1,2,4]triazolo[4,3-a]pyridin-3(2H)-on

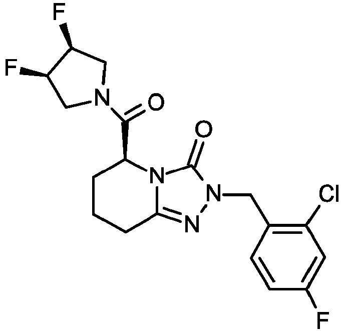

(5S)-2-(2-Chlor-4-fluorbenzyl)-3-oxo-2,3,5,6,7,8-hexahydro[1,2,4]triazolo[4,3-a]pyridin-5-carbonsäure (63.2 mg, 185 µmol) wurde in THF (3.0 ml) vorgelegt und HBTU (91.4 mg, 241 µmol) und N,N-Diisopropylethylamin (130 µl, 740 µmol) wurden anschließend zugegeben. Nach 15 min Rühren bei Raumtemperatur wurde (3R,4S)-3,4-Difluorpyrrolidinhydrochlorid (31.9 mg, 222 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 70.0 mg (91 % d. Th.) der Titelverbindung erhalten.
LC-MS (Methode 3): Rₜ = 1.43 min; MS (ESIpos): m/z = 415 [M+H]⁺
¹H-NMR (400 MHz, DMSO-d6) δ [ppm]: -0.149 (1.38), -0.008 (13.47), 0.008 (11.40), 0.146 (1.38), 1.666 (2.35), 1.736 (2.79), 1.973 (2.17), 2.009 (3.15), 2.019 (3.44), 2.053 (2.14), 2.060 (2.14), 2.073 (5.00), 2.089 (1.70), 2.327 (1.67), 2.366 (1.12), 2.523 (6.84), 2.567 (4.71), 2.577 (3.40), 2.592 (3.95), 2.607 (5.43), 2.618 (3.11), 2.649 (1.88), 2.669 (2.03), 2.709 (1.34), 3.455 (1.19), 3.489 (2.24), 3.498 (1.77), 3.511 (1.88), 3.521 (1.92), 3.544 (1.99), 3.573 (1.45), 3.616 (1.48), 3.630 (1.63), 3.648 (1.05), 3.671 (1.67), 3.685 (3.19), 3.700 (2.46), 3.719 (2.46), 3.733 (2.28), 3.750 (1.95), 3.763 (1.95), 3.798 (1.01), 3.868 (1.48), 3.907 (1.09), 3.926 (1.45), 3.940 (1.63), 3.955 (1.05), 3.975 (1.52), 3.990 (1.63), 4.005 (1.09), 4.018 (0.94), 4.130 (0.98), 4.145 (1.23), 4.159 (1.12), 4.171 (1.85), 4.185 (1.23), 4.198 (0.98), 4.213 (1.05), 4.796 (4.27), 4.808 (5.97), 4.819 (4.16), 4.868 (16.00), 4.875 (9.59), 4.886 (8.65), 4.926 (1.81), 5.264 (1.63), 5.275 (1.85), 5.287 (1.59), 5.297 (1.16), 5.329 (1.41), 5.351 (1.41), 5.388 (1.99), 5.408 (1.63), 5.457 (1.34), 7.201 (1.30), 7.223 (6.33), 7.228 (6.73), 7.238 (9.38), 7.244 (8.36), 7.248 (9.81), 7.254 (6.91), 7.456 (5.36), 7.479 (5.57).

### Beispiel 475

### (5S)-2-(2-Chlor-4-fluorbenzyl)-5-{[(3S)-3-fluorpyrrolidin-1 -yl]carbonyl}-5,6,7,8-tetrahydro[1,2,4]triazolo[4,3-a]pyridin-3(2H)-on

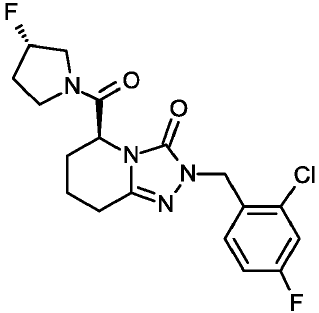

(5S)-2-(2-Chlor-4-fluorbenzyl)-3-oxo-2,3,5,6,7,8-hexahydro[1,2,4]triazolo[4,3-a]pyridin-5-carbonsäure (63.2 mg, 185 µmol) wurde in THF (3.0 ml) vorgelegt und HBTU (91.4 mg, 241 µmol) und N,N-Diisopropylethylamin (130 µl, 740 µmol) wurden anschließend zugegeben. Nach 15 min Rühren bei Raumtemperatur wurde (3S)-3-Fluorpyrrolidinhydrochlorid (27.9 mg, 222 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 63.6 mg (86 % d. Th.) der Titelverbindung erhalten.
LC-MS (Methode 4): Rₜ = 0.73 min; MS (ESIpos): m/z = 397 [M+H]⁺
¹H-NMR (400 MHz, DMSO-d6) δ [ppm]: -0.149 (1.33), -0.008 (11.31), 0.008 (10.12), 0.146 (1.33), 1.735 (5.83), 1.878 (1.15), 1.911 (1.86), 1.994 (1.64), 2.030 (2.74), 2.064 (2.61), 2.073 (4.69), 2.090 (3.49), 2.101 (3.93), 2.137 (2.78), 2.220 (1.59), 2.268 (2.08), 2.327 (2.03), 2.366 (1.33), 2.558 (4.24), 2.568 (4.15), 2.583 (3.23), 2.612 (4.82), 2.669 (2.78), 2.710 (1.59), 3.272 (1.24), 3.359 (1.46), 3.368 (1.46), 3.395 (1.64), 3.405 (1.68), 3.466 (1.24), 3.493 (1.59), 3.502 (1.55), 3.608 (1.06), 3.633 (4.95), 3.653 (4.24), 3.678 (2.78), 3.695 (2.03), 3.726 (2.48), 3.745 (3.58), 3.768 (2.83), 3.788 (2.43), 3.821 (0.71), 3.855 (4.77), 4.691 (2.30), 4.700 (2.78), 4.706 (2.92), 4.715 (2.30), 4.748 (2.87), 4.757 (3.31), 4.763 (3.76), 4.772 (2.74), 4.823 (3.40), 4.863 (16.00), 4.878 (8.71), 4.885 (9.86), 4.925 (2.21), 5.258 (2.25), 5.390 (2.96), 5.515 (1.72), 5.944 (0.57), 7.199 (1.37), 7.220 (6.81), 7.226 (7.47), 7.241 (8.09), 7.248 (9.15), 7.274 (1.46), 7.456 (5.70), 7.462 (5.57), 7.478 (5.79), 7.483 (5.52).

### Beispiel 476

### (5S)-2-(2-Chlor-4-fluorbenzyl)-5-[(3-fluorazetidin-1 -yl)carbonyl]-5,6,7,8-tetrahydro[1,2,4]triazolo[4,3-a]pyridin-3(2H)-on

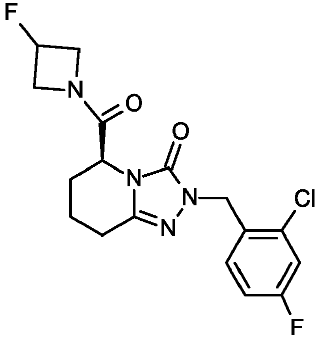

(5S)-2-(2-Chlor-4-fluorbenzyl)-3-oxo-2,3,5,6,7,8-hexahydro[1,2,4]triazolo[4,3-a]pyridin-5-carbonsäure (63.2 mg, 185 µmol) wurde in THF (3.0 ml) vorgelegt und HBTU (91.4 mg, 241 µmol) und N,N-Diisopropylethylamin (130 µl, 740 µmol) wurden anschließend zugegeben. Nach 15 min Rühren bei Raumtemperatur wurde 3-Fluorazetidinhydrochlorid (24.8 mg, 222 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 57.0 mg (73 % d. Th.) der Titelverbindung erhalten.
LC-MS (Methode 4): Rₜ = 0.70 min; MS (ESIpos): m/z = 383 [M+H]⁺
¹H-NMR (400 MHz, DMSO-d6) δ [ppm]: -0.008 (3.58), 0.008 (2.92), 1.688 (2.51), 1.712 (4.48), 1.721 (4.07), 1.964 (2.44), 1.975 (2.30), 2.008 (1.96), 2.019 (2.00), 2.030 (2.15), 2.073 (5.14), 2.327 (0.53), 2.366 (0.51), 2.523 (2.70), 2.581 (3.90), 2.595 (5.88), 2.609 (3.00), 2.624 (1.16), 2.638 (1.95), 2.651 (0.89), 2.670 (0.66), 2.709 (0.55), 3.898 (0.79), 3.927 (1.67), 3.958 (1.70), 3.991 (1.66), 4.018 (0.96), 4.157 (0.70), 4.171 (0.81), 4.187 (0.66), 4.223 (1.42), 4.239 (1.38), 4.252 (1.29), 4.274 (1.71), 4.302 (1.49), 4.334 (0.93), 4.362 (1.42), 4.394 (1.05), 4.431 (0.75), 4.456 (1.03), 4.506 (0.77), 4.520 (0.88), 4.548 (4.00), 4.560 (6.36), 4.573 (4.15), 4.600 (0.64), 4.635 (0.68), 4.649 (0.81), 4.687 (0.81), 4.702 (0.82), 4.728 (0.62), 4.834 (1.19), 4.874 (16.00), 4.884 (6.86), 4.925 (0.82), 5.353 (1.00), 5.406 (1.00), 5.496 (0.99), 5.549 (1.00), 7.202 (1.12), 7.224 (4.47), 7.242 (10.36), 7.258 (7.49), 7.279 (2.00), 7.455 (3.92), 7.461 (3.86), 7.477 (4.01), 7.483 (3.93).

### Beispiel 477

### (5S)-2-(2-Chlor-4-fluorbenzyl)-5-[(3,3-difluorpyrrolidin-1-yl)carbonyl]-5,6,7,8-tetrahydro[1,2,4]triazolo[4,3-a]pyridin-3(2H)-on

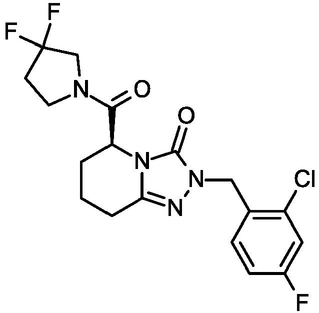

(5S)-2-(2-Chlor-4-fluorbenzyl)-3-oxo-2,3,5,6,7,8-hexahydro[1,2,4]triazolo[4,3-a]pyridin-5-carbonsäure (63.2 mg, 185 µmol) wurde in THF (3.0 ml) vorgelegt und HBTU (91.4 mg, 241 µmol) und N,N-Diisopropylethylamin (130 µl, 740 µmol) wurden anschließend zugegeben. Nach 15 min Rühren bei Raumtemperatur wurde 3,3-Difluorpyrrolidinhydrochlorid (31.9 mg, 222 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 64.5 mg (84 % d. Th.) der Titelverbindung erhalten.
LC-MS (Methode 1): Rₜ = 1.09 min; MS (ESIpos): m/z = 415 [M+H]⁺
¹H-NMR (400 MHz, DMSO-d6) δ [ppm]: -0.149 (1.75), -0.008 (14.84), 0.008 (12.90), 0.146 (1.55), 1.675 (2.23), 1.723 (2.91), 1.911 (0.82), 2.004 (3.30), 2.012 (3.15), 2.039 (2.57), 2.073 (9.41), 2.327 (2.33), 2.366 (2.28), 2.381 (1.89), 2.410 (2.28), 2.430 (2.13), 2.563 (5.62), 2.572 (6.06), 2.587 (5.53), 2.607 (6.01), 2.650 (1.79), 2.670 (2.57), 2.710 (1.99), 3.536 (2.96), 3.556 (4.12), 3.567 (2.33), 3.575 (2.18), 3.636 (0.82), 3.670 (2.47), 3.703 (2.96), 3.740 (1.94), 3.773 (3.25), 3.784 (2.18), 3.809 (4.27), 3.828 (1.21), 3.890 (1.31), 3.909 (2.81), 3.936 (1.99), 3.954 (0.92), 3.993 (1.70), 4.021 (1.31), 4.035 (1.60), 4.063 (0.97), 4.145 (1.12), 4.176 (1.50), 4.201 (1.55), 4.760 (2.47), 4.775 (3.20), 4.785 (2.33), 4.830 (3.35), 4.849 (3.25), 4.868 (15.03), 4.882 (16.00), 4.922 (2.67), 7.199 (1.36), 7.205 (1.36), 7.220 (6.55), 7.226 (7.18), 7.236 (9.31), 7.247 (7.90), 7.252 (9.70), 7.274 (1.65), 7.456 (6.64), 7.462 (6.69), 7.478 (6.50), 7.484 (6.40).

### Beispiel 478

### (5S)-5-{[(3R)-3-Fluorpyrrolidin-1-yl]carbonyl}-2-{[3-fluor-2-(trifluormethyl)pyridin-4-yl]methyl}-5,6,7,8-tetrahydro[1,2,4]triazolo[4,3-a]pyridin-3(2H)-on

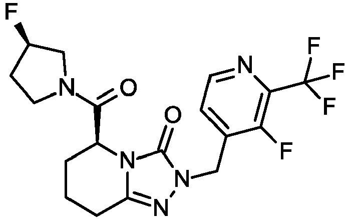

(5S)-2-{[3-Fluor-2-(trifluormethyl)pyridin-4-yl]methyl}-3-oxo-2,3,5,6,7,8-hexahydro[1,2,4]triazolo[4,3-a]pyridin-5-carbonsäure (60.0 mg, 167 µmol) wurde in THF (2.0 ml) bei Raumtemperatur vorgelegt. Anschließend wurden HATU (82.3 mg, 217 µmol) und Triethylamin (70 µl, 500 µmol) zugegeben. Nach 15 min Rühren wurde (3R)-3-Fluorpyrrolidinhydrochlorid (25.1 mg, 200 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 37.0 mg (52 % d. Th.) der Titelverbindung erhalten.
LC-MS (Methode 3): Rₜ = 1.31 min; MS (ESIpos): m/z = 432 [M+H]⁺
¹H-NMR (400 MHz, DMSO-d6) δ [ppm]: -0.149 (0.80), -0.008 (12.00), 0.008 (8.00), 0.146 (0.80), 1.655 (0.80), 1.760 (0.80), 2.016 (2.00), 2.027 (1.60), 2.072 (2.00), 2.101 (0.80), 2.128 (0.80), 2.263 (0.40), 2.324 (1.60), 2.329 (2.40), 2.333 (1.60), 2.367 (2.00), 2.410 (0.40), 2.520 (16.00), 2.524 (14.00), 2.558 (2.80), 2.584 (1.60), 2.595 (1.20), 2.615 (1.20), 2.626 (2.00), 2.638 (1.20), 2.666 (2.40), 2.671 (3.20), 2.711 (2.00), 3.136 (0.40), 3.176 (0.80), 3.209 (0.80), 3.383 (3.20), 3.458 (0.80), 3.528 (2.00), 3.551 (1.60), 3.578 (1.20), 3.603 (2.00), 3.659 (0.80), 3.684 (0.40), 3.748 (0.40), 3.787 (0.40), 3.919 (0.40), 3.942 (0.80), 3.983 (0.40), 4.013 (0.40), 4.043 (0.40), 4.841 (0.80), 4.855 (1.20), 4.866 (0.80), 4.890 (0.80), 5.068 (6.40), 5.272 (0.40), 5.348 (0.40), 5.400 (0.40), 5.481 (0.40), 7.561 (2.00), 8.564 (2.80), 8.575 (2.80).

### Beispiel 479

### (5S)-2-{[3-Fluor-2-(trifluormethyl)pyridin-4-yl]methyl}-5-{[(3S)-3-hydroxypyrrolidin-1-yl]carbonyl}-5,6,7,8-tetrahydro[1,2,4]triazolo[4,3-a]pyridin-3(2H)-on

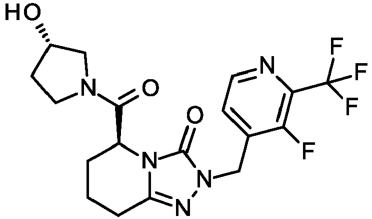

(5S)-2-{[3-Fluor-2-(trifluormethyl)pyridin-4-yl]methyl}-3-oxo-2,3,5,6,7,8-hexahydro[1,2,4]triazolo[4,3-a]pyridin-5-carbonsäure (30.0 mg, 83.3 µmol) wurde in THF (2.0 ml) bei Raumtemperatur vorgelegt. Anschließend wurden HBTU (37.9 mg, 99.9 µmol) und N,N-Diisopropylethylamin (44 µl, 250 µmol) zugegeben. Nach 15 min Rühren wurde (3S)-Pyrrolidin-3-ol (9.43 mg, 108 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 17.0 mg (48 % d. Th.) der Titelverbindung erhalten.
LC-MS (Methode 3): Rₜ = 1.09 min; MS (ESIpos): m/z = 430 [M+H]⁺
¹H-NMR (400 MHz, DMSO-d6) δ [ppm]: -0.149 (1.30), -0.008 (10.80), 0.008 (9.76), 0.146 (1.30), 1.406 (2.34), 1.672 (2.21), 1.750 (4.42), 1.850 (2.47), 1.860 (2.86), 1.872 (2.60), 1.882 (3.25), 1.985 (3.51), 2.008 (3.25), 2.029 (3.90), 2.039 (4.03), 2.075 (2.47), 2.327 (3.64), 2.366 (2.34), 2.518 (16.00), 2.523 (12.88), 2.565 (4.42), 2.576 (4.16), 2.591 (3.12), 2.624 (5.59), 2.665 (5.20), 2.669 (5.20), 2.674 (3.77), 2.710 (2.47), 3.204 (0.91), 3.236 (1.30), 3.346 (4.94), 3.379 (4.42), 3.398 (2.08), 3.433 (1.56), 3.443 (1.56), 3.455 (2.21), 3.463 (2.73), 3.485 (1.95), 3.575 (2.73), 3.593 (1.95), 3.638 (2.47), 3.649 (3.64), 3.665 (2.34), 3.676 (2.86), 3.756 (0.78), 4.270 (2.60), 4.361 (2.86), 4.710 (2.08), 4.719 (2.47), 4.726 (2.60), 4.734 (2.08), 4.752 (2.21), 4.767 (3.38), 4.775 (2.34), 4.834 (0.91), 4.955 (5.07), 4.963 (6.89), 4.972 (2.08), 5.014 (1.17), 5.065 (15.87), 5.078 (7.93), 5.087 (7.02), 5.106 (1.43), 7.542 (4.03), 7.554 (7.93), 7.567 (4.42), 8.562 (9.76), 8.573 (9.76).

### Beispiel 480

### (5S)-5-{[(3R,4S)-3,4-Difluorpyrrolidin-1-yl]carbonyl}-2-{[3-fluor-2-(trifluormethyl)pyridin-4-yl]methyl}-5,6,7,8-tetrahydro[1,2,4]triazolo[4,3-a]pyridin-3(2H)-on

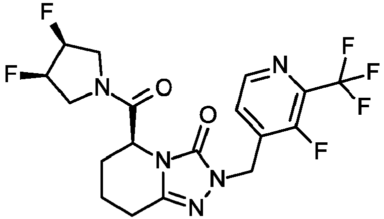

(5S)-2-{[3-Fluor-2-(trifluormethyl)pyridin-4-yl]methyl}-3-oxo-2,3,5,6,7,8-hexahydro[1 ,2,4]triazolo[4,3-a]pyridin-5-carbonsäure (1.19 g, 3.15 mmol) wurde in THF (25 ml) vorgelegt und HBTU (1.55 g, 4.09 mmol) und N,N-Diisopropylethylamin (1.6 ml, 9.4 mmol) wurden anschließend zugegeben. Nach 15 min Rühren bei Raumtemperatur wurde (3R,4S)-3,4-Difluorpyrrolidin (371 mg, 3.46 mmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 1.04 g (73 % d. Th.) der Titelverbindung erhalten.
LC-MS (Methode 3): Rₜ = 1.37 min; MS (ESIpos): m/z = 450 [M+H]⁺
¹H-NMR (400 MHz, DMSO-d6) δ [ppm]: -0.149 (0.54), -0.008 (6.70), 0.008 (4.70), 0.146 (0.62), 1.412 (0.79), 1.655 (1.63), 1.747 (1.90), 1.987 (1.56), 2.025 (2.55), 2.034 (2.62), 2.073 (16.00), 2.095 (1.11), 2.102 (1.16), 2.328 (0.62), 2.561 (2.20), 2.576 (2.30), 2.587 (2.97), 2.597 (2.25), 2.612 (3.07), 2.625 (4.03), 2.637 (2.32), 2.669 (1.88), 2.710 (0.47), 3.461 (0.79), 3.495 (1.66), 3.505 (1.19), 3.517 (1.43), 3.527 (1.48), 3.537 (1.26), 3.551 (1.36), 3.581 (0.96), 3.622 (1.04), 3.636 (1.19), 3.655 (0.82), 3.677 (1.24), 3.691 (2.32), 3.704 (1.68), 3.724 (1.88), 3.738 (1.68), 3.755 (1.34), 3.768 (1.43), 3.802 (0.69), 3.873 (1.01), 3.909 (0.84), 3.930 (1.06), 3.944 (1.19), 3.960 (0.74), 3.980 (1.14), 3.995 (1.11), 4.009 (0.72), 4.022 (0.62), 4.131 (0.69), 4.146 (0.79), 4.160 (0.79), 4.172 (1.21), 4.186 (0.91), 4.199 (0.74), 4.214 (0.67), 4.816 (3.24), 4.830 (4.23), 4.840 (2.94), 5.021 (1.34), 5.063 (12.44), 5.074 (6.87), 5.116 (0.89), 5.266 (1.19), 5.276 (1.29), 5.288 (1.16), 5.314 (0.94), 5.329 (0.96), 5.353 (1.01), 5.389 (1.36), 5.408 (1.21), 5.420 (1.21), 5.431 (0.89), 5.444 (0.87), 5.457 (0.99), 7.549 (2.67), 7.561 (4.33), 8.564 (5.98), 8.576 (5.76).

### Beispiel 481

### (5S)-2-{[3-Fluor-2-(trifluormethyl)pyridin-4-yl]methyl}-5-[(3-hydroxyazetidin-1-yl)carbonyl]-5,6,7,8-tetrahydro[1,2,4]triazolo[4,3-a]pyridin-3(2H)-on

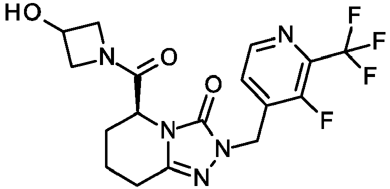

(5S)-2-{[3-Fluor-2-(trifluormethyl)pyridin-4-yl]methyl}-3-oxo-2,3,5,6,7,8-hexahydro[1 ,2,4]triazolo[4,3-a]pyridin-5-carbonsäure (30.0 mg, 83.3 µmol) wurde in THF (2.0 ml) bei Raumtemperatur vorgelegt. Anschließend wurden HBTU (41.1 mg, 108 µmol) und N,N-Diisopropylethylamin (44 µl, 250 µmol) zugegeben. Nach 15 min Rühren wurde Azetidin-3-olhydrochlorid (10.9 mg, 99.9 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und der Rückstand wurde über präparative HPLC aufgereinigt (Methode 10). Die produkthaltigen Fraktionen wurden im Vakuum eingeengt und es wurden 10.0 mg (29 % d. Th.) der Titelverbindung erhalten.
LC-MS (Methode 4): Rₜ = 0.61 min; MS (ESIpos): m/z = 416 [M+H]⁺
¹H-NMR (400 MHz, DMSO-d6) δ [ppm]: -0.149 (1.83), -0.008 (16.00), 0.008 (13.26), 0.146 (1.74), 1.962 (0.46), 2.327 (2.74), 2.366 (2.56), 2.523 (9.05), 2.669 (2.65), 2.710 (2.47), 3.187 (0.55), 4.046 (0.46), 4.523 (0.55), 4.550 (0.64), 5.063 (1.74), 7.561 (0.64), 8.559 (0.64).

### Beispiel 482

### (5S)-5-[(3,3-Difluorpyrrolidin-1-yl)carbonyl]-2-{[3-fluor-2-(trifluormethyl)pyridin-4-yl]methyl}-5,6,7,8-tetrahydro[1,2,4]triazolo[4,3-a]pyridin-3(2H)-on

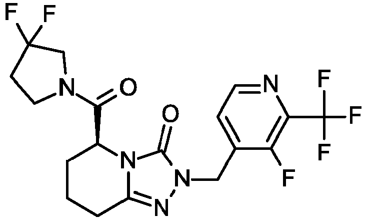

(5S)-2-{[3-Fluor-2-(trifluormethyl)pyridin-4-yl]methyl}-3-oxo-2,3,5,6,7,8-hexahydro[1,2,4]triazolo[4,3-a]pyridin-5-carbonsäure (180 mg, 53 % Reinheit, 266 µmol) wurde in THF (2.4 ml) vorgelegt und HBTU (263 mg, 692 µmol) und N,N-Diisopropylethylamin (280 µl, 1.6 mmol) wurden anschließend zugegeben. Nach 15 min Rühren bei Raumtemperatur wurde 3,3-Difluorpyrrolidinhydrochlorid (91.8 mg, 639 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 106 mg (89 % d. Th.) der Titelverbindung erhalten.
LC-MS (Methode 3): Rₜ = 1.45 min; MS (ESIpos): m/z = 450 [M+H]⁺
¹H-NMR (400 MHz, DMSO-d6) δ [ppm]: -0.149 (0.81), 0.145 (0.69), 1.173 (1.04), 1.191 (0.75), 1.225 (2.76), 1.243 (13.73), 1.259 (16.00), 1.273 (7.74), 1.673 (2.04), 1.748 (2.50), 1.902 (0.95), 1.978 (1.38), 2.019 (3.08), 2.327 (1.53), 2.366 (1.87), 2.382 (1.96), 2.411 (2.36), 2.432 (2.45), 2.455 (2.22), 2.583 (4.23), 2.592 (4.12), 2.608 (4.37), 2.625 (4.72), 2.669 (2.62), 2.710 (0.92), 3.145 (1.27), 3.541 (2.53), 3.560 (3.91), 3.573 (2.13), 3.581 (2.16), 3.641 (1.29), 3.674 (2.27), 3.709 (2.50), 3.744 (1.64), 3.779 (2.91), 3.814 (4.20), 3.833 (1.21), 3.891 (1.21), 3.909 (2.42), 3.936 (1.76), 3.955 (0.83), 3.967 (0.78), 3.997 (1.61), 4.025 (1.12), 4.040 (1.41), 4.067 (0.86), 4.150 (0.83), 4.179 (1.41), 4.205 (1.50), 4.238 (0.55), 4.782 (2.13), 4.797 (2.88), 4.806 (2.07), 4.853 (2.07), 4.868 (2.73), 4.877 (2.13), 5.021 (1.44), 5.063 (13.32), 5.071 (14.39), 5.113 (1.53), 6.513 (0.89), 7.547 (4.03), 7.560 (7.63), 7.573 (4.20), 8.564 (8.86), 8.575 (8.72).

### Beispiel 483

### (5S)-5-[(3-Fluorazetidin-1-yl)carbonyl]-2-{[3-fluor-2-(trifluormethyl)pyridin-4-yl]methyl}-5,6,7,8-tetrahydro[1,2,4]triazolo[4,3-a]pyridin-3(2H)-on

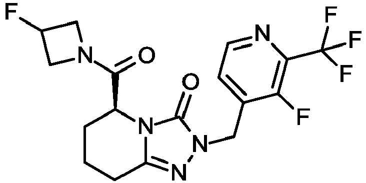

(5S)-2-{[3-Fluor-2-(trifluormethyl)pyridin-4-yl]methyl}-3-oxo-2,3,5,6,7,8-hexahydro[1 ,2,4]triazolo[4,3-a]pyridin-5-carbonsäure (30.0 mg, 83.3 µmol) wurde in THF (2.0 ml) vorgelegt und HBTU (41.1 mg, 108 µmol) und N,N-Diisopropylethylamin (44 µl, 250 µmol) wurden anschließend zugegeben. Nach 15 min Rühren bei Raumtemperatur wurde 3-Fluorazetidinhydrochlorid (11.1 mg, 99.9 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 15.0 mg (43 % d. Th.) der Titelverbindung erhalten.
LC-MS (Methode 4): Rₜ = 0.73 min; MS (ESIpos): m/z = 418 [M+H]⁺
¹H-NMR (400 MHz, DMSO-d6) δ [ppm]: -0.150 (0.74), -0.008 (7.63), 0.008 (6.07), 0.146 (0.74), 1.673 (1.71), 1.683 (2.20), 1.698 (2.34), 1.709 (2.72), 1.730 (3.20), 1.982 (2.42), 1.995 (2.49), 2.020 (2.27), 2.327 (1.45), 2.366 (1.38), 2.523 (5.77), 2.574 (3.20), 2.600 (4.24), 2.614 (6.10), 2.627 (3.20), 2.642 (1.30), 2.656 (2.16), 2.669 (2.46), 2.710 (1.60), 3.909 (0.82), 3.935 (1.64), 3.965 (1.64), 3.997 (1.71), 4.023 (1.00), 4.166 (0.71), 4.178 (0.86), 4.194 (0.67), 4.243 (1.34), 4.278 (1.64), 4.309 (1.60), 4.343 (0.93), 4.371 (1.64), 4.398 (1.12), 4.432 (0.82), 4.460 (1.04), 4.516 (0.74), 4.530 (0.89), 4.572 (4.43), 4.585 (6.55), 4.597 (4.24), 4.634 (0.74), 4.649 (0.82), 4.685 (0.78), 4.702 (0.78), 4.726 (0.63), 5.023 (0.78), 5.065 (16.00), 5.115 (0.63), 5.355 (1.00), 5.408 (1.00), 5.497 (1.04), 5.551 (1.04), 7.556 (4.09), 7.569 (7.74), 7.581 (4.28), 8.561 (6.96), 8.573 (7.00).

### Beispiel 484

### (5S)-5-{[(3S)-3-Fluorpyrrolidin-1-yl]carbonyl}-2-{[3-fluor-2-(trifluormethyl)pyridin-4-yl]methyl}-5,6,7,8-tetrahydro[1,2,4]triazolo[4,3-a]pyridin-3(2H)-on

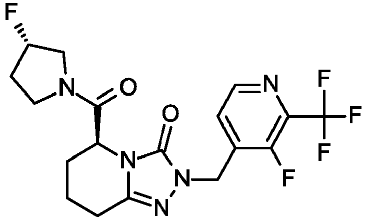

(5S)-2-{[3-Fluor-2-(trifluormethyl)pyridin-4-yl]methyl}-3-oxo-2,3,5,6,7,8-hexahydro[1,2,4]triazolo[4,3-a]pyridin-5-carbonsäure (180 mg, 53 % Reinheit, 266 µmol) wurde in THF (2.4 ml) vorgelegt und HBTU (263 mg, 692 µmol) und N,N-Diisopropylethylamin (280 µl, 1.6 mmol) wurden anschließend zugegeben. Nach 15 min Rühren bei Raumtemperatur wurde (3S)-3-Fluorpyrrolidinhydrochlorid (80.3 mg, 639 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 98.9 mg (86 % d. Th.) der Titelverbindung erhalten.
LC-MS (Methode 3): Rₜ = 1.31 min; MS (ESIpos): m/z = 432 [M+H]⁺
¹H-NMR (400 MHz, DMSO-d6) δ [ppm]: -0.149 (1.74), -0.008 (16.00), 0.008 (13.44), 0.146 (1.64), 1.748 (1.64), 1.934 (0.62), 2.106 (1.64), 2.267 (0.82), 2.327 (2.67), 2.366 (2.15), 2.523 (7.49), 2.587 (1.33), 2.602 (1.13), 2.629 (1.74), 2.669 (3.28), 2.709 (1.95), 3.408 (0.51), 3.462 (0.51), 3.498 (0.62), 3.634 (1.64), 3.652 (1.33), 3.679 (1.13), 3.703 (0.72), 3.751 (1.23), 3.774 (1.13), 3.792 (0.82), 3.857 (1.74), 4.717 (1.13), 4.780 (1.33), 4.790 (1.13), 5.016 (0.92), 5.057 (5.95), 5.071 (4.21), 5.112 (0.62), 5.260 (0.82), 5.393 (1.03), 5.515 (0.62), 7.548 (1.54), 7.561 (3.08), 7.573 (1.74), 8.564 (3.69), 8.575 (3.69).

### Beispiel 485

### (5S)-2-{[3-Fluor-2-(trifluormethyl)pyridin-4-yl]methyl}-5-(1,3-thiazolidin-3-ylcarbonyl)-5,6,7,8-tetrahydro[1,2,4]triazolo[4,3-a]pyridin-3(2H)-on

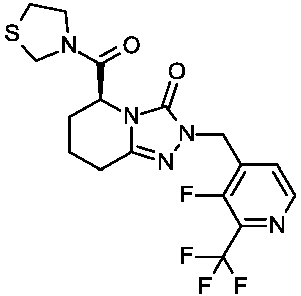

(5S)-2-{[3-Fluor-2-(trifluormethyl)pyridin-4-yl]methyl}-3-oxo-2,3,5,6,7,8-hexahydro[1,2,4]triazolo[4,3-a]pyridin-5-carbonsäure (100 mg, 278 µmol) wurde in THF (2.1 ml, 26 mmol) vorgelegt und HBTU (137 mg, 361 µmol) und N,N-Diisopropylethylamin (150 µl, 830 µmol) wurden anschließend zugegeben. Nach 15 min Rühren bei Raumtemperatur wurde 1,3-Thiazolidin (29.7 mg, 333 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 61.0 mg (51 % d. Th.) der Titelverbindung erhalten.
LC-MS (Methode 3): Rₜ = 1.40 min; MS (ESIpos): m/z = 432 [M+H]⁺
¹H-NMR (400 MHz, DMSO-d6) δ [ppm]: -0.149 (1.81), -0.008 (16.00), 0.008 (14.45), 0.146 (1.94), 1.622 (0.52), 1.764 (0.65), 2.037 (0.90), 2.327 (2.84), 2.366 (2.45), 2.524 (6.32), 2.566 (2.06), 2.581 (1.42), 2.629 (1.29), 2.669 (3.48), 2.710 (2.71), 3.020 (1.42), 3.036 (0.77), 3.130 (0.90), 3.145 (1.81), 3.160 (1.03), 3.643 (0.52), 3.696 (0.52), 3.801 (0.52), 3.945 (0.52), 4.374 (0.90), 4.400 (1.16), 4.556 (1.03), 4.581 (0.90), 4.614 (0.65), 4.637 (0.77), 4.811 (0.77), 4.833 (0.65), 4.904 (0.65), 4.991 (0.52), 5.067 (3.35), 5.075 (3.61), 7.547 (0.90), 7.560 (1.81), 7.574 (0.90), 8.564 (2.06), 8.576 (2.06).

### Beispiel 486

### (5S)-2-(2,4-Difluorbenzyl)-5-[(3-fluorazetidin-1-yl)carbonyl]-5,6,7,8-tetrahydro[1,2,4]triazolo[4,3-a]pyridin-3(2H)-on

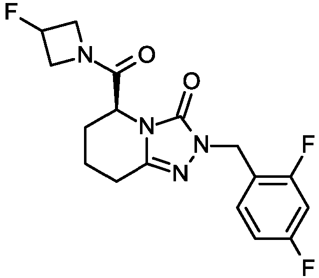

(5S)-2-(2,4-Difluorbenzyl)-3-oxo-2,3,5,6,7,8-hexahydro[1,2,4]triazolo[4,3-a]pyridin-5-carbonsäure (60.0 mg, 194 µmol) wurde in THF (3.0 ml) vorgelegt und HBTU (95.6 mg, 252 µmol) und N,N-Diisopropylethylamin (140 µl, 780 µmol) wurden anschließend zugegeben. Nach 15 min Rühren bei Raumtemperatur wurde 3-Fluorazetidinhydrochlorid (26.0 mg, 233 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 53.1 mg (75 % d. Th.) der Titelverbindung erhalten.
LC-MS (Methode 3): Rₜ = 1.22 min; MS (ESIpos): m/z = 367 [M+H]⁺
¹H-NMR (400 MHz, DMSO-d6) δ [ppm]: 0.008 (2.45), 1.702 (7.00), 1.942 (3.26), 2.005 (2.78), 2.016 (2.91), 2.073 (5.89), 2.327 (0.68), 2.366 (0.55), 2.524 (5.41), 2.566 (6.11), 2.580 (8.21), 2.593 (4.09), 2.609 (1.51), 2.622 (2.63), 2.635 (1.12), 2.669 (0.79), 2.710 (0.61), 3.760 (0.96), 3.778 (0.79), 3.894 (1.12), 3.923 (2.21), 3.952 (2.34), 3.985 (2.23), 4.016 (1.34), 4.152 (0.98), 4.167 (1.09), 4.180 (0.90), 4.205 (1.25), 4.220 (2.01), 4.235 (1.97), 4.250 (2.15), 4.264 (2.34), 4.287 (2.30), 4.319 (1.99), 4.348 (1.44), 4.391 (1.40), 4.426 (1.03), 4.450 (1.36), 4.496 (1.09), 4.521 (5.41), 4.533 (8.38), 4.546 (5.32), 4.566 (1.23), 4.591 (0.79), 4.629 (0.96), 4.642 (1.12), 4.681 (1.07), 4.697 (1.07), 4.721 (0.88), 4.784 (2.28), 4.822 (16.00), 4.877 (1.42), 5.351 (1.40), 5.405 (1.38), 5.494 (1.36), 5.548 (1.38), 7.056 (2.78), 7.078 (6.02), 7.097 (3.37), 7.219 (2.93), 7.244 (5.67), 7.268 (3.02), 7.284 (2.85), 7.304 (5.87), 7.322 (5.60), 7.343 (2.34).

### Beispiel 487

### (5S)-2-(2,4-Difluorbenzyl)-5-[(3,3-difluorpyrrolidin-1-yl)carbonyl]-5,6,7,8-tetrahydro[1,2,4]triazolo[4,3-a]pyridin-3(2H)-on

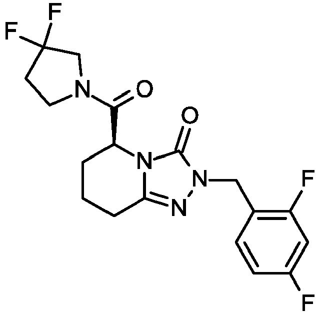

(5S)-2-(2,4-Difluorbenzyl)-3-oxo-2,3,5,6,7,8-hexahydro[1,2,4]triazolo[4,3-a]pyridin-5-carbonsäure (60.0 mg, 194 µmol) wurde in THF (3.0 ml) vorgelegt und HBTU (95.6 mg, 252 µmol) und N,N-Diisopropylethylamin (140 µl, 780 µmol) wurden anschließend zugegeben. Nach 15 min Rühren bei Raumtemperatur wurde 3,3-Difluorpyrrolidinhydrochlorid (33.4 mg, 233 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 68.6 mg (89 % d. Th.) der Titelverbindung erhalten.
LC-MS (Methode 3): Rₜ = 1.42 min; MS (ESIpos): m/z = 399 [M+H]⁺
¹H-NMR (400 MHz, DMSO-d6) δ [ppm]: -0.009 (7.97), 1.707 (3.42), 1.991 (3.13), 2.072 (3.13), 2.327 (1.65), 2.365 (1.99), 2.408 (2.33), 2.572 (6.04), 2.590 (6.43), 2.604 (3.99), 2.633 (1.94), 2.669 (1.77), 2.710 (1.77), 3.533 (2.85), 3.544 (4.10), 3.554 (4.38), 3.664 (2.56), 3.697 (2.96), 3.739 (1.59), 3.772 (3.53), 3.804 (5.52), 3.904 (2.90), 3.930 (2.05), 3.989 (1.77), 4.031 (1.59), 4.169 (1.59), 4.196 (1.65), 4.748 (3.19), 4.779 (2.79), 4.817 (16.00), 4.836 (14.92), 4.875 (2.85), 7.059 (2.51), 7.081 (5.47), 7.102 (2.96), 7.218 (3.19), 7.225 (3.07), 7.244 (5.01), 7.268 (3.36), 7.275 (5.07), 7.298 (6.21), 7.315 (5.98), 7.336 (2.62).

### Beispiel 488

### (5S)-2-(2,4-Difluorbenzyl)-5-{[(3S)-3-fluorpyrrolidin-1-yl]carbonyl}-5,6,7,8-tetrahydro[1,2,4]triazolo[4,3-a]pyridin-3(2H)-on

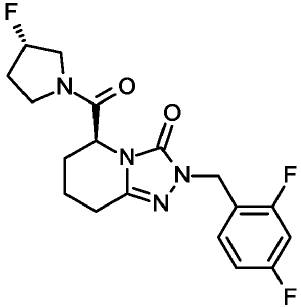

(5S)-2-(2,4-Difluorbenzyl)-3-oxo-2,3,5,6,7,8-hexahydro[1,2,4]triazolo[4,3-a]pyridin-5-carbonsäure (60.0 mg, 194 µmol) wurde in THF (3.1 ml) vorgelegt und HBTU (95.6 mg, 252 µmol) und N,N-Diisopropylethylamin (140 µl, 780 µmol) wurden anschließend zugegeben. Nach 15 min Rühren bei Raumtemperatur wurde (3S)-3-Fluorpyrrolidinhydrochlorid (29.2 mg, 233 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 71.0 mg (96 % d. Th.) der Titelverbindung erhalten.
LC-MS (Methode 3): Rₜ = 1.26 min; MS (ESIpos): m/z = 381 [M+H]⁺
¹H-NMR (400 MHz, DMSO-d6) δ [ppm]: -0.149 (0.47), -0.008 (4.61), 0.008 (3.24), 0.146 (0.44), 1.244 (0.93), 1.259 (0.88), 1.274 (0.58), 1.710 (5.63), 1.721 (6.37), 1.858 (1.32), 1.869 (1.26), 1.882 (1.15), 1.892 (1.62), 1.970 (1.59), 1.984 (1.56), 1.996 (2.22), 2.006 (2.91), 2.019 (2.66), 2.050 (2.52), 2.060 (3.16), 2.073 (10.65), 2.086 (3.81), 2.105 (3.24), 2.123 (2.14), 2.134 (2.58), 2.170 (0.93), 2.189 (0.63), 2.217 (1.70), 2.237 (1.81), 2.266 (2.25), 2.323 (0.93), 2.328 (1.07), 2.366 (1.13), 2.519 (4.01), 2.568 (2.96), 2.591 (4.80), 2.596 (4.97), 2.638 (1.87), 2.670 (1.04), 2.690 (1.78), 2.710 (1.04), 2.731 (0.96), 2.890 (1.29), 3.267 (1.13), 3.286 (1.40), 3.296 (2.09), 3.314 (2.11), 3.324 (1.45), 3.342 (1.23), 3.357 (1.26), 3.365 (1.45), 3.369 (1.29), 3.391 (1.67), 3.400 (1.70), 3.453 (1.29), 3.462 (1.34), 3.489 (1.89), 3.498 (1.73), 3.606 (1.23), 3.631 (6.26), 3.649 (4.50), 3.654 (4.12), 3.674 (2.83), 3.693 (2.42), 3.719 (2.61), 3.740 (4.28), 3.766 (3.51), 3.773 (2.74), 3.782 (2.96), 3.814 (0.80), 3.851 (5.46), 4.491 (2.42), 4.668 (4.34), 4.677 (4.78), 4.683 (4.91), 4.693 (4.14), 4.724 (4.69), 4.734 (5.08), 4.740 (5.57), 4.749 (4.36), 4.773 (4.94), 4.812 (16.00), 4.838 (10.21), 4.877 (3.18), 5.258 (2.69), 5.382 (3.35), 5.389 (3.46), 5.510 (1.95), 5.943 (0.63), 6.954 (0.41), 7.054 (2.58), 7.060 (2.74), 7.075 (5.79), 7.081 (6.37), 7.097 (3.27), 7.102 (3.32), 7.218 (3.10), 7.225 (2.94), 7.243 (5.43), 7.249 (5.05), 7.268 (3.29), 7.274 (4.47), 7.280 (2.52), 7.297 (5.52), 7.302 (4.36), 7.314 (3.68), 7.318 (5.46), 7.335 (1.65), 7.340 (1.84).

### Beispiel 489

### (5S)-2-(2,4-Difluorbenzyl)-5-{[(3R,4S)-3,4-difluorpyrrolidin-1-yl]carbonyl}-5,6,7,8-tetrahydro[1,2,4]triazolo[4,3-a]pyridin-3(2H)-on

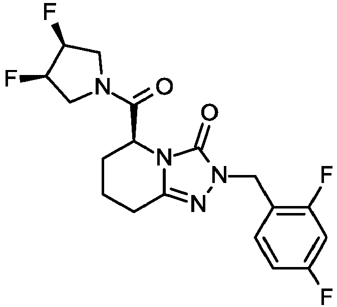

(5S)-2-(2,4-Difluorbenzyl)-3-oxo-2,3,5,6,7,8-hexahydro[1,2,4]triazolo[4,3-a]pyridin-5-carbonsäure (60.0 mg, 194 µmol) wurde in THF (3.1 ml) vorgelegt und HBTU (95.6 mg, 252 µmol) und N,N-Diisopropylethylamin (140 µl, 780 µmol) wurden anschließend zugegeben. Nach 15 min Rühren bei Raumtemperatur wurde (3R,4S)-3,4-Difluorpyrrolidinhydrochlorid (33.4 mg, 233 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 70.3 mg (91 % d. Th.) der Titelverbindung erhalten.
LC-MS (Methode 3): Rₜ = 1.34 min; MS (ESIpos): m/z = 399 [M+H]⁺
¹H-NMR (400 MHz, DMSO-d6) δ [ppm]: -0.149 (0.55), -0.008 (5.38), 0.008 (5.26), 0.146 (0.55), 1.021 (0.70), 1.038 (0.74), 1.409 (0.50), 1.637 (2.21), 1.661 (2.91), 1.670 (2.58), 1.697 (2.83), 1.709 (3.33), 1.718 (3.71), 1.732 (3.08), 1.909 (1.50), 1.951 (2.82), 1.959 (2.44), 1.975 (1.85), 1.997 (3.57), 2.021 (2.24), 2.032 (2.52), 2.039 (2.71), 2.047 (2.58), 2.056 (2.19), 2.073 (9.63), 2.082 (1.94), 2.110 (0.67), 2.327 (0.77), 2.562 (4.05), 2.577 (5.02), 2.591 (6.66), 2.602 (3.78), 2.621 (1.42), 2.633 (2.28), 2.645 (1.22), 2.670 (0.89), 2.710 (0.67), 3.440 (0.78), 3.449 (1.35), 3.458 (0.84), 3.483 (2.30), 3.504 (1.67), 3.512 (1.74), 3.521 (2.00), 3.530 (2.80), 3.538 (2.63), 3.563 (1.19), 3.573 (1.72), 3.583 (1.09), 3.611 (1.83), 3.625 (2.06), 3.644 (1.28), 3.658 (1.45), 3.666 (2.06), 3.681 (2.99), 3.699 (3.74), 3.714 (2.35), 3.722 (2.38), 3.731 (2.44), 3.751 (2.28), 3.762 (2.16), 3.784 (1.24), 3.797 (1.17), 3.826 (0.81), 3.856 (1.44), 3.862 (1.78), 3.891 (1.17), 3.898 (1.31), 3.922 (1.80), 3.936 (2.03), 3.951 (1.24), 3.965 (1.42), 3.971 (1.92), 3.985 (1.92), 4.000 (1.20), 4.014 (1.11), 4.125 (1.20), 4.140 (1.39), 4.153 (1.36), 4.167 (2.31), 4.181 (1.49), 4.194 (1.33), 4.209 (1.22), 4.780 (9.35), 4.793 (4.99), 4.817 (16.00), 4.831 (9.32), 4.839 (9.45), 4.870 (1.49), 4.879 (2.33), 5.069 (0.81), 5.244 (1.25), 5.253 (1.99), 5.265 (2.00), 5.275 (2.21), 5.283 (1.78), 5.296 (1.38), 5.309 (1.24), 5.317 (1.27), 5.329 (1.60), 5.337 (1.63), 5.349 (1.70), 5.364 (1.55), 5.374 (2.16), 5.387 (2.41), 5.398 (2.00), 5.406 (1.99), 5.414 (1.78), 5.439 (1.24), 5.447 (1.36), 5.457 (1.70), 5.470 (1.64), 5.479 (1.72), 5.493 (1.25), 5.502 (0.83), 7.057 (2.83), 7.060 (2.85), 7.078 (6.16), 7.081 (6.15), 7.100 (3.50), 7.103 (3.46), 7.220 (2.96), 7.246 (5.77), 7.271 (4.75), 7.293 (4.41), 7.301 (4.41), 7.309 (3.89), 7.317 (4.60), 7.330 (1.78), 7.339 (1.81).

### Beispiel 490

### (5S)-5-[(3,3-Difluorpyrrolidin-1-yl)carbonyl]-2-(2,4,5-trifluorbenzyl)-5,6,7,8-tetrahydro[1,2,4]triazolo[4,3-a]pyridin-3(2H)-on

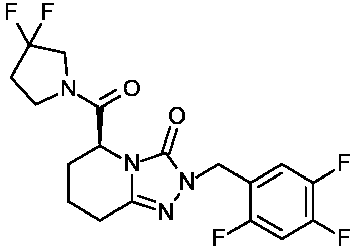

(5S)-3-Oxo-2-(2,4,5-trifluorbenzyl)-2,3,5,6,7,8-hexahydro[1,2,4]triazolo[4,3-a]pyridin-5-carbonsäure (50.0 mg, 153 µmol) wurde in THF (3.0 ml) vorgelegt und HBTU (75.3 mg, 199 µmol) und N,N-Diisopropylethylamin (80 µl, 460 µmol) wurden anschließend zugegeben. Nach 15 min Rühren bei Raumtemperatur wurde 3,3-Difluorpyrrolidinhydrochlorid (26.3 mg, 183 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 34.2 mg (54 % d. Th.) der Titelverbindung erhalten.
LC-MS (Methode 3): Rₜ = 1.50 min; MS (ESIpos): m/z = 417 [M+H]⁺
¹H-NMR (400 MHz, DMSO-d6) δ [ppm]: -0.149 (0.45), -0.008 (4.97), 0.007 (3.87), 0.146 (0.52), 0.966 (0.45), 0.981 (0.45), 1.663 (2.32), 1.688 (1.81), 1.727 (2.84), 1.982 (2.65), 1.995 (3.16), 2.006 (3.42), 2.021 (2.84), 2.034 (2.32), 2.042 (2.39), 2.049 (2.26), 2.059 (2.00), 2.071 (2.00), 2.086 (1.61), 2.327 (1.48), 2.366 (1.74), 2.380 (1.87), 2.409 (2.19), 2.430 (2.19), 2.451 (1.74), 2.560 (5.94), 2.570 (6.97), 2.586 (5.87), 2.604 (6.58), 2.616 (3.16), 2.633 (1.35), 2.646 (2.06), 2.669 (1.81), 2.709 (1.48), 3.530 (2.71), 3.535 (2.90), 3.547 (4.06), 3.556 (4.39), 3.566 (2.52), 3.575 (2.26), 3.636 (0.77), 3.669 (3.29), 3.702 (3.10), 3.737 (2.52), 3.770 (3.42), 3.780 (2.39), 3.788 (1.87), 3.805 (4.65), 3.825 (1.42), 3.835 (0.77), 3.888 (1.35), 3.907 (2.90), 3.925 (1.61), 3.933 (2.06), 3.951 (0.97), 3.961 (0.97), 3.990 (1.87), 4.003 (0.90), 4.018 (1.23), 4.032 (1.68), 4.061 (1.03), 4.143 (1.10), 4.175 (1.68), 4.200 (1.68), 4.230 (0.65), 4.750 (2.39), 4.764 (3.29), 4.774 (2.45), 4.791 (2.19), 4.830 (15.23), 4.845 (16.00), 4.885 (2.52), 7.263 (2.39), 7.280 (2.77), 7.285 (3.16), 7.307 (3.16), 7.312 (2.90), 7.329 (2.45), 7.540 (2.52), 7.556 (2.77), 7.566 (3.87), 7.582 (3.87), 7.591 (2.90), 7.607 (2.65).

### Beispiel 491

### (5S)-5-[(3-Fluorazetidin-1-yl)carbonyl]-2-(2,4,5-trifluorbenzyl)-5,6,7,8-tetrahydro[1,2,4]triazolo[4,3-a]pyridin-3(2H)-on

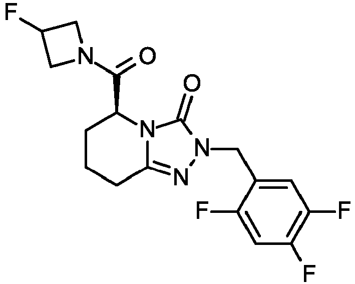

(5S)-3-Oxo-2-(2,4,5-trifluorbenzyl)-2,3,5,6,7,8-hexahydro[1,2,4]triazolo[4,3-a]pyridin-5-carbonsäure (50.0 mg, 153 µmol) wurde in THF (3.0 ml) vorgelegt und HBTU (75.3 mg, 199 µmol) und N,N-Diisopropylethylamin (80 µl, 460 µmol) wurden anschließend zugegeben. Nach 15 min Rühren bei Raumtemperatur wurde 3-Fluorazetidinhydrochlorid (20.5 mg, 183 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 27.2 mg (46 % d. Th.) der Titelverbindung erhalten.
LC-MS (Methode 3): Rₜ = 1.31 min; MS (ESIpos): m/z = 385 [M+H]⁺
¹H-NMR (400 MHz, DMSO-d6) δ [ppm]: -0.149 (1.49), -0.008 (13.66), 0.008 (11.22), 0.146 (1.44), 1.675 (2.84), 1.699 (4.55), 1.709 (4.73), 1.956 (2.93), 1.965 (2.75), 2.023 (2.61), 2.073 (1.58), 2.327 (1.49), 2.366 (1.62), 2.523 (5.72), 2.578 (5.18), 2.593 (7.66), 2.606 (3.97), 2.621 (1.58), 2.635 (2.61), 2.648 (1.22), 2.670 (1.67), 2.710 (1.80), 3.899 (0.99), 3.929 (2.03), 3.959 (2.03), 3.991 (2.07), 4.021 (1.22), 4.158 (0.90), 4.171 (1.04), 4.188 (0.86), 4.224 (1.71), 4.238 (1.71), 4.268 (2.30), 4.290 (2.12), 4.321 (1.71), 4.353 (1.35), 4.392 (1.26), 4.429 (0.95), 4.455 (1.31), 4.505 (0.95), 4.538 (4.64), 4.550 (7.62), 4.562 (4.91), 4.600 (0.77), 4.629 (0.81), 4.648 (0.99), 4.682 (0.99), 4.699 (1.04), 4.794 (1.76), 4.834 (16.00), 4.886 (1.17), 5.353 (1.26), 5.406 (1.22), 5.495 (1.22), 5.550 (1.22), 7.272 (1.89), 7.297 (3.15), 7.315 (3.20), 7.339 (1.89), 7.539 (1.89), 7.564 (3.61), 7.581 (3.65), 7.606 (1.98).

### Beispiel 492

### (5S)-5-{[(3R,4S)-3,4-Difluorpyrrolidin-1 -yl]carbonyl}-2-(2,4,5-trifluorbenzyl)-5,6,7,8-tetrahydro[1,2,4]triazolo[4,3-a]pyridin-3(2H)-on

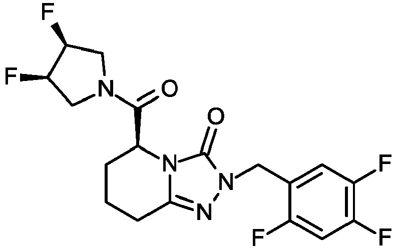

(5S)-3-Oxo-2-(2,4,5-trifluorbenzyl)-2,3,5,6,7,8-hexahydro[1,2,4]triazolo[4,3-a]pyridin-5-carbonsäure (50.0 mg, 153 µmol) wurde in THF (3.0 ml) vorgelegt und HBTU (75.3 mg, 199 µmol) und N,N-Diisopropylethylamin (80 µl, 460 µmol) wurden anschließend zugegeben. Nach 15 min Rühren bei Raumtemperatur wurde (3R,4S)-3,4-Difluorpyrrolidinhydrochlorid (26.3 mg, 183 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 29.0 mg (46 % d. Th.) der Titelverbindung erhalten.
LC-MS (Methode 3): Rₜ = 1.41 min; MS (ESIpos): m/z = 417 [M+H]⁺
¹H-NMR (400 MHz, DMSO-d6) δ [ppm]: -0.149 (1.81), -0.008 (16.00), 0.008 (13.82), 0.146 (1.81), 1.654 (0.75), 1.728 (0.90), 2.012 (0.94), 2.327 (2.07), 2.366 (1.62), 2.523 (6.70), 2.562 (1.69), 2.603 (1.77), 2.645 (0.68), 2.669 (2.11), 2.709 (1.62), 3.487 (1.28), 3.615 (0.60), 3.685 (1.02), 3.699 (0.83), 3.715 (0.75), 3.937 (0.60), 3.987 (0.53), 4.170 (0.56), 4.795 (2.07), 4.830 (4.10), 4.840 (2.52), 4.888 (0.53), 5.387 (0.64), 7.282 (0.98), 7.307 (1.02), 7.537 (0.56), 7.565 (1.20), 7.581 (1.17), 7.605 (0.56).

### Beispiel 493

### (5S)-5-{[(3S)-3-Fluorpyrrolidin-1-yl]carbonyl}-2-(2,4,5-trifluorbenzyl)-5,6,7,8-tetrahydro[1,2,4]triazolo[4,3-a]pyridin-3(2H)-on

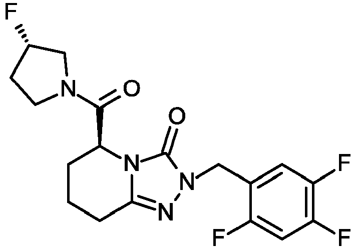

(5S)-3-Oxo-2-(2,4,5-trifluorbenzyl)-2,3,5,6,7,8-hexahydro[1,2,4]triazolo[4,3-a]pyridin-5-carbonsäure (50.0 mg, 153 µmol) wurde in THF (3.0 ml) vorgelegt und HBTU (75.3 mg, 199 µmol) und N,N-Diisopropylethylamin (80 µl, 460 µmol) wurden anschließend zugegeben. Nach 15 min Rühren bei Raumtemperatur wurde (3S)-3-Fluorpyrrolidinhydrochlorid (23.0 mg, 183 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 24.7 mg (41 % d. Th.) der Titelverbindung erhalten.
LC-MS (Methode 3): Rₜ = 1.35 min; MS (ESIpos): m/z = 399 [M+H]⁺
¹H-NMR (400 MHz, DMSO-d6) δ [ppm]: -0.149 (1.39), 0.008 (10.78), 0.146 (1.39), 0.934 (0.70), 1.728 (4.52), 1.910 (1.57), 2.073 (4.35), 2.266 (1.91), 2.327 (4.35), 2.366 (4.52), 2.524 (16.00), 2.580 (2.96), 2.609 (4.35), 2.670 (4.52), 2.710 (4.17), 3.395 (1.57), 3.465 (1.04), 3.493 (1.39), 3.631 (4.70), 3.651 (3.83), 3.697 (1.91), 3.741 (3.30), 3.767 (2.78), 3.851 (4.17), 4.695 (2.43), 4.737 (2.61), 4.752 (3.13), 4.761 (2.43), 4.786 (2.26), 4.825 (11.65), 4.846 (7.65), 4.885 (1.91), 5.259 (2.09), 5.390 (2.61), 5.510 (1.57), 7.263 (2.09), 7.284 (2.78), 7.306 (2.96), 7.329 (1.91), 7.540 (1.91), 7.565 (3.30), 7.582 (3.48), 7.607 (2.09).

### Beispiel 494

### (5S)-5-[(3-Fluorazetidin-1-yl)carbonyl]-2-{[2-(trifluormethyl)chinolin-4-yl]methyl}-5,6,7,8-tetrahydro[1,2,4]triazolo[4,3-a]pyridin-3(2H)-on

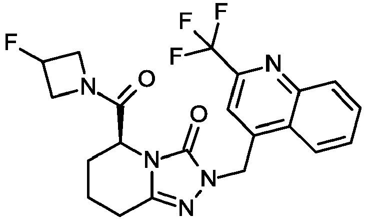

(5S)-3-Oxo-2-{[2-(trifluormethyl)chinolin-4-yl]methyl}-2,3,5,6,7,8-hexahydro[1,2,4]triazolo[4,3-a]pyridin-5-carbonsäure (60.0 mg, 153 µmol) wurde in THF (3.0 ml) vorgelegt und HBTU (69.6 mg, 184 µmol) und N,N-Diisopropylethylamin (80 µl, 460 µmol) wurden anschließend zugegeben. Nach 15 min Rühren bei Raumtemperatur wurde 3-Fluorazetidinhydrochlorid (25.6 mg, 229 µmol) zugegeben und das Reaktionsgemisch für 6 Tage bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 63.7 mg (93 % d. Th.) der Titelverbindung erhalten.
LC-MS (Methode 3): Rₜ = 1.50 min; MS (ESIpos): m/z = 450 [M+H]⁺
¹H-NMR (400 MHz, DMSO-d6) δ [ppm]: -0.149 (1.76), -0.008 (16.00), 0.008 (14.24), 0.146 (1.92), 1.686 (2.08), 1.710 (2.88), 1.988 (2.72), 2.073 (2.56), 2.327 (3.68), 2.366 (3.84), 2.523 (13.60), 2.586 (3.20), 2.598 (5.60), 2.611 (3.20), 2.628 (1.44), 2.641 (2.24), 2.670 (4.16), 2.710 (4.00), 3.943 (1.28), 4.002 (1.60), 4.272 (1.92), 4.298 (1.60), 4.330 (1.44), 4.358 (0.96), 4.407 (1.12), 4.472 (0.96), 4.524 (0.80), 4.608 (3.52), 4.620 (5.12), 4.632 (3.36), 5.356 (0.96), 5.423 (3.04), 5.463 (13.44), 5.472 (6.24), 5.484 (5.44), 5.513 (1.44), 7.747 (9.12), 7.846 (3.52), 7.863 (2.40), 7.948 (3.84), 7.966 (5.92), 7.984 (3.36), 8.214 (7.04), 8.236 (6.08), 8.368 (4.32), 8.389 (4.00).

### Beispiel 495

### (5S)-5-[(3-Hydroxyazetidin-1-yl)carbonyl]-2-{[2-(trifluormethyl)chinolin-4-yl]methyl}-5,6,7,8-tetrahydro[1,2,4]triazolo[4,3-a]pyridin-3(2H)-on

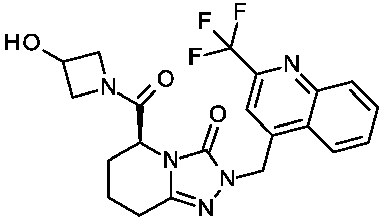

(5S)-3-Oxo-2-{[2-(trifluormethyl)chinolin-4-yl]methyl}-2,3,5,6,7,8-hexahydro[1,2,4]triazolo[4,3-a]pyridin-5-carbonsäure (60.0 mg, 153 µmol) wurde in THF (3.0 ml) vorgelegt und HBTU (75.4 mg, 199 µmol) und N,N-Diisopropylethylamin (80 µl, 460 µmol) wurden anschließend zugegeben. Nach 15 min Rühren bei Raumtemperatur wurde Azetidin-3-olhydrochlorid (20.1 mg, 184 µmol) zugegeben und das Reaktionsgemisch für 6 Tage bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 41.4 mg (61 % d. Th.) der Titelverbindung erhalten.
LC-MS (Methode 3): Rₜ = 1.29 min; MS (ESIpos): m/z = 448 [M+H]⁺
¹H-NMR (400 MHz, DMSO-d6) δ [ppm]: -0.149 (0.53), -0.008 (5.42), 0.008 (4.04), 0.146 (0.53), 1.651 (3.35), 1.717 (4.41), 1.919 (2.45), 1.931 (2.34), 1.944 (3.72), 1.954 (5.95), 1.966 (5.58), 1.998 (3.61), 2.008 (3.72), 2.017 (3.46), 2.073 (1.65), 2.328 (1.17), 2.333 (0.90), 2.367 (1.22), 2.557 (4.04), 2.582 (4.52), 2.594 (8.50), 2.605 (4.62), 2.623 (2.29), 2.635 (3.56), 2.647 (1.65), 2.670 (1.33), 2.711 (1.22), 3.611 (2.82), 3.619 (3.30), 3.637 (3.67), 3.645 (4.47), 3.661 (3.30), 3.676 (3.19), 3.686 (3.14), 3.933 (3.72), 3.945 (4.15), 3.966 (1.01), 4.035 (5.05), 4.054 (6.80), 4.062 (5.48), 4.077 (3.03), 4.115 (2.45), 4.131 (3.03), 4.141 (2.60), 4.156 (2.39), 4.341 (2.50), 4.358 (4.20), 4.379 (2.98), 4.485 (3.03), 4.501 (4.36), 4.518 (7.28), 4.533 (7.44), 4.566 (3.99), 4.582 (8.40), 4.591 (7.71), 4.606 (3.35), 5.413 (2.23), 5.420 (2.18), 5.454 (12.54), 5.461 (13.50), 5.469 (14.41), 5.474 (13.61), 5.510 (2.45), 5.516 (2.34), 5.811 (11.32), 5.826 (5.58), 7.745 (15.63), 7.753 (16.00), 7.829 (3.46), 7.847 (7.97), 7.864 (5.00), 7.945 (7.23), 7.966 (10.79), 7.984 (6.49), 8.214 (13.29), 8.235 (11.48), 8.363 (6.64), 8.373 (7.02), 8.384 (6.43), 8.394 (6.27).

### Beispiel 496

### (5S)-5-{[(3S)-3-Hydroxypyrrolidin-1-yl]carbonyl}-2-{[2-(trifluormethyl)chinolin-4-yl]methyl}-5,6,7,8-tetrahydro[1,2,4]triazolo[4,3-a]pyridin-3(2H)-on

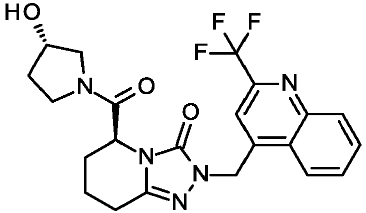

(5S)-3-Oxo-2-{[2-(trifluormethyl)chinolin-4-yl]methyl}-2,3,5,6,7,8-hexahydro[1,2,4]triazolo[4,3-a]pyridin-5-carbonsäure (60.0 mg, 153 µmol) wurde in THF (3.0 ml, 37 mmol) vorgelegt und HBTU (75.4 mg, 199 µmol) und N,N-Diisopropylethylamin (80 µl, 460 µmol) wurden anschließend zugegeben. Nach 15 min Rühren bei Raumtemperatur wurde (3S)-Pyrrolidin-3-olhydrochlorid (22.7 mg, 184 µmol) zugegeben und das Reaktionsgemisch für 6 Tage bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 62.3 mg (88 % d. Th.) der Titelverbindung erhalten.
LC-MS (Methode 3): Rₜ = 1.31 min; MS (ESIpos): m/z = 462 [M+H]⁺
¹H-NMR (400 MHz, DMSO-d6) δ [ppm]: -0.008 (3.53), 0.008 (2.56), 1.658 (1.13), 1.670 (1.16), 1.682 (1.13), 1.731 (2.20), 1.741 (2.13), 1.862 (1.43), 1.872 (1.73), 1.884 (1.43), 1.894 (1.83), 1.905 (1.43), 1.928 (0.50), 1.988 (2.20), 2.011 (2.23), 2.021 (2.63), 2.031 (2.56), 2.042 (2.03), 2.054 (1.36), 2.062 (1.56), 2.073 (16.00), 2.328 (0.77), 2.367 (0.57), 2.519 (4.39), 2.523 (4.56), 2.564 (1.66), 2.608 (2.89), 2.620 (1.66), 2.640 (0.96), 2.650 (1.33), 2.665 (1.03), 2.670 (0.96), 2.710 (0.63), 3.209 (0.40), 3.241 (0.60), 3.350 (2.69), 3.379 (2.23), 3.390 (1.20), 3.411 (0.93), 3.443 (0.83), 3.453 (0.86), 3.465 (1.06), 3.473 (1.50), 3.495 (0.93), 3.503 (0.90), 3.569 (1.53), 3.591 (0.93), 3.654 (1.40), 3.665 (2.06), 3.681 (1.50), 3.692 (1.60), 4.276 (1.50), 4.371 (1.60), 4.742 (1.23), 4.750 (1.43), 4.757 (1.53), 4.765 (1.23), 4.783 (1.30), 4.798 (1.70), 4.806 (1.16), 4.864 (0.47), 4.970 (2.10), 5.085 (2.03), 5.406 (1.46), 5.447 (4.79), 5.479 (6.35), 5.520 (1.80), 7.743 (10.31), 7.753 (2.59), 7.758 (1.86), 7.825 (1.53), 7.845 (3.29), 7.863 (2.16), 7.945 (2.96), 7.965 (4.46), 7.984 (2.66), 8.213 (5.52), 8.234 (4.72), 8.366 (2.93), 8.387 (2.79).

### Beispiel 497

### (5S)-5-{[(3R,4S)-3,4-Difluorpyrrolidin-1-yl]carbonyl}-2-{[2-(trifluormethyl)chinolin-4-yl]methyl}-5,6,7,8-tetrahydro[1,2,4]triazolo[4,3-a]pyridin-3(2H)-on

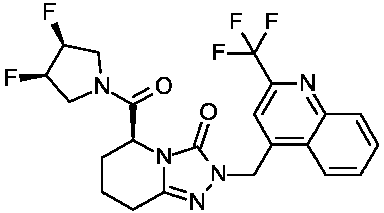

(5S)-3-Oxo-2-{[2-(trifluormethyl)chinolin-4-yl]methyl}-2,3,5,6,7,8-hexahydro[1,2,4]triazolo[4,3-a]pyridin-5-carbonsäure (60.0 mg, 153 µmol) wurde in THF (3.0 ml) vorgelegt und HBTU (75.4 mg, 199 µmol) und N,N-Diisopropylethylamin (80 µl, 460 µmol) wurden anschließend zugegeben. Nach 15 min Rühren bei Raumtemperatur wurde (3R,4S)-3,4-Difluorpyrrolidinhydrochlorid (26.3 mg, 184 µmol) zugegeben und das Reaktionsgemisch für 6 Tage bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 62.0 mg (84 % d. Th.) der Titelverbindung erhalten.
LC-MS (Methode 3): Rₜ = 1.60 min; MS (ESIpos): m/z = 482 [M+H]⁺
¹H-NMR (400 MHz, DMSO-d6) δ [ppm]: -0.149 (1.71), -0.008 (16.00), 0.008 (11.82), 0.147 (1.50), 1.648 (1.55), 1.733 (1.67), 2.023 (2.46), 2.328 (2.21), 2.367 (1.67), 2.572 (2.42), 2.609 (3.47), 2.670 (2.59), 2.710 (1.75), 3.501 (1.30), 3.522 (1.46), 3.641 (1.09), 3.696 (2.13), 3.729 (2.05), 3.773 (1.30), 3.930 (1.55), 3.978 (1.09), 4.189 (1.09), 4.854 (2.80), 4.867 (3.89), 4.878 (2.92), 5.265 (1.17), 5.357 (1.04), 5.415 (2.42), 5.456 (5.43), 5.464 (5.60), 5.478 (5.35), 5.492 (5.35), 5.534 (1.84), 7.548 (1.17), 7.597 (1.50), 7.627 (1.84), 7.644 (1.09), 7.735 (7.06), 7.743 (8.15), 7.849 (2.92), 7.870 (1.92), 7.946 (3.09), 7.966 (4.89), 7.983 (2.92), 8.215 (6.35), 8.236 (5.31), 8.359 (2.80), 8.367 (3.17), 8.380 (2.76), 8.388 (2.97).

### Beispiel 498

### (5S)-2-{[6-Fluor-2-(trifluormethyl)chinolin-4-yl]methyl}-5-(pyrrolidin-1-ylcarbonyl)-5,6,7,8-tetrahydro[1,2,4]triazolo[4,3-a]pyridin-3(2H)-on

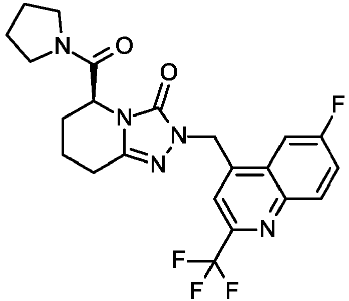

(5S)-2-{[6-Fluor-2-(trifluormethyl)chinolin-4-yl]methyl}-3-oxo-2,3,5,6,7,8-hexahydro[1,2,4]triazolo[4,3-a]pyridin-5-carbonsäure (44.0 mg, 107 µmol) wurde in THF (2.0 ml) bei Raumtemperatur vorgelegt. Anschließend wurden HATU (53.0 mg, 139 µmol) und Triethylamin (45 µl, 320 µmol) zugegeben. Nach 15 min Rühren wurde Pyrrolidin (11 µl, 130 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 28.6 mg (58 % d. Th.) der Titelverbindung erhalten.
LC-MS (Methode 3): Rₜ = 1.65 min; MS (ESIpos): m/z = 464 [M+H]⁺
¹H-NMR (400 MHz, DMSO-d6) δ [ppm]: -0.150 (1.01), 0.146 (1.06), 1.668 (2.28), 1.719 (3.21), 1.764 (2.32), 1.781 (6.04), 1.799 (10.55), 1.816 (8.53), 1.833 (2.66), 1.899 (2.58), 1.916 (7.89), 1.933 (10.26), 1.949 (6.25), 1.966 (1.98), 2.012 (5.91), 2.022 (5.99), 2.035 (3.80), 2.327 (2.62), 2.366 (1.94), 2.569 (3.29), 2.596 (3.08), 2.606 (4.98), 2.618 (3.17), 2.636 (1.60), 2.647 (2.32), 2.669 (2.96), 2.709 (2.07), 3.234 (1.52), 3.251 (3.29), 3.263 (3.29), 3.280 (5.91), 3.333 (3.55), 3.351 (5.78), 3.368 (3.21), 3.380 (3.42), 3.398 (1.60), 3.438 (1.77), 3.456 (3.67), 3.480 (4.64), 3.497 (2.20), 3.612 (2.07), 3.628 (4.56), 3.645 (2.70), 3.653 (3.63), 3.670 (1.65), 4.797 (4.18), 4.811 (5.87), 4.820 (3.93), 5.374 (2.74), 5.415 (12.37), 5.434 (12.50), 5.475 (2.70), 7.797 (16.00), 7.872 (2.53), 7.879 (2.79), 7.894 (4.18), 7.901 (4.69), 7.916 (3.00), 7.923 (3.04), 8.151 (4.98), 8.158 (5.15), 8.177 (5.11), 8.184 (5.02), 8.295 (4.69), 8.309 (4.90), 8.319 (4.73), 8.333 (4.43).

### Beispiel 499

### (5S)-5-[(3,3-Difluorazetidin-1-yl)carbonyl]-2-{[3-(trifluormethyl)-1,2,4-oxadiazol-5-yl]methyl}-5,6,7,8-tetrahydro[1,2,4]triazolo[4,3-a]pyridin-3(2H)-on

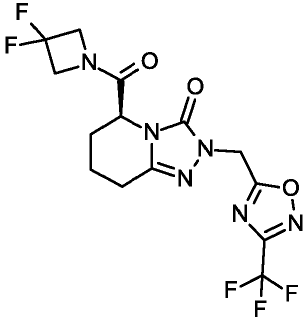

(5S)-3-Oxo-2-{[3-(trifluormethyl)-1,2,4-oxadiazol-5-yl]methyl}-2,3,5,6,7,8-hexahydro[1,2,4]triazolo[4,3-a]pyridin-5-carbonsäure (85.0 mg, 82 % Reinheit, 209 µmol) wurde in THF (3.0 ml) vorgelegt und HBTU (103 mg, 272 µmol) und N,N-Diisopropylethylamin (110 µl, 630 µmol) wurden anschließend zugegeben. Nach 15 min Rühren bei Raumtemperatur wurde 3,3-Difluorazetidinhydrochlorid (32.5 mg, 251 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 46.8 mg (55 % d. Th.) der Titelverbindung erhalten.
LC-MS (Methode 4): Rₜ = 0.73 min; MS (ESIpos): m/z = 409 [M+H]⁺
¹H-NMR (400 MHz, DMSO-d6) δ [ppm]: -0.008 (3.49), 0.008 (2.81), 1.723 (2.72), 1.737 (3.89), 1.753 (2.88), 1.766 (1.17), 1.963 (1.10), 1.975 (1.10), 1.987 (1.10), 1.999 (1.76), 2.012 (1.52), 2.023 (0.68), 2.048 (0.75), 2.063 (1.38), 2.083 (1.48), 2.098 (1.38), 2.117 (0.80), 2.327 (0.63), 2.366 (0.84), 2.523 (1.99), 2.576 (0.82), 2.601 (1.90), 2.619 (3.02), 2.629 (2.08), 2.637 (2.32), 2.643 (3.77), 2.657 (1.80), 2.670 (1.12), 2.686 (0.98), 2.700 (0.45), 2.710 (0.89), 4.339 (1.24), 4.376 (2.11), 4.400 (1.27), 4.620 (2.51), 4.635 (3.98), 4.648 (2.51), 4.727 (1.12), 4.758 (1.31), 4.787 (0.68), 4.803 (0.70), 4.835 (1.41), 4.863 (1.31), 5.392 (0.40), 5.437 (16.00), 5.481 (0.45).

### Beispiel 500

### (5S)-5-{[(3R,4S)-3,4-Difluorpyrrolidin-1-yl]carbonyl}-2-{[3-(trifluormethyl)-1,2,4-oxadiazol-5-yl]methyl}-5,6,7,8-tetrahydro[1,2,4]triazolo[4,3-a]pyridin-3(2H)-on

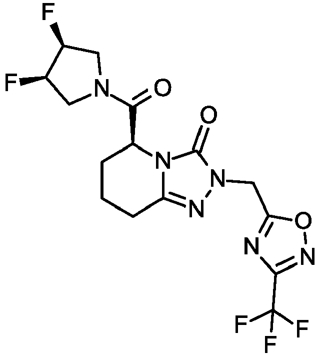

(5S)-3-Oxo-2-{[3-(trifluormethyl)-1,2,4-oxadiazol-5-yl]methyl}-2,3,5,6,7,8-hexahydro[1,2,4]triazolo[4,3-a]pyridin-5-carbonsäure (85.0 mg, 82 % Reinheit, 209 µmol) wurde in THF (3.0 ml) vorgelegt und HBTU (103 mg, 272 µmol) und N,N-Diisopropylethylamin (110 µl, 630 µmol) wurden anschließend zugegeben. Nach 15 min Rühren bei Raumtemperatur wurde (3R,4S)-3,4-Difluorpyrrolidinhydrochlorid (36.0 mg, 251 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 49.1 mg (56 % d. Th.) der Titelverbindung erhalten.
LC-MS (Methode 4): Rₜ = 0.72 min; MS (ESIpos): m/z = 423 [M+H]⁺
¹H-NMR (400 MHz, DMSO-d6) δ [ppm]: -0.149 (0.99), -0.008 (9.46), 0.008 (7.63), 0.146 (0.99), 1.690 (1.73), 1.744 (2.14), 1.972 (1.73), 2.012 (1.52), 2.076 (1.78), 2.111 (1.41), 2.327 (1.83), 2.366 (1.73), 2.522 (5.39), 2.602 (2.09), 2.611 (3.03), 2.626 (3.71), 2.638 (4.65), 2.652 (2.41), 2.669 (2.67), 2.690 (1.31), 2.710 (1.93), 3.456 (0.78), 3.519 (1.52), 3.528 (1.31), 3.538 (1.41), 3.571 (1.05), 3.616 (1.10), 3.630 (1.31), 3.648 (0.73), 3.685 (1.93), 3.705 (1.52), 3.717 (1.52), 3.726 (1.57), 3.743 (1.41), 3.757 (1.41), 3.777 (1.15), 3.870 (1.10), 3.905 (0.89), 3.927 (1.20), 3.942 (1.20), 3.957 (0.84), 3.977 (1.20), 3.991 (1.20), 4.007 (0.78), 4.020 (0.84), 4.126 (0.78), 4.141 (0.84), 4.168 (1.36), 4.181 (0.94), 4.193 (0.89), 4.209 (0.73), 4.819 (3.50), 4.829 (4.34), 4.835 (4.55), 4.844 (3.61), 5.266 (1.25), 5.377 (2.14), 5.416 (16.00), 5.419 (13.91), 5.427 (10.88), 5.470 (1.78).

### Beispiel 501

### (5S)-5-[(3,3-Difluorpyrrolidin-1-yl)carbonyl]-2-{[3-(trifluormethyl)-1,2,4-oxadiazol-5-yl]methyl}-5,6,7,8-tetrahydro[1,2,4]triazolo[4,3-a]pyridin-3(2H)-on

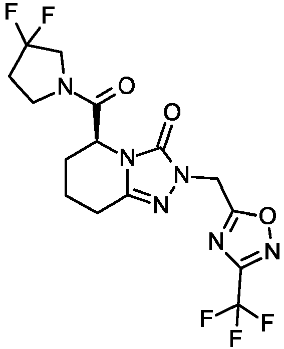

(5S)-3-Oxo-2-{[3-(trifluormethyl)-1,2,4-oxadiazol-5-yl]methyl}-2,3,5,6,7,8-hexahydro[1,2,4]triazolo[4,3-a]pyridin-5-carbonsäure (85.0 mg, 82 % Reinheit, 209 µmol) wurde in THF (3.0 ml) vorgelegt und HBTU (103 mg, 272 µmol) und N,N-Diisopropylethylamin (110 µl, 630 µmol) wurden anschließend zugegeben. Nach 15 min Rühren bei Raumtemperatur wurde 3,3-Difluorpyrrolidinhydrochlorid (36.0 mg, 251 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 69.8 mg (77 % d. Th.) der Titelverbindung erhalten.
LC-MS (Methode 3): Rₜ = 1.44 min; MS (ESIpos): m/z = 423 [M+H]⁺
¹H-NMR (400 MHz, DMSO-d6) δ [ppm]: -0.149 (0.41), -0.008 (3.87), 0.008 (3.12), 0.146 (0.41), 1.704 (1.58), 1.719 (1.84), 1.732 (2.09), 1.741 (2.09), 1.978 (1.42), 1.991 (1.56), 2.002 (1.64), 2.013 (1.44), 2.028 (1.46), 2.052 (1.01), 2.063 (1.30), 2.072 (1.66), 2.078 (1.40), 2.088 (1.34), 2.098 (1.09), 2.107 (1.15), 2.327 (0.79), 2.366 (1.13), 2.380 (1.09), 2.408 (1.40), 2.428 (1.38), 2.450 (1.01), 2.568 (2.17), 2.593 (2.88), 2.608 (3.12), 2.617 (2.94), 2.631 (3.44), 2.640 (3.71), 2.652 (1.94), 2.669 (1.44), 2.683 (1.03), 2.710 (0.95), 3.532 (2.57), 3.551 (4.23), 3.570 (2.05), 3.637 (0.47), 3.670 (1.52), 3.704 (1.66), 3.737 (1.50), 3.768 (2.33), 3.786 (1.36), 3.802 (2.11), 3.811 (1.88), 3.830 (0.97), 3.887 (0.87), 3.906 (1.76), 3.925 (1.03), 3.931 (1.22), 3.950 (0.55), 3.965 (0.57), 3.994 (1.09), 4.007 (0.55), 4.022 (0.83), 4.037 (1.01), 4.065 (0.63), 4.146 (0.69), 4.179 (1.03), 4.202 (0.99), 4.235 (0.47), 4.783 (1.44), 4.793 (1.70), 4.798 (1.88), 4.808 (1.38), 4.858 (1.82), 4.873 (2.27), 4.883 (1.46), 5.375 (0.85), 5.419 (15.23), 5.423 (16.00), 5.467 (1.03), 5.632 (0.45).

### Beispiel 502

### (5S)-2-[(1RS)-1-(4-Methylphenyl)ethyl]-5-(pyrrolidin-1-ylcarbonyl)-5,6,7,8-tetrahydro[1,2,4]triazolo[4,3-a]pyridin-3(2H)-on (Diastereomerengemisch, 2 Isomere)

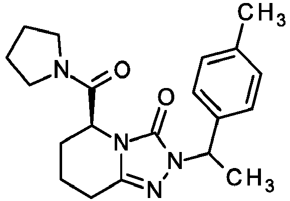

(5S)-2-[(1RS)-1-(4-Methylphenyl)ethyl]-3-oxo-2,3,5,6,7,8-hexahydro[1,2,4]triazolo[4,3-a]pyridin-5-carbonsäure (Isomer 1) (58.0 mg, 192 µmol) wurde in THF (2.3 ml) bei Raumtemperatur vorgelegt. Anschließend wurden HBTU (94.9 mg, 250 µmol) und N,N-Diisopropylethylamin (130 µl, 770 µmol) zugegeben. Nach 15 min Rühren wurde Pyrrolidin (19 µl, 230 µmol) zugegeben und das Reaktionsgemisch über Nacht bei Raumtemperatur gerührt. Das Reaktionsgemisch wurde mit Wasser und Ethylacetat versetzt. Die organische Phase wurde abgetrennt und die wässrige Phase dreimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 49.8 mg (73 % d. Th.) der Titelverbindung erhalten.
LC-MS (Methode 3): Rₜ = 1.46 min; MS (ESIpos): m/z = 355 [M+H]⁺
¹H-NMR (400 MHz, DMSO-d6) δ [ppm]: -0.149 (1.79), -0.008 (16.00), 0.008 (13.92), 0.146 (1.84), 1.575 (7.98), 1.593 (8.07), 1.710 (1.84), 1.755 (2.17), 1.772 (3.54), 1.789 (2.64), 1.896 (2.31), 1.912 (3.02), 1.929 (2.12), 2.267 (15.10), 2.327 (2.64), 2.366 (1.89), 2.523 (8.12), 2.615 (1.79), 2.670 (2.78), 2.709 (1.98), 3.214 (1.18), 3.226 (1.13), 3.244 (1.94), 3.261 (1.04), 3.343 (1.42), 3.442 (1.23), 3.467 (1.42), 3.592 (1.37), 4.706 (1.60), 4.716 (1.23), 5.225 (1.94), 5.243 (1.75), 7.108 (2.64), 7.128 (5.71), 7.160 (6.75), 7.180 (2.88).

### Beispiel 503

### (5S)-5-(1,3-Thiazolidin-3-ylcarbonyl)-2-{[6-(trifluormethyl)pyridin-3-yl]methyl}-5,6,7,8-tetrahydro[1,2,4]triazolo[4,3-a]pyridin-3(2H)-on

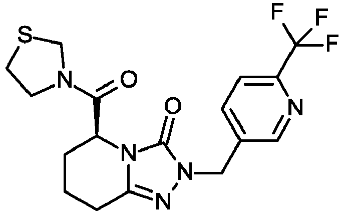

(5S)-3-Oxo-2-{[6-(trifluormethyl)pyridin-3-yl]methyl}-2,3,5,6,7,8-hexahydro[1,2,4]triazolo[4,3-a]pyridin-5-carbonsäure (100 mg, 292 µmol) wurde in THF (2.0 ml) vorgelegt und HBTU (144 mg, 380 µmol) und N,N-Diisopropylethylamin (150 µl, 880 µmol) wurden anschließend zugegeben. Nach 15 min Rühren bei Raumtemperatur wurde 1,3-Thiazolidin (31.3 mg, 351 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 36.5 mg (30 % d. Th.) der Titelverbindung erhalten.
LC-MS (Methode 1): Rₜ = 0.97 min; MS (ESIpos): m/z = 414 [M+H]⁺
¹H-NMR (400 MHz, DMSO-d6) δ [ppm]: -0.149 (0.48), -0.008 (3.99), 0.008 (4.42), 0.146 (0.48), 1.619 (0.94), 1.743 (1.45), 1.988 (0.73), 2.020 (1.88), 2.327 (0.94), 2.366 (0.67), 2.567 (2.15), 2.579 (2.06), 2.593 (1.91), 2.613 (2.66), 2.624 (1.54), 2.669 (1.30), 2.710 (0.73), 2.914 (0.42), 3.003 (1.45), 3.019 (3.12), 3.035 (1.72), 3.130 (1.97), 3.146 (4.14), 3.161 (2.21), 3.595 (0.45), 3.611 (0.64), 3.626 (0.82), 3.640 (1.12), 3.655 (0.54), 3.677 (0.60), 3.695 (1.12), 3.712 (0.73), 3.724 (0.60), 3.758 (0.51), 3.773 (0.97), 3.785 (0.88), 3.799 (1.21), 3.814 (0.60), 3.929 (0.57), 3.943 (1.21), 3.958 (0.82), 3.970 (0.94), 3.986 (0.42), 4.371 (1.88), 4.396 (2.42), 4.557 (2.54), 4.583 (2.00), 4.611 (1.42), 4.634 (1.72), 4.809 (1.69), 4.832 (1.42), 4.893 (1.36), 4.972 (1.51), 5.015 (10.37), 5.058 (0.48), 7.910 (15.09), 7.913 (16.00), 8.642 (5.60).

### Beispiel 504

### (5S)-2-[(5-Brompyridin-2-yl)methyl]-5-{[(3R,4S)-3,4-difluorpyrrolidin-1-yl]carbonyl}-5,6,7,8-tetrahydro[1,2,4]triazolo[4,3-a]pyridin-3(2H)-on

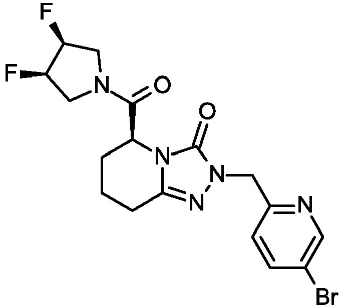

(5S)-2-[(5-Brompyridin-2-yl)methyl]-3-oxo-2,3,5,6,7,8-hexahydro[1,2,4]triazolo[4,3-a]pyridin-5-carbonsäure (100 mg, 283 µmol) wurde in THF (23 µl) vorgelegt und (3R,4S)-3,4-Difluorpyrrolidinhydrochlorid (48.8 mg, 340 µmol) und (200 µl, 1.4 mmol) wurden anschließend zugegeben. Nach 15 min Rühren bei Raumtemperatur wurde HBTU (140 mg, 368 µmol) zugegeben und das Reaktionsgemisch für 4 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 36.5 mg (30 % d. Th.) der Titelverbindung erhalten.
LC-MS (Methode 3): Rₜ = 0.97 min; MS (ESIpos): m/z = 442 [M+H]⁺
¹H-NMR (400 MHz, DMSO-d6) delta [ppm]: -0.149 (1.70), -0.008 (12.77), 0.008 (16.00), 0.146 (1.62), 1.740 (1.45), 2.015 (1.45), 2.073 (2.13), 2.327 (2.89), 2.366 (3.06), 2.571 (2.55), 2.595 (2.30), 2.605 (2.98), 2.648 (1.11), 2.669 (3.06), 2.710 (2.98), 3.491 (1.11), 3.513 (0.94), 3.632 (0.85), 3.687 (1.45), 3.720 (1.19), 3.785 (0.77), 3.946 (0.77), 3.996 (0.68), 4.174 (0.68), 4.808 (2.81), 4.886 (13.53), 5.275 (0.77), 5.385 (0.85), 5.450 (0.60), 7.145 (3.23), 7.165 (3.57), 8.034 (2.64), 8.055 (2.55), 8.650 (3.15).

### Beispiel 505

### (5S)-2-[(5-Chlorpyridin-2-yl)methyl]-5-(1,3-thiazolidin-3-ylcarbonyl)-5,6,7,8-tetrahydro[1,2,4]triazolo[4,3-a]pyridin-3(2H)-on

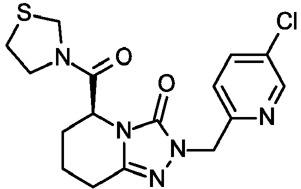

(5S)-2-[(5-Chlorpyridin-2-yl)methyl]-3-oxo-2,3,5,6,7,8-hexahydro[1,2,4]triazolo[4,3-a]pyridin-5-carbonsäure (100 mg, 324 µmol) wurde in THF (2.0 ml) vorgelegt und HBTU (160 mg, 421 µmol) und N,N-Diisopropylethylamin (170 µl, 970 µmol) wurden anschließend zugegeben. Nach 15 min Rühren bei Raumtemperatur wurde 1,3-Thiazolidin (34.7 mg, 389 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 45.0 mg (37 % d. Th.) der Titelverbindung erhalten.
LC-MS (Methode 3): Rₜ = 1.15 min; MS (ESIpos): m/z = 380 [M+H]⁺
¹H-NMR (400 MHz, DMSO-d6) δ [ppm]: -0.008 (2.72), 0.008 (2.67), 1.245 (0.93), 1.261 (1.57), 1.278 (0.76), 1.661 (0.95), 1.746 (1.42), 1.796 (0.77), 1.980 (0.76), 2.016 (1.80), 2.073 (1.11), 2.327 (0.55), 2.524 (1.84), 2.578 (2.03), 2.592 (2.04), 2.610 (2.62), 2.622 (1.51), 2.640 (0.63), 2.652 (0.92), 2.665 (0.84), 2.913 (0.50), 3.002 (1.47), 3.018 (3.08), 3.034 (1.74), 3.130 (2.02), 3.146 (4.22), 3.161 (2.18), 3.596 (0.43), 3.614 (0.67), 3.629 (0.91), 3.644 (1.14), 3.659 (0.60), 3.673 (0.63), 3.690 (1.14), 3.707 (0.71), 3.720 (0.60), 3.761 (0.51), 3.777 (0.94), 3.788 (0.91), 3.803 (1.24), 3.818 (0.61), 3.932 (0.60), 3.947 (1.19), 3.961 (0.83), 3.973 (0.92), 3.989 (0.43), 4.244 (0.93), 4.373 (1.89), 4.399 (2.43), 4.486 (0.45), 4.553 (2.48), 4.578 (1.94), 4.618 (1.41), 4.640 (1.72), 4.808 (1.68), 4.831 (1.34), 4.875 (1.22), 4.891 (1.45), 4.900 (1.35), 4.916 (16.00), 4.958 (1.10), 4.974 (1.11), 7.200 (4.68), 7.221 (5.05), 7.914 (4.06), 7.920 (4.18), 7.935 (3.90), 7.941 (4.01), 8.572 (4.54), 8.578 (4.62).

### Beispiel 506

### (5S)-2-[(5-Brompyridin-3-yl)methyl]-5-{[(3R,4S)-3,4-difluorpyrrolidin-1-yl]carbonyl}-5,6,7,8-tetrahydro[1,2,4]triazolo[4,3-a]pyridin-3(2H)-on

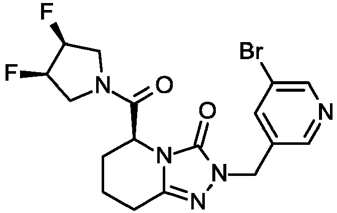

(5S)-2-[(5-Brompyridin-3-yl)methyl]-3-oxo-2,3,5,6,7,8-hexahydro[1,2,4]triazolo[4,3-a]pyridin-5-carbonsäure (100 mg, 283 µmol) wurde in THF (1.7 ml) vorgelegt und HBTU (140 mg, 368 µmol) und N,N-Diisopropylethylamin (250 µl, 1.4 mmol) wurden anschließend zugegeben. Nach 15 min Rühren bei Raumtemperatur wurde (3R,4S)-3,4-Difluorpyrrolidinhydrochlorid (48.8 mg, 340 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 51.1 mg (41 % d. Th.) der Titelverbindung erhalten.
LC-MS (Methode 3): Rₜ = 1.19 min; MS (ESIpos): m/z = 442 [M+H]⁺
¹H-NMR (400 MHz, DMSO-d6) δ [ppm]: -0.149 (0.64), -0.008 (5.43), 0.008 (5.61), 0.146 (0.67), 1.664 (0.94), 1.732 (1.25), 2.014 (1.37), 2.051 (1.07), 2.067 (0.90), 2.086 (0.77), 2.327 (0.97), 2.366 (0.94), 2.382 (0.97), 2.412 (1.01), 2.431 (0.99), 2.454 (0.88), 2.572 (3.14), 2.583 (2.36), 2.598 (2.26), 2.614 (2.51), 2.669 (1.31), 2.710 (0.71), 3.532 (1.05), 3.541 (1.22), 3.550 (1.83), 3.562 (2.06), 3.580 (0.92), 3.670 (1.18), 3.704 (1.31), 3.746 (0.75), 3.779 (1.91), 3.809 (2.08), 3.826 (0.69), 3.893 (0.60), 3.911 (1.31), 3.937 (0.99), 3.956 (0.54), 3.990 (0.82), 4.018 (0.56), 4.033 (0.77), 4.061 (0.47), 4.149 (0.45), 4.181 (0.75), 4.206 (0.71), 4.765 (1.03), 4.780 (1.44), 4.790 (1.12), 4.837 (1.12), 4.852 (1.50), 4.861 (1.12), 4.905 (16.00), 7.887 (3.24), 7.892 (5.73), 7.897 (3.52), 8.446 (5.61), 8.450 (5.65), 8.639 (5.35), 8.644 (5.37).

### Beispiel 507

### (5S)-2-[(5-Chlor-3-fluorpyridin-2-yl)methyl]-5-(1,3-thiazolidin-3-ylcarbonyl)-5,6,7,8-tetrahydro[1,2,4]triazolo[4,3-a]pyridin-3(2H)-on

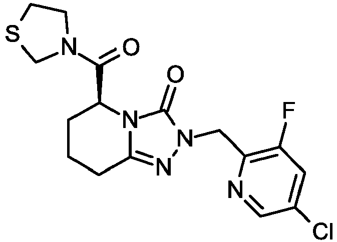

(5S)-2-[(5-Chlor-3-fluorpyridin-2-yl)methyl]-3-oxo-2,3,5,6,7,8-hexahydro[1,2,4]triazolo[4,3-a]pyridin-5-carbonsäure (100 mg, 306 µmol) wurde in THF (4.6 ml) vorgelegt und HBTU (139 mg, 367 µmol) und N,N-Diisopropylethylamin (160 µl, 920 µmol) wurden anschließend zugegeben. Nach 15 min Rühren bei Raumtemperatur wurde 1,3-Thiazolidin (35.5 mg, 398 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 40.0 mg (33 % d. Th.) der Titelverbindung erhalten.
LC-MS (Methode 3): Rₜ = 1.19 min; MS (ESIpos): m/z = 398 [M+H]⁺
¹H-NMR (400 MHz, DMSO-d6) δ [ppm]: -0.149 (1.06), -0.008 (9.48), 0.008 (8.72), 0.147 (1.06), 1.653 (2.73), 1.711 (4.09), 1.949 (2.12), 1.983 (4.70), 2.046 (3.03), 2.073 (3.49), 2.327 (2.88), 2.366 (0.76), 2.470 (2.35), 2.525 (8.95), 2.569 (7.73), 2.581 (4.40), 2.599 (1.97), 2.612 (2.65), 2.670 (3.11), 2.710 (1.14), 2.744 (0.76), 2.800 (0.91), 2.998 (3.79), 3.014 (8.27), 3.030 (4.63), 3.128 (5.31), 3.144 (11.37), 3.159 (5.84), 3.610 (1.44), 3.625 (2.27), 3.640 (3.03), 3.663 (1.82), 3.681 (3.11), 3.698 (1.82), 3.711 (1.44), 3.752 (1.36), 3.767 (2.58), 3.780 (2.27), 3.794 (3.18), 3.810 (1.59), 3.924 (1.59), 3.940 (3.18), 3.952 (2.20), 3.966 (2.35), 3.981 (1.14), 4.367 (5.00), 4.392 (6.82), 4.543 (6.67), 4.568 (5.23), 4.609 (3.72), 4.632 (4.40), 4.797 (4.32), 4.820 (3.64), 4.859 (3.79), 4.900 (5.61), 4.905 (5.84), 4.939 (16.00), 4.943 (15.17), 4.991 (11.98), 5.030 (4.85), 8.089 (8.42), 8.094 (8.95), 8.113 (8.87), 8.118 (8.95), 8.478 (9.33), 8.482 (9.25).

### Beispiel 508

### (5S)-2-[(5-Chlorpyridin-2-yl)methyl]-5-{[(3S)-3-fluorpyrrolidin-1-yl]carbonyl}-5,6,7,8-tetrahydro[1,2,4]triazolo[4,3-a]pyridin-3(2H)-on

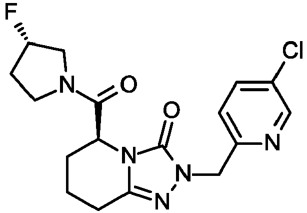

(5S)-2-[(5-Chlorpyridin-2-yl)methyl]-3-oxo-2,3,5,6,7,8-hexahydro[1,2,4]triazolo[4,3-a]pyridin-5-carbonsäure (100 mg, 324 µmol) wurde in THF (2.0 ml) vorgelegt und HBTU (160 mg, 421 µmol) und N,N-Diisopropylethylamin (280 µl, 1.6 mmol) wurden anschließend zugegeben. Nach 15 min Rühren bei Raumtemperatur wurde (3S)-3-Fluorpyrrolidinhydrochlorid (48.8 mg, 389 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 67.0 mg (54 % d. Th.) der Titelverbindung erhalten.
LC-MS (Methode 3): Rₜ = 1.06 min; MS (ESIpos): m/z = 380 [M+H]⁺
¹H-NMR (400 MHz, DMSO-d6) δ [ppm]: -0.149 (0.82), -0.008 (6.88), 0.008 (6.28), 0.146 (0.82), 1.743 (4.02), 1.883 (0.88), 1.930 (1.00), 1.997 (1.06), 2.031 (1.66), 2.109 (2.66), 2.138 (2.02), 2.269 (1.42), 2.327 (1.63), 2.366 (0.69), 2.572 (2.81), 2.588 (2.17), 2.612 (3.32), 2.654 (1.30), 2.669 (1.87), 2.709 (0.69), 2.881 (0.42), 3.276 (1.24), 3.350 (1.18), 3.372 (1.03), 3.399 (1.12), 3.407 (1.09), 3.469 (0.91), 3.496 (1.12), 3.505 (1.09), 3.612 (0.72), 3.636 (3.38), 3.655 (2.90), 3.681 (1.87), 3.697 (1.36), 3.732 (2.17), 3.752 (2.35), 3.778 (1.99), 3.794 (1.57), 3.860 (3.29), 4.695 (1.54), 4.704 (1.93), 4.710 (2.14), 4.720 (1.57), 4.752 (2.11), 4.761 (2.32), 4.767 (2.66), 4.776 (1.96), 4.865 (1.21), 4.906 (16.00), 4.913 (11.14), 4.955 (0.91), 5.259 (1.66), 5.389 (2.05), 5.516 (1.09), 5.944 (0.39), 7.200 (6.85), 7.221 (7.37), 7.913 (5.80), 7.920 (5.89), 7.934 (5.40), 7.941 (5.68), 8.572 (6.67), 8.578 (6.70).

### Beispiel 509

### (5S)-2-[(5-Brompyridin-3-yl)methyl]-5-{[(3S)-3-fluorpyrrolidin-1-yl]carbonyl}-5,6,7,8-tetrahydro[1,2,4]triazolo[4,3-a]pyridin-3(2H)-on

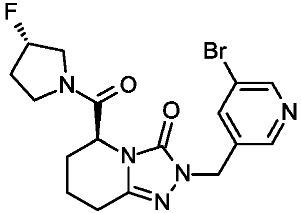

(5S)-2-[(5-Brompyridin-3-yl)methyl]-3-oxo-2,3,5,6,7,8-hexahydro[1,2,4]triazolo[4,3-a]pyridin-5-carbonsäure (100 mg, 283 µmol) wurde in THF (1.7 ml) vorgelegt und HBTU (140 mg, 368 µmol) und N,N-Diisopropylethylamin (250 µl, 1.4 mmol) wurden anschließend zugegeben. Nach 15 min Rühren bei Raumtemperatur wurde (3S)-3-Fluorpyrrolidinhydrochlorid (42.7 mg, 340 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 42.7 mg (36 % d. Th.) der Titelverbindung erhalten.
LC-MS (Methode 3): Rₜ = 1.05 min; MS (ESIpos): m/z = 424 [M+H]⁺
¹H-NMR (400 MHz, DMSO-d6) δ [ppm]: -0.149 (1.52), -0.008 (13.23), 0.008 (14.75), 0.146 (1.57), 1.734 (2.39), 1.910 (0.81), 2.027 (1.25), 2.103 (2.17), 2.137 (1.57), 2.270 (1.08), 2.327 (2.22), 2.366 (1.46), 2.565 (2.12), 2.592 (1.63), 2.616 (2.44), 2.669 (2.77), 2.710 (1.46), 3.397 (0.87), 3.468 (0.71), 3.495 (0.87), 3.638 (2.66), 3.662 (2.01), 3.679 (1.63), 3.703 (1.08), 3.723 (1.14), 3.744 (2.44), 3.769 (1.63), 3.854 (2.28), 4.694 (1.14), 4.709 (1.52), 4.719 (1.19), 4.751 (1.46), 4.766 (1.90), 4.775 (1.46), 4.901 (16.00), 5.259 (1.25), 5.390 (1.63), 5.515 (0.87), 7.893 (4.99), 8.448 (5.91), 8.639 (4.72), 8.644 (4.99).

### Beispiel 510

### (5S)-2-[(5-Brompyridin-2-yl)methyl]-5-{[(3S)-3-fluorpyrrolidin-1-yl]carbonyl}-5,6,7,8-tetrahydro[1,2,4]triazolo[4,3-a]pyridin-3(2H)-on

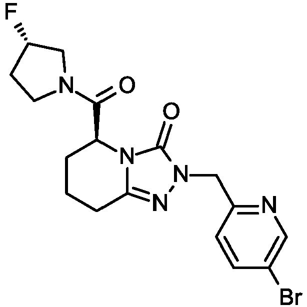

(5S)-2-[(5-Brompyridin-2-yl)methyl]-3-oxo-2,3,5,6,7,8-hexahydro[1,2,4]triazolo[4,3-a]pyridin-5-carbonsäure (100 mg, 283 µmol) wurde in THF (23 µl) vorgelegt und (3S)-3-Fluorpyrrolidinhydrochlorid (42.7 mg, 340 µmol) und (3S)-3-Fluorpyrrolidinhydrochlorid (200 µl, 1.4 mmol) wurden anschließend zugegeben. Nach 15 min Rühren bei Raumtemperatur wurde HBTU (140 mg, 368 µmol) zugegeben und das Reaktionsgemisch für 4 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 56.0 mg (47 % d. Th.) der Titelverbindung erhalten.
LC-MS (Methode 3): Rₜ = 1.13 min; MS (ESIpos): m/z = 424 [M+H]⁺
¹H-NMR (400 MHz, DMSO-d6) δ [ppm]: -0.149 (0.76), -0.008 (6.64), 0.008 (6.30), 0.146 (0.76), 1.729 (3.63), 1.742 (4.16), 1.882 (0.86), 1.921 (1.05), 1.993 (1.10), 2.034 (1.81), 2.072 (2.05), 2.101 (2.77), 2.138 (2.15), 2.219 (1.19), 2.269 (1.48), 2.327 (1.72), 2.366 (0.91), 2.571 (3.10), 2.586 (2.39), 2.610 (3.58), 2.651 (1.29), 2.669 (2.05), 2.710 (1.10), 3.274 (1.19), 3.349 (1.53), 3.362 (1.29), 3.371 (1.29), 3.398 (1.24), 3.407 (1.29), 3.468 (0.96), 3.495 (1.19), 3.504 (1.15), 3.610 (0.81), 3.635 (3.68), 3.656 (3.06), 3.681 (2.01), 3.701 (1.58), 3.730 (2.24), 3.750 (2.58), 3.778 (2.10), 3.794 (1.67), 3.826 (0.57), 3.860 (3.49), 4.693 (1.72), 4.702 (2.05), 4.708 (2.20), 4.717 (1.72), 4.750 (2.10), 4.759 (2.48), 4.765 (2.77), 4.774 (2.01), 4.841 (1.39), 4.882 (16.00), 4.889 (11.27), 4.930 (1.05), 5.258 (1.62), 5.390 (2.24), 5.515 (1.29), 7.144 (7.02), 7.165 (7.55), 8.032 (5.68), 8.038 (5.83), 8.053 (5.59), 8.059 (5.64), 8.648 (6.83), 8.654 (6.93).

### Beispiel 511

### (2S)-1-{[(5S)-3-Oxo-2-{[6-(trifluormethyl)pyridin-3-yl]methyl}-2,3,5,6,7,8-hexahydro[1,2,4]triazolo[4,3-a]pyridin-5-yl]carbonyl}pyrrolidin-2-carbonitril

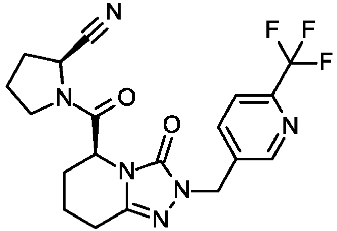

(5S)-3-Oxo-2-{[6-(trifluormethyl)pyridin-3-yl]methyl}-2,3,5,6,7,8-hexahydro[1,2,4]triazolo[4,3-a]pyridin-5-carbonsäure (97.0 mg, 283 µmol) wurde in THF (2.0 ml) vorgelegt und HBTU (140 mg, 368 µmol) und N,N-Diisopropylethylamin (150 µl, 850 µmol) wurden anschließend zugegeben. Nach 15 min Rühren bei Raumtemperatur wurde (2S)-Pyrrolidin-2-carbonitrilhydrochlorid (45.1 mg, 340 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 36.0 mg (30 % d. Th.) der Titelverbindung erhalten.
LC-MS (Methode 1): Rₜ = 0.97 min; MS (ESIpos): m/z = 421 [M+H]⁺
¹H-NMR (400 MHz, DMSO-d6) δ [ppm]: -0.149 (1.50), 0.146 (1.50), 1.244 (1.05), 1.260 (1.05), 1.641 (1.50), 1.780 (1.64), 2.045 (4.49), 2.061 (6.88), 2.077 (5.98), 2.094 (2.99), 2.117 (2.54), 2.136 (2.39), 2.150 (2.99), 2.161 (2.54), 2.189 (1.35), 2.209 (2.99), 2.228 (2.84), 2.240 (1.79), 2.259 (1.50), 2.327 (3.89), 2.366 (2.84), 2.570 (3.29), 2.586 (2.39), 2.597 (2.39), 2.610 (2.09), 2.646 (3.14), 2.669 (4.34), 2.710 (3.14), 3.676 (4.64), 3.692 (9.27), 3.709 (4.64), 4.798 (2.99), 4.809 (3.44), 4.818 (4.49), 4.830 (4.93), 4.847 (2.54), 4.971 (1.05), 5.011 (9.12), 5.021 (8.52), 5.061 (1.05), 7.915 (16.00), 8.642 (6.13).

### Beispiel 512

### (2S)-1-({(5S)-2-[(5-Chlorpyridin-2-yl)methyl]-3-oxo-2,3,5,6,7,8-hexahydro[1,2,4]triazolo[4,3-a]pyridin-5-yl}carbonyl)pyrrolidin-2-carbonitril

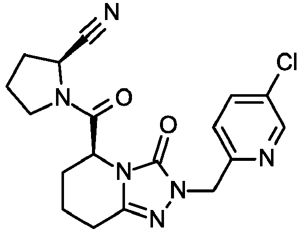

(5S)-2-[(5-Chlorpyridin-2-yl)methyl]-3-oxo-2,3,5,6,7,8-hexahydro[1,2,4]triazolo[4,3-a]pyridin-5-carbonsäure (100 mg, 324 µmol) wurde in THF (2.0 ml) vorgelegt und HBTU (160 mg, 421 µmol) und N,N-Diisopropylethylamin (170 µl, 970 µmol) wurden anschließend zugegeben. Nach 15 min Rühren bei Raumtemperatur wurde (2S)-Pyrrolidin-2-carbonitril (37.4 mg, 389 µmol) zugegeben und das Reaktionsgemisch für 72 Stunden bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 31.0 mg (25 % d. Th.) der Titelverbindung erhalten.
LC-MS (Methode 3): Rₜ = 1.16 min; MS (ESIpos): m/z = 387 [M+H]⁺
¹H-NMR (400 MHz, DMSO-d6) δ [ppm]: -0.149 (0.55), -0.008 (4.70), 0.008 (4.51), 0.146 (0.56), 1.675 (0.88), 1.791 (1.13), 2.028 (1.26), 2.047 (3.33), 2.063 (4.78), 2.079 (4.08), 2.095 (1.90), 2.106 (1.56), 2.120 (1.82), 2.130 (1.52), 2.137 (1.65), 2.151 (2.09), 2.163 (1.80), 2.177 (0.77), 2.185 (0.98), 2.205 (2.07), 2.225 (2.01), 2.237 (1.20), 2.244 (0.73), 2.256 (0.96), 2.327 (0.85), 2.569 (1.65), 2.584 (1.45), 2.595 (1.58), 2.609 (1.26), 2.628 (1.15), 2.640 (2.16), 2.653 (1.26), 2.670 (1.33), 2.682 (1.02), 3.679 (3.23), 3.695 (6.66), 3.712 (3.27), 4.791 (2.37), 4.802 (2.50), 4.811 (2.76), 4.821 (3.53), 4.828 (2.41), 4.834 (2.61), 4.843 (1.88), 4.915 (16.00), 7.195 (4.32), 7.216 (4.66), 7.921 (3.06), 7.927 (3.05), 7.942 (2.91), 7.949 (2.99), 8.573 (3.80), 8.579 (3.82).

### Beispiel 513

### (2S)-1-({(5S)-2-[(3,5-Dichlorpyridin-2-yl)methyl]-3-oxo-2,3,5,6,7,8-hexahydro[1,2,4]triazolo[4,3-a]pyridin-5-yl}carbonyl)pyrrolidin-2-carbonitril

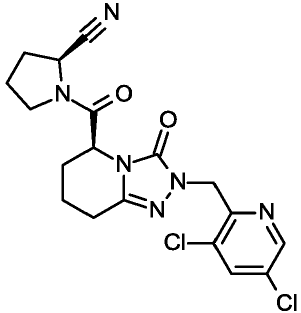

(5S)-2-[(3,5-Dichlorpyridin-2-yl)methyl]-3-oxo-2,3,5,6,7,8-hexahydro[1,2,4]triazolo[4,3-a]pyridin-5-carbonsäure (70.0 mg, 204 µmol) wurde in THF (1.7 ml) vorgelegt und HBTU (155 mg, 408 µmol) und N,N-Diisopropylethylamin (140 µl, 820 µmol) wurden anschließend zugegeben. Nach 15 min Rühren bei Raumtemperatur wurde (2S)-Pyrrolidin-2-carbonitrilhydrochlorid (40.6 mg, 306 µmol) zugeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und der Rückstand wurde über präparative HPLC aufgereinigt (Methode 10). Die produkthaltigen Fraktionen wurden im Vakuum eingeengt und es wurden 7.50 mg (94 % Reinheit, 8 % d. Th.) der Titelverbindung erhalten.
LC-MS (Methode 4): Rₜ = 0.70 min; MS (ESIpos): m/z = 421 [M+H]⁺
¹H-NMR (400 MHz, DMSO-d6) δ [ppm]: -0.149 (1.95), -0.008 (16.00), 0.008 (15.18), 0.146 (1.88), 1.665 (0.69), 1.759 (0.88), 2.045 (2.07), 2.060 (2.89), 2.077 (2.95), 2.093 (1.63), 2.136 (1.19), 2.149 (1.57), 2.161 (1.19), 2.181 (0.75), 2.201 (1.57), 2.221 (1.44), 2.233 (0.94), 2.252 (0.88), 2.327 (2.38), 2.366 (2.51), 2.577 (1.38), 2.606 (1.88), 2.617 (1.13), 2.669 (2.51), 2.710 (2.45), 3.672 (2.51), 3.689 (5.02), 3.706 (2.32), 4.780 (1.63), 4.791 (1.95), 4.801 (3.39), 4.811 (3.26), 4.826 (1.44), 4.964 (1.19), 5.004 (6.40), 5.018 (6.53), 5.058 (1.25), 8.251 (2.51), 8.256 (2.70), 8.566 (2.89), 8.572 (2.82).

### Beispiel 514

### (2S)-1-({(5S)-2-[(5-Chlor-3-fluorpyridin-2-yl)methyl]-3-oxo-2,3,5,6,7,8-hexahydro[1,2,4]triazolo[4,3-a]pyridin-5-yl}carbonyl)pyrrolidin-2-carbonitril

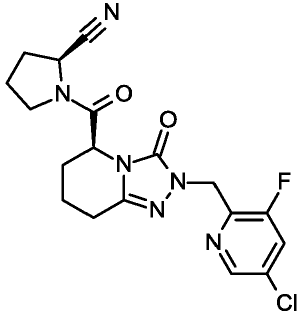

(5S)-2-[(5-Chlor-3-fluorpyridin-2-yl)methyl]-3-oxo-2,3,5,6,7,8-hexahydro[1,2,4]triazolo[4,3-a]pyridin-5-carbonsäure (100 mg, 306 µmol) wurde in THF (4.6 ml) vorgelegt und HBTU (139 mg, 367 µmol) und N,N-Diisopropylethylamin (160 µl, 920 µmol) wurden anschließend zugegeben. Nach 15 min Rühren bei Raumtemperatur wurde (2S)-Pyrrolidin-2-carbonitril (38.3 mg, 398 µmol) zugegeben und das Reaktionsgemisch für 72 Stunden gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 35.0 mg (28 % d. Th.) der Titelverbindung erhalten.
LC-MS (Methode 3): Rₜ = 1.19 min; MS (ESIpos): m/z = 405[M+H]⁺
¹H-NMR (400 MHz, DMSO-d6) δ [ppm]: -0.008 (3.01), 0.008 (2.62), 1.247 (1.39), 1.264 (2.35), 1.281 (1.26), 1.655 (2.13), 1.746 (2.54), 1.757 (2.62), 1.772 (1.94), 1.982 (1.68), 2.001 (2.40), 2.017 (3.51), 2.025 (4.06), 2.046 (5.71), 2.059 (9.52), 2.078 (9.69), 2.094 (5.49), 2.104 (3.31), 2.115 (3.01), 2.129 (2.61), 2.137 (3.62), 2.149 (4.47), 2.160 (3.59), 2.176 (1.91), 2.182 (2.56), 2.202 (4.62), 2.214 (1.69), 2.221 (4.22), 2.234 (2.64), 2.241 (1.72), 2.253 (2.06), 2.274 (0.86), 2.287 (0.55), 2.328 (1.08), 2.567 (3.13), 2.584 (3.05), 2.597 (5.37), 2.610 (2.96), 2.627 (1.37), 2.639 (2.19), 2.651 (0.94), 2.665 (0.85), 2.670 (1.04), 3.502 (0.56), 3.521 (0.44), 3.670 (7.94), 3.687 (16.00), 3.703 (7.75), 4.739 (0.60), 4.783 (6.98), 4.786 (5.83), 4.793 (8.96), 4.802 (12.61), 4.812 (9.58), 4.910 (3.03), 4.915 (3.01), 4.949 (10.52), 4.953 (10.25), 4.975 (10.53), 4.979 (10.66), 5.014 (3.03), 5.019 (2.99), 5.040 (0.48), 6.973 (0.87), 7.101 (0.90), 7.229 (0.82), 8.090 (7.00), 8.095 (7.61), 8.114 (7.09), 8.118 (7.72), 8.480 (9.30), 8.483 (9.78).

### Beispiel 515

### (5S)-5-{[(3S)-3-Fluorpyrrolidin-1-yl]carbonyl}-2-{[5-(trifluormethyl)pyridin-3-yl]methyl}-5,6,7,8-tetrahydro[1,2,4]triazolo[4,3-a]pyridin-3(2H)-on

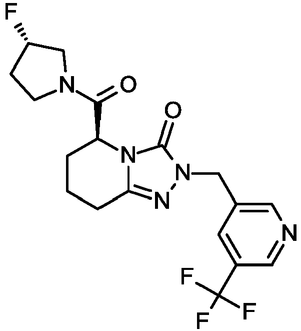

(5S)-3-Oxo-2-{[5-(trifluormethyl)pyridin-3-yl]methyl}-2,3,5,6,7,8-hexahydro[1,2,4]triazolo[4,3-a]pyridin-5-carbonsäure (46.5 mg, 136 µmol) wurde in THF (11 µl) vorgelegt und (3S)-3-Fluorpyrrolidinhydrochlorid (20.5 mg, 163 µmol) und (3S)-3-Fluorpyrrolidinhydrochlorid (95 µl, 680 µmol) wurden anschließend zugegeben. Nach 15 min Rühren bei Raumtemperatur wurde HBTU (67.0 mg, 177 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und der Rückstand wurde über präparative HPLC aufgereinigt (Methode 11). Die produkthaltigen Fraktionen wurden im Vakuum eingeengt und es wurden 29.5 mg (53 % d. Th.) der Titelverbindung erhalten.
LC-MS (Methode 3): Rₜ = 1.16 min; MS (ESIpos): m/z = 414 [M+H]⁺
¹H-NMR (400 MHz, DMSO-d6) δ [ppm]: 1.695 (1.26), 1.734 (2.23), 1.888 (0.52), 1.924 (0.76), 1.999 (0.84), 2.034 (1.38), 2.047 (1.33), 2.068 (1.37), 2.088 (1.85), 2.104 (2.27), 2.138 (1.59), 2.221 (0.85), 2.242 (0.78), 2.256 (0.80), 2.270 (1.14), 2.327 (0.56), 2.569 (1.57), 2.577 (1.70), 2.591 (1.48), 2.617 (2.57), 2.660 (1.13), 2.670 (0.97), 3.361 (1.30), 3.371 (1.17), 3.398 (1.01), 3.406 (0.99), 3.460 (0.64), 3.468 (0.71), 3.495 (0.89), 3.504 (0.86), 3.613 (0.59), 3.630 (2.11), 3.637 (2.26), 3.654 (2.41), 3.666 (1.56), 3.681 (1.50), 3.698 (1.11), 3.725 (1.28), 3.746 (1.94), 3.770 (1.64), 3.784 (1.49), 3.855 (2.48), 4.703 (1.17), 4.712 (1.42), 4.719 (1.49), 4.728 (1.18), 4.762 (1.50), 4.770 (1.68), 4.777 (1.93), 4.786 (1.45), 5.017 (16.00), 5.260 (1.25), 5.384 (1.52), 5.391 (1.57), 5.512 (0.89), 8.059 (5.28), 8.742 (5.83), 8.917 (5.45).

### Beispiel 516

### (5S)-5-{[(3S)-3-Fluorpyrrolidin-1-yl]carbonyl}-2-[2-(4-methylphenyl)-2-oxoethyl]-5,6,7,8-tetrahydro[1,2,4]triazolo[4,3-a]pyridin-3(2H)-on

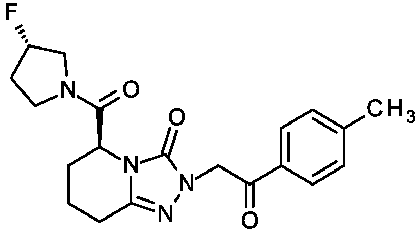

(5S)-2-[2-(4-Methylphenyl)-2-oxoethyl]-3-oxo-2,3,5,6,7,8-hexahydro[1,2,4]triazolo[4,3-a]pyridin-5-carbonsäure (121 mg, 383 µmol) wurde in THF (7.0 ml) vorgelegt und HBTU (189 mg, 498 µmol) und N,N-Diisopropylethylamin (200 µl, 1.1 mmol) wurden anschließend zugegeben. Nach 15 min Rühren bei Raumtemperatur wurde (3S)-3-Fluorpyrrolidinhydrochlorid (1:1) (57.7 mg, 459 µmol) zugegeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 77.3 mg (52 % d. Th.) der Titelverbindung erhalten.
LC-MS (Methode 3): Rₜ = 1.27 min; MS (ESIpos): m/z = 387 [M+H]⁺
¹H-NMR (400 MHz, DMSO-d6) δ [ppm]: -0.149 (1.36), -0.008 (16.00), 0.008 (8.62), 0.146 (1.10), 1.760 (1.10), 2.110 (0.79), 2.327 (2.33), 2.366 (3.25), 2.397 (11.03), 2.523 (12.00), 2.577 (0.97), 2.597 (1.23), 2.622 (1.14), 2.669 (1.89), 2.709 (1.76), 3.658 (0.75), 3.757 (0.57), 3.870 (0.75), 4.704 (0.48), 4.758 (0.57), 5.186 (4.75), 5.254 (0.40), 5.384 (0.48), 7.358 (2.46), 7.378 (2.55), 7.908 (2.59), 7.927 (2.37).

### Beispiel 517

### (5S)-2-[2,2-Difluor-2-(4-methylphenyl)ethyl]-5-{[(3S)-3-fluorpyrrolidin-1-yl]carbonyl}-5,6,7,8-tetrahydro[1,2,4]triazolo[4,3-a]pyridin-3(2H)-on

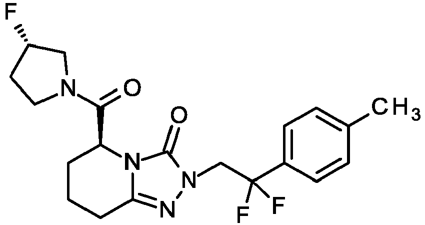

(5S)-5-{[(3S)-3-Fluorpyrrolidin-1-yl]carbonyl}-2-[2-(4-methylphenyl)-2-oxoethyl]-5,6,7,8-tetrahydro[1,2,4]triazolo[4,3-a]pyridin-3(2H)-on (51.5 mg, 133 µmol) wurde in 1,2-Dichlorethan (2.0 ml) gelöst, Diethylaminoschwefeltrifluorid (1.0 ml, 90 % Reinheit, 6.8 mmol) und 1 Tropfen Ethanol wurden zugegeben. Das Reaktionsgemisch wurde für 3 Stunden bei 70 °C gerührt. Das Reaktionsgemisch wurde in eine wässrige gesättigte Natriumhydrogencarbonat-Lösung getropft. Es wurde Dichlormethan zugegeben und die organische Phase abgetrennt. Die organische Phase wurde mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 30mm; Laufmittel: Acetonitril/Wasser-Gradient). Die produkthaltigen Fraktionen wurden im Vakuum eingeengt und es wurden 16.8 mg (31 % d. Th.) der Titelverbindung erhalten.
LC-MS (Methode 3): Rₜ = 1.49 min; MS (ESIpos): m/z = 409 [M+H]⁺
¹H-NMR (400 MHz, DMSO-d6) δ [ppm]: 0.007 (6.14), 1.148 (1.48), 1.729 (2.36), 2.084 (1.37), 2.347 (16.00), 2.365 (1.48), 2.568 (2.08), 2.608 (1.70), 2.669 (1.37), 2.710 (0.99), 3.615 (1.92), 3.640 (1.48), 3.726 (1.32), 3.750 (1.04), 3.839 (1.70), 4.275 (1.26), 4.315 (1.64), 4.658 (0.99), 4.716 (1.26), 5.383 (1.15), 7.288 (3.84), 7.308 (5.81), 7.393 (4.88), 7.413 (3.18).

### Die folgenden Beispiele 518- 546 wurde wie in Beispiel 517 beschrieben dargestellt.

### Beispiel 518

### (5S)-2-{2-[3-Chlor-5-(trifluormethyl)pyridin-2-yl]ethyl}-5-[(3-fluorazetidin-1-yl)carbonyl]-5,6,7,8-tetrahydro[1,2,4]triazolo[4,3-a]pyridin-3(2H)-on

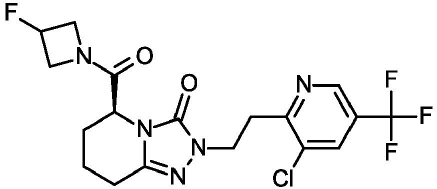

(5S)-2-{2-[3-Chlor-5-(trifluormethyl)pyridin-2-yl]ethyl}-3-oxo-2,3,5,6,7,8-hexahydro[1,2,4]triazolo[4,3-a]pyridin-5-carbonsäure (75.0 mg, 72 % Reinheit, 138 µmol), N,N-Diisopropylethylamin (110 µl, 620 µmol), HBTU (99.6 mg, 263 µmol), 3-Fluorazetidinhydrochlorid (1:1) (26.2 mg, 235 µmol). Rühren über Nacht bei Raumtemperatur. Es wurden nach Aufreinigung 44.1 mg (71 % d. Th.) der Titelverbindung erhalten.

LC-MS (Methode 3): Rₜ = 1.43 min; MS (ESIpos): m/z = 448 [M+H]⁺¹H-NMR (400 MHz, DMSO-d6) δ [ppm]: -0.149 (3.72), 0.146 (3.72), 1.707 (9.96), 1.931 (3.82), 1.992 (4.03), 2.072 (3.02), 2.327 (5.53), 2.366 (4.03), 2.584 (7.95), 2.625 (2.72), 2.669 (5.53), 2.709 (3.52), 3.283 (11.07), 3.910 (2.92), 4.024 (5.94), 4.041 (13.08), 4.059 (9.96), 4.235 (3.62), 4.324 (2.72), 4.441 (6.14), 4.454 (9.86), 4.468 (6.44), 4.658 (1.81), 5.385 (1.81), 5.517 (1.81), 8.413 (16.00), 8.876 (14.79).

### Beispiel 519

### (5S)-5-[(3,3-Difluorpyrrolidin-1-yl)carbonyl]-2-[2-(4-fluorphenyl)ethyl]-5,6,7,8-tetrahydro[1,2,4]triazolo[4,3-a]pyridin-3(2H)-on

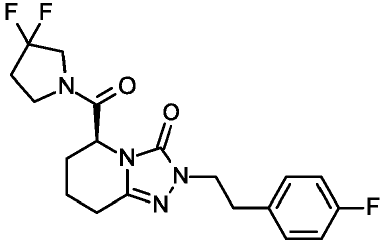

(5S)-2-[2-(4-Fluorphenyl)ethyl]-3-oxo-2,3,5,6,7,8-hexahydro[1,2,4]triazolo[4,3-a]pyridin-5-carbonsäure (73.1 mg, 240 µmol), N,N-Diisopropylethylamin (130 µl, 720 µmol), HBTU (118 mg, 311 µmol), 3,3-Difluorpyrrolidinhydrochlorid (1:1) (41.3 mg, 287 µmol). Rühren für 72 Stunden bei Raumtemperatur. Es wurden nach Aufreinigung 53.2 mg (56 % d. Th.) der Titelverbindung erhalten.
LC-MS (Methode 3): Rₜ = 1.48 min; MS (ESIpos): m/z = 395 [M+H]⁺
¹H-NMR (400 MHz, DMSO-d6) δ [ppm]: 0.008 (2.61), 1.683 (1.93), 1.709 (3.74), 1.891 (1.36), 1.971 (3.06), 2.011 (1.82), 2.026 (1.70), 2.037 (1.82), 2.328 (1.93), 2.353 (0.68), 2.367 (2.16), 2.400 (1.59), 2.523 (7.72), 2.567 (4.43), 2.576 (4.99), 2.591 (3.40), 2.604 (2.50), 2.616 (4.31), 2.660 (2.16), 2.670 (2.84), 2.710 (1.70), 2.895 (5.90), 2.914 (12.26), 2.932 (6.47), 3.518 (3.29), 3.538 (5.22), 3.557 (2.50), 3.623 (0.68), 3.658 (1.93), 3.691 (2.16), 3.721 (1.48), 3.751 (4.20), 3.767 (5.56), 3.785 (13.39), 3.803 (10.55), 3.822 (3.86), 3.838 (2.04), 3.857 (1.13), 3.882 (2.27), 3.909 (1.70), 3.928 (0.79), 3.949 (0.68), 3.979 (1.48), 4.008 (1.02), 4.020 (1.36), 4.050 (0.79), 4.115 (0.79), 4.147 (1.36), 4.174 (1.36), 4.204 (0.57), 4.668 (1.82), 4.683 (2.61), 4.693 (1.82), 4.744 (1.93), 4.759 (2.61), 4.769 (1.82), 7.062 (7.15), 7.085 (16.00), 7.107 (10.33), 7.203 (8.74), 7.217 (9.87), 7.225 (8.17), 7.239 (6.70).

### Beispiel 520

### (5S)-5-[(3-Fluorazetidin-1-yl)carbonyl]-2-[2-(4-fluorphenyl)ethyl]-5,6,7,8-tetrahydro[1,2,4]triazolo[4,3-a]pyridin-3(2H)-on

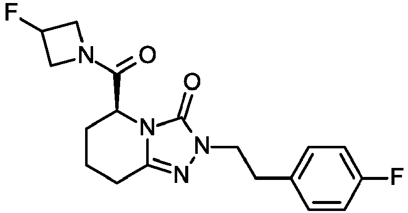

(5S)-2-[2-(4-Fluorphenyl)ethyl]-3-oxo-2,3,5,6,7,8-hexahydro[1,2,4]triazolo[4,3-a]pyridin-5-carbonsäure (73.1 mg, 240 µmol), N,N-Diisopropylethylamin (130 µl, 720 µmol), HBTU (118 mg, 311 µmol), 3-Fluorazetidinhydrochlorid (1:1) (32.1 mg, 287 µmol). Rühren für 72 Stunden bei Raumtemperatur. Es wurden nach Aufreinigung 37.7 mg (43 % d. Th.) der Titelverbindung erhalten.
LC-MS (Methode 3): Rₜ = 1.49 min; MS (ESIpos): m/z = 363 [M+H]⁺
¹H-NMR (400 MHz, DMSO-d6) δ [ppm]: -0.149 (2.06), -0.008 (15.54), 0.008 (16.00), 0.146 (2.06), 1.715 (3.20), 1.943 (1.26), 1.972 (1.14), 1.991 (1.37), 2.006 (1.26), 2.328 (2.40), 2.366 (1.94), 2.523 (7.43), 2.567 (2.74), 2.588 (1.83), 2.606 (2.97), 2.619 (1.71), 2.648 (1.03), 2.670 (2.74), 2.710 (1.94), 2.898 (2.74), 2.917 (5.60), 2.935 (3.09), 3.782 (2.17), 3.799 (4.00), 3.969 (0.80), 4.220 (1.14), 4.238 (1.14), 4.277 (0.80), 4.411 (0.57), 4.448 (2.40), 4.462 (3.43), 4.475 (2.17), 4.663 (0.46), 5.332 (0.57), 5.520 (0.57), 7.064 (2.29), 7.085 (5.03), 7.106 (3.09), 7.223 (4.00).

### Beispiel 521

### (5S)-2-[2-(4-Methylphenyl)ethyl]-5-(pyrrolidin-1-ylcarbonyl)-5,6,7,8-tetrahydro[1,2,4]triazolo[4,3-a]pyridin-3(2H)-on

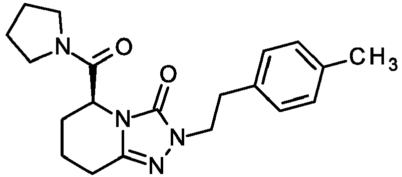

(5S)-2-[2-(4-Methylphenyl)ethyl]-3-oxo-2,3,5,6,7,8-hexahydro[1,2,4]triazolo[4,3-a]pyridin-5-carbonsäure (200 mg, 664 µmol), N,N-Diisopropylethylamin (460 µl, 2.7 mmol), HBTU (327 mg, 863 µmol), Pyrrolidin (66 µl, 800 µmol). Rühren über Nacht bei Raumtemperatur. Es wurden nach Aufreinigung 163 mg (69 % d. Th.) der Titelverbindung erhalten.
LC-MS (Methode 3): Rₜ = 1.45 min; MS (ESIpos): m/z = 355 [M+H]⁺
¹H-NMR (400 MHz, DMSO-d6) δ [ppm]: -0.008 (1.86), 0.008 (1.55), 1.703 (0.76), 1.714 (1.01), 1.733 (0.71), 1.746 (0.55), 1.764 (1.00), 1.781 (1.71), 1.798 (1.34), 1.815 (0.41), 1.883 (0.41), 1.900 (1.27), 1.916 (1.67), 1.933 (1.07), 1.951 (0.54), 1.962 (0.61), 1.973 (0.54), 1.982 (0.43), 1.997 (0.44), 2.018 (0.40), 2.257 (9.36), 2.560 (0.68), 2.580 (0.44), 2.602 (0.42), 2.613 (0.87), 2.626 (0.44), 2.848 (1.00), 2.868 (1.97), 2.887 (1.03), 3.233 (0.51), 3.246 (0.52), 3.263 (0.91), 3.280 (0.45), 3.328 (1.08), 3.346 (0.51), 3.357 (0.53), 3.440 (0.58), 3.447 (0.42), 3.465 (0.74), 3.589 (0.70), 3.606 (0.41), 3.613 (0.54), 3.729 (0.62), 3.748 (0.95), 3.758 (0.65), 3.766 (0.59), 3.777 (0.97), 3.797 (0.54), 4.659 (0.65), 4.669 (0.75), 4.675 (0.88), 4.684 (0.64), 7.085 (16.00).

### Beispiel 522

### (5S)-5-{[(3R,4S)-3,4-Difluorpyrrolidin-1-yl]carbonyl}-2-[2-(4-methoxyphenyl)ethyl]-5,6,7,8-tetrahydro[1,2,4]triazolo[4,3-a]pyridin-3(2H)-on

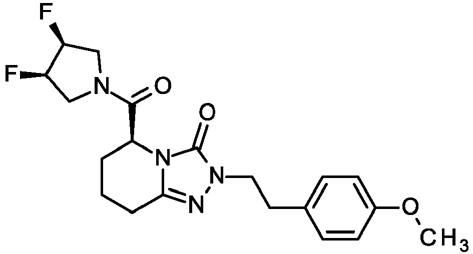

(5S)-2-[2-(4-Methoxyphenyl)ethyl]-3-oxo-2,3,5,6,7,8-hexahydro[1,2,4]triazolo[4,3-a]pyridin-5-carbonsäure (92.0 mg, 290 µmol), N,N-Diisopropylethylamin (150 µl, 870 µmol), HBTU (143 mg, 377 µmol), (3R,4S)-3,4-Difluorpyrrolidinhydrochlorid (49.9 mg, 348 µmol). Rühren Für 72 Stunden bei Raumtemperatur. Es wurden nach Aufreinigung 71.1 mg (60 % d. Th.) der Titelverbindung erhalten.
LC-MS (Methode 4): Rₜ = 0.72 min; MS (ESIpos): m/z = 407 [M+H]⁺
¹H-NMR (400 MHz, DMSO-d6) δ [ppm]: 1.680 (0.47), 1.704 (0.90), 1.715 (1.09), 1.727 (1.01), 1.939 (0.47), 1.976 (0.55), 1.986 (0.65), 1.998 (0.48), 2.012 (0.44), 2.021 (0.42), 2.028 (0.46), 2.036 (0.44), 2.045 (0.41), 2.073 (1.37), 2.563 (0.77), 2.575 (0.79), 2.586 (0.63), 2.620 (1.09), 2.631 (0.63), 2.663 (0.51), 2.830 (1.48), 2.848 (2.93), 2.867 (1.56), 3.477 (0.48), 3.499 (0.42), 3.509 (0.44), 3.532 (0.49), 3.673 (0.68), 3.689 (0.68), 3.715 (16.00), 3.742 (2.34), 3.750 (1.16), 3.761 (1.70), 3.769 (1.37), 3.782 (0.78), 3.804 (0.47), 4.152 (0.43), 4.710 (0.96), 4.723 (1.33), 4.734 (0.92), 6.828 (2.79), 6.846 (2.98), 6.850 (2.86), 7.095 (2.14), 7.104 (2.47), 7.116 (1.95), 7.125 (2.09).

### Beispiel 523

### (5S)-5-[(3,3-Difluorazetidin-1-yl)carbonyl]-2-[2-(4-methoxyphenyl)ethyl]-5,6,7,8-tetrahydro[1,2,4]triazolo[4,3-a]pyridin-3(2H)-on

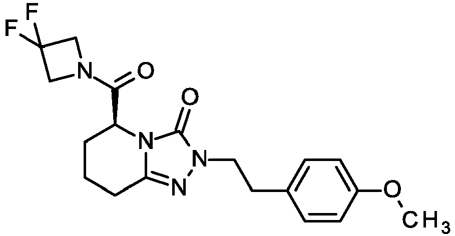

(5S)-2-[2-(4-Methoxyphenyl)ethyl]-3-oxo-2,3,5,6,7,8-hexahydro[1,2,4]triazolo[4,3-a]pyridin-5-carbonsäure (92.0 mg, 290 µmol), N,N-Diisopropylethylamin (150 µl, 870 µmol), HBTU (143 mg, 377 µmol), 3,3-Difluorazetidinhydrochlorid (45.1 mg, 348 µmol). Rühren für 72 Stunden bei Raumtemperatur. Es wurden nach Aufreinigung 64.5 mg (57 % d. Th.) der Titelverbindung erhalten.
LC-MS (Methode 3): Rₜ = 1.46 min; MS (ESIpos): m/z = 393 [M+H]⁺
¹H-NMR (400 MHz, DMSO-d6) δ [ppm]: -0.008 (0.90), 0.008 (0.75), 1.711 (0.65), 1.726 (1.00), 1.738 (0.92), 1.749 (0.63), 1.960 (0.54), 1.971 (0.50), 2.016 (0.41), 2.028 (0.40), 2.037 (0.48), 2.569 (0.65), 2.589 (0.83), 2.605 (0.98), 2.618 (1.19), 2.632 (0.57), 2.835 (1.24), 2.854 (2.48), 2.872 (1.35), 3.713 (16.00), 3.743 (0.87), 3.750 (0.92), 3.760 (1.69), 3.770 (1.68), 3.779 (0.83), 3.789 (0.76), 4.342 (0.64), 4.357 (0.64), 4.501 (0.86), 4.515 (1.44), 4.528 (0.85), 4.700 (0.45), 4.807 (0.43), 6.825 (3.10), 6.847 (3.66), 6.854 (0.44), 7.102 (3.30), 7.123 (2.85).

### Beispiel 524

### (5S)-5-[(3,3-Difluorpyrrolidin-1-yl)carbonyl]-2-{[1-(4-methylphenyl)cyclopropyl]methyl}-5,6,7,8-tetrahydro[1,2,4]triazolo[4,3-a]pyridin-3(2H)-on

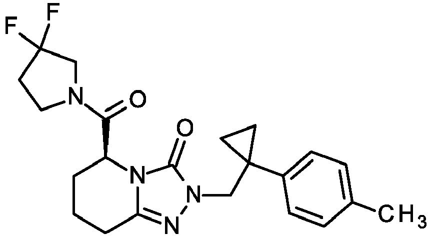

(5S)-2-{[1-(4-Methylphenyl)cyclopropyl]methyl}-3-oxo-2,3,5,6,7,8-hexahydro[1,2,4]triazolo[4,3-a]pyridin-5-carbonsäure (45.0 mg, 137 µmol), N,N-Diisopropylethylamin (72 µl, 410 µmol), HBTU (67.8 mg, 179 µmol), 3,3-Difluorpyrrolidinhydrochlorid (23.7 mg, 165 µmol). Rühren über Nacht bei Raumtemperatur. Es wurden nach Aufreinigung 27.3 mg (48 % d. Th.) der Titelverbindung erhalten.
LC-MS (Methode 3): Rₜ = 1.72 min; MS (ESIpos): m/z = 417 [M+H]⁺
¹H-NMR (400 MHz, DMSO-d6) δ [ppm]: -0.008 (2.67), 0.008 (2.32), 0.677 (0.52), 0.687 (0.71), 0.702 (1.17), 0.713 (1.14), 0.720 (0.89), 0.786 (1.23), 0.796 (1.02), 0.811 (0.74), 0.821 (0.61), 0.921 (0.53), 0.931 (0.55), 0.945 (1.24), 0.955 (1.62), 0.966 (2.20), 0.974 (1.56), 0.988 (1.08), 0.998 (0.49), 1.662 (0.70), 1.688 (1.08), 1.958 (0.93), 1.972 (0.93), 1.985 (0.91), 2.012 (0.58), 2.022 (0.44), 2.073 (1.06), 2.235 (16.00), 2.327 (0.53), 2.366 (0.85), 2.393 (0.59), 2.416 (0.56), 2.435 (0.42), 2.467 (0.58), 2.523 (2.70), 2.566 (1.36), 2.580 (1.61), 2.622 (0.61), 2.669 (0.58), 2.710 (0.45), 3.509 (0.99), 3.528 (1.58), 3.549 (0.71), 3.644 (1.67), 3.649 (1.91), 3.680 (2.35), 3.685 (2.14), 3.712 (0.55), 3.744 (1.27), 3.772 (1.06), 3.869 (0.74), 3.895 (0.58), 3.915 (1.88), 3.922 (1.67), 3.951 (1.38), 3.958 (1.74), 4.000 (0.47), 4.133 (0.47), 4.159 (0.42), 4.657 (0.64), 4.671 (0.88), 4.681 (0.62), 4.731 (0.67), 4.747 (0.85), 4.756 (0.62), 7.025 (3.25), 7.045 (5.44), 7.105 (6.96), 7.125 (4.16).

### Beispiel 525

### (5S)-5-{[(3S)-3-Hydroxypyrrolidin-1-yl]carbonyl}-2-{[1-(4-methylphenyl)cyclopropyl]methyl}-5,6,7,8-tetrahydro[1,2,4]triazolo[4,3-a]pyridin-3(2H)-on

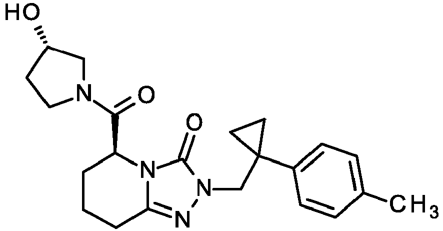

(5S)-2-{[1-(4-Methylphenyl)cyclopropyl]methyl}-3-oxo-2,3,5,6,7,8-hexahydro[1,2,4]triazolo[4,3-a]pyridin-5-carbonsäure (45.0 mg, 137 µmol), N,N-Diisopropylethylamin (72 µl, 410 µmol), HBTU (67.8 mg, 179 µmol), (3S)-Pyrrolidin-3-olhydrochlorid (1:1) (20.4 mg, 165 µmol). Rühren über Nacht bei Raumtemperatur. Es wurden nach Aufreinigung 24.4 mg (45 % d. Th.) der Titelverbindung erhalten.
LC-MS (Methode 4): Rₜ = 0.74 min; MS (ESIpos): m/z = 397 [M+H]⁺
¹H-NMR (400 MHz, DMSO-d6) δ [ppm]: -0.008 (2.87), 0.008 (2.23), 0.683 (0.77), 0.706 (1.23), 0.786 (1.29), 0.809 (0.80), 0.820 (0.58), 0.921 (0.45), 0.933 (0.56), 0.944 (1.00), 0.956 (1.70), 0.966 (2.06), 0.992 (0.79), 1.387 (0.55), 1.692 (1.47), 1.703 (1.54), 1.752 (0.58), 1.762 (0.51), 1.831 (0.77), 1.842 (0.86), 1.853 (0.73), 1.863 (0.91), 1.874 (0.74), 1.930 (0.79), 1.964 (1.45), 1.975 (1.42), 1.987 (1.40), 1.997 (0.95), 2.013 (0.66), 2.072 (1.15), 2.236 (16.00), 2.327 (0.57), 2.366 (0.53), 2.447 (0.64), 2.464 (0.80), 2.523 (1.85), 2.565 (0.86), 2.577 (1.36), 2.619 (0.60), 2.669 (0.51), 2.709 (0.44), 3.243 (0.42), 3.276 (2.35), 3.287 (2.17), 3.297 (1.50), 3.404 (1.13), 3.413 (0.96), 3.427 (0.92), 3.434 (1.00), 3.456 (0.55), 3.464 (0.45), 3.528 (0.58), 3.549 (0.45), 3.606 (0.87), 3.617 (1.32), 3.634 (3.12), 3.644 (1.09), 3.671 (2.81), 3.918 (1.76), 3.928 (1.40), 3.954 (1.38), 3.964 (1.11), 4.251 (0.78), 4.349 (0.85), 4.591 (0.77), 4.599 (0.88), 4.606 (0.95), 4.614 (0.77), 4.633 (0.63), 4.647 (0.81), 4.656 (0.57), 7.026 (3.04), 7.046 (4.91), 7.106 (4.38), 7.112 (4.57), 7.126 (2.87), 7.132 (2.62).

### Beispiel 526

### (5S)-5-{[(3S)-3-Fluorpyrrolidin-1-yl]carbonyl}-2-{[1-(4-methylphenyl)cyclopropyl]methyl}-5,6,7,8-tetrahydro[1,2,4]triazolo[4,3-a]pyridin-3(2H)-on

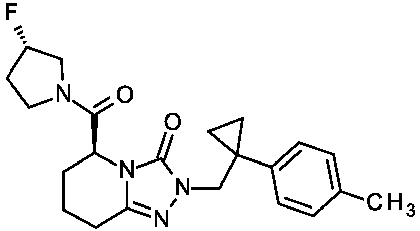

(5S)-2-{[1-(4-Methylphenyl)cyclopropyl]methyl}-3-oxo-2,3,5,6,7,8-hexahydro[1,2,4]triazolo[4,3-a]pyridin-5-carbonsäure (45.0 mg, 137 µmol), N,N-Diisopropylethylamin (72 µl, 410 µmol), HBTU (67.8 mg, 179 µmol), (3S)-3-Fluorpyrrolidinhydrochlorid (20.7 mg, 165 µmol). Rühren über Nacht bei Raumtemperatur. Es wurden nach Aufreinigung 21.6 mg (39 % d. Th.) der Titelverbindung erhalten.
LC-MS (Methode 3): Rₜ = 1.58 min; MS (ESIpos): m/z = 399 [M+H]⁺
¹H-NMR (400 MHz, DMSO-d6) δ [ppm]: -0.149 (0.60), -0.008 (5.27), 0.008 (4.23), 0.146 (0.55), 0.674 (0.50), 0.684 (0.73), 0.700 (1.15), 0.707 (1.10), 0.789 (1.04), 0.947 (0.89), 0.966 (1.93), 0.989 (0.86), 1.387 (3.45), 1.708 (1.54), 1.870 (0.42), 1.984 (0.86), 1.994 (0.94), 2.031 (0.73), 2.049 (0.65), 2.077 (0.65), 2.126 (0.50), 2.235 (16.00), 2.327 (0.97), 2.366 (1.07), 2.523 (4.28), 2.580 (1.28), 2.625 (0.65), 2.669 (1.20), 2.709 (1.10), 3.379 (0.47), 3.476 (0.44), 3.603 (0.99), 3.633 (2.01), 3.641 (1.80), 3.670 (2.17), 3.678 (1.54), 3.709 (0.99), 3.733 (0.70), 3.755 (0.57), 3.821 (1.15), 3.911 (1.33), 3.929 (1.54), 3.947 (1.04), 3.965 (1.23), 4.592 (0.57), 4.601 (0.68), 4.608 (0.65), 4.617 (0.57), 4.650 (0.76), 4.665 (0.94), 4.674 (0.70), 4.910 (0.55), 5.247 (0.57), 5.377 (0.63), 5.499 (0.44), 7.026 (2.90), 7.046 (4.72), 7.102 (3.47), 7.108 (4.33), 7.123 (2.35), 7.128 (2.45).

### Beispiel 527

### (5S)-5-[(3-Fluorazetidin-1-yl)carbonyl]-2-({1-[4-(trifluormethyl)phenyl]cyclopropyl}methyl)-5,6,7,8-tetrahydro[1,2,4]triazolo[4,3-a]pyridin-3(2H)-on

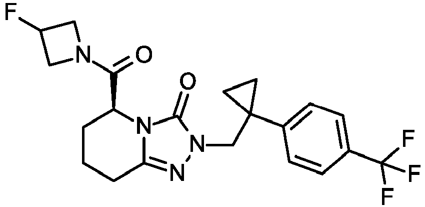

(5S)-3-Oxo-2-({1-[4-(trifluormethyl)phenyl]cyclopropyl}methyl)-2,3,5,6,7,8-hexahydro[1,2,4]triazolo[4,3-a]pyridin-5-carbonsäure (57.4 mg, 151 µmol), N,N-Diisopropylethylamin (79 µl, 450 µmol), HBTU (74.2 mg, 196 µmol), 3-Fluorazetidinhydrochlorid (20.1 mg, 181 µmol). Rühren über Nacht bei Raumtemperatur. Es wurden nach Aufreinigung 37.9 mg (57 % d. Th.) der Titelverbindung erhalten.
LC-MS (Methode 4): Rₜ = 0.92 min; MS (ESIpos): m/z = 439 [M+H]⁺
¹H-NMR (400 MHz, DMSO-d6) δ [ppm]: -0.149 (1.76), -0.008 (16.00), 0.008 (14.16), 0.146 (1.84), 0.852 (2.09), 0.877 (6.79), 0.925 (5.03), 0.949 (2.09), 1.078 (1.17), 1.111 (14.24), 1.691 (4.86), 1.932 (2.60), 1.967 (2.09), 2.072 (2.85), 2.327 (2.43), 2.366 (2.76), 2.523 (11.64), 2.569 (4.69), 2.611 (1.76), 2.669 (2.76), 2.710 (2.85), 3.799 (4.94), 3.835 (8.54), 3.877 (1.34), 3.908 (4.77), 3.923 (3.77), 3.946 (2.76), 3.961 (2.93), 4.189 (1.93), 4.219 (1.93), 4.254 (1.34), 4.274 (1.17), 4.321 (1.01), 4.357 (0.75), 4.385 (0.92), 4.449 (5.11), 4.613 (0.84), 5.316 (0.84), 5.370 (0.92), 5.462 (0.84), 5.505 (0.84), 7.434 (8.80), 7.454 (11.81), 7.572 (10.05), 7.592 (7.71).

### Beispiel 528

### (5S)-5-{[(3S)-3-Fluorpyrrolidin-1-yl]carbonyl}-2-{[1-(4-methoxyphenyl)cyclopropyl]methyl}-5,6,7,8-tetrahydro[1,2,4]triazolo[4,3-a]pyridin-3(2H)-on

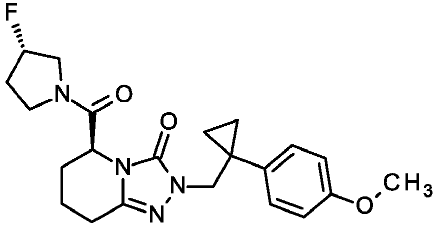

(5S)-2-{[1-(4-Methoxyphenyl)cyclopropyl]methyl}-3-oxo-2,3,5,6,7,8-hexahydro[1,2,4]triazolo[4,3-a]pyridin-5-carbonsäure (93.2 mg, 50 % Reinheit, 136 µmol), N,N-Diisopropylethylamin (140 µl, 810 µmol), HBTU (134 mg, 353 µmol), (3S)-3-Fluorpyrrolidinhydrochlorid (1:1) (20.4 mg, 163 µmol). Rühren über Nacht bei Raumtemperatur. Es wurden nach Aufreinigung 42.9 mg (76 % d. Th.) der Titelverbindung erhalten.
LC-MS (Methode 4): Rₜ = 0.79 min; MS (ESIpos): m/z = 415 [M+H]⁺
¹H-NMR (400 MHz, DMSO-d6) δ [ppm]: -0.008 (1.55), 0.008 (1.21), 0.659 (0.47), 0.676 (0.69), 0.686 (0.80), 0.756 (0.77), 0.780 (0.43), 0.922 (0.63), 0.940 (1.59), 1.712 (1.04), 1.987 (0.54), 1.997 (0.67), 2.009 (0.56), 2.021 (0.47), 2.035 (0.49), 2.050 (0.46), 2.073 (7.98), 2.523 (1.28), 2.591 (0.77), 3.282 (0.42), 3.613 (1.24), 3.621 (1.22), 3.636 (0.59), 3.649 (1.32), 3.657 (1.14), 3.702 (16.00), 3.736 (0.53), 3.821 (0.80), 3.862 (0.89), 3.881 (1.07), 3.898 (0.68), 3.918 (0.80), 4.598 (0.45), 4.605 (0.46), 4.647 (0.48), 4.662 (0.62), 4.670 (0.45), 6.777 (2.71), 6.798 (2.98), 7.123 (1.91), 7.130 (2.47), 7.145 (1.88), 7.152 (2.06).

### Beispiel 529

### (5S)-5-[(3,3-Difluorpyrrolidin-1-yl)carbonyl]-2-{[1-(4-methoxyphenyl)cyclopropyl]methyl}-5,6,7,8-tetrahydro[1,2,4]triazolo[4,3-a]pyridin-3(2H)-on

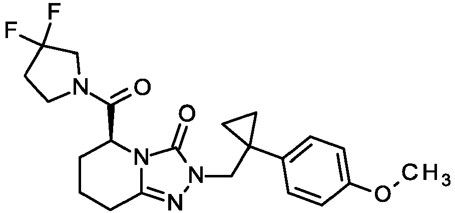

(5S)-2-{[1-(4-Methoxyphenyl)cyclopropyl]methyl}-3-oxo-2,3,5,6,7,8-hexahydro[1,2,4]triazolo[4,3-a]pyridin-5-carbonsäure (93.2 mg, 50 % Reinheit, 136 µmol), N,N-Diisopropylethylamin (140 µl, 810 µmol), HBTU (134 mg, 353 µmol), 3,3-Difluorpyrrolidinhydrochlorid (1:1) (23.4 mg, 163 µmol). Rühren über Nacht bei Raumtemperatur. Es wurden nach Aufreinigung 41.1 mg (70 % d. Th.) der Titelverbindung erhalten.
LC-MS (Methode 4): Rₜ = 0.86 min; MS (ESIpos): m/z = 433 [M+H]⁺
¹H-NMR (400 MHz, DMSO-d6) δ [ppm]: -0.149 (0.44), -0.008 (4.06), 0.008 (3.72), 0.147 (0.48), 0.664 (0.44), 0.685 (0.72), 0.754 (0.78), 0.777 (0.48), 0.919 (0.72), 0.939 (1.64), 0.958 (0.65), 1.693 (0.68), 1.975 (0.61), 2.327 (0.78), 2.366 (1.19), 2.392 (0.41), 2.523 (3.21), 2.584 (1.13), 2.628 (0.44), 2.670 (0.92), 2.710 (0.92), 3.508 (0.65), 3.527 (1.06), 3.547 (0.58), 3.627 (0.89), 3.663 (1.16), 3.682 (0.55), 3.701 (16.00), 3.743 (0.72), 3.771 (0.78), 3.870 (1.36), 3.910 (0.96), 4.668 (0.55), 4.743 (0.55), 4.752 (0.41), 6.776 (3.14), 6.798 (3.58), 7.127 (3.79), 7.149 (3.38).

### Beispiel 530

### (5S)-2-{[1-(2,4-Difluorphenyl)cyclopropyl]methyl}-5-(pyrrolidin-1-ylcarbonyl)-5,6,7,8-tetrahydro[1,2,4]triazolo[4,3-a]pyridin-3(2H)-on

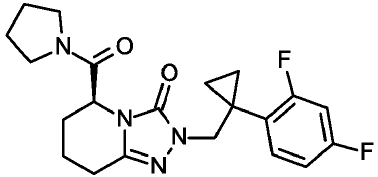

(5S)-2-{[1-(2,4-Difluorphenyl)cyclopropyl]methyl}-3-oxo-2,3,5,6,7,8-hexahydro[1,2,4]triazolo[4,3-a]pyridin-5-carbonsäure (78.0 mg, 223 µmol), N,N-Diisopropylethylamin (120 µl, 670 µmol), HBTU (110 mg, 290 µmol), Pyrrolidin (22 µl, 270 µmol). Rühren über Nacht bei Raumtemperatur. Es wurden nach Aufreinigung 9.80 mg (11 % d. Th.) der Titelverbindung erhalten.
LC-MS (Methode 3): Rₜ = 1.53 min; MS (ESIpos): m/z = 403 [M+H]⁺
¹H-NMR (400 MHz, DMSO-d6) δ [ppm]: -0.149 (0.71), 0.146 (0.71), 0.705 (1.90), 0.730 (7.14), 0.761 (8.00), 0.786 (2.29), 0.968 (1.24), 1.001 (16.00), 1.036 (0.90), 1.237 (0.71), 1.676 (5.05), 1.684 (5.29), 1.695 (4.43), 1.707 (2.24), 1.732 (1.52), 1.749 (5.90), 1.766 (10.43), 1.783 (8.14), 1.800 (2.48), 1.865 (2.33), 1.881 (6.90), 1.898 (8.90), 1.915 (6.19), 1.939 (6.10), 1.953 (5.67), 1.975 (2.43), 1.990 (1.90), 2.073 (5.00), 2.327 (1.29), 2.367 (1.00), 2.418 (1.19), 2.435 (1.48), 2.459 (3.52), 2.574 (1.67), 2.586 (2.48), 2.670 (1.43), 2.710 (1.10), 3.196 (1.29), 3.213 (2.71), 3.225 (3.05), 3.242 (5.24), 3.259 (2.57), 3.281 (3.24), 3.345 (2.14), 3.388 (1.76), 3.405 (3.52), 3.412 (2.76), 3.430 (4.52), 3.447 (2.05), 3.533 (2.05), 3.550 (4.19), 3.566 (2.48), 3.574 (3.24), 3.600 (7.00), 3.636 (9.14), 3.813 (8.90), 3.849 (6.48), 4.598 (3.76), 4.612 (5.62), 4.622 (3.81), 6.883 (1.90), 6.889 (2.10), 6.904 (4.05), 6.910 (4.43), 6.925 (2.33), 6.932 (2.43), 7.087 (2.14), 7.094 (2.33), 7.116 (3.86), 7.137 (3.90), 7.144 (2.67), 7.156 (5.05), 7.173 (4.71), 7.195 (2.19).

### Beispiel 531

### (5S)-5-(Pyrrolidin-1-ylcarbonyl)-2-{[5-(trifluormethyl)pyridin-2-yl]methyl}-5,6,7,8-tetrahydro[1,2,4]triazolo[4,3-a]pyridin-3(2H)-on

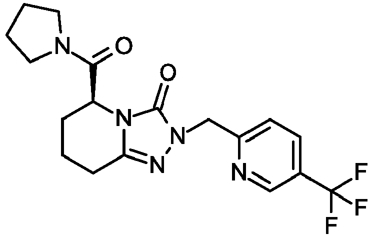

(5S)-3-Oxo-2-{[5-(trifluormethyl)pyridin-2-yl]methyl}-2,3,5,6,7,8-hexahydro[1,2,4]triazolo[4,3-a]pyridin-5-carbonsäure (60.0 mg, 175 µmol), Triethylamin (73 µl, 530 µmol), HATU (86.6 mg, 228 µmol), Pyrrolidin (15.0 mg, 210 µmol). Rühren über Nacht bei Raumtemperatur. Es wurden nach Aufreinigung 51.6 mg (74 % d. Th.) der Titelverbindung erhalten.
LC-MS (Methode 3): Rₜ = 1.23 min; MS (ESIpos): m/z = 396 [M+H]⁺
¹H-NMR (400 MHz, DMSO-d6) δ [ppm]: -0.008 (2.08), 0.008 (1.90), 1.154 (3.17), 1.172 (6.53), 1.190 (3.27), 1.689 (0.90), 1.702 (1.25), 1.713 (1.69), 1.725 (2.58), 1.738 (3.07), 1.748 (2.44), 1.756 (2.05), 1.774 (4.73), 1.792 (7.66), 1.809 (5.78), 1.826 (1.71), 1.894 (1.71), 1.909 (6.12), 1.927 (5.83), 1.944 (3.53), 1.961 (1.02), 1.977 (0.83), 1.989 (0.80), 2.002 (1.29), 2.013 (2.31), 2.026 (2.61), 2.038 (1.76), 2.053 (1.85), 2.063 (1.34), 2.070 (1.25), 2.078 (1.22), 2.089 (0.73), 2.104 (0.42), 2.328 (0.69), 2.367 (0.47), 2.523 (3.17), 2.565 (2.44), 2.574 (2.24), 2.589 (1.92), 2.600 (1.83), 2.612 (3.36), 2.624 (1.90), 2.642 (0.85), 2.654 (1.29), 2.666 (1.14), 2.710 (0.56), 2.866 (1.29), 3.008 (1.19), 3.026 (3.44), 3.044 (3.36), 3.062 (1.12), 3.235 (1.08), 3.252 (2.19), 3.265 (2.12), 3.282 (3.95), 3.328 (3.27), 3.347 (4.14), 3.359 (1.61), 3.364 (2.17), 3.376 (2.29), 3.394 (1.03), 3.449 (1.15), 3.467 (2.46), 3.475 (1.83), 3.485 (1.49), 3.492 (3.15), 3.509 (1.42), 3.598 (1.47), 3.615 (2.86), 3.623 (1.51), 3.632 (1.69), 3.640 (2.25), 3.656 (1.05), 4.760 (2.66), 4.769 (2.95), 4.775 (3.56), 4.784 (2.64), 5.026 (16.00), 7.194 (0.95), 7.383 (4.80), 7.404 (5.03), 8.207 (3.02), 8.212 (3.10), 8.228 (2.95), 8.233 (2.97), 8.929 (4.64).

### Beispiel 532

### (5S)-5-[(3,3-Difluorazetidin-1-yl)carbonyl]-2-[(5-methoxypyridin-2-yl)methyl]-5,6,7,8-tetrahydro[1,2,4]triazolo[4,3-a]pyridin-3(2H)-on

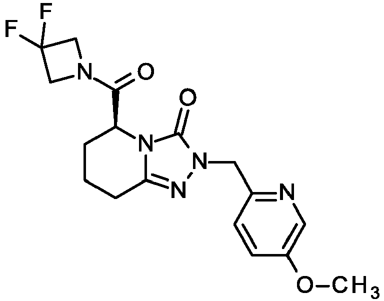

(5S)-2-[(5-Methoxypyridin-2-yl)methyl]-3-oxo-2,3,5,6,7,8-hexahydro[1,2,4]triazolo[4,3-a]pyridin-5-carbonsäure (45.0 mg, 148 µmol), N,N-Diisopropylethylamin (77 µl, 440 µmol), HBTU (72.9 mg, 192 µmol), 3,3-Difluorazetidinhydrochlorid (23.0 mg, 177 µmol). Rühren für 72 Stunden bei Raumtemperatur. Es wurden nach Aufreinigung 20.0 mg (36 % d. Th.) der Titelverbindung erhalten.
LC-MS (Methode 4): Rₜ = 0.54 min; MS (ESIpos): m/z = 380 [M+H]⁺
¹H-NMR (400 MHz, DMSO-d6) δ [ppm]: 1.710 (1.02), 1.725 (1.59), 1.739 (1.12), 1.755 (0.41), 1.977 (0.42), 1.989 (0.76), 2.001 (0.67), 2.043 (0.64), 2.061 (0.68), 2.078 (0.51), 2.524 (0.77), 2.608 (0.64), 3.809 (16.00), 4.331 (0.41), 4.361 (0.69), 4.384 (0.69), 4.414 (0.42), 4.582 (0.92), 4.597 (1.47), 4.610 (0.91), 4.730 (0.43), 4.760 (0.50), 4.838 (6.91), 4.876 (0.46), 7.128 (1.79), 7.149 (2.18), 7.352 (1.38), 7.360 (1.40), 7.374 (1.15), 7.381 (1.19), 8.205 (1.83), 8.212 (1.80).

### Beispiel 533

### (5S)-2-{[3-Chlor-4-(trifluormethyl)pyridin-2-yl]methyl}-5-(pyrrolidin-1-ylcarbonyl)-5,6,7,8-tetrahydro[1,2,4]triazolo[4,3-a]pyridin-3(2H)-on

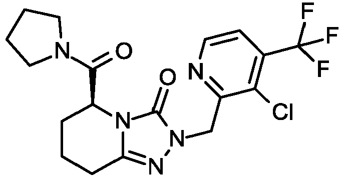

(5S)-2-{[3-Chlor-4-(trifluormethyl)pyridin-2-yl]methyl}-3-oxo-2,3,5,6,7,8-hexahydro[1,2,4]triazolo[4,3-a]pyridin-5-carbonsäure (53.0 mg, 141 µmol), Triethylamin (59 µl, 420 µmol), HATU (69.5 mg, 183 µmol), Pyrrolidin (14 µl, 170 µmol). Rühren über Nacht bei Raumtemperatur. Es wurden nach Aufreinigung 40.0 mg (66 % d. Th.) der Titelverbindung erhalten.
LC-MS (Methode 4): Rₜ = 0.74 min; MS (ESIpos): m/z = 430 [M+H]⁺
¹H-NMR (400 MHz, DMSO-d6) δ [ppm]: -0.150 (0.42), -0.008 (3.83), 0.008 (3.11), 0.146 (0.41), 1.171 (0.73), 1.189 (0.45), 1.271 (0.69), 1.289 (0.69), 1.353 (3.47), 1.371 (3.53), 1.677 (1.03), 1.691 (2.06), 1.701 (5.00), 1.714 (6.44), 1.732 (4.67), 1.744 (2.31), 1.753 (1.98), 1.771 (6.88), 1.788 (11.86), 1.806 (9.09), 1.822 (2.66), 1.891 (2.78), 1.908 (8.33), 1.924 (10.44), 1.941 (6.73), 1.958 (3.14), 1.972 (2.09), 1.983 (3.64), 1.994 (3.30), 2.010 (1.77), 2.025 (2.72), 2.039 (2.05), 2.047 (2.56), 2.061 (2.41), 2.082 (1.11), 2.097 (0.59), 2.327 (0.67), 2.366 (0.64), 2.519 (5.92), 2.560 (3.36), 2.569 (3.11), 2.581 (6.08), 2.594 (3.08), 2.611 (1.28), 2.623 (2.17), 2.636 (0.92), 2.669 (0.75), 2.674 (0.58), 2.710 (0.66), 3.229 (1.62), 3.246 (3.50), 3.259 (3.55), 3.276 (6.45), 3.293 (4.66), 3.339 (6.78), 3.351 (2.56), 3.356 (3.48), 3.368 (3.94), 3.386 (1.69), 3.442 (1.84), 3.459 (3.97), 3.466 (2.95), 3.476 (2.39), 3.484 (5.08), 3.501 (2.19), 3.596 (2.28), 3.613 (5.03), 3.621 (2.52), 3.630 (2.78), 3.638 (3.70), 3.653 (2.63), 3.740 (7.44), 3.902 (4.11), 3.921 (0.52), 4.024 (0.61), 4.042 (0.59), 4.099 (0.61), 4.200 (0.53), 4.216 (0.50), 4.738 (4.37), 4.747 (5.06), 4.754 (5.78), 4.763 (4.33), 5.093 (4.67), 5.133 (16.00), 5.161 (15.97), 5.201 (4.61), 7.843 (9.97), 7.855 (10.39), 8.738 (8.03), 8.750 (7.84).

### Beispiel 534

### (5S)-5-[(3-Fluorazetidin-1-yl)carbonyl]-2-[(1-methyl-1H-pyrazolo[3,4-b]pyridin-3-yl)methyl]-5,6,7,8-tetrahydro[1,2,4]triazolo[4,3-a]pyridin-3(2H)-on

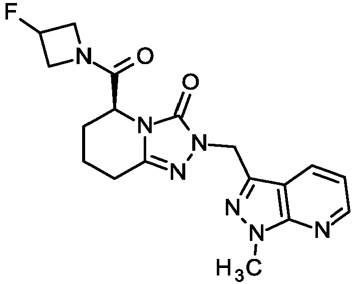

(5S)-2-[(1-Methyl-1H-pyrazolo[3,4-b]pyridin-3-yl)methyl]-3-oxo-2,3,5,6,7,8-hexahydro[1,2,4]triazolo[4,3-a]pyridin-5-carbonsäure (38.0 mg, 116 µmol), N,N-Diisopropylethylamin (60 µl, 350 µmol), HBTU (57.1 mg, 150 µmol), 3-Fluorazetidinhydrochlorid (1:1) (15.5 mg, 139 µmol). Rühren über Nacht bei Raumtemperatur. Es wurden nach Aufreinigung 24.7 mg (100 % Reinheit, 55 % d. Th.) der Titelverbindung erhalten.
LC-MS (Methode 3): Rₜ = 0.93 min; MS (ESIpos): m/z = 386 [M+H]⁺
¹H-NMR (400 MHz, DMSO-d6) δ [ppm]: -0.008 (4.40), 0.008 (2.66), 1.677 (1.65), 1.935 (0.83), 2.004 (0.72), 2.327 (0.63), 2.366 (0.74), 2.523 (4.40), 2.562 (1.52), 2.578 (0.60), 2.592 (0.76), 2.665 (0.63), 2.669 (0.72), 2.709 (0.83), 3.931 (0.56), 3.963 (0.60), 4.007 (1.07), 4.025 (16.00), 4.231 (0.51), 4.260 (0.69), 4.287 (0.60), 4.316 (0.51), 4.541 (1.41), 4.553 (2.10), 4.566 (1.39), 5.116 (6.39), 7.190 (1.01), 7.196 (1.14), 7.204 (1.34), 7.216 (1.07), 8.156 (1.72), 8.177 (1.59), 8.543 (2.21), 8.547 (2.23), 8.554 (2.21), 8.558 (2.01).

### Beispiel 535

### (5S)-5-(Pyrrolidin-1-ylcarbonyl)-2-{[5-(trifluormethyl)pyridin-3-yl]methyl}-5,6,7,8-tetrahydro[1,2,4]triazolo[4,3-a]pyridin-3(2H)-on

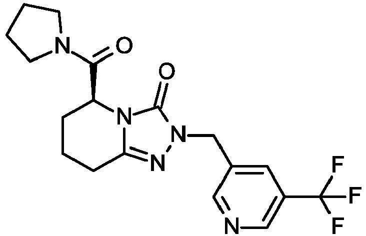

(5S)-3-Oxo-2-{[5-(trifluormethyl)pyridin-3-yl]methyl}-2,3,5,6,7,8-hexahydro[1,2,4]triazolo[4,3-a]pyridin-5-carbonsäure (35.4 mg, 103 µmol), Triethylamin (43 µl, 310 µmol), HATU (59.0 mg, 155 µmol), Pyrrolidin (13 µl, 160 µmol). Rühren über Nacht bei Raumtemperatur. Es wurden nach Aufreinigung 16.7 mg (41 % d. Th.) der Titelverbindung erhalten.
LC-MS (Methode 3): Rₜ = 1.20 min; MS (ESIpos): m/z = 396 [M+H]⁺
¹H-NMR (400 MHz, DMSO-d6) δ [ppm]: -0.149 (0.71), -0.008 (6.63), 0.008 (5.76), 0.146 (0.68), 1.681 (1.20), 1.722 (2.27), 1.739 (2.24), 1.756 (2.05), 1.773 (3.94), 1.791 (6.63), 1.808 (5.19), 1.824 (1.51), 1.893 (1.49), 1.909 (4.48), 1.926 (5.64), 1.943 (3.35), 1.961 (1.04), 2.013 (2.69), 2.036 (1.46), 2.056 (1.20), 2.327 (1.49), 2.366 (0.85), 2.567 (2.19), 2.581 (1.86), 2.597 (1.65), 2.608 (2.93), 2.620 (1.75), 2.650 (1.23), 2.669 (1.53), 2.710 (0.80), 3.228 (1.18), 3.245 (2.27), 3.257 (2.27), 3.274 (3.54), 3.292 (1.94), 3.340 (5.12), 3.358 (2.17), 3.370 (2.17), 3.388 (1.04), 3.437 (1.01), 3.454 (2.15), 3.461 (1.65), 3.479 (2.81), 3.496 (1.16), 3.593 (1.20), 3.610 (2.57), 3.627 (1.49), 3.635 (2.01), 3.652 (1.01), 4.754 (2.38), 4.763 (2.67), 4.769 (3.12), 4.778 (2.34), 5.012 (16.00), 8.063 (5.22), 8.737 (5.29), 8.912 (4.96).

### Beispiel 536

### (5S)-5-[(3,3-Difluorpyrrolidin-1-yl)carbonyl]-2-[(6-fluorpyridin-3-yl)methyl]-5,6,7,8-tetrahydro[1,2,4]triazolo[4,3-a]pyridin-3(2H)-on

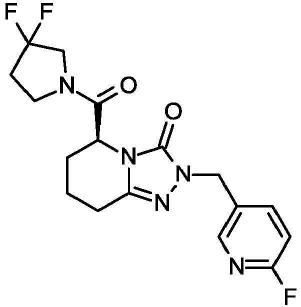

(5S)-2-[(6-Fluorpyridin-3-yl)methyl]-3-oxo-2,3,5,6,7,8-hexahydro[1,2,4]triazolo[4,3-a]pyridin-5-carbonsäure (60.0 mg, 205 µmol), N,N-Diisopropylethylamin (110 µl, 620 µmol), HBTU (101 mg, 267 µmol), 3,3-Difluorpyrrolidinhydrochlorid (35.4 mg, 246 µmol). Rühren über Nacht bei Raumtemperatur. Es wurden nach Aufreinigung 43.3 mg (55 % d. Th.) der Titelverbindung erhalten.
LC-MS (Methode 3): Rₜ = 1.05 min; MS (ESIpos): m/z = 382 [M+H]⁺
¹H-NMR (400 MHz, DMSO-d6) δ [ppm]: -0.149 (0.48), -0.008 (5.78), 0.008 (3.49), 0.146 (0.48), 1.700 (1.48), 1.713 (1.68), 1.725 (1.91), 1.915 (0.89), 1.981 (1.88), 1.992 (1.52), 2.005 (1.58), 2.021 (1.68), 2.049 (1.27), 2.058 (1.32), 2.064 (1.38), 2.073 (3.23), 2.085 (0.96), 2.093 (0.96), 2.100 (0.82), 2.327 (0.71), 2.366 (1.04), 2.381 (1.12), 2.411 (1.37), 2.433 (1.33), 2.519 (5.01), 2.524 (4.66), 2.567 (3.46), 2.576 (3.26), 2.591 (3.81), 2.607 (3.64), 2.619 (1.71), 2.635 (0.71), 2.650 (1.07), 2.665 (0.89), 2.669 (0.87), 2.690 (2.78), 2.710 (0.69), 3.539 (1.68), 3.558 (2.41), 3.571 (1.32), 3.578 (1.27), 3.639 (0.44), 3.673 (1.38), 3.706 (1.63), 3.742 (1.35), 3.774 (2.11), 3.791 (1.29), 3.799 (1.17), 3.809 (2.09), 3.817 (1.76), 3.835 (0.81), 3.844 (0.69), 3.895 (0.77), 3.914 (1.61), 3.933 (0.91), 3.940 (1.10), 3.958 (0.53), 3.973 (0.51), 4.002 (1.02), 4.014 (0.56), 4.029 (0.71), 4.043 (0.89), 4.071 (0.54), 4.149 (0.63), 4.181 (0.89), 4.205 (0.92), 4.760 (1.40), 4.775 (1.75), 4.785 (1.27), 4.835 (1.40), 4.845 (1.61), 4.850 (1.81), 4.860 (1.43), 4.908 (16.00), 4.942 (0.46), 4.949 (0.44), 6.519 (1.88), 7.226 (3.00), 7.237 (3.08), 7.248 (3.35), 7.260 (3.20), 7.698 (1.93), 7.706 (1.99), 7.720 (3.54), 7.728 (3.59), 7.742 (1.73), 7.750 (1.76), 8.512 (5.68), 8.520 (5.44).

### Beispiel 537

### (5S)-5-[(3,3-Difluorazetidin-1 -yl)carbonyl]-2-[(6-fluorpyridin-3-yl)methyl]-5,6,7,8-tetrahydro[1,2,4]triazolo[4,3-a]pyridin-3(2H)-on

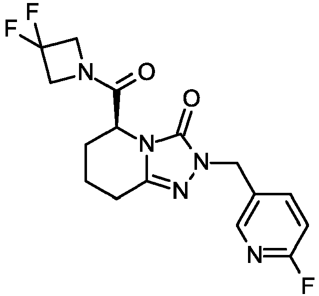

(5S)-2-[(6-Fluorpyridin-3-yl)methyl]-3-oxo-2,3,5,6,7,8-hexahydro[1,2,4]triazolo[4,3-a]pyridin-5-carbonsäure (60.0 mg, 205 µmol), N,N-Diisopropylethylamin (110 µl, 620 µmol), HBTU (101 mg, 267 µmol), 3,3-Difluorazetidinhydrochlorid (31.9 mg, 246 µmol). Rühren für 72 Stunden bei Raumtemperatur. Es wurden nach Aufreinigung 41.5 mg (55 % d. Th.) der Titelverbindung erhalten.
LC-MS (Methode 3): Rₜ = 1.20 min; MS (ESIpos): m/z = 368 [M+H]⁺
¹H-NMR (400 MHz, DMSO-d6) δ [ppm]: -0.008 (1.07), 1.716 (2.85), 1.730 (4.22), 1.745 (2.96), 1.759 (1.15), 1.961 (0.99), 1.974 (1.04), 1.985 (1.17), 1.997 (1.96), 2.009 (1.75), 2.021 (0.75), 2.034 (0.84), 2.050 (1.58), 2.070 (1.74), 2.085 (1.40), 2.103 (0.84), 2.560 (1.95), 2.618 (1.73), 2.632 (0.58), 2.647 (0.96), 2.661 (0.43), 4.333 (1.13), 4.363 (1.88), 4.385 (1.88), 4.416 (1.14), 4.597 (2.54), 4.612 (3.97), 4.624 (2.50), 4.705 (0.40), 4.734 (1.19), 4.763 (1.35), 4.794 (0.66), 4.816 (0.68), 4.847 (1.36), 4.877 (1.28), 4.918 (16.00), 7.251 (2.70), 7.262 (2.75), 7.272 (3.02), 7.283 (2.90), 7.699 (1.82), 7.706 (1.90), 7.721 (3.45), 7.728 (3.55), 7.742 (1.69), 7.750 (1.72), 8.507 (4.62), 8.514 (4.60).

### Beispiel 538

### (5S)-2-[(6-Methoxypyridin-3-yl)methyl]-5-(pyrrolidin-1-ylcarbonyl)-5,6,7,8-tetrahydro[1,2,4]triazolo[4,3-a]pyridin-3(2H)-on

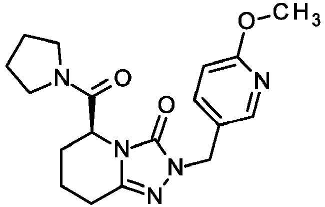

(5S)-2-[(6-Methoxypyridin-3-yl)methyl]-3-oxo-2,3,5,6,7,8-hexahydro[1,2,4]triazolo[4,3-a]pyridin-5-carbonsäure (467 mg, 1.53 mmol), N,N-Diisopropylethylamin (800 µl, 4.6 mmol), HBTU (757 mg, 2.00 mmol), Pyrrolidin (150 µl, 1.8 mmol). Rühren über Nacht bei Raumtemperatur. Es wurden nach Aufreinigung 40.0 mg (95 % Reinheit, 7 % d. Th.) der Titelverbindung erhalten.
LC-MS (Methode 4): Rₜ = 0.58 min; MS (ESIpos): m/z = 358 [M+H]⁺
¹H-NMR (400 MHz, DMSO-d6) δ [ppm]: -0.149 (0.63), -0.008 (5.45), 0.008 (4.76), 0.146 (0.63), 1.706 (0.80), 1.772 (1.20), 1.789 (2.01), 1.806 (1.61), 1.823 (0.52), 1.891 (0.69), 1.908 (1.55), 1.924 (1.89), 1.941 (1.20), 1.960 (0.57), 1.971 (0.80), 2.017 (0.52), 2.327 (1.38), 2.366 (1.20), 2.523 (5.10), 2.570 (0.92), 2.583 (1.15), 2.625 (0.46), 2.665 (1.20), 2.670 (1.55), 2.710 (1.26), 2.847 (0.46), 2.865 (0.92), 3.239 (0.69), 3.251 (0.75), 3.269 (1.38), 3.340 (2.24), 3.358 (1.09), 3.369 (0.86), 3.387 (0.52), 3.449 (0.75), 3.474 (0.97), 3.491 (0.46), 3.589 (0.40), 3.606 (0.86), 3.630 (0.69), 3.826 (16.00), 4.711 (0.75), 4.726 (1.03), 4.736 (0.75), 4.760 (5.79), 6.782 (1.72), 6.803 (1.89), 7.552 (1.15), 7.558 (1.15), 7.573 (1.09), 7.580 (1.15), 8.050 (1.43), 8.056 (1.38).

### Beispiel 539

### (5S)-5-{[(3S)-3-Fluorpyrrolidin-1-yl]carbonyl}-2-{[2-(trifluormethyl)chinolin-4-yl]methyl}-5,6,7,8-tetrahydro[1,2,4]triazolo[4,3-a]pyridin-3(2H)-on

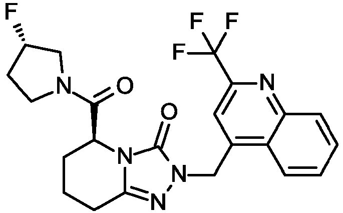

(5S)-3-Oxo-2-{[2-(trifluormethyl)chinolin-4-yl]methyl}-2,3,5,6,7,8-hexahydro[1,2,4]triazolo[4,3-a]pyridin-5-carbonsäure (38.5 mg, 98.1 µmol), N,N-Diisopropylethylamin (51 µl, 290 µmol), HBTU (48.4 mg, 128 µmol), (3S)-3-Fluorpyrrolidinhydrochlorid (14.8 mg, 118 µmol). Rühren über Nacht bei Raumtemperatur. Es wurden nach Aufreinigung 30.9 mg (68 % d. Th.) der Titelverbindung erhalten.
LC-MS (Methode 3): Rₜ = 1.54 min; MS (ESIpos): m/z = 464 [M+H]⁺
¹H-NMR (400 MHz, DMSO-d6) δ [ppm]: -0.149 (1.85), -0.008 (16.00), 0.008 (13.66), 0.146 (1.95), 1.732 (2.93), 2.006 (3.22), 2.327 (4.10), 2.366 (2.34), 2.609 (4.59), 2.670 (4.39), 2.709 (2.24), 3.527 (2.54), 3.554 (2.54), 3.608 (2.54), 3.651 (2.34), 3.748 (2.44), 3.777 (11.02), 3.862 (1.76), 3.960 (1.46), 4.328 (6.54), 4.759 (1.46), 4.810 (1.66), 4.890 (1.76), 5.267 (1.95), 5.408 (3.22), 5.459 (6.15), 5.481 (6.54), 5.522 (2.15), 5.800 (2.24), 6.282 (3.22), 7.741 (7.32), 7.748 (5.56), 7.756 (4.68), 7.847 (4.39), 7.865 (3.02), 7.946 (4.10), 7.966 (6.15), 7.983 (3.90), 8.214 (8.39), 8.235 (7.02), 8.371 (4.10), 8.387 (3.90).

### Beispiel 540

### (5S)-5-[(3-Fluorazetidin-1-yl)carbonyl]-2-{[3-(trifluormethyl)-1,2,4-oxadiazol-5-yl]methyl}-5,6,7,8-tetrahydro[1,2,4]triazolo[4,3-a]pyridin-3(2H)-on

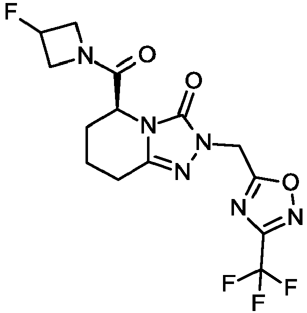

(5S)-3-Oxo-2-{[3-(trifluormethyl)-1,2,4-oxadiazol-5-yl]methyl}-2,3,5,6,7,8-hexahydro[1,2,4]triazolo[4,3-a]pyridin-5-carbonsäure (85.0 mg, 82 % Reinheit, 209 µmol), N,N-Diisopropylethylamin (110 µl, 630 µmol), HBTU (103 mg, 272 µmol), 3-Fluorazetidinhydrochlorid (28.0 mg, 251 µmol). Rühren über Nacht bei Raumtemperatur. Es wurden nach Aufreinigung 53.0 mg (65 % d. Th.) der Titelverbindung erhalten.
LC-MS (Methode 3): Rₜ = 1.24 min; MS (ESIpos): m/z = 391 [M+H]⁺
¹H-NMR (400 MHz, DMSO-d6) δ [ppm]: -0.149 (0.75), -0.008 (6.77), 0.008 (6.05), 0.146 (0.77), 1.706 (3.09), 1.728 (5.31), 1.738 (4.23), 1.954 (2.13), 1.967 (2.27), 1.976 (1.96), 2.041 (1.91), 2.053 (2.07), 2.073 (4.26), 2.328 (0.77), 2.366 (0.88), 2.524 (2.07), 2.558 (1.66), 2.586 (2.85), 2.602 (3.73), 2.620 (4.67), 2.632 (6.49), 2.646 (3.21), 2.661 (1.38), 2.675 (2.40), 2.689 (0.88), 2.710 (1.02), 3.926 (1.22), 3.989 (1.44), 4.013 (1.02), 4.160 (0.72), 4.175 (0.80), 4.191 (0.69), 4.215 (1.46), 4.230 (1.52), 4.245 (1.35), 4.261 (1.55), 4.280 (1.52), 4.299 (1.46), 4.338 (0.88), 4.363 (1.60), 4.393 (1.13), 4.428 (0.83), 4.452 (1.02), 4.510 (0.69), 4.527 (0.88), 4.567 (3.76), 4.580 (6.36), 4.593 (3.65), 4.622 (0.80), 4.639 (0.83), 4.676 (0.80), 4.690 (0.80), 4.717 (0.61), 5.349 (1.05), 5.421 (16.00), 5.476 (0.88), 5.492 (1.13), 5.546 (1.05).

### Beispiel 541

### (5S)-5-[(3,3-Difluorpyrrolidin-1-yl)carbonyl]-2-{[5-(trifluormethyl)-1,3,4-oxadiazol-2-yl]methyl}-5,6,7,8-tetrahydro[1,2,4]triazolo[4,3-a]pyridin-3(2H)-on

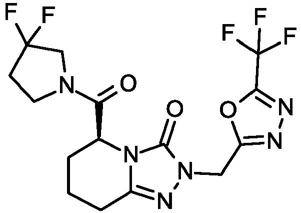

(5S)-3-Oxo-2-{[5-(trifluormethyl)-1,3,4-oxadiazol-2-yl]methyl}-2,3,5,6,7,8-hexahydro[1,2,4]triazolo[4,3-a]pyridin-5-carbonsäure (100 mg, 80 % Reinheit, 240 µmol), N,N-Diisopropylethylamin (130 µl, 720 µmol), HBTU (118 mg, 312 µmol), 3,3-Difluorpyrrolidinhydrochlorid (1:1) (41.4 mg, 288 µmol). Rühren für 72 Stunden bei Raumtemperatur. Es wurden nach Aufreinigung 18.0 mg (18 % d. Th.) der Titelverbindung erhalten.
LC-MS (Methode 3): Rₜ = 1.27 min; MS (ESIpos): m/z = 423 [M+H]⁺
¹H-NMR (400 MHz, DMSO-d6) δ [ppm]: -0.149 (0.80), -0.008 (6.76), 0.008 (5.45), 0.146 (0.80), 1.257 (1.67), 1.726 (2.55), 1.919 (3.78), 1.979 (4.73), 2.015 (2.04), 2.059 (1.75), 2.076 (1.67), 2.327 (2.47), 2.366 (3.05), 2.380 (1.45), 2.409 (1.75), 2.430 (1.82), 2.577 (4.22), 2.591 (4.15), 2.601 (3.93), 2.616 (4.29), 2.631 (4.29), 2.670 (3.56), 2.710 (2.91), 3.529 (3.35), 3.548 (5.38), 3.567 (2.55), 3.669 (1.96), 3.703 (2.11), 3.730 (1.38), 3.765 (2.76), 3.794 (2.62), 3.811 (2.33), 3.827 (2.11), 3.888 (0.95), 3.908 (2.25), 3.934 (1.60), 3.954 (0.80), 3.993 (1.38), 4.035 (1.16), 4.064 (0.73), 4.148 (0.87), 4.173 (1.16), 4.205 (1.24), 4.769 (1.82), 4.785 (2.40), 4.795 (1.82), 4.844 (1.89), 4.860 (2.18), 4.869 (1.67), 5.253 (1.82), 5.295 (15.13), 5.305 (16.00), 5.347 (3.56), 6.510 (0.65).

### Beispiel 542

### (5S)-5-[(3-Fluorazetidin-1-yl)carbonyl]-2-{[5-(trifluormethyl)-1,3,4-oxadiazol-2-yl]methyl}-5,6,7,8-tetrahydro[1,2,4]triazolo[4,3-a]pyridin-3(2H)-on

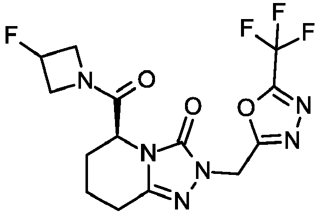

(5S)-3-Oxo-2-{[5-(trifluormethyl)-1,3,4-oxadiazol-2-yl]methyl}-2,3,5,6,7,8-hexahydro[1 ,2,4]triazolo[4,3-a]pyridin-5-carbonsäure (100 mg, 80 % Reinheit, 240 µmol), N,N-Diisopropylethylamin (130 µl, 720 µmol), HBTU (118 mg, 312 µmol), 3-Fluorazetidinhydrochlorid (32.1 mg, 288 µmol). Rühren über Nacht bei Raumtemperatur. Es wurden nach Aufreinigung 21.0 mg (22 % d. Th.) der Titelverbindung erhalten.
LC-MS (Methode 3): Rₜ = 1.06 min; MS (ESIpos): m/z = 391 [M+H]⁺
¹H-NMR (400 MHz, DMSO-d6) δ [ppm]: -0.149 (0.62), -0.008 (4.52), 0.008 (4.71), 0.146 (0.67), 1.406 (0.72), 1.722 (5.05), 1.962 (2.21), 2.040 (2.07), 2.328 (1.83), 2.366 (1.68), 2.523 (4.85), 2.571 (2.88), 2.588 (3.70), 2.609 (4.56), 2.624 (5.67), 2.636 (3.08), 2.652 (1.25), 2.665 (3.22), 2.710 (1.73), 3.826 (0.82), 3.938 (1.35), 3.987 (1.25), 4.175 (0.77), 4.211 (1.39), 4.228 (1.44), 4.258 (1.44), 4.295 (1.44), 4.334 (0.86), 4.360 (1.35), 4.392 (1.06), 4.430 (0.91), 4.459 (1.01), 4.520 (0.82), 4.555 (3.51), 4.567 (5.67), 4.579 (3.51), 4.642 (0.82), 5.260 (1.01), 5.302 (16.00), 5.311 (7.06), 5.353 (1.44), 5.404 (0.96), 5.493 (1.01), 5.546 (0.96), 6.510 (1.20).

### Beispiel 543

### (5S)-5-{[(3R,4S)-3,4-Difluorpyrrolidin-1-yl]carbonyl}-2-{[5-(trifluormethyl)-1,3,4-oxadiazol-2-yl]methyl}-5,6,7,8-tetrahydro[1,2,4]triazolo[4,3-a]pyridin-3(2H)-on

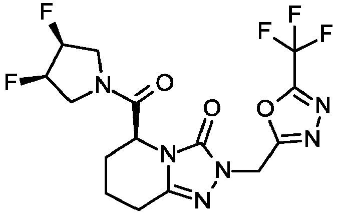

(5S)-3-Oxo-2-{[5-(trifluormethyl)-1,3,4-oxadiazol-2-yl]methyl}-2,3,5,6,7,8-hexahydro[1,2,4]triazolo[4,3-a]pyridin-5-carbonsäure (100 mg, 80 % Reinheit, 240 µmol), N,N-Diisopropylethylamin (130 µl, 720 µmol), HBTU (118 mg, 312 µmol), (3R,4S)-3,4-Difluorpyrrolidinhydrochlorid (1:1) (41.4 mg, 288 µmol). Rühren über Nacht bei Raumtemperatur. Es wurden nach Aufreinigung 30.0 mg (30 % d. Th.) der Titelverbindung erhalten.
LC-MS (Methode 3): Rₜ = 1.23 min; MS (ESIpos): m/z = 423 [M+H]⁺
¹H-NMR (400 MHz, DMSO-d6) δ [ppm]: -0.149 (1.10), -0.008 (9.43), 0.008 (8.71), 0.146 (1.14), 1.413 (1.14), 1.684 (1.95), 1.738 (2.57), 1.750 (2.19), 1.919 (1.05), 1.979 (2.00), 2.001 (1.76), 2.045 (1.48), 2.062 (1.86), 2.071 (1.86), 2.080 (1.52), 2.097 (1.43), 2.327 (1.71), 2.366 (1.57), 2.522 (4.71), 2.571 (2.86), 2.584 (2.81), 2.595 (3.67), 2.604 (2.71), 2.618 (3.81), 2.630 (4.95), 2.643 (2.95), 2.670 (3.29), 2.710 (1.86), 3.455 (0.90), 3.489 (1.62), 3.513 (1.67), 3.535 (1.48), 3.545 (1.67), 3.557 (1.10), 3.567 (1.24), 3.614 (1.19), 3.627 (1.43), 3.647 (1.05), 3.670 (1.52), 3.684 (2.14), 3.703 (1.62), 3.717 (1.71), 3.741 (1.52), 3.752 (1.52), 3.772 (1.29), 3.826 (1.00), 3.870 (1.29), 3.927 (1.19), 3.941 (1.48), 3.957 (0.90), 3.977 (1.29), 3.991 (1.38), 4.007 (0.90), 4.021 (0.81), 4.127 (0.81), 4.142 (1.00), 4.155 (0.86), 4.169 (1.62), 4.183 (1.05), 4.196 (0.95), 4.211 (0.90), 4.806 (4.05), 4.816 (4.81), 4.821 (5.48), 4.831 (4.14), 5.253 (3.14), 5.276 (1.67), 5.294 (16.00), 5.309 (10.86), 5.340 (1.86), 5.351 (3.05), 5.365 (1.24), 5.376 (1.67), 5.388 (1.71), 5.407 (1.43), 5.473 (1.24), 5.481 (1.24).

### Beispiel 544

### (5S)-5-{[(3S)-3-Fluorpyrrolidin-1-yl]carbonyl}-2-{[5-(trifluormethyl)-1,3,4-oxadiazol-2-yl]methyl}-5,6,7,8-tetrahydro[1,2,4]triazolo[4,3-a]pyridin-3(2H)-on

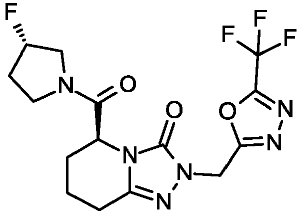

(5S)-3-Oxo-2-{[5-(trifluormethyl)-1,3,4-oxadiazol-2-yl]methyl}-2,3,5,6,7,8-hexahydro[1,2,4]triazolo[4,3-a]pyridin-5-carbonsäure (100 mg, 80 % Reinheit, 240 µmol), N,N-Diisopropylethylamin (130 µl, 720 µmol), HBTU (118 mg, 312 µmol), (3S)-3-Fluorpyrrolidinhydrochlorid (36.2 mg, 288 µmol). Rühren über Nacht bei Raumtemperatur. Es wurden nach Aufreinigung 18.9 mg (19 % d. Th.) der Titelverbindung erhalten.
LC-MS (Methode 3): Rₜ = 1.16 min; MS (ESIpos): m/z = 405 [M+H]⁺
¹H-NMR (400 MHz, DMSO-d6) δ [ppm]: -0.150 (4.07), 0.145 (4.07), 1.740 (4.95), 1.910 (1.45), 2.029 (2.04), 2.110 (3.49), 2.268 (1.75), 2.327 (8.44), 2.366 (9.89), 2.584 (4.95), 2.631 (5.53), 2.669 (10.18), 2.709 (11.35), 3.395 (2.62), 3.501 (1.75), 3.651 (3.20), 3.722 (2.91), 3.745 (3.49), 3.770 (2.91), 3.857 (4.07), 4.715 (2.33), 4.774 (3.20), 5.246 (2.91), 5.288 (16.00), 5.297 (9.02), 5.303 (10.47), 5.346 (1.75), 5.388 (2.91), 5.512 (1.75).

### Beispiel 545

### (5S)-2-{[5-Chlor-6-(trifluormethyl)pyridin-3-yl]methyl}-5-(pyrrolidin-1-ylcarbonyl)-5,6,7,8-tetrahydro[1,2,4]triazolo[4,3-a]pyridin-3(2H)-on

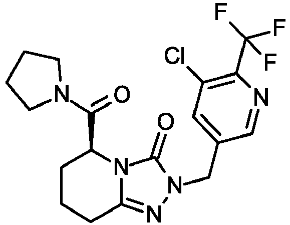

(5S)-2-{[5-Chlor-6-(trifluormethyl)pyridin-3-yl]methyl}-3-oxo-2,3,5,6,7,8-hexahydro[1,2,4]triazolo[4,3-a]pyridin-5-carbonsäure (35.0 mg, 92.9 µmol), N,N-Diisopropylethylamin (49 µl, 280 µmol), HBTU (45.8 mg, 121 µmol), Pyrrolidin (9.3 µl, 110 µmol). Rühren für 72 Stundne bei Raumtemperatur. Es wurden nach Aufreinigung 25.0 mg (63 % d. Th.) der Titelverbindung erhalten.
LC-MS (Methode 3): Rₜ = 1.49 min; MS (ESIpos): m/z = 430 [M+H]⁺
¹H-NMR (400 MHz, DMSO-d6) δ [ppm]: -0.008 (3.06), 0.008 (2.74), 1.243 (2.70), 1.259 (3.33), 1.647 (1.15), 1.667 (1.47), 1.681 (1.59), 1.691 (1.31), 1.708 (1.15), 1.734 (2.34), 1.744 (2.18), 1.757 (2.50), 1.775 (5.76), 1.792 (9.37), 1.809 (7.07), 1.826 (1.99), 1.894 (1.91), 1.910 (5.56), 1.927 (6.87), 1.943 (4.17), 1.960 (1.23), 1.979 (0.91), 1.992 (0.87), 2.006 (1.67), 2.019 (3.69), 2.028 (4.01), 2.044 (2.18), 2.056 (1.63), 2.063 (1.67), 2.071 (1.59), 2.328 (0.91), 2.366 (0.67), 2.523 (4.09), 2.564 (3.10), 2.575 (2.78), 2.590 (2.30), 2.605 (2.26), 2.616 (3.93), 2.628 (2.34), 2.646 (1.07), 2.660 (1.67), 2.670 (1.79), 2.690 (0.71), 2.710 (0.79), 2.885 (3.65), 3.033 (1.91), 3.235 (1.23), 3.251 (2.54), 3.264 (2.66), 3.281 (4.96), 3.330 (3.73), 3.348 (4.96), 3.360 (1.99), 3.366 (2.62), 3.378 (2.90), 3.396 (1.67), 3.403 (2.02), 3.442 (1.43), 3.459 (3.06), 3.466 (2.26), 3.476 (1.87), 3.484 (3.89), 3.501 (1.75), 3.593 (1.95), 3.610 (3.77), 3.618 (2.14), 3.627 (2.38), 3.635 (3.06), 3.652 (1.43), 4.760 (3.30), 4.769 (3.73), 4.775 (4.53), 4.784 (3.18), 4.992 (0.60), 5.035 (16.00), 5.078 (0.56), 8.067 (6.83), 8.537 (6.95), 8.541 (6.83).

### Beispiel 546

### (5S)-5-[(3-Fluorazetidin-1-yl)carbonyl]-2-(4-fluorbenzyl)-5,6,7,8-tetrahydro[1,2,4]triazolo[4,3-a]pyridin-3(2H)-on

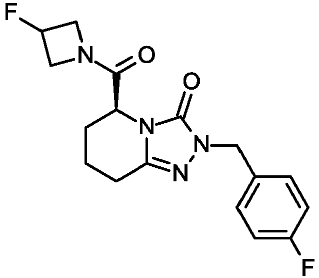

(5S)-2-(4-Fluorbenzyl)-3-oxo-2,3,5,6,7,8-hexahydro[1,2,4]triazolo[4,3-a]pyridin-5-carbonsäure (62.8 mg, 215 µmol), N,N-Diisopropylethylamin (110 µl, 650 µmol), HBTU (106 mg, 280 µmol), 3-Fluorazetidinhydrochlorid (1:1) (28.8 mg, 259 µmol). Rühren über Nacht bei Raumtemperatur. Es wurden nach Aufreinigung 15.9 mg (21 % d. Th.) der Titelverbindung erhalten.
LC-MS (Methode 4): Rₜ = 0.65 min; MS (ESIpos): m/z = 349 [M+H]⁺
¹H-NMR (400 MHz, DMSO-d6) δ [ppm]: -0.150 (0.69), -0.008 (9.17), 0.008 (5.47), 0.146 (0.72), 1.175 (0.67), 1.706 (4.47), 1.908 (2.61), 1.947 (2.08), 1.988 (2.36), 2.010 (1.81), 2.022 (1.89), 2.072 (0.81), 2.327 (0.69), 2.366 (0.61), 2.571 (4.19), 2.585 (5.22), 2.598 (2.61), 2.613 (1.08), 2.627 (1.61), 2.640 (0.72), 2.669 (0.81), 2.709 (0.67), 3.894 (0.78), 3.927 (1.42), 3.954 (1.50), 3.987 (1.39), 4.020 (1.14), 4.152 (0.64), 4.166 (0.75), 4.182 (0.61), 4.222 (1.28), 4.236 (1.28), 4.252 (1.53), 4.264 (1.33), 4.288 (1.42), 4.321 (1.28), 4.346 (0.86), 4.392 (0.89), 4.429 (0.67), 4.455 (0.89), 4.496 (0.86), 4.525 (3.69), 4.538 (5.50), 4.551 (3.61), 4.590 (0.50), 4.633 (0.61), 4.648 (0.72), 4.684 (0.64), 4.697 (0.67), 4.798 (16.00), 5.351 (0.89), 5.404 (0.89), 5.494 (0.89), 5.547 (0.86), 7.140 (4.42), 7.161 (9.78), 7.184 (5.97), 7.263 (5.64), 7.277 (6.94), 7.295 (3.83).

### Beispiel 547

### (5S,8RS)-2-[(5-Chlorpyridin-2-yl)methyl]-5-{[(3S)-3-fluorpyrrolidin-1-yl]carbonyl}-8-hydroxy-5,6,7,8-tetrahydro[1,2,4]triazolo[4,3-a]pyridin-3(2H)-on (Diastereomerengemisch; 2 Isomere)

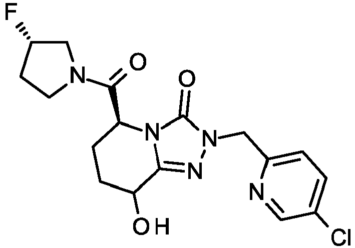

(5S)-2-[(5-Chlorpyridin-2-yl)methyl]-5-{[(3S)-3-fluorpyrrolidin-1-yl]carbonyl}-5,6,7,8-tetrahydro[1,2,4]triazolo[4,3-a]pyridin-3(2H)-on (66.6 mg, 175 µmol) und Cer(IV)sulfat (350 mg, 1.05 mmol) wurden in tert-Butanol (230 µl) bei Raumtemperatur suspendiert. Anschließend wurde 1 N wässrige Schwefelsäure (230 µl, 4.4 mmol) zugegeben und das Reaktionsgemisch über Nacht bei 70°C gerührt. Das Reaktionsgemisch wurde auf Raumtemperatur abgekühlt und mit 2 N wässriger Natriumhydroxid-Lösung auf pH 9 gestellt. Die Suspension wurde filtriert und das Filtrat dreimal mit Dichlormethan extrahiert. Die vereinigten organischen Phasen wurden über Natriumsulfat getrocknet, filtriert und das Filtrat eingeengt. Es wurden 40.4 mg (55 % d. Th.) der Titelverbindung erhalten.
LC-MS (Methode 1): Rₜ = 0.73 min; MS (ESIpos): m/z = 396 [M+H]⁺
¹H-NMR (400 MHz, DMSO-d6) δ [ppm]: -0.008 (3.50), 1.167 (0.78), 1.215 (0.80), 1.236 (1.14), 1.273 (0.68), 1.283 (0.48), 1.352 (0.59), 1.425 (0.57), 1.526 (0.41), 1.743 (1.70), 1.754 (2.02), 1.767 (2.20), 1.780 (2.30), 1.799 (1.41), 1.812 (1.70), 1.822 (2.02), 1.855 (1.40), 1.873 (0.98), 1.906 (1.16), 1.942 (1.08), 1.988 (0.74), 2.026 (0.56), 2.107 (1.89), 2.124 (1.73), 2.132 (1.74), 2.222 (1.05), 2.234 (1.05), 2.270 (1.43), 2.327 (0.99), 2.366 (1.04), 2.391 (1.34), 2.403 (1.29), 2.432 (0.84), 2.669 (0.72), 3.269 (0.74), 3.343 (0.69), 3.361 (0.68), 3.388 (0.77), 3.398 (0.71), 3.451 (0.53), 3.459 (0.60), 3.486 (0.83), 3.495 (0.78), 3.629 (1.85), 3.641 (2.63), 3.657 (2.17), 3.665 (2.21), 3.685 (1.68), 3.738 (1.68), 3.759 (1.83), 3.779 (1.65), 3.801 (1.05), 3.834 (0.50), 3.866 (2.24), 4.488 (0.63), 4.501 (0.59), 4.526 (3.47), 4.537 (3.85), 4.554 (0.99), 4.673 (0.62), 4.742 (1.35), 4.754 (1.28), 4.798 (1.71), 4.809 (1.65), 4.896 (0.56), 4.906 (0.72), 4.937 (2.42), 4.956 (16.00), 4.998 (0.56), 5.258 (1.44), 5.388 (2.08), 5.517 (1.20), 5.754 (6.83), 5.778 (4.87), 5.789 (5.77), 5.802 (1.23), 7.201 (3.74), 7.223 (3.98), 7.236 (1.17), 7.256 (1.19), 7.926 (4.56), 7.932 (4.63), 7.947 (4.21), 7.953 (4.26), 8.582 (4.62), 8.588 (4.12).

### Beispiel 548

### (5S)-2-[(5-Chlorpyridin-3-yl)methyl]-5-{[trans-3,4-difluorpyrrolidin-1 -yl]carbonyl}-5,6,7,8-tetrahydro[1,2,4]triazolo[4,3-a]pyridin-3(2H)-on (Diastereomerengemisch; 2 Isomere)

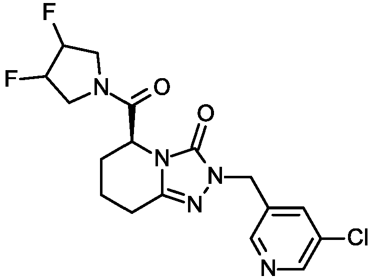

(5S)-2-[(5-Chlorpyridin-3-yl)methyl]-3-oxo-2,3,5,6,7,8-hexahydro[1,2,4]triazolo[4,3-a]pyridin-5-carbonsäure (162 mg, 525 µmol) wurde in THF (11 ml) bei Raumtemperatur vorgelegt. Anschließend wurde HBTU (259 mg, 683 µmol) und N,N-Diisopropylethylamin (460 µl, 2.6 mmol) zugegeben. Nach 5 min Rühren wurde trans-3,4-Difluorpyrrolidinhydrochlorid (90.5 mg, 630 µmol) zugeben und das Reaktionsgemisch über Nacht bei Raumtemperatur gerührt. Es wurde erneut HBTU (259 mg, 683 µmol) und N,N-Diisopropylethylamin (460 µl, 2.6 mmol) zugegeben und über Nacht bei Raumtemperatur gerührt. Das Reaktionsgemisch wurde mit gesättigter wässriger Natriumhydrogencarbonat-Lösung versetzt und zweimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC (Methode 12) aufgereinigt. Die produkthaltigen Fraktionen wurden im Vakuum eingeengt und es wurden 2.90 mg (1.4 % d. Th.) der Titelverbindung erhalten.
LC-MS (Methode 3): Rₜ = 1.09 min; MS (ESIpos): m/z = 398 [M+H]⁺
¹H-NMR (400 MHz, DMSO-d6) δ [ppm]: -0.149 (1.68), -0.008 (14.62), 0.008 (11.46), 0.146 (1.68), 1.146 (0.59), 1.735 (2.72), 1.936 (1.14), 2.000 (1.28), 2.073 (1.23), 2.327 (3.01), 2.366 (1.63), 2.564 (3.01), 2.579 (2.72), 2.590 (2.67), 2.604 (3.80), 2.617 (4.15), 2.629 (2.47), 2.670 (3.60), 2.710 (1.73), 3.570 (0.84), 3.637 (3.36), 3.674 (1.09), 3.712 (1.63), 3.733 (1.19), 3.757 (1.43), 3.822 (1.63), 3.860 (1.09), 3.940 (3.60), 4.014 (1.58), 4.138 (1.19), 4.171 (0.94), 4.203 (1.19), 4.236 (0.94), 4.771 (2.12), 4.780 (1.58), 4.916 (16.00), 5.309 (1.78), 5.444 (2.22), 5.537 (0.84), 7.760 (7.85), 8.425 (7.90), 8.564 (7.26), 8.570 (7.26).

### Beispiel 549

### (5S)-2-[(5-Chlor-3-fluorpyridin-2-yl)methyl]-5-{[trans-3,4-difluorpyrrolidin-1-yl]carbonyl}-5,6,7,8-tetrahydro[1,2,4]triazolo[4,3-a]pyridin-3(2H)-on (Diastereomerengemisch; 2 Isomere)

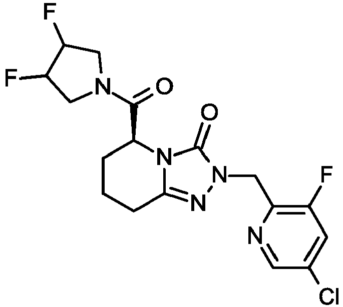

(5S)-2-[(5-Chlor-3-fluorpyridin-2-yl)methyl]-3-oxo-2,3,5,6,7,8-hexahydro[1,2,4]triazolo[4,3-a]pyridin-5-carbonsäure (212 mg, 650 µmol) wurde in THF (14 ml) bei Raumtemperatur vorgelegt. Anschließend wurde HBTU (320 mg, 845 µmol) und N,N-Diisopropylethylamin (570 µl, 3.2 mmol) zugegeben. Nach 5 min Rühren wurde trans-3,4-Difluorpyrrolidinhydrochlorid (112 mg, 780 µmol) zugeben und das Reaktionsgemisch über Nacht bei Raumtemperatur gerührt. Es wurde erneut HBTU (320 mg, 845 µmol) und N,N-Diisopropylethylamin (570 µl, 3.2 mmol) zugegeben und über Nacht bei Raumtemperatur gerührt. Das Reaktionsgemisch wurde mit gesättigter wässriger Natriumhydrogencarbonat-Lösung versetzt und zweimal mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC (Methode 12) aufgereinigt. Die produkthaltigen Fraktionen wurden im Vakuum eingeengt und es wurden 26.5 mg (10 % d. Th.) der Titelverbindung erhalten.
LC-MS (Methode 3): Rₜ = 1.18 min; MS (ESIpos): m/z = 416 [M+H]⁺
¹H-NMR (400 MHz, DMSO-d6) δ [ppm]: -0.149 (2.07), -0.008 (16.00), 0.008 (12.99), 0.146 (1.88), 1.147 (0.75), 1.719 (6.31), 1.731 (4.52), 1.879 (1.41), 1.902 (1.98), 1.914 (1.79), 1.968 (1.79), 2.035 (1.51), 2.055 (1.88), 2.074 (2.35), 2.093 (2.45), 2.110 (1.88), 2.128 (1.13), 2.327 (4.14), 2.366 (2.07), 2.558 (9.13), 2.571 (9.13), 2.584 (4.80), 2.600 (2.07), 2.613 (3.01), 2.669 (4.99), 2.709 (2.45), 3.527 (1.32), 3.563 (1.60), 3.627 (6.12), 3.665 (1.88), 3.711 (4.24), 3.745 (2.45), 3.782 (1.69), 3.813 (3.01), 3.850 (2.35), 3.936 (5.84), 4.015 (3.67), 4.127 (1.88), 4.160 (1.60), 4.190 (1.98), 4.223 (1.69), 4.726 (2.73), 4.737 (3.48), 4.742 (3.67), 4.752 (2.92), 4.855 (2.73), 4.870 (3.58), 4.880 (2.64), 4.902 (4.42), 4.941 (10.82), 4.990 (10.82), 5.029 (4.24), 5.308 (2.73), 5.427 (3.86), 5.441 (3.58), 5.533 (1.51), 5.565 (1.41), 8.089 (6.59), 8.094 (7.25), 8.114 (7.06), 8.118 (7.44), 8.478 (8.19), 8.482 (8.38).

### Beispiel 550

### (5S)-2-[(6-Chlorpyridin-3-yl)methyl]-5-{[trans-3,4-difluorpyrrolidin-1-yl]carbonyl}-5,6,7,8-tetrahydro[1,2,4]triazolo[4,3-a]pyridin-3(2H)-on (Diastereomerengemisch; 2 Isomere)

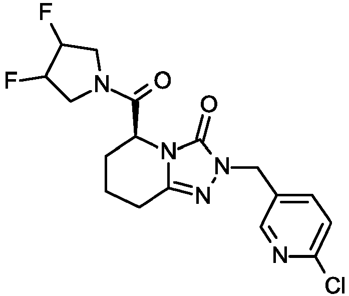

(5S)-2-[(6-Chlorpyridin-3-yl)methyl]-3-oxo-2,3,5,6,7,8-hexahydro[1,2,4]triazolo[4,3-a]pyridin-5-carbonsäure (200 mg, 649 µmol) wurde in THF (14 ml) bei Raumtemperatur vorgelegt. Anschließend wurde HBTU (320 mg, 843 µmol) und N,N-Diisopropylethylamin (570 µl, 3.2 mmol) zugegeben. Nach 5 min Rühren wurde trans-3,4-Difluorpyrrolidinhydrochlorid (112 mg, 779 µmol) zugeben und das Reaktionsgemisch über Nacht bei Raumtemperatur gerührt. Die Suspension wurde filtriert und das Filtrat mit gesättigter wässriger Natriumhydrogencarbonat-Lösung versetzt und zweimal mit Ethylacetat extrahiert. Die wässrige Phase wurde mit 1 N wässriger Salzsäure angesäuert und mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 40mm; Laufmittel: (Acetonitril/Wasser mit 0.1% Ameisensäure)-Gradient). Die produkthaltigen Fraktionen wurden im Vakuum eingeengt und es wurden 71.5 mg (28 % d. Th.) der Titelverbindung erhalten.
LC-MS (Methode 3): Rₜ = 1.10 min; MS (ESIpos): m/z = 398 [M+H]⁺
¹H-NMR (400 MHz, DMSO-d6) δ [ppm]: -0.149 (0.57), -0.008 (4.53), 0.008 (5.49), 0.146 (0.57), 1.648 (0.77), 1.672 (0.75), 1.720 (2.21), 1.730 (2.59), 1.742 (2.35), 1.887 (0.75), 1.912 (0.77), 1.928 (0.99), 1.959 (0.50), 1.995 (1.10), 2.020 (0.70), 2.030 (0.70), 2.037 (0.68), 2.045 (0.79), 2.057 (0.75), 2.065 (0.88), 2.073 (1.22), 2.082 (0.95), 2.099 (1.10), 2.108 (1.02), 2.116 (0.83), 2.124 (0.84), 2.134 (0.72), 2.327 (0.79), 2.366 (0.75), 2.524 (3.00), 2.566 (2.71), 2.576 (2.64), 2.591 (3.79), 2.604 (4.17), 2.616 (2.26), 2.633 (0.83), 2.646 (1.29), 2.660 (0.77), 2.665 (0.74), 2.669 (0.88), 2.710 (0.74), 3.528 (0.65), 3.565 (0.90), 3.633 (3.32), 3.667 (1.08), 3.709 (1.83), 3.730 (1.33), 3.753 (1.42), 3.766 (0.63), 3.789 (1.08), 3.820 (1.85), 3.845 (0.77), 3.857 (1.15), 3.914 (0.65), 3.936 (3.36), 4.013 (1.96), 4.023 (1.35), 4.132 (1.02), 4.166 (0.88), 4.196 (1.06), 4.230 (0.83), 4.739 (1.67), 4.749 (2.03), 4.755 (2.15), 4.765 (1.63), 4.883 (16.00), 4.907 (1.53), 5.257 (0.41), 5.310 (1.56), 5.399 (0.84), 5.425 (1.87), 5.442 (1.99), 5.533 (0.75), 5.567 (0.74), 7.506 (6.29), 7.527 (7.96), 7.690 (4.02), 7.696 (4.04), 7.710 (3.34), 7.716 (3.29), 8.299 (5.30), 8.304 (5.14).

### Beispiel 551

### (5S)-2-[(6-Chlorpyridin-3-yl)methyl]-5-{[trans-3,4-difluorpyrrolidin-1-yl]carbonyl}-5,6,7,8-tetrahydro[1,2,4]triazolo[4,3-a]pyridin-3(2H)-on (Isomer 1)

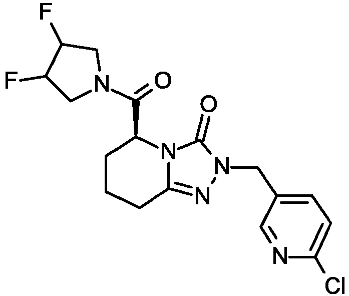

(5S)-2-[(6-Chlorpyridin-3-yl)methyl]-5-{[trans-3,4-difluorpyrrolidin-1-yl]carbonyl}-5,6,7,8-tetrahydro[1,2,4]triazolo[4,3-a]pyridin-3(2H)-on (Diastereomerengemisch; 2 Isomere) wurde durch chirale präparative HPLC getrennt [Probenvorbereitung: 71.5 mg gelöst in 2 ml Ethanol und 2 ml Dichlormethan; Injektionsvolumen: 0.3 ml; Säule: Daicel Chiralpak^{®} IF 5 µm, 250 x 20 mm; Laufmittel: n-Heptan/Ethanol 35:65; Fluss: 15 ml/min; Temperatur 25°C; UV-Detektion: 210 nm]. Nach der Trennung wurden 19.2 mg von Isomer 1, welches zuerst eluiert, und 20.3 mg von Isomer 2, welches später eluiert, isoliert.

### Isomer 1:

Analytische chirale HPLC: Rₜ = 2.66 min, d.e. = 99% [Säule: Daicel Chiralpak^{®} IF-3 50 x 4.6 mm; Laufmittel: n-Heptan/Ethanol 50:50; Fluss: 1 ml/min; UV-Detektion: 220 nm].
LC-MS (Methode 4): Rₜ = 0.59 min; MS (ESIpos): m/z = 398 [M+H]⁺
¹H-NMR (400 MHz, DMSO-d6) δ [ppm]: -0.149 (0.77), -0.008 (5.90), 0.008 (6.28), 0.146 (0.70), 1.237 (0.41), 1.647 (0.98), 1.732 (1.32), 1.741 (1.34), 1.755 (1.01), 1.958 (0.70), 1.995 (1.68), 2.020 (1.08), 2.029 (1.03), 2.045 (1.15), 2.057 (1.10), 2.065 (1.20), 2.073 (1.08), 2.327 (1.39), 2.366 (0.91), 2.523 (4.61), 2.565 (2.30), 2.576 (2.25), 2.591 (2.95), 2.604 (3.05), 2.616 (1.80), 2.634 (0.72), 2.646 (1.01), 2.669 (1.61), 2.710 (1.03), 3.092 (1.49), 3.633 (4.37), 3.672 (0.67), 3.708 (2.37), 3.729 (1.63), 3.765 (0.41), 3.784 (1.13), 3.811 (0.86), 3.844 (1.10), 3.914 (0.84), 3.947 (1.10), 4.132 (1.54), 4.166 (1.34), 4.196 (1.63), 4.229 (1.25), 4.888 (16.00), 4.907 (2.42), 5.310 (1.15), 5.426 (1.82), 5.532 (1.13), 7.507 (4.56), 7.527 (5.83), 7.688 (3.24), 7.695 (3.33), 7.709 (2.69), 7.715 (2.81), 8.299 (3.72), 8.304 (3.79).

### Beispiel 552

### (5S)-2-[(6-Chlorpyridin-3-yl)methyl]-5-{[trans-3,4-difluorpyrrolidin-1-yl]carbonyl}-5,6,7,8-tetrahydro[1,2,4]triazolo[4,3-a]pyridin-3(2H)-on (Isomer 2)

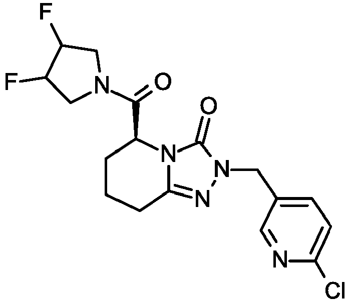

(5S)-2-[(6-Chlorpyridin-3-yl)methyl]-5-{[trans-3,4-difluorpyrrolidin-1 -yl]carbonyl}-5,6,7,8-tetrahydro[1,2,4]triazolo[4,3-a]pyridin-3(2H)-on (Diastereomerengemisch; 2 Isomere) wurde durch chirale präparative HPLC getrennt [Probenvorbereitung: 71.5 mg gelöst in 2 ml Ethanol und 2 ml Dichlormethan; Injektionsvolumen: 0.3 ml; Säule: Daicel Chiralpak^{®} IF 5 µm, 250 x 20 mm; Laufmittel: n-Heptan/Ethanol 35:65; Fluss: 15 ml/min; Temperatur 25°C; UV-Detektion: 210 nm]. Nach der Trennung wurden 19.2 mg von Isomer 1, welches zuerst eluiert, und 20.3 mg von Isomer 2, welches später eluiert, isoliert.

### Isomer 2:

Analytische chirale HPLC: Rₜ = 3.85 min, d.e. = 99% [Säule: Daicel Chiralpak^{®} IF-3 50 x 4.6 mm; Laufmittel: n-Heptan/Ethanol 50:50; Fluss: 1 ml/min; UV-Detektion: 220 nm].
LC-MS (Methode 4): Rₜ = 0.60 min; MS (ESIpos): m/z = 398 [M+H]⁺
¹H-NMR (400 MHz, DMSO-d6) δ [ppm]: -0.149 (0.56), -0.008 (4.82), 0.008 (5.43), 0.146 (0.58), 1.236 (0.48), 1.719 (2.19), 1.730 (2.33), 1.742 (1.97), 1.889 (0.92), 1.928 (1.31), 2.098 (1.19), 2.108 (1.05), 2.117 (1.01), 2.124 (1.07), 2.134 (0.86), 2.327 (1.15), 2.366 (0.42), 2.567 (2.57), 2.575 (2.43), 2.590 (3.10), 2.604 (3.42), 2.617 (1.87), 2.633 (0.88), 2.646 (1.15), 2.669 (1.57), 2.710 (0.78), 3.092 (1.25), 3.528 (0.86), 3.565 (1.21), 3.630 (0.98), 3.666 (1.19), 3.701 (0.56), 3.733 (0.54), 3.753 (1.89), 3.789 (1.49), 3.821 (1.93), 3.857 (1.29), 3.935 (4.26), 3.979 (0.44), 4.014 (2.75), 4.739 (2.19), 4.749 (2.77), 4.754 (2.93), 4.764 (2.23), 4.883 (16.00), 5.309 (1.19), 5.443 (1.95), 5.568 (1.07), 7.506 (4.64), 7.527 (5.95), 7.690 (3.22), 7.696 (3.46), 7.711 (2.75), 7.717 (2.87), 8.298 (4.10), 8.304 (4.34).

### Beispiel 553

### (5S)-5-{[trans-3,4-Difluorpyrrolidin-1-yl]carbonyl}-2-{[6-(trifluormethyl)pyridin-3-yl]methyl}-5,6,7,8-tetrahydro[1,2,4]triazolo[4,3-a]pyridin-3(2H)-on (Isomer 1)

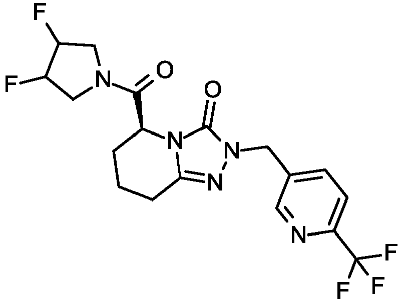

(5S)-3-Oxo-2-{[6-(trifluormethyl)pyridin-3-yl]methyl}-2,3,5,6,7,8-hexahydro[1,2,4]triazolo[4,3-a]pyridin-5-carbonsäure (389 mg, 88 % Reinheit, 1.00 mmol) wurde in THF (21 ml) bei Raumtemperatur vorgelegt. Anschließend wurde HBTU (493 mg, 1.30 mmol) und N,N-Diisopropylethylamin (870 µl, 5.0 mmol) zugegeben. Nach 5 min Rühren wurde trans-3,4-Difluorpyrrolidinhydrochlorid (172 mg, 1.20 mmol) zugeben und das Reaktionsgemisch über Nacht bei Raumtemperatur gerührt. Die Suspension wurde filtriert und das Filtrat mit gesättigter wässriger Natriumhydrogencarbonat-Lösung versetzt und zweimal mit Ethylacetat extrahiert. Die wässrige Phase wurde mit 1 N wässriger Salzsäure angesäuert und mit Ethylacetat extrahiert. Die vereinigten organischen Phasen wurden mit gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm x 40mm; Laufmittel: (Acetonitril/Wasser mit 0.1% Ameisensäure)-Gradient). Die produkthaltigen Fraktionen wurden im Vakuum eingeengt und es wurden 128.5 mg (84 % Reinheit, 25 % d. Th.) eines Diastereomerengemisches (2 Isomere) erhalten.

Das Diastereomerengemisch (2 Isomere) wurde durch chirale präparative HPLC getrennt [Probenvorbereitung: 128.5 mg gelöst in 2 ml Ethanol und 2 ml n-Heptan; Injektionsvolumen: 0.4 ml; Säule: Daicel Chiralpak^{®} IC 5 µm, 250 x 20 mm; Laufmittel: n-Heptan/Ethanol 50:50; Fluss: 15 ml/min; Temperatur 40°C; UV-Detektion: 220 nm]. Nach der Trennung wurden 43 mg von Isomer 1, welches zuerst eluiert, und 45.5 mg von Isomer 2, welches später eluiert, isoliert.

### Isomer 1:

Analytische chirale HPLC: Rₜ = 1.74 min, d.e. = 99% [Säule: Daicel Chiralpak^{®} IC-3 3 µm, 50 x 4.6 mm; Laufmittel: n-Heptan/Ethanol 50:50; Fluss: 1 ml/min; UV-Detektion: 220 nm].
LC-MS (Methode 3): Rₜ = 1.25 min; MS (ESIpos): m/z = 432 [M+H]⁺
¹H-NMR (400 MHz, DMSO-d6) δ [ppm]: -0.149 (1.66), -0.008 (13.19), 0.008 (12.41), 0.146 (1.56), 1.196 (0.61), 1.213 (1.39), 1.231 (0.78), 1.651 (1.02), 1.751 (1.36), 2.008 (1.69), 2.031 (1.39), 2.055 (1.15), 2.075 (1.15), 2.327 (1.36), 2.366 (0.68), 2.560 (2.37), 2.575 (2.10), 2.586 (1.97), 2.600 (2.88), 2.613 (2.98), 2.626 (1.73), 2.669 (1.97), 2.710 (0.68), 3.638 (4.24), 3.677 (0.75), 3.713 (2.37), 3.735 (1.56), 3.773 (0.44), 3.815 (0.92), 3.849 (1.05), 3.919 (0.81), 3.952 (1.12), 4.139 (1.59), 4.172 (1.39), 4.202 (1.76), 4.235 (1.25), 4.901 (2.14), 4.916 (2.95), 4.926 (2.10), 4.972 (0.58), 5.013 (9.08), 5.058 (0.58), 5.312 (1.15), 5.427 (1.93), 5.533 (1.19), 7.911 (14.88), 7.915 (16.00), 8.644 (5.59).

### Beispiel 554

### (5S)-5-{[trans-3,4-Difluorpyrrolidin-1-yl]carbonyl}-2-{[6-(trifluormethyl)pyridin-3-yl]methyl}-5,6,7,8-tetrahydro[1,2,4]triazolo[4,3-a]pyridin-3(2H)-on (Isomer 2)

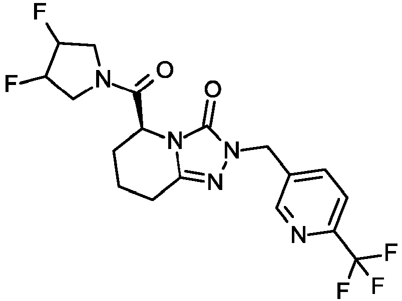

(5S)-5-{[trans-3,4-Difluorpyrrolidin-1 -yl]carbonyl}-2-{[6-(trifluormethyl)pyridin-3-yl]methyl}-5,6,7,8-tetrahydro[1,2,4]triazolo[4,3-a]pyridin-3(2H)-on (Diastereomerengemisch; 2 Isomere) wurde durch chirale präparative HPLC getrennt [Probenvorbereitung: 128.5 mg gelöst in 2 ml Ethanol und 2 ml n-Heptan; Injektionsvolumen: 0.4 ml; Säule: Daicel Chiralpak^{®} IC 5 µm, 250 x 20 mm; Laufmittel: n-Heptan/Ethanol 50:50; Fluss: 15 ml/min; Temperatur 40°C; UV-Detektion: 220 nm]. Nach der Trennung wurden 43 mg von Isomer 1, welches zuerst eluiert, und 45.5 mg von Isomer 2, welches später eluiert, isoliert.

### Isomer 2:

Analytische chirale HPLC: Rₜ = 2.51 min, d.e. = 99% [Säule: Daicel Chiralpak^{®} IC-3 3 µm, 50 x 4.6 mm; Laufmittel: n-Heptan/Ethanol 50:50; Fluss: 1 ml/min; UV-Detektion: 220 nm].
LC-MS (Methode 8): Rₜ = 1.80 min; MS (ESIpos): m/z = 432 [M+H]⁺
¹H-NMR (400 MHz, DMSO-d6) δ [ppm]: -0.149 (1.97), -0.008 (16.00), 0.146 (1.86), 1.726 (1.43), 1.740 (1.55), 1.939 (0.89), 2.108 (0.81), 2.328 (1.66), 2.366 (1.28), 2.576 (1.70), 2.584 (1.55), 2.600 (2.09), 2.613 (2.28), 2.626 (1.20), 2.670 (2.01), 2.710 (1.28), 3.534 (0.58), 3.570 (0.85), 3.635 (0.58), 3.671 (0.81), 3.759 (1.20), 3.796 (0.89), 3.827 (1.24), 3.864 (0.85), 3.940 (2.90), 4.019 (1.97), 4.755 (1.43), 4.770 (1.97), 4.780 (1.51), 5.010 (9.89), 5.310 (0.85), 5.446 (1.35), 5.574 (0.73), 7.912 (8.81), 7.915 (10.01), 8.645 (3.29).

### Beispiel 555

### (5S)-2-[(5-Chlorpyridin-2-yl)methyl]-8,8-difluor-5-{[(3S)-3-fluorpyrrolidin-1 -yl]carbonyl}-5,6,7,8-tetrahydro[1,2,4]triazolo[4,3-a]pyridin-3(2H)-on

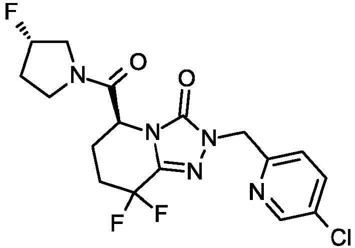

Unter Argon: (5S)-2-[(5-Chlorpyridin-2-yl)methyl]-5-{[(3S)-3-fluorpyrrolidin-1-yl]carbonyl}-6,7-dihydro[1,2,4]triazolo[4,3-a]pyridin-3,8(2H,5H)-dion (20.7 mg, 71 % Reinheit, 37.2 µmol) wurde in Dichlormethan (3.8 ml) bei Raumtemperatur vorgelegt. Anschließend wurde Diethylaminoschwefeltrifluorid (15 µl, 110 µmol) zugegeben und bei 40°C über Nacht gerührt. Das Reaktionsgemisch wurde mit Wasser und gesättigter wässriger Natriumhydrogencarbonat-Lösung versetzt. Die wässrige Phase wurde viermal mit Dichlormethan extrahiert und die vereinigten organischen Phasen wurden über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Die produkthaltigen Fraktionen wurden im Vakuum eingeengt und es wurden 7.00 mg (44 % d. Th.) der Titelverbindung erhalten.
LC-MS (Methode 4): Rₜ = 0.77 min; MS (ESIpos): m/z = 416 [M+H]⁺
¹H-NMR (400 MHz, DMSO-d6) δ [ppm]: -0.149 (1.99), -0.008 (15.19), 0.008 (16.00), 0.146 (1.74), 1.072 (1.31), 1.126 (1.25), 1.146 (1.31), 1.178 (1.56), 1.190 (1.43), 1.364 (0.93), 2.151 (1.06), 2.327 (5.23), 2.366 (3.24), 2.669 (4.86), 2.710 (2.24), 3.526 (0.75), 3.645 (1.43), 3.673 (1.31), 3.700 (1.12), 3.777 (1.18), 3.889 (1.25), 4.896 (0.81), 4.960 (1.12), 5.064 (5.60), 5.078 (3.55), 5.276 (0.75), 5.406 (1.00), 7.282 (2.93), 7.303 (3.18), 7.951 (2.18), 7.957 (2.37), 7.972 (2.24), 7.979 (2.30), 8.440 (0.68), 8.590 (2.49).

### Beispiel 556

### (5S)-2-[(5-Chlor-3-fluorpyridin-2-yl)methyl]-8,8-difluor-5-{[(3S)-3-fluorpyrrolidin-1-yl]carbonyl}-5,6,7,8-tetrahydro[1,2,4]triazolo[4,3-a]pyridin-3(2H)-on

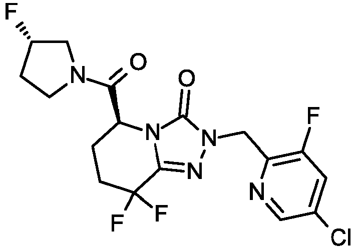

Unter Argon: (5S)-2-[(5-Chlor-3-fluorpyridin-2-yl)methyl]-5-{[(3S)-3-fluorpyrrolidin-1-yl]carbonyl}-6,7-dihydro[1,2,4]triazolo[4,3-a]pyridin-3,8(2H,5H)-dion (25.8 mg, 79 % Reinheit, 49.4 µmol) wurde in Dichlormethan (5.1 ml) bei Raumtemperatur vorgelegt. Anschließend wurde Diethylaminoschwefeltrifluorid (20 µl, 150 µmol) zugegeben und bei 40°C über Nacht gerührt. Es wurde erneut Diethylaminoschwefeltrifluorid (8 µl, 59 µmol) zugegeben and bei 40°C für 2 Stunden gerührt. Das Reaktionsgemisch wurde mit Wasser und gesättigter wässriger Natriumhydrogencarbonat-Lösung versetzt. Die wässrige Phase wurde viermal mit Dichlormethan extrahiert und die vereinigten organischen Phasen wurden über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Die produkthaltigen Fraktionen wurden im Vakuum eingeengt und es wurden 9.10 mg (42 % d. Th.) der Titelverbindung erhalten.
LC-MS (Methode 3): Rₜ = 1.42 min; MS (ESIpos): m/z = 434 [M+H]⁺
¹H-NMR (400 MHz, DMSO-d6) δ [ppm]: -0.149 (1.72), -0.008 (14.25), 0.008 (16.00), 0.146 (1.68), 1.148 (0.62), 2.144 (1.21), 2.249 (1.13), 2.327 (4.24), 2.366 (2.01), 2.670 (3.11), 2.710 (1.46), 3.416 (0.95), 3.477 (0.69), 3.520 (0.77), 3.611 (0.62), 3.637 (1.86), 3.668 (1.72), 3.698 (1.28), 3.745 (0.99), 3.767 (1.53), 3.790 (1.21), 3.813 (0.88), 3.881 (1.79), 4.893 (1.13), 4.952 (1.46), 5.084 (1.02), 5.123 (4.05), 5.144 (2.63), 5.183 (0.73), 5.273 (0.99), 5.403 (1.21), 5.528 (0.66), 8.136 (2.30), 8.155 (2.23), 8.160 (2.37), 8.501 (3.14).

### Beispiel 557

### (5S)-2-[(6-Chlorpyridin-3-yl)methyl]-5-[(3,3-difluorpyrrolidin-1-yl)carbonyl]-8,8-difluor-5,6,7,8-tetrahydro[1,2,4]triazolo[4,3-a]pyridin-3(2H)-on

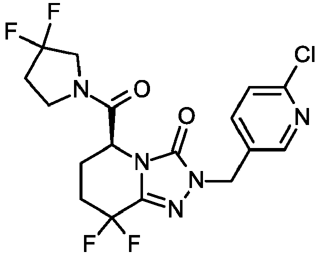

(5S)-2-[(6-Chlorpyridin-3-yl)methyl]-5-[(3,3-difluorpyrrolidin-1-yl)carbonyl]-6,7-dihydro[1,2,4]triazolo[4,3-a]pyridin-3,8(2H,5H)-dion (47.3 mg, 115 µmol) wurde unter Argon in Dichlormethan (12 ml, 180 mmol) bei Raumtemperatur vorgelegt. Anschließend wurde Diethylaminoschwefeltrifluorid (46 µl, 340 µmol) zugegeben und bei 40°C über Nacht gerührt. Das Reaktionsgemisch wurde mit Wasser und gesättigter wässriger Natriumhydrogencarbonat-Lösung versetzt. Die wässrige Phase wurde viermal mit Dichlormethan extrahiert und die vereinigten organischen Phasen wurden über Natriumsulfat getrocknet, filtriert und das Filtrat eingeengt. Der Rückstand wurde über präparative HPLC aufgereinigt (Chromatorex C18, 10µm, 125mm x 30mm; Laufmittel: (Acetonitril/Wasser mit 0.1% Ameisensäure)-Gradient). Die produkthaltigen Fraktionen wurden im Vakuum eingeengt und es wurden 40 mg (80 % d. Th.) der Titelverbindung erhalten.
LC-MS (Methode 3): Rₜ = 1.50 min; MS (ESIpos): m/z = 434 [M+H]⁺
¹H-NMR (400 MHz, DMSO-d6) δ [ppm]: -0.149 (0.94), -0.008 (7.23), 0.008 (7.13), 0.146 (0.88), 2.281 (2.47), 2.323 (2.09), 2.327 (2.43), 2.366 (1.18), 2.416 (1.59), 2.442 (1.91), 2.463 (1.83), 2.523 (3.29), 2.564 (1.51), 2.580 (1.04), 2.597 (0.78), 2.617 (0.48), 2.665 (1.00), 2.670 (1.26), 2.710 (0.58), 3.556 (1.97), 3.575 (3.31), 3.595 (1.83), 3.696 (1.10), 3.729 (1.30), 3.760 (0.88), 3.791 (1.35), 3.824 (1.73), 3.843 (1.32), 3.862 (0.80), 3.881 (0.72), 3.900 (1.41), 3.919 (0.78), 3.927 (0.84), 4.042 (0.78), 4.070 (0.52), 4.085 (0.72), 4.112 (0.42), 4.146 (0.50), 4.181 (0.66), 4.204 (0.70), 4.936 (1.59), 5.008 (1.59), 5.045 (16.00), 7.539 (4.94), 7.560 (6.24), 7.724 (3.37), 7.730 (3.41), 7.744 (2.75), 7.750 (2.87), 8.334 (4.38), 8.340 (4.38).

### Beispiel 558

### (5S)-5-{[(3R,4S)-3,4-Difluorpyrrolidin-1-yl]carbonyl}-2-{[6-(trifluormethyl)pyridin-3-yl]methyl}-5,6-dihydro[1,2,4]triazolo[4,3-a]pyridin-3(2H)-on

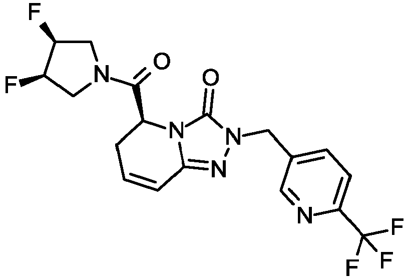

(5S)-5-{[(3R,4S)-3,4-Difluorpyrrolidin-1-yl]carbonyl}-2-{[6-(trifluormethyl)pyridin-3-yl]methyl}-5,6,7,8-tetrahydro[1,2,4]triazolo[4,3-a]pyridin-3(2H)-on (176 mg, 408 µmol) und Cer(IV)sulfat (542 mg, 1.63 mmol) wurden in tert-Butanol (1.7 ml) bei Raumtemperatur suspendiert. Anschließend wurde 1 N wässrige Schwefelsäure (1.7 ml, 32 mmol) zugegeben und das Reaktionsgemisch für 72 Stunden bei 70°C gerührt. Das Reaktionsgemisch wurde auf Raumtemperatur abgekühlt und mit 2 N wässriger Natriumhydroxid-Lösung auf pH 9 gestellt. Die Suspension wurde filtriert und das Filtrat dreimal mit Dichlormethan extrahiert. Die vereinigten organischen Phasen wurden über Natriumsulfat getrocknet, filtriert und das Filtrat eingeengt. Der Rückstand wurde über präparative HPLC aufgereinigt (Chromatorex C18, 10µm, 125mm x 40mm; Laufmittel: (Acetonitril/Wasser mit 0.1% Ameisensäure)-Gradient). Die produkthaltigen Fraktionen wurden im Vakuum eingeengt und der Rückstand wurde durch chirale präparative HPLC getrennt [Probenvorbereitung: 34.8 mg in 3 ml Ethanol gelöst; Injektionsvolumen: 0.3 ml; Säule: Daicel Chiralcel OX-H 5 µm, 250 x 20 mm; Laufmittel: n-Heptan/Ethanol 25:75; Fluss: 15 ml/min; Temperatur 50°C; UV-Detektion: 220 nm]. Die produkthaltigen Fraktionen wurden im Vakuum eingeengt und es wurden 11.0 mg (6 % d. Th.) der Titelverbindung erhalten.
LC-MS (Methode 1): Rₜ = 1.01 min; MS (ESIpos): m/z = 430 [M+H]⁺
¹H-NMR (400 MHz, DMSO-d6) δ [ppm]: -0.149 (1.91), -0.008 (15.66), 0.008 (16.00), 0.146 (1.78), 0.996 (3.35), 1.006 (3.56), 1.131 (1.98), 1.150 (3.83), 1.168 (2.05), 1.223 (2.53), 1.240 (2.74), 1.347 (2.87), 1.475 (2.74), 2.287 (7.04), 2.327 (2.46), 2.366 (1.85), 2.670 (2.94), 2.709 (1.85), 2.906 (1.37), 3.777 (0.62), 3.896 (0.62), 3.950 (0.55), 5.087 (4.85), 5.108 (0.82), 6.250 (0.62), 6.316 (1.23), 7.923 (4.72), 8.661 (2.05).

### Beispiel 559

### (5S)-5-{[(3S)-3-Fluorpyrrolidin-1-yl]carbonyl}-2-{[2-(trifluormethyl)pyrimidin-5-yl]methyl}-5,6,7,8-tetrahydro[1,2,4]triazolo[4,3-a]pyridin-3(2H)-on

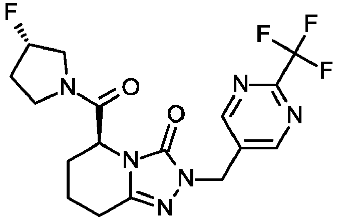

(5S)-3-Oxo-2-{[2-(trifluormethyl)pyrimidin-5-yl]methyl}-2,3,5,6,7,8-hexahydro[1,2,4]triazolo[4,3-a]pyridin-5-carbonsäure (22.0 mg, 79 % Reinheit, 26.9 µmol) wurde in THF (2.0 ml) bei Raumtemperatur vorgelegt. Anschließend wurde HBTU (31.6 mg, 83.3 µmol) und N,N-Diisopropylethylamin (56 µl, 320 µmol) zugegeben. Nach 5 min Rühren wurde (3S)-3-Fluorpyrrolidinhydrochlorid (9.66 mg, 76.9 µmol) zugeben und das Reaktionsgemisch über Nacht bei Raumtemperatur gerührt. Das Reaktionsgemisch wurde mit Ethylacetat versetzt. Die organische Phase wurde mit gesättigter Natriumhydrogencarbonat-Lösung, Wasser und gesättigter wässriger Natriumchlorid-Lösung gewaschen, über Natriumsulfat getrocknet, filtriert und das Filtrat eingeengt. Der Rückstand wurde über präparative HPLC aufgereinigt (Methode 14). Die produkthaltigen Fraktionen wurden im Vakuum eingeengt und es wurden 0.4 mg (3.6 % d. Th.) der Titelverbindung erhalten.
LC-MS (Methode 3): Rₜ = 1.30 min; MS (ESIpos): m/z = 415 [M+H]⁺
¹H-NMR (400 MHz, DMSO-d6) δ [ppm]: -0.149 (2.02), -0.008 (16.00), 0.008 (15.11), 0.146 (1.96), 1.148 (0.70), 1.405 (2.85), 2.327 (4.62), 2.366 (1.96), 2.393 (5.82), 2.670 (4.17), 2.710 (1.71), 3.876 (0.57), 9.068 (1.90).

### Beispiel 560

### (5S)-2-[(3-Chlor-5-fluorpyridin-2-yl)methyl]-5-{[(3S)-3-fluorpyrrolidin-1-yl]carbonyl}-5,6,7,8-tetrahydro[1,2,4]triazolo[4,3-a]pyridin-3(2H)-on

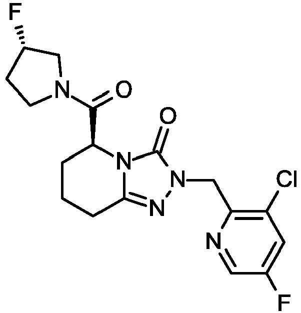

(5S)-2-[(3-Chlor-5-fluorpyridin-2-yl)methyl]-3-oxo-2,3,5,6,7,8-hexahydro[1,2,4]triazolo[4,3-a]pyridin-5-carbonsäure (150 mg, 459 µmol) wurde in THF (37 µl) vorgelegt und HBTU (226 mg, 597 µmol) und N,N-Diisopropylethylamin (240 µl, 1.4 mmol) wurden anschließend zugegeben. Nach 15 min Rühren bei Raumtemperatur wurde (3S)-3-Fluorpyrrolidinhydrochlorid (63.4 mg, 505 µmol) zugeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die Produkthaltigen Fraktionen wurden im Vakuum eingeengt und es wurden 42.7 mg (23 % d. Th.) der Titelverbindung erhalten.
LC-MS (Methode 3): Rₜ = 1.04 min; MS (ESIpos): m/z = 398 [M+H]⁺
¹H-NMR (400 MHz, DMSO-d6) δ [ppm]: -0.149 (0.48), -0.008 (3.94), 0.008 (3.64), 0.146 (0.42), 1.243 (0.46), 1.258 (0.46), 1.726 (1.88), 1.894 (0.48), 2.016 (0.71), 2.073 (16.00), 2.094 (1.03), 2.264 (0.57), 2.327 (1.25), 2.366 (0.69), 2.578 (1.45), 2.621 (0.53), 2.669 (1.25), 2.710 (0.67), 3.359 (0.42), 3.405 (0.48), 3.501 (0.46), 3.628 (1.23), 3.651 (1.19), 3.680 (0.77), 3.724 (0.91), 3.746 (1.03), 3.769 (0.83), 3.790 (0.67), 3.857 (1.37), 4.680 (0.65), 4.695 (0.79), 4.705 (0.65), 4.736 (0.79), 4.745 (0.89), 4.751 (1.01), 4.761 (0.77), 4.940 (1.21), 4.980 (3.19), 5.016 (1.88), 5.026 (2.16), 5.056 (0.63), 5.065 (0.87), 5.257 (0.65), 5.388 (0.85), 5.509 (0.51), 8.101 (1.66), 8.107 (1.82), 8.122 (1.78), 8.129 (1.84), 8.546 (3.25), 8.553 (3.19).

### Beispiel 561

### (5RS)-2-[(6-Chlorpyridin-3-yl)methyl]-5-{[(3S)-3-fluorpyrrolidin-1-yl]carbonyl}-5,6,7,8-tetrahydro[1,2,4]triazolo[4,3-a]pyridin-3(2H)-on (Racemat)

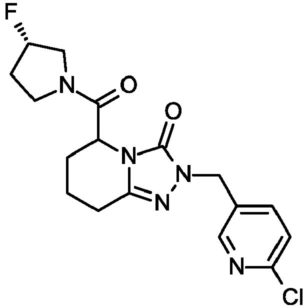

(5RS)-2-[(6-Chlorpyridin-3-yl)methyl]-3-oxo-2,3,5,6,7,8-hexahydro[1,2,4]triazolo[4,3-a]pyridin-5-carbonsäure (Racemat)(130 mg, 421 µmol) wurde in THF (34 µl) vorgelegt und 1-[Bis(dimethylamino)methylen]-1H-benzotriazol-1-ium-3-oxidhexafluorophosphat (208 mg, 547 µmol) und N,N-Diisopropylethylamin (220 µl, 1.3 mmol) wurden anschließend zugegeben. Nach 15 min Rühren bei Raumtemperatur wurde (3S)-3-Fluorpyrrolidinhydrochlorid (58.2 mg, 463 µmol) zugeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm x 30mm; Laufmittel: Acetonitril/Wasser-Gradient). Die Produkthaltigen Fraktionen wurden im Vakuum eingeengt und es wurden 73.6 mg (, 46 % d. Th.) der Titelverbindung erhalten.
LC-MS (Methode 3): Rₜ = 1.02 min; MS (ESIpos): m/z = 380 [M+H]⁺
¹H-NMR (400 MHz, DMSO-d6) δ [ppm]: -0.149 (1.02), -0.008 (13.52), 0.008 (9.53), 0.146 (1.12), 1.110 (0.63), 1.147 (0.44), 1.727 (2.58), 1.908 (0.63), 1.993 (3.02), 2.102 (2.14), 2.137 (1.70), 2.255 (1.26), 2.366 (1.70), 2.518 (12.01), 2.523 (9.78), 2.558 (3.50), 2.601 (3.84), 2.613 (2.33), 2.644 (1.41), 2.710 (1.80), 2.881 (14.25), 3.391 (1.07), 3.519 (1.70), 3.543 (1.60), 3.568 (1.46), 3.595 (2.67), 3.632 (1.95), 3.654 (1.75), 3.675 (1.51), 3.696 (0.78), 3.717 (0.92), 3.741 (1.75), 3.772 (1.26), 3.781 (1.22), 3.851 (1.60), 3.939 (1.22), 4.002 (0.63), 4.035 (0.39), 4.678 (0.83), 4.687 (0.97), 4.751 (1.26), 4.822 (1.41), 4.860 (1.80), 4.881 (16.00), 5.259 (1.12), 5.349 (0.83), 5.390 (1.22), 5.479 (0.83), 5.510 (0.63), 7.505 (6.42), 7.525 (7.88), 7.683 (3.02), 7.690 (5.45), 7.696 (3.21), 7.704 (2.48), 7.710 (4.38), 7.717 (2.33), 8.297 (5.25).

### Beispiel 562

### (5RS,7RS)-2-{[3-Chlor-5-(trifluormethyl)pyridin-2-yl]methyl}-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on (Diasteromerengemisch, 2 Isomere)

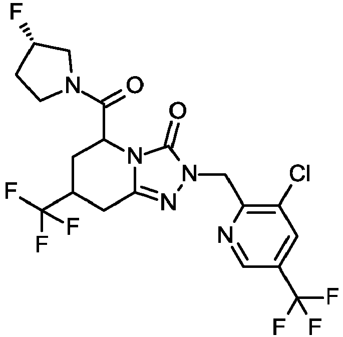

(5RS,7RS)-2-{[3-Chlor-5-(trifluormethyl)pyridin-2-yl]methyl}-3-oxo-7-(trifluormethyl)-2,3,5,6,7,8-hexahydro[1,2,4]triazolo[4,3-a]pyridin-5-carbonsäure (Diasteromeregemisch, 4 Isomere) (423 mg, 50 % Reinheit, 476 µmol) wurde in THF (4.5 ml) vorgelegt und HBTU (234 mg, 618 µmol) und N,N-Diisopropylethylamin (250 µl, 1.4 mmol) wurden anschließend zugegeben. Nach 15 min Rühren bei Raumtemperatur wurde (3S)-3-Fluorpyrrolidinhydrochlorid (71.7 mg, 571 µmol) zugeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die Produkthaltigen Fraktionen wurden im Vakuum eingeengt und es wurden 54.6 mg (22 % d. Th.) der Titelverbindung erhalten.
LC-MS (Methode 3): Rₜ = 1.70 min; MS (ESIpos): m/z = 516 [M+H]⁺
¹H-NMR (400 MHz, DMSO-d6) δ [ppm]: -0.149 (1.14), -0.008 (16.00), 0.008 (8.79), 0.146 (1.14), 1.148 (0.44), 2.166 (1.01), 2.230 (1.31), 2.278 (1.11), 2.327 (2.72), 2.366 (1.34), 2.523 (8.12), 2.669 (3.05), 2.696 (1.07), 2.710 (1.84), 2.729 (0.64), 2.963 (1.61), 2.995 (1.27), 3.572 (0.57), 3.637 (0.84), 3.670 (0.77), 3.853 (0.50), 3.998 (0.57), 4.946 (0.44), 4.982 (0.54), 5.023 (0.50), 5.105 (0.70), 5.147 (2.62), 5.167 (1.88), 5.177 (1.88), 5.218 (0.64), 5.268 (0.54), 5.399 (0.64), 8.501 (2.48), 8.893 (2.55).

### Beispiel 563

### (5RS,7RS)-2-{[3-Chlor-5-(trifluormethyl)pyridin-2-yl]methyl}-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on (Isomer 2)

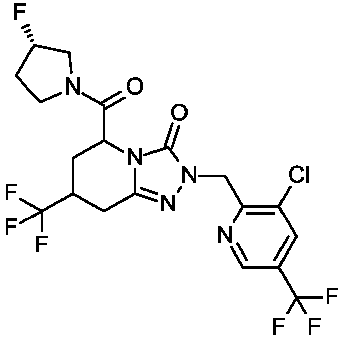

(5RS,7RS)-2-{[3-Chlor-5-(trifluormethyl)pyridin-2-yl]methyl}-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on (Diasteromeregemisch, 2 Isomere) wurde durch chirale präparative HPLC getrennt [Probenvorbereitung: 55 mg gelöst in 8 ml Acetonitril; Injektionsvolumen: 0.9 ml; Säule: Daicel Chiralpak^{®} AD, 250 × 20 mm; Laufmittel: CO₂/Isopropanol 85:15; Fluss: 80 ml/min; Temperatur 40°C; UV Detektion: 210 nm]. Nach der Trennung wurden 17 mg von Isomer 1, welches zuerst eluierte, und 18 mg von Isomer 2, welches später eluierte, isoliert.

### Isomer 2:

Analytische chirale HPLC: Rₜ = 2.4 min, d.e. = 98% [Säule: Daicel Chiralpak^{®} AD 50 × 4.6 mm; Laufmittel: CO₂/Isopropanol 85:15; Fluss: 3 ml/min; UV-Detektion: 210 nm].
LC-MS (Methode 3): Rₜ = 1.70 min; MS (ESIpos): m/z = 516 [M+H]⁺
¹H-NMR (400 MHz, DMSO-d6) δ [ppm]: -0.149 (0.86), -0.008 (6.12), 0.008 (5.73), 0.068 (0.53), 0.146 (0.79), 1.029 (0.66), 1.045 (0.66), 1.146 (0.66), 1.999 (1.05), 2.104 (2.17), 2.137 (3.42), 2.164 (6.45), 2.200 (4.61), 2.215 (4.48), 2.231 (5.40), 2.245 (4.61), 2.280 (6.98), 2.322 (4.48), 2.327 (4.81), 2.366 (1.51), 2.523 (10.01), 2.652 (2.77), 2.669 (5.86), 2.685 (4.41), 2.697 (5.73), 2.710 (5.14), 2.728 (3.75), 2.743 (2.44), 2.967 (9.81), 2.999 (8.36), 3.342 (2.77), 3.353 (2.90), 3.371 (2.90), 3.380 (2.17), 3.399 (2.83), 3.433 (2.37), 3.441 (2.30), 3.503 (1.71), 3.530 (2.37), 3.539 (2.24), 3.576 (5.40), 3.631 (8.95), 3.643 (3.75), 3.670 (4.81), 3.694 (5.40), 3.724 (3.29), 3.786 (1.19), 3.831 (2.70), 3.852 (4.61), 3.874 (2.90), 3.905 (1.98), 3.927 (2.24), 3.960 (1.58), 3.985 (2.50), 4.016 (1.32), 4.933 (3.62), 4.947 (3.88), 4.983 (5.53), 4.997 (5.40), 5.101 (4.48), 5.142 (14.42), 5.167 (9.81), 5.178 (11.98), 5.208 (2.44), 5.218 (4.21), 5.267 (3.49), 5.401 (4.67), 5.525 (2.37), 7.799 (0.99), 7.821 (1.51), 7.946 (1.45), 7.967 (1.25), 8.500 (15.01), 8.504 (16.00), 8.894 (15.54).

### Beispiel 564

### (5RS,7RS)-2-{[3-Chlor-5-(trifluormethyl)pyridin-2-yl]methyl}-5-[(3,3-difluorpyrrolidin-1-yl)carbonyl]-7-(trifluormethyl)-5,6,7,8-tetrahydro[1,2,4]triazolo[4,3-a]pyridin-3(2H)-on (Diastereomer 1; Racemat)

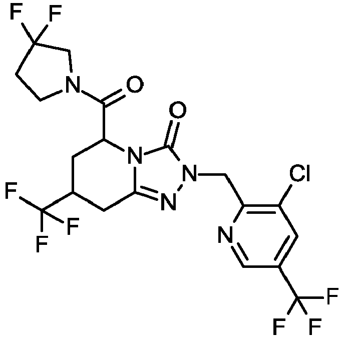

(5RS,7RS)-2-{[3-Chlor-5-(trifluormethyl)pyridin-2-yl]methyl}-3-oxo-7-(trifluormethyl)-2,3,5,6,7,8-hexahydro[1,2,4]triazolo[4,3-a]pyridin-5-carbonsäure (Diasteromeregemisch, 4 Isomere) (423 mg, 951 µmol) wurde in THF (9.0 ml) vorgelegt und 1-[Bis(dimethylamino)methylen]-1H-benzotriazol-1-ium-3-oxidhexafluorophosphat (469 mg, 1.24 mmol) und N,N-Diisopropylethylamin (500 µl, 2.9 mmol) wurden anschließend zugegeben. Nach 15 min Rühren bei Raumtemperatur wurde 3,3-Difluorpyrrolidinhydrochlorid (164 mg, 1.14 mmol) zugeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient) und das Diastereomer 1 isoliert. Die Produkthaltigen Fraktionen wurden im Vakuum eingeengt und es wurden 48.0 mg (9 % d. Th.) der Titelverbindung erhalten.
LC-MS (Methode 3): Rₜ = 1.85 min; MS (ESIpos): m/z = 534 [M+H]⁺
¹H-NMR (400 MHz, DMSO-d6) δ [ppm]: -0.149 (0.81), -0.008 (6.61), 0.008 (7.06), 0.146 (0.71), 2.073 (16.00), 2.158 (0.58), 2.194 (0.81), 2.240 (0.58), 2.272 (1.10), 2.323 (1.45), 2.327 (1.74), 2.366 (0.84), 2.424 (0.87), 2.440 (0.87), 2.523 (4.03), 2.669 (2.06), 2.702 (1.32), 2.710 (1.58), 2.734 (0.81), 2.959 (2.10), 2.996 (1.35), 3.540 (0.68), 3.560 (1.32), 3.581 (1.16), 3.601 (0.65), 3.711 (0.61), 3.746 (0.97), 3.781 (1.03), 3.808 (0.61), 3.905 (0.68), 3.921 (1.39), 3.939 (1.48), 3.957 (0.61), 4.137 (0.55), 4.179 (0.90), 4.205 (0.87), 4.238 (0.52), 4.966 (1.06), 4.980 (1.03), 5.050 (1.06), 5.067 (1.00), 5.107 (1.16), 5.148 (4.26), 5.174 (4.48), 5.214 (1.23), 8.505 (3.45), 8.890 (3.42).

### Beispiel 565

### (5RS,7RS)-2-{[3-Chlor-5-(trifluormethyl)pyridin-2-yl]methyl}-5-[(3,3-difluorpyrrolidin-1-yl)carbonyl]-7-(trifluormethyl)-5,6,7,8-tetrahydro[1,2,4]triazolo[4,3-a]pyridin-3(2H)-on (Enantiomer 2)

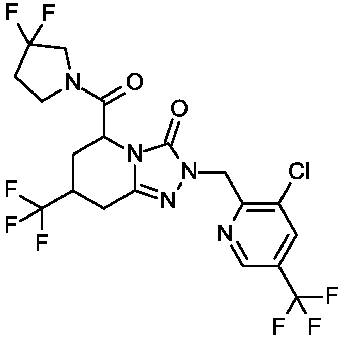

(5RS,7RS)-2-{[3-Chlor-5-(trifluormethyl)pyridin-2-yl]methyl}-5-[(3,3-difluorpyrrolidin-1-yl)carbonyl]-7-(trifluormethyl)-5,6,7,8-tetrahydro[1,2,4]triazolo[4,3-a]pyridin-3(2H)-on (Diastereomer 1; Racemat) wurde durch chirale präparative HPLC getrennt [Probenvorbereitung: 48 mg gelöst in 2.5 ml Ethanol/Acetonitril (1:1); Injektionsvolumen: 0.15 ml; Säule: Daicel Chiralcel^{®} OX-H 5 µm, 250 × 20 mm; Laufmittel: n-Heptan/Ethanol (10/80) + 0.2 % Diethylamin; Fluss: 30 ml/min; Temperatur 28°C; UV Detektion: 220 nm]. Nach der Trennung wurden 9.9 mg von Enantiomer 1, welches zuerst eluierte, und 9.1 mg von Enantiomer 2, welches später eluierte, isoliert.

### Enantiomer 2:

Analytische chirale HPLC: Rₜ = 6.38 min, e.e. = 100% [Säule: Daicel Chiralcel^{®} OX-H-3 250 × 4.6 mm; Laufmittel: Heptan/Ethanol (1:1) + 0.2 % Diethylamin; Fluss: 1 ml/min; UV-Detektion: 220 nm].
LC-MS (Methode 3): Rₜ = 1.82 min; MS (ESIpos): m/z = 534 [M+H]⁺
¹H-NMR (400 MHz, DMSO-d6) δ [ppm]: -0.149 (2.14), -0.008 (15.25), 0.008 (16.00), 0.146 (1.94), 0.854 (0.45), 1.075 (0.89), 1.141 (1.44), 1.235 (1.49), 2.160 (1.34), 2.194 (2.09), 2.225 (1.19), 2.240 (0.99), 2.274 (2.63), 2.323 (3.43), 2.327 (4.17), 2.366 (2.09), 2.395 (1.44), 2.425 (1.89), 2.669 (5.17), 2.692 (2.34), 2.702 (3.38), 2.710 (4.22), 2.735 (1.94), 2.961 (5.22), 2.994 (3.33), 3.509 (0.84), 3.541 (1.54), 3.561 (3.18), 3.582 (2.78), 3.602 (1.44), 3.712 (1.39), 3.746 (2.34), 3.781 (2.53), 3.808 (1.49), 3.905 (1.64), 3.922 (3.28), 3.939 (3.58), 3.957 (1.49), 4.138 (1.39), 4.180 (2.14), 4.206 (2.04), 4.236 (1.14), 4.965 (2.43), 4.979 (2.43), 5.051 (2.53), 5.065 (2.48), 5.107 (2.78), 5.148 (10.68), 5.174 (11.33), 5.214 (2.93), 8.502 (7.90), 8.890 (7.90).

### Beispiel 566

### (5RS,7RS)-2-[(5-Chlorpyridin-2-yl)methyl]-5-[(3,3-difluorpyrrolidin-1-yl)carbonyl]-7-(trifluormethyl)-5,6,7,8-tetrahydro[1,2,4]triazolo[4,3-a]pyridin-3(2H)-on (Diastereomer 1; Racemat)

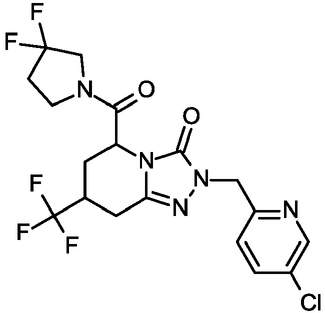

(5RS,7RS)-2-[(5-Chlorpyridin-2-yl)methyl]-3-oxo-7-(trifluormethyl)-2,3,5,6,7,8-hexahydro[1,2,4]triazolo[4,3-a]pyridin-5-carbonsäure (Diasteromeregemisch, 4 Isomere) (393 mg, 76 % Reinheit, 793 µmol) wurde in THF (3.8 ml) vorgelegt und HBTU (391 mg, 1.03 mmol) und N,N-Diisopropylethylamin (410 µl, 2.4 mmol) wurden anschließend zugegeben. Nach 15 min Rühren bei Raumtemperatur wurde 3,3-Difluorpyrrolidinhydrochlorid (137 mg, 951 µmol) zugeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die Produkthaltigen Fraktionen wurden im Vakuum eingeengt und es wurden 171 mg (45 % d. Th.) der Titelverbindung erhalten.
LC-MS (Methode 4): Rₜ = 0.87 min; MS (ESIpos): m/z = 466 [M+H]⁺
¹H-NMR (400 MHz, DMSO-d6) δ [ppm]: 2.125 (0.60), 2.141 (0.72), 2.161 (1.46), 2.172 (1.67), 2.192 (1.64), 2.206 (2.29), 2.220 (1.51), 2.235 (1.39), 2.251 (1.32), 2.273 (2.84), 2.290 (3.01), 2.327 (2.24), 2.377 (0.97), 2.396 (1.79), 2.426 (2.36), 2.443 (2.27), 2.461 (1.61), 2.565 (2.70), 2.583 (2.16), 2.603 (1.46), 2.686 (1.65), 2.714 (2.61), 2.723 (3.94), 2.732 (2.44), 2.754 (2.45), 2.936 (2.06), 2.972 (5.32), 3.013 (2.95), 3.518 (0.44), 3.538 (0.84), 3.549 (1.85), 3.567 (4.66), 3.586 (3.76), 3.602 (1.90), 3.681 (0.54), 3.714 (1.84), 3.753 (3.10), 3.785 (3.25), 3.816 (1.94), 3.850 (0.49), 3.879 (0.44), 3.905 (1.92), 3.922 (3.99), 3.939 (4.34), 3.957 (1.84), 3.982 (0.44), 4.112 (0.52), 4.141 (1.65), 4.169 (1.47), 4.181 (2.52), 4.211 (2.65), 4.239 (1.52), 4.896 (2.75), 4.937 (14.75), 4.953 (16.00), 4.979 (3.36), 4.993 (3.14), 5.046 (3.20), 5.060 (3.14), 7.221 (8.97), 7.242 (9.62), 7.919 (6.01), 7.926 (6.46), 7.941 (5.80), 7.947 (6.12), 8.574 (8.32), 8.579 (8.50).

### Beispiel 567

### (5RS,7RS)-2-[(5-Chlorpyridin-2-yl)methyl]-5-[(3,3-difluorpyrrolidin-1-yl)carbonyl]-7-(trifluormethyl)-5,6,7,8-tetrahydro[1,2,4]triazolo[4,3-a]pyridin-3(2H)-on (Enantiomer 2)

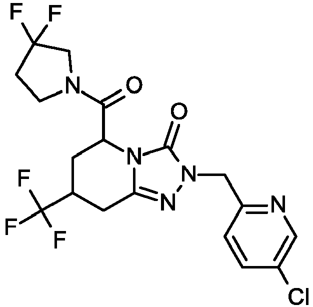

(5RS,7RS)-2-[(5-Chlorpyridin-2-yl)methyl]-5-[(3,3-difluorpyrrolidin-1-yl)carbonyl]-7-(trifluormethyl)-5,6,7,8-tetrahydro[1,2,4]triazolo[4,3-a]pyridin-3(2H)-on (Diastereomer 1; Racemat) wurde durch chirale präparative HPLC getrennt [Probenvorbereitung: 170 mg gelöst in 5 ml Ethanol/n-Heptan (3:2) ; Injektionsvolumen: 0.5 ml; Säule: Daicel Chiralpak^{®} ID, 250 × 20 mm; Laufmittel: n-Heptan/Ethanol 60:40; Fluss: 15 ml/min; Temperatur 45°C; UV Detektion: 210 nm]. Nach der Trennung wurden 77 mg von Isomer 1, welches zuerst eluierte, und 75 mg von Isomer 2, welches später eluierte, isoliert.

### Enantiomer 2:

Analytische chirale HPLC: Rₜ = 3.17 min, e.e. = 100% [Säule: Daicel Chiralpak^{®} IC-3 50 × 4.6 mm; Laufmittel: n-Heptan/Ethanol 70:30; Fluss: 1 ml/min; UV-Detektion: 220 nm].
LC-MS (Methode 3): Rₜ = 1.58 min; MS (ESIpos): m/z = 466 [M+H]⁺
¹H-NMR (400 MHz, DMSO-d6) δ [ppm]: -0.149 (1.00), -0.008 (7.03), 0.008 (7.68), 0.146 (0.79), 1.149 (0.72), 1.233 (0.50), 2.161 (1.43), 2.176 (1.43), 2.205 (2.01), 2.235 (1.22), 2.250 (1.22), 2.291 (2.58), 2.327 (4.66), 2.366 (2.37), 2.395 (1.65), 2.425 (2.15), 2.441 (2.01), 2.523 (9.54), 2.564 (3.73), 2.582 (2.51), 2.669 (4.23), 2.711 (3.66), 2.723 (3.80), 2.732 (2.51), 2.753 (2.37), 2.972 (4.66), 3.013 (2.73), 3.548 (1.79), 3.567 (4.30), 3.586 (3.37), 3.601 (1.79), 3.680 (0.57), 3.714 (1.72), 3.753 (2.80), 3.784 (2.80), 3.816 (1.72), 3.848 (0.57), 3.904 (1.94), 3.922 (3.52), 3.937 (3.80), 3.956 (1.72), 4.141 (1.51), 4.179 (2.22), 4.209 (2.37), 4.236 (1.36), 4.895 (2.73), 4.936 (14.28), 4.952 (16.00), 4.978 (2.94), 4.993 (3.16), 5.045 (2.87), 5.059 (2.94), 7.220 (8.47), 7.241 (9.26), 7.920 (6.67), 7.926 (6.82), 7.941 (6.39), 7.947 (6.67), 8.574 (5.96), 8.579 (6.10).

### Beispiel 568

### (5S)-2-[(5-Chlor-3-fluorpyridin-2-yl)methyl]-5-{[(3S)-3-fluorpyrrolidin-1-yl]carbonyl}-5,6,7,8-tetrahydro[1,2,4]triazolo[4,3-a]pyridin-3(2H)-on

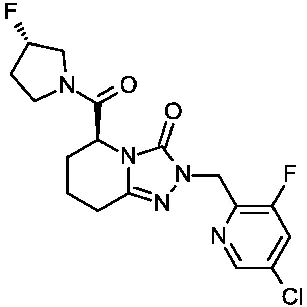

(5S)-2-[(5-Chlor-3-fluorpyridin-2-yl)methyl]-3-oxo-2,3,5,6,7,8-hexahydro[1,2,4]triazolo[4,3-a]pyridin-5-carbonsäure (500 mg, 1.53 mmol) wurde in THF (10 ml) vorgelegt und HBTU (755 mg, 1.99 mmol) und N,N-Diisopropylethylamin (800 µl, 4.6 mmol) wurden anschließend zugegeben. Nach 15 min Rühren bei Raumtemperatur wurde (3S)-3-Fluorpyrrolidinhydrochlorid (211 mg, 1.68 mmol) zugeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die Produkthaltigen Fraktionen werden im Vakuum eingeengt und es wurden 224 mg (37 % d. Th.) der Titelverbindung erhalten.
LC-MS (Methode 4): Rₜ = 0.64 min; MS (ESIpos): m/z = 398 [M+H]⁺
¹H-NMR (400 MHz, DMSO-d6) δ [ppm]: -0.149 (0.47), -0.008 (4.50), 0.008 (3.40), 0.146 (0.48), 1.177 (0.66), 1.707 (8.23), 1.716 (9.32), 1.848 (1.55), 1.872 (1.42), 1.884 (2.38), 1.894 (2.16), 1.962 (2.26), 1.996 (3.78), 2.033 (2.11), 2.051 (3.61), 2.069 (5.37), 2.085 (4.80), 2.104 (4.92), 2.120 (2.73), 2.133 (2.92), 2.215 (1.98), 2.236 (2.33), 2.265 (2.73), 2.327 (1.24), 2.366 (0.60), 2.465 (1.95), 2.524 (5.83), 2.567 (6.06), 2.572 (6.32), 2.614 (2.23), 2.670 (1.05), 2.710 (0.41), 2.750 (0.55), 3.268 (1.76), 3.342 (2.24), 3.357 (1.80), 3.366 (1.83), 3.393 (2.05), 3.401 (2.04), 3.455 (1.48), 3.463 (1.61), 3.490 (2.14), 3.499 (2.00), 3.609 (1.40), 3.627 (6.06), 3.652 (6.49), 3.661 (5.02), 3.678 (3.80), 3.688 (2.92), 3.720 (3.38), 3.740 (4.82), 3.768 (3.62), 3.776 (3.09), 3.786 (3.42), 3.819 (0.66), 3.855 (6.40), 4.666 (3.14), 4.675 (3.97), 4.681 (4.07), 4.691 (3.11), 4.722 (4.04), 4.732 (4.63), 4.738 (5.18), 4.747 (3.80), 4.896 (5.44), 4.934 (13.27), 4.978 (6.77), 4.983 (8.22), 4.986 (9.25), 4.991 (8.06), 5.017 (2.71), 5.025 (4.04), 5.030 (3.54), 5.256 (3.12), 5.388 (4.35), 5.510 (2.31), 5.942 (0.72), 8.088 (9.86), 8.093 (9.96), 8.112 (9.86), 8.117 (10.03), 8.135 (5.61), 8.478 (16.00), 8.483 (15.45).

### Beispiel 569

### (5RS,7RS)-2-[(3,5-Difluorpyridin-2-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on (Diastereomerengemisch; 4 Isomere)

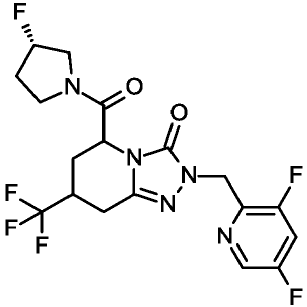

(5RS,7RS)-2-[(3,5-Difluorpyridin-2-yl)methyl]-3-oxo-7-(trifluormethyl)-2,3,5,6,7,8-hexahydro[1,2,4]triazolo[4,3-a]pyridin-5-carbonsäure (Diastereomerengemsich, 4 Isomere) (123 mg, 96 % Reinheit, 312 µmol) wurde in THF (10 ml) vorgelegt und HBTU (154 mg, 406 µmol) und N,N-Diisopropylethylamin (160 µl, 940 µmol) wurden anschließend zugegeben. Nach 15 min Rühren bei Raumtemperatur wurde (3S)-3-Fluorpyrrolidinhydrochlorid (47.0 mg, 375 µmol) zugeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die Produkthaltigen Fraktionen wurden im Vakuum eingeengt und es wurden 92.9 mg (63 % d. Th.) der Titelverbindung erhalten.
LC-MS (Methode 4): Rₜ = 0.73 min; MS (ESIpos): m/z = 450 [M+H]⁺
¹H-NMR (400 MHz, DMSO-d6) δ [ppm]: -0.149 (0.66), -0.008 (5.04), 0.008 (4.55), 0.146 (0.66), 1.312 (0.45), 2.000 (0.83), 2.073 (1.32), 2.118 (3.31), 2.149 (5.50), 2.168 (3.97), 2.185 (5.50), 2.199 (5.37), 2.216 (5.04), 2.262 (4.38), 2.294 (2.81), 2.328 (2.19), 2.366 (0.95), 2.634 (1.90), 2.643 (1.78), 2.666 (5.13), 2.677 (6.82), 2.688 (2.98), 2.709 (4.38), 2.945 (8.19), 2.977 (5.75), 3.332 (2.69), 3.344 (3.22), 3.362 (3.02), 3.373 (2.11), 3.390 (2.36), 3.426 (1.24), 3.434 (1.28), 3.475 (0.79), 3.501 (1.45), 3.511 (1.36), 3.523 (1.32), 3.568 (2.94), 3.597 (2.89), 3.616 (2.85), 3.632 (4.05), 3.641 (3.10), 3.668 (3.93), 3.693 (3.14), 3.724 (1.45), 3.766 (0.95), 3.789 (1.49), 3.820 (2.11), 3.844 (2.94), 3.864 (1.49), 3.885 (1.41), 3.917 (1.57), 3.951 (0.83), 3.976 (2.32), 3.997 (2.36), 4.020 (1.86), 4.050 (1.32), 4.080 (1.03), 4.905 (1.98), 4.919 (5.75), 4.962 (11.66), 4.994 (2.65), 5.010 (9.22), 5.052 (5.37), 5.266 (2.48), 5.274 (2.32), 5.356 (1.57), 5.400 (3.18), 5.489 (1.45), 5.526 (1.20), 7.937 (4.26), 7.943 (4.63), 7.962 (6.78), 7.966 (7.24), 7.985 (4.51), 7.990 (4.80), 8.467 (15.71), 8.472 (16.00).

### Beispiel 570

### (5RS,7RS)-2-[(3,5-Difluorpyridin-2-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on (Isomer 2)

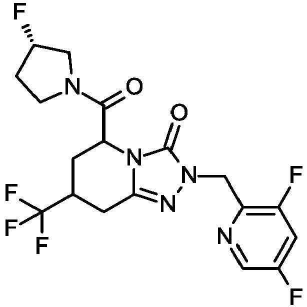

### (5RS,7RS)-2-[(3,5-Difluorpyridin-2-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on

(Diastereomerengemisch; 4 Isomere) wurde durch chirale präparative HPLC getrennt [Probenvorbereitung: 82.6 mg gelöst in 4 ml Ethanol/Acetonitril (3:1); Injektionsvolumen: 0.30 ml; Säule: Daicel Chiralcel^{®} IC 5 µm, 250 × 20 mm; Laufmittel: n-Heptan/Ethanol (50:50); Fluss: 15 ml/min; Temperatur 30°C; UV-Detektion: 210 nm]. Nach der Trennung wurden 34.4 mg von Isomer 1, welches zuerst eluierte, und 36.6mg von Isomer 2, welches später eluierte, isoliert.

### Isomer 2:

Analytische chirale HPLC: Rₜ = 2.237 min, d.e. = 100% [Säule: Daicel Chiralcel^{®} IC-3 50 × 4.6 mm; Laufmittel: n-Heptan/Ethanol 50:50; Fluss: 1 ml/min; UV-Detektion: 220 nm].
LC-MS (Methode 3): Rₜ = 1.35 min; MS (ESIpos): m/z = 450 [M+H]⁺
¹H-NMR (400 MHz, DMSO-d6) δ [ppm]: -0.150 (1.82), -0.008 (12.17), 0.008 (12.50), 0.146 (1.68), 2.148 (5.04), 2.186 (3.97), 2.215 (3.56), 2.262 (5.38), 2.296 (3.63), 2.327 (3.03), 2.366 (2.69), 2.665 (5.65), 2.678 (5.51), 2.692 (2.89), 2.710 (5.38), 2.946 (8.07), 2.981 (6.45), 3.390 (2.62), 3.434 (1.95), 3.523 (1.82), 3.646 (3.03), 3.668 (4.64), 3.692 (4.91), 3.722 (2.29), 3.820 (3.23), 3.845 (3.83), 3.867 (2.08), 3.919 (2.62), 3.976 (1.88), 4.917 (7.13), 4.956 (11.83), 5.012 (6.45), 5.052 (3.03), 5.265 (2.62), 5.396 (4.17), 5.530 (1.95), 7.937 (4.10), 7.943 (4.64), 7.967 (6.79), 7.985 (4.37), 7.991 (4.37), 8.467 (16.00), 8.472 (15.93).

### Beispiel 571

### (5RS,7RS)-2-[(3-Chlor-5-fluorpyridin-2-yl)methyl]-5-{[(3S)-3-fluorpyrrolidin-1 -yl]carbonyl}-7-(trifluormethyl)-5,6,7,8-tetrahydro[1,2,4]triazolo[4,3-a]pyridin-3(2H)-on (Diastereomerengemisch 1; 2 Isomere)

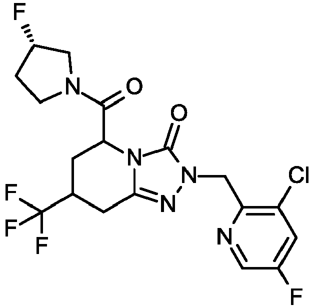

(5RS,7RS)-2-[(3-Chlor-5-fluorpyridin-2-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere) (100 mg, 253 µmol) wurde in THF (3.0 ml) vorgelegt und HBTU (125 mg, 329 µmol) und N,N-Diisopropylethylamin (130 µl, 760 µmol) wurden anschließend zugegeben. Nach 15 min Rühren bei Raumtemperatur wurde (3S)-3-Fluorpyrrolidinhydrochlorid (38.2 mg, 304 µmol) zugeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die Produkthaltigen Fraktionen wurden im Vakuum eingeengt und es wurden 85.0 mg (72 % d. Th.) der Titelverbindung erhalten.
LC-MS (Methode 3): Rₜ = 1.45 min; MS (ESIpos): m/z = 466 [M+H]⁺
¹H-NMR (400 MHz, DMSO-d6) δ [ppm]: -0.150 (0.48), -0.008 (4.20), 0.146 (0.46), 1.197 (4.10), 1.213 (4.14), 1.229 (0.78), 1.245 (3.16), 1.260 (4.36), 1.277 (2.13), 1.999 (0.66), 2.073 (6.51), 2.127 (2.51), 2.158 (4.28), 2.195 (4.66), 2.210 (4.58), 2.224 (5.13), 2.270 (4.30), 2.327 (1.21), 2.366 (0.82), 2.643 (1.61), 2.676 (3.50), 2.688 (5.07), 2.699 (2.37), 2.710 (1.65), 2.720 (2.79), 2.730 (1.89), 2.951 (6.59), 2.982 (5.39), 3.345 (2.13), 3.362 (1.95), 3.393 (1.51), 3.437 (0.94), 3.474 (0.54), 3.500 (1.39), 3.527 (1.00), 3.570 (2.35), 3.596 (2.47), 3.633 (3.26), 3.669 (2.89), 3.688 (2.01), 3.723 (1.47), 3.767 (0.68), 3.790 (1.05), 3.822 (1.47), 3.848 (1.99), 3.873 (1.27), 3.887 (1.01), 3.921 (1.05), 3.955 (0.68), 3.980 (1.85), 3.999 (2.01), 4.025 (1.53), 4.053 (1.05), 4.085 (0.76), 4.922 (1.57), 4.935 (1.67), 4.970 (4.56), 4.975 (4.56), 5.015 (10.99), 5.050 (7.88), 5.060 (9.85), 5.089 (2.65), 5.100 (3.48), 5.266 (2.03), 5.356 (1.19), 5.399 (2.57), 5.489 (1.15), 5.524 (1.05), 8.112 (7.30), 8.118 (7.84), 8.133 (7.48), 8.140 (7.86), 8.543 (16.00), 8.549 (15.62).

### Beispiel 572

### (5RS,7RS)-2-[(3-Chlor-5-fluorpyridin-2-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on (Isomer 2)

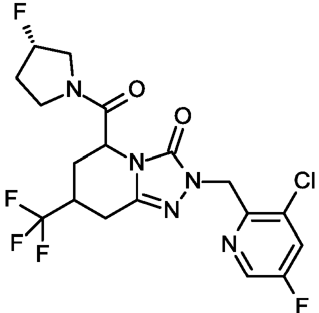

### (5RS,7RS)-2-[(3-Chlor-5-fluorpyridin-2-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on

(Diastereomerengemisch 1; 2 Isomere) wurde durch chirale präparative HPLC getrennt [Probenvorbereitung: 83.0 mg gelöst in 5 ml Iso-Propanol/Acetonitril (1:1); Injektionsvolumen: 0.30 ml; Säule: Daicel Chiralcel^{®} IB 5 µm, 250 × 20 mm; Laufmittel: n-Heptan/Iso-Propanol (25:75); Fluss: 15 ml/min; Temperatur 35°C; UV-Detektion: 220 nm]. Nach der Trennung wurden 32.5 mg von Isomer 1, welches zuerst eluierte, und 32.6 mg von Isomer 2, welches später eluierte, isoliert.

### Isomer 2:

Analytische chirale HPLC: Rₜ = 4.109 min, d.e. = 100% [Säule: Daicel Chiralcel^{®} IB-3 50 × 4.6 mm; Laufmittel: n-Heptan/Iso-Propanol 50:50; Fluss: 1 ml/min; UV-Detektion: 220 nm].
LC-MS (Methode 3): Rₜ = 1.46 min; MS (ESIpos): m/z = 466 [M+H]⁺
¹H-NMR (400 MHz, DMSO-d6) δ [ppm]: -0.150 (1.21), -0.008 (9.38), 0.008 (8.11), 0.146 (1.05), 0.852 (0.55), 1.173 (1.38), 1.235 (2.76), 1.973 (0.77), 1.999 (0.88), 2.122 (2.70), 2.159 (5.85), 2.195 (4.36), 2.210 (4.08), 2.225 (4.86), 2.240 (4.14), 2.273 (6.68), 2.297 (2.92), 2.322 (2.37), 2.327 (3.20), 2.366 (2.10), 2.523 (7.12), 2.643 (2.37), 2.660 (2.98), 2.665 (2.92), 2.669 (3.70), 2.675 (5.02), 2.688 (5.02), 2.702 (3.31), 2.709 (3.09), 2.720 (3.53), 2.734 (2.26), 2.943 (4.91), 2.954 (8.61), 2.987 (7.50), 3.338 (1.82), 3.348 (2.43), 3.366 (2.32), 3.376 (1.71), 3.394 (2.59), 3.429 (1.99), 3.438 (1.99), 3.491 (1.38), 3.500 (1.43), 3.527 (2.04), 3.535 (1.93), 3.630 (2.70), 3.642 (3.14), 3.671 (3.86), 3.678 (3.42), 3.695 (3.97), 3.723 (3.20), 3.786 (1.10), 3.821 (2.92), 3.848 (4.14), 3.873 (2.54), 3.907 (1.71), 3.922 (2.15), 3.956 (1.27), 3.981 (2.15), 4.013 (1.16), 4.922 (3.42), 4.934 (3.70), 4.970 (9.71), 4.983 (4.91), 5.010 (11.75), 5.015 (9.82), 5.051 (8.28), 5.060 (10.32), 5.090 (2.76), 5.100 (4.41), 5.266 (2.98), 5.394 (4.25), 5.525 (2.15), 8.113 (8.17), 8.119 (8.77), 8.134 (8.33), 8.140 (8.99), 8.543 (16.00), 8.549 (15.78).

### Beispiel 573

### (5RS,7RS)-2-[(3,5-Difluorpyridin-2-yl)methyl]-5-[(3,3-difluorpyrrolidin-1-yl)carbonyl]-7-(trifluormethyl)-5,6,7,8-tetrahydro[1,2,4]triazolo[4,3-a]pyridin-3(2H)-on (Diastereomer 1; Racemat)

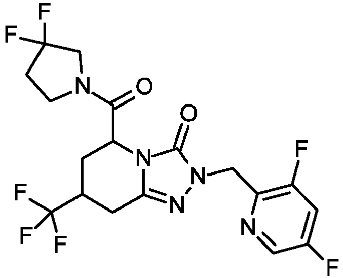

(5RS,7RS)-2-[(3,5-Difluorpyridin-2-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere) (90.0 mg, 96 % Reinheit, 228 µmol) wurde in THF (2.0 ml) vorgelegt und HBTU (113 mg, 297 µmol) und N,N-Diisopropylethylamin (120 µl, 690 µmol) wurden anschließend zugegeben. Nach 15 min Rühren bei Raumtemperatur wurde 3,3-Difluorpyrrolidinhydrochlorid (39.4 mg, 274 µmol) zugeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die Produkthaltigen Fraktionen wurden im Vakuum eingeengt und es wurden 68.7 mg (62 % d. Th.) der Titelverbindung erhalten.
LC-MS (Methode 4): Rₜ = 0.83 min; MS (ESIpos): m/z = 468 [M+H]⁺
¹H-NMR (400 MHz, DMSO-d6) δ [ppm]: -0.008 (1.74), 1.404 (2.01), 2.098 (1.04), 2.114 (1.19), 2.133 (2.22), 2.144 (2.67), 2.163 (2.48), 2.179 (3.71), 2.194 (2.19), 2.209 (2.15), 2.225 (2.01), 2.257 (4.64), 2.283 (2.63), 2.328 (0.83), 2.375 (1.44), 2.394 (2.76), 2.412 (2.48), 2.424 (3.58), 2.441 (3.18), 2.460 (2.18), 2.469 (1.85), 2.524 (2.66), 2.562 (4.10), 2.582 (3.23), 2.602 (2.30), 2.620 (1.20), 2.639 (1.82), 2.648 (2.48), 2.673 (4.46), 2.683 (6.28), 2.692 (4.09), 2.716 (3.58), 2.724 (3.18), 2.940 (9.27), 2.981 (5.71), 3.512 (0.75), 3.531 (1.47), 3.542 (3.00), 3.560 (7.48), 3.580 (5.90), 3.596 (2.96), 3.609 (1.20), 3.626 (0.72), 3.673 (0.89), 3.707 (2.73), 3.746 (4.58), 3.776 (4.66), 3.808 (2.96), 3.844 (1.14), 3.873 (0.81), 3.892 (1.79), 3.899 (2.90), 3.917 (7.20), 3.936 (7.09), 3.956 (2.73), 3.962 (1.95), 3.982 (0.81), 4.105 (0.92), 4.134 (2.60), 4.146 (0.99), 4.163 (2.09), 4.176 (4.09), 4.204 (3.97), 4.237 (2.24), 4.266 (0.65), 4.925 (5.80), 4.936 (5.21), 4.961 (13.01), 5.015 (15.41), 5.034 (4.96), 5.050 (5.57), 7.938 (4.48), 7.943 (4.78), 7.962 (6.94), 7.966 (7.24), 7.985 (4.63), 7.991 (4.78), 8.465 (16.00), 8.471 (15.52).

### Beispiel 574

### (5RS,7RS)-2-[(3,5-Difluorpyridin-2-yl)methyl]-5-[(3,3-difluorpyrrolidin-1-yl)carbonyl]-7-(trifluormethyl)-5,6,7,8-tetrahydro[1,2,4]triazolo[4,3-a]pyridin-3(2H)-on (Enantiomer 1)

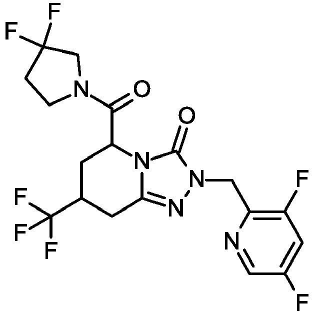

(5RS,7RS)-2-[(3,5-Difluorpyridin-2-yl)methyl]-5-[(3,3-difluorpyrrolidin-1-yl)carbonyl]-7-(trifluormethyl)-5,6,7,8-tetrahydro[1,2,4]triazolo[4,3-a]pyridin-3(2H)-on (Diastereomer 1; Racemat) wurde durch chirale präparative HPLC getrennt [Probenvorbereitung: 59.9 mg gelöst in 4 ml Ethanol/Acetonitril (1:1); Injektionsvolumen: 0.20 ml; Säule: Daicel Chiralcel^{®} IC 5 µm, 250 × 20 mm; Laufmittel: n-Heptan/Ethanol (50:50); Fluss: 15 ml/min; Temperatur 30°C; UV-Detektion: 210 nm]. Nach der Trennung wurden 27.80 mg von Enantiomer 1, welches zuerst eluierte, und 27.2 mg von Enantiomer 2, welches später eluierte, isoliert.

### Enantiomer 1:

Analytische chirale HPLC: Rₜ = 1.483 min, e.e. = 100% [Säule: Daicel Chiralcel^{®} IC-3 50 × 4.6 mm; Laufmittel: n-Heptan/Ethanol 50:50; Fluss: 1 ml/min; UV-Detektion: 220 nm].
LC-MS (Methode 3): Rₜ = 1.50 min; MS (ESIpos): m/z = 468 [M+H]⁺
¹H-NMR (400 MHz, DMSO-d6) δ [ppm]: 0.008 (6.37), 2.179 (2.78), 2.257 (3.92), 2.327 (2.12), 2.366 (2.22), 2.673 (4.20), 2.682 (4.63), 2.710 (3.16), 2.941 (6.80), 2.978 (4.11), 3.541 (2.22), 3.560 (5.90), 3.580 (4.44), 3.707 (2.22), 3.747 (3.40), 3.776 (3.59), 3.809 (1.98), 3.899 (2.31), 3.917 (5.47), 3.936 (5.24), 3.956 (2.03), 4.133 (1.89), 4.177 (2.97), 4.204 (2.97), 4.235 (1.79), 4.924 (4.34), 4.961 (9.82), 5.014 (11.66), 5.034 (3.87), 5.052 (4.20), 7.938 (3.82), 7.944 (4.29), 7.967 (6.18), 7.985 (3.73), 7.991 (4.20), 8.465 (16.00), 8.471 (15.24).

### Beispiel 575

### (5RS,7RS)-2-[(3-Chlor-5-fluorpyridin-2-yl)methyl]-5-[(3,3-difluorpyrrolidin-1-yl)carbonyl]-7-(trifluormethyl)-5,6,7,8-tetrahydro[1,2,4]triazolo[4,3-a]pyridin-3(2H)-on (Diastereomer 1; Racemat)

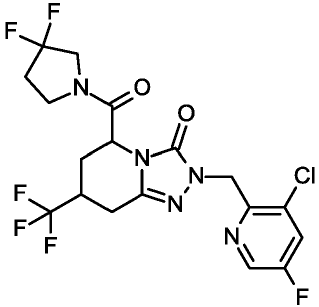

(5RS,7RS)-2-[(3-Chlor-5-fluorpyridin-2-yl)methyl]-3-oxo-7-(trifluormethyl)-2,3,5,6,7,8-hexahydro[1,2,4]triazolo[4,3-a]pyridin-5-carbonsäure (Diastereomerengemisch; 4 Isomere) (100 mg, 253 µmol) wurde in THF (3.0 ml) vorgelegt und HBTU (125 mg, 329 µmol) und N,N-Diisopropylethylamin (130 µl, 760 µmol) wurden anschließend zugegeben. Nach 15 min Rühren bei Raumtemperatur wurde 3,3-Difluorpyrrolidinhydrochlorid (43.6 mg, 304 µmol) zugeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die Produkthaltigen Fraktionen wurden im Vakuum eingeengt und es wurden 98.5 mg (80 % d. Th.) der Titelverbindung erhalten.
LC-MS (Methode 3): Rₜ = 1.60 min; MS (ESIpos): m/z = 484 [M+H]⁺
¹H-NMR (400 MHz, DMSO-d6) δ [ppm]: 2.073 (6.45), 2.189 (2.26), 2.257 (2.86), 2.328 (1.53), 2.425 (2.10), 2.693 (3.86), 2.736 (2.40), 2.950 (5.52), 2.983 (3.83), 3.541 (1.70), 3.560 (4.29), 3.581 (3.93), 3.598 (1.83), 3.710 (1.43), 3.745 (2.73), 3.779 (3.03), 3.807 (1.76), 3.920 (4.22), 3.939 (4.29), 3.958 (1.66), 4.134 (1.40), 4.178 (2.49), 4.206 (2.23), 4.238 (1.56), 4.953 (2.89), 4.977 (4.86), 5.017 (12.87), 5.038 (3.29), 5.056 (13.87), 5.097 (4.56), 8.113 (6.82), 8.119 (7.38), 8.134 (6.69), 8.140 (7.32), 8.540 (16.00), 8.546 (15.67).

### Beispiel 576

### (5RS,7RS)-2-[(3-Chlor-5-fluorpyridin-2-yl)methyl]-5-[(3,3-difluorpyrrolidin-1-yl)carbonyl]-7-(trifluormethyl)-5,6,7,8-tetrahydro[1,2,4]triazolo[4,3-a]pyridin-3(2H)-on (Enantiomer 2)

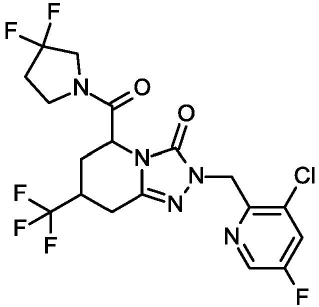

(5RS,7RS)-2-[(3-Chlor-5-fluorpyridin-2-yl)methyl]-5-[(3,3-difluorpyrrolidin-1 -yl)carbonyl]-7-(trifluormethyl)-5,6,7,8-tetrahydro[1,2,4]triazolo[4,3-a]pyridin-3(2H)-on (Diastereomer 1; Racemat) wurde durch chirale präparative HPLC getrennt [Probenvorbereitung: 93.0 mg gelöst in 4 ml Ethanol/Acetonitril (1:1); Injektionsvolumen: 0.20 ml; Säule: Daicel Chiralcel^{®} IC 5 µm, 250 × 20 mm; Laufmittel: n-Heptan/Ethanol (1:1); Fluss: 15 ml/min; Temperatur 30°C; UV-Detektion: 220 nm]. Nach der Trennung wurden 40.9 mg von Enantiomer 1, welches zuerst eluierte, und 38.3 mg von Enantiomer 2, welches später eluierte, isoliert.

### Enantiomer 2:

Analytische chirale HPLC: Rₜ = 1.874 min, e.e. = 100% [Säule: Daicel Chiralcel^{®} IC-3 50 × 4.6 mm; Laufmittel: n-Heptan/Ethanol, 50:50; Fluss: 1 ml/min; UV-Detektion: 220 nm].
LC-MS (Methode 3): Rₜ = 1.60 min; MS (ESIpos): m/z = 484 [M+H]⁺
¹H-NMR (400 MHz, DMSO-d6) δ [ppm]: -0.149 (2.11), -0.008 (16.00), 0.008 (13.37), 0.146 (1.89), 2.266 (3.05), 2.327 (7.16), 2.366 (4.95), 2.669 (6.11), 2.694 (2.63), 2.710 (3.79), 2.950 (4.84), 2.983 (3.37), 3.559 (3.58), 3.578 (3.05), 3.745 (2.42), 3.775 (2.53), 3.919 (3.47), 3.937 (3.58), 4.178 (2.42), 4.204 (2.32), 4.976 (4.32), 5.016 (10.53), 5.056 (12.21), 5.096 (3.79), 8.113 (5.89), 8.119 (6.11), 8.134 (5.47), 8.140 (5.89), 8.540 (13.47), 8.546 (12.42).

### Beispiel 577

### (5S)-5-{[(3S)-3-Fluorpyrrolidin-1-yl]carbonyl}-2-{[5-(trifluormethyl)pyridin-3-yl]methyl}-5,6,7,8-tetrahydro[1,2,4]triazolo[4,3-a]pyridin-3(2H)-on

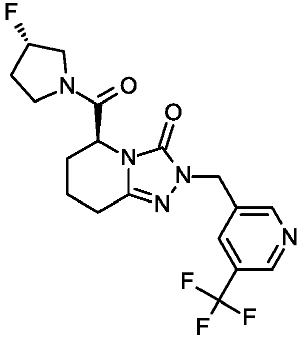

(5S)-3-Oxo-2-{[5-(trifluormethyl)pyridin-3-yl]methyl}-2,3,5,6,7,8-hexahydro[1,2,4]triazolo[4,3-a]pyridin-5-carbonsäure (46.5 mg, 136 µmol) wurde in THF (1.1 ml) vorgelegt und HBTU (67.0 mg, 177 µmol) und N,N-Diisopropylethylamin (95 µl, 680 µmol) wurden anschließend zugegeben. Nach 15 min Rühren bei Raumtemperatur wurde (3S)-3-Fluorpyrrolidinhydrochlorid (20.5 mg, 163 µmol) zugeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die Produkthaltigen Fraktionen wurden im Vakuum eingeengt und es wurden 29.5 mg (53 % d. Th.) der Titelverbindung erhalten.
LC-MS (Methode 3): Rₜ = 1.16 min; MS (ESIpos): m/z = 414 [M+H]⁺
¹H-NMR (400 MHz, DMSO-d6) δ [ppm]: 1.695 (1.26), 1.734 (2.23), 1.888 (0.52), 1.924 (0.76), 1.999 (0.84), 2.034 (1.38), 2.047 (1.33), 2.068 (1.37), 2.088 (1.85), 2.104 (2.27), 2.138 (1.59), 2.221 (0.85), 2.242 (0.78), 2.256 (0.80), 2.270 (1.14), 2.327 (0.56), 2.569 (1.57), 2.577 (1.70), 2.591 (1.48), 2.617 (2.57), 2.660 (1.13), 2.670 (0.97), 3.361 (1.30), 3.371 (1.17), 3.398 (1.01), 3.406 (0.99), 3.460 (0.64), 3.468 (0.71), 3.495 (0.89), 3.504 (0.86), 3.613 (0.59), 3.630 (2.11), 3.637 (2.26), 3.654 (2.41), 3.666 (1.56), 3.681 (1.50), 3.698 (1.11), 3.725 (1.28), 3.746 (1.94), 3.770 (1.64), 3.784 (1.49), 3.855 (2.48), 4.703 (1.17), 4.712 (1.42), 4.719 (1.49), 4.728 (1.18), 4.762 (1.50), 4.770 (1.68), 4.777 (1.93), 4.786 (1.45), 5.017 (16.00), 5.260 (1.25), 5.384 (1.52), 5.391 (1.57), 5.512 (0.89), 8.059 (5.28), 8.742 (5.83), 8.917 (5.45).

### Beispiel 578

### (5S)-5-{[(3R,4S)-3,4-Difluorpyrrolidin-1-yl]carbonyl}-2-{[5-(trifluormethyl)pyridin-3-yl]methyl}-5,6,7,8-tetrahydro[1,2,4]triazolo[4,3-a]pyridin-3(2H)-on

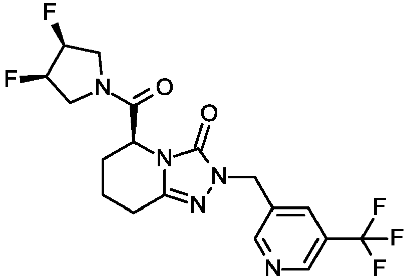

(5S)-3-Oxo-2-{[5-(trifluormethyl)pyridin-3-yl]methyl}-2,3,5,6,7,8-hexahydro[1,2,4]triazolo[4,3-a]pyridin-5-carbonsäure (130 mg, 380 µmol) wurde in THF (3.1 ml) vorgelegt und HBTU (187 mg, 494 µmol) und N,N-Diisopropylethylamin (200 µl, 1.1 mmol) wurden anschließend zugegeben. Nach 15 min Rühren bei Raumtemperatur wurde (3R,4S)-3,4-Difluorpyrrolidinhydrochlorid (60.0 mg, 418 µmol) zugeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die Produkthaltigen Fraktionen wurden im Vakuum eingeengt und es wurden 28.1 mg (17 % d. Th.) der Titelverbindung erhalten.
LC-MS (Methode 4): Rₜ = 0.71 min; MS (ESIpos): m/z = 432 [M+H]⁺
¹H-NMR (400 MHz, DMSO-d6) δ [ppm]: -0.149 (1.89), -0.008 (14.86), 0.008 (16.00), 0.146 (1.81), 1.739 (0.46), 2.025 (0.59), 2.073 (1.52), 2.327 (1.60), 2.332 (1.26), 2.366 (1.09), 2.523 (5.09), 2.575 (1.14), 2.614 (1.22), 2.670 (2.02), 2.674 (1.52), 2.710 (1.26), 3.684 (0.55), 3.729 (0.46), 4.825 (1.09), 5.020 (5.09), 8.061 (1.56), 8.740 (1.73), 8.915 (1.52).

### Beispiel 579

### (5S)-5-[(3,3-Difluorpyrrolidin-1-yl)carbonyl]-2-{[5-(trifluormethyl)pyridin-3-yl]methyl}-5,6,7,8-tetrahydro[1,2,4]triazolo[4,3-a]pyridin-3(2H)-on

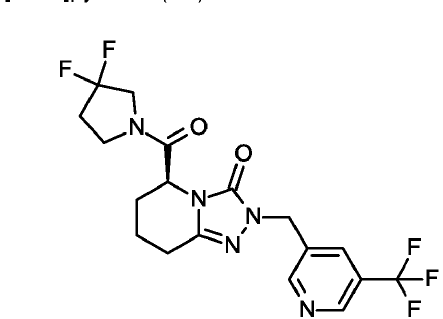

(5S)-3-Oxo-2-{[5-(trifluormethyl)pyridin-3-yl]methyl}-2,3,5,6,7,8-hexahydro[1,2,4]triazolo[4,3-a]pyridin-5-carbonsäure (130 mg, 380 µmol) wurde in THF (3.1 ml) vorgelegt und 1-[Bis(dimethylamino)methylen]-1H-benzotriazol-1-ium-3-oxidhexafluorophosphat (187 mg, 494 µmol) und N,N-Diisopropylethylamin (200 µl, 1.1 mmol) wurden anschließend zugegeben. Nach 15 min Rühren bei Raumtemperatur wurde 3,3-Difluorpyrrolidinhydrochlorid (60.0 mg, 418 µmol) zugeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die Produkthaltigen Fraktionen wurden im Vakuum eingeengt und es wurden 32.6 mg (20 % d. Th.) der Titelverbindung erhalten.
LC-MS (Methode 3): Rₜ = 1.34 min; MS (ESIpos): m/z = 432 [M+H]⁺
¹H-NMR (400 MHz, DMSO-d6) δ [ppm]: -0.149 (1.49), -0.008 (11.08), 0.008 (11.23), 0.146 (1.49), 1.656 (1.12), 1.735 (1.37), 2.009 (1.73), 2.072 (2.13), 2.088 (0.97), 2.327 (2.00), 2.366 (1.46), 2.425 (1.28), 2.571 (3.49), 2.582 (2.49), 2.596 (2.37), 2.615 (2.76), 2.670 (2.31), 2.710 (1.09), 3.534 (1.70), 3.553 (2.55), 3.568 (1.28), 3.670 (1.31), 3.703 (1.49), 3.738 (1.24), 3.769 (1.85), 3.783 (1.12), 3.801 (1.85), 3.827 (0.79), 3.895 (0.64), 3.913 (1.46), 3.939 (1.00), 3.958 (0.76), 3.990 (0.91), 4.032 (0.82), 4.058 (0.52), 4.150 (0.49), 4.184 (0.82), 4.208 (0.88), 4.776 (1.15), 4.792 (1.70), 4.801 (1.15), 4.848 (1.24), 4.863 (1.64), 4.872 (1.18), 5.019 (16.00), 8.060 (5.25), 8.741 (5.22), 8.918 (4.80).

### Beispiel 580

### (5S)-2-[(3-Chlor-5-fluorpyridin-2-yl)methyl]-5-{[(3S)-3-fluorpyrrolidin-1-yl]carbonyl}-5,6,7,8-tetrahydro[1,2,4]triazolo[4,3-a]pyridin-3(2H)-on

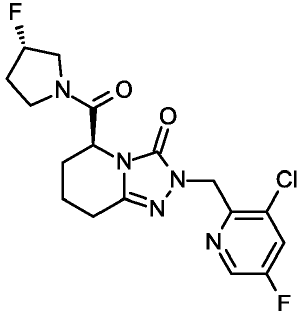

(5S)-2-[(3-Chlor-5-fluorpyridin-2-yl)methyl]-3-oxo-2,3,5,6,7,8-hexahydro[1,2,4]triazolo[4,3-a]pyridin-5-carbonsäure (150 mg, 459 µmol) wurde in THF (3.4 ml) vorgelegt und 1-[Bis(dimethylamino)methylen]-1H-benzotriazol-1-ium-3-oxidhexafluorophosphat (226 mg, 597 µmol) und N,N-Diisopropylethylamin (240 µl, 1.4 mmol) wurden anschließend zugegeben. Nach 15 min Rühren bei Raumtemperatur wurde (3S)-3-Fluorpyrrolidinhydrochlorid (63.4 mg, 505 µmol) zugeben und das Reaktionsgemisch über Nacht bei Raumtemperatur gerührt. Das Lösungsmittel wurde unter Vakuum entfernt und der Rückstand über präparative HPLC aufgereinigt (Chromatorex C18, 10µm, 125mm × 30mm; Laufmittel: Acetonitril/Wasser-Gradient). Die Produkthaltigen Fraktionen wurden im Vakuum eingeengt und es wurden 42.7 mg (23 % d. Th.) der Titelverbindung erhalten.
LC-MS (Methode 3): Rₜ = 1.04 min; MS (ESIpos): m/z = 398 [M+H]⁺
¹H-NMR (400 MHz, DMSO-d6) δ [ppm]: -0.149 (0.48), -0.008 (3.94), 0.008 (3.64), 0.146 (0.42), 1.243 (0.46), 1.258 (0.46), 1.726 (1.88), 1.894 (0.48), 2.016 (0.71), 2.073 (16.00), 2.094 (1.03), 2.264 (0.57), 2.327 (1.25), 2.366 (0.69), 2.578 (1.45), 2.621 (0.53), 2.669 (1.25), 2.710 (0.67), 3.359 (0.42), 3.405 (0.48), 3.501 (0.46), 3.628 (1.23), 3.651 (1.19), 3.680 (0.77), 3.724 (0.91), 3.746 (1.03), 3.769 (0.83), 3.790 (0.67), 3.857 (1.37), 4.680 (0.65), 4.695 (0.79), 4.705 (0.65), 4.736 (0.79), 4.745 (0.89), 4.751 (1.01), 4.761 (0.77), 4.940 (1.21), 4.980 (3.19), 5.016 (1.88), 5.026 (2.16), 5.056 (0.63), 5.065 (0.87), 5.257 (0.65), 5.388 (0.85), 5.509 (0.51), 8.101 (1.66), 8.107 (1.82), 8.122 (1.78), 8.129 (1.84), 8.546 (3.25), 8.553 (3.19).

### Beispiel 581

### (5S)-2-[(3,5-Difluorpyridin-2-yl)methyl]-5-{[(3S)-3-fluorpyrrolidin-1-yl]carbonyl}-5,6,7,8-tetrahydro[1,2,4]triazolo[4,3-a]pyridin-3(2H)-on

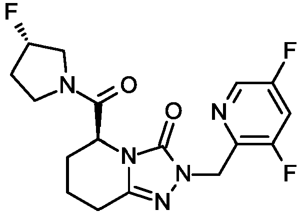

(5S)-2-[(3,5-Difluorpyridin-2-yl)methyl]-3-oxo-2,3,5,6,7,8-hexahydro[1,2,4]triazolo[4,3-a]pyridin-5-carbonsäure (185 mg, 596 µmol) wurde in THF (5.0 ml) bei Raumtemperatur vorgelegt. Anschließend wurde HBTU (294 mg, 775 µmol) und N,N-Diisopropylethylamin (520 µl, 3.0 mmol) zugegeben. Nach 5 min Rühren wurde (S)-(+)-3-Fluorpyrrolidin Hydrochlorid (89.9 mg, 716 µmol) zugeben und das Reaktionsgemisch über das Wochenende bei Raumtemperatur gerührt. Das Reaktionsgemisch wurde mit Wasser und Ethylacetat versetzt. Die organische Phase wurde abgetrennt und organischen Phasen wurden mit gesättigter wässriger Hydrogencarbonat-Lösung, Wasser und gesättigter wässriger Natriumchlorid-Lösung gewaschen, über Natriumsulfat getrocknet, filtriert und das Filtrat eingeengt. Der Rückstand wurde über präparative HPLC aufgereinigt (Chromatorex C18, 10µm, 125mm × 30mm; Laufmittel: (Acetonitril/ mit 0.1% Ameisensäure)-Gradient). Die produkthaltigen Fraktionen wurden im Vakuum eingeengt und es wurden 38.2 mg (17 % d. Th.) der Titelverbindung erhalten.
LC-MS (Methode 3): Rₜ = 0.96 min; MS (ESIpos): m/z = 382 [M+H]⁺
¹H-NMR (400 MHz, DMSO-d6) δ [ppm]: 0.008 (2.60), 1.706 (9.68), 1.715 (10.82), 1.847 (1.79), 1.857 (1.68), 1.871 (1.71), 1.883 (2.85), 1.893 (2.55), 1.968 (2.44), 1.994 (4.47), 2.029 (2.47), 2.050 (4.18), 2.067 (6.02), 2.084 (5.61), 2.103 (5.56), 2.120 (2.96), 2.134 (3.42), 2.187 (0.87), 2.220 (2.22), 2.236 (2.79), 2.266 (3.12), 2.328 (1.19), 2.366 (1.08), 2.463 (1.87), 2.565 (7.46), 2.570 (7.65), 2.611 (2.82), 2.670 (1.19), 2.710 (0.98), 2.882 (1.11), 3.268 (1.90), 3.286 (2.96), 3.342 (2.25), 3.357 (2.12), 3.366 (2.25), 3.393 (2.47), 3.401 (2.58), 3.455 (1.84), 3.464 (1.87), 3.490 (2.55), 3.499 (2.52), 3.610 (1.68), 3.628 (7.08), 3.653 (7.89), 3.661 (6.07), 3.679 (4.53), 3.688 (3.47), 3.720 (4.04), 3.741 (5.88), 3.770 (3.93), 3.778 (3.88), 3.785 (4.18), 3.819 (0.81), 3.855 (7.62), 4.664 (3.72), 4.674 (4.66), 4.680 (4.75), 4.690 (3.69), 4.721 (4.61), 4.730 (5.37), 4.736 (6.07), 4.746 (4.61), 4.887 (6.07), 4.925 (14.56), 4.976 (9.90), 5.015 (4.15), 5.257 (3.66), 5.382 (4.91), 5.388 (5.13), 5.510 (2.74), 7.924 (4.47), 7.930 (4.66), 7.948 (7.65), 7.953 (7.86), 7.972 (4.58), 7.978 (4.69), 8.465 (16.00), 8.471 (15.38).

### Beispiel 582

### (5RS,7RS)-5-{[rel-(3R,4R)-3,4-Difluorpyrrolidin-1-yl]carbonyl}-7-(trifluormethyl)-2-{[6-(trifluormethyl)pyridin-3-yl]methyl}-5,6,7,8-tetrahydro[1,2,4]triazolo[4,3-a]pyridin-3(2H)-on (Diastereomerengemisch; 8 Isomere)

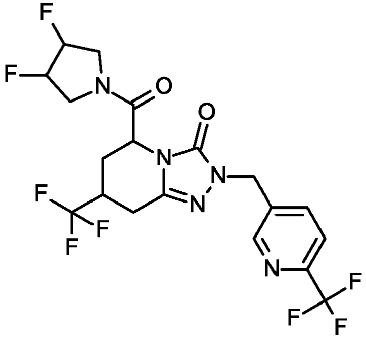

(5RS,7RS)-3-Oxo-7-(trifluormethyl)-2-{[6-(trifluormethyl)pyridin-3-yl]methyl}-2,3,5,6,7,8-hexahydro[1,2,4]triazolo[4,3-a]pyridin-5-carbonsäure (Diastereomerengemisch; 4 Isomere) (259 mg, 92 % Reinheit, 581 µmol) wurde in THF (5.3 ml) bei Raumtemperatur vorgelegt. Anschließend wurde HBTU (286 mg, 755 µmol) und N,N-Diisopropylethylamin (300 µl, 1.7 mmol) zugegeben. Nach 5 min Rühren wurde rel-(3R,4R)-3,4-Difluorpyrrolidinhydrochlorid (100 mg, 697 µmol) zugeben und das Reaktionsgemisch über Nacht bei Raumtemperatur gerührt. Das Reaktionsgemisch wurde mit Wasser und Ethylacetat versetzt. Die organische Phase wurde abgetrennt und organischen Phasen wurden dreimal mit gesättigter wässriger Hydrogencarbonate und 1 N wässrige Salzsäure gewaschen. Die wässrige Phase wurde mit Ethylacetat extrahiert und die vereinigten organischen Phasen über Natriumsulfat getrocknet, filtriert und das Filtrat eingeengt. Der Rückstand wurde über präparative HPLC aufgereinigt (Chromatorex C18, 10µm, 125mm × 30mm; Laufmittel: (Acetonitril/ mit 0.1% Ameisensäure)-Gradient). Die produkthaltigen Fraktionen wurden im Vakuum eingeengt und es wurden 311 mg (97 % d. Th., Diastereomerengemisch, 8 Isomere) Titelverbindung erhalten.
LC-MS (Methode 3): Rₜ = 1.56 und 1.62 min; MS (ESIpos): m/z = 500 [M+H]⁺
¹H-NMR (400 MHz, DMSO-d6) δ [ppm]: -0.008 (1.18), 0.008 (1.10), 1.171 (2.12), 1.194 (15.97), 1.210 (16.00), 2.073 (1.12), 2.200 (0.77), 2.216 (1.54), 2.229 (1.70), 2.254 (1.42), 2.289 (0.48), 2.451 (1.81), 2.489 (6.72), 2.564 (1.85), 2.690 (0.88), 2.701 (0.92), 2.729 (1.49), 2.739 (1.37), 2.769 (1.51), 2.895 (0.95), 2.933 (0.63), 2.975 (1.58), 2.985 (2.01), 3.014 (1.57), 3.023 (1.95), 3.501 (0.87), 3.627 (0.56), 3.661 (0.78), 3.676 (1.06), 3.729 (0.55), 3.754 (2.19), 3.795 (0.53), 3.825 (0.69), 3.863 (0.48), 3.982 (0.54), 4.050 (0.44), 4.094 (1.10), 4.126 (0.41), 4.157 (0.66), 4.192 (0.96), 4.225 (0.54), 4.256 (0.68), 4.289 (0.48), 4.985 (1.04), 5.021 (0.69), 5.052 (8.26), 5.107 (1.17), 5.118 (1.11), 5.322 (0.83), 5.440 (1.32), 5.457 (1.10), 5.549 (0.50), 7.920 (10.95), 7.923 (10.77), 7.945 (0.58), 8.147 (2.92), 8.646 (4.33), 8.678 (0.41).

### Beispiel 583

### (5RS,7RS)-5-{[rel-(3R,4R)-3,4-Difluorpyrrolidin-1-yl]carbonyl}-7-(trifluormethyl)-2-{[6-(trifluormethyl)pyridin-3-yl]methyl}-5,6,7,8-tetrahydro[1,2,4]triazolo[4,3-a]pyridin-3(2H)-on (Isomer 3)

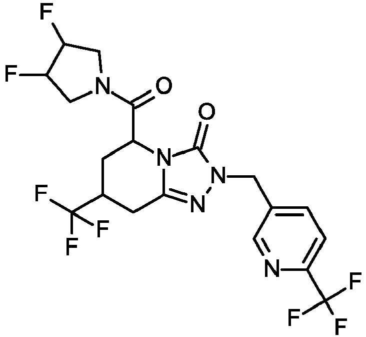

(5RS,7RS)-5-{[rel-(3R,4R)-3,4-Difluorpyrrolidin-1-yl]carbonyl}-7-(trifluormethyl)-2-{[6-(trifluormethyl)pyridin-3-yl]methyl}-5,6,7,8-tetrahydro[1,2,4]triazolo[4,3-a]pyridin-3(2H)-on (Diastereomerengemisch; 8 Isomere) wurde durch chirale präparative HPLC getrennt [Probenvorbereitung: 311 mg gelöst in 2.5 ml Acetonitril und 2.5 ml Ethanol; Injektionsvolumen: 0.1 ml; Säule: Daicel Chiralcel OX-H 5 µm, 250 × 20 mm; Laufmittel: n-Heptan/Ethanol 70:30; Fluss: 40 ml/min; Temperatur 35°C; UV-Detektion: 220 nm]. Nach der Trennung, wurden 4 Hauptdiastereomeren isoliert. (82 mg von Diastereomerengemisch (Isomer 1 und Isomer 2), welches zuerst eluiert, 46.7 mg von Isomer 3, welches als zweites eluiert, und 39.4 mg von Isomer 4, welches zuletzt eluiert)

### Isomer 3:

Analytische chirale HPLC: Rₜ = 2.63 min, d.e. = 99% [Säule: Daicel Chiralpak^{®} IA-3, 50 × 4.6 mm; Laufmittel: i-Hexan/Ethanol 50:50; Fluss: 1 ml/min; UV-Detektion: 220 nm].
LC-MS (Methode 3): Rₜ = 1.62 min; MS (ESIpos): m/z = 500 [M+H]⁺
¹H-NMR (400 MHz, DMSO-d6) δ [ppm]: -0.149 (0.84), -0.008 (6.67), 0.008 (6.52), 0.146 (0.70), 1.100 (0.43), 1.243 (3.42), 1.258 (3.30), 1.273 (2.09), 2.163 (0.64), 2.198 (1.62), 2.214 (1.45), 2.228 (1.77), 2.256 (2.75), 2.291 (1.16), 2.327 (1.57), 2.366 (1.16), 2.669 (1.88), 2.700 (1.71), 2.709 (1.19), 2.729 (2.70), 2.739 (2.26), 2.768 (2.75), 2.911 (1.01), 2.974 (2.41), 3.012 (1.65), 3.023 (1.36), 3.675 (2.52), 3.711 (0.52), 3.752 (4.38), 3.947 (0.90), 3.980 (1.10), 4.050 (0.81), 4.083 (1.10), 4.190 (1.71), 4.223 (1.39), 4.255 (1.80), 4.288 (1.19), 5.051 (15.10), 5.105 (3.13), 5.117 (2.84), 5.323 (1.16), 5.439 (2.29), 5.549 (1.13), 7.919 (16.00), 7.923 (15.77), 8.645 (5.74).

### Beispiel 584

### (5RS,7RS)-5-{[rel-(3R,4R)-3,4-Difluorpyrrolidin-1-yl]carbonyl}-7-(trifluormethyl)-2-{[6-(trifluormethyl)pyridin-3-yl]methyl}-5,6,7,8-tetrahydro[1,2,4]triazolo[4,3-a]pyridin-3(2H)-on (Isomer 4)

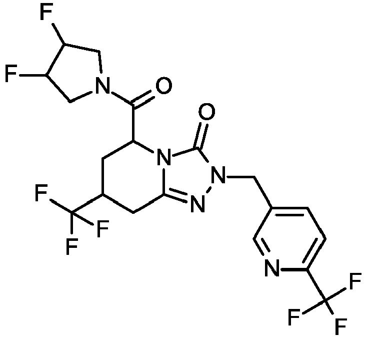

(5RS,7RS)-5-{[rel-(3R,4R)-3,4-Difluorpyrrolidin-1-yl]carbonyl}-7-(trifluormethyl)-2-{[6-(trifluormethyl)pyridin-3-yl]methyl}-5,6,7,8-tetrahydro[1,2,4]triazolo[4,3-a]pyridin-3(2H)-on (Diastereomerengemisch; 8 Isomere) wurde durch chirale präparative HPLC getrennt [Probenvorbereitung: 311 mg gelöst in 2.5 ml Acetonitril und 2.5 ml Ethanol; Injektionsvolumen: 0.1 ml; Säule: Daicel Chiralcel OX-H 5 µm, 250 × 20 mm; Laufmittel: n-Heptan/Ethanol 70:30; Fluss: 40 ml/min; Temperatur 35°C; UV-Detektion: 220 nm]. Nach der Trennung, wurden 4 Hauptdiastereomeren isoliert. (82 mg von Diastereomerengemisch (Isomer 1 und Isomer 2), welches zuerst eluiert, 46.7 mg von Isomer 3, welches als zweites eluiert, und 39.4 mg von Isomer 4, welches zuletzt eluiert)

### Isomer 4:

Analytische chirale HPLC: Rₜ = 3.25 min, d.e. = 96.7% [Säule: Daicel Chiralpak^{®} IA-3, 50 × 4.6 mm; Laufmittel: i-Hexan/Ethanol 50:50; Fluss: 1 ml/min; UV-Detektion: 220 nm].
LC-MS (Methode 3): Rₜ = 1.62 min; MS (ESIpos): m/z = 500 [M+H]⁺
¹H-NMR (400 MHz, DMSO-d6) δ [ppm]: -0.148 (0.80), -0.008 (5.80), 0.008 (5.01), 0.146 (0.89), 1.243 (4.40), 1.259 (4.16), 1.273 (2.95), 2.224 (3.88), 2.327 (1.96), 2.367 (1.68), 2.670 (2.85), 2.710 (1.82), 2.726 (2.15), 2.736 (2.95), 2.767 (1.73), 2.986 (2.95), 3.022 (2.11), 3.626 (1.50), 3.728 (1.22), 3.755 (1.96), 3.791 (1.08), 3.823 (1.96), 3.860 (1.08), 3.993 (1.22), 4.094 (2.71), 4.125 (1.22), 4.155 (1.92), 4.190 (1.03), 4.983 (2.99), 5.045 (7.77), 5.051 (8.05), 5.322 (1.08), 5.456 (1.73), 5.589 (1.12), 7.919 (16.00), 7.923 (15.20), 8.645 (5.80).

### Beispiel 585

### (5S,8SR)-2-[(3,5-Difluorpyridin-2-yl)methyl]-5-{[(3S)-3-fluorpyrrolidin-1-yl]carbonyl}-8-hydroxy-5,6,7,8-tetrahydro[1,2,4]triazolo[4,3-a]pyridin-3(2H)-on (Diastereomerengemisch; 2 Isomere)

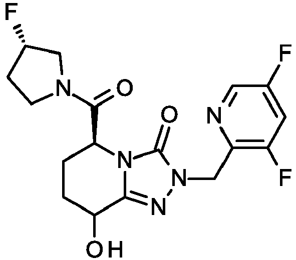

(5S)-2-[(3,5-Difluorpyridin-2-yl)methyl]-5-{[(3S)-3-fluorpyrrolidin-1-yl]carbonyl}-5,6,7,8-tetrahydro[1,2,4]triazolo[4,3-a]pyridin-3(2H)-on (46.0 mg, 121 µmol) und Cer(IV)sulfat (240 mg, 724 µmol) wurden in tert-Butanol (160 µl) bei Raumtemperatur suspendiert. Anschließend wurde 1 N wässrige Schwefelsäure (160 µl, 3.0 mmol) zugegeben und das Reaktionsgemisch über Nacht bei 70°C gerührt. Es wurde erneut Cer(IV)sulfat (240 mg, 724 µmol) und 1 N wässrige Schwefelsäure (160 µl, 3.0 mmol) zugegeben und für weitere über Nacht bei 70°C gerührt. Das Reaktionsgemisch wurde auf Raumtemperatur abgekühlt und mit 1 N wässriger Natriumhydroxid-Lösung auf pH 9 gestellt. Der Feststoff wurde abgesaugt und das Filtrat dreimal mit Dichlormethan extrahiert. Die vereinigten organischen Phasen wurden über Natriumsulfat getrocknet, filtriert und das Filtrat eingeengt. Der Rückstand wurde über präparative HPLC aufgereinigt (Phenomenex, 5µm Kieselgel, 21.2mm × 100mm; Laufmittel: Acetonitril/Wasser-Gradient). Die Produkthaltigen Fraktionen wurden im Vakuum eingeengt und es wurden 4.40 mg (9 % d. Th.) der Titelverbindung erhalten.
LC-MS (Methode 3): Rₜ = 0.76 min; MS (ESIpos): m/z = 398 [M+H]⁺
¹H-NMR (400 MHz, DMSO-d6) δ [ppm]: 1.735 (4.24), 2.100 (3.29), 2.327 (11.14), 2.366 (8.63), 2.669 (9.73), 2.709 (6.27), 3.636 (5.65), 3.751 (4.08), 3.770 (4.08), 3.860 (5.33), 4.485 (7.84), 4.717 (3.29), 4.772 (3.92), 4.931 (4.08), 4.971 (10.04), 5.015 (7.53), 5.056 (3.76), 5.256 (2.82), 5.383 (4.86), 5.743 (10.82), 5.755 (11.29), 7.945 (4.55), 7.964 (7.53), 7.993 (3.92), 8.479 (16.00).

### Beispiel 586

### (5S)-2-[(5-Chlorpyridin-2-yl)methyl]-5-[(3,3-difluorpyrrolidin-1-yl)carbonyl]-8,8-difluor-5,6,7,8-tetrahydro[1,2,4]triazolo[4,3-a]pyridin-3(2H)-on

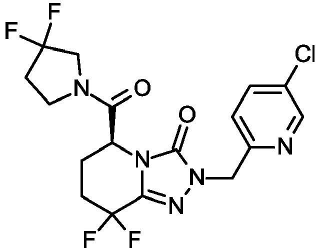

Unter Argon, im Teflonkolben: (5S)-2-[(5-Chlorpyridin-2-yl)methyl]-5-[(3,3-difluorpyrrolidin-1-yl)carbonyl]-6,7-dihydro[1,2,4]triazolo[4,3-a]pyridin-3,8(2H,5H)-dion (79.4 mg, 88 % Reinheit, 170 µmol) wurde in Dichlormethan (3.0 ml) bei Raumtemperatur vorgelegt. Anschließend wurde Diethylaminoschwefeltrifluorid (67 µl, 510 µmol) zugegeben und bei 40°C über Nacht gerührt. Das Reaktionsgemisch wurde mit Wasser und gesättigter wässriger Natriumhydrogencarbonat-Lösung versetzt. Die wässrige Phase wurde viermal mit Dichlormethan extrahiert und die vereinigten organischen Phasen wurden über Natriumsulfat getrocknet, filtriert und das Filtrat eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 53.6 mg (72 % d. Th.) der Titelverbindung erhalten.
LC-MS (Methode 3): Rₜ = 1.51 min; MS (ESIpos): m/z = 434 [M+H]⁺
¹H-NMR (500 MHz, DMSO-d6) δ [ppm]: -0.120 (1.43), -0.007 (16.00), 0.006 (9.20), 0.116 (1.20), 2.292 (2.63), 2.340 (1.37), 2.358 (2.34), 2.361 (2.74), 2.365 (2.23), 2.440 (2.40), 2.518 (1.89), 2.522 (1.49), 2.566 (1.14), 2.580 (0.86), 2.593 (0.63), 2.631 (1.43), 2.635 (1.94), 2.639 (1.31), 3.563 (2.29), 3.578 (3.77), 3.593 (2.17), 3.705 (1.14), 3.732 (1.26), 3.766 (0.69), 3.792 (1.37), 3.818 (1.20), 3.825 (1.03), 3.831 (0.97), 3.846 (1.66), 3.861 (0.69), 3.893 (0.69), 3.908 (1.54), 3.923 (0.86), 3.929 (0.97), 3.944 (0.40), 4.027 (0.40), 4.050 (0.86), 4.061 (0.46), 4.072 (0.57), 4.085 (0.69), 4.107 (0.46), 4.159 (0.51), 4.183 (0.69), 4.203 (0.74), 4.961 (1.71), 5.036 (2.40), 5.047 (1.37), 5.068 (6.91), 5.078 (10.06), 5.110 (1.49), 7.284 (5.09), 7.301 (5.37), 7.953 (5.37), 7.958 (5.26), 7.970 (4.97), 7.975 (5.03), 8.590 (6.57), 8.594 (6.34).

### Beispiel 587

### (5S)-2-[(5-Chlorpyridin-2-yl)methyl]-8,8-difluor-5-[(3-fluorazetidin-1-yl)carbonyl]-5,6,7,8-tetrahydro[1,2,4]triazolo[4,3-a]pyridin-3(2H)-on

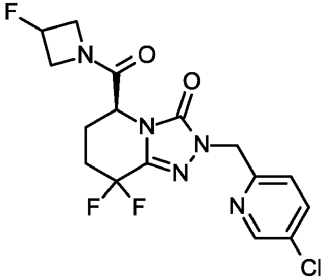

Unter Argon: (5S)-2-[(5-Chlorpyridin-2-yl)methyl]-5-[(3-fluorazetidin-1-yl)carbonyl]-6,7-dihydro[1,2,4]triazolo[4,3-a]pyridin-3,8(2H,5H)-dion (37.1 mg, 63 % Reinheit, 61.5 µmol) wurde in Dichlormethan (1.7 ml) bei Raumtemperatur vorgelegt. Anschließend wurde Diethylaminoschwefeltrifluorid (39 µl, 290 µmol) zugegeben und bei 40°C über Nacht gerührt. Das Reaktionsgemisch wurde mit Wasser und gesättigter wässriger Natriumhydrogencarbonat-Lösung versetzt. Die wässrige Phase wurde zweimal mit Dichlormethan extrahiert und die vereinigten organischen Phasen wurden über Natriumsulfat getrocknet, filtriert und das Filtrat eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die produkthaltigen Fraktionen wurden im Vakuum eingeengt und es wurden 12.4 mg (48 % d. Th.) der Titelverbindung erhalten.
LC-MS (Methode 3): Rₜ = 1.33 min; MS (ESIpos): m/z = 402 [M+H]⁺
¹H-NMR (500 MHz, DMSO-d6) δ [ppm]: -0.120 (1.40), -0.007 (15.46), 0.006 (8.74), 0.117 (1.13), 1.058 (0.72), 1.067 (0.90), 1.072 (1.35), 1.087 (0.77), 1.097 (0.81), 1.104 (0.77), 1.112 (1.26), 1.119 (1.35), 1.126 (1.13), 1.133 (0.95), 1.165 (0.68), 1.179 (0.86), 1.190 (0.86), 1.273 (1.62), 1.345 (0.41), 2.224 (2.07), 2.237 (2.07), 2.303 (3.38), 2.312 (3.29), 2.358 (3.06), 2.361 (3.65), 2.365 (2.66), 2.405 (1.31), 2.428 (2.57), 2.518 (1.94), 2.522 (1.22), 2.631 (1.76), 2.635 (2.52), 2.639 (1.67), 3.313 (10.05), 3.955 (1.58), 3.978 (1.80), 4.003 (1.53), 4.026 (1.58), 4.201 (0.81), 4.216 (0.86), 4.227 (0.77), 4.248 (1.53), 4.260 (1.53), 4.272 (1.40), 4.283 (1.31), 4.302 (0.90), 4.315 (0.77), 4.328 (0.68), 4.360 (0.95), 4.381 (1.76), 4.403 (1.76), 4.430 (1.85), 4.451 (1.04), 4.574 (0.77), 4.589 (0.90), 4.618 (0.81), 4.642 (1.31), 4.654 (1.26), 4.682 (0.90), 4.698 (0.86), 4.705 (0.86), 4.727 (3.43), 4.735 (6.58), 5.040 (1.31), 5.071 (16.00), 5.080 (7.53), 5.112 (0.86), 5.373 (1.13), 5.429 (1.13), 5.487 (1.13), 5.537 (0.99), 5.543 (1.08), 7.292 (7.12), 7.309 (7.44), 7.953 (4.91), 7.956 (4.96), 7.970 (4.64), 7.973 (4.60), 8.288 (15.95), 8.583 (5.09), 8.589 (7.35), 8.595 (4.60).

### Beispiel 588

### (5S)-2-[(3,5-Difluorpyridin-2-yl)methyl]-5-[(3,3-difluorpyrrolidin-1-yl)carbonyl]-8,8-difluor-5,6,7,8-tetrahydro[1,2,4]triazolo[4,3-a]pyridin-3(2H)-on

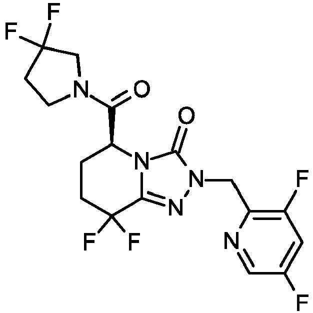

Unter Argon: (5S)-2-[(3,5-Difluorpyridin-2-yl)methyl]-5-[(3,3-difluorpyrrolidin-1-yl)carbonyl]-6,7-dihydro[1,2,4]triazolo[4,3-a]pyridin-3,8(2H,5H)-dion (67.3 mg, 163 µmol) wurde in Dichlormethan (7.0 ml) bei Raumtemperatur vorgelegt. Anschließend wurde Diethylaminoschwefeltrifluorid (86 µl, 650 µmol) zugegeben und bei 40°C über Nacht gerührt. Es wurden erneut Diethylaminoschwefeltrifluorid (86 µl, 650 µmol) zugegeben und über Nacht bei Raumtemperatur gerührt. Das Reaktionsgemisch wurde mit Dichloromethan verdünnen, mit gesättigter wässriger Natriumhydrogencarbonat-Lösung und gesättigter wässriger Natriumchlorid-Lösung gewaschen. Die organische Phase wurde über Natriumsulfat getrocknet, filtriert und das Filtrat eingeengt. Der Rückstand wurde über präparative HPLC aufgereinigt (Chromatorex C18, 10µm, 125mm × 30mm; Laufmittel: Acetonitril/Wasser-Gradient). Die produkthaltigen Fraktionen wurden im Vakuum eingeengt und es in Ethylacetat aufgelöst. Die organische Phase wurde mit 1 N wässrige Salzsäure und Wasser gewaschen, über Natriumsulfat getrocknet, filtriert und das Filtrat eingeengt. Es wurden 13.6 mg (19 % d. Th.) der Titelverbindung erhalten.
LC-MS (Methode 3): Rₜ = 1.42 min; MS (ESIpos): m/z = 436 [M+H]⁺
¹H-NMR (400 MHz, DMSO-d6) δ [ppm]: -0.150 (2.27), 0.008 (12.80), 1.348 (3.47), 1.504 (5.73), 1.908 (2.27), 2.271 (6.53), 2.327 (7.60), 2.366 (6.13), 2.670 (4.40), 2.710 (4.93), 3.553 (5.33), 3.573 (8.67), 3.591 (4.53), 3.694 (3.07), 3.729 (3.47), 3.783 (4.00), 3.814 (4.27), 3.900 (4.00), 4.035 (2.00), 4.197 (2.00), 4.950 (4.40), 5.028 (3.73), 5.076 (2.27), 5.117 (12.40), 5.133 (12.27), 5.169 (2.67), 7.969 (4.67), 7.975 (4.80), 7.999 (6.40), 8.015 (4.40), 8.484 (16.00), 8.490 (15.07).

### Beispiel 589

### (5S)-2-{[3-Chlor-5-(trifluormethyl)pyridin-2-yl]methyl}-8,8-difluor-5-{[(3S)-3-fluorpyrrolidin-1-yl]carbonyl}-5,6,7,8-tetrahydro[1,2,4]triazolo[4,3-a]pyridin-3(2H)-on

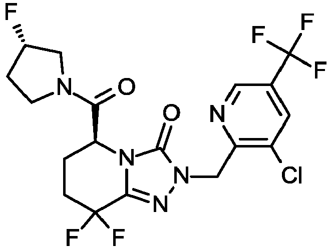

Unter Argon, im Teflonkolben: (5S)-2-{[3-Chlor-5-(trifluormethyl)pyridin-2-yl]methyl}-5-{[(3S)-3-fluorpyrrolidin-1-yl]carbonyl}-6,7-dihydro[1,2,4]triazolo[4,3-a]pyridin-3,8(2H,5H)-dion (75.0 mg, 162 µmol) wurde in Dichlormethan (10 ml) bei Raumtemperatur vorgelegt. Anschließend wurde Diethylaminoschwefeltrifluorid (110 µl, 810 µmol) zugegeben und bei 40°C über Nacht gerührt. Das Reaktionsgemisch wurde mit gesättigter wässriger Natriumhydrogencarbonat-Lösung versetzt. Die wässrige Phase wurde mit Dichlormethan extrahiert und die Organische Phase über Natriumsulfat getrocknet, filtriert und das Filtrat eingeengt. Der Rückstand wurde über präparative HPLC aufgereinigt (Chromatorex C18, 10µm), 125mm × 30mm; Laufmittel: (Acetonitril/Wasser mit 0.1% Ameisensäure)-Gradient). Die produkthaltigen Fraktionen wurden im Vakuum eingeengt und es wurden 55.5 mg (71 % d. Th.) der Titelverbindung erhalten.
LC-MS (Methode 4): Rₜ = 0.88 min; MS (ESIpos): m/z = 484 [M+H]⁺
¹H-NMR (400 MHz, DMSO-d6) δ [ppm]: -0.149 (0.66), -0.008 (4.35), 0.008 (3.90), 0.147 (0.51), 1.168 (0.96), 1.185 (1.97), 1.203 (0.91), 1.292 (0.86), 1.310 (1.87), 1.328 (0.81), 2.012 (0.46), 2.117 (1.57), 2.150 (1.92), 2.251 (1.57), 2.282 (2.38), 2.332 (4.56), 2.346 (3.09), 2.366 (3.80), 2.407 (1.37), 2.447 (1.82), 2.524 (4.86), 2.670 (1.77), 2.710 (1.77), 3.349 (1.62), 3.394 (1.11), 3.421 (1.11), 3.483 (0.91), 3.519 (1.11), 3.614 (0.76), 3.639 (3.44), 3.667 (3.49), 3.696 (2.28), 3.732 (1.06), 3.755 (1.52), 3.777 (2.43), 3.799 (2.13), 3.823 (1.47), 3.887 (3.04), 3.900 (1.06), 3.910 (0.96), 3.918 (1.06), 4.925 (2.13), 4.984 (2.63), 5.274 (1.82), 5.299 (16.00), 5.348 (0.76), 5.406 (2.03), 5.527 (1.16), 8.531 (7.49), 8.916 (7.59).

### Beispiel 590

### (5S)-2-{[3-Chlor-5-(trifluormethyl)pyridin-2-yl]methyl}-5-[(3,3-difluorpyrrolidin-1-yl)carbonyl]-8,8-difluor-5,6,7,8-tetrahydro[1,2,4]triazolo[4,3-a]pyridin-3(2H)-on

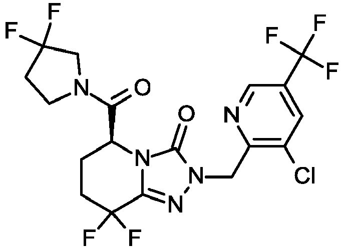

Unter Argon, im Teflonkolben: (5S)-2-{[3-Chlor-5-(trifluormethyl)pyridin-2-yl]methyl}-5-[(3,3-difluorpyrrolidin-1-yl)carbonyl]-6,7-dihydro[1,2,4]triazolo[4,3-a]pyridin-3,8(2H,5H)-dion (65.0 mg, 48 % Reinheit, 65.0 µmol) wurde in Dichlormethan (2.0 ml) bei Raumtemperatur vorgelegt. Anschließend wurde Diethylaminoschwefeltrifluorid (26 µl, 200 µmol) zugegeben und bei 40°C über Nacht gerührt. Es wurde erneut Diethylaminoschwefeltrifluorid (26 µl, 200 µmol) zugegeben und bei 40°C über Nacht gerührt. Es wurde erneut Diethylaminoschwefeltrifluorid (86 µl, 650 µmol) zugegeben und bei 40°C über Nacht gerührt. Das Reaktionsgemisch wurde mit gesättigter wässriger Natriumhydrogencarbonat-Lösung versetzt. Die wässrige Phase wurde mit Dichlormethan extrahiert und die organische Phase wurde mit Wasser und gesättigter wässriger Natriumchlorid-Lösung gewaschen, über Natriumsulfat getrocknet, filtriert und das Filtrat eingeengt. Der Rückstand wurde über präparative HPLC aufgereinigt (Methode 14). Die produkthaltigen Fraktionen wurden im Vakuum eingeengt und es wurden 4.00 mg (12 % d. Th.) der Titelverbindung erhalten.
LC-MS (Methode 3): Rₜ = 1.76 min; MS (ESIpos): m/z = 502 [M+H]⁺
¹H-NMR (400 MHz, DMSO-d6) δ [ppm]: 0.008 (16.00), 0.146 (3.40), 2.327 (13.00), 2.366 (8.00), 2.669 (12.60), 2.710 (6.60), 3.576 (4.00), 5.305 (13.80), 8.531 (6.80), 8.915 (5.60).

### Beispiel 591

### (5S,8RS)-2-[(6-Chlorpyridin-3-yl)methyl]-5-[(3,3-difluorpyrrolidin-1-yl)carbonyl]-8-hydroxy-5,6,7,8-tetrahydro[1,2,4]triazolo[4,3-a]pyridin-3(2H)-on (Diastereomerengemisch; 2 Isomere)

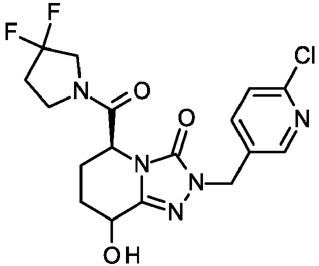

(5S)-2-[(6-Chlorpyridin-3-yl)methyl]-5-[(3,3-difluorpyrrolidin-1 -yl)carbonyl]-5,6,7,8-tetrahydro[1,2,4]triazolo[4,3-a]pyridin-3(2H)-on (100 mg, 244 µmol) und Cer(IV)sulfat (486 mg, 1.46 mmol) wurden in tert-Butanol (330 µl) bei Raumtemperatur suspendiert. Anschließend wurde 1 N wässriger Schwefelsäure (330 µl, 6.1 mmol) zugegeben und das Reaktionsgemisch über Nacht bei 70°C gerührt. Das Reaktionsgemisch wurde auf Raumtemperatur abgekühlt und mit 2 N wässriger Natriumhydroxid-Lösung auf pH 9 gestellt. Die Suspension wurde filtriert und das Filtrat dreimal mit Dichlormethan extrahiert. Die vereinigten organischen Phasen wurden über Natriumsulfat getrocknet, filtriert und das Filtrat eingeengt. Es wurden 66.9 mg (90 % Reinheit, 60 % d. Th.) der Titelverbindung erhalten.
LC-MS (Methode 3): Rₜ = 0.98 min; MS (ESIpos): m/z = 414 [M+H]⁺

### Beispiel 592

### (5S,8RS)-2-[(5-Chlor-3-fluorpyridin-2-yl)methyl]-5-{[(3S)-3-fluorpyrrolidin-1-yl]carbonyl}-8-hydroxy-5,6,7,8-tetrahydro[1,2,4]triazolo[4,3-a]pyridin-3(2H)-on (Diastereomerengemisch; 2 Isomere)

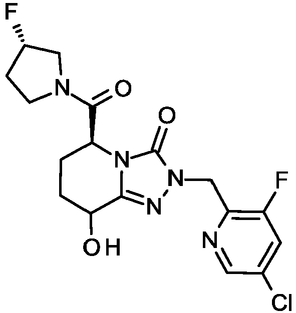

(5S)-2-[(5-Chlor-3-fluorpyridin-2-yl)methyl]-5-{[(3S)-3-fluorpyrrolidin-1-yl]carbonyl}-5,6,7,8-tetrahydro[1,2,4]triazolo[4,3-a]pyridin-3(2H)-on (95.7 mg, 229 µmol) und Cer(IV)sulfat (456 mg, 1.37 mmol) wurden in tert-Butanol (310 µl) bei Raumtemperatur suspendiert. Anschließend wurde 1 N wässriger Schwefelsäure (310 µl, 5.7 mmol) zugegeben und das Reaktionsgemisch über Nacht bei 70°C gerührt. Das Reaktionsgemisch wurde auf Raumtemperatur abgekühlt und mit 2 N wässriger Natriumhydroxid-Lösung auf pH 9 gestellt. Die Suspension wurde filtriert und das Filtrat dreimal mit Dichlormethan extrahiert. Die vereinigten organischen Phasen wurden über Natriumsulfat getrocknet, filtriert und das Filtrat eingeengt. Es wurden 51.7 mg (86 % Reinheit, 47 % d. Th.) der Titelverbindung erhalten.
LC-MS (Methode 1): Rₜ = 0.74 min; MS (ESIpos): m/z = 414 [M+H]⁺

### Beispiel 593

### (5S,8RS)-2-{[3-Chlor-5-(trifluormethyl)pyridin-2-yl]methyl}-5-{[(3S)-3-fluorpyrrolidin-1-yl]carbonyl}-8-hydroxy-5,6,7,8-tetrahydro[1,2,4]triazolo[4,3-a]pyridin-3(2H)-on (Diastereomerengemisch; 2 Isomere)

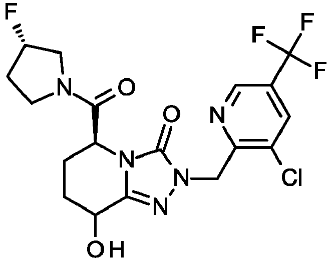

(5S)-2-{[3-Chlor-5-(trifluormethyl)pyridin-2-yl]methyl}-5-{[(3S)-3-fluorpyrrolidin-1-yl]carbonyl}-5,6,7,8-tetrahydro[1,2,4]triazolo[4,3-a]pyridin-3(2H)-on (685 mg, 1.53 mmol) und Cer(IV)sulfat (3.05 g, 9.18 mmol) wurden in tert-Butanol (2.0 ml) bei Raumtemperatur suspendiert. Anschließend wurde 1 N wässrige Schwefelsäure (2.0 ml, 38 mmol) zugegeben und das Reaktionsgemisch über Nacht bei 70°C gerührt. Das Reaktionsgemisch wurde auf Raumtemperatur abgekühlt und mit 2 N wässriger Natriumhydroxid-Lösung auf pH 9 gestellt. Der Feststoff wurde abgesaugt und das Filtrat dreimal mit Dichlormethan extrahiert. Die vereinigten organischen Phasen wurden über Natriumsulfat getrocknet, filtriert und das Filtrat eingeengt. Der Rückstand wurde über präparative HPLC aufgereinigt (Chromatorex C18, 10µm, 125mm × 30mm; Laufmittel: (Acetonitril/Wasser mit 0.1% Ameisensäure)-Gradient). Die Produkthaltigen Fraktionen wurden im Vakuum eingeengt und es wurden 93.8 mg (13 % d. Th.) der Titelverbindung erhalten.
LC-MS (Methode 4): Rₜ = 0.64 min; MS (ESIpos): m/z = 464 [M+H]⁺
¹H-NMR (400 MHz, DMSO-d6) δ [ppm]: -0.149 (1.95), -0.008 (11.35), 1.747 (4.47), 1.844 (3.91), 2.136 (3.53), 2.268 (3.44), 2.328 (4.00), 2.365 (5.86), 2.669 (3.26), 2.710 (3.81), 3.485 (2.05), 3.613 (4.09), 3.639 (5.30), 3.655 (5.02), 3.755 (4.00), 3.865 (4.84), 4.502 (7.53), 4.512 (7.26), 4.727 (3.35), 4.783 (4.00), 4.794 (3.72), 5.117 (3.44), 5.156 (15.35), 5.171 (8.47), 5.180 (10.33), 5.221 (3.07), 5.256 (3.35), 5.381 (4.56), 5.513 (2.51), 5.748 (10.05), 5.760 (10.05), 6.606 (1.49), 8.501 (16.00), 8.915 (14.88).

### Beispiel 594

### (5S,8RS)-2-{[3-Chlor-5-(trifluormethyl)pyridin-2-yl]methyl}-5-[(3,3-difluorpyrrolidin-1 - yl)carbonyl]-8-hydroxy-5,6,7,8-tetrahydro[1,2,4]triazolo[4,3-a]pyridin-3(2H)-on (Diastereomerengemisch; 2 Isomere)

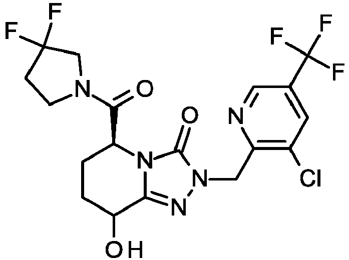

(5S)-2-{[3-Chlor-5-(trifluormethyl)pyridin-2-yl]methyl}-5-[(3,3-difluorpyrrolidin-1-yl)carbonyl]-5,6,7,8-tetrahydro[1,2,4]triazolo[4,3-a]pyridin-3(2H)-on (630 mg, 95 % Reinheit, 1.28 mmol) und Cer(IV)sulfat (2.56 g, 7.71 mmol) wurden in tert-Butanol (1.7 ml) bei Raumtemperatur suspendiert. Anschließend wurde 1 N wässrige Schwefelsäure (1.7 ml, 32 mmol) zugegeben und das Reaktionsgemisch für 20 Stunden bei 70°C gerührt. Es wurde erneut 1 N wässrige Schwefelsäure (1.7 ml, 32 mmol) zugegeben und für weitere über Nacht bei 70°C gerührt. Das Reaktionsgemisch wurde auf Raumtemperatur abgekühlt und mit 2 N wässriger Natriumhydroxid-Lösung auf pH 9 gestellt. Der Feststoff wurde abgesaugt und das Filtrat dreimal mit Dichlormethan extrahiert. Die vereinigten organischen Phasen wurden über Natriumsulfat getrocknet, filtriert und das Filtrat eingeengt. Der Rückstand wurde über präparative HPLC aufgereinigt (Chromatorex C18, 10µm, 125mm × 40mm; Laufmittel: (Acetonitril/Wasser mit 0.1% Ameisensäure)-Gradient). Die Produkthaltigen Fraktionen wurden im Vakuum eingeengt und es wurden 58.7 mg (9 % d. Th.) der Titelverbindung erhalten.
LC-MS (Methode 3): Rₜ = 1.28 min; MS (ESIpos): m/z = 482 [M+H]⁺
¹H-NMR (400 MHz, DMSO-d6) δ [ppm]: -0.149 (1.45), -0.008 (10.12), 0.008 (9.85), 1.424 (2.44), 1.445 (1.90), 1.742 (3.98), 1.815 (3.07), 1.898 (2.80), 2.132 (2.26), 2.327 (4.43), 2.366 (5.06), 2.405 (3.25), 2.669 (2.89), 2.709 (3.34), 3.521 (4.16), 3.540 (7.32), 3.560 (4.34), 3.658 (2.44), 3.692 (2.53), 3.725 (2.71), 3.758 (2.89), 3.791 (4.07), 3.819 (3.07), 3.916 (3.07), 3.996 (1.81), 4.188 (2.17), 4.212 (1.90), 4.501 (6.51), 4.797 (3.07), 4.807 (2.98), 4.884 (2.98), 5.123 (2.26), 5.163 (15.82), 5.176 (16.00), 5.217 (2.62), 5.763 (11.30), 5.775 (11.39), 8.505 (14.73), 8.914 (13.02).

### Beispiel 595

### (5S,8RS)-2-[(5-Chlorpyridin-2-yl)methyl]-5-[(3,3-difluorpyrrolidin-1-yl)carbonyl]-8-hydroxy-5,6,7,8-tetrahydro[1,2,4]triazolo[4,3-a]pyridin-3(2H)-on (Diastereomerengemisch; 2 Isomere)

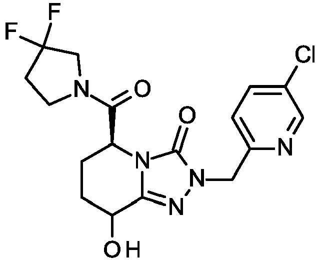

(5S)-2-[(5-Chlorpyridin-2-yl)methyl]-5-[(3,3-difluorpyrrolidin-1 -yl)carbonyl]-5,6,7,8-tetrahydro[1,2,4]triazolo[4,3-a]pyridin-3(2H)-on (174 mg, 74 % Reinheit, 324 µmol) und Cer(IV)sulfat (645 mg, 1.94 mmol) wurden in tert-Butanol (430 µl) bei Raumtemperatur suspendiert. Anschließend wurde 1 N wässrige Schwefelsäure (430 µl, 8.1 mmol) zugegeben und das Reaktionsgemisch über Nacht bei 70°C gerührt. Das Reaktionsgemisch wurde auf Raumtemperatur abgekühlt und mit 2 N wässriger Natriumhydroxid-Lösung auf pH 9 gestellt. Der Feststoff wurde abgesaugt und das Filtrat dreimal mit Dichlormethan extrahiert. Die vereinigten organischen Phasen wurden mit Wasser und gesättigter wässriger Natriumchlorid-Lösung gewaschen, über Natriumsulfat getrocknet, filtriert und das Filtrat eingeengt. Es wurden 103 mg (85 % Reinheit, 65 % d. Th.) der Titelverbindung erhalten.
LC-MS (Methode 1): Rₜ = 0.82 min; MS (ESIpos): m/z = 414 [M+H]⁺

### Beispiel 596

### (5S,8RS)-2-[(5-Chlorpyridin-2-yl)methyl]-5-[(3-fluorazetidin-1-yl)carbonyl]-8-hydroxy-5,6,7,8-tetrahydro[1,2,4]triazolo[4,3-a]pyridin-3(2H)-on (Diastereomerengemisch; 2 Isomere)

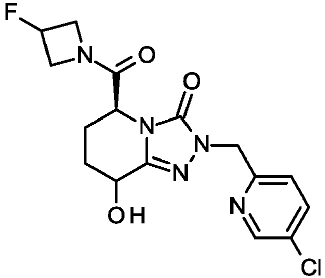

(5S)-2-[(5-Chlorpyridin-2-yl)methyl]-5-[(3-fluorazetidin-1-yl)carbonyl]-5,6,7,8-tetrahydro[1,2,4]triazolo[4,3-a]pyridin-3(2H)-on (166 mg, 98 % Reinheit, 445 µmol) und Cer(IV)sulfat (887 mg, 2.67 mmol) wurden in tert-Butanol (590 µl) bei Raumtemperatur suspendiert. Anschließend wurde 1 N wässrige Schwefelsäure (590 µl, 11 mmol) zugegeben und das Reaktionsgemisch über Nacht bei 70°C gerührt. Das Reaktionsgemisch wurde auf Raumtemperatur abgekühlt und mit 2 N wässriger Natriumhydroxid-Lösung auf pH 9 gestellt. Der Feststoff wurde abgesaugt und das Filtrat dreimal mit Dichlormethan extrahiert. Die vereinigten organischen Phasen wurden mit Wasser und gesättigter wässriger Natriumchlorid-Lösung gewaschen, über Natriumsulfat getrocknet, filtriert und das Filtrat eingeengt. Es wurden 48.1 mg (84 % Reinheit, 24 % d. Th.) der Titelverbindung erhalten.
LC-MS (Methode 3): Rₜ = 0.83 min; MS (ESIpos): m/z = 382 [M+H]⁺

### Beispiel 597

### (5S,8RS)-2-[(3,5-Difluorpyridin-2-yl)methyl]-5-[(3,3-difluorpyrrolidin-1-yl)carbonyl]-8-hydroxy-5,6,7,8-tetrahydro[1,2,4]triazolo[4,3-a]pyridin-3(2H)-on (Diastereomerengemisch; 2 Isomere)

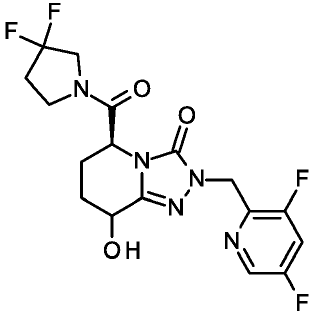

(5S)-2-[(3,5-Difluorpyridin-2-yl)methyl]-5-[(3,3-difluorpyrrolidin-1 -yl)carbonyl]-5,6,7,8-tetrahydro[1,2,4]triazolo[4,3-a]pyridin-3(2H)-on (226 mg, 567 µmol) und Cer(IV)sulfat (1.13 g, 3.40 mmol) wurden in tert-Butanol (770 µl) bei Raumtemperatur suspendiert. Anschließend wurde 1 N wässrige Schwefelsäure (770 µl, 770 µmol) zugegeben und das Reaktionsgemisch über Nacht bei 70°C gerührt. Das Reaktionsgemisch wurde auf Raumtemperatur abgekühlt und mit 2 N wässriger Natriumhydroxid-Lösung auf pH 9 gestellt. Die wässrige Phase wurde dreimal mit Ethylacetat extrahiert und die vereinigten organischen Phasen über Natriumsulfat getrocknet, filtriert und das Filtrat eingeengt. Es wurden 116 mg (49 % d. Th.) der Titelverbindung erhalten.
LC-MS (Methode 3): Rₜ = 0.91 min; MS (ESIpos): m/z = 416 [M+H]⁺
¹H-NMR (400 MHz, DMSO-d6) δ [ppm]: 1.097 (3.87), 1.110 (1.22), 1.135 (4.05), 1.157 (1.27), 1.175 (2.34), 1.193 (1.34), 1.227 (16.00), 1.736 (6.08), 1.768 (0.64), 1.797 (0.65), 1.845 (0.62), 1.858 (0.55), 1.880 (1.03), 1.892 (0.81), 1.939 (0.47), 1.959 (0.47), 1.988 (4.06), 2.328 (0.69), 2.359 (0.53), 2.378 (0.68), 2.407 (0.68), 2.425 (0.68), 2.448 (0.57), 2.570 (0.72), 2.589 (0.50), 3.518 (0.78), 3.538 (1.26), 3.559 (0.77), 3.653 (0.55), 3.687 (0.65), 3.722 (0.49), 3.758 (0.71), 3.790 (0.95), 3.812 (0.72), 3.914 (0.65), 3.940 (0.50), 3.991 (0.42), 4.002 (0.53), 4.021 (1.25), 4.038 (1.17), 4.056 (0.42), 4.186 (0.45), 4.388 (1.59), 4.486 (1.30), 4.495 (1.21), 4.776 (0.62), 4.786 (0.63), 4.853 (0.63), 4.863 (0.64), 4.940 (0.71), 4.978 (2.02), 5.018 (2.18), 5.056 (0.84), 5.280 (0.84), 5.294 (0.72), 5.757 (2.51), 5.769 (2.47), 5.780 (0.44), 5.794 (0.41), 7.940 (0.81), 7.945 (0.83), 7.964 (1.33), 7.968 (1.37), 7.987 (0.86), 7.993 (0.84), 8.446 (0.74), 8.452 (0.75), 8.477 (3.04), 8.483 (2.64).

### B. Bewertung der pharmakologischen Wirksamkeit

Die pharmakologische Aktivität der erfindungsgemäßen Verbindungen kann durch in vitro- und in vivo-Untersuchungen, wie sie dem Fachmann bekannt sind, nachgewiesen werden. Die nachfolgenden Anwendungsbeispiele beschreiben die biologische Wirkung der erfindungsgemäßen Verbindungen, ohne die Erfindung auf diese Beispiele zu beschränken.

### Abkürzungen und Akronyme:

- ATP: Adenosintriphosphat
- COPD: Chronisch-Obstruktive Lungenerkrankung
- LPS: Lipopolysaccharid
- PGP: Prolin-Glycin-Prolin
- Poly(I:C): Polyinosin-Polycytidylsäure

### B-1 Biochemischer humaner Prolyl Endopeptidase (PREP) Assay zur Identifizierung von Inhibitoren der PREP-Aktivität unter Benutzung eines fluoreszent markierten Substrats

### Prinzip des Assays:

Der enzymatische Umsatz des fluoreszenten Peptidsubstrats wurde anhand der Messung der Fluoreszenzintensität beobachtet. Die Enzymaktivität wurde durch die Ermittlung der Anfangssteigung in der Fluoreszenzzunahme bestimmt. Verbindungen, die PREP inhibieren, wurden anhand der Verringerung der Anfangssteigung im Vergleich zu einem Reaktionsansatz ohne Testverbindung identifiziert.

### Aktivitätsbestimmung:

IC₅₀-Werte wurden durch die Auftragung der prozentualen PREP-Aktivität gegen die Konzentration der Testsubstanz durch Interpolation ermittelt.

### Assaybeschreibung:

Zu rekombinanter volle-Länge humaner Prolyl Endopeptidase (PREP, R&D-Systems, 4308-SE; Endkonzentration z.B. 0.4 nM, Volumen: 25 µl) in Reaktionspuffer (50 mM Tris-HCl, pH 7.5; 150 mM NaCl; 0.13 % BSA, 5 mM EDTA, 3 mM GSH, 0.005% Brij-35) wurde eine Testverbindung (in DMSO, in einem passenden Endkonzentrationsbereich von 1 nM bis 30 µM, Volumen: 1 µl) in einer Vertiefung einer 384-well-Mikrotiterplatte zugegeben. Die Reaktion wurde durch Zugabe des Substrats Z-Gly-Pro-AMC (Endkonzentration 50 µl; Z = Carboxybenzyl; AMC = 7-Amino-4-methylcoumarin, Volumen: 25 µl) gestartet. Der Fortschritt der PREP Reaktion wurde durch Messung der Fluoreszenzintensität in einem Tecan SAFIRE II Plattenspektrophotometer über einen Zeitraum von 60 min bei 32°C beobachtet (Anregungswellenlänge: 360 nm, Emissionswellenlänge: 465 nm).

In der folgenden Tabelle B-1 sind die so erhaltenen IC₅₀-Werte aus dem humanem Prolylendopeptidase Assay für individuelle Ausführungsbeispiele der Erfindung sowie einiger Referenzbeispiele, die nicht unter die Ansprüche fallen, zusammengestellt (zum Teil als Mittelwerte aus mehreren unabhängigen Einzelbestimmungen):

**Tabelle B-1**

| **Beispiel Nr.** | **Prolylendopeptidase (human) IC₅₀ [mol/l]** |
|---|---|
| 1 | 1.75E-09 |
| 2 | 1.54E-08 |
| 3 | 6.00E-09 |
| 4 | 2.20E-09 |
| 5 | 8.00E-09 |
| 6 | 1.20E-07 |
| 7 | 8.00E-09 |
| 8 | 9.90E-10 |
| 9 | 3.90E-08 |
| 10 | 2.40E-08 |
| 11 | 7.00E-10 |
| 12 | 9.95E-08 |
| 13 | 5.40E-09 |
| 14 | 3.00E-10 |
| 15 | 8.40E-09 |
| 16 | 2.20E-09 |
| 17 | 1.20E-09 |
| 18 | 5.75E-10 |
| 19 | 5.70E-10 |
| 20 | 1.50E-09 |
| 21 | 6.90E-09 |
| 22 | 4.20E-08 |
| 23 | 4.95E-09 |
| 24 | 4.90E-09 |
| 25 | 7.60E-10 |
| 26 | 7.30E-10 |
| 27 | 1.28E-09 |
| 28 | 2.40E-08 |
| 29 | 1.20E-09 |
| 30 | 1.60E-09 |
| 31 | 2.90E-09 |
| 32 | 1.48E-09 |
| 33 | 7.70E-09 |
| 34 | 7.50E-09 |
| 35 | 1.50E-08 |
| 36 | 3.65E-09 |
| 37 | 8.30E-10 |
| 38 | 1.00E-09 |
| 39 | 5.50E-10 |
| 40 | 5.20E-09 |
| 41 | 1.30E-08 |
| 42 | 1.45E-09 |
| 43 | 1.90E-09 |
| 44 | 2.10E-09 |
| 45 | 1.60E-09 |
| 46 | 9.50E-10 |
| 47 | 1.60E-09 |
| 48 | 5.00E-09 |
| 49 | 1.75E-09 |
| 50 | 1.20E-08 |
| 51 | 8.90E-10 |
| 52 | 4.60E-09 |
| 53 | 7.10E-10 |
| 54 | 1.30E-09 |
| 55 | 1.40E-09 |
| 56 | 5.60E-09 |
| 57 | 4.10E-09 |
| 58 | 4.00E-09 |
| 59 | 1.22E-08 |
| 60 | 1.85E-08 |
| 61 | 1.60E-08 |
| 62 | 2.00E-09 |
| 63 | 7.40E-10 |
| 64 | 9.40E-10 |
| 65 | 1.85E-09 |
| 66 | 3.80E-10 |
| 67 | 8.70E-09 |
| 68 | 3.90E-09 |
| 69 | 2.10E-07 |
| 70 | 1.10E-08 |
| 71 | 7.10E-10 |
| 72 | 8.30E-10 |
| 73 | 1.50E-09 |
| 74 | 4.75E-09 |
| 75 | 1.10E-09 |
| 76 | 3.80E-10 |
| 77 | 4.80E-09 |
| 78 | 6.00E-09 |
| 79 | 6.70E-09 |
| 80 | 1.60E-08 |
| 81 | 8.40E-10 |
| 82 | 1.75E-09 |
| 83 | 2.10E-09 |
| 84 | 5.70E-09 |
| 85 | 2.10E-09 |
| 86 | 1.00E-09 |
| 87 | 1.17E-09 |
| 88 | 2.70E-10 |
| 89 | 1.30E-09 |
| 90 | 9.80E-09 |
| 91 | 2.30E-09 |
| 92 | 3.00E-09 |
| 93 | 6.00E-09 |
| 94 | 5.70E-08 |
| 95 | 9.00E-09 |
| 96 | 7.40E-09 |
| 97 | 6.50E-09 |
| 98 | 2.57E-07 |
| 99 | 1.27E-08 |
| 100 | 7.90E-09 |
| 101 | 3.05E-09 |
| 102 | 2.50E-10 |
| 103 | 1.60E-10 |
| 104 | 4.60E-10 |
| 105 | 1.05E-08 |
| 106 | 1.70E-09 |
| 107 | 2.10E-09 |
| 108 | 1.50E-10 |
| 109 | 2.10E-10 |
| 110 | 3.20E-10 |
| 111 | 4.10E-10 |
| 112 | 1.59E-09 |
| 113 | 4.20E-10 |
| 114 | 1.95E-09 |
| 115 | 1.10E-09 |
| 116 | 3.00E-09 |
| 117 | 3.15E-09 |
| 118 | 5.60E-09 |
| 119 | 1.40E-09 |
| 120 | 9.60E-09 |
| 121 | 4.30E-09 |
| 122 | 1.02E-08 |
| 123 | 1.20E-08 |
| 124 | 1.40E-08 |
| 125 | 2.50E-08 |
| 126 | 2.84E-08 |
| 127 | 4.35E-08 |
| 128 | 4.60E-08 |
| 129 | 1.00E-08 |
| 130 | 5.05E-08 |
| 131 | 1.07E-07 |
| 132 | 1.41E-07 |
| 133 | 1.45E-07 |
| 134 | 7.90E-08 |
| 135 | 3.45E-08 |
| 136 | 1.17E-09 |
| 137 | 1.30E-07 |
| 138 | 1.00E-09 |
| 139 | 8.70E-10 |
| 140 | 1.60E-08 |
| 141 | 8.30E-10 |
| 142 | 2.55E-09 |
| 143 | 1.31E-08 |
| 144 | 1.70E-09 |
| 145 | 1.27E-08 |
| 146 | 9.30E-10 |
| 147 | 7.00E-08 |
| 148 | 2.13E-08 |
| 149 | 2.50E-09 |
| 150 | 5.80E-10 |
| 151 | 2.60E-10 |
| 152 | 3.80E-08 |
| 153 | 3.60E-08 |
| 154 | 7.90E-07 |
| 155 | 2.10E-07 |
| 156 | 6.90E-08 |
| 157 | 1.30E-09 |
| 158 | 8.90E-09 |
| 159 | 1.60E-08 |
| 160 | 3.20E-08 |
| 161 | 3.70E-08 |
| 162 | 1.30E-07 |
| 163 | 2.80E-09 |
| 164 | 3.70E-09 |
| 165 | 8.90E-10 |
| 166 | 2.20E-08 |
| 167 | 5.65E-08 |
| 168 | 4.10E-09 |
| 169 | 3.15E-08 |
| 170 | 9.00E-08 |
| 171 | 6.30E-09 |
| 172 | 6.30E-09 |
| 173 | 5.85E-09 |
| 174 | 1.15E-09 |
| 175 | 7.00E-09 |
| 176 | 4.45E-07 |
| 177 | 6.90E-09 |
| 178 | 1.30E-09 |
| 179 | 6.00E-07 |
| 180 | 3.20E-09 |
| 181 | 2.48E-08 |
| 182 | 3.75E-08 |
| 183 | 1.09E-07 |
| 184 | 4.50E-09 |
| 185 | 3.70E-10 |
| 186 | 1.25E-09 |
| 187 | 1.02E-07 |
| 188 | 9.10E-11 |
| 189 | 3.40E-08 |
| 190 | 1.30E-10 |
| 191 | 2.80E-09 |
| 192 | 3.20E-10 |
| 193 | 4.20E-10 |
| 194 | 3.75E-10 |
| 195 | 6.90E-10 |
| 196 | 4.00E-10 |
| 197 | 2.35E-07 |
| 198 | 1.03E-09 |
| 199 | 5.90E-08 |
| 200 | 4.55E-07 |
| 201 | 5.45E-09 |
| 202 | 1.90E-08 |
| 203 | 8.20E-10 |
| 204 | 3.20E-07 |
| 205 | 1.00E-08 |
| 206 | 1.20E-09 |
| 207 | 4.20E-09 |
| 208 | 1.50E-08 |
| 209 | 5.50E-09 |
| 210 | 2.70E-10 |
| 211 | 1.50E-10 |
| 212 | 4.10E-11 |
| 213 | 7.70E-10 |
| 214 | 2.00E-10 |
| 215 | 5.30E-10 |
| 216 | 5.60E-10 |
| 217 | 1.20E-9 |
| 218 | 3.30E-9 |
| 219 | 7.30E-9 |
| 220 | 1.50E-09 |
| 221 | 9.80E-07 |
| 222 | 1.10E-09 |
| 223 | 4.15E-10 |
| 224 | 5.60E-10 |
| 225 | 2.30E-10 |
| 226 | 3.60E-09 |
| 227 | 2.40E-08 |
| 228 | 2.20E-09 |
| 229 | 3.40E-10 |
| 230 | 2.30E-09 |
| 231 | 4.80E-08 |
| 232 | 4.00E-07 |
| 233 | 2.03E-07 |
| 234 | 1.20E-07 |
| 235 | 6.80E-09 |
| 236 | 1.40E-09 |
| 237 | 9.40E-10 |
| 238 | 7.30E-08 |
| 239 | 1.12E-08 |
| 240 | 4.20E-09 |
| 241 | 5.80E-09 |
| 242 | 2.00E-09 |
| 243 | 6.23E-10 |
| 244 | 1.10E-08 |
| 245 | 6.90E-09 |
| 246 | 1.30E-09 |
| 247 | 9.10E-07 |
| 248 | 1.20E-08 |
| 249 | 8.30E-10 |
| 250 | 9.20E-10 |
| 251 | 3.40E-09 |
| 252 | 3.10E-09 |
| 253 | 1.20E-07 |
| 254 | 3.05E-08 |
| 255 | 1.51E-08 |
| 256 | 1.70E-09 |
| 257 | 5.95E-10 |
| 258 | 9.15E-08 |
| 259 | 1.70E-08 |
| 260 | 2.60E-09 |
| 261 | 1.00E-09 |
| 262 | 7.90E-07 |
| 263 | 2.80E-09 |
| 264 | 2.55E-09 |
| 265 | 1.30E-09 |
| 266 | 5.10E-09 |
| 267 | 2.30E-09 |
| 268 | 2.30E-08 |
| 269 | 4.20E-09 |
| 270 | 2.00E-09 |
| 271 | 1.10E-09 |
| 272 | 6.95E-10 |
| 273 | 7.60E-09 |
| 274 | 5.90E-09 |
| 275 | 1.15E-09 |
| 276 | 3.60E-09 |
| 278 | 3.30E-09 |
| 279 | 4.85E-10 |
| 280 | 3.80E-09 |
| 281 | 2.20E-09 |
| 282 | 1.50E-08 |
| 283 | 6.20E-09 |
| 284 | 3.00E-09 |
| 285 | 1.30E-09 |
| 286 | 9.85E-08 |
| 287 | 2.30E-08 |
| 288 | 1.60E-08 |
| 289 | 1.17E-07 |
| 290 | 8.20E-08 |
| 291 | 9.65E-08 |
| 292 | 3.70E-08 |
| 293 | 8.50E-08 |
| 294 | 2.00E-08 |
| 295 | 1.20E-08 |
| 296 | 1.80E-7 |
| 297 | 1.15E-07 |
| 298 | 2.40E-08 |
| 299 | 3.50E-09 |
| 300 | 1.90E-8 |
| 301 | 1.95E-08 |
| 302 | 4.95E-08 |
| 303 | 3.50E-08 |
| 304 | 3.50E-08 |
| 305 | 1.40E-08 |
| 306 | 2.15E-07 |
| 307 | 9.85E-08 |
| 308 | 1.25E-07 |
| 309 | 6.40E-08 |
| 310 | 1.50E-08 |
| 311 | 3.00E-08 |
| 312 | 8.90E-09 |
| 313 | 4.00E-09 |
| 314 | 1.40E-09 |
| 315 | 1.40E-09 |
| 316 | 2.60E-09 |
| 317 | 4.10E-10 |
| 318 | 5.18E-07 |
| 319 | 6.30E-10 |
| 320 | 6.90E-10 |
| 321 | 9.30E-10 |
| 322 | 9.40E-10 |
| 323 | 9.60E-10 |
| 324 | 1.20E-09 |
| 325 | 1.50E-09 |
| 326 | 1.50E-09 |
| 327 | 1.90E-09 |
| 328 | 4.10E-09 |
| 329 | 4.30E-09 |
| 330 | 4.90E-09 |
| 331 | 6.80E-10 |
| 332 | 3.90E-09 |
| 333 | 2.10E-09 |
| 334 | 2.95E-09 |
| 335 | 1.05E-09 |
| 336 | 6.60E-10 |
| 337 | 7.50E-09 |
| 338 | 1.50E-09 |
| 339 | 4.90E-10 |
| 340 | 4.80E-10 |
| 341 | 3.70E-09 |
| 342 | 1.75E-09 |
| 343 | 7.90E-09 |
| 344 | 5.30E-10 |
| 345 | 6.40E-09 |
| 346 | 4.20E-09 |
| 347 | 4.80E-09 |
| 348 | 3.80E-09 |
| 349 | 1.45E-09 |
| 350 | 3.50E-10 |
| 351 | 1.60E-09 |
| 352 | 3.20E-10 |
| 353 | 2.30E-09 |
| 354 | 1.90E-08 |
| 355 | 1.30E-09 |
| 356 | 3.00E-10 |
| 357 | 4.30E-10 |
| 358 | 6.80E-11 |
| 359 | 1.10E-09 |
| 360 | 8.10E-10 |
| 361 | 1.20E-09 |
| 362 | 1.35E-09 |
| 363 | 2.30E-09 |
| 364 | 2.90E-08 |
| 365 | 2.30E-09 |
| 366 | 2.10E-09 |
| 367 | 4.25E-10 |
| 368 | 9.00E-09 |
| 369 | 1.30E-08 |
| 370 | 1.00E-09 |
| 371 | 1.20E-09 |
| 372 | 1.30E-09 |
| 373 | 1.35E-07 |
| 374 | 5.15E-08 |
| 375 | 2.60E-09 |
| 376 | 4.10E-10 |
| 377 | 6.86E-09 |
| 378 | 2.60E-09 |
| 379 | 2.50E-09 |
| 380 | 2.10E-09 |
| 381 | 5.50E-09 |
| 382 | 5.90E-09 |
| 383 | 8.55E-09 |
| 384 | 1.10E-08 |
| 385 | 1.80E-09 |
| 386 | 9.40E-10 |
| 387 | 3.40E-09 |
| 388 | 5.75E-10 |
| 389 | 1.45E-09 |
| 390 | 1.35E-08 |
| 391 | 8.65E-09 |
| 392 | 4.60E-10 |
| 393 | 1.40E-09 |
| 394 | 2.80E-08 |
| 395 | 1.60E-09 |
| 396 | 6.00E-09 |
| 397 | 6.90E-09 |
| 398 | 1.19E-09 |
| 399 | 1.55E-09 |
| 400 | 4.55E-10 |
| 401 | 3.40E-09 |
| 402 | 2.70E-09 |
| 403 | 1.89E-07 |
| 404 | 2.33E-09 |
| 405 | 2.25E-10 |
| 406 | 3.60E-09 |
| 407 | 1.35E-09 |
| 408 | 4.60E-10 |
| 409 | 1.40E-08 |
| 410 | 7.70E-09 |
| 411 | 2.50E-09 |
| 412 | 3.00E-09 |
| 413 | 1.90E-09 |
| 414 | 7.90E-10 |
| 415 | 5.40E-09 |
| 416 | 3.40E-09 |
| 417 | 1.80E-08 |
| 418 | 2.24E-09 |
| 419 | 8.80E-10 |
| 420 | 1.70E-09 |
| 421 | 9.10E-10 |
| 422 | 6.30E-10 |
| 423 | 1.85E-09 |
| 424 | 3.80E-10 |
| 425 | 3.50E-08 |
| 426 | 6.93E-09 |
| 427 | 1.10E-09 |
| 428 | 5.30E-08 |
| 429 | 3.70E-09 |
| 430 | 1.10E-09 |
| 431 | 9.95E-10 |
| 432 | 1.30E-10 |
| 433 | 3.10E-09 |
| 434 | 2.40E-09 |
| 435 | 2.65E-09 |
| 436 | 4.70E-09 |
| 437 | 7.10E-09 |
| 438 | 1.68E-09 |
| 439 | 2.20E-09 |
| 440 | 7.15E-10 |
| 441 | 2.00E-09 |
| 442 | 1.00E-09 |
| 443 | 6.10E-10 |
| 444 | 1.90E-10 |
| 445 | 7.60E-10 |
| 446 | 7.30E-10 |
| 447 | 1.40E-09 |
| 448 | 6.15E-10 |
| 449 | 5.55E-09 |
| 450 | 1.10E-09 |
| 451 | 1.10E-09 |
| 452 | 1.30E-09 |
| 453 | 3.75E-10 |
| 454 | 6.30E-11 |
| 455 | 7.70E-10 |
| 456 | 2.70E-10 |
| 457 | 1.32E-09 |
| 458 | 5.10E-10 |
| 459 | 2.00E-10 |
| 460 | 1.70E-10 |
| 461 | 6.25E-10 |
| 462 | 2.85E-10 |
| 463 | 2.25E-10 |
| 464 | 5.10E-10 |
| 465 | 2.10E-09 |
| 466 | 4.10E-10 |
| 467 | 4.90E-10 |
| 468 | 1.40E-09 |
| 469 | 7.95E-10 |
| 470 | 8.20E-10 |
| 471 | 4.25E-10 |
| 472 | 2.45E-10 |
| 473 | 1.70E-10 |
| 474 | 3.60E-10 |
| 475 | 3.30E-10 |
| 476 | 7.80E-10 |
| 477 | 2.10E-10 |
| 478 | 2.65E-09 |
| 479 | 2.50E-08 |
| 480 | 5.20E-10 |
| 481 | 2.75E-08 |
| 482 | 2.80E-09 |
| 483 | 2.10E-09 |
| 484 | 2.95E-10 |
| 485 | 4.30E-09 |
| 486 | 4.65E-10 |
| 487 | 7.25E-10 |
| 488 | 1.50E-10 |
| 489 | 1.10E-10 |
| 490 | 3.80E-10 |
| 491 | 4.40E-10 |
| 492 | 5.20E-10 |
| 493 | 3.20E-10 |
| 494 | 9.10E-10 |
| 495 | 1.65E-08 |
| 496 | 1.80E-08 |
| 497 | 1.80E-10 |
| 498 | 4.40E-10 |
| 499 | 1.10E-07 |
| 500 | 5.15E-09 |
| 501 | 2.55E-08 |
| 502 | 4.10E-09 |
| 503 | 2.00E-09 |
| 504 | 1.80E-09 |
| 505 | 1.40E-09 |
| 506 | 1.20E-09 |
| 507 | 9.40E-10 |
| 508 | 9.10E-10 |
| 509 | 7.00E-10 |
| 510 | 5.35E-10 |
| 511 | 3.65E-10 |
| 512 | 1.50E-10 |
| 513 | 1.30E-10 |
| 514 | 6.10E-11 |
| 515 | 5.90E-10 |
| 516 | 4.20E-09 |
| 517 | 9.50E-10 |
| 518 | 1.50E-08 |
| 519 | 1.40E-09 |
| 520 | 1.70E-09 |
| 521 | 4.40E-10 |
| 522 | 1.50E-09 |
| 523 | 2.85E-08 |
| 524 | 2.90E-09 |
| 525 | 1.52E-08 |
| 526 | 3.80E-10 |
| 527 | 1.20E-09 |
| 528 | 7.20E-10 |
| 529 | 9.60E-10 |
| 530 | 2.20E-10 |
| 531 | 3.00E-09 |
| 532 | 3.40E-08 |
| 533 | 4.40E-10 |
| 534 | 1.23E-09 |
| 535 | 5.10E-09 |
| 536 | 9.60E-09 |
| 537 | 8.80E-09 |
| 538 | 8.20E-10 |
| 539 | 8.50E-10 |
| 540 | 2.70E-08 |
| 541 | 6.30E-08 |
| 542 | 9.40E-08 |
| 543 | 3.35E-08 |
| 544 | 4.40E-09 |
| 545 | 6.70E-10 |
| 546 | 5.70E-10 |
| 547 | 4.30E-10 |
| 548 | 3.10E-8 |
| 549 | 3.60E-8 |
| 550 | 1.80E-8 |
| 551 | 4.80E-7 |
| 552 | 6.40E-9 |
| 553 | 5.75E-7 |
| 554 | 1.00E-8 |
| 555 | 5.40E-10 |
| 556 | 6.30E-10 |
| 557 | 3.20E-9 |
| 558 | 1.90E-9 |
| 559 | 3.50E-8 |
| 560 | 4.30E-10 |
| 561 | 3.70E-10 |
| 562 | 1.33E-9 |
| 563 | 3.30E-10 |
| 564 | 4.40E-9 |
| 565 | 1.50E-9 |
| 566 | 4.10E-9 |
| 567 | 2.70E-9 |
| 568 | 6.20E-10 |
| 569 | 8.50E-10 |
| 570 | 5.70E-10 |
| 571 | 1.10E-9 |
| 572 | 1.40E-9 |
| 573 | 5.90E-9 |
| 574 | 5.20E-9 |
| 575 | 7.40E-9 |
| 576 | 8.70E-9 |
| 577 | 5.90E-10 |
| 578 | 2.30E-9 |
| 579 | 1.10E-9 |
| 580 | 4.30E-10 |
| 581 | 3.00E-10 |
| 582 | 1.40E-7 |
| 583 | 9.70E-7 |
| 584 | 5.00E-8 |
| 585 | 4.30E-10 |
| 586 | 1.40E-8 |
| 587 | 6.40E-9 |
| 588 | 1.80E-8 |
| 589 | 3.50E-10 |
| 590 | 3.90E-9 |

### B-2 Biochemischer muriner Prolyl Endopeptidase (PREP) Assay

### Aktivitätsbestimmung:

IC₅₀-Werte wurden durch die Auftragung der prozentualen PREP-Aktivität gegen die Konzentration der Testsubstanz durch Interpolation ermittelt.

### Mausgehirnhomogenat Präparation:

Maushirn aus BalbC Mäusen in 0.8 ml eines Gemisches aus 100mM Natrium-Phosphat (pH 7.0) sowie 3 mM Dithiothreitol wurde im OmniBead Ruptor 4x25sec homogenisiert. Das erhaltene Homogenat wurde 20 min bei 13000 Upm und 4°C zentrifugiert. Der aliquotierte Überstand wurde bei -80°C eingefroren.

### Assaybeschreibung:

Mausgehirnhomogenat wurde 1:100 verdünnt in einem Reaktionspuffer (50 mM Tris-HCl, pH 7,5; 150 mM NaCl; 0,13 % BSA, 5 mM EDTA, 3 mM GSH, 0,005% Brij-35) und 25 µl von der Lösung wurden in eine Vertiefung einer 384-well-Mikrotiterplatte gegeben. Eine Testverbindung (in DMSO, in einem passenden Endkonzentrationsbereich von z.B. 1 nM bis 30 µM, Volumen: 1 µl) wurde zugegeben. Die Reaktion wird durch Zugabe des Substrats Z-Gly-Pro-AMC (Endkonzentration 50 µM; Z = Carboxybenzyl; AMC = 7-Amino-4-methylcoumarin, Volumen: 25 µl) gestartet. Der Fortschritt der PREP Reaktion wurde durch Messung der Fluoreszenzintensität in einem geeigneten Plattenspektrometer über einen Zeitraum von z.B. 60 min bei 32°C beobachtet (Anregungswellenlänge: 360 nm, Emissionswellenlänge: 465 nm).

In der folgenden Tabelle 1 sind die so erhaltenen IC₅₀-Werte aus dem KDR- und dem PDGFRβ-Kinase-Assay für individuelle Ausführungsbeispiele der Erfindung zusammengestellt (zum Teil als Mittelwerte aus mehreren unabhängigen Einzelbestimmungen):

### B-3 Wirksamkeit von PREP-Inhibitoren in Zigarettenrauch-exponierten Mäusen

Die Eignung der erfindungsgemäßen Substanzen für die Behandlung/Prophylaxe der genannten Krankheiten kann in folgendem Tiermodell gezeigt werden.

### Material und Methoden:

**Mäuse:** Stamm: BALB/C, Herkunft: Charles River Niederlande, Geschlecht: männlich, Alter: 8 bis 10 Wochen, Gewicht: 25 g, n = 21.
**Vehikel:** Solutol, EtOH, Water (S: 40 %, EtOH 10 %, Water 50 % (v/v))
Für die Verabreichung von PREP-Inhibitoren per Schlundsonde wurden diese in dem oben beschriebenen Vehikel gelöst (c = 3750 µg/ml).

Die Mäuse wurden in drei Gruppen zu je sieben Tieren eingeteilt und folgendermaßen behandelt:
Gruppe 1: keine Rauchexposition (Raumluft), zweimal täglich 200 µl Vehikel ohne Wirkstoff
Gruppe 2: Rauchexposition, zweimal täglich 200 µl Vehikel ohne Wirkstoff
Gruppe 3: Rauchexposition, zweimal täglich 200 µl Vehikel mit Wirkstoff (jeweils 30 mg/kg)

### Rauchexposition:

Zur Exposition mit Zigarettenrauch wurden die Tiere der Gruppen 2 und 3 (die Tiere der Gruppe 1 erfuhren keine Rauchexposition und verblieben in den Haltungskäfigen) zweimal täglich in eine Expositionskammer mit einem Gesamtvolumen von 52 Litern eingebracht, die durch Trenngitter in 16 Einzelkäfige eingeteilt war. Dabei wurden die Tiere, die gruppenweise gehalten wurden (7 Tiere pro Käfig), zur Berauchung in jeweils einen Einzelkäfig der Expositionskammer eingebracht (7 Tiere pro Käfig). Zwischen den zweimal täglichen Expositionen wurde eine rauchfreie Zeit von 5 Stunden gelegt. Insgesamt wurden die Tiere an fünf aufeinanderfolgenden Tagen beraucht. Zigaretten wurden jeweils paarweise verbrannt. Der Hauptstromrauch der Zigaretten wurde in die Expositionskammer eingeleitet.

Die Rauchexposition wurde nach folgenden Schema durchgeführt:
- Tag 1: 1. Berauchung: 2 mal 2 Zigaretten, 2. Berauchung: 3 mal 2 Zigaretten
- Tag 2: 1. Berauchung: 4mal 2 Zigaretten, 2. Berauchung: 5 mal 2 Zigaretten
- Tag 3: 1. Berauchung: 6 mal 2 Zigaretten, 2. Berauchung: 7 mal 2 Zigaretten
- Tage 4 und 5: 1. und 2. Berauchung: jeweils 7 mal 2 Zigaretten

Die Behandlung der Tiere mit PREP-Inhibitoren bzw. Vehikel wurde 15 Minuten vor der ersten Berauchung am Tag 1 begonnen. Im Folgenden erhielten die Tiere zweimal täglich im Abstand von 8 Stunden PREP-Inhibitoren bzw. Vehikel in der oben beschriebenen Dosierung.

Am Tag 6 wurden die Tiere durch die intraperitoneale Gabe von 150 mg/kg Pentobarbital (Euthesate^{®}) getötet. Die Tracheen wurden freipräpariert und zur Einführung von Kanülen eingeschnitten. Über diese Kanülen wurden die Lungen viermal mit 37 °C warmer, physiologischer Kochsalzlösung gespült. Die Zellen dieser jeweils vier Fraktionen der Broncho-Alveolären Lavage-Flüssigkeit (BALF) wurden bei 4 °C bei 400 xg für 5 Minuten zentrifugiert. Im Anschluss wurden die Zellpellets vereinigt und in jeweils 150 µl physiologischer Kochlösung (4°C) resuspendiert. Im Anschluss an die Färbung mit Türk'scher Lösung wurden die Gesamtzahl der Zellen lichtmikroskopisch gezählt. Zur Quantifizierung der neutrophilen Granulozyten (kurz: Neutrophile) wurden die Zellen auf Objektträger transferiert (Cytospin) und mit DiffQuick (Dade A.G., Düdingen, Switzerland) gefärbt. Abschließend wurden Neutrophile, Makrophagen und Lymphozyten gezählt und die relativen Anteile an der zuvor bestimmten Gesamtzahl berechnet. Zur Bestimmung der PGP-Konzentration in der BALF wurden jeweils 200 µl des Überstandes der ersten BALF-Fraktion mit Bestatin versetzt (Endkonzentration: 1 mM) und bis zur Messung bei -20 °C gelagert.

### Quantifizierung von PGP in der Broncho-Alveolären Lavage-Flüssigkeit (BALF)

PGP wurde nach der Methode von Hardison et al. [Hardison et al., J. Immunol. 2009, 182:4423-4431] unter Verwendung folgender Ausrüstung bestimmt: ESI-LC-MS/MS (Shimadzu HPLC, Columbia, MA, USA) mit einem Finnigan TSQ quantum discovery Max Quarupole Massenspektrometer in Verbindung mit Elektrospray-Wärmeionisation (Thermo Fisher Scientific, San Jose, CA, USA) und einer Atlantis dC18 Säule (100 mm × 2.1 mm, dp = 3 µm, Waters Chromatography, Milford, MA, USA) bzw. einer Atlantis pre-column Vorsäule (10 mm × 2.1 mm, dp = 3 µm, Waters).

### B-4 Reinigung, Kristallalisation, und Einkristallstrukturbestimmung von PREP (Schwein) im Komplex mit Beispielen 26, 108, 113, 157, 237, 358 und 454

Abkürzungen:
GST = Glutathion-S-Transferasen
IPTG = Isopropyl-β-D-thiogalactopyranosid
OD600 = Optical Density at 600 nM
TRIS-HCl = Tris(hydroxymethyl)-aminomethan-hydrochlorid
EDTA = Ethylendiamintetraessigsäure
DTT = Dithiothreitol
PAGE = Polyacrylamidgelelektrophorese
TEV = Tobacco Etch Virus
SEC = Size Exclusion Chromatography (Größenausschlussverfahren)
FPLC = Fast protein liquid chromatography
NaCl = Natriumchlorid
PEG = Polyethylenglycol
MES = 2-(N-Morpholino)ethansulfonsäure
DMSO = Dimethylsulfoxid
CCD = Charge Coupled Device

### 4.1 Expression und Reinigung von PREP

### Expressionsystem:

### Aufbau eines E. coli Expressionsvektor zur Herstellung von GST-PREP (Schwein) - Uniprot Nummer P23687

Der verwendete PREP (Schwein) Expressionsvektor (basiert auf pET-22b) kodiert für ein Fusionsprotein bestehend aus GST-Tag, attB1-5# (Gateway, Invitrogen), TEV-Spaltstelle, PREP (Schwein) Region (1-710) und attB2-3#, (Gateway, Invitrogen).

### E. coli Expression im Bioreaktor:

Der E. coli-Stamm BL21 DE3 wurde mit obigem Expressionsvektor transformiert und durch Ampicillinselektion ein stabiler Stamm etabliert. Dieser transformierte Stamm wurde zur Expression in einen 10 L Bioreaktor (Satorius) eingesetzt. Die Bedingungen für den Bioreaktorlauf waren: Circle grwo Medium, Inkubation für 16 h bei 17°C eingesetzt, Induktion mit 500 µM IPTG bei OD600, 200 µg/mL Ampicillin).

### Reinigung von PREP (Schwein):

Das E. coli Pellet aus 9 L Bioreaktorkultur wurden in 200 mL Lysepuffer (50 mM TRIS-HCl pH 7,5; 150 mM NaCl; 5mM EDTA; 5mM DTT) suspendiert, mit 20 µL Benzonase (Roche) versetzt und im einem Microfluidizer (3 × 900 bar) aufgeschlossen. Die Lösliche Fraktion des Lysats (Zentrifugation 100.000 × g 40 min, 4 °C) wurde für die weitere chromatographische Reinigung eingesetzt.

In einer anschließenden Affinitätschromatographie wurde eine Glutathionmatrix (Protino GST/4B) mit der löslichen Lysatfraktion inkubiert, hierdurch wurde das GST-PREP (Schwein) an die Matrix gebunden, mit Lysepuffer (50 mM Tris HCl pH 7,5; 150 mM NaCl; 5mM EDTA; 5mM DTT und Puffer A (50 mM TRIS-HCl pH 7,5; 150 mM NaCl; 1mM EDTA; 1mM DTT) gewaschen. Anschließend wurde das Fusionsprotein GST-PREP (Schwein) mit Puffer B (50 mM Tris HCl pH 7,5; 150 mM NaCl; 1 mM EDTA; 15 mM Glutathion; 1 mM DTT) eluiert. Die Elutionsfraktionen wurden im Coomassie/PAGE analysiert und die Fraktionen des Elutionspeaks mit GST-PREP (Schwein) wurden vereinigt. (Ausbeute 172 mg Fusionsprotein aus 9 L E. coli Kultur).

Zur Abspaltung des GST-Tags wurde eine Inkubation mit TEV Protease in Lösung während der Dialyse durchgeführt. Hierzu wurde das gereinigte GST-PREP (Schwein) (172 mg) mit TEV Protease (In-Haus-Produktion) im Verhältnis TEV:Fusionprotein von 1:50 w/w versetzt und in einem Dialysesystem (SlideA-Lyzer cut off 10.000 Da) gegen 2 × 2 Liter Puffer A dialysiert (17 h, 6 °C). Mittels Coomassie/PAGE wurde der Spaltansatz analysiert und eine 90 % Umsetzung ermittelt.

In einer weiteren Säulen-Affinitätschromatographie mit Glutathionmatrix (Protino GST/4B) wurde aus dem Spaltansatz im Durchlauf das Protein PREP (Schwein) (ohne GST-Tag) isoliert (110 mg) und mittels einer Ultrafiltrationeinheit (Amicon Ultra millipore 50 kDa cut off) zur weiteren Verarbeitung konzentriert.
Anschließend wurde eine Größenausschluss-Chromatographie durchgeführt um die Monomerfraktion von PREP (Schwein) zu isolieren. Hierzu wurde eine Superdex S200 26/60 Säule (GE) mit SEC-Puffer (50 mM Tris HCl pH 7,5; 150 mM NaCl; 1mM EDTA; 1mM DTT) verwendet. Die finalen Proben wurden aliquotiert (1 mL), in flüssigem Stickstoff schockgefroren und bei -80 °C gelagert. Lagerpuffer: 50 mM Tris-CI pH 7,5; 150 mM NaCl; 1mM EDTA)
Finale Konzentration PREP (Schwein): 3,24 mg/mL, 20 mL, 64,8 mg total, Ausbeute aus 9L E. coli Kultur 7,2 mg/L
Um PREP (Schwein) aus dem Lagerpuffer (siehe oben) in den Kristallisations-Puffer (10mM TRIS-HCl pH7,5; 100mM NaCl) zu überführen, wurde eine Umpufferung mittels einer FPLC Anlage mit einer Entsalzungssäule DS26/10 (bei 6 °C) und anschließender Konzentrierung (Ultrafiltrationseinheit Millipore UFC 905096, 50.000 Da cut off) durchgeführt.

### 4.2 Komplexbildung und Kristallisation von PREP (Schwein)

Das Protein pig PREP lag bei einer Konzentration von ~34mg/ml in 10mM Tris bei pH=7,5 und 100mM NaCl vor. Die Proteinlösung wurde jeweils mit DMSO Lösungen der Beispielverbindungen versetzt, so dass eine Endkonzentration der Beispielverbindugnen von 4mM im Proteinpuffer vorlag. Die Protein-komplexlösung wurde für mindestens 3 Stunden bei 4°C auf einem Schüttler inkubiert und die Lösung anschließend 5 Minuten zentrifugiert. Messbare Einkristalle konnten mit Hilfe der der hanging-drop Methode bei 20°C erhalten werden. Hierzu wurden gleiche Volumina der Proteinlösung und der Reservoirlösung (13-15% PEG8000, 0,2M Magnesiumacetat-tetrahydrat, 0,1M MES bei pH=6,25) zusammen pipettiert und mit Kristallkeimen von pig PREP versetzt. Kristalle von pig PREP entstehen meist über Nacht.

### 4.3 Datensammlung und Prozessierung

Ein optisch gut aussehender Einkristall wurde für sehr kurze Zeit in einer Lösung mit 13-15% PEG8000, 0,2M Magnesiumacetat-tetrahydrat, 0,1M MES bei pH=6,25 und 20% Glycerol gegeben und anschliessend in flüssigen Stickstoff schockck-gefroren. Die Kristalle der Beispiele [237, 108, 113] wurden auf einem Rigaku 007HF Generator der Firma Rigaku bei 100K und einer Wellenlänge von 1.5418Ä vermessen. Als Detektionsgerät diente ein Pilatus 2K Zähler. Die Daten wurden mit dem Programm HKL3000 prozessiert. Die Kristalle der Beispiele [358, 454, 157, 026] sind auf einem Bruker Proteum System bei 100K und einer Wellenlänge von 1.5418Å vermessen wurden. Als Detektionsgerät diente ein CCD Zähler. Die Daten wurden mit dem Programm SAINT integriert und mit dem Programm SADABS skaliert (beide enthalten im Bruker Proterum Programm Paket).

Die Kristalle kristallierten in der orthorhombischen Raumgruppe P2(1)2(1)2(1) mit einem Molekül in der asymetrischen Einheit.

### 4.3 Struktur Bestimmung und Verfeinerung

Die Struktur von PREP (Schwein) konnte mit der Methode des molekeularen Ersatzes (Molecular Replacement) mit einer weiteren internen Struktur als Suchmodell und dem Programm MOLREP (CCP4 Programmpaket) gelöst werden. Alle Beispiele [xxx] wurden mit Hife des Programms Discovery Studio als 3D Modell erzeugt und mit dem Programm PRODRG wurde ein Parameterfile erzeugt. Alle Beispiele wurden manuell in die jeweiligen Elektronendichte plaziert und im Programm COOT in der Elektronendichte minimiert. Die weitere Verfeinerung erfolgte iterativ mit den Programmen REFMAC5.5 und COOT (beide CCP4 Programmpaket). Daten und Verfeinerungsstatistiken aller Beispiele sind in den Tabellen B 2-8 zusammengefasst.

**Tabelle B-2-8: Datensammlungs und Verfeinerungsstatistken für pig PREP im Komplex mit Beispielen 237, 358, 454, 108, 113, 157 und 26.**

| Beispiel | | 237 |
|---|---|---|
| Wellenlänge [Å] | | 1,5418 |
| Auflösung [Å] | | 50,00-1,67 |
| Reflexe (beobachtet/gemittelt) | | 313006 / 91758 |
| Vollständigkeit [%]*^{a}* | | 98,4 (94,5) |
| l/s*^{a}* | | 43,3 (4,2) |
| R_{merge}*^{a,b}* | | 0,042 (0,174) |
| Raumgruppe | | P2(1)2(1)2(1) |
| Zellparameter [Å] | | |
| | *a* | 70,844 |
| | *b* | 99,754 |
| | *c* | 111,326 |
| R_{cryst}*^{c}* | | 0,156 |
| R_{free}*^{d}* | | 0,194 |
| Wilson Temperaturfaktor [Å²] | | 23,1 |
| RMSD Bindungslängen [Å]^{e} | | 0,03 |
| RMSD Bindungswinkel [°] | | 2,495 |

| Beispiel | | 358 |
|---|---|---|
| Wellenlänge [Å] | | 1,5418 |
| Auflösung [Å] | | 74,17-1,72 |
| Reflexe (beobachtet/gemittelt) | | 585565 / 82393 |
| Vollständigkeit [%]*^{a}* | | 97,4 (93,2) |
| l/s*^{a}* | | 23,1 (2,4) |
| R_{merge}*^{a,b}* | | 0,045 (0,419) |
| Raumgruppe | | P2(1)2(1)2(1) |
| Zellparameter [Å] | | |
| | *a* | 70,799 |
| | *b* | 99,485 |
| | *c* | 111,289 |
| R_{cryst}*^{c}* | | 0,177 |
| R_{free}*^{d}* | | 0,215 |
| Wilson Temperaturfaktor [Å²] | | 29,4 |
| RMSD Bindungslängen [Å]*^{e}* | | 0,029 |
| RMSD Bindungswinkel [°] | | 2,41 |

| Beispiel | | 454 |
|---|---|---|
| Wellenlänge [Å] | | 1,5418 |
| Auflösung [Å] | | 73,86 - 1,95 |
| Reflexe (beobachtet/gemittelt) | | 521863 / 56951 |
| Vollständigkeit [%]*^{a}* | | 99,0 (94,4) |
| l/s*^{a}* | | 21,1 (2,6) |
| R_{merge}*^{a,b}* | | 0,061 (0,258) |
| Raumgruppe | | P2(1)2(1)2(1) |
| Zellparameter [Å] | | |
| | *a* | 70,662 |
| | *b* | 98,967 |
| | *c* | 110,958 |
| R_{cryst}*^{c}* | | 0,159 |
| R_{free}*^{d}* | | 0,207 |
| Wilson Temperaturfaktor [Å²] | | 28,6 |
| RMSD Bindungslängen [Å]*^{e}* | | 0,024 |
| RMSD Bindungswinkel [°] | | 2,223 |

| Beispiel | | 108 |
|---|---|---|
| Wellenlänge [Å] | | 1,5418 |
| Auflösung [Å] | | 74,88 - 2,20 |
| Reflexe (beobachtet/gemittelt) | | 186219 / 40375 |
| Vollständigkeit [%]*^{a}* | | 96,9 (88,4) |
| l/s*^{a}* | | 19,4(2,9) |
| R_{merge}*^{a,b}* | | 0,063 (0,299) |
| Raumgruppe | | P2(1)2(1)2(1) |
| Zellparameter [Å] | | |
| | *a* | 71,542 |
| | *b* | 100,758 |
| | *c* | 111,916 |
| R_{cryst}*^{c}* | | 0,199 |
| R_{free}*^{d}* | | 0,264 |
| Wilson Temperaturfaktor [Å²] | | 21,7 |
| RMSD Bindungslängen [Å]*^{e}* | | 0,016 |
| RMSD Bindungswinkel [°] | | 1,714 |

| Beispiel | | 113 |
|---|---|---|
| Wellenlänge [Å] | | 1,5418 |
| Auflösung [Å] | | 74,73 - 1,80 |
| Reflexe (beobachtet/gemittelt) | | 185943 / 68415 |
| Vollständigkeit [%]*^{a}* | | 91,7 (85,7) |
| l/s*^{a}* | | 9,7 (3,6) |
| R_{merge}*^{a,b}* | | 0,090 (0,312) |
| Raumgruppe | | P2(1)2(1)2(1) |
| Zellparameter [Å] | | |
| | *a* | 71,383 |
| | *b* | 100,533 |
| | *c* | 111,728 |
| R_{cryst}*^{c}* | | 0,248 |
| R_{free}*^{d}* | | 0,289 |
| Wilson Temperaturfaktor [Å²] | | 24,0 |
| RMSD Bindungslängen [Å]*^{e}* | | 0,028 |
| RMSD Bindungswinkel [°] | | 2,536 |

| Beispiel | | 157 |
|---|---|---|
| Wellenlänge [Å] | | 1,5418 |
| Auflösung [Å] | | 74,58 - 1,70 |
| Reflexe (beobachtet/gemittelt) | | 440687 / 86834 |
| Vollständigkeit [%]*^{a}* | | 98,2 (92,0) |
| l/s*^{a}* | | 16,1 (3,9) |
| R_{merge}*^{a,b}* | | 0,063 (0,328) |
| Raumgruppe | | P2(1)2(1)2(1) |
| Zellparameter [Å] | | |
| | *a* | 71,183 |
| | *b* | 100,38 |
| | *c* | 111,434 |
| R_{cryst}*^{c}* | | 0,180 |
| R_{free}*^{d}* | | 0,220 |
| Wilson Temperaturfaktor [Å²] | | 18,25 |
| RMSD Bindungslängen [Å]*^{e}* | | 0,028 |
| RMSD Bindungswinkel [°] | | 2,403 |

| Beispiel | | 26 |
|---|---|---|
| Wellenlänge [Å] | | 1,5418 |
| Auflösung [Å] | | 73,92 - 2,30 |
| Reflexe (beobachtet/gemittelt) | | 146137 / 33493 |
| Vollständigkeit [%]*^{a}* | | 94,7 (67,8) |
| l/s*^{a}* | | 17,8 (5,5) |
| R_{merge}*^{a,b}* | | 0,059 (0,104) |
| Raumgruppe | | P2(1)2(1)2(1) |
| Zellparameter [Å] | | |
| | *a* | 70,675 |
| | *b* | 99,198 |
| | *c* | 110,862 |
| R_{cryst}*^{c}* | | 0.158 |
| R_{free}*^{d}* | | 0,233 |
| Wilson Temperaturfaktor [Å²] | | 29,37 |
| RMSD Bindungslängen [Å]*^{e}* | | 0,019 |
| RMSD Bindungswinkel [°] | | 1,920 |

| | | |
|---|---|---|
| *^{a}* Die Werte in Klammern sind für die äußerste Auflösungsschale *^{b}* R_{merge} = Σhkl \|Iₕₖₗ - 〈Iₕₖₗ〉\| / Σhkl 〈Iₕₖₗ〉; Iₕₖₗ ist die Intensität der Reflexe hkl und 〈Iₕₖₗ〉 ist der Mittelwert von mehrfach gemessenen Intensitäten *^{c}* R_{cryst} = Σ \|F_{obs}- F_{calc}\| / Σ F_{obs}; F_{obs} and F_{calc} sind die beobachteten und berechneten Structurfaktoren *^{d}* 5% Testset *^{e}* RMSD, *root mean square deviation* vom Paramterset der idealen Bindungsgeometrie | | |

### 4.4 Absolutstrukturbestimmung von Beispiel 237 in PREP (Schwein)

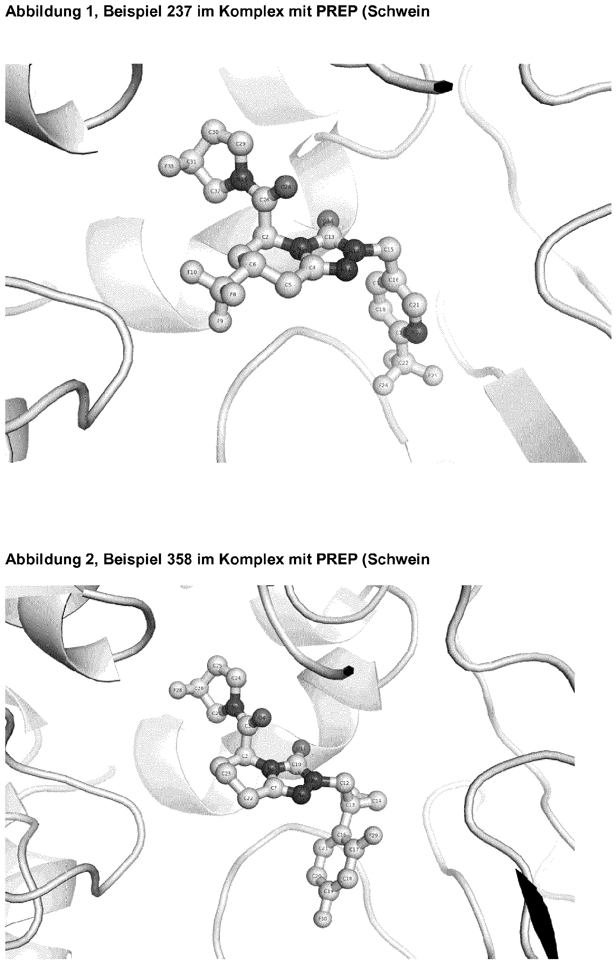

Der Komplex von pig-PREP mit Beispiel 237 kristallisiert mit einem Molekül in der asymetrischen Einheit. Die Stereochemie von Beispiel 237 wird eindeutig durch die Kenntnis der Stereochemie des Proteins pig PREP bestimmt. In Beispiel 237 liegt an den Stereozentren C31 und C2 eindeutig die S-Konfiguration vor, am C6 liegt R-Konfiguration vor (Abbildung 1).

### Struktur aus Beispiel 237: (5S,7R)-5-{[(3S)-3-Fluorpyrrolidin-1-yl]carbonyl}-7-(trifluormethyl)-2-{[6-(trifluormethyl)pyridin-3-yl]methyl}-5,6,7,8-tetrahydro[1,2,4]triazolo[4,3-a]pyridin-3(2H)-on mit der folgenden Formel

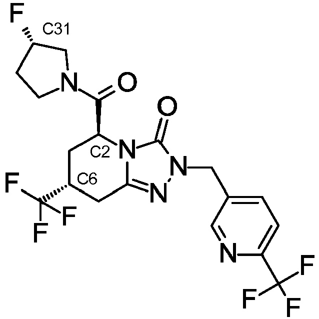

### 4.5 Absolutstrukturbestimmung von Beispiel 358 in PREP (Schwein

Der Komplex von pig-PREP mit Beispiel 358 kristallisiert mit einem Molekül in der asymetrischen Einheit. Die Stereochemie von Beispiel 358 wird eindeutig durch die Kenntnis der Stereochemie des Proteins pig PREP bestimmt. In Beispiel 358 liegt an beiden Stereozentren C26 und C2 eindeutig die S-Konfiguration vor (Abbildung 2).

### Struktur aus Beispiel 358: (5S)-5-[(3-Fluorazetidin-1-yl)carbonyl]-2-{[1-(4-methoxyphenyl)cyclopropyl]methyl}-5,6,7,8-tetrahydro[1,2,4]triazolo[4,3-a]pyridin-3(2H)-on mit der folgenden Formel

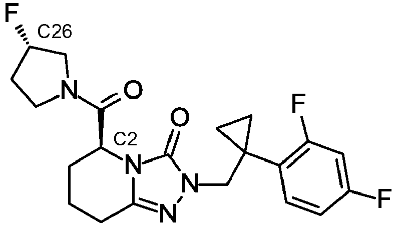

### 4.6 Absolutstrukturbestimmung von Beispiel 454 in PREP (Schwein

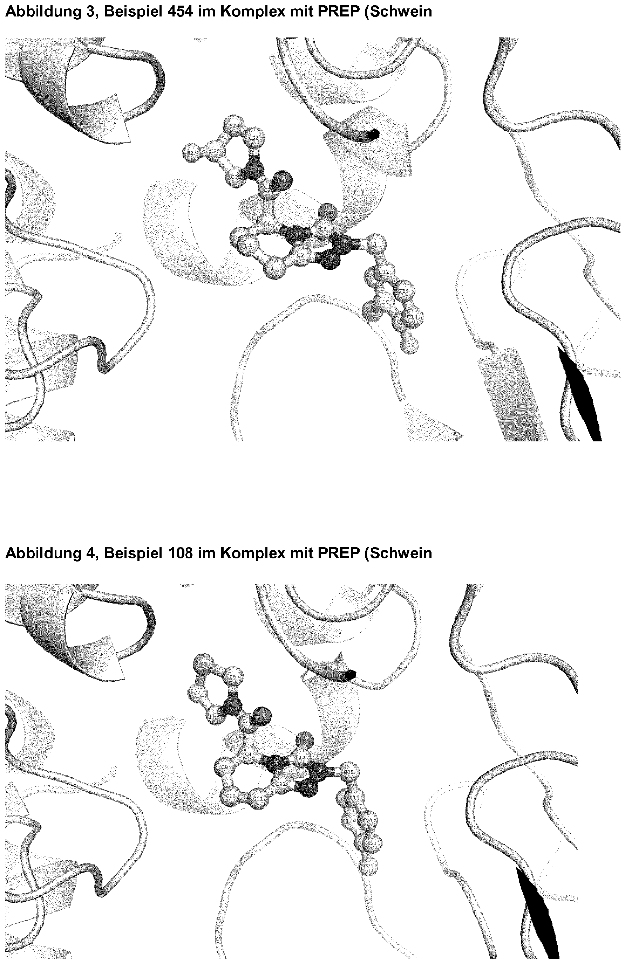

Der Komplex von pig-PREP mit Beispiel 454 kristallisiert mit einem Molekül in der asymetrischen Einheit. Die Stereochemie von Beispiel 454 wird eindeutig durch die Kenntnis der Stereochemie des Proteins pig PREP bestimmt. In Beispiel 454_liegt an beiden Stereozentren C25 und C6 eindeutig die S-Konfiguration vor (Abbildung 3).

### Struktur aus Beispiel 454: (5S)-2-(3-Chlor-4-fluorbenzyl)-5-{[(3S)-3-fluorpyrrolidin-1-yl]carbonyl}-5,6,7,8-tetrahydro[1,2,4]triazolo[4,3-a]pyridin-3(2H)-on mit der folgenden Formel

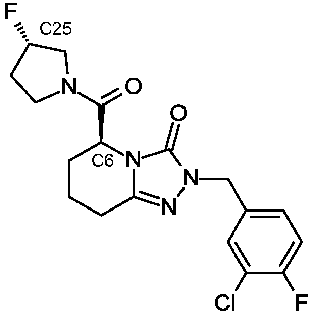

### 4.7 Absolutstrukturbestimmung von Beispiel 108 in PREP (Schwein

Der Komplex von pig-PREP mit Beispiel 108 kristallisiert mit einem Molekül in der asymetrischen Einheit. Die Stereochemie von Beispiel 108 wird eindeutig durch die Kenntnis der Stereochemie des Proteins pig PREP bestimmt. In Beispiel 108 liegt am Stereozentren C8 eindeutig die S-Konfiguration vor (Abbildung 4).

### Struktur aus Beispiel 108: (2-{[(1S)-1-(3-Chlorphenyl)-2-fluorethyl]amino}-7-methoxy-1,3-benzoxazol-5-yl)[(2S,5S)-5-(2-hydroxyethyl)--methylmorpholin-4-yl]methanon mit der folgenden Formel

### (5S)-2-(4-Methylbenzyl)-5-(1,3-thiazolidin-3-ylcarbonyl)-5,6,7,8-tetrahydro[1,2,4]triazolo[4,3-a]pyridin-3(2H)-on

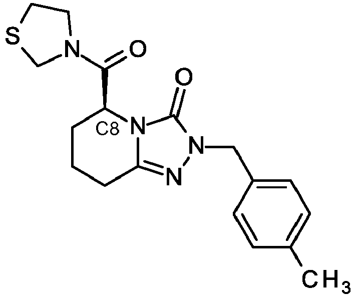

### 4.8 Absolutstrukturbestimmung von Beispiel 113 in PREP (Schwein

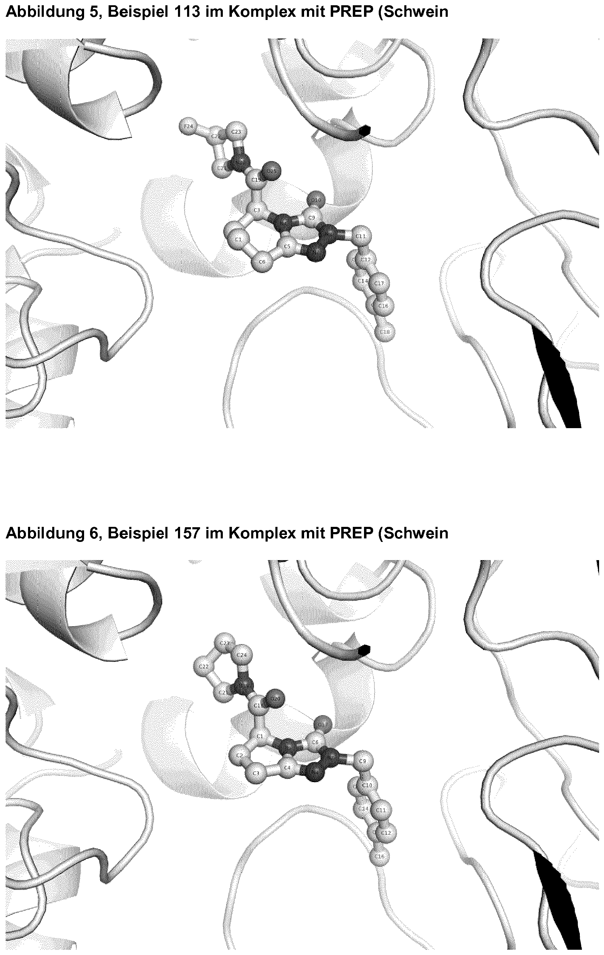

Der Komplex von pig-PREP mit Beispiel 113 kristallisiert mit einem Molekül in der asymetrischen Einheit. Die Stereochemie von Beispiel 113 wird eindeutig durch die Kenntnis der Stereochemie des Proteins pig PREP bestimmt. In Beispiel 113 liegt am Stereozentren C3 eindeutig die S-Konfiguration vor (Abbildung 5).

### Struktur aus Beispiel 113: (5S)-5-[(3-Fluorazetidin-1-yl)carbonyl]-2-(4-methylbenzyl)-5,6,7,8-tetrahydro[1,2,4]triazolo[4,3-a]pyridin-3(2H)-on mit der folgenden Formel

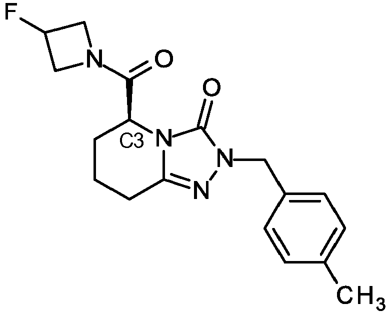

### 4.9 Absolutstrukturbestimmung von Beispiel 157 in PREP (Schwein

Der Komplex von pig-PREP mit Beispiel 157 kristallisiert mit einem Molekül in der asymetrischen Einheit. Die Stereochemie von Beispiel 157 wird eindeutig durch die Kenntnis der Stereochemie des Proteins pig PREP bestimmt. In Beispiel 157 liegt am Stereozentren C1 eindeutig die S-Konfiguration vor (Abbildung 6).

### Struktur aus Beispiel 157: (5S)-2-(4-Methylbenzyl)-5-(pyrrolidin-1-ylcarbonyl)-2,5,6,7-tetrahydro-3H-pyrrolo[2,1-c][1,2,4]triazol-3-on mit der folgenden Formel

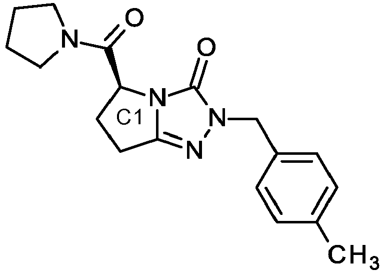

### 4.10 Absolutstrukturbestimmung von Beispiel 26 in PREP (Schwein

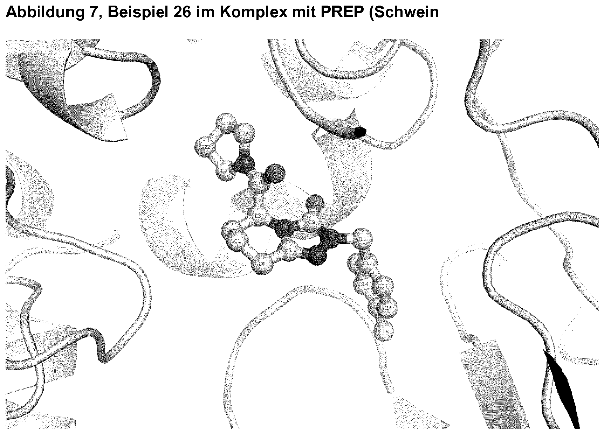

Der Komplex von pig-PREP mit Beispiel 26 kristallisiert mit einem Molekül in der asymetrischen Einheit. Die Stereochemie von Beispiel 26 wird eindeutig durch die Kenntnis der Stereochemie des Proteins pig PREP bestimmt. In Beispiel 26 liegt am Stereozentren C3 eindeutig die S-Konfiguration vor (Abbildung 7).

### Struktur aus Beispiel 26: (5S)-2-(4-Methylbenzyl)-5-(pyrrolidin-1-ylcarbonyl)-5,6,7,8-tetrahydro[1,2,4]triazolo[4,3-a]pyridin-3(2H)-on mit der folgenden Formel

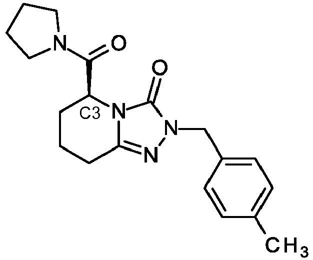

### Literatur B-4:

Emsley P. et al. (2010) Acta Cryst. D66:486-501
Evans P.R, (2005) Acta Cryst. D62, 72-82
Long et al. F (2008) Acta Cryst. D64, 125-132
Murshudo G.N. et al. (1997) Acta Cryst. D53, 240-255
**C. Ausführungsbeispiele für pharmazeutische Zusammensetzungen**

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesium¬stearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5 %-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96 %), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 mL orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungs¬gemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Natriumchlorid-Lösung, Glucose¬lösung 5 % und/oder PEG 400-Lösung 30 %) gelöst. Die Lösung wird steril filtriert und in sterile und pyrogenfreie Injektions¬behältnisse abgefüllt.

## Patentansprüche

1. Verbindung der Formel (I) 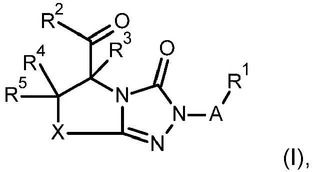 in welcher
A für (C₁-C₄)-Alkylen oder CD₂ steht, wobei (C₁-C₄)-Alkylen mit Hydroxy, (C₁-C₄)-Alkoxy sowie bis zu fünfmal mit Fluor substituiert sein kann,
oder
für eine Gruppe der Formel 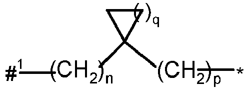 steht, worin
n für 0 oder 1 steht,
p für 0 oder 1 steht,
q für 1 oder 2 steht,
wobei
#¹ die Verknüpfung zum Stickstoffatom des 5-Oxo-4,5-dihydro-1H-1,2,4-triazol-1-yl-Ringes markiert,
* die Verknüpfung zu R¹ markiert,
X für -CR⁶R⁷-, #²-CR⁶R⁷-CR⁸R⁹-**, #²-CR⁶=CR⁸-** oder #²-CR⁶R⁷-CR⁸R⁹-CR¹⁰R¹¹-** steht,
wobei #² die Verknüpfung zum Kohlenstoffatom der CR⁴R⁵-Gruppe markiert,
wobei ** die Verknüpfung zum Kohlenstoffatom des 5-Oxo-4,5-dihydro-1H-1,2,4-triazol-1-yl-Ringes markiert,
wobei
R⁶ für Wasserstoff, Fluor, (C₁-C₄)-Alkyl, (C₁-C₄)-Alkoxy, Trifluormethoxy, Hydroxy, Mono-(C₁-C₄)-alkylamino oder Di-(C₁-C₄)-alkylamino steht,
worin (C₁-C₄)-Alkyl mit (C₁-C₄)-Alkoxy, Hydroxy, Mono-(C₁-C₄)-alkylamino oder Di-(C₁-C₄)-alkylamino sowie bis zu fünfmal mit Fluor substituiert sein kann,
R⁷ für Wasserstoff, Fluor oder (C₁-C₄)-Alkyl steht,
worin (C₁-C₄)-Alkyl bis zu fünfmal mit Fluor substituiert sein kann,
oder
R⁶ und R⁷ zusammen mit dem Kohlenstoffatom, an das sie gebunden sind eine Carbonylgruppe bilden,
oder
R⁶ und R⁷ zusammen mit dem Kohlenstoffatom, an das sie gebunden sind, einen Cyclopropyl-, Cyclobutyl-Ring oder Cyclopentyl-Ring bilden,
oder
R⁶ und R⁴ zusammen mit dem Kohlenstoffatom, an die sie gebunden sind, einen Cyclopropyl-, Cyclobutyl-Ring oder Cyclopentyl-Ring bilden,
R⁸ für Wasserstoff, (C₁-C₄)-Alkyl, (C₁-C₄)-Alkoxy, Fluor, Hydroxy, Mono-(C₁-C₄)-alkylamino oder Di-(C₁-C₄)-alkylamino steht,
worin (C₁-C₄)-Alkyl mit (C₁-C₄)-Alkoxy, Hydroxy, Mono-(C₁-C₄)-alkylamino oder Di-(C₁-C₄)-alkylamino sowie bis zu fünfmal mit Fluor substituiert sein kann,
R⁹ für Wasserstoff, Fluor oder (C₁-C₄)-Alkyl steht, worin (C₁-C₄)-Alkyl bis zu fünfmal mit Fluor substituiert sein kann,
oder
R⁸ und R⁹ zusammen mit dem Kohlenstoffatom, an das sie gebunden sind, einen Cyclopropyl-, Cyclobutyl-Ring oder Cyclopentyl-Ring bilden,
oder
R⁷ und R⁹ zusammen mit den Kohlenstoffatomen, an die sie gebunden sind, einen Cyclopropyl-, Cyclobutyl-Ring oder Cyclopentyl-Ring bilden,
R¹⁰ für Wasserstoff, (C₁-C₄)-Alkyl, (C₁-C₄)-Alkoxy, Fluor, Hydroxy, Mono-(C₁-C₄)-alkylamino oder Di-(C₁-C₄)-alkylamino steht,
worin (C₁-C₄)-Alkyl mit (C₁-C₄)-Alkoxy, Hydroxy, Mono-(C₁-C₄)-alkylamino oder Di-(C₁-C₄)-alkylamino sowie bis zu fünfmal mit Fluor substituiert sein kann,
R¹¹ für Wasserstoff, Fluor oder (C₁-C₄)-Alkyl steht,
worin (C₁-C₄)-Alkyl bis zu fünfmal mit Fluor substituiert sein kann,
oder
R¹⁰ und R¹¹ zusammen mit dem Kohlenstoffatom, an das sie gebunden sind, einen Cyclopropyl-, Cyclobutyl-Ring oder Cyclopentyl-Ring bilden,
R¹ für Phenyl oder 5- bis 6-gliedriges Heteroaryl steht,
wobei Phenyl mit 1 bis 3 Substituenten unabhängig voneinander ausgewählt aus der Gruppe Fluor, Chlor, Cyano, Methyl, Ethyl, Trifluormethyl, Methoxy, Difluormethoxy, Trifluormethoxy, Hydroxycarbonyl, Methoxycarbonyl, Ethoxycarbonyl, tert-Butoxycarbonyl, Methylaminocarbonyl, Dimethylaminocarbonyl, Methylsulfanyl, Methylsulfonyl, Methylsulfonimidoyl, Aminosulfonyl oder Methylsulfinyl substituiert ist,
oder
wobei Phenyl mit Cyclopentyl, Cyclohexyl, 5- bis 6-gliedriges Heterocyclyl oder 5- bis 6-gliedriges Heteroaryl anneliert sein kann,
worin (Cyclopentyl, Cyclohexyl, (C₅-C₆)-Heterocycyl und 5- bis 6-gliedriges Heteroaryl mit 1 oder 2 Substituenten Methyl oder Ethyl substituiert sein können,
oder
wobei 5- bis 6-gliedriges Heteroaryl mit 1 bis 3 Substituenten unabhängig voneinander ausgewählt aus der Gruppe Fluor, Chlor, Cyano, Methyl, Ethyl, Trifluormethyl, Methoxy, Ethoxy, Difluormethoxy, Trifluormethoxy, 2,2,2-Trifluorethoxy, Methylaminocarbonyl, tert-Butylaminocarbonyl, Dimethylaminocarbonyl, substituiert sein kann,
wobei 5- bis 6-gliedriges Heteroaryl mit Cyclopentyl, Cyclohexyl, 5- bis 6-gliedriges Heterocyclyl, Phenyl oder 5- bis 6-gliedriges Heteroaryl anneliert sein kann,
worin 5- bis 6-gliedriges Heteroaryl mit Methyl, Ethyl, Chlor, Fluor oder Methoxy substituiert sein kann,
worin Cyclopentyl, Cyclohexyl, 5- bis 6-gliedriges Heterocyclyl, Phenyl und 5- bis 6-gliedriges Heteroaryl mit 1 oder 2 Substituenten (C₁-C₄)-Alkyl substituiert sein können,
R² für eine Gruppe der Formel 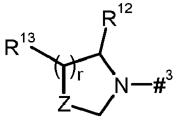 steht, wobei
#³ die Verknüpfung zum Carbonyl-Kohlenstoffatom markiert,
r für 0 oder 1 steht,
Z für O, NR¹⁸, S, SO, SO₂ oder CR^{14A}R^{14B} steht,
worin
R^{14A} für Wasserstoff, Halogen, Cyano, (C₁-C₄)-Alkyl, (C₃-C₆)-Cycloalkyl, Trifluormethyl, Difluormethyl, Fluormethyl, Hydroxy, (C₁-C₄)-Alkoxy, (C₃-C₅)-Cycloalkoxy, Difluormethoxy, Trifluormethoxy oder 2,2,2-Trifluorethoxy steht,
worin (C₁-C₄)-Alkyl mit Hydroxy, Amino, Mono-(C₁-C₄)-alkylamino oder Di-(C₁-C₄)-alkylamino substituiert sein kann,
R^{14B} für Wasserstoff, Fluor oder (C₁-C₄)-Alkyl, steht,
worin (C₁-C₄)-Alkyl bis zu fünfmal mit Fluor substituiert sein kann,
oder
R^{14A} und R^{14B} zusammen mit dem Kohlenstoffatom, an das sie gebunden sind, eine Carbonylgruppe bilden,
R¹⁸ für Wasserstoff oder Methyl steht,
R¹² für Wasserstoff, Cyano, (C₁-C₄)-Alkyl, Acetyl oder Formyl steht,
worin (C₁-C₄)-Alkyl mit Hydroxy oder bis zu fünfmal mit Fluor substituiert sein kann,
worin Acetyl mit Hydroxy oder bis zu dreimal mit Fluor substituiert sein kann,
R¹³ für Wasserstoff, Fluor oder (C₁-C₄)-Alkyl steht,
oder
R¹² und R¹³ zusammen mit den Kohlenstoffatomen, an die sie gebunden sind, einen Cyclopropyl- oder Cyclobutyl-Ring bilden,
worin der Cyclopropyl- oder Cyclobutyl-Ring bis zu zweimal mit Fluor substituiert sein kann,
oder
R¹³ und R^{14A} zusammen mit den Kohlenstoffatomen, an die sie gebunden sind, einen Cyclopropyl- oder Cyclobutyl-Ring bilden,
worin der Cyclopropyl- oder Cyclobutyl -Ring bis zu zweimal mit Fluor substituiert sein kann,
oder
R^{14A} und R^{14B} zusammen mit dem Kohlenstoffatom, an das sie gebunden sind, einen Cyclopropyl- oder Cyclobutyl-Ring bilden,
worin der Cyclopropyl- oder Cyclobutyl-Ring bis zu zweimal mit Fluor substituiert sein kann,
wobei R¹³, R^{14A} und R^{14B} für Wasserstoff stehen, wenn R¹² nicht für Wasserstoff steht,
wobei R¹² für Wasserstoff steht, wenn einer der Substituenten R¹³,
R^{14A} oder R^{14B} nicht für Wasserstoff steht,
oder
für eine Gruppe der Formel 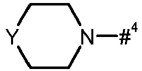 steht, wobei
#⁴ die Verknüpfung zum Carbonyl-Kohlenstoffatom markiert,
Y für NR¹⁵, CR^{16A}R^{16B}, Sauerstoff oder Schwefel steht,
worin
R¹⁵ für Wasserstoff oder Methyl steht,
R^{16A} für Wasserstoff oder Methyl steht,
R^{16B} für Wasserstoff oder Methyl steht,
R³ für Wasserstoff oder (C₁-C₄)-Alkyl steht,
R⁴ für Wasserstoff, (C₁-C₄)-Alkyl, (C₁-C₄)-Alkoxy, Fluor, Hydroxy, Mono-(C₁-C₄)-alkylamino oder Di-(C₁-C₄)-alkylamino steht,
worin (C₁-C₄)-Alkyl mit (C₁-C₄)-Alkoxy, Hydroxy, Mono-(C₁-C₄)-alkylamino oder Di-(C₁-C₄)-alkylamino sowie bis zu fünfmal mit Fluor substituiert sein kann,
R⁵ für Wasserstoff oder (C₁-C₄)-Alkyl steht,
worin (C₁-C₄)-Alkyl bis zu fünfmal mit Fluor substituiert sein kann,
oder
R⁴ und R⁵ zusammen mit dem Kohlenstoffatom, an das sie gebunden sind, eine Carbonylgruppe bilden,
oder
R⁴ und R⁵ zusammen mit dem Kohlenstoffatom, an das sie gebunden sind, einen Cyclopropyl-, Cyclobutyl-Ring oder Cyclopentyl-Ring bilden,
sowie die Salze, Solvate und Solvate der Salze der Verbindungen der Formel (I).

2. Verbindung der Formel (I) nach Anspruch 1, in welcher
A für -CH₂-,-CH(CH₃)-, -CH₂CH₂-, #⁵-CH₂CH(CH₃)-***, #⁵-CH₂C(CH₃)₂-***, #⁵-CH₂CHF-*** oder #⁵-CH₂CF₂-*** steht,
wobei #⁵ die Verknüpfung zum Stickstoffatom des 5-Oxo-4,5-dihydro-1H-1,2,4-triazol-1-yl-Ringes markiert,
wobei *** die Verknüpfung zu der R¹-Gruppe markiert,
X für - #²-CR⁶R⁷-CR⁸R⁹-** steht,
wobei #² die Verknüpfung zum Kohlenstoffatom der CR⁴R⁵-Gruppe markiert,
wobei ** die Verknüpfung zum Kohlenstoffatom des 5-Oxo-4,5-dihydro-1H-1,2,4-triazol-1-yl-Ringes markiert,
wobei
R⁶ für Wasserstoff, Fluor, Methyl, Trifluormethyl oder Hydroxy steht,
R⁷ für Wasserstoff, Fluor oder Methyl steht,
oder
R⁶ und R⁷ zusammen mit dem Kohlenstoffatom, an das sie gebunden sind, einen Cyclopropyl-Ring bilden,
R⁸ für Wasserstoff, Fluor, Methyl oder Trifluormethyl steht,
R⁹ für Wasserstoff, Fluor oder Methyl steht,
R¹ für Phenyl oder 5- bis 6-gliedriges Heteroaryl steht,
wobei Phenyl mit 1 bis 3 Substituenten unabhängig voneinander ausgewählt aus der Gruppe Fluor, Chlor, Cyano, Methyl, Ethyl, Trifluormethyl, Methoxy, Difluormethoxy, Trifluormethoxy, Hydroxycarbonyl, Methoxycarbonyl, Ethoxycarbonyl, tert-Butoxycarbonyl, Methylaminocarbonyl, Dimethylaminocarbonyl, Methylsulfanyl, Methylsulfonyl, Methylsulfonimidoyl, Aminosulfonyl oder Methylsulfinyl substituiert ist,
oder
wobei Phenyl mit Cyclopentyl, Cyclohexyl, 5- bis 6-gliedriges Heterocyclyl oder 5- bis 6-gliedriges Heteroaryl anneliert sein kann,
worin (Cyclopentyl, Cyclohexyl, (C₅-C₆)-Heterocycyl und 5- bis 6-gliedriges Heteroaryl mit 1 oder 2 Substituenten Methyl oder Ethyl substituiert sein können,
oder
wobei 5- bis 6-gliedriges Heteroaryl mit 1 bis 3 Substituenten unabhängig voneinander ausgewählt aus der Gruppe Fluor, Chlor, Cyano, Methyl, Ethyl, Trifluormethyl, Methoxy, Ethoxy, Difluormethoxy, Trifluormethoxy, 2,2,2-Trifluorethoxy, Methylaminocarbonyl, tert-Butylaminocarbonyl und Dimethylaminocarbonyl substituiert sein kann,
wobei 5- bis 6-gliedriges Heteroaryl mit Cyclopentyl, Cyclohexyl, 5- bis 6-gliedriges Heterocyclyl, Phenyl oder 5- bis 6-gliedriges Heteroaryl anneliert sein kann,
worin 5- bis 6-gliedriges Heteroaryl mit Methyl, Ethyl, Chlor, Fluor oder Methoxy substituiert sein kann,
worin Cyclopentyl, Cyclohexyl, 5- bis 6-gliedriges Heterocyclyl, Phenyl und 5- bis 6-gliedriges Heteroaryl mit 1 oder 2 Substituenten (C₁-C₄)-Alkyl substituiert sein können,
R² für eine Gruppe der Formel 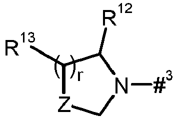 steht, wobei
#³ die Verknüpfung zum Carbonyl-Kohlenstoffatom markiert,
r für 0 oder 1 steht,
Z für S oder CR^{14A}R^{14B} steht, wenn r für 0 steht,
Z für S, SO, SO₂ oder CR^{14A}R^{14B} steht, wenn r für 1 steht, worin jeweils
R^{14A} für Wasserstoff, Fluor, Methyl, Trifluormethyl, Difluormethyl, Fluormethyl, Hydroxy, Methoxy, Difluormethoxy oder Trifluormethoxy steht,
R^{14B} für Wasserstoff oder Fluor steht,
oder
R^{14A} und R^{14B} zusammen mit dem Kohlenstoffatom, an das sie gebunden sind, eine Carbonylgruppe bilden,
R¹² für Wasserstoff, Cyano, Methyl, Acetyl oder Formyl steht, worin Acetyl mit Hydroxy oder bis zu dreimal mit Fluor substituiert ist,
R¹³ für Wasserstoff, Fluor oder Methyl steht,
oder
R¹² und R¹³ zusammen mit den Kohlenstoffatomen, an die sie gebunden sind, einen Cyclopropyl-Ring bilden,
oder
R¹³ und R^{14A} zusammen mit dem Kohlenstoffatom, an die sie gebunden sind, einen Cyclopropyl -Ring bilden,
worin der Cyclopropyl-Ring bis zu zweimal mit Fluor substituiert sein kann,
oder
R^{14A} und R^{14B} zusammen mit dem Kohlenstoffatom, an das sie gebunden sind, einen Cyclopropyl-Ring bilden,
worin der Cyclopropyl-Ring bis zu zweimal mit Fluor substituiert sein kann,
wobei R¹³, R^{14A} und R^{14B} für Wasserstoff stehen, wenn R¹² nicht für Wasserstoff steht,
wobei R¹² für Wasserstoff steht, wenn einer der Substituenten R¹³, R^{14A} oder R^{14B} nicht für Wasserstoff steht,
R³ für Wasserstoff steht,
R⁴ für Wasserstoff, Fluor oder Methyl steht,
R⁵ für Wasserstoff steht,
sowie die Salze, Solvate und Solvate der Salze der Verbindungen der Formel (I).

3. Verbindung der Formel (I) nach Anspruch 1 oder 2 in welcher
A für -CH₂-,-CH(CH₃)-, -CH₂CH₂-, #⁵-CH₂CH(CH₃)-***, #⁵-CH₂C(CH₃)₂-***, #⁵-CH₂CHF-*** oder #⁵-CH₂CF₂-*** steht,
wobei #⁵ die Verknüpfung zum Stickstoffatom des 5-Oxo-4,5-dihydro-1H-1,2,4-triazol-1-yl-Ringes markiert,
wobei *** die Verknüpfung zu der R¹-Gruppe markiert,
X für - #²-CR⁶R⁷-CR⁸R⁹-** steht,
wobei #² die Verknüpfung zum Kohlenstoffatom der CR⁴R⁵-Gruppe markiert,
wobei ** die Verknüpfung zum Kohlenstoffatom des 5-Oxo-4,5-dihydro-1H-1,2,4-triazol-1-yl-Ringes markiert,
wobei
R⁶ für Wasserstoff, Fluor, Methyl, Trifluormethyl oder Hydroxy steht,
R⁷ für Wasserstoff, Fluor oder Methyl steht,
oder
R⁶ und R⁷ zusammen mit dem Kohlenstoffatom, an das sie gebunden sind, einen Cyclopropyl-Ring bilden,
R⁸ für Wasserstoff, Fluor, Methyl oder Trifluormethyl steht,
R⁹ für Wasserstoff, Fluor oder Methyl steht,
R¹ für Phenyl oder 5- bis 6-gliedriges Heteroaryl steht,
wobei Phenyl mit 1 bis 3 Substituenten unabhängig voneinander ausgewählt aus der Gruppe Fluor, Chlor, Cyano, Methyl, Ethyl, Trifluormethyl, Methoxy, Difluormethoxy, Trifluormethoxy, Hydroxycarbonyl, Methoxycarbonyl, Ethoxycarbonyl, tert-Butoxycarbonyl, Methylaminocarbonyl, Dimethylaminocarbonyl, Methylsulfanyl, Methylsulfonyl, Methylsulfonimidoyl, Aminosulfonyl und Methylsulfinyl substituiert ist,
oder
wobei Phenyl mit Cyclopentyl, Cyclohexyl, 5- bis 6-gliedriges Heterocyclyl oder 5- bis 6-gliedriges Heteroaryl anneliert sein kann,
worin (Cyclopentyl, Cyclohexyl, 5- bis 6-gliedriges Heterocyclyl und 5- bis 6-gliedriges Heteroaryl mit 1 oder 2 Substituenten Methyl oder Ethyl substituiert sein können,
oder
wobei 5- bis 6-gliedriges Heteroaryl mit 1 bis 3 Substituenten unabhängig voneinander ausgewählt aus der Gruppe Fluor, Chlor, Cyano, Methyl, Ethyl, Trifluormethyl, Methoxy, Ethoxy, Difluormethoxy, Trifluormethoxy, 2,2,2-Trifluorethoxy, Methylaminocarbonyl, tert-Butylaminocarbonyl und Dimethylaminocarbonylsubstituiert sein kann,
wobei 5- bis 6-gliedriges Heteroaryl mit Cyclopentyl, Cyclohexyl, 5- bis 6-gliedriges Heterocyclyl, Phenyl oder 5- bis 6-gliedriges Heteroaryl anneliert sein kann,
worin 5- bis 6-gliedriges Heteroaryl mit Methyl, Ethyl, Chlor, Fluor oder Methoxy substituiert sein kann,
worin Cyclopentyl, Cyclohexyl 5- bis 6-gliedriges Heterocyclyl, Phenyl und 5- bis 6-gliedriges Heteroaryl mit 1 oder 2 Substituenten (C₁-C₄)-Alkyl substituiert sein können,
R² für eine Gruppe der Formel 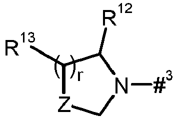 steht, wobei
#³ die Verknüpfung zum Carbonyl-Kohlenstoffatom markiert,
r für 0 oder 1 steht,
Z für S oder CR^{14A}R^{14B} steht, wenn r für 0 steht,
Z für S, SO, SO₂ oder CR^{14A}R^{14B} steht, wenn r für 1 steht, worin jeweils
R^{14A} für Wasserstoff, Fluor, Methyl, Trifluormethyl, Difluormethyl, Fluormethyl, Hydroxy, Methoxy, Difluormethoxy oder Trifluormethoxy steht,
R^{14B} für Wasserstoff oder Fluor steht,
oder
R^{14A} und R^{14B} zusammen mit dem Kohlenstoffatom, an das sie gebunden sind, eine Carbonylgruppe bilden,
R¹² für Wasserstoff, Cyano, Methyl, Acetyl oder Formyl steht, worin Acetyl mit Hydroxy oder bis zu dreimal mit Fluor substituiert ist,
R¹³ für Wasserstoff, Fluor oder Methyl steht,
oder
R¹² und R¹³ zusammen mit den Kohlenstoffatomen, an die sie gebunden sind, einen Cyclopropyl-Ring bilden,
oder
R¹³ und R^{14A} zusammen mit dem Kohlenstoffatom, an die sie gebunden sind, einen Cyclopropyl -Ring bilden,
worin der Cyclopropyl-Ring bis zu zweimal mit Fluor substituiert sein kann,
oder
R^{14A} und R^{14B} zusammen mit dem Kohlenstoffatom, an das sie gebunden sind, einen Cyclopropyl-Ring bilden,
worin der Cyclopropyl-Ring bis zu zweimal mit Fluor substituiert sein kann,
wobei R¹³, R^{14A} und R^{14B} für Wasserstoff stehen, wenn R¹² nicht für Wasserstoff steht,
wobei R¹² für Wasserstoff steht, wenn einer der Substituenten R¹³, R^{14A} oder R^{14B} nicht für Wasserstoff steht,
R³ für Wasserstoff steht,
R⁴ für Wasserstoff, Fluor oder Methyl steht,
R⁵ für Wasserstoff steht,
sowie die Salze, Solvate und Solvate der Salze der Verbindungen der Formel (I).

4. Verbindung der Formel (I) nach Anspruch 1 oder 2, in welcher
A für -CH₂-,-CH(CH₃)-, -CH₂CH₂-, #⁵-CH₂CH(CH₃)-***, #⁵-CH₂C(CH₃)₂-***, #⁵-CH₂CHF-*** oder #⁵-CH₂CF₂-*** steht,
wobei #⁵ die Verknüpfung zum Stickstoffatom des 5-Oxo-4,5-dihydro-1H-1,2,4-triazol-1-yl-Ringes markiert,
wobei *** die Verknüpfung zu der R¹-Gruppe markiert,
X für - #²-CR⁶R⁷-CR⁸R⁹-** steht,
wobei #² die Verknüpfung zum Kohlenstoffatom der CR⁴R⁵-Gruppe markiert,
wobei ** die Verknüpfung zum Kohlenstoffatom des 5-Oxo-4,5-dihydro-1H-1,2,4-triazol-1-yl-Ringes markiert,
wobei
R⁶ für Wasserstoff, Fluor, Methyl, Trifluormethyl oder Hydroxy steht,
R⁷ für Wasserstoff, Fluor oder Methyl steht,
oder
R⁶ und R⁷ zusammen mit dem Kohlenstoffatom, an das sie gebunden sind, einen Cyclopropyl-Ring bilden,
R⁸ für Wasserstoff, Fluor, Methyl oder Trifluormethyl steht,
R⁹ für Wasserstoff, Fluor oder Methyl steht,
R¹ für Phenyl, Pyrazolyl, Imidazolyl, Thiazolyl, Thiophenyl, Oxazolyl, Oxadiazolyl oder Pyridyl steht,
wobei Phenyl mit 1 bis 3 Substituenten unabhängig voneinander ausgewählt aus der Gruppe Fluor, Chlor, Cyano, Methyl, Ethyl, Trifluormethyl, Methoxy, Difluormethoxy, Trifluormethoxy, Hydroxycarbonyl, Methoxycarbonyl, Ethoxycarbonyl, tert-Butoxycarbonyl, Methylaminocarbonyl, Dimethylaminocarbonyl, Methylsulfanyl, Methylsulfonyl, Methylsulfonimidoyl, Aminosulfonyl und Methylsulfinyl substituiert ist,
oder
wobei Phenyl mit Cyclopentyl, Cyclohexyl, Pyrazolyl oder Pyridyl anneliert sein kann,
worin (Cyclopentyl, Cyclohexyl, Pyrazolyl oder Pyridyl mit 1 oder 2 Substituenten Methyl oder Ethyl substituiert sein können,
oder
wobei Pyrazolyl, Imidazolyl, Thiazolyl, Thiophenyl, Oxazolyl, Oxadiazolyl oder Pyridyl mit 1 bis 3 Substituenten unabhängig voneinander ausgewählt aus der Gruppe Fluor, Chlor, Cyano, Methyl, Ethyl, Trifluormethyl, Methoxy, Ethoxy, Difluormethoxy, Trifluormethoxy, 2,2,2-Trifluorethoxy, Methylaminocarbonyl, tert-Butylaminocarbonyl und Dimethylaminocarbonyl, substituiert sein können,
wobei Pyrazolyl, Imidazolyl, Thiazolyl, Thiophenyl, Oxazolyl, Oxadiazolyl oder Pyridyl mit Cyclopentyl, Cyclohexyl, Phenyl oder Pyrdiyl anneliert sein können,
worin Pyridyl mit Methyl, Ethyl, Chlor, Fluor oder Methoxy substituiert sein kann,
worin Cyclopentyl, Cyclohexyl, Phenyl und Pyridyl mit 1 oder 2 Substituenten (C₁-C₄)-Alkyl substituiert sein können,
R² für eine Gruppe der Formel 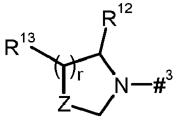 steht, wobei
#³ die Verknüpfung zum Carbonyl-Kohlenstoffatom markiert,
r für 0 oder 1 steht,
Z für S oder CR^{14A}R^{14B} steht, wenn r für 0 steht,
Z für S, SO, SO₂ oder CR^{14A}R^{14B} steht, wenn r für 1 steht,
worin jeweils
R^{14A} für Wasserstoff, Fluor, Methyl, Trifluormethyl, Difluormethyl, Fluormethyl, Hydroxy, Methoxy, Difluormethoxy oder Trifluormethoxy steht,
R^{14B} für Wasserstoff oder Fluor steht,
oder
R^{14A} und R^{14B} zusammen mit dem Kohlenstoffatom, an das sie gebunden sind, eine Carbonylgruppe bilden,
R¹² für Wasserstoff, Cyano, Methyl, Acetyl oder Formyl steht,
worin Acetyl mit Hydroxy oder bis zu dreimal mit Fluor substituiert ist,
R¹³ für Wasserstoff, Fluor oder Methyl steht,
oder
R¹² und R¹³ zusammen mit den Kohlenstoffatomen, an die sie gebunden sind, einen Cyclopropyl-Ring bilden,
oder
R¹³ und R^{14A} zusammen mit dem Kohlenstoffatom, an die sie gebunden sind, einen Cyclopropyl -Ring bilden,
worin der Cyclopropyl-Ring bis zu zweimal mit Fluor substituiert sein kann,
oder
R^{14A} und R^{14B} zusammen mit dem Kohlenstoffatom, an das sie gebunden sind, einen Cyclopropyl-Ring bilden,
worin der Cyclopropyl-Ring bis zu zweimal mit Fluor substituiert sein kann,
wobei R¹³, R^{14A} und R^{14B} für Wasserstoff stehen, wenn R¹² nicht für Wasserstoff steht,
wobei R¹² für Wasserstoff steht, wenn einer der Substituenten R¹³, R^{14A} oder R^{14B} nicht für Wasserstoff steht,
R³ für Wasserstoff steht,
R⁴ für Wasserstoff, Fluor oder Methyl steht,
R⁵ für Wasserstoff steht,
sowie die Salze, Solvate und Solvate der Salze der Verbindungen der Formel (I).

5. Verbindung der Formel (I) gemäß Anspruch 1 mit dem systematischen Namen (5RS,7RS)-2-[(6-Chlorpyridin-3-yl)methyl]-5-{[(3R,4S)-3,4-difluoropyrrolidin-1-yl]carbonyl}-7-(trifluormethyl)-5,6,7,8-tetrahydro[1,2,4]triazolo[4,3-a]pyridin-3(2H)-on und der Strukturformel

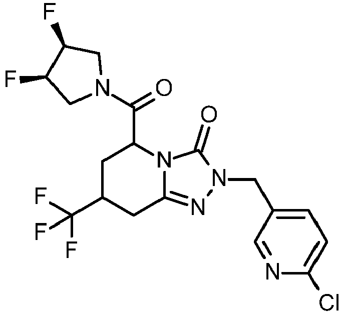

6. Verbindung der Formel (I) gemäß Anspruch 1 mit dem systematischen Namen (5S,7R)-5-{[(3S)-3-Fluorpyrrolidin-1-yl]carbonyl}-7-(trifluormethyl)-2-{[6-(trifluormethyl)pyridin-3-yl]methyl}-5,6,7,8-tetrahydro[1,2,4]triazolo[4,3-a]pyridin-3(2H)-on und der Strukturformel

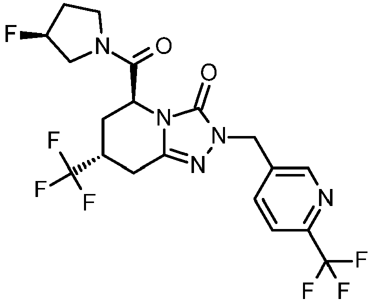

7. Verbindung der Formel (I) gemäß Anspruch 1 mit dem systematischen Namen (5S)-5-{[(3R,4S)-3,4-Difluorpyrrolidin-1-yl]carbonyl}-2-{[3-fluoro-2-(trifluormethyl)pyridin-4-yl]methyl}-5,6,7,8-tetrahydro[1,2,4]triazolo[4,3-a]pyridin-3(2H)-on und der Strukturformel

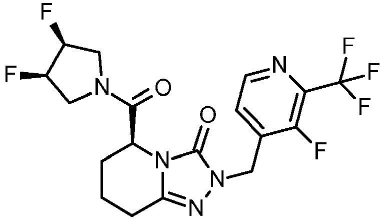

8. Verbindung der Formel (I) gemäß Anspruch 1 mit dem systematischen Namen (5S)-5-{[(3S)-3-Fluorpyrrolidin-1-yl]carbonyl}-2-{[6-(trifluormethyl)pyridin-3-yl]methyl}-5,6,7,8-tetrahydro[1,2,4]triazolo[4,3-a]pyridin-3(2H)-on und der Strukturformel

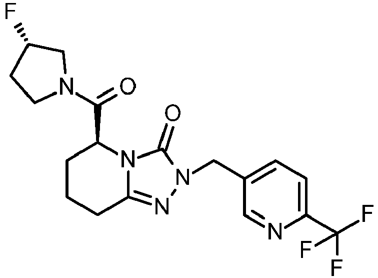

9. Verbindung der Formel (I) gemäß Anspruch 1 mit dem systematischen Namen (5RS,7RS)-2-[(6-Chlorpyridin-3-yl)methyl]-5-{[(3S)-3-fluorpyrrolidin-1-yl]carbonyl}-7-(trifluormethyl)-5,6,7,8-tetrahydro[1,2,4]triazolo[4,3-a]pyridin-3(2H)-on und der Strukturformel

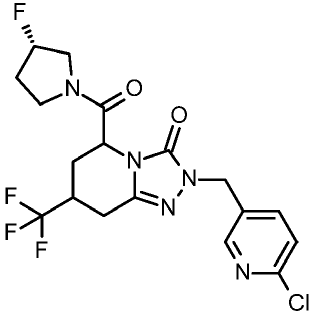

10. Verfahren zur Herstellung einer Verbindung, wie in den Ansprüchen 1 bis 5 definiert, **dadurch gekennzeichnet, dass** man entweder
[A] eine Verbindung der Formel (II) 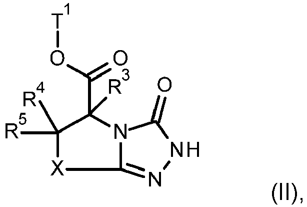 in welcher R³, R⁴, R⁵ und X jeweils die in Anspruch 1 genannten Bedeutungen haben,
und
T¹ für (C₁-C₄)-Alkyl oder Benzyl steht,
in einem inerten Lösungsmittel in Gegenwart einer geeigneten Base mit einer Verbindung der Formel (III) 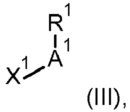 in welcher
A¹ für -CH₂-,-CH(CH₃)-, -CH₂CH₂-, #⁵-CH₂CH(CH₃)-***, #⁵-CH₂C(CH₃)₂-***, #⁵-CH₂CHF-*** oder #⁵-CH₂CF₂-*** steht,
wobei #⁵ die Verknüpfung zu X¹ markiert,
wobei *** die Verknüpfung zu der R¹-Gruppe markiert,
R¹ die in Anspruch 1 genannten Bedeutungen hat,
und
X¹ für eine geeignete Abgangsgruppe, insbesondere Chlor, Brom, lod, Mesylat {(methylsulfonyl)oxy}, Triflat {[(trifluormethyl)sulfonyl]oxy}, Nonaflat {[(nonafluor-butyl)sulfonyl]oxy}, Nosylat {[(4-nitro-phenyl)sulfonyl]oxy} oder Tosylat {[(4-methyl-phenyl)sulfonyl]oxy} steht,
zu einer Verbindung der Formel (IV) 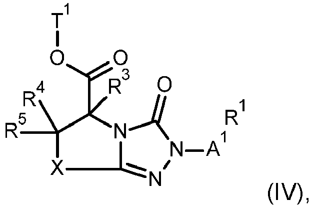 in welcher A¹, R¹, R³, R⁴, R⁵ und X jeweils die in Anspruch 1 genannten Bedeutungen haben,
und
T¹ für (C₁-C₄)-Alkyl oder Benzyl steht,
umsetzt,
diese dann durch Entfernen der Gruppe "T¹" in einem inerten Lösungsmittel in Gegenwart einer geeigneten Base oder Säure in eine Verbindung der Formel (V) 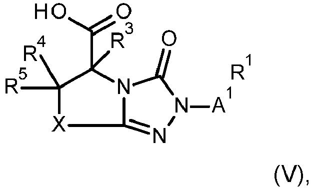 überführt,
in welcher A¹, R¹, R³, R⁴, R⁵ und X jeweils die in Anspruch 1 genannten Bedeutungen haben,
und diese dann in einem inerten Lösungsmittel unter Amidkupplungsbedingungen mit einem Amin der Formel (VI-A) bzw. (VI-B), 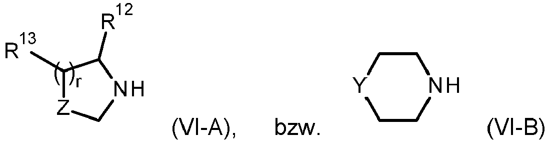 in welcher r, Y, Z, R¹² und R¹³ jeweils die in Anspruch 1 genannten Bedeutungen haben,
umsetzt und anschließend gegebenfalls vorhandene Schutzgruppen nach dem Fachmann bekannten Methoden abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt,
oder
[B] eine Verbindung der Formel (II) 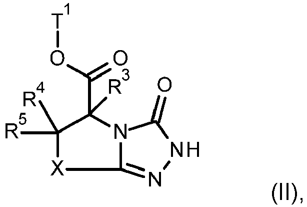 in welcher R³, R⁴, R⁵ und X jeweils die in Anspruch 1 genannten Bedeutungen haben,
und
T¹ für (C₁-C₄)-Alkyl oder Benzyl steht,
in einem inerten Lösungsmittel in Gegenwart einer geeigneten Base mit einem Amin der Formel (VI-A) bzw. (VI-B), 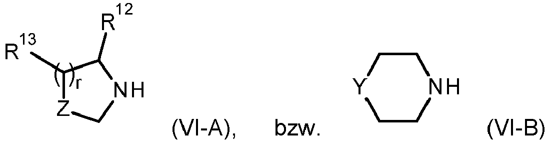 in welcher r, Y, Z, R¹² und R¹³ jeweils die in Anspruch 1 genannten Bedeutungen haben,
zu einer Verbindung der Formel (XXIII-A) bzw. (XXIII-B), 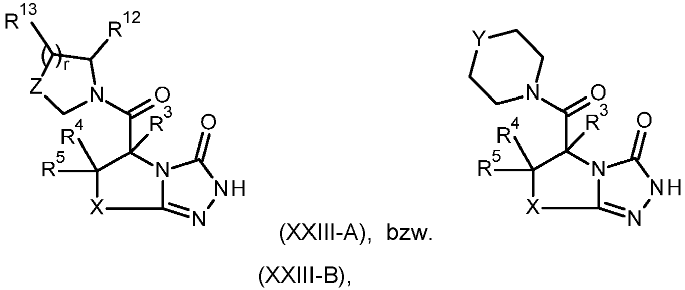 in welcher r, R³, R⁴, R⁵, R¹², R¹³, X, Y und Z jeweils die in Anspruch 1 genannten Bedeutungen haben,
umsetzt,
diese in einem inerten Lösungsmittel in Gegenwart einer geeigneten Base mit einer Verbindung der Formel (III) 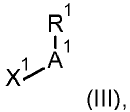 in welcher
A¹ für -CH₂-,-CH(CH₃)-, -CH₂CH₂-, #⁵-CH₂CH(CH₃)-***, #⁵-CH₂C(CH₃)₂-***, #⁵-CH₂CHF-*** oder #⁵-CH₂CF₂-*** steht,
wobei #⁵ die Verknüpfung zu X¹ markiert,
wobei *** die Verknüpfung zu der R¹-Gruppe markiert,
R¹ die in Anspruch 1 genannten Bedeutungen hat,
und
X¹ für eine geeignete Abgangsgruppe, insbesondere Chlor, Brom, lod, Mesylat {(methylsulfonyl)oxy}, Triflat {[(trifluormethyl)sulfonyl]oxy}, Nonaflat {[(nonafluor-butyl)sulfonyl]oxy}, Nosylat {[(4-nitro-phenyl)sulfonyl]oxy} oder Tosylat {[(4-methyl-phenyl)sulfonyl]oxy} steht,
umsetzt und anschließend gegebenfalls vorhandene Schutzgruppen nach dem Fachmann bekannten Methoden abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt, oder
[C] eine Verbindung der Formel (VII) 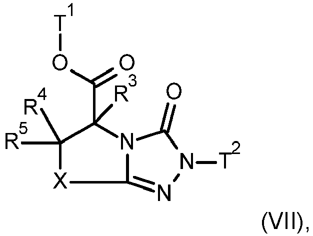 in welcher R³, R⁴, R⁵ und X jeweils die in Anspruch 1 genannten Bedeutungen haben und
T¹ für (C₁-C₄)-Alkyl oder Benzyl steht,
T² für 4-Methoxy-Benzyl, Benzyl, Allyl, β-(Trimethylsilyl)ethoxymethyl (SEM), Methoxymethyl (MOM) oder Benzyloxymethyl steht,
durch Hydrolyse der Estergruppe in einem inerten Lösungsmittel in Gegenwart einer geeigneten Base oder Säure in eine Verbindung der Formel (VIII) 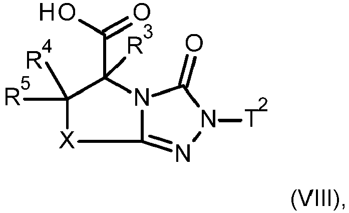 überführt,
in welcher R³, R⁴, R⁵und X jeweils die in Anspruch 1 sowie die oben genannten Bedeutungen haben,
und
T² für 4-Methoxy-Benzyl, Benzyl, Allyl, β-(Trimethylsilyl)ethoxymethyl (SEM), Methoxymethyl (MOM) oder Benzyloxymethyl steht,
diese dann in einem inerten Lösungsmittel unter Amidkupplungsbedingungen mit einem Amin der Formel (VI-A) 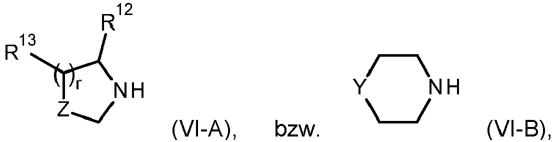 in welcher r, Y, Z, R¹² und R¹³ jeweils die in Anspruch 1 genannten Bedeutungen haben,
zu einer Verbindung der Formel (IX-A) bzw. (IX-B), 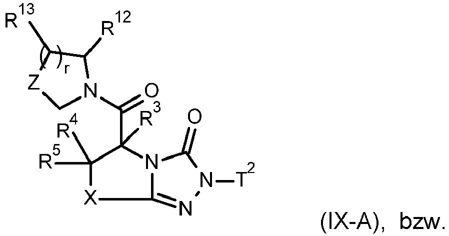 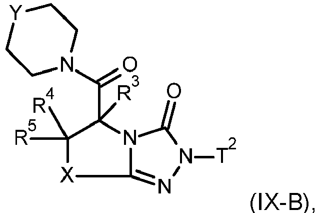 in welcher r, R³, R⁴, R⁵, R¹², R¹³, T², X, Y und Z jeweils die oben genannten Bedeutungen haben,
umsetzt,
die Schutzgruppe "T²" in einem inerten Lösungsmittel in Gegenwart einer geeigneten Base oder Säure oder gegebenenfalls in Anwesenheit eines geeigneten Palladium-Katalysators entfernt und die resultierende Verbindung der Formel (X-A) bzw. (X-B), 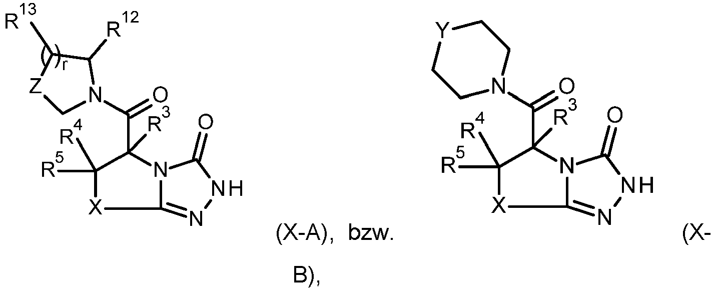 in welcher r, R³, R⁴, R⁵, R¹², R¹³, X, Y und Z jeweils die in Anspruch 1 genannten Bedeutungen haben,
in einem inerten Lösungsmittel in Gegenwart einer geeigneten Base mit einer Verbindung der Formel (III) 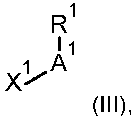 in welcher
A¹ für -CH₂-,-CH(CH₃)-, -CH₂CH₂-, #⁵-CH₂CH(CH₃)-***, #⁵-CH₂C(CH₃)₂-***, #⁵-CH₂CHF-*** oder #⁵-CH₂CF₂-*** steht,
wobei #⁵ die Verknüpfung zu X¹ markiert,
wobei *** die Verknüpfung zu der R¹-Gruppe markiert,
R¹ die in Anspruch 1 genannten Bedeutungen hat,
und
X¹ für eine geeignete Abgangsgruppe, insbesondere Chlor, Brom, lod, Mesylat {(methylsulfonyl)oxy}, Triflat {[(trifluormethyl)sulfonyl]oxy}, Nonaflat {[(nonafluor-butyl)sulfonyl]oxy}, Nosylat {[(4-nitro-phenyl)sulfonyl]oxy} oder Tosylat {[(4-methyl-phenyl)sulfonyl]oxy} steht,
umsetzt und anschließend gegebenfalls vorhandene Schutzgruppen nach dem Fachmann bekannten Methoden abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

11. Verbindung, wie in einem der Ansprüche 1 bis 9 definiert, zur Behandlung und/oder Prophylaxe von Krankheiten.

12. Verbindung, wie in einem der Ansprüche 1 bis 9 definiert, zur Verwendung in einem Verfahren zur Behandlung und/oder Prophylaxe von inflammatorischen Lungenerkrankungen, vor allem der chronisch-obstruktiven Lungenerkrankung (COPD), des Lungenemphysems, der chronischen Bronchitis, der Bronchiektasie, der pulmonalen Hypertonie in der COPD (PH-COPD), der akuten Exazerbation in der COPD, der zystischen Fibrose (Mukoviszidose, CF), des Asthmas, sowie der idiopathischen Lungenfibrose (IPF) des Bronchiolitis obliterans-Syndrom (BOS), der Arteriosklerose, der Myokarditis, sowie entzündlichen Hautund Augenerkrankungen bzw. entzündlichen Erkrankungen der inneren Organe.

13. Verbindung der Formel (I), wie in den Ansprüchen 1 bis 9 definiert, zur Herstellung eines Arzneimittels zur Behandlung und/oder Prophylaxe von inflammatorischen Lungenerkrankungen, vor allem der chronisch-obstruktiven Lungenerkrankung (COPD), des Lungenemphysems, der chronischen Bronchitis, der Bronchiektasie, der pulmonalen Hypertonie in der COPD (PH-COPD), der akuten Exazerbation in der COPD, der zystischen Fibrose (Mukoviszidose, CF), des Asthmas, sowie der idiopathischen Lungenfibrose (IPF) des Bronchiolitis obliterans-Syndrom (BOS), der Arteriosklerose, der Myokarditis, sowie entzündlichen Haut-und Augenerkrankungen bzw. entzündlichen Erkrankungen der inneren Organe.

14. Arzneimittel enthaltend eine Verbindung, wie in einem der Ansprüche 1 bis 9 definiert, in Kombination mit einem oder mehreren inerten, nichttoxischen, pharmazeutisch geeigneten Hilfsstoffen.

15. Arzneimittel enthaltend eine Verbindung, wie in den Ansprüchen 1 bis 9 definiert, in Kombination mit einem oder mehreren Wirkstoffen ausgewählt aus der Gruppe der PDE 5-Inhibitoren, sGC-Aktivatoren, sGC-Stimulatoren, Prostacyclin-Analoga, IP-Rezeptor-Agonisten, Endothelin-Antagonisten, der die Signaltransduktionskaskade inhibierenden Verbindungen und Pirfenidon.

16. Arzneimittel nach Anspruch 14 oder 15 zur Behandlung und/oder Prophylaxe von inflammatorischen Lungenerkrankungen, vor allem der chronisch-obstruktiven Lungenerkrankung (COPD), des Lungenemphysems, der chronischen Bronchitis, der Bronchiektasie, der pulmonalen Hypertonie in der COPD (PH-COPD), der akuten Exazerbation in der COPD, der zystischen Fibrose (Mukoviszidose, CF), des Asthmas, sowie der idiopathischen Lungenfibrose (IPF) des Bronchiolitis obliterans-Syndrom (BOS), der Arteriosklerose, der Myokarditis, sowie entzündlichen Haut- und Augenerkrankungen bzw. entzündlichen Erkrankungen der inneren Organe.

## Claims

1. Compound of the formula (I) 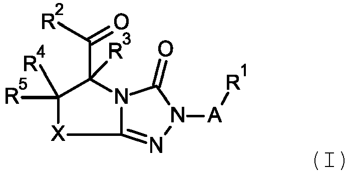 in which
A is (C₁-C₄) -alkylene or CD₂, where (C₁-C₄) -alkylene may be substituted by hydroxyl and (C₁-C₄) -alkoxy and up to pentasubstituted by fluorine,
or
is a group of the formula 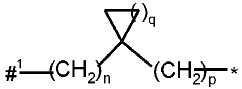 in which
n is 0 or 1,
p is 0 or 1,
q is 1 or 2,
where
#¹ marks the bond to the nitrogen atom of the 5-oxo-4,5-dihydro-1H-1,2,4-triazol-1-yl ring,
* marks the bond to R¹,
X is -CR⁶R⁷-, *²-CR⁶R⁷-CR⁸R⁹-**, #²-CR⁶=CR⁸-** or #²-CR⁶R⁷-CR⁸R⁹-CR¹⁰R¹¹-**,
where #² marks the bond to the carbon atom of the CR⁴R⁵- group,
where ** marks the bond to the carbon atom of the 5-oxo-4,5-dihydro-1H-1,2,4-triazol-1-yl ring,
where
R⁶ is hydrogen, fluorine, (C₁-C₄) -alkyl, (C₁-C₄)-alkoxy, trifluoromethoxy, hydroxyl, mono- (C₁-C₄) -alkylamino or di-(C₁-C₄)-alkylamino,
in which (C₁-C₄) -alkyl may be substituted by (C₁-C₄) -alkoxy, hydroxyl, mono- (C₁-C₄) -alkylamino or di-(C₁-C₄)-alkylamino and up to pentasubstituted by fluorine,
R⁷ is hydrogen, fluorine or (C₁-C₄) -alkyl, in which (C₁-C₄)-alkyl may be up to pentasubstituted by fluorine
or
R⁶ and R⁷ together with the carbon atom to which they are bonded form a carbonyl group,
or
R⁶ and R⁷ together with the carbon atom to which they are bonded form a cyclopropyl ring, cyclobutyl ring or cyclopentyl ring,
or
R⁶ and R⁴ together with the carbon atom to which they are bonded form a cyclopropyl ring, cyclobutyl ring or cyclopentyl ring,
R⁸ is hydrogen, (C₁-C₄) -alkyl, (C₁-C₄) -alkoxy, fluorine, hydroxyl, mono-(C₁-C₄)-alkylamino or di-(C₁-C₄)-alkylamino,
in which (C₁-C₄) -alkyl may be substituted by (C₁-C₄) -alkoxy, hydroxyl, mono- (C₁-C₄) -alkylamino or di-(C₁-C₄)-alkylamino and up to pentasubstituted by fluorine,
R⁹ is hydrogen, fluorine or (C₁-C₄)-alkyl,
in which (C₁-C₄) -alkyl may be up to pentasubstituted by fluorine
or
R⁸ and R⁹ together with the carbon atom to which they are bonded form a cyclopropyl ring, cyclobutyl ring or cyclopentyl ring,
or
R⁷ and R⁹ together with the carbon atoms to which they are bonded form a cyclopropyl ring, cyclobutyl ring or cyclopentyl ring,
R¹⁰ is hydrogen, (C₁-C₄) -alkyl, (C₁-C₄) -alkoxy, fluorine, hydroxyl, mono-(C₁-C₄)-alkylamino or di-(C₁-C₄)-alkylamino,
in which (C₁-C₄) -alkyl may be substituted by (C₁-C₄) -alkoxy, hydroxyl, mono- (C₁-C₄) -alkylamino or di-(C₁-C₄)-alkylamino and up to pentasubstituted by fluorine,
R¹¹ is hydrogen, fluorine or (C₁-C₄) -alkyl, in which (C₁-C₄)-alkyl may be up to pentasubstituted by fluorine
or
R¹⁰ and R¹¹ together with the carbon atom to which they are bonded form a cyclopropyl ring, cyclobutyl ring or cyclopentyl ring,
R¹ is phenyl or 5- to 6-membered heteroaryl,
where phenyl is substituted by 1 to 3 substituents independently selected from the group of fluorine, chlorine, cyano, methyl, ethyl, trifluoromethyl, methoxy, difluoro-methoxy, trifluoromethoxy, hydroxycarbonyl, methoxycarbonyl, ethoxycarbonyl, tert-butoxycarbonyl, methylaminocarbonyl, dimethylaminocarbonyl, methylsulfanyl, methylsulfonyl, methylsulfonimidoyl, aminosulfonyl or methylsulfinyl,
or
where phenyl may be fused to cyclopentyl, cyclohexyl, 5- to 6-membered heterocyclyl or 5-to 6-membered heteroaryl,
in which cyclopentyl, cyclohexyl, (C₅-C₆)-heterocyclyl and 5- to 6-membered heteroaryl may be substituted by 1 or 2 methyl or ethyl substituents,
or
where 5- to 6-membered heteroaryl may be substituted by 1 to 3 substituents independently selected from the group of fluorine, chlorine, cyano, methyl, ethyl, trifluoromethyl, methoxy, ethoxy, difluoromethoxy, trifluoromethoxy, 2,2,2-trifluoroethoxy, methylaminocarbonyl, tert-butylaminocarbonyl, dimethylaminocarbonyl, where 5- to 6-membered heteroaryl may be fused to cyclopentyl, cyclohexyl, 5- to 6-membered heterocyclyl, phenyl or 5- to 6-membered heteroaryl,
where 5- to 6-membered heteroaryl may be substituted by methyl, ethyl, chlorine, fluorine or methoxy,
in which cyclopentyl, cyclohexyl, 5- to 6-membered heterocyclyl, phenyl and 5- to 6-membered heteroaryl may be substituted by 1 or 2 (C₁-C₄)-alkyl substituents,
R² is a group of the formula 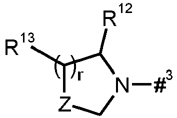 where
#³ marks the bond to the carbonyl carbon atom,
r is 0 or 1,
Z is O, NR¹⁸, S, SO, SO₂ or CR^{14A}R^{14B},
in which
R^{14A} is hydrogen, halogen, cyano, (C₁-C₄)-alkyl, (C₃-C₆)-cycloalkyl, trifluoromethyl, difluoromethyl, fluoromethyl, hydroxyl, (C₁-C₄)-alkoxy, (C₃-C₅)-cycloalkoxy, difluoromethoxy, trifluoromethoxy or 2,2,2-trifluoroethoxy,
in which (C₁-C₄) -alkyl may be substituted by hydroxyl, amino, mono-(C₁-C₄)-alkylamino or di-(C₁-C₄) -alkylamino,
R^{14B} is hydrogen, fluorine or (C₁-C₄)-alkyl,
in which (C₁-C₄)-alkyl may be up to pentasubstituted by fluorine
or
R^{14A} and R^{14B} together with the carbon atom to which they are bonded form a carbonyl group,
R¹⁸ is hydrogen or methyl,
R¹² is hydrogen, cyano, (C₁-C₄) -alkyl, acetyl or formyl,
in which (C₁-C₄) -alkyl may be substituted by hydroxyl or up to pentasubstituted by fluorine, in which acetyl may be substituted by hydroxyl or up to trisubstituted by fluorine,
R¹³ is hydrogen, fluorine or (C₁-C₄)-alkyl,
or
R¹² and R¹³ together with the carbon atoms to which they are bonded form a cyclopropyl or cyclobutyl ring,
in which the cyclopropyl or cyclobutyl ring may be up to disubstituted by fluorine,
or
R¹³ and R^{14A} together with the carbon atoms to which they are bonded form a cyclopropyl or cyclobutyl ring, in which the cyclopropyl or cyclobutyl ring may be up to disubstituted by fluorine,
or
R^{14A} and R^{14B} together with the carbon atom to which they are bonded form a cyclopropyl or cyclobutyl ring,
in which the cyclopropyl or cyclobutyl ring may be up to disubstituted by fluorine,
where R¹³, R^{14A} and R^{14B} are hydrogen when R¹² is not hydrogen,
where R¹² is hydrogen when one of the R¹³, R^{14A} and R^{14B} substituents is not hydrogen,
or
is a group of the formula 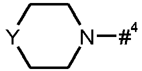 where
#⁴ marks the bond to the carbonyl carbon atom,
Y is NR¹⁵, CR^{16A}R^{16B}, oxygen or sulfur,
in which
R¹⁵ is hydrogen or methyl,
R^{16A} is hydrogen or methyl,
R^{16B} is hydrogen or methyl,
R³ is hydrogen or (C₁-C₄) -alkyl,
R⁴ is hydrogen, (C₁-C₄) -alkyl, (C₁-C₄) -alkoxy, fluorine, hydroxyl, mono-(C₁-C₄)-alkylamino or di-(C₁-C₄) -alkylamino,
in which (C₁-C₄)-alkyl may be substituted by (C₁-C₄)-alkoxy, hydroxyl, mono-(C₁-C₄)-alkylamino or di-(C₁-C₄)-alkylamino and up to pentasubstituted by fluorine,
R⁵ is hydrogen or (C₁-C₄) -alkyl, in which (C₁-C₄) -alkyl may be up to pentasubstituted by fluorine
or
R⁴ and R⁵ together with the carbon atom to which they are bonded form a carbonyl group,
or
R⁴ and R⁵ together with the carbon atom to which they are bonded form a cyclopropyl ring, cyclobutyl ring or cyclopentyl ring,
and the salts, solvates and solvates of the salts of the compounds of the formula (I).

2. Compound of the formula (I) according to Claim 1, in which
A is -CH₂-,-CH (CH₃)-, -CH₂CH₂-, #⁵-CH₂CH(CH₃)-***, #⁵-CH₂C(CH₃)₂-***, #⁵-CH₂CHF-*** or #⁵-CH₂CF₂-***,
where #⁵ marks the bond to the nitrogen atom of the 5-oxo-4,5-dihydro-1H-1,2,4-triazol-1-yl ring,
where *** marks the bond to the R¹ group,
X is - #²-CR⁶R⁷-CR⁸R⁹-**,
where #² marks the bond to the carbon atom of the CR⁴R⁵- group,
where ** marks the bond to the carbon atom of the 5-oxo-4,5-dihydro-1H-1,2,4-triazol-1-yl ring,
where
R⁶ is hydrogen, fluorine, methyl, trifluoromethyl or hydroxyl,
R⁷ is hydrogen, fluorine or methyl,
or
R⁶ and R⁷ together with the carbon atom to which they are bonded form a cyclopropyl ring,
R⁸ is hydrogen, fluorine, methyl or trifluoromethyl,
R⁹ is hydrogen, fluorine or methyl,
R¹ is phenyl or 5- to 6-membered heteroaryl,
where phenyl is substituted by 1 to 3 substituents independently selected from the group of fluorine, chlorine, cyano, methyl, ethyl, trifluoromethyl, methoxy, difluoro-methoxy, trifluoromethoxy, hydroxycarbonyl, methoxycarbonyl, ethoxycarbonyl, tert-butoxycarbonyl, methylaminocarbonyl, dimethylaminocarbonyl, methylsulfanyl, methylsulfonyl, methylsulfonimidoyl, aminosulfonyl or methylsulfinyl,
or
where phenyl may be fused to cyclopentyl, cyclohexyl, 5- to 6-membered heterocyclyl or 5-to 6-membered heteroaryl,
in which cyclopentyl, cyclohexyl, (C₅-C₆)-heterocyclyl and 5- to 6-membered heteroaryl may be substituted by 1 or 2 methyl or ethyl substituents,
or
where 5- to 6-membered heteroaryl may be substituted by 1 to 3 substituents independently selected from the group of fluorine, chlorine, cyano, methyl, ethyl, trifluoromethyl, methoxy, ethoxy, difluoromethoxy, trifluoromethoxy, 2,2,2-trifluoroethoxy, methylaminocarbonyl, tert-butylaminocarbonyl and dimethylaminocarbonyl, where 5- to 6-membered heteroaryl may be fused to cyclopentyl, cyclohexyl, 5- to 6-membered heterocyclyl, phenyl or 5- to 6-membered heteroaryl,
where 5- to 6-membered heteroaryl may be substituted by methyl, ethyl, chlorine, fluorine or methoxy, in which cyclopentyl, cyclohexyl, 5- to 6-membered heterocyclyl, phenyl and 5- to 6-membered heteroaryl may be substituted by 1 or 2 (C₁-C₄)-alkyl substituents,
R² is a group of the formula 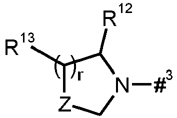 where
#³ marks the bond to the carbonyl carbon atom,
r is 0 or 1,
Z is S or CR^{14A}R^{14B} when r is 0,
Z is S, SO, SO₂ or CR^{14A}R^{14B} when r is 1, in each of which
R^{14A} is hydrogen, fluorine, methyl, trifluoromethyl, difluoromethyl, fluoromethyl, hydroxyl, methoxy, difluoromethoxy or trifluoromethoxy,
R^{14B} is hydrogen or fluorine, or
R^{14A} and R^{14B} together with the carbon atom to which they are bonded form a carbonyl group,
R¹² is hydrogen, cyano, methyl, acetyl or formyl,
in which acetyl is substituted by hydroxyl or up to trisubstituted by fluorine,
R¹³ is hydrogen, fluorine or methyl,
or
R¹² and R¹³ together with the carbon atoms to which they are bonded form a cyclopropyl ring,
or
R¹³ and R^{14A} together with the carbon atom to which they are bonded form a cyclopropyl ring, in which the cyclopropyl ring may be up to disubstituted by fluorine,
or
R^{14A} and R^{14B} together with the carbon atom to which they are bonded form a cyclopropyl ring,
in which the cyclopropyl ring may be up to disubstituted by fluorine,
where R¹³, R^{14A} and R^{14B} are hydrogen when R¹² is not hydrogen, where R¹² is hydrogen when one of the R¹³, R^{14A} and R^{14B} substituents is not hydrogen,
R³ is hydrogen,
R⁴ is hydrogen, fluorine or methyl,
R⁵ is hydrogen,
and the salts, solvates and solvates of the salts of the compounds of the formula (I).

3. Compound of the formula (I) according to Claim 1 or 2, in which
A is -CH₂-,-CH (CH₃)-, -CH₂CH₂-, #⁵-CH₂CH(CH₃)-***, #⁵-CH₂C(CH₃)₂-***, #⁵-CH₂CHF-*** or #⁵-CH₂CF₂-***,
where #⁵ marks the bond to the nitrogen atom of the 5-oxo-4,5-dihydro-1H-1,2,4-triazol-1-yl ring,
where *** marks the bond to the R¹ group,
X is - #²-CR⁶R⁷-CR⁸R⁹-**,
where #² marks the bond to the carbon atom of the CR⁴R⁵- group,
where ** marks the bond to the carbon atom of the 5-oxo-4,5-dihydro-1H-1,2,4-triazol-1-yl ring,
where
R⁶ is hydrogen, fluorine, methyl, trifluoromethyl or hydroxyl,
R⁷ is hydrogen, fluorine or methyl,
or
R⁶ and R⁷ together with the carbon atom to which they are bonded form a cyclopropyl ring,
R⁸ is hydrogen, fluorine, methyl or trifluoromethyl,
R⁹ is hydrogen, fluorine or methyl,
R¹ is phenyl or 5- to 6-membered heteroaryl,
where phenyl is substituted by 1 to 3 substituents independently selected from the group of fluorine, chlorine, cyano, methyl, ethyl, trifluoromethyl, methoxy, difluoromethoxy, trifluoromethoxy, hydroxy-carbonyl, methoxycarbonyl, ethoxycarbonyl, tert-butoxycarbonyl, methylaminocarbonyl, dimethylaminocarbonyl, methylsulfanyl, methylsulfonyl, methylsulfonimidoyl, aminosulfonyl and methylsulfinyl,
or
where phenyl may be fused to cyclopentyl, cyclohexyl, 5- to 6-membered heterocyclyl or 5-to 6-membered heteroaryl,
in which cyclopentyl, cyclohexyl, (C₅-C₆)-heterocyclyl and 5- to 6-membered heteroaryl may be substituted by 1 or 2 methyl or ethyl substituents,
or
where 5- to 6-membered heteroaryl may be substituted by 1 to 3 substituents independently selected from the group of fluorine, chlorine, cyano, methyl, ethyl, trifluoromethyl, methoxy, ethoxy, difluoromethoxy, trifluoromethoxy, 2,2,2-trifluoroethoxy, methylaminocarbonyl, tert-butylaminocarbonyl and dimethylaminocarbonyl, where 5- to 6-membered heteroaryl may be fused to cyclopentyl, cyclohexyl, 5- to 6-membered heterocyclyl, phenyl or 5- to 6-membered heteroaryl,
where 5- to 6-membered heteroaryl may be substituted by methyl, ethyl, chlorine, fluorine or methoxy, in which cyclopentyl, cyclohexyl, 5- to 6-membered heterocyclyl, phenyl and 5- to 6-membered heteroaryl may be substituted by 1 or 2 (C₁-C₄)-alkyl substituents,
R² is a group of the formula 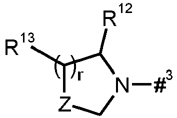 where
#³ marks the bond to the carbonyl carbon atom, ris 0 or 1,
Zis S or CR^{14A}R^{14B} when r is 0,
Zis S, SO, SO₂ or CR^{14A}R^{14B} when r is 1,
in each of which
R^{14A} is hydrogen, fluorine, methyl, trifluoromethyl, difluoromethyl, fluoromethyl, hydroxyl, methoxy, difluoromethoxy or trifluoromethoxy,
R^{14B} is hydrogen or fluorine,
or
R^{14A} and R^{14B} together with the carbon atom to which they are bonded form a carbonyl group,
R¹² is hydrogen, cyano, methyl, acetyl or formyl,
in which acetyl is substituted by hydroxyl or up to trisubstituted by fluorine,
R¹³ is hydrogen, fluorine or methyl,
or
R¹² and R¹³ together with the carbon atoms to which they are bonded form a cyclopropyl ring,
or
R¹³ and R^{14A} together with the carbon atom to which they are bonded form a cyclopropyl ring,
in which the cyclopropyl ring may be up to disubstituted by fluorine,
or
R^{14A} and R^{14B} together with the carbon atom to which they are bonded form a cyclopropyl ring,
in which the cyclopropyl ring may be up to disubstituted by fluorine,
where R¹³, R^{14A} and R^{14B} are hydrogen when R¹² is not hydrogen,
where R¹² is hydrogen when one of the R¹³, R^{14A} and R^{14B} substituents is not hydrogen,
R³ is hydrogen,
R⁴ is hydrogen, fluorine or methyl,
R⁵ is hydrogen,
and the salts, solvates and solvates of the salts of the compounds of the formula (I).

4. Compound of the formula (I) according to Claim 1 or 2, in which
A is -CH₂-,-CH (CH₃)-, -CH₂CH₂-, #⁵-CH₂CH(CH₃)-***, #⁵-CH₂C(CH₃)₂-***, #⁵-CH₂CHF-*** or #⁵-CH₂CF₂-***,
where #⁵ marks the bond to the nitrogen atom of the 5-oxo-4,5-dihydro-1H-1,2,4-triazol-1-yl ring,
where *** marks the bond to the R¹ group,
X is - #²-CR⁶R⁷-CR⁸R⁹-**,
where #² marks the bond to the carbon atom of the CR⁴R⁵- group,
where ** marks the bond to the carbon atom of the 5-oxo-4,5-dihydro-1H-1,2,4-triazol-1-yl ring,
where
R⁶ is hydrogen, fluorine, methyl, trifluoromethyl or hydroxyl,
R⁷ is hydrogen, fluorine or methyl,
or
R⁶ and R⁷ together with the carbon atom to which they are bonded form a cyclopropyl ring,
R⁸ is hydrogen, fluorine, methyl or trifluoromethyl,
R⁹ is hydrogen, fluorine or methyl,
R¹ is phenyl, pyrazolyl, imidazolyl, thiazolyl, thiophenyl, oxazolyl, oxadiazolyl or pyridyl,
where phenyl is substituted by 1 to 3 substituents independently selected from the group of fluorine, chlorine, cyano, methyl, ethyl, trifluoromethyl, methoxy, difluoromethoxy, trifluoromethoxy, hydroxy-carbonyl, methoxycarbonyl, ethoxycarbonyl, tert-butoxycarbonyl, methylaminocarbonyl, dimethylaminocarbonyl, methylsulfanyl, methylsulfonyl, methylsulfonimidoyl, aminosulfonyl and methylsulfinyl,
or
where phenyl may be fused to cyclopentyl, cyclohexyl, pyrazolyl or pyridyl,
in which cyclopentyl, cyclohexyl, pyrazolyl or pyridyl may be substituted by 1 or 2 methyl or ethyl substituents,
or
where pyrazolyl, imidazolyl, thiazolyl, thiophenyl, oxazolyl, oxadiazolyl or pyridyl may be substituted by 1 to 3 substituents independently selected from the group of fluorine, chlorine, cyano, methyl, ethyl, trifluoromethyl, methoxy, ethoxy, difluoromethoxy, trifluoromethoxy, 2,2,2-trifluoroethoxy, methylaminocarbonyl, tert-butylaminocarbonyl and dimethylaminocarbonyl, where pyrazolyl, imidazolyl, thiazolyl, thiophenyl, oxazolyl, oxadiazolyl or pyridyl may be fused to cyclopentyl, cyclohexyl, phenyl or pyridyl,
in which pyridyl may be substituted by methyl, ethyl, chlorine, fluorine or methoxy,
in which cyclopentyl, cyclohexyl, phenyl and pyridyl may be substituted by 1 or 2 (C₁-C₄)-alkyl substituents,
R² is a group of the formula 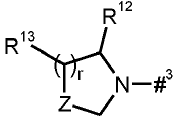 where
#³ marks the bond to the carbonyl carbon atom,
r is 0 or 1,
Z is S or CR^{14A}R^{14B} when r is 0,
Z is S, SO, SO₂ or CR^{14A}R^{14B} when r is 1, in each of which
R^{14A} is hydrogen, fluorine, methyl, trifluoromethyl, difluoromethyl, fluoromethyl, hydroxyl, methoxy, difluoromethoxy or trifluoromethoxy,
R^{14B} is hydrogen or fluorine,
or
R^{14A} and R^{14B} together with the carbon atom to which they are bonded form a carbonyl group,
R¹² is hydrogen, cyano, methyl, acetyl or formyl,
in which acetyl is substituted by hydroxyl or up to trisubstituted by fluorine,
R¹³ is hydrogen, fluorine or methyl,
or
R¹² and R¹³ together with the carbon atoms to which they are bonded form a cyclopropyl ring,
or
R¹³ and R^{14A} together with the carbon atom to which they are bonded form a cyclopropyl ring,
in which the cyclopropyl ring may be up to disubstituted by fluorine,
or
R^{14A} and R^{14B} together with the carbon atom to which they are bonded form a cyclopropyl ring,
in which the cyclopropyl ring may be up to disubstituted by fluorine,
where R¹³, R^{14A} and R^{14B} are hydrogen when R¹² is not hydrogen,
where R¹² is hydrogen when one of the R¹³, R^{14A} and R^{14B} substituents is not hydrogen,
R³ is hydrogen,
R⁴ is hydrogen, fluorine or methyl,
R⁵ is hydrogen,
and the salts, solvates and solvates of the salts of the compounds of the formula (I).

5. Compound of the formula (I) according to Claim 1 having the systematic name (5RS,7RS)-2-[(6-chloropyridin-3-yl)methyl]-5-{[(3R,4S)-3,4-difluoropyrrolidin-1-yl]carbonyl}-7-(trifluoro-methyl)-5,6,7,8-tetrahydro[1,2,4]triazolo[4,3-a]-pyridin-3(2H)-one and of the structural formula

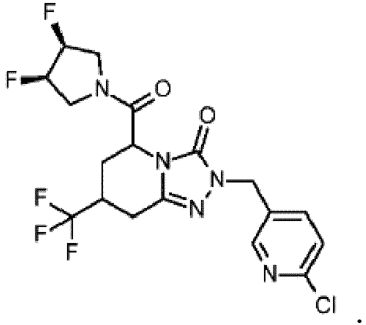

6. Compound of the formula (I) according to Claim 1 having the systematic name (5S,7R)-5-{[(3S)-3-fluoropyrrolidin-1-yl]carbonyl}-7-(trifluoro-methyl)-2-{[6-(trifluormethyl)pyridin-3-yl]-methyl}-5,6,7,8-tetrahydro[1,2,4]triazolo[4,3-a]-pyridin-3(2H)-one and of the structural formula

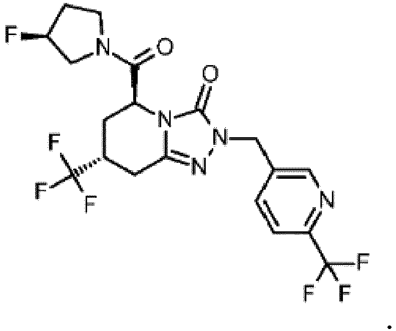

7. Compound of the formula (I) according to Claim 1 having the systematic name (5S)-5-{[(3R,4S)-3,4-difluoropyrrolidin-1-yl]carbonyl}-2-{[3-fluoro-2-(trifluormethyl)pyridin-4-yl]methyl}-5,6,7,8-tetrahydro[1,2,4]triazolo[4,3-a]pyridin-3(2H)-one and of the structural formula

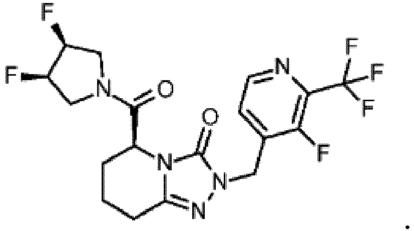

8. Compound of the formula (I) according to Claim 1 having the systematic name (5S)-5-{[(3S)-3-fluoropyrrolidin-1-yl]carbonyl}-2-{[6-(trifluoromethyl)pyridin-3-yl]methyl}-5,6,7,8-tetrahydro[1,2,4]triazolo[4,3-a]pyridin-3(2H)-one and of the structural formula

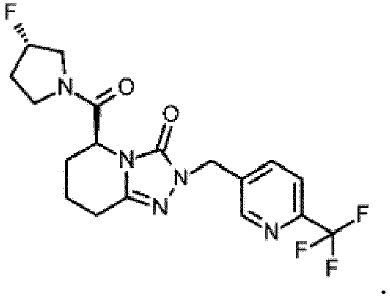

9. Compound of the formula (I) according to Claim 1 having the systematic name (5RS,7RS)-2-[(6-chloropyridin-3-yl)methyl]-5-{[(3S)-3-fluoro-pyrrolidin-1-yl]carbonyl}-7-(trifluoromethyl)-5,6,7,8-tetrahydro[1,2,4]triazolo[4,3-a]pyridin- 3(2H)-one and of the structural formula

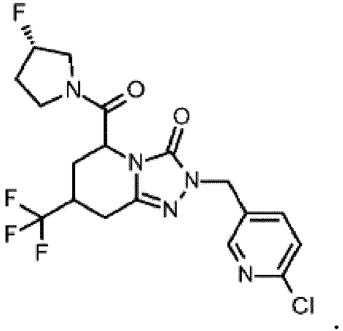

10. Process for preparing a compound as defined in Claims 1 to 5, **characterized in that** either
[A] a compound of the formula (II) 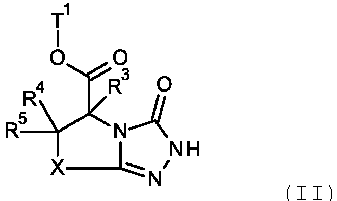 in which R³, R⁴, R⁵ and X each have the definitions given in Claim 1,
and
T¹ is (C₁-C₄) -alkyl or benzyl,
is reacted in an inert solvent in the presence of a suitable base with a compound of the formula (III) 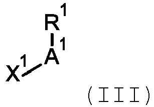 in which
A¹ is -CH₂-, -CH (CH₃)-, -CH₂CH₂-, #⁵-CH₂CH (CH₃)-***, #⁵-CH₂C(CH₃)₂-***, #⁵-CH₂CHF-*** or #⁵-CH₂CF₂-***,
where #⁵ marks the bond to X¹,
where *** marks the bond to the R¹ group,
R¹ has the definitions given in Claim 1,
and
X¹ is a suitable leaving group, especially chlorine, bromine, iodine, mesylate {(methylsulfonyl)oxy}, triflate {[(trifluoromethyl)sulfonyl]oxy}, nonaflate {[(nonafluorobutyl)sulfonyl]-oxy}, nosylate {[(4-nitrophenyl)sulfonyl]oxy} or tosylate {[(4-methylphenyl)sulfonyl]oxy},
to give a compound of the formula (IV) 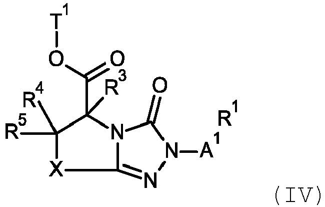 in which A¹, R¹, R³, R⁴, R⁵ and X each have the definitions given in Claim 1
and
T¹ is (C₁-C₄) -alkyl or benzyl,
then the latter are converted by removing the "T¹" group in an inert solvent in the presence of a suitable base or acid to a compound of the formula (V) 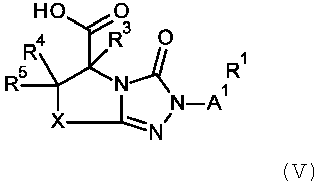 in which A¹, R¹, R³, R⁴, R⁵ and X each have the definitions given in Claim 1, and then the latter is reacted in an inert solvent under amide coupling conditions with an amine of the formula (VI-A) or (VI-B) 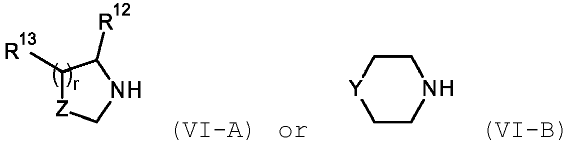 in which Y, Z, R¹² and R¹³ each have the definitions given in Claim 1,
and then any protecting groups present are detached by methods known to those skilled in the art, and the resulting compounds of the formula (I) are optionally converted with the appropriate (i) solvents and/or (ii) acids or bases to the solvates, salts and/or solvates of the salts thereof, or
[B] a compound of the formula (VII) 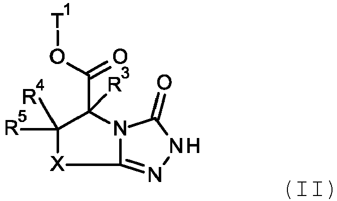 in which A¹, R¹, R³, R⁴, R⁵ and X each have the definitions given in Claim 1,
and
T¹ is (C₁-C₄) -alkyl or benzyl,
is reacted in an inert solvent in the presence of a suitable base with
an amine of the formula (VI-A) or (VI-B) 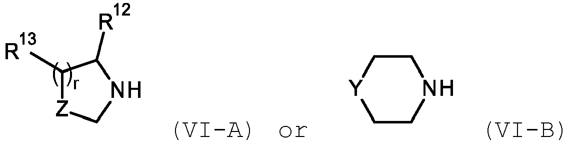 in which r, Y, Z, R¹² and R¹³ each have the definitions given in Claim 1,
to give a compound of the formula (XXIII-A) or (XXIII-B) 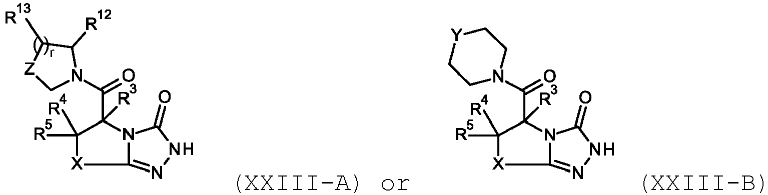 in which r, R³, R⁴, R⁵, R¹², R¹³, X, Y and Z each have the definitions given in Claim 1,
the latter is reacted in an inert solvent in the presence of a suitable base with a compound of the formula (III) 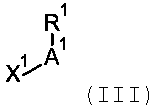 in which
A¹ is -CH₂-, -CH(CH₃)-, -CH₂CH₂-, #⁵-CH₂CH (CH₃)-***, #⁵-CH₂C(CH₃)₂-***, #⁵-CH₂CHF-*** or #⁵-CH₂CF₂-***,
where #⁵ marks the bond to X¹,
where *** marks the bond to the R¹ group,
R¹ has the definitions given in Claim 1,
and
X¹ is a suitable leaving group, especially chlorine, bromine, iodine, mesylate {(methylsulfonyl)oxy}, triflate {[(trifluoromethyl)sulfonyl]oxy}, nonaflate {[(nonafluorobutyl)sulfonyl]-oxy}, nosylate {[(4-nitrophenyl)-sulfonyl]oxy} or tosylate {[(4-methylphenyl)sulfonyl]oxy},
and then any protecting groups present are detached by methods known to those skilled in the art, and the resulting compounds of the formula (I) are optionally converted with the appropriate (i) solvents and/or (ii) acids or bases to the solvates, salts and/or solvates of the salts thereof, or
[C] a compound of the formula (VII) 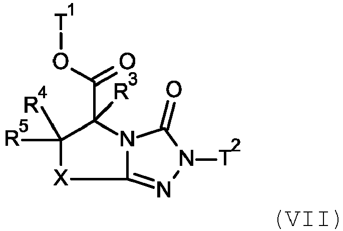 in which R³, R⁴, R⁵ and X each have the definitions given in Claim 1 and
T¹ is (C₁-C₄) -alkyl or benzyl,
T² is 4-methoxybenzyl, benzyl, allyl, β-(trimethylsilyl)ethoxymethyl (SEM), methoxymethyl (MOM) or benzyloxymethyl,
is converted by hydrolysis of the ester group in an inert solvent in the presence of a suitable base or acid to a compound of the formula (VIII) 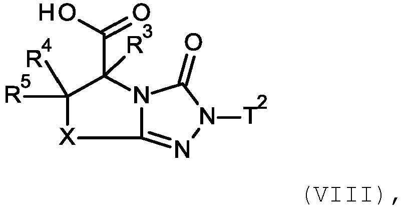 in which R³, R⁴, R⁵ and X each have the definitions given in Claim 1
and
T² is 4-methoxybenzyl, benzyl, allyl, β-(trimethylsilyl)ethoxymethyl (SEM), methoxymethyl (MOM) or benzyloxymethyl,
then the latter is reacted in an inert solvent under amide coupling conditions with an amine of the formula (VI-A) 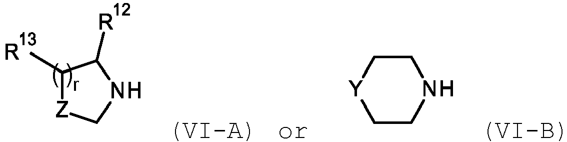 in which r, Y, Z, R¹² and R¹³ each have the definitions given in Claim 1,
to give a compound of the formula (IX-A) or (IX-B) 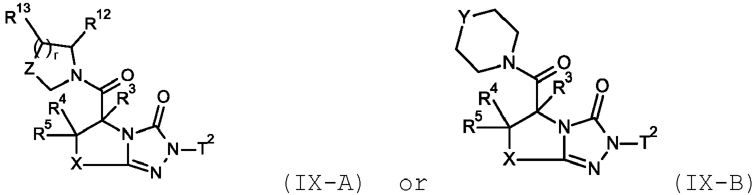 in which r, R³, R⁴, R⁵, R¹², R¹³, T², X, Y and Z each have the definitions given in Claim 1, the protecting group "T²" is removed in an inert solvent in the presence of a suitable base or acid or optionally in the presence of a suitable palladium catalyst and the resulting compound of the formula (X-A) or (X-B) 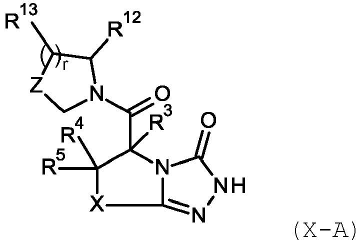 or 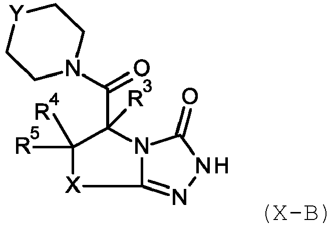 in which r, R³, R⁴, R⁵, R¹², R¹³, X, Y and Z each have the definitions given in Claim 1,
is reacted in an inert solvent in the presence of a suitable base with a compound of the formula (III) 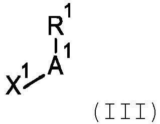 in which
A¹ is -CH₂-, -CH (CH₃)-, -CH₂CH₂-, #⁵-CH₂CH(CH₃)-***, #⁵-CH₂C(CH₃)₂-***, #⁵-CH₂CHF-*** or #⁵-CH₂CF₂-***,
where #⁵ marks the bond to X¹,
where *** marks the bond to the R¹ group,
R¹ has the definitions given in Claim 1,
and
X¹ is a suitable leaving group, especially chlorine, bromine, iodine, mesylate {(methylsulfonyl)oxy}, triflate {[(trifluoromethyl)sulfonyl]oxy}, nonaflate {[(nonafluorobutyl)sulfonyl]-oxy}, nosylate {[(4-nitrophenyl)sulfonyl]oxy} or tosylate {[(4-methylphenyl)sulfonyl]oxy},
and then any protecting groups present are detached by methods known to those skilled in the art, and the resulting compounds of the formula (I) are optionally converted with the appropriate (i) solvents and/or (ii) acids or bases to the solvates, salts and/or solvates of the salts thereof.

11. Compound as defined in any of Claims 1 to 9 for treatment and/or prophylaxis of diseases.

12. Compound as defined in Claims 1 to 9 for use in a method for treatment and/or prophylaxis of lung inflammation disorders, particularly of chronic-obstructive lung disease (COPD), of pulmonary emphysema, of chronic bronchitis, of bronchiectasis, of pulmonary hypertension in COPD (PH-COPD), of acute exacerbation in COPD, of cystic fibrosis (mucoviscidosis, CF), of asthma, and idiopathic pulmonary fibrosis (IPF), of bronchiolitis obliterans syndrome (BOS), of arteriosclerosis, of myocarditis, and of inflammatory skin and eye disorders or inflammatory disorders of the internal organs.

13. Compound of the formula (I) as defined in Claims 1 to 9 for production of a medicament for treatment and/or prophylaxis of lung inflammation disorders, particularly of chronic-obstructive lung disease (COPD), of pulmonary emphysema, of chronic bronchitis, of bronchiectasis, of pulmonary hypertension in COPD (PH-COPD), of acute exacerbation in COPD, of cystic fibrosis (mucoviscidosis, CF), of asthma, and idiopathic pulmonary fibrosis (IPF), of bronchiolitis obliterans syndrome (BOS), of arteriosclerosis, of myocarditis, and of inflammatory skin and eye disorders or inflammatory disorders of the internal organs.

14. Medicament comprising a compound as defined in any of Claims 1 to 9 in combination with one or more inert, nontoxic, pharmaceutically suitable excipients.

15. Medicament comprising a compound as defined in any of Claims 1 to 9 in combination with one or more active ingredients selected from the group of the PDE 5 inhibitors, sGC activators, sGC stimulators, prostacyclin analogues, IP receptor agonists, endothelin antagonists, compounds that inhibit the signal transduction cascade, and pirfenidone.

16. Medicament according to Claim 14 or 15 for treatment and/or prophylaxis of lung inflammation disorders, particularly of chronic-obstructive lung disease (COPD), of pulmonary emphysema, of chronic bronchitis, of bronchiectasis, of pulmonary hypertension in COPD (PH-COPD), of acute exacerbation in COPD, of cystic fibrosis (mucoviscidosis, CF), of asthma, and idiopathic pulmonary fibrosis (IPF), of bronchiolitis obliterans syndrome (BOS), of arteriosclerosis, of myocarditis, and of inflammatory skin and eye disorders or inflammatory disorders of the internal organs.

## Revendications

1. Composé de formule (I) 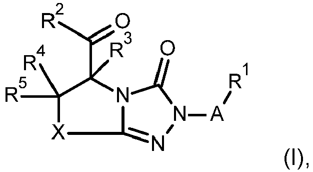 dans laquelle
A représente (C₁-C₄) -alkylène ou CD₂,
(C₁-C₄)-alkylène pouvant être substitué par hydroxy,
(C₁-C₄)-alcoxy ainsi que jusqu'à pentasubstitué par fluor, ou
représente un groupe de formule 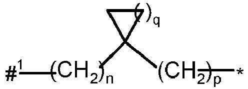 dans laquelle
n représente 0 ou 1,
p représente 0 ou 1,
q représente 1 ou 2,
#¹ marquant la liaison à l'atome d'azote du cycle 5-oxo-4,5-dihydro-1H-1,2,4-triazol-1-yle,
* marquant la liaison à R¹,
X représente -CR⁶R⁷-, #²-CR⁶R⁷-CR⁸R⁹-** , #²-CR⁶-CR⁸-** ou #²-CR⁶R⁷-CR⁸R⁹-CR¹⁰R¹¹-**,
#² marquant la liaison à l'atome de carbone du groupe CR⁴R⁵,
** marquant la liaison à l'atome de carbone du cycle 5-oxo-4,5-dihydro-1H-1,2,4-triazol-1-yle
R⁶ représentant hydrogène, fluor, (C₁-C₄) -alkyle, (C₁-C₄) -alcoxy, trifluorométhoxy, hydroxy, mono-(C₁-C₄)-alkylamino ou di-(C₁-C₄)-alkylamino,
où (C₁-C₄)-alkyle peut être substitué par (C₁-C₄) -alcoxy, hydroxy, mono-(C₁-C₄)-alkylamino ou di-(C₁-C₄)-alkylamino ainsi que jusqu'à pentasubstitué par fluor,
R⁷ représentant hydrogène, fluor ou (C₁-C₄)-alkyle,
où (C₁-C₄)-alkyle peut être jusqu'à pentasubstitué par fluor, ou
R⁶ et R⁷ formant, conjointement avec l'atome de carbone auquel ils sont liés, un groupe carbonyle ou
R⁶ et R⁷ formant, conjointement avec l'atome de carbone auquel ils sont liés, un cycle cyclopropyle, cyclobutyle ou cyclopentyle ou
R⁶ et R⁴ formant, conjointement avec l'atome de carbone auquel ils sont liés, un cycle cyclopropyle, cyclobutyle ou cyclopentyle,
R⁸ représentant hydrogène, (C₁-C₄) -alkyle, (C₁-C₄)-alcoxy, fluor, hydroxy, mono-(C₁-C₄)-alkylamino ou di-(C₁-C₄)-alkylamino,
où (C₁-C₄)-alkyle peut être substitué par (C₁-C₄) -alcoxy, hydroxy, mono- (C₁-C₄)-alkylamino ou di-(C₁-C₄)-alkylamino ainsi que jusqu'à pentasubstitué par fluor,
R⁹ représentant hydrogène, fluor ou (C₁-C₄)-alkyle,
où (C₁-C₄)-alkyle peut être jusqu'à pentasubstitué par fluor, ou
R⁸ et R⁹ formant, conjointement avec l'atome de carbone auquel ils sont liés, un cycle cyclopropyle, cyclobutyle ou cyclopentyle ou
R⁷ et R⁹ formant, conjointement avec les atomes de carbone auxquels ils sont liés, un cycle cyclopropyle, cyclobutyle ou cyclopentyle,
R¹⁰ représentant hydrogène, (C₁-C₄) -alkyle, (C₁-C₄)-alcoxy, fluor, hydroxy, mono-(C₁-C₄)-alkylamino ou di-(C₁-C₄)-alkylamino,
où (C₁-C₄)-alkyle peut être substitué par (C₁-C₄) -alcoxy, hydroxy, mono-(C₁-C₄)-alkylamino ou di-(C₁-C₄)-alkylamino ainsi que jusqu'à pentasubstitué par fluor,
R¹¹ représentant hydrogène, fluor ou (C₁-C₄)-alkyle,
où (C₁-C₄)-alkyle peut être jusqu'à pentasubstitué par fluor, ou
R¹⁰ et R¹¹ formant, conjointement avec l'atome de carbone auquel ils sont liés, un cycle cyclopropyle, cyclobutyle ou cyclopentyle,
R¹ représente phényle ou hétéroaryle de 5 à 6 chaînons, phényle étant substitué par 1 à 3 substituants choisis, indépendamment les uns des autres, dans le groupe formé par fluor, chlore, cyano, méthyle, éthyle, trifluorométhyle, méthoxy, difluorométhoxy, trifluorométhoxy, hydroxycarbonyle, méthoxycarbonyle, éthoxycarbonyle, tert-butoxycarbonyle, méthylaminocarbonyle, diméthylaminocarbonyle, méthylsulfanyle, méthylsulfonyle, méthylsulfonimidoyle, aminosulfonyle ou méthylsulfinyle, ou
phényle pouvant être condensé avec cyclopentyle, cyclohexyle, hétérocyclyle de 5 à 6 chaînons ou hétéroaryle de 5 à 6 chaînons,
où cyclopentyle, cyclohexyle, (C₅-C₆)-hétérocyclyle et hétéroaryle de 5 à 6 chaînons peuvent être substitués par 1 ou 2 substituants méthyle ou éthyle ou
hétéroaryle de 5 à 6 chaînons pouvant être substitué par 1 à 3 substituants choisis, indépendamment les uns des autres, dans le groupe formé par fluor, chlore, cyano, méthyle, éthyle, trifluorométhyle, méthoxy, éthoxy, difluorométhoxy, trifluorométhoxy, 2,2,2-trifluoroéthoxy, méthylaminocarbonyle, tert-butylaminocarbonyle, diméthylaminocarbonyle, hétéroaryle de 5 à 6 chaînons pouvant être condensé avec cyclopentyle, cyclohexyle, hétérocyclyle de 5 à 6 chaînons, phényle ou hétéroaryle de 5 à 6 chaînons,
où hétéroaryle de 5 à 6 chaînons peut être substitué par méthyle, éthyle, chlore, fluor ou méthoxy,
où cyclopentyle, cyclohexyle, hétérocyclyle de 5 à 6 chaînons, phényle et hétéroaryle de 5 à 6 chaînons peuvent être substitués par 1 ou 2 substituants (C₁-C₄)-alkyle,
R² représente un groupe de formule 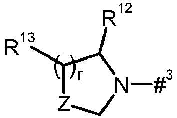 dans laquelle
#³ marque la liaison avec l'atome de carbone à fonction carbonyle,
r représente 0 ou 1,
Z représente O, NR¹⁸, S, SO, SO₂ ou CR^{14A}R^{14B},
où
R^{14A} représente hydrogène, halogène, cyano, (C₁-C₄)-alkyle, (C₃-C₆) -cycloalkyle, trifluorométhyle, difluorométhyle, fluorométhyle, hydroxy, (C₁-C₄) -alcoxy, (C₃-C₅) -cycloalcoxy, difluorométhoxy, trifluorométhoxy ou 2,2,2-trifluoroéthoxy,
où (C₁-C₄)-alkyle peut être substitué par hydroxy, amino, mono-(C₁-C₄)-alkylamino ou di-(C₁-C₄) -alkylamino,
R^{14B} représente hydrogène, fluor ou (C₁-C₄)-alkyle,
où (C₁-C₄)-alkyle peut être jusqu'à pentasubstitué par fluor, ou
R^{14A} et R^{14B} forment, conjointement avec l'atome de carbone auquel ils sont liés, un groupe carbonyle,
R¹⁸ représente hydrogène ou méthyle,
R¹² représente hydrogène, cyano, (C₁-C₄) -alkyle, acétyle ou formyle,
où (C₁-C₄)-alkyle peut être substitué par hydroxy ou jusqu'à pentasubstitué par fluor,
où acétyle peut être substitué par hydroxy ou jusqu'à trisubstitué par fluor,
R¹³ représente hydrogène, fluor ou (C₁-C₄) -alkyle ou
R¹² et R¹³ forment, conjointement avec les atomes de carbone auxquels ils sont liés, un cycle cyclopropyle ou cyclobutyle,
où le cycle cyclopropyle ou cyclobutyle peut être jusqu'à disubstitué par fluor ou
R¹³ et R^{14A} forment, conjointement avec les atomes de carbone auxquels ils sont liés, un cycle cyclopropyle ou cyclobutyle,
où le cycle cyclopropyle ou cyclobutyle peut être jusqu'à disubstitué par fluor ou
R^{14A} forment, conjointement avec l'atome de carbone auquel ils sont liés, un cycle cyclopropyle ou cyclobutyle,
où le cycle cyclopropyle ou cyclobutyle peut être jusqu'à disubstitué par fluor,
et R^{14B} R¹³, R^{14A} et R^{14B} représentant hydrogène lorsque
R¹² ne représente pas hydrogène,
R¹² représentant hydrogène lorsqu'un des substituants R¹³, R^{14A} ou R^{14B} ne représente pas hydrogène ou
représente un groupe de formule 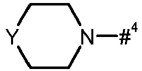 dans laquelle
#⁴ marque la liaison avec l'atome de carbone à fonction carbonyle,
Y représente NR¹⁵, CR^{16A}R^{16B}, oxygène ou soufre,
où
R¹⁵ représente hydrogène ou méthyle,
R^{16A} représente hydrogène ou méthyle,
R^{16B} représente hydrogène ou méthyle,
R³ représente hydrogène ou (C₁-C₄)-alkyle,
R⁴ représentant hydrogène, (C₁-C₄) -alkyle, (C₁-C₄)-alcoxy, fluor, hydroxy, mono- (C₁-C₄) -alkylamino ou di-(C₁-C₄)-alkylamino,
où (C₁-C₄)-alkyle peut être substitué par (C₁-C₄)-alcoxy, hydroxy, mono-(C₁-C₄)-alkylamino ou di-(C₁-C₄)-alkylamino ainsi que jusqu'à pentasubstitué par fluor,
R⁵ représente hydrogène ou (C₁-C₄)-alkyle,
où (C₁-C₄)-alkyle peut être jusqu'à pentasubstitué par fluor, ou
R⁴ et R⁵ forment, conjointement avec l'atome de carbone auquel ils sont liés, un groupe carbonyle ou
R⁴ et R⁵ forment, conjointement avec l'atome de carbone auquel ils sont liés, un cycle cyclopropyle, cyclobutyle ou cyclopentyle,
ainsi que les sels, solvates et solvates des sels des composés de formule (I).

2. Composé de formule (I) selon la revendication 1, dans laquelle
A représente -CH₂-, -CH(CH₃)-, -CH₂CH₂-, #⁵-CH₂CH(CH₃)-***, #⁵-CH₂C(CH₃)₂-***, #⁵-CH₂CHF-*** ou #⁵-CH₂CF₂-***,
#⁵ marquant la liaison à l'atome d'azote du cycle 5-oxo-4,5-dihydro-1H-1,2,4-triazol-1-yle,
*** marquant la liaison au groupe R¹,
X représente - #²-CR⁶R⁷-CR⁸R⁹-**,
#² marquant la liaison à l'atome de carbone du groupe CR⁴R⁵,
** marquant la liaison à l'atome de carbone du cycle 5-oxo-4,5-dihydro-1H-1,2,4-triazol-1-yle,
R⁶ représentant hydrogène, fluor, méthyle, trifluorométhyle ou hydroxy,
R⁷ représentant hydrogène, fluor ou méthyle ou
R⁶ et R⁷ formant, conjointement avec l'atome de carbone auquel ils sont liés, un cycle cyclopropyle,
R⁸ représentant hydrogène, fluor, méthyle ou trifluorométhyle,
R⁹ représentant hydrogène, fluor ou méthyle,
R¹ représente phényle ou hétéroaryle de 5 à 6 chaînons, phényle étant substitué par 1 à 3 substituants choisis, indépendamment les uns des autres, dans le groupe formé par fluor, chlore, cyano, méthyle, éthyle, trifluorométhyle, méthoxy, difluorométhoxy, trifluorométhoxy, hydroxycarbonyle, méthoxycarbonyle, éthoxycarbonyle, tert-butoxycarbonyle, méthylaminocarbonyle, diméthylaminocarbonyle, méthylsulfanyle, méthylsulfonyle, méthylsulfonimidoyle, aminosulfonyle ou méthylsulfinyle ou
phényle pouvant être condensé avec cyclopentyle, cyclohexyle, hétérocyclyle de 5 à 6 chaînons ou hétéroaryle de 5 à 6 chaînons,
où cyclopentyle, cyclohexyle, (C₅-C₆)-hétérocyclyle et hétéroaryle de 5 à 6 chaînons peuvent être substitués par 1 ou 2 substituants méthyle ou éthyle ou
hétéroaryle de 5 à 6 chaînons pouvant être substitué par 1 à 3 substituants choisis, indépendamment les uns des autres, dans le groupe formé par fluor, chlore, cyano, méthyle, éthyle, trifluorométhyle, méthoxy, éthoxy, difluorométhoxy, trifluorométhoxy, 2,2,2-trifluoroéthoxy, méthylaminocarbonyle, tert-butylaminocarbonyle et diméthylaminocarbonyle,
hétéroaryle de 5 à 6 chaînons pouvant être condensé avec cyclopentyle, cyclohexyle, hétérocyclyle de 5 à 6 chaînons, phényle ou hétéroaryle de 5 à 6 chaînons,
où hétéroaryle de 5 à 6 chaînons peut être substitué par méthyle, éthyle, chlore, fluor ou méthoxy,
où cyclopentyle, cyclohexyle, hétérocyclyle de 5 à 6 chaînons, phényle et hétéroaryle de 5 à 6 chaînons peuvent être substitués par 1 ou 2 substituants (C₁-C₄)-alkyle,
R² représente un groupe de formule 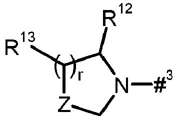 dans laquelle
#³ marque la liaison avec l'atome de carbone à fonction carbonyle,
r représente 0 ou 1,
Z représente S ou CR^{14A}R^{14B} lorsque r représente 0,
Z représente S, SO, SO₂ ou CR^{14A}R^{14B} lorsque r représente 1,
où, à chaque fois
R^{14A} représente hydrogène, fluor, méthyle, trifluorométhyle, difluorométhyle, fluorométhyle, hydroxy, méthoxy, difluorométhoxy ou trifluorométhoxy,
R^{14B} représente hydrogène ou fluor ou
R^{14A} et R^{14B} forment, conjointement avec
l'atome de carbone auquel ils sont liés, un groupe carbonyle,
R¹² représente hydrogène, cyano, méthyle, acétyle ou formyle,
où acétyle est substitué par hydroxy ou jusqu'à trisubstitué par fluor,
R¹³ représente hydrogène, fluor ou méthyle ou
R¹² et R¹³ forment, conjointement avec les atomes de carbone auxquels ils sont liés, un cycle cyclopropyle ou
R¹³ et R^{14A} forment, conjointement avec l'atome de carbone auquel ils sont liés, un cycle cyclopropyle
où le cycle cyclopropyle peut être jusqu'à disubstitué par fluor ou
R^{14A} et R^{14B} forment, conjointement avec l'atome de carbone auquel ils sont liés, un cycle cyclopropyle,
où le cycle cyclopropyle peut être jusqu'à disubstitué par fluor,
R¹³, R^{14A} et R^{14B} représentant hydrogène lorsque
R¹² ne représente pas hydrogène,
R¹² représentant hydrogène lorsqu'un des substituants R¹³, R^{14A} ou R^{14B} ne représente pas hydrogène
R³ représente hydrogène,
R⁴ représente hydrogène, fluor ou méthyle,
R⁵ représente hydrogène,
ainsi que les sels, solvates et solvates des sels des composés de formule (I).

3. Composé de formule (I) selon la revendication 1 ou 2, dans laquelle
A représente -CH₂-, -CH (CH₃)-, -CH₂CH₂-, #⁵-CH₂CH (CH₃)-***, #⁵-CH₂C(CH₃)₂-***, #⁵-CH₂CHF-*** ou #⁵-CH₂CF₂-***,
#⁵ marquant la liaison à l'atome d'azote du cycle 5-oxo-4,5-dihydro-1H-1,2,4-triazol-1-yle,
*** marquant la liaison au groupe R¹,
X représente - #²-CR⁶R⁷-CR⁸R⁹-**,
#² marquant la liaison à l'atome de carbone du groupe CR⁴R⁵,
** marquant la liaison à l'atome de carbone du cycle 5-oxo-4,5-dihydro-1H-1,2,4-triazol-1-yle,
R⁶ représentant hydrogène, fluor, méthyle, trifluorométhyle ou hydroxy,
R⁷ représentant hydrogène, fluor ou méthyle ou
R⁶ et R⁷ formant, conjointement avec l'atome de carbone auquel ils sont liés, un cycle cyclopropyle,
R⁸ représentant hydrogène, fluor, méthyle ou trifluorométhyle,
R⁹ représentant hydrogène, fluor ou méthyle,
R¹ représente phényle ou hétéroaryle de 5 à 6 chaînons, phényle étant substitué par 1 à 3 substituants choisis, indépendamment les uns des autres, dans le groupe formé par fluor, chlore, cyano, méthyle, éthyle, trifluorométhyle, méthoxy, difluorométhoxy, trifluorométhoxy, hydroxycarbonyle, méthoxycarbonyle, éthoxycarbonyle, tert-butoxycarbonyle, méthylaminocarbonyle, diméthylaminocarbonyle, méthylsulfanyle, méthylsulfonyle, méthylsulfonimidoyle, aminosulfonyle et méthylsulfinyle ou
phényle pouvant être condensé avec cyclopentyle, cyclohexyle, hétérocyclyle de 5 à 6 chaînons ou hétéroaryle de 5 à 6 chaînons,
où cyclopentyle, cyclohexyle, hétérocyclyle de 5 à 6 chaînons et hétéroaryle de 5 à 6 chaînons peuvent être substitués par 1 ou 2 substituants méthyle ou éthyle ou
hétéroaryle de 5 à 6 chaînons pouvant être substitué par 1 à 3 substituants choisis, indépendamment les uns des autres, dans le groupe formé par fluor, chlore, cyano, méthyle, éthyle, trifluorométhyle, méthoxy, éthoxy, difluorométhoxy, trifluorométhoxy, 2,2,2-trifluoroéthoxy, méthylaminocarbonyle, tert-butylaminocarbonyle et diméthylaminocarbonyle,
hétéroaryle de 5 à 6 chaînons pouvant être condensé avec cyclopentyle, cyclohexyle, hétérocyclyle de 5 à 6 chaînons, phényle ou hétéroaryle de 5 à 6 chaînons,
où hétéroaryle de 5 à 6 chaînons peut être substitué par méthyle, éthyle, chlore, fluor ou méthoxy,
où cyclopentyle, cyclohexyle, hétérocyclyle de 5 à 6 chaînons, phényle et hétéroaryle de 5 à 6 chaînons peuvent être substitués par 1 ou 2 substituants (C₁-C₄)-alkyle,
R² représente un roue de formule 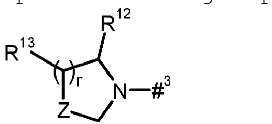 dans laquelle
#³ marque la liaison avec l'atome de carbone à fonction carbonyle,
r représente 0 ou 1,
Z représente S ou CR^{14A}R^{14B} lorsque r représente 0,
Z représente S, SO, SO₂ ou CR^{14A}R^{14B} lorsque r représente 1,
où, à chaque fois
R^{14A} représente hydrogène, fluor, méthyle, trifluorométhyle, difluorométhyle, fluorométhyle, hydroxy, méthoxy, difluorométhoxy ou trifluorométhoxy,
R^{14B} représente hydrogène ou fluor ou
R^{14A} et R^{14B} forment, conjointement avec l'atome de carbone auquel ils sont liés, un groupe carbonyle,
R¹² représente hydrogène, cyano, méthyle, acétyle ou formyle, où acétyle est substitué par hydroxy ou jusqu'à trisubstitué par fluor,
R¹³ représente hydrogène, fluor ou méthyle ou
R¹² et R¹³ forment, conjointement avec les atomes de carbone auxquels ils sont liés, un cycle cyclopropyle ou
R¹³ et R^{14A} forment, conjointement avec l'atome de carbone auquel ils sont liés, un cycle cyclopropyle
où le cycle cyclopropyle peut être jusqu'à disubstitué par fluor ou
R^{14A} et R^{14B} forment, conjointement avec l'atome de carbone auquel ils sont liés, un cycle cyclopropyle,
où le cycle cyclopropyle peut être jusqu'à disubstitué par fluor,
R¹³, R^{14A} et R^{14B} représentant hydrogène lorsque
R¹² ne représente pas hydrogène,
R¹² représentant hydrogène lorsqu'un des substituants R¹³, R^{14A} ou R^{14B} ne représente pas hydrogène
R³ représente hydrogène,
R⁴ représente hydrogène, fluor ou méthyle,
R⁵ représente hydrogène,
ainsi que les sels, solvates et solvates des sels des composés de formule (I).

4. Composé de formule (I) selon la revendication 1 ou 2, dans laquelle
A représente -CH₂-, -CH (CH₃)-, -CH₂CH₂-, #⁵-CH₂CH(CH₃)-***, #⁵-CH₂C(CH₃)₂-***, #⁵-CH₂CHF-*** ou #⁵-CH₂CF₂-***,
#⁵ marquant la liaison à l'atome d'azote du cycle 5-oxo-4,5-dihydro-1H-1,2,4-triazol-1-yle,
*** marquant la liaison au groupe R¹,
X représente - #²-CR⁶R⁷-CR⁸R⁹-**,
#² marquant la liaison à l'atome de carbone du groupe CR⁴R⁵,
** marquant la liaison à l'atome de carbone du cycle 5-oxo-4,5-dihydro-1H-1,2,4-triazol-1-yle,
R⁶ représentant hydrogène, fluor, méthyle, trifluorométhyle ou hydroxy,
R⁷ représentant hydrogène, fluor ou méthyle ou
R⁶ et R⁷ formant, conjointement avec l'atome de carbone auquel ils sont liés, un cycle cyclopropyle,
R⁸ représentant hydrogène, fluor, méthyle ou trifluorométhyle,
R⁹ représentant hydrogène, fluor ou méthyle,
R¹ représente phényle, pyrazolyle, imidazolyle, thiazolyle, thiophényle, oxazolyle, oxadiazolyle ou pyridyle,
phényle étant substitué par 1 à 3 substituants choisis, indépendamment les uns des autres, dans le groupe formé par fluor, chlore, cyano, méthyle, éthyle, trifluorométhyle, méthoxy, difluorométhoxy, trifluorométhoxy, hydroxycarbonyle, méthoxycarbonyle, éthoxycarbonyle, tert-butoxycarbonyle, méthylaminocarbonyle, diméthylaminocarbonyle, méthylsulfanyle, méthylsulfonyle, méthylsulfonimidoyle, aminosulfonyle et méthylsulfinyle ou
phényle pouvant être condensé avec cyclopentyle, cyclohexyle, pyrazolyle ou pyridyle,
où cyclopentyle, cyclohexyle, pyrazolyle ou pyridyle peuvent être substitués par 1 ou 2 substituants méthyle ou éthyle ou
pyrazolyle, imidazolyle, thiazolyle, thiophényle, oxazolyle, oxadiazolyle ou pyridyle pouvant être substitués par 1 à 3 substituants choisis, indépendamment les uns des autres, dans le groupe formé par fluor, chlore, cyano, méthyle, éthyle, trifluorométhyle, méthoxy, éthoxy, difluorométhoxy, trifluorométhoxy, 2,2,2-trifluoroéthoxy, méthylaminocarbonyle, tert-butylaminocarbonyle et diméthylaminocarbonyle,
pyrazolyle, imidazolyle, thiazolyle, thiophényle, oxazolyle, oxadiazolyle ou pyridyle pouvant être condensés avec cyclopentyle, cyclohexyle, phényle ou pyridyle,
où pyridyle peut être substitué par méthyle, éthyle, chlore, fluor ou méthoxy,
où cyclopentyle, cyclohexyle, phényle et pyridyle peuvent être substitués par 1 ou 2 substituants (C₁-C₄)-alkyle,
R² représente un groupe de formule 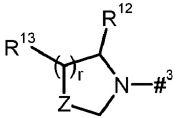 dans laquelle
#³ marque la liaison avec l'atome de carbone à fonction carbonyle,
r représente 0 ou 1,
Z représente S ou CR^{14A}R^{14B} lorsque r représente 0,
Z représente S, SO, SO₂ ou CR^{14A}R^{14B} lorsque r représente 1,
où, à chaque fois
R^{14A} représente hydrogène, fluor, méthyle, trifluorométhyle, difluorométhyle, fluorométhyle, hydroxy, méthoxy, difluorométhoxy ou trifluorométhoxy,
R^{14B} représente hydrogène ou fluor ou
R^{14A} et R^{14B} forment, conjointement avec l'atome de carbone auquel ils sont liés, un groupe carbonyle,
R¹² représente hydrogène, cyano, méthyle, acétyle ou formyle,
où acétyle est substitué par hydroxy ou jusqu'à trisubstitué par fluor,
R¹³ représente hydrogène, fluor ou méthyle ou
R¹² et R¹³ forment, conjointement avec les atomes de carbone auxquels ils sont liés, un cycle cyclopropyle ou
R¹³ et R^{14A} forment, conjointement avec l'atome de carbone auquel ils sont liés, un cycle cyclopropyle
où le cycle cyclopropyle peut être jusqu'à disubstitué par fluor ou
R^{14A} et R^{14B} forment, conjointement avec l'atome de carbone auquel ils sont liés, un cycle cyclopropyle,
où le cycle cyclopropyle peut être jusqu'à disubstitué par fluor,
R¹³, R^{14A} et R^{14B} représentant hydrogène lorsque
R¹² ne représente pas hydrogène,
R¹² représentant hydrogène lorsqu'un des substituants R¹³, R^{14A} ou R^{14B} ne représente pas hydrogène
R³ représente hydrogène,
R⁴ représente hydrogène, fluor ou méthyle,
R⁵ représente hydrogène,
ainsi que les sels, solvates et solvates des sels des composés de formule (I).

5. Composé de formule (I) selon la revendication 1, portant le nom systématique (5RS,7RS)-2-[(6-chloropyridin-3-yl)méthyl]-5-{[(3R,4S)-3,4-difluoropyrrolidin-1-yl]carbonyl}-7-(trifluorométhyl)-5,6,7,8-tétrahydro[1,2,4]triazolo[4,3-a]pyridin-3(2H)-one et présentant la formule développée

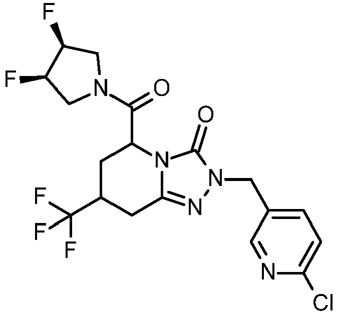

6. Composé de formule (I) selon la revendication 1, portant le nom systématique (5S,7R)-5-{[(3S)-3-fluoropyrrolidin-1-yl]carbonyl}-7-(trifluorométhyl)-2-{[6-(trifluorométhyl)pyridin-3-yl]méthyl}-5,6,7,8-tétrahydro[1,2,4]triazolo[4,3-a]pyridin-3(2H)-one et présentant la formule développée

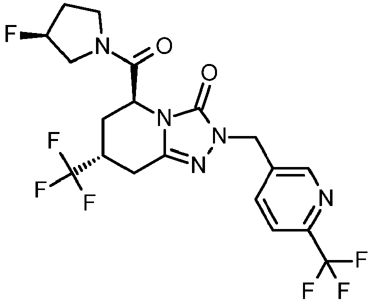

7. Composé de formule (I) selon la revendication 1, portant le nom systématique (5S)-5-{[(3R,4S)-3,4-difluoropyrrolidin-1-yl]carbonyl}-2-{[3-fluoro-2-(trifluorométhyl)pyridin-4-yl]méthyl}-5,6,7,8-tétrahydro[1,2,4]triazolo[4,3-a]pyridin-3(2H)-one et présentant la formule développée

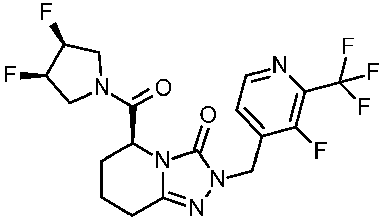

8. Composé de formule (I) selon la revendication 1, portant le nom systématique (5S)-5-{[(3S)-3-fluoropyrrolidin-1-yl]carbonyl}-2-{[6-(trifluorométhyl)pyridin-3-yl]méthyl}-5,6,7,8-tétrahydro[1,2,4]triazolo[4,3-a]pyridin-3(2H)-one et présentant la formule développée

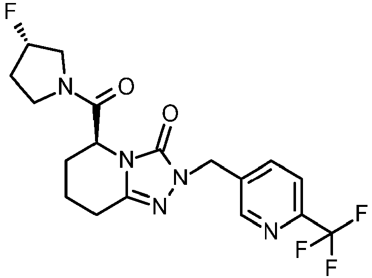

9. Composé de formule (I) selon la revendication 1, portant le nom systématique (5RS,7RS)-2-[(6-chloropyridin-3-yl)méthyl]-5-{[(3S)-3-fluoropyrrolidin-1-yl]carbonyl}-7-(trifluorométhyl)-5,6,7,8-tétrahydro[1,2,4]triazolo[4,3-a]pyridin-3(2H)-one et présentant la formule développée

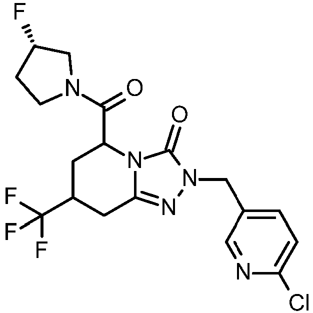

10. Procédé pour la préparation d'un composé tel que défini dans les revendications 1 à 5, **caractérisé en ce qu'**on transforme soit
[A] un composé de formule (II) 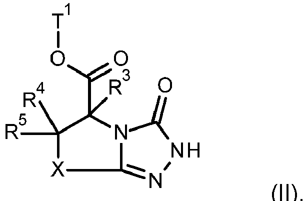 dans laquelle R³, R⁴, R⁵ et X présentent à chaque fois les significations mentionnées dans la revendication 1 et
T¹ représente (C₁-C₄) -alkyle ou benzyle,
dans un solvant inerte en présence d'une base appropriée avec un composé de formule (III) 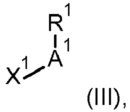 dans laquelle
A¹ représente -CH₂-, -CH(CH₃)-, -CH₂CH₂-, #⁵-CH₂CH(CH₃)-***, #⁵-CH₂C(CH₃)₂-***, #⁵-CH₂CHF-*** ou #⁵-CH₂CF₂-***,
#⁵ marquant la liaison à X¹,
*** marquant la liaison au groupe R¹,
R¹ présente les significations mentionnées dans la revendication 1 et
X¹ représente un groupe partant approprié, en particulier chlore, brome, iode, mésylate {(méthylsulfonyl)oxy}, triflate {[(trifluorométhyl)sulfonyl]oxy}, nonaflate {[(nonafluorobutyl)sulfonyl]oxy}, nosylate {[(4-nitrophényl)sulfonyl]oxy} ou tosylate {[(4-méthylphényl)sulfonyl]oxy},
en un composé de formule (IV) 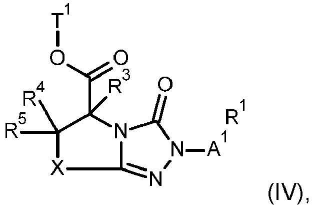 dans laquelle A¹, R¹, R³, R⁴, R⁵ et X présentent à chaque fois les significations mentionnées dans la revendication 1 et
T¹ représente (C₁-C₄) -alkyle ou benzyle,
puis on transforme celui-ci, par élimination du groupe "T¹" dans un solvant inerte en présence d'une base ou d'un acide approprié (e) en un composé de formule (V) 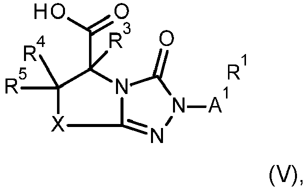 dans laquelle A¹, R¹, R³, R⁴, R⁵ et X présentent à chaque fois les significations mentionnées dans la revendication 1
et on transforme celui-ci ensuite dans un solvant inerte dans des conditions de couplage d'amide avec une amine de formule (VI-A) ou (VI-B), 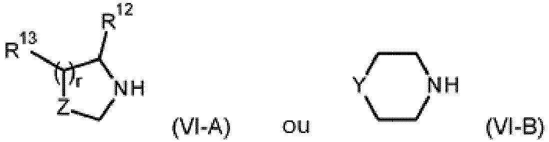 dans lesquelles r, Y, Z, R¹² et R¹³ présentent à chaque fois les significations mentionnées dans la revendication 1,
et ensuite, on dissocie les groupes de protection le cas échéant présents selon des procédés connus par l'homme du métier et on transforme les composés obtenus de formule (I) le cas échéant avec (i) les solvants et/ou (ii) les bases ou acides correspondants en leurs solvates, sels et/ou solvates des sels, soit
[B] un composé de formule (II) 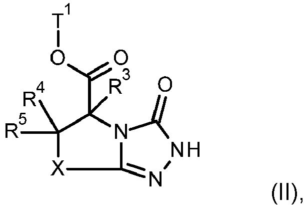 dans laquelle R³, R⁴, R⁵ et X présentent à chaque fois les significations mentionnées dans la revendication 1 et
T¹ représente (C₁-C₄) -alkyle ou benzyle,
dans un solvant inerte en présence d'une base appropriée avec une amine de formule (VI-A) ou (VI-B), 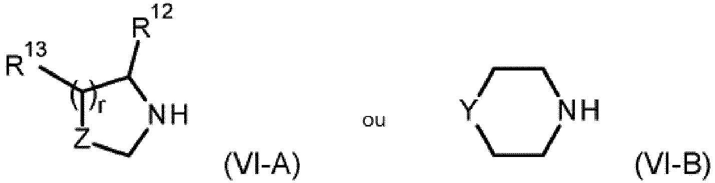 dans lesquelles r, Y, Z, R¹² et R¹³ présentent à chaque fois les significations mentionnées dans la revendication 1,
en un composé de formule (XXIII-A) ou (XXIII-B), 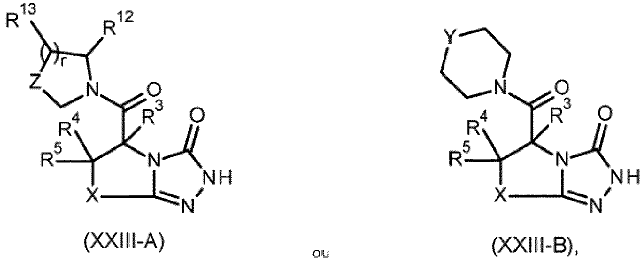 dans lesquelles r, R³, R⁴, R⁵, R¹², R¹³, X, Y et Z présentent à chaque fois les significations mentionnées dans la revendication 1,
on transforme celui-ci dans un solvant inerte en présence d'une base appropriée avec un composé de formule (III) 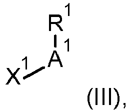 dans laquelle
A¹ représente -CH₂-, -CH (CH₃)-, -CH₂CH₂-, #⁵-CH₂CH (CH₃)-***, #⁵-CH₂C(CH₃)₂-***, #⁵-CH₂CHF-*** ou #⁵-CH₂CF₂-***,
#5 marquant la liaison à X¹,
*** marquant la liaison au groupe R¹,
R¹ présente les significations mentionnées dans la revendication 1 et
X¹ représente un groupe partant approprié, en particulier chlore, brome, iode, mésylate {(méthylsulfonyl)oxy}, triflate {[(trifluorométhyl)sulfonyl]oxy}, nonaflate {[(nonafluorobutyl)sulfonyl]oxy}, nosylate {[(4-nitrophényl)sulfonyl]oxy} ou tosylate {[(4-méthylphényl)sulfonyl]oxy},
et ensuite, on dissocie les groupes de protection le cas échéant présents selon des procédés connus par l'homme du métier et on transforme les composés obtenus de formule (I) le cas échéant avec (i) les solvants et/ou (ii) les bases ou acides correspondants en leurs solvates, sels et/ou solvates des sels, soit
[C] un composé de formule (VII) 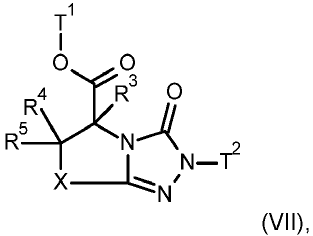 dans laquelle R³, R⁴, R⁵ et X présentent à chaque fois les significations mentionnées dans la revendication 1 et
T¹ représente (C₁-C₄)-alkyle ou benzyle,
T² représente 4-méthoxy-benzyle, benzyle, allyle, β-(triméthylsilyl)éthoxyméthyle (SEM), méthoxyméthyle (MOM) ou benzyloxyméthyle,
par hydrolyse du groupe ester dans un solvant inerte en présence d'une base ou d'un acide approprié(e) en un composé de formule (VIII) 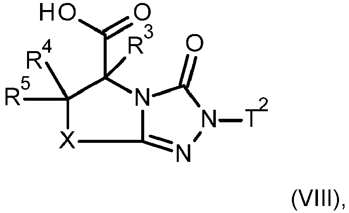 dans laquelle R³, R⁴, R⁵ et X présentent à chaque fois les significations mentionnées dans la revendication 1 et ci-dessus et
T² représente 4-méthoxy-benzyle, benzyle, allyle, β-(triméthylsilyl)éthoxyméthyle (SEM), méthoxyméthyle (MOM) ou benzyloxyméthyle,
on transforme celui-ci ensuite dans un solvant inerte dans des conditions de couplage d'amide avec une amine de formule (VI-A), 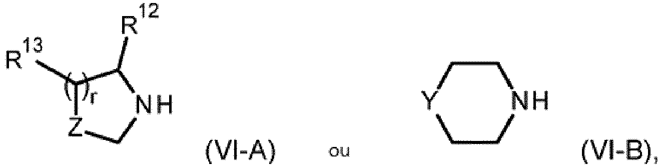 dans laquelle r, Y, Z, R¹² et R¹³ présentent à chaque fois les significations mentionnées dans la revendication 1,
en un composé de formule (IX-A) ou (IX-B), 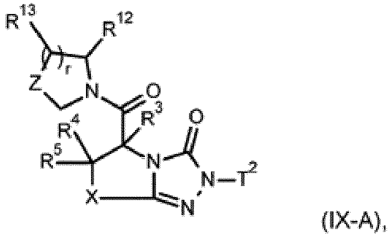 ou 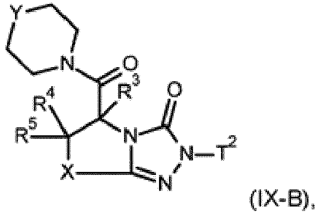 dans lesquelles r, R³, R⁴, R⁵, R¹², R¹³, T², X, Y et Z présentent à chaque fois les significations susmentionnées,
on élimine le groupe de protection "T²" dans un solvant inerte en présence d'une base ou d'un acide approprié(e) ou le cas échéant en présence d'un catalyseur à base de palladium approprié et on transforme le composé obtenu de formule (X-A) ou (X-B), 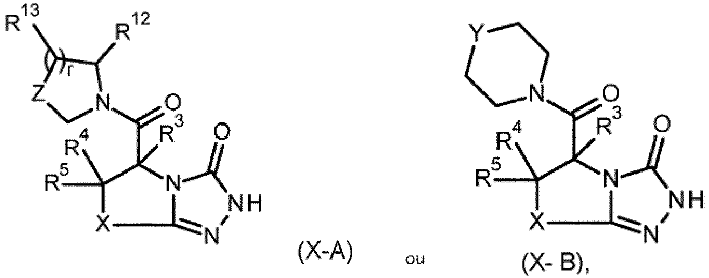 dans lesquelles r, R³, R⁴, R⁵, R¹², R¹³, X, Y et Z présentent à chaque fois les significations mentionnées dans la revendication 1,
dans un solvant inerte en présence d'une base appropriée avec un composé de formule (III) 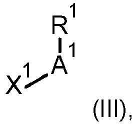 dans laquelle
A¹ représente -CH₂-, -CH (CH₃)-, -CH₂CH₂-, #⁵-CH₂CH (CH₃)-***, #⁵-CH₂C(CH₃)₂-***, #⁵-CH₂CHF-*** ou #⁵-CH₂CF₂-***,
#⁵ marquant la liaison à X¹,
*** marquant la liaison au groupe R¹,
R¹ présente les significations mentionnées dans la revendication 1 et
X¹ représente un groupe partant approprié, en particulier chlore, brome, iode, mésylate {(méthylsulfonyl)oxy}, triflate {[(trifluorométhyl)sulfonyl]oxy}, nonaflate {[(nonafluorobutyl)sulfonyl]oxy}, nosylate {[(4-nitrophényl)sulfonyl]oxy} ou tosylate {[(4-méthylphényl)sulfonyl]oxy},
et ensuite, on dissocie les groupes de protection le cas échéant présents selon des procédés connus par l'homme du métier et on transforme les composés obtenus de formule (I) le cas échéant avec (i) les solvants et/ou (ii) les bases ou acides correspondants en leurs solvates, sels et/ou solvates des sels.

11. Composé tel que défini dans l'une quelconque des revendications 1 à 9, destiné au traitement et/ou à la prophylaxie de maladies.

12. Composé, tel que défini dans l'une quelconque des revendications 1 à 9, destiné à une utilisation dans un procédé pour le traitement et/ou la prophylaxie de maladies pulmonaires inflammatoires, principalement la bronchopneumopathie chronique obstructive (BPCO), de l'emphysème pulmonaire, de la bronchite chronique, de la bronchiectasie, de l'hypertonie pulmonaire dans la BPCO (HP-BPCO), de l'exacerbation aiguë dans la BPCO, de la fibrose kystique (mucoviscidose, FK), de l'asthme ainsi que de la fibrose pulmonaire idiopathique (FPI), du syndrome de la bronchiolite oblitérante (SBO), de l'artériosclérose, de la myocardite ainsi que de maladies inflammatoires de la peau et des yeux ou de maladies inflammatoires des organes internes.

13. Composé de formule (I), tel que défini dans les revendications 1 à 9, destiné à la préparation d'un médicament pour le traitement et/ou la prophylaxie de maladies pulmonaires inflammatoires, principalement la bronchopneumopathie chronique obstructive (BPCO), de l'emphysème pulmonaire, de la bronchite chronique, de la bronchiectasie, de l'hypertonie pulmonaire dans la BPCO (HP-BPCO), de l'exacerbation aiguë dans la BPCO, de la fibrose kystique (mucoviscidose, FK), de l'asthme ainsi que de la fibrose pulmonaire idiopathique (FPI), du syndrome de la bronchiolite oblitérante (SBO), de l'artériosclérose, de la myocardite ainsi que de maladies inflammatoires de la peau et des yeux ou de maladies inflammatoires des organes internes.

14. Médicament contenant un composé, tel que défini dans l'une quelconque des revendications 1 à 9, en combinaison avec un ou plusieurs adjuvants inertes, non toxiques, pharmaceutiquement appropriés.

15. Médicament, contenant un composé tel que défini dans les revendications 1 à 9, en combinaison avec un ou plusieurs principes actifs choisis dans le groupe formé par les inhibiteurs de PDE 5, les activateurs de sGC, les stimulateurs de sGC, les analogues de la prostacycline, les agonistes du récepteur IP, les antagonistes de l'endothéline, les composés inhibant la cascade de transduction des signaux et la pirfénidone.

16. Médicament selon la revendication 14 ou 15 destiné au traitement et/ou à la prophylaxie de maladies pulmonaires inflammatoires, principalement la bronchopneumopathie chronique obstructive (BPCO), de l'emphysème pulmonaire, de la bronchite chronique, de la bronchiectasie, de l'hypertonie pulmonaire dans la BPCO (HP-BPCO), de l'exacerbation aiguë dans la BPCO, de la fibrose kystique (mucoviscidose, FK), de l'asthme ainsi que de la fibrose pulmonaire idiopathique (FPI), du syndrome de la bronchiolite oblitérante (SBO), de l'artériosclérose, de la myocardite ainsi que de maladies inflammatoires de la peau et des yeux ou de maladies inflammatoires des organes internes.
